(19) Europäisches Patentamt
European Patent Office
Office européen des brevets

(11) EP 3 906 932 A2

(12) EUROPEAN PATENT APPLICATION

(43) Date of publication:
**10.11.2021 Bulletin 2021/45**

(21) Application number: **20214632.0**

(22) Date of filing: **15.09.2016**

(51) Int Cl.:
***A61K 38/05*** (2006.01) ***A61K 38/07*** (2006.01)
***A61K 38/08*** (2019.01) ***A61K 38/10*** (2006.01)
***A61K 38/12*** (2006.01) ***A61K 38/16*** (2006.01)
***A61K 39/395*** (2006.01) ***A61K 45/06*** (2006.01)
***A61K 31/70*** (2006.01) ***C07K 16/28*** (2006.01)
***A61P 35/00*** (2006.01) ***A61K 38/18*** (2006.01)
***A61K 31/7105*** (2006.01) ***A61P 43/00*** (2006.01)
***C12N 15/113*** (2010.01)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **17.09.2015 US 201562219728 P**

(62) Document number(s) of the earlier application(s) in accordance with Art. 76 EPC:
**16766294.9 / 3 349 776**

(71) Applicant: **Histide AG**
**8834 Schindellegi (CH)**

(72) Inventors:
- **ZOUANI, OMAR**
**8834 Schindellegi (CH)**
- **GOCHEVA, Veronika**
**8834 Schindellegi (CH)**

(74) Representative: **Cabinet Beau de Loménie**
**158, rue de l'Université**
**75340 Paris Cedex 07 (FR)**

Remarks:
•The complete document including Reference Tables and the Sequence Listing can be downloaded from the EPO website
•This application was filed on 16.12.2020 as a divisional application to the application mentioned under INID code 62.

# (54) PHARMACEUTICAL ASSOCIATION OF GROWTH FACTOR RECEPTOR AGONIST AND ADHESION PROTEIN INHIBITOR FOR CONVERTING A NEOPLASTIC CELL INTO A NON-NEOPLASTIC CELL AND USES THEREOF

(57) A pharmaceutical association comprising at least one growth factor receptor-binding compound, which activates at least one growth factor receptor of a neoplastic cell, and at least one adhesion protein inhibitor which inhibits at least one transmembrane cell adhesion protein of said neoplastic cell.

FIGURE 1

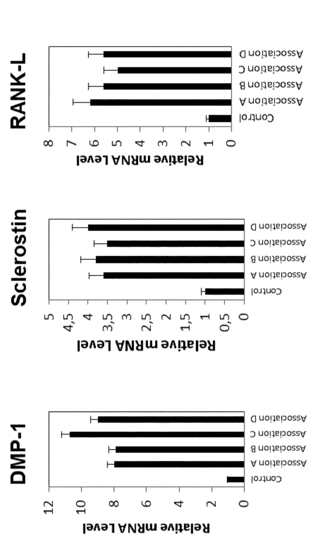


EP 3 906 932 A2

## Description

## FIELD OF THE INVENTION

The invention relates to associations, combinations, compositions, kits, methods and processes for the design, preparation, manufacture and/or formulation thereof, and methods and uses thereof for converting or recoding a neoplastic cell into a non-neoplastic cell and treating and/or preventing a neoplastic disease.

## BACKGROUND

Neoplastic cells, such as cancer cells, are generally characterized by abnormal and/or uncontrolled proliferation leading, in most cases, to the development of a neoplastic disease, such as cancer. Conventional methods for treating neoplastic diseases include surgical treatments, radiotherapy and chemotherapy.

Surgery is usually practised in order to extract localised (non-circulating) neoplastic cells from a patient's body and is most generally combined with radiotherapy treatments. As well as being limited to the treatment of very early stage, non-metastatic tumors, surgery is an invasive medical procedure which remains traumatic for the treated patient, involves the permanent removal of tissues or organs (which is sometimes not possible as some organs or organ parts are not accessible or cannot be removed due to life threatening consequences), in some cases has been shown to "unblock" dormant tumors, while only offering a "visual" selectivity to distinguish between healthy and tumor cells. Radiotherapy also presents some significant drawbacks for the treated patients including the high cost of the radiation therapy equipment, the high cost of the treatment itself, side effects associated with the damage or destruction of healthy cells, limited effectiveness against metastasized neoplastic diseases, skin rashes caused by external beam radiation, the potential deleterious impact on the functioning of tissues, glands or organs located near the site of treatment, and the possible development of secondary cancers as a result of exposure to the radiations.

Conventional chemotherapy methods generally involve the administration of small synthetic regulatory molecules which inhibit specific intracellular target proteins thought to be responsible for the neoplastic phenotype of the cell. One example is the inhibition of tyrosine kinase signal transduction by small molecule inhibitors to regulate uncontrolled cell proliferation. Typical chemotherapy methods also include treatments wherein DNA is covalently altered by e.g. DNA strands crosslinking, or treatments wherein the polymerisation and depolymerisation of microtubules is enhanced prevented thus provoking apoptosis of the damaged cell.

Another method for treating neoplastic diseases includes gene therapy wherein a missing or defective gene is replaced with a functional, healthy copy, which is delivered to the target dysfunctional cells using a "vector." Gene transfer therapy can be done outside the body (ex vivo) by extracting bone marrow or blood from the patient and growing the cells in a laboratory. The corrected copy of the gene is then introduced and allowed to penetrate the cells' DNA before being injected back into the body. Gene transfers can also be done directly inside the patient's body (in vivo). Gene therapy has been applied to a few specific cases of blood cancers (chronic lymphocytic leukemia (CLL), acute lymphocytic leukemia (ALL) and multiple myeloma) through a particular form thereof in which the genetically modified cells were not the neoplastic cells themselves but instead the immune T-cells. Modified T-cells could then target and destroy specific blood cells (neoplastic as well as healthy). The body of the patient is then able to produce healthy blood cells and eventually provide a treatment to certain blood cancer types. However, gene therapy is generally best suited for the treatment of diseases caused by a single defective gene, not neoplastic diseases, which often involve multiple defective genes. Overall, issues such as the correct integration of therapeutic DNA into the genome; the immune system response to the introduction of foreign DNA into the cell; the toxicity, immune and inflammatory responses; gene control and targeting issues of the vectors (usually viral) required to transport the DNA inside the cell; the difficulties associated with the treatment of multigene-associated neoplastic cells; the possibilities of inducing tumors if the DNA is integrated in the wrong place in the genome; and/or the significant cost usually involved with such a therapy, have greatly undermined the development of gene therapy.

Other intracellular therapies have been contemplated for the treatment of neoplastic diseases using, for instance, micro-ribonucleic acids (miRNAs) or small interfering ribonucleic acids (siRNA). Under these conditions, the neoplastic cell is generally forced to down-regulate or repress the expression of one or more specific target genes (*e.g.* oncogenes) thus inhibiting the expression of defective and/or defecting proteins (*e.g.* oncoproteins). One example is the repression of genes encoding key proteins in the proliferation of cancer cells such as vascular endothelial growth factor (VEGF) and kinesin spindle protein (KSP), thus controlling cancer proliferation.

Neoplastic diseases may also be treated using immunotherapy such as antibody therapies wherein the antibodies bind to a target antigen typically on the surface of the neoplastic cell. Cell surface receptors are common targets for antibody therapies and include the epidermal growth factor receptor, HER2, CD52, the vascular endothelial growth factor-A and CD30. Once bound to an antigen (*e.g.* a cancer antigen), antibodies can induce antibody-dependent cell-mediated cytotoxicity, activate the complement system, prevent a receptor interacting with its ligand or deliver a payload

of chemotherapy or radiation, which may all lead to the induction of neoplastic cell death. For instance, Cetuximab (Erbitux) is a chimeric IgG1 monoclonal antibody that targets the extracellular domain of the epidermal growth factor receptor (EGFR). Once a ligand binds to the EGFR on the surface of the cell, signalling pathways are activated inside the cell that are associated with malignant characteristics such as cancer cell proliferation and invasion. Cetuximab competitively inhibits ligand binding, thereby preventing EGFR activation and subsequent cellular signalling. It also activates programmed cell death (apoptosis).

Other intracellular treatments such as messenger ribonucleic acids (mRNAs) -based therapies have also been used in the treatment of neoplastic disease wherein administration of mRNA material into a neoplastic cell causes the neoplastic cell *e.g.* to express specifically encoded antigens and causing the neoplastic cell to be eliminated by the host immune system.

Differentiation therapy is another technique which was developed on the concept that the acquisition of the malignant phenotype (such as neoplasia) in a cell is considered as a progressive de-differentiation or a defective differentiation of that cell. Thus, as e.g. tumor cell populations evolve to greater degrees of malignancy, they usually lose more and more differentiation markers. This led to the suggestion that it may be possible to treat cancer by inducing differentiation of cancer cells. However, some scientific reports have shown that the differentiation therapy does not in fact induce cancer cells differentiation but instead restrains their growth thus allowing the application of more conventional therapies (such as chemotherapy) to eradicate the malignant cells. Examples of differentiation therapy involve the forced (re-)expression of some specific micro-RNAs and thus rely on an intracellular action generally presenting the same drawbacks as in any other intracellular therapies such the siRNA and gene therapies.

A shortcoming of the medical therapies relying on previously reported methods of treatment are numerous and mainly resides in the incapacity of providing a sustainable therapeutic effect i.e. the treated cells are not healed but instead are either destroyed (*e.g.* through induced apoptosis) or their proliferation reduced or temporarily halted using a sustained administration of therapeutic molecules until, in most case scenarios, neoplastic cells are able to adapt themselves and render the therapy ineffective. Interrupting a known therapy will usually lead to resumption of the neoplastic cell state.

Other drawbacks associated with previously reported therapies are numerous. For example, they may be very invasive and traumatic for the patient; they may necessitate permanently removing neoplastic tissues or organs which may be in some cases not practicable or feasible (organs or organ parts not accessible) or not possible due to life-threatening consequences; they may not be applied to or have limited effectiveness against metastatic neoplastic cells; they may not possess the ability to remove or treat all neoplastic cell types (*i.e.* neoplastic cells having different invasiveness levels and/or of different lineage origins); they may potentially "unblock" dormant tumors; they are often expensive; they may damage or destroy healthy cells alongside neoplastic cells thereby causing adverse treatment side effects; they may cause skin rashes and skin sensitivity; they may not target cancer stem cells as these are not proliferating; they may have a mutagenic action even towards healthy cells; they may require sustained administration to maintain treatment therapeutic effects; they may display very high cytotoxicity; they may cause multi-drug resistance whereby a drug having an intracellular action is no longer imported inside the cancer cell or is systematically exported outside of the cell.

None of these previously known methods allows for the effective recoding of a neoplastic cell thus permitting conversion of a neoplastic cell into a non-neoplastic cell.

The present invention thus provides associations, combinations, compositions, kits, methods and processes for the design, preparation, manufacture and/or formulation thereof, and methods and uses thereof for converting a neoplastic cell into a non-neoplastic cell including converting or recoding the neoplastic cell to induce, provide and/or reintroduce self-recovery or self-healing capabilities thereto.

## BRIEF DESCRIPTION OF THE DRAWINGS

FIG. 1 is: a representation of a Quantitative Real Time PCR analysis of the expression of DMP-1, Sclerostin and RANK-L in neoplastic cells (osteosarcoma cells) cultured in the absence (control) or in the presence of pharmaceutical associations according to certain embodiments of the present disclosure comprising various GFR-binding compounds and adhesion protein inhibitors after 24 hours of culture, ($P<0.001$).

FIG. 2 is a representation of Quantitative Real Time PCR analysis of the expression of MLC-1, GATA-4 and a-Sarcomeric actin in neoplastic cells (Rabdomiosarcoma cells) cultured in the absence (control) or in the presence of pharmaceutical associations according to certain embodiments of the present disclosure comprising various GFR-binding compounds and adhesion protein inhibitors after 24 hours of culture, ($P<0.005$).

FIG. 3 is a representation of Quantitative Real Time PCR analysis of the expression of Sox-9, IBSP and Collagen-IV in neoplastic cells (chondrosarcoma cells) cultured in the absence (control) or in the presence of pharmaceutical

associations according to certain embodiments of the present disclosure comprising various GFR-binding compounds and adhesion protein inhibitors after 24 hours of culture, (P<0.001).

FIG. 4 is a representation of Quantitative Real Time PCR analysis of the expression of MMP-9, Vimentin and a-SMA in neoplastic cells (adenocarcinoma cells) cultured in the absence (control) or in the presence of pharmaceutical associations according to certain embodiments of the present disclosure comprising various GFR-binding compounds and adhesion protein inhibitors after 24 hours of culture, (P<0.005).

FIG. 5 is (a) a diagram representing a relative quantification from western blot of the amount of p53 protein present in neoplastic cells (osteosarcoma cells) cultured in the absence (control) or in the presence of pharmaceutical associations according to certain embodiments of the present disclosure comprising various GFR-binding compounds and adhesion protein inhibitors after 24 hours of culture, (b) a diagram representing a relative quantification from western blot of the extent of phosphorylation of the pRB protein present in neoplastic cells cultured without (control) and in the presence of pharmaceutical associations according to certain embodiments of the present disclosure comprising various GFR-binding compounds and adhesion protein inhibitors after 24 hours of culture.

FIG. 6 is diagrams representing a relative quantification from western blot of the extent of phosphorylation of the ERK protein (a) and of the Src kinase (b) present in neoplastic cells (osteosarcoma cells) in the absence (control) or in the presence of pharmaceutical associations according to certain embodiments of the present disclosure comprising various GFR-binding compounds and adhesion protein inhibitors after 24 hours of culture, (c) a diagram representing a relative quantification from western blot of the amount of PDK1 protein present in neoplastic cells cultured in the absence (control) or in the presence of pharmaceutical associations according to certain embodiments of the present disclosure comprising various GFR-binding compounds and adhesion protein inhibitors after 24 hours of culture.

FIG. 7 is a representation of a Quantitative Real Time PCR analysis of the expression of Paxillin (a) and Vinculin (b) in neoplastic cells (osteosarcoma cells) cultured in the absence (control) or in the presence of pharmaceutical associations according to certain embodiments of the present disclosure comprising various GFR-binding compounds and adhesion protein inhibitors after 24 hours of culture, (P<0.001).

FIG. 8 is a representation of a Quantitative Real Time PCR analysis of the expression of Cyclin D (arithmetic mean of gene expression of Cyclin D1, D2 and D3) at different time intervals during 24 hours in neoplastic cells (osteosarcoma cells) cultured in the absence (control) or in the presence of pharmaceutical associations according to certain embodiments of the present disclosure comprising various GFR-binding compounds and adhesion protein inhibitors.

FIG. 9 is (a) a representation of a Quantitative Real Time PCR analysis of the expression of DMP-1, SCT and RANK-L in neoplastic cells (osteosarcoma cells) cultured on a native polymer scaffold (control) and on a polymer scaffold grafted with a mixture comprising a GFR-binding compound as defined in the present disclosure and a RGD peptide, after 24 hours of culture, (b) immunofluorescence staining of a representative neoplastic cell (osteosarcoma cells) cultured on a polymer scaffold grafted with a mixture comprising a GFR-binding compound as defined in the present disclosure (ID SEQ NO: 2) and a RGD peptide, after 24 hours of culture. The actin filaments were immunostained by using Alexa 488-phalloidin. The focal adhesions were represented by immunostaining with anti-vinculin. The nucleus was stained with DAPI and is represented by a circle in the center of the cell. Scale bar: 5 $\mu$m. (c) is a diagram representing the grafting density of a radiolabeled GFR-binding compound as defined in the present disclosure covalently grafted on a polymer scaffold and of a radiolabeled GFR-binding compound and a non-labeled RGD peptide both grafted on a polymer scaffold. The measurements were performed by using radioactivity quantification (no significant difference was observed). (d) Relative cell spreading function as the presence of different integrin subunits ($\alpha$ and $\beta$) couples (anti-$\alpha 3\beta 1$, anti- $\alpha v\beta 3$ and anti-$\alpha 5\beta 3$ in neoplastic cells cultured on a non-modified polymer scaffold, after 24h of culture.

FIG. 10 is (a) Western blot analysis of the expression integrin $\alpha 3$ and $\beta 1$ before and after transduction with two independent integrin $\alpha 3$ and $\beta 1$ shRNAs, which efficiently reduced endogenous integrin $\alpha 3\beta 1$ protein levels. (b) Immunofluorescence visualization of a neoplastic cell (osteosarcoma cells) after silencing integrins $\alpha 3\beta 1$ by using shRNAs, after 24 hours of culture on a polymer scaffold covalently grafted with RGD peptides. The silencing of these specific integrin proteins were shown to significantly reduce or suppress neoplastic cell adhesion on the RGD grafted polymers. The actin filaments were immunostained by using Alexa 488-phalloidin. The focal adhesions were represented by immunostaining with anti-vinculin. The nucleus was stained with DAPI and is represented by a circle in the center of the cell. Scale bar: 5 $\mu$m. (c) Immunofluorescence visualization of neoplastic cells cultured on a polymer scaffold covalently modified with a mixture comprising a GFR-binding compound as defined in the present disclosure (SEQ ID NO: 2) and a RGD peptide, after 24 hours of culture. Scale bar: 500 $\mu$m. A magnification of one region (right, Scale bar: 50 $\mu$m) indicates that Spheroid-like structures of neoplastic cells become predominant in this case as a result of the RGD induced integrin engagement. The actin filaments were immunostained by using Alexa 488-phalloidin. The focal adhesions were represented by immunostaining with anti-vinculin. The nucleus was stained with DAPI and is represented by a circle in the center of the cell.

FIG. 11 is a screen shot of the Standard Protein Blast online software used in the RMSD calculation procedure.

## DETAILED DESCRIPTION

Neoplastic diseases start when a cell (or neoplastic cell) is somehow altered so that it multiplies out of control. Tumors and cancers are some examples of neoplastic diseases. A tumor is a mass composed of a cluster of such abnormal cells. Most cancers form tumors, but not all tumors are cancerous. Benign, or non-cancerous, tumors - such as freckles and moles - stop growing, do not spread to other parts of the body, and do not create new tumors. Malignant, or cancerous, tumors crowd out healthy cells, interfere with body functions, and draw nutrients from body tissues. Cancers continue to grow and spread by direct extension or through a process called metastasis, whereby the malignant cells travel through the lymphatic or blood vessels, eventually forming new tumors in other parts of the body.

The term "cancer" generally encompasses more than one hundred diseases affecting nearly every part of the body, and all are potentially life threatening. The major types of cancer are carcinoma, sarcoma, melanoma, lymphoma, and leukemia.

Carcinoma is a type of cancer that develops from epithelial cells. Specifically, a carcinoma is a cancer that begins in a tissue that lines the inner or outer surfaces of the body, and that generally arises from cells originating in the endodermal or ectodermal germ layer during embryogenesis.

Sarcoma is a cancer that arises from transformed cells of mesenchymal origin. Thus, malignant tumors made of cancerous bone, cartilage, fat, muscle, vascular or hematopoietic tissues are, by definition, considered sarcomas. This is in contrast to a malignant tumor originating from epithelial cells, which are termed carcinoma. Human sarcomas are quite rare. Common malignancies, such as breast, colon, and lung cancer, are almost always carcinoma.

Melanoma is a type of skin cancer, which forms from melanocytes (pigment-containing cells in the skin).

Lymphoma is a group of blood cell tumors that develop from lymphocytes. It is sometimes used to refer to just the cancerous ones rather than all tumors. There are two main types: Hodgkin lymphoma (HL) and non-Hodgkin lymphoma (NHL), with two others, multiple myeloma and immunoproliferative diseases, also included by the World Health Organization within the category. Non-Hodgkin lymphoma makes up about 90% of cases and includes a large number of sub-types. Lymphomas are part of the broader group of neoplasms called tumors of the hematopoietic and lymphoid tissues.

Leukemia is a group of cancers that usually begins in the bone marrow and results in high numbers of abnormal white blood cells. These white blood cells are not fully developed and are called blasts or leukemia cells. Symptoms may include bleeding and bruising problems, feeling very tired, and an increased risk of infections. These symptoms occur due to a lack of normal blood cells. Diagnosis is typically by blood tests or bone marrow biopsy.

Cancers include, but are not limited to, Adrenal Cancer, Anal Cancer, Bile Duct Cancer, Bladder Cancer, Bone Cancer, Brain/CNS Tumors In Adults, Brain/CNS Tumors In Children, Breast Cancer, Breast Cancer In Men, Cancer in Adolescents, Cancer in Children, Cancer in Young Adults, Cancer of Unknown Primary, Castleman Disease, Cervical Cancer, Colon/Rectum Cancer, Endometrial Cancer, Esophagus Cancer, Ewing Family Of Tumors, Eye Cancer, Gallbladder Cancer, Gastrointestinal Carcinoid Tumors, Gastrointestinal Stromal Tumor (GIST), Gestational Trophoblastic Disease, Hodgkin Disease, Kaposi Sarcoma, Kidney Cancer, Laryngeal and Hypopharyngeal Cancer, Leukemia, Leukemia - Acute Lymphocytic (ALL) in Adults, Leukemia - Acute Myeloid (AML), Leukemia - Chronic Lymphocytic (CLL), Leukemia - Chronic Myeloid (CML), Leukemia - Chronic Myelomonocytic (CMML), Leukemia in Children, Liver Cancer, Lung Cancer, Lung Cancer - Non-Small Cell, Lung Cancer - Small Cell, Lung, Carcinoid Tumor, Lymphoma, Lymphoma of the Skin, Malignant Mesothelioma, Multiple Myeloma, Myelodysplastic Syndrome, Nasal Cavity and Paranasal Sinus Cancer, Nasopharyngeal Cancer, Neuroblastoma, Non-Hodgkin Lymphoma, Non-Hodgkin Lymphoma In Children, Oral Cavity and Oropharyngeal Cancer, Osteosarcoma, Ovarian Cancer, Pancreatic Cancer, Penile Cancer, Pituitary Tumors, Prostate Cancer, Retinoblastoma, Rhabdomyosarcoma, Salivary Gland Cancer, Sarcoma - Adult Soft Tissue Cancer, Skin Cancer, Skin Cancer - Basal and Squamous Cell, Skin Cancer - Melanoma, Skin Cancer - Merkel Cell, Small Intestine Cancer, Stomach Cancer, Testicular Cancer, Thymus Cancer, Thyroid Cancer, Uterine Sarcoma, Vaginal Cancer, Vulvar Cancer, Waldenstrom Macroglobulinemia, Wilms Tumor.

The activation of growth factor receptors (GFR) is commonly known to promote neoplastic cell proliferation. For instance, many cancer treatments rely on the inhibition of growth factors, growth factors receptors and/or downstream signalling proteins such as protein kinases. Such inhibitors include, non-exhaustively, anti-Met (*e.g.* ARQ-197), anti-VEGF (*e.g.* Bevacizumab), anti-VEGFR (*e.g.* Sunitinib or Semaxinib), anti-Her2 (*e.g.* Trastuzumab), anti-EGFR (*e.g.* Cetuximab, Gefitinib or Erlotinib), anti-PDGFR (*e.g.* Imatinib), anti-IGF-1 (*e.g.* IMC-A12), anti-Ras (*e.g.* Tipifarnib), anti-Raf (*e.g.* Sorafenib), anti-src (*e.g.* Dastinib or Saracatinib), anti-Mek (*e.g.* C1040 or PD-0325901), anti-PI3K (*e.g.* LY294002), anti-PDK (*e.g.* UNC01), anti-HSP90 (*e.g.* 17-AGG or IPI-504), anti-CDK (*e.g.* Flavopiridol) and anti-mTOR (*e.g.* Everolimus).

Unlike previously reported treatments, it has been surprisingly demonstrated that the pharmaceutical associations, combinations and compositions disclosed herein can be used to treat, prevent and/or diagnose neoplasms (*e.g.* tumors or cancers) by activating growth factor receptors of vertebrate cells (such as mammalian cells, especially human cells).

The present invention also aims at providing mechanisms for solving and/or avoiding at least one of, preferably a plurality of, the problems, issues and/or shortcomings associated with previously reported neoplasm treatment therapies.

The present disclosure thus provides embodiments for:

- Converting or recoding a neoplastic cell to induce and/or promote and/or improve self-healing and/or self-recovery thereof, particularly within a shorter period of time
- Converting or recoding a neoplastic cell to induce and/or promote and/or improve self-healing and/or self-recovery thereof, particularly with a higher conversion yield;
- Restoring a neoplastic cell's ability to undergo conversion into a physiologically functional and/or healthy cell, particularly within a shorter period of time;
- Restoring a neoplastic cell's ability to undergo differentiation, particularly within a shorter period of time;
- Converting and/or recoding a circulating or non-circulating neoplastic cell such as a cancer cell, into a non-neoplastic cell, particularly within a shorter period of time;
- Protecting a subject from a neoplastic disease, disorder, condition, or pathology such as cancer, or any symptoms thereof, particularly within a shorter period of time;
- Providing and/or producing a physiologically functional and/or healthy cell including an osteocyte and/or a chondrocyte and/or an adipocyte and/or a myocyte and/or a keratinocyte and/or a fibrocyte and/or a podocyte and/or a neurocyte and/or a mature endothelial cell and/or a osteoblast and/or a chondroblast and/or a neuroblast and/or a Sertoli cell and/or a Leydig cell and/or a germ cell and/or an endothelial cell and/or an angioblast and/or a fibroblast from a neoplastic cell, particularly within a shorter period of time;
- Re-establishing, restoring and/or reactivating a cell adhesion checkpoint of a neoplastic cell;
- Inducing and/or promoting and/or enhancing neoplastic cell differentiation, particularly within a shorter period of time;
- Inducing and/or promoting and/or enhancing a decrease in Cyclin D gene and protein expression;
- Inducing and/or promoting and/or enhancing a G1 to G0 cell cycle phase transition;
- Inducing and/or promoting and/or enhancing the inactivation of a protein complex between a Cyclin D and at least one of Cyclin-dependent kinase (CDK) 4 or 6;
- Preventing or reducing the phosphorylation of the retinoblastoma (Rb) protein thus activating its tumour suppressor functions;
- Increasing the phosphorylation of the p53 protein thus reducing its degradation and activating its tumor suppressor functions;
- Preventing or reducing the activation of the Ras/MAP kinase signalling cascade;
- Identifying and/or analysing the features and/or markers of a neoplastic cell;
- Regulating and/or activating growth factor receptors of a neoplastic cell;
- Modulating and/or regulating the adhesion between a cell (in particular a neoplastic cell) and its micro-environment i.e. the surrounding extracellular matrix;
- Treating, recoding or converting a neoplastic cell without temporarily or permanently damaging or killing said neoplastic cell and/or without permanently inducing a quiescent state thereof;
- Dampening and/or reducing or suppressing cell division and/or cell proliferation of a neoplastic cell;
- Activating and/or promoting and/or regulating anti-mitogen activity and/or tumor suppressor pathways in a neoplastic cell;
- Inhibiting and/or reducing anti-oncogenic activity in a neoplastic cell;
- Converting or recoding a neoplastic cell to induce and/or promote and/or improve self-healing and/or self-recovery thereof, and/or protecting a subject from a neoplastic disease, disorder, condition or pathology such as cancer using extracellular means;
- Converting or recoding a neoplastic cell to induce and/or promote and/or improve self-healing and/or self-recovery thereof, protecting a subject from a neoplastic disease, disorder, condition or pathology such as cancer without modifying the genome of said cell;
- Providing diagnostic tools for diagnosing neoplastic diseases in a subject.

## I. Definitions

Those skilled in the art will recognize, or be able to ascertain using no more than routine experimentation, many equivalents to the specific embodiments in accordance with the invention described herein. The scope of the present invention is not intended to be limited to the present description, but rather is as set forth in the appended claims.

In the claims, articles such as "a", "an", and "the" may mean one or more than one unless indicated to the contrary or otherwise evident from the context. Claims or descriptions that include "or" between one or more members of a group are considered satisfied if one, more than one, or all of the group members are present in, employed in, or

otherwise relevant to a given product or process unless indicated to the contrary or otherwise evident from the context. The invention includes embodiments in which exactly one member of the group is present in, employed in, or otherwise relevant to a given product or process. The invention includes embodiments in which more than one, or all of the group members are present in, employed in, or otherwise relevant to a given product or process.

It is also noted that the term "comprising" is intended to be open and permits but does not require the inclusion of additional elements or steps. When the term "comprising" is used herein, the terms "consisting of", "consisting essentially of", "consisting substantially of" and "consisting exclusively of" are thus also encompassed and disclosed.

As used herein, the term "approximately" or "about," as applied to one or more values of interest, refers to a value that is similar to a stated reference value. In certain embodiments, the term "approximately" or "about" refers to a range of values that fall within 25%, 20%, 19%, 18%, 17%, 16%, 15%, 14%, 13%, 12%, 11%, 10%, 9%, 8%, 7%, 6%, 5%, 4%, 3%, 2%, 1%, or less in either direction (greater than or less than) of the stated reference value unless otherwise indicated, self-evident or contradictory in context (e.g. except where such number would exceed 100% of a possible value).

As used herein and unless otherwise indicated or contradictory in context, the term "with" followed by a specific number of amino acids, when used to define a particular peptide, variant or analog thereof, such as in "a peptide with three amino acids", means that such peptide, variant or analog thereof, contains exclusively the specific number of amino acids specified after this term.

As used herein and unless otherwise indicated or contradictory in context, the term "Ci-alkyl" is intended to specifically and individually disclose any branched or unbranched radical, moiety or functional group having "i" carbon atom(s).

The carbon atom content of the various hydrocarbon-containing moieties herein may be indicated by a prefix designating the minimum and maximum number of carbon atoms in the moiety. For example, in certain embodiments, (Ca-Cb)alkyl indicates an alkyl moiety of the integer "a" to the integer "b" carbon atoms, inclusive.

At various places in the present specification, substituents of compounds of the present disclosure may be disclosed in groups or in ranges. It is specifically intended that the present disclosure include each and every individual sub-combination of the members of such groups and ranges. For example, in certain embodiments, the term "C1-C5 alkyl" is an abbreviation for (and thus is specifically intended to individually disclose) C1-alkyl (*i.e.* methyl), C2-alkyl (*i.e.* ethyl), C3-alkyl (*i.e.* 1-propyl and 2-propyl), C4-alkyl (*i.e.* 1-butyl, sec-butyl, *iso*-butyl and *tert*-butyl), and C5-alkyl (*i.e.* 1-pentyl, 2-pentyl, 3-pentyl, 2-methyl-1-butyl, 3-methyl-1-butyl, 2-methyl-2-butyl, 3-methyl-2-butyl, 2,2-dimethyl-1-propyl and 1,1-dimethyl-1-propyl).

As used herein, unless indicated otherwise or contradictory in context, the terms "alkyl" and "(Ca-Cb)alkyl" refer to monovalent hydrocarbon radicals containing the requisite number of carbon atoms as described above, having straight or branched moieties or combinations thereof. As used herein, alkyl groups may be optionally substituted with between one to four substitutes. Non-limiting examples of alkyl groups include, *e.g.* methyl, ethyl, n-propyl, isopropyl, n-butyl, isobutyl, sec-butyl, t-butyl, etc. Of course, other alkyl groups will be readily apparent to those of skilled in the art given the benefit of the present disclosure.

Where ranges are given, endpoints are included. Furthermore, it is to be understood that unless otherwise indicated or otherwise evident from the context and understanding of one of ordinary skill in the art, values that are expressed as ranges can assume any specific value or sub-range within the stated ranges in different embodiments of the invention, to the tenth of the unit of the lower limit of the range, unless the context clearly dictates otherwise. For example, in certain embodiments, a disclosed 0-10 range would, for example, in certain embodiments, also specifically and individually disclose the following values and ranges: 0, 1, 2, 3, 4, 5, 6, 7, 8, 9, 10, 0.1, 0.2, 0.3, 0.4, 0.5, 0.6, 0.7, 0.8, 0.9, 1, 1.1, 1.2, 1.3, 1.4, 1.5, 1.6, 1.7, 1.8, 1.9, 2, 2.1, 2.2, 2.3, 2.4, 2.5, 2.6, 2.7, 2.8, 2.9, 3, 3.1, 3.2, 3.3, 3.4, 3.5, 3.6, 3.7, 3.8, 3.9, 4, 4.1, 4.2, 4.3, 4.4, 4.5, 4.6, 4.7, 4.8, 4.9, 5, 5.1, 5.2, 5.3, 5.4, 5.5, 5.6, 5.7, 5.8, 5.9, 6, 6.1, 6.2, 6.3, 6.4, 6.5, 6.6, 6.7, 6.8, 6.9, 7, 7.1, 7.2, 7.3, 7.4, 7.5, 7.6, 7.7, 7.8, 7.9, 8, 8.1, 8.2, 8.3, 8.4, 8.5, 8.6, 8.7, 8.8, 8.9, 9, 9.1, 9.2, 9.3, 9.4, 9.5, 9.6, 9.7, 9.8, 9.9, 10, 0-1, 0-2, 0-3, 0-4, 0-5, 0-6, 0-7, 0-8, 0-9, 1-2, 1-3, 1-4, 1-5, 1-6, 1-7, 1-8, 1-9, 2-3, 2-4, 2-5, 2-6, 2-7, 2-8, 2-9, 2-10, 3-4, 3-5, 3-6, 3-7, 3-8, 3-9, 3-10, 4-5, 4-6, 4-7, 4-8, 4-9, 4-10, 5-6, 5-7, 5-8, 5-9, 5-10, 6-7, 6-8, 6-9, 6-10, 7-8, 7-9, 7-10, 8-9, 8-10, 9-10, 0-0.1, 0-0.2, 0-0.3, 0-0.4, 0-0.5, 0-0.6, 0-0.7, 0-0.8, 0-0.9, 0-1.1, 0-1.2, etc.

As used herein and unless otherwise indicated or contradictory in context, the term "substantially" refers to the qualitative condition of exhibiting total or near-total extent or degree of a characteristic or property of interest. One of ordinary skill in the biological arts will understand that biological and chemical phenomena rarely, if ever, go to completion and/or proceed to completeness or achieve or avoid an absolute result. The term "substantially" is therefore used herein to capture the potential lack of completeness inherent in many biological and chemical phenomena.

In addition, it is to be understood that any particular embodiment of the present invention that falls within the prior art may be explicitly excluded from any one or more of the claims using the appropriate disclaimer(s) or proviso(s). Since such embodiments are deemed to be known to one of ordinary skill in the art, they may be excluded even if the exclusion is not set forth explicitly herein. Any particular embodiment of the compositions of the invention (e.g., any peptide or peptidomimetic; any method of production; any method of use; etc.) can be excluded from any one or more

claims, for any reason, whether or not related to the existence of prior art.

All cited sources, for example, in certain embodiments, references, publications, databases, database entries, and art cited herein, are incorporated into this application by reference in their entirety, even if not expressly stated in the citation. In case of conflicting statements of a cited source and the instant application, the statement in the instant application shall control.

As the case may be, and unless otherwise indicated or contradictory in context, macromolecules molecular weights should be understood in the present description as being number averaged molecular weights.

The peptides mentioned in the present description may not follow the usual representation conventions. For instance, the N-terminal amino acid of a peptide sequence may be the first amino acid in the sequence or the last amino acid. Likewise, the C-terminal amino acid of a peptide sequence may be the first amino acid in the sequence or the last amino acid. For example, in the peptide sequence NAIS, "N" may be N-terminal or C-terminal, and "S" may be N-terminal or C-terminal. Consequently, for the purpose of the present disclosure, e.g. NAIS also covers SIAN, SAIS also covers SIAS, SPIN also covers NIPS, etc.

In the present application, when reference is made to a certain peptide (*e.g.* a GFR-binding compound as provided herein) comprising one or more other peptide(s), said one or more other peptide(s) is(are) understood to be stably (in most cases, covalently) attached/bound to at least one part of said peptide. The attachment/binding may be located anywhere on the peptide unless indicated otherwise, contradictory in context or contradictory to general scientific rules. No specific attachment/binding location of said one or more other peptide(s) to said peptide shall be assumed unless specifically mentioned.

**Peptide or polypeptide:** As used herein, the term "peptide" or "polypeptide" are used interchangeably and refers to a polymer of less than or equal to 100 amino acids long, e.g., about 2, 3, 4, 5, 10, 15, 20, 25, 30, 35, 40, 45, 50, 55, 60, 65, 70, 75, 80, 85, 90, 95 or 100 amino acids long. The terms apply to amino acid polymers in which one or more amino acid residue is an artificial chemical mimetic of a corresponding naturally occurring amino acid, as well as to naturally occurring amino acid polymers, non-naturally occurring amino acid polymers, peptide analogs, peptide variants and peptide mimetics. Conventional techniques for synthesising peptides involve the activation of the carboxylic acid function of an amino acid or of a peptide, using a coupling agent. This activated acid is then contacted with an amino acid or a peptide in which the N-terminal amino acid is not protected, thus forming an amide bond also called peptide bond. Coupling reaction conditions together with coupling agents are well known in the art and described, for instance, in Greene, "Protective Groups in Organic Synthesis", Wiley, New York, 2007 4th edition. In addition, suitable peptide synthesis routes are described, for instance, in Hojo H., Recent progress in the chemical synthesis of proteins, Curr Opin Struct Biol. 2014; 26C:16-23 and Saranya Chandrudu, et al., Chemical Methods for Peptide and Protein Production, Molecules, 2013, 18, 4373-4388, each of which is incorporated herein by reference in its entirety. There are two main strategies for peptide synthesis i.e. liquid-phase peptide synthesis and solid-phase peptide synthesis (SPPS) which is now most commonly used for peptide synthesis. Instead of C-terminal protection with a chemical group, the C-terminus of the first amino acid is coupled to an activated solid support, such as polystyrene or polyacrylamide. This type of approach has a two-fold function: the resin acts as the C-terminal protecting group and provides a rapid method to separate the growing peptide product from the different reaction mixtures during synthesis. As with many different biological manufacturing processes, peptide synthesizers have been developed for automation and high-throughput peptide production. SPPS allows the synthesis of natural peptides which are difficult to express in bacteria, the incorporation of unnatural amino acids, peptide/protein backbone modification, and the synthesis of D-proteins, which consist of D-amino acids. Very long peptide can be accessed by using native chemical ligation to couple two peptides together with quantitative yields.

**Peptide analogs:** As used herein, unless indicated otherwise or contradictory in context, the term "peptide analogs" refers to polypeptide variants which differ by one or more amino acid alterations, e.g., substitutions, additions or deletions of amino acid residues that still maintain one or more of the properties of the parent or starting peptide.

**Peptide variants:** As used herein, unless indicated otherwise or contradictory in context, the term "peptide variants" refers to a peptide which has a certain identity with a native or reference compound sequence. In one example, the peptide variant refers to any post- administration, application, injection modified peptide. Such post- administration, application, injection modifications include, but are not limited to, phosphorylation, acetylation, glutamylation, tyrosination, palmitoylation, glycosylation, myristoylation, palmitoylation, isoprenylation, glypiation, lipoylation, phosphopantetheinylation, acylation, alkylation, amidation, arginylation, polyglutamylation, polyglycylation, butyrylation, gamma-carboxylation, glycosylation, polysialylation, malonylation, hydroxylation, iodination, nucleotide addition, oxidation, adenylylation, propionylation, pyroglutamate formation, S-glutathionylation, S-nitrosylation, succinylation, sulfation, glycation, biotinylation, pegylation, ISGylation, SUMOylation, ubiquitination, Neddylation, Pupylation, citrullination, deamidation, eliminylation, carbamylation, and racemization.

**Peptido-mimetic:** As used herein, unless indicated otherwise or contradictory in context, the term "peptido-mimetic" or "peptidomimetic" refers to a synthetic chemical compound which comprises amino acids but not only and that is able to mimic the biological action of a peptide, often because the mimetic has a basic structure that mimics the

basic structure of the peptide and/or has the salient biological properties of that peptide. In one particular example, a peptidomimetic is a hybrid molecule containing both, at least one peptide, and at least one of a polysaccharide, a polynucleotide or a linear or branched, saturated or unsaturated, hydrocarbon chain.

**Linear peptide:** As used herein, unless indicated otherwise or contradictory in context, the term "linear peptide" means a peptide in which the C-terminal and the N-terminal amino acid residues do not covalently interact with each other and none of the C-terminal or the N-terminal amino acid residues covalently interacts with another amino acid residue of the peptide chain.

**Cyclic peptide:** As used herein, unless indicated otherwise or contradictory in context, the term "cyclic peptide" means peptide in which the C-terminal and N-terminal amino acid residues do covalently interact with each other or the C-terminal and/or the N-terminal amino acid residues covalently interact with at least one other amino acid residue of the peptide chain so as to form a ring-like structure.

**Amino acid:** As used herein, unless indicated otherwise or contradictory in context, the term "amino acid" refers to naturally occurring and non-naturally occurring amino acids including amino acid analogs. Naturally occurring amino acids are those encoded by the genetic code, as well as those amino acids that are later modified, e.g., hydroxyproline, [gamma]-carboxyglutamate, and O-phosphoserine. Naturally encoded amino acids are the 20 common amino acids glycine (Gly, G), alanine (Ala, A), valine (Val, V), leucine (Leu, L), isoleucine (lie, I), serine (Ser, S), threonine (Thr, T), phenylalanine (Phe, F), tyrosine (Tyr, Y), tryptophane (Trp, W), cysteine (Cys, C), methionine (Met, M), proline (Pro, P), aspartic acid (Asp, D), asparagine (Asn, N), glutamine (Gin, Q), glutamic acid (Glu, E), histidine (His, H), arginine (Arg, R) et lysine (Lys, K) and pyrrolysine and selenocysteine. Non-naturally occurring amino acids include, but are not limited to, the dextrogyre (D) isomers of the above-cited naturally-occurring amino acids. Amino acid analogs refers to compounds that have the same basic chemical structure as a naturally occurring amino acid *i.e.,* an [alpha] carbon that is bound to a hydrogen, a carboxyl group, an amino group, and an R group (*i.e.* side chain), and which may be used in replacement thereof without substantially affecting the overall function of the peptide to which it belongs. Amino acid analogs (or non-naturally occurring amino acids) that may be suitable for implementing embodiments of the present invention include, but are not limited to, amino acids comprising a photoactivatable cross-linker, spin-labeled amino acids, fluorescent amino acids, metal binding amino acids, metal-containing amino acids, radioactive amino acids, amino acids with novel functional groups, amino acids that covalently or noncovalently interact with other molecules, photocaged and/or photoisomerizable amino acids, amino acids comprising biotin or a biotin analogue, glycosylated amino acids such as a sugar substituted serine, other carbohydrate modified amino acids, keto-containing amino acids, amino acids comprising polyethylene glycol or polyether, heavy atom substituted amino acids, chemically cleavable and/or photocleavable amino acids, amino acids with an elongated side chains as compared to natural amino acids, including but not limited to, polyethers or long chain hydrocarbons, including but not limited to, greater than about 5 or greater than about 10 carbons, carbon-linked sugar-containing amino acids, redox-active amino acids, amino thioacid containing amino acids, and amino acids comprising one or more toxic moiety. The term "**$AA^{I}$**" (AA roman numeral one) may be used in the description and refers to an amino acid which may be any amino acid as defined above in particular any naturally occurring and non-naturally occurring amino acids.

**Amino acid side chain:** As used herein, unless indicated otherwise or contradictory in context, the term "amino acid side chain" means the functional group of an amino acid that differentiates it from other amino acids. All amino acid structures have a carboxyl group, an amine group and a specific side chain.

**$AA^{II}$** (AA roman numeral two): As used herein, unless indicated otherwise or contradictory in context, the terms "polar amino acid" or "AA"" means amino acids having a polar, non-charged group-containing side chain. Polar amino acids are protonated at physiological pH (about 7). Examples of polar amino acids include, but are not limited to, Cys (C), Asn (N), Gin (Q), Ser (S), Thr (T), or Tyr (Y).

**$AA^{III}$** (AA roman numeral three): As used herein, unless indicated otherwise or contradictory in context, the terms "acidic amino acid" or "$AA^{III}$" means amino acids having an acidic group-containing side chain. Acidic amino acid deprotonated forms predominate at physiological pH (about 7). Examples of acidic amino acids include, but are not limited to, Asn (N) and Glu (E).

**$AA^{IV}$** (AA roman numeral four): As used herein, unless indicated otherwise or contradictory in context, the terms "aliphatic amino acid" or "$AA^{IV}$" means amino acids having an aliphatic side chain. Examples of aliphatic amino acids include, but are not limited to, Ala (A), Leu (L), lie (I), Gly (G), Val (V) and any analogs and derivatives thereof.

**$AA^{V}$** (AA roman numeral five): As used herein, unless indicated otherwise or contradictory in context, the terms "apolar amino acid" or "$AA^{V}$" means amino acids having an apolar side chain. Examples of apolar amino acids include, but are not limited to, Ala (A), Phe (F), Gly (G), lie (I), Leu (L), Met (M), Pro (P), Val (V) or Trp (W).

**$AA^{VI}$** (AA roman numeral six): As used herein, unless indicated otherwise or contradictory in context, the term "aromatic amino acid" or "$AA^{VI}$" means amino acids having an aromatic group-containing side chain. Examples of aromatic amino acids include, but are not limited to, Trp (W), Tyr (Y) or Phe (F).

**$AA^{VII}$** (AA roman numeral seven): As used herein, unless indicated otherwise or contradictory in context, the term "basic amino acid" or "$AA^{VII}$" means amino acids having a basic group-containing side chain. Basic amino acid

protonated forms predominate at physiological pH (about 7). Examples of basic amino acids include, but are not limited to, Arg (R), His (H), or Lys (K).

**AA$^{VIII}$"** (AA roman numeral eight): As used herein, unless indicated otherwise or contradictory in context, the term "AA$^{VIII}$" means Leu (L) or lie (I) and any analogs and derivatives thereof.

**AA$^{IX}$** (AA roman numeral nine): As used herein, unless indicated otherwise or contradictory in context, the term "charged amino acid" or "AA$^{IX}$" means amino acids having either an acidic group-containing side chain or an basic group-containing side chain. Charged amino acid charged forms predominate at physiological pH (about 7). Examples of charged amino acids include, but are not limited to, Asn (N), Glu (E), His (H), Lys (K) or Arg (R).

**AA$^n$:** As used herein, unless indicated otherwise or contradictory in context, the term "AA$^n$", in which n is a positive integer arbitrarily chosen to identify a specific position within the primary sequence of a peptide. For instance, AA$^{13}$ means the amino acid of position 13. The terms "amino acid" and "AA" are interchangeably used in the present description.

**N-terminal:** As used herein, unless indicated otherwise or contradictory in context, the term "N-terminal" means the amine (-$NH_2$) function/group/moiety located at one (terminal) end of a protein or polypeptide. This functional group is the only amine group which is not engage in n amide peptide bond.

**C-terminal:** As used herein, unless indicated otherwise or contradictory in context, the term "C-terminal" means the carboxylate (-$CO_2H$) function/group/moiety located at one (terminal) end of a protein or polypeptide. This functional group is the only carboxylic acid group which is not engage in n amide peptide bond.

**Naturally-occurring peptide:** As used herein, unless indicated otherwise or contradictory in context, the terms "naturally-occurring peptide" or "natural peptide" means a peptide which may be found in nature without human direct intervention (except for its extraction and/or isolation).

**Synthetic peptide:** As used herein, unless indicated otherwise or contradictory in context, the terms "synthetic peptide" or "non-natural peptide" means a peptide which may not be found in nature without human direct intervention (except for its extraction and/or isolation). For example, in certain embodiments, a synthetic peptide may have the amino acid sequence of a natural peptide except for at least one amino acid deletion or substitution relative to the natural sequence. In the case of a substitution, an amino acid from the natural sequence is replaced by another, different, naturally-occurring or non-naturally occurring amino acid. For example, in certain embodiments, a synthetic peptide may not possess a post-translational modification of the natural peptide such as the attachment of an acetate group, a phosphate group, a lipid, a carbohydrate, or the formation of a disulfide bridge.

**Covalent interaction:** As used herein, unless indicated otherwise or contradictory in context, the term "interact covalently", "covalent interaction" or "covalent bond" are interchangeably used and means a chemical bond or interaction that involves the sharing of electron pairs between atoms. Examples of such interactions are $\sigma$-bonding and $\pi$-bonding.

**Non-covalent interaction:** As used herein, unless indicated otherwise or contradictory in context, the term "interact non-covalently", "non-covalent interaction" or "non-covalent bond" are interchangeably used and means a chemical bond or interaction that does not involve the sharing of electron pairs between atoms but rather involves more dispersed variations of electromagnetic interactions between molecules or within a molecule. Non-covalent interactions can be generally classified into four categories, electrostatic interactions, $\pi$-interactions, van der Waals forces, and hydrophobic interactions.

**Electrophile:** As used herein, unless indicated otherwise or contradictory in context, the term "electrophile" means an organic molecule attracted to electrons that participates in a chemical reaction by accepting an electron pair in order to bond to a nucleophile. Most electrophiles are positively charged, have an atom that carries a partial positive charge, or have an atom that does not have an octet of electrons.

**Nucleophile:** As used herein, unless indicated otherwise or contradictory in context, the term "nucleophile" means an organic molecule that donates an electron pair to an electrophile to form a chemical bond in relation to a reaction. All molecules or ions with a free pair of electrons or at least one pi bond can act as nucleophiles.

**Polysaccharide:** As used herein, unless indicated otherwise or contradictory in context, the term "polysaccharide" means polymeric carbohydrate molecules composed of long chains of monosaccharide units bound together by glycosidic linkages and which upon hydrolysis provide monosaccharides or oligosaccharides. They range in structure from linear to highly branched polymers.

**Polynucleotide:** As used herein, the term "polynucleotide" or "nucleic acid", which are used interchangeably, refers to the phosphate ester polymeric form of ribonucleosides ("RNA molecules") or deoxyribonucleosides ("DNA molecules"), or any phosphoester analogs thereof, such as phosphorothioates and thioesters, in either single stranded form, or a double-stranded helix. The term "nucleic acid" includes double-stranded DNA round, inter alia, in linear (e.g., restriction fragments) or circular DNA molecules. In particular, nucleic acids as used herein refer to nucleic acids such as RNAs encoding for agonist of growth factor receptors as defined herein.

**Nucleoside:** As used herein, the term "nucleoside" refers to a compound containing a sugar molecule (e.g., a pentose or ribose) or derivative thereof in combination with an organic base (e.g., a purine or pyrimidine) or a derivative thereof (also referred to herein as "nucleobase").

**Nucleotide:** As used herein, the term "nucleotide" refers to a nucleoside including a phosphate group.

**Dendrimer:** As used herein, unless indicated otherwise or contradictory in context, the term "dendrimer" means any repetitively branched molecules. Examples of dendrimers are phosphorous dendrimers, polylysine dendrimers, polypropylenimine dendrimers and PAMAM dendrimers, such as the ones described, for instance, in Scientific World Journal. 2013; 2013:732340; Curr Opin Chem Biol. 1998; 2(6):733-42; J Pept Sci. 1999; 5(5):203-20; and J Pept Sci. 2008; 14(1):2-43, which may be used for implementing embodiments of the present invention, each of which being herein incorporated by reference in its entirety.

**Synthetic molecule:** As used herein, unless indicated otherwise or contradictory in context, the term "synthetic molecule" means a molecule which may not be found in nature without human direct intervention (except for its extraction and/or isolation).

**Synthetic polymers:** As used herein, unless indicated otherwise or contradictory in context, the term "synthetic polymer" refers to a macromolecule or polymer which may not be found in nature without human direct intervention (except for its extraction and/or isolation).

**Antibody:** As used herein, unless indicated otherwise or contradictory in context, the term "antibody" encompasses the various forms of antibodies including, but not limited to, whole antibodies, human antibodies, humanized antibodies and genetically engineered antibodies like monoclonal antibodies, chimeric antibodies or recombinant antibodies as well as fragments of such antibodies as long as the characteristic properties according to the present disclosure are retained.

**Monoclonal antibody:** As used herein, unless indicated otherwise or contradictory in context, the term "monoclonal antibody" refers to an antibody obtained from a population of substantially homogeneous antibodies, i.e., the individual antibodies comprising the population are identical except for possible naturally occurring mutations that may be present in minor amounts. Monoclonal antibodies are highly specific, being directed against a single antigenic site. Furthermore, in contrast to polyclonal antibody preparations which include different antibodies directed against different determinants (epitopes), each monoclonal antibody is directed against a single determinant on the antigen. In addition to their specificity, the monoclonal antibodies are advantageous in that they may be synthesized uncontaminated by other antibodies. The modifier "monoclonal" is not to be construed as requiring production of the antibody by any particular method. For example, the monoclonal antibodies useful in the present invention may be prepared by the hybridoma methodology described by Kohler et al., Nature, 256:495 (1975), or may be made using recombinant DNA methods in bacterial, eukaryotic animal or plant cells (such as in U.S. Pat. No. 4,816,567). The "monoclonal antibodies" may also be isolated from phage antibody libraries using the techniques described in Clackson et al., Nature, 352:624-628 (1991) and Marks et al., J. Mol. Biol., 222:581-597 (1991), for example. All previously cited references being hereby incorporated by reference in their entirety.

**Chimeric antibody:** As used herein, unless indicated otherwise or contradictory in context, the term "chimeric antibody" refers to a monoclonal antibody comprising a variable region, i.e., binding region, from one source or species and at least a portion of a constant region derived from a different source or species, usually prepared by recombinant DNA techniques. Chimeric antibodies comprising a murine variable region and a human constant region are especially preferred. Such murine/human chimeric antibodies are the product of expressed immunoglobulin genes comprising DNA segments encoding murine immunoglobulin variable regions and DNA segments encoding human immunoglobulin constant regions. Other forms of "chimeric antibodies" encompassed by the present invention are those in which the class or subclass has been modified or changed from that of the original antibody. Such "chimeric" antibodies are also referred to as "class-switched antibodies." Methods for producing chimeric antibodies involve conventional recombinant DNA and gene transfection techniques now well known in the art such as in Morrison, S. L., et al., Proc. Natl. Acad. Sci. USA 81 (1984) 6851-6855 and U.S. Patent No. 5,204,244, which are hereby incorporated by reference in their entirety.

**Humanized antibody:** As used herein, unless indicated otherwise or contradictory in context, the term "humanized antibody" refers to antibodies in which the framework or "complementarity determining regions" (CDR) have been modified to comprise the CDR of an immunoglobulin of different specificity as compared to that of the parent immunoglobulin.

**Human antibody:** As used herein, unless indicated otherwise or contradictory in context, the term "human antibody" includes antibodies having variable and constant regions derived from human germline immunoglobulin sequences. Based on this technology, human antibodies against a great variety of targets can be produced. Examples of human antibodies are for example described in Kellermann, S. A., et al., Curr Opin Biotechnol. 13 (2002) 593-597, which is hereby incorporated by reference in its entirety.

**Recombinant human antibody:** As used herein, unless indicated otherwise or contradictory in context, the term "recombinant human antibody" includes all human antibodies that are prepared, expressed, created or isolated by recombinant means, such as antibodies isolated from a host cell such as a NS0 or CHO cell or from an animal (e.g. a mouse) that is transgenic for human immunoglobulin genes or antibodies expressed using a recombinant expression vector transfected into a host cell. Such recombinant human antibodies have variable and constant regions derived from human germline immunoglobulin sequences in a rearranged form.

**Antigen:** As used herein, unless indicated otherwise or contradictory in context, the term "antigen" is a predetermined antigen to which an antibody can selectively bind. The target antigen may be polypeptide, carbohydrate, nucleic acid, lipid, hapten or other naturally occurring or synthetic compound. Preferably, the target antigen is a polypeptide of the transmembrane receptor family such as integrins, syndecans, selectins or dystroglycans. An antibody "which binds" an antigen of interest, e.g. an integrin expressed on the surface of a neoplastic cell, is one that binds the antigen with sufficient affinity such that the antibody is useful as a therapeutic agent in targeting a cell or tissue expressing the antigen, and does not significantly cross-react with other proteins. In such embodiments, the extent of binding of the antibody to a "non-target" protein will be less than about 10% of the binding of the antibody to its particular target protein as determined by fluorescence activated cell sorting (FACS) analysis or radioimmunoprecipitation (RIA). With regard to the binding of an antibody to a target molecule, the term "specific binding" or "specifically binds to" or is "specific for" a particular polypeptide or an epitope on a particular polypeptide target means binding that is measurably different from a non-specific interaction. Specific binding can be measured, for example, by determining binding of a molecule compared to binding of a control molecule, which generally is a molecule of similar structure that does not have binding activity. For example, specific binding can be determined by competition with a control molecule that is similar to the target, for example, an excess of non-labeled target. In this case, specific binding is indicated if the binding of the labeled target to a probe is competitively inhibited by excess unlabeled target. The term "specific binding" or "specifically binds to" or is "specific for" a particular polypeptide or an epitope on a particular polypeptide target as used herein can be exhibited, for example, by a molecule having a Kd for the target of at least about $10^{-4}$ M, alternatively at least about $10^{-5}$ M, alternatively at least about $10^{-6}$ M, alternatively at least about $10^{-7}$ M, alternatively at least about $10^{-8}$ M, alternatively at least about $10^{-9}$ M, alternatively at least about $10^{-10}$ M, alternatively at least about $10^{-11}$ M, alternatively at least about $10^{-12}$ M, or greater. In one embodiment, the term "specific binding" refers to binding where a molecule binds to a particular polypeptide or epitope on a particular polypeptide without substantially binding to any other polypeptide or polypeptide epitope.

**Constant domains:** As used herein, unless indicated otherwise or contradictory in context, the term "constant domains" refers to domains that are not involved directly in binding the antibody to an antigen but are involved in the effector functions (ADCC, complement binding, and CDC).

**Variable region:** As used herein, unless indicated otherwise or contradictory in context, the term "variable region" (variable region of a light chain (VL), variable region of a heavy chain (VH)) refers to each of the pair of light and heavy chains which is involved directly in binding the antibody to the antigen. The domains of variable human light and heavy chains have the same general structure and each domain comprises four framework (FR) regions whose sequences are widely conserved, connected by three "hypervariable regions" (or complementarity determining regions, CDRs). The framework regions adopt a b-sheet conformation and the CDRs may form loops connecting the b-sheet structure. The CDRs in each chain are held in their three-dimensional structure by the framework regions and form together with the CDRs from the other chain the antigen binding site.

**Hypervariable region:** As used herein, unless indicated otherwise or contradictory in context, the term "hypervariable region" or "antigen-binding portion of an antibody" refers to the amino acid residues of an antibody which are responsible for antigen-binding. The hypervariable region comprises amino acid residues from the "complementarity determining regions" or "CDRs". "Framework" or "FR" regions are those variable domain regions other than the hypervariable region residues as herein defined. Therefore, the light and heavy chains of an antibody comprise from N- to C-terminus the domains FR1, CDR1, FR2, CDR2, FR3, CDR3, and FR4. Especially, CDR3 of the heavy chain is the region which contributes most to antigen binding. CDR and FR regions are determined according to the standard definition of Kabat, et al., Sequences of Proteins of Immunological Interest, 5th ed., Public Health Service, National Institutes of Health, Bethesda, Md. (1991), and/or those residues from a "hypervariable loop".

**Biocompatible:** As used herein, unless indicated otherwise or contradictory in context, the term "biocompatible" means compatible with living cells, tissues, organs or systems posing little to no risk of injury, toxicity or rejection by the immune system.

**Biologically active:** As used herein, unless indicated otherwise or contradictory in context, the term "biologically active" refers to a characteristic of any substance that has activity in a biological system and/or organism. For instance, a substance that, when administered to an organism, has a biological effect on that organism, is considered to be biologically active. In particular examples, a compound, substance or pharmaceutical composition of the present disclosure may be considered biologically active even if a portion of the compound, substance or pharmaceutical composition is biologically active or mimics an activity considered biologically relevant.

**Stem cells:** As used herein, unless indicated otherwise or contradictory in context, the term "stem cell" refers to the term as it is generally understood in the art. For example, in certain embodiments, stem cells, regardless of their source, are cells that are capable of dividing and renewing themselves for long periods, are at least to a degree unspecialized (undifferentiated), and can give rise to (differentiate into) specialized cell types (i.e., they are progenitor or precursor cells for a variety of different, specialized cell types).

**Mesenchymal stem cells:** As used herein, unless indicated otherwise or contradictory in context, the term

"mesenchymal stem cells" generally means multipotent adult stromal cells that can differentiate into a variety of cell types, such as osteoblasts, chondrocytes, and adipocytes.

**Stem cell-like:** As used herein, unless indicated otherwise or contradictory in context, the term "Stem cell-like" refers to a cell which is not a stem cell by its origin but functions as a stem cell and presents similar characteristics such as, for example, the expression of stemness markers like Stro-1 and/or is multipotent thus has the ability to differentiate into various cell types.

**Progenitor cells:** As used herein, unless indicated otherwise or contradictory in context, the term "progenitor cells" generally means a biological cell that, like any stem cell, has a tendency to differentiate into a specific type of cell, but is already more specific than a stem cell and is pushed to differentiate into its "target" cell. Stem cells can generally replicate indefinitely, whereas progenitor cells can divide only a limited number of times.

**Adult stem cells:** As used herein, unless indicated otherwise or contradictory in context, the term "adult stem cells" means undifferentiated cells, found throughout the body after development, that multiply by cell division to replenish dying cells and regenerate damaged tissues. Also known as somatic stem cells, they can be found in juvenile as well as adult animals and human bodies.

**Differentiation:** As used herein, unless indicated otherwise or contradictory in context, the term "differentiation" refers to the process by which a less specialized cell becomes a more specialized cell type and involves a switch from one gene expression pattern to another.

**Differentiated cells:** As used herein, unless indicated otherwise or contradictory in context, the term "differentiated cells" generally means any cell of a specific lineage at the exception of cells containing stem cell specific markers.

**Non-terminally differentiated:** As used herein, unless indicated otherwise or contradictory in context, the term "non-terminally differentiated", when used in relation to a cell, refers to a differentiated cell as defined herein which has not reached its final state of differentiation. For example, in certain embodiments, in the Osteoblast cell lineage, a non-terminally differentiated cell is any differentiated cell of the lineage at the exception of an osteocyte.

**Terminally differentiated:** As used herein, unless indicated otherwise or contradictory in context, the term "terminally differentiated", when used in relation to a cell, refers to a differentiated cell as defined herein which has reached its final state of differentiation. For example, in certain embodiments, in the Osteoblast cell lineage, a terminally differentiated cell is an osteocyte.

**Methods for obtaining stem cells:** Methods for obtaining such stem cells and providing initial culture conditions, such as a liquid culture or semi-solid culture medium, are known in the art. The cells are initially expanded in vivo or in vitro, by contacting the source of the stem cells with a suitable reagent that expands or enriches such cells in the tissue source or in culture. Preferably, adult stem cells are isolated from a tissue source and then expanded or enriched in vitro by exposure to a suitable agent. Cells are obtained from an individual by any suitable method for obtaining a cell sample from an animal, including, but not limited to, collection of bone marrow collection of a bodily fluid (e.g., blood), collection of umbilical cord blood, tissue punch, and tissue dissection, including particularly, but not limited to, any biopsies of skin, intestine, cornea, spinal cord, brain tissue, scalp, stomach, breast, lung (*e.g.,* including lavage and bronchioschopy), fine needle aspirates of the bone marrow, amniotic fluid, placenta and yolk sac.

**Cell lineage:** As used herein, unless indicated otherwise or contradictory in context, the term "cell lineage" refers to the developmental history of a particular cell from its primary state in the fertilized egg or embryo through to its fully differentiated state. The different steps and phases involved in the development of a cell produces many intermediate cells which may be referred to as progenitor or precursor cells in the present application and form an integral part of the cell lineage.

**Bone:** It is conventionally known that mature osteoblasts are the cells responsible for bone formation and are derived from osteoblast precursors. Differentiation of human bone marrow mesenchymal stem cells and osteoblast precursors is one of the important processes for bone regeneration. Osteoblasts differentiate from mesenchymal stem cells. Mature osteoblasts differentiate from osteoblast precursors and into osteocytes which are non-dividing cells. Bone-related neoplastic diseases include, but are not limited to, bone primary tumors (benign tumors or cancers) such as osteoma, osteoid osteoma, osteochondroma, osteoblastoma, enchondroma, giant cell tumor of bone, aneurysmal bone cyst, fibrous dysplasia of bone, osteosarcoma, chondrosarcoma, Ewing's sarcoma, fibrosarcoma; and secondary tumors (i.e. metastasize) such as, for example, in certain embodiments, carcinomas of the prostate, breasts, lungs, thyroid, and kidneys.

**Osteoblast cell lineage:** As used herein, unless indicated otherwise or contradictory in context, the term "osteoblast cell lineage" refers to bone cells at any stage of their development and thus include, but are not limited to, mesenchymal stem cells, osteoblasts, osteocytes or any precursors thereof.

**Cartilage:** Native chondrocytes are responsible for the synthesis and turnover of the cartilage extracellular matrix (ECM), which provides an environment of nutrition diffusion for chondrocytes and provides the joint surface with biomechanical competence. Chondrogenic cells arise from pluripotential adult mesenchymal stem cells (MSCs) through a series of differentiation pathways. Chondrogenic differentiation of MSCs is induced by various intrinsic and extrinsic factors. Growth factors play an important role in this process. For instance, in the hyaline cartilage, growth factors regulate

homeostasis and integrity, as well as development. Cartilage-related neoplastic diseases include, but are not limited to, Chondroma/ecchondroma/enchondroma (Enchondromatosis, Extraskeletal chondroma), Chondrosarcoma (Mesenchymal chondrosarcoma, Myxoid chondrosarcoma), Osteochondroma (Osteochondromatosis), Chondromyxoid fibroma, and Chondroblastoma,

**Chondrocytic cell lineage:** As used herein, unless indicated otherwise or contradictory in context, the term "chondrocytic cell lineage" refers to cartilage cells at any stage of their development and thus include, but are not limited to, mesenchymal stem cells, chondroblasts, chondrocytes or any precursors thereof.

**Muscles:** Skeletal muscle is a highly complex and heterogeneous tissue serving a multitude of functions in the organism. The process of generating muscle - myogenesis - can be divided into several distinct phases. During embryonic myogenesis, mesoderm-derived structures generate the first muscle fibers of the body proper, and in subsequent waves additional fibers are generated along these template fibers. In the perinatal phase, muscle resident myogenic progenitors initially proliferate extensively but, later on, decrease as the number of myonuclei reaches a steady state and myofibrillar protein synthesis peaks. Once the muscle has matured, these progenitors will enter quiescence and henceforth reside within it as satellite cells. Adult skeletal muscle, like all renewing organs, relies on a mechanism that compensates for the turnover of terminally differentiated cells to maintain tissue homeostasis. This type of myogenesis depends on the activation of satellite cells that have the potential to differentiate into new fibers. The most comprehensively studied form of myogenesis takes place when mature muscle is damaged and large cohorts of satellite cells expand mitotically and differentiate to repair the tissue and reestablish homeostasis. It is now generally accepted that satellite cells are closely related to progenitors of somitic origin. The activation of the network of transcription factors that controls skeletal muscle development depends on paracrine factors that are released by adjacent tissues, such as the neural tube, notochord, surface ectoderm and lateral mesoderm. Several secreted factors have been identified that determine the spatial and temporal onset of myogenesis. Signalling molecules, such as Noggin and bone morphogenetic proteins (BMPs) - which inactivate and activate receptors of the transforming growth factor-β (TGFβ) superfamily, respectively - are reported to participate in the orchestration of the activation of myogenesis. Muscle-related neoplastic diseases include, but are not limited to, Rhabdomyosarcoma, and Leiomyosarcoma.

**Muscle cell lineage:** As used herein, unless indicated otherwise or contradictory in context, the term "muscle cell lineage" refers to muscle cells at any stage of their development and thus include, but are not limited to, mesenchymal stem cells, myoblasts, myocytes or any precursors thereof.

**Vascular:** The vasculature in the human body forms through two distinct processes: vasculogenesis and angiogenesis. Vasculogenesis is defined as the process of de novo blood vessel formation occurring when endothelial precursor cells (angioblasts) migrate and differentiate into endothelial cells which form the new vessel. These vascular trees are then extended through angiogenesis which is defined as the new vessel formation secondary to proliferation of endothelial cells from pre-existing vessels. Vasculogenesis as well as angiogenesis occur during the embryologic development of the circulatory system but also in the adult organism from circulating endothelial progenitor cells (derivatives of stem cells) able to contribute, albeit to varying degrees, to neovascularization. Vascular-related neoplastic diseases include, but are not limited to, Hemangiosarcoma, Kaposi's sarcoma, Lymphangiosarcoma, and Infantile hemangio-pericytoma.

**Vascular cell lineage:** As used herein, unless indicated otherwise or contradictory in context, the term "vascular cell lineage" refers to vascular cells at any stage of their development and thus include, but are not limited to, mesenchymal stem cells, angioblast, pericytes and endothelial cells or any precursors thereof.

**Neurons:** It was recently reported that neural cells can be regenerated from neural stem cells (NSCs). These are self-renewing, multipotent adult stem cells that generate the main phenotype of the nervous system. They undergo asymmetric cell division into two daughter cells, one non-specialized and one specialized. NSCs primarily differentiate into neurons, astrocytes, and oligodendrocytes. NSCs are generated throughout an adult's life via the process of neurogenesis. NSCs can be differentiated to replace lost or injured neurons or in many cases even glial cells. NSCs are stimulated to begin differentiation via exogenous cues from their microenvironment, or the neural stem cell niche. This niche defines a zone in which stem cells are retained after embryonic development for the production of new cells of the nervous system. This continual supply of new neurons and glia then provides the postnatal and adult brain with an added capacity for cellular plasticity. Critical to the maintenance of the stem cell niche are microenvironmental cues and cell-cell interactions that act to balance stem cell quiescence with proliferation and to direct neurogenesis versus gliogenesis lineage decisions. Several proteins like different growth factors are involved in the mechanisms of the neural stem cell niche as well as in the maintenance and growth of the newly formed neurons. These include the BMPs, FGFs, PDGF, VEGF, TGF β, BDNF and others. Neuron-related neoplastic diseases include, but are not limited to, Anaplastic astrocytoma, Astrocytoma, Central neurocytoma, Choroid plexus carcinoma, Choroid plexus papilloma, Choroid plexus tumor, Dysembryoplastic neuroepithelial tumour, Ependymal tumor, Fibrillary astrocytoma, Giant-cell glioblastoma, Glioblastoma multiforme, Gliomatosis cerebri, Gliosarcoma, Hemangiopericytoma, Medulloblastoma, Medulloepithelioma, Meningeal carcinomatosis, Neuroblastoma, Neurocytoma, Oligoastrocytoma, Oligodendroglioma, Optic nerve sheath meningioma, Pediatric ependymoma, Pilocytic astrocytoma, Pinealoblastoma, Pineocytoma, Pleomorphic anaplastic neuroblastoma, Pleomorphic xanthoastrocytoma, Primary central nervous system lymphoma, Sphenoid wing meningioma, Subependymal giant cell astrocytoma, Subependymoma, and Trilateral retinoblastoma.

**Neuronal cell lineage:** As used herein, unless indicated otherwise or contradictory in context, the term "neuron lineage" refers to brain cells at any stage of their development and thus include, but are not limited to, neural stem cells, neuroblast, neurocyte and neuroglial cells or any precursors thereof.

**Eye retina:** The vertebrate retina is a light-sensitive layer of tissue, lining the inner surface of the eye. Retinal development involves a complex progression of tissue induction, proliferation of retinal progenitor cell (RPC) populations and terminal differentiation of these cells into specific functional types. Bone morphogenetic protein (BMP), is a member of the transforming growth factor (TGF)-β family of signaling molecules plays an important role in the retinal cell development. BMP-2, -4, and -7 and their receptors (BMPRs) are expressed in the eye during embryogenesis and are essential for multiple aspects of retinal development. Retina-related neoplastic diseases include, but are not limited to, Retinoblastoma. It should also be noted that eye cancers can be primary (starts within the eye) and metastatic (spread to the eye from another organ). The two most common cancers that would spread to the eyes from another organ are breast cancer and lung cancer. Other, less common, sites of origin include prostate, kidney, thyroid, skin, colon and blood or bone marrow.

**Retinal cell lineage:** As used herein, unless indicated otherwise or contradictory in context, the term "retinal cell lineage" refers to eye retina cells at any stage of their development and thus include, but are not limited to, photoreceptor, bipolar cells, rod and cone cells or any precursors thereof.

**Kidneys:** The kidneys are composed of complex tissues consisting of several different cell types including glomerular podocytes, endothelial cells, mesangial cells, interstitial cells, tubular epithelial cells, and connecting duct cells. These cell types interact to establish a precise cellular environment that functions as an efficient tissue. Kidneys-related neoplastic diseases include, but are not limited to, Squamous cell carcinoma, Juxtaglomerular cell, tumor (reninoma), Angiomyolipoma, Renal oncocytoma, Bellini duct carcinoma, Clear-cell sarcoma of the kidney, Mesoblastic nephroma, Wilms' tumor, Mixed epithelial stromal tumor, Clear cell adenocarcinoma, Transitional cell carcinoma, Inverted papilloma, Renal lymphoma, Teratoma, Carcinosarcoma, and Carcinoid tumor.

**Renal cell lineage:** As used herein, unless indicated otherwise or contradictory in context, the term "renal cell lineage" refers to renal cells at any stage of their development and thus include, but are not limited to, mesenchymal stem cells, podocytes, or any precursors thereof.

**Ligaments and Tendons:** Tendons and ligaments (T/L) are dense connective tissues of mesodermal origin. They connect and transmit force from muscle to bone and bone to bone, respectively. Both tissues are able to store elastic energy and withstand hightensile forces, on which locomotion is entirely dependent. T/L are predominantly composed of collagen type I fibrils organized in a highly hierarchical manner that is unique for the T/L. Other collagens (types III-VI, XI, XII, XIV, and XV) and various proteoglycans (decorin, cartilage oligomeric matrix protein (COMP), byglican, lumican, fibromodulin, tenascin-C, etc.) are building the remaining T/L substance. The cellular content of T/L is dominated by tendon-specific fibroblasts named tenocytes. During embryonic development, the tendon-specific cells descend from a sub-set of mesenchymal progenitors condensed in the syndetome, a dorsolateral domain of the sclerotome. L/T-related neoplastic diseases include, but are not limited to, Fibrosarcoma, Malignant fibrous, Hystiocytoma, and Dermatofibrosarcoma.

**Ligament and tendon cell lineage:** As used herein, unless indicated otherwise or contradictory in context, the term "ligament and tendon cell lineage" or "L/T cell lineage" refers to bone or cartilage cells at any stage of their development and thus include, but are not limited to, mesenchymal stem cells, fibroblasts, fibrocytes, or any precursors thereof.

**Skin:** The skin constantly renews itself throughout adult life. Stem cells (SCs) residing in the epidermis ensure the maintenance of adult skin homeostasis, but they also participate in the repair of the epidermis after injuries. The skin protects the body from dehydration, injury and infection. The skin consists of an underlying dermis, separated by a basement membrane from the multilayered overlaying epidermis. The dermis is of mesodermal embryonic origin and contains as adult stem cells fibroblastic mesenchymal stem-cell-like cells. These cells have a multi-lineage differentiation potential, being also able to form adipose tissue or bones. Skin-related neoplastic diseases include, but are not limited to, basal cell carcinoma, squamous cell carcinoma, malignant melanoma, dermatofibrosarcoma protuberans, Merkel cell carcinoma, Kaposi's sarcoma, keratoacanthoma, spindle cell tumors, sebaceous carcinomas, microcystic adnexal carcinoma, Paget's disease of the breast, atypical fibroxanthoma, leiomyosarcoma, and angiosarcoma.

**Fibroblast lineage:** As used herein, unless indicated otherwise or contradictory in context, the term "fibroblast lineage" refers to skin cells at any stage of their development and thus include, but are not limited to, mesenchymal stem cells, fibroblasts, keratinocytes, Merkel cells, melanocytes, Langerhans cells, and any precursor cells thereof.

**Reproduction:** Reproduction (or procreation) is the biological process by which new offspring individual organisms are produced from their parents. Sexual reproduction is a biological process by which organisms create descendants that have a combination of genetic material contributed from two (usually) different members of the species. The development and physiological functions of basic structures in the mammalian reproductive system are influenced

by the tissue-specific expression of members of different growth factors families like the BMP family. The establishment of the germ line is a fundamental aspect of reproduction. Germ cell determination is induced in epiblast cells by the extraembryonic ectoderm, and is not acquired through the inheritance of preformed germ plasma. There is some strong evidence that BMP-4 and -8b play a central role in determining primordial germ cell (PGC) formation in the embryo. The genes encoding BMP-4 and -8b have overlapping expression in the extraembryonic ectoderm before gastrulation, i.e., before PGCs are seen. Thus, PGC formation requires BMP-4 expression. There is also evidence from knockout mammals that BMP-8b is required for PGC formation. Furthermore, there is increasing evidence that locally produced BMPs play a major role in the differentiation of the pituitary gonadotrope. Reproduction-related neoplastic diseases include, but are not limited to, Prostate cancer, Ovary cancer (adenocarcinoma, or glandular cancer) also known as carcinoma of the prostate or prostatic intraepithelial neoplasia.

**Reproduction system lineage:** As used herein, unless indicated otherwise or contradictory in context, the term "reproduction system lineage" refers to Sertoli cells, Leydig cell and Germ cell at any stage of their development, in particular, mesenchymal stem cells.

**Blood:** Blood is a bodily fluid in animals that delivers necessary substances such as nutrients and oxygen to the cells and transports metabolic waste products away from those cells. When it reaches the lungs, gas exchange occurs wherein carbon dioxide is diffused out of the blood into the alveoli and oxygen is diffused into the blood. This oxygenated blood is pumped to the left hand side of the heart in the pulmonary vein and enters the left atrium. From here it passes through the bicuspid valve, through the ventricle and taken all around the body by the aorta. Blood contains antibodies, nutrients, oxygen and much more to help the body work. In vertebrates, it is composed of blood cells suspended in blood plasma. Plasma, which constitutes 55% of blood fluid, is mostly water (92% by volume), and contains dissipated proteins, glucose, mineral ions, hormones, carbon dioxide (plasma being the main medium for excretory product transportation), and blood cells themselves. Albumin is the main protein in plasma, and it functions to regulate the colloidal osmotic pressure of blood. Hematopoietic stem cells (HSCs) are the blood cells that give rise to all the other blood cells and are derived from the mesoderm. They are located in the red bone marrow, which is contained in the core of most bones. The HSCs give rise to the myeloid lineage (monocytes and macrophages, neutrophils, basophils, eosinophils, erythrocytes, megakaryocytes/platelets, dendritic cells), and to the lymphoid lineages (T-cells, B-cells, NK-cells). The most abundant cells in the vertebrate blood are red blood cells (also called RBSs or erythrocytes). These contain hemoglobin, an iron-containing protein, which facilitates oxygen transport by reversibly binding to this respiratory gas and greatly increasing its solubility in blood. Blood-related neoplastic diseases include, but are not limited to, acute lymphoblastic leukemia (ALL), acute myeloid leukemia (AML), chronic lymphocytic leukemia (CLL) and chronic myeloid leukemia (CML).

**Blood cell lineages (myeloid lineage and lymphoid lineage):** As used herein, unless indicated otherwise or contradictory in context, the term "blood cell lineages" refers to blood cells at any stage of their development from the myeloid or from the lymphoid lineage, and thus include, but are not limited to, hematopoietic stem cells (HSC), myeloid progenitors, lymphoid progenitors, mast cells, myeloblasts, monocytes, macrophages, neutrophils, basophils, eosinophils, erythrocytes, megakaryocytes, thrombocytes, dendritic cells, small lymphocytes, T-lymphocytes (T-cells), B-lymphocytes (B-cells), natural killer (NK)-cells, and any precursor cells thereof.

**Adipose tissue:** Adipose tissue is loose connective tissue composed mostly of adipocytes. In addition to adipocytes, adipose tissue contains the stromal vascular fraction (SVF) of cells including preadipocytes, fibroblasts, vascular endothelial cells and a variety of immune cells (i.e. adipose tissue macrophages (ATMs)). Adipose tissue is derived from preadipocytes. Its main role is to store energy in the form of lipids, although it also cushions and insulates the body. Pre-adipocytes are thought to be undifferentiated fibroblasts that can be stimulated to form adipocytes. The pre-adipocytes originate from mesenchymal stem cells. Areolar connective tissue is composed of adipocytes. The term "lipoblast" is used to describe the precursor of the adult cell. The term "lipoblastoma" is used to describe a tumor of this cell type. Adipose tissue-related neoplastic diseases include, but are not limited to, lipoma, Adenolipomas, Angiolipoleiomyomas, Angiolipomas, Corpus callosum lipoma, Cerebellar pontine angle and internal auditory canal lipomas, Chondroid lipomas, Hibernomas, Intradermal spindle cell lipomas, Neural fibrolipomas, Pleomorphic lipomas, Spindle-cell lipomas, Superficial subcutaneous lipomas, Lipoblastoma, Liposarcoma.

**Adipocyte lineage:** As used herein, unless indicated otherwise or contradictory in context, the term "adipocyte cell lineage" refers to adipocyte cells at any stage of their development and thus include, but are not limited to, mesenchymal stem cells, areolar connective cells, adipocytes, pre-adipocytes/lipoblasts, and any precursor cells thereof.

**Digestive system:** The human digestive system is composed mainly of the gastrointestinal tract (including the esophagus, stomach, small intestine, large intestine, rectum and anus) and the accessory digestive glands (the liver, the gall bladder and the pancreas). To achieve the goal of providing energy and nutrients to the body, six major functions take place in the digestive system: ingestion, secretion, mixing and movement, digestion, absorption, excretion. The gastrointestinal wall refers to the specialized series of tissue layers surrounding the lumen of the gastrointestinal tract. The general structure involves the four following layers (ordered from the lumen outward): mucosa, submucosa, muscularis externa, serosa (if the tissue is intraperitoneal) / adventitia (if the tissue is retroperitoneal). Gastrointestinal cancer

refers to malignant conditions of the gastrointestinal tract (GI tract) and accessory organs of digestion. The symptoms relate to the organ affected and can include obstruction (leading to difficulty swallowing or defecating), abnormal bleeding or other associated problems. Gastrointestinal tissue-related neoplastic diseases include, but are not limited to, Esophageal cancer, Stomach cancer, Pancreatic cancer, Liver cancer, Gallbladder cancer, MALT lymphoma, Gastrointestinal stromal tumors and Cancers of the biliary tree, including cholangiocarcinoma.

**Gastrointestinal cell lineages:** As used herein, unless indicated otherwise or contradictory in context, the term " gastrointestinal cell lineages" refers to gastrointestinal cells and cells of the digestive accessory organs at any stage of their development and thus include, but are not limited to interstitial cells of Cajal, gastrointestinal epithelial cells, parietal cells, acinar cells, chief cells, mucus cells, goblet cells, G cells, endocrine I cells, endocrine S cells, endocrine K cells, endocrine M cells, ECL (enterochromaffin) cells, D cells, enteroendocrine cells, APUD cells, hepatocytes, sinusoidal hepatic endothelial cells, Kupffer cells, hepatic stellate cells, centroacinar cells, pancreatic stellate cells, $\alpha$-cells, $\gamma$-cells, $\beta$-cells, $\delta$-cells, centroacinar cells, basophilic cells, ductal cells, columnar cells, cholecystocytes and any precursor cells thereof.

**Lung:** The lung is the essential respiration organ in many air-breathing animals. In mammals the two lungs are located near the backbone on either side of the heart. Their principal function is to transport oxygen from the atmosphere into the bloodstream, and to release carbon dioxide from the bloodstream into the atmosphere. A large surface area is needed for this exchange of gases, which is accomplished by the mosaic of specialized cells that form millions of tiny, exceptionally thin-walled air sacs called alveoli. Lung cells include, but are not limited to, type I pneumocytes, type II pneumocytes, clara cells and goblet cells. Lung tissue-related neoplastic diseases include, but are not limited to, lung cancer also known as carcinoma of the lung or pulmonary carcinoma, Epithelial cells or small-cell lung carcinoma (SCLC) and non-small-cell lung carcinoma (NSCLC).

**Lung cell Lineages:** As used herein, unless indicated otherwise or contradictory in context, the term "lung cell Lineage" refers to lung cells at any stage of their development and thus include, but are not limited to, epithelial cells, erythrocytes, alveolar cells and any precursor cells thereof.

**Head and neck cancer:** As used herein, unless indicated otherwise or contradictory in context, the term "head and neck cancer" refers to a group of cancers that usually starts in the lip, oral cavity, nasal cavity, paranasal sinuses, pharynx, and larynx. About 90% of head and neck cancers are squamous cell carcinomas. Thus, neoplastic diseases such as head and neck cancers include, but are not limited to, oral cancer, nasopharynx cancer, oropharyngeal cancer, hypopharynx cancer and laryngeal cancer.

The cell lineage involved in head and neck cancers includes all the cells involved in the formation of such cancers at any stage of their development and thus include, but are not limited to, cells of the oral cavity, cells of the pharynx, cells of the larynx, cells of the paranasal sinuses and nasal cavity, cells of the salivary glands and any precursor cells thereof.

**Ratio:** As used herein, unless indicated otherwise or contradictory in context, the term "ratio", when used in relation to GFR-binding compound with respect to the adhesion protein inhibitor in the pharmaceutical association or composition disclosed herein, refers to the (molar, weight or part as specified) ratio between the quantity of GFR-binding compound and the quantity of adhesion protein inhibitor. The ratio may be a molar ratio, a weight ratio or a part ratio and will be specified as needed on a case by case basis. Quantity units may conventionally be mole, millimole, gram, milligram or parts. For example, in certain embodiments, it is convenient to express the relative quantity between GFR-binding compounds and adhesion protein inhibitors using densities. It shall be understood that this ratio may be varied according to the neoplastic cell type to be treated.

**Density:** As used herein, unless indicated otherwise or contradictory in context, the term "density", when used in relation to GFR-binding compound with respect to the adhesion protein inhibitor in the pharmaceutical composition disclosed herein, refers to the quantity of GFR-binding compounds, expressed in *e.g.* mole, millimole, gram, or milligram, with respect to one standardised surface unit *e.g.* squared millimetre ($mm^2$), squared micrometre ($\mu m^2$), or squared nanometre ($nm^2$)). For example, in certain embodiments, the ratio between a GFR-binding compound and an adhesion protein inhibitor in the pharmaceutical association or composition disclosed herein may be expressed in pmol per $mm^2$ or pmol/$mm^2$.

**Cell cycle:** As used herein, unless indicated otherwise or contradictory in context, the term "cell cycle" refers to the process through which a vertebrate cell self-replicate. In eukaryote cells, cell cycle consists of four discrete phases: G1, S, G2, and M. Together, the G1, S, and G2 phases make up the period known as interphase. During the S or "synthesis" phase, the cell replicates its DNA creating an exact copy of all of its chromosomes. In the M or "mitotic" phase, the cell division actually occurs and separates the chromosomes in its cell nucleus into two identical sets in two nuclei. The first phase within interphase, from the end of the previous M phase until the beginning of DNA synthesis, is called G1. During this phase the biosynthetic activities of the cell, which are considerably slowed down during M phase, resume at a high rate. This phase is marked by the formation of millions of proteins and later on enzymes that are required in S phase, mainly those needed for DNA replication. The G2 phase, or pre-mitotic phase, is the third and final sub-phase of Interphase in the cell cycle directly preceding Mitosis. It follows the successful completion of S phase and

ends with the onset of prophase, the first phase of mitosis. In order to move from one phase of the cell cycle to the next, a cell must "validate" numerous checkpoints. At each checkpoint, specialized proteins determine whether the necessary conditions exist. If so, the cell is free to enter into the next phase. If not, progression through the cell cycle is halted. For example, in certain embodiments, during G1, the cell passes through a "validation" window punctuated by the restriction point R. During the "validation" phase, different checkpoints ensure that environmental conditions are favourable for replication. If conditions are not favourable, the cell may enter a resting state known as G0. The G0 phase or resting phase is a period in the cell cycle in which cells exist in a quiescent state. G0 phase is viewed as either an extended G1 phase, where the cell is neither dividing nor preparing to divide, or a distinct quiescent stage that occurs outside of the cell cycle. Typically, a healthy vertebrate cell undergoes cell division when needed e.g. to regenerate damaged tissues or simply replace old tissues by new ones, by switching from a G0 resting state into the G1 state of the cell cycle and resume cell division. In contrast, neoplastic cells (such as cancer cells) have lost their ability to suspend cell division and switch to the G0 phase therefore undergoing permanent cell division which is generally referred to uncontrolled cell division or proliferation. Another "validation" window takes place later in the cell cycle, just before a cell moves from G2 to mitosis. Here, a number of proteins scrutinize the cell’s DNA ensuring proper replication has taken place. Finally, another cell cycle "validation" window takes place during mitosis in which different checkpoints determines whether chromosomes are correctly attached to the spindle, and to the network of microtubules that will separate them during cell division.

**Quiescence:** As used herein, unless indicated otherwise or contradictory in context, the term "quiescence", when used in relation to a cell, refers to a resting state during which the cell does not divide, duplicate or proliferate. This state is also called the G0 state. As used herein, "inducing quiescence of a neoplastic cell" thus means to provoke the passage from G1 to G0 of a neoplastic cell which usually has lost the ability to do so. One method to detect the passage from G1 to G0 is, for instance, to monitor the state of phosphorylation of the protein Rb via Western Blot. The Rb protein is normally not phosphorylated during the G0 phase but becomes phosphorylated or even hyper-phosphorylated during the rest of the cell cycle. Consequently, observing the absence (or substantial reduction *i.e.* at least 90% reduction, in comparison with the quantity present in the G1 phase) of phosphorylated Rb protein on a western blot confirms that a neoplastic cell has undergone (at some point in time) a switch to the G0 phase.

**Cell division or cell proliferation:** As used herein, unless indicated otherwise or contradictory in context, the term "cell division" or "cell proliferation", refers to the process by which a cell self-replicate, replicate or is caused to replicate.

**Proliferate:** As used herein, unless indicated otherwise or contradictory in context, the term "proliferate" means to grow, expand or increase or cause to grow, expand or increase. "Proliferative" means having the ability to proliferate. "Anti-proliferative" means having properties to counter, reduce, or inhibit proliferation.

**Hyperproliferation:** As used herein, unless indicated otherwise or contradictory in context, the term "hyperproliferation" or "uncontrolled proliferation" means the abnormal growth, expansion or increase or causing the abnormal growth, expansion or increase. Abnormal growth typically originates from the abnormal regulation of the cell cycle preventing the cell to reach the G0 state and stop cell division and replication. One consequence of hyper or uncontrolled proliferation is the development of neoplastic diseases such as tumours and cancers.

**Hyperproliferative diseases:** As used herein, unless indicated otherwise or contradictory in context, the term "hyperproliferative diseases" is used interchangeably with "neoplastic diseases" and refers to diseases that are characterized by uncontrolled cellular proliferation and/or disruption in programmed cell death. The loss of a cell’s ability to control cellular proliferation is often caused by genetic damage to the cellular pathways responsible for regulating cellular functions, including but not limited to, for example, in certain embodiments, metabolism, cell cycle progression, cell adhesion, vascular function, apoptosis, and angiogenesis.

**Anti-mitogen activity:** As used herein, unless indicated otherwise or contradictory in context, the term "anti-mitogen activity", refers to biological pathways that down-regulate, partially inhibit or suppress cell mitosis *i.e.* cell division. As used herein, a substance or pharmaceutical association which promotes, induces or favours anti-mitogen activity thus means a substance or pharmaceutical association which provides at least part of its effective biologic or therapeutic action by down-regulating, partially inhibiting or suppressing mitosis of the neoplastic cell to be treated.

**Tumor suppressor pathways:** As used herein, unless indicated otherwise or contradictory in context, the term "tumor suppressor pathways" refers to biological pathways that down-regulate, partially inhibit or suppress tumor activity *i.e.* protect the cell against cell defects which could cause tumors or cancers. As used herein, a substance or pharmaceutical association which promotes, induces or favours tumor suppressor pathways thus means a substance or pharmaceutical association which provides at least part of its effective biologic or therapeutic action by up-regulating, activating or promoting the tumor suppressor genes or proteins of the neoplastic cell to be treated. Known tumor suppressor proteins which have a dampening or repressive effect on the regulation of the cell cycle or promote apoptosis include, but are not limited to, p53, pRb, pVHL, APC, CD95, ST5, YPEL3, ST7, and ST14.

**Anti-oncogenic activity:** As used herein, unless indicated otherwise or contradictory in context, the term "anti-oncogenic activity" refers to any molecule having the ability to inhibit, repress or down-regulate the gene or protein

expression of oncogenes. An oncogene is a gene that has the potential to cause neoplastic diseases such as cancer. Examples of anti-oncogene molecules include tumor suppressor proteins.

By **"without temporarily or permanently damaging or killing a neoplastic cell",** is meant, unless indicated otherwise or contradictory in context, that a neoplastic disease, such as cancer, is treated without inducing the entry of the neoplastic cells into apoptosis as it may be assessed by the positive expression of proteins caspase 3, 6 and 7.

By **"without permanently inducing a quiescent state in a neoplastic cell",** is meant, unless indicated otherwise or contradictory in context, that a neoplastic disease, such as cancer, is treated by inducing a temporary quiescent state in the neoplastic cells (the cells enter the G0 phase) and then differentiate into a more specialised state.

**Non-mutagenic:** As used herein, unless indicated otherwise or contradictory in context, the term "non-mutagenic", when used in relation to a therapy or treatment, refers to a therapy which does not involve the alteration or modification of a cell's genome.

**Recoding:** As used herein, unless indicated otherwise or contradictory in context, the term "recoding" or "converting", when used in relation to a cell (in particular a neoplastic cell such as a cancer cell), refers to the action of providing, to a neoplastic cell to be treated, a suitable extracellular micro-environment (e.g. in the form of a pharmaceutical association or composition as defined herein) providing appropriate extracellular signals so that the cell may undergo self-recovery or -healing and be converted into a partially or fully differentiated non-neoplastic cell.

**Recoding therapy:** As used herein, unless indicated otherwise or contradictory in context, the term "recoding therapy" refers to a therapy that promotes and stimulates the cell's natural abilities to redirect its own fate by integrating accurate micro-environmental recoding signals.

**Extracellular micro-environment:** As used herein, unless indicated otherwise or contradictory in context, the term "extracellular micro-environment" refers to the environment surrounding (in functional proximity with) a specific cell which is characterized by biophysical, mechanical and biochemical properties specific for each tissue and is able to regulate cell behavior. Modification of the extracellular micro-environment of a neoplastic cell using, for instance, pharmaceutical associations or compositions as defined herein allows for the conversion or recoding of said neoplastic cell into a healthy, functional, non-neoplastic cell.

**Self-recovery or self-healing:** As used herein, unless indicated otherwise or contradictory in context, the term "self-recovery" or "self-healing", when used in relation to a neoplastic cell such as a cancer cell, means that the neoplastic cell operates its own internal biological changes once it has been recoded or treated using a pharmaceutical association or composition as defined herein, so that it becomes a functional, healthy, non-neoplastic cell. The cell heal itself when in contact with the pharmaceutical composition or association as defined herein and, in contrast with previously reported methods wherein the neoplastic cell is forced to die or maintained in a temporarily reduced or non-proliferative state.

**Physiologically functional cell:** As used herein, unless indicated otherwise or contradictory in context, the term "physiologically functional cell" refers to a cell which is able to perform normally all of the cell functions associated with a particular cell type and necessary for the normal physiology of a cell. These functions include all of the intracellular molecular mechanisms but also all of the activities necessary for a normal communication between the cell and its microenvironment. One method which may be used to verify if a cell is physiologically functional is the grafting of the cell, after the introduction of fluorescent markers, in other mammalian model organisms such as mouse models. The cell is grafted in the tissue corresponding to its cell type. The cell characteristics and normal functions are monitored after a period of time with various methods such as in vivo microscopy or histological staining. The term "functional" when used in relation to a molecule, compound or substance refers to a biological molecule in a form in which it exhibits a property and/or activity by which it is characterized.

**Healthy cell:** As used herein, unless indicated otherwise or contradictory in context, the term "healthy cell" refers to a cell which presents a normal morphology, normal cell functions and normal cell growth which are not damaged, altered or inactivated by a neoplastic disease.

**Shorter period of time:** As used herein, unless indicated otherwise or contradictory in context, the term "shorter period of time", when used in relation to conversion or recoding duration, means substantially shorter to provide a substantial benefit for the treated patient in comparison with existing treatments. In certain embodiments, a shorter period of time includes at least 1.5-fold, at least 2-fold, at least 2.5-fold, at least 3-fold, at least 3.5-fold, at least 4-fold, at least 4.5-fold, at least 5-fold, at least 6-fold, at least 7-fold, at least 8-fold, at least 9-fold or at least 10-fold reduction with respect to an existing treatment.

**Exogenous:** As used herein, unless indicated otherwise or contradictory in context, the term "exogenous" refers to a substance coming from outside a living system such as a cell, an organ, or an individual organism. For example, in certain embodiments, exogenous factors in medicine include pathogens and therapeutics. DNA introduced into a cell via transfection or viral infection may be considered as an exogenous factor. Carcinogens are also commonly referred to as exogenous factors.

**Endogenous:** As used herein, unless indicated otherwise or contradictory in context, the term "endogenous" refers to substances that originate from within an organism, tissue, or cell.

**Intracellular:** As used herein, unless indicated otherwise or contradictory in context, the term "intracellular"

generally means "inside the cell". In vertebrates, such as animals, the cell membrane is the barrier between the inside of the cell and the outside of the cell (the extracellular milieu). Thus, treatments and therapies in which at least one substance, compound, pharmaceutical association, combination or composition penetrates the cell wall of a cell to be treated in order to produce/deliver its (effective) biological effect are considered as intracellular treatments and therapies.

**Extracellular:** As used herein, unless indicated otherwise or contradictory in context, the term "extracellular" means "outside the cell". In vertebrates, such as animals, the cell membrane is the barrier between the inside of the cell (the intracellular milieu) and the outside of the cell. Thus, treatments and therapies in which no substance, compound, pharmaceutical association, combination or composition requires penetration of the cell wall in order to produce/deliver its (effective) biological effect (*e.g.* by interacting with trans-membrane receptors) are considered as extracellular treatments and therapies. However, a therapy using a plurality of substances in order to provide the desired biological effect wherein one or more of these substances require the entry into the intracellular compartment to provide (or deliver) its biological effect is still considered as an extracellular therapy in the sense of the present disclosure insofar as at least one of these substances provide (or deliver) its biological effect without entering the intracellular compartment. In one example, a therapy involving the extracellular action of a GFR-binding compound and the intracellular action of an adhesion protein inhibitor (*e.g.* via gene silencing using si-RNAs or transcription inhibition using micro-RNAs) would still be considered as an extracellular therapy for the purpose of the present disclosure.

**Cytostatic:** As used herein, unless indicated otherwise or contradictory in context, the term "cytostatic" refers to inhibiting, reducing, or suppressing the growth, division, or multiplication of a cell (*e.g.,* a mammalian cell (e.g., a human cell)), bacterium, virus, fungus, protozoan, parasite, prion, or a combination thereof.

**Cytotoxic:** As used herein, unless indicated otherwise or contradictory in context, the term "cytotoxic" refers to killing or causing injurious, toxic, or deadly effect on a cell (*e.g.,* a mammalian cell (e.g., a human cell)), bacterium, virus, fungus, protozoan, parasite, prion, or a combination thereof.

**In vitro:** As used herein, unless indicated otherwise or contradictory in context, the term "in vitro" refers to events that occur in an artificial environment, *e.g.,* in a test tube or reaction vessel, in cell culture, in a Petri dish, etc., rather than within an organism (*e.g.,* animal, plant, or microbe).

**In vivo:** As used herein, unless indicated otherwise or contradictory in context, the term "in vivo" refers to events that occur within an organism (*e.g.,* animal, plant, or microbe or cell or tissue thereof).

**Ex vivo:** As used herein, unless indicated otherwise or contradictory in context, the term "ex vivo" refers to events that occur in an external environment on tissues sourced from an organism (*e.g.,* animal, plant, or microbe) in an attempt to replicate natural living conditions outside such an organism.

**Syndecans:** As used herein, unless indicated otherwise or contradictory in context, the term "syndecans" refers to single transmembrane domain proteins that are thought to act as co-receptors, especially for G protein-coupled receptors. These core proteins carry three to five heparan sulfate and chondroitin sulfate chains, which allow for interaction with a large variety of ligands including fibroblast growth factors, vascular endothelial growth factor, transforming growth factor-beta, fibronectin and antithrombin-1. Interactions between fibronectin and some syndecans can be modulated by the extracellular matrix protein tenascin C. The syndecan protein family has four members. Syndecans 1 and 3 and syndecans 2 and 4, making up separate subfamilies, arose by gene duplication and divergent evolution from a single ancestral gene. The syndecan numbers reflect the order in which the cDNAs for each family member were cloned. All syndecans have an N-terminal signal peptide, an ectodomain, a single hydrophobic transmembrane domain, and a short C-terminal cytoplasmic domain. All syndecans are anchored to plasma membrane via a 24-25 amino acid long hydrophobic transmembrane domain. In mammalian cells, syndecans are expressed by unique genes located on different chromosomes. All members of the syndecan family have 5 exons. The difference in size of the syndecans is credited to the variable length of exon 3, which encodes a spacer domain. In humans, the amino acid length of syndecan 1, 2, 3 and 4 is 310, 201, 346 and 198 respectively. Glycosaminoglycan chains, a member of the heparan sulfate group, are an important component of syndecans and are responsible for a diverse set of syndecan functions. The addition of glycosaminoglycans to syndecan is controlled by a series of post- translational events.

**Cyclin-dependent kinases:** As used herein, unless indicated otherwise or contradictory in context, the term "Cyclin-dependent kinases" or "CDKs" refers to a family of protein involved in the regulation of the cell cycle. They are present in all known eukaryotes, and their regulatory function in the cell cycle has been evolutionarily conserved. By definition, a CDK binds a regulatory protein called a cyclin. It is reported that, without cyclin, CDK has little kinase activity; only the cyclin-CDK complex is considered to be an active kinase. CDKs phosphorylate their substrates on serines and threonines, so they can be said to belong to the serine-threonine kinase family. CDKs and cyclins are thus highly conserved across species and are present in all cell types including those having a neoplastic phenotype (e.g. cancer cells). Most of the known cyclin-CDK complexes regulate the progression through the cell cycle. Animal cells contain at least nine CDKs, four of which, CDK 1, 2, 3, 4 and 6, are reported to be directly involved in cell cycle regulation: CDK1 regulated by cyclin A, cyclin B; CDK2 regulated by cyclin A, cyclin E; CDK3 regulated by cyclin C; CDK4 regulated by cyclin D1, cyclin D2, cyclin D3; CDK5 regulated by CDK5R1, CDK5R2; CDK6 regulated by cyclin D1, cyclin D2, cyclin D3; CDK7 regulated by cyclin H; CDK8 regulated by cyclin C; CDK9 regulated by cyclin T1, cyclin T2a, cyclin T2b, cyclin K.

**Cyclins:** As used herein, unless indicated otherwise or contradictory in context, the term "cyclins" refers to a family of proteins that control the progression of cells through the cell cycle by activating cyclin-dependent kinase (CDK) enzymes. Cyclin D is one of the major cyclins produced in terms of its functional importance. It is known to interact with four CDKs: CDK2, 4, 5, and 6. In proliferating cells, cyclin D-CDK4/6 complex accumulation is of great importance for cell cycle progression. For instance, cyclin D-CDK4/6 complexes partially phosphorylates retinoblastoma tumor suppressor protein (Rb), whose inhibition can induce expression of some genes important for S phase progression.

**Patient/subject:** As used herein, unless indicated otherwise or contradictory in context, the term "patient" or "subject", which are used interchangeably, refers to any organism to which a composition in accordance with the invention may be administered, e.g., for experimental, diagnostic, prophylactic, and/or therapeutic purposes. Typical subjects include animals (e.g., mammals such as mice, rats, rabbits, non-human primates, and humans) and/or plants. As used herein, patients/subjects include those individuals who may seek or be in need of treatment, requires treatment, is receiving treatment, will receive treatment, or a subject who is under care by a trained professional for a particular disease or condition.

**Purified:** As used herein, unless indicated otherwise or contradictory in context, the term "purify," "purified," "purification" means to make substantially pure or clear from unwanted components, material defilement, admixture or imperfection.

**Targeted Cells:** As used herein, unless indicated otherwise or contradictory in context, the term "targeted cells" refers to any one or more cells of interest. The cells may be found in vitro, in vivo, in situ or in the tissue or organ of an organism. The organism may be an animal, preferably a mammal, more preferably a human and most preferably a patient.

**Molecule length:** As used herein, unless indicated otherwise or contradictory in context, the term molecule or peptide "length" or "size" means the longest 2D or 3D distance which may possibly be measured within the molecule. For cyclic molecules, "length" or "size" means the longest measurable distance across the cyclic structure. Throughout the present disclosure, when a molecule size or length is given (in general using the nanometre, nm, unit), the following procedures were used to calculate them:

- The so-called « 2D » procedure: a 2D chemical structure was drawn in e.g. the ChemDraw® Software. Then, size measurement was carried out via the available ChemDraw length measurement tools. The length value given herein corresponds to the longest 2D length of the molecule using the default settings 2D bond sizes and angles of the software.
- Alternatively, the so-called "3D" procedure may be followed:

  (1) Drawing of the chemical structure of the molecule using suitable softwares (such as ChemDraw).
  (2) Creating a 3D structure model of the molecule hereby drawn using SCWRL (Protein Sci. 2003; 12(9):2001-14) or MODELLER (Current Protocols in Bioinformatics. 15:5.6:5.6.1-5.6.30), each of which is hereby incorporated by reference in its entirety.
  (3) Incubating the obtained 3D structure model in a box simulation containing water for few milliseconds using AMBER (J. Computat. Chem. 2005; 26, 1668-1688), which is hereby incorporated by reference in its entirety.
  (4) Measuring the size of the molecule hereby obtained using softwares such as Pymol® using available Pymol length measurement tools (DeLano Scientific LLC, http://www.pymol.org).

**Root Mean Square Deviation:** As used herein, unless indicated otherwise or contradictory in context, the term "Root Mean Square Deviation" or "RMSD" is well known in the art and means the square root of the arithmetic mean of the square of the distances between certain matched atoms. One can represent a molecular conformation as a vector whose components are the Cartesian coordinates of the molecule's atoms. Therefore, a conformation for a molecule with N atoms can be represented as a 3N-dimensional vector of real numbers. To calculate the RMSD of a pair of peptides or peptidomimetics (e.g. x and y), each one of them must be represented as a 3N-length (assuming N atoms) vector of coordinates. The RMSD is therefore the square root of the arithmetic mean of the square of the distances between corresponding atoms of x and y. It is a measure of the average atomic displacement between the conformations of the two structures:

$$\sqrt{\frac{1}{N}\sum_{i=1}^{N}|xi - yi|1^2}$$

In other words, the RMSD is the measure of the average distance between the atoms (usually the backbone atoms) of superimposed polypeptides or peptidomimetics. In the study of globular protein conformations, one customarily

measures the similarity in three-dimensional structure by the RMSD of the Cα atomic coordinates after optimal rigid body superposition.

The RMSD value of a given peptide or peptidomimetic with respect to a specifically selected reference structure (hereinafter may also be referred to as "PEPREF") may be calculated using various methods all well know by the skilled person. However, for the purpose of the present disclosure and for the avoidance of doubts, the RMSD of a given peptide or peptidomimetic as used in the present disclosure is obtained precisely using the following procedure:

STEP 1: Creating a 3-dimensional model of (i.e. obtaining 3D structure coordinates for) a peptide or peptidomimetic for which the RMSD is to be calculated, by:

STEP 1.1: Obtaining a set of polypeptide 3D structure coordinates based on the alignment with the sequence of a peptide or peptidomometic for which the RMSD value is to be calculated, using the BLAST algorithm according to the following procedure:

1. Open the following link to access the "Standard Protein Blast" tool: **http://blast.ncbi.nlm.nih.gov/Blast.cgi?PROGRAM=blastp&PAGE_TYPE=BlastSearch&LINK_LO C=blasthome**
2. Enter the amino acid sequence of the peptide or peptidomimetic of interest in the "Enter Query Sequence" section. The alignment is performed one sequence after the other (this is not a multiple alignment tool).
3. In the section "Choose Search Set", choose the following database: Protein Data Bank Proteins (pdb).
4. In the section "Choose Search Set", do not exclude « Models (XM/XP) » and do not Exclude « Uncultured /environmental sample sequences ».
5. In the section "Program Selection", choose the following algorithm: blastp (protein-protein BLAST)
6. Leave other fields as shown on the screenshot in Figure 20.
7. Run BLAST.
8. The results obtained from the query are presented in the form of several pdb files.
9. From this output results, the first ten (10) PDB files corresponding to the best sequence alignments are retained. This set of 10 PDB files or structures will be used in the next step (Step 2: structural alignments with STAMP).
10.Finally, clean up the 10 structures contained in the 10 PDB files by removing all e.g. additional small molecules, receptors or portions thereof, dimers or portions thereof, so as to retain only the polypeptide chain of interest.

The set of pdf files contains the polypeptide 3D structure coordinates of the 10 structures having the highest sequence homology with the peptide or peptidomometic for which the RMSD value is to be calculated.

STEP 1.2: Performing the structural alignment of the set of 3D structure coordinates obtained in STEP 1.1, thereby obtaining a set of aligned polypeptide 3D structure coordinates, by using STAMP (Structural Alignment of Multiple Proteins Version 4.2) according to the following procedure:

1. Open the following link to access the "STAMP superposition" tool: **http://www.russelllab.org/cgi-bin/pdc/stamp.pl**
2. In the section entitled "Structure A", input the PDB file corresponding to the first structure from the set of ten 3D structure coordinates obtained in STEP 1.1, which corresponds to the best sequence alignment with BLAST.
3. In the section entitled "Structure B", input the PDB file corresponding to the second structure from the set of ten 3D structure coordinates obtained in STEP 1.1, which corresponds to the second best sequence alignment with BLAST.
4. Run STAMP.
5. Repeat steps 2 to 4 with the other eight pdb files from the set of ten 3D structure coordinates identified in STEP 1.1 by successively entering the PDB files in the field "Structure B".
6. As a result, the structural alignment of the 10 structures contained in the set of PDB files obtained in STEP 1.1 is obtained in the form of 9 disctinct PDB files each containing a pair of aligned polypeptide 3D structure (structure 1 with structure 2, structure 1 with structure 3, structure 1 with structure 4, ... , structure 1 with structure 10).
7. From these 9 "pair" PDB files, 10 PDB files each containing one of structures 1 to 10 are created.
8. 10 PDB files containing the aligned 3D structure coordinates are thus obtained from STEP 1.2. for use in the next step.

STEP 1.3: Modelling the sequence of peptide or peptidomometic for which the RMSD value is to be calculated

against the set of aligned polypeptide 3D structure coordinates obtained in STEP 1.2, thereby obtaining a set of 3D structure coordinates for the peptide or peptidomometic for which the RMSD value is to be calculated, using SCWRL (reference: "SCWRL and MollDE: computer programs for side-chain conformation prediction and homology modeling", Nature Protocols VOL.3 NO.12 2008, Qiang Wang et al.; which is hereby incorporated by reference in its entirety) according to the following procedure:

1. Insert the input sequence of a peptide or peptidomometic for which the RMSD value is to be calculated in Fasta format.
2. Import the first PDB file containing the aligned polypeptide 3D structure coordinates obtained in STEP 1.2.
3. Run SCWRL by typing the following command for Unix based systems: "scwrl_path/scwrl3 -i inputpdbfile -o outputpdbfile -s sequencefile 4 logfile".
4. As a result, a first PBD file is obtained containing the predicted 3D structure coordinates of the peptide or peptidomometic for which the RMSD value is to be calculated.
5. Repeat steps 1 to 3 using the 9 remaining PDB files obtained in STEP 1.2.
6. 10 PDB files are obtained from STEP 1.3 for use in the following STEP 1.4.

STEP 1.4: Minimizing the free energy (ΔG) of the set of 3D structure coordinates for the peptide or peptidomometic for which the RMSD value is to be calculated obtained in STEP 1.3 using GROMACS (Reference: Hess B, Kutzner C, Van Der Spoel D, Lindahl E (2008). "GROMACS 4: Algorithms for Highly Efficient, Load-Balanced, and Scalable Molecular Simulation". J Chem Theory Comput 4 (2): 435; which is hereby incorporated by reference in its entirety) according to the following procedure:

1. Create a Gromacs topology (gmx) file from the first PDB file of the modeled peptide or peptidomometic for which the RMSD value is to be calculated obtained in STEP 1.3, by using the command « pdb2gmx -f NOMDUFICHIERPDB.pdb -water spc». "NOMDUFICHIERPDB" is the name of the input PDB file.
2. Create a box around the imported modeled peptide or peptidomometic by using the command « editconf -f conf.gro -bt cubic d 0.7 o box.gro».
3. Add solvent (water) molecules into the box by using the command « genbox -cp box.gro -cs spc216.gro -p topol.top - o solvated.gro».
4. Prepare the input for the molecular dynamics (MD) run with the command « vim em.mdp ». Default run is set to 1000 nsteps.
5. Create an input for the MD run by using the command « grompp -f em.mdp -p topol.top -c solvated.gro -o em.tpr».
6. Run the command « mdrun -v -deffnm em» to perform the actual energy minimization.
7. Run the command « g energy -f em.edr -s em.tpr -o em.xvg » and then run option « 7 ».
8. As a result, a first XMG file is obtained. To view the XMG file run the command « xmgrace em.xvg ».
9. Repeat steps 1 to 8 with the 9 remaining structures obtained in STEP 1.3. 10 XMG files are thus obtained.
10.The structure of lowest energy is obtained from each XMG file in the form of a PDB file. 10 PDB files each containing one structure of lowest energy are thus obtained from STEP 1.4 for use in the next step.

STEP 2: Calculating the RMSD of the peptide or peptidomimetic for which the RMSD value is to be calculated by comparing the 3D structure coordinates of the peptide or peptidomimetic obtained in STEP 1.4 with the 3D structure coordinates of PEPREF to obtain the lowest possible RMSD value using FATCAT (Flexible structure AlignmenT by Chaining Aligned fragment pairs allowing Twists) according to the following procedure:

1. Open the following link to access the "FATCAT" software: **http://fatcat.burnham.org**
2. Open the "pairwise alignment" tool.
3. Import the PDB file containing the structure coordinates of PEPREF in the "Get the 1st structure" section.
4. Import the first PDB file of the peptide or peptidomimetic for which the RMSD value is to be calculated with minimized energy obtained in STEP 1.4.
5. Run FATCAT.
6. As a result, a first RMSD value of the first structure of the peptide or peptidomimetic for which the RMSD value is to be calculated as obtained in STEP 1.4 will be obtained in the output report.
7. Repeat steps 1 to 5 with the 9 remaining structures (PDB files) obtained from STEP 1.4.
8. The peptide or peptidomimetic structure with the lowest RMSD (out of the ten RMSD values successively obtained) is the value taken into account in the present application.

**3D structure coordinates of PEPREF:** As used herein, unless indicated otherwise or contradictory in context,

the 3D structure coordinates of PEPREF are as follows:

| | | | | | | | | |
|---|---|---|---|---|---|---|---|---|
| ATOM | 511 | N | LYS | A | 1 | -14.570 | 46.437 | 27.424 |
| ATOM | 512 | CA | LYS | A | 1 | -13.512 | 45.748 | 28.151 |
| ATOM | 513 | C | LYS | A | 1 | -13.655 | 44.259 | 27.884 |
| ATOM | 514 | O | LYS | A | 1 | -12.769 | 43.463 | 28.197 |
| ATOM | 515 | CB | LYS | A | 1 | -13.605 | 46.029 | 29.652 |
| ATOM | 516 | CG | LYS | A | 1 | -13.640 | 47.509 | 29.991 |
| ATOM | 517 | CD | LYS | A | 1 | -12.615 | 48.297 | 29.183 |
| ATOM | 518 | CE | LYS | A | 1 | -12.625 | 49.768 | 29.575 |
| ATOM | 519 | NZ | LYS | A | 1 | -13.994 | 50.369 | 29.497 |
| ATOM | 520 | N | ILE | A | 2 | -14.792 | 43.890 | 27.309 |
| ATOM | 521 | CA | ILE | A | 2 | -15.051 | 42.499 | 26.967 |
| ATOM | 522 | C | ILE | A | 2 | -14.911 | 42.370 | 25.444 |
| ATOM | 523 | O | ILE | A | 2 | -15.531 | 43.125 | 24.683 |
| ATOM | 524 | CB | ILE | A | 2 | -16.466 | 42.065 | 27.401 |
| ATOM | 525 | CG1 | ILE | A | 2 | -16.630 | 42.238 | 28.915 |
| ATOM | 526 | CG2 | ILE | A | 2 | -16.710 | 40.629 | 26.985 |
| ATOM | 527 | CD1 | ILE | A | 2 | -15.631 | 41.478 | 29.30 |
| ATOM | 528 | N | PRO | A | 3 | -14.085 | 41.411 | 24.989 |
| ATOM | 529 | CA | PRO | A | 3 | -13.789 | 41.109 | 23.588 |
| ATOM | 530 | C | PRO | A | 3 | -14.998 | 40.695 | 22.768 |
| ATOM | 531 | O | PRO | A | 3 | -15.969 | 40.164 | 23.305 |
| ATOM | 532 | CB | PRO | A | 3 | -12.785 | 39.968 | 23.688 |
| ATOM | 533 | CG | PRO | A | 3 | -12.156 | 40.166 | 25.007 |
| ATOM | 534 | CD | PRO | A | 3 | -13.330 | 40.506 | 25.867 |
| ATOM | 535 | N | LYS | A | 4 | -14.937 | 40.937 | 21.463 |
| ATOM | 536 | CA | LYS | A | 4 | -16.023 | 40.529 | 20.590 |
| ATOM | 537 | C | LYS | A | 4 | -15.886 | 39.015 | 20.391 |
| ATOM | 538 | O | LYS | A | 4 | -14.903 | 38.415 | 20.831 |
| ATOM | 539 | CB | LYS | A | 4 | -15.926 | 41.244 | 19.245 |
| ATOM | 540 | CG | LYS | A | 4 | -15.802 | 42.751 | 19.355 |
| ATOM | 541 | CD | LYS | A | 4 | -16.292 | 43.433 | 18.083 |
| ATOM | 542 | CE | LYS | A | 4 | -16.162 | 44.943 | 18.177 |
| ATOM | 543 | NZ | LYS | A | 4 | -16.825 | 45.628 | 17.019 |
| ATOM | 544 | N | ALA | A | 5 | -16.85 | 38.393 | 19.759 |
| ATOM | 545 | CA | ALA | A | 5 | -16.811 | 36.955 | 19.507 |
| ATOM | 546 | C | ALA | A | 5 | -15.772 | 36.771 | 18.416 |
| ATOM | 547 | O | ALA | A | 5 | -15.727 | 37.534 | 17.455 |
| ATOM | 548 | CB | ALA | A | 5 | -18.168 | 36.419 | 19.043 |

(continued)

| | | | | | | | | |
|---|---|---|---|---|---|---|---|---|
| ATOM | 549 | N | CYS | A | 6 | -14.935 | 35.756 | 18.562 |
| ATOM | 550 | CA | CYS | A | 6 | -13.887 | 35.518 | 17.584 |
| ATOM | 551 | C | CYS | A | 6 | -14.347 | 34.765 | 16.338 |
| ATOM | 552 | O | CYS | A | 6 | -15.327 | 34.018 | 16.368 |
| ATOM | 553 | CB | CYS | A | 6 | -12.743 | 34.768 | 18.241 |
| ATOM | 554 | SG | CYS | A | 6 | -11.198 | 34.959 | 17.353 |
| ATOM | 555 | N | CYS | A | 7 | -13.623 | 34.973 | 15.243 |
| ATOM | 556 | CA | CYS | A | 7 | -13.931 | 34.328 | 13.969 |
| ATOM | 557 | C | CYS | A | 7 | -13.091 | 33.071 | 13.798 |
| ATOM | 558 | O | CYS | A | 7 | -11.961 | 33.123 | 13.302 |
| ATOM | 559 | CB | CYS | A | 7 | -13.653 | 35.290 | 12.824 |
| ATOM | 560 | SG | CYS | A | 7 | -13.930 | 34.633 | 11.154 |
| ATOM | 561 | N | VAL | A | 8 | -13.654 | 31.941 | 14.209 |
| ATOM | 562 | CA | VAL | A | 8 | -12.949 | 30.684 | 14.110 |
| ATOM | 563 | C | VAL | A | 8 | -13.653 | 29.733 | 13.157 |
| ATOM | 564 | O | VAL | A | 8 | -14.759 | 30.016 | 12.687 |
| ATOM | 565 | CB | VAL | A | 8 | -12.814 | 30.038 | 15.492 |
| ATOM | 566 | CG1 | VAL | A | 8 | -11.807 | 30.825 | 16.337 |
| ATOM | 567 | CG2 | VAL | A | 8 | -14.161 | 30.006 | 16.170 |
| ATOM | 568 | N | PRO | A | 9 | -13.003 | 28.601 | 12.828 |
| ATOM | 569 | CA | PRO | A | 9 | -13.593 | 27.615 | 11.918 |
| ATOM | 570 | C | PRO | A | 9 | -14.726 | 26.886 | 12.631 |
| ATOM | 571 | O | PRO | A | 9 | -14.581 | 26.476 | 13.780 |
| ATOM | 572 | CB | PRO | A | 9 | -12.423 | 26.676 | 11.601 |
| ATOM | 573 | CG | PRO | A | 9 | -11.204 | 27.487 | 11.925 |
| ATOM | 574 | CD | PRO | A | 9 | -11.620 | 28.226 | 13.163 |
| ATOM | 575 | N | THR | A | 10 | -15.847 | 26.721 | 11.942 |
| ATOM | 576 | CA | THR | A | 10 | -16.999 | 26.060 | 12.527 |
| ATOM | 577 | C | THR | A | 10 | -17.334 | 24.767 | 11.804 |
| ATOM | 578 | O | THR | A | 10 | -18.097 | 23.943 | 12.303 |
| ATOM | 579 | CB | THR | A | 10 | -18.211 | 27.010 | 12.523 |
| ATOM | 580 | OG1 | THR | A | 10 | -18.491 | 27.445 | 11.185 |
| ATOM | 581 | CG2 | THR | A | 10 | -17.902 | 28.230 | 13.375 |
| ATOM | 582 | N | GLU | A | 11 | -16.750 | 24.586 | 10.627 |
| ATOM | 583 | CA | GLU | A | 11 | -16.980 | 23.377 | 9.848 |
| ATOM | 584 | C | GLU | A | 11 | -15.643 | 22.935 | 9.246 |
| ATOM | 585 | O | GLU | A | 11 | -15.029 | 23.666 | 8.464 |
| ATOM | 586 | CB | GLU | A | 11 | -17.981 | 23.624 | 8.715 |
| ATOM | 587 | CG | GLU | A | 11 | -19.421 | 23.807 | 9.163 |

(continued)

| | | | | | | | | |
|---|---|---|---|---|---|---|---|---|
| ATOM | 588 | CD | GLU | A | 11 | -19.686 | 25.166 | 9.770 |
| ATOM | 589 | OE1 | GLU | A | 11 | -19.478 | 26.175 | 9.073 |
| ATOM | 590 | OE2 | GLU | A | 11 | -20.111 | 25.227 | 10.939 |
| ATOM | 591 | N | LEU | A | 12 | -15.183 | 21.749 | 9.622 |
| ATOM | 592 | CA | LEU | A | 12 | -13.923 | 21.254 | 9.104 |
| ATOM | 593 | C | LEU | A | 12 | -14.062 | 19.912 | 8.386 |
| ATOM | 594 | O | LEU | A | 12 | -15.136 | 19.299 | 8.359 |
| ATOM | 595 | CB | LEU | A | 12 | -12.893 | 21.144 | 10.230 |
| ATOM | 596 | CG | LEU | A | 12 | -12.660 | 22.422 | 11.054 |
| ATOM | 597 | CD1 | LEU | A | 12 | -13.475 | 22.350 | 12.337 |
| ATOM | 598 | CD2 | LEU | A | 12 | -11.181 | 22.586 | 11.399 |
| ATOM | 599 | N | SER | A | 13 | -12.971 | 19.476 | 7.771 |
| ATOM | 600 | CA | SER | A | 13 | -12.964 | 18.218 | 7.046 |
| ATOM | 601 | C | SER | A | 13 | -11.568 | 17.628 | 7.164 |
| ATOM | 602 | O | SER | A | 13 | -10.613 | 18.320 | 7.550 |
| ATOM | 603 | CB | SER | A | 13 | -13.346 | 18.435 | 5.578 |
| ATOM | 604 | OG | SER | A | 13 | -12.404 | 19.261 | 4.923 |
| ATOM | 605 | N | ALA | A | 13 | -11.449 | 16.352 | 6.818 |
| ATOM | 606 | CA | ALA | A | 13 | -10.179 | 15.665 | 6.949 |
| ATOM | 607 | C | ALA | A | 13 | -9.421 | 15.471 | 5.652 |
| ATOM | 608 | O | ALA | A | 13 | -9.941 | 15.720 | 4.563 |
| ATOM | 609 | CB | ALA | A | 13 | -10.413 | 14.306 | 7.626 |
| ATOM | 610 | N | ILE | A | 14 | -8.171 | 15.046 | 5.783 |
| ATOM | 611 | CA | ILE | A | 14 | -7.343 | 14.746 | 4.623 |
| ATOM | 612 | C | ILE | A | 14 | -6.475 | 13.559 | 5.004 |
| ATOM | 613 | O | ILE | A | 14 | -6.212 | 13.316 | 6.183 |
| ATOM | 614 | CB | ILE | A | 14 | -6.401 | 15.916 | 4.183 |
| ATOM | 615 | CG1 | ILE | A | 14 | -5.284 | 16.106 | 5.200 |
| ATOM | 616 | CG2 | ILE | A | 14 | -7.188 | 17.211 | 3.982 |
| ATOM | 617 | CD1 | ILE | A | 14 | -4.173 | 16.973 | 4.696 |
| ATOM | 618 | N | SER | A | 15 | -6.045 | 12.806 | 3.999 |
| ATOM | 619 | CA | SER | A | 15 | -5.187 | 11.662 | 4.242 |
| ATOM | 620 | C | SER | A | 15 | -3.740 | 12.089 | 4.217 |
| ATOM | 621 | O | SER | A | 15 | -3.360 | 13.020 | 3.508 |
| ATOM | 622 | CB | SER | A | 15 | -5.416 | 10.584 | 3.185 |
| ATOM | 623 | OG | SER | A | 15 | -6.667 | 9.971 | 3.401 |
| ATOM | 624 | N | MET | A | 16 | -2.933 | 11.409 | 5.012 |
| ATOM | 625 | CA | MET | A | 16 | -1.518 | 11.700 | 5.047 |
| ATOM | 626 | C | MET | A | 16 | -0.778 | 10.414 | 5.244 |

(continued)

| | | | | | | | | |
|---|---|---|---|---|---|---|---|---|
| ATOM | 627 | O | MET | A | 16 | -1.137 | 9.594 | 6.078 |
| ATOM | 628 | CB | MET | A | 16 | -1.170 | 12.694 | 6.164 |
| ATOM | 629 | CG | MET | A | 16 | -1.848 | 14.042 | 5.974 |
| ATOM | 630 | SD | MET | A | 16 | -1.017 | 15.431 | 6.760 |
| ATOM | 631 | CE | MET | A | 16 | -0.799 | 14.823 | 8.475 |
| ATOM | 632 | N | LEU | A | 17 | 0.238 | 10.231 | 4.426 |
| ATOM | 633 | CA | LEU | A | 17 | 1.077 | 9.065 | 4.508 |
| ATOM | 634 | C | LEU | A | 17 | 2.289 | 9.610 | 5.264 |
| ATOM | 635 | O | LEU | A | 17 | 2.939 | 10.565 | 4.818 |
| ATOM | 636 | CB | LEU | A | 17 | 1.461 | 8.608 | 3.100 |
| ATOM | 637 | CG | LEU | A | 17 | 2.324 | 7.355 | 2.955 |
| ATOM | 638 | CD1 | LEU | A | 17 | 1.553 | 6.145 | 3.445 |
| ATOM | 639 | CD2 | LEU | A | 17 | 2.723 | 7.190 | 1.492 |
| ATOM | 640 | N | TYR | A | 18 | 2.581 | 9.029 | 6.418 |
| ATOM | 641 | CA | TYR | A | 18 | 3.706 | 9.501 | 7.196 |
| ATOM | 642 | C | TYR | A | 18 | 4.434 | 8.333 | 7.835 |
| ATOM | 643 | O | TYR | A | 18 | 4.081 | 7.186 | 7.603 |
| ATOM | 644 | CB | TYR | A | 18 | 3.222 | 10.458 | 8.281 |
| ATOM | 645 | CG | TYR | A | 18 | 2.386 | 9.782 | 9.346 |
| ATOM | 646 | CD1 | TYR | A | 18 | 1.029 | 9.527 | 9.147 |
| ATOM | 647 | CD2 | TYR | A | 18 | 2.961 | 9.379 | 10.550 |
| ATOM | 648 | CE1 | TYR | A | 18 | 0.273 | 8.894 | 10.128 |
| ATOM | 649 | CE2 | TYR | A | 18 | 2.218 | 8.745 | 11.526 |
| ATOM | 650 | CZ | TYR | A | 18 | 0.877 | 8.508 | 11.317 |
| ATOM | 651 | OH | TYR | A | 18 | 0.134 | 7.922 | 12.318 |
| ATOM | 652 | N | LEU | A | 19 | 5.439 | 8.651 | 8.650 |
| ATOM | 653 | CA | LEU | A | 19 | 6.255 | 7.661 | 9.347 |
| ATOM | 654 | C | LEU | A | 19 | 6.210 | 7.946 | 10.847 |
| ATOM | 655 | O | LEU | A | 19 | 6.685 | 8.992 | 11.288 |
| ATOM | 656 | CB | LEU | A | 19 | 7.701 | 7.763 | 8.871 |
| ATOM | 657 | CG | LEU | A | 19 | 7.901 | 7.850 | 7.359 |
| ATOM | 658 | CD1 | LEU | A | 19 | 9.300 | 8.379 | 7.039 |
| ATOM | 659 | CD2 | LEU | A | 19 | 7.669 | 6.482 | 6.748 |
| ATOM | 660 | N | ASP | A | 20 | 5.652 | 7.035 | 11.639 |
| ATOM | 661 | CA | ASP | A | 20 | 5.594 | 7.282 | 13.071 |
| ATOM | 662 | C | ASP | A | 20 | 7.001 | 7.289 | 13.662 |
| ATOM | 663 | O | ASP | A | 20 | 7.985 | 7.185 | 12.932 |
| ATOM | 664 | CB | ASP | A | 20 | 4.714 | 6.244 | 13.786 |
| ATOM | 665 | CG | ASP | A | 20 | 5.292 | 4.837 | 13.752 |

(continued)

| | | | | | | | | |
|---|---|---|---|---|---|---|---|---|
| ATOM | 666 | OD1 | ASP | A | 20 | 6.533 | 4.678 | 13.775 |
| ATOM | 667 | OD2 | ASP | A | 20 | 4.491 | 3.882 | 13.732 |
| ATOM | 668 | N | GLU | A | 21 | 7.089 | 7.413 | 14.981 |
| ATOM | 669 | CA | GLU | A | 21 | 8.375 | 7.451 | 15.669 |
| ATOM | 670 | C | GLU | A | 21 | 9.250 | 6.212 | 15.463 |
| ATOM | 671 | O | GLU | A | 21 | 10.459 | 6.246 | 15.738 |
| ATOM | 672 | CB | GLU | A | 21 | 8.164 | 7.699 | 17.171 |
| ATOM | 673 | CG | GLU | A | 21 | 7.220 | 6.731 | 17.868 |
| ATOM | 674 | CD | GLU | A | 21 | 5.828 | 6.726 | 17.257 |
| ATOM | 675 | OE1 | GLU | A | 21 | 5.347 | 7.810 | 16.856 |
| ATOM | 676 | OE2 | GLU | A | 21 | 5.210 | 5.640 | 17.185 |
| ATOM | 677 | N | ASN | A | 22 | 8.646 | 5.133 | 14.966 |
| ATOM | 678 | CA | ASN | A | 22 | 9.377 | 3.889 | 14.727 |
| ATOM | 679 | C | ASN | A | 22 | 9.682 | 3.647 | 13.249 |
| ATOM | 680 | O | ASN | A | 22 | 10.095 | 2.554 | 12.853 |
| ATOM | 681 | CB | ASN | A | 22 | 8.591 | 2.707 | 15.299 |
| ATOM | 682 | CG | ASN | A | 22 | 8.384 | 2.827 | 16.794 |
| ATOM | 683 | OD1 | ASN | A | 22 | 9.349 | 2.930 | 17.560 |
| ATOM | 684 | ND2 | ASN | A | 22 | 7.125 | 2.819 | 17.221 |
| ATOM | 685 | N | GLU | A | 23 | 9.485 | 4.672 | 12.435 |
| ATOM | 686 | CA | GLU | A | 23 | 9.749 | 4.558 | 11.015 |
| ATOM | 687 | C | GLU | A | 23 | 8.790 | 3.603 | 10.299 |
| ATOM | 688 | O | GLU | A | 23 | 9.145 | 2.996 | 9.292 |
| ATOM | 689 | CB | GLU | A | 23 | 11.203 | 4.129 | 10.784 |
| ATOM | 690 | CG | GLU | A | 23 | 12.232 | 5.210 | 11.082 |
| ATOM | 691 | CD | GLU | A | 23 | 12.072 | 6.410 | 10.166 |
| ATOM | 692 | OE1 | GLU | A | 23 | 12.108 | 6.218 | 8.928 |
| ATOM | 693 | OE2 | GLU | A | 23 | 11.907 | 7.541 | 10.677 |
| ATOM | 694 | N | LYS | A | 24 | 7.580 | 3.459 | 10.826 |
| ATOM | 695 | CA | LYS | A | 24 | 6.583 | 2.624 | 10.175 |
| ATOM | 696 | C | LYS | A | 24 | 5.815 | 3.540 | 9.228 |
| ATOM | 697 | O | LYS | A | 24 | 5.553 | 4.692 | 9.552 |
| ATOM | 698 | CB | LYS | A | 24 | 5.602 | 2.039 | 11.182 |
| ATOM | 699 | CG | LYS | A | 24 | 6.163 | 0.963 | 12.058 |
| ATOM | 700 | CD | LYS | A | 24 | 5.068 | 0.387 | 12.947 |
| ATOM | 701 | CE | LYS | A | 24 | 5.648 | -0.511 | 14.031 |
| ATOM | 702 | NZ | LYS | A | 24 | 4.577 | -1.091 | 14.886 |
| ATOM | 703 | N | VAL | A | 25 | 5.455 | 3.037 | 8.057 |
| ATOM | 704 | CA | VAL | A | 25 | 4.713 | 3.849 | 7.116 |

(continued)

| | | | | | | | | |
|---|---|---|---|---|---|---|---|---|
| ATOM | 705 | C | VAL | A | 25 | 3.237 | 3.737 | 7.444 |
| ATOM | 706 | O | VAL | A | 25 | 2.657 | 2.659 | 7.383 |
| ATOM | 707 | CB | VAL | A | 25 | 4.969 | 3.396 | 5.681 |
| ATOM | 708 | CG1 | VAL | A | 25 | 4.250 | 4.317 | 4.708 |
| ATOM | 709 | CG2 | VAL | A | 25 | 6.462 | 3.393 | 5.422 |
| ATOM | 710 | N | VAL | A | 25 | 2.637 | 4.864 | 7.800 |
| ATOM | 711 | CA | VAL | A | 25 | 1.231 | 4.916 | 8.170 |
| ATOM | 712 | C | VAL | A | 25 | 0.418 | 5.778 | 7.213 |
| ATOM | 713 | O | VAL | A | 25 | 0.871 | 6.841 | 6.774 |
| ATOM | 714 | CB | VAL | A | 25 | 1.062 | 5.531 | 9.577 |
| ATOM | 715 | CG1 | VAL | A | 25 | -0.341 | 5.253 | 10.115 |
| ATOM | 716 | CG2 | VAL | A | 25 | 2.128 | 5.006 | 10.506 |
| ATOM | 717 | N | LEU | A | 26 | -0.779 | 5.306 | 6.887 |
| ATOM | 718 | CA | LEU | A | 26 | -1.706 | 6.048 | 6.040 |
| ATOM | 719 | C | LEU | A | 26 | -2.862 | 6.358 | 6.992 |
| ATOM | 720 | O | LEU | A | 26 | -3.679 | 5.497 | 7.297 |
| ATOM | 721 | CB | LEU | A | 26 | -2.201 | 5.189 | 4.876 |
| ATOM | 722 | CG | LEU | A | 26 | -3.204 | 5.898 | 3.952 |
| ATOM | 723 | CD1 | LEU | A | 26 | -2.519 | 7.077 | 3.262 |
| ATOM | 724 | CD2 | LEU | A | 26 | -3.755 | 4.925 | 2.929 |
| ATOM | 725 | N | LYS | A | 27 | -2.910 | 7.584 | 7.489 |
| ATOM | 726 | CA | LYS | A | 27 | -3.952 | 7.965 | 8.430 |
| ATOM | 727 | C | LYS | A | 27 | -4.831 | 9.082 | 7.881 |
| ATOM | 728 | O | LYS | A | 27 | -4.374 | 9.924 | 7.103 |
| ATOM | 729 | CB | LYS | A | 27 | -3.305 | 8.409 | 9.738 |
| ATOM | 730 | CG | LYS | A | 27 | -4.265 | 8.720 | 10.859 |
| ATOM | 731 | CD | LYS | A | 27 | -3.509 | 9.382 | 11.994 |
| ATOM | 732 | CE | LYS | A | 27 | -4.397 | 9.654 | 13.186 |
| ATOM | 733 | NZ | LYS | A | 27 | -4.839 | 8.377 | 13.816 |
| ATOM | 734 | N | ASN | A | 28 | -6.098 | 9.078 | 8.279 |
| ATOM | 735 | CA | ASN | A | 28 | -7.045 | 10.095 | 7.843 |
| ATOM | 736 | C | ASN | A | 28 | -7.147 | 11.103 | 8.979 |
| ATOM | 737 | O | ASN | A | 28 | -7.815 | 10.842 | 9.969 |
| ATOM | 738 | CB | ASN | A | 28 | -8.408 | 9.455 | 7.560 |
| ATOM | 739 | CG | ASN | A | 28 | -9.518 | 10.485 | 7.364 |
| ATOM | 740 | OD1 | ASN | A | 28 | -9.360 | 11.444 | 6.597 |
| ATOM | 741 | ND2 | ASN | A | 28 | -10.653 | 10.288 | 8.050 |
| ATOM | 742 | N | TYR | A | 29 | -6.473 | 12.241 | 8.837 |
| ATOM | 743 | CA | TYR | A | 29 | -6.470 | 13.278 | 9.869 |

(continued)

| | | | | | | | | |
|---|---|---|---|---|---|---|---|---|
| ATOM | 744 | C | TYR | A | 29 | -7.680 | 14.204 | 9.836 |
| ATOM | 745 | O | TYR | A | 29 | -7.928 | 14.870 | 8.835 |
| ATOM | 746 | CB | TYR | A | 29 | -5.197 | 14.130 | 9.768 |
| ATOM | 747 | CG | TYR | A | 29 | -3.913 | 13.420 | 10.155 |
| ATOM | 748 | CD1 | TYR | A | 29 | -3.378 | 12.394 | 9.363 |
| ATOM | 749 | CD2 | TYR | A | 29 | -3.220 | 13.792 | 11.306 |
| ATOM | 750 | CE1 | TYR | A | 29 | -2.181 | 11.765 | 9.713 |
| ATOM | 751 | CE2 | TYR | A | 29 | -2.032 | 13.171 | 11.665 |
| ATOM | 752 | CZ | TYR | A | 29 | -1.516 | 12.167 | 10.869 |
| ATOM | 753 | OH | TYR | A | 29 | -0.319 | 11.603 | 11.231 |
| ATOM | 754 | N | GLN | A | 30 | -8.408 | 14.254 | 10.950 |
| ATOM | 755 | CA | GLN | A | 30 | -9.606 | 15.088 | 11.089 |
| ATOM | 756 | C | GLN | A | 30 | -9.287 | 16.541 | 11.431 |
| ATOM | 757 | O | GLN | A | 30 | -8.213 | 16.847 | 11.925 |
| ATOM | 758 | CB | GLN | A | 30 | -10.500 | 14.541 | 12.202 |
| ATOM | 759 | CG | GLN | A | 30 | -10.807 | 13.074 | 12.104 |
| ATOM | 760 | CD | GLN | A | 30 | -11.744 | 12.749 | 10.969 |
| ATOM | 761 | OE1 | GLN | A | 30 | -11.900 | 11.582 | 10.602 |
| ATOM | 762 | NE2 | GLN | A | 30 | -12.385 | 13.773 | 10.405 |
| ATOM | 763 | N | ASP | A | 31 | -10.238 | 17.430 | 11.173 |
| ATOM | 764 | CA | ASP | A | 31 | -10.085 | 18.851 | 11.495 |
| ATOM | 765 | C | ASP | A | 31 | -8.867 | 19.494 | 10.842 |
| ATOM | 766 | O | ASP | A | 31 | -8.195 | 20.335 | 11.442 |
| ATOM | 767 | CB | ASP | A | 31 | -10.000 | 19.025 | 13.019 |
| ATOM | 768 | CG | ASP | A | 31 | -11.247 | 18.520 | 13.742 |
| ATOM | 769 | OD1 | ASP | A | 31 | -12.349 | 18.587 | 13.155 |
| ATOM | 770 | OD2 | ASP | A | 31 | -11.130 | 18.069 | 14.908 |
| ATOM | 771 | N | MET | A | 32 | -8.582 | 19.113 | 9.606 |
| ATOM | 772 | CA | MET | A | 32 | -7.426 | 19.662 | 8.918 |
| ATOM | 773 | C | MET | A | 32 | -7.780 | 20.808 | 7.987 |
| ATOM | 774 | O | MET | A | 32 | -7.021 | 21.768 | 7.855 |
| ATOM | 775 | CB | MET | A | 32 | -6.741 | 18.559 | 8.125 |
| ATOM | 776 | CG | MET | A | 32 | -6.037 | 17.540 | 8.980 |
| ATOM | 777 | SD | MET | A | 32 | -4.559 | 18.231 | 9.714 |
| ATOM | 778 | CE | MET | A | 32 | -3.406 | 18.041 | 8.318 |
| ATOM | 779 | N | VAL | A | 33 | -8.942 | 20.707 | 7.351 |
| ATOM | 780 | CA | VAL | A | 33 | -9.381 | 21.719 | 6.401 |
| ATOM | 781 | C | VAL | A | 33 | -10.579 | 22.536 | 6.858 |
| ATOM | 782 | O | VAL | A | 33 | -11.586 | 21.988 | 7.292 |

(continued)

| | | | | | | | | |
|---|---|---|---|---|---|---|---|---|
| ATOM | 783 | CB | VAL | A | 33 | -9.701 | 21.056 | 5.027 |
| ATOM | 784 | CG1 | VAL | A | 33 | -10.388 | 22.040 | 4.087 |
| ATOM | 785 | CG2 | VAL | A | 33 | -8.423 | 20.561 | 4.412 |
| ATOM | 786 | N | VAL | A | 34 | -10.465 | 23.855 | 6.739 |
| ATOM | 787 | CA | VAL | A | 34 | -11.553 | 24.745 | 7.113 |
| ATOM | 788 | C | VAL | A | 34 | -12.610 | 24.835 | 6.016 |
| ATOM | 789 | O | VAL | A | 34 | -12.373 | 25.403 | 4.950 |
| ATOM | 790 | CB | VAL | A | 34 | -11.057 | 26.176 | 7.407 |
| ATOM | 791 | CG1 | VAL | A | 34 | -12.240 | 27.097 | 7.601 |
| ATOM | 792 | CG2 | VAL | A | 34 | -10.222 | 26.186 | 8.655 |
| ATOM | 793 | N | GLU | A | 35 | -13.779 | 24.266 | 6.282 |
| ATOM | 794 | CA | GLU | A | 35 | -14.870 | 24.311 | 5.321 |
| ATOM | 795 | C | GLU | A | 35 | -15.702 | 25.565 | 5.572 |
| ATOM | 796 | O | GLU | A | 35 | -16.011 | 26.303 | 4.645 |
| ATOM | 797 | CB | GLU | A | 35 | -15.733 | 23.054 | 5.447 |
| ATOM | 798 | CG | GLU | A | 35 | -14.969 | 21.782 | 5.128 |
| ATOM | 799 | CD | GLU | A | 35 | -14.515 | 21.732 | 3.676 |
| ATOM | 800 | OE1 | GLU | A | 35 | -15.036 | 22.519 | 2.858 |
| ATOM | 801 | OE2 | GLU | A | 35 | -13.643 | 20.902 | 3.344 |
| ATOM | 802 | N | GLY | A | 36 | -16.053 | 25.811 | 6.829 |
| ATOM | 803 | CA | GLY | A | 36 | -16.834 | 26.990 | 7.152 |
| ATOM | 804 | C | GLY | A | 36 | -16.322 | 27.785 | 8.341 |
| ATOM | 805 | O | GLY | A | 36 | -15.671 | 27.249 | 9.237 |
| ATOM | 806 | N | CYS | A | 37 | -16.616 | 29.077 | 8.360 |
| ATOM | 807 | CA | CYS | A | 37 | -16.177 | 29.917 | 9.466 |
| ATOM | 808 | C | CYS | A | 37 | -17.347 | 30.502 | 10.249 |
| ATOM | 809 | O | CYS | A | 37 | -18.467 | 30.628 | 9.741 |
| ATOM | 810 | CB | CYS | A | 37 | -15.301 | 31.058 | 8.967 |
| ATOM | 811 | SG | CYS | A | 37 | -13.785 | 30.558 | 8.098 |
| ATOM | 812 | N | GLY | A | 38 | -17.079 | 30.867 | 11.494 |
| ATOM | 813 | CA | GLY | A | 38 | -18.129 | 31.430 | 12.309 |
| ATOM | 814 | C | GLY | A | 38 | -17.622 | 32.176 | 13.518 |
| ATOM | 815 | O | GLY | A | 38 | -16.425 | 32.235 | 13.791 |
| ATOM | 816 | N | CYS | A | 39 | -18.564 | 32.753 | 14.245 |
| ATOM | 817 | CA | CYS | A | 39 | -18.253 | 33.505 | 15.439 |
| ATOM | 818 | C | CYS | A | 39 | -18.543 | 32.670 | 16.665 |
| ATOM | 819 | O | CYS | A | 39 | -19.620 | 32.084 | 16.794 |
| ATOM | 820 | CB | CYS | A | 39 | -19.068 | 34.779 | 15.442 |
| ATOM | 821 | SG | CYS | A | 39 | -18.539 | 35.799 | 14.053 |

**Structure coordinates:** As used herein, unless indicated otherwise or contradictory in context, the "structure coordinates" refers to Cartesian coordinates derived from mathematical equations related to the patterns obtained on diffraction of a monochromatic beam of X-rays by the atoms (scattering centers) of a protein, protein complex or peptide in crystal form. The diffraction data are used to calculate an electron density map of the repeating unit of the crystal. The electron density maps are then used to establish the positions of the individual atoms of the molecule or molecular complex.

**STAMP:** STAMP (Structural Alignment of Multiple Proteins) is a tool for aligning protein sequences based on a three-dimensional structure. Its algorithm minimizes the C$\alpha$ distance between aligned residues of each molecule by applying globally optimal rigid-body rotations and translations. This program provides some information on the equivalence of the residues between the selected models.

**SCWRL:** This program predicts and optimizes the protein side-chain conformations. It is using the backbone of a support protein and a backbone-dependent rotamer library. The possible conformations are explored by minimizing the steric hindrance between the side-chains and between the side-chains and the backbone.

**GROMACS:** GROMACS is a molecular dynamics package. The "gmx rms" tool included in GROMACS compares two structures by computing the root mean square deviation (RMSD).

In one aspect, the present disclosure provides for non-mutagenic extracellular therapies having the ability to direct cell fate. It is thus possible to convert or recode a neoplastic cell by modifying its surrounding extracellular micro-environment, in-vitro, ex-vivo or in-vivo, so that the cell operates self-recovery or self-healing and a subject possessing such a neoplastic cell may be protected from a neoplastic disease.

In one example, a neoplastic cell may operate self-recovery or self-healing e.g. by inducing a quiescence state so that the neoplastic cell may remain inactive or dormant for seconds, minutes, hours, days, weeks, months or years, in particular, will never resume neoplasia; and/or by preventing, reducing or suppressing cell division and/or cell proliferation, preferably uncontrolled cell division and/or cell proliferation of said neoplastic cell; and/or by regulating or promoting anti-mitogen activity and/or tumour suppressor pathways and/or anti-oncogenic activity in said neoplastic cell; and/or by inducing cytostaticity and not cytotoxicity in the neoplastic cell; and/or by inducing differentiation; and/or by regulating and/or modulating the adhesion or interactions between the cell and its micro-environment (i.e. the surrounding ECM) so as activate, reactivate or restore cell adhesion checkpoints in said neoplastic cell.

In one aspect, the present disclosure provides a pharmaceutical association or combination having the ability to convert or recode, extracellularly, a neoplastic cell, in-vitro, ex-vivo or in-vivo, so that it may be used in the treatment, prevention and/or diagnostic of a neoplastic disease, said association comprising at least one growth factor receptor-binding compound which activates at least one growth factor receptor of a cell (in particular a neoplastic cell) and at least one adhesion protein inhibitor or antagonist which inhibits or antagonises at least one transmembrane cell adhesion protein of said cell.

In certain embodiments, said pharmaceutical association or combination reduces, down-regulates, inhibits or suppresses (i) the gene and/or protein expression of at least one cyclin-D in the cell (in particular a neoplastic cell) and/or (ii) the formation of at least one complex formed between said at least one cyclin-D and at least one of cyclin dependent-kinase (CDK) 4 or 6 in the cell (in particular a neoplastic cell).

## II. Growth factor receptor-binding compounds

In one aspect, the present disclosure provides for pharmaceutical associations or combinations comprising at least one growth factor receptor-binding compound as defined herein and at least one adhesion protein inhibitor or antagonist as defined herein, said associations or combinations having the ability to convert or recode a neoplastic cell into a non-neoplastic cell.

As used herein, the term "Growth factor receptor-binding compound" or "GFR-binding compound" refers to an exogenous or endogenous compound, molecule or substance (a) having an (binding) affinity for a growth factor receptor as defined herein and (b) comprising the ability to activate a growth factor receptor as defined herein.

There are many ways to test, measure and present the binding affinity of a given substance for a given receptor, but for the purpose of the present disclosure, and for the avoidance of any doubts, the (binding) affinity values of a given GFR-binding compound to a given GFR are provided using the method of fluorescence anisotropy. In this method, a GFR-binding compound is fluorescently labelled using technics well established in the art. Binding of the resulting labelled compound to a growth factor receptor results in a fluctuation of fluorescence anisotropy which is used to construct an affinity binding curve from which the GFR-binding compound binding affinity value is derived. Using this technique, binding affinity values are given in the form of dissociation constants Kd. In certain embodiments, GFR-binding compounds of the present disclosure have Kd values as measured by fluorescence anisotropy of more than 1 (one) picomolar (pM). In certain embodiments, GFR-binding compounds of the present disclosure have Kd values as measured by fluorescence anisotropy of more than 1 (one) nanomolar (nM). In certain embodiments, GFR-binding compounds of the present disclosure have Kd values as measured by fluorescence anisotropy of more than 10 (ten) nanomolar (nM). In certain

embodiments, GFR-binding compounds of the present disclosure have Kd values as measured by fluorescence anisotropy of more than 100 (one hundred) nanomolar (nM). In certain embodiments, GFR-binding compounds of the present disclosure have Kd values as measured by fluorescence anisotropy of more than 1 (one) micromolar (μM). In certain embodiments, GFR-binding compounds of the present disclosure have Kd values as measured by fluorescence anisotropy of more than 10 (ten) micromolar (μM). In certain embodiments, GFR-binding compounds of the present disclosure have Kd values as measured by fluorescence anisotropy of more than 100 (one hundred) micromolar (μM).

There are many ways to test and measure the ability of a given substance to activate a given receptor, but for the purpose of the present disclosure, and for the avoidance of any doubts, a given GFR-binding compound activates a growth factor receptor if it induces growth factor receptor phosphorylation as measured by the western blot method. There are many distinct phosphorylation sites on growth factor receptors and they may widely vary according to the type of growth factor receptor as reported in the published scientific article from Mark A. Lemmon and Joseph Schlessinger, "Cell Signaling by Receptor Tyrosine Kinases", Cell. 2010; 141(7), 1117-1134, which is hereby incorporated by reference in its entirety.

**Growth factor receptor:** As used herein, unless indicated otherwise or contradictory in context, the term "growth factor receptor" or "GFR" is a receptor which binds to growth factors which are naturally occurring substances capable of stimulating, for instance, cellular growth, proliferation, healing, and cellular differentiation. Suitable as growth factor receptors for implementing embodiments of the present invention include epidermal growth factor receptors (EGFR), fibroblast growth factor receptors (FGFR), vascular endothelial growth factor receptors (VEGFR), nerve growth factor receptors (NGFR), Insulin receptor family, Trk receptor family, Eph receptor family, AXL receptor family, LTK receptor family, TIE receptor family, ROR receptor family, DDR receptor family, RET receptor family, KLG receptor family, RYK receptor family, MuSK receptor family, hepatocyte growth factor receptors (HGFR), somatomedin or insulin-like growth factor receptors (SGFR), platelet-derived growth factor receptors (PDGFR), transforming growth factor beta (TGF-β) superfamily proteins such as AMH, ARTN, BMP10, BMP15, BMP2, BMP3, BMP4, BMP5, BMP6, BMP7, BMP8A, BMP8B, GDF1, GDF10, GDF11, GDF15, GDF2, GDF3, GDF3A, GDF5, GDF6, GDF7, GDF8, GDF9, GDNF, INHA, INHBA, INHBB, INHBC, INHBE, LEFTY1, LEFTY2, MSTN, NODAL, NRTN, PSPN, TGFB1, TGFB2 and TGFB3, and any combination thereof.

**Growth factor:** As used herein, unless indicated otherwise or contradictory in context, the term "growth factor" refers to any substance(s) having the ability to bind to a growth factor receptor and produce (a) biological effect(s) or reaction(s), such as promoting the growth of tissues, by activating such a growth factor receptor. Exemplary growth factors include, but are not limited to, platelet-derived growth factor (PDGF), platelet-derived angiogenesis factor (PDAF), vascular endotheial growth factor (VEGF), platelet-derived epidermal growth factor (PDEGF), transforming growth factor beta (TGF-β), transforming growth factor A (TGF-A), epidermal growth factor (EGF), fibroblast growth factor (FGF), acidic fibroblast growth factor (FGF-A), basic fibroblast growth factor (FGF-B), insulin-like growth factors 1 and 2 (IGF-I and IGF-2), keratinocyte growth factor (KGF), tumor necrosis factor (TNF), fibroblast growth factor (FGF) and interleukin-1 (IL-I), Keratinocyte Growth Factor-2 (KGF-2), and combinations thereof.

**Activation of growth factor receptors:** As used herein, unless indicated otherwise or contradictory in context, the term "activating" or "activation of", when used in relation to a growth factor receptor, refers to the phosphorylation of the tyrosine kinase domain of such a growth factor receptor.

In one aspect, the present disclosure provides a GFR-binding compound, as part of a pharmaceutical association, combination or composition as defined herein, as an active principle for use in methods and uses described herein.

In one particular example, the growth factor receptor involved in the interaction with said GFR-binding compound is an epidermal growth factor receptor. In one particular example, the growth factor receptor involved in the interaction with said GFR-binding compound is a fibroblast growth factor receptor. In one particular example, the growth factor receptor involved in the interaction with said GFR-binding compound is a vascular endothelial growth factor receptor. In one particular example, the growth factor receptor involved in the interaction with said GFR-binding compound is a nerve growth factor receptor. In one particular example, the growth factor receptor involved in the interaction with said GFR-binding compound is a hepatocyte growth factor receptor. In one particular example, the growth factor receptor involved in the interaction with said GFR-binding compound is a somatomedin or insulin-like growth factor receptor. In one particular example, the growth factor receptor involved in the interaction with said GFR-binding compound is a platelet-derived growth factor receptor. In one particular example, the growth factor receptor involved in the interaction with said GFR-binding compound is a protein from the transforming growth factor beta (TGF-β) superfamily.

In one particular example, the growth factor receptor(s) involved in the interaction with said GFR-binding compound is (are) preferably selected from epidermal growth factor receptors, fibroblast growth factor receptors, vascular endothelial growth factor receptors, nerve growth factor receptors, hepatocyte growth factor receptors, somatomedin or insulin-like growth factor receptors, platelet-derived growth factor receptors, and transforming growth factor beta (TGF-β) superfamily proteins.

In one particular example, the gene expression of cyclin-D, in a neoplastic cell, is reduced, downregulated, inhibited or suppressed during phase G1 of the cell cycle. In one particular example, the gene expression of cyclin-D is

reduced or suppressed for substantially at least the entire duration of phase G1 of a cell cycle. In one particular example, the gene expression of cyclin-D is reduced by at least 20%. In one particular example, the gene expression of cyclin-D is reduced by at least 30%. In one particular example, the gene expression of cyclin-D is reduced by at least 40%. In one particular example, the gene expression of cyclin-D is reduced by at least 50%. In one particular example, the gene expression of cyclin-D is reduced by at least 60%. In one particular example, the gene expression of cyclin-D is reduced by at least 70%. In one particular example, the gene expression of cyclin-D is reduced by at least 80%. At least 40% is particularly preferred. Reduction of cyclin D gene expression is assessed with respect to the wild-type gene expression of cyclin-D and is measured by Quantitative Real Time Polymerase Chain Reaction (Q-PCR or RT-PCR) by (i) extracting RNA from the treated cells, (ii) converting the extracted RNA into the corresponding cDNA, (iii) subjecting the obtained cDNA to a real-time PCR amplification, (iv) analysing the data obtained from the real-time PCR amplification and comparing them with the data obtained by the ΔΔCt method, and (v) comparing the obtained values to the wild-type value.

In more details, Quantitative Real Time Polymerase Chain Reaction is carried out by (i) extracting RNA from the treated cells using the RNeasy total RNA kit from Qiagen®, (ii) converting the extracted RNA into the corresponding cDNA using a reverse transcription reaction (Gibco Brl®) and random primers from Invitrogen®, (iii) subjecting the obtained cDNA to a real-time PCR amplification in the presence of SYBR green reagents from Bio-Rad® in a thermocycler (iCycler, Biorad®), (iv) analysing the data obtained from the real-time PCR amplification with the iCycler IQ™ software following the iCycler iQ™ Real-Time PCR Detection System's instruction manual (Catalog Number 170-8740) and using weighted mean as a digital filter, PCR Baseline Subtracted Curve Fit as the analysis mode, FAM/490 as a fluorophore, the automatic calculation of the baseline cycles and the threshold, and the default settings for all other parameters, and comparing them with the data obtained by the ΔΔCt method as reported and described in Livak, K. J. and T. D. Schmittgen. "Analysis of relative gene expression data using real-time quantitative PCR and the 2(-Delta Delta C(T)) Method", Methods (2001) 25(4): 402-408, hereby incorporated by reference in its entirety, and (v) comparing the obtained values to the wild-type value.

In one particular example, the gene expression of cyclin-D is maintained at such a reduced level (at least 20%, at least 30%, at least 40%, at least 50%, at least 60%, at least 70%, or at least 80%) during phase G1 of a cell cycle of the treated neoplastic cell. In one example, the gene expression of cyclin-D is maintained at such a reduced level during substantially the entire duration of the G1 phase. In one example, the gene expression of cyclin-D is maintained at such a reduced level during substantially the entire duration of the G1 and S phases. In one example, the gene expression of cyclin-D is maintained at such a reduced level during substantially the entire duration of the G1, S and G2 phases. In one example, the gene expression of cyclin-D is maintained at such a reduced level during substantially the entire duration of the G1, S, G2 and M phases i.e. during substantially the entire duration of a cell cycle of a treated neoplastic cell. Reduction of the gene expression level of cyclin D during substantially the entire duration of G1 is preferred.

As used herein, unless indicated otherwise or contradictory in context, the term "wild-type expression" of a protein or a gene, refers to the expression of a protein or a gene observed in normal, standard biological conditions i.e., in the present disclosure, without the presence of, or prior to the provision or administration to a neoplastic cell of a pharmaceutical association, combination or composition as defined herein. In-vitro, ex-vivo or in-vivo natural expression level of a protein or a gene in a neoplastic cell may thus be used as a comparative data (or control) to assess and quantify the effect of the presence or administration of a pharmaceutical association, combination or composition as defined herein on the expression level of such a protein or gene in that cell.

Suitable GFR-binding compounds for implementing certain embodiments of the invention include, without being limited to, linear (i.e. non-cyclic) GFR-binding compounds such as peptides, or variants or analogs thereof, or peptidomimetics, and cyclic GFR-binding compounds such as cyclic peptides, or variants or analogs thereof, or cyclic peptidomimetics.

### 11.1. Non-cyclic GFR-binding compounds

In one example, said (non-cyclic) GFR-binding compound has a molecular weight of less than 8,000 Daltons. In one particular example, said (non-cyclic) GFR-binding compound has a molecular weight of less than 6,000 Daltons. In one particular example, said (non-cyclic) GFR-binding compound has a molecular weight comprised between 2,000 and 8,000 Daltons. In one particular example, said (non-cyclic) GFR-binding compound has a molecular weight comprised between 2,000 and 6,000 Daltons. Between 2,000 and 6,000 Daltons is particularly preferred.

In one particular example, said GFR-binding compound is a (non-cyclic) peptide, a variant or analog thereof, as defined herein, with (exclusively consisting of, or constituted of) between 20 and 60 (in particular between 20 and 50, and more particularly, between 20 and 45) amino acids, having growth factor receptor-binding capability or capabilities.

In one particular example, said GFR-binding compound is a (non-cyclic) peptidomimetic as defined herein, having growth factor receptor-binding capability or capabilities, comprising (consecutively or non consecutively) between 20-60 amino acids, in particular between 20-50 or 20-45 amino acids, and more particularly, between 23-60, 23-50 or 23-45 amino acids; wherein said GFR-binding compound has a molecular weight comprised between 2,000 and 8,000

Daltons (in particular, between 2,000 and 6,000 Da).

In one particular example, said GFR-binding compound is a peptidomimetic as defined herein, having growth factor receptor-binding capability or capabilities, comprising (consecutively or non consecutively) between 20-60 amino acids, in particular between 20-50 or 20-45 amino acids, and more particularly, between 23-60, 23-50 or 23-45 amino acids; and containing at least one peptide portion or fragment with between 5-20 amino acids (in particular containing two peptide portions or fragments, each of which with between 5-20 amino acids); wherein said GFR-binding compound has a molecular weight comprised between 2,000 and 8,000 Daltons (in particular, between 2,000 and 6,000 Da).

In one particular example, said GFR-binding compound is a peptide, a variant or analog thereof, or a peptidomimetic as defined herein, with (comprising, or exclusively consisting of, or constituted of) between 20 and 60 (in particular between 20 and 50, and more particularly, between 20 and 45) amino acids, having growth factor receptor-binding capability or capabilities, comprising a peptide with four amino acids (PEP1) and a peptide with five amino acids (PEP2).

In one particular example, said GFR-binding compound is a peptide, a variant or analog thereof, or a peptidomimetic as defined herein, with (comprising, or exclusively consisting of, or constituted of) between 20 and 60 (in particular between 20 and 50, and more particularly, between 20 and 45) amino acids, comprising a peptide with eight amino acids (PEP12) and a peptide with five amino acids (PEP2).

In one particular example, said GFR-binding compound is a peptide, a variant or analog thereof, or a peptidomimetic as defined herein, with (comprising, or exclusively consisting of, or constituted of) between 20 and 60 (in particular between 20 and 50, and more particularly, between 20 and 45) amino acids, comprising a peptide with four amino acids (PEP1) and a peptide with five amino acids (PEP2); wherein said GFR-binding compound further comprises a peptide with three amino acids (PEP3) and a peptide with three amino acids (PEP4).

In one particular example, said GFR-binding compound is a peptide, a variant or analog thereof, or a peptidomimetic as defined herein, with (comprising, or exclusively consisting of, or constituted of) between 20 and 60 (in particular between 20 and 50, and more particularly, between 20 and 45) amino acids, comprising a peptide with eight amino acids (PEP12) and a peptide with five amino acids (PEP2); wherein said GFR-binding compound further comprises a peptide with three amino acids (PEP3) and a peptide with three amino acids (PEP4).

In one particular example, said GFR-binding compound is a peptide, a variant or analog thereof, or a peptidomimetic as defined herein, with (comprising, or exclusively consisting of, or constituted of) between 20 and 60 (in particular between 20 and 50, and more particularly, between 20 and 45) amino acids, comprising a peptide with four amino acids (PEP1) and a peptide with five amino acids (PEP2); wherein said GFR-binding compound further comprises a peptide with five amino acids (PEP5) and a peptide with between five and seven amino acids (PEP6).

In one particular example, said GFR-binding compound is a peptide, a variant or analog thereof, or a peptidomimetic as defined herein, with (comprising, or exclusively consisting of, or constituted of) between 20 and 60 (in particular between 20 and 50, and more particularly, between 20 and 45) amino acids, comprising a peptide with eight amino acids (PEP12) and a peptide with five amino acids (PEP2); wherein said GFR-binding compound further comprises a peptide with five amino acids (PEP5) and a peptide with between five and seven amino acids (PEP6).

In one particular example, said GFR-binding compound is a peptide, a variant or analog thereof, or a peptidomimetic as defined herein, with (comprising, or exclusively consisting of, or constituted of) between 20 and 60 (in particular between 20 and 50, and more particularly, between 20 and 45) amino acids, comprising a peptide with four amino acids (PEP1) and a peptide with five amino acids (PEP2); wherein said GFR-binding compound further comprises a peptide with between six and twelve amino acids (PEP9) and a peptide with between six and eleven amino acids (PEP10).

In one particular example, said GFR-binding compound is a peptide, a variant or analog thereof, or a peptidomimetic as defined herein, with (comprising, or exclusively consisting of, or constituted of) between 20 and 60 (in particular between 20 and 50, and more particularly, between 20 and 45) amino acids, comprising a peptide with eight amino acids (PEP12) and a peptide with five amino acids (PEP2); wherein said GFR-binding compound further comprises a peptide with between six and twelve amino acids (PEP9) and a peptide with between six and eleven amino acids (PEP10).

In one particular example, said GFR-binding compound is a peptide, a variant or analog thereof, or a peptidomimetic as defined herein, with (comprising, or exclusively consisting of, or constituted of) between 20 and 60 (in particular between 20 and 50, and more particularly, between 20 and 45) amino acids, comprising a peptide with four amino acids (PEP1) and a peptide with five amino acids (PEP2); wherein said GFR-binding compound further comprises a peptide with three amino acids (PEP3) and a peptide with between five and seven amino acids (PEP6).

In one particular example, said GFR-binding compound is a peptide, a variant or analog thereof, or a peptidomimetic as defined herein, with (comprising, or exclusively consisting of, or constituted of) between 20 and 60 (in particular between 20 and 50, and more particularly, between 20 and 45) amino acids, comprising a peptide with eight amino acids (PEP12) and a peptide with five amino acids (PEP2); wherein said GFR-binding compound further comprises a peptide with three amino acids (PEP3) and a peptide with between five and seven amino acids (PEP6).

In one particular example, said GFR-binding compound is a peptide, a variant or analog thereof, or a peptidomimetic as defined herein, with (comprising, or exclusively consisting of, or constituted of) between 20 and 60 (in particular between 20 and 50, and more particularly, between 20 and 45) amino acids, comprising a peptide with four amino acids

(PEP1) and a peptide with five amino acids (PEP2); wherein said GFR-binding compound further comprises a peptide with three amino acids (PEP3) and a peptide with between six and eleven amino acids (PEP10).

In one particular example, said GFR-binding compound is a peptide, a variant or analog thereof, or a peptidomimetic as defined herein, with (comprising, or exclusively consisting of, or constituted of) between 20 and 60 (in particular between 20 and 50, and more particularly, between 20 and 45) amino acids, comprising a peptide with eight amino acids (PEP12) and a peptide with five amino acids (PEP2); wherein said GFR-binding compound further comprises a peptide with three amino acids (PEP3) and a peptide with between six and eleven amino acids (PEP10).

In one particular example, said GFR-binding compound is a peptide, a variant or analog thereof, or a peptidomimetic as defined herein, with (comprising, or exclusively consisting of, or constituted of) between 20 and 60 (in particular between 20 and 50, and more particularly, between 20 and 45) amino acids, comprising a peptide with four amino acids (PEP1) and a peptide with five amino acids (PEP2); wherein said GFR-binding compound further comprises a peptide with five amino acids (PEP5) and a peptide with three amino acids (PEP4).

In one particular example, said GFR-binding compound is a peptide, a variant or analog thereof, or a peptidomimetic as defined herein, with (comprising, or exclusively consisting of, or constituted of) between 20 and 60 (in particular between 20 and 50, and more particularly, between 20 and 45) amino acids, comprising a peptide with four amino acids (PEP12) and a peptide with five amino acids (PEP2); wherein said GFR-binding compound further comprises a peptide with five amino acids (PEP5) and a peptide with three amino acids (PEP4).

In one particular example, said GFR-binding compound is a peptide, a variant or analog thereof, or a peptidomimetic as defined herein, with (comprising, or exclusively consisting of, or constituted of) between 20 and 60 (in particular between 20 and 50, and more particularly, between 20 and 45) amino acids, comprising a peptide with four amino acids (PEP1) and a peptide with five amino acids (PEP2); wherein said GFR-binding compound further comprises a peptide with between six and twelve amino acids (PEP9) and a peptide with three amino acids (PEP4).

In one particular example, said GFR-binding compound is a peptide, a variant or analog thereof, or a peptidomimetic as defined herein, with (comprising, or exclusively consisting of, or constituted of) between 20 and 60 (in particular between 20 and 50, and more particularly, between 20 and 45) amino acids, comprising a peptide with four amino acids (PEP12) and a peptide with five amino acids (PEP2); wherein said GFR-binding compound further comprises a peptide with between six and twelve amino acids (PEP9) and a peptide with three amino acids (PEP4).

In one particular example, said GFR-binding compound is a peptide, a variant or analog thereof, or a peptidomimetic as defined herein, with (comprising, or exclusively consisting of, or constituted of) between 20 and 60 (in particular between 20 and 50, and more particularly, between 20 and 45) amino acids, comprising a peptide with four amino acids (PEP1) and a peptide with five amino acids (PEP2); wherein said GFR-binding compound further comprises a peptide with five amino acids (PEP5) and a peptide with between six and eleven amino acids (PEP10).

In one particular example, said GFR-binding compound is a peptide, a variant or analog thereof, or a peptidomimetic as defined herein, with (comprising, or exclusively consisting of, or constituted of) between 20 and 60 (in particular between 20 and 50, and more particularly, between 20 and 45) amino acids, comprising a peptide with four amino acids (PEP12) and a peptide with five amino acids (PEP2); wherein said GFR-binding compound further comprises a peptide with five amino acids (PEP5) and a peptide with between six and eleven amino acids (PEP10).

In one particular example, said GFR-binding compound is a peptide, a variant or analog thereof, or a peptidomimetic as defined herein, with (comprising, or exclusively consisting of, or constituted of) between 20 and 60 (in particular between 20 and 50, and more particularly, between 20 and 45) amino acids, comprising a peptide with four amino acids (PEP1) and a peptide with five amino acids (PEP2); wherein said GFR-binding compound further comprises a peptide with between six and twelve amino acids (PEP9) and a peptide with between five and seven amino acids (PEP6).

In one particular example, said GFR-binding compound is a peptide, a variant or analog thereof, or a peptidomimetic as defined herein, with (comprising, or exclusively consisting of, or constituted of) between 20 and 60 (in particular between 20 and 50, and more particularly, between 20 and 45) amino acids, comprising a peptide with four amino acids (PEP12) and a peptide with five amino acids (PEP2); wherein said GFR-binding compound further comprises a peptide with between six and twelve amino acids (PEP9) and a peptide with between five and seven amino acids (PEP6).

In one particular example, said GFR-binding compound is a peptide, a variant or analog thereof, or a peptidomimetic as defined herein, with (comprising, or exclusively consisting of, or constituted of) between 20 and 60 (in particular between 20 and 50, and more particularly, between 20 and 45) amino acids, comprising a peptide with four amino acids (PEP1) and a peptide with five amino acids (PEP2); wherein said GFR-binding compound further comprises a peptide with three amino acids (PEP3), an amino acid or a peptide with between two and seven amino acids (PEP7), and a peptide with three amino acids (PEP4).

In one particular example, said GFR-binding compound is a peptide, a variant or analog thereof, or a peptidomimetic as defined herein, with (comprising, or exclusively consisting of, or constituted of) between 20 and 60 (in particular between 20 and 50, and more particularly, between 20 and 45) amino acids, comprising a peptide with four amino acids (PEP12) and a peptide with five amino acids (PEP2); wherein said GFR-binding compound further comprises a peptide with three amino acids (PEP3), an amino acid or a peptide with between two and seven amino acids (PEP7), and a

peptide with three amino acids (PEP4).

In one particular example, said GFR-binding compound is a peptide, a variant or analog thereof, or a peptidomimetic as defined herein, with (comprising, or exclusively consisting of, or constituted of) between 20 and 60 (in particular between 20 and 50, and more particularly, between 20 and 45) amino acids, comprising a peptide with four amino acids (PEP1) and a peptide with five amino acids (PEP2); wherein said GFR-binding compound further comprises a peptide with three amino acids (PEP3), an amino acid or a peptide with between two and seven amino acids (PEP7), and a peptide with between five and seven amino acids (PEP6).

In one particular example, said GFR-binding compound is a peptide, a variant or analog thereof, or a peptidomimetic as defined herein, with (comprising, or exclusively consisting of, or constituted of) between 20 and 60 (in particular between 20 and 50, and more particularly, between 20 and 45) amino acids, comprising a peptide with four amino acids (PEP12) and a peptide with five amino acids (PEP2); wherein said GFR-binding compound further comprises a peptide with three amino acids (PEP3), an amino acid or a peptide with between two and seven amino acids (PEP7), and a peptide with between five and seven amino acids (PEP6).

In one particular example, said GFR-binding compound is a peptide, a variant or analog thereof, or a peptidomimetic as defined herein, with (comprising, or exclusively consisting of, or constituted of) between 20 and 60 (in particular between 20 and 50, and more particularly, between 20 and 45) amino acids, comprising a peptide with four amino acids (PEP1) and a peptide with five amino acids (PEP2); wherein said GFR-binding compound further comprises a peptide with three amino acids (PEP3), an amino acid or a peptide with between two and seven amino acids (PEP7), and a peptide with between six and eleven amino acids (PEP10).

In one particular example, said GFR-binding compound is a peptide, a variant or analog thereof, or a peptidomimetic as defined herein, with (comprising, or exclusively consisting of, or constituted of) between 20 and 60 (in particular between 20 and 50, and more particularly, between 20 and 45) amino acids, comprising a peptide with four amino acids (PEP12) and a peptide with five amino acids (PEP2); wherein said GFR-binding compound further comprises a peptide with three amino acids (PEP3), an amino acid or a peptide with between two and seven amino acids (PEP7), and a peptide with between six and eleven amino acids (PEP10).

In one particular example, said GFR-binding compound is a peptide, a variant or analog thereof, or a peptidomimetic as defined herein, with (comprising, or exclusively consisting of, or constituted of) between 20 and 60 (in particular between 20 and 50, and more particularly, between 20 and 45) amino acids, comprising a peptide with four amino acids (PEP1) and a peptide with five amino acids (PEP2); wherein said GFR-binding compound further comprises a peptide with three amino acids (PEP3), an amino acid or a peptide with between two and seven amino acids (PEP7), a peptide with three amino acids (PEP4), and an amino acid or a peptide with between two and six amino acids (PEP8).

In one particular example, said GFR-binding compound is a peptide, a variant or analog thereof, or a peptidomimetic as defined herein, with (comprising, or exclusively consisting of, or constituted of) between 20 and 60 (in particular between 20 and 50, and more particularly, between 20 and 45) amino acids, comprising a peptide with four amino acids (PEP12) and a peptide with five amino acids (PEP2); wherein said GFR-binding compound further comprises a peptide with three amino acids (PEP3), an amino acid or a peptide with between two and seven amino acids (PEP7), a peptide with three amino acids (PEP4), and an amino acid or a peptide with between two and six amino acids (PEP8).

In one particular example, said GFR-binding compound is a peptide, a variant or analog thereof, or a peptidomimetic as defined herein, with (comprising, or exclusively consisting of, or constituted of) between 20 and 60 (in particular between 20 and 50, and more particularly, between 20 and 45) amino acids, comprising a peptide with four amino acids (PEP1) and a peptide with five amino acids (PEP2); wherein said GFR-binding compound further comprises a peptide with three amino acids (PEP3), an amino acid or a peptide with between two and seven amino acids (PEP7), a peptide with between five and seven amino acids (PEP6), and an amino acid or a peptide with between two and six amino acids (PEP8).

In one particular example, said GFR-binding compound is a peptide, a variant or analog thereof, or a peptidomimetic as defined herein, with (comprising, or exclusively consisting of, or constituted of) between 20 and 60 (in particular between 20 and 50, and more particularly, between 20 and 45) amino acids, comprising a peptide with four amino acids (PEP12) and a peptide with five amino acids (PEP2); wherein said GFR-binding compound further comprises a peptide with three amino acids (PEP3), an amino acid or a peptide with between two and seven amino acids (PEP7), a peptide with between five and seven amino acids (PEP6), and an amino acid or a peptide with between two and six amino acids (PEP8).

In one particular example, said GFR-binding compound is a peptide, a variant or analog thereof, or a peptidomimetic as defined herein, with (comprising, or exclusively consisting of, or constituted of) between 20 and 60 (in particular between 20 and 50, and more particularly, between 20 and 45) amino acids, comprising a peptide with four amino acids (PEP1) and a peptide with five amino acids (PEP2); wherein said GFR-binding compound further comprises a peptide with five amino acids (PEP5), an amino acid or a peptide with between two and seven amino acids (PEP7), and a peptide with three amino acids (PEP4).

In one particular example, said GFR-binding compound is a peptide, a variant or analog thereof, or a peptido-

mimetic as defined herein, with (comprising, or exclusively consisting of, or constituted of) between 20 and 60 (in particular between 20 and 50, and more particularly, between 20 and 45) amino acids, comprising a peptide with four amino acids (PEP12) and a peptide with five amino acids (PEP2); wherein said GFR-binding compound further comprises a peptide with five amino acids (PEP5), an amino acid or a peptide with between two and seven amino acids (PEP7), and a peptide with three amino acids (PEP4).

In one particular example, said GFR-binding compound is a peptide, a variant or analog thereof, or a peptidomimetic as defined herein, with (comprising, or exclusively consisting of, or constituted of) between 20 and 60 (in particular between 20 and 50, and more particularly, between 20 and 45) amino acids, comprising a peptide with four amino acids (PEP1) and a peptide with five amino acids (PEP2); wherein said GFR-binding compound further comprises a peptide with five amino acids (PEP5), an amino acid or a peptide with between two and seven amino acids (PEP7), and a peptide with between five and seven amino acids (PEP6).

In one particular example, said GFR-binding compound is a peptide, a variant or analog thereof, or a peptidomimetic as defined herein, with (comprising, or exclusively consisting of, or constituted of) between 20 and 60 (in particular between 20 and 50, and more particularly, between 20 and 45) amino acids, comprising a peptide with four amino acids (PEP12) and a peptide with five amino acids (PEP2); wherein said GFR-binding compound further comprises a peptide with five amino acids (PEP5), an amino acid or a peptide with between two and seven amino acids (PEP7), and a peptide with between five and seven amino acids (PEP6).

In one particular example, said GFR-binding compound is a peptide, a variant or analog thereof, or a peptidomimetic as defined herein, with (comprising, or exclusively consisting of, or constituted of) between 20 and 60 (in particular between 20 and 50, and more particularly, between 20 and 45) amino acids, comprising a peptide with four amino acids (PEP1) and a peptide with five amino acids (PEP2); wherein said GFR-binding compound further comprises a peptide with five amino acids (PEP5), an amino acid or a peptide with between two and seven amino acids (PEP7), and a peptide with between six and eleven amino acids (PEP10).

In one particular example, said GFR-binding compound is a peptide, a variant or analog thereof, or a peptidomimetic as defined herein, with (comprising, or exclusively consisting of, or constituted of) between 20 and 60 (in particular between 20 and 50, and more particularly, between 20 and 45) amino acids, comprising a peptide with four amino acids (PEP12) and a peptide with five amino acids (PEP2); wherein said GFR-binding compound further comprises a peptide with five amino acids (PEP5), an amino acid or a peptide with between two and seven amino acids (PEP7), and a peptide with between six and eleven amino acids (PEP10).

In one particular example, said GFR-binding compound is a peptide, a variant or analog thereof, or a peptidomimetic as defined herein, with (comprising, or exclusively consisting of, or constituted of) between 20 and 60 (in particular between 20 and 50, and more particularly, between 20 and 45) amino acids, comprising a peptide with four amino acids (PEP1) and a peptide with five amino acids (PEP2); wherein said GFR-binding compound further comprises a peptide with five amino acids (PEP5), an amino acid or a peptide with between two and seven amino acids (PEP7), a peptide with three amino acids (PEP4), and an amino acid or a peptide with between two and six amino acids (PEP8).

In one particular example, said GFR-binding compound is a peptide, a variant or analog thereof, or a peptidomimetic as defined herein, with (comprising, or exclusively consisting of, or constituted of) between 20 and 60 (in particular between 20 and 50, and more particularly, between 20 and 45) amino acids, comprising a peptide with four amino acids (PEP12) and a peptide with five amino acids (PEP2); wherein said GFR-binding compound further comprises a peptide with five amino acids (PEP5), an amino acid or a peptide with between two and seven amino acids (PEP7), a peptide with three amino acids (PEP4), and an amino acid or a peptide with between two and six amino acids (PEP8).

In one particular example, said GFR-binding compound is a peptide, a variant or analog thereof, or a peptidomimetic as defined herein, with (comprising, or exclusively consisting of, or constituted of) between 20 and 60 (in particular between 20 and 50, and more particularly, between 20 and 45) amino acids, comprising a peptide with four amino acids (PEP1) and a peptide with five amino acids (PEP2); wherein said GFR-binding compound further comprises a peptide with five amino acids (PEP5), an amino acid or a peptide with between two and seven amino acids (PEP7), a peptide with between five and seven amino acids (PEP6), and an amino acid or a peptide with between two and six amino acids (PEP8).

In one particular example, said GFR-binding compound is a peptide, a variant or analog thereof, or a peptidomimetic as defined herein, with (comprising, or exclusively consisting of, or constituted of) between 20 and 60 (in particular between 20 and 50, and more particularly, between 20 and 45) amino acids, comprising a peptide with four amino acids (PEP12) and a peptide with five amino acids (PEP2); wherein said GFR-binding compound further comprises a peptide with five amino acids (PEP5), an amino acid or a peptide with between two and seven amino acids (PEP7), a peptide with between five and seven amino acids (PEP6), and an amino acid or a peptide with between two and six amino acids (PEP8).

In one particular example, said GFR-binding compound is a peptide, a variant or analog thereof, or a peptidomimetic as defined herein, with (comprising, or exclusively consisting of, or constituted of) between 20 and 60 (in particular between 20 and 50, and more particularly, between 20 and 45) amino acids, comprising a peptide with four amino acids

(PEP1) and a peptide with five amino acids (PEP2); wherein said GFR-binding compound further comprises a peptide with between six and twelve amino acids (PEP9), a peptide with three amino acids (PEP4), and an amino acid or a peptide with between two and six amino acids (PEP8).

In one particular example, said GFR-binding compound is a peptide, a variant or analog thereof, or a peptidomimetic as defined herein, with (comprising, or exclusively consisting of, or constituted of) between 20 and 60 (in particular between 20 and 50, and more particularly, between 20 and 45) amino acids, comprising a peptide with four amino acids (PEP12) and a peptide with five amino acids (PEP2); wherein said GFR-binding compound further comprises a peptide with between six and twelve amino acids (PEP9), a peptide with three amino acids (PEP4), and an amino acid or a peptide with between two and six amino acids (PEP8).

In one particular example, said GFR-binding compound is a peptide, a variant or analog thereof, or a peptidomimetic as defined herein, with (comprising, or exclusively consisting of, or constituted of) between 20 and 60 (in particular between 20 and 50, and more particularly, between 20 and 45) amino acids, comprising a peptide with four amino acids (PEP1) and a peptide with five amino acids (PEP2); wherein said GFR-binding compound further comprises a peptide with between six and twelve amino acids (PEP9), a peptide with between five and seven amino acids (PEP6), and an amino acid or a peptide with between two and six amino acids (PEP8).

In one particular example, said GFR-binding compound is a peptide, a variant or analog thereof, or a peptidomimetic as defined herein, with (comprising, or exclusively consisting of, or constituted of) between 20 and 60 (in particular between 20 and 50, and more particularly, between 20 and 45) amino acids, comprising a peptide with four amino acids (PEP12) and a peptide with five amino acids (PEP2); wherein said GFR-binding compound further comprises a peptide with between six and twelve amino acids (PEP9), a peptide with between five and seven amino acids (PEP6), and an amino acid or a peptide with between two and six amino acids (PEP8).

In one particular example, said GFR-binding compound is a peptide, a variant or analog thereof, or a peptidomimetic as defined herein, with (comprising, or exclusively consisting of, or constituted of) between 20 and 60 (in particular between 20 and 50, and more particularly, between 20 and 45) amino acids, having the following general formula (Ia) (hereinafter may also be referred to as compound (Ia) or peptide (I)):

PEP(A)-LINKER-PEP(B) (Ia)

wherein one end of LINKER interacts covalently with one end of PEP(A); wherein another end of LINKER interacts covalently with one end of PEP(B); wherein PEP(A) comprises PEP1; wherein PEP(B) comprises PEP2; wherein LINKER is a linear or branched organic divalent radical, moiety or compound having a molecular weight (Mw) comprised between about 28 and about 2,000 Da, in particular, between about 300 and about 1000 Da.

In the present description, the molecular weight of LINKER refer to the calculated molecular weight prior to being connected to / reacted with any of the elements it is configured to connect to or react with e.g. PEP(A), PEP(B) or any other groups defined herein.

In one particular example, said GFR-binding compound is a peptide, a variant or analog thereof, or a peptidomimetic as defined herein, with (comprising, or exclusively consisting of, or constituted of) between 20 and 60 (in particular between 20 and 50, and more particularly, between 20 and 45) amino acids, having the following general formula (Ia) (hereinafter may also be referred to as compound (Ia) or peptide (Ia)):

PEP(A)-LINKER-PEP(B) (Ia)

wherein one end of LINKER interacts covalently with one end of PEP(A); wherein another end of LINKER interacts covalently with one end of PEP(B); wherein PEP(A) comprises PEP12; wherein PEP(B) comprises PEP2; wherein LINKER is a linear or branched organic divalent radical, moiety or compound having a molecular weight (Mw) comprised between about 28 and about 2,000 Da, in particular, between about 300 and about 1000 Da.

In one aspect, the present disclosure provides a GFR-binding compound of general formula (Ia), wherein PEP(A) comprises PEP1; wherein PEP(B) comprises PEP2; wherein PEP(A) further comprises PEP3; wherein PEP(B) further comprises PEP4.

In one aspect, the present disclosure provides a GFR-binding compound of general formula (Ia), wherein PEP(A) comprises PEP12; wherein PEP(B) comprises PEP2; wherein PEP(A) further comprises PEP3; wherein PEP(B) further comprises PEP4.

In one aspect, the present disclosure provides a GFR-binding compound of general formula (Ia), wherein PEP(A) comprises PEP1; wherein PEP(B) comprises PEP2; wherein PEP(A) further comprises PEP5; wherein PEP(B) further comprises PEP6.

In one aspect, the present disclosure provides a GFR-binding compound of general formula (Ia), wherein PEP(A) comprises PEP12; wherein PEP(B) comprises PEP2; wherein PEP(A) further comprises PEP5; wherein PEP(B) further comprises PEP6.

In one aspect, the present disclosure provides a GFR-binding compound of general formula (Ia), wherein PEP(A) comprises PEP1; wherein PEP(B) comprises PEP2; wherein PEP(A) further comprises PEP9; wherein PEP(B) further comprises PEP10.

In one aspect, the present disclosure provides a GFR-binding compound of general formula (Ia), wherein PEP(A) comprises PEP12; wherein PEP(B) comprises PEP2; wherein PEP(A) further comprises PEP9; wherein PEP(B) further comprises PEP10.

In one aspect, the present disclosure provides a GFR-binding compound of general formula (Ia), wherein PEP(A) comprises PEP1; wherein PEP(B) comprises PEP2; wherein PEP(A) further comprises PEP3; wherein PEP(B) further comprises PEP6.

In one aspect, the present disclosure provides a GFR-binding compound of general formula (Ia), wherein PEP(A) comprises PEP12; wherein PEP(B) comprises PEP2; wherein PEP(A) further comprises PEP3; wherein PEP(B) further comprises PEP6.

In one aspect, the present disclosure provides a GFR-binding compound of general formula (Ia), wherein PEP(A) comprises PEP1; wherein PEP(B) comprises PEP2; wherein PEP(A) further comprises PEP3; wherein PEP(B) further comprises PEP10.

In one aspect, the present disclosure provides a GFR-binding compound of general formula (Ia), wherein PEP(A) comprises PEP12; wherein PEP(B) comprises PEP2; wherein PEP(A) further comprises PEP3; wherein PEP(B) further comprises PEP10.

In one aspect, the present disclosure provides a GFR-binding compound of general formula (Ia), wherein PEP(A) comprises PEP1; wherein PEP(B) comprises PEP2; wherein PEP(A) further comprises PEP5; wherein PEP(B) further comprises PEP4.

In one aspect, the present disclosure provides a GFR-binding compound of general formula (Ia), wherein PEP(A) comprises PEP12; wherein PEP(B) comprises PEP2; wherein PEP(A) further comprises PEP5; wherein PEP(B) further comprises PEP4.

In one aspect, the present disclosure provides a GFR-binding compound of general formula (Ia), wherein PEP(A) comprises PEP1; wherein PEP(B) comprises PEP2; wherein PEP(A) further comprises PEP9; wherein PEP(B) further comprises PEP4.

In one aspect, the present disclosure provides a GFR-binding compound of general formula (Ia), wherein PEP(A) comprises PEP12; wherein PEP(B) comprises PEP2; wherein PEP(A) further comprises PEP9; wherein PEP(B) further comprises PEP4.

In one aspect, the present disclosure provides a GFR-binding compound of general formula (Ia), wherein PEP(A) comprises PEP1; wherein PEP(B) comprises PEP2; wherein PEP(A) further comprises PEP5; wherein PEP(B) further comprises PEP10.

In one aspect, the present disclosure provides a GFR-binding compound of general formula (Ia), wherein PEP(A) comprises PEP12; wherein PEP(B) comprises PEP2; wherein PEP(A) further comprises PEP5; wherein PEP(B) further comprises PEP10.

In one aspect, the present disclosure provides a GFR-binding compound of general formula (Ia), wherein PEP(A) comprises PEP1; wherein PEP(B) comprises PEP2; wherein PEP(A) further comprises PEP9; wherein PEP(B) further comprises PEP6.

In one aspect, the present disclosure provides a GFR-binding compound of general formula (Ia), wherein PEP(A) comprises PEP12; wherein PEP(B) comprises PEP2; wherein PEP(A) further comprises PEP9; wherein PEP(B) further comprises PEP6.

In one aspect, the present disclosure provides a GFR-binding compound of general formula (Ia), wherein PEP(A) comprises PEP1; wherein PEP(B) comprises PEP2; wherein PEP(A) further comprises PEP3 and PEP7; wherein PEP(B) further comprises PEP4.

In one aspect, the present disclosure provides a GFR-binding compound of general formula (Ia), wherein PEP(A) comprises PEP12; wherein PEP(B) comprises PEP2; wherein PEP(A) further comprises PEP3 and PEP7; wherein PEP(B) further comprises PEP4.

In one aspect, the present disclosure provides a GFR-binding compound of general formula (Ia), wherein PEP(A) comprises PEP1; wherein PEP(B) comprises PEP2; wherein PEP(A) further comprises PEP3 and PEP7; wherein PEP(B) further comprises PEP6.

In one aspect, the present disclosure provides a GFR-binding compound of general formula (Ia), wherein PEP(A) comprises PEP12; wherein PEP(B) comprises PEP2; wherein PEP(A) further comprises PEP3 and PEP7; wherein PEP(B) further comprises PEP6.

In one aspect, the present disclosure provides a GFR-binding compound of general formula (Ia), wherein PEP(A) comprises PEP1; wherein PEP(B) comprises PEP2; wherein PEP(A) further comprises PEP3 and PEP7; wherein PEP(B) further comprises PEP10.

In one aspect, the present disclosure provides a GFR-binding compound of general formula (Ia), wherein

PEP(A) comprises PEP12; wherein PEP(B) comprises PEP2; wherein PEP(A) further comprises PEP3 and PEP7; wherein PEP(B) further comprises PEP10.

In one aspect, the present disclosure provides a GFR-binding compound of general formula (Ia), wherein PEP(A) comprises PEP1; wherein PEP(B) comprises PEP2; wherein PEP(A) further comprises PEP7; wherein PEP(B) further comprises PEP4 and PEP8.

In one aspect, the present disclosure provides a GFR-binding compound of general formula (Ia), wherein PEP(A) comprises PEP12; wherein PEP(B) comprises PEP2; wherein PEP(A) further comprises PEP7; wherein PEP(B) further comprises PEP4 and PEP8.

In one aspect, the present disclosure provides a GFR-binding compound of general formula (Ia), wherein PEP(A) comprises PEP1; wherein PEP(B) comprises PEP2; wherein PEP(A) further comprises PEP7; wherein PEP(B) further comprises PEP6 and PEP8.

In one aspect, the present disclosure provides a GFR-binding compound of general formula (Ia), wherein PEP(A) comprises PEP12; wherein PEP(B) comprises PEP2; wherein PEP(A) further comprises PEP7; wherein PEP(B) further comprises PEP6 and PEP8.

In one aspect, the present disclosure provides a GFR-binding compound of general formula (Ia), wherein PEP(A) comprises PEP1; wherein PEP(B) comprises PEP2; wherein PEP(A) further comprises PEP5 and PEP7; wherein PEP(B) further comprises PEP4.

In one aspect, the present disclosure provides a GFR-binding compound of general formula (Ia), wherein PEP(A) comprises PEP12; wherein PEP(B) comprises PEP2; wherein PEP(A) further comprises PEP5 and PEP7; wherein PEP(B) further comprises PEP4.

In one aspect, the present disclosure provides a GFR-binding compound of general formula (Ia), wherein PEP(A) comprises PEP1; wherein PEP(B) comprises PEP2; wherein PEP(A) further comprises PEP5 and PEP7; wherein PEP(B) further comprises PEP6.

In one aspect, the present disclosure provides a GFR-binding compound of general formula (Ia), wherein PEP(A) comprises PEP12; wherein PEP(B) comprises PEP2; wherein PEP(A) further comprises PEP5 and PEP7; wherein PEP(B) further comprises PEP6.

In one aspect, the present disclosure provides a GFR-binding compound of general formula (Ia), wherein PEP(A) comprises PEP1; wherein PEP(B) comprises PEP2; wherein PEP(A) further comprises PEP5 and PEP7; wherein PEP(B) further comprises PEP10.

In one aspect, the present disclosure provides a GFR-binding compound of general formula (Ia), wherein PEP(A) comprises PEP12; wherein PEP(B) comprises PEP2; wherein PEP(A) further comprises PEP5 and PEP7; wherein PEP(B) further comprises PEP10.

In one aspect, the present disclosure provides a GFR-binding compound of general formula (Ia), wherein PEP(A) comprises PEP1; wherein PEP(B) comprises PEP2; wherein PEP(A) further comprises PEP5 and PEP7; wherein PEP(B) further comprises PEP4 and PEP8.

In one aspect, the present disclosure provides a GFR-binding compound of general formula (Ia), wherein PEP(A) comprises PEP12; wherein PEP(B) comprises PEP2; wherein PEP(A) further comprises PEP5 and PEP7; wherein PEP(B) further comprises PEP4 and PEP8.

In one aspect, the present disclosure provides a GFR-binding compound of general formula (Ia), wherein PEP(A) comprises PEP1; wherein PEP(B) comprises PEP2; wherein PEP(A) further comprises PEP5 and PEP7; wherein PEP(B) further comprises PEP6 and PEP8.

In one aspect, the present disclosure provides a GFR-binding compound of general formula (Ia), wherein PEP(A) comprises PEP12; wherein PEP(B) comprises PEP2; wherein PEP(A) further comprises PEP5 and PEP7; wherein PEP(B) further comprises PEP6 and PEP8.

In one aspect, the present disclosure provides a GFR-binding compound of general formula (Ia), wherein PEP(A) comprises PEP1; wherein PEP(B) comprises PEP2; wherein PEP(A) further comprises PEP9; wherein PEP(B) further comprises PEP4 and PEP8.

In one aspect, the present disclosure provides a GFR-binding compound of general formula (Ia), wherein PEP(A) comprises PEP12; wherein PEP(B) comprises PEP2; wherein PEP(A) further comprises PEP9; wherein PEP(B) further comprises PEP4 and PEP8.

In one aspect, the present disclosure provides a GFR-binding compound of general formula (Ia), wherein PEP(A) comprises PEP1; wherein PEP(B) comprises PEP2; wherein PEP(A) further comprises PEP9; wherein PEP(B) further comprises PEP6 and PEP8.

In one aspect, the present disclosure provides a GFR-binding compound of general formula (Ia), wherein PEP(A) comprises PEP12; wherein PEP(B) comprises PEP2; wherein PEP(A) further comprises PEP9; wherein PEP(B) further comprises PEP6 and PEP8.

In one particular example, said GFR-binding compound is a peptide, a variant or analog thereof, or a peptidomimetic as defined herein, with (comprising, or exclusively consisting of, or constituted of) between 20 and 60 (in particular

between 20 and 50, and more particularly, between 20 and 45) amino acids, having the following general formula (IIa) (hereinafter may also be referred to as compound (IIa) or peptide (IIa)):

PEP(C)-PEP12-LINKER-PEP2-PEP(D) (IIa)

wherein LINKER is a linear or branched organic divalent radical, moiety or compound having a molecular weight (Mw) comprised between about 28 and about 2,000 Da, in particular, between about 300 and about 1000 Da; wherein PEP12 is a peptide with 8 amino acids of formula PEP1-$AA^{17}$-PEP11 as defined herein; wherein PEP2 is a peptide with five amino acids as already defined herein; wherein one end of PEP(C) interacts covalently with PEP12 via one end of PEP1; wherein one end of PEP(D) interacts covalently with one end of PEP2; wherein one end of LINKER interacts covalently with one end of PEP12 via one end of PEP11; wherein another end of LINKER interacts covalently with another end of PEP2; wherein PEP(C) is a peptide with at least 5 amino acids, in particular a peptide with between 5 and 12 amino acids; wherein PEP(D) is a peptide with at least 5 amino acids, in particular a peptide with between 5 and 11 amino acids.

In one aspect, the present disclosure provides a GFR-binding compound of general formula (IIa), wherein PEP(C) comprises PEP3; wherein PEP(D) comprises PEP4.

In one aspect, the present disclosure provides a GFR-binding compound of general formula (IIa), wherein PEP(C) comprises PEP5; wherein PEP(D) comprises PEP6. In one particular example, PEP(C) is PEP5; and PEP(D) is PEP6.

In one aspect, the present disclosure provides a GFR-binding compound of general formula (IIa), wherein PEP(C) comprises PEP9; wherein PEP(D) comprises PEP10. In one particular example, PEP(C) is PEP9; and PEP(D) is PEP10.

In one aspect, the present disclosure provides a GFR-binding compound of general formula (IIa), wherein PEP(C) comprises PEP3; wherein PEP(D) comprises PEP6. In one particular example, PEP(C) comprises PEP3 and PEP(D) is PEP6.

In one aspect, the present disclosure provides a GFR-binding compound of general formula (IIa), wherein PEP(C) comprises PEP3; wherein PEP(D) comprises PEP10. In one particular example, PEP(C) comprises PEP3 and PEP(D) is PEP10.

In one aspect, the present disclosure provides a GFR-binding compound of general formula (IIa), wherein PEP(C) comprises PEP5; wherein PEP(D) comprises PEP4. In one particular example, PEP(C) is PEP5 and PEP(D) comprises PEP4.

In one aspect, the present disclosure provides a GFR-binding compound of general formula (IIa), wherein PEP(C) comprises PEP9; wherein PEP(D) comprises PEP4. In one particular example, PEP(C) is PEP9 and PEP(D) comprises PEP4.

In one aspect, the present disclosure provides a GFR-binding compound of general formula (IIa), wherein PEP(C) comprises PEP5; wherein PEP(D) comprises PEP10. In one particular example, PEP(C) is PEP5 and PEP(D) is PEP10.

In one aspect, the present disclosure provides a GFR-binding compound of general formula (IIa), wherein PEP(C) comprises PEP9; wherein PEP(D) comprises PEP6. In one particular example, PEP(C) is PEP9 and PEP(D) is PEP6.

In one aspect, the present disclosure provides a GFR-binding compound of general formula (IIa), wherein PEP(C) comprises PEP3 and PEP7; wherein PEP(D) comprises PEP4.

In one aspect, the present disclosure provides a GFR-binding compound of general formula (IIa), wherein PEP(C) comprises PEP3 and PEP7; wherein PEP(D) comprises PEP6. In one particular example, PEP(C) comprises PEP3 and PEP7, and PEP(D) is PEP6.

In one aspect, the present disclosure provides a GFR-binding compound of general formula (IIa), wherein PEP(C) comprises PEP3 and PEP7; wherein PEP(D) comprises PEP10. In one particular example, PEP(C) comprises PEP3 and PEP7, and PEP(D) is PEP10.

In one aspect, the present disclosure provides a GFR-binding compound of general formula (IIa), wherein PEP(C) comprises PEP7; wherein PEP(D) comprises PEP4 and PEP8.

In one aspect, the present disclosure provides a GFR-binding compound of general formula (IIa), wherein PEP(C) comprises PEP7; wherein PEP(D) comprises PEP6 and PEP8.

In one aspect, the present disclosure provides a GFR-binding compound of general formula (IIa), wherein PEP(C) comprises PEP5 and PEP7; wherein PEP(D) comprises PEP4.

In one aspect, the present disclosure provides a GFR-binding compound of general formula (IIa), wherein PEP(C) comprises PEP5 and PEP7; wherein PEP(D) comprises PEP6.

In one aspect, the present disclosure provides a GFR-binding compound of general formula (IIa), wherein PEP(C) comprises PEP5 and PEP7; wherein PEP(D) comprises PEP10.

In one aspect, the present disclosure provides a GFR-binding compound of general formula (IIa), wherein

PEP(C) comprises PEP5 and PEP7; wherein PEP(D) comprises PEP4 and PEP8.

In one aspect, the present disclosure provides a GFR-binding compound of general formula (IIa), wherein PEP(C) comprises PEP5 and PEP7; wherein PEP(D) comprises PEP6 and PEP8.

In one aspect, the present disclosure provides a GFR-binding compound of general formula (IIa), wherein PEP(C) comprises PEP9; wherein PEP(D) comprises PEP4 and PEP8.

In one aspect, the present disclosure provides a GFR-binding compound of general formula (IIa), wherein PEP(C) comprises PEP9; wherein PEP(D) comprises PEP6 and PEP8.

In one aspect, the present disclosure provides a GFR-binding compound of general formula (IIa), wherein PEP(C) is PEP5 or PEP9 and wherein PEP(D) is PEP6 or PEP10.

In one particular example, said GFR-binding compound is a peptide, a variant or analog thereof, or a peptidomimetic as defined herein, with (comprising, or exclusively consisting of, or constituted of) between 20 and 60 (in particular between 20 and 50, and more particularly, between 20 and 45) amino acids, having the following general formula (IIIa) (hereinafter may also be referred to as compound (IIIa) or peptide (IIIa)):

PEP7-PEP5-PEP12-LINKER-PEP2-PEP6-PEP8 (IIIa)

wherein LINKER is a linear or branched organic divalent radical, moiety or compound having a molecular weight (Mw) comprised between about 28 and about 2,000 Da, in particular, between about 300 and about 1000 Da; wherein PEP12 is a peptide with 8 amino acids of formula PEP1-$AA^{17}$-PEP11 as defined herein; wherein PEP2 is a peptide with five amino acids as already defined herein; wherein PEP5 is a peptide with five amino acids as already defined herein; wherein PEP6 is a peptide with between five and seven amino acids as already defined herein; wherein PEP7 an amino acid or a peptide with between two and seven amino acids as already defined herein; wherein PEP8 an amino acid or a peptide with between two and six amino acids as already defined herein; wherein one end of LINKER interacts covalently with one end of PEP12 via $AA^{20}$; wherein another end of LINKER interacts covalently with one end of PEP2 via $AA^{21}$; wherein one end of PEP5 interacts covalently with another end of PEP12 via $AA^{12}$; wherein another end of PEP5 interacts covalently with one end of PEP7 via $AA^{8}$; wherein one end of PEP6 interacts covalently with another end of PEP2 via $AA^{26}$; wherein another end of PEP6 interacts covalently with one end of PEP8 via $AA^{32}$.

In one particular example, said GFR-binding compound is a peptide, a variant or analog thereof, or a peptidomimetic as defined herein, with (comprising, or exclusively consisting of, or constituted of) between 20 and 60 (in particular between 20 and 50, and more particularly, between 20 and 45) amino acids, having the following general formula (IVa) (hereinafter may also be referred to as compound (IVa) or peptide (IVa)):

$AA^{1}$-$AA^{2}$-$AA^{3}$-$AA^{4}$-$AA^{5}$-$AA^{6}$-$AA^{7}$-$AA^{8}$-$AA^{9}$-$AA^{10}$-$AA^{11}$-$AA^{12}$-$AA^{13}$-$AA^{14}$-$AA^{15}$-$AA^{16}$-$AA^{17}$-$AA^{18}$-$AA^{19}$-$AA^{20}$-LINKER-$AA^{21}$-$AA^{22}$-$AA^{23}$-$AA^{24}$-$AA^{25}$-$AA^{26}$-$AA^{27}$-$AA^{28}$-$AA^{29}$-$AA^{30}$-$AA^{31}$-$AA^{32}$-$AA^{33}$-$AA^{34}$-$AA^{35}$-$AA^{36}$-$AA^{37}$-$AA^{38}$ (IVa)

wherein LINKER is a linear or branched organic divalent radical, moiety or compound having a molecular weight (Mw) comprised between about 28 and about 2,000 Da, in particular, between about 300 and about 1000 Da; wherein $AA^{1}$-$AA^{2}$-$AA^{3}$-$AA^{4}$-$AA^{5}$-$AA^{6}$-$AA^{7}$ is PEP7 as defined herein; wherein $AA^{13}$-$AA^{14}$-$AA^{15}$-$AA^{16}$-$AA^{17}$-$AA^{18}$-$AA^{19}$-$AA^{20}$ is PEP12 as defined herein; wherein $AA^{21}$-$AA^{22}$-$AA^{23}$-$AA^{24}$-$AA^{25}$ is PEP2 as already defined herein; wherein $AA^{8}$-$AA^{9}$-$AA^{10}$ is PEP3 as defined herein; wherein $AA^{30}$-$AA^{31}$-$AA^{32}$ is PEP4 as defined herein; wherein $AA^{33}$-$AA^{34}$-$AA^{35}$-$AA^{36}$-$AA^{37}$-$AA^{38}$ is PEP8 as defined herein; wherein $AA^{11}$, $AA^{12}$, $AA^{26}$, $AA^{27}$, $AA^{28}$, and $AA^{29}$ are as defined herein; wherein one end of LINKER interacts covalently with $AA^{20}$; wherein another end of LINKER interacts covalently with $AA^{21}$; wherein $AA^{1}$ and $AA^{21}$ may be both N-terminal amino acids or both C-terminal amino acids; wherein $AA^{38}$ and $AA^{20}$ may be both N-terminal amino acids or both C-terminal amino acids.

In certain embodiments, PEP1 is selected from the group consisting of SAIS, SSLS, NAIS, SATS, SPIS, EPIS, SPIN, KPLS, EPLP, EPLT, SNIT, RSVK and RPVQ.

In certain embodiments, PEP2 is selected from the group consisting of LKNYQ, LKVYP, LKKYR, LRKHR, LKYHY, KFKYE, YGKIP, YKQYE, DHHKD, EQLSN, IGEMS, LGEMS, KEVQV and KKATV.

In certain embodiments, the pair PEP1:PEP2 is selected from the group consisting of SAIS:LKNYQ, SSLS:LKVYP, NAIS:LKKYR, SATS:LRKHR, SPIS:LKYHY, EPIS:KFKYE, SPIN:YGKIP, SPIS:YKQYE, KPLS:DHHKD, EPLP:EQLSN, EPLT:EQLSN, SNIT:IGEMS, SNIT:LGEMS, RSVK:KEVQV and RPVQ:KKATV.

In certain embodiments, PEP3 is selected from the group consisting of VPT, VPE, APT, TPT, VPA, APV, VPQ, VSQ, SRV and TQV.

In certain embodiments, PEP4 is selected from the group consisting of VVE, TVE, VVR, VVK, VAE, AVS, VVD, VEE, VRS, VKS, QHN, EHS, EEH and EDH.

In certain embodiments, PEP5 is a peptide of general formula PEP3-$AA^{11}$-$AA^{12}$; wherein PEP3 is selected

from the group consisting of VPT, VPE, APT, TPT, VPA, APV, VPQ, VSQ, SRV and TQV; wherein $AA^{11}$ is selected from the group consisting of E, K, Q, R, A, D, G and H; and wherein $AA^{12}$ is selected from the group consisting of L, M, T, E, Q and H. In one particular example, PEP5 is selected from the group consisting of VPTEL, VPEKM, APTKL, APTQL, VPTKL, TPTKM, VPARL, VPTRL, APVKT, VPQAL, VSQDL, VPQDL, VPTEE, VPTGQ, SRVHH and TQVQL.

In certain embodiments, PEP6 is a peptide of general formula $AA^{26}$-$AA^{27}$-$AA^{28}$-$AA^{29}$-PEP4; wherein PEP4 is selected from the group consisting of VVE, TVE, VVR, VVK, VAE, AVS, VVD, VEE, VRS, VKS, QHN, EHS, EEH and EDH; wherein $AA^{26}$ is absent or selected from the group consisting of $AA^{III}$ amino acids, preferably is absent or is E; wherein $AA^{27}$ and $AA^{28}$ are independently selected from the group consisting of $AA^{III}$ and $AA^{V}$ amino acids; and wherein $AA^{29}$ is absent or selected from the group consisting of $AA^{II}$ amino acids, preferably is absent or is S. In one particular example, PEP6 is selected from the group consisting of DMVVE, NMTVE, EMVVE, NMVVR, NMVVK, EGMSVAE, GMAVS, GMVVD, MIVEE, MIVRS, MIVKS, MVVKS, FLQHN, LEEHS, RLEEH and TLEDH.

In certain embodiments, PEP7 is an amino acid or a peptide with between two and seven amino acids of general formula $AA^{1}$-$AA^{2}$-$AA^{3}$-$AA^{4}$-$AA^{5}$-$AA^{6}$-$AA^{7}$; wherein wherein $AA^{1}$, $AA^{2}$, $AA^{3}$, $AA^{4}$, and $AA^{5}$ are independently absent or $AA^{1}$ as defined herein; wherein $AA^{6}$ is absent or selected from the group consisting of S, T, C, E, Q, P and R; wherein $AA^{7}$ is absent or is selected from the group consisting of S, T, C, E, Q, P and R, and wherein at least one of $AA^{1}$, $AA^{2}$, $AA^{3}$, $AA^{4}$, $AA^{5}$, $AA^{6}$ or $AA^{7}$ is not absent. In one particular example, PEP7 is selected from the group consisting of KIPKAXX, GIPEPXX, SIPKAXX, HVTKPTX, YVPKPXX, TVPKPXX, AVPKAXX, KVGKAXX, KASKAXX, GSAGPXX, AAPASXX, STPPTXX, HVPKPXX, RVPSTXX, ASAAPXX, ASASPXX, NDEGLEX, SSVKXQP and RNVQXRP, wherein X is C or S throughout the present description.

In certain embodiments, PEP8 is an amino acid or a peptide with between two and six amino acids of general formula $AA^{33}$-$AA^{34}$-$AA^{35}$-$AA^{36}$-$AA^{37}$-$AA^{38}$; wherein $AA^{33}$ is absent or is selected from the group consisting of $AA^{I}$ amino acids at the exception of $AA^{VI}$ amino acids; $AA^{34}$ is absent or is selected from the group consisting of $AA^{III}$ and $AA^{IV}$ amino acids; wherein $AA^{35}$ is absent or is selected from the group consisting of $AA^{II}$ amino acids, preferably is S or C; wherein $AA^{36}$ is absent or is selected from the group consisting of $AA^{III}$ and $AA^{IV}$ amino acids; wherein $AA^{37}$ is absent or is selected from the group consisting of $AA^{II}$ amino acids, preferably is S or C; wherein $AA^{38}$ is absent or is selected from the group consisting of $AA^{I}$; and wherein at least one of $AA^{33}$, $AA^{34}$, $AA^{35}$, $AA^{36}$, $AA^{37}$ or $AA^{38}$ is not absent. In one particular example, PEP8 is selected from the group consisting of GXGXR, SXAXR, SXGXH, AXGXH, XGXR, EXGXR, RXGXS, AXGXR, SXGXR, XGXL, XKXS, KXEXR, QXEXR, LEXAXA and LAXKXE.

In certain embodiments, PEP9 is a peptide of general formula PEP7-PEP5; wherein PEP5 is a peptide of formula PEP3-$AA^{11}$-$AA^{12}$; wherein PEP3 is selected from the group consisting of VPT, VPE, APT, TPT, VPA, APV, VPQ, VSQ, SRV and TQV; wherein $AA^{11}$ is selected from the group consisting of E, K, Q, R, A, D, G and H; and wherein $AA^{12}$ is selected from the group consisting of L, M, T, E, Q and H; wherein PEP7 is an amino acid or a peptide with between two and seven amino acids of general formula $AA^{1}$-$AA^{2}$-$AA^{3}$-$AA^{4}$-$AA^{5}$-$AA^{6}$-$AA^{7}$; wherein $AA^{1}$, $AA^{2}$, $AA^{3}$, $AA^{4}$, and $AA^{5}$ are independently absent or $AA^{1}$ as defined herein; wherein $AA^{6}$ is absent or selected from the group consisting of S, T, C, E, Q, P and R; wherein $AA^{7}$ is absent or is selected from the group consisting of S, T, C, E, Q, P and R. In one particular example, PEP9 is selected from the group consisting of KIPKAXXVPTEL, GIPEPXXVPEKM, SIPKAXXVPTEL, HVTKPTXAPTKL, YVPKPXXAPTKL, TVPKPXXAPTQL, AVPKAXXAPTKL, KVGKAXXVPTKL, KASKAXXVPTKL, GSAGPXXTPTKM, AAPASXXVPARL, STPPTXXVPTRL, HVPKPXXAPTKL, RVPSTXXAPVKT, ASAAPXXVPQAL, ASASPXXVSQDL, ASASPXXVPQDL, NDEGLEXVPTEE, NDEGLEXVPTGQ, SSVKXQPSRVHH and RNVQXRPTQVQL, wherein X is C or S throughout the present description.

In certain embodiments, PEP10 is a peptide of general formula PEP6-PEP8; wherein PEP6 is a peptide of formula $AA^{26}$-$AA^{27}$-$AA^{28}$-$AA^{29}$-PEP4; wherein PEP4 is selected from the group consisting of VVE, TVE, VVR, VVK, VAE, AVS, VVD, VEE, VRS, VKS, QHN, EHS, EEH and EDH; wherein $AA^{26}$ is absent or selected from the group consisting of $AA^{III}$ amino acids, preferably is absent or is E; wherein $AA^{27}$ and $AA^{28}$ are independently selected from the group consisting of $AA^{III}$ and $AA^{V}$ amino acids; and wherein $AA^{29}$ is absent or selected from the group consisting of $AA^{II}$ amino acids, preferably is absent or is S; wherein PEP8 is an amino acid or a peptide with between two and six amino acids of general formula $AA^{33}$-$AA^{34}$-$AA^{35}$-$AA^{36}$-$AA^{37}$-$AA^{38}$; $AA^{33}$ is absent or is selected from the group consisting of $AA^{I}$ amino acids at the exception of $AA^{VI}$ amino acids; $AA^{34}$ is absent or is selected from the group consisting of $AA^{III}$ and $AA^{IV}$ amino acids; wherein $AA^{35}$ is absent or is selected from the group consisting of $AA^{II}$ amino acids, preferably is S or C; wherein $AA^{36}$ is absent or is selected from the group consisting of $AA^{III}$ and $AA^{IV}$ amino acids; wherein $AA^{37}$ is absent or is selected from the group consisting of $AA^{II}$ amino acids, preferably is S or C; wherein $AA^{38}$ is absent or is selected from the group consisting of $AA^{I}$. In one particular example, PEP10 is selected from the group consisting of DMVVEGXGXR, NMTVESXAXR, EMVVEGXGXR, NMVVRSXGXH, NMVVRAXGXH, NMVVKAXGXH, EGMSVAEXGXR, GMAVSEXGXR, GMVVDRXGXS, DMVVEAXGXR, DMVVESXGXR, MIVEEXGXL, MIVRSXKXS, MIVKSXKXS, MVVKSXKXS, FLQHNKXEXR, LEEHSQXEXR, RLEEHLEXAXA and TLEDHLAXKXE.

In certain embodiments, PEP12 is a peptide of general formula PEP1-$AA^{17}$-PEP11; wherein $AA^{17}$ is selected from the group consisting of G, A, V, L, I, P, F, M, W, T and S (in particular is selected from the group consisting of M, I, L, V and T); wherein PEP1 is selected from the group consisting of SAIS, SSLS, NAIS, SATS, SPIS, EPIS, SPIN,

KPLS, EPLP, EPLT, SNIT, RSVK and RPVQ.

In certain embodiments, the pair PEP12:PEP2 is selected from the group consisting of SAIS-$AA^{17}$-LYL:LKNYQ, SSLS-$AA^{17}$-LFF:LKVYP, NAIS-$AA^{17}$-LYF:LKKYR, SATS-$AA^{17}$-LYY:LRKHR, SPIS-$AA^{17}$-LYK:LKYHY, EPIS-$AA^{17}$-LYL:KFKYE, SPIN-$AA^{17}$-LYF:YGKIP, SPIS-$AA^{17}$-LYI:YKQYE, SPIS-$AA^{17}$-LFI:YKQYE, KPLS-$AA^{17}$-LYV:DHHKD, EPLP-$AA^{17}$-VYY:EQLSN, EPLT-$AA^{17}$-LYY:EQLSN, SNIT-$AA^{17}$-QIM:IGEMS, SNIT-$AA^{17}$-QIM:LGEMS, RSVK-$AA^{17}$-AKV:KEVQV and RPVQ-$AA^{17}$-RKI:KKATV ; wherein $AA^{17}$ is selected from the group consisting of G, A, V, L, I, P, F, M, W, T and S (in particular is selected from the group consisting of M, I, L, V and T).

In certain embodiments, PEP11 is a peptide with 3 amino acids of general formula $AA^{18}$-$AA^{19}$-$AA^{20}$; wherein $AA^{18}$ is selected from the group consisting of L, V, Q, A and R; wherein $AA^{19}$ is selected from the group consisting of F, W, H, Y, I and K; wherein $AA^{20}$ is selected from the group consisting of L, F, Y, K, I, V and M. In one particular example, PEP11 is selected from the group consisting of LYL, LFF, LYF, LYY, LYK, LYI, LFI, LYV, VYY, QIM, AKV and RKI.

In certain embodiments, PEP3 is selected from the group consisting of VPT, VPE, APT, TPT, VPA, APV, VPQ, VSQ, SRV and TQV; PEP4 is selected from the group consisting of VVE, TVE, VVR, VVK, VAE, AVS, VVD, VEE, VRS, VKS, QHN, EHS, EEH and EDH; and wherein the pair PEP3:PEP4 is selected from the group consisting of VPT:VVE, VPE:TVE, APT:VVR, APT:VVK, VPT:VAE, VPT:AVS, TPT:VVD, VPA:VVE, APV:VEE, VPQ:VRS, VSQ:VKS, VPQ:VKS, VPT:QHN, VPT:EHS, SRV:EEH and TQV:EDH.

In certain embodiments, PEP5 is selected from the group consisting of VPT-$AA^{11}$-$AA^{12}$, VPE-$AA^{11}$-$AA^{12}$-$AA^{11}$-$AA^{12}$, APT-$AA^{11}$-$AA^{12}$, TPT-$AA^{11}$-$AA^{12}$, VPA-$AA^{11}$-$AA^{12}$, VPT-$AA^{11}$-$AA^{12}$, APV-$AA^{11}$-$AA^{12}$, VPQ-$AA^{11}$-$AA^{12}$, VSQ-$AA^{11}$-$AA^{12}$, VPQ-$AA^{11}$-$AA^{12}$, SRV-$AA^{11}$-$AA^{12}$ and TQV-$AA^{11}$-$AA^{12}$; wherein $AA^{11}$ is selected from the group consisting of E, K, Q, R, A, D, G and H; and wherein $AA^{12}$ is selected from the group consisting of L, M, T, E, Q and H; PEP6 is selected from the group consisting of $AA^{26}$-$AA^{27}$-$AA^{28}$-$AA^{29}$-VVE, $AA^{26}$-$AA^{27}$-$AA^{28}$-$AA^{29}$-TVE, $AA^{26}$-$AA^{27}$-$AA^{28}$-$AA^{29}$-VVR, $AA^{26}$-$AA^{27}$-$AA^{28}$-$AA^{29}$-VVK, $AA^{26}$-$AA^{27}$-$AA^{28}$-$AA^{29}$-VAE, $AA^{26}$-$AA^{27}$-$AA^{28}$-$AA^{29}$-AVS, $AA^{26}$-$AA^{27}$-$AA^{28}$-$AA^{29}$-VVD, $AA^{26}$-$AA^{27}$-$AA^{28}$-$AA^{29}$-VEE, $AA^{26}$-$AA^{27}$-$AA^{28}$-$AA^{29}$-VRS, $AA^{26}$-$AA^{27}$-$AA^{28}$-$AA^{29}$-VKS, $AA^{26}$-$AA^{27}$-$AA^{28}$-$AA^{29}$-QHN, $AA^{26}$-$AA^{27}$-$AA^{28}$-$AA^{29}$-EHS, $AA^{26}$-$AA^{27}$-$AA^{28}$-$AA^{29}$-EEH and $AA^{26}$-$AA^{27}$-$AA^{28}$-$AA^{29}$-EDH; wherein $AA^{26}$ is absent or selected from the group consisting of $AA^{III}$ amino acids, preferably is absent or is E; wherein $AA^{27}$ and $AA^{28}$ are independently selected from the group consisting of $AA^{III}$ and $AA^{V}$ amino acids; and wherein $AA^{29}$ is absent or selected from the group consisting of $AA^{II}$ amino acids, preferably is absent or is S; wherein the pair PEP5:PEP6 is selected from the group consisting of VPT-$AA^{11}$-$AA^{12}$:$AA^{26}$-$AA^{27}$-$AA^{28}$-$AA^{29}$-VVE, VPE-$AA^{11}$-$AA^{12}$:$AA^{26}$-$AA^{27}$-$AA^{28}$-$AA^{29}$-TVE, APT-$AA^{11}$-$AA^{12}$:$AA^{26}$-$AA^{27}$-$AA^{28}$-$AA^{29}$-VVR, APT-$AA^{11}$-$AA^{12}$:$AA^{26}$-$AA^{27}$-$AA^{28}$-$AA^{29}$-VVK, VPT-$AA^{11}$-$AA^{12}$:$AA^{26}$-$AA^{27}$-$AA^{28}$-$AA^{29}$-VAE, VPT-$AA^{11}$-$AA^{12}$:$AA^{26}$-$AA^{27}$-$AA^{28}$-$AA^{29}$-AVS, TPT-$AA^{11}$-$AA^{12}$:$AA^{26}$-$AA^{27}$-$AA^{28}$-$AA^{29}$-VVD, VPA-$AA^{11}$-$AA^{12}$ :$AA^{26}$-$AA^{27}$-$AA^{28}$-$AA^{29}$-VVE, APV-$AA^{11}$-$AA^{12}$:$AA^{26}$-$AA^{27}$-$AA^{28}$-$AA^{29}$-VEE, VPQ-$AA^{11}$-$AA^{12}$:$AA^{26}$-$AA^{27}$-$AA^{28}$-$AA^{29}$-VRS, VSQ-$AA^{11}$-$AA^{12}$:$AA^{26}$-$AA^{27}$-$AA^{28}$-$AA^{29}$-VKS, VPQ-$AA^{11}$-$AA^{12}$:$AA^{26}$-$AA^{27}$-$AA^{28}$-$AA^{29}$-VKS, VPT-$AA^{11}$-$AA^{12}$:$AA^{26}$-$AA^{27}$-$AA^{28}$-$AA^{29}$-QHN, VPT-$AA^{11}$-$AA^{12}$:$AA^{26}$-$AA^{27}$-$AA^{28}$-$AA^{29}$-EHS, SRV-$AA^{11}$-$AA^{12}$:$AA^{26}$-$AA^{27}$-$AA^{28}$-$AA^{29}$-EEH and TQV-$AA^{11}$-$AA^{12}$:$AA2^{6}$-$AA^{27}$-$AA^{28}$-$AA^{29}$-EDH.

In certain embodiments, PEP5 is selected from the group consisting of VPTEL, VPEKM, APTKL, APTQL, VPTKL, TPTKM, VPARL, VPTRL, APVKT, VPQAL, VSQDL, VPQDL, VPTEE, VPTGQ, SRVHH and TQVQL; PEP6 is selected from the group consisting of DMVVE, NMTVE, EMVVE, NMVVR, NMVVK, EGMSVAE, GMAVS, GMVVD, MIVEE, MIVRS, MIVKS, MVVKS, FLQHN, LEEHS, RLEEH and TLEDH; and wherein the pair PEP5:PEP6 is selected from the group consisting of VPTEL:DMVVE, VPEKM:NMTVE, VPTEL:EMVVE, APTKL:NMVVR, APTQL:NMVVR, APTKL:NMVVK, VPTKL:EGMSVAE, VPTKL:GMAVS, TPTKM:GMVVD, VPARL:DMVVE, VPTRL:DMVVE, APVKT:MIVEE, VPQAL:MIVRS, VSQDL:MIVKS, VPQDL:MVVKS, VPTEE:FLQHN, VPTGQ:LEEHS, SRVHH:RLEEH and TQVQL:TLEDH.

In certain embodiments, PEP7 is selected from the group consisting of KIPKAXX, GIPEPXX, SIPKAXX, HVTKPTX, YVPKPXX, TVPKPXX, AVPKAXX, KVGKAXX, KASKAXX, GSAGPXX, AAPASXX, STPPTXX, HVPKPXX, RVPSTXX, ASAAPXX, ASASPXX, NDEGLEX, SSVKXQP and RNVQXRP; wherein PEP8 is selected from the group consisting of GXGXR, SXAXR, SXGXH, AXGXH, XGXR, EXGXR, RXGXS, AXGXR, SXGXR, XGXL, XKXS, KXEXR, QXEXR, LEXAXA and LAXKXE; and wherein the pair PEP7:PEP8 is selected from the group consisting of KIPKAXX:GXGXR, GIPEPXX:SXAXR, SIPKAXX:GXGXR, HVTKPTX:SXGXH, YVPKPXX:SXGXH, TVPKPXX:AXGXH, AVPKAXX:AXGXH, KVGKAXX:XGXR, KASKAXX:EXGXR, GSAGPXX:RXGXS, AAPASXX:AXGXR, STPPTXX:SXGXR, HVPKPXX:SXGXH, RVPSTXX:XGXL, ASAAPXX:XKXS, ASASPXX:XKXS, NDEGLEX:KXEXR, NDEGLEX:QXEXR, SSVKXQP:LEXAXA and RNVQXRP:LAXKXE.

In certain embodiments, PEP9 is selected from the group consisting of KIPKAXXVPT-$AA^{11}$-$AA^{12}$, GIPEPXXVPE-$AA^{11}$-$AA^{12}$, SIPKAXXVPT-$AA^{11}$-$AA^{12}$, HVTKPTXAPT-$AA^{11}$-$AA^{12}$, YVPKPXXAPT-$AA^{11}$-$AA^{12}$, TVPKPXXAPT-$AA^{11}$-$AA^{12}$, AVPKAXXAPT-$AA^{11}$-$AA^{12}$, KVGKAXXVPT-$AA^{11}$-$AA^{12}$, KASKAXXVPT-$AA^{11}$-$AA^{12}$, GSAGPXXTPT-$AA^{11}$-$AA^{12}$, AAPASXXVPA-$AA^{11}$-$AA^{12}$, STPPTXXVPT-$AA^{11}$-$AA^{12}$, HVPKPXXAPT-$AA^{11}$-$AA^{12}$, RVPSTXXAPV-$AA^{11}$-$AA^{12}$, ASAAPXXVPQ-$AA^{11}$-$AA^{12}$, ASASPXXVSQ-$AA^{11}$-$AA^{12}$, ASASPXXVPQ-$AA^{11}$-$AA^{12}$; NDEGLEXVPT-

$AA^{11}$-$AA^{12}$, NDEGLEXVPT-$AA^{11}$-$AA^{12}$, SSVKXQPSRV-$AA^{11}$-$AA^{12}$ and RNVQXRPTQV-$AA^{11}$-$AA^{12}$; wherein $AA^{11}$ is selected from the group consisting of E, K, Q, R, A, D, G and H; and wherein $AA^{12}$ is selected from the group consisting of L, M, T, E, Q and H; PEP10 is selected from the group consisting of $AA^{26}$-$AA^{27}$-$AA^{28}$-$AA^{29}$-VVEGXGXR, $AA^{26}$-$AA^{27}$-$AA^{28}$-$AA^{29}$-TVESXAXR, $AA^{26}$-$AA^{27}$-$AA^{28}$-$AA^{29}$-VVEGXGXR, $AA^{26}$-$AA^{27}$-$AA^{28}$-$AA^{29}$-VVRSXGXH, $AA^{26}$-$AA^{27}$-$AA^{28}$-$AA^{29}$-VVRAXGXH, $AA^{26}$-$AA^{27}$-$AA^{28}$-$AA^{29}$-VVKAXGXH, $AA^{26}$-$AA^{27}$-$AA^{28}$-$AA^{29}$-VAEXGXR, $AA^{26}$-$AA^{27}$-$AA^{28}$-$AA^{29}$-AVSEXGXR, $AA^{26}$-$AA^{27}$-$AA^{28}$-$AA^{29}$-VVDRXGXS, $AA^{26}$-$AA^{27}$-$AA^{28}$-$AA^{29}$-VVEAXGXR, $AA^{26}$-$AA^{27}$-$AA^{28}$-$AA^{29}$-VVESXGXR, $AA^{26}$-$AA^{27}$-$AA^{28}$-$AA^{29}$-VEEXGXL, $AA^{26}$-$AA^{27}$-$AA^{28}$-$AA^{29}$-VRSXKXS, $AA^{26}$-$AA^{27}$-$AA^{28}$-$AA^{29}$-VKSXKXS, $AA^{26}$-$AA^{27}$-$AA^{28}$-$AA^{29}$-VKSXKXS, $AA^{26}$-$AA^{27}$-$AA^{28}$-$AA^{29}$-QHNKXEXR, $AA^{26}$-$AA^{27}$-$AA^{28}$-$AA^{29}$-EHSQXEXR, $AA^{26}$-$AA^{27}$-$AA^{28}$-$AA^{29}$-EEHLEXAXA and $AA^{26}$-$AA^{27}$-$AA^{28}$-$AA^{29}$-EDHLAXKXE; wherein $AA^{26}$ is absent or selected from the group consisting of $AA^{III}$ amino acids, preferably is absent or is E; wherein $AA^{27}$ and $AA^{28}$ are independently selected from the group consisting of $AA^{III}$ and $AA^{V}$ amino acids; and wherein $AA^{29}$ is absent or selected from the group consisting of $AA^{II}$ amino acids, preferably is absent or is S; and wherein the pair PEP9:PEP10 is selected from the group consisting of KIPKAXXVPT-$AA^{11}$-$AA^{12}$:$AA^{26}$-$AA^{27}$-$AA^{28}$-$AA^{29}$-VVEGXGXR, GIPEPXXVPE-$AA^{11}$-$AA^{12}$:$AA^{26}$-$AA^{27}$-$AA^{28}$-$AA^{29}$-TVESXAXR, SIPKAXXVPT-$AA^{11}$-$AA^{12}$:$AA^{26}$-$AA^{27}$-$AA^{28}$-$AA^{29}$-VVEGXGXR, HVTKPTXAPT-$AA^{11}$-$AA^{12}$:$AA^{26}$-$AA^{27}$-$AA^{28}$-$AA^{29}$-VVRSXGXH, YVP-KPXXAPT-$AA^{11}$-$AA^{12}$:$AA^{26}$-$AA^{27}$-$AA^{28}$-$AA^{29}$-VVRSXGXH, TVPKPXXAPT-$AA^{11}$-$AA^{12}$:$AA^{26}$-$AA^{27}$-$AA^{28}$-$AA^{29}$-VVRAXGXH, AVPKAXXAPT-$AA^{11}$-$AA^{12}$:$AA^{26}$-$AA^{27}$-$AA^{28}$-$AA^{29}$-VVKAXGXH, KVG-KAXXVPT-$AA^{11}$-$AA^{12}$:$AA^{26}$-$AA^{27}$-$AA^{28}$-$AA^{29}$-VAEXGXR, KASKAXXVPT-$AA^{11}$-$AA^{12}$-$AA^{11}$-$AA^{12}$:$AA^{26}$-$AA^{27}$-$AA^{28}$-$AA^{29}$-AVSEXGXR, GSAGPXXTPT-$AA^{11}$-$AA^{12}$:$AA^{26}$-$AA^{27}$-$AA^{28}$-$AA^{29}$-VVDRXGXS, AAPASXXVPA-$AA^{11}$-$AA^{12}$:$AA^{26}$-$AA^{27}$-$AA^{28}$-$AA^{29}$-VVEAXGXR, STPPTXXVPT-$AA^{11}$-$AA^{12}$-$AA^{11}$-$AA^{12}$:$AA^{26}$-$AA^{27}$-$AA^{28}$-$AA^{29}$-VVESXGXR, HVPKPXXAPT-$AA^{11}$-$AA^{12}$:$AA^{26}$-$AA^{27}$-$AA^{28}$-$AA^{29}$-VVRSXGXH, RVPSTXXAPV-$AA^{11}$-$AA^{12}$:$AA^{26}$-$AA^{27}$-$AA^{28}$-$AA^{29}$-VEEXGXL, ASAAPXXVPQ-$AA^{11}$-$AA^{12}$:$AA^{26}$-$AA^{27}$-$AA^{28}$-$AA^{29}$-VRSXKXS, ASASPXXVSQ-$AA^{11}$-$AA^{12}$:$AA^{26}$-$AA^{26}$-$AA^{28}$-$AA^{29}$-VK-SXKXS, ASASPXXVPQ-$AA^{11}$-$AA^{12}$:$AA^{26}$-$AA^{27}$-$AA^{28}$-$AA^{29}$-VKSXKXS, NDEGLEXVPT-$AA^{11}$-$AA^{12}$:$AA^{26}$-$AA^{27}$-$AA^{28}$-$AA^{29}$-QHNKXEXR, NDEGLEXVPT-$AA^{11}$-$AA^{12}$:$AA^{26}$-$AA^{27}$-$AA^{28}$-$AA^{29}$-EHSQXEXR, SS-VKXQPSRV-$AA^{11}$-$AA^{12}$:$AA^{26}$-$AA^{27}$-$AA^{28}$-$AA^{29}$-EEHLEXAXA and RNVQXRPTQV-$AA^{11}$-$AA^{12}$:$AA^{26}$-$AA^{27}$-$AA^{28}$-$AA^{29}$-EDHLAXKXE.

In certain embodiments, PEP9 is selected from the group consisting of KIPKAXXVPTEL, GIPEPXXVPEKM, SIPKAXXVPTEL, HVTKPTXAPTKL, YVPKPXXAPTKL, TVPKPXXAPTQL, AVPKAXXAPTKL, KVGKAXXVPTKL, KASKAXXVPTKL, GSAGPXXTPTKM, AAPASXXVPARL, STPPTXXVPTRL, HVPKPXXAPTKL, RVPSTXXAPVKT, ASAAPXXVPQAL, ASASPXXVSQDL, ASASPXXVPQDL, NDEGLEXVPTEE, NDEGLEXVPTGQ, SSVKXQPSRVHH and RNVQXRPTQVQL; PEP10 is selected from the group consisting of DMVVEGXGXR, NMTVESXAXR, EMVVEGXGXR, NMVVRSXGXH, NMVVRAXGXH, NMVVKAXGXH, EGMSVAEXGXR, GMAVSEXGXR, GMVVDRXGXS, DMVVEAXGXR, DMVVESXGXR, MIVEEXGXL, MIVRSXKXS, MIVKSXKXS, MVVKSXKXS, FLQHNKXEXR, LEEHSQXEXR, RLEEHLEXAXA and TLEDHLAXKXE; and wherein the pair PEP9:PEP10 is selected from the group consisting of KIPKAXXVPTEL:DMVVEGXGXR, GIPEPXXVPEKM:NMTVESXAXR, SIPKAXXVPTEL:EMVVEGXGXR, HVTKPTXAPTKL:NMVVRSXGXH, YVPKPXXAPTKL:NMVVRSXGXH, TVPKPXXAPTQL:NMVVRAXGXH, AVPKAXXAPTKL:NMVVKAXGXH, KVGKAXXVPTKL:EGMSVAEXGXR, KASKAXXVPTKL:GMAVSEXGXR, GSAGPXXTPTKM:GMVVDRXGXS, AAPASXXVPARL:DMVVEAXGXR, STPPTXXVPTRL:DMVVESXGXR, HVPKPXXAPTKL:NMVVRSXGXH, RVPSTXXAPVKT:MIVEEXGXL, ASAAPXXVPQAL:MIVRSXKXS, ASASPXXVSQDL:MIVKSXKXS, ASASPXXVPQDL:MVVKSXKXS, NDEGLEXVPTEE:FLQHNKXEXR, NDEGLEXVPTGQ:LEEHSQXEXR, NDEGLEXVPTEE:FLQHNKXEXR, NDEGLEXVPTEE:FLQHNKXEXR, SSVKXQPSRVHH:RLEEHLEXAXA and RNVQXRPTQVQL:TLEDHLAXKXE.

In certain embodiments, PEP5 is selected from the group consisting of VPTEL, VPEKM, APTKL, APTQL, VPTKL, TPTKM, VPARL, VPTRL, APVKT, VPQAL, VSQDL, VPQDL, VPTEE, VPTGQ, SRVHH and TQVQL; PEP6 is selected from the group consisting of DMVVE, NMTVE, EMVVE, NMVVR, NMVVK, EGMSVAE, GMAVS, GMVVD, MIVEE, MIVRS, MIVKS, MVVKS, FLQHN, LEEHS, RLEEH and TLEDH; wherein the pair PEP5:PEP6 is selected from the group consisting of VPTEL:DMVVE, VPEKM:NMTVE, VPTEL:EMVVE, APTKL:NMVVR, APTQL:NMVVR, APTKL:NMVVK, VPTKL:EGMSVAE, VPTKL:GMAVS, TPTKM:GMVVD, VPARL:DMVVE, VPTRL:DMVVE, APVKT:MIVEE, VPQAL:MIVRS, VSQDL:MIVKS, VPQDL:MVVKS, VPTEE:FLQHN, VPTGQ:LEEHS, SRVHH:RLEEH and TQVQL:TLEDH; and wherein the pair PEP5:PEP6 is not VPTEL:DMVVE when PEP1 is SAIS and PEP2 is LKNYQ; wherein the pair PEP5:PEP6 is not VPEKM:NMTVE when PEP1 is SSLS and PEP2 is LKVYP; wherein the pair PEP5:PEP6 is not VPTEL:EMVVE when PEP1 is SAIS and PEP2 is LKNYQ; wherein the pair PEP5:PEP6 is not APTKL:NMVVR when PEP1 is NAIS and PEP2 is LKKYR; wherein the pair PEP5:PEP6 is not APTQL:NMVVR when PEP1 is NAIS and PEP2 is LKKYR; wherein the pair PEP5:PEP6 is not APTKL:NMVVK when PEP1 is SATS and PEP2 is LRKHR; wherein the pair PEP5:PEP6 is not VPTKL:EGMSVAE when PEP1 is SPIS and PEP2 is LKYHY; wherein the pair PEP5:PEP6 is not VPTKL:GMAVS when PEP1 is EPIS and PEP2 is KFKYE; wherein the pair PEP5:PEP6 is not TPTKM:GMVVD when PEP1 is SPIN and PEP2 is YGKIP; wherein the pair PEP5:PEP6 is not VPARL:DMVVE when

PEP1 is SPIS and PEP2 is YKQYE; wherein the pair PEP5:PEP6 is not VPTRL:DMVVE when PEP1 is SPIS and PEP2 is YKQYE; wherein the pair PEP5:PEP6 is not APVKT:MIVEE when PEP1 is KPLS and PEP2 is DHHKD; wherein the pair PEP5:PEP6 is not VPQAL:MIVRS when PEP1 is EPLP and PEP2 is EQLSN; wherein the pair PEP5:PEP6 is not VSQDL:MIVKS when PEP1 is EPLT and PEP2 is EQLSN; wherein the pair PEP5:PEP6 is not VPQDL:MVVKS when PEP1 is EPLT and PEP2 is EQLSN; wherein the pair PEP5:PEP6 is not VPTEE:FLQHN when PEP1 is SNIT and PEP2 is IGEMS; wherein the pair PEP5:PEP6 is not VPTGQ:LEEHS when PEP1 is SNIT and PEP2 is LGEMS; wherein the pair PEP5:PEP6 is not SRVHH:RLEEH when PEP1 is RSVK and PEP2 is KEVQV; and wherein the pair PEP5:PEP6 is not TQVQL:TLEDH when PEP1 is RPVQ and PEP2 is KKATV.

In certain embodiments, PEP9 is selected from the group consisting of KIPKAXXVPT-$AA^{11}$-$AA^{12}$, GIPEPXXVPE-$AA^{11}$-$AA^{12}$, SIPKAXXVPT-$AA^{11}$-$AA^{12}$, HVTKPTXAPT-$AA^{11}$-$AA^{12}$, YVPKPXXAPT-$AA^{11}$-$AA^{12}$, TVPKPXXAPT-$AA^{11}$-$AA^{12}$, AVPKAXXAPT-$AA^{11}$-$AA^{12}$, KVGKAXXVPT-$AA^{11}$-$AA^{12}$, KASKAXXVPT-$AA^{11}$-$AA^{12}$, GSAGPXXTPT-$AA^{11}$-$AA^{12}$, AAPASXXVPA-$AA^{11}$-$AA^{12}$, STPPTXXVPT-$AA^{11}$-$AA^{12}$, HVPKPXXAPT-$AA^{11}$-$AA^{12}$, RVPSTXXAPV-$AA^{11}$-$AA^{12}$, ASAAPXXVPQ-$AA^{11}$-$AA^{12}$, ASASPXXVSQ-$AA^{11}$-$AA^{12}$, ASASPXXVPQ-$AA^{11}$-$AA^{12}$; NDEGLEXVPT-$AA^{11}$-$AA^{12}$, NDEGLEXVPT-$AA^{11}$-$AA^{12}$, SSVKXQPSRV-$AA^{11}$-$AA^{12}$ and RNVQXRPTQV-$AA^{11}$-$AA^{12}$; wherein $AA^{11}$ is selected from the group consisting of E, K, Q, R, A, D, G and H; and wherein $AA^{12}$ is selected from the group consisting of L, M, T, E, Q and H; PEP10 is selected from the group consisting of $AA^{26}$-$AA^{27}$-$AA^{28}$-$AA^{29}$-VVEGXGXR, $AA^{26}$-$AA^{27}$-$AA^{28}$-$AA^{29}$-TVESXAXR, $AA^{26}$-$AA^{27}$-$AA^{28}$-$AA^{29}$-VVEGXGXR, $AA^{26}$-$AA^{27}$-$AA^{28}$-$AA^{29}$-VVRSXGXH, $AA^{26}$-$AA^{27}$-$AA^{28}$-$AA^{29}$-VVRAXGXH, $AA^{26}$-$AA^{27}$-$AA^{28}$-$AA^{29}$-VVKAXGXH, $AA^{26}$-$AA^{27}$-$AA^{28}$-$AA^{29}$-VAEXGXR, $AA^{26}$-$AA^{27}$-$AA^{28}$-$AA^{29}$-AVSEXGXR, $AA^{26}$-$AA^{27}$-$AA^{28}$-$AA^{29}$-VVDRXGXS, $AA^{26}$-$AA^{27}$-$AA^{28}$-$AA^{29}$-VVEAXGXR, $AA^{26}$-$AA^{27}$-$AA^{28}$-$AA^{29}$-VVESXGXR, $AA^{26}$-$AA^{27}$-$AA^{28}$-$AA^{29}$-VEEXGXL, $AA^{26}$-$AA^{27}$-$AA^{28}$-$AA^{29}$-VRSXKXS, $AA^{26}$-$AA^{27}$-$AA^{28}$-$AA^{29}$-VKSXKXS, $AA^{26}$-$AA^{27}$-$AA^{28}$-$AA^{29}$-VKSXKXS, $AA^{26}$-$AA^{27}$-$AA^{28}$-$AA^{29}$-QHNKXEXR, $AA^{26}$-$AA^{27}$-$AA^{28}$-$AA^{29}$-EHSQXEXR, $AA^{26}$-$AA^{27}$-$AA^{28}$-$AA^{29}$-EEHLEXAXA and $AA^{26}$-$AA^{27}$-$AA^{28}$-$AA^{29}$-EDHLAXKXE; wherein $AA^{26}$ is absent or selected from the group consisting of $AA^{III}$ amino acids, preferably is absent or is E; wherein $AA^{27}$ and $AA^{28}$ are independently selected from the group consisting of $AA^{III}$ and $AA^{V}$ amino acids; and wherein $AA^{29}$ is absent or selected from the group consisting of $AA^{II}$ amino acids, preferably is absent or is S; and wherein the pair PEP9:PEP10 is selected from the group consisting of KIPKAXXVPT-$AA^{11}$-$AA^{12}$:$AA^{26}$-$AA^{27}$-$AA^{28}$-$AA^{29}$-VVEGXGXR, GIPEPXXVPE-$AA^{11}$-$AA^{12}$:$AA^{26}$-$AA^{27}$-$AA^{28}$-$AA^{29}$-TVESXAXR, SIPKAXXVPT-$AA^{11}$-$AA^{12}$:$AA^{26}$-$AA^{27}$-$AA^{28}$-$AA^{29}$-VVEGXGXR, HVTKPTXAPT-$AA^{11}$-$AA^{12}$:$AA^{26}$-$AA^{27}$-$AA^{28}$-$AA^{29}$-VVRSXGXH, YVPKPXXAPT-$AA^{11}$-$AA^{12}$:$AA^{26}$-$AA^{27}$-$AA^{28}$-$AA^{29}$-VVRSXGXH, TVPKPXXAPT-$AA^{11}$-$AA^{12}$:$AA^{26}$-$AA^{27}$-$AA^{28}$-$AA^{29}$-VVRAXGXH, AVPKAXXAPT-$AA^{11}$-$AA^{12}$:$AA^{26}$-$AA^{27}$-$AA^{28}$-$AA^{29}$-VVKAXGXH, KVGKAXXVPT-$AA^{11}$-$AA^{12}$:$AA^{26}$-$AA^{27}$-$AA^{28}$-$AA^{29}$-VAEXGXR, KASKAXXVPT-$AA^{11}$-$AA^{12}$-$AA^{11}$-$AA^{12}$:$AA^{26}$-$AA^{27}$-$AA^{28}$-$AA^{29}$-AVSEXGXR, GSAGPXXTPT-$AA^{11}$-$AA^{12}$:$AA^{26}$-$AA^{27}$-$AA^{28}$-$AA^{29}$-VVDRXGXS, AAPASXXVPA-$AA^{11}$-$AA^{12}$:$AA^{26}$-$AA^{27}$-$AA^{28}$-$AA^{29}$-VVEAXGXR, STPPTXXVPT-$AA^{11}$-$AA^{12}$-$AA^{11}$-$AA^{12}$:$AA^{26}$-$AA^{27}$-$AA^{28}$-$AA^{29}$-VVESXGXR, HVPKPXXAPT-$AA^{11}$-$AA^{12}$:$AA^{26}$-$AA^{27}$-$AA^{28}$-$AA^{29}$-VVRSXGXH, RVPSTXXAPV-$AA^{11}$-$AA^{12}$:$AA^{26}$-$AA^{27}$-$AA^{28}$-$AA^{29}$-VEEXGXL, ASAAPXXVPQ-$AA^{11}$-$AA^{12}$:$AA^{26}$-$AA^{27}$-$AA^{28}$-$AA^{29}$-VRSXKXS, ASASPXXVSQ-$AA^{11}$-$AA^{12}$:$AA^{26}$-$AA^{27}$-$AA^{28}$-$AA^{29}$-VKSXKXS, ASASPXXVPQ-$AA^{11}$-$AA^{12}$:$AA^{26}$-$AA^{27}$-$AA^{28}$-$AA^{29}$-VKSXKXS, NDEGLEXVPT-$AA^{11}$-$AA^{12}$:$AA^{26}$-$AA^{27}$-$AA^{28}$-$AA^{29}$-QHNKXEXR, NDEGLEXVPT-$AA^{11}$-$AA^{12}$:$AA^{26}$-$AA^{27}$-$AA^{28}$-$AA^{29}$-EHSQXEXR, SSVKXQPSRV-$AA^{11}$-$AA^{12}$:$AA^{26}$-$AA^{27}$-$AA^{28}$-$AA^{29}$-EEHLEXAXA and RNVQXRPTQV-$AA^{11}$-$AA^{12}$:$AA^{26}$-$AA^{27}$-$AA^{28}$-$AA^{29}$-EDHLAXKXE; and wherein PEP9:PEP10 is not KIPKAXXVPT-$AA^{11}$-$AA^{12}$:$AA^{26}$-$AA^{27}$-$AA^{28}$-$AA^{29}$-VVEGXGXR when PEP1 is SAIS and PEP2 is LKNYQ ; wherein PEP9:PEP10 is not GIPEPXXVPE-$AA^{11}$-$AA^{12}$:$AA^{26}$-$AA^{27}$-$AA^{28}$-$AA^{29}$-TVESXAXR when PEP1 is SSLS and PEP2 is LKVYP; wherein PEP9:PEP10 is not SIPKAXXVPT-$AA^{11}$-$AA^{12}$:$AA^{26}$-$AA^{27}$-$AA^{28}$-$AA^{29}$-VVEGXGXR when PEP1 is SAIS and PEP2 is LKNYQ; wherein PEP9:PEP10 is not HVTKPTXAPT-$AA^{11}$-$AA^{12}$:$AA^{26}$-$AA^{27}$-$AA^{28}$-$AA^{29}$-VVRSXGXH when PEP1 is NAIS and PEP2 is LKKYR; wherein PEP9:PEP10 is not YVPKPXXAPT-$AA^{11}$-$AA^{12}$:$AA^{26}$-$AA^{27}$-$AA^{28}$-$AA^{29}$-VVRSXGXH when PEP1 is NAIS and PEP2 is LKKYR; wherein PEP9:PEP10 is not TVPKPXXAPT-$AA^{11}$-$AA^{12}$:$AA^{26}$-$AA^{27}$-$AA^{28}$-$AA^{29}$-VVRAXGXH when PEP1 is NAIS and PEP2 is LKKYR; wherein PEP9:PEP10 is not AVPKAXXAPT-$AA^{11}$-$AA^{12}$:$AA^{26}$-$AA^{27}$-$AA^{28}$-$AA^{29}$-VVKAXGXH when PEP1 is SATS and PEP2 is LRKHR; wherein PEP9:PEP10 is not KVGKAXXVPT-$AA^{11}$-$AA^{12}$:$AA^{26}$-$AA^{27}$-$AA^{28}$-$AA^{29}$-VAEXGXR when PEP1 is SPIS and PEP2 is LKYHY; wherein PEP9:PEP10 is not KASKAXXVPT-$AA^{11}$-$AA^{12}$-$AA^{11}$-$AA^{12}$:$AA^{26}$-$AA^{27}$-$AA^{28}$-$AA^{29}$-AVSEXGXR when PEP1 is EPIS and PEP2 is KFKYE; wherein PEP9:PEP10 is not GSAGPXXTPT-$AA^{11}$-$AA^{12}$:$AA^{26}$-$AA^{27}$-$AA^{28}$-$AA^{29}$-VVDRXGXS when PEP1 is SPIN and PEP2 is YGKIP; wherein PEP9:PEP10 is not AAPASXXVPA-$AA^{11}$-$AA^{12}$:$AA^{26}$-$AA^{27}$-$AA^{28}$-$AA^{29}$-VVEAXGXR when PEP1 is SPIS and PEP2 is YKQYE; wherein PEP9:PEP10 is not STPPTXXVPT-$AA^{11}$-$AA^{12}$-$AA^{11}$-$AA^{12}$:$AA^{26}$-$AA^{27}$-$AA^{28}$-$AA^{29}$-VVESXGXR when PEP1 is SPIS and PEP2 is YKQYE; wherein PEP9:PEP10 is not HVPKPXXAPT-$AA^{11}$-$AA^{11}$:$AA^{26}$-$AA^{27}$-$AA^{28}$-$AA^{29}$-VVRSXGXH when PEP1 is NAIS and PEP2 is LKKYR; wherein PEP9:PEP10 is not RVPSTXXAPV-$AA^{11}$-$AA^{12}$:$AA^{26}$-$AA^{27}$-$AA^{28}$-$AA^{29}$-VEEXGXL when PEP1 is KPLS and PEP2 is DHHKD; wherein PEP9:PEP10 is not ASAAPXXVPQ-$AA^{11}$-$AA^{12}$:$AA^{26}$-$AA^{27}$-$AA^{28}$-$AA^{29}$-VRSXKXS when PEP1 is EPLP and PEP2 is EQLSN; ASASPXXVSQ-

AA$^{11}$-AA$^{12}$:AA$^{26}$-AA$^{27}$-AA$^{28}$-AA$^{29}$-VKSXKXS when PEP1 is EPLT and PEP2 is EQLSN; wherein PEP9:PEP10 is not ASASPXXVPQ-AA$^{11}$-AA$^{12}$:AA$^{26}$-AA$^{27}$-AA$^{28}$-AA$^{29}$-VKSXKXS when PEP1 is EPLT and PEP2 is EQLSN; wherein PEP9:PEP10 is not NDEGLEXVPT-AA$^{11}$-AA$^{12}$:AA$^{26}$-AA$^{27}$-AA$^{28}$-AA$^{29}$-QHNKXEXR when PEP1 is SNIT and PEP2 is IGEMS; wherein PEP9:PEP10 is not NDEGLEXVPT-AA$^{11}$-AA$^{12}$:AA$^{26}$-AA$^{27}$-AA$^{28}$-AA$^{29}$-EHSQXEXR when PEP1 is SNIT and PEP2 is LGEMS; wherein PEP9:PEP10 is not SSVKXQPSRV-AA$^{11}$-AA$^{12}$:AA$^{26}$-AA$^{27}$-AA$^{28}$-AA$^{29}$-EEHLEXAXA when PEP1 is RSVK and PEP2 is KEVQV; and wherein PEP9:PEP10 is not RNVQXRPTQV-AA$^{11}$-AA$^{12}$:AA$^{26}$-AA$^{27}$-AA$^{28}$-AA$^{29}$-EDHLAXKXE when PEP1 is RPVQ and PEP2 is KKATV.

In certain embodiments, PEP9 is selected from the group consisting of KIPKAXXVPTEL, GIPEPXXVPEKM, SIPKAXXVPTEL, HVTKPTXAPTKL, YVPKPXXAPTKL, TVPKPXXAPTQL, AVPKAXXAPTKL, KVGKAXXVPTKL, KASKAXXVPTKL, GSAGPXXTPTKM, AAPASXXVPARL, STPPTXXVPTRL, HVPKPXXAPTKL, RVPSTXXAPVKT, ASAAPXXVPQAL, ASASPXXVSQDL, ASASPXXVPQDL, NDEGLEXVPTEE, NDEGLEXVPTGQ, SSVKXQPSRVHH and RNVQXRPTQVQL; PEP10 is selected from the group consisting of DMVVEGXGXR, NMTVESXAXR, EMVVEGXGXR, NMVVRSXGXH, NMVVRAXGXH, NMVVKAXGXH, EGMSVAEXGXR, GMAVSEXGXR, GMVVDRXGXS, DMVVEAXGXR, DMVVESXGXR, MIVEEXGXL, MIVRSXKXS, MIVKSXKXS, MVVKSXKXS, FLQHNKXEXR, LEEHSQXEXR, RLEEHLEXAXA and TLEDHLAXKXE; and wherein the pair PEP9:PEP10 is selected from the group consisting of KIPKAXXVPTEL:DMVVEGXGXR, GIPEPXXVPEKM:NMTVESXAXR, SIPKAXXVPTEL:EMVVEGXGXR, HVTKPTXAPTKL: NMVVRSXGXH, YVPKPXXAPTKL:NMVVRSXGXH, TVPKPXXAPTQL:NMVVRAXGXH, AVPKAXXAPTKL:NMVVKAXGXH, KVGKAXXVPTKL:EGMSVAEXGXR, KASKAXXVPTKL:GMAVSEXGXR, GSAGPXXTPTKM:GMVVDRXGXS, AAPASXXVPARL:DMVVEAXGXR, STPPTXXVPTRL:DMVVESXGXR, HVPKPXXAPTKL:NMVVRSXGXH, RVPSTXXAPVKT:MIVEEXGXL, ASAAPXXVPQAL:M IVRSXKXS, ASASPXXVSQDL:MIVKSXKXS, ASASPXXVPQDL:MVVKSXKXS, NDEGLEXVPTEE:FLQHNKXEXR, NDEGLEXVPTGQ: LEEHSQXEXR, NDEGLEXVPTEE:FLQHNKXEXR, NDEGLEXVPTEE:FLQHNKXEXR, SSVKXQPSRVHH:RLEEHLEXAXA and RNVQXRPTQVQL:TLEDHLAXKXE; and wherein PEP9:PEP10 is not KIPKAXXVPTEL:DMVVEGXGXR when PEP1 is SAIS and PEP2 is LKNYQ ; wherein PEP9:PEP10 is not GIPEPXXVPEKM:NMTVESXAXR when PEP1 is SSLS and PEP2 is LKVYP; wherein PEP9:PEP10 is not SIPKAXXVPTEL:EMVVEGXGXR when PEP1 is SAIS and PEP2 is LKNYQ; wherein PEP9:PEP10 is not HVTKPTXAPTKL:NMVVRSXGXH when PEP1 is NAIS and PEP2 is LKKYR; wherein PEP9:PEP10 is not YVPKPXXAPTKL:NMVVRSXGXH when PEP1 is NAIS and PEP2 is LKKYR; wherein PEP9:PEP10 is not TVPKPXXAPTQL:NMVVRAXGXH when PEP1 is NAIS and PEP2 is LKKYR; wherein PEP9:PEP10 is not AVPKAXXAPTKL:NMVVKAXGXH when PEP1 is SATS and PEP2 is LRKHR; wherein PEP9:PEP10 is not KVGKAXXVPTKL:EGMSVAEXGXR when PEP1 is SPIS and PEP2 is LKYHY; wherein PEP9:PEP10 is not KASKAXXVPTKL:GMAVSEXGXR when PEP1 is EPIS and PEP2 is KFKYE; wherein PEP9:PEP10 is not GSAGPXXTPTKM:GMVVDRXGXS when PEP1 is SPIN and PEP2 is YGKIP; wherein PEP9:PEP10 is not AAPASXXVPARL:DMVVEAXGXR when PEP1 is SPIS and PEP2 is YKQYE; wherein PEP9:PEP10 is not STPPTXXVPTRL:DMVVESXGXR when PEP1 is SPIS and PEP2 is YKQYE; wherein PEP9:PEP10 is not HVPKPXXAPTKL:NMVVRSXGXH when PEP1 is NAIS and PEP2 is LKKYR; wherein PEP9:PEP10 is not RVPSTXXAPVKT:MIVEEXGXL when PEP1 is KPLS and PEP2 is DHHKD; wherein PEP9:PEP10 is not ASAAPXXVPQAL:MIVRSXKXS when PEP1 is EPLP and PEP2 is EQLSN; ASASPXXVSQDL:MIVKSXKXS when PEP1 is EPLT and PEP2 is EQLSN; wherein PEP9:PEP10 is not ASASPXXVPQDL:MVVKSXKXS when PEP1 is EPLT and PEP2 is EQLSN; wherein PEP9:PEP10 is not NDEGLEXVPTEE:FLQHNKXEXR when PEP1 is SNIT and PEP2 is IGEMS; wherein PEP9:PEP10 is not NDEGLEXVPTGQ:LEEHSQXEXR when PEP1 is SNIT and PEP2 is LGEMS ; wherein PEP9:PEP10 is not NDEGLEXVPTEE:FLQHNKXEXR when PEP1 is SNIT and PEP2 is IGEMS; wherein PEP9:PEP10 is not NDEGLEXVPTEE:FLQHNKXEXR when PEP1 is SNIT and PEP2 is IGEMS; wherein PEP9:PEP10 is not SSVKXQPSRVHH:RLEEHLEXAXA when PEP1 is RSVK and PEP2 is KEVQV; and wherein PEP9:PEP10 is not RNVQXRPTQVQL:TLEDHLAXKXE when PEP1 is RPVQ and PEP2 is KKATV.

In certain embodiments, PEP1 is selected from the group consisting of SAIS, SSLS, NAIS, SATS, SPIS, EPIS, SPIN, KPLS, EPLP, EPLT, SNIT, RSVK and RPVQ; PEP11 is selected from the group consisting of LYL, LFF, LYF, LYY, LYK, LYI, LFI, LYV, VYY, QIM, AKV and RKI; and the pair PEP1:PEP11 is selected from the group consisting of SAIS:LYL, SSLS:LFF, NAIS:LYF, SATS:LYY, SPIS:LYK, SPIS:LYI, SPIS:LFI, EPIS:LYL, SPIN:LYF, KPLS:LYV, EPLP:VYY, EPLT:LYY, SNIT:QIM, RSVK:AKV and RPVQ:RKI.

In one particular example, said GFR-binding compound is a peptide, a variant or analog thereof, or a peptidomimetic as defined herein, with (comprising, or exclusively consisting of, or constituted of) between 20 and 60 (in particular between 20 and 50, and more particularly, between 20 and 45) amino acids, comprising a peptide with four amino acids (PEP1) or a peptide with eight amino acids (PEP12), a peptide with five amino acids (PEP2), and optionally a pair of peptides selected form the group consisting of PEP3:PEP4, PEP3:PEP6, PEP3:PEP10, PEP5:PEP4, PEP9:PEP4, PEP5:PEP6, PEP5:PEP10, PEP9:PEP6 and PEP9:PEP10; wherein said GFR-binding compound does not comprise the pair of peptides VPTEL:DMVVE when PEP1 is SAIS and PEP2 is LKNYQ; wherein said GFR-binding compound does not comprise the pair of peptides VPEKM:NMTVE when PEP1 is SSLS and PEP2 is LKVYP; wherein said GFR-binding compound does not comprise the pair of peptides VPTEL:EMVVE when PEP1 is SAIS and PEP2 is LKNYQ;

wherein said GFR-binding compound does not comprise the pair of peptides APTKL:NMVVR when PEP1 is NAIS and PEP2 is LKKYR; wherein said GFR-binding compound does not comprise the pair of peptides APTQL:NMVVR when PEP1 is NAIS and PEP2 is LKKYR; wherein said GFR-binding compound does not comprise the pair of peptides APTKL:NMVVK when PEP1 is SATS and PEP2 is LRKHR; wherein said GFR-binding compound does not comprise the pair of peptides VPTKL:EGMSVAE when PEP1 is SPIS and PEP2 is LKYHY; wherein said GFR-binding compound does not comprise the pair of peptides VPTKL:GMAVS when PEP1 is EPIS and PEP2 is KFKYE; wherein said GFR-binding compound does not comprise the pair of peptides TPTKM:GMVVD when PEP1 is SPIN and PEP2 is YGKIP; wherein said GFR-binding compound does not comprise the pair of peptides VPARL:DMVVE when PEP1 is SPIS and PEP2 is YKQYE; wherein said GFR-binding compound does not comprise the pair of peptides VPTRL:DMVVE when PEP1 is SPIS and PEP2 is YKQYE; wherein said GFR-binding compound does not comprise the pair of peptides APVKT:MIVEE when PEP1 is KPLS and PEP2 is DHHKD; wherein said GFR-binding compound does not comprise the pair of peptides VPQAL:MIVRS when PEP1 is EPLP and PEP2 is EQLSN; wherein said GFR-binding compound does not comprise the pair of peptides VSQDL:MIVKS when PEP1 is EPLT and PEP2 is EQLSN; wherein said GFR-binding compound does not comprise the pair of peptides VPQDL:MVVKS when PEP1 is EPLT and PEP2 is EQLSN; wherein said GFR-binding compound does not comprise the pair of peptides VPTEE:FLQHN when PEP1 is SNIT and PEP2 is IGEMS; wherein said GFR-binding compound does not comprise the pair of peptides VPTGQ:LEEHS when PEP1 is SNIT and PEP2 is LGEMS; wherein said GFR-binding compound does not comprise the pair of peptides SRVHH:RLEEH when PEP1 is RSVK and PEP2 is KEVQV; and wherein said GFR-binding compound does not comprise the pair of peptides TQVQL:TLEDH when PEP1 is RPVQ and PEP2 is KKATV.

In one particular example, said GFR-binding compound is a peptide, a variant or analog thereof, or a peptidomimetic as defined herein, with (comprising, or exclusively consisting of, or constituted of) between 20 and 60 (in particular between 20 and 50, and more particularly, between 20 and 45) amino acids, comprising a peptide with four amino acids (PEP1) or a peptide with eight amino acids (PEP12), a peptide with five amino acids (PEP2), and optionally a pair of peptides selected form the group consisting of PEP3:PEP4, PEP3:PEP6, PEP3:PEP10, PEP5:PEP4, PEP9:PEP4, PEP5:PEP6, PEP5:PEP10, PEP9:PEP6 and PEP9:PEP10; wherein said GFR-binding compound does not comprise the pair of peptides KIPKAXXVPTEL:DMVVEGXGXR when PEP1 is SAIS and PEP2 is LKNYQ ; wherein said GFR-binding compound does not comprise the pair of peptides GIPEPXXVPEKM:NMTVESXAXR when PEP1 is SSLS and PEP2 is LKVYP; wherein said GFR-binding compound does not comprise the pair of peptides SIPKAXXVPTEL:EMVVEGXGXR when PEP1 is SAIS and PEP2 is LKNYQ; wherein said GFR-binding compound does not comprise the pair of peptides HVTKPTXAPTKL:NMVVRSXGXH when PEP1 is NAIS and PEP2 is LKKYR; wherein said GFR-binding compound does not comprise the pair of peptides YVPKPXXAPTKL:NMVVRSXGXH when PEP1 is NAIS and PEP2 is LKKYR; wherein said GFR-binding compound does not comprise the pair of peptides TVPKPXXAPTQL:NMVVRAXGXH when PEP1 is NAIS and PEP2 is LKKYR; wherein said GFR-binding compound does not comprise the pair of peptides AVPKAXXAPTKL:NMVVKAXGXH when PEP1 is SATS and PEP2 is LRKHR; wherein said GFR-binding compound does not comprise the pair of peptides KVGKAXXVPTKL:EGMSVAEXGXR when PEP1 is SPIS and PEP2 is LKYHY; wherein said GFR-binding compound does not comprise the pair of peptides KASKAXXVPTKL:GMAVSEXGXR when PEP1 is EPIS and PEP2 is KFKYE; wherein said GFR-binding compound does not comprise the pair of peptides GSAGPXXTPTKM:GMVVDRXGXS when PEP1 is SPIN and PEP2 is YGKIP; wherein said GFR-binding compound does not comprise the pair of peptides AAPASXXVPARL:DMVVEAXGXR when PEP1 is SPIS and PEP2 is YKQYE; wherein said GFR-binding compound does not comprise the pair of peptides STPPTXXVPTRL:DMVVESXGXR when PEP1 is SPIS and PEP2 is YKQYE; wherein said GFR-binding compound does not comprise the pair of peptides HVPKPXXAPTKL:NMVVRSXGXH when PEP1 is NAIS and PEP2 is LKKYR; wherein said GFR-binding compound does not comprise the pair of peptides RVPSTXXAPVKT:MIVEEXGXL when PEP1 is KPLS and PEP2 is DHHKD; wherein said GFR-binding compound does not comprise the pair of peptides ASAAPXXVPQAL:MIVRSXKXS when PEP1 is EPLP and PEP2 is EQLSN; ASASPXXVSQDL:MIVKSXKXS when PEP1 is EPLT and PEP2 is EQLSN; wherein said GFR-binding compound does not comprise the pair of peptides ASASPXXVPQDL:MVVKSXKXS when PEP1 is EPLT and PEP2 is EQLSN; wherein said GFR-binding compound does not comprise the pair of peptides NDEGLEXVPTEE:FLQHNKXEXR when PEP1 is SNIT and PEP2 is IGEMS; wherein said GFR-binding compound does not comprise the pair of peptides NDEGLEXVPTGQ:LEEHSQXEXR when PEP1 is SNIT and PEP2 is LGEMS ; wherein said GFR-binding compound does not comprise the pair of peptides NDEGLEXVPTEE:FLQHNKXEXR when PEP1 is SNIT and PEP2 is IGEMS; wherein said GFR-binding compound does not comprise the pair of peptides NDEGLEXVPTEE:FLQHNKXEXR when PEP1 is SNIT and PEP2 is IGEMS; wherein said GFR-binding compound does not comprise the pair of peptides SSVKXQPSRVHH:RLEEHLEXAXA when PEP1 is RSVK and PEP2 is KEVQV; wherein said GFR-binding compound does not comprise the pair of peptides RNVQXRPTQVQL:TLEDHLAXKXE when PEP1 is RPVQ and PEP2 is KKATV.

In particular, in certain embodiments, the quadruplet PEP3:PEP1:PEP2:PEP4 is selected from the group consisting of VPT:SAIS:LKNYQ:VVE, VPE:SAIS:LKNYQ:TVE, APT:SAIS:LKNYQ:VVR, APT:SAIS:LKNYQ:VVK, VPT:SAIS:LKNYQ:VAE, VPT:SAIS:LKNYQ:AVS, TPT:SAIS:LKNYQ:VVD, VPA:SAIS:LKNYQ:VVE, APV:SAIS:LKNYQ:VEE,

VPQ:SAIS:LKNYQ:VRS, VSQ:SAIS:LKNYQ:VKS, VPQ:SAIS:LKNYQ:VKS, VPT:SAIS:LKNYQ:QHN, VPT:SAIS:LKNYQ:EHS, SRV:SAIS:LKNYQ:EEH, TQV:SAIS:LKNYQ:EDH, VPE:SSLS:LKVYP:TVE, VPT:SSLS:LKVYP:VVE, APT:SSLS:LKVYP:VVR, APT:SSLS:LKVYP:VVK, VPT:SSLS:LKVYP:VAE, VPT:SSLS:LKVYP:AVS, TPT:SSLS:LKVYP:VVD, VPA:SSLS:LKVYP:VVE, APV:SSLS:LKVYP:VEE, VPQ:SSLS:LKVYP:VRS, VSQ:SSLS:LKVYP:VKS, VPQ:SSLS:LKVYP:VKS, VPT:SSLS:LKVYP:QHN, VPT:SSLS:LKVYP:EHS, SRV:SSLS:LKVYP:EEH, TQV:SSLS:LKVYP:EDH, APT:NAIS:LKKYR:VVR, VPT:NAIS:LKKYR:VVE, VPE:NAIS:LKKYR:TVE, APT:NAIS:LKKYR:VVK, VPT:NAIS:LKKYR:VAE, VPT:NAIS:LKKYR:AVS, TPT:NAIS:LKKYR:VVD, VPA:NAIS:LKKYR:VVE, APV:NAIS:LKKYR:VEE, VPQ:NAIS:LKKYR:VRS, VSQ:NAIS:LKKYR:VKS, VPQ:NAIS:LKKYR:VKS, VPT:NAIS:LKKYR:QHN, VPT:NAIS:LKKYR:EHS, SRV:NAIS:LKKYR:EEH, TQV:NAIS:LKKYR:EDH, APT:SATS:LRKHR:VVK, VPT:SATS:LRKHR:VVE, VPE:SATS:LRKHR:TVE, APT:SATS:LRKHR:VVR, VPT:SATS:LRKHR:VAE, VPT:SATS:LRKHR:AVS, TPT:SATS:LRKHR:VVD, VPA:SATS:LRKHR:VVE, APV:SATS:LRKHR:VEE, VPQ:SATS:LRKHR:VRS, VSQ:SATS:LRKHR:VKS, VPQ:SATS:LRKHR:VKS, VPT:SATS:LRKHR:QHN, VPT:SATS:LRKHR:EHS, SRV:SATS:LRKHR:EEH, TQV:SATS:LRKHR:EDH, VPT:SPIS:LKYHY:VAE, VPT:SPIS:LKYHY:VVE, VPE:SPIS:LKYHY:TVE, APT:SPIS:LKYHY:VVR, APT:SPIS:LKYHY:VVK, VPT:SPIS:LKYHY:AVS, TPT:SPIS:LKYHY:VVD, VPA:SPIS:LKYHY:VVE, APV:SPIS:LKYHY:VEE, VPQ:SPIS:LKYHY:VRS, VSQ:SPIS:LKYHY:VKS, VPQ:SPIS:LKYHY:VKS, VPT:SPIS:LKYHY:QHN, VPT:SPIS:LKYHY:EHS, SRV:SPIS:LKYHY:EEH, TQV:SPIS:LKYHY:EDH, VPT:EPIS:KFKYE:AVS, VPT:EPIS:KFKYE:VVE, VPE:EPIS:KFKYE:TVE, APT:EPIS:KFKYE:VVR, APT:EPIS:KFKYE:VVK, VPT:EPIS:KFKYE:VAE, TPT:EPIS:KFKYE:VVD, VPA:EPIS:KFKYE:VVE, APV:EPIS:KFKYE:VEE, VPQ:EPIS:KFKYE:VRS, VSQ:EPIS:KFKYE:VKS, VPQ:EPIS:KFKYE:VKS, VPT:EPIS:KFKYE:QHN, VPT:EPIS:KFKYE:EHS, SRV:EPIS:KFKYE:EEH, TQV:EPIS:KFKYE:EDH, TPT:SPIN:YGKIP:VVD, VPT:SPIN:YGKIP:VVE, VPE:SPIN:YGKIP:TVE, APT:SPIN:YGKIP:VVR, APT:SPIN:YGKIP:VVK, VPT:SPIN:YGKIP:VAE, VPT:SPIN:YGKIP:AVS, VPA:SPIN:YGKIP:VVE, APV:SPIN:YGKIP:VEE, VPQ:SPIN:YGKIP:VRS, VSQ:SPIN:YGKIP:VKS, VPQ:SPIN:YGKIP:VKS, VPT:SPIN:YGKIP:QHN, VPT:SPIN:YGKIP:EHS, SRV:SPIN:YGKIP:EEH, TQV:SPIN:YGKIP:EDH, VPA:SPIS:YKQYE:VVE, VPT:SPIS:YKQYE:VVE, VPE:SPIS:YKQYE:TVE, APT:SPIS:YKQYE:VVR, APT:SPIS:YKQYE:VVK, VPT:SPIS:YKQYE:VAE, VPT:SPIS:YKQYE:AVS, TPT:SPIS:YKQYE:VVD, APV:SPIS:YKQYE:VEE, VPQ:SPIS:YKQYE:VRS, VSQ:SPIS:YKQYE:VKS, VPQ:SPIS:YKQYE:VKS, VPT:SPIS:YKQYE:QHN, VPT:SPIS:YKQYE:EHS, SRV:SPIS:YKQYE:EEH, TQV:SPIS:YKQYE:EDH, APV:KPLS:DHHKD:VEE, VPT:KPLS:DHHKD:VVE, VPE:KPLS:DHHKD:TVE, APT:KPLS:DHHKD:VVR, APT:KPLS:DHHKD:VVK, VPT:KPLS:DHHKD:VAE, VPT:KPLS:DHHKD:AVS, TPT:KPLS:DHHKD:VVD, VPA:KPLS:DHHKD:VVE, VPQ:KPLS:DHHKD:VRS, VSQ:KPLS:DHHKD:VKS, VPQ:KPLS:DHHKD:VKS, VPT:KPLS:DHHKD:QHN, VPT:KPLS:DHHKD:EHS, SRV:KPLS:DHHKD:EEH, TQV:KPLS:DHHKD:EDH, VPQ:EPLP:EQLSN:VRS, VPT:EPLP:EQLSN:VVE, VPE:EPLP:EQLSN:TVE, APT:EPLP:EQLSN:VVR, APT:EPLP:EQLSN:VVK, VPT:EPLP:EQLSN:VAE, VPT:EPLP:EQLSN:AVS, TPT:EPLP:EQLSN:VVD, VPA:EPLP:EQLSN:VVE, APV:EPLP:EQLSN:VEE, VSQ:EPLP:EQLSN:VKS, VPQ:EPLP:EQLSN:VKS, VPT:EPLP:EQLSN:QHN, VPT:EPLP:EQLSN:EHS, SRV:EPLP:EQLSN:EEH, TQV:EPLP:EQLSN:EDH, VSQ:EPLT:EQLSN:VKS, VPT:EPLT:EQLSN:VVE, VPE:EPLT:EQLSN:TVE, APT:EPLT:EQLSN:VVR, APT:EPLT:EQLSN:VVK, VPT:EPLT:EQLSN:VAE, VPT:EPLT:EQLSN:AVS, TPT:EPLT:EQLSN:VVD, VPA:EPLT:EQLSN:VVE, APV:EPLT:EQLSN:VEE, VPQ:EPLT:EQLSN:VRS, VPQ:EPLT:EQLSN:VKS, VPT:EPLT:EQLSN:QHN, VPT:EPLT:EQLSN:EHS, SRV:EPLT:EQLSN:EEH, TQV:EPLT:EQLSN:EDH, VPT:SNIT:IGEMS:QHN, VPT:SNIT:IGEMS:VVE, VPE:SNIT:IGEMS:TVE, APT:SNIT:IGEMS:VVR, APT:SNIT:IGEMS:VVK, VPT:SNIT:IGEMS:VAE, VPT:SNIT:IGEMS:AVS, TPT:SNIT:IGEMS:VVD, VPA:SNIT:IGEMS:VVE, APV:SNIT:IGEMS:VEE, VPQ:SNIT:IGEMS:VRS, VSQ:SNIT:IGEMS:VKS, VPQ:SNIT:IGEMS:VKS, VPT:SNIT:IGEMS:EHS, SRV:SNIT:IGEMS:EEH, TQV:SNIT:IGEMS:EDH, VPT:SNIT:LGEMS:EHS, VPT:SNIT:LGEMS:VVE, VPE:SNIT:LGEMS:TVE, APT:SNIT:LGEMS:VVR, APT:SNIT:LGEMS:VVK, VPT:SNIT:LGEMS:VAE, VPT:SNIT:LGEMS:AVS, TPT:SNIT:LGEMS:VVD, VPA:SNIT:LGEMS:VVE, APV:SNIT:LGEMS:VEE, VPQ:SNIT:LGEMS:VRS, VSQ:SNIT:LGEMS:VKS, VPQ:SNIT:LGEMS:VKS, VPT:SNIT:LGEMS:QHN, SRV:SNIT:LGEMS:EEH, TQV:SNIT:LGEMS:EDH, SRV:RSVK:KEVQV:EEH, VPT:RSVK:KEVQV:VVE, VPE:RSVK:KEVQV:TVE, APT:RSVK:KEVQV:VVR, APT:RSVK:KEVQV:VVK, VPT:RSVK:KEVQV:VAE, VPT:RSVK:KEVQV:AVS, TPT:RSVK:KEVQV:VVD, VPA:RSVK:KEVQV:VVE, APV:RSVK:KEVQV:VEE, VPQ:RSVK:KEVQV:VRS, VSQ:RSVK:KEVQV:VKS, VPQ:RSVK:KEVQV:VKS, VPT:RSVK:KEVQV:QHN, VPT:RSVK:KEVQV:EHS, TQV:RSVK:KEVQV:EDH, TQV:RPVQ:KKATV:EDH, VPT:RPVQ:KKATV:VVE, VPE:RPVQ:KKATV:TVE, APT:RPVQ:KKATV:VVR, APT:RPVQ:KKATV:VVK, VPT:RPVQ:KKATV:VAE, VPT:RPVQ:KKATV:AVS, TPT:RPVQ:KKATV:VVD, VPA:RPVQ:KKATV:VVE, APV:RPVQ:KKATV:VEE, VPQ:RPVQ:KKATV:VRS, VSQ:RPVQ:KKATV:VKS, VPQ:RPVQ:KKATV:VKS, VPT:RPVQ:KKATV:QHN, VPT:RPVQ:KKATV:EHS and SRV:RPVQ:KKATV:EEH.

In particular, in certain embodiments, the quadruplet PEP3:PEP12:PEP2:PEP4 is selected from the group consisting of VPT:SAIS-AA$^{17}$-LYL:LKNYQ:VVE, VPE:SAIS-AA$^{17}$-LYL:LKNYQ:TVE, APT:SAIS-AA$^{17}$-LYL:LKNYQ:VVR,

APT:SAIS-AA$^{17}$-LYL:LKNYQ:VVK, VPT:SAIS-AA$^{17}$-LYL:LKNYQ:VAE, VPT:SAIS-AA$^{17}$-LYL:LKNYQ:AVS, TPT:SAIS-AA$^{17}$-LYL:LKNYQ:VVD, VPA:SAIS-AA$^{17}$-LYL:LKNYQ:VVE, APV:SAIS-AA$^{17}$-LYL:LKNYQ:VEE, VPQ:SAIS_AA$^{17}$-LYL:LKNYQ:VRS, VSQ:SAIS-AA$^{17}$-LYL:LKNYQ:VKS, VPQ:SAIS-AA$^{17}$-LYL:LKNYQ:VKS, VPT:SAIS-AA$^{17}$-LYL:LKNYQ:QHN, VPT:SAIS-AA$^{17}$-LYL:LKNYQ:EHS, SRV:SAIS-AA$^{17}$-LYL:LKNYQ:EEH, TQV:SAIS-AA$^{17}$-LYL:LKNYQ:EDH, VPE:SSLS-AA$^{17}$-LFF:LKVYP:TVE, VPT:SSLS-AA$^{17}$-LFF:LKVYP:VVE, APT:SSLS-AA$^{17}$-LFF:LKVYP:VVR, APT:SSLS-AA$^{17}$-LFF:LKVYP:VVK, VPT:SSLS-AA$^{17}$-LFF:LKVYP:VAE, VPT:SSLS-AA$^{17}$-LFF:LKVYP:AVS, TPT:SSLS-AA$^{17}$-LFF:LKVYP:VVD, VPA:SSLS-AA$^{17}$-LFF:LKVYP:VVE, APV:SSLS-AA$^{17}$-LFF:LKVYP:VEE, VPQ:SSLS-AA$^{17}$-LFF:LKVYP:VRS, VSQ:SSLS-AA$^{17}$-LFF:LKVYP:VKS, VPQ:SSLS-AA$^{17}$-LFF:LKVYP:VKS, VPT:SSLS-AA$^{17}$-LFF:LKVYP:QHN, VPT:SSLS-AA$^{17}$-LFF:LKVYP:EHS, SRV:SSLS-AA$^{17}$-LFF:LKVYP:EEH, TQV:SSLS-AA$^{17}$-LFF:LKVYP:EDH, APT:NAIS-AA$^{17}$-LYF:LKKYRVVR, VPT:NAIS-AA$^{17}$-LYF:LKKYR:VVE, VPE:NAIS-AA$^{17}$-LYF:LKKYR:TVE, APT:NAIS-AA$^{17}$-LYF:LKKYR:VVK, VPT:NAIS-AA$^{17}$-LYF:LKKYR:VAE, VPT:NAIS-AA$^{17}$-LYF:LKKYR:AVS, TPT:NAIS-AA$^{17}$-LYF:LKKYR:VVD, VPA:NAIS-AA$^{17}$-LYF:LKKYR:VVE, APV:NAIS-AA$^{17}$-LYF:LKKYR:VEE, VPQ:NAIS-AA$^{17}$-LYF:LKKYR:VRS, VSQ:NAIS-AA$^{17}$-LYF:LKKYR:VKS, VPQ:NAIS-AA$^{17}$-LYF:LKKYR:VKS, VPT:NAIS-AA$^{17}$-LYF:LKKYR:QHN, VPT:NAIS-AA$^{17}$-LYF:LKKYR:EHS, SRV:NAIS-AA$^{17}$-LYF:LKKYR:EEH, TQV:NAIS-AA$^{17}$-LYF:LKKYR:EDH, APT:SATS-AA$^{17}$-LYY:LRKHR:VVK, VPT:SATS-AA$^{17}$-LYY:LRKHR:VVE, VPE:SATS-AA$^{17}$-LYY:LRKHR:TVE, APT:SATS-AA$^{17}$-LYY:LRKHR:VVR, VPT:SATS-AA$^{17}$-LYY:LRKHR:VAE, VPT:SATS-AA$^{17}$-LYY:LRKHR:AVS, TPT:SATS-AA$^{17}$-LYY:LRKHR:VVD, VPA:SATS-AA$^{17}$-LYY:LRKHR:VVE, APV:SATS-AA$^{17}$-LYY:LRKHR:VEE, VPQ:SATS-AA$^{17}$-LYY:LRKHR:VRS, VSQ:SATS-AA$^{17}$-LYY:LRKHR:VKS, VPQ:SATS-AA$^{17}$-LYY:LRKHR:VKS, VPT:SATS-AA$^{17}$-LYY:LRKHR:QHN, VPT:SATS-AA$^{17}$-LYY:LRKHR:EHS, SRV:SATS-AA$^{17}$-LYY:LRKHR:EEH, TQV:SATS-AA$^{17}$-LYY:LRKHR:EDH, VPT:SPIS-AA$^{17}$-LYK:LKYHY:VAE, VPT:SPIS-AA$^{17}$-LYK:LKYHY:VVE, VPE:SPIS-AA$^{17}$-LYK:LKYHY:TVE, APT:SPIS-AA$^{17}$-LYK:LKYHY:VVR, APT:SPIS-AA$^{17}$-LYK:LKYHY:VVK, VPT:SPIS-AA$^{17}$-LYK:LKYHY:AVS, TPT:SPIS-AA$^{17}$-LYK:LKYHY:VVD, VPA:SPIS-AA$^{17}$-LYK:LKYHY:VVE, APV:SPIS-AA$^{17}$-LYK:LKYHY:VEE, VPQ:SPIS-AA$^{17}$-LYK:LKYHY:VRS, VSQ:SPIS-AA$^{17}$-LYK:LKYHY:VKS, VPQ:SPIS-AA$^{17}$-LYK:LKYHY:VKS, VPT:SPIS-AA$^{17}$-LYK:LKYHY:QHN, VPT:SPIS-AA$^{17}$-LYK:LKYHY:EHS, SRV:SPIS-AA$^{17}$-LYK:LKYHY:EEH, TQV:SPIS-AA$^{17}$-LYK:LKYHY:EDH, VPT:EPIS-AA$^{17}$-LYL:KFKYE:AVS, VPT:EPIS-AA$^{17}$-LYL:KFKYE:VVE, VPE:EPIS-AA$^{17}$-LYL:KFKYE:TVE, APT:EPIS-AA$^{17}$-LYL:KFKYE:VVR, APT:EPIS-AA$^{17}$-LYL:KFKYE:VVK, VPT:EPIS-AA$^{17}$-LYL:KFKYE:VAE, TPT:EPIS-AA$^{17}$-LYL:KFKYE:VVD, VPA:EPIS-AA$^{17}$-LYL:KFKYE:VVE, APV:EPIS-AA$^{17}$-LYL:KFKYE:VEE, VPQ:EPIS-AA$^{17}$-LYL:KFKYE:VRS, VSQ:EPIS-AA$^{17}$-LYL:KFKYE:VKS, VPQ:EPIS-AA$^{17}$-LYL:KFKYE:VKS, VPT:EPIS-AA$^{17}$-LYL:KFKYE:QHN, VPT:EPIS-AA$^{17}$-LYL:KFKYE:EHS, SRV:EPIS-AA$^{17}$-LYL:KFKYE:EEH, TQV:EPIS-AA$^{17}$-LYL:KFKYE:EDH, TPT:SPIN-AA$^{17}$-LYF:YGKIP:VVD, VPT:SPIN-AA$^{17}$-LYF:YGKIP:VVE, VPE:SPIN-AA$^{17}$-LYF:YGKIP:TVE, APT:SPIN-AA$^{17}$-LYF:YGKIP:VVR, APT:SPIN-AA$^{17}$-LYF:YGKIP:VVK, VPT:SPIN-AA$^{17}$-LYF:YGKIP:VAE, VPT:SPIN-AA$^{17}$-LYF:YGKIP:AVS, VPA:SPIN-AA$^{17}$-LYF:YGKIP:VVE, APV:SPIN-AA$^{17}$-LYF:YGKIP:VEE, VPQ:SPIN-AA$^{17}$-LYF:YGKIP:VRS, VSQ:SPIN-AA$^{17}$-LYF:YGKIP:VKS, VPQ:SPIN-AA$^{17}$-LYF:YGKIP:VKS, VPT:SPIN-AA$^{17}$-LYF:YGKIP:QHN, VPT:SPIN-AA$^{17}$-LYF:YGKIP:EHS, SRV:SPIN-AA$^{17}$-LYF:YGKIP:EEH, TQV:SPIN-AA$^{17}$-LYF:YGKIP:EDH, VPA:SPIS-AA$^{17}$-LYI:YKQYE:VVE, VPT:SPIS-AA$^{17}$-LYI:YKQYE:VVE, VPE:SPIS-AA$^{17}$-LYI:YKQYE:TVE, APT:SPIS-AA$^{17}$-LYI:YKQYE:VVR, APT:SPIS-AA$^{17}$-LYI:YKQYE:VVK, VPT:SPIS-AA$^{17}$-LYI:YKQYE:VAE, VPT:SPIS-AA$^{17}$-LYI:YKQYE:AVS, TPT:SPIS-AA$^{17}$-LYI:YKQYE:VVD, APV:SPIS-AA$^{17}$-LYI:YKQYE:VEE, VPQ:SPIS-AA$^{17}$-LYI:YKQYE:VRS, VSQ:SPIS-AA$^{17}$-LYI:YKQYE:VKS, VPQ:SPIS-AA$^{17}$-LYI:YKQYE:VKS, VPT:SPIS-AA$^{17}$-LYI:YKQYE:QHN, VPT:SPIS-AA$^{17}$-LYI:YKQYE:EHS, SRV:SPIS-AA$^{17}$-LYI:YKQYE:EEH, TQV:SPIS-AA$^{17}$-LYI:YKQYE:EDH, VPT:SPIS-AA$^{17}$-LFI:YKQYE:VVE, VPE:SPIS-AA$^{17}$-LFI:YKQYE:TVE, APT:SPIS-AA$^{17}$-LFI:YKQYE:VVR, APT:SPIS-AA$^{17}$-LFI:YKQYE:VVK, VPT:SPIS-AA$^{17}$-LFI:YKQYE:VAE, VPT:SPIS-AA$^{17}$-LFI:YKQYE:AVS, TPT:SPIS-AA$^{17}$-LFI:YKQYE:VVD, VPA:SPIS-AA$^{17}$-LFI:YKQYE:VVE, APV:SPIS-AA$^{17}$-LFI:YKQYE:VEE, VPQ:SPIS-AA$^{17}$-LFI:YKQYE:VRS, VSQ:SPIS-AA$^{17}$-LFI:YKQYE:VKS, VPQ:SPIS-AA$^{11}$-LFI:YKQYE:VKS, VPT:SPIS-AA$^{17}$-LFI:YKQYE:QHN, VPT:SPIS-AA$^{17}$-LFI:YKQYE:EHS, SRV:SPIS-AA$^{17}$-LFI:YKQYE:EEH, TQV:SPIS-AA$^{17}$-LFI:YKQYE:EDH, APV:KPLS-AA$^{17}$-LYV:DHHKD:VEE, VPT:KPLS-AA$^{17}$-LYV:DHHKD:VVE, VPE:KPLS-AA$^{17}$-LYV:DHHKD:TVE, APT:KPLS-AA$^{17}$-LYV:DHHKD:VVR, APT:KPLS-AA$^{17}$-LYV:DHHKD:VVK, VPT:KPLS-AA$^{17}$-LYV:DHHKD:VAE, VPT:KPLS-AA$^{17}$-LYV:DHHKD:AVS, TPT:KPLS-AA$^{17}$-LYV:DHHKD:VVD, VPA:KPLS-AA$^{17}$-LYV:DHHKD:VVE, VPQ:KPLS-AA$^{17}$-LYV:DHHKD:VRS, VSQ:KPLS-AA$^{17}$-LYV:DHHKD:VKS, VPQ:KPLS-AA$^{17}$-LYV:DHHKD:VKS, VPT:KPLS-AA$^{17}$-LYV:DHHKD:QHN, VPT:KPLS-AA$^{17}$-LYV:DHHKD:EHS, SRV:KPLS-AA$^{17}$-LYV:DHHKD:EEH, TQV:KPLS-AA$^{17}$-LYV:DHHKD:EDH, VPQ:EPLP-AA$^{17}$-VYY:EQLSN:VRS, VPT:EPLP-AA$^{17}$-VYY:EQLSN:VVE, VPE:EPLP-AA$^{17}$-VYY:EQLSN:TVE, APT:EPLP-AA$^{17}$-VYY:EQLSN:VVR, APT:EPLP-AA$^{17}$-VYY:EQLSN:VVK, VPT:EPLP-AA$^{17}$-VYY:EQLSN:VAE, VPT:EPLP-AA$^{17}$-VYY:EQLSN:AVS, TPT:EPLP-AA$^{17}$-VYY:EQLSN:VVD, VPA:EPLP-AA$^{17}$-VYY:EQLSN:VVE, APV:EPLP-AA$^{17}$-VYY:EQLSN:VEE, VSQ:EPLP-AA$^{17}$-VYY:EQLSN:VKS, VPQ:EPLP-AA$^{17}$-VYY:EQLSN:VKS, VPT:EPLP-AA$^{17}$-VYY:EQLSN:QHN, VPT:EPLP-AA$^{17}$-VYY:EQLSN:EHS, SRV:EPLP-AA$^{17}$-VYY:EQLSN:EEH, TQV:EPLP-AA$^{17}$-VYY:EQLSN:EDH, VSQ:EPLT-AA$^{17}$-LYY:EQLSN:VKS, VPT:EPLT-

AA$^{17}$-LYY:EQLSN:VVE, VPE:EPLT-AA$^{17}$-LYY:EQLSN:TVE, APT:EPLT-AA$^{17}$-LYY:EQLSN:VVR, APT:EPLT-AA$^{17}$-LYY:EQLSN:VVK, VPT:EPLT-AA$^{17}$-LYY:EQLSN:VAE, VPT:EPLT-AA$^{17}$-LYY:EQLSN:AVS, TPT:EPLT-AA$^{17}$-LYY:EQLSN:VVD, VPA:EPLT-AA$^{17}$-LYY:EQLSN:VVE, APV:EPLT-AA$^{17}$-LYY:EQLSN:VEE, VPQ:EPLT-AA$^{17}$-LYY:EQLSN:VRS, VPQ:EPLT-AA$^{17}$-LYY:EQLSN:VKS, VPT:EPLT-AA$^{17}$-LYY:EQLSN:QHN, VPT:EPLT-AA$^{17}$-LYY:EQLSN:EHS, SRV:EPLT-AA$^{17}$-LYY:EQLSN:EEH, TQV:EPLT-AA$^{17}$-LYY:EQLSN:EDH, VPT:SNIT-AA$^{17}$-QIM :IGEMS:QHN, VPT:SNIT-AA$^{17}$-QIM:IGEMS:VVE, VPE:SNIT-AA$^{17}$-QIM:IGEMS:TVE, APT:SNIT-AA$^{17}$-QIM:IGEMS:VVR, APT:SNIT-AA$^{17}$-QIM:IGEMS:VVK, VPT:SNIT-AA$^{17}$-QIM:IGEMS:VAE, VPT:SNIT-AA$^{17}$-QIM:IGEMS:AVS, TPT:SNIT-AA$^{17}$-Q,M:IGEMS:VVD, VPASNIT-AA$^{17}$-QIM:IGEMS:VVE, APV:SNIT-AA$^{17}$-QIM:IGEMS:VEE, VPQ:SNIT-AA$^{17}$-QIM:IGEMS:VRS, VSQ:SNIT-AA$^{17}$-QIM:IGEMSVKS, VPQ:SNIT-AA$^{17}$-QIM:IGEMS:VKS, VPT:SNIT-AA$^{17}$-QIM:IGEMS:EHS, SRV:SNIT-AA$^{17}$-QIM:IGEMS:EEH, TQV:SNIT-AA$^{17}$-Q,M: IGEMS:EDH, VPT:SNIT-AA$^{17}$-QIM:LGEMS:EHS, VPT:SNIT-AA$^{17}$-QIM:LGEMS:VVE, VPE:SNIT-AA$^{17}$-QIM:LGEMS:TVE, APT:SNIT-AA$^{17}$-QIM:LGEMS:VVR, APT:SNIT-AA$^{17}$-QIM :LGEMS:VVK, VPT:SNIT-AA$^{17}$-QIM:LGEMS:VAE, VPT:SNIT-AA$^{17}$-QIM:LGEMS:AVS, TPT:SNIT-AA$^{17}$-QIM:LGEMS:VVD, VPA:SNIT-AA$^{17}$-QIM:LGEMS:VVE, APV:SNIT-AA$^{17}$-QIM:LGEMS:VEE, VPQ:SNIT-AA$^{17}$-QIM:LGEMS:VRS, VSQ:SNIT-AA$^{17}$-QIM:LGEMS:VKS, VPQ:SNIT-AA$^{17}$-QIM:LGEMS:VKS, VPT:SNIT-AA$^{17}$-QIM:LGEMS:QHN, SRV:SNIT-AA$^{17}$-QIM:LGEMS:EEH, TQV:SNIT-AA$^{17}$-QIM:LGEMS:EDH, SRV:RSVK-AA$^{17}$-AKV:KEVQV:EEH, VPT:RSVK-AA$^{17}$-AKV:KEVQV:VVE, VPE:RSVK-AA$^{17}$-AKV:KEVQV:TVE, APT:RSVK-AA$^{17}$-AKV:KEVQV:VVR, APT:RSVK-AA$^{17}$-AKV:KEVQV:VVK, VPT:RSVK-AA$^{17}$-AKV:KEVQV:VAE, VPT:RSVK-AA$^{17}$-AKV:KEVQV:AVS, TPT:RSVK-AA$^{17}$-AKV:KEVQV:VVD, VPA:RSVK-AA$^{17}$-AKV:KEVQV:VVE, APV:RSVK-AA$^{17}$-AKV:KEVQV:VEE, VPQ:RSVK-AA$^{17}$-AKV:KEVQV:VRS, VSQ:RSVK-AA$^{17}$-AKV:KEVQV:VKS, VPQ:RSVK-AA$^{17}$-AKV:KEVQV:VKS, VPT:RSVK-AA$^{17}$-AKV:KEVQV:QHN, VPT:RSVK-AA$^{17}$-AKV:KEVQV:EHS, TQV:RSVK-AA$^{17}$-AKV:KEVQV:EDH, TQV:RPVQ-AA$^{17}$-RKI:KKATV:EDH, VPT:RPVQ-AA$^{17}$-RKI:KKATV:VVE, VPE:RPVQ-AA$^{17}$-RKI:KKATV:TVE, APT:RPVQ-AA$^{17}$-RKI:KKATV:VVR, APT:RPVQ-AA$^{17}$-RKI:KKATV:VVK, VPT:RPVQ-AA$^{17}$-RKI:KKATV:VAE, VPT:RPVQ-AA$^{17}$-RKI:KKATV:AVS, TPT:RPVQ-AA$^{17}$-RKI:KKATV:VVD, VPA:RPVQ-AA$^{17}$-RKI:KKATV:VVE, APV:RPVQ-AA$^{17}$-RKI:KKATV:VEE, VPQ:RPVQ-AA$^{17}$-RKI:KKATV:VRS, VSQ:RPVQ-AA$^{17}$-RKI:KKATV:VKS, VPQ:RPVQ-AA$^{17}$-RKI:KKATV:VKS, VPT:RPVQ-AA$^{17}$-RKI:KKATV:QHN, VPT:RPVQ-AA$^{17}$-RKI:KKATV:EHS and SRV:RPVQ-AA17-RKI:KKATV:EEH; and wherein AA$^{17}$ is selected from the group consisting of G, A, V, L, I, P, F, M, W, T and S (in particular is selected from the group consisting of M, I, L, V and T).

In particular, in certain embodiments, the quadruplet PEP5:PEP1:PEP2:PEP6 is selected from the group consisting of VPTKM:SAIS:LKNYQ:NMVVE, VPTEL:SAIS:LKNYQ:EMVVE, VPTKL:SAIS:LKNYQ:NMVVE, VPTQL:SAIS:LKNYQ:NMVVE, VPTKL:SAIS:LKNYQ:EGMSVVE, VPTKL:SAIS:LKNYQ:GMVVE, VPTKM:SAIS:LKNYQ:GMVVE, VPTRL:SAIS:LKNYQ:DMVVE, VPTKT:SAIS:LKNYQ:MIVVE, VPTAL:SAIS:LKNYQ:MIVVE, VPTDL:SAIS:LKNYQ:MIVVE, VPTDL:SAIS:LKNYQ:MVVVE, VPTEE:SAIS:LKNYQ:FLVVE, VPTGQ:SAIS:LKNYQ:LEVVE, VPTHH:SAIS:LKNYQ:RLVVE, VPTQL:SAIS:LKNYQ:TLVVE, VPEKM:SAIS:LKNYQ:NMTVE, APTKL:SAIS:LKNYQ:NMVVR, APTQL:SAIS:LKNYQ:NMVVR, APTKL:SAIS:LKNYQ:NMVVK, VPTKL:SAIS:LKNYQ:EGMSVAE, VPTKL:SAIS:LKNYQ:GMAVS, TPTKM:SAIS:LKNYQ:GMVVD, VPARL:SAIS:LKNYQ:DMVVE, APVKT:SAIS:LKNYQ:MIVEE, VPQAL:SAIS:LKNYQ:MIVRS, VSQDL:SAIS:LKNYQ:MIVKS, VPQDL:SAIS:LKNYQ:MVVKS, VPTEE:SAIS:LKNYQ:FLQHN, VPTGQ:SAIS:LKNYQ:LEEHS, SRVHH:SAIS:LKNYQ:RLEEH, TQVQL:SAIS:LKNYQ:TLEDH, VPEEL:SSLS:LKVYP:DMTVE, VPEEL:SSLS:LKVYP:EMTVE, VPEKL:SSLS:LKVYP:NMTVE, VPEQL:SSLS:LKVYP:NMTVE, VPEKL:SSLS:LKVYP:EGMSTVE, VPEKL:SSLS:LKVYP:GMTVE, VPEKM:SSLS:LKVYP:GMTVE, VPERL:SSLS:LKVYP:DMTVE, VPEKT:SSLS:LKVYP:MITVE, VPEAL:SSLS:LKVYP:MITVE, VPEDL:SSLS:LKVYP:MITVE, VPEDL:SSLS:LKVYP:MVTVE, VPEEE:SSLS:LKVYP:FLTVE, VPEGQ:SSLS:LKVYP:LETVE, VPEHH:SSLS:LKVYP:RLTVE, VPEQL:SSLS:LKVYP:TLTVE, VPTEL:SSLS:LKVYP:DMVVE, VPTEL:SSLS:LKVYP:EMVVE, APTKL:SSLS:LKVYP:NMVVR, APTQL:SSLS:LKVYP:NMVVR, APTKL:SSLS:LKVYP:NMVVK, VPTKL:SSLS:LKVYP:EGMSVAE, VPTKL:SSLS:LKVYP:GMAVS, TPTKM:SSLS:LKVYP:GMVVD, VPARL:SSLS:LKVYP:DMVVE, VPTRL:SSLS:LKVYP:DMVVE, APVKT:SSLS:LKVYP:MIVEE, VPQAL:SSLS:LKVYP:MIVRS, VSQDL:SSLS:LKVYP:MIVKS, VPQDL:SSLS:LKVYP:MVVKS, VPTEE:SSLS:LKVYP:FLQHN, VPTGQ:SSLS:LKVYP:LEEHS, SRVHH:SSLS:LKVYP:RLEEH, TQVQL:SSLS:LKVYP:TLEDH, VPTEL:SAIS:LKNYQ:DMVVE, APTEL:NAIS:LKKYR:DMVVR, APTKM:NAIS:LKKYR:NMVVR, APTEL:NAIS:LKKYR:EMVVR, APTKL:NAIS:LKKYR:NMVVR, APTQL:NAIS:LKKYR:NMVVR, APTKL:NAIS:LKKYR:EGMSVVR, APTKL:NAIS:LKKYR:GMVVR, APTKM:NAIS:LKKYR:GMVVR, APTRL:NAIS:LKKYR:DMVVR, APTKT:NAIS:LKKYR:MIVVR, APTAL:NAIS:LKKYR:MIVVR, APTDL:NAIS:LKKYR:MIVVR, APTDL:NAIS:LKKYR:MVVVR, APTEE:NAIS:LKKYR:FLVVR, APTGQ:NAIS:LKKYR:LEVVR, APTHH:NAIS:LKKYR:RLVVR, APTQL:NAIS:LKKYR:TLVVR, VPTEL:NAIS:LKKYR:DMVVE, VPEKM:NAIS:LKKYR:NMTVE, VPTEL:NAIS:LKKYR:EMVVE, APTKL:NAIS:LKKYR:NMVVK, VPTKL:NAIS:LKKYR:EGMSVAE, VPTKL:NAIS:LKKYR:GMAVS, TPTKM:NAIS:LKKYR:GMVVD, VPARL:NAIS:LKKYR:DMVVE, VPTRL:NAIS:LKKYR:DMVVE, APVKT:NAIS:LKKYR:MIVEE, VPQAL:NAIS:LKKYR:MIVRS, VSQDL:NAIS:LKKYR:MIVKS, VPQDL:NAIS:LKKYR:MVVKS, VPTEE:NAIS:LKKYR:FLQHN, VPTGQ:NAIS:LKKYR:LEEHS, SRVHH:NAIS:LKKYR:RLEEH,

TQVQL:NAIS:LKKYR:TLEDH, APTEL:SATS:LRKHR:DMVVK, APTKM:SATS:LRKHR:NMVVK, APTEL:SATS:LRKHR:EMVVK, APTKL:SATS:LRKHR:NMVVK, APTQL:SATS:LRKHR:NMVVK, APTKL:SATS:LRKHR:EGMSVVK, APTKL:SATS:LRKHR:GMVVK, APTKM:SATS:LRKHR:GMVVK, APTRL:SATS:LRKHR:DMVVK, APTKT:SATS:LRKHR:MIVVK, APTAL:SATS:LRKHR:MIVVK, APTDL:SATS:LRKHR:MIVVK, APTDL:SATS:LRKHR:MVVVK, APTEE:SATS:LRKHR:FLVVK, APTGQ:SATS:LRKHR:LEVVK, APTHH:SATS:LRKHR:RLVVK, APTQL:SATS:LRKHR:TLVVK, VPTEL:SATS:LRKHR:DMVVE, VPEKM:SATS:LRKHR:NMTVE, VPTEL:SATS:LRKHR:EMVVE, APTKL:SATS:LRKHR:NMVVR, APTQL:SATS:LRKHR:NMVVR, VPTKL:SATS:LRKHR:EGMSVAE, VPTKL:SATS:LRKHR:GMAVS, TPTKM:SATS:LRKHR:GMVVD, VPARL:SATS:LRKHR:DMVVE, VPTRL:SATS:LRKHR:DMVVE, APVKT:SATS:LRKHR:MIVEE, VPQAL:SATS:LRKHR:MIVRS, VSQDL:SATS:LRKHR:MIVKS, VPQDL:SATS:LRKHR:MVVKS, VPTEE:SATS:LRKHR:FLQHN, VPTGQ:SATS:LRKHR:LEEHS, SRVHH:SATS:LRKHR:RLEEH, TQVQL:SATS:LRKHR:TLEDH, VPTEL:SPIS:LKYHY:DMVAE, VPTKM:SPIS:LKYHY:NMVAE, VPTEL:SPIS:LKYHY:EMVAE, VPTKL:SPIS:LKYHY:NMVAE, VPTQL:SPIS:LKYHY:NMVAE, VPTKL:SPIS:LKYHY:GMVAE, VPTKM:SPIS:LKYHY:GMVAE, VPTRL:SPIS:LKYHY:DMVAE, VPTKT:SPIS:LKYHY:MIVAE, VPTAL:SPIS:LKYHY:MIVAE, VPTDL:SPIS:LKYHY:MIVAE, VPTDL:SPIS:LKYHY:MVVAE, VPTEE:SPIS:LKYHY:FLVAE, VPTGQ:SPIS:LKYHY:LEVAE, VPTHH:SPIS:LKYHY:RLVAE, VPTQL:SPIS:LKYHY:TLVAE, VPTEL:SPIS:LKYHY:DMVVE, VPEKM:SPIS:LKYHY:NMTVE, VPTEL:SPIS:LKYHY:EMVVE, APTKL:SPIS:LKYHY:NMVVR, APTQL:SPIS:LKYHY:NMVVR, APTKL:SPIS:LKYHY:NMVVK, VPTKL:SPIS:LKYHY:GMAVS, TPTKM:SPIS:LKYHY:GMVVD, VPARL:SPIS:LKYHY:DMVVE, VPTRL:SPIS:LKYHY:DMVVE, APVKT:SPIS:LKYHY:MIVEE, VPQAL:SPIS:LKYHY:MIVRS, VSQDL:SPIS:LKYHY:MIVKS, VPQDL:SPIS:LKYHY:MVVKS, VPTEE:SPIS:LKYHY:FLQHN, VPTGQ:SPIS:LKYHY:LEEHS, SRVHH:SPIS:LKYHY:RLEEH, TQVQL:SPIS:LKYHY:TLEDH, VPTEL:EPIS:KFKYE:DMAVS, VPTKM:EPIS:KFKYE:NMAVS, VPTEL:EPIS:KFKYE:EMAVS, VPTKL:EPIS:KFKYE:NMAVS, VPTQL:EPIS:KFKYE:NMAVS, VPTKL:EPIS:KFKYE:EGMSAVS, VPTKM:EPIS:KFKYE:GMAVS, VPTRL:EPIS:KFKYE:DMAVS, VPTKT:EPIS:KFKYE:MIAVS, VPTAL:EPIS:KFKYE:MIAVS, VPTDL:EPIS:KFKYE:MIAVS, VPTDL:EPIS:KFKYE:MVAVS, VPTEE:EPIS:KFKYE:FLAVS, VPTGQ:EPIS:KFKYE:LEAVS, VPTHH:EPIS:KFKYE:RLAVS, VPTQL:EPIS:KFKYE:TLAVS, VPTEL:EPIS:KFKYE:DMVVE, VPEKM:EPIS:KFKYE:NMTVE, VPTEL:EPIS:KFKYE:EMVVE, APTKL:EPIS:KFKYE:NMVVR, APTQL:EPIS:KFKYE:NMVVR, APTKL:EPIS:KFKYE:NMVVK, VPTKL:EPIS:KFKYE:EGMSVAE, TPTKM:EPIS:KFKYE:GMVVD, VPARL:EPIS:KFKYE:DMVVE, VPTRL:EPIS:KFKYE:DMVVE, APVKT:EPIS:KFKYE:MIVEE, VPQAL:EPIS:KFKYE:MIVRS, VSQDL:EPIS:KFKYE:MIVKS, VPQDL:EPIS:KFKYE:MVVKS, VPTEE:EPIS:KFKYE:FLQHN, VPTGQ:EPIS:KFKYE:LEEHS, SRVHH:EPIS:KFKYE:RLEEH, TQVQL:EPIS:KFKYE:TLEDH, TPTEL:SPIN:YGKIP:DMVVD, TPTKM:SPIN:YGKIP:NMVVD, TPTEL:SPIN:YGKIP:EMVVD, TPTKL:SPIN:YGKIP:NMVVD, TPTQL:SPIN:YGKIP:NMVVD, TPTKL:SPIN:YGKIP:EGMSVVD, TPTKL:SPIN:YGKIP:GMVVD, TPTRL:SPIN:YGKIP:DMVVD, TPTKT:SPIN:YGKIP:MIVVD, TPTAL:SPIN:YGKIP:MIVVD, TPTDL:SPIN:YGKIP:MIVVD, TPTDL:SPIN:YGKIP:MVVVD, TPTEE:SPIN:YGKIP:FLVVD, TPTGQ:SPIN:YGKIP:LEVVD, TPTHH:SPIN:YGKIP:RLVVD, TPTQL:SPIN:YGKIP:TLVVD, VPTEL:SPIN:YGKIP:DMVVE, VPEKM:SPIN:YGKIP:NMTVE, VPTEL:SPIN:YGKIP:EMVVE, APTKL:SPIN:YGKIP:NMVVR, APTQL:SPIN:YGKIP:NMVVR, APTKL:SPIN:YGKIP:NMVVK, VPTKL:SPIN:YGKIP:EGMSVAE, VPTKL:SPIN:YGKIP:GMAVS, VPARL:SPIN:YGKIP:DMVVE, VPTRL:SPIN:YGKIP:DMVVE, APVKT:SPIN:YGKIP:MIVEE, VPQAL:SPIN:YGKIP:MIVRS, VSQDL:SPIN:YGKIP:MIVKS, VPQDL:SPIN:YGKIP:MVVKS, VPTEE:SPIN:YGKIP:FLQHN, VPTGQ:SPIN:YGKIP:LEEHS, SRVHH:SPIN:YGKIP:RLEEH, TQVQL:SPIN:YGKIP:TLEDH, VPAEL:SPIS:YKQYE:DMVVE, VPAKM:SPIS:YKQYE:NMVVE, VPAEL:SPIS:YKQYE:EMVVE, VPAKL:SPIS:YKQYE:NMVVE, VPAQL:SPIS:YKQYE:NMVVE, VPAKL:SPIS:YKQYE:EGMSVVE, VPAKL:SPIS:YKQYE:GMVVE, VPAKM:SPIS:YKQYE:GMVVE, VPARL:SPIS:YKQYE:DMVVE, VPAKT:SPIS:YKQYE:MIVVE, VPAAL:SPIS:YKQYE:MIVVE, VPADL:SPIS:YKQYE:MIVVE, VPADL:SPIS:YKQYE:MVVVE, VPAEE:SPIS:YKQYE:FLVVE, VPAGQ:SPIS:YKQYE:LEVVE, VPAHH:SPIS:YKQYE:RLVVE, VPAQL:SPIS:YKQYE:TLVVE, VPTEL:SPIS:YKQYE:DMVVE, VPEKM:SPIS:YKQYE:NMTVE, VPTEL:SPIS:YKQYE:EMVVE, APTKL:SPIS:YKQYE:NMVVR, APTQL:SPIS:YKQYE:NMVVR, APTKL:SPIS:YKQYE:NMVVK, VPTKL:SPIS:YKQYE:EGMSVAE, VPTKL:SPIS:YKQYE:GMAVS, TPTKM:SPIS:YKQYE:GMVVD, VPTRL:SPIS:YKQYE:DMVVE, APVKT:SPIS:YKQYE:MIVEE, VPQAL:SPIS:YKQYE:MIVRS, VSQDL:SPIS:YKQYE:MIVKS, VPQDL:SPIS:YKQYE:MVVKS, VPTEE:SPIS:YKQYE:FLQHN, VPTGQ:SPIS:YKQYE:LEEHS, SRVHH:SPIS:YKQYE:RLEEH, TQVQL:SPIS:YKQYE:TLEDH, VPTKM:SPIS:YKQYE:NMVVE, VPTKL:SPIS:YKQYE:NMVVE, VPTQL:SPIS:YKQYE:NMVVE, VPTKL:SPIS:YKQYE:EGMSVVE, VPTKL:SPIS:YKQYE:GMVVE, VPTKM:SPIS:YKQYE:GMVVE, VPTKT:SPIS:YKQYE:MIVVE, VPTAL:SPIS:YKQYE:MIVVE, VPTDL:SPIS:YKQYE:MIVVE, VPTDL:SPIS:YKQYE:MVVVE, VPTEE:SPIS:YKQYE:FLVVE, VPTGQ:SPIS:YKQYE:LEVVE, VPTHH:SPIS:YKQYE:RLVVE, VPTQL:SPIS:YKQYE:TLVVE, APVEL:KPLS:DHHKD:DMVEE, APVKM:KPLS:DHHKD:NMVEE, APVEL:KPLS:DHHKD:EMVEE, APVKL:KPLS:DHHKD:NMVEE,

APVQL:KPLS:DHHKD:NMVEE, APVKL:KPLS:DHHKD:EGMSVEE, APVKL:KPLS:DHHKD:GMVEE, APVKM:KPLS:DHHKD:GMVEE, APVRL:KPLS:DHHKD:DMVEE, APVAL:KPLS:DHHKD:MIVEE, APVDL:KPLS:DHHKD:MIVEE, APVDL:KPLS:DHHKD:MVVEE, APVEE:KPLS:DHHKD:FLVEE, APVGQ:KPLS:DHHKD:LEVEE, APVHH:KPLS:DHHKD:RLVEE, APVQL:KPLS:DHHKD:TLVEE, VPTEL:KPLS:DHHKD:DMVVE, VPEKM:KPLS:DHHKD:NMTVE, VPTEL:KPLS:DHHKD:EMVVE, APTKL:KPLS:DHHKD:NMVVR, APTQL:KPLS:DHHKD:NMVVR, APTKL:KPLS:DHHKD:NMVVK, VPTKL:KPLS:DHHKD:EGMSVAE, VPTKL:KPLS:DHHKD:GMAVS, TPTKM:KPLS:DHHKD:GMVVD, VPARL:KPLS:DHHKD:DMVVE, VPTRL:KPLS:DHHKD:DMVVE, VPQAL:KPLS:DHHKD:MIVRS, VSQDL:KPLS:DHHKD:MIVKS, VPQDL:KPLS:DHHKD:MVVKS, VPTEE:KPLS:DHHKD:FLQHN, VPTGQ:KPLS:DHHKD:LEEHS, SRVHH:KPLS:DHHKD:RLEEH, TQVQL:KPLS:DHHKD:TLEDH, VPQEL:EPLP:EQLSN:DMVRS, VPQKM:EPLP:EQLSN:NMVRS, VPQEL:EPLP:EQLSN:EMVRS, VPQKL:EPLP:EQLSN:NMVRS, VPQQL:EPLP:EQLSN:NMVRS, VPQKL:EPLP:EQLSN:EGMSVRS, VPQKL:EPLP:EQLSN:GMVRS, VPQKM:EPLP:EQLSN:GMVRS, VPQRL:EPLP:EQLSN:DMVRS, VPQKT:EPLP:EQLSN:MIVRS, VPQDL:EPLP:EQLSN:MIVRS, VPQDL:EPLP:EQLSN:MVVRS, VPQEE:EPLP:EQLSN:FLVRS, VPQGQ:EPLP:EQLSN:LEVRS, VPQHH:EPLP:EQLSN:RLVRS, VPQQL:EPLP:EQLSN:TLVRS, VPTEL:EPLP:EQLSN:DMVVE, VPEKM:EPLP:EQLSN:NMTVE, VPTEL:EPLP:EQLSN:EMVVE, APTKL:EPLP:EQLSN:NMVVR, APTQL:EPLP:EQLSN:NMVVR, APTKL:EPLP:EQLSN:NMVVK, VPTKL:EPLP:EQLSN:EGMSVAE, VPTKL:EPLP:EQLSN:GMAVS, TPTKM:EPLP:EQLSN:GMVVD, VPARL:EPLP:EQLSN:DMVVE, VPTRL:EPLP:EQLSN:DMVVE, APVKT:EPLP:EQLSN:MIVEE, VSQDL:EPLP:EQLSN:MIVKS, VPQDL:EPLP:EQLSN:MVVKS, VPTEE:EPLP:EQLSN:FLQHN, VPTGQ:EPLP:EQLSN:LEEHS, SRVHH:EPLP:EQLSN:RLEEH, TQVQL:EPLP:EQLSN:TLEDH, VSQEL:EPLT:EQLSN:DMVKS, VSQKM:EPLT:EQLSN:NMVKS, VSQEL:EPLT:EQLSN:EMVKS, VSQKL:EPLT:EQLSN:NMVKS, VSQQL:EPLT:EQLSN:NMVKS, VSQKL:EPLT:EQLSN:EGMSVKS, VSQKL:EPLT:EQLSN:GMVKS, VSQKM:EPLT:EQLSN:GMVKS, VSQRL:EPLT:EQLSN:DMVKS, VSQKT :EPLT :EQLSN:MIVKS, VSQAL:EPL T:EQLSN:MIVKS, VSQDL:EPLT:EQLSN:MVVKS, VSQEE:EPLT:EQLSN:FLVKS, VSQGQ:EPLT:EQLSN:LEVKS, VSQHH:EPLT:EQLSN:RLVKS, VSQQL:EPLT:EQLSN:TLVKS, VPTEL:EPLT:EQLSN:DMVVE, VPEKM:EPLT:EQLSN:NMTVE, VPTEL:EPLT:EQLSN:EMVVE, APTKL:EPLT:EQLSN:NMVVR, APTQL:EPLT:EQLSN:NMVVR, APTKL:EPLT:EQLSN:NMVVK, VPTKL:EPLT:EQLSN:EGMSVAE, VPTKL:EPLT:EQLSN:GMAVS, TPTKM:EPLT:EQLSN:GMVVD, VPARL:EPLT:EQLSN:DMVVE, VPTRL:EPLT:EQLSN:DMVVE, APVKT :EPLT :EQLSN:MIVEE, VPQAL:EPLT:EQLSN:MIVRS, VPQDL:EPLT:EQLSN:MVVKS, VPTEE:EPLT:EQLSN:FLQHN, VPTGQ:EPLT:EQLSN:LEEHS, SRVHH:EPLT:EQLSN:RLEEH, TQVQL:EPLT:EQLSN:TLEDH, VPQEL:EPLT:EQLSN:DMVKS, VPQKM:EPLT:EQLSN:NMVKS, VPQEL:EPLT:EQLSN:EMVKS, VPQKL:EPLT:EQLSN:NMVKS, VPQQL:EPLT:EQLSN:NMVKS, VPQKL:EPLT:EQLSN:EGMSVKS, VPQKL:EPLT:EQLSN:GMVKS, VPQKM:EPLT:EQLSN:GMVKS, VPQRL:EPLT:EQLSN:DMVKS, VPQKT:EPLT:EQLSN:MIVKS, VPQAL:EPLT:EQLSN:MIVKS, VPQDL:EPLT:EQLSN:MIVKS, VPQEE:EPLT:EQLSN:FLVKS, VPQGQ:EPLT:EQLSN:LEVKS, VPQHH:EPLT:EQLSN:RLVKS, VPQQL:EPLT:EQLSN:TLVKS, VSQDL:EPLT:EQLSN:MIVKS, VPTEL:SNIT:IGEMS:DMQHN, VPTKM:SNIT:IGEMS:NMQHN, VPTEL:SNIT:IGEMS:EMQHN, VPTKL:SNIT:IGEMS:NMQHN, VPTQL:SNIT:IGEMS:NMQHN, VPTKL:SNIT:IGEMS:EGMSQHN, VPTKL:SNIT:IGEMS:GMQHN, VPTKM:SNIT:IGEMS:GMQHN, VPTRL:SNIT:IGEMS:DMQHN, VPTKT:SNIT:IGEMS:MIQHN, VPTAL:SNIT:IGEMS:MIQHN, VPTDL:SNIT:IGEMS:MIQHN, VPTDL:SNIT:IGEMS:MVQHN, VPTGQ:SNIT:IGEMS:LEQHN, VPTEE:SNIT:IGEMS:FLQHN, VPTHH:SNIT:IGEMS:RLQHN, VPTQL:SNIT:IGEMS:TLQHN, VPTEL:SNIT:IGEMS:DMVVE, VPEKM:SNIT:IGEMS:NMTVE, VPTEL:SNIT:IGEMS:EMVVE, APTKL:SNIT:IGEMS:NMVVR, APTQL:SNIT:IGEMS:NMVVR, APTKL:SNIT: IGEMS:NMVVK, VPTKL:SNIT: IGEMS:EGMSVAE, VPTKL:SNIT:IGEMS:GMAVS, TPTKM:SNIT:IGEMS:GMVVD, VPARL:SNIT: IGEMS:DMVVE, VPTRL:SNIT: IGEMS:DMVVE, APVKT:SNIT:IGEMS:MIVEE, VPQAL:SNIT:IGEMS:MIVRS, VSQDL:SNIT:IGEMS:MIVKS, VPQDL:SNIT:IGEMS:MVVKS, VPTGQ:SNIT:IGEMS:LEEHS, SRVHH:SNIT:IGEMS:RLEEH, TQVQL:SNIT:IGEMS:TLEDH, VPTEL:SNIT:LGEMS:DMEHS, VPTKM:SNIT:LGEMS:NMEHS, VPTEL:SNIT:LGEMS:EMEHS, VPTKL:SNIT:LGEMS:NMEHS, VPTQL:SNIT:LGEMS:NMEHS, VPTKL:SNIT:LGEMS:EGMSEHS, VPTKL:SNIT:LGEMS:GMEHS, VPTKM:SNIT:LGEMS:GMEHS, VPTRL:SNIT:LGEMS:DMEHS, VPTKT:SNIT:LGEMS:MIEHS, VPTAL:SNIT:LGEMS:MIEHS, VPTDL:SNIT:LGEMS:MIEHS, VPTDL:SNIT:LGEMS:MVEHS, VPTEE:SNIT:LGEMS:FLEHS, VPTHH:SNIT:LGEMS:RLEHS, VPTQL:SNIT:LGEMS:TLEHS, VPTEL:SNIT:LGEMS:DMVVE, VPEKM:SNIT:LGEMS:NMTVE, VPTEL:SNIT:LGEMS:EMVVE, APTKL:SNIT:LGEMS:NMVVR, APTQL:SNIT:LGEMS:NMVVR, APTKL:SNIT:LGEMS:NMVVK, VPTKL:SNIT:LGEMS:EGMSVAE, VPTKL:SNIT:LGEMS:GMAVS, TPTKM:SNIT:LGEMS:GMVVD, VPARL:SNIT:LGEMS:DMVVE, VPTRL:SNIT:LGEMS:DMVVE, APVKT:SNIT:LGEMS:MIVEE, VPQAL:SNIT:LGEMS:MIVRS, VSQDL:SNIT:LGEMS:MIVKS, VPQDL:SNIT:LGEMS:MVVKS, VPTEE:SNIT:LGEMS:FLQHN, SRVHH:SNIT:LGEMS:RLEEH, TQVQL:SNIT:LGEMS:TLEDH, SRVEL:RSVK:KEVQV:DMEEH, SRVKM:RSVK:KEVQV:NMEEH, SRVEL:RSVK:KEVQV:EMEEH, SRVKL:RSVK:KEVQV:NMEEH, SRVQL:RSVK:KEVQV:NMEEH, SRVKL:RSVK:KEVQV:EGMSEEH, SRVKL:RSVK:KEVQV:GMEEH, SRVKM:RSVK:KEVQV:GMEEH, SRVRL:RSVK:KEVQV:DMEEH, SRVKT:RSVK:KEVQV:MIEEH, SRVAL:RSVK:KEVQV:MIEEH, SRVDL:RSVK:KEVQV:MIEEH, SRVDL:RSVK:KEVQV:MVEEH, SRVEE:RSVK:KEVQV:FLEEH, SRVGQ:RSVK:KEVQV:LEEEH, SRVQL:RSVK:KEVQV:TLEEH, VPTEL:RSVK:KEVQV:DMVVE, VPEKM:RSVK:KEVQV:NMTVE, VPTEL:RSVK:KEVQV:EMVVE, APTKL:RSVK:KEVQV:NMVVR, APTQL:RSVK:KEVQV:NMVVR, APTKL:RSVK:KEVQV:NMVVK, VPTKL:RSVK:KEVQV:EGMSVAE, VPTKL:RSVK:KEVQV:GMAVS, TPTKM:RSVK:KEVQV:GMVVD, VPARL:RSVK:KEVQV:DMVVE, VPTRL:RSVK:KEVQV:DMVVE, APVKT:RSVK:KEVQV:MIVEE, VPQAL:RSVK:KEVQV:MIVRS, VSQDL:RSVK:KEVQV:MIVKS, VPQDL:RSVK:KEVQV:MVVKS, VPTEE:RSVK:KEVQV:FLQHN, VPTGQ:RSVK:KEVQV:LEEHS, TQVQL:RSVK:KEVQV:TLEDH, TQVEL:RPVQ:KKATV:DMEDH, TQVKM:RPVQ:KKATV:NMEDH, TQVEL:RPVQ:KKATV:EMEDH, TQVKL:RPVQ:KKATV:NMEDH, TQVQL:RPVQ:KKATV:NMEDH, TQVKL:RPVQ:KKATV:EGMSEDH, TQVKL:RPVQ:KKATV:GMEDH, TQVKM:RPVQ:KKATV:GMEDH, TQVRL:RPVQ:KKATV:DMEDH, TQVKT:RPVQ:KKATV:MIEDH, TQVAL:RPVQ:KKATV:MIEDH, TQVDL:RPVQ:KKATV:MIEDH, TQVDL:RPVQ:KKATV:MVEDH, TQVEE:RPVQ:KKATV:FLEDH, TQVGQ:RPVQ:KKATV:LEEDH, TQVHH:RPVQ:KKATV:RLEDH, VPTEL:RPVQ:KKATV:DMVVE, VPEKM:RPVQ:KKATV:NMTVE, VPTEL:RPVQ:KKATV:EMVVE, APTKL:RPVQ:KKATV:NMVVR, APTQL:RPVQ:KKATV:NMVVR, APTKL:RPVQ:KKATV:NMVVK, VPTKL:RPVQ:KKATV:EGMSVAE, VPTKL:RPVQ:KKATV:GMAVS, TPTKM:RPVQ:KKATV:GMVVD, VPARL:RPVQ:KKATV:DMVVE, VPTRL:RPVQ:KKATV:DMVVE, APVKT:RPVQ:KKATV:MIVEE, VPQAL:RPVQ:KKATV:MIVRS, VSQDL:RPVQ:KKATV:MIVKS, VPQDL:RPVQ:KKATV:MVVKS, VPTEE:RPVQ:KKATV:FLQHN, VPTGQ:RPVQ:KKATV:LEEHS and SRVHH:RPVQ:KKATV:RLEEH. More particularly, the quadruplet PEP5:PEP1:PEP2:PEP6 is selected from the group consisting of VPTKM:SAIS:LKNYQ:NMVVE, VPTKL:SAIS:LKNYQ:NMVVE, VPTQL:SAIS:LKNYQ:NMVVE, VPTKL:SAIS:LKNYQ:EGMSVVE, VPTKL:SAIS:LKNYQ:GMVVE, VPTKM:SAIS:LKNYQ:GMVVE, VPTRL:SAIS:LKNYQ:DMVVE, VPTKT:SAIS:LKNYQ:MIVVE, VPTAL:SAIS:LKNYQ:MIVVE, VPTDL:SAIS:LKNYQ:MIVVE, VPTDL:SAIS:LKNYQ:MVVVE, VPEKM:SAIS:LKNYQ:NMTVE, APTKL:SAIS:LKNYQ:NMVVR, APTQL:SAIS:LKNYQ:NMVVR, APTKL:SAIS:LKNYQ:NMVVK, VPTKL:SAIS:LKNYQ:EGMSVAE, VPTKL:SAIS:LKNYQ:GMAVS, TPTKM:SAIS:LKNYQ:GMVVD, VPARL:SAIS:LKNYQ:DMVVE, APVKT:SAIS:LKNYQ:MIVEE, VPQAL:SAIS:LKNYQ:MIVRS, VSQDL:SAIS:LKNYQ:MIVKS, VPQDL:SAIS:LKNYQ:MVVKS, VPTEE:SAIS:LKNYQ:FLQHN, VPTGQ:SAIS:LKNYQ:LEEHS, SRVHH:SAIS:LKNYQ:RLEEH, TQVQL:SAIS:LKNYQ:TLEDH, VPEEL:SSLS:LKVYP:DMTVE, VPEEL:SSLS:LKVYP:EMTVE, VPEKL:SSLS:LKVYP:NMTVE, VPEQL:SSLS:LKVYP:NMTVE, VPEKL:SSLS:LKVYP:EGMSTVE, VPEKL:SSLS:LKVYP:GMTVE, VPEKM:SSLS:LKVYP:GMTVE, VPERL:SSLS:LKVYP:DMTVE, VPEKT:SSLS:LKVYP:MITVE, VPEAL:SSLS:LKVYP:MITVE, VPEDL:SSLS:LKVYP:MITVE, VPEDL:SSLS:LKVYP:MVTVE, VPTEL:SSLS:LKVYP:DMVVE, VPTEL:SSLS:LKVYP:EMVVE, APTKL:SSLS:LKVYP:NMVVR, APTQL:SSLS:LKVYP:NMVVR, APTKL:SSLS:LKVYP:NMVVK, VPTKL:SSLS:LKVYP:EGMSVAE, VPTKL:SSLS:LKVYP:GMAVS, TPTKM:SSLS:LKVYP:GMVVD, VPARL:SSLS:LKVYP:DMVVE, VPTRL:SSLS:LKVYP:DMVVE, APVKT:SSLS:LKVYP:MIVEE, VPQAL:SSLS:LKVYP:MIVRS, VSQDL:SSLS:LKVYP:MIVKS, VPQDL:SSLS:LKVYP:MVVKS, VPTEE:SSLS:LKVYP:FLQHN, VPTGQ:SSLS:LKVYP:LEEHS, SRVHH:SSLS:LKVYP:RLEEH, TQVQL:SSLS:LKVYP:TLEDH, APTEL:NAIS:LKKYR:DMVVR, APTKM:NAIS:LKKYR:NMVVR, APTEL:NAIS:LKKYR:EMVVR, APTKL:NAIS:LKKYR:NMVVR, APTKL:NAIS:LKKYR:EGMSVVR, APTKL:NAIS:LKKYR:GMVVR, APTKM:NAIS:LKKYR:GMVVR, APTRL:NAIS:LKKYR:DMVVR, APTKT:NAIS:LKKYR:MIVVR, APTAL:NAIS:LKKYR:MIVVR, APTDL:NAIS:LKKYR:MIVVR, APTDL:NAIS:LKKYR:MVVVR, VPTEL:NAIS:LKKYR:DMVVE, VPEKM:NAIS:LKKYR:NMTVE, VPTEL:NAIS:LKKYR:EMVVE, APTKL:NAIS:LKKYR:NMVVK, VPTKL:NAIS:LKKYR:EGMSVAE, VPTKL:NAIS:LKKYR:GMAVS, TPTKM:NAIS:LKKYR:GMVVD, VPARL:NAIS:LKKYR:DMVVE, VPTRL:NAIS:LKKYR:DMVVE, APVKT:NAIS:LKKYR:MIVEE, VPQAL:NAIS:LKKYR:MIVRS, VSQDL:NAIS:LKKYR:MIVKS, VPQDL:NAIS:LKKYR:MVVKS, VPTEE:NAIS:LKKYR:FLQHN, VPTGQ:NAIS:LKKYR:LEEHS, SRVHH:NAIS:LKKYR:RLEEH, TQVQL:NAIS:LKKYR:TLEDH, APTEL:SATS:LRKHR:DMVVK, APTKM:SATS:LRKHR:NMVVK, APTEL:SATS:LRKHR:EMVVK, APTKL:SATS:LRKHR:NMVVK, APTQL:SATS:LRKHR:NMVVK, APTKL:SATS:LRKHR:EGMSVVK, APTKL:SATS:LRKHR:GMVVK, APTKM:SATS:LRKHR:GMVVK, APTRL:SATS:LRKHR:DMVVK, APTKT:SATS:LRKHR:MIVVK, APTAL:SATS:LRKHR:MIVVK, APTDL:SATS:LRKHR:MIVVK, APTDL:SATS:LRKHR:MVVVK, VPTEL:SATS:LRKHR:DMVVE, VPEKM:SATS:LRKHR:NMTVE, VPTEL:SATS:LRKHR:EMVVE, APTKL:SATS:LRKHR:NMVVR, APTQL:SATS:LRKHR:NMVVR, VPTKL:SATS:LRKHR:EGMSVAE, VPTKL:SATS:LRKHR:GMAVS, TPTKM:SATS:LRKHR:GMVVD, VPARL:SATS:LRKHR:DMVVE, VPTRL:SATS:LRKHR:DMVVE, APVKT:SATS:LRKHR:MIVEE, VPQAL:SATS:LRKHR:MIVRS, VSQDL:SATS:LRKHR:MIVKS, VPQDL:SATS:LRKHR:MVVKS, VPTEE:SATS:LRKHR:FLQHN, VPTGQ:SATS:LRKHR:LEEHS, SRVHH:SATS:LRKHR:RLEEH, TQVQL:SATS:LRKHR:TLEDH, VPTEL:SPIS:LKYHY:DMVAE, VPTKM:SPIS:LKYHY:NMVAE, VPTEL:SPIS:LKYHY:EMVAE, VPTKL:SPIS:LKYHY:NMVAE, VPTQL:SPIS:LKYHY:NMVAE, VPTKL:SPIS:LKYHY:GMVAE, VPTKM:SPIS:LKYHY:GMVAE, VPTRL:SPIS:LKYHY:DMVAE, VPTKT:SPIS:LKYHY:MIVAE, VPTAL:SPIS:LKYHY:MIVAE, VPTDL:SPIS:LKYHY:MIVAE, VPTDL:SPIS:LKYHY:MVVAE, VPTEL:SPIS:LKYHY:DMVVE, VPEKM:SPIS:LKYHY:NMTVE, VPTEL:SPIS:LKYHY:EMVVE, APTKL:SPIS:LKYHY:NMVVR, APTQL:SPIS:LKYHY:NMVVR, APTKL:SPIS:LKYHY:NMVVK, VPTKL:SPIS:LKYHY:GMAVS, TPTKM:SPIS:LKYHY:GMVVD, VPARL:SPIS:LKYHY:DMVVE, VPTRL:SPIS:LKYHY:DMVVE, APVKT:SPIS:LKYHY:MIVEE, VPQAL:SPIS:LKYHY:MIVRS, VSQDL:SPIS:LKYHY:MIVKS, VPQDL:SPIS:LKYHY:MVVKS, VPTEE:SPIS:LKYHY:FLQHN, VPTGQ:SPIS:LKYHY:LEEHS, SRVHH:SPIS:LKYHY:RLEEH, TQVQL:SPIS:LKYHY:TLEDH, VPTEL:EPIS:KFKYE:DMAVS, VPTKM:EPIS:KFKYE:NMAVS, VPTEL:EPIS:KFKYE:EMAVS, VPTKL:EPIS:KFKYE:NMAVS, VPTQL:EPIS:KFKYE:NMAVS, VPTKL:EPIS:KFKYE:EGMSAVS, VPTKM:EPIS:KFKYE:GMAVS, VPTRL:EPIS:KFKYE:DMAVS, VPTKT:EPIS:KFKYE:MIAVS, VPTAL:EPIS:KFKYE:MIAVS, VPTDL:EPIS:KFKYE:MIAVS, VPTDL:EPIS:KFKYE:MVAVS, VPTEL:EPIS:KFKYE:DMVVE, VPEKM:EPIS:KFKYE:NMTVE, VPTEL:EPIS:KFKYE:EMVVE, APTKL:EPIS:KFKYE:NMVVR, APTQL:EPIS:KFKYE:NMVVR, APTKL:EPIS:KFKYE:NMVVK, VPTKL:EPIS:KFKYE:EGMSVAE, TPTKM:EPIS:KFKYE:GMVVD, VPARL:EPIS:KFKYE:DMVVE, VPTRL:EPIS:KFKYE:DMVVE, APVKT:EPIS:KFKYE:MIVEE, VPQAL:EPIS:KFKYE:MIVRS, VSQDL:EPIS:KFKYE:MIVKS, VPQDL:EPIS:KFKYE:MVVKS, VPTEE:EPIS:KFKYE:FLQHN, VPTGQ:EPIS:KFKYE:LEEHS, SRVHH:EPIS:KFKYE:RLEEH, TQVQL:EPIS:KFKYE:TLEDH, TPTEL:SPIN:YGKIP:DMVVD, TPTKM:SPIN:YGKIP:NMVVD, TPTEL:SPIN:YGKIP:EMVVD, TPTKL:SPIN:YGKIP:NMVVD, TPTQL:SPIN:YGKIP:NMVVD, TPTKL:SPIN:YGKIP:EGMSVVD, TPTKL:SPIN:YGKIP:GMVVD, TPTRL:SPIN:YGKIP:DMVVD, TPTKT:SPIN:YGKIP:MIVVD, TPTAL:SPIN:YGKIP:MIVVD, TPTDL:SPIN:YGKIP:MIVVD, TPTDL:SPIN:YGKIP:MVVVD, VPTEL:SPIN:YGKIP:DMVVE, VPEKM:SPIN:YGKIP:NMTVE, VPTEL:SPIN:YGKIP:EMVVE, APTKL:SPIN:YGKIP:NMVVR, APTQL:SPIN:YGKIP:NMVVR, APTKL:SPIN:YGKIP:NMVVK, VPTKL:SPIN:YGKIP:EGMSVAE, VPTKL:SPIN:YGKIP:GMAVS, VPARL:SPIN:YGKIP:DMVVE, VPTRL:SPIN:YGKIP:DMVVE, APVKT:SPIN:YGKIP:MIVEE, VPQAL:SPIN:YGKIP:MIVRS, VSQDL:SPIN:YGKIP:MIVKS, VPQDL:SPIN:YGKIP:MVVKS, VPTEE:SPIN:YGKIP:FLQHN, VPTGQ:SPIN:YGKIP:LEEHS, SRVHH:SPIN:YGKIP:RLEEH, TQVQL:SPIN:YGKIP:TLEDH, VPAEL:SPIS:YKQYE:DMVVE, VPAKM:SPIS:YKQYE:NMVVE, VPAEL:SPIS:YKQYE:EMVVE, VPAKL:SPIS:YKQYE:NMVVE, VPAQL:SPIS:YKQYE:NMVVE, VPAKL:SPIS:YKQYE:EGMSVVE, VPAKL:SPIS:YKQYE:GMVVE, VPAKM:SPIS:YKQYE:GMVVE, VPARL:SPIS:YKQYE:DMVVE, VPAKT:SPIS:YKQYE:MIVVE, VPAAL:SPIS:YKQYE:MIVVE, VPADL:SPIS:YKQYE:MIVVE, VPADL:SPIS:YKQYE:MVVVE, VPTEL:SPIS:YKQYE:DMVVE, VPEKM:SPIS:YKQYE:NMTVE, VPTEL:SPIS:YKQYE:EMVVE, APTKL:SPIS:YKQYE:NMVVR, APTQL:SPIS:YKQYE:NMVVR, APTKL:SPIS:YKQYE:NMVVK, VPTKL:SPIS:YKQYE:EGMSVAE, VPTKL:SPIS:YKQYE:GMAVS, TPTKM:SPIS:YKQYE:GMVVD, VPTRL:SPIS:YKQYE:DMVVE, APVKT:SPIS:YKQYE:MIVEE, VPQAL:SPIS:YKQYE:MIVRS, VSQDL:SPIS:YKQYE:MIVKS, VPQDL:SPIS:YKQYE:MVVKS, VPTEE:SPIS:YKQYE:FLQHN, VPTGQ:SPIS:YKQYE:LEEHS, SRVHH:SPIS:YKQYE:RLEEH, TQVQL:SPIS:YKQYE:TLEDH, VPTKM:SPIS:YKQYE:NMVVE, VPTKL:SPIS:YKQYE:NMVVE, VPTQL:SPIS:YKQYE:NMVVE, VPTKL:SPIS:YKQYE:EGMSVVE, VPTKL:SPIS:YKQYE:GMVVE, VPTKM:SPIS:YKQYE:GMVVE, VPTKT:SPIS:YKQYE:MIVVE, VPTAL:SPIS:YKQYE:MIVVE, VPTDL:SPIS:YKQYE:MIVVE, VPTDL:SPIS:YKQYE:MVVVE, APVEL:KPLS:DHHKD:DMVEE, APVKM:KPLS:DHHKD:NMVEE, APVEL:KPLS:DHHKD:EMVEE, APVKL:KPLS:DHHKD:NMVEE, APVQL:KPLS:DHHKD:NMVEE, APVKL:KPLS:DHHKD:EGMSVEE, APVKL:KPLS:DHHKD:GMVEE, APVKM:KPLS:DHHKD:GMVEE, APVRL:KPLS:DHHKD:DMVEE, APVAL:KPLS:DHHKD:MIVEE, APVDL:KPLS:DHHKD:MIVEE, APVDL:KPLS:DHHKD:MVVEE, VPTEL:KPLS:DHHKD:DMVVE, VPEKM:KPLS:DHHKD:NMTVE, VPTEL:KPLS:DHHKD:EMVVE, APTKL:KPLS:DHHKD:NMVVR, APTQL:KPLS:DHHKD:NMVVR, APTKL:KPLS:DHHKD:NMVVK, VPTKL:KPLS:DHHKD:EGMSVAE, VPTKL:KPLS:DHHKD:GMAVS, TPTKM:KPLS:DHHKD:GMVVD, VPARL:KPLS:DHHKD:DMVVE, VPTRL:KPLS:DHHKD:DMVVE, VPQAL:KPLS:DHHKD:MIVRS, VSQDL:KPLS:DHHKD:MIVKS, VPQDL:KPLS:DHHKD:MVVKS, VPTEE:KPLS:DHHKD:FLQHN, VPTGQ:KPLS:DHHKD:LEEHS, SRVHH:KPLS:DHHKD:RLEEH, TQVQL:KPLS:DHHKD:TLEDH, VPQEL:EPLP:EQLSN:DMVRS, VPQKM:EPLP:EQLSN:NMVRS, VPQEL:EPLP:EQLSN:EMVRS, VPQKL:EPLP:EQLSN:NMVRS, VPQQL:EPLP:EQLSN:NMVRS, VPQKL:EPLP:EQLSN:EGMSVRS, VPQKL:EPLP:EQLSN:GMVRS, VPQKM:EPLP:EQLSN:GMVRS, VPQRL:EPLP:EQLSN:DMVRS, VPQKT:EPLP:EQLSN:MIVRS, VPQDL:EPLP:EQLSN:MIVRS, VPQDL:EPLP:EQLSN:MVVRS, VPTEL:EPLP:EQLSN:DMVVE, VPEKM:EPLP:EQLSN:NMTVE, VPTEL:EPLP:EQLSN:EMVVE, APTKL:EPLP:EQLSN:NMVVR, APTQL:EPLP:EQLSN:NMVVR, APTKL:EPLP:EQLSN:NMVVK, VPTKL:EPLP:EQLSN:EGMSVAE, VPTKL:EPLP:EQLSN:GMAVS, TPTKM:EPLP:EQLSN:GMVVD, VPARL:EPLP:EQLSN:DMVVE, VPTRL:EPLP:EQLSN:DMVVE, APVKT:EPLP:EQLSN:MIVEE, VSQDL:EPLP:EQLSN:MIVKS, VPQDL:EPLP:EQLSN:MVVKS, VPTEE:EPLP:EQLSN:FLQHN, VPTGQ:EPLP:EQLSN:LEEHS, SRVHH:EPLP:EQLSN:RLEEH, TQVQL:EPLP:EQLSN:TLEDH, VSQEL:EPLT:EQLSN:DMVKS, VSQKM:EPLT:EQLSN:NMVKS, VSQEL:EPLT:EQLSN:EMVKS, VSQKL:EPLT:EQLSN:NMVKS, VSQQL:EPLT:EQLSN:NMVKS, VSQKL:EPLT:EQLSN:EGMSVKS, VSQKL:EPLT:EQLSN:GMVKS, VSQKM:EPLT:EQLSN:GMVKS, VSQRL:EPLT:EQLSN:DMVKS, VSQKT:EPLT:EQLSN:MIVKS, VSQAL:EPLT:EQLSN:MIVKS, VSQDL:EPLT:EQLSN:MVVKS, VPTEL:EPLT:EQLSN:DMVVE, VPEKM:EPLT:EQLSN:NMTVE, VPTEL:EPLT:EQLSN:EMVVE, APTKL:EPLT:EQLSN:NMVVR, APTQL:EPLT:EQLSN:NMVVR, APTKL:EPLT:EQLSN:NMVVK, VPTKL:EPLT:EQLSN:EGMSVAE, VPTKL:EPLT:EQLSN:GMAVS, TPTKM:EPLT:EQLSN:GMVVD, VPARL:EPLT:EQLSN:DMVVE, VPTRL:EPLT:EQLSN:DMVVE, APVKT:EPLT:EQLSN:MIVEE, VPQAL:EPLT:EQLSN:MIVRS, VPTEE:EPLT:EQLSN:FLQHN, VPTGQ:EPLT:EQLSN:LEEHS, SRVHH:EPLT:EQLSN:RLEEH, TQVQL:EPLT:EQLSN:TLEDH, VPQEL:EPLT:EQLSN:DMVKS, VPQKM:EPLT:EQLSN:NMVKS, VPQEL:EPLT:EQLSN:EMVKS, VPQKL:EPLT:EQLSN:NMVKS, VPQQL:EPLT:EQLSN:NMVKS, VPQKL:EPLT:EQLSN:EGMSVKS, VPQKL:EPLT:EQLSN:GMVKS, VPQKM:EPLT:EQLSN:GMVKS, VPQRL:EPLT:EQLSN:DMVKS, VPQKT:EPLT:EQLSN:MIVKS, VPQAL:EPLT:EQLSN:MIVKS, VPQDL:EPLT:EQLSN:MIVKS, VPTGQ:SNIT:IGEMS:LEQHN, VPTEL:SNIT:IGEMS:DMVVE, VPEKM:SNIT:IGEMS:NMTVE, VPTEL:SNIT:IGEMS:EMVVE, APTKL:SNIT:IGEMS:NMVVR, APTQL:SNIT:IGEMS:NMVVR, APTKL:SNIT:IGEMS:NMVVK, VPTKL:SNIT:IGEMS:EGMSVAE, VPTKL:SNIT:IGEMS:GMAVS, TPTKM:SNIT:IGEMS:GMVVD, VPARL:SNIT:IGEMS:DMVVE, VPTRL:SNIT:IGEMS:DMVVE, APVKT:SNIT:IGEMS:MIVEE, VPQAL:SNIT:IGEMS:MIVRS, VSQDL:SNIT:IGEMS:MIVKS, VPQDL:SNIT:IGEMS:MVVKS, VPTGQ:SNIT:IGEMS:LEEHS, SRVHH:SNIT:IGEMS:RLEEH, TQVQL:SNIT:IGEMS:TLEDH, VPTEE:SNIT:LGEMS:FLEHS, VPTEL:SNIT:LGEMS:DMVVE, VPEKM:SNIT:LGEMS:NMTVE, VPTEL:SNIT:LGEMS:EMVVE, APTKL:SNIT:LGEMS:NMVVR, APTQL:SNIT:LGEMS:NMVVR, APTKL:SNIT:LGEMS:NMVVK, VPTKL:SNIT:LGEMS:EGMSVAE, VPTKL:SNIT:LGEMS:GMAVS, TPTKM:SNIT:LGEMS:GMVVD, VPARL:SNIT:LGEMS:DMVVE, VPTRL:SNIT:LGEMS:DMVVE, APVKT:SNIT:LGEMS:MIVEE, VPQAL:SNIT:LGEMS:MIVRS, VSQDL:SNIT:LGEMS:MIVKS, VPQDL:SNIT:LGEMS:MVVKS, VPTEE:SNIT:LGEMS:FLQHN, SRVHH:SNIT:LGEMS:RLEEH, TQVQL:SNIT:LGEMS:TLEDH, SRVQL:RSVK:KEVQV:TLEEH, VPTEL:RSVK:KEVQV:DMVVE, VPEKM:RSVK:KEVQV:NMTVE, VPTEL:RSVK:KEVQV:EMVVE, APTKL:RSVK:KEVQV:NMVVR, APTQL:RSVK:KEVQV:NMVVR, APTKL:RSVK:KEVQV:NMVVK, VPTKL:RSVK:KEVQV:EGMSVAE, VPTKL:RSVK:KEVQV:GMAVS, TPTKM:RSVK:KEVQV:GMVVD, VPARL:RSVK:KEVQV:DMVVE, VPTRL:RSVK:KEVQV:DMVVE, APVKT:RSVK:KEVQV:MIVEE, VPQAL:RSVK:KEVQV:MIVRS, VSQDL:RSVK:KEVQV:MIVKS, VPQDL:RSVK:KEVQV:MVVKS, VPTEE:RSVK:KEVQV:FLQHN, VPTGQ:RSVK:KEVQV:LEEHS, TQVQL:RSVK:KEVQV:TLEDH, TQVHH:RPVQ:KKATV:RLEDH, VPTEL:RPVQ:KKATV:DMVVE, VPEKM:RPVQ:KKATV:NMTVE, VPTEL:RPVQ:KKATV:EMVVE, APTKL:RPVQ:KKATV:NMVVR, APTQL:RPVQ:KKATV:NMVVR, APTKL:RPVQ:KKATV:NMVVK, VPTKL:RPVQ:KKATV:EGMSVAE, VPTKL:RPVQ:KKATV:GMAVS, TPTKM:RPVQ:KKATV:GMVVD, VPARL:RPVQ:KKATV:DMVVE, VPTRL:RPVQ:KKATV:DMVVE, APVKT:RPVQ:KKATV:MIVEE, VPQAL:RPVQ:KKATV:MIVRS, VSQDL:RPVQ:KKATV:MIVKS, VPQDL:RPVQ:KKATV:MVVKS, VPTEE:RPVQ:KKATV:FLQHN, VPTGQ:RPVQ:KKATV:LEEHS and SRVHH:RPVQ:KKATV:RLEEH.

In particular, in certain embodiments, the quadruplet PEP5:PEP12:PEP2:PEP6 is selected from the group consisting of VPTKM:SAIS-AA$^{17}$-LYL:LKNYQ:NMVVE, VPTEL:SAIS-AA$^{17}$-LYL:LKNYQ:EMVVE, VPTKL:SAIS-AA$^{17}$-LYL:LKNYQ:NMVVE, VPTQL:SAIS-AA$^{17}$-LYL:LKNYQ:NMVVE, VPTKL:SAIS-AA$^{17}$-LYL:LKNYQ:EGMSVVE, VPTKL:SAIS-AA$^{17}$-LYL:LKNYQ:GMVVE, VPTKM:SAIS-AA$^{17}$-LYL:LKNYQ:GMVVE, VPTRL:SAIS-AA$^{17}$-LYL:LKNYQ:DMVVE, VPTKT:SAIS-AA$^{17}$-LYL:LKNYQ:MIVVE, VPTAL:SAIS-AA$^{17}$-LYL:LKNYQ:MIVVE, VPTDL:SAIS-AA$^{17}$-LYL:LKNYQ:MIVVE, VPTDL:SAIS-AA$^{17}$-LYL:LKNYQ:MVVVE, VPTEE:SAIS-AA$^{17}$-LYL:LKNYQ:FLVVE, VPTGQ:SAIS-AA$^{17}$-LYL:LKNYQ:LEVVE, VPTHH:SAIS-AA$^{17}$-LYL:LKNYQ:RLVVE, VPTQL:SAIS-AA$^{17}$-LYL:LKNYQ:TLVVE, VPEKM:SAIS-AA$^{17}$-LYL:LKNYQ:NMTVE, APTKL:SAIS-AA$^{17}$-LYL:LKNYQ:NMVVR, APTQL:SAIS-AA$^{17}$-LYL:LKNYQ:NMVVR, APTKL:SAIS-AA$^{17}$-LYL:LKNYQ:NMVVK, VPTKL:SAIS-AA$^{17}$-LYL:LKNYQ:EGMSVAE, VPTKL:SAIS-AA$^{17}$-LYL:LKNYQ:GMAVS, TPTKM:SAIS-AA$^{17}$-LYL:LKNYQ:GMVVD, VPARL:SAIS-AA$^{17}$-LYL:LKNYQ:DMVVE, APVKT:SAIS-AA$^{17}$-LYL:LKNYQ:MIVEE, VPQAL:SAIS-AA$^{17}$-LYL:LKNYQ:MIVRS, VSQDL:SAIS-AA$^{17}$-LYL:LKNYQ:MIVKS, VPQDL:SAIS-AA$^{17}$-LYL:LKNYQ:MVVKS, VPTEE:SAIS-AA$^{17}$-LYL:LKNYQ:FLQHN, VPTGQ:SAIS-AA$^{17}$-LYL:LKNYQ:LEEHS, SRVHH:SAIS-AA$^{17}$-LYL:LKNYQ:RLEEH, TQVQL:SAIS-AA$^{17}$-LYL:LKNYQ:TLEDH, VPEEL:SSLS-AA$^{17}$-LFF:LKVYP:DMTVE, VPEEL:SSLS-AA$^{17}$-LFF:LKVYP:EMTVE, VPEKL:SSLS-AA$^{17}$-LFF:LKVYP:NMTVE, VPEQL:SSLS-AA$^{17}$-LFF:LKVYP:NMTVE, VPEKL:SSLS-AA$^{17}$-LFF:LKVYP:EGMSTVE, VPEKL:SSLS-AA$^{17}$-LFF:LKVYP:GMTVE, VPEKM:SSLS-AA$^{17}$-LFF:LKVYP:GMTVE, VPERL:SSLS-AA$^{17}$-LFF:LKVYP:DMTVE, VPEKT:SSLS-AA$^{17}$-LFF:LKVYP:MITVE, VPEAL:SSLS-AA$^{17}$-LFF:LKVYP:MITVE, VPEDL:SSLS-AA$^{17}$-LFF:LKVYP:MITVE, VPEDL:SSLS-AA$^{17}$-LFF:LKVYP:MVTVE, VPEEE:SSLS-AA$^{17}$-LFF:LKVYP:FLTVE, VPEGQ:SSLS-AA$^{17}$-LFF:LKVYP:LETVE, VPEHH:SSLS-AA$^{17}$-LFF:LKVYP:RLTVE, VPEQL:SSLS-AA$^{17}$-LFF:LKVYP:TLTVE, VPTEL:SSLS-AA$^{17}$-LFF:LKVYP:DMVVE, VPTEL:SSLS-AA$^{17}$-LFF:LKVYP:EMVVE, APTKL:SSLS-AA$^{17}$-LFF:LKVYP:NMVVR, APTQL:SSLS-AA$^{17}$-LFF:LKVYP:NMVVR, APTKL:SSLS-AA$^{17}$-LFF:LKVYP:NMVVK, VPTKL:SSLS-AA$^{17}$-LFF:LKVYP:EGMSVAE, VPTKL:SSLS-AA$^{17}$-LFF:LKVYP:GMAVS, TPTKM:SSLS-AA$^{17}$-LFF:LKVYP:GMVVD, VPARL:SSLS-AA$^{17}$-LFF:LKVYP:DMVVE, VPTRL:SSLS-AA$^{17}$-LFF:LKVYP:DMVVE, APVKT:SSLS-AA$^{17}$-LFF:LKVYP:MIVEE, VPQAL:SSLS-AA$^{17}$-LFF:LKVYP:MIVRS, VSQDL:SSLS-AA$^{17}$-LFF:LKVYP:MIVKS, VPQDL:SSLS-AA$^{17}$-LFF:LKVYP:MVVKS, VPTEE:SSLS-AA$^{17}$-LFF:LKVYP:FLQHN, VPTGQ:SSLS-AA$^{17}$-LFF:LKVYP:LEEHS, SRVHH:SSLS-AA$^{17}$-LFF:LKVYP:RLEEH, TQVQL:SSLS-AA$^{17}$-LFF:LKVYP:TLEDH, VPTEL:SAIS-AA$^{17}$-LYL:LKNYQ:DMVVE, APTEL:NAIS-

AA$^{17}$-LYF:LKKYR:DMVVR, APTKM:NAIS-AA$^{17}$-LYF:LKKYR:NMVVR, APTEL:NAIS-AA$^{17}$-LYF:LKKYR:EMVVR, APTKL:NAIS-AA$^{17}$-LYF:LKKYR:NMVVR, APTQL:NAIS-AA$^{17}$-LYF:LKKYR:NMVVR, APTKL:NAIS-AA$^{17}$-LYF:LKKYR:EGMSVVR, APTKL:NAIS-AA$^{17}$-LYF:LKKYR:GMVVR, APTKM:NAIS-AA$^{17}$-LYF:LKKYR:GMVVR, APTRL:NAIS-AA$^{17}$-LYF:LKKYR:DMVVR, APTKT:NAIS-AA$^{17}$-LYF:LKKYR:MIVVR, APTAL:NAIS-AA$^{17}$-LYF:LKKYR:MIVVR, APTDL:NAIS-AA$^{17}$-LYF:LKKYR:MIVVR, APTDL:NAIS-AA$^{17}$-LYF:LKKYR:MVVVR, APTEE:NAIS-AA$^{17}$-LYF:LKKYR:FLVVR, APTGQ:NAIS-AA$^{17}$-LYF:LKKYR:LEVVR, APTHH:NAIS-AA$^{17}$-LYF:LKKYR:RLVVR, APTQL:NAIS-AA$^{17}$-LYF:LKKYR:TLVVR, VPTEL:NAIS-AA$^{17}$-LYF:LKKYR:DMVVE, VPEKM:NAIS-AA$^{17}$-LYF:LKKYR:NMTVE, VPTEL:NAIS-AA$^{17}$-LYF:LKKYR:EMVVE, APTKL:NAIS-AA$^{17}$-LYF:LKKYR:NMVVK, VPTKL:NAIS-AA$^{17}$-LYF:LKKYR:EGMSVAE, VPTKL:NAIS-AA$^{17}$-LYF:LKKYR:GMAVS, TPTKM:NAIS-AA$^{17}$-LYF:LKKYR:GMVVD, VPARL:NAIS-AA$^{17}$-LYF:LKKYR:DMVVE, VPTRL:NAIS-AA$^{17}$-LYF:LKKYR:DMVVE, APVKT:NAIS-AA$^{17}$-LYF:LKKYR:MIVEE, VPQAL:NAIS-AA$^{17}$-LYF:LKKYR:MIVRS, VSQDL:NAIS-AA$^{17}$-LYF:LKKYR:MIVKS, VPQDL:NAIS-AA$^{17}$-LYF:LKKYR:MVVKS, VPTEE:NAIS-AA$^{17}$-LYF:LKKYR:FLQHN, VPTGQ:NAIS-AA$^{17}$-LYF:LKKYR:LEEHS, SRVHH:NAIS-AA$^{17}$-LYF:LKKYR:RLEEH, TQVQL:NAIS-AA$^{17}$-LYF:LKKYR:TLEDH, APTEL:SATS-AA$^{17}$-LYY:LRKHR:DMVVK, APTKM:SATS-AA$^{17}$-LYY:LRKHR:NMVVK, APTEL:SATS-AA$^{17}$-LYY:LRKHR:EMVVK, APTKL:SATS-AA$^{17}$-LYY:LRKHR:NMVVK, APTQL:SATS-AA$^{17}$-LYY:LRKHR:NMVVK, APTKL:SATS-AA$^{17}$-LYY:LRKHR:EGMSVVK, APTKL:SATS-AA$^{17}$-LYY:LRKHR:GMVVK, APTKM:SATS-AA$^{17}$-LYY:LRKHR:GMVVK, APTRL:SATS-AA$^{17}$-LYY:LRKHR:DMVVK, APTKT:SATS-AA$^{17}$-LYY:LRKHR:MIVVK, APTAL:SATS-AA$^{17}$-LYY:LRKHR:MIVVK, APTDL:SATS-AA$^{17}$-LYY:LRKHR:MIVVK, APTDL:SATS-AA$^{17}$-LYY:LRKHR:MVVVK, APTEE:SATS-AA$^{17}$-LYY:LRKHR:FLVVK, APTGQ:SATS-AA$^{17}$-LYY:LRKHR:LEVVK, APTHH:SATS-AA$^{17}$-LYY:LRKHR:RLVVK, APTQL:SATS-AA$^{17}$-LYY:LRKHR:TLVVK, VPTEL:SATS-AA $^{17}$-LYY:LRKHR:DMVVE, VPEKM:SATS-AA$^{17}$-LYY:LRKHR:NMTVE, VPTEL:SATS-AA$^{17}$-LYY:LRKHR:EMVVE, APTKL:SATS-AA$^{17}$-LYY:LRKHR:NMVVR, APTQL:SATS-AA $^{17}$-LYY:LRKHR:NMVVR, VPTKL:SATS-AA$^{17}$-LY:LRKHR:EGMSVAE, VPTKL:SATS-AA$^{17}$-LYY:LRKHR:GMAVS, TPTKM:SATS-AA$^{17}$-LYY:LRKHR:GMVVD, VPARL:SATS-AA$^{17}$-LYY:LRKHR:DMVVE, VPTRL:SATS-AA$^{17}$-LYY:LRKHR:DMVVE, APVKT:SATS-AA$^{17}$-LYY:LRKHR:MIVEE, VPQAL:SATS-AA$^{17}$-LYY:LRKHR:MIVRS, VSQDL:SATS_AA$^{17}$-LYY:LRKHR:MIVKS, VPQDL:SATS-AA$^{17}$-LYY:LRKHR:MVVKS, VPTEE:SATS-AA$^{17}$-LYY:LRKHR:FLQHN, VPTGQ:SATS-AA$^{17}$-LYY:LRKHR:LEEHS, SRVHH:SATS_AA$^{17}$-LYY:LRKHR:RLEEH, TQVQL:SATS-AA$^{17}$-LYY:LRKHR:TLEDH, VPTEL:SPIS-AA$^{17}$-LYK:LKYHY:DMVAE, VPTKM:SPIS-AA$^{17}$-LYK:LKYHY:NMVAE, VPTEL:SPIS-AA$^{17}$-LYK:LKYHY:EMVAE, VPTKL:SPIS-AA$^{17}$-LYK:LKYHY:NMVAE, VPTQL:SPIS-AA$^{17}$-LYK:LKYHY:NMVAE, VPTKL:SPIS-AA$^{17}$-LYK:LKYHY:GMVAE, VPTKM:SPIS-AA$^{17}$-LYK:LKYHY:GMVAE, VPTRL:SPIS-AA$^{17}$-LYK:LKYHY:DMVAE, VPTKT: SPIS_AA$^{17}$-LYK:LKYHY:MIVAE, VPTAL:SPIS-AA$^{17}$-LYK:LKYHY:MIVAE, VPTDL :SPIS_AA$^{17}$-LYK:LKYHY:MIVAE, VPTDL:SPIS-AA$^{17}$-LYK:LKYHY:MVVAE, VPTEE:SPIS-AA$^{17}$-LYK:LKYHY:FLVAE, VPTGQ:SPIS-AA$^{17}$-LYK:LKYHY:LEVAE, VPTHH:SPIS-AA$^{17}$-LYK:LKYHY:RLVAE, VPTQL:SPIS-AA$^{17}$-LYK:LKYHY:TLVAE, VPTEL:SPIS-AA$^{17}$-LYK:LKYHY:DMVVE, VPEKM:SPIS-AA$^{17}$-LYK:LKYHY:NMTVE, VPTEL:SPIS-AA$^{17}$-LYK:LKYHY:EMVVE, APTKL:SPIS-AA$^{17}$-LYK:LKYHY:NMVVR, APTQL:SPIS-AA$^{17}$-LYK:LKYHY:NMVVR, APTKL:SPIS-AA$^{17}$-LYK:LKYHY:NMVVK, VPTKL:SPIS-AA$^{17}$-LYK:LKYHY:GMAVS, TPTKM:SPIS-AA$^{17}$-LYK:LKYHY:GMVVD, VPARL:SPIS-AA$^{17}$-LYK:LKYHY:DMVVE, VPTRL:SPIS-AA$^{17}$-LYK:LKYHY:DMVVE, APVKT:SPIS-AA$^{17}$-LYK:LKYHY:MIVEE, VPQAL:SPIS-AA$^{17}$-LYK:LKYHY:MIVRS, VSQDL:SPIS-AA$^{17}$-LYK:LKYHY:MIVKS, VPQDL:SPIS-AA$^{17}$-LYK:LKYHY:MVVKS, VPTEE:SPIS-AA$^{17}$-LYK:LKYHY:FLQHN, VPTGQ:SPIS_AA$^{17}$-LYK:LKYHY:LEEHS, SRVHH:SPIS-AA$^{17}$-LYK:LKYHY:RLEEH, TQVQL:SPIS-AA$^{17}$-LYK:LKYHY:TLEDH, VPTEL:EPIS-AA$^{17}$-LYL:KFKYE:DMAVS, VPTKM:EPIS-AA$^{17}$-LYL:KFKYE:NMAVS, VPTEL:EPIS-AA$^{17}$-LYL:KFKYE:EMAVS, VPTKL:EPIS-AA$^{17}$-LYL:KFKYE:NMAVS, VPTQL:EPIS-AA$^{17}$-LYL:KFKYE:NMAVS, VPTKL:EPIS-AA$^{17}$-LYL:KFKYE:EGMSAVS, VPTKM:EPIS-AA$^{17}$-LYL:KFKYE:GMAVS, VPTRL:EPIS-AA$^{17}$-LYL:KFKYE:DMAVS, VPTKT:EPIS-AA$^{17}$-LYL:KFKYE:MIAVS, VPTAL:EPIS-AA$^{17}$-LYL:KFKYE:MIAVS, VPTDL:EPIS_AA$^{17}$-LYL:KFKYE:MIAVS, VPTDL:EPIS-AA$^{17}$-LYL:KFKYE:MVAVS, VPTEE:EPIS-AA$^{17}$-LYL:KFKYE:FLAVS, VPTGQ:EPIS-AA$^{17}$-LYL:KFKYE:LEAVS, VPTHH:EPIS-AA$^{17}$-LYL:KFKYE:RLAVS, VPTQL:EPIS-AA$^{17}$-LYL:KFKYE:TLAVS, VPTEL:EPIS-AA$^{17}$-LYL:KFKYE:DMVVE, VPEKM:EPIS-AA$^{17}$-LYL:KFKYE:NMTVE, VPTEL:EPIS-AA$^{17}$-LYL:KFKYE:EMVVE, APTKL:EPIS-AA$^{17}$-LYL:KFKYE:NMVVR, APTQL:EPIS-AA$^{17}$-LYL:KFKYE:NMVVR, APTKL:EPIS-AA$^{17}$-LYL:KFKYE:NMVVK, VPTKL:EPIS-AA$^{17}$-LYL:KFKYE:EGMSVAE, TPTKM:EPIS-AA$^{17}$-LYL:KFKYE:GMVVD, VPARL:EPIS-AA$^{17}$-LYL:KFKYE:DMVVE, VPTRL:EPIS-AA$^{17}$-LYL:KFKYE:DMVVE, APVKT:EPIS-AA$^{17}$-LYL:KFKYE:MIVEE, VPQAL:EPIS-AA$^{17}$-LYL:KFKYE:MIVRS, VSQDL:EPIS-AA$^{17}$-LYL:KFKYE:MIVKS, VPQDL:EPIS-AA$^{17}$-LYL:KFKYE:MVVKS, VPTEE:EPIS-AA$^{17}$-LYL:KFKYE:FLQHN, VPTGQ:EPIS-AA$^{17}$-LYL:KFKYE:LEEHS, SRVHH:EPIS-AA$^{17}$-LYL:KFKYE:RLEEH, TQVQL:EPIS-AA$^{17}$-LYL:KFKYE:TLEDH, TPTEL:SPIN-AA$^{17}$-LYF:YGKIP:DMVVD, TPTKM:SPIN _AA$^{17}$-LYF:YGKIP:NMVVD, TPTEL:SPIN-AA$^{17}$-LYF:YGKIP:EMVVD, TPTKL:SPIN-AA$^{17}$-LYF:YGKIP:NMVVD, TPTQL:SPIN-AA$^{17}$-LYF:YGKIP:NMVVD, TPTKL:SPIN-AA$^{17}$-LYF:YGKIP:EGMSVVD, TPTKL:SPIN-AA$^{17}$-LYF:YGKIP:GMVVD, TPTRL:SPIN-AA$^{17}$-LYF:YGKIP:DMVVD, TPTKT:SPIN-AA$^{17}$-LYF:YGKIP:MIVVD, TPTAL:SPIN-AA$^{17}$-LYF:YGKIP:MIVVD, TPTDL:SPIN-AA$^{17}$-LYF:YGKIP:MIVVD, TPTDL:SPIN-AA$^{17}$-LYF:YGKIP:MVVVD, TPTEE:SPIN-AA$^{17}$-LYF:YGKIP:FLVVD, TPT-

GQ:SPIN-AA$^{17}$-LYF:YGKIP:LEVVD, TPTHH:SPIN-AA$^{17}$-LYF:YGKIP:RLVVD, TPTQL:SPIN-AA$^{17}$-LYF:YGKIP:TLVVD, VPTEL:SPIN-AA$^{17}$-LYF:YGKIP:DMVVE, VPEKM:SPIN-AA$^{17}$-LYF:YGKIP:NMTVE, VPTEL:SPIN-AA$^{17}$-LYF:YGKIP:EMVVE, APTKL:SPIN-AA$^{17}$-LYF:YGKIP:NMVVR, APTQL:SPIN-AA$^{17}$-LYF:YGKIP:NMVVR, APTKL:SPIN-AA$^{17}$-LYF:YGKIP:NMVVK, VPTKL:SPIN-AA$^{17}$-LYF:YGKIP:EGMSVAE, VPTKL:SPIN-AA$^{17}$-LYF:YGKIP:GMAVS, VPARL:SPIN-AA$^{17}$-LYF:YGKIP:DMVVE, VPTRL:SPIN-AA$^{17}$-LYF:YGKIP:DMVVE, APVKT:SPIN-AA$^{17}$-LYF:YGKIP:MIVEE, VPQAL:SPIN-AA$^{17}$-LYF:YGKIP:MIVRS, VSQDL:SPIN-AA$^{17}$-LYF:YGKIP:MIVKS, VPQDL:SPIN-AA$^{17}$-LYF:YGKIP:MVVKS, VPTEE:SPIN-AA$^{17}$-LYF:YGKIP:FLQHN, VPTGQ:SPIN-AA$^{17}$-LYF:YGKIP:LEEHS, SRVHH:SPIN-AA$^{17}$-LYF:YGKIP:RLEEH, TQVQL:SPIN-AA$^{17}$-LYF:YGKIP:TLEDH, VPAEL:SPIS-AA$^{17}$-LYI:YKQYE:DMVVE, VPAKM:SPIS-AA$^{17}$-LYI:YKQYE:NMVVE, VPAEL:SPIS-AA$^{17}$-LYI:YKQYE:EMVVE, VPAKL:SPIS-AA$^{17}$-LYI:YKQYE:NMVVE, VPAQL:SPIS-AA$^{17}$-LYI:YKQYE:NMVVE, VPAKL:SPIS_AA$^{17}$-LYI:YKQYE:EGMSVVE, VPAKL:SPIS-AA$^{17}$-LYI:YKQYE:GMVVE, VPAKM:SPIS-AA$^{17}$-LYI:YKQYE:GMVVE, VPARL:SPIS-AA$^{17}$-LYI:YKQYE:DMVVE, VPAKT:SPIS-AA$^{17}$-LYI:YKQYE:MIVVE, VPAAL:SPIS-AA$^{17}$-LYI:YKQYE:MIVVE, VPADL:SPIS-AA $^{17}$– LYI:YKQYE:MIVVE, VPADL:SPIS-AA$^{17}$-LYI:YKQYE:MVVVE, VPAEE:SPIS-AA$^{17}$-LYI:YKQYE:FLVVE, VPAG Q:SPIS-AA$^{17}$-LYI:YKQYE:LEVVE, VPAHH:SPIS-AA$^{17}$-LYI:YKQYE:RLVVE, VPAQL:SPIS-AA$^{17}$-LYI:YKQYE:TLVVE, VPTEL :SPIS-AA$^{17}$-LYI:YKQYE:DMVVE, VPEKM:SPIS-AA$^{17}$-LYI:YKQYE:NMTVE, VPTEL:SPIS-AA$^{17}$-LYI:YKQYE:EMVVE, APTKL:SPIS-AA$^{17}$-LYI:YKQYE:NMVVR, APTQL:SPIS-AA$^{17}$-LYI:YKQYE:NMVVR, APTKL:SPIS-AA$^{17}$-LYI:YKQYE:NMVVK, VPTKL:SPIS-AA$^{17}$-LYI:YKQYE:EGMSVAE, VPTKL:SPIS-AA$^{17}$-LYI:YKQYE:GMAVS, TPTKM:SPIS-AA$^{17}$-LYI:YKQYE:GMVVD, VPTRL:SPIS-AA$^{17}$-LYI:YKQYE:DMVVE, APVKT:SPIS-AA$^{17}$-LYI:YKQYE:MIVEE, VPQAL:SPIS-AA$^{17}$-LYI:YKQYE:MIVRS, VSQDL:SPIS-AA$^{17}$-LYI:YKQYE:MIVKS, VPQDL:SPIS-AA$^{17}$-LYI:YKQYE:MVVKS, VPTEE:SPIS-AA$^{17}$-LYI:YKQYE:FLQHN, VPTGQ:SPIS-AA$^{17}$-LYI:YKQYE:LEEHS, SRVHH:SPIS-AA$^{17}$-LYI:YKQYE:RLEEH, TQVQL:SPIS-AA$^{17}$-LYI:YKQYE:TLEDH, VPTEL:SPIS-AA$^{17}$-LFI:YKQYE:DMVVE, VPTKM:SPIS-AA$^{17}$-LFI:YKQYE:NMVVE, VPTEL:SPIS-AA$^{17}$-LFI:YKQYE:EMVVE, VPTKL:SPIS-AA$^{17}$-LFI:YKQYE:NMVVE, VPTQL:SPIS-AA$^{17}$-LFI:YKQYE:NMVVE, VPTKL:SPIS-AA$^{17}$-LFI:YKQYE:EGMSVVE, VPTKL:SPIS-AA$^{17}$-LFI:YKQYE:GMVVE, VPTKM:SPIS-AA$^{17}$-LFI:YKQYE:GMVVE, VPTRL:SPIS-AA$^{17}$-LFI:YKQYE:DMVVE, VPTKT: SPIS-AA$^{17}$-LFI:YKQYE:MIVVE, VPTAL:SPIS-AA$^{17}$-LFI:YKQYE:MIVVE, VPTDL:SPIS-AA$^{17}$-LFI:YKQYE:MIVVE, VPTDL :SPIS-AA$^{17}$-LFI:YKQYE:MVVVE, VPTEE:SPIS-AA$^{17}$-LFI:YKQYE:FLVVE, VPTGQ:SPIS-AA$^{17}$-LFI:YKQYE:LEVVE, VPTHH:SPIS-AA$^{17}$-LFI:YKQYE:RLVVE, VPTQL:SPIS-AA$^{17}$-LFI:YKQYE:TLVVE, VPEKM :SPIS-AA$^{17}$-LFI:YKQYE:NMTVE, APTKL:SPIS-AA$^{17}$-LFI:YKQYE:NMVVR, APTQL:SPIS-AA$^{17}$-LFI:YKQYE:NMVVR, APTKL:SPIS-AA$^{17}$-LFI:YKQYE:NMVVK, VPTKL:SPIS-AA$^{17}$-LFI:YKQYE:EGMSVAE, VPTKL:SPIS-AA$^{17}$-LFI:YKQYE:GMAVS, TPTKM:SPIS-AA$^{17}$-LFI:YKQYE:GMVVD, VPARL:SPIS-AA$^{17}$-LFI:YKQYE:DMVVE, APVKT:SPIS-AA$^{17}$-LFI:YKQYE:MIVEE, VPQAL:SPIS-AA$^{17}$-LFI:YKQYE:MIVRS, VSQDL:SPIS-AA$^{17}$-LFI:YKQYE:MIVKS, VPQDL:SPIS-AA$^{17}$-LFI:YKQYE:MVVKS, VPTEE:SPIS-AA$^{17}$-LFI:YKQYE:FLQHN, VPTGQ:SPIS-AA$^{17}$-LFI:YKQYE:LEEHS, SRVHH:SPIS-AA$^{17}$-LFI:YKQYE:RLEEH, TQVQL:SPIS-AA$^{17}$-LFI:YKQYE:TLEDH, APVEL:KPLS-AA$^{17}$-LYV:DHHKD:DMVEE, APVKM:KPLS-AA$^{17}$-LYV:DHHKD:NMVEE, APVEL:KPLS-AA$^{17}$-LYV:DHHKD:EMVEE, APVKL:KPLS-AA$^{17}$-LYV:DHHKD:NMVEE, APVQL:KPLS-AA$^{17}$-LYV:DHHKD:NMVEE, APVKL:KPLS-AA$^{17}$-LYV:DHHKD:EGMSVEE, APVKL:KPLS-AA$^{17}$-LYV:DHHKD:GMVEE, APVKM:KPLS-AA$^{17}$-LYV:DHHKD:GMVEE, APVRL:KPLS-AA$^{17}$-LYV:DHHKD:DMVEE, APVAL:KPLS-AA$^{17}$-LYV:DHHKD:MIVEE, APVDL:KPLS-AA$^{17}$-LYV:DHHKD:MIVEE, APVDL:PLS-AA$^{17}$-LYV:DHHKD:MVVEE, APVEE:KPLS-AA$^{17}$-LYV:DHHKD:FLVEE, APVGQ:KPLS-AA$^{17}$-LYV:DHHKD:LEVEE, APVHH:KPLS-AA$^{17}$-LYV:DHHKD:RLVEE, APVQL:KPLS-AA$^{17}$-LYV:DHHKD:TLVEE, VPTEL:KPLS-AA$^{17}$-LYV:DHHKD:DMVVE, VPEKM:KPLS-AA$^{17}$-LYV:DHHKD:NMTVE, VPTEL:KPLS-AA$^{17}$-LYV:DHHKD:EMVVE, APTKL:KPLS-AA$^{17}$-LYV:DHHKD:NMVVR, APTQL:KPLS-AA$^{17}$-LYV:DHHKD:NMVVR, APTKL:KPLS-AA$^{17}$-LYV:DHHKD:NMVVK, VPTKL:KPLS-AA$^{17}$-LYV:DHHKD:EGMSVAE, VPTKL:KPLS-AA$^{17}$-LYV:DHHKD:GMAVS, TPTKM:KPLS-AA$^{17}$-LYV:DHHKD:GMVVD, VPARL:KPLS-AA$^{17}$-LYV:DHHKD:DMVVE, VPTRL:KPLS-AA$^{17}$-LYV:DHHKD:DMVVE, VPQAL:KPLS-AA$^{17}$-LYV:DHHKD:MIVRS, VSQDL:KPLS-AA$^{17}$-LYV:DHHKD:MIVKS, VPQDL:KPLS-AA$^{17}$-LYV:DHHKD:MVVKS, VPTEE:KPLS-AA$^{17}$-LYV:DHHKD:FLQHN, VPTGQ:KPLS-AA$^{17}$-LYV:DHHKD:LEEHS, SRVHH:KPLS-AA$^{17}$-LYV:DHHKD:RLEEH, TQVQL:KPLS-AA$^{17}$-LYV:DHHKD:TLEDH, VPQEL:EPLP-AA$^{17}$-VYY:EQLSN:DMVRS, VPQKM:EPLP-AA$^{17}$-VYY:EQLSN:NMVRS, VPQEL:EPLP-AA$^{17}$-VYY:EQLSN:EMVRS, VPQKL:EPLP-AA$^{17}$-VYY:EQLSN:NMVRS, VPQQL:EPLP-AA$^{17}$-VYY:EQLSN:NMVRS, VPQKL:EPLP-AA$^{17}$-VYY:EQLSN:EGMSVRS, VPQKL:EPLP-AA$^{17}$-VYY:EQLSN:GMVRS, VPQKM:EPLP-AA$^{17}$-VYY:EQLSN:GMVRS, VPQRL:EPLP-AA$^{17}$-VYY:EQLSN:DMVRS, VPQKT:EPLP-AA$^{17}$-VYY:EQLSN:MIVRS, VPQDL:EPLP-AA$^{17}$-VYY:EQLSN:MIVRS, VPQDL:EPLP-AA$^{17}$-VYY:EQLSN :MVVRS, VPQEE:EPLP-AA$^{17}$-VYY:EQLSN:FLVRS, VPQGQ:EPLP-AA$^{17}$-VYY:EQLSN:LEVRS, VPQHH:EPLP-AA$^{17}$-VYY:EQLSN:RLVRS, VPQQL:EPLP-AA$^{17}$-VYY:EQLSN:TLVRS, VPTEL:EPLP-AA$^{17}$-VYY:EQLSN:DMVVE, VPEKM:EPLP-AA$^{17}$-VYY:EQLSN:NMTVE, VPTEL:EPLP-AA$^{17}$-VYY:EQLSN:EMVVE, APTKL:EPLP-AA$^{17}$-VYY:EQLSN:NMVVR, APTQL:EPLP-AA$^{17}$-VYY:EQLSN:NMVVR, APTKL:EPLP-AA$^{17}$-VYY:EQLSN:NMVVK, VPTKL:EPLP-AA$^{17}$-VYY:EQLSN:EGMSVAE, VPTKL:EPLP-AA$^{17}$-VYY:EQLSN:GMAVS, TPTKM:EPLP-AA$^{17}$-VYY:EQLSN:GMVVD, VPARL:EPLP-AA$^{17}$-VYY:EQLSN:DMVVE, VPTRL:EPLP-AA$^{17}$-VYY:EQLSN:DMVVE,

APVKT:EPLP-AA$^{17}$-VYY:EQLSN:MIVEE, VSQDL:EPLP-AA$^{17}$-VYY:EQLSN:M IVKS, VPQDL:EPLP-AA$^{17}$-VYY:EQLSN:MVVKS, VPTEE:EPLP-AA$^{17}$-VYY:EQLSN:FLQHN, VPTGQ:EPLP-AA$^{17}$-VYY:EQLSN:LEEHS, SRVHH:EPLP-AA$^{17}$-VYY:EQLSN:RLEEH, TQVQL:EPLP-AA$^{17}$-VYY:EQLSN:TLEDH, VSQEL:EPLT-AA$^{17}$-LYY:EQLSN:DMVKS, VSQKM:EPLT-AA$^{17}$-LYY:EQLSN:NMVKS, VSQEL:EPLT-AA$^{17}$-LYY:EQLSN:EMVKS, VSQKL:EPLT-AA$^{17}$-LYY:EQLSN:NMVKS, VSQQL:EPLT-AA$^{17}$-LYY:EQLSN:NMVKS, VSQKL:EPLT-AA$^{17}$-LYY:EQLSN:EGMSVKS, VSQKL:EPLT-AA$^{17}$-LYY:EQLSN:GMVKS, VSQKM:EPLT-AA$^{17}$-LYY:EQLSN:GMVKS, VSQRL:EPLT-AA$^{17}$-LYY:EQLSN:DMVKS, VSQKT:EPLT-AA$^{17}$-LYY:EQLSN:MIVKS, VSQAL:EPLT-AA$^{17}$-LYY:EQLSN:MIVKS, VSQDL:EPLT-AA$^{17}$-LYY:EQLSN:MVVKS, VSQEE:EPLT-AA$^{17}$-LYY:EQLSN:FLVKS, VSQGQ:EPLT-AA$^{17}$-LYY:EQLSN:LEVKS, VSQHH:EPLT-AA$^{17}$-LYY:EQLSN:RLVKS, VSQQL:EPLT-AA$^{17}$-LYY:EQLSN:TLVKS, VPTEL:EPLT-AA$^{17}$-LYY:EQLSN:DMVVE, VPEKM:EPLT-AA$^{17}$-LYY:EQLSN:NMTVE, VPTEL:EPLT-AA$^{17}$-LYY:EQLSN:EMVVE, APTKL:EPLT-AA$^{17}$-LYY:EQLSN:NMVVR, APTQL:EPLT-AA$^{17}$-LYY:EQLSN:NMVVR, APTKL:EPLT-AA$^{17}$-LYY:EQLSN:NMVVK, VPTKL:EPLT-AA$^{17}$-LYY:EQLSN:EGMSVAE, VPTKL:EPLT-AA$^{17}$-LYY:EQLSN:GMAVS, TPTKM: EPLT-AA$^{17}$-LYY:EQLSN:GMVVD, VPARL:EPLT-AA$^{17}$-LYY:EQLSN:DMVVE, VPTRL:EPLT-AA$^{17}$-LYY:EQLSN:DMVVE, APVKT:EPLT-AA$^{17}$-LYY:EQLSN:MIVEE, VPQAL:EPLT-AA$^{17}$-LYY:EQLSN:MIVRS, VPQDL:EPLT-AA$^{17}$-LYY:EQLSN:MVVKS, VPTEE:EPLT-AA$^{17}$-LYY:EQLSN:FLQHN, VPTGQ:EPLT-AA$^{17}$-LYY:EQLSN:LEEHS, SRVHH:EPLT-AA$^{17}$-LYY:EQLSN:RLEEH, TQVQL:EPLT-AA$^{17}$-LYY:EQLSN:TLEDH, VPQEL:EPLT-AA$^{17}$-LYY:EQLSN:DMVKS, VPQKM:EPLT-AA$^{17}$-LYY:EQLSN:NMVKS, VPQEL:EPLT-AA$^{17}$-LYY:EQLSN:EMVKS, VPQKL:EPLT-AA$^{17}$-LYY:EQLSN:NMVKS, VPQQL:EPLT-AA$^{17}$-LYY:EQLSN:NMVKS, VPQKL:EPLT-AA$^{17}$-LYY:EQLSN:EGMSVKS, VPQKL:EPLT-AA$^{17}$-LYY:EQLSN:GMVKS, VPQKM:EPLT-AA$^{17}$-LYY:EQLSN:GMVKS, VPQRL:EPLT-AA$^{17}$-LYY:EQLSN:DMVKS, VPQKT:EPLT-AA$^{17}$-LYY:EQLSN:MIVKS, VPQAL:EPLT-AA$^{17}$-LYY:EQLSN:MIVKS, VPQDL:EPLT-AA$^{17}$-LYY:EQLSN:MIVKS, VPQEE:EPLT-AA$^{17}$-LYY:EQLSN:FLVKS, VPQGQ:EPLT-AA$^{17}$-LYY:EQLSN:LEVKS, VPQHH:EPLT-AA$^{17}$-LYY:EQLSN:RLVKS, VPQQL:EPLT-AA$^{17}$-LYY:EQLSN:TLVKS, VSQDL:EPLT-AA$^{17}$-LYY:EQLSN:MIVKS, VPTEL:SNIT-AA$^{17}$-QIM :IGEMS:DMQHN, VPTKM:SNIT-AA$^{17}$-QIM:IGEMS:NMQHN, VPTEL:SNIT-AA$^{17}$-QIM:IGEMS:EMQHN, VPTKL:SNIT-AA$^{17}$-QIM:IGEMS:NMQHN, VPTQL:SNIT-AA$^{17}$-Q,M:IGEMS:NMQHN, VPTKL:SNIT-AA$^{17}$-QIM:IGEMS:EGMSQHN, VPTKL:SNIT-AA$^{17}$-QIM:IGEMS:GMQHN, VPTKM:SNIT-AA$^{17}$-QIM:IGEMS:GMQHN, VPTRL:SNIT-AA$^{17}$-QIM:IGEMS:DMQHN, VPTKT:SNIT-AA$^{17}$-Q,M:IGEMS:MIQHN, VPTAL:SNIT-AA$^{17}$-Q,M:IGEMS:MIQHN, VPTDL:SNIT-AA$^{17}$-QIM:IGEMS:MIQHN, VPTDL:SNIT-AA$^{17}$-QIM:IGEMS:MVQHN, VPTGQ:SNIT-AA$^{17}$-QIM:IGEMS:LEQHN, VPTEE:SNIT-AA$^{17}$-Q,M:IGEMS:FLQHN, VPTHH:SNIT-AA$^{17}$-QIM:IGEMS:RLQHN, VPTQL:SNIT-AA$^{17}$-Q,M:IGEMS:TLQHN, VPTEL:SNIT-AA$^{17}$-Q,M: IGEMS:DMVVE, VPEKM:SNIT-AA$^{17}$-QIM:IGEMS:NMTVE, VPTEL:SNIT-AA$^{17}$-Q,M:IGEMS:EMVVE, APTKL:SNIT-AA$^{17}$-QIM:IGEMS:NMVVR, APTQL:SNIT-AA$^{17}$-Q,M: IGEMS:NMVVR, APTKL:SNIT-AA$^{17}$-QIM:IGEMS:NMVVK, VPTKL:SNIT-AA$^{17}$-QIM:IGEMS:EGMSVAE, VPTKL:SNIT-AA$^{17}$-QIM :IGEMS:GMAVS, TPTKM:SNIT-AA$^{17}$-QIM:IGEMS:GMVVD, VPARL:SNIT-AA$^{17}$-QIM:IGEMS:DMVVE, VPTRL:SNIT-AA$^{17}$-QIM:IGEMS:DMVVE, APVKT:SNIT-AA$^{17}$-Q,M:IGEMS:MIVEE, VPQAL:SNIT-AA$^{17}$-Q,M:IGEMS:MIVRS, VSQDL:SNIT-AA$^{17}$-QIM:IGEMS:MIVKS, VPQDL:SNIT-AA$^{17}$-QIM:IGEMS:MVVKS, VPTGQ:SNIT-AA$^{17}$-QIM:IGEMS:LEEHS, SRVHH:SNIT-AA$^{17}$-QIM:IGEMS:RLEEH, TQVQL:SNIT-AA$^{17}$-QIM :IGEMS:TLEDH, VPTEL:SNIT-AA$^{17}$-QIM:LGEMS:DMEHS, VPTKM:SNIT-AA$^{17}$-QIM:LGEMS:NMEHS, VPTEL:SNIT-AA$^{17}$-QIM:LGEMS:EMEHS, VPTKL:SNIT-AA$^{17}$-QIM :LGEMS:NMEHS, VPTQL:SNIT-AA$^{17}$-QIM:LGEMS:NMEHS, VPTKL:SNIT-AA$^{17}$-QIM:LGEMS:EGMSEHS, VPTKL:SNIT-AA$^{17}$-QIM:LGEMS:GMEHS, VPTKM:SNIT-AA$^{17}$-QIM:LGEMS:GMEHS, VPTRL:SNIT-AA$^{17}$-QIM:LGEMS:DMEHS, VPTKT:SNIT-AA$^{17}$-QIM:LGEMS:MIEHS, VPTAL:SNIT-AA$^{17}$-QIM:LGEMS:MIEHS, VPTDL:SNIT-AA$^{17}$-QIM:LGEMS:MIEHS, VPTDL:SNIT-AA$^{17}$-QI M:LGEMS:MVEHS, VPTEE:SNIT-AA$^{17}$-QIM:LGEMS:FLEHS, VPTHH:SNIT-AA$^{17}$-QIM:LGEMS:RLEHS, VPTQL:SNIT-AA$^{17}$-QIM:LGEMS:TLEHS, VPTEL:SNIT-AA$^{17}$-QIM:LGEMS:DMVVE, VPEKM:SNIT-AA$^{17}$-QIM :LGEMS:NMTVE, VPTEL:SNIT-AA$^{17}$-QIM:LGEMS:EMVVE, APTKL:SNIT-AA$^{17}$-QIM:LGEMS:NMVVR, APTQL:SNIT-AA$^{17}$-QIM:LGEMS:NMVVR, APTKL:SNIT-AA$^{17}$-QIM:LGEMS:NMVVK, VPTKL:SNIT-AA$^{17}$-QIM:LGEMS:EGMSVAE, VPTKL:SNIT-AA$^{17}$-QIM:LGEMS:GMAVS, TPTKM:SNIT-AA$^{17}$-QIM:LGEMS:GMVVD, VPARL:SNIT-AA$^{17}$-QIM:LGEMS:DMVVE, VPTRL:SNIT-AA$^{17}$-QIM:LGEMS:DMVVE, APVKT:SNIT-AA$^{17}$-QIM:LGEMS:MIVEE, VPQAL:SNIT-AA$^{17}$-QIM :LGEMS:MIVRS, VSQDL:SNIT-AA$^{17}$-QIM :LGEMS:MIVKS, VPQDL:SNIT-AA$^{17}$-QIM:LGEMS:MVVKS, VPTEE:SNIT-AA$^{17}$-QIM :LGEMS:FLQHN, SRVHH:SNIT-AA$^{17}$-QIM:LGEMS:RLEEH, TQVQL:SNIT-AA$^{17}$-QIM :LGEMS:TLEDH, SRVEL:RSVK-AA$^{17}$-AKV:KEVQV:DMEEH, SRVKM:RSVK-AA$^{17}$-AKV:KEVQV:NMEEH, SRVEL:RSVK-AA$^{17}$-AKV:KEVQV:EMEEH, SRVKL:RSVK-AA$^{17}$-AKV:KEVQV:NMEEH, SRVQL:RSVK-AA$^{17}$-AKV:KEVQV:NMEEH, SRVKL:RSVK-AA$^{17}$-AKV:KEVQV:EGMSEEH, SRVKL:RSVK-AA$^{17}$-AKV:KEVQV:GMEEH, SRVKM:RSVK-AA$^{17}$-AKV:KEVQV:GMEEH, SRVRL:RSVK-AA$^{17}$-AKV:KEVQV:DMEEH, SRVKT:RSVK-AA$^{17}$-AKV:KEVQV:MIEEH, SRVAL:RSVK-AA$^{17}$-AKV:KEVQV:MIEEH, SRVDL:RSVK-AA$^{17}$-AKV:KEVQV:MIEEH, SRVDL:RSVK-AA$^{17}$-AKV:KEVQV:MVEEH, SRVEE:RSVK-AA$^{17}$-AKV:KEVQV:FLEEH, SRVGQ:RSVK-AA$^{17}$-AKV:KEVQV:LEEEH, SRVQL:RSVK-AA$^{17}$-AKV:KEVQV:TLEEH, VPTEL:RSVK-AA$^{17}$-AKV:KEVQV:DMVVE, VPEKM:RSVK-AA$^{17}$-AKV:KEVQV:NMTVE, VPTEL:RSVK-AA$^{17}$-AKV:KEVQV:EMVVE, APTKL:RSVK-AA$^{17}$-AKV:KEVQV:NMVVR, APTQL:RSVK-AA$^{17}$-AKV:KEVQV:NMVVR, APTKL:RSVK-

AA$^{17}$-AKV:KEVQV:NMVVK, VPTKL:RSVK-AA$^{17}$-AKV:KEVQV:EGMSVAE, VPTKL:RSVK-AA$^{17}$-AKV:KEVQV:GMAVS, TPTKM:RSVK-AA$^{17}$-AKV:KEVQV:GMVVD, VPARL:RSVK-AA$^{17}$-AKV:KEVQV:DMVVE, VPTRL:RSVK-AA$^{17}$-AKV:KEVQV:DMVVE, APVKT:RSVK-AA$^{17}$-AKV:KEVQV:MIVEE, VPQAL:RSVK-AA$^{17}$-AKV:KEVQV:MIVRS, VSQDL:RSVK-AA$^{17}$-AKV:KEVQV:MIVKS, VPQDL:RSVK-AA$^{17}$-AKV:KEVQV:MVVKS, VPTEE:RSVK-AA$^{17}$-AKV:KEVQV:FLQHN, VPTGQ:RSVK-AA$^{17}$-AKV:KEVQV:LEEHS, TQVQL:RSVK-AA$^{17}$-AKV:KEVQV:TLEDH, TQVEL:RPVQ-AA$^{17}$-RKI:KKATV:DMEDH, TQVKM:RPVQ-AA$^{17}$-RKI:KKATV:NMEDH, TQVEL:RPVQ-AA$^{17}$-RKI:KKATV:EMEDH, TQVKL:RPVQ-AA$^{17}$-RKI:KKATV:NMEDH, TQVQL:RPVQ-AA$^{17}$-RKI:KKATV:NMEDH, TQVKL:RPVQ-AA$^{17}$-RKI:KKATV:EGMSEDH, TQVKL:RPVQ-AA$^{17}$-RKI:KKATV:GMEDH, TQVKM:RPVQ-AA$^{17}$-RKI:KKATV:GMEDH, TQVRL:RPVQ-AA$^{17}$-RKI:KKATV:DMEDH, TQVKT:RPVQ-AA$^{17}$-RKI:KKATV:MIEDH, TQVAL:RPVQ-AA$^{17}$-RKI:KKATV:MIEDH, TQVDL:RPVQ-AA$^{17}$-RKI:KKATV:MIEDH, TQVDL:RPVQ-AA$^{17}$-RKI:KKATV:MVEDH, TQVEE:RPVQ-AA$^{17}$-RKI:KKATV:FLEDH, TQVGQ:RPVQ-AA$^{17}$-RKI:KKATV:LEEDH, TQVHH:RPVQ-AA$^{17}$-RKI:KKATV:RLEDH, VPTEL:RPVQ-AA$^{17}$-RKI:KKATV:DMVVE, VPEKM:RPVQ-AA$^{17}$-RKI:KKATV:NMTVE, VPTEL:RPVQ-AA$^{17}$-RKI:KKATV:EMVVE, APTKL:RPVQ-AA$^{17}$-RKI:KKATV:NMVVR, APTQL:RPVQ-AA$^{17}$-RKI:KKATV:NMVVR, APTKL:RPVQ-AA$^{17}$-RKI:KKATV:NMVVK, VPTKL:RPVQ-AA$^{17}$-RKI:KKATV:EGMSVAE, VPTKL:RPVQ-AA$^{17}$-RKI:KKATV:GMAVS, TPTKM:RPVQ-AA$^{17}$-RKI:KKATV:GMVVD, VPARL:RPVQ-AA$^{17}$-RKI:KKATV:DMVVE, VPTRL:RPVQ-AA$^{17}$-RKI:KKATV:DMVVE, APVKT:RPVQ-AA$^{17}$-RKI:KKATV:MIVEE, VPQAL:RPVQ-AA$^{17}$-RKI:KKATV:MIVRS, VSQDL:RPVQ-AA$^{17}$-RKI:KKATV:MIVKS, VPQDL:RPVQ-AA$^{17}$-RKI:KKATV:MVVKS, VPTEE:RPVQ-AA$^{17}$-RKI:KKATV:FLQHN, VPTGQ:RPVQ-AA$^{17}$-RKI:KKATV:LEEHS and SRVHH:RPVQ-AA$^{17}$-RKI:KKATV:RLEEH; and wherein AA$^{17}$ is selected from the group consisting of G, A, V, L, I, P, F, M, W, T and S (in particular is selected from the group consisting of M, I, L, V and T). More particularly, the quadruplet PEP5:PEP12:PEP2:PEP6 is selected from the group consisting of VPTKM:SAIS-AA$^{17}$-LYL:LKNYQ:NMVVE, VPTKL:SAIS_AA$^{17}$-LYL:LKNYQ:NMVVE, VPTQL:SAIS-AA$^{17}$-LYL:LKNYQ:NMVVE, VPTKL:SAIS-AA$^{17}$-LYL:LKNYQ:EGMSVVE, VPTKL:SAIS-AA$^{17}$-LYL:LKNYQ:GMVVE, VPTKM:SAIS-AA$^{17}$-LYL:LKNYQ:GMVVE, VPTRL:SAIS-AA$^{17}$-LYL:LKNYQ:DMVVE, VPTKT:SAIS-AA$^{17}$-LYL:LKNYQ:MIVVE, VPTAL:SAIS-AA$^{17}$-LYL:LKNYQ:MIVVE, VPTDL:SAIS-AA$^{17}$-LYL:LKNYQ:MIVVE, VPTDL:SAIS-AA$^{17}$-LYL:LKNYQ:MVVVE, VPEKM:SAIS-AA$^{17}$-LYL:LKNYQ:NMTVE, APTKL:SAIS-AA$^{17}$-LYL:LKNYQ:NMVVR, APTQL:SAIS-AA$^{17}$-LYL:LKNYQ:NMVVR, APTKL:SAIS-AA$^{17}$-LYL:LKNYQ:NMVVK, VPTKL:SAIS-AA$^{17}$-LYL:LKNYQ:EGMSVAE, VPTKL:SAIS-AA$^{17}$-LYL:LKNYQ:GMAVS, TPTKM:SAIS-AA$^{17}$-LYL:LKNYQ:GMVVD, VPARL:SAIS-AA$^{17}$-LYL:LKNYQ:DMVVE, APVKT:SAIS-AA$^{17}$-LYL:LKNYQ:MIVEE, VPQAL:SAIS-AA$^{17}$-LYL:LKNYQ:MIVRS, VSQDL:SAIS-AA$^{17}$-LYL:LKNYQ:MIVKS, VPQDL:SAIS-AA$^{17}$-LYL:LKNYQ:MVVKS, VPTEE:SAIS-AA$^{17}$-LYL:LKNYQ:FLQHN, VPTGQ:SAIS-AA$^{17}$-LYL:LKNYQ:LEEHS, SRVHH:SAIS-AA$^{17}$-LYL:LKNYQ:RLEEH, TQVQL:SAIS-AA$^{17}$-LYL:LKNYQ:TLEDH, VPEEL:SSLS-AA$^{17}$-LFF:LKVYP:DMTVE, VPEEL:SSLS-AA$^{17}$-LFF:LKVYP:EMTVE, VPEKL:SSLS-AA$^{17}$-LFF:LKVYP:NMTVE, VPEQL:SSLS-AA$^{17}$-LFF:LKVYP:NMTVE, VPEKL:SSLS-AA$^{17}$-LFF:LKVYP:EGMSTVE, VPEKL:SSLS-AA$^{17}$-LFF:LKVYP:GMTVE, VPEKM:SSLS-AA$^{17}$-LFF:LKVYP:GMTVE, VPERL:SSLS-AA$^{17}$-LFF:LKVYP:DMTVE, VPEKT:SSLS-AA$^{17}$-LFF:LKVYP:MITVE, VPEAL:SSLS-AA$^{17}$-LFF:LKVYP:MITVE, VPEDL:SSLS-AA$^{17}$-LFF:LKVYP:MITVE, VPEDL:SSLS-AA$^{17}$-LFF:LKVYP:MVTVE, VPTEL:SSLS-AA$^{17}$-LFF:LKVYP:DMVVE, VPTEL:SSLS-AA$^{17}$-LFF:LKVYP:EMVVE, APTKL:SSLS-AA$^{17}$-LFF:LKVYP:NMVVR, APTQL:SSLS-AA$^{17}$-LFF:LKVYP:NMVVR, APTKL:SSLS-AA$^{17}$-LFF:LKVYP:NMVVK, VPTKL:SSLS-AA$^{17}$-LFF:LKVYP:EGMSVAE, VPTKL:SSLS-AA$^{17}$-LFF:LKVYP:GMAVS, TPTKM:SSLS-AA$^{17}$-LFF:LKVYP:GMVVD, VPARL:SSLS-AA$^{17}$-LFF:LKVYP:DMVVE, VPTRL:SSLS-AA$^{17}$-LFF:LKVYP:DMVVE, APVKT:SSLS-AA$^{17}$-LFF:LKVYP:MIVEE, VPQAL:SSLS-AA$^{17}$-LFF:LKVYP:MIVRS, VSQDL:SSLS-AA$^{17}$-LFF:LKVYP:MIVKS, VPQDL:SSLS-AA$^{17}$-LFF:LKVYP:MVVKS, VPTEE:SSLS-AA$^{17}$-LFF:LKVYP:FLQHN, VPTGQ:SSLS-AA$^{17}$-LFF:LKVYP:LEEHS, SRVHH:SSLS-AA$^{17}$-LFF:LKVYP:RLEEH, TQVQL:SSLS-AA$^{17}$-LFF:LKVYP:TLEDH, APTEL:NAIS-AA$^{17}$-LYF:LKKYR:DMVVR, APTKM:NAIS-AA$^{17}$-LYF:LKKYR:NMVVR, APTEL:NAIS-AA$^{17}$-LYF:LKKYR:EMVVR, APTKL:NAIS-AA$^{17}$-LYF:LKKYR:NMVVR, APTKL:NAIS-AA$^{17}$-LYF:LKKYR:EGMSVVR, APTKL:NAIS-AA$^{17}$-LYF:LKKYR:GMVVR, APTKM:NAIS-AA$^{17}$-LYF:LKKYR:GMVVR, APTRL:NAIS-AA$^{17}$-LYF:LKKYR:DMVVR, APTKT:NAIS-AA$^{17}$-LYF:LKKYR:MIVVR, APTAL:NAIS-AA$^{17}$-LYF:LKKYR:MIVVR, APTDL:NAIS-AA$^{17}$-LYF:LKKYR:MIVVR, APTDL:NAIS-AA$^{17}$-LYF:LKKYR:MVVVR, VPTEL:NAIS-AA$^{17}$-LYF:LKKYR:DMVVE, VPEKM:NAIS-AA$^{17}$-LYF:LKKYR:NMTVE, VPTEL:NAIS-AA$^{17}$-LYF:LKKYR:EMVVE, APTKL:NAIS-AA$^{17}$-LYF:LKKYR:NMVVK, VPTKL:NAIS-AA$^{17}$-LYF:LKKYR:EGMSVAE, VPTKL:NAIS-AA$^{17}$-LYF:LKKYR:GMAVS, TPTKM:NAIS-AA$^{17}$-LYF:LKKYR:GMVVD, VPARL:NAIS-AA$^{17}$-LYF:LKKYR:DMVVE, VPTRL:NAIS-AA$^{17}$-LYF:LKKYR:DMVVE, APVKT:NAIS-AA$^{17}$-LYF:LKKYR:MIVEE, VPQAL:NAIS-AA$^{17}$-LYF:LKKYR:MIVRS, VSQDL:NAIS-AA$^{17}$-LYF:LKKYR:MIVKS, VPQDL:NAIS-AA$^{17}$-LYF:LKKYR:MVVKS, VPTEE:NAIS-AA$^{17}$-LYF:LKKYR:FLQHN, VPTGQ:NAIS-AA$^{17}$-LYF:LKKYR:LEEHS, SRVHH:NAIS-AA$^{17}$-LYF:LKKYR:RLEEH, TQVQL:NAIS-AA$^{17}$-LYF:LKKYR:TLEDH, APTEL:SATS-AA$^{17}$-LYY:LRKHR:DMVVK, APTKM:SATS-AA$^{17}$-LYY:LRKHR:NMVVK, APTEL:SATS-AA$^{17}$-LYY:LRKHR:EMVVK, APTKL:SATS-AA$^{17}$-LYY:LRKHR:NMVVK, APTQL:SATS-AA$^{17}$-LYY:LRKHR:NMVVK, APTKL:SATS-AA$^{17}$-LYY:LRKHR:EGMSVVK, APTKL:SATS-AA$^{17}$-LYY:LRKHR:GMVVK, APTKM:SATS-AA$^{17}$-LYY:LRKHR:GMVVK, APTRL:SATS-AA$^{17}$-LYY:LRKHR:DMVVK, APTKT:SATS-

AA$^{17}$-LYY:LRKHR:MIVVK, APTAL:SATS-AA$^{17}$-LYY:LRKHR:MIVVK, APTDL:SATS-AA$^{17}$-LYY:LRKHR:MIVVK, APTDL:SATS-AA$^{17}$-LYY:LRKHR:MVVVK, VPTEL:SATS-AA$^{17}$-LYY:LRKHR:DMVVE, VPEKM:SATS-AA$^{17}$-LYY:LRKHR:NMTVE, VPTEL:SATS-AA$^{17}$-LYY:LRKHR:EMVVE, APTKL:SATS-AA$^{17}$-LYY:LRKHR:NMVVR, APTQL:SATS-AA$^{17}$-LYY:LRKHR:NMVVR, VPTKL:SATS-AA$^{17}$-LYY:LRKHR:EGMSVAE, VPTKL:SATS-AA$^{17}$-LYY:LRKHR:GMAVS, TPTKM:SATS-AA$^{17}$-LYY:LRKHR:GMVVD, VPARL:SATS-AA$^{17}$-LYY:LRKHR:DMVVE, VPTRL:SATS-AA$^{17}$-LYY:LRKHR:DMVVE, APVKT:SATS-AA$^{17}$-LYY:LRKHR:MIVEE, VPQAL:SATS-AA$^{17}$-LYY:LRKHR:MIVRS, VSQDL:SATS-AA$^{17}$-LYY:LRKHR:MIVKS, VPQDL:SATS-AA$^{17}$-LYY:LRKHR:MVVKS, VPTEE:SATS-AA$^{17}$-LYY:LRKHR:FLQHN, VPTGQ:SATS-AA$^{17}$-LYY:LRKHR:LEEHS, SRVHH:SATS-AA$^{17}$-LYY:LRKHR:RLEEH, TQVQL:SATS-AA$^{17}$-LYY:LRKHR:TLEDH, VPTEL:SPIS-AA$^{17}$-LYK:LKYHY:DMVAE, VPTKM:SPIS-AA$^{17}$-LYK:LKYHY:NMVAE, VPTEL:SPIS-AA$^{17}$-LYK:LKYHY:EMVAE, VPTKL:SPIS-AA$^{17}$-LYK:LKYHY:NMVAE, VPTQL:SPIS-AA$^{17}$-LYK:LKYHY:NMVAE, VPTKL:SPIS-AA$^{17}$-LYK:LKYHY:GMVAE, VPTKM:SPIS-AA$^{17}$-LYK:LKYHY:GMVAE, VPTRL:SPIS-AA$^{17}$-LYK:LKYHY:DMVAE, VPTKT:SPIS-AA$^{17}$-LYK:LKYHY:MIVAE, VPTAL:SPIS-AA$^{17}$-LYK:LKYHY:MIVAE, VPTDL:SPIS-AA$^{17}$-LYK:LKYHY:MIVAE, VPTDL :SPIS-AA$^{17}$-LYK:LKYHY:MVVAE, VPTEL:SPIS-AA$^{17}$-LYK:LKYHY:DMVVE, VPEKM:SPIS-AA$^{17}$-LYK:LKYHY:NMTVE, VPTEL:SPIS-AA$^{17}$-LYK:LKYHY:EMVVE, APTKL:SPIS-AA$^{17}$-LYK:LKYHY:NMVVR, APTQL:SPIS-AA$^{17}$-LYK:LKYHY:NMVVR, APTKL:SPIS-AA$^{17}$-LYK:LKYHY:NMVVK, VPTKL:SPIS-AA$^{17}$-LYK:LKYHY:GMAVS, TPTKM:SPIS-AA$^{17}$-LYK:LKYHY:GMVVD, VPARL:SPIS-AA$^{17}$-LYK:LKYHY:DMVVE, VPTRL :SPIS-AA$^{17}$-LYK:LKYHY:DMVVE, APVKT:SPIS-AA$^{17}$-LYK:LKYHY:MIVEE, VPQAL:SPIS-AA$^{17}$-LYK:LKYHY:MIVRS, VSQDL:SPIS-AA$^{17}$-LYK:LKYHY:MIVKS, VPQDL:SPIS-AA$^{17}$-LYK:LKYHY:MVVKS, VPTEE:SPIS-AA$^{17}$-LYK:LKYHY:FLQHN, VPTGQ:SPIS-AA$^{17}$-LYK:LKYHY:LEEHS, SRVHH:SPIS-AA$^{17}$-LYK:LKYHY:RLEEH, TQVQL:SPIS-AA$^{17}$-LYK:LKYHY:TLEDH, VPTEL:EPIS-AA$^{17}$-LYL:KFKYE:DMAVS, VPTKM:EPIS-AA$^{17}$-LYL:KFKYE:NMAVS, VPTEL:EPIS-AA$^{17}$-LYL:KFKYE:EMAVS, VPTKL:EPIS-AA$^{17}$-LYL:KFKYE:NMAVS, VPTQL:EPIS-AA$^{17}$-LYL:KFKYE:NMAVS, VPTKL:EPIS-AA$^{17}$-LYL:KFKYE:EGMSAVS, VPTKM:EPIS-AA$^{17}$-LYL:KFKYE:GMAVS, VPTRL:EPIS-AA$^{17}$-LYL:KFKYE:DMAVS, VPTKT:EPIS-AA$^{17}$-LYL:KFKYE:MIAVS, VPTAL:EPIS-AA$^{17}$-LYL:KFKYE:MIAVS, VPTDL:EPIS-AA$^{17}$-LYL:KFKYE:MIAVS, VPTDL:EPIS-AA$^{17}$-LYL:KFKYE:MVAVS, VPTEL:EPIS-AA$^{17}$-LYL:KFKYE:DMVVE, VPEKM:EPIS-AA$^{17}$-LYL:KFKYE:NMTVE, VPTEL:EPIS-AA$^{17}$-LYL:KFKYE:EMVVE, APTKL:EPIS-AA$^{17}$-LYL:KFKYE:NMVVR, APTQL:EPIS-AA$^{17}$-LYL:KFKYE:NMVVR, APTKL:EPIS-AA$^{17}$-LYL:KFKYE:NMVVK, VPTKL:EPIS-AA$^{17}$-LYL:KFKYE:EGMSVAE, TPTKM:EPIS-AA$^{17}$-LYL:KFKYE:GMVVD, VPARL:EPIS-AA$^{17}$-LYL:KFKYE:DMVVE, VPTRL:EPIS-AA$^{17}$-LYL:KFKYE:DMVVE, APVKT:EPIS-AA$^{17}$-LYL:KFKYE:MIVEE, VPQAL:EPIS-AA$^{17}$-LYL:KFKYE:MIVRS, VSQDL:EPIS-AA$^{17}$-LYL:KFKYE:MIVKS, VPQDL:EPIS-AA$^{17}$-LYL:KFKYE:MVVKS, VPTEE:EPIS-AA$^{17}$-LYL:KFKYE:FLQHN, VPTGQ:EPIS-AA$^{17}$-LYL:KFKYE:LEEHS, SRVHH:EPIS-AA$^{17}$-LYL:KFKYE:RLEEH, TQVQL:EPIS-AA$^{17}$-LYL:KFKYE:TLEDH, TPTEL:SPIN-AA$^{17}$-LYF:YGKIP:DMVVD, TPTKM:SPIN-AA$^{17}$-LYF:YGKIP:NMVVD, TPTEL:SPIN-AA$^{17}$-LYF:YGKIP:EMVVD, TPTKL:SPIN-AA$^{17}$-LYF:YGKIP:NMVVD, TPTQL:SPIN-AA$^{17}$-LYF:YGKIP:NMVVD, TPTKL:SPIN-AA$^{17}$-LYF:YGKIP:EGMSVVD, TPTKL:SPIN-AA$^{17}$-LYF:YGKIP:GMVVD, TPTRL:SPIN_AA$^{17}$-LYF:YGKIP:DMVVD, TPTKT:SPIN_AA$^{17}$-LYF:YGKIP:MIVVD, TPTAL:SPIN-AA$^{17}$-LYF:YGKIP:MIVVD, TPTDL:SPIN-AA$^{17}$-LYF:YGKIP:MIVVD, TPTDL:SPIN-AA$^{17}$-LYF:YGKIP:MVVVD, VPTEL:SPIN_AA$^{17}$-LYF:YGKIP:DMVVE, VPEKM:SPIN-AA$^{17}$-LYF:YGKIP:NMTVE, VPTEL:SPIN-AA$^{17}$-LYF:YGKIP:EMVVE, APTKL:SPIN-AA$^{17}$-LYF:YGKIP:NMVVR, APTQL:SPIN-AA$^{17}$-LYF:YGKIP:NMVVR, APTKL:SPIN_AA$^{17}$-LYF:YGKIP:NMVVK, VPTKL:SPIN-AA$^{17}$-LYF:YGKIP:EGMSVAE, VPTKL:SPIN-AA$^{17}$-LYF:YGKIP:GMAVS, VPARL:SPIN-AA$^{17}$-LYF:YGKIP:DMVVE, VPTRL:SPIN-AA$^{17}$-LYF:YGKIP:DMVVE, APVKT :SPIN-AA$^{17}$-LYF:YGKIP:M IVEE, VPQAL:SPIN-AA$^{17}$-LYF:YGKIP:MIVRS, VSQDL:SPIN-AA$^{17}$-LYF:YGKIP:MIVKS, VPQDL:SPIN-AA$^{17}$-LYF:YGKIP:MVVKS, VPTEE:SPIN-AA$^{17}$-LYF:YGKIP:FLQHN, VPTGQ:SPIN_AA$^{17}$-LYF:YGKIP:LEEHS, SRVHH:SPIN-AA$^{17}$-LYF:YGKIP:RLEEH, TQVQL:SPIN-AA$^{17}$-LYF:YGKIP:TLEDH, VPAEL:SPIS-AA$^{17}$-LYI:YKQYE:DMVVE, VPAKM:SPIS-AA$^{17}$-LYI:YKQYE:NMVVE, VPAEL:SPIS-AA$^{17}$-LYI:YKQYE:EMVVE, VPAKL:SPIS-AA$^{17}$-LYI:YKQYE:NMVVE, VPAQL:SPIS-AA$^{17}$-LYI:YKQYE:NMVVE, VPAKL:SPIS-AA$^{17}$-LYI:YKQYE:EGMSVVE, VPAKL:SPIS-AA$^{17}$-LYI:YKQYE:GMVVE, VPAKM:SPIS-AA$^{17}$-LYI:YKQYE:GMVVE, VPARL:SPIS-AA$^{17}$-LYI:YKQYE:DMVVE, VPAKT:SPIS-AA$^{17}$-LYI:YKQYE:MIVVE, VPAAL:SPIS-AA$^{17}$-LYI:YKQYE:MIVVE, VPADL:SPIS-AA$^{17}$-LYI:YKQYE:MIVVE, VPADL:SPIS-AA$^{17}$-LYI:YKQYE:MVVVE, VPTEL:SPIS-AA$^{17}$-LYI:YKQYE:DMVVE, VPEKM:SPIS-AA$^{17}$-LYI:YKQYE:NMTVE, VPTEL:SPIS-AA$^{17}$-LYI:YKQYE:EMVVE, APTKL:SPIS-AA$^{17}$-LYI:YKQYE:NMVVR, APTQL:SPIS-AA$^{17}$-LYI:YKQYE:NMVVR, APTKL:SPIS-AA$^{17}$-LYI:YKQYE:NMVVK, VPTKL:SPIS-AA$^{17}$-LYI:YKQYE:EGMSVAE, VPTKL:SPIS-AA$^{17}$-LYI:YKQYE:GMAVS, TPTKM:SPIS-AA$^{17}$-LYI:YKQYE:GMVVD, VPTRL:SPIS-AA$^{17}$-LYI:YKQYE:DMVVE, APVKT:SPIS-AA$^{17}$-LYI:YKQYE:MIVEE, VPQAL:SPIS-AA$^{17}$-LYI:YKQYE:MIVRS, VSQDL:SPIS-AA$^{17}$-LYI:YKQYE:MIVKS, VPQDL:SPIS-AA$^{17}$-LYI:YKQYE:MVVKS, VPTEE:SPIS-AA$^{17}$-LYI:YKQYE:FLQHN, VPTGQ:SPIS-AA$^{17}$-LYI:YKQYE:LEEHS, SRVHH:SPIS-AA$^{17}$-LYI:YKQYE:RLEEH, TQVQL:SPIS-AA$^{17}$-LYI:YKQYE:TLEDH, VPTEL:SPIS-AA$^{17}$-LFI:YKQYE:DMVVE, VPTKM:SPIS-AA$^{17}$-LFI:YKQYE:NMVVE, VPTEL:SPIS-AA$^{17}$-LFI:YKQYE:EMVVE, VPTKL:SPIS-AA$^{17}$-LFI:YKQYE:NMVVE, VPTQL:SPIS-AA $^{17}$ -LFI:YKQYE:NMVVE, VPTKL:SPIS-AA$^{17}$-LFI:YKQYE:EGMSVVE, VPTKL:SPIS-AA$^{17}$-LFI:YKQYE:GMVVE, VPTKM:SPIS-AA$^{17}$-LFI:YKQYE:GMVVE, VP-

TRL:SPIS-AA$^{17}$-LFI:YKQYE:DMVVE, VPTKT:SPIS-AA$^{17}$-LFI:YKQYE:MIVVE, VPTAL:SPIS-AA$^{17}$-LFI:YKQYE:MIVVE, VPTDL:SPIS-AA$^{17}$-LFI:YKQYE:MIVVE, VPTDL:SPIS-AA$^{17}$-LFI:YKQYE:MVVVE, VPEKM:SPIS-AA$^{17}$-LFI:YKQYE:NMTVE, APTKL:SPIS-AA$^{17}$-LFI:YKQYE:NMVVR, APTQL:SPIS-AA$^{17}$-LFI:YKQYE:NMVVR, APTKL:SPIS-AA$^{17}$-LFI:YKQYE:NMVVK, VPTKL:SPIS-AA$^{17}$-LFI:YKQYE:EGMSVAE, VPTKL:SPIS-AA$^{17}$-LFI:YKQYE:GMAVS, TPTKM:SPIS-AA$^{17}$-LFI:YKQYE:GMVVD, VPARL:SPIS-AA$^{17}$-LFI:YKQYE:DMVVE, APVKT:SPIS-AA$^{17}$-LFI:YKQYE:MIVEE, VPQAL:SPIS-AA$^{17}$-LFI:YKQYE:MIVRS, VSQDL:SPIS-AA$^{17}$-LFI:YKQYE:MIVKS, VPQDL:SPIS-AA$^{17}$-LFI:YKQYE:MVVKS, VPTEE:SPIS-AA$^{17}$-LFI:YKQYE:FLQHN, VPTGQ:SPIS-AA$^{17}$-LFI:YKQYE:LEEHS, SRVHH:SPIS-AA$^{17}$-LFI:YKQYE:RLEEH, TQVQL:SPIS-AA$^{17}$-LFI:YKQYE:TLEDH, APVEL:KPLS-AA $^{17}$– LYV:DHHKD:DMVEE, APVKM:KPLS-AA$^{17}$-LYV:DHHKD:NMVEE, APVEL:KPLS-AA$^{17}$-LYV:DHHKD:EMVEE, APVKL:KPLS_AA$^{17}$-LYV:DHHKD:NMVEE, APVQL:KPLS-AA$^{17}$-LYV:DHHKD:NMVEE, APVKL:KPLS-AA$^{17}$-LYV:DHHKD:EGMSVEE, APVKL:KPLS-AA$^{17}$-LYV:DHHKD:GMVEE, APVKM:KPLS-AA$^{17}$-LYV:DHHKD:GMVEE, APVRL:KPLS-AA$^{17}$-LYV:DHHKD:DMVEE, APVAL:KPLS-AA$^{17}$-LYV:DHHKD:MIVEE, APVDL:KPLS-AA$^{17}$-LYV:DHHKD:MIVEE, APVDL:KPLS-AA$^{17}$-LYV:DHHKD:MVVEE, VPTEL:KPLS-AA$^{17}$-LYV:DHHKD:DMVVE, VPEKM:KPLS-AA$^{17}$-LYV:DHHKD:NMTVE, VPTEL:KPLS-AA$^{17}$-LYV:DHHKD:EMVVE, APTKL:KPLS-AA$^{17}$-LYV:DHHKD:NMVVR, APTQL:KPLS-AA$^{17}$-LYV:DHHKD:NMVVR, APTKL:KPLS-AA$^{17}$-LYV:DHHKD:NMVVK, VPTKL:KPLS-AA$^{17}$-LYV:DHHKD:EGMSVAE, VPTKL:KPLS-AA$^{17}$-LYV:DHHKD:GMAVS, TPTKM:KPLS-AA$^{17}$-LYV:DHHKD:GMVVD, VPARL:KPLS-AA$^{17}$-LYV:DHHKD:DMVVE, VPTRL:KPLS-AA$^{17}$-LYV:DHHKD:DMVVE, VPQAL:KPLS-AA$^{17}$-LYV:DHHKD:MIVRS, VSQDL:KPLS-AA$^{17}$-LYV:DHHKD:MIVKS, VPQDL:KPLS-AA$^{17}$-LYV:DHHKD:MVVKS, VPTEE:KPLS-AA$^{17}$-LYV:DHHKD:FLQHN, VPTGQ:KPLS-AA$^{17}$-LYV:DHHKD:LEEHS, SRVHH:KPLS-AA$^{17}$-LYV:DHHKD:RLEEH, TQVQL:KPLS-AA$^{17}$-LYV:DHHKD:TLEDH, VPQEL:EPLP-AA$^{17}$-VYY:EQLSN:DMVRS, VPQKM:EPLP-AA$^{17}$-VYY:EQLSN:NMVRS, VPQEL:EPLP-AA$^{17}$-VYY:EQLSN:EMVRS, VPQKL:EPLP-AA$^{17}$-VYY:EQLSN:NMVRS, VPQQL:EPLP-AA$^{17}$-VYY:EQLSN:NMVRS, VPQKL:EPLP-AA$^{17}$-VYY:EQLSN:EGMSVRS, VPQKL:EPLP-AA$^{17}$-VYY:EQLSN:GMVRS, VPQKM:EPLP-AA$^{17}$-VYY:EQLSN:GMVRS, VPQRL:EPLP-AA $^{17}$-VYY:EQLSN:DMVRS, VPQKT :EPLP-AA$^{17}$-VYY:EQLSN:MIVRS, VPQDL:EPLP-AA $^{17}$-VYY:EQLSN:MIVRS, VPQDL:EPLP-AA$^{17}$-VYY:EQLSN:MVVRS, VPTEL:EPLP-AA$^{17}$-VYY:EQLSN:DMVVE, VPEKM:EPLP-AA$^{17}$-VYY:EQLSN:NMTVE, VPTEL:EPLP-AA $^{17}$-VYY:EQLSN:EMVVE, APTKL:EPLP-AA$^{17}$-VYY:EQLSN:NMVVR, APTQL:EPLP-AA$^{17}$-VYY:EQLSN:NMVVR, APTKL:EPLP-AA$^{17}$-VYY:EQLSN:NMVVK, VPTKL:EPLP-AA$^{17}$-VYY:EQLSN:EGMSVAE, VPTKL:EPLP-AA$^{17}$-VYY:EQLSN:GMAVS, TPTKM:EPLP-AA$^{17}$-VYY:EQLSN:GMVVD, VPARL:EPLP-AA$^{17}$-VYY:EQLSN:DMVVE, VPTRL:EPLP-AA$^{17}$-VYY:EQLSN:DMVVE, APVKT :EPLP-AA$^{17}$-VYY:EQLSN:MIVEE, VSQDL:EPLP-AA$^{17}$-VYY:EQLSN:MIVKS, VPQDL:EPLP-AA$^{17}$-VYY:EQLSN:MVVKS, VPTEE:EPLP-AA$^{17}$-VYY:EQLSN:FLQHN, VPTGQ:EPLP-AA$^{17}$-VYY:EQLSN:LEEHS, SRVHH:EPLP-AA$^{17}$-VYY:EQLSN:RLEEH, TQVQL:EPLP-AA$^{17}$-VYY:EQLSN:TLEDH, VSQEL:EPLT-AA$^{17}$-LYY:EQLSN:DMVKS, VSQKM:EPLT-AA$^{17}$-LYY:EQLSN:NMVKS, VSQEL:EPLT-AA$^{17}$-LYY:EQLSN:EMVKS, VSQKL:EPLT-AA$^{17}$-LYY:EQLSN:NMVKS, VSQQL:EPLT-AA$^{17}$-LYY:EQLSN:NMVKS, VSQKL:EPLT-AA$^{17}$-LYY:EQLSN:EGMSVKS, VSQKL:EPLT-AA$^{17}$-LYY:EQLSN:GMVKS, VSQKM:EPLT-AA$^{17}$-LYY:EQLSN:GMVKS, VSQRL:EPLT-AA$^{17}$-LYY:EQLSN:DMVKS, VSQKT:EPLT-AA$^{17}$-LYY:EQLSN:MIVKS, VSQAL:EPLT-AA$^{17}$-LYY:EQLSN:MIVKS, VSQDL:EPLT-AA$^{17}$-LYY:EQLSN:MVVKS, VPTEL:EPLT-AA$^{17}$-LYY:EQLSN:DMVVE, VPEKM:EPLT-AA$^{17}$-LYY:EQLSN:NMTVE, VPTEL:EPLT-AA$^{17}$-LYY:EQLSN:EMVVE, APTKL:EPLT-AA$^{17}$-LYY:EQLSN:NMVVR, APTQL:EPLT-AA$^{17}$-LYY:EQLSN:NMVVR, APTKL:EPLT-AA$^{17}$-LYY:EQLSN:NMVVK, VPTKL:EPLT-AA$^{17}$-LYY:EQLSN:EGMSVAE, VPTKL:EPLT-AA$^{17}$-LYY:EQLSN:GMAVS, TPTKM:EPLT-AA$^{17}$-LYY:EQLSN:GMVVD, VPARL:EPLT-AA$^{17}$-LYY:EQLSN:DMVVE, VPTRL:EPLT-AA$^{17}$-LYY:EQLSN:DMVVE, APVKT:EPLT-AA$^{17}$-LYY:EQLSN:MIVEE, VPQAL:EPLT-AA$^{17}$-LYY:EQLSN:MIVRS, VPTEE:EPLT-AA$^{17}$-LYY:EQLSN:FLQHN, VPTGQ:EPLT-AA$^{17}$-LYY:EQLSN:LEEHS, SRVHH:EPLT-AA$^{17}$-LYY:EQLSN:RLEEH, TQVQL:EPLT-AA$^{17}$-LYY:EQLSN:TLEDH, VPQEL:EPLT-AA$^{17}$-LYY:EQLSN:DMVKS, VPQKM:EPLT-AA$^{17}$-LYY:EQLSN:NMVKS, VPQEL:EPLT-AA$^{17}$-LYY:EQLSN:EMVKS, VPQKL:EPLT-AA$^{17}$-LYY:EQLSN:NMVKS, VPQQL:EPLT-AA$^{17}$-LYY:EQLSN:NMVKS, VPQKL:EPLT-AA$^{17}$-LYY:EQLSN:EGMSVKS, VPQKL:EPLT-AA$^{17}$-LYY:EQLSN:GMVKS, VPQKM:EPLT-AA$^{17}$-LYY:EQLSN:GMVKS, VPQRL:EPLT-AA$^{17}$-LYY:EQLSN:DMVKS, VPQKT:EPLT-AA$^{17}$-LYY:EQLSN:MIVKS, VPQAL:EPLT-AA$^{17}$-LYY:EQLSN:MIVKS, VPQDL:EPLT-AA$^{17}$-LYY:EQLSN:MIVKS, VPTGQ:SNIT-AA$^{17}$-QIM:IGEMS:LEQHN, VPTEL:SNIT-AA$^{17}$-Q,M: IGEMS:DMVVE, VPEKM:SNIT-AA$^{17}$-QIM :IGEMS:NMTVE, VPTEL:SNIT-AA$^{17}$-QIM:IGEMS:EMVVE, APTKL:SNIT-AA$^{17}$-QIM :IGEMS:NMVVR, APTQL:SNIT-AA$^{17}$-QIM:IGEMS:NMVVR, APTKL:SNIT-AA$^{17}$-QIM :IGEMS:NMVVK, VPTKL:SNIT-AA$^{17}$-QIM:IGEMS:EGMSVAE, VPTKL:SNIT-AA$^{17}$-QIM :IGEMS:GMAVS, TPTKM:SNIT-AA$^{17}$-QIM:IGEMS:GMVVD, VPARL:SNIT-AA$^{17}$-QIM:IGEMS:DMVVE, VPTRL:SNIT-AA$^{17}$-QIM:IGEMS:DMVVE, APVKT:SNIT-AA$^{17}$-Q,M:IGEMS:MIVEE, VPQAL:SNIT-AA$^{17}$-QIM:IGEMS:MIVRS, VSQDL:SNIT-AA$^{17}$-QIM:IGEMS:MIVKS, VPQDL:SNIT-AA$^{17}$-QIM:IGEMS:MVVKS, VPTGQ:SNIT-AA$^{17}$-QIM:IGEMS:LEEHS, SRVHH:SNIT-AA$^{17}$-QIM:IGEMS:RLEEH, TQVQL:SNIT-AA$^{17}$-QIM:IGEMS:TLEDH, VPTEE:SNIT-AA$^{17}$-QIM:LGEMS:FLEHS, VPTEL:SNIT-AA$^{17}$-QIM:LGEMS:DMVVE, VPEKM:SNIT-AA$^{17}$-QIM:LGEMS:NMTVE, VPTEL:SNIT-AA $^{17}$–QIM :LGEMS:EMVVE, APTKL:SNIT-AA$^{17}$-QIM :LGEMS:NMVVR, APTQL:SNIT-AA$^{17}$-QIM:LGEMS:NMVVR, APTKL:SNIT-AA$^{17}$-QIM :LGEMS:NMVVK, VPTKL:SNIT-AA$^{17}$-QIM:LGEMS:EGMSVAE, VPTKL:SNIT-AA$^{17}$-QIM:LGEMS:GMAVS,

TPTKM:SNIT-AA$^{17}$-QIM:LGEMS:GMVVD, VPARL:SNIT-AA$^{17}$-QIM:LGEMS:DMVVE, VPTRL:SNIT-AA$^{17}$-QIM:LGEMS:DMVVE, APVKT:SNIT-AA$^{17}$-QIM :LGEMS:MIVEE, VPQAL:SNIT-AA$^{17}$-QIM :LGEMS:MIVRS, VSQDL:SNIT-AA$^{17}$-QIM :LGEMS:MIVKS, VPQDL:SNIT-AA$^{17}$-QIM:LGEMS:MVVKS, VPTEE:SNIT-AA$^{17}$-QIM:LGEMS:FLQHN, SRVHH:SNIT-AA$^{17}$-QIM:LGEMS:RLEEH, TQVQL:SNIT-AA$^{17}$-QIM:LGEMS:TLEDH, SRVQL:RSVK-AA$^{17}$-AKV:KEVQV:TLEEH, VPTEL:RSVK-AA$^{17}$-AKV:KEVQV:DMVVE, VPEKM:RSVK-AA $^{17}$–AKV:KEVQV:NMTVE, VPTEL:RSVK-AA$^{17}$-AKV:KEVQV:EMVVE, APTKL:RSVK-AA$^{17}$-AKV:KEVQV:NMVVR, APTQL:RSVK-AA$^{17}$-AKV:KEVQV:NMVVR, APTKL:RSVK-AA$^{17}$-AKV:KEVQV:NMVVK, VPTKL:RSVK-AA$^{17}$-AKV:KEVQV:EGMSVAE, VPTKL:RSVK-AA$^{17}$-AKV:KEVQV:GMAVS, TPTKM:RSVK-AA$^{17}$-AKV:KEVQV:GMVVD, VPARL:RSVK-AA$^{17}$-AKV:KEVQV:DMVVE, VPTRL:RSVK-AA$^{17}$-AKV:KEVQV:DMVVE, APVKT:RSVK-AA$^{17}$-AKV:KEVQV:MIVEE, VPQAL:RSVK-AA$^{17}$-AKV:KEVQV:MIVRS, VSQDL:RSVK-AA$^{17}$-AKV:KEVQV:MIVKS, VPQDL:RSVK-AA$^{17}$ -AKV:KEVQV:MVVKS, VPTEE:RSVK-AA$^{17}$-AKV:KEVQV:FLQHN, VPTGQ:RSVK-AA$^{17}$-AKV:KEVQV:LEEHS, TQVQL:RSVK-AA$^{17}$-AKV:KEVQV:TLEDH, TQVHH:RPVQ-AA$^{17}$-RKI:KKATV:RLEDH, VPTEL:RPVQ-AA$^{17}$-RKI:KKATV:DMVVE, VPEKM:RPVQ-AA$^{17}$-RKI:KKATV:NMTVE, VPTEL:RPVQ-AA$^{17}$-RKI:KKATV:EMVVE, APTKL:RPVQ-AA$^{17}$-RKI:KKATV:NMVVR, APTQL:RPVQ-AA$^{17}$-RKI:KKATV:NMVVR, APTKL:RPVQ-AA$^{17}$-RKI:KKATV:NMVVK, VPTKL:RPVQ-AA$^{17}$-RKI:KKATV:EGMSVAE, VPTKL:RPVQ-AA$^{17}$-RKI:KKATV:GMAVS, TPTKM:RPVQ-AA$^{17}$-RKI:KKATV:GMVVD, VPARL:RPVQ-AA$^{17}$-RKI:KKATV:DMVVE, VPTRL:RPVQ-AA$^{17}$-RKI:KKATV:DMVVE, APVKT:RPVQ-AA$^{17}$-RKI:KKATV:MIVEE, VPQAL:RPVQ-AA$^{17}$-RKI:KKATV:MIVRS, VSQDL:RPVQ-AA$^{17}$-RKI:KKATV:MIVKS, VPQDL:RPVQ-AA$^{17}$-RKI:KKATV:MVVKS, VPTEE:RPVQ-AA$^{17}$-RKI:KKATV:FLQHN, VPTGQ:RPVQ-AA$^{17}$-RKI:KKATV:LEEHS and SRVHH:RPVQ-AA$^{17}$-RKI:KKATV:RLEEH; and wherein AA$^{17}$-is selected from the group consisting of G, A, V, L, I, P, F, M, W, T and S (in particular is selected from the group consisting of M, I, L, V and T).

In particular, in certain embodiments, the quadruplet PEP9:PEP1:PEP2:PEP10 is selected from the group consisting of GIPEPXXVPTKM:SAIS:LKNYQ:NMVVESXAXR, SIPKAXXVPTEL:SAIS:LKNYQ:EMVVEGXGXR, HVTKPTXVPTKL:SAIS:LKNYQ:NMVVESXGXH, YVPKPXXVPTKL:SAIS:LKNYQ:NMVVESXGXH, TVPKPXXVPTQL:SAIS:LKNYQ:NMVVEAXGXH, AVPKAXXVPTKL:SAIS:LKNYQ:NMVVEAXGXH, KVGKAXXVPTKL:SAIS:LKNYQ:EGMSVVEXGXR, KASKAXXVPTKL:SAIS:LKNYQ:GMVVEEXGXR, GSAGPXXVPTKM:SAIS:LKNYQ:GMVVERXGXS, AAPASXXVPTRL:SAIS:LKNYQ:DMVVEAXGXR, STPPTXXVPTRL:SAIS:LKNYQ:DMVVESXGXR, HVPKPXXVPTKL:SAIS:LKNYQ:NMVVESXGXH, RVPSTXXVPTKT:SAIS:LKNYQ:MIVVEXGXL, ASAAPXXVPTAL:SAIS:LKNYQ:MIVVEXKXS, ASASPXXVPTDL:SAIS:LKNYQ:MIVVEXKXS, ASASPXXVPTDL:SAIS:LKNYQ:MVVVEXKXS, NDEGLEXVPTEE:SAIS:LKNYQ:FLVVEKXEXR, NDEGLEXVPTGQ:SAIS:LKNYQ:LEVVEQXEXR, SSVKXQPVPTHH:SAIS:LKNYQ:RLVVELEXAXA, RNVQXRPVPTQL:SAIS:LKNYQ:TLVVELAXKXE, GIPEPXXVPEKM:SAIS:LKNYQ:NMTVESXAXR, HVTKPTXAPTKL:SAIS:LKNYQ:NMVVRSXGXH, YVPKPXXAPTKL:SAIS:LKNYQ:NMVVRSXGXH, TVPKPXXAPTQL:SAIS:LKNYQ:NMVVRAXGXH, AVPKAXXAPTKL:SAIS:LKNYQ:NMVVKAXGXH, KVGKAXXVPTKL:SAIS:LKNYQ:EGMSVAEXGXR, KASKAXXVPTKL:SAIS:LKNYQ:GMAVSEXGXR, GSAGPXXTPTKM:SAIS:LKNYQ:GMVVDRXGXS, AAPASXXVPARL:SAIS:LKNYQ:DMVVEAXGXR, HVPKPXXAPTKL:SAIS:LKNYQ:NMVVRSXGXH, RVPSTXXAPVKT:SAIS:LKNYQ:MIVEEXGXL, ASAAPXXVPQAL:SAIS:LKNYQ:MIVRSXKXS, ASASPXXVSQDL:SAIS:LKNYQ:MIVKSXKXS, ASASPXXVPQDL:SAIS:LKNYQ:MVVKSXKXS, NDEGLEXVPTEE:SAIS:LKNYQ:FLQHNKXEXR, NDEGLEXVPTGQ:SAIS:LKNYQ:LEEHSQXEXR, SSVKXQPSRVHH:SAIS:LKNYQ:RLEEHLEXAXA, RNVQXRPTQVQL:SAIS:LKNYQ:TLEDHLAXKXE, KIPKAXXVPEEL:SSLS:LKVYP:DMTVEGXGXR, SIPKAXXVPEEL:SSLS:LKVYP:EMTVEGXGXR, HVTKPTXVPEKL:SSLS:LKVYP:NMTVESXGXH, YVPKPXXVPEKL:SSLS:LKVYP:NMTVESXGXH, TVPKPXXVPEQL:SSLS:LKVYP:NMTVEAXGXH, AVPKAXXVPEKL:SSLS:LKVYP:NMTVEAXGXH, KVGKAXXVPEKL:SSLS:LKVYP:EGMSTVEXGXR, KASKAXXVPEKL:SSLS:LKVYP:GMTVEEXGXR, GSAGPXXVPEKM:SSLS:LKVYP:GMTVERXGXS, AAPASXXVPERL:SSLS:LKVYP:DMTVEAXGXR, STPPTXXVPERL:SSLS:LKVYP:DMTVESXGXR, HVPKPXXVPEKL:SSLS:LKVYP:NMTVESXGXH, RVPSTXXVPEKT:SSLS:LKVYP:MITVEXGXL, ASAAPXXVPEAL:SSLS:LKVYP:MITVEXKXS, ASASPXXVPEDL:SSLS:LKVYP:MITVEXKXS, ASASPXXVPEDL:SSLS:LKVYP:MVTVEXKXS, NDEGLEXVPEEE:SSLS:LKVYP:FLTVEKXEXR, NDEGLEXVPEGQ:SSLS:LKVYP:LETVEQXEXR, SSVKXQPVPEHH:SSLS:LKVYP:RLTVELEXAXA, RNVQXRPVPEQL:SSLS:LKVYP:TLTVELAXKXE, KIPKAXXVPTEL:SSLS:LKVYP:DMVVEGXGXR, SIPKAXXVPTEL:SSLS:LKVYP:EMVVEGXGXR, HVTKPTXAPTKL:SSLS:LKVYP:NMVVRSXGXH, YVPKPXXAPTKL:SSLS:LKVYP:NMVVRSXGXH, TVPKPXXAPTQL:SSLS:LKVYP:NMVVRAXGXH, AVPKAXXAPTKL:SSLS:LKVYP:NMVVKAXGXH, KVGKAXXVPTKL:SSLS:LKVYP:EGMSVAEXGXR, KASKAXXVPTKL:SSLS:LKVYP:GMAVSEXGXR, GSAGPXXTPTKM:SSLS:LKVYP:GMVVDRXGXS, AAPASXXVPARL:SSLS:LKVYP:DMVVEAXGXR, STPPTXXVPTRL:SSLS:LKVYP:DMVVESXGXR, HVPKPXXAPTKL:SSLS:LKVYP:NMVVRSXGXH, RVPSTXXAPVKT:SSLS:LKVYP:MIVEEXGXL, ASAAPXXVPQAL:SSLS:LKVYP:M IVRSXKXS, ASASPXXVSQDL:SSLS: LKVYP:M IVKSXKXS, ASASPXXVPQDL:SSLS:LKVYP:MVVKSXKXS, NDEGLEXVPTEE:SSLS:LKVYP:FLQHNKXEXR, NDEGLEXVPTGQ:SSLS:LKVYP:LEEHSQXEXR, SSVKXQPSRVHH:SSLS:LKVYP:RLEEHLEXAXA, RNVQXRPTQVQL:SSLS:LKVYP:TLEDHLAXKXE, KIPKAXXVPTEL:SAIS:LKNYQ:DMVVEGXGXR, KIPKAXXAPTEL:NAIS:LKKYR:DMVVRGXGXR, GIPEPXXAPTKM:NAIS:LKKYR:NMVVRSXAXR, SIPKAXXAPTEL:NAIS:LKKYR:EMVVRGXGXR, YVPKPXXAPTKL:NAIS:LKKYR:NMVVRSXGXH, TVPKPXXAPTQL:NAIS:LKKYR:NMVVRAXGXH, AVPKAXXAPTKL:NAIS:LKKYR:NMVVRAXGXH, KVGKAXXAPTKL:NAIS:LKKYR:EGMSVVRXGXR, KASKAXXAPTKL:NAIS:LKKYR:GMVVREXGXR, GSAGPXXAPTKM:NAIS:LKKYR:GMVVRRXGXS, AAPASXXAPTRL:NAIS:LKKYR:DMVVRAXGXR, STPPTXXAPTRL:NAIS:LKKYR:DMVVRSXGXR, HVPKPXXAPTKL:NAIS:LKKYR:NMVVRSXGXH, RVPSTXXAPTKT:NAIS:LKKYR:M IVVRXGXL, ASAAPXXAPTAL: NAIS:LKKYR:M IVVRXKXS, ASASPXXAPTDL:NAIS:LKKYR:MIVVRXKXS, ASASPXXAPTDL:NAIS:LKKYR:MVVVRXKXS, NDEGLESAPTEE:NAIS:LKKYR:FLVVRKXEXR, NDEGLESAPTGQ:NAIS:LKKYR:LEVVRQXEXR, SSVKXQPAPTHH:NAIS:LKKYR:RLVVRLEXAXA, RNVQXRPAPTQL:NAIS:LKKYR:TLVVRLAXKXE, KIPKAXXVPTEL:NAIS:LKKYR:DMVVEGXGXR, GIPEPXXVPEKM:NAIS:LKKYR:NMTVESXAXR, SIPKAXXVPTEL:NAIS:LKKYR:EMVVEGXGXR, AVPKAXXAPTKL:NAIS:LKKYR:NMVVKAXGXH, KVGKAXXVPTKL:NAIS:LKKYR:EGMSVAEXGXR, KASKAXXVPTKL:NAIS:LKKYR:GMAVSEXGXR, GSAGPXXTPTKM:NAIS:LKKYR:GMVVDRXGXS, AAPASXXVPARL:NAIS:LKKYR:DMVVEAXGXR, STPPTXXVPTRL:NAIS:LKKYR:DMVVESXGXR, RVPSTXXAPVKT:NAIS:LKKYR:MIVEEXGXL, ASAAPXXVPQAL:NAIS: LKKYR: MIVRSXKXS, ASASPXXVSQDL:NAIS: LKKYR:MIVKSXKXS, ASASPXXVPQDL:NAIS:LKKYR:MVVKSXKXS, NDEGLEXVPTEE:NAIS:LKKYR:FLQHNKXEXR, NDEGLEXVPTGQ:NAIS:LKKYR:LEEHSQXEXR, SSVKXQPSRVHH:NAIS:LKKYR:RLEEHLEXAXA, RNVQXRPTQVQL:NAIS:LKKYR:TLEDHLAXKXE, HVTKPTXAPTKL:NAIS:LKKYR:NMVVRSXGXH, KIPKAXXAPTEL:SATS:LRKHR:DMVVKGXGXR, GIPEPXXAPTKM:SATS:LRKHR:NMVVKSXAXR, SIPKAXXAPTEL:SATS:LRKHR:EMVVKGXGXR, HVTKPTXAPTKL:SATS:LRKHR:NMVVKSXGXH, YVPKPXXAPTKL:SATS:LRKHR:NMVVKSXGXH, TVPKPXXAPTQL:SATS:LRKHR:NMVVKAXGXH, KVGKAXXAPTKL:SATS:LRKHR:EGMSVVKXGXR, KASKAXXAPTKL:SATS:LRKHR:GMVVKEXGXR, GSAGPXXAPTKM:SATS:LRKHR:GMVVKRXGXS, AAPASXXAPTRL:SATS:LRKHR:DMVVKAXGXR, STPPTXXAPTRL:SATS:LRKHR:DMVVKSXGXR, HVPKPXXAPTKL:SATS:LRKHR:NMVVKSXGXH, RVPSTXXAPTKT:SATS:LRKHR:MIVVKXGXL, ASAAPXXAPTAL:SATS:LRKHR:MIVVKXKXS, ASASPXXAPTDL:SATS:LRKHR:MIVVKXKXS, ASASPXXAPTDL:SATS:LRKHR:MVVVKXKXS, NDEGLESAPTEE:SATS:LRKHR:FLVVKKXEXR, NDEGLESAPTGQ:SATS:LRKHR:LEVVKQXEXR, SSVKXQPAPTHH:SATS:LRKHR:RLVVKLEXAXA, RNVQXRPAPTQL:SATS:LRKHR:TLVVKLAXKXE, KIPKAXXVPTEL:SATS:LRKHR:DMVVEGXGXR, GIPEPXXVPEKM:SATS:LRKHR:NMTVESXAXR, SIPKAXXVPTEL:SATS:LRKHR:EMVVEGXGXR, HVTKPTXAPTKL:SATS:LRKHR:NMVVRSXGXH, YVPKPXXAPTKL:SATS:LRKHR:NMVVRSXGXH, TVPKPXXAPTQL:SATS:LRKHR:NMVVRAXGXH, KVGKAXXVPTKL:SATS:LRKHR:EGMSVAEXGXR, KASKAXXVPTKL:SATS:LRKHR:GMAVSEXGXR, GSAGPXXTPTKM:SATS:LRKHR:GMVVDRXGXS, AAPASXXVPARL:SATS:LRKHR:DMVVEAXGXR, STPPTXXVPTRL:SATS:LRKHR:DMVVESXGXR, HVPKPXXAPTKL:SATS:LRKHR:NMVVRSXGXH, RVPSTXXAPVKT:SATS: LRKH R: MIVEEXGXL, ASAAPXXVPQAL:SATS:LRKHR:MIVRSXKXS, ASASPXXVSQDL:SATS:LRKHR:MIVKSXKXS, ASASPXXVPQDL:SATS:LRKHR:MVVKSXKXS, NDEGLEXVPTEE:SATS:LRKHR:FLQHNKXEXR, NDEGLEXVPTGQ:SATS:LRKHR:LEEHSQXEXR, SSVKXQPSRVHH:SATS:LRKHR:RLEEHLEXAXA, RNVQXRPTQVQL:SATS:LRKHR:TLEDHLAXKXE, KIPKAXXVPTEL:SPIS:LKYHY:DMVAEGXGXR, GIPEPXXVPTKM:SPIS:LKYHY:NMVAESXAXR, SIPKAXXVPTEL:SPIS:LKYHY:EMVAEGXGXR, HVTKPTXVPTKL:SPIS:LKYHY:NMVAESXGXH, YVPKPXXVPTKL:SPIS:LKYHY:NMVAESXGXH, TVPKPXXVPTQL:SPIS:LKYHY:NMVAEAXGXH, AVPKAXXVPTKL:SPIS:LKYHY:NMVAEAXGXH, KASKAXXVPTKL:SPIS:LKYHY:GMVAEEXGXR, GSAGPXXVPTKM:SPIS:LKYHY:GMVAERXGXS, AAPASXXVPTRL:SPIS:LKYHY:DMVAEAXGXR, STPPTXXVPTRL:SPIS:LKYHY:DMVAESXGXR, HVPKPXXVPTKL:SPIS:LKYHY:NMVAESXGXH, RVPSTXXVPTKT:SPIS:LKYHY:MIVAEXGXL, ASAAPXXVPTAL:SPIS:LKYHY:MIVAEXKXS, ASASPXXVPTDL:SPIS:LKYHY:MIVAEXKXS, ASASPXXVPTDL:SPIS:LKYHY:MVVAEXKXS, NDEGLEXVPTEE:SPIS:LKYHY:FLVAEKXEXR, NDEGLEXVPTGQ:SPIS:LKYHY:LEVAEQXEXR, SSVKXQPVPTHH:SPIS:LKYHY:RLVAELEXAXA, RNVQXRPVPTQL:SPIS:LKYHY:TLVAELAXKXE, KIPKAXXVPTEL:SPIS:LKYHY:DMVVEGXGXR, GIPEPXXVPEKM:SPIS:LKYHY:NMTVESXAXR, SIPKAXXVPTEL:SPIS:LKYHY:EMVVEGXGXR, HVTKPTXAPTKL:SPIS:LKYHY:NMVVRSXGXH, YVPKPXXAPTKL:SPIS:LKYHY:NMVVRSXGXH, TVPKPXXAPTQL:SPIS:LKYHY:NMVVRAXGXH, AVPKAXXAPTKL:SPIS:LKYHY:NMVVKAXGXH, KASKAXXVPTKL:SPIS:LKYHY:GMAVSEXGXR, GSAGPXXTPTKM:SPIS:LKYHY:GMVVDRXGXS, AAPASXXVPARL:SPIS:LKYHY:DMVVEAXGXR, STPPTXXVPTRL:SPIS:LKYHY:DMVVESXGXR, HVPKPXXAPTKL:SPIS:LKYHY:NMVVRSXGXH, RVPSTXXAPVKT:SPIS:LKYHY:MIVEEXGXL, ASAAPXXVPQAL:SPIS:LKYHY:MIVRSXKXS, ASASPXXVSQDL:SPIS:LKYHY:MIVKSXKXS, ASASPXXVPQDL:SPIS:LKYHY:MVVKSXKXS, NDEGLEXVPTEE:SPIS:LKYHY:FLQHNKXEXR, NDEGLEXVPTGQ:SPIS:LKYHY:LEEHSQXEXR, SSVKXQPSRVHH:SPIS:LKYHY:RLEEHLEXAXA, RNVQXRPTQVQL:SPIS:LKYHY:TLEDHLAXKXE, KIPKAXXVPTEL:EPIS:KFKYE:DMAVXGXGSR, GIPEPXXVPTKM:EPIS:KFKYE:NMAVSSXAXR, SIPKAXXVPTEL:EPIS:KFKYE:EMAVXGXGSR, HVTKPTXVPTKL:EPIS:KFKYE:NMAVSSXGXH, YVPKPXXVPTKL:EPIS:KFKYE:NMAVSSXGXH, TVPKPXXVPTQL:EPIS:KFKYE:NMAVSAXGXH, AVPKAXXVPTKL:EPIS:KFKYE:NMAVSAXGXH, KVGKAXXVPTKL:EPIS:KFKYE:EGMSAVSXGXR, GSAGPXXVPTKM:EPIS:KFKYE:GMAVSRXGXS, AAPASXXVPTRL:EPIS:KFKYE:DMAVSAXGXR, STPPTXXVPTRL:EPIS:KFKYE:DMAVSSXGXR, HVPKPXXVPTKL:EPIS:KFKYE:NMAVSSXGXH, RVPSTXXVPTKT:EPIS:KFKYE:MIAVSXGXL, ASAAPXXVPTAL:EPIS:KFKYE:MIAVSXKXS, ASASPXXVPTDL:EPIS:KFKYE:MIAVSXKXS, ASASPXXVPTDL:EPIS:KFKYE:MVAVSXKXS, NDEGLEXVPTEE:EPIS:KFKYE:FLAVXKXESR, NDEGLEXVPTGQ:EPIS:KFKYE:LEAVSQXEXR, SSVKXQPVPTHH:EPIS:KFKYE:RLAVSLEXAXA, RNVQXRPVPTQL:EPIS:KFKYE:TLAVSLAXKXE, KIPKAXXVPTEL:EPIS:KFKYE:DMVVEGXGXR, GIPEPXXVPEKM:EPIS:KFKYE:NMTVESXAXR, SIPKAXXVPTEL:EPIS:KFKYE:EMVVEGXGXR, HVTKPTXAPTKL:EPIS:KFKYE:NMVVRSXGXH, YVPKPXXAPTKL:EPIS:KFKYE:NMVVRSXGXH, TVPKPXXAPTQL:EPIS:KFKYE:NMVVRAXGXH, AVPKAXXAPTKL:EPIS:KFKYE:NMVVKAXGXH, KVGKAXXVPTKL:EPIS:KFKYE:EGMSVAEXGXR, GSAGPXXTPTKM:EPIS:KFKYE:GMVVDRXGXS, AAPASXXVPARL:EPIS:KFKYE:DMVVEAXGXR, STPPTXXVPTRL:EPIS:KFKYE:DMVVESXGXR, HVPKPXXAPTKL:EPIS:KFKYE:NMVVRSXGXH, RVPSTXXAPVKT:EPIS:KFKYE:MIVEEXGXL, ASAAPXXVPQAL:EPIS:KFKYE:MIVRSXKXS, ASASPXXVSQDL:EPIS:KFKYE:MIVKSXKXS, ASASPXXVPQDL:EPIS:KFKYE:MVVKSXKXS, NDEGLEXVPTEE:EPIS:KFKYE:FLQHNKXEXR, NDEGLEXVPTGQ:EPIS:KFKYE:LEEHSQXEXR, SSVKXQPSRVHH:EPIS:KFKYE:RLEEHLEXAXA, RNVQXRPTQVQL:EPIS:KFKYE:TLEDHLAXKXE, KIPKAXXTPTEL:SPIN:YGKIP:DMVVDGXGXR, GIPEPXXTPTKM:SPIN:YGKI P: NMVVDSXAXR, SIPKAXXTPTEL:SPIN:YGKIP:EMVVDGXGXR, HVTKPTXTPTKL:SPIN:YGKIP:NMVVDSXGXH, YVPKPXXTPTKL:SPIN:YGKIP:NMVVDSXGXH, TVPKPXXTPTQL:SPIN:YGKIP:NMVVDAXGXH, AVPKAXXTPTKL:SPIN:YGKIP:NMVVDAXGXH, KVGKAXXTPTKL:SPIN:YGKIP:EGMSVVDXGXR, KASKAXXTPTKL:SPIN:YGKIP:G MVVDEXGXR, AAPASXXTPTRL:SPI N:YGKIP:DMVVDAXGXR, STPPTXXTPTRL:SPIN:YGKIP:DMVVDSXGXR, HVPKPXXTPTKL:SPIN:YGKIP:NMVVDSXGXH, RVPSTXXTPTKT:SPIN:YGKIP:MIVVDXGXL, ASAAPXXTPTAL:SPIN:YGKIP:MIVVDXKXS, ASASPXXTPTDL:SPIN:YGKIP:MIVVDXKXS, ASASPXXTPTDL:SPIN:YGKIP:MVVVDXKXS, NDEGLESTPTEE:SPIN:YGKIP:FLVVDKXEXR, NDEGLESTPTGQ:SPIN:YGKIP:LEVVDQXEXR, SSVKXQPTPTHH:SPIN:YGKIP:RLVVDLEXAXA, RNVQXRPTPTQL:SPIN:YGKIP:TLVVDLAXKXE, KIPKAXXVPTEL:SPIN:YGKIP:DMVVEGXGXR, GIPEPXXVPEKM:SPIN:YGKIP:NMTVESXAXR, SIPKAXXVPTEL:SPIN:YGKIP:EMVVEGXGXR, HVTKPTXAPTKL:SPIN:YGKIP:NMVVRSXGXH, YVPKPXXAPTKL:SPIN:YGKIP:NMVVRSXGXH, TVPKPXXAPTQL:SPIN:YGKIP:NMVVRAXGXH, AVPKAXXAPTKL:SPIN:YGKIP:NMVVKAXGXH, KVGKAXXVPTKL:SPIN:YGKIP:EGMSVAEXGXR, KASKAXXVPTKL:SPIN:YGKIP:GMAVSEXGXR, AAPASXXVPARL:SPIN:YGKIP:DMVVEAXGXR, STPPTXXVPTRL:SPIN:YGKIP:DMVVESXGXR, HVPKPXXAPTKL:SPIN:YGKIP:NMVVRSXGXH, RVPSTXXAPVKT:SPIN:YGKIP:MIVEEXGXL, ASAAPXXVPQAL:SPIN:YGKIP:MIVRSXKXS, ASASPXXVSQDL:SPIN:YGKIP:MIVKSXKXS, ASASPXXVPQDL:SPIN:YGKIP:MVVKSXKXS, NDEGLEXVPTEE:SPIN:YGKIP:FLQHNKXEXR, NDEGLEXVPTGQ:SPIN:YGKIP:LEEHSQXEXR, SSVKXQPSRVHH:SPIN:YGKIP:RLEEHLEXAXA, RNVQXRPTQVQL:SPIN:YGKIP:TLEDHLAXKXE, KIPKAXXVPAEL:SPIS:YKQYE:DMVVEGXGXR, GIPEPXXVPAKM:SPIS:YKQYE:NMVVESXAXR, SIPKAXXVPAEL:SPIS:YKQYE:EMVVEGXGXR, HVTKPTXVPAKL:SPIS:YKQYE:NMVVESXGXH, YVPKPXXVPAKL:SPIS:YKQYE:NMVVESXGXH, TVPKPXXVPAQL:SPIS:YKQYE:NMVVEAXGXH, AVPKAXXVPAKL:SPIS:YKQYE:NMVVEAXGXH, KVGKAXXVPAKL:SPIS:YKQYE:EGMSVVEXGXR, KASKAXXVPAKL:SPIS:YKQYE:GMVVEEXGXR, GSAGPXXVPAKM:SPIS:YKQYE:GMVVERXGXS, STPPTXXVPARL:SPIS:YKQYE:DMVVESXGXR, HVPKPXXVPAKL:SPIS:YKQYE:NMVVESXGXH, RVPSTXXVPAKT:SPIS:YKQYE:M IVVEXGXL, ASAAPXXVPAAL:SPIS:YKQYE:MIVVEXKXS, ASASPXXVPADL:SPIS:YKQYE:MIVVEXKXS, ASASPXXVPADL:SPIS:YKQYE:MVVVEXKXS, NDEGLEXVPAEE:SPIS:YKQYE:FLVVEKXEXR, NDEGLEXVPAGQ:SPIS:YKQYE:LEVVEQXEXR, SSVKXQPVPAHH:SPIS:YKQYE:RLVVELEXAXA, RNVQXRPVPAQL:SPIS:YKQYE:TLVVELAXKXE, KIPKAXXVPTEL:SPIS:YKQYE:DMVVEGXGXR, GIPEPXXVPEKM:SPIS:YKQYE:NMTVESXAXR, SIPKAXXVPTEL:SPIS:YKQYE:EMVVEGXGXR, HVTKPTXAPTKL:SPIS:YKQYE:NMVVRSXGXH, YVPKPXXAPTKL:SPIS:YKQYE:NMVVRSXGXH, TVPKPXXAPTQL:SPIS:YKQYE:NMVVRAXGXH, AVPKAXXAPTKL:SPIS:YKQYE:NMVVKAXGXH, KVGKAXXVPTKL:SPIS:YKQYE:EGMSVAEXGXR, KASKAXXVPTKL:SPIS:YKQYE:GMAVSEXGXR, GSAGPXXTPTKM:SPIS:YKQYE:GMVVDRXGXS, STPPTXXVPTRL:SPIS:YKQYE:DMVVESXGXR, HVPKPXXAPTKL:SPIS:YKQYE:NMVVRSXGXH, RVPSTXXAPVKT:SPIS:YKQYE:MIVEEXGXL, ASAAPXXVPQAL:SPIS:YKQYE:MIVRSXKXS, ASASPXXVSQDL:SPIS:YKQYE:MIVKSXKXS, ASASPXXVPQDL:SPIS:YKQYE:MVVKSXKXS, NDEGLEXVPTEE:SPIS:YKQYE:FLQHNKXEXR, NDEGLEXVPTGQ:SPIS:YKQYE:LEEHSQXEXR, SSVKXQPSRVHH:SPIS:YKQYE:RLEEHLEXAXA, RNVQXRPTQVQL:SPIS:YKQYE:TLEDHLAXKXE, GIPEPXXVPTKM:SPIS:YKQYE:NMVVESXAXR, HVTKPTXVPTKL:SPIS:YKQYE:NMVVESXGXH, YVPKPXXVPTKL:SPIS:YKQYE:NMVVESXGXH, TVPKPXXVPTQL:SPIS:YKQYE:NMVVEAXGXH, AVPKAXXVPTKL:SPIS:YKQYE:NMVVEAXGXH, KVGKAXXVPTKL:SPIS:YKQYE:EGMSVVEXGXR,

KASKAXXVPTKL:SPIS:YKQYE:GMVVEEXGXR, GSAGPXXVPTKM:SPIS:YKQYE:GMVVERXGXS, AAPASXXVPTRL:SPIS:YKQYE:DMVVEAXGXR, HVPKPXXVPTKL:SPIS:YKQYE:NMVVESXGXH, RVPSTXXVPTKT:SPIS:YKQYE:MIVVEXGXL, ASAAPXXVPTAL:SPIS:YKQYE:MIVVEXKXS, ASASPXXVPTDL:SPIS:YKQYE:MIVVEXKXS, ASASPXXVPTDL:SPIS:YKQYE:MVVVEXKXS, NDEGLEXVPTEE:SPIS:YKQYE:FLVVEKXEXR, NDEGLEXVPTGQ:SPIS:YKQYE:LEVVEQXEXR, SSVKXQPVPTHH:SPIS:YKQYE:RLVVELEXAXA, RNVQXRPVPTQL:SPIS:YKQYE:TLVVELAXKXE, AAPASXXVPARL:SPIS:YKQYE:DMVVEAXGXR, KIPKAXXAPVEL:KPLS:DHHKD:DMVEEGXGXR, GIPEPXXAPVKM:KPLS:DHHKD:NMVEESXAXR, SIPKAXXAPVEL:KPLS:DHHKD:EMVEEGXGXR, HVTKPTXAPVKL:KPLS:DHHKD:NMVEESXGXH, YVPKPXXAPVKL:KPLS:DHHKD:NMVEESXGXH, TVPKPXXAPVQL:KPLS:DHHKD:NMVEEAXGXH, AVPKAXXAPVKL:KPLS:DHHKD:NMVEEAXGXH, KVGKAXXAPVKL:KPLS:DHHKD:EGMSVEEXGXR, KASKAXXAPVKL:KPLS:DHHKD:GMVEEEXGXR, GSAGPXXAPVKM:KPLS:DHHKD:GMVEERXGXS, AAPASXXAPVRL:KPLS:DHHKD:DMVEEAXGXR, STPPTXXAPVRL:KPLS:DHHKD:DMVEESXGXR, HVPKPXXAPVKL:KPLS:DHHKD:NMVEESXGXH, ASAAPXXAPVAL:KPLS:DHHKD:MIVEEXKXS, ASASPXXAPVDL:KPLS:DHHKD:MIVEEXKXS, ASASPXXAPVDL:KPLS:DHHKD:MVVEEXKXS, NDEGLESAPVEE:KPLS:DHHKD:FLVEEKXEXR, NDEGLESAPVGQ:KPLS:DHHKD:LEVEEQXEXR, SSVKXQPAPVHH:KPLS:DHHKD:RLVEELEXAXA, RNVQXRPAPVQL:KPLS:DHHKD:TLVEELAXKXE, KIPKAXXVPTEL:KPLS:DHHKD:DMVVEGXGXR, GIPEPXXVPEKM:KPLS:DHHKD:NMTVESXAXR, SIPKAXXVPTEL:KPLS:DHHKD:EMVVEGXGXR, HVTKPTXAPTKL:KPLS:DHHKD:NMVVRSXGXH, YVPKPXXAPTKL:KPLS:DHHKD:NMVVRSXGXH, TVPKPXXAPTQL:KPLS:DHHKD:NMVVRAXGXH, AVPKAXXAPTKL:KPLS:DHHKD:NMVVKAXGXH, KVGKAXXVPTKL:KPLS:DHHKD:EGMSVAEXGXR, KASKAXXVPTKL:KPLS:DHHKD:GMAVSEXGXR, GSAGPXXTPTKM:KPLS:DHHKD:GMVVDRXGXS, AAPASXXVPARL:KPLS:DHHKD:DMVVEAXGXR, STPPTXXVPTRL:KPLS:DHHKD:DMVVESXGXR, HVPKPXXAPTKL:KPLS:DHHKD:NMVVRSXGXH, ASAAPXXVPQAL:KPLS:DHHKD:MIVRSXKXS, ASASPXXVSQDL:KPLS:DHHKD:MIVKSXKXS, ASASPXXVPQDL:KPLS:DHHKD:MVVKSXKXS, NDEGLEXVPTEE:KPLS:DHHKD:FLQHNKXEXR, NDEGLEXVPTGQ:KPLS:DHHKD:LEEHSQXEXR, SSVKXQPSRVHH:KPLS:DHHKD:RLEEHLEXAXA, RNVQXRPTQVQL:KPLS:DHHKD:TLEDHLAXKXE, KIPKAXXVPQEL:EPLP:EQLSN:DMVRXGXGSR, GIPEPXXVPQKM:EPLP:EQLSN:NMVRSSXAXR, SIPKAXXVPQEL:EPLP:EQLSN:EMVRXGXGSR, HVTKPTXVPQKL:EPLP:EQLSN:NMVRSSXGXH, YVPKPXXVPQKL:EPLP:EQLSN:NMVRSSXGXH, TVPKPXXVPQQL:EPLP:EQLSN:NMVRSAXGXH, AVPKAXXVPQKL:EPLP:EQLSN:NMVRSAXGXH, KVGKAXXVPQKL:EPLP:EQLSN:EGMSVRSXGXR, KASKAXXVPQKL:EPLP:EQLSN:GMVRSEXGXR, GSAGPXXVPQKM:EPLP:EQLSN:GMVRSRXGXS, AAPASXXVPQRL:EPLP:EQLSN:DMVRSAXGXR, STPPTXXVPQRL:EPLP:EQLSN:DMVRSSXGXR, HVPKPXXVPQKL:EPLP:EQLSN:NMVRSSXGXH, RVPSTXXVPQKT:EPLP:EQLSN:MIVRSXGXL, ASASPXXVPQDL:EPLP:EQLSN:MIVRSXKXS, ASASPXXVPQDL:EPLP:EQLSN:MVVRSXKXS, NDEGLEXVPQEE:EPLP:EQLSN:FLVRXKXESR, NDEGLEXVPQGQ:EPLP:EQLSN:LEVRSQXEXR, SSVKXQPVPQHH:EPLP:EQLSN:RLVRSLEXAXA, RNVQXRPVPQQL:EPLP:EQLSN:TLVRSLAXKXE, KIPKAXXVPTEL:EPLP:EQLSN:DMVVEGXGXR, GIPEPXXVPEKM:EPLP:EQLSN:NMTVESXAXR, SIPKAXXVPTEL:EPLP:EQLSN:EMVVEGXGXR, HVTKPTXAPTKL:EPLP:EQLSN:NMVVRSXGXH, YVPKPXXAPTKL:EPLP:EQLSN:NMVVRSXGXH, TVPKPXXAPTQL:EPLP:EQLSN:NMVVRAXGXH, AVPKAXXAPTKL:EPLP:EQLSN:NMVVKAXGXH, KVGKAXXVPTKL:EPLP:EQLSN:EGMSVAEXGXR, KASKAXXVPTKL:EPLP:EQLSN:GMAVSEXGXR, GSAGPXXTPTKM:EPLP:EQLSN:GMVVDRXGXS, AAPASXXVPARL:EPLP:EQLSN:DMVVEAXGXR, STPPTXXVPTRL:EPLP:EQLSN:DMVVESXGXR, HVPKPXXAPTKL:EPLP:EQLSN:NMVVRSXGXH, RVPSTXXAPVKT:EPLP:EQLSN:MIVEEXGXL, ASASPXXVSQDL:EPLP:EQLSN:MIVKSXKXS, ASASPXXVPQDL:EPLP:EQLSN:MVVKSXKXS, NDEGLEXVPTEE:EPLP:EQLSN:FLQHNKXEXR, NDEGLEXVPTGQ:EPLP:EQLSN:LEEHSQXEXR, SSVKXQPSRVHH:EPLP:EQLSN:RLEEHLEXAXA, RNVQXRPTQVQL:EPLP:EQLSN:TLEDHLAXKXE, KIPKAXXVSQEL:EPLT:EQLSN:DMVKXGXGSR, GIPEPXXVSQKM:EPLT:EQLSN:NMVKSSXAXR, SIPKAXXVSQEL:EPLT:EQLSN:EMVKXGXGSR, HVTKPTXVSQKL:EPLT:EQLSN:NMVKSSXGXH, YVPKPXXVSQKL:EPLT:EQLSN:NMVKSSXGXH, TVPKPXXVSQQL:EPLT:EQLSN:NMVKSAXGXH, AVPKAXXVSQKL:EPLT:EQLSN:NMVKSAXGXH, KVGKAXXVSQKL:EPLT:EQLSN:EGMSVKSXGXR, KASKAXXVSQKL:EPLT:EQLSN:GMVKSEXGXR, GSAGPXXVSQKM:EPLT:EQLSN:GMVKSRXGXS, AAPASXXVSQRL:EPLT:EQLSN:DMVKSAXGXR, STPPTXXVSQRL:EPLT:EQLSN:DMVKSSXGXR, HVPKPXXVSQKL:EPLT:EQLSN:NMVKSSXGXH, RVPSTXXVSQKT:EPLT:EQLSN:MIVKSXGXL, ASAAPXXVSQAL:EPLT:EQLSN:MIVKSXKXS, ASASPXXVSQDL:EPLT:EQLSN:MVVKSXKXS, NDEGLEXVSQEE:EPLT:EQLSN:FLVKXKXESR, NDEGLEXVSQGQ:EPLT:EQLSN:LEVKSQXEXR, SSVKXQPVSQHH:EPLT:EQLSN:RLVKSLEXAXA, RNVQXRPVSQQL:EPLT:EQLSN:TLVKSLAXKXE, KIPKAXXVPTEL:EPLT:EQLSN:DMVVEGXGXR, GIPEPXXVPEKM:EPLT:EQLSN:NMTVESXAXR, SIPKAXXVPTEL:EPLT:EQLSN:EMVVEGXGXR, HVTKPTXAPTKL:EPLT:EQLSN:NMVVRSXGXH, YVPKPXXAPTKL:EPLT:EQLSN:NMVVRSXGXH, TVPKPXXAPTQL:EPLT:EQLSN:NMVVRAXGXH, AVPKAXXAPTKL:EPLT:EQLSN:NMVVKAXGXH, KVGKAXXVPTKL:EPLT:EQLSN:EGMSVAEXGXR, KASKAXXVPTKL:EPLT:EQLSN:GMAVSEXGXR, GSAGPXXTPTKM:EPLT:EQLSN:GMVVDRXGXS, AAPASXXVPARL:EPLT:EQLSN:DMVVEAXGXR, STPPTXXVPTRL:EPLT:EQLSN:DMVVESXGXR, HVPKPXXAPTKL:EPLT:EQLSN:NMVVRSXGXH, RVPSTXXAPVKT:EPLT:EQLSN:MIVEEXGXL, ASAAPXXVPQAL:EPLT:EQLSN:MIVRSXKXS, ASASPXXVPQDL:EPLT:EQLSN:MVVKSXKXS, NDEGLEXVPTEE:EPLT:EQLSN:FLQHNKXEXR, NDEGLEXVPTGQ:EPLT:EQLSN:LEEHSQXEXR, SSVKXQPSRVHH:EPLT:EQLSN:RLEEHLEXAXA, RNVQXRPTQVQL:EPLT:EQLSN:TLEDHLAXKXE, KIPKAXXVPQEL:EPLT:EQLSN:DMVKXGXGSR, GIPEPXXVPQKM:EPLT:EQLSN:NMVKSSXAXR, SIPKAXXVPQEL:EPLT:EQLSN:EMVKXGXGSR, HVTKPTXVPQKL:EPLT:EQLSN:NMVKSSXGXH, YVPKPXXVPQKL:EPLT:EQLSN:NMVKSSXGXH, TVPKPXXVPQQL:EPLT:EQLSN:NMVKSAXGXH, AVPKAXXVPQKL:EPLT:EQLSN:NMVKSAXGXH, KVGKAXXVPQKL:EPLT:EQLSN:EGMSVKSXGXR, KASKAXXVPQKL:EPLT:EQLSN:GMVKSEXGXR, GSAGPXXVPQKM:EPLT:EQLSN:GMVKSRXGXS, AAPASXXVPQRL:EPLT:EQLSN:DMVKSAXGXR, STPPTXXVPQRL:EPLT:EQLSN:DMVKSSXGXR, HVPKPXXVPQKL:EPLT:EQLSN:NMVKSSXGXH, RVPSTXXVPQKT:EPLT:EQLSN:MIVKSXGXL, ASAAPXXVPQAL:EPLT:EQLSN:MIVKSXKXS, ASASPXXVPQDL:EPLT:EQLSN:MIVKSXKXS, NDEGLEXVPQEE:EPLT:EQLSN:FLVKXKXESR, NDEGLEXVPQGQ:EPLT:EQLSN:LEVKSQXEXR, SSVKXQPVPQHH:EPLT:EQLSN:RLVKSLEXAXA, RNVQXRPVPQQL:EPLT:EQLSN:TLVKSLAXKXE, ASASPXXVSQDL:EPLT:EQLSN:MIVKSXKXS, KIPKAXXVPTEL:SNIT:IGEMS:DMQHNGXGXR, GIPEPXXVPTKM:SNIT:IGEMS:NMQHNSXAXR, SIPKAXXVPTEL:SNIT:IGEMS:EMQHNGXGXR, HVTKPTXVPTKL:SNIT:IGEMS:NMQHNSXGXH, YVPKPXXVPTKL:SNIT:IGEMS:NMQHNSXGXH, TVPKPXXVPTQL:SNIT:IGEMS:NMQHNAXGXH, AVPKAXXVPTKL:SNIT:IGEMS:NMQHNAXGXH, KVGKAXXVPTKL:SNIT:IGEMS:EGMSQHNXGXR, KASKAXXVPTKL:SNIT:IGEMS:GMQHNEXGXR, GSAGPXXVPTKM:SNIT:IGEMS:GMQHNRXGXS, AAPASXXVPTRL:SNIT:IGEMS:DMQHNAXGXR, STPPTXXVPTRL:SNIT:IGEMS:DMQHNSXGXR, HVPKPXXVPTKL:SNIT:IGEMS:NMQHNSXGXH, RVPSTXXVPTKT:SNIT:IGEMS:MIQHNXGXL, ASAAPXXVPTAL:SNIT:IGEMS:MIQHNXKXS, ASASPXXVPTDL:SNIT:IGEMS:MIQHNXKXS, ASASPXXVPTDL:SNIT:IGEMS:MVQHNXKXS, NDEGLEXVPTGQ:SNIT:IGEMS:LEQHNQXEXR, NDEGLEXVPTEE:SNIT:IGEMS:FLQHNKXEXR, SSVKXQPVPTHH:SNIT:IGEMS:RLQHNLEXAXA, RNVQXRPVPTQL:SNIT:IGEMS:TLQHNLAXKXE, KIPKAXXVPTEL:SNIT:IGEMS:DMVVEGXGXR, GIPEPXXVPEKM:SNIT:IGEMS:NMTVESXAXR, SIPKAXXVPTEL:SNIT:IGEMS:EMVVEGXGXR, HVTKPTXAPTKL:SNIT:IGEMS:NMVVRSXGXH, YVPKPXXAPTKL:SNIT:IGEMS:NMVVRSXGXH, TVPKPXXAPTQL:SNIT:IGEMS:NMVVRAXGXH, AVPKAXXAPTKL:SNIT:IGEMS:NMVVKAXGXH, KVGKAXXVPTKL:SNIT:IGEMS:EGMSVAEXGXR, KASKAXXVPTKL:SNIT:IGEMS:GMAVSEXGXR, GSAGPXXTPTKM:SNIT:IGEMS:GMVVDRXGXS, AAPASXXVPARL:SNIT:IGEMS:DMVVEAXGXR, STPPTXXVPTRL:SNIT:IGEMS:DMVVESXGXR, HVPKPXXAPTKL:SNIT:IGEMS:NMVVRSXGXH, RVPSTXXAPVKT:SNIT:IGEMS:MIVEEXGXL, ASAAPXXVPQAL:SNIT:IGEMS:MIVRSXKXS, ASASPXXVSQDL:SNIT:IGEMS:MIVKSXKXS, ASASPXXVPQDL:SNIT:IGEMS:MVVKSXKXS, NDEGLEXVPTGQ:SNIT:IGEMS:LEEHSQXEXR, SSVKXQPSRVHH:SNIT:IGEMS:RLEEHLEXAXA, RNVQXRPTQVQL:SNIT:IGEMS:TLEDHLAXKXE, KIPKAXXVPTEL:SNIT:LGEMS:DMEHXGXGSR, GIPEPXXVPTKM:SNIT:LGEMS:NMEHSSXAXR, SIPKAXXVPTEL:SNIT:LGEMS:EMEHXGXGSR, HVTKPTXVPTKL:SNIT:LGEMS:NMEHSSXGXH, YVPKPXXVPTKL:SNIT:LGEMS:NMEHSSXGXH, TVPKPXXVPTQL:SNIT:LGEMS:NMEHSAXGXH, AVPKAXXVPTKL:SNIT:LGEMS:NMEHSAXGXH, KVGKAXXVPTKL:SNIT:LGEMS:EGMSEHSXGXR, KASKAXXVPTKL:SNIT:LGEMS:GMEHSEXGXR, GSAGPXXVPTKM:SNIT:LGEMS:GMEHSRXGXS, AAPASXXVPTRL:SNIT:LGEMS:DMEHSAXGXR, STPPTXXVPTRL:SNIT:LGEMS:DMEHSSXGXR, HVPKPXXVPTKL:SNIT:LGEMS:NMEHSSXGXH, RVPSTXXVPTKT:SNIT:LGEMS:MIEHSXGXL, ASAAPXXVPTAL:SNIT:LGEMS:MIEHSXKXS, ASASPXXVPTDL:SNIT:LGEMS:MIEHSXKXS, ASASPXXVPTDL:SNIT:LGEMS:MVEHSXKXS, NDEGLEXVPTEE:SNIT:LGEMS:FLEHXKXESR, SSVKXQPVPTHH:SNIT:LGEMS:RLEHSLEXAXA, RNVQXRPVPTQL:SNIT:LGEMS:TLEHSLAXKXE, KIPKAXXVPTEL:SNIT:LGEMS:DMVVEGXGXR, GIPEPXXVPEKM:SNIT:LGEMS:NMTVESXAXR, SIPKAXXVPTEL:SNIT:LGEMS:EMVVEGXGXR, HVTKPTXAPTKL:SNIT:LGEMS:NMVVRSXGXH, YVPKPXXAPTKL:SNIT:LGEMS:NMVVRSXGXH, TVPKPXXAPTQL:SNIT:LGEMS:NMVVRAXGXH, AVPKAXXAPTKL:SNIT:LGEMS:NMVVKAXGXH, KVGKAXXVPTKL:SNIT:LGEMS:EGMSVAEXGXR, KASKAXXVPTKL:SNIT:LGEMS:GMAVSEXGXR, GSAGPXXTPTKM:SNIT:LGEMS:GMVVDRXGXS, AAPASXXVPARL:SNIT:LGEMS:DMVVEAXGXR, STPPTXXVPTRL:SNIT:LGEMS:DMVVESXGXR, HVPKPXXAPTKL:SNIT:LGEMS:NMVVRSXGXH, RVPSTXXAPVKT:SNIT:LGEMS:MIVEEXGXL, ASAAPXXVPQAL:SNIT:LGEMS:MIVRSXKXS, ASASPXXVSQDL:SNIT:LGEMS:MIVKSXKXS, ASASPXXVPQDL:SNIT:LGEMS:MVVKSXKXS, NDEGLEXVPTEE:SNIT:LGEMS:FLQHNKXEXR, SSVKXQPSRVHH:SNIT:LGEMS:RLEEHLEXAXA, RNVQXRPTQVQL:SNIT:LGEMS:TLEDHLAXKXE, KIPKASSSRVEL:RSVK:KEVQV:DMEEHGXGXR, GIPEPSSSRVKM:RSVK:KEVQV:NMEEHSXAXR, SIPKASSSRVEL:RSVK:KEVQV:EMEEHGXGXR, HVTKPTXSRVKL:RSVK:KEVQV:NMEEHSXGXH, YVPKPSSSRVKL:RSVK:KEVQV:NMEEHSXGXH, TVPKPSSSRVQL:RSVK:KEVQV:NMEEHAXGXH, AVPKASSSRVKL:RSVK:KEVQV:NMEEHAXGXH, KVGKASSSRVKL:RSVK:KEVQV:EGMSEEHXGXR, KASKASSSRVKL:RSVK:KEVQV:GMEEHEXGXR, GSAGPSSSRVKM:RSVK:KEVQV:GMEEHRXGXS, AAPASSSSRVRL:RSVK:KEVQV:DMEEHAXGXR, STPPTSSSRVRL:RSVK:KEVQV:DMEEHSXGXR, HVPKPSSSRVKL:RSVK:KEVQV:NMEEHSXGXH, RVPSTSSSRVKT:RSVK:KEVQV:MIEEHXGXL, ASAAPSSSRVAL:RSVK:KEVQV:MIEEHXKXS, ASASPSSSRVDL:RSVK:KEVQV:MIEEHXKXS, ASASPSSSRVDL:RSVK:KEVQV:MVEEHXKXS, NDEGLESSRVEE:RSVK:KEVQV:FLEEHKXEXR, NDEGLESSRVGQ:RSVK:KEVQV:LEEEHQXEXR, RNVQXRPSRVQL:RSVK:KEVQV:TLEEHLAXKXE, KIPKAXXVPTEL:RSVK:KEVQV:DMVVEGXGXR, GIPEPXXVPEKM:RSVK:KEVQV:NMTVESXAXR, SIPKAXXVPTEL:RSVK:KEVQV:EMVVEGXGXR, HVTKPTXAPTKL:RSVK:KEVQV:NMVVRSXGXH, YVPKPXXAPTKL:RSVK:KEVQV:NMVVRSXGXH, TVPKPXXAPTQL:RSVK:KEVQV:NMVVRAXGXH, AVPKAXXAPTKL:RSVK:KEVQV:NMVVKAXGXH, KVGKAXXVPTKL:RSVK:KEVQV:EGMSVAEXGXR, KASKAXXVPTKL:RSVK:KEVQV:GMAVSEXGXR, GSAGPXXTPTKM:RSVK:KEVQV:GMVVDRXGXS, AAPASXXVPARL:RSVK:KEVQV:DMVVEAXGXR, STPPTXXVPTRL:RSVK:KEVQV:DMVVESXGXR, HVPKPXXAPTKL:RSVK:KEVQV:NMVVRSXGXH, RVPSTXXAPVKT: RSVK: KEVQV: MIVEEXGXL, ASAAPXXVPQAL:RSVK:KEVQV:MIVRSXKXS, ASASPXXVSQDL:RSVK:KEVQV:MIVKSXKXS, ASASPXXVPQDL:RSVK:KEVQV:MVVKSXKXS, NDEGLEXVPTEE:RSVK:KEVQV:FLQHNKXEXR, NDEGLEXVPTGQ:RSVK:KEVQV:LEEHSQXEXR, RNVQXRPTQVQL:RSVK:KEVQV:TLEDHLAXKXE, KIPKASSTQVEL:RPVQ:KKATV:DMEDHGXGXR, GIPEPSSTQVKM:RPVQ:KKATV:NMEDHSXAXR, SIPKASSTQVEL:RPVQ:KKATV:EMEDHGXGXR, HVTKPTXTQVKL:RPVQ:KKATV:NMEDHSXGXH, YVPKPSSTQVKL:RPVQ:KKATV:NMEDHSXGXH, TVPKPSSTQVQL:RPVQ:KKATV:NMEDHAXGXH, AVPKASSTQVKL:RPVQ:KKATV:NMEDHAXGXH, KVGKASSTQVKL:RPVQ:KKATV:EGMSEDHXGXR, KASKASSTQVKL:RPVQ:KKATV:GMEDHEXGXR, GSAGPSSTQVKM:RPVQ:KKATV:GMEDHRXGXS, AAPASSSTQVRL:RPVQ:KKATV:DMEDHAXGXR, STPPTSSTQVRL:RPVQ:KKATV:DMEDHSXGXR, HVPKPSSTQVKL:RPVQ:KKATV:NMEDHSXGXH, RVPSTSSTQVKT:RPVQ:KKATV:MIEDHXGXL, ASAAPSSTQVAL:RPVQ:KKATV:MIEDHXKXS, ASASPSSTQVDL:RPVQ:KKATV:MIEDHXKXS, ASASPSSTQVDL:RPVQ:KKATV:MVEDHXKXS, NDEGLESTQVEE:RPVQ:KKATV:FLEDHKXEXR, NDEGLESTQVGQ:RPVQ:KKATV:LEEDHQXEXR, SSVKXQPTQVHH:RPVQ:KKATV:RLEDHLEXAXA, KIPKAXXVPTEL:RPVQ:KKATV:DMVVEGXGXR, GIPEPXXVPEKM:RPVQ:KKATV:NMTVESXAXR, SIPKAXXVPTEL:RPVQ:KKATV:EMVVEGXGXR, HVTKPTXAPTKL:RPVQ:KKATV:NMVVRSXGXH, YVPKPXXAPTKL:RPVQ:KKATV:NMVVRSXGXH, TVPKPXXAPTQL:RPVQ:KKATV:NMVVRAXGXH, AVPKAXXAPTKL:RPVQ:KKATV:NMVVKAXGXH, KVGKAXXVPTKL:RPVQ:KKATV:EGMSVAEXGXR, KASKAXXVPTKL:RPVQ:KKATV:GMAVSEXGXR, GSAGPXXTPTKM:RPVQ:KKATV:GMVVDRXGXS, AAPASXXVPARL:RPVQ:KKATV:DMVVEAXGXR, STPPTXXVPTRL:RPVQ:KKATV:DMVVESXGXR, HVPKPXXAPTKL:RPVQ:KKATV:NMVVRSXGXH, RVPSTXXAPVKT:RPVQ:KKATV:M IVEEXGXL, ASAAPXXVPQAL:RPVQ:KKATV:MIVRSXKXS, ASASPXXVSQDL:RPVQ:KKATV:MIVKSXKXS, ASASPXXVPQDL:RPVQ:KKATV:MVVKSXKXS, NDEGLEXVPTEE:RPVQ:KKATV:FLQHNKXEXR, NDEGLEXVPTGQ:RPVQ:KKATV:LEEHSQXEXR and SSVKXQPSRVHH:RPVQ:KKATV:RLEEHLEXAXA. More particularly, the quadruplet PEP9:PEP1:PEP2:PEP10 is selected from the group consisting of GIPEPXXVPTKM:SAIS:LKNYQ:NMVVESXAXR, HVTKPTXVPTKL:SAIS:LKNYQ:NMVVESXGXH, YVPKPXXVPTKL:SAIS:LKNYQ:NMVVESXGXH, TVPKPXXVPTQL:SAIS:LKNYQ:NMVVEAXGXH, AVPKAXXVPTKL:SAIS:LKNYQ:NMVVEAXGXH, KVGKAXXVPTKL:SAIS:LKNYQ:EGMSVVEXGXR, KASKAXXVPTKL:SAIS:LKNYQ:GMVVEEXGXR, GSAGPXXVPTKM:SAIS:LKNYQ:GMVVERXGXS, AAPASXXVPTRL:SAIS:LKNYQ:DMVVEAXGXR, STPPTXXVPTRL:SAIS:LKNYQ:DMVVESXGXR, HVPKPXXVPTKL:SAIS:LKNYQ:NMVVESXGXH, RVPSTXXVPTKT:SAIS:LKNYQ:MIVVEXGXL, ASAAPXXVPTAL:SAIS:LKNYQ:MIVVEXKXS, ASASPXXVPTDL:SAIS:LKNYQ:MIVVEXKXS, ASASPXXVPTDL:SAIS:LKNYQ:MVVVEXKXS, GIPEPXXVPEKM:SAIS:LKNYQ:NMTVESXAXR, HVTKPTXAPTKL:SAIS:LKNYQ:NMVVRSXGXH, YVPKPXXAPTKL:SAIS:LKNYQ:NMVVRSXGXH, TVPKPXXAPTQL:SAIS:LKNYQ:NMVVRAXGXH, AVPKAXXAPTKL:SAIS:LKNYQ:NMVVKAXGXH, KVGKAXXVPTKL:SAIS:LKNYQ:EGMSVAEXGXR, KASKAXXVPTKL:SAIS:LKNYQ:GMAVSEXGXR, GSAGPXXTPTKM:SAIS:LKNYQ:GMVVDRXGXS, AAPASXXVPARL:SAIS:LKNYQ:DMVVEAXGXR, HVPKPXXAPTKL:SAIS:LKNYQ:NMVVRSXGXH, RVPSTXXAPVKT:SAIS:LKNYQ:MIVEEXGXL, ASAAPXXVPQAL:SAIS:LKNYQ:MIVRSXKXS, ASASPSSVSQDL:SAIS:LKNYQ:MIVKSXKXS, ASASPXXVPQDL:SAIS:LKNYQ:MVVKSXKXS, NDEGLEXVPTEE:SAIS:LKNYQ:FLQHNKXEXR, NDEGLEXVPTGQ:SAIS:LKNYQ:LEEHSQXEXR, SSVKXQPSRVHH:SAIS:LKNYQ:RLEEHLEXAXA, RNVQXRPTQVQL:SAIS:LKNYQ:TLEDHLAXKXE, KIPKAXXVPEEL:SSLS:LKVYP:DMTVEGXGXR, SIPKAXXVPEEL:SSLS:LKVYP:EMTVEGXGXR, HVTKPTXVPEKL:SSLS:LKVYP:NMTVESXGXH, YVPKPXXVPEKL:SSLS:LKVYP:NMTVESXGXH, TVPKPXXVPEQL:SSLS:LKVYP:NMTVEAXGXH, AVPKAXXVPEKL:SSLS:LKVYP:NMTVEAXGXH, KVGKAXXVPEKL:SSLS:LKVYP:EGMSTVEXGXR, KASKAXXVPEKL:SSLS:LKVYP:GMTVEEXGXR, GSAGPXXVPEKM:SSLS:LKVYP:GMTVERXGXS, AAPASXXVPERL:SSLS:LKVYP:DMTVEAXGXR, STPPTXXVPERL:SSLS:LKVYP:DMTVESXGXR, HVPKPXXVPEKL:SSLS:LKVYP:NMTVESXGXH, RVPSTXXVPEKT:SSLS:LKVYP:MITVEXGXL,

ASAAPXXVPEAL:SSLS:LKVYP:MITVEXKXS, ASASPXXVPEDL:SSLS:LKVYP:MITVEXKXS, ASASPXXVPEDL:SSLS:LKVYP:MVTVEXKXS, KIPKAXXVPTEL:SSLS:LKVYP:DMVVEGXGXR, SIPKAXXVPTEL:SSLS:LKVYP:EMVVEGXGXR, HVTKPTXAPTKL:SSLS:LKVYP:NMVVRSXGXH, YVPKPXXAPTKL:SSLS:LKVYP:NMVVRSXGXH, TVPKPXXAPTQL:SSLS:LKVYP:NMVVRAXGXH, AVPKAXXAPTKL:SSLS:LKVYP:NMVVKAXGXH, KVGKAXXVPTKL:SSLS:LKVYP:EGMSVAEXGXR, KASKAXXVPTKL:SSLS:LKVYP:GMAVSEXGXR, GSAGPXXTPTKM:SSLS:LKVYP:GMVVDRXGXS, AAPASXXVPARL:SSLS:LKVYP:DMVVEAXGXR, STPPTXXVPTRL:SSLS:LKVYP:DMVVESXGXR, HVPKPXXAPTKL:SSLS:LKVYP:NMVVRSXGXH, RVPSTXXAPVKT:SSLS:LKVYP:MIVEEXGXL, ASAAPXXVPQAL:SSLS:LKVYP: MIVRSXKXS, ASASPSSVSQDL:SSLS: LKVYP:MIVKSXKXS, ASASPXXVPQDL:SSLS:LKVYP:MVVKSXKXS, NDEGLEXVPTEE:SSLS:LKVYP:FLQHNKXEXR, NDEGLEXVPTGQ:SSLS:LKVYP:LEEHSQXEXR, SSVKXQPSRVHH:SSLS:LKVYP:RLEEHLEXAXA, RNVQXRPTQVQL:SSLS:LKVYP:TLEDHLAXKXE, KIPKAXXAPTEL:NAIS:LKKYR:DMVVRGXGXR, GIPEPXXAPTKM:NAIS:LKKYR:NMVVRSXAXR, SIPKAXXAPTEL:NAIS:LKKYR:EMVVRGXGXR, AVPKAXXAPTKL:NAIS:LKKYR:NMVVRAXGXH, KVGKAXXAPTKL:NAIS:LKKYR:EGMSVVRXGXR, KASKAXXAPTKL:NAIS:LKKYR:GMVVREXGXR, GSAGPXXAPTKM:NAIS:LKKYR:GMVVRRXGXS, AAPASXXAPTRL:NAIS:LKKYR:DMVVRAXGXR, STPPTXXAPTRL:NAIS:LKKYR:DMVVRSXGXR, RVPSTXXAPTKT:NAIS:LKKYR:MIVVRXGXL, ASAAPXXAPTAL:NAIS:LKKYR:MIVVRXKXS, ASASPXXAPTDL:NAIS:LKKYR:MIVVRXKXS, ASASPXXAPTDL:NAIS:LKKYR:MVVVRXKXS, KIPKAXXVPTEL:NAIS:LKKYR:DMVVEGXGXR, GIPEPXXVPEKM:NAIS:LKKYR:NMTVESXAXR, SIPKAXXVPTEL:NAIS:LKKYR:EMVVEGXGXR, AVPKAXXAPTKL:NAIS:LKKYR:NMVVKAXGXH, KVGKAXXVPTKL:NAIS:LKKYR:EGMSVAEXGXR, KASKAXXVPTKL:NAIS:LKKYR:GMAVSEXGXR, GSAGPXXTPTKM:NAIS:LKKYR:GMVVDRXGXS, AAPASXXVPARL:NAIS:LKKYR:DMVVEAXGXR, STPPTXXVPTRL:NAIS:LKKYR:DMVVESXGXR, RVPSTXXAPVKT:NAIS:LKKYR:MIVEEXGXL, ASAAPXXVPQAL:NAIS:LKKYR:MIVRSXKXS, ASASPSSVSQDL:NAIS:LKKYR:MIVKSXKXS, ASASPXXVPQDL:NAIS:LKKYR:MVVKSXKXS, NDEGLEXVPTEE:NAIS:LKKYR:FLQHNKXEXR, NDEGLEXVPTGQ:NAIS:LKKYR:LEEHSQXEXR, SSVKXQPSRVHH:NAIS:LKKYR:RLEEHLEXAXA, RNVQXRPTQVQL:NAIS:LKKYR:TLEDHLAXKXE, KIPKAXXAPTEL:SATS:LRKHR:DMVVKGXGXR, GIPEPXXAPTKM:SATS:LRKHR:NMVVKSXAXR, SIPKAXXAPTEL:SATS:LRKHR:EMVVKGXGXR, HVTKPTXAPTKL:SATS:LRKHR:NMVVKSXGXH, YVPKPXXAPTKL:SATS:LRKHR:NMVVKSXGXH, TVPKPXXAPTQL:SATS:LRKHR:NMVVKAXGXH, KVGKAXXAPTKL:SATS:LRKHR:EGMSVVKXGXR, KASKAXXAPTKL:SATS:LRKHR:GMVVKEXGXR, GSAGPXXAPTKM:SATS:LRKHR:GMVVKRXGXS, AAPASXXAPTRL:SATS:LRKHR:DMVVKAXGXR, STPPTXXAPTRL:SATS:LRKHR:DMVVKSXGXR, HVPKPXXAPTKL:SATS:LRKHR:NMVVKSXGXH, RVPSTXXAPTKT:SATS:LRKHR:MIVVKXGXL, ASAAPXXAPTAL:SATS:LRKHR:MIVVKXKXS, ASASPXXAPTDL:SATS:LRKHR:MIVVKXKXS, ASASPXXAPTDL:SATS:LRKHR:MVVVKXKXS, KIPKAXXVPTEL:SATS:LRKHR:DMVVEGXGXR, GIPEPXXVPEKM:SATS:LRKHR:NMTVESXAXR, SIPKAXXVPTEL:SATS:LRKHR:EMVVEGXGXR, HVTKPTXAPTKL:SATS:LRKHR:NMVVRSXGXH, YVPKPXXAPTKL:SATS:LRKHR:NMVVRSXGXH, TVPKPXXAPTQL:SATS:LRKHR:NMVVRAXGXH, KVGKAXXVPTKL:SATS:LRKHR:EGMSVAEXGXR, KASKAXXVPTKL:SATS:LRKHR:GMAVSEXGXR, GSAGPXXTPTKM:SATS:LRKHR:GMVVDRXGXS, AAPASXXVPARL:SATS:LRKHR:DMVVEAXGXR, STPPTXXVPTRL:SATS:LRKHR:DMVVESXGXR, HVPKPXXAPTKL:SATS:LRKHR:NMVVRSXGXH, RVPSTXXAPVKT:SATS:LRKHR:MIVEEXGXL, ASAAPXXVPQAL:SATS:LRKHR:MIVRSXKXS, ASASPSSVSQDL:SATS:LRKHR:MIVKSXKXS, ASASPXXVPQDL:SATS:LRKHR:MVVKSXKXS, NDEGLEXVPTEE:SATS:LRKHR:FLQHNKXEXR, NDEGLEXVPTGQ:SATS:LRKHR:LEEHSQXEXR, SSVKXQPSRVHH:SATS:LRKHR:RLEEHLEXAXA, RNVQXRPTQVQL:SATS:LRKHR:TLEDHLAXKXE, KIPKAXXVPTEL:SPIS:LKYHY:DMVAEGXGXR, GIPEPXXVPTKM:SPIS:LKYHY:NMVAESXAXR, SIPKAXXVPTEL:SPIS:LKYHY:EMVAEGXGXR, HVTKPTXVPTKL:SPIS:LKYHY:NMVAESXGXH, YVPKPXXVPTKL:SPIS:LKYHY:NMVAESXGXH, TVPKPXXVPTQL:SPIS:LKYHY:NMVAEAXGXH, AVPKAXXVPTKL:SPIS:LKYHY:NMVAEAXGXH, KASKAXXVPTKL:SPIS:LKYHY:GMVAEEXGXR, GSAGPXXVPTKM:SPIS:LKYHY:GMVAERXGXS, AAPASXXVPTRL:SPIS:LKYHY:DMVAEAXGXR, STPPTXXVPTRL:SPIS:LKYHY:DMVAESXGXR, HVPKPXXVPTKL:SPIS:LKYHY:NMVAESXGXH, RVPSTXXVPTKT:SPIS:LKYHY:MIVAEXGXL, ASAAPXXVPTAL:SPIS:LKYHY:MIVAEXKXS, ASASPXXVPTDL:SPIS:LKYHY:MIVAEXKXS, ASASPXXVPTDL:SPIS:LKYHY:MVVAEXKXS, KIPKAXXVPTEL:SPIS:LKYHY:DMVVEGXGXR, GIPEPXXVPEKM:SPIS:LKYHY:NMTVESXAXR, SIPKAXXVPTEL:SPIS:LKYHY:EMVVEGXGXR, HVTKPTXAPTKL:SPIS:LKYHY:NMVVRSXGXH, YVPKPXXAPTKL:SPIS:LKYHY:NMVVRSXGXH, TVPKPXXAPTQL:SPIS:LKYHY:NMVVRAXGXH, AVPKAXXAPTKL:SPIS:LKYHY:NMVVKAXGXH, KASKAXXVPTKL:SPIS:LKYHY:GMAVSEXGXR, GSAGPXXTPTKM:SPIS:LKYHY:GMVVDRXGXS, AAPASXXVPARL:SPIS:LKYHY:DMVVEAXGXR, STPPTXXVPTRL:SPIS:LKYHY:DMVVESXGXR, HVPKPXXAPTKL:SPIS:LKYHY:NMVVRSXGXH, RVPSTXXAPVKT:SPIS:LKYHY:MIVEEXGXL, ASAAPXXVPQAL:SPIS:LKYHY:MIVRSXKXS, ASASPSSVSQDL:SPIS:LKYHY:MIVKSXKXS, ASASPXXVPQDL:SPIS:LKYHY:MVVKSXKXS, NDEGLEXVPTEE:SPIS:LKYHY:FLQHNKXEXR, NDEGLEXVPTGQ:SPIS:LKYHY:LEEHSQXEXR, SSVKXQPSRVHH:SPIS:LKYHY:RLEEHLEXAXA, RNVQXRPTQVQL:SPIS:LKYHY:TLEDHLAXKXE, KIPKAXXVPTEL:EPIS:KFKYE:DMAVXGXGSR, GIPEPXXVPTKM:EPIS:KFKYE:NMAVSSXAXR, SIPKAXXVPTEL:EPIS:KFKYE:EMAVXGXGSR, HVTKPTXVPTKL:EPIS:KFKYE:NMAVSSXGXH, YVPKPXXVPTKL:EPIS:KFKYE:NMAVSSXGXH, TVPKPXXVPTQL:EPIS:KFKYE:NMAVSAXGXH, AVPKAXXVPTKL:EPIS:KFKYE:NMAVSAXGXH, KVGKAXXVPTKL:EPIS:KFKYE:EGMSAVSXGXR, GSAGPXXVPTKM:EPIS:KFKYE:GMAVSRXGXS, AAPASXXVPTRL:EPIS:KFKYE:DMAVSAXGXR, STPPTXXVPTRL:EPIS:KFKYE:DMAVSSXGXR, HVPKPXXVPTKL:EPIS:KFKYE:NMAVSSXGXH, RVPSTXXVPTKT:EPIS:KFKYE:MIAVSXGXL, ASAAPXXVPTAL:EPIS:KFKYE:MIAVSXKXS, ASASPXXVPTDL:EPIS:KFKYE:MIAVSXKXS, ASASPXXVPTDL:EPIS:KFKYE:MVAVSXKXS, KIPKAXXVPTEL:EPIS:KFKYE:DMVVEGXGXR, GIPEPXXVPEKM:EPIS:KFKYE:NMTVESXAXR, SIPKAXXVPTEL:EPIS:KFKYE:EMVVEGXGXR, HVTKPTXAPTKL:EPIS:KFKYE:NMVVRSXGXH, YVPKPXXAPTKL:EPIS:KFKYE:NMVVRSXGXH, TVPKPXXAPTQL:EPIS:KFKYE:NMVVRAXGXH, AVPKAXXAPTKL:EPIS:KFKYE:NMVVKAXGXH, KVGKAXXVPTKL:EPIS:KFKYE:EGMSVAEXGXR, GSAGPXXTPTKM:EPIS:KFKYE:GMVVDRXGXS, AAPASXXVPARL:EPIS:KFKYE:DMVVEAXGXR, STPPTXXVPTRL:EPIS:KFKYE:DMVVESXGXR, HVPKPXXAPTKL:EPIS:KFKYE:NMVVRSXGXH, RVPSTXXAPVKT:EPIS:KFKYE:MIVEEXGXL, ASAAPXXVPQAL:EPIS:KFKYE:MIVRSXKXS, ASASPSSVSQDL:EPIS:KFKYE:MIVKSXKXS, ASASPXXVPQDL:EPIS:KFKYE:MVVKSXKXS, NDEGLEXVPTEE:EPIS:KFKYE:FLQHNKXEXR, NDEGLEXVPTGQ:EPIS:KFKYE:LEEHSQXEXR, SSVKXQPSRVHH:EPIS:KFKYE:RLEEHLEXAXA, RNVQXRPTQVQL:EPIS:KFKYE:TLEDHLAXKXE, KIPKAXXTPTEL:SPIN:YGKIP:DMVVDGXGXR, GIPEPXXTPTKM:SPIN:YGKIP:NMVVDSXAXR, SIPKAXXTPTEL:SPIN:YGKIP:EMVVDGXGXR, HVTKPTXTPTKL:SPIN:YGKIP:NMVVDSXGXH, YVPKPXXTPTKL:SPIN:YGKIP:NMVVDSXGXH, TVPKPXXTPTQL:SPIN:YGKIP:NMVVDAXGXH, AVPKAXXTPTKL:SPIN:YGKIP:NMVVDAXGXH, KVGKAXXTPTKL:SPIN:YGKIP:EGMSVVDXGXR, KASKAXXTPTKL:SPIN:YGKIP:GMVVDEXGXR, AAPASXXTPTRL:SPIN:YGKIP:DMVVDAXGXR, STPPTXXTPTRL:SPIN:YGKIP:DMVVDSXGXR, HVPKPXXTPTKL:SPIN:YGKIP:NMVVDSXGXH, RVPSTXXTPTKT:SPIN:YGKIP:MIVVDXGXL, ASAAPXXTPTAL:SPIN:YGKIP:MIVVDXKXS, ASASPXXTPTDL:SPIN:YGKIP:MIVVDXKXS, ASASPXXTPTDL:SPIN:YGKIP:MVVVDXKXS, KIPKAXXVPTEL:SPIN:YGKIP:DMVVEGXGXR, GIPEPXXVPEKM:SPIN:YGKIP:NMTVESXAXR, SIPKAXXVPTEL:SPIN:YGKIP:EMVVEGXGXR, HVTKPTXAPTKL:SPIN:YGKIP:NMVVRSXGXH, YVPKPXXAPTKL:SPIN:YGKIP:NMVVRSXGXH, TVPKPXXAPTQL:SPIN:YGKIP:NMVVRAXGXH, AVPKAXXAPTKL:SPIN:YGKIP:NMVVKAXGXH, KVGKAXXVPTKL:SPIN:YGKIP:EGMSVAEXGXR, KASKAXXVPTKL:SPIN:YGKIP:GMAVSEXGXR, AAPASXXVPARL:SPIN:YGKIP:DMVVEAXGXR, STPPTXXVPTRL:SPIN:YGKIP:DMVVESXGXR, HVPKPXXAPTKL:SPIN:YGKIP:NMVVRSXGXH, RVPSTXXAPVKT:SPIN:YGKIP:MIVEEXGXL, ASAAPXXVPQAL:SPIN:YGKIP:MIVRSXKXS, ASASPSSVSQDL:SPIN:YGKIP:MIVKSXKXS, ASASPXXVPQDL:SPIN:YGKIP:MVVKSXKXS, NDEGLEXVPTEE:SPIN:YGKIP:FLQHNKXEXR, NDEGLEXVPTGQ:SPIN:YGKIP:LEEHSQXEXR, SSVKXQPSRVHH:SPIN:YGKIP:RLEEHLEXAXA, RNVQXRPTQVQL:SPIN:YGKIP:TLEDHLAXKXE, KIPKAXXVPAEL:SPIS:YKQYE:DMVVEGXGXR, GIPEPXXVPAKM:SPIS:YKQYE:NMVVESXAXR, SIPKAXXVPAEL:SPIS:YKQYE:EMVVEGXGXR, HVTKPTXVPAKL:SPIS:YKQYE:NMVVESXGXH, YVPKPXXVPAKL:SPIS:YKQYE:NMVVESXGXH, TVPKPXXVPAQL:SPIS:YKQYE:NMVVEAXGXH, AVPKAXXVPAKL:SPIS:YKQYE:NMVVEAXGXH, KVGKAXXVPAKL:SPIS:YKQYE:EGMSVVEXGXR, KASKAXXVPAKL:SPIS:YKQYE:GMVVEEXGXR, GSAGPXXVPAKM:SPIS:YKQYE:GMVVERXGXS, STPPTXXVPARL:SPIS:YKQYE:DMVVESXGXR, HVPKPXXVPAKL:SPIS:YKQYE:NMVVESXGXH, RVPSTXXVPAKT:SPIS:YKQYE:MIVVEXGXL, ASAAPXXVPAAL:SPIS:YKQYE:MIVVEXKXS, ASASPXXVPADL:SPIS:YKQYE:MIVVEXKXS, ASASPXXVPADL:SPIS:YKQYE:MVVVEXKXS, KIPKAXXVPTEL:SPIS:YKQYE:DMVVEGXGXR, GIPEPXXVPEKM:SPIS:YKQYE:NMTVESXAXR, SIPKAXXVPTEL:SPIS:YKQYE:EMVVEGXGXR, HVTKPTXAPTKL:SPIS:YKQYE:NMVVRSXGXH, YVPKPXXAPTKL:SPIS:YKQYE:NMVVRSXGXH, TVPKPXXAPTQL:SPIS:YKQYE:NMVVRAXGXH, AVPKAXXAPTKL:SPIS:YKQYE:NMVVKAXGXH, KVGKAXXVPTKL:SPIS:YKQYE:EGMSVAEXGXR, KASKAXXVPTKL:SPIS:YKQYE:GMAVSEXGXR, GSAGPXXTPTKM:SPIS:YKQYE:GMVVDRXGXS, STPPTXXVPTRL:SPIS:YKQYE:DMVVESXGXR, HVPKPXXAPTKL:SPIS:YKQYE:NMVVRSXGXH, RVPSTXXAPVKT:SPIS:YKQYE:MIVEEXGXL, ASAAPXXVPQAL:SPIS:YKQYE:MIVRSXKXS, ASASPSSVSQDL:SPIS:YKQYE:MIVKSXKXS, ASASPXXVPQDL:SPIS:YKQYE:MVVKSXKXS, NDEGLEXVPTEE:SPIS:YKQYE:FLQHNKXEXR, NDEGLEXVPTGQ:SPIS:YKQYE:LEEHSQXEXR, SSVKXQPSRVHH:SPIS:YKQYE:RLEEHLEXAXA, RNVQXRPTQVQL:SPIS:YKQYE:TLEDHLAXKXE, GIPEPXXVPTKM:SPIS:YKQYE:NMVVESXAXR, HVTKPTXVPTKL:SPIS:YKQYE:NMVVESXGXH, YVPKPXXVPTKL:SPIS:YKQYE:NMVVESXGXH, TVPKPXXVPTQL:SPIS:YKQYE:NMVVEAXGXH, AVPKAXXVPTKL:SPIS:YKQYE:NMVVEAXGXH, KVGKAXXVPTKL:SPIS:YKQYE:EGMSVVEXGXR, KASKAXXVPTKL:SPIS:YKQYE:GMVVEEXGXR, GSAGPXXVPTKM:SPIS:YKQYE:GMVVERXGXS, AAPASXXVPTRL:SPIS:YKQYE:DMVVEAXGXR, HVPKPXXVPTKL:SPIS:YKQYE:NMVVESXGXH, RVPSTXXVPTKT:SPIS:YKQYE:MIVVEXGXL, ASAAPXXVPTAL:SPIS:YKQYE:MIVVEXKXS, ASASPXXVPTDL:SPIS:YKQYE:MIVVEXKXS, ASASPXXVPTDL:SPIS:YKQYE:MVVVEXKXS, AAPASXXVPARL:SPIS:YKQYE:DMVVEAXGXR, KIPKAXXAPVEL:KPLS:DHHKD:DMVEEGXGXR, GIPEPXXAPVKM:KPLS:DHHKD:NMVEESXAXR, SIPKAXXAPVEL:KPLS:DHHKD:EMVEEGXGXR, HVTKPTXAPVKL:KPLS:DHHKD:NMVEESXGXH, YVPKPXXAPVKL:KPLS:DHHKD:NMVEESXGXH, TVPKPXXAPVQL:KPLS:DHHKD:NMVEEAXGXH, AVPKAXXAPVKL:KPLS:DHHKD:NMVEEAXGXH, KVGKAXXAPVKL:KPLS:DHHKD:EGMSVEEXGXR, KASKAXXAPVKL:KPLS:DHHKD:GMVEEEXGXR, GSAGPXXAPVKM:KPLS:DHHKD:GMVEERXGXS, AAPASXXAPVRL:KPLS:DHHKD:DMVEEAXGXR, STPPTXXAPVRL:KPLS:DHHKD:DMVEESXGXR, HVPKPXXAPVKL:KPLS:DHHKD:NMVEESXGXH, ASAAPXXAPVAL:KPLS:DHHKD:MIVEEXKXS, ASASPXXAPVDL:KPLS:DHHKD:MIVEEXKXS, ASASPXXAPVDL:KPLS:DHHKD:MVVEEXKXS, KIPKAXXVPTEL:KPLS:DHHKD:DMVVEGXGXR, GIPEPXXVPEKM:KPLS:DHHKD:NMTVESXAXR, SIPKAXXVPTEL:KPLS:DHHKD:EMVVEGXGXR, HVTKPTXAPTKL:KPLS:DHHKD:NMVVRSXGXH, YVPKPXXAPTKL:KPLS:DHHKD:NMVVRSXGXH, TVPKPXXAPTQL:KPLS:DHHKD:NMVVRAXGXH, AVPKAXXAPTKL:KPLS:DHHKD:NMVVKAXGXH, KVGKAXXVPTKL:KPLS:DHHKD:EGMSVAEXGXR, KASKAXXVPTKL:KPLS:DHHKD:GMAVSEXGXR, GSAGPXXTPTKM:KPLS:DHHKD:GMVVDRXGXS, AAPASXXVPARL:KPLS:DHHKD:DMVVEAXGXR, STPPTXXVPTRL:KPLS:DHHKD:DMVVESXGXR, HVPKPXXAPTKL:KPLS:DHHKD:NMVVRSXGXH, ASAAPXXVPQAL:KPLS:DHHKD:MIVRSXKXS, ASASPSSVSQDL:KPLS:DHHKD:MIVKSXKXS, ASASPXXVPQDL:KPLS:DHHKD:MVVKSXKXS, NDEGLEXVPTEE:KPLS:DHHKD:FLQHNKXEXR, NDEGLEXVPTGQ:KPLS:DHHKD:LEEHSQXEXR, SSVKXQPSRVHH:KPLS:DHHKD:RLEEHLEXAXA, RNVQXRPTQVQL:KPLS:DHHKD:TLEDHLAXKXE, KIPKAXXVPQEL:EPLP:EQLSN:DMVRXGXGSR, GIPEPXXVPQKM:EPLP:EQLSN:NMVRSSXAXR, SIPKAXXVPQEL:EPLP:EQLSN:EMVRXGXGSR, HVTKPTXVPQKL:EPLP:EQLSN:NMVRSSXGXH, YVPKPXXVPQKL:EPLP:EQLSN:NMVRSSXGXH, TVPKPXXVPQQL:EPLP:EQLSN:NMVRSAXGXH, AVPKAXXVPQKL:EPLP:EQLSN:NMVRSAXGXH, KVGKAXXVPQKL:EPLP:EQLSN:EGMSVRSXGXR, KASKAXXVPQKL:EPLP:EQLSN:GMVRSEXGXR, GSAGPXXVPQKM:EPLP:EQLSN:GMVRSRXGXS, AAPASXXVPQRL:EPLP:EQLSN:DMVRSAXGXR, STPPTXXVPQRL:EPLP:EQLSN:DMVRSSXGXR, HVPKPXXVPQKL:EPLP:EQLSN:NMVRSSXGXH, RVPSTXXVPQKT:EPLP:EQLSN:MIVRSXGXL, ASASPXXVPQDL:EPLP:EQLSN:MIVRSXKXS, ASASPXXVPQDL:EPLP:EQLSN:MVVRSXKXS, KIPKAXXVPTEL:EPLP:EQLSN:DMVVEGXGXR, GIPEPXXVPEKM:EPLP:EQLSN:NMTVESXAXR, SIPKAXXVPTEL:EPLP:EQLSN:EMVVEGXGXR, HVTKPTXAPTKL:EPLP:EQLSN:NMVVRSXGXH, YVPKPXXAPTKL:EPLP:EQLSN:NMVVRSXGXH, TVPKPXXAPTQL:EPLP:EQLSN:NMVVRAXGXH, AVPKAXXAPTKL:EPLP:EQLSN:NMVVKAXGXH, KVGKAXXVPTKL:EPLP:EQLSN:EGMSVAEXGXR, KASKAXXVPTKL:EPLP:EQLSN:GMAVSEXGXR, GSAGPXXTPTKM:EPLP:EQLSN:GMVVDRXGXS, AAPASXXVPARL:EPLP:EQLSN:DMVVEAXGXR, STPPTXXVPTRL:EPLP:EQLSN:DMVVESXGXR, HVPKPXXAPTKL:EPLP:EQLSN:NMVVRSXGXH, RVPSTXXAPVKT:EPLP:EQLSN:MIVEEXGXL, ASASPSSVSQDL:EPLP:EQLSN:MIVKSXKXS, ASASPXXVPQDL:EPLP:EQLSN:MVVKSXKXS, NDEGLEXVPTEE:EPLP:EQLSN:FLQHNKXEXR, NDEGLEXVPTGQ:EPLP:EQLSN:LEEHSQXEXR, SSVKXQPSRVHH:EPLP:EQLSN:RLEEHLEXAXA, RNVQXRPTQVQL:EPLP:EQLSN:TLEDHLAXKXE, KIPKASSVSQEL:EPLT:EQLSN:DMVKXGXGSR, GIPEPSSVSQKM:EPLT:EQLSN:NMVKSSXAXR, SIPKASSVSQEL:EPLT:EQLSN:EMVKXGXGSR, HVTKPTXVSQKL:EPLT:EQLSN:NMVKSSXGXH, YVPKPSSVSQKL:EPLT:EQLSN:NMVKSSXGXH, TVPKPSSVSQQL:EPLT:EQLSN:NMVKSAXGXH, AVPKASSVSQKL:EPLT:EQLSN:NMVKSAXGXH, KVGKASSVSQKL:EPLT:EQLSN:EGMSVKSXGXR, KASKASSVSQKL:EPLT:EQLSN:GMVKSEXGXR, GSAGPSSVSQKM:EPLT:EQLSN:GMVKSRXGXS, AAPASSSVSQRL:EPLT:EQLSN:DMVKSAXGXR, STPPTSSVSQRL:EPLT:EQLSN:DMVKSSXGXR, HVPKPSSVSQKL:EPLT:EQLSN:NMVKSSXGXH, RVPSTSSVSQKT:EPLT:EQLSN:MIVKSXGXL, ASAAPSSVSQAL:EPLT:EQLSN:MIVKSXKXS, ASASPSSVSQDL:EPLT:EQLSN:MVVKSXKXS, KIPKAXXVPTEL:EPLT:EQLSN:DMVVEGXGXR, GIPEPXXVPEKM:EPLT:EQLSN:NMTVESXAXR, SIPKAXXVPTEL:EPLT:EQLSN:EMVVEGXGXR, HVTKPTXAPTKL:EPLT:EQLSN:NMVVRSXGXH, YVPKPXXAPTKL:EPLT:EQLSN:NMVVRSXGXH, TVPKPXXAPTQL:EPLT:EQLSN:NMVVRAXGXH, AVPKAXXAPTKL:EPLT:EQLSN:NMVVKAXGXH, KVGKAXXVPTKL:EPLT:EQLSN:EGMSVAEXGXR, KASKAXXVPTKL:EPLT:EQLSN:GMAVSEXGXR, GSAGPXXTPTKM:EPLT:EQLSN:GMVVDRXGXS, AAPASXXVPARL:EPLT:EQLSN:DMVVEAXGXR, STPPTXXVPTRL:EPLT:EQLSN:DMVVESXGXR, HVPKPXXAPTKL:EPLT:EQLSN:NMVVRSXGXH, RVPSTXXAPVKT:EPLT:EQLSN:MIVEEXGXL, ASAAPXXVPQAL:EPLT:EQLSN:MIVRSXKXS, NDEGLEXVPTEE:EPLT:EQLSN:FLQHNKXEXR, NDEGLEXVPTGQ:EPLT:EQLSN:LEEHSQXEXR, SSVKXQPSRVHH:EPLT:EQLSN:RLEEHLEXAXA, RNVQXRPTQVQL:EPLT:EQLSN:TLEDHLAXKXE, KIPKAXXVPQEL:EPLT:EQLSN:DMVKXGXGSR, GIPEPXXVPQKM:EPLT:EQLSN:NMVKSSXAXR, SIPKAXXVPQEL:EPLT:EQLSN:EMVKXGXGSR, HVTKPTXVPQKL:EPLT:EQLSN:NMVKSSXGXH, YVPKPXXVPQKL:EPLT:EQLSN:NMVKSSXGXH, TVPKPXXVPQQL:EPLT:EQLSN:NMVKSAXGXH, AVPKAXXVPQKL:EPLT:EQLSN:NMVKSAXGXH, KVGKAXXVPQKL:EPLT:EQLSN:EGMSVKSXGXR, KASKAXXVPQKL:EPLT:EQLSN:GMVKSEXGXR, GSAGPXXVPQKM:EPLT:EQLSN:GMVKSRXGXS, AAPASXXVPQRL:EPLT:EQLSN:DMVKSAXGXR, STPPTXXVPQRL:EPLT:EQLSN:DMVKSSXGXR, HVPKPXXVPQKL:EPLT:EQLSN:NMVKSSXGXH, RVPSTXXVPQKT:EPLT:EQLSN:MIVKSXGXL,

ASAAPXXVPQAL:EPLT:EQLSN:MIVKSXKXS, ASASPXXVPQDL:EPLT:EQLSN:MIVKSXKXS, NDEGLEXVPTGQ:SNIT:IGEMS:LEQHNQXEXR, KIPKAXXVPTEL:SNIT:IGEMS:DMVVEGXGXR, GIPEPXXVPEKM:SNIT:IGEMS:NMTVESXAXR, SIPKAXXVPTEL:SNIT:IGEMS:EMVVEGXGXR, HVTKPTXAPTKL:SNIT:IGEMS:NMVVRSXGXH, YVPKPXXAPTKL:SN IT:IGEMS:N MVVRSXGXH, TVPKPXXAPTQL:SNIT:IGEMS:NMVVRAXGXH, AVPKAXXAPTKL:SNIT:IGEMS:NMVVKAXGXH, KVGKAXXVPTKL:SNIT:IGEMS:EGMSVAEXGXR, KASKAXXVPTKL:SNIT:IGEMS:GMAVSEXGXR, GSAGPXXTPTKM:SNIT:IGEMS:GMVVDRXGXS, AAPASXXVPARL:SNIT:IGEMS:DMVVEAXGXR, STPPTXXVPTRL:SNIT:IGEMS:DMVVESXGXR, HVPKPXXAPTKL:SNIT:IGEMS:NMVVRSXGXH, RVPSTXXAPVKT:SNIT:IGEMS:MIVEEXGXL, ASAAPXXVPQAL:SNIT:IGEMS:MIVRSXKXS, ASASPSSVSQDL:SNIT:IGEMS:MIVKSXKXS, ASASPXXVPQDL:SNIT:IGEMS:MVVKSXKXS, NDEGLEXVPTGQ:SNIT:IGEMS:LEEHSQXEXR, SSVKXQPSRVHH:SNIT:IGEMS:RLEEHLEXAXA, RNVQXRPTQVQL:SNIT:IGEMS:TLEDHLAXKXE, NDEGLEXVPTEE:SNIT:LGEMS:FLEHXKXESR, KIPKAXXVPTEL:SNIT:LGEMS:DMVVEGXGXR, GIPEPXXVPEKM:SNIT:LGEMS:NMTVESXAXR, SIPKAXXVPTEL:SNIT:LGEMS:EMVVEGXGXR, HVTKPTXAPTKL:SNIT:LGEMS:NMVVRSXGXH, YVPKPXXAPTKL:SNIT:LGEMS:NMVVRSXGXH, TVPKPXXAPTQL:SNIT:LGEMS:NMVVRAXGXH, AVPKAXXAPTKL:SNIT:LGEMS:NMVVKAXGXH, KVGKAXXVPTKL:SNIT:LGEMS:EGMSVAEXGXR, KASKAXXVPTKL:SNIT:LGEMS:GMAVSEXGXR, GSAGPXXTPTKM:SNIT:LGEMS:GMVVDRXGXS, AAPASXXVPARL:SNIT:LGEMS:DMVVEAXGXR, STPPTXXVPTRL:SNIT:LGEMS:DMVVESXGXR, HVPKPXXAPTKL:SNIT:LGEMS:NMVVRSXGXH, RVPSTXXAPVKT:SN IT:LGEMS:MIVEEXGXL, ASAAPXXVPQAL:SNIT:LGEMS:MIVRSXKXS, ASASPSSVSQDL:SNIT:LGEMS:MIVKSXKXS, ASASPXXVPQDL:SNIT:LGEMS:MVVKSXKXS, NDEGLEXVPTEE:SNIT:LGEMS:FLQHNKXEXR, SSVKXQPSRVHH:SNIT:LGEMS:RLEEHLEXAXA, RNVQXRPTQVQL:SNIT:LGEMS:TLEDHLAXKXE, RNVQXRPSRVQL:RSVK:KEVQV:TLEEHLAXKXE, KIPKAXXVPTEL:RSVK:KEVQV:DMVVEGXGXR, GIPEPXXVPEKM:RSVK:KEVQV:NMTVESXAXR, SIPKAXXVPTEL:RSVK:KEVQV:EMVVEGXGXR, HVTKPTXAPTKL:RSVK:KEVQV:NMVVRSXGXH, YVPKPXXAPTKL:RSVK:KEVQV:NMVVRSXGXH, TVPKPXXAPTQL:RSVK:KEVQV:NMVVRAXGXH, AVPKAXXAPTKL:RSVK:KEVQV:NMVVKAXGXH, KVGKAXXVPTKL:RSVK:KEVQV:EGMSVAEXGXR, KASKAXXVPTKL:RSVK:KEVQV:GMAVSEXGXR, GSAGPXXTPTKM:RSVK:KEVQV:GMVVDRXGXS, AAPASXXVPARL:RSVK:KEVQV:DMVVEAXGXR, STPPTXXVPTRL:RSVK:KEVQV:DMVVESXGXR, HVPKPXXAPTKL:RSVK:KEVQV:NMVVRSXGXH, RVPSTXXAPVKT:RSVK:KEVQV:MIVEEXGXL, ASAAPXXVPQAL:RSVK:KEVQV:MIVRSXKXS, ASASPSSVSQDL:RSVK:KEVQV:MIVKSXKXS, ASASPXXVPQDL:RSVK:KEVQV:MVVKSXKXS, NDEGLEXVPTEE:RSVK:KEVQV:FLQHNKXEXR, NDEGLEXVPTGQ:RSVK:KEVQV:LEEHSQXEXR, RNVQXRPTQVQL:RSVK:KEVQV:TLEDHLAXKXE, SSVKXQPTQVHH:RPVQ:KKATV:RLEDHLEXAXA, KIPKAXXVPTEL:RPVQ:KKATV:DMVVEGXGXR, GIPEPXXVPEKM:RPVQ:KKATV:NMTVESXAXR, SIPKAXXVPTEL:RPVQ:KKATV:EMVVEGXGXR, HVTKPTXAPTKL:RPVQ:KKATV:NMVVRSXGXH, YVPKPXXAPTKL:RPVQ:KKATV:NMVVRSXGXH, TVPKPXXAPTQL:RPVQ:KKATV:NMVVRAXGXH, AVPKAXXAPTKL:RPVQ:KKATV:NMVVKAXGXH, KVGKAXXVPTKL:RPVQ:KKATV:EGMSVAEXGXR, KASKAXXVPTKL:RPVQ:KKATV:GMAVSEXGXR, GSAGPXXTPTKM:RPVQ:KKATV:GMVVDRXGXS, AAPASXXVPARL:RPVQ:KKATV:DMVVEAXGXR, STPPTXXVPTRL:RPVQ:KKATV:DMVVESXGXR, HVPKPXXAPTKL:RPVQ:KKATV:NMVVRSXGXH, RVPSTXXAPVKT: RPVQ:KKATV:MIVEEXGXL, ASAAPXXVPQAL:RPVQ:KKATV:MIVRSXKXS, ASASPSSVSQDL:RPVQ:KKATV:MIVKSXKXS, ASASPXXVPQDL:RPVQ:KKATV:MVVKSXKXS, NDEGLEXVPTEE:RPVQ:KKATV:FLQHNKXEXR, NDEGLEXVPTGQ:RPVQ:KKATV:LEEHSQXEXR and SSVKXQPSRVHH:RPVQ:KKATV:RLEEHLEXAXA.

In particular, in certain embodiments, the quadruplet PEP9:PEP12:PEP2:PEP10 is selected from the group consisting of GIPEPXXVPTKM:SAIS-AA$^{17}$-LYL:LKNYQ:NMVVESXAXR, SIPKAXXVPTEL:SAIS-AA$^{17}$-LYL:LKNYQ:EMVVEGXGXR, HVTKPTXVPTKL:SAIS-AA$^{17}$-LYL:LKNYQ:NMVVESXGXH, YVPKPXXVPTKL:SAIS-AA$^{17}$-LYL:LKNYQ:NMVVESXGXH, TVPKPXXVPTQL:SAIS-AA$^{17}$-LYL:LKNYQ:NMVVEAXGXH, AVPKAXXVPTKL:SAIS-AA$^{17}$-LYL:LKNYQ:NMVVEAXGXH, KVGKAXXVPTKL:SAIS-AA$^{17}$-LYL:LKNYQ:EGMSVVEXGXR, KASKAXXVPTKL:SAIS-AA$^{17}$-LYL:LKNYQ:GMVVEEXGXR, GSAGPXXVPTKM:SAIS-AA$^{17}$-LYL:LKNYQ:GMVVERXGXS, AAPASXXVPTRL:SAIS-AA$^{17}$-LYL:LKNYQ:DMVVEAXGXR, STPPTXXVPTRL:SAIS-AA$^{17}$-LYL:LKNYQ:DMVVESXGXR, HVPKPXXVPTKL:SAIS-AA$^{17}$-LYL:LKNYQ:NMVVESXGXH, RVPSTXXVPTKT:SAIS-AA$^{17}$-LYL:LKNYQ:MIVVEXGXL, ASAAPXXVPTAL:SAIS-AA$^{17}$-LYL:LKNYQ:MIVVEXKXS, ASASPXXVPTDL:SAIS-AA$^{17}$-LYL:LKNYQ:MIVVEXKXS, ASASPXXVPTDL:SAIS-AA$^{17}$-LYL:LKNYQ:MVVVEXKXS, NDEGLEXVPTEE:SAIS-AA$^{17}$-LYL:LKNYQ:FLVVEKXEXR, NDEGLEXVPTGQ:SAIS-AA$^{17}$-LYL:LKNYQ:LEVVEQXEXR, SSVKXQPVPTHH:SAIS-AA$^{17}$-LYL:LKNYQ:RLVVELEXAXA, RNVQXRPVPTQL:SAIS-AA$^{17}$-LYL:LKNYQ:TLVVELAXKXE, GIPEPXXVPEKM:SAIS-AA$^{17}$-LYL:LKNYQ:NMTVESXAXR, HVTKPTXAPTKL:SAIS-AA$^{17}$-LYL:LKNYQ:NMVVRSXGXH, YVPKPXXAPTKL:SAIS-AA$^{17}$-LYL:LKNYQ:NMVVRSXGXH, TVPKPXXAPTQL:SAIS-AA$^{17}$-LYL:LKNYQ:NMVVRAXGXH, AVPKAXXAPTKL:SAIS-AA$^{17}$-LYL:LKNYQ:NMVVKAXGXH, KVGKAXXVPTKL:SAIS-AA$^{17}$-LYL:LKNYQ:EGMSVAEXGXR, KASKAXXVPTKL:SAIS-AA$^{17}$-LYL:LKNYQ:GMAVSEXGXR,

GSAGPXXTPTKM:SAIS-AA$^{17}$-LYL:LKNYQ:GMVVDRXGXS, AAPASXXVPARL:SAIS-AA$^{17}$-LYL:LKNYQ:DMVVEAXGXR, HVPKPXXAPTKL:SAIS-AA$^{17}$-LYL:LKNYQ:NMVVRSXGXH, RVPSTXXAPVKT:SAIS-AA$^{17}$-LYL:LKNYQ:MIVEEXGXL, ASAAPXXVPQAL:SAIS-AA$^{17}$-LYL:LKNYQ:MIVRSXKXS, ASASPXXVSQDL:SAIS-AA$^{17}$-LYL:LKNYQ:MIVKSXKXS, ASASPXXVPQDL:SAIS-AA$^{17}$-LYL:LKNYQ:MVVKSXKXS, NDEGLEXVPTEE:SAIS-AA$^{17}$-LYL:LKNYQ:FLQHNKXEXR, NDEGLEXVPTGQ:SAIS-AA$^{17}$-LYL:LKNYQ:LEEHSQXEXR, SSVKXQPSRVHH:SAIS-AA$^{17}$-LYL:LKNYQ:RLEEHLEXAXA, RNVQXRPTQVQL:SAIS-AA$^{17}$-LYL:LKNYQ:TLEDHLAXKXE, KIPKAXXVPEEL:SSLS-AA$^{17}$-LFF:LKVYP:DMTVEGXGXR, SIPKAXXVPEEL:SSLS-AA$^{17}$-LFF:LKVYP:EMTVEGXGXR, HVTKPTXVPEKL:SSLS-AA$^{17}$-LFF:LKVYP:NMTVESXGXH, YVPKPXXVPEKL:SSLS-AA$^{17}$-LFF:LKVYP:NMTVESXGXH, TVPKPXXVPEQL:SSLS-AA$^{17}$-LFF:LKVYP:NMTVEAXGXH, AVPKAXXVPEKL:SSLS-AA$^{17}$-LFF:LKVYP:NMTVEAXGXH, KVGKAXXVPEKL:SSLS-AA$^{17}$-LFF:LKVYP:EGMSTVEXGXR, KASKAXXVPEKL:SSLS-AA$^{17}$-LFF:LKVYP:GMTVEEXGXR, GSAGPXXVPEKM:SSLS-AA$^{17}$-LFF:LKVYP:GMTVERXGXS, AAPASXXVPERL:SSLS-AA$^{17}$-LFF:LKVYP:DMTVEAXGXR, STPPTXXVPERL:SSLS-AA$^{17}$-LFF:LKVYP:DMTVESXGXR, HVPKPXXVPEKL:SSLS-AA$^{17}$-LFF:LKVYP:NMTVESXGXH, RVPSTXXVPEKT:SSLS-AA$^{17}$-LFF:LKVYP:MITVEXGXL, ASAAPXXVPEAL:SSLS-AA$^{17}$-LFF:LKVYP:MITVEXKXS, ASASPXXVPEDL:SSLS-AA$^{17}$-LFF:LKVYP:MITVEXKXS, ASASPXXVPEDL:SSLS-AA$^{17}$-LFF:LKVYP:MVTVEXKXS, NDEGLEXVPEEE:SSLS-AA$^{17}$-LFF:LKVYP:FLTVEKXEXR, NDEGLEXVPEGQ:SSLS-AA$^{17}$-LFF:LKVYP:LETVEQXEXR, SSVKXQPVPEHH:SSLS-AA$^{17}$-LFF:LKVYP:RLTVELEXAXA, RNVQXRPVPEQL:SSLS-AA$^{17}$-LFF:LKVYP:TLTVELAXKXE, KIPKAXXVPTEL:SSLS-AA$^{17}$-LFF:LKVYP:DMVVEGXGXR, SIPKAXXVPTEL:SSLS-AA$^{17}$-LFF:LKVYP:EMVVEGXGXR, HVTKPTXAPTKL:SSLS-AA$^{17}$-LFF:LKVYP:NMVVRSXGXH, YVPKPXXAPTKL:SSLS-AA$^{17}$-LFF:LKVYP:NMVVRSXGXH, TVPKPXXAPTQL:SSLS-AA$^{17}$-LFF:LKVYP:NMVVRAXGXH, AVPKAXXAPTKL:SSLS-AA$^{17}$-LFF:LKVYP:NMVVKAXGXH, KVGKAXXVPTKL:SSLS-AA$^{17}$-LFF:LKVYP:EGMSVAEXGXR, KASKAXXVPTKL:SSLS-AA$^{17}$-LFF:LKVYP:GMAVSEXGXR, GSAGPXXTPTKM:SSLS-AA$^{17}$-LFF:LKVYP:GMVVDRXGXS, AAPASXXVPARL:SSLS-AA$^{17}$-LFF:LKVYP:DMVVEAXGXR, STPPTXXVPTRL:SSLS-AA$^{17}$-LFF:LKVYP:DMVVESXGXR, HVPKPXXAPTKL:SSLS-AA$^{17}$-LFF:LKVYP:NMVVRSXGXH, RVPSTXXAPVKT:SSLS-AA$^{17}$-LFF:LKVYP:MIVEEXGXL, ASAAPXXVPQAL:SSLS-AA$^{17}$-LFF:LKVYP:MIVRSXKXS, ASASPXXVSQDL:SSLS-AA$^{17}$-LFF:LKVYP:MIVKSXKXS, ASASPXXVPQDL:SSLS-AA$^{17}$-LFF:LKVYP:MVVKSXKXS, NDEGLEXVPTEE:SSLS-AA$^{17}$-LFF:LKVYP:FLQHNKXEXR, NDEGLEXVPTGQ:SSLS-AA$^{17}$-LFF:LKVYP:LEEHSQXEXR, SSVKXQPSRVHH:SSLS-AA$^{17}$-LFF:LKVYP:RLEEHLEXAXA, RNVQXRPTQVQL:SSLS-AA$^{17}$-LFF:LKVYP:TLEDHLAXKXE, KIPKAXXVPTEL:SAIS-AA$^{17}$-LYL:LKNYQ:DMVVEGXGXR, KIPKAXXAPTEL:NAIS-AA$^{17}$-LYF:LKKYR:DMVVRGXGXR, GIPEPXXAPTKM:NAIS-AA$^{17}$-LYF:LKKYR:NMVVRSXAXR, SIPKAXXAPTEL:NAIS-AA$^{17}$-LYF:LKKYR:EMVVRGXGXR, YVPKPXXAPTKL:NAIS-AA$^{17}$-LYF:LKKYR:NMVVRSXGXH, TVPKPXXAPTQL:NAIS-AA$^{17}$-LYF:LKKYR:NMVVRAXGXH, AVPKAXXAPTKL:NAIS-AA$^{17}$-LYF:LKKYR:NMVVRAXGXH, KVGKAXXAPTKL:NAIS-AA$^{17}$-LYF:LKKYR:EGMSVVRXGXR, KASKAXXAPTKL:NAIS-AA$^{17}$-LYF:LKKYR:GMVVREXGXR, GSAGPXXAPTKM:NAIS-AA$^{17}$-LYF:LKKYR:GMVVRRXGXS, AAPASXXAPTRL:NAIS-AA$^{17}$-LYF:LKKYR:DMVVRAXGXR, STPPTXXAPTRL:NAIS-AA$^{17}$-LYF:LKKYR:DMVVRSXGXR, HVPKPXXAPTKL:NAIS-AA$^{17}$-LYF:LKKYR:NMVVRSXGXH, RVPSTXXAPTKT:NAIS-AA$^{17}$-LYF:LKKYR:MIVVRXGXL, ASAAPXXAPTAL:NAIS-AA$^{17}$-LYF:LKKYR:MIVVRXKXS, ASASPXXAPTDL:NAIS-AA$^{17}$-LYF:LKKYR:MIVVRXKXS, ASASPXXAPTDL:NAIS-AA$^{17}$-LYF:LKKYR:MVVVRXKXS, NDEGLESAPTEE:NAIS-AA$^{17}$-LYF:LKKYR:FLVVRKXEXR, NDEGLESAPTGQ:NAIS-AA$^{17}$-LYF:LKKYR:LEVVRQXEXR, SSVKXQPAPTHH:NAIS-AA$^{17}$-LYF:LKKYR:RLVVRLEXAXA, RNVQXRPAPTQL:NAIS-AA$^{17}$-LYF:LKKYR:TLVVRLAXKXE, KIPKAXXVPTEL:NAIS-AA$^{17}$-LYF:LKKYR:DMVVEGXGXR, GIPEPXXVPEKM:NAIS-AA$^{17}$-LYF:LKKYR:NMTVESXAXR, SIPKAXXVPTEL:NAIS-AA$^{17}$-LYF:LKKYR:EMVVEGXGXR, AVPKAXXAPTKL:NAIS-AA$^{17}$-LYF:LKKYR:NMVVKAXGXH, KVGKAXXVPTKL:NAIS-AA$^{17}$-LYF:LKKYR:EGMSVAEXGXR, KASKAXXVPTKL:NAIS-AA$^{17}$-LYF:LKKYR:GMAVSEXGXR, GSAGPXXTPTKM:NAIS-AA$^{17}$-LYF:LKKYR:GMVVDRXGXS, AAPASXXVPARL:NAIS-AA$^{17}$-LYF:LKKYR:DMVVEAXGXR, STPPTXXVPTRL:NAIS-AA$^{17}$-LYF:LKKYR:DMVVESXGXR, RVPSTXXAPVKT:NAIS-AA$^{17}$-LYF:LKKYR:MIVEEXGXL, ASAAPXXVPQAL:NAIS-AA$^{17}$-LYF:LKKYR:MIVRSXKXS, ASASPXXVSQDL:NAIS-AA$^{17}$-LYF:LKKYR:MIVKSXKXS, ASASPXXVPQDL:NAIS-AA$^{17}$-LYF:LKKYR:MVVKSXKXS, NDEGLEXVPTEE:NAIS-AA$^{17}$-LYF:LKKYR:FLQHNKXEXR, NDEGLEXVPTGQ:NAIS-AA$^{17}$-LYF:LKKYR:LEEHSQXEXR, SSVKXQPSRVHH:NAIS-AA$^{17}$-LYF:LKKYR:RLEEHLEXAXA, RNVQXRPTQVQL:NAIS-AA$^{17}$-LYF:LKKYR:TLEDHLAXKXE, HVTKPTXAPTKL:NAIS-AA$^{17}$-LYF:LKKYR:NMVVRSXGXH, KIPKAXXAPTEL:SATS-AA$^{17}$-LYY:LRKHR:DMVVKGXGXR, GIPEPXXAPTKM:SATS-AA$^{17}$-LYY:LRKHR:NMVVKSXAXR, SIPKAXXAPTEL:SATS-AA$^{17}$-LYY:LRKHR:EMVVKGXGXR, HVTKPTXAPTKL:SATS-AA$^{17}$-LYY:LRKHR:NMVVKSXGXH, YVPKPXXAPTKL:SATS-AA$^{17}$-LYY:LRKHR:NMVVKSXGXH, TVPKPXXAPTQL:SATS-AA$^{17}$-LYY:LRKHR:NMVVKAXGXH, KVGKAXXAPTKL:SATS-AA$^{17}$-LYY:LRKHR:EGMSVVKXGXR, KASKAXXAPTKL:SATS-AA$^{17}$-LYY:LRKHR:GMVVKEXGXR, GSAGPXXAPTKM:SATS-AA$^{17}$-LYY:LRKHR:GMVVKRXGXS, AAPASXXAPTRL:SATS-AA$^{17}$-LYY:LRKHR:DMVVKAXGXR, STPPTXXAPTRL:SATS-AA$^{17}$-LYY:LRKHR:DMVVKSXGXR, HVPKPXXAPTKL:SATS-AA$^{17}$-LYY:LRKHR:NMVVKSXGXH, RVPSTXXAPTKT:SATS-AA$^{17}$-LYY:LRKHR:MIVVKXGXL, ASAAPXXAPTAL:SATS-AA$^{17}$-LYY:LRKHR:MIVVKXKXS, ASASPXXAPTDL:SATS-AA$^{17}$-LYY:LRKHR:MIVVKXKXS, ASASPXXAP-

TDL:SATS-AA$^{17}$-LYY:LRKHR:MVVVKXKXS, NDEGLESAPTEE:SATS-AA$^{17}$-LYY:LRKHR:FLVVKKXEXR, NDEGLESAPTGQ:SATS-AA$^{17}$-LYY:LRKHR:LEVVKQXEXR, SSVKXQPAPTHH:SATS-AA$^{17}$-LYY:LRKHR:RLVVKLEXAXA, RNVQXRPAPTQL:SATS-AA$^{17}$-LYY:LRKHR:TLVVKLAXKXE, KIPKAXXVPTEL:SATS-AA$^{17}$-LYY:LRKHR:DMVVEGXGXR, GIPEPXXVPEKM:SATS-AA$^{17}$-LYY:LRKHR:NMTVESXAXR, SIPKAXXVPTEL:SATS-AA$^{17}$-LYY:LRKHR:EMVVEGXGXR, HVTKPTXAPTKL:SATS-AA$^{17}$-LYY:LRKHR:NMVVRSXGXH, YVPKPXXAPTKL:SATS-AA$^{17}$-LYY:LRKHR:NMVVRSXGXH, TVPKPXXAPTQL:SATS-AA$^{17}$-LYY:LRKHR:NMVVRAXGXH, KVGKAXXVPTKL:SATS-AA$^{17}$-LYY:LRKHR:EGMSVAEXGXR, KASKAXXVPTKL:SATS-AA$^{17}$-LYY:LRKHR:GMAVSEXGXR, GSAGPXXTPTKM:SATS-AA$^{17}$-LYY:LRKHR:GMVVDRXGXS, AAPASXXVPARL:SATS-AA$^{17}$-LYY:LRKHR:DMVVEAXGXR, STPPTXXVPTRL:SATS-AA$^{17}$-LYY:LRKHR:DMVVESXGXR, HVPKPXXAPTKL:SATS-AA$^{17}$-LYY:LRKHR:NMVVRSXGXH, RVPSTXXAPVKT:SATS-AA$^{17}$-LYY:LRKHR:MIVEEXGXL, ASAAPXXVPQAL:SATS-AA$^{17}$-LYY:LRKHR:MIVRSXKXS, ASASPXXVSQDL:SATS-AA$^{17}$-LYY:LRKHR:MIVKSXKXS, ASASPXXVPQDL:SATS-AA$^{17}$-LYY:LRKHR:MVVKSXKXS, NDEGLEXVPTEE:SATS-AA$^{17}$-LYY:LRKHR:FLQHNKXEXR, NDEGLEXVPTGQ:SATS-AA$^{17}$-LYY:LRKHR:LEEHSQXEXR, SSVKXQPSRVHH:SATS-AA$^{17}$-LYY:LRKHR:RLEEHLEXAXA, RNVQXRPTQVQL:SATS-AA$^{17}$-LYY:LRKHR:TLEDHLAXKXE, KIPKAXXVPTEL:SPIS-AA$^{17}$-LYK:LKYHY:DMVAEGXGXR, GIPEPXXVPTKM:SPIS-AA$^{17}$-LYK:LKYHY:NMVAESXAXR, SIPKAXXVPTEL:SPIS-AA$^{17}$-LYK:LKYHY:EMVAEGXGXR, HVTKPTXVPTKL:SPIS-AA$^{17}$-LYK:LKYHY:NMVAESXGXH, YVPKPXXVPTKL:SPIS-AA$^{17}$-LYK:LKYHY:NMVAESXGXH, TVPKPXXVPTQL:SPIS-AA$^{17}$-LYK:LKYHY:NMVAEAXGXH, AVPKAXXVPTKL:SPIS-AA$^{17}$-LYK:LKYHY:NMVAEAXGXH, KASKAXXVPTKL:SPIS-AA$^{17}$-LYK:LKYHY:GMVAEEXGXR, GSAGPXXVPTKM:SPIS-AA$^{17}$-LYK:LKYHY:GMVAERXGXS, AAPASXXVPTRL:SPIS-AA$^{17}$-LYK:LKYHY:DMVAEAXGXR, STPPTXXVPTRL:SPIS-AA$^{17}$-LYK:LKYHY:DMVAESXGXR, HVPKPXXVPTKL:SPIS-AA$^{17}$-LYK:LKYHY:NMVAESXGXH, RVPSTXXVPTKT:SPIS-AA$^{17}$-LYK:LKYHY:MIVAEXGXL, ASAAPXXVPTAL:SPIS-AA$^{17}$-LYK: LKYHY: MIVAEXKXS, ASASPXXVPTDL:SPIS-AA$^{17}$-LYK:LKYHY:MIVAEXKXS, ASASPXXVPTDL:SPIS-AA$^{17}$-LYK:LKYHY:MVVAEXKXS, NDEGLEXVPTEE:SPIS-AA$^{17}$-LYK:LKYHY:FLVAEKXEXR, NDEGLEXVPTGQ:SPIS-AA$^{17}$-LYK:LKYHY:LEVAEQXEXR, SSVKXQPVPTHH:SPIS-AA$^{17}$-LYK:LKYHY:RLVAELEXAXA, RNVQXRPVPTQL:SPIS-AA$^{17}$-LYK:LKYHY:TLVAELAXKXE, KIPKAXXVPTEL:SPIS-AA$^{17}$-LYK:LKYHY:DMVVEGXGXR, GIPEPXXVPEKM:SPIS-AA$^{17}$-LYK:LKYHY:NMTVESXAXR, SIPKAXXVPTEL:SPIS-AA$^{17}$-LYK:LKYHY:EMVVEGXGXR, HVTKPTXAPTKL:SPIS-AA$^{17}$-LYK:LKYHY:NMVVRSXGXH, YVPKPXXAPTKL:SPIS-AA$^{17}$-LYK:LKYHY:NMVVRSXGXH, TVPKPXXAPTQL:SPIS-AA$^{17}$-LYK:LKYHY:NMVVRAXGXH, AVPKAXXAPTKL:SPIS-AA$^{17}$-LYK:LKYHY:NMVVKAXGXH, KASKAXXVPTKL:SPIS-AA$^{17}$-LYK:LKYHY:GMAVSEXGXR, GSAGPXXTPTKM:SPIS-AA$^{17}$-LYK:LKYHY:GMVVDRXGXS, AAPASXXVPARL:SPIS-AA$^{17}$-LYK:LKYHY:DMVVEAXGXR, STPPTXXVPTRL:SPIS-AA$^{17}$-LYK:LKYHY:DMVVESXGXR, HVPKPXXAPTKL:SPIS-AA$^{17}$-LYK:LKYHY:NMVVRSXGXH, RVPSTXXAPVKT:SPIS-AA$^{17}$-LYK:LKYHY:MIVEEXGXL, ASAAPXXVPQAL:SPIS-AA$^{17}$-LYK:LKYHY:MIVRSXKXS, ASASPXXVSQDL:SPIS-AA$^{17}$-LYK:LKYHY: MIVKSXKXS, ASASPXXVPQDL:SPIS-AA$^{17}$-LYK:LKYHY:MVVKSXKXS, NDEGLEXVPTEE:SPIS-AA$^{17}$-LYK:LKYHY:FLQHNKXEXR, NDEGLEXVPTGQ:SPIS-AA$^{17}$-LYK:LKYHY:LEEHSQXEXR, SSVKXQPSRVHH:SPIS-AA$^{17}$-LYK:LKYHY:RLEEHLEXAXA, RNVQXRPTQVQL:SPIS-AA$^{17}$-LYK:LKYHY:TLEDHLAXKXE, KIPKAXXVPTEL:EPIS-AA$^{17}$-LYL:KFKYE:DMAVXGXGSR, GIPEPXXVPTKM:EPIS-AA$^{17}$-LYL:KFKYE:NMAVSSXAXR, SIPKAXXVPTEL:EPIS-AA$^{17}$-LYL:KFKYE:EMAVXGXGSR, HVTKPTXVPTKL:EPIS-AA$^{17}$-LYL:KFKYE:NMAVSSXGXH, YVPKPXXVPTKL:EPIS-AA$^{17}$-LYL:KFKYE:NMAVSSXGXH, TVPKPXXVPTQL:EPIS-AA$^{17}$-LYL:KFKYE:NMAVSAXGXH, AVPKAXXVPTKL:EPIS-AA$^{17}$-LYL:KFKYE:NMAVSAXGXH, KVGKAXXVPTKL:EPIS-AA$^{17}$-LYL:KFKYE:EGMSAVSXGXR, GSAGPXXVPTKM:EPIS-AA$^{17}$-LYL:KFKYE:GMAVSRXGXS, AAPASXXVPTRL:EPIS-AA$^{17}$-LYL:KFKYE:DMAVSAXGXR, STPPTXXVPTRL:EPIS-AA$^{17}$-LYL:KFKYE:DMAVSSXGXR, HVPKPXXVPTKL:EPIS-AA$^{17}$-LYL:KFKYE:NMAVSSXGXH, RVPSTXXVPTKT:EPIS-AA$^{17}$-LYL:KFKYE:MIAVSXGXL, ASAAPXXVPTAL:EPIS-AA$^{17}$-LYL:KFKYE:MIAVSXKXS, ASASPXXVPTDL:EPIS-AA$^{17}$-LYL:KFKYE:MIAVSXKXS, ASASPXXVPTDL:EPIS-AA$^{17}$-LYL:KFKYE:MVAVSXKXS, NDEGLEXVPTEE:EPIS-AA$^{17}$-LYL:KFKYE:FLAVXKXESR, NDEGLEXVPTGQ:EPIS-AA$^{17}$-LYL:KFKYE:LEAVSQXEXR, SSVKXQPVPTHH:EPIS-AA$^{17}$-LYL:KFKYE:RLAVSLEXAXA, RNVQXRPVPTQL:EPIS-AA$^{17}$-LYL:KFKYE:TLAVSLAXKXE, KIPKAXXVPTEL:EPIS-AA$^{17}$-LYL:KFKYE:DMVVEGXGXR, GIPEPXXVPEKM:EPIS-AA$^{17}$-LYL:KFKYE:NMTVESXAXR, SIPKAXXVPTEL:EPIS-AA$^{17}$-LYL:KFKYE:EMVVEGXGXR, HVTKPTXAPTKL:EPIS-AA$^{17}$-LYL:KFKYE:NMVVRSXGXH, YVPKPXXAPTKL:EPIS-AA$^{17}$-LYL:KFKYE:NMVVRSXGXH, TVPKPXXAPTQL:EPIS-AA$^{17}$-LYL:KFKYE:NMVVRAXGXH, AVPKAXXAPTKL:EPIS-AA$^{17}$-LYL:KFKYE:NMVVKAXGXH, KVGKAXXVPTKL:EPIS-AA$^{17}$-LYL:KFKYE:EGMSVAEXGXR, GSAGPXXTPTKM:EPIS-AA$^{17}$-LYL:KFKYE:GMVVDRXGXS, AAPASXXVPARL:EPIS-AA$^{17}$-LYL:KFKYE:DMVVEAXGXR, STPPTXXVPTRL:EPIS-AA$^{17}$-LYL:KFKYE:DMVVESXGXR, HVPKPXXAPTKL:EPIS-AA$^{17}$-LYL:KFKYE:NMVVRSXGXH, RVPSTXXAPVKT:EPIS-AA$^{17}$-LYL:KFKYE:MIVEEXGXL, ASAAPXXVPQAL:EPIS-AA$^{17}$-LYL:KFKYE:MIVRSXKXS, ASASPXXVSQDL:EPIS-AA$^{17}$-LYL:KFKYE:MIVKSXKXS, ASASPXXVPQDL:EPIS-AA$^{17}$-LYL:KFKYE:MVVKSXKXS, NDEGLEXVPTEE:EPIS-AA$^{17}$-LYL:KFKYE:FLQHNKXEXR, NDEGLEXVPTGQ:EPIS-AA$^{17}$-LYL:KFKYE:LEEHSQXEXR, SSVKXQPSRVHH:EPIS-AA$^{17}$-LYL:KFKYE:RLEEHLEXAXA, RNVQXRPTQVQL:EPIS-AA$^{17}$-LYL:KFKYE:TLEDHLAXKXE, KIPKASSTPTEL:SPIN-AA$^{17}$-LYF:YGKIP:DMVVDGXGXR,

GIPEPXXTPTKM:SPIN-AA$^{17}$-LYF:YGKIP:NMVVDSXAXR, SIPKASSTPTEL:SPIN-AA$^{17}$-LYF:YGKIP:EMVVDGXGXR, HVTKPTSTPTKL:SPIN-AA$^{17}$-LYF:YGKIP:NMVVDSXGXH, YVPKPXXTPTKL:SPIN-AA$^{17}$-LYF:YGKIP:NMVVDSXGXH, TVPKPXXTPTQL:SPIN-AA$^{17}$-LYF:YGKIP:NMVVDAXGXH, AVPKASSTPTKL:SPIN-AA$^{17}$-LYF:YGKIP:NMVVDAXGXH, KVGKASSTPTKL:SPIN-AA$^{17}$-LYF:YGKIP:EGMSVVDXGXR, KASKASSTPTKL:SPIN-AA$^{17}$-LYF:YGKIP:GMVVDEXGXR, AAPASSSTPTRL:SPIN-AA$^{17}$-LYF:YGKIP:DMVVDAXGXR, STPPTSSTPTRL:SPIN-AA$^{17}$-LYF:YGKIP:DMVVDSXGXR, HVPKPXXTPTKL:SPIN-AA$^{17}$-LYF:YGKIP:NMVVDSXGXH, RVPSTSSTPTKT:SPIN-AA$^{17}$-LYF:YGKIP:MIVVDXGXL, ASAAPXXTPTAL:SPIN-AA$^{17}$-LYF:YGKIP:MIVVDXKXS, ASASPXXTPTDL:SPIN-AA$^{17}$-LYF:YGKIP:MIVVDXKXS, ASASPXXTPTDL:SPIN-AA$^{17}$-LYF:YGKIP:MVVVDXKXS, NDEGLESTPTEE:SPIN-AA$^{17}$-LYF:YGKIP:FLVVDKXEXR, NDEGLESTPTGQ:SPIN-AA$^{17}$-LYF:YGKIP:LEVVDQXEXR, SSVKXQPTPTHH:SPIN-AA$^{17}$-LYF:YGKIP:RLVVDLEXAXA, RNVQXRPTPTQL:SPIN-AA$^{17}$-LYF:YGKIP:TLVVDLAXKXE, KIPKAXXVPTEL:SPIN-AA$^{17}$-LYF:YGKIP:DMVVEGXGXR, GIPEPXXVPEKM:SPIN-AA$^{17}$-LYF:YGKIP:NMTVESXAXR, SIPKAXXVPTEL:SPIN-AA$^{17}$-LYF:YGKIP:EMVVEGXGXR, HVTKPTXAPTKL:SPIN-AA$^{17}$-LYF:YGKIP:NMVVRSXGXH, YVPKPXXAPTKL:SPIN-AA$^{17}$-LYF:YGKIP:NMVVRSXGXH, TVPKPXXAPTQL:SPIN-AA$^{17}$-LYF:YGKIP:NMVVRAXGXH, AVPKAXXAPTKL:SPIN-AA$^{17}$-LYF:YGKIP:NMVVKAXGXH, KVGKAXXVPTKL:SPIN-AA$^{17}$-LYF:YGKIP:EGMSVAEXGXR, KASKAXXVPTKL:SPIN-AA$^{17}$-LYF:YGKIP:GMAVSEXGXR, AAPASXXVPARL:SPIN-AA$^{17}$-LYF:YGKIP:DMVVEAXGXR, STPPTXXVPTRL:SPIN-AA$^{17}$-LYF:YGKIP:DMVVESXGXR, HVPKPXXAPTKL:SPIN-AA$^{17}$-LYF:YGKIP:NMVVRSXGXH, RVPSTXXAPVKT:SPIN-AA$^{17}$-LYF:YGKIP:MIVEEXGXL, ASAAPXXVPQAL:SPIN-AA$^{17}$-LYF:YGKIP:MIVRSXKXS, ASASPXXVSQDL:SPIN-AA$^{17}$-LYF:YGKIP:MIVKSXKXS, ASASPXXVPQDL:SPIN-AA$^{17}$-LYF:YGKIP:MVVKSXKXS, NDEGLEXVPTEE:SPIN-AA$^{17}$-LYF:YGKIP:FLQHNKXEXR, NDEGLEXVPTGQ:SPIN-AA$^{17}$-LYF:YGKIP:LEEHSQXEXR, SSVKXQPSRVHH:SPIN-AA$^{17}$-LYF:YGKIP:RLEEHLEXAXA, RNVQXRPTQVQL:SPIN-AA$^{17}$-LYF:YGKIP:TLEDHLAXKXE, KIPKAXXVPAEL:SPIS-AA$^{17}$-LYI:YKQYE:DMVVEGXGXR, GIPEPXXVPAKM:SPIS-AA$^{17}$-LYI:YKQYE:NMVVESXAXR, SIPKAXXVPAEL:SPIS-AA$^{17}$-LYI:YKQYE:EMVVEGXGXR, HVTKPTXVPAKL:SPIS-AA$^{17}$-LYI:YKQYE:NMVVESXGXH, YVPKPXXVPAKL:SPIS-AA$^{17}$-LYI:YKQYE:NMVVESXGXH, TVPKPXXVPAQL:SPIS-AA$^{17}$-LYI:YKQYE:NMVVEAXGXH, AVPKAXXVPAKL:SPIS-AA$^{17}$-LYI:YKQYE:NMVVEAXGXH, KVGKAXXVPAKL:SPIS-AA$^{17}$-LYI:YKQYE:EGMSVVEXGXR, KASKAXXVPAKL:SPIS-AA$^{17}$-LYI:YKQYE:GMVVEEXGXR, GSAGPXXVPAKM:SPIS-AA$^{17}$-LYI:YKQYE:GMVVERXGXS, STPPTXXVPARL:SPIS-AA$^{17}$-LYI:YKQYE:DMVVESXGXR, HVPKPXXVPAKL:SPIS-AA$^{17}$-LYI:YKQYE:NMVVESXGXH, RVPSTXXVPAKT:SPIS-AA$^{17}$-LYI:YKQYE:MIVVEXGXL, ASAAPXXVPAAL:SPIS-AA$^{17}$-LYI:YKQYE:MIVVEXKXS, ASASPXXVPADL:SPIS-AA$^{17}$-LYI:YKQYE:MIVVEXKXS, ASASPXXVPADL:SPIS-AA$^{17}$-LYI:YKQYE:MVVVEXKXS, NDEGLEXVPAEE:SPIS-AA$^{17}$-LYI:YKQYE:FLVVEKXEXR, NDEGLEXVPAGQ:SPIS-AA$^{17}$-LYI:YKQYE:LEVVEQXEXR, SSVKXQPVPAHH:SPIS-AA$^{17}$-LYI:YKQYE:RLVVELEXAXA, RNVQXRPVPAQL:SPIS-AA$^{17}$-LYI:YKQYE:TLVVELAXKXE, KIPKAXXVPTEL:SPIS-AA$^{17}$-LYI:YKQYE:DMVVEGXGXR, GIPEPXXVPEKM:SPIS-AA$^{17}$-LYI:YKQYE:NMTVESXAXR, SIPKAXXVPTEL:SPIS-AA$^{17}$-LYI:YKQYE:EMVVEGXGXR, HVTKPTXAPTKL:SPIS-AA$^{17}$-LYI:YKQYE:NMVVRSXGXH, YVPKPXXAPTKL:SPIS-AA$^{17}$-LYI:YKQYE:NMVVRSXGXH, TVPKPXXAPTQL:SPIS-AA$^{17}$-LYI:YKQYE:NMVVRAXGXH, AVPKAXXAPTKL:SPIS-AA$^{17}$-LYI:YKQYE:NMVVKAXGXH, KVGKAXXVPTKL:SPIS-AA$^{17}$-LYI:YKQYE:EGMSVAEXGXR, KASKAXXVPTKL:SPIS-AA$^{17}$-LYI:YKQYE:GMAVSEXGXR, GSAGPXXTPTKM:SPIS-AA$^{17}$-LYI:YKQYE:GMVVDRXGXS, STPPTXXVPTRL:SPIS-AA$^{17}$-LYI:YKQYE:DMVVESXGXR, HVPKPXXAPTKL:SPIS-AA$^{17}$-LYI:YKQYE:NMVVRSXGXH, RVPSTXXAPVKT:SPIS-AA$^{17}$-LYI:YKQYE:MIVEEXGXL, ASAAPXXVPQAL:SPIS-AA$^{17}$-LYI:YKQYE:MIVRSXKXS, ASASPXXVSQDL:SPIS-AA$^{17}$-LYI:YKQYE:MIVKSXKXS, ASASPXXVPQDL:SPIS-AA$^{17}$-LYI:YKQYE:MVVKSXKXS, NDEGLEXVPTEE:SPIS-AA$^{17}$-LYI:YKQYE:FLQHNKXEXR, NDEGLEXVPTGQ:SPIS-AA$^{17}$-LYI:YKQYE:LEEHSQXEXR, SSVKXQPSRVHH:SPIS-AA$^{17}$-LYI:YKQYE:RLEEHLEXAXA, RNVQXRPTQVQL:SPIS-AA$^{17}$-LYI:YKQYE:TLEDHLAXKXE, KIPKAXXVPTEL:SPIS-AA$^{17}$-LFI:YKQYE:DMVVEGXGXR, GIPEPXXVPTKM:SPIS-AA$^{17}$-LFI:YKQYE:NMVVESXAXR, SIPKAXXVPTEL:SPIS-AA$^{17}$-LFI:YKQYE:EMVVEGXGXR, HVTKPTXVPTKL:SPIS-AA$^{17}$-LFI:YKQYE:NMVVESXGXH, YVPKPXXVPTKL:SPIS-AA$^{17}$-LFI:YKQYE:NMVVESXGXH, TVPKPXXVPTQL:SPIS-AA$^{17}$-LFI:YKQYE:NMVVEAXGXH, AVPKAXXVPTKL:SPIS-AA$^{17}$-LFI:YKQYE:NMVVEAXGXH, KVGKAXXVPTKL:SPIS-AA$^{17}$-LFI:YKQYE:EGMSVVEXGXR, KASKAXXVPTKL:SPIS-AA$^{17}$-LFI:YKQYE:GMVVEEXGXR, GSAGPXXVPTKM:SPIS-AA$^{17}$-LFI:YKQYE:GMVVERXGXS, AAPASXXVPTRL:SPIS-AA$^{17}$-LFI:YKQYE:DMVVEAXGXR, HVPKPXXVPTKL:SPIS-AA$^{17}$-LFI:YKQYE:NMVVESXGXH, RVPSTXXVPTKT:SPIS-AA$^{17}$-LFI:YKQYE:MIVVEXGXL, ASAAPXXVPTAL:SPIS-AA$^{17}$-LFI:YKQYE:MIVVEXKXS, ASASPXXVPTDLSPIS-AA$^{17}$-LFI:YKQYE:MIVVEXKXS, ASASPXXVPTDL:SPIS-AA$^{17}$-LFI:YKQYE:MVVVEXKXS, NDEGLEXVPTEE:SPIS-AA$^{17}$-LFI:YKQYE:FLVVEKXEXR, NDEGLEXVPTGQ:SPIS-AA$^{17}$-LFI:YKQYE:LEVVEQXEXR, SSVKXQPVPTHH:SPIS-AA$^{17}$-LFI:YKQYE:RLVVELEXAXA, RNVQXRPVPTQL:SPIS-AA$^{17}$-LFI:YKQYE:TLVVELAXKXE, GIPEPXXVPEKM:SPIS-AA$^{17}$-LFI:YKQYE:NMTVESXAXR, HVTKPTXAPTKL:SPIS-AA$^{17}$-LFI:YKQYE:NMVVRSXGXH, YVPKPXXAPTKL:SPIS-AA$^{17}$-LFI:YKQYE:NMVVRSXGXH, TVPKPXXAPTQL:SPIS-AA$^{17}$-LFI:YKQYE:NMVVRAXGXH, AVPKAXXAPTKL:SPIS-AA$^{17}$-LFI:YKQYE:NMVVKAXGXH, KVGKAXXVPTKL:SPIS-AA$^{17}$-LFI:YKQYE:EGMSVAEXGXR, KASKAXXVPTKL:SPIS-AA$^{17}$-LFI:YKQYE:GMAVSEXGXR, GSAGPXXTPTKM:SPIS-AA$^{17}$-LFI:YKQYE:GMVVDRXGXS, AAPASXXVPARL:SPIS-AA$^{17}$-LFI:YKQYE:DM-

VVEAXGXR, HVPKPXXAPTKL:SPIS-AA$^{17}$-LFI:YKQYE:NMVVRSXGXH, RVPSTXXAPVKT:SPIS-AA$^{17}$-LFI:YKQYE:MIVEEXGXL, ASAAPXXVPQAL:SPIS-AA$^{17}$-LFI:YKQYE:MIVRSXKXS, ASASPXXVSQDL:SPIS-AA$^{17}$-LFI:YKQYE:MIVKSXKXS, ASASPXXVPQDL:SPIS-AA$^{17}$-LFI:YKQYE:MVVKSXKXS, NDEGLEXVPTEE:SPIS-AA$^{17}$-LFI:YKQYE:FLQHNKXEXR, NDEGLEXVPTGQ:SPIS-AA$^{17}$-LFI:YKQYE:LEEHSQXEXR, SSVKXQP-SRVHH:SPIS-AA$^{17}$-LFI:YKQYE:RLEEHLEXAXA, RNVQXRPTQVQL:SPIS-AA$^{17}$-LFI:YKQYE:TLEDHLAXKXE, KIP-KAXXAPVEL:KPLS-AA$^{17}$-LYV:DHHKD:DMVEEGXGXR, GIPEPXXAPVKM:KPLS-AA$^{17}$-LYV:DHHKD:NMVEESXAXR, SIPKAXXAPVEL:KPLS-AA$^{17}$-LYV:DHHKD:EMVEEGXGXR, HVTKPTXAPVKL:KPLS-AA$^{17}$-LYV:DH-HKD:NMVEESXGXH, YVPKPXXAPVKL:KPLS-AA$^{17}$-LYV:DHHKD:NMVEESXGXH, TVPKPXXAPVQL:KPLS-AA$^{17}$-LYV:DHHKD:NMVEEAXGXH, AVPKAXXAPVKL:KPLS-AA$^{17}$-LYV:DHHKD:NMVEEAXGXH, KVGKAXXA-PVKL:KPLS-AA$^{17}$-LYV:DHHKD:EGMSVEEXGXR, KASKAXXAPVKL:KPLS-AA$^{17}$-LYV:DHHKD:GMVEEEXGXR, GSAGPXXAPVKM:KPLS-AA$^{17}$-LYV:DHHKD:GMVEERXGXS, AAPASXXAPVRL:KPLS-AA$^{17}$-LYV:DHHKD:DMVEE-AXGXR, STPPTXXAPVRL:KPLS-AA$^{17}$-LYV:DHHKD:DMVEESXGXR, HVPKPXXAPVKL:KPLS-AA$^{17}$-LYV:DH-HKD:NMVEESXGXH, ASAAPXXAPVAL:KPLS-AA$^{17}$-LYV:DHHKD:MIVEEXKXS, ASASPXXAPVDL:KPLS-AA$^{17}$-LYV:DHHKD:MIVEEXKXS, ASASPXXAPVDL:KPLS-AA$^{17}$-LYV:DHHKD:MVVEEXKXS, NDEGLES-APVEE:KPLS-AA$^{17}$-LYV:DHHKD:FLVEEKXEXR, NDEGLESAPVGQ:KPLS-AA$^{17}$-LYV:DHHKD:LEVEEQXEXR, SS-VKXQPAPVHH:KPLS-AA$^{17}$-LYV:DHHKD:RLVEELEXAXA, RNVQXRPAPVQL:KPLS-AA$^{17}$-LYV:DH-HKD:TLVEELAXKXE, KIPKAXXVPTEL:KPLS-AA$^{17}$-LYV:DHHKD:DMVVEGXGXR, GIPEPXXVPEKM:KPLS-AA$^{17}$-LYV:DHHKD:NMTVESXAXR, SIPKAXXVPTEL:KPLS-AA$^{17}$-LYV:DHHKD:EMVVEGXGXR, HVTKPTXA-PTKL:KPLS-AA$^{17}$-LYV:DHHKD:NMVVRSXGXH, YVPKPXXAPTKL:KPLS-AA$^{17}$-LYV:DHHKD:NMVVRSXGXH, TVPK-PXXAPTQL:KPLS-AA$^{17}$-LYV:DHHKD:NMVVRAXGXH, AVPKAXXAPTKL:KPLS-AA$^{17}$-LYV:DHHKD:NMVVKAXGXH, KVGKAXXVPTKL:KPLS-AA$^{17}$-LYV:DHHKD:EGMSVAEXGXR, KASKAXXVPTKL:KPLS-AA$^{17}$-LYV:DHHKD:GMAV-SEXGXR, GSAGPXXTPTKM:KPLS-AA$^{17}$-LYV:DHHKD:GMVVDRXGXS, AAPASXXVPARL:KPLS-AA$^{17}$-LYV:DH-HKD:DMVVEAXGXR, STPPTXXVPTRL:KPLS-AA$^{17}$-LYV:DHHKD:DMVVESXGXR, HVPKPXXAPTKL:KPLS-AA$^{17}$-LYV:DHHKD:NMVVRSXGXH, ASAAPXXVPQAL:KPLS-AA$^{17}$-LYV:DHHKD:MIVRSXKXS, ASAS-PXXVSQDL:KPLS-AA$^{17}$-LYV:DHHKD:MIVKSXKXS, ASASPXXVPQDL:KPLS-AA$^{17}$-LYV:DHHKD:MVVKSXKXS, NDEGLEXVPTEE:KPLS-AA$^{17}$-LYV:DHHKD:FLQHNKXEXR, NDEGLEXVPTGQ:KPLS-AA$^{17}$-LYV:DHHKD:LEEHSQX-EXR, SSVKXQPSRVHH:KPLS-AA$^{17}$-LYV:DHHKD:RLEEHLEXAXA, RNVQXRPTQVQL:KPLS-AA$^{17}$-LYV:DH-HKD:TLEDHLAXKXE, KIPKAXXVPQEL:EPLP-AA$^{17}$-VYY:EQLSN:DMVRXGXGSR, GIPEPXXVPQKM:EPLP-AA$^{17}$-VYY:EQLSN:NMVRSSXAXR, SIPKAXXVPQEL:EPLP-AA$^{17}$-VYY:EQLSN:EMVRXGXGSR, HVTKP-TXVPQKL:EPLP-AA$^{17}$-VYY:EQLSN:NMVRSSXGXH, YVPKPXXVPQKL:EPLP-AA$^{17}$-VYY:EQLSN:NMVRSSXGXH, TVPKPXXVPQQL:EPLP-AA$^{17}$-VYY:EQLSN:NMVRSAXGXH, AVPKAXXVPQKL:EPLP-AA$^{17}$-VYY:EQLSN:NMVR-SAXGXH, KVGKAXXVPQKL:EPLP-AA$^{17}$-VYY:EQLSN:EGMSVRSXGXR, KASKAXXVPQKL:EPLP-AA$^{17}$-VYY:EQL-SN:GMVRSEXGXR, GSAGPXXVPQKM:EPLP-AA$^{17}$-VYY:EQLSN:GMVRSRXGXS, AAPASXXVPQRL:EPLP-AA$^{17}$-VYY:EQLSN:DMVRSAXGXR, STPPTXXVPQRL:EPLP-AA$^{17}$-VYY:EQLSN:DMVRSSXGXR, HVPK-PXXVPQKL:EPLP-AA$^{17}$-VYY:EQLSN:NMVRSSXGXH, RVPSTXXVPQKT:EPLP-AA$^{17}$-VYY:EQLSN:MIVRSXGXL, ASASPXXVPQDL:EPLP-AA$^{17}$-VYY:EQLSN:MIVRSXKXS, ASASPXXVPQDL:EPLP-AA$^{17}$-VYY:EQLSN:MV-VRSXKXS, NDEGLEXVPQEE:EPLP-AA$^{17}$-VYY:EQLSN:FLVRXKXESR, NDEGLEXVPQGQ:EPLP-AA$^{17}$-VYY:EQL-SN:LEVRSQXEXR, SSVKXQPVPQHH:EPLP-AA$^{17}$-VYY:EQLSN:RLVRSLEXAXA, RNVQXRPVPQQL:EPLP-AA$^{17}$-VYY:EQLSN:TLVRSLAXKXE, KIPKAXXVPTEL:EPLP-AA$^{17}$-VYY:EQLSN:DMVVEGXGXR, GIPEPXXVPEKM:EPLP-AA$^{17}$-VYY:EQLSN:NMTVESXAXR, SIPKAXXVPTEL:EPLP-AA$^{17}$-VYY:EQLSN:EM-VVEGXGXR, HVTKPTXAPTKL:EPLP-AA$^{17}$-VYY:EQLSN:NMVVRSXGXH, YVPKPXXAPTKL:EPLP-AA$^{17}$-VYY:EQL-SN:NMVVRSXGXH, TVPKPXXAPTQL:EPLP-AA$^{17}$-VYY:EQLSN:NMVVRAXGXH, AVPKAXXAPTKL:EPLP-AA$^{17}$-VYY:EQLSN:NMVVKAXGXH, KVGKAXXVPTKL:EPLP-AA$^{17}$-VYY:EQLSN:EGMSVAEXGXR, KASKAXXVPTKL:EPLP-AA$^{17}$-VYY:EQLSN:GMAVSEXGXR, GSAGPXXTPTKM:EPLP-AA$^{17}$-VYY:EQLSN:GMV-VDRXGXS, AAPASXXVPARL:EPLP-AA$^{17}$-VYY:EQLSN:DMVVEAXGXR, STPPTXXVPTRL:EPLP-AA$^{17}$-VYY:EQL-SN:DMVVESXGXR, HVPKPXXAPTKL:EPLP-AA$^{17}$-VYY:EQLSN:NMVVRSXGXH, RVPSTXXAPVKT:EPLP-AA$^{17}$-VYY:EQLSN:MIVEEXGXL, ASASPXXVSQDL:EPLP-AA$^{17}$-VYY:EQLSN:MIVKSXKXS, ASASPXXVPQDL:EPLP-AA$^{17}$-VYY:EQLSN:MVVKSXKXS, NDEGLEXVPTEE:EPLP-AA$^{17}$-VYY:EQLSN:FLQHNKXEXR, NDEGLEXVPT-GQ:EPLP-AA$^{17}$-VYY:EQLSN:LEEHSQXEXR, SSVKXQPSRVHH:EPLP-AA$^{17}$-VYY:EQLSN:RLEEHLEXAXA, RNVQXRPTQVQL:EPLP-AA$^{17}$-VYY:EQLSN:TLEDHLAXKXE, KIPKASSVSQEL:EPLT-AA$^{17}$-LYY:EQLSN:DM-VKXGXGSR, GIPEPXXVSQKM:EPLT-AA$^{17}$-LYY:EQLSN:NMVKSSXAXR, SIPKASSVSQEL:EPLT-AA$^{17}$-LYY:EQL-SN:EMVKXGXGSR, HVTKPTXVSQKL:EPLT-AA$^{17}$-LYY:EQLSN:NMVKSSXGXH, YVPKPXXVSQKL:EPLT-AA$^{17}$-LYY:EQLSN:NMVKSSXGXH, TVPKPXXVSQQL:EPLT-AA$^{17}$-LYY:EQLSN:NMVKSAXGXH, AVPKASS-VSQKL:EPLT-AA$^{17}$-LYY:EQLSN:NMVKSAXGXH, KVGKASSVSQKL:EPLT-AA$^{17}$-LYY:EQLSN:EGMSVKSXGXR, KASKASSVSQKL:EPLT-AA$^{17}$-LYY:EQLSN:GMVKSEXGXR, GSAGPXXVSQKM:EPLT-AA$^{17}$-LYY:EQLSN:GMVK-SRXGXS, AAPASSSVSQRL:EPLT-AA$^{17}$-LYY:EQLSN:DMVKSAXGXR, STPPTSSVSQRL:EPLT-AA$^{17}$-LYY:EQL-SN:DMVKSSXGXR, HVPKPXXVSQKL:EPLT-AA$^{17}$-LYY:EQLSN:NMVKSSXGXH, RVPSTSSVSQKT:EPLT-AA$^{17}$-LYY:EQLSN:MIVKSXGXL, ASAAPXXVSQAL:EPLT-AA$^{17}$-LYY:EQLSN:MIVKSXKXS, ASASPXXVSQDL:EPLT-

AA$^{17}$-LYY:EQLSN:MVVKSXKXS, NDEGLEXVSQEE:EPLT-AA$^{17}$-LYY:EQLSN:FLVKXKXESR, NDEGLEXVSQGQ:EPLT-AA$^{17}$-LYY:EQLSN:LEVKSQXEXR, SSVKXQPVSQHH:EPLT-AA$^{17}$-LYY:EQLSN:RLVKSLEXAXA, RNVQXRPVSQQL:EPLT-AA$^{17}$-LYY:EQLSN:TLVKSLAXKXE, KIPKAXXVPTEL:EPLT-AA$^{17}$-LYY:EQLSN:DMVVEGXGXR, GIPEPXXVPEKM:EPLT-AA$^{17}$-LYY:EQLSN:NMTVESXAXR, SIPKAXXVPTEL:EPLT-AA$^{17}$-LYY:EQLSN:EMVVEGXGXR, HVTKPTXAPTKL:EPLT-AA$^{17}$-LYY:EQLSN:NMVVRSXGXH, YVPKPXXAPTKL:EPLT-AA$^{17}$-LYY:EQLSN:NMVVRSXGXH, TVPKPXXAPTQL:EPLT-AA$^{17}$-LYY:EQLSN:NMVVRAXGXH, AVPKAXXAPTKL:EPLT-AA$^{17}$-LYY:EQLSN:NMVVKAXGXH, KVGKAXXVPTKL:EPLT-AA$^{17}$-LYY:EQLSN:EGMSVAEXGXR, KASKAXXVPTKL:EPLT-AA$^{17}$-LYY:EQLSN:GMAVSEXGXR, GSAGPXXTPTKM:EPLT-AA$^{17}$-LYY:EQLSN:GMVVDRXGXS, AAPASXXVPARL:EPLT-AA$^{17}$-LYY:EQLSN:DMVVEAXGXR, STPPTXXVPTRL:EPLT-AA$^{17}$-LYY:EQLSN:DMVVESXGXR, HVPKPXXAPTKL:EPLT-AA$^{17}$-LYY:EQLSN:NMVVRSXGXH, RVPSTXXAPVKT:EPLT-AA$^{17}$-LYY:EQLSN:MIVEEXGXL, ASAAPXXVPQAL:EPLT-AA$^{17}$-LYY:EQLSN:MIVRSXKXS, ASASPXXVPQDL:EPLT-AA$^{17}$-LYY:EQLSN:MVVKSXKXS, NDEGLEXVPTEE:EPLT-AA$^{17}$-LYY:EQLSN:FLQHNKXEXR, NDEGLEXVPTGQ:EPLT-AA$^{17}$-LYY:EQLSN:LEEHSQXEXR, SSVKXQPSRVHH:EPLT-AA$^{17}$-LYY:EQLSN:RLEEHLEXAXA, RNVQXRPTQVQL:EPLT-AA$^{17}$-LYY:EQLSN:TLEDHLAXKXE, KIPKAXXVPQEL:EPLT-AA$^{17}$-LYY:EQLSN:DMVKXGXGSR, GIPEPXXVPQKM:EPLT-AA$^{17}$-LYY:EQLSN:NMVKSSXAXR, SIPKAXXVPQEL:EPLT-AA$^{17}$-LYY:EQLSN:EMVKXGXGSR, HVTKPTXVPQKL:EPLT-AA$^{17}$-LYY:EQLSN:NMVKSSXGXH, YVPKPXXVPQKL:EPLT-AA$^{17}$-LYY:EQLSN:NMVKSSXGXH, TVPKPXXVPQQL:EPLT-AA$^{17}$-LYY:EQLSN:NMVKSAXGXH, AVPKAXXVPQKL:EPLT-AA$^{17}$-LYY:EQLSN:NMVKSAXGXH, KVGKAXXVPQKL:EPLT-AA$^{17}$-LYY:EQLSN:EGMSVKSXGXR, KASKAXXVPQKL:EPLT-AA$^{17}$-LYY:EQLSN:GMVKSEXGXR, GSAGPXXVPQKM:EPLT-AA$^{17}$-LYY:EQLSN:GMVKSRXGXS, AAPASXXVPQRL:EPLT-AA$^{17}$-LYY:EQLSN:DMVKSAXGXR, STPPTXXVPQRL:EPLT-AA$^{17}$-LYY:EQLSN:DMVKSSXGXR, HVPKPXXVPQKL:EPLT-AA$^{17}$-LYY:EQLSN:NMVKSSXGXH, RVPSTXXVPQKT:EPLT-AA$^{17}$-LYY:EQLSN:MIVKSXGXL, ASAAPXXVPQAL:EPLT-AA$^{17}$-LYY:EQLSN:MIVKSXKXS, ASASPXXVPQDL:EPLT-AA$^{17}$-LYY:EQLSN:MIVKSXKXS, NDEGLEXVPQEE:EPLT-AA$^{17}$-LYY:EQLSN:FLVKXKXESR, NDEGLEXVPQGQ:EPLT-AA$^{17}$-LYY:EQLSN:LEVKSQXEXR, SSVKXQPVPQHH:EPLT-AA$^{17}$-LYY:EQLSN:RLVKSLEXAXA, RNVQXRPVPQQL:EPLT-AA$^{17}$-LYY:EQLSN:TLVKSLAXKXE, ASASPXXVSQDL:EPLT-AA$^{17}$-LYY:EQLSN:MIVKSXKXS, KIPKAXXVPTEL:SNIT-AA$^{17}$-QIM:IGEMS:DMQHNGXGXR, GIPEPXXVPTKM:SNIT-AA$^{17}$-QIM:IGEMS:NMQHNSXAXR, SIPKAXXVPTEL:SNIT-AA$^{17}$-QIM:IGEMS:EMQHNGXGXR, HVTKPTXVPTKL:SNIT-AA$^{17}$-QIM:IGEMS:NMQHNSXGXH, YVPKPXXVPTKL:SNIT-AA$^{17}$-QIM:IGEMS:NMQHNSXGXH, TVPKPXXVPTQL:SNIT-AA$^{17}$-QIM:IGEMS:NMQHNAXGXH, AVPKAXXVPTKL:SNIT-AA$^{17}$-QIM:IGEMS:NMQHNAXGXH, KVGKAXXVPTKL:SNIT-AA$^{17}$-QIM:IGEMS:EGMSQHNXGXR, KASKAXXVPTKL:SNIT-AA$^{17}$-QIM:IGEMS:GMQHNEXGXR, GSAGPXXVPTKM:SNIT-AA$^{17}$-QIM:IGEMS:GMQHNRXGXS, AAPASXXVPTRL:SNIT-AA$^{17}$-QIM:IGEMS:DMQHNAXGXR, STPPTXXVPTRL:SNIT-AA$^{17}$-QIM:IGEMS:DMQHNSXGXR, HVPKPXXVPTKL:SNIT-AA$^{17}$-QIM:IGEMS:NMQHNSXGXH, RVPSTXXVPTKT:SNIT-AA$^{17}$-QIM:IGEMS:MIQHNXGXL, ASAAPXXVPTAL:SNIT-AA$^{17}$-QIM:IGEMS:MIQHNXKXS, ASASPXXVPTDL:SNIT-AA$^{17}$-QIM:IGEMS:MIQHNXKXS, ASASPXXVPTDL:SNIT-AA$^{17}$-QIM:IGEMS:MVQHNXKXS, NDEGLEXVPTGQ:SNIT-AA$^{17}$-QIM:IGEMS:LEQHNQXEXR, NDEGLEXVPTEE:SNIT-AA$^{17}$-QIM:IGEMS:FLQHNKXEXR, SSVKXQPVPTHH:SNIT-AA$^{17}$-QIM:IGEMS:RLQHNLEXAXA, RNVQXRPVPTQL:SNIT-AA$^{17}$-QIM:IGEMS:TLQHNLAXKXE, KIPKAXXVPTEL:SNIT-AA$^{17}$-QIM:IGEMS:DMVVEGXGXR, GIPEPXXVPEKM:SNIT-AA$^{17}$-QIM:IGEMS:NMTVESXAXR, SIPKAXXVPTEL:SNIT-AA$^{17}$-QIM:IGEMS:EMVVEGXGXR, HVTKPTXAPTKL:SNIT-AA$^{17}$-QIM:IGEMS:NMVVRSXGXH, YVPKPXXAPTKL:SNIT-AA$^{17}$-QIM:IGEMS:NMVVRSXGXH, TVPKPXXAPTQL:SNIT-AA$^{17}$-QIM:IGEMS:NMVVRAXGXH, AVPKAXXAPTKL:SNIT-AA$^{17}$-QIM:IGEMS:NMVVKAXGXH, KVGKAXXVPTKL:SNIT-AA$^{17}$-QIM:IGEMS:EGMSVAEXGXR, KASKAXXVPTKL:SNIT-AA$^{17}$-QIM:IGEMS:GMAVSEXGXR, GSAGPXXTPTKM:SNIT-AA$^{17}$-QIM:IGEMS:GMVVDRXGXS, AAPASXXVPARL:SNIT-AA$^{17}$-QIM:IGEMS:DMVVEAXGXR, STPPTXXVPTRL:SNIT-AA$^{17}$-QIM:IGEMS:DMVVESXGXR, HVPKPXXAPTKL:SNIT-AA$^{17}$-QIM:IGEMS:NMVVRSXGXH, RVPSTXXAPVKT:SNIT-AA$^{17}$-QIM:IGEMS:MIVEEXGXL, ASAAPXXVPQAL:SNIT-AA$^{17}$-QIM:IGEMS:MIVRSXKXS, ASASPXXVSQDL:SNIT-AA$^{17}$-QIM:IGEMS:MIVKSXKXS, ASASPXXVPQDL:SNIT-AA$^{17}$-QIM:IGEMS:MVVKSXKXS, NDEGLEXVPTGQ:SNIT-AA$^{17}$-QIM:IGEMS:LEEHSQXEXR, SSVKXQPSRVHH:SNIT-AA$^{17}$-QIM:IGEMS:RLEEHLEXAXA, RNVQXRPTQVQL:SNIT-AA$^{17}$-QIM:IGEMS:TLEDHLAXKXE, KIPKAXXVPTEL:SNIT-AA$^{17}$-QIM:LGEMS:DMEHXGXGSR, GIPEPXXVPTKM:SNIT-AA$^{17}$-QIM:LGEMS:NMEHSSXAXR, SIPKAXXVPTEL:SNIT-AA$^{17}$-QIM:LGEMS:EMEHXGXGSR, HVTKPTXVPTKL:SNIT-AA$^{17}$-QIM:LGEMS:NMEHSSXGXH, YVPKPXXVPTKL:SNIT-AA$^{17}$-QIM:LGEMS:NMEHSSXGXH, TVPKPXXVPTQL:SNIT-AA$^{17}$-QIM:LGEMS:NMEHSAXGXH, AVPKAXXVPTKL:SNIT-AA$^{17}$-QIM:LGEMS:NMEHSAXGXH, KVGKAXXVPTKL:SNIT-AA$^{17}$-QIM:LGEMS:EGMSEHSXGXR, KASKAXXVPTKL:SNIT-AA$^{17}$-QIM:LGEMS:GMEHSEXGXR, GSAGPXXVPTKM:SNIT-AA$^{17}$-QIM:LGEMS:GMEHSRXGXS, AAPASXXVPTRL:SNIT-AA$^{17}$-QIM:LGEMS:DMEHSAXGXR, STPPTXXVPTRL:SNIT-AA$^{17}$-QIM:LGEMS:DMEHSSXGXR, HVPKPXXVPTKL:SNIT-AA$^{17}$-QIM:LGEMS:NMEHSSXGXH, RVPSTXXVPTKT:SNIT-AA$^{17}$-QIM:LGEMS:MIEHSXGXL, ASAAPXXVPTAL:SNIT-AA$^{17}$-QIM:LGEMS:MIEHSXKXS, ASASPXXVPTDL:SNIT-AA$^{17}$-QIM:LGEMS:MIEHSXKXS, ASASPXXVPTDL:SNIT-AA$^{17}$-QIM:LGEMS:MVEHSXKXS, NDE-

GLEXVPTEE:SNIT-AA$^{17}$-QIM:LGEMS:FLEHXKXESR, SSVKXQPVPTHH:SNIT-AA$^{17}$-QIM:LGEMS:RLEHSLEXAXA, RNVQXRPVPTQL:SNIT-AA$^{17}$-QIM:LGEMS:TLEHSLAXKXE, KIPKAXXVPTEL:SNIT-AA$^{17}$-QIM:LGEMS:DMVVEGXGXR, GIPEPXXVPEKM:SNIT-AA$^{17}$-QIM:LGEMS:NMTVESXAXR, SIPKAXXVPTEL:SNIT-AA$^{17}$-QIM:LGEMS:EMVVEGXGXR, HVTKPTXAPTKL:SNIT-AA$^{17}$-QIM:LGEMS:NMVVRSXGXH, YVPKPXXAPTKL:SNIT-AA$^{17}$-QIM:LGEMS:NMVVRSXGXH, TVPKPXXAPTQL:SNIT-AA$^{17}$-QIM:LGEMS:NMVVRAXGXH, AVPKAXXAPTKL:SNIT-AA$^{17}$-QIM:LGEMS:NMVVKAXGXH, KVGKAXXVPTKL:SNIT-AA$^{17}$-QIM:LGEMS:EGMSVAEXGXR, KASKAXXVPTKL:SNIT-AA$^{17}$-QIM:LGEMS:GMAVSEXGXR, GSAGPXXTPTKM:SNIT-AA$^{17}$-QIM:LGEMS:GMVVDRXGXS, AAPASXXVPARL:SNIT-AA$^{17}$-QIM:LGEMS:DMVVEAXGXR, STPPTXXVPTRL:SNIT-AA$^{17}$-QIM:LGEMS:DMVVESXGXR, HVPKPXXAPTKL:SNIT-AA$^{17}$-QIM:LGEMS:NMVVRSXGXH, RVPSTXXAPVKT:SNIT-AA$^{17}$-QIM:LGEMS:MIVEEXGXL, ASAAPXXVPQAL:SNIT-AA$^{17}$-QIM:LGEMS:MIVRSXKXS, ASASPXXVSQDL:SNIT-AA$^{17}$-QIM:LGEMS:MIVKSXKXS, ASASPXXVPQDL:SNIT-AA$^{17}$-QIM:LGEMS:MVVKSXKXS, NDEGLEXVPTEE:SNIT-AA$^{17}$-QIM:LGEMS:FLQHNKXEXR, SSVKXQPSRVHH:SNIT-AA$^{17}$-QIM:LGEMS:RLEEHLEXAXA, RNVQXRPTQVQL:SNIT-AA$^{17}$-QIM:LGEMS:TLEDHLAXKXE, KIPKASSSRVEL:RSVK-AA$^{17}$-AKV:KEVQV:DMEEHGXGXR, GIPEPXXSRVKM:RSVK-AA$^{17}$-AKV:KEVQV:NMEEHSXAXR, SIPKASSSRVEL:RSVK-AA$^{17}$-AKV:KEVQV:EMEEHGXGXR, HVTKPTSSRVKLRSVK-AA$^{17}$-AKV:KEVQV:NMEEHSXGXH, YVPKPXXSRVKL:RSVK-AA$^{17}$-AKV:KEVQV:NMEEHSXGXH, TVPKPXXSRVQL:RSVK-AA$^{17}$-AKV:KEVQV:NMEEHAXGXH, AVPKASSSRVKL:RSVK-AA$^{17}$-AKV:KEVQV:NMEEHAXGXH, KVGKASSSRVKL:RSVK-AA$^{17}$-AKV:KEVQV:EGMSEEHXGXR, KASKASSSRVKL:RSVK-AA$^{17}$-AKV:KEVQV:GMEEHEXGXR, GSAGPXXSRVKM:RSVK-AA$^{17}$-AKV:KEVQV:GMEEHRXGXS, AAPASSSSRVRL:RSVK-AA$^{17}$-AKV:KEVQV:DMEEHAXGXR, STPPTSSSRVRL:RSVK-AA$^{17}$-AKV:KEVQV:DMEEHSXGXR, HVPKPXXSRVKL:RSVK-AA$^{17}$-AKV:KEVQV:NMEEHSXGXH, RVPSTSSSRVKT:RSVK-AA$^{17}$-AKV:KEVQV:MIEEHXGXL, ASAAPXXSRVAL:RSVK-AA$^{17}$-AKV:KEVQV:MIEEHXKXS, ASASPXXSRVDL:RSVK-AA$^{17}$-AKV:KEVQV:MIEEHXKXS, ASASPXXSRVDL:RSVK-AA$^{17}$-AKV:KEVQV:MVEEHXKXS, NDEGLESSRVEE:RSVK-AA$^{17}$-AKV:KEVQV:FLEEHKXEXR, NDEGLESSRVGQ:RSVK-AA$^{17}$-AKV:KEVQV:LEEEHQXEXR, RNVQXRPSRVQL:RSVK-AA$^{17}$-AKV:KEVQV:TLEEHLAXKXE, KIPKAXXVPTEL:RSVK-AA$^{17}$-AKV:KEVQV:DMVVEGXGXR, GIPEPXXVPEKM:RSVK-AA$^{17}$-AKV:KEVQV:NMTVESXAXR, SIPKAXXVPTEL:RSVK-AA$^{17}$-AKV:KEVQV:EMVVEGXGXR, HVTKPTXAPTKL:RSVK-AA$^{17}$-AKV:KEVQV:NMVVRSXGXH, YVPKPXXAPTKLRSVK-AA$^{17}$-AKV:KEVQV:NMVVRSXGXH, TVPKPXXAPTQL:RSVK-AA$^{17}$-AKV:KEVQV:NMVVRAXGXH, AVPKAXXAPTKL:RSVK-AA$^{17}$-AKV:KEVQV:NMVVKAXGXH, KVGKAXXVPTKL:RSVK-AA$^{17}$-AKV:KEVQV:EGMSVAEXGXR, KASKAXXVPTKL:RSVK-AA$^{17}$-AKV:KEVQV:GMAVSEXGXR, GSAGPXXTPTKM:RSVK-AA$^{17}$-AKV:KEVQV:GMVVDRXGXS, AAPASXXVPARL:RSVK-AA$^{17}$-AKV:KEVQV:DMVVEAXGXR, STPPTXXVPTRL:RSVK-AA$^{17}$-AKV:KEVQV:DMVVESXGXR, HVPKPXXAPTKL:RSVK-AA$^{17}$-AKV:KEVQV:NMVVRSXGXH, RVPSTXXAPVKT:RSVK-AA$^{17}$-AKV:KEVQV:MIVEEXGXL, ASAAPXXVPQAL:RSVK-AA$^{17}$-AKV:KEVQV:MIVRSXKXS, ASASPXXVSQDL:RSVK-AA$^{17}$-AKV:KEVQV:MIVKSXKXS, ASASPXXVPQDL:RSVK-AA$^{17}$-AKV:KEVQV:MVVKSXKXS, NDEGLEXVPTEE:RSVK-AA$^{17}$-AKV:KEVQV:FLQHNKXEXR, NDEGLEXVPTGQ:RSVK-AA$^{17}$-AKV:KEVQV:LEEHSQXEXR, RNVQXRPTQVQL:RSVK-AA$^{17}$-AKV:KEVQV:TLEDHLAXKXE, KIPKASSTQVEL:RPVQ-AA$^{17}$-RKI:KKATV:DMEDHGXGXR, GIPEPXXTQVKM:RPVQ-AA$^{17}$-RKI:KKATV:NMEDHSXAXR, SIPKASSTQVEL:RPVQ-AA$^{17}$-RKI:KKATV:EMEDHGXGXR, HVTKPTSTQVKL:RPVQ-AA$^{17}$-RKI:KKATV:NMEDHSXGXH, YVPKPXXTQVKL:RPVQ-AA$^{17}$-RKI:KKATV:NMEDHSXGXH, TVPKPXXTQVQL:RPVQ-AA$^{17}$-RKI:KKATV:NMEDHAXGXH, AVPKASSTQVKL:RPVQ-AA$^{17}$-RKI:KKATV:NMEDHAXGXH, KVGKASSTQVKL:RPVQ-AA$^{17}$-RKI:KKATV:EGMSEDHXGXR, KASKASSTQVKL:RPVQ-AA$^{17}$-RKI:KKATV:GMEDHEXGXR, GSAGPXXTQVKM:RPVQ-AA$^{17}$-RKI:KKATV:GMEDHRXGXS, AAPASSSTQVRL:RPVQ-AA$^{17}$-RKI:KKATV:DMEDHAXGXR, STPPTSSTQVRL:RPVQ-AA$^{17}$-RKI:KKATV:DMEDHSXGXR, HVPKPXXTQVKL:RPVQ-AA$^{17}$-RKI:KKATV:NMEDHSXGXH, RVPSTSSTQVKT:RPVQ-AA$^{17}$-RKI:KKATV:MIEDHXGXL, ASAAPXXTQVAL:RPVQ-AA$^{17}$-RKI:KKATV:MIEDHXKXS, ASASPXXTQVDL:RPVQ-AA$^{17}$-RKI:KKATV:MIEDHXKXS, ASASPXXTQVDL:RPVQ-AA$^{17}$-RKI:KKATV:MVEDHXKXS, NDEGLESTQVEE:RPVQ-AA$^{17}$-RKI:KKATV:FLEDHKXEXR, NDEGLESTQVGQ:RPVQ-AA$^{17}$-RKI:KKATV:LEEDHQXEXR, SSVKXQPTQVHH:RPVQ-AA$^{17}$-RKI:KKATV:RLEDHLEXAXA, KIPKAXXVPTEL:RPVQ-AA$^{17}$-RKI:KKATV:DMVVEGXGXR, GIPEPXXVPEKM:RPVQ-AA$^{17}$-RKI:KKATV:NMTVESXAXR, SIPKAXXVPTEL:RPVQ-AA$^{17}$-RKI:KKATV:EMVVEGXGXR, HVTKPTXAPTKL:RPVQ-AA$^{17}$-RKI:KKATV:NMVVRSXGXH, YVPKPXXAPTKL:RPVQ-AA$^{17}$-RKI:KKATV:NMVVRSXGXH, TVPKPXXAPTQL:RPVQ-AA$^{17}$-RKI:KKATV:NMVVRAXGXH, AVPKAXXAPTKL:RPVQ-AA$^{17}$-RKI:KKATV:NMVVKAXGXH, KVGKAXXVPTKL:RPVQ-AA$^{17}$-RKI:KKATV:EGMSVAEXGXR, KASKAXXVPTKL:RPVQ-AA$^{17}$-RKI:KKATV:GMAVSEXGXR, GSAGPXXTPTKM:RPVQ-AA$^{17}$-RKI:KKATV:GMVVDRXGXS, AAPASXXVPARL:RPVQ-AA$^{17}$-RKI:KKATV:DMVVEAXGXR, STPPTXXVPTRL:RPVQ-AA$^{17}$-RKI:KKATV:DMVVESXGXR, HVPKPXXAPTKL:RPVQ-AA$^{17}$-RKI:KKATV:NMVVRSXGXH, RVPSTXXAPVKT:RPVQ-AA$^{17}$-RKI:KKATV:MIVEEXGXL, ASAAPXXVPQAL:RPVQ-AA$^{17}$-RKI:KKATV:MIVRSXKXS, ASASPXXVSQDL:RPVQ-AA$^{17}$-RKI:KKATV:MIVKSXKXS, ASASPXXVPQDL:RPVQ-AA$^{17}$-RKI:KKATV:MVVKSXKXS, NDEGLEXVPTEE:RPVQ-AA$^{17}$-RKI:KKATV:FLQHNKXEXR, NDEGLEXVPTGQ:RPVQ-AA$^{17}$-RKI:KKATV:LEEHSQXEXR and SSVKXQPSRVHH:RPVQ-

AA$^{17}$-RKI:KKATV:RLEEHLEXAXA; and wherein AA$^{17}$ is selected from the group consisting of G, A, V, L, I, P, F, M, W, T and S (in particular is selected from the group consisting of M, I, L, V and T). More particularly, the quadruplet PEP9:PEP12:PEP2:PEP10 is selected from the group consisting of GIPEPXXVPTKM:SAIS-AA$^{17}$-LYL:LKNYQ:NMVVESXAXR, HVTKPTXVPTKL:SAIS-AA$^{17}$-LYL:LKNYQ:NMVVESXGXH, YVPKPXXVPTKL:SAIS-AA$^{17}$-LYL:LKNYQ:NMVVESXGXH, TVPKPXXVPTQL:SAIS-AA$^{17}$-LYL:LKNYQ:NMVVEAXGXH, AVPKAXXVPTKL:SAIS-AA$^{17}$-LYL:LKNYQ:NMVVEAXGXH, KVGKAXXVPTKL:SAIS-AA$^{17}$-LYL:LKNYQ:EGMSVVEXGXR, KASKAXXVPTKL:SAIS-AA$^{17}$-LYL:LKNYQ:GMVVEEXGXR, GSAGPXXVPTKM:SAIS-AA$^{17}$-LYL:LKNYQ:GMVVERXGXS, AAPASXXVPTRL:SAIS-AA$^{17}$-LYL:LKNYQ:DMVVEAXGXR, STPPTXXVPTRL:SAIS-AA$^{17}$-LYL:LKNYQ:DMVVESXGXR, HVPKPXXVPTKL:SAIS-AA$^{17}$-LYL:LKNYQ:NMVVESXGXH, RVPSTXXVPTKT:SAIS-AA$^{17}$-LYL:LKNYQ:MIVVEXGXL, ASAAPXXVPTAL:SAIS-AA$^{17}$-LYL:LKNYQ:MIVVEXKXS, ASASPXXVPTDL:SAIS-AA$^{17}$-LYL:LKNYQ:MIVVEXKXS, ASASPXXVPTDL:SAIS-AA$^{17}$-LYL:LKNYQ:MVVVEXKXS, GIPEPXXVPEKM:SAIS-AA$^{17}$-LYL:LKNYQ:NMTVESXAXR, HVTKPTXAPTKL:SAIS-AA$^{17}$-LYL:LKNYQ:NMVVRSXGXH, YVPKPXXAPTKL:SAIS-AA$^{17}$-LYL:LKNYQ:NMVVRSXGXH, TVPKPXXAPTQL:SAIS-AA$^{17}$-LYL:LKNYQ:NMVVRAXGXH, AVPKAXXAPTKL:SAIS-AA$^{17}$-LYL:LKNYQ:NMVVKAXGXH, KVGKAXXVPTKL:SAIS-AA$^{17}$-LYL:LKNYQ:EGMSVAEXGXR, KASKAXXVPTKL:SAIS-AA$^{17}$-LYL:LKNYQ:GMAVSEXGXR, GSAGPXXTPTKM:SAIS-AA$^{17}$-LYL:LKNYQ:GMVVDRXGXS, AAPASXXVPARL:SAIS-AA$^{17}$-LYL:LKNYQ:DMVVEAXGXR, HVPKPXXAPTKL:SAIS-AA$^{17}$-LYL:LKNYQ:NMVVRSXGXH, RVPSTXXAPVKT:SAIS-AA$^{17}$-LYL:LKNYQ:MIVEEXGXL, ASAAPXXVPQAL:SAIS-AA$^{17}$-LYL:LKNYQ:MIVRSXKXS, ASASPXXVSQDL:SAIS-AA$^{17}$-LYL:LKNYQ:MIVKSXKXS, ASASPXXVPQDL:SAIS-AA$^{17}$-LYL:LKNYQ:MVVKSXKXS, NDEGLEXVPTEE:SAIS-AA$^{17}$-LYL:LKNYQ:FLQHNKXEXR, NDEGLEXVPTGQ:SAIS-AA$^{17}$-LYL:LKNYQ:LEEHSQXEXR, SSVKXQPSRVHH:SAIS-AA$^{17}$-LYL:LKNYQ:RLEEHLEXAXA, RNVQXRPTQVQL:SAIS-AA$^{17}$-LYL:LKNYQ:TLEDHLAXKXE, KIPKAXXVPEEL:SSLS-AA$^{17}$-LFF:LKVYP:DMTVEGXGXR, SIPKAXXVPEEL:SSLS-AA$^{17}$-LFF:LKVYP:EMTVEGXGXR, HVTKPTXVPEKL:SSLS-AA$^{17}$-LFF:LKVYP:NMTVESXGXH, YVPKPXXVPEKL:SSLS-AA$^{17}$-LFF:LKVYP:NMTVESXGXH, TVPKPXXVPEQL:SSLS-AA$^{17}$-LFF:LKVYP:NMTVEAXGXH, AVPKAXXVPEKL:SSLS-AA$^{17}$-LFF:LKVYP:NMTVEAXGXH, KVGKAXXVPEKL:SSLS-AA$^{17}$-LFF:LKVYP:EGMSTVEXGXR, KASKAXXVPEKL:SSLS-AA$^{17}$-LFF:LKVYP:GMTVEEXGXR, GSAGPXXVPEKM:SSLS-AA$^{17}$-LFF:LKVYP:GMTVERXGXS, AAPASXXVPERL:SSLS-AA$^{17}$-LFF:LKVYP:DMTVEAXGXR, STPPTXXVPERL:SSLS-AA$^{17}$-LFF:LKVYP:DMTVESXGXR, HVPKPXXVPEKL:SSLS-AA$^{17}$-LFF:LKVYP:NMTVESXGXH, RVPSTXXVPEKT:SSLS-AA$^{17}$-LFF:LKVYP:MITVEXGXL, ASAAPXXVPEAL:SSLS-AA$^{17}$-LFF:LKVYP:MITVEXKXS, ASASPXXVPEDL:SSLS-AA$^{17}$-LFF:LKVYP:MITVEXKXS, ASASPXXVPEDL:SSLS-AA$^{17}$-LFF:LKVYP:MVTVEXKXS, KIPKAXXVPTEL:SSLS-AA$^{17}$-LFF:LKVYP:DMVVEGXGXR, SIPKAXXVPTEL:SSLS-AA$^{17}$-LFF:LKVYP:EMVVEGXGXR, HVTKPTXAPTKL:SSLS-AA$^{17}$-LFF:LKVYP:NMVVRSXGXH, YVPKPXXAPTKL:SSLS-AA$^{17}$-LFF:LKVYP:NMVVRSXGXH, TVPKPXXAPTQL:SSLS-AA$^{17}$-LFF:LKVYP:NMVVRAXGXH, AVPKAXXAPTKL:SSLS-AA$^{17}$-LFF:LKVYP:NMVVKAXGXH, KVGKAXXVPTKL:SSLS-AA$^{17}$-LFF:LKVYP:EGMSVAEXGXR, KASKAXXVPTKL:SSLS-AA$^{17}$-LFF:LKVYP:GMAVSEXGXR, GSAGPXXTPTKM:SSLS-AA$^{17}$-LFF:LKVYP:GMVVDRXGXS, AAPASXXVPARL:SSLS-AA$^{17}$-LFF:LKVYP:DMVVEAXGXR, STPPTXXVPTRL:SSLS-AA$^{17}$-LFF:LKVYP:DMVVESXGXR, HVPKPXXAPTKL:SSLS-AA$^{17}$-LFF:LKVYP:NMVVRSXGXH, RVPSTXXAPVKT:SSLS-AA$^{17}$-LFF:LKVYP:MIVEEXGXL, ASAAPXXVPQAL:SSLS-AA$^{17}$-LFF:LKVYP:MIVRSXKXS, ASASPXXVSQDL:SSLS-AA$^{17}$-LFF:LKVYP:MIVKSXKXS, ASASPXXVPQDL:SSLS-AA$^{17}$-LFF:LKVYP:MVVKSXKXS, NDEGLEXVPTEE:SSLS-AA$^{17}$-LFF:LKVYP:FLQHNKXEXR, NDEGLEXVPTGQ:SSLS-AA$^{17}$-LFF:LKVYP:LEEHSQXEXR, SSVKXQPSRVHH:SSLS-AA$^{17}$-LFF:LKVYP:RLEEHLEXAXA, RNVQXRPTQVQL:SSLS-AA$^{17}$-LFF:LKVYP:TLEDHLAXKXE, KIPKAXXAPTEL:NAIS-AA$^{17}$-LYF:LKKYR:DMVVRGXGXR, GIPEPXXAPTKM:NAIS-AA$^{17}$-LYF:LKKYR:NMVVRSXAXR, SIPKAXXAPTEL:NAIS-AA$^{17}$-LYF:LKKYR:EMVVRGXGXR, AVPKAXXAPTKL:NAIS-AA$^{17}$-LYF:LKKYR:NMVVRAXGXH, KVGKAXXAPTKL:NAIS-AA$^{17}$-LYF:LKKYR:EGMSVVRXGXR, KASKAXXAPTKL:NAIS-AA$^{17}$-LYF:LKKYR:GMVVREXGXR, GSAGPXXAPTKM:NAIS-AA$^{17}$-LYF:LKKYR:GMVVRRXGXS, AAPASXXAPTRL:NAIS-AA$^{17}$-LYF:LKKYR:DMVVRAXGXR, STPPTXXAPTRL:NAIS-AA$^{17}$-LYF:LKKYR:DMVVRSXGXR, RVPSTXXAPTKT:NAIS-AA$^{17}$-LYF:LKKYR:MIVVRXGXL, ASAAPXXAPTAL:NAIS-AA$^{17}$-LYF:LKKYR:MIVVRXKXS, ASASPXXAPTDL:NAIS-AA$^{17}$-LYF:LKKYR:MIVVRXKXS, ASASPXXAPTDL:NAIS-AA$^{17}$-LYF:LKKYR:MVVVRXKXS, KIPKAXXVPTEL:NAIS-AA$^{17}$-LYF:LKKYR:DMVVEGXGXR, GIPEPXXVPEKM:NAIS-AA$^{17}$-LYF:LKKYR:NMTVESXAXR, SIPKAXXVPTEL:NAIS-AA$^{17}$-LYF:LKKYR:EMVVEGXGXR, AVPKAXXAPTKL:NAIS-AA$^{17}$-LYF:LKKYR:NMVVKAXGXH, KVGKAXXVPTKL:NAIS-AA$^{17}$-LYF:LKKYR:EGMSVAEXGXR, KASKAXXVPTKL:NAIS-AA$^{17}$-LYF:LKKYR:GMAVSEXGXR, GSAGPXXTPTKM:NAIS-AA$^{17}$-LYF:LKKYR:GMVVDRXGXS, AAPASXXVPARL:NAIS-AA$^{17}$-LYF:LKKYR:DMVVEAXGXR, STPPTXXVPTRL:NAIS-AA$^{17}$-LYF:LKKYR:DMVVESXGXR, RVPSTXXAPVKT:NAIS-AA$^{17}$-LYF:LKKYR:MIVEEXGXL, ASAAPXXVPQAL:NAIS-AA$^{17}$-LYF:LKKYR:MIVRSXKXS, ASASPXXVSQDL:NAIS-AA$^{17}$-LYF:LKKYR:MIVKSXKXS, ASASPXXVPQDL:NAIS-AA$^{17}$-LYF:LKKYR:MVVKSXKXS, NDEGLEXVPTEE:NAIS-AA$^{17}$-LYF:LKKYR:FLQHNKXEXR, NDEGLEXVPTGQ:NAIS-AA$^{17}$-LYF:LKKYR:LEEHSQXEXR, SSVKXQPSRVHH:NAIS-AA$^{17}$-LYF:LKKYR:RLEEHLEXAXA, RNVQXRPTQVQL:NAIS-AA$^{17}$-LYF:LKKYR:TLEDHLAXKXE, KIPKAXXAPTEL:SATS-AA$^{17}$-LYY:LRKHR:DMVVKGXGXR, GIPEPXXAPTKM:SATS-AA$^{17}$-LYY:LRKHR:NMVVKSXAXR,

SIPKAXXAPTEL:SATS-AA$^{17}$-LYY:LRKHR:EMVVKGXGXR, HVTKPTXAPTKL:SATS-AA$^{17}$-LYY:LRKHR:NMVVKSXGXH, YVPKPXXAPTKL:SATS-AA$^{17}$-LYY:LRKHR:NMVVKSXGXH, TVPKPXXAPTQL:SATS-AA$^{17}$-LYY:LRKHR:NMVVKAXGXH, KVGKAXXAPTKL:SATS-AA$^{17}$-LYY:LRKHR:EGMSVVKXGXR, KASKAXXAPTKL:SATS-AA$^{17}$-LYY:LRKHR:GMVVKEXGXR, GSAGPXXAPTKM:SATS-AA$^{17}$-LYY:LRKHR:GMVVKRXGXS, AAPASXXAPTRL:SATS-AA$^{17}$-LYY:LRKHR:DMVVKAXGXR, STPPTXXAPTRL:SATS-AA$^{17}$-LYY:LRKHR:DMVVKSXGXR, HVPKPXXAPTKL:SATS-AA$^{17}$-LYY:LRKHR:NMVVKSXGXH, RVPSTXXAPTKT:SATS-AA$^{17}$-LYY:LRKHR:MIVVKXGXL, ASAAPXXAPTAL:SATS-AA$^{17}$-LYY:LRKHR:MIVVKXKXS, ASASPXXAPTDL:SATS-AA$^{17}$-LYY:LRKHR:MIVVKXKXS, ASASPXXAPTDL:SATS-AA$^{17}$-LYY:LRKHR:MVVVKXKXS, KIPKAXXVPTEL:SATS-AA$^{17}$-LYY:LRKHR:DMVVEGXGXR, GIPEPXXVPEKM:SATS-AA$^{17}$-LYY:LRKHR:NMTVESXAXR, SIPKAXXVPTEL:SATS-AA$^{17}$-LYY:LRKHR:EMVVEGXGXR, HVTKPTXAPTKL:SATS-AA$^{17}$-LYY:LRKHR:NMVVRSXGXH, YVPKPXXAPTKL:SATS-AA$^{17}$-LYY:LRKHR:NMVVRSXGXH, TVPKPXXAPTQL:SATS-AA$^{17}$-LYY:LRKHR:NMVVRAXGXH, KVGKAXXVPTKL:SATS-AA$^{17}$-LYY:LRKHR:EGMSVAEXGXR, KASKAXXVPTKL:SATS-AA$^{17}$-LYY:LRKHR:GMAVSEXGXR, GSAGPXXTPTKM:SATS-AA$^{17}$-LYY:LRKHR:GMVVDRXGXS, AAPASXXVPARL:SATS-AA$^{17}$-LYY:LRKHR:DMVVEAXGXR, STPPTXXVPTRL:SATS-AA$^{17}$-LYY:LRKHR:DMVVESXGXR, HVPKPXXAPTKL:SATS-AA$^{17}$-LYY:LRKHR:NMVVRSXGXH, RVPSTXXAPVKT:SATS-AA$^{17}$-LYY:LRKHR:MIVEEXGXL, ASAAPXXVPQAL:SATS-AA$^{17}$-LYY:LRKHR:MIVRSXKXS, ASASPXXVSQDL:SATS-AA$^{17}$-LYY:LRKHR:MIVKSXKXS, ASASPXXVPQDL:SATS-AA$^{17}$-LYY:LRKHR:MVVKSXKXS, NDEGLEXVPTEE:SATS-AA$^{17}$-LYY:LRKHR:FLQHNKXEXR, NDEGLEXVPTGQ:SATS-AA$^{17}$-LYY:LRKHR:LEEHSQXEXR, SSVKXQPSRVHH:SATS-AA$^{17}$-LYY:LRKHR:RLEEHLEXAXA, RNVQXRPTQVQL:SATS-AA$^{17}$-LYY:LRKHR:TLEDHLAXKXE, KIPKAXXVPTEL:SPIS-AA$^{17}$-LYK:LKYHY:DMVAEGXGXR, GIPEPXXVPTKM:SPIS-AA$^{17}$-LYK:LKYHY:NMVAESXAXR, SIPKAXXVPTEL:SPIS-AA$^{17}$-LYK:LKYHY:EMVAEGXGXR, HVTKPTXVPTKL:SPIS-AA$^{17}$-LYK:LKYHY:NMVAESXGXH, YVPKPXXVPTKL:SPIS-AA$^{17}$-LYK:LKYHY:NMVAESXGXH, TVPKPXXVPTQL:SPIS-AA$^{17}$-LYK:LKYHY:NMVAEAXGXH, AVPKAXXVPTKL:SPIS-AA$^{17}$-LYK:LKYHY:NMVAEAXGXH, KASKAXXVPTKL:SPIS-AA$^{17}$-LYK:LKYHY:GMVAEEXGXR, GSAGPXXVPTKM:SPIS-AA$^{17}$-LYK:LKYHY:GMVAERXGXS, AAPASXXVPTRL:SPIS-AA$^{17}$-LYK:LKYHY:DMVAEAXGXR, STPPTXXVPTRL:SPIS-AA$^{17}$-LYK:LKYHY:DMVAESXGXR, HVPKPXXVPTKL:SPIS-AA$^{17}$-LYK:LKYHY:NMVAESXGXH, RVPSTXXVPTKT:SPIS-AA$^{17}$-LYK:LKYHY:MIVAEXGXL, ASAAPXXVPTAL:SPIS-AA$^{17}$-LYK:LKYHY:MIVAEXKXS, ASASPXXVPTDL:SPIS-AA$^{17}$-LYK:LKYHY:MIVAEXKXS, ASASPXXVPTDL:SPIS-AA$^{17}$-LYK:LKYHY:MVVAEXKXS, KIPKAXXVPTEL:SPIS-AA$^{17}$-LYK:LKYHY:DMVVEGXGXR, GIPEPXXVPEKM:SPIS-AA$^{17}$-LYK:LKYHY:NMTVESXAXR, SIPKAXXVPTEL:SPIS-AA$^{17}$-LYK:LKYHY:EMVVEGXGXR, HVTKPTXAPTKL:SPIS-AA$^{17}$-LYK:LKYHY:NMVVRSXGXH, YVPKPXXAPTKL:SPIS-AA$^{17}$-LYK:LKYHY:NMVVRSXGXH, TVPKPXXAPTQL:SPIS-AA$^{17}$-LYK:LKYHY:NMVVRAXGXH, AVPKAXXAPTKL:SPIS-AA$^{17}$-LYK:LKYHY:NMVVKAXGXH, KASKAXXVPTKL:SPIS-AA$^{17}$-LYK:LKYHY:GMAVSEXGXR, GSAGPXXTPTKM:SPIS-AA$^{17}$-LYK:LKYHY:GMVVDRXGXS, AAPASXXVPARL:SPIS-AA$^{17}$-LYK:LKYHY:DMVVEAXGXR, STPPTXXVPTRL:SPIS-AA$^{17}$-LYK:LKYHY:DMVVESXGXR, HVPKPXXAPTKL:SPIS-AA$^{17}$-LYK:LKYHY:NMVVRSXGXH, RVPSTXXAPVKT:SPIS-AA$^{17}$-LYK:LKYHY:MIVEEXGXL, ASAAPXXVPQAL:SPIS-AA$^{17}$-LYK:LKYHY:MIVRSXKXS, ASASPXXVSQDL:SPIS-AA$^{17}$-LYK:LKYHY:MIVKSXKXS, ASASPXXVPQDL:SPIS-AA$^{17}$-LYK:LKYHY:MVVKSXKXS, NDEGLEXVPTEE:SPIS-AA$^{17}$-LYK:LKYHY:FLQHNKXEXR, NDEGLEXVPTGQ:SPIS-AA$^{17}$-LYK:LKYHY:LEEHSQXEXR, SSVKXQPSRVHH:SPIS-AA$^{17}$-LYK:LKYHY:RLEEHLEXAXA, RNVQXRPTQVQL:SPIS-AA$^{17}$-LYK:LKYHY:TLEDHLAXKXE, KIPKAXXVPTEL:EPIS-AA$^{17}$-LYL:KFKYE:DMAVXGXGSR, GIPEPXXVPTKM:EPIS-AA$^{17}$-LYL:KFKYE:NMAVSSXAXR, SIPKAXXVPTEL:EPIS-AA$^{17}$-LYL:KFKYE:EMAVXGXGSR, HVTKPTXVPTKL:EPIS-AA$^{17}$-LYL:KFKYE:NMAVSSXGXH, YVPKPXXVPTKL:EPIS-AA$^{17}$-LYL:KFKYE:NMAVSSXGXH, TVPKPXXVPTQL:EPIS-AA$^{17}$-LYL:KFKYE:NMAVSAXGXH, AVPKAXXVPTKL:EPIS-AA$^{17}$-LYL:KFKYE:NMAVSAXGXH, KVGKAXXVPTKL:EPIS-AA$^{17}$-LYL:KFKYE:EGMSAVSXGXR, GSAGPXXVPTKM:EPIS-AA$^{17}$-LYL:KFKYE:GMAVSRXGXS, AAPASXXVPTRL:EPIS-AA$^{17}$-LYL:KFKYE:DMAVSAXGXR, STPPTXXVPTRL:EPIS-AA17-LYL:KFKYE:DMAVSSXGXR, HVPKPXXVPTKL:EPIS-AA$^{17}$-LYL:KFKYE:NMAVSSXGXH, RVPSTXXVPTKT:EPIS-AA$^{17}$-LYL:KFKYE:MIAVSXGXL, ASAAPXXVPTAL:EPIS-AA$^{17}$-LYL:KFKYE:MIAVSXKXS, ASASPXXVPTDL:EPIS-AA$^{17}$-LYL:KFKYE:MIAVSXKXS, ASASPXXVPTDL:EPIS-AA$^{17}$-LYL:KFKYE:MVAVSXKXS, KIPKAXXVPTEL:EPIS-AA$^{17}$-LYL:KFKYE:DMVVEGXGXR, GIPEPXXVPEKM:EPIS-AA$^{17}$-LYL:KFKYE:NMTVESXAXR, SIPKAXXVPTEL:EPIS-AA$^{17}$-LYL:KFKYE:EMVVEGXGXR, HVTKPTXAPTKL:EPIS-AA$^{17}$-LYL:KFKYE:NMVVRSXGXH, YVPKPXXAPTKL:EPIS-AA$^{17}$-LYL:KFKYE:NMVVRSXGXH, TVPKPXXAPTQL:EPIS-AA$^{17}$-LYL:KFKYE:NMVVRAXGXH, AVPKAXXAPTKL:EPIS-AA$^{17}$-LYL:KFKYE:NMVVKAXGXH, KVGKAXXVPTKL:EPIS-AA$^{17}$-LYL:KFKYE:EGMSVAEXGXR, GSAGPXXTPTKM:EPIS-AA$^{17}$-LYL:KFKYE:GMVVDRXGXS, AAPASXXVPARL:EPIS-AA$^{17}$-LYL:KFKYE:DMVVEAXGXR, STPPTXXVPTRL:EPIS-AA$^{17}$-LYL:KFKYE:DMVVESXGXR, HVPKPXXAPTKL:EPIS-AA$^{17}$-LYL:KFKYE:NMVVRSXGXH, RVPSTXXAPVKT:EPIS-AA$^{17}$-LYL:KFKYE:MIVEEXGXL, ASAAPXXVPQAL:EPIS-AA$^{17}$-LYL:KFKYE:MIVRSXKXS, ASASPXXVSQDL:EPIS-AA$^{17}$-LYL:KFKYE:MIVKSXKXS, ASASPXXVPQDL:EPIS-AA$^{17}$-LYL:KFKYE:MVVKSXKXS, NDEGLEXVPTEE:EPIS-AA$^{17}$-LYL:KFKYE:FLQHNKXEXR, NDEGLEXVPTGQ:EPIS-AA$^{17}$-LYL:KFKYE:LEEHSQXEXR, SSVKXQPSRVHH:EPIS-AA$^{17}$-LYL:KFKYE:RLEEHLEXAXA, RNVQXRPTQVQL:EPIS-AA$^{17}$-LYL:KFKYE:TLEDHLAXKXE, KIPKAXXTPTEL:SPIN-AA$^{17}$-LYF:YGKIP:DMVVDGXGXR,

GIPEPXXTPTKM:SPIN_AA$^{17}$-LYF:YGKIP:NMVVDSXAXR, SIPKAXXTPTEL:SPIN-AA$^{17}$-LYF:YGKIP:EMVVDGXGXR, HVTKPTSTPTKL:SPIN-AA$^{17}$-LYF:YGKIP:NMVVDSXGXH, YVPKPXXTPTKL:SPIN-AA$^{17}$-LYF:YGKIP:NMVVDSXGXH, TVPKPXXTPTQL:SPIN-AA$^{17}$-LYF:YGKIP:NMVVDAXGXH, AVPKAXXTPTKL:SPIN-AA$^{17}$-LYF:YGKIP:NMVVDAXGXH, KVGKAXXTPTKL:SPIN-AA$^{17}$-LYF:YGKIP:EGMSVVDXGXR, KASKAXXTPTKL:SPIN-AA$^{17}$-LYF:YGKIP:GMVVDEXGXR, AAPASXXTPTRL:SPIN-AA$^{17}$-LYF:YGKIP:DMVVDAXGXR, STPPTXXTPTRL:SPIN-AA$^{17}$-LYF:YGKIP:DMVVDSXGXR, HVPKPXXTPTKL:SPIN-AA$^{17}$-LYF:YGKIP:NMVVDSXGXH, RVPSTXXTPTKT:SPIN-AA$^{17}$-LYF:YGKIP:MIVVDXGXL, ASAAPXXTPTAL:SPIN-AA$^{17}$-LYF:YGKIP:MIVVDXKXS, ASASPXXTPTDL:SPIN-AA$^{17}$-LYF:YGKIP:MIVVDXKXS, ASASPXXTPTDL:SPIN-AA$^{17}$-LYF:YGKIP:MVVVDXKXS, KIPKAXXVPTEL:SPIN-AA$^{17}$-LYF:YGKIP:DMVVEGXGXR, GIPEPXXVPEKM:SPIN-AA$^{17}$-LYF:YGKIP:NMTVESXAXR, SIPKAXXVPTEL:SPIN-AA$^{17}$-LYF:YGKIP:EMVVEGXGXR, HVTKPTXAPTKL:SPIN-AA$^{17}$-LYF:YGKIP:NMVVRSXGXH, YVPKPXXAPTKL:SPIN-AA$^{17}$-LYF:YGKIP:NMVVRSXGXH, TVPKPXXAPTQL:SPIN-AA$^{17}$-LYF:YGKIP:NMVVRAXGXH, AVPKAXXAPTKL:SPIN-AA$^{17}$-LYF:YGKIP:NMVVKAXGXH, KVGKAXXVPTKL:SPIN-AA$^{17}$-LYF:YGKIP:EGMSVAEXGXR, KASKAXXVPTKL:SPIN-AA$^{17}$-LYF:YGKIP:GMAVSEXGXR, AAPASXXVPARL:SPIN-AA$^{17}$-LYF:YGKIP:DMVVEAXGXR, STPPTXXVPTRL:SPIN-AA$^{17}$-LYF:YGKIP:DMVVESXGXR, HVPKPXXAPTKL:SPIN-AA$^{17}$-LYF:YGKIP:NMVVRSXGXH, RVPSTXXAPVKT:SPIN-AA$^{17}$-LYF:YGKIP:MIVEEXGXL, ASAAPXXVPQAL:SPIN-AA$^{17}$-LYF:YGKIP:MIVRSXKXS, ASASPXXVSQDL:SPIN-AA$^{17}$-LYF:YGKIP:MIVKSXKXS, ASASPXXVPQDL:SPIN-AA$^{17}$-LYF:YGKIP:MVVKSXKXS, NDEGLEXVPTEE:SPIN-AA$^{17}$-LYF:YGKIP:FLQHNKXEXR, NDEGLEXVPTGQ:SPIN-AA$^{17}$-LYF:YGKIP:LEEHSQXEXR, SSVKXQPSRVHH:SPIN-AA$^{17}$-LYF:YGKIP:RLEEHLEXAXA, RNVQXRPTQVQL:SPIN-AA$^{17}$-LYF:YGKIP:TLEDHLAXKXE, KIPKAXXVPAEL:SPIS-AA$^{17}$-LYI:YKQYE:DMVVEGXGXR, GIPEPXXVPAKM:SPIS-AA$^{17}$-LYI:YKQYE:NMVVESXAXR, SIPKAXXVPAEL:SPIS-AA$^{17}$-LYI:YKQYE:EMVVEGXGXR, HVTKPTXVPAKL:SPIS-AA$^{17}$-LYI:YKQYE:NMVVESXGXH, YVPKPXXVPAKL:SPIS-AA$^{17}$-LYI:YKQYE:NMVVESXGXH, TVPKPXXVPAQL:SPIS-AA$^{17}$-LYI:YKQYE:NMVVEAXGXH, AVPKAXXVPAKL:SPIS-AA$^{17}$-LYI:YKQYE:NMVVEAXGXH, KVGKAXXVPAKL:SPIS-AA$^{17}$-LYI:YKQYE:EGMSVVEXGXR, KASKAXXVPAKL:SPIS-AA$^{17}$-LYI:YKQYE:GMVVEEXGXR, GSAGPXXVPAKM:SPIS-AA$^{17}$-LYI:YKQYE:GMVVERXGXS, STPPTXXVPARL:SPIS-AA$^{17}$-LYI:YKQYE:DMVVESXGXR, HVPKPXXVPAKL:SPIS-AA$^{17}$-LYI:YKQYE:NMVVESXGXH, RVPSTXXVPAKT:SPIS-AA$^{17}$-LYI:YKQYE:MIVVEXGXL, ASAAPXXVPAAL:SPIS-AA$^{17}$-LYI:YKQYE: MIVVEXKXS, ASASPXXVPADL:SPIS_AA$^{17}$-LYI:YKQYE:MIVVEXKXS, ASASPXXVPADL:SPIS-AA$^{17}$-LYI:YKQYE:MVVVEXKXS, KIPKAXXVPTEL:SPIS-AA$^{17}$-LYI:YKQYE:DMVVEGXGXR, GIPEPXXVPEKM:SPIS-AA$^{17}$-LYI:YKQYE:NMTVESXAXR, SIPKAXXVPTEL:SPIS-AA$^{17}$-LYI:YKQYE:EMVVEGXGXR, HVTKPTXAPTKL:SPIS-AA$^{17}$-LYI:YKQYE:NMVVRSXGXH, YVPKPXXAPTKL:SPIS-AA$^{17}$-LYI:YKQYE:NMVVRSXGXH, TVPKPXXAPTQL:SPIS-AA$^{17}$-LYI:YKQYE:NMVVRAXGXH, AVPKAXXAPTKL:SPIS-AA$^{17}$-LYI:YKQYE:NMVVKAXGXH, KVGKAXXVPTKL:SPIS-AA$^{17}$-LYI:YKQYE:EGMSVAEXGXR, KASKAXXVPTKL:SPIS-AA$^{17}$-LYI:YKQYE:GMAVSEXGXR, GSAGPXXTPTKM:SPIS-AA$^{17}$-LYI:YKQYE:GMVVDRXGXS, STPPTXXVPTRL:SPIS-AA$^{17}$-LYI:YKQYE:DMVVESXGXR, HVPKPXXAPTKL:SPIS-AA$^{17}$-LYI:YKQYE:NMVVRSXGXH, RVPSTXXAPVKT:SPIS-AA$^{17}$-LYI:YKQYE:MIVEEXGXL, ASAAPXXVPQAL:SPIS-AA$^{17}$-LYI:YKQYE:MIVRSXKXS, ASASPXXVSQDL:SPIS-AA$^{17}$-LYI:YKQYE:MIVKSXKXS, ASASPXXVPQDL:SPIS-AA$^{17}$-LYI:YKQYE:MVVKSXKXS, NDEGLEXVPTEE:SPIS-AA$^{17}$-LYI:YKQYE:FLQHNKXEXR, NDEGLEXVPTGQ:SPIS-AA$^{17}$-LYI:YKQYE:LEEHSQXEXR, SSVKXQPSRVHH:SPIS-AA$^{17}$-LYI:YKQYE:RLEEHLEXAXA, RNVQXRPTQVQL:SPIS-AA$^{17}$-LYI:YKQYE:TLEDHLAXKXE, KIPKAXXVPTEL:SPIS-AA$^{17}$-LFI:YKQYE:DMVVEGXGXR, GIPEPXXVPTKM:SPIS-AA$^{17}$-LFI:YKQYE:NMVVESXAXR, SIPKAXXVPTEL:SPIS-AA$^{17}$-LFI:YKQYE:EMVVEGXGXR, HVTKPTXVPTKL:SPIS-AA$^{17}$-LFI:YKQYE:NMVVESXGXH, YVPKPXXVPTKL:SPIS-AA$^{17}$-LFI:YKQYE:NMVVESXGXH, TVPKPXXVPTQL:SPIS-AA$^{17}$-LFI:YKQYE:NMVVEAXGXH, AVPKAXXVPTKL:SPIS-AA$^{17}$-LFI:YKQYE:NMVVEAXGXH, KVGKAXXVPTKL:SPIS-AA$^{17}$-LFI:YKQYE:EGMSVVEXGXR, KASKAXXVPTKL:SPIS-AA$^{17}$-LFI:YKQYE:GMVVEEXGXR, GSAGPXXVPTKM:SPIS-AA$^{17}$-LFI:YKQYE:GMVVERXGXS, AAPASXXVPTRL:SPIS-AA$^{17}$-LFI:YKQYE:DMVVEAXGXR, HVPKPXXVPTKL:SPIS-AA$^{17}$-LFI:YKQYE:NMVVESXGXH, RVPSTXXVPTKT:SPIS-AA$^{17}$-LFI:YKQYE:MIVVEXGXL, ASAAPXXVPTAL:SPIS-AA$^{17}$-LFI:YKQYE:MIVVEXKXS, ASASPXXVPTDL:SPIS-AA$^{17}$-LFI:YKQYE:MIVVEXKXS, ASASPXXVPTDL:SPIS-AA$^{17}$-LFI:YKQYE:MVVVEXKXS, GIPEPXXVPEKM:SPIS-AA$^{17}$-LFI:YKQYE:NMTVESXAXR, HVTKPTXAPTKL:SPIS-AA$^{17}$-LFI:YKQYE:NMVVRSXGXH, YVPKPXXAPTKLSPIS-AA$^{17}$-LFI:YKQYE:NMVVRSXGXH, TVPKPXXAPTQL:SPIS-AA$^{17}$-LFI:YKQYE:NMVVRAXGXH, AVPKAXXAPTKL:SPIS-AA$^{17}$-LFI:YKQYE:NMVVKAXGXH, KVGKAXXVPTKLSPIS-AA$^{17}$-LFI:YKQYE:EGMSVAEXGXR, KASKAXXVPTKL:SPIS-AA$^{17}$-LFI:YKQYE:GMAVSEXGXR, GSAGPXXTPTKM:SPIS-AA$^{17}$-LFI:YKQYE:GMVVDRXGXS, AAPASXXVPARL-SPIS-AA$^{17}$-LFI:YKQYE:DMVVEAXGXR, HVPKPXXAPTKL:SPIS-AA$^{17}$-LFI:YKQYE:NMVVRSXGXH, RVPSTXXAPVKT:SPIS-AA$^{17}$-LFI:YKQYE:M IVEEXGXL, ASAAPXXVPQALSPIS-AA$^{17}$-LFI:YKQYE:MIVRSXKXS, ASASPXXVSQDL:SPIS-AA$^{17}$-LFI:YKQYE:MIVKSXKXS, ASASPXXVPQDL:SPIS-AA$^{17}$-LFI:YKQYE:MVVKSXKXS, NDEGLEXVPTEE:SPIS-AA$^{17}$-LFI:YKQYE:FLQHNKXEXR, NDEGLEXVPTGQ:SPIS-AA$^{17}$-LFI:YKQYE:LEEHSQXEXR, SSVKXQPSRVHH:SPIS-AA$^{17}$-LFI:YKQYE:RLEEHLEXAXA, RNVQXRPTQVQL:SPIS-AA$^{17}$-LFI:YKQYE:TLEDHLAXKXE, KIPKAXXAPVEL:KPLS-AA$^{17}$-LYV:DHHKD:DMVEEGXGXR, GIPEPXXAPVKM:KPLS-AA$^{17}$-LYV:DHHKD:NMVEESXAXR, SIPKAXXAPVEL:KPLS-AA$^{17}$-LYV:DHHKD:EMVEEGXGXR, HVTKPTXAPVKL:KPLS-

AA$^{17}$-LYV:DHHKD:NMVEESXGXH, YVPKPXXAPVKL:KPLS-AA$^{17}$-LYV:DHHKD:NMVEESXGXH, TVPKPXXAPVQL:KPLS-AA$^{17}$-LYV:DHHKD:NMVEEAXGXH, AVPKAXXAPVKL:KPLS-AA$^{17}$-LYV:DHHKD:NMVEEAXGXH, KVGKAXXAPVKL:KPLS-AA$^{17}$-LYV:DHHKD:EGMSVEEXGXR, KASKAXXAPVKL:KPLS-AA$^{17}$-LYV:DHHKD:GMVEEEXGXR, GSAGPXXAPVKM:KPLS-AA$^{17}$-LYV:DHHKD:GMVEERXGXS, AAPASXXAPVRL:KPLS-AA$^{17}$-LYV:DHHKD:DMVEEAXGXR, STPPTXXAPVRL:KPLS-AA$^{17}$-LYV:DHHKD:DMVEESXGXR, HVPKPXXAPVKL:KPLS-AA$^{17}$-LYV:DHHKD:NMVEESXGXH, ASAAPXXAPVAL:KPLS-AA$^{17}$-LYV:DHHKD:MIVEEXKXS, ASASPXXAPVDL:KPLS-AA$^{17}$-LYV:DHHKD:MIVEEXKXS, ASASPXXAPVDL:KPLS-AA$^{17}$-LYV:DHHKD:MVVEEXKXS, KIPKAXXVPTEL:KPLS-AA$^{17}$-LYV:DHHKD:DMVVEGXGXR, GIPEPXXVPEKM:KPLS-AA$^{17}$-LYV:DHHKD:NMTVESXAXR, SIPKAXXVPTEL:KPLS-AA$^{17}$-LYV:DHHKD:EMVVEGXGXR, HVTKPTXAPTKL:KPLS-AA$^{17}$-LYV:DHHKD:NMVVRSXGXH, YVPKPXXAPTKL:KPLS-AA$^{17}$-LYV:DHHKD:NMVVRSXGXH, TVPKPXXAPTQL:KPLS-AA$^{17}$-LYV:DHHKD:NMVVRAXGXH, AVPKAXXAPTKL:KPLS_AA$^{17}$-LYV:DHHKD:NMVVKAXGXH, KVGKAXXVPTKL:KPLS-AA$^{17}$-LYV:DHHKD:EGMSVAEXGXR, KASKAXXVPTKL:KPLS-AA$^{17}$-LYV:DHHKD:GMAVSEXGXR, GSAGPXXTPTKM:KPLS-AA$^{17}$-LYV:DHHKD:GMVVDRXGXS, AAPASXXVPARL:KPLS-AA$^{17}$-LYV:DHHKD:DMVVEAXGXR, STPPTXXVPTRL:KPLS-AA$^{17}$-LYV:DHHKD:DMVVESXGXR, HVPKPXXAPTKL:KPLS-AA$^{17}$-LYV:DHHKD:NMVVRSXGXH, ASAAPXXVPQAL:KPLS-AA$^{17}$-LYV:DHHKD:MIVRSXKXS, ASASPXXVSQDL:KPLS-AA$^{17}$-LYV:DHHKD:MIVKSXKXS, ASASPXXVPQDL:KPLS-AA$^{17}$-LYV:DHHKD:MVVKSXKXS, NDEGLEXVPTEE:KPLS-AA$^{17}$-LYV:DHHKD:FLQHNKXEXR, NDEGLEXVPTGQ:KPLS-AA$^{17}$-LYV:DHHKD:LEEHSQXEXR, SSVKXQPSRVHH:KPLS-AA$^{17}$-LYV:DHHKD:RLEEHLEXAXA, RNVQXRPTQVQL:KPLS-AA$^{17}$-LYV:DHHKD:TLEDHLAXKXE, KIPKAXXVPQEL:EPLP-AA$^{17}$-VYY:EQLSN:DMVRXGXGSR, GIPEPXXVPQKM:EPLP-AA$^{17}$-VYY:EQLSN:NMVRSSXAXR, SIPKAXXVPQEL:EPLP-AA$^{17}$-VYY:EQLSN:EMVRXGXGSR, HVTKPTXVPQKL:EPLP-AA$^{17}$-VYY:EQLSN:NMVRSSXGXH, YVPKPXXVPQKL:EPLP-AA$^{17}$-VYY:EQLSN:NMVRSSXGXH, TVPKPXXVPQQL:EPLP-AA$^{17}$-VYY:EQLSN:NMVRSAXGXH, AVPKAXXVPQKL:EPLP-AA$^{17}$-VYY:EQLSN:NMVRSAXGXH, KVGKAXXVPQKL:EPLP-AA$^{17}$-VYY:EQLSN:EGMSVRSXGXR, KASKAXXVPQKL:EPLP-AA$^{17}$-VYY:EQLSN:GMVRSEXGXR, GSAGPXXVPQKM:EPLP-AA$^{17}$-VYY:EQLSN:GMVRSRXGXS, AAPASXXVPQRL:EPLP-AA$^{17}$-VYY:EQLSN:DMVRSAXGXR, STPPTXXVPQRL:EPLP-AA$^{17}$-VYY:EQLSN:DMVRSSXGXR, HVPKPXXVPQKL:EPLP-AA$^{17}$-VYY:EQLSN:NMVRSSXGXH, RVPSTXXVPQKT:EPLP-AA$^{17}$-VYY:EQLSN:MIVRSXGXL, ASASPXXVPQDL:EPLP-AA$^{17}$-VYY:EQLSN:MIVRSXKXS, ASASPXXVPQDL:EPLP-AA$^{17}$-VYY:EQLSN:MVVRSXKXS, KIPKAXXVPTEL:EPLP-AA$^{17}$-VYY:EQLSN:DMVVEGXGXR, GIPEPXXVPEKM:EPLP-AA$^{17}$-VYY:EQLSN:NMTVESXAXR, SIPKAXXVPTEL:EPLP-AA$^{17}$-VYY:EQLSN:EMVVEGXGXR, HVTKPTXAPTKL:EPLP-AA$^{17}$-VYY: EQLSN:NMVVRSXGXH, YVPKPXXAPTKL:EPLP-AA$^{17}$-VYY:EQLSN:NMVVRSXGXH, TVPKPXXAPTQL:EPLP-AA$^{17}$-VYY:EQLSN:NMVVRAXGXH, AVPKAXXAPTKL:EPLP-AA$^{17}$-VYY:EQLSN:NMVVKAXGXH, KVGKAXXVPTKL:EPLP-AA$^{17}$-VYY:EQLSN:EGMSVAEXGXR, KASKAXXVPTKL:EPLP-AA$^{17}$-VYY:EQLSN:GMAVSEXGXR, GSAGPXXTPTKM:EPLP-AA$^{17}$-VYY:EQLSN:GMVVDRXGXS, AAPASXXVPARL:EPLP-AA$^{17}$-VYY:EQLSN:DMVVEAXGXR, STPPTXXVPTRL:EPLP-AA$^{17}$-VYY:EQLSN:DMVVESXGXR, HVPKPXXAPTKL:EPLP-AA$^{17}$-VYY:EQLSN:NMVVRSXGXH, RVPSTXXAPVKT:EPLP-AA$^{17}$-VYY:EQLSN:MIVEEXGXL, ASASPXXVSQDL:EPLP-AA$^{17}$-VYY:EQLSN:MIVKSXKXS, ASASPXXVPQDL:EPLP-AA$^{17}$-VYY:EQLSN:MVVKSXKXS, NDEGLEXVPTEE:EPLP-AA$^{17}$-VYY:EQLSN:FLQHNKXEXR, NDEGLEXVPTGQ:EPLP-AA$^{17}$-VYY:EQLSN:LEEHSQXEXR, SSVKXQPSRVHH:EPLP-AA$^{17}$-VYY:EQLSN:RLEEHLEXAXA, RNVQXRPTQVQL:EPLP-AA$^{17}$-VYY:EQLSN:TLEDHLAXKXE, KIPKAXXVSQEL:EPLT-AA$^{17}$-LYY:EQLSN:DMVKXGXGSR, GIPEPXXVSQKM:EPLT-AA$^{17}$-LYY:EQLSN:NMVKSSXAXR, SIPKAXXVSQEL:EPLT-AA$^{17}$-LYY:EQLSN:EMVKXGXGSR, HVTKPTXVSQKL:EPLT-AA$^{17}$-LYY:EQLSN:NMVKSSXGXH, YVPKPXXVSQKL:EPLT-AA$^{17}$-LYY:EQLSN:NMVKSSXGXH, TVPKPXXVSQQL:EPLT-AA$^{17}$-LYY:EQLSN:NMVKSAXGXH, AVPKAXXVSQKL:EPLT-AA$^{17}$-LYY:EQLSN:NMVKSAXGXH, KVGKAXXVSQKL:EPLT-AA$^{17}$-LYY:EQLSN:EGMSVKSXGXR, KASKAXXVSQKL:EPLT-AA$^{17}$-LYY:EQLSN:GMVKSEXGXR, GSAGPXXVSQKM:EPLT-AA$^{17}$-LYY:EQLSN:GMVKSRXGXS, AAPASXXVSQRL:EPLT-AA$^{17}$-LYY:EQLSN:DMVKSAXGXR, STPPTXXVSQRL:EPLT-AA$^{17}$-LYY:EQLSN:DMVKSSXGXR, HVPKPXXVSQKL:EPLT-AA$^{17}$-LYY:EQLSN:NMVKSSXGXH, RVPSTXXVSQKT:EPLT-AA$^{17}$-LYY:EQLSN:MIVKSXGXL, ASAAPXXVSQAL:EPLT-AA$^{17}$-LYY:EQLSN:MIVKSXKXS, ASASPXXVSQDL:EPLT-AA$^{17}$-LYY:EQLSN:MVVKSXKXS, KIPKAXXVPTEL:EPLT-AA$^{17}$-LYY:EQLSN:DMVVEGXGXR, GIPEPXXVPEKM:EPLT-AA$^{17}$-LYY:EQLSN:NMTVESXAXR, SIPKAXXVPTEL:EPLT-AA$^{17}$-LYY:EQLSN:EMVVEGXGXR, HVTKPTXAPTKL:EPLT-AA$^{17}$-LYY:EQLSN:NMVVRSXGXH, YVPKPXXAPTKL:EPLT-AA$^{17}$-LYY:EQLSN:NMVVRSXGXH, TVPKPXXAPTQL:EPLT-AA$^{17}$-LYY:EQLSN:NMVVRAXGXH, AVPKAXXAPTKL:EPLT-AA$^{17}$-LYY:EQLSN:NMVVKAXGXH, KVGKAXXVPTKL:EPLT-AA$^{17}$-LYY:EQLSN:EGMSVAEXGXR, KASKAXXVPTKL:EPLT-AA$^{17}$-LYY:EQLSN:GMAVSEXGXR, GSAGPXXTPTKM:EPLT-AA$^{17}$-LYY:EQLSN:GMVVDRXGXS, AAPASXXVPARL:EPLT-AA$^{17}$-LYY:EQLSN:DMVVEAXGXR, STPPTXXVPTRL:EPLT-AA$^{17}$-LYY:EQLSN:DMVVESXGXR, HVPKPXXAPTKL:EPLT-AA$^{17}$-LYY:EQLSN:NMVVRSXGXH, RVPSTXXAPVKT:EPLT-AA$^{17}$-LYY:EQLSN:MIVEEXGXL, ASAAPXXVPQAL:EPLT-AA$^{17}$-LYY:EQLSN:MIVRSXKXS, NDEGLEXVPTEE:EPLT-AA$^{17}$-LYY:EQLSN:FLQHNKXEXR, NDEGLEXVPTGQ:EPLT-AA$^{17}$-LYY:EQLSN:LEEHSQXEXR, SSVKXQPSRVHH:EPLT-AA$^{17}$-LYY:EQLSN:RLEEHLEXAXA, RNVQXRPTQVQL:EPLT-AA$^{17}$-LYY:EQLSN:TLEDHLAXKXE, KIPKAXXVPQEL:EPLT-AA$^{17}$-LYY:EQLSN:DMVKXGXGSR,

GIPEPXXVPQKM:EPLT-AA$^{17}$-LYY:EQLSN:NMVKSSXAXR, SIPKAXXVPQEL:EPLT-AA$^{17}$-LYY:EQLSN:EMVKXGXG-SR, HVTKPTXVPQKL:EPLT-AA$^{17}$-LYY:EQLSN:NMVKSSXGXH, YVPKPXXVPQKL:EPLT-AA$^{17}$-LYY:EQLSN:NMVKSSXGXH, TVPKPXXVPQQL:EPLT-AA$^{17}$-LYY:EQLSN:NMVKSAXGXH, AVPKAXXVPQKL:EPLT-AA$^{17}$-LYY:EQLSN:NMVKSAXGXH, KVGKAXXVPQKL:EPLT-AA$^{17}$-LYY:EQLSN:EGMSVKSXGXR, KASKAXXVPQKL:EPLT-AA$^{17}$-LYY:EQLSN:GMVKSEXGXR, GSAGPXXVPQKM:EPLT-AA$^{17}$-LYY:EQLSN:GMVK-SRXGXS, AAPASXXVPQRL:EPLT-AA$^{17}$-LYY:EQLSN:DMVKSAXGXR, STPPTXXVPQRL:EPLT-AA$^{17}$-LYY:EQLSN:DMVKSSXGXR, HVPKPXXVPQKL:EPLT-AA$^{17}$-LYY:EQLSN:NMVKSSXGXH, RVPSTXXVPQKT:EPLT-AA$^{17}$-LYY:EQLSN:MIVKSXGXL, ASAAPXXVPQAL:EPLT-AA$^{17}$-LYY:EQLSN:MIVKSXKXS, ASASPXXVPQDL:EPLT-AA$^{17}$-LYY:EQLSN:MIVKSXKXS, NDEGLEXVPTGQ:SNIT-AA$^{17}$-QIM:IGEMS:LEQHNQXEXR, KIPKAXXVPTEL:SNIT-AA$^{17}$-QIM:IGEMS:DMVVEGXGXR, GIPEPXXVPEKM:SNIT-AA$^{17}$-QIM:IGEMS:NMTVESXAXR, SIPKAXXVP-TEL:SNIT-AA$^{17}$-QIM:IGEMS:EMVVEGXGXR, HVTKPTXAPTKL:SNIT-AA$^{17}$-QIM:IGEMS:NMVVRSXGXH, YVPKPXX-APTKL:SNIT-AA$^{17}$-QIM:IGEMS:NMVVRSXGXH, TVPKPXXAPTQL:SNIT-AA$^{17}$-QIM:IGEMS:NMVVRAXGXH, AVP-KAXXAPTKL:SNIT-AA$^{17}$-QIM:IGEMS:NMVVKAXGXH, KVGKAXXVPTKL:SNIT-AA$^{17}$-QIM:IGEMS:EGMSVAEXGXR, KASKAXXVPTKL:SNIT-AA$^{17}$-QIM:IGEMS:GMAVSEXGXR, GSAGPXXTPTKM:SNIT-AA$^{17}$-QIM:IGEMS:GMV-VDRXGXS, AAPASXXVPARL:SNIT-AA$^{17}$-QIM:IGEMS:DMVVEAXGXR, STPPTXXVPTRL:SNIT-AA$^{17}$-QIM:IGEMS:DMVVESXGXR, HVPKPXXAPTKL:SNIT-AA$^{17}$-QIM:IGEMS:NMVVRSXGXH, RVPSTXXA-PVKT:SNIT-AA$^{17}$-QIM:IGEMS:MIVEEXGXL, ASAAPXXVPQAL:SNIT-AA$^{17}$-QIM:IGEMS:MIVRSXKXS, ASAS-PXXVSQDL:SNIT-AA$^{17}$-QIM:IGEMS:MIVKSXKXS, ASASPXXVPQDL:SNIT-AA$^{17}$-QIM:IGEMS:MVVKSXKXS, NDE-GLEXVPTGQ:SNIT-AA$^{17}$-QIM:IGEMS:LEEHSQXEXR, SSVKXQPSRVHH:SNIT-AA$^{17}$-QIM:IGEMS:RLEEHLEXAXA, RNVQXRPTQVQL:SNIT-AA$^{17}$-QIM:IGEMS:TLEDHLAXKXE, NDEGLEXVPTEE:SNIT-AA$^{17}$-QIM:LGEMS:FLEHXKX-ESR, KIPKAXXVPTEL:SNIT-AA$^{17}$-QIM:LGEMS:DMVVEGXGXR, GIPEPXXVPEKM:SNIT-AA$^{17}$-QIM:LGEMS:NMTVESXAXR, SIPKAXXVPTEL:SNIT-AA$^{17}$-QIM:LGEMS:EMVVEGXGXR, HVTKPTXA-PTKL:SNIT-AA$^{17}$-QIM:LGEMS:NMVVRSXGXH, YVPKPXXAPTKL:SNIT-AA$^{17}$-QIM:LGEMS:NMVVRSXGXH, TVPK-PXXAPTQL:SNIT-AA$^{17}$-QIM:LGEMS:NMVVRAXGXH, AVPKAXXAPTKL:SNIT-AA$^{17}$-QIM:LGEMS:NMVVKAXGXH, KVGKAXXVPTKL:SNIT-AA$^{17}$-QIM:LGEMS:EGMSVAEXGXR, KASKAXXVPTKL:SNIT-AA$^{17}$-QIM:LGEMS:GMAV-SEXGXR, GSAGPXXTPTKM:SNIT-AA$^{17}$-QIM:LGEMS:GMVVDRXGXS, AAPASXXVPARL:SNIT-AA$^{17}$-QIM:LGEMS:DMVVEAXGXR, STPPTXXVPTRL:SNIT-AA$^{17}$-QIM:LGEMS:DMVVESXGXR, HVPKPXXA-PTKL:SNIT-AA$^{17}$-QIM:LGEMS:NMVVRSXGXH, RVPSTXXAPVKT:SNIT-AA$^{17}$-QIM:LGEMS:MIVEEXGXL, ASAAPXXVPQAL:SNIT-AA$^{17}$-QIM:LGEMS:MIVRSXKXS, ASASPXXVSQDL:SNIT-AA$^{17}$-QIM:LGEMS:MIVKSXKXS, ASASPXXVPQDL:SNIT-AA$^{17}$-QIM:LGEMS:MVVKSXKXS, NDEGLEXVPTEE:SNIT-AA$^{17}$-QIM:LGEMS:FLQHNKX-EXR, SSVKXQPSRVHH:SNIT-AA$^{17}$-QIM:LGEMS:RLEEHLEXAXA, RNVQXRPTQVQL:SNIT-AA$^{17}$-QIM:LGEMS:TLEDHLAXKXE, RNVQXRPSRVQL:RSVK-AA$^{17}$-AKV:KEVQV:TLEEHLAXKXE, KIPKAXXVP-TEL:RSVK-AA$^{17}$-AKV:KEVQV:DMVVEGXGXR, GIPEPXXVPEKM:RSVK-AA$^{17}$-AKV:KEVQV:NMTVESXAXR, SIP-KAXXVPTEL:RSVK-AA$^{17}$-AKV:KEVQV:EMVVEGXGXR, HVTKPTXAPTKL:RSVK-AA$^{17}$-AKV:KEVQV:NMV-VRSXGXH, YVPKPXXAPTKL:RSVK-AA$^{17}$-AKV:KEVQV:NMVVRSXGXH, TVPKPXXAPTQL:RSVK-AA$^{17}$-AKV:KEVQV:NMVVRAXGXH, AVPKAXXAPTKL:RSVK-AA$^{17}$-AKV:KEVQV:NMVVKAXGXH, KVG-KAXXVPTKL:RSVK-AA$^{17}$-AKV:KEVQV:EGMSVAEXGXR, KASKAXXVPTKL:RSVK-AA$^{17}$-AKV:KEVQV:GMAV-SEXGXR, GSAGPXXTPTKM:RSVK-AA$^{17}$-AKV:KEVQV:GMVVDRXGXS, AAPASXXVPARL:RSVK-AA$^{17}$-AKV:KEVQV:DMVVEAXGXR, STPPTXXVPTRL:RSVK-AA$^{17}$-AKV:KEVQV:DMVVESXGXR, HVPKPXXA-PTKL:RSVK-AA$^{17}$-AKV:KEVQV:NMVVRSXGXH, RVPSTXXAPVKT:RSVK-AA$^{17}$-AKV:KEVQV:MIVEEXGXL, ASAAPXXVPQAL:RSVK-AA$^{17}$-AKV:KEVQV:MIVRSXKXS, ASASPXXVSQDL:RSVK-AA$^{17}$-AKV:KEVQV:MIVK-SXKXS, ASASPXXVPQDL:RSVK-AA$^{17}$-AKV:KEVQV:MVVKSXKXS, NDEGLEXVPTEE:RSVK-AA$^{17}$-AKV:KEVQV:FLQHNKXEXR, NDEGLEXVPTGQ:RSVK-AA$^{17}$-AKV:KEVQV:LEEHSQXEXR, RNVQXRP-TQVQL:RSVK-AA$^{17}$-AKV:KEVQV:TLEDHLAXKXE, SSVKXQPTQVHH:RPVQ-AA$^{17}$-RKI:KKATV:RLEDHLEXAXA, KIPKAXXVPTEL:RPVQ-AA$^{17}$-RKI:KKATV:DMVVEGXGXR, GIPEPXXVPEKM:RPVQ-AA$^{17}$-RKI:KKATV:NMTVESX-AXR, SIPKAXXVPTEL:RPVQ-AA$^{17}$-RKI:KKATV:EMVVEGXGXR, HVTKPTXAPTKL:RPVQ-AA$^{17}$-RKI:KKATV:NMV-VRSXGXH, YVPKPXXAPTKL:RPVQ-AA$^{17}$-RKI:KKATV:NMVVRSXGXH, TVPKPXXAPTQL:RPVQ-AA$^{17}$-RKI:KKATV:NMVVRAXGXH, AVPKAXXAPTKL:RPVQ-AA$^{17}$-RKI:KKATV:NMVVKAXGXH, KVG-KAXXVPTKL:RPVQ-AA$^{17}$-RKI:KKATV:EGMSVAEXGXR, KASKAXXVPTKL:RPVQ-AA$^{17}$-RKI:KKATV:GMAV-SEXGXR, GSAGPXXTPTKM:RPVQ-AA$^{17}$-RKI:KKATV:GMVVDRXGXS, AAPASXXVPARL:RPVQ-AA$^{17}$-RKI:KKATV:DMVVEAXGXR, STPPTXXVPTRL:RPVQ-AA$^{17}$-RKI:KKATV:DMVVESXGXR, HVPKPXXA-PTKL:RPVQ-AA$^{17}$-RKI:KKATV:NMVVRSXGXH, RVPSTXXAPVKT:RPVQ-AA$^{17}$-RKI:KKATV:MIVEEXGXL, ASAAPXXVPQAL:RPVQ-AA$^{17}$-RKI:KKATV:MIVRSXKXS, ASASPXXVSQDL:RPVQ-AA$^{17}$-RKI:KKATV:MIVKSXKXS, ASASPXXVPQDL:RPVQ-AA$^{17}$-RKI:KKATV:MVVKSXKXS, NDEGLEXVPTEE:RPVQ-AA$^{17}$-RKI:KKATV:FLQHNKX-EXR, NDEGLEXVPTGQ:RPVQ-AA$^{17}$-RKI:KKATV:LEEHSQXEXR and SSVKXQPSRVHH:RPVQ-AA17-RKI:KKATV:RLEEHLEXAXA; and wherein AA$^{17}$ is selected from the group consisting of G, A, V, L, I, P, F, M, W, T and S (in particular is selected from the group consisting of M, I, L, V and T).

In particular, in certain embodiments, the hexaplet PEP7:PEP3:PEP1:PEP2:PEP4:PEP8 is selected from the

group consisting of GIPEPXX:VPT:SAIS:LKNYQ:VVE:SXAXR, SIPKAXX:VPT:SAIS:LKNYQ:VVE:GXGXR, HVTKPTX:VPT:SAIS:LKNYQ:VVE:SXGXH, YVPKPXX:VPT:SAIS:LKNYQ:VVE:SXGXH, TVPKPXX:VPT:SAIS:LKNYQ:VVE:AXGXH, AVPKAXX:VPT:SAIS:LKNYQ:VVE:AXGXH, KVGKAXX:VPT:SAIS:LKNYQ:VVE:XGXR, KASKAXX:VPT:SAIS:LKNYQ:VVE:EXGXR, GSAGPXX:VPT:SAIS:LKNYQ:VVE:RXGXS, AAPASXX:VPT:SAIS:LKNYQ:VVE:AXGXR, STPPTXX:VPT:SAIS:LKNYQ:VVE:SXGXR, HVPKPXX:VPT:SAIS:LKNYQ:VVE:SXGXH, RVPSTXX:VPT:SAIS:LKNYQ:VVE:XGXL, ASAAPXX:VPT:SAIS:LKNYQ:VVE:XKXS, ASASPXX:VPT:SAIS:LKNYQ:VVE:XKXS, NDEGLEX:VPT:SAIS:LKNYQ:VVE:KXEXR, NDEGLEX:VPT:SAIS:LKNYQ:VVE:QXEXR, SSVKXQP:VPT:SAIS:LKNYQ:VVE:LEXAXA, RNVQXRP:VPT:SAIS:LKNYQ:VVE:LAXKXE, GIPEPXX:VPE:SAIS:LKNYQ:TVE:SXAXR, HVTKPTX:APT:SAIS:LKNYQ:VVR:SXGXH, YVPKPXX:APT:SAIS:LKNYQ:VVR:SXGXH, TVPKPXX:APT:SAIS:LKNYQ:VVR:AXGXH, AVPKAXX:APT:SAIS:LKNYQ:VVK:AXGXH, KVGKAXX:VPT:SAIS:LKNYQ:VAE:XGXR, KASKAXX:VPT:SAIS:LKNYQ:AVS:EXGXR, GSAGPXX:TPT:SAIS:LKNYQ:VVD:RXGXS, AAPASXX:VPA:SAIS:LKNYQ:VVE:AXGXR, HVPKPXX:APT:SAIS:LKNYQ:VVR:SXGXH, RVPSTXX:APV:SAIS:LKNYQ:VEE:XGXL, ASAAPXX:VPQ:SAIS:LKNYQ:VRS:XKXS, ASASPXX:VSQ:SAIS:LKNYQ:VKS:XKXS, ASASPXX:VPQ:SAIS:LKNYQ:VKS:XKXS, NDEGLEX:VPT:SAIS:LKNYQ:QHN:KXEXR, NDEGLEX:VPT:SAIS:LKNYQ:EHS:QXEXR, SSVKXQP:SRV:SAIS:LKNYQ:EEH:LEXAXA, RNVQXRP:TQV:SAIS:LKNYQ:EDH:LAXKXE, KIPKAXX:VPE:SSLS:LKVYP:TVE:GXGXR, SIPKAXX:VPE:SSLS:LKVYP:TVE:GXGXR, HVTKPTX:VPE:SSLS:LKVYP:TVE:SXGXH, YVPKPXX:VPE:SSLS:LKVYP:TVE:SXGXH, TVPKPXX:VPE:SSLS:LKVYP:TVE:AXGXH, AVPKAXX:VPE:SSLS:LKVYP:TVE:AXGXH, KVGKAXX:VPE:SSLS:LKVYP:TVE:XGXR, KASKAXX:VPE:SSLS:LKVYP:TVE:EXGXR, GSAGPXX:VPE:SSLS:LKVYP:TVE:RXGXS, AAPASXX:VPE:SSLS:LKVYP:TVE:AXGXR, STPPTXX:VPE:SSLS:LKVYP:TVE:SXGXR, HVPKPXX:VPE:SSLS:LKVYP:TVE:SXGXH, RVPSTXX:VPE:SSLS:LKVYP:TVE:XGXL, ASAAPXX:VPE:SSLS:LKVYP:TVE:XKXS, ASASPXX:VPE:SSLS:LKVYP:TVE:XKXS, NDEGLEX:VPE:SSLS:LKVYP:TVE:KXEXR, NDEGLEX:VPE:SSLS:LKVYP:TVE:QXEXR, SSVKXQP:VPE:SSLS:LKVYP:TVE:LEXAXA, RNVQXRP:VPE:SSLS:LKVYP:TVE:LAXKXE, KIPKAXX:VPT:SSLS:LKVYP:VVE:GXGXR, SIPKAXX:VPT:SSLS:LKVYP:VVE:GXGXR, HVTKPTX:APT:SSLS:LKVYP:VVR:SXGXH, YVPKPXX:APT:SSLS:LKVYP:VVR:SXGXH, TVPKPXX:APT:SSLS:LKVYP:VVR:AXGXH, AVPKAXX:APT:SSLS:LKVYP:VVK:AXGXH, KVGKAXX:VPT:SSLS:LKVYP:VAE:XGXR, KASKAXX:VPT:SSLS:LKVYP:AVS:EXGXR, GSAGPXX:TPT:SSLS:LKVYP:VVD:RXGXS, AAPASXX:VPA:SSLS:LKVYP:VVE:AXGXR, STPPTXX:VPT:SSLS:LKVYP:VVE:SXGXR, HVPKPXX:APT:SSLS:LKVYP:VVR:SXGXH, RVPSTXX:APV:SSLS:LKVYP:VEE:XGXL, ASAAPXX:VPQ:SSLS:LKVYP:VRS:XKXS, ASASPXX:VSQ:SSLS:LKVYP:VKS:XKXS, ASASPXX:VPQ:SSLS:LKVYP:VKS:XKXS, NDEGLEX:VPT:SSLS:LKVYP:QHN:KXEXR, NDEGLEX:VPT:SSLS:LKVYP:EHS:QXEXR, SSVKXQP:SRV:SSLS:LKVYP:EEH:LEXAXA, RNVQXRP:TQV:SSLS:LKVYP:EDH:LAXKXE, KIPKAXX:VPT:SAIS:LKNYQ:VVE:GXGXR, KIPKAXX:APT:NAIS:LKKYR:VVR:GXGXR, GIPEPXX:APT:NAIS:LKKYR:VVR:SXAXR, SIPKAXX:APT:NAIS:LKKYR:VVR:GXGXR, YVPKPXX:APT:NAIS:LKKYR:VVR:SXGXH, TVPKPXX:APT:NAIS:LKKYR:VVR:AXGXH, AVPKAXX:APT:NAIS:LKKYR:VVR:AXGXH, KVGKAXX:APT:NAIS:LKKYR:VVR:XGXR, KASKAXX:APT:NAIS:LKKYR:VVR:EXGXR, GSAGPXX:APT:NAIS:LKKYR:VVR:RXGXS, AAPASXX:APT:NAIS:LKKYR:VVR:AXGXR, STPPTXX:APT:NAIS:LKKYR:VVR:SXGXR, HVPKPXX:APT:NAIS:LKKYR:VVR:SXGXH, RVPSTXX:APT:NAIS:LKKYR:VVR:XGXL, ASAAPXX:APT:NAIS:LKKYR:VVR:XKXS, ASASPXX:APT:NAIS:LKKYR:VVR:XKXS, NDEGLEX:APT:NAIS:LKKYR:VVR:KXEXR, NDEGLEX:APT:NAIS:LKKYR:VVR:QXEXR, SSVKXQP:APT:NAIS:LKKYR:VVR:LEXAXA, RNVQXRP:APT:NAIS:LKKYR:VVR:LAXKXE, KIPKAXX:VPT:NAIS:LKKYR:VVE:GXGXR, GIPEPXX:VPE:NAIS:LKKYR:TVE:SXAXR, SIPKAXX:VPT:NAIS:LKKYR:VVE:GXGXR, AVPKAXX:APT:NAIS:LKKYR:VVK:AXGXH, KVGKAXX:VPT:NAIS:LKKYR:VAE:XGXR, KASKAXX:VPT:NAIS:LKKYR:AVS:EXGXR, GSAGPXX:TPT:NAIS:LKKYR:VVD:RXGXS, AAPASXX:VPA:NAIS:LKKYR:VVE:AXGXR, STPPTXX:VPT:NAIS:LKKYR:VVE:SXGXR, RVPSTXX:APV:NAIS:LKKYR:VEE:XGXL, ASAAPXX:VPQ:NAIS:LKKYR:VRS:XKXS, ASASPXX:VSQ:NAIS:LKKYR:VKS:XKXS, ASASPXX:VPQ:NAIS:LKKYR:VKS:XKXS, NDEGLEX:VPT:NAIS:LKKYR:QHN:KXEXR, NDEGLEX:VPT:NAIS:LKKYR:EHS:QXEXR, SSVKXQP:SRV:NAIS:LKKYR:EEH:LEXAXA, RNVQXRP:TQV:NAIS:LKKYR:EDH:LAXKXE, HVTKPTX:APT:NAIS:LKKYR:VVR:SXGXH, KIPKAXX:APT:SATS:LRKHR:VVK:GXGXR, GIPEPXX:APT:SATS:LRKHR:VVK:SXAXR, SIPKAXX:APT:SATS:LRKHR:VVK:GXGXR, HVTKPTX:APT:SATS:LRKHR:VVK:SXGXH, YVPKPXX:APT:SATS:LRKHR:VVK:SXGXH, TVPKPXX:APT:SATS:LRKHR:VVK:AXGXH, KVGKAXX:APT:SATS:LRKHR:VVK:XGXR, KASKAXX:APT:SATS:LRKHR:VVK:EXGXR, GSAGPXX:APT:SATS:LRKHR:VVK:RXGXS, AAPASXX:APT:SATS:LRKHR:VVK:AXGXR, STPPTXX:APT:SATS:LRKHR:VVK:SXGXR, HVPKPXX:APT:SATS:LRKHR:VVK:SXGXH, RVPSTXX:APT:SATS:LRKHR:VVK:XGXL, ASAAPXX:APT:SATS:LRKHR:VVK:XKXS, ASASPXX:APT:SATS:LRKHR:VVK:XKXS, NDEGLEX:APT:SATS:LRKHR:VVK:KXEXR, NDEGLEX:APT:SATS:LRKHR:VVK:QXEXR, SSVKXQP:APT:SATS:LRKHR:VVK:LEXAXA, RNVQXRP:APT:SATS:LRKHR:VVK:LAXKXE, KIPKAXX:VPT:SATS:LRKHR:VVE:GXGXR, GIPEPXX:VPE:SATS:LRKHR:TVE:SXAXR, SIPKAXX:VPT:SATS:LRKHR:VVE:GXGXR, HVTKPTX:APT:SATS:LRKHR:VVR:SXGXH, YVPKPXX:APT:SATS:LRKHR:VVR:SXGXH, TVPKPXX:APT:SATS:LRKHR:VVR:AXGXH, KVGKAXX:VPT:SATS:LRKHR:VAE:XGXR, KASKAXX:VPT:SATS:LRKHR:AVS:EXGXR, GSAGPXX:TPT:SATS:LRKHR:VVD:RXGXS, AAPASXX:VPA:SATS:LRKHR:VVE:AXGXR, STPPTXX:VPT:SATS:LRKHR:VVE:SXGXR, HVPKPXX:APT:SATS:LRKHR:VVR:SXGXH, RVPSTXX:APV:SATS:LRKHR:VEE:XGXL, ASAAPXX:VPQ:SATS:LRKHR:VRS:XKXS, ASASPXX:VSQ:SATS:LRKHR:VKS:XKXS, ASASPXX:VPQ:SATS:LRKHR:VKS:XKXS, NDEGLEX:VPT:SATS:LRKHR:QHN:KXEXR, NDEGLEX:VPT:SATS:LRKHR:EHS:QXEXR, SSVKXQP:SRV:SATS:LRKHR:EEH:LEXAXA, RNVQXRP:TQV:SATS:LRKHR:EDH:LAXKXE, KIPKAXX:VPT:SPIS:LKYHY:VAE:GXGXR, GIPEPXX:VPT:SPIS:LKYHY:VAE:SXAXR, SIPKAXX:VPT:SPIS:LKYHY:VAE:GXGXR, HVTKPTX:VPT:SPIS:LKYHY:VAE:SXGXH, YVPKPXX:VPT:SPIS:LKYHY:VAE:SXGXH, TVPKPXX:VPT:SPIS:LKYHY:VAE:AXGXH, AVPKAXX:VPT:SPIS:LKYHY:VAE:AXGXH, KASKAXX:VPT:SPIS:LKYHY:VAE:EXGXR, GSAGPXX:VPT:SPIS:LKYHY:VAE:RXGXS, AAPASXX:VPT:SPIS:LKYHY:VAE:AXGXR, STPPTXX:VPT:SPIS:LKYHY:VAE:SXGXR, HVPKPXX:VPT:SPIS:LKYHY:VAE:SXGXH, RVPSTXX:VPT:SPIS:LKYHY:VAE:XGXL, ASAAPXX:VPT:SPIS:LKYHY:VAE:XKXS, ASASPXX:VPT:SPIS:LKYHY:VAE:XKXS, NDEGLEX:VPT:SPIS:LKYHY:VAE:KXEXR, NDEGLEX:VPT:SPIS:LKYHY:VAE:QXEXR, SSVKXQP:VPT:SPIS:LKYHY:VAE:LEXAXA, RNVQXRP:VPT:SPIS:LKYHY:VAE:LAXKXE, KIPKAXX:VPT:SPIS:LKYHY:VVE:GXGXR, GIPEPXX:VPE:SPIS:LKYHY:TVE:SXAXR, SIPKAXX:VPT:SPIS:LKYHY:VVE:GXGXR, HVTKPTX:APT:SPIS:LKYHY:VVR:SXGXH, YVPKPXX:APT:SPIS:LKYHY:VVR:SXGXH, TVPKPXX:APT:SPIS:LKYHY:VVR:AXGXH, AVPKAXX:APT:SPIS:LKYHY:VVK:AXGXH, KASKAXX:VPT:SPIS:LKYHY:AVS:EXGXR, GSAGPXX:TPT:SPIS:LKYHY:VVD:RXGXS, AAPASXX:VPA:SPIS:LKYHY:VVE:AXGXR, STPPTXX:VPT:SPIS:LKYHY:VVE:SXGXR, HVPKPXX:APT:SPIS:LKYHY:VVR:SXGXH, RVPSTXX:APV:SPIS:LKYHY:VEE:XGXL, ASAAPXX:VPQ:SPIS:LKYHY:VRS:XKXS, ASASPXX:VSQ:SPIS:LKYHY:VKS:XKXS, ASASPXX:VPQ:SPIS:LKYHY:VKS:XKXS, NDEGLEX:VPT:SPIS:LKYHY:QHN:KXEXR, NDEGLEX:VPT:SPIS:LKYHY:EHS:QXEXR, SSVKXQP:SRV:SPIS:LKYHY:EEH:LEXAXA, RNVQXRP:TQV:SPIS:LKYHY:EDH:LAXKXE, KIPKAXX:VPT:EPIS:KFKYE:AVS:GXGXR, GIPEPXX:VPT:EPIS:KFKYE:AVS:SXAXR, SIPKAXX:VPT:EPIS:KFKYE:AVS:GXGXR, HVTKPTX:VPT:EPIS:KFKYE:AVS:SXGXH, YVPKPXX:VPT:EPIS:KFKYE:AVS:SXGXH, TVPKPXX:VPT:EPIS:KFKYE:AVS:AXGXH, AVPKAXX:VPT:EPIS:KFKYE:AVS:AXGXH, KVGKAXX:VPT:EPIS:KFKYE:AVS:XGXR, GSAGPXX:VPT:EPIS:KFKYE:AVS:RXGXS, AAPASXX:VPT:EPIS:KFKYE:AVS:AXGXR, STPPTXX:VPT:EPIS:KFKYE:AVS:SXGXR, HVPKPXX:VPT:EPIS:KFKYE:AVS:SXGXH, RVPSTXX:VPT:EPIS:KFKYE:AVS:XGXL, ASAAPXX:VPT:EPIS:KFKYE:AVS:XKXS, ASASPXX:VPT:EPIS:KFKYE:AVS:XKXS, NDEGLEX:VPT:EPIS:KFKYE:AVS:KXEXR, NDEGLEX:VPT:EPIS:KFKYE:AVS:QXEXR, SSVKXQP:VPT:EPIS:KFKYE:AVS:LEXAXA, RNVQXRP:VPT:EPIS:KFKYE:AVS:LAXKXE, KIPKAXX:VPT:EPIS:KFKYE:VVE:GXGXR, GIPEPXX:VPE:EPIS:KFKYE:TVE:SXAXR, SIPKAXX:VPT:EPIS:KFKYE:VVE:GXGXR, HVTKPTX:APT:EPIS:KFKYE:VVR:SXGXH, YVPKPXX:APT:EPIS:KFKYE:VVR:SXGXH, TVPKPXX:APT:EPIS:KFKYE:VVR:AXGXH, AVPKAXX:APT:EPIS:KFKYE:VVK:AXGXH, KVGKAXX:VPT:EPIS:KFKYE:VAE:XGXR, GSAGPXX:TPT:EPIS:KFKYE:VVD:RXGXS, AAPASXX:VPA:EPIS:KFKYE:VVE:AXGXR, STPPTXX:VPT:EPIS:KFKYE:VVE:SXGXR, HVPKPXX:APT:EPIS:KFKYE:VVR:SXGXH, RVPSTXX:APV:EPIS:KFKYE:VEE:XGXL, ASAAPXX:VPQ:EPIS:KFKYE:VRS:XKXS, ASASPXX:VSQ:EPIS:KFKYE:VKS:XKXS, ASASPXX:VPQ:EPIS:KFKYE:VKS:XKXS, NDEGLEX:VPT:EPIS:KFKYE:QHN:KXEXR, NDEGLEX:VPT:EPIS:KFKYE:EHS:QXEXR, SSVKXQP:SRV:EPIS:KFKYE:EEH:LEXAXA, RNVQXRP:TQV:EPIS:KFKYE:EDH:LAXKXE, KIPKAXX:TPT:SPIN:YGKIP:VVD:GXGXR, GIPEPXX:TPT:SPIN:YGKIP:VVD:SXAXR, SIPKAXX:TPT:SPIN:YGKIP:VVD:GXGXR, HVTKPTX:TPT:SPIN:YGKIP:VVD:SXGXH, YVPKPXX:TPT:SPIN:YGKIP:VVD:SXGXH, TVPKPXX:TPT:SPIN:YGKIP:VVD:AXGXH, AVPKAXX:TPT:SPIN:YGKIP:VVD:AXGXH, KVGKAXX:TPT:SPIN:YGKIP:VVD:XGXR, KASKAXX:TPT:SPIN:YGKIP:VVD:EXGXR, AAPASXX:TPT:SPIN:YGKIP:VVD:AXGXR, STPPTXX:TPT:SPIN:YGKIP:VVD:SXGXR, HVPKPXX:TPT:SPIN:YGKIP:VVD:SXGXH, RVPSTXX:TPT:SPIN:YGKIP:VVD:XGXL, ASAAPXX:TPT:SPIN:YGKIP:VVD:XKXS, ASASPXX:TPT:SPIN:YGKIP:VVD:XKXS, NDEGLEX:TPT:SPIN:YGKIP:VVD:KXEXR, NDEGLEX:TPT:SPIN:YGKIP:VVD:QXEXR, SSVKXQP:TPT:SPIN:YGKIP:VVD:LEXAXA, RNVQXRP:TPT:SPIN:YGKIP:VVD:LAXKXE, KIPKAXX:VPT:SPIN:YGKIP:VVE:GXGXR, GIPEPXX:VPE:SPIN:YGKIP:TVE:SXAXR, SIPKAXX:VPT:SPIN:YGKIP:VVE:GXGXR, HVTKPTX:APT:SPIN:YGKIP:VVR:SXGXH, YVPKPXX:APT:SPIN:YGKIP:VVR:SXGXH, TVPKPXX:APT:SPIN:YGKIP:VVR:AXGXH, AVPKAXX:APT:SPIN:YGKIP:VVK:AXGXH, KVGKAXX:VPT:SPIN:YGKIP:VAE:XGXR, KASKAXX:VPT:SPIN:YGKIP:AVS:EXGXR, AAPASXX:VPA:SPIN:YGKIP:VVE:AXGXR, STPPTXX:VPT:SPIN:YGKIP:VVE:SXGXR, HVPKPXX:APT:SPIN:YGKIP:VVR:SXGXH, RVPSTXX:APV:SPIN:YGKIP:VEE:XGXL, ASAAPXX:VPQ:SPIN:YGKIP:VRS:XKXS, ASASPXX:VSQ:SPIN:YGKIP:VKS:XKXS, ASASPXX:VPQ:SPIN:YGKIP:VKS:XKXS, NDEGLEX:VPT:SPIN:YGKIP:QHN:KXEXR, NDEGLEX:VPT:SPIN:YGKIP:EHS:QXEXR, SSVKXQP:SRV:SPIN:YGKIP:EEH:LEXAXA, RNVQXRP:TQV:SPIN:YGKIP:EDH:LAXKXE, KIPKAXX:VPA:SPIS:YKQYE:VVE:GXGXR, GIPEPXX:VPA:SPIS:YKQYE:VVE:SXAXR, SIPKAXX:VPA:SPIS:YKQYE:VVE:GXGXR, HVTKPTX:VPA:SPIS:YKQYE:VVE:SXGXH, YVPKPXX:VPA:SPIS:YKQYE:VVE:SXGXH, TVPKPXX:VPA:SPIS:YKQYE:VVE:AXGXH, AVPKAXX:VPA:SPIS:YKQYE:VVE:AXGXH, KVGKAXX:VPA:SPIS:YKQYE:VVE:XGXR, KASKAXX:VPA:SPIS:YKQYE:VVE:EXGXR, GSAGPXX:VPA:SPIS:YKQYE:VVE:RXGXS, STPPTXX:VPA:SPIS:YKQYE:VVE:SXGXR, HVPKPXX:VPA:SPIS:YKQYE:VVE:SXGXH, RVPSTXX:VPA:SPIS:YKQYE:VVE:XGXL, ASAAPXX:VPA:SPIS:YKQYE:VVE:XKXS, ASASPXX:VPA:SPIS:YKQYE:VVE:XKXS, NDEGLEX:VPA:SPIS:YKQYE:VVE:KXEXR, NDEGLEX:VPA:SPIS:YKQYE:VVE:QXEXR, SSVKXQP:VPA:SPIS:YKQYE:VVE:LEXAXA, RNVQXRP:VPA:SPIS:YKQYE:VVE:LAXKXE, KIPKAXX:VPT:SPIS:YKQYE:VVE:GXGXR, GIPEPXX:VPE:SPIS:YKQYE:TVE:SXAXR, SIPKAXX:VPT:SPIS:YKQYE:VVE:GXGXR, HVTKPTX:APT:SPIS:YKQYE:VVR:SXGXH, YVPKPXX:APT:SPIS:YKQYE:VVR:SXGXH, TVPKPXX:APT:SPIS:YKQYE:VVR:AXGXH, AVPKAXX:APT:SPIS:YKQYE:VVK:AXGXH, KVGKAXX:VPT:SPIS:YKQYE:VAE:XGXR, KASKAXX:VPT:SPIS:YKQYE:AVS:EXGXR, GSAGPXX:TPT:SPIS:YKQYE:VVD:RXGXS, STPPTXX:VPT:SPIS:YKQYE:VVE:SXGXR, HVPKPXX:APT:SPIS:YKQYE:VVR:SXGXH, RVPSTXX:APV:SPIS:YKQYE:VEE:XGXL, ASAAPXX:VPQ:SPIS:YKQYE:VRS:XKXS, ASASPXX:VSQ:SPIS:YKQYE:VKS:XKXS, ASASPXX:VPQ:SPIS:YKQYE:VKS:XKXS, NDEGLEX:VPT:SPIS:YKQYE:QHN:KXEXR, NDEGLEX:VPT:SPIS:YKQYE:EHS:QXEXR, SSVKXQP:SRV:SPIS:YKQYE:EEH:LEXAXA, RNVQXRP:TQV:SPIS:YKQYE:EDH:LAXKXE, GIPEPXX:VPT:SPIS:YKQYE:VVE:SXAXR, HVTKPTX:VPT:SPIS:YKQYE:VVE:SXGXH, YVPKPXX:VPT:SPIS:YKQYE:VVE:SXGXH, TVPKPXX:VPT:SPIS:YKQYE:VVE:AXGXH, AVPKAXX:VPT:SPIS:YKQYE:VVE:AXGXH, KVGKAXX:VPT:SPIS:YKQYE:VVE:XGXR, KASKAXX:VPT:SPIS:YKQYE:VVE:EXGXR, GSAGPXX:VPT:SPIS:YKQYE:VVE:RXGXS, AAPASXX:VPT:SPIS:YKQYE:VVE:AXGXR, HVPKPXX:VPT:SPIS:YKQYE:VVE:SXGXH, RVPSTXX:VPT:SPIS:YKQYE:VVE:XGXL, ASAAPXX:VPT:SPIS:YKQYE:VVE:XKXS, ASASPXX:VPT:SPIS:YKQYE:VVE:XKXS, NDEGLEX:VPT:SPIS:YKQYE:VVE:KXEXR, NDEGLEX:VPT:SPIS:YKQYE:VVE:QXEXR, SSVKXQP:VPT:SPIS:YKQYE:VVE:LEXAXA, RNVQXRP:VPT:SPIS:YKQYE:VVE:LAXKXE, AAPASXX:VPA:SPIS:YKQYE:VVE:AXGXR, KIPKAXX:APV:KPLS:DHHKD:VEE:GXGXR, GIPEPXX:APV:KPLS:DHHKD:VEE:SXAXR, SIPKAXX:APV:KPLS:DHHKD:VEE:GXGXR, HVTKPTX:APV:KPLS:DHHKD:VEE:SXGXH, YVPKPXX:APV:KPLS:DHHKD:VEE:SXGXH, TVPKPXX:APV:KPLS:DHHKD:VEE:AXGXH, AVPKAXX:APV:KPLS:DHHKD:VEE:AXGXH, KVGKAXX:APV:KPLS:DHHKD:VEE:XGXR, KASKAXX:APV:KPLS:DHHKD:VEE:EXGXR, GSAGPXX:APV:KPLS:DHHKD:VEE:RXGXS, AAPASXX:APV:KPLS:DHHKD:VEE:AXGXR, STPPTXX:APV:KPLS:DHHKD:VEE:SXGXR, HVPKPXX:APV:KPLS:DHHKD:VEE:SXGXH, ASAAPXX:APV:KPLS:DHHKD:VEE:XKXS, ASASPXX:APV:KPLS:DHHKD:VEE:XKXS, NDEGLEX:APV:KPLS:DHHKD:VEE:KXEXR, NDEGLEX:APV:KPLS:DHHKD:VEE:QXEXR, SSVKXQP:APV:KPLS:DHHKD:VEE:LEXAXA, RNVQXRP:APV:KPLS:DHHKD:VEE:LAXKXE, KIPKAXX:VPT:KPLS:DHHKD:VVE:GXGXR, GIPEPXX:VPE:KPLS:DHHKD:TVE:SXAXR, SIPKAXX:VPT:KPLS:DHHKD:VVE:GXGXR, HVTKPTX:APT:KPLS:DHHKD:VVR:SXGXH, YVPKPXX:APT:KPLS:DHHKD:VVR:SXGXH, TVPKPXX:APT:KPLS:DHHKD:VVR:AXGXH, AVPKAXX:APT:KPLS:DHHKD:VVK:AXGXH, KVGKAXX:VPT:KPLS:DHHKD:VAE:XGXR, KASKAXX:VPT:KPLS:DHHKD:AVS:EXGXR, GSAGPXX:TPT:KPLS:DHHKD:VVD:RXGXS, AAPASXX:VPA:KPLS:DHHKD:VVE:AXGXR, STPPTXX:VPT:KPLS:DHHKD:VVE:SXGXR, HVPKPXX:APT:KPLS:DHHKD:VVR:SXGXH, ASAAPXX:VPQ:KPLS:DHHKD:VRS:XKXS, ASASPXX:VSQ:KPLS:DHHKD:VKS:XKXS, ASASPXX:VPQ:KPLS:DHHKD:VKS:XKXS, NDEGLEX:VPT:KPLS:DHHKD:QHN:KXEXR, NDEGLEX:VPT:KPLS:DHHKD:EHS:QXEXR, SSVKXQP:SRV:KPLS:DHHKD:EEH:LEXAXA, RNVQXRP:TQV:KPLS:DHHKD:EDH:LAXKXE, KIPKAXX:VPQ:EPLP:EQLSN:VRS:GXGXR, GIPEPXX:VPQ:EPLP:EQLSN:VRS:SXAXR, SIPKAXX:VPQ:EPLP:EQLSN:VRS:GXGXR, HVTKPTX:VPQ:EPLP:EQLSN:VRS:SXGXH, YVPKPXX:VPQ:EPLP:EQLSN:VRS:SXGXH, TVPKPXX:VPQ:EPLP:EQLSN:VRS:AXGXH, AVPKAXX:VPQ:EPLP:EQLSN:VRS:AXGXH, KVGKAXX:VPQ:EPLP:EQLSN:VRS:XGXR, KASKAXX:VPQ:EPLP:EQLSN:VRS:EXGXR, GSAGPXX:VPQ:EPLP:EQLSN:VRS:RXGXS, AAPASXX:VPQ:EPLP:EQLSN:VRS:AXGXR, STPPTXX:VPQ:EPLP:EQLSN:VRS:SXGXR, HVPKPXX:VPQ:EPLP:EQLSN:VRS:SXGXH, RVPSTXX:VPQ:EPLP:EQLSN:VRS:XGXL, ASASPXX:VPQ:EPLP:EQLSN:VRS:XKXS, NDEGLEX:VPQ:EPLP:EQLSN:VRS:KXEXR, NDEGLEX:VPQ:EPLP:EQLSN:VRS:QXEXR, SSVKXQP:VPQ:EPLP:EQLSN:VRS:LEXAXA, RNVQXRP:VPQ:EPLP:EQLSN:VRS:LAXKXE, KIPKAXX:VPT:EPLP:EQLSN:VVE:GXGXR, GIPEPXX:VPE:EPLP:EQLSN:TVE:SXAXR, SIPKAXX:VPT:EPLP:EQLSN:VVE:GXGXR, HVTKPTX:APT:EPLP:EQLSN:VVR:SXGXH, YVPKPXX:APT:EPLP:EQLSN:VVR:SXGXH, TVPKPXX:APT:EPLP:EQLSN:VVR:AXGXH, AVPKAXX:APT:EPLP:EQLSN:VVK:AXGXH,

KVGKAXX:VPT:EPLP:EQLSN:VAE:XGXR, KASKAXX:VPT:EPLP:EQLSN:AVS:EXGXR, GSAGPXX:TPT:EPLP:EQLSN:VVD:RXGXS, AAPASXX:VPA:EPLP:EQLSN:VVE:AXGXR, STPPTXX:VPT:EPLP:EQLSN:VVE:SXGXR, HVPKPXX:APT:EPLP:EQLSN:VVR:SXGXH, RVPSTXX:APV:EPLP:EQLSN:VEE:XGXL, ASASPXX:VSQ:EPLP:EQLSN:VKS:XKXS, ASASPXX:VPQ:EPLP:EQLSN:VKS:XKXS, NDEGLEX:VPT:EPLP:EQLSN:QHN:KXEXR, NDEGLEX:VPT:EPLP:EQLSN:EHS:QXEXR, SSVKXQP:SRV:EPLP:EQLSN:EEH:LEXAXA, RNVQXRP:TQV:EPLP:EQLSN:EDH:LAXKXE, KIPKAXX:VSQ:EPLT:EQLSN:VKS:GXGXR, GIPEPXX:VSQ:EPLT:EQLSN:VKS:SXAXR, SIPKAXX:VSQ:EPLT:EQLSN:VKS:GXGXR, HVTKPTX:VSQ:EPLT:EQLSN:VKS:SXGXH, YVPKPXX:VSQ:EPLT:EQLSN:VKS:SXGXH, TVPKPXX:VSQ:EPLT:EQLSN:VKS:AXGXH, AVPKAXX:VSQ:EPLT:EQLSN:VKS:AXGXH, KVGKAXX:VSQ:EPLT:EQLSN:VKS:XGXR, KASKAXX:VSQ:EPLT:EQLSN:VKS:EXGXR, GSAGPXX:VSQ:EPLT:EQLSN:VKS:RXGXS, AAPASXX:VSQ:EPLT:EQLSN:VKS:AXGXR, STPPTXX:VSQ:EPLT:EQLSN:VKS:SXGXR, HVPKPXX:VSQ:EPLT:EQLSN:VKS:SXGXH, RVPSTXX:VSQ:EPLT:EQLSN:VKS:XGXL, ASAAPXX:VSQ:EPLT:EQLSN:VKS:XKXS, ASASPXX:VSQ:EPLT:EQLSN:VKS:XKXS, NDEGLEX:VSQ:EPLT:EQLSN:VKS:KXEXR, NDEGLEX:VSQ:EPLT:EQLSN:VKS:QXEXR, SSVKXQP:VSQ:EPLT:EQLSN:VKS:LEXAXA, RNVQXRP:VSQ:EPLT:EQLSN:VKS:LAXKXE, KIPKAXX:VPT:EPLT:EQLSN:VVE:GXGXR, GIPEPXX:VPE:EPLT:EQLSN:TVE:SXAXR, SIPKAXX:VPT:EPLT:EQLSN:VVE:GXGXR, HVTKPTX:APT:EPLT:EQLSN:VVR:SXGXH, YVPKPXX:APT:EPLT:EQLSN:VVR:SXGXH, TVPKPXX:APT:EPLT:EQLSN:VVR:AXGXH, AVPKAXX:APT:EPLT:EQLSN:VVK:AXGXH, KVGKAXX:VPT:EPLT:EQLSN:VAE:XGXR, KASKAXX:VPT:EPLT:EQLSN:AVS:EXGXR, GSAGPXX:TPT:EPLT:EQLSN:VVD:RXGXS, AAPASXX:VPA:EPLT:EQLSN:VVE:AXGXR, STPPTXX:VPT:EPLT:EQLSN:VVE:SXGXR, HVPKPXX:APT:EPLT:EQLSN:VVR:SXGXH, RVPSTXX:APV:EPLT:EQLSN:VEE:XGXL, ASAAPXX:VPQ:EPLT:EQLSN:VRS:XKXS, ASASPXX:VPQ:EPLT:EQLSN:VKS:XKXS, NDEGLEX:VPT:EPLT:EQLSN:QHN:KXEXR, NDEGLEX:VPT:EPLT:EQLSN:EHS:QXEXR, SSVKXQP:SRV:EPLT:EQLSN:EEH:LEXAXA, RNVQXRP:TQV:EPLT:EQLSN:EDH:LAXKXE, KIPKAXX:VPQ:EPLT:EQLSN:VKS:GXGXR, GIPEPXX:VPQ:EPLT:EQLSN:VKS:SXAXR, SIPKAXX:VPQ:EPLT:EQLSN:VKS:GXGXR, HVTKPTX:VPQ:EPLT:EQLSN:VKS:SXGXH, YVPKPXX:VPQ:EPLT:EQLSN:VKS:SXGXH, TVPKPXX:VPQ:EPLT:EQLSN:VKS:AXGXH, AVPKAXX:VPQ:EPLT:EQLSN:VKS:AXGXH, KVGKAXX:VPQ:EPLT:EQLSN:VKS:XGXR, KASKAXX:VPQ:EPLT:EQLSN:VKS:EXGXR, GSAGPXX:VPQ:EPLT:EQLSN:VKS:RXGXS, AAPASXX:VPQ:EPLT:EQLSN:VKS:AXGXR, STPPTXX:VPQ:EPLT:EQLSN:VKS:SXGXR, HVPKPXX:VPQ:EPLT:EQLSN:VKS:SXGXH, RVPSTXX:VPQ:EPLT:EQLSN:VKS:XGXL, ASAAPXX:VPQ:EPLT:EQLSN:VKS:XKXS, NDEGLEX:VPQ:EPLT:EQLSN:VKS:KXEXR, NDEGLEX:VPQ:EPLT:EQLSN:VKS:QXEXR, SSVKXQP:VPQ:EPLT:EQLSN:VKS:LEXAXA, RNVQXRP:VPQ:EPLT:EQLSN:VKS:LAXKXE, KIPKAXX:VPT:SNIT:IGEMS:QHN:GXGXR, GIPEPXX:VPT:SNIT:IGEMS:QHN:SXAXR, SIPKAXX:VPT:SNIT:IGEMS:QHN:GXGXR, HVTKPTX:VPT:SNIT:IGEMS:QHN:SXGXH, YVPKPXX:VPT:SNIT:IGEMS:QHN:SXGXH, TVPKPXX:VPT:SNIT:IGEMS:QHN:AXGXH, AVPKAXX:VPT:SNIT:IGEMS:QHN:AXGXH, KVGKAXX:VPT:SNIT:IGEMS:QHN:XGXR, KASKAXX:VPT:SNIT:IGEMS:QHN:EXGXR, GSAGPXX:VPT:SNIT:IGEMS:QHN:RXGXS, AAPASXX:VPT:SNIT:IGEMS:QHN:AXGXR, STPPTXX:VPT:SNIT:IGEMS:QHN:SXGXR, HVPKPXX:VPT:SNIT:IGEMS:QHN:SXGXH, RVPSTXX:VPT:SNIT:IGEMS:QHN:XGXL, ASAAPXX:VPT:SNIT:IGEMS:QHN:XKXS, ASASPXX:VPT:SNIT:IGEMS:QHN:XKXS, NDEGLEX:VPT:SNIT:IGEMS:QHN:QXEXR, NDEGLEX:VPT:SNIT:IGEMS:QHN:KXEXR, SSVKXQP:VPT:SNIT:IGEMS:QHN:LEXAXA, RNVQXRP:VPT:SNIT:IGEMS:QHN:LAXKXE, KIPKAXX:VPT:SNIT:IGEMS:VVE:GXGXR, GIPEPXX:VPE:SNIT:IGEMS:TVE:SXAXR, SIPKAXX:VPT:SNIT:IGEMS:VVE:GXGXR, HVTKPTX:APT:SNIT:IGEMS:VVR:SXGXH, YVPKPXX:APT:SNIT:IGEMS:VVR:SXGXH, TVPKPXX:APT:SNIT:IGEMS:VVR:AXGXH, AVPKAXX:APT:SNIT:IGEMS:VVK:AXGXH, KVGKAXX:VPT:SNIT:IGEMS:VAE:XGXR, KASKAXX:VPT:SNIT:IGEMS:AVS:EXGXR, GSAGPXX:TPT:SNIT:IGEMS:VVD:RXGXS, AAPASXX:VPA:SNIT:IGEMS:VVE:AXGXR, STPPTXX:VPT:SNIT:IGEMS:VVE:SXGXR, HVPKPXX:APT:SNIT:IGEMS:VVR:SXGXH, RVPSTXX:APV:SNIT:IGEMS:VEE:XGXL, ASAAPXX:VPQ:SNIT:IGEMS:VRS:XKXS, ASASPXX:VSQ:SNIT:IGEMS:VKS:XKXS, ASASPXX:VPQ:SNIT:IGEMS:VKS:XKXS, NDEGLEX:VPT:SNIT:IGEMS:EHS:QXEXR, SSVKXQP:SRV:SNIT:IGEMS:EEH:LEXAXA, RNVQXRP:TQV:SNIT:IGEMS:EDH:LAXKXE, KIPKAXX:VPT:SNIT:LGEMS:EHS:GXGXR, GIPEPXX:VPT:SNIT:LGEMS:EHS:SXAXR, SIPKAXX:VPT:SNIT:LGEMS:EHS:GXGXR, HVTKPTX:VPT:SNIT:LGEMS:EHS:SXGXH, YVPKPXX:VPT:SNIT:LGEMS:EHS:SXGXH, TVPKPXX:VPT:SNIT:LGEMS:EHS:AXGXH, AVPKAXX:VPT:SNIT:LGEMS:EHS:AXGXH, KVGKAXX:VPT:SNIT:LGEMS:EHS:XGXR, KASKAXX:VPT:SNIT:LGEMS:EHS:EXGXR, GSAGPXX:VPT:SNIT:LGEMS:EHS:RXGXS, AAPASXX:VPT:SNIT:LGEMS:EHS:AXGXR, STPPTXX:VPT:SNIT:LGEMS:EHS:SXGXR, HVPKPXX:VPT:SNIT:LGEMS:EHS:SXGXH, RVPSTXX:VPT:SNIT:LGEMS:EHS:XGXL, ASAAPXX:VPT:SNIT:LGEMS:EHS:XKXS, ASASPXX:VPT:SNIT:LGEMS:EHS:XKXS, NDEGLEX:VPT:SNIT:LGEMS:EHS:KXEXR, SSVKXQP:VPT:SNIT:LGEMS:EHS:LEXAXA,

RNVQXRP:VPT:SNIT:LGEMS:EHS:LAXKXE, KIPKAXX:VPT:SNIT:LGEMS:VVE:GXGXR, GIPEPXX:VPE:SNIT:LGEMS:TVE:SXAXR, SIPKAXX:VPT:SNIT:LGEMS:VVE:GXGXR, HVTKPTX:APT:SNIT:LGEMS:VVR:SXGXH, YVPKPXX:APT:SNIT:LGEMS:VVR:SXGXH, TVPKPXX:APT:SNIT:LGEMS:VVR:AXGXH, AVPKAXX:APT:SNIT:LGEMS:VVK:AXGXH, KVGKAXX:VPT:SNIT:LGEMS:VAE:XGXR, KASKAXX:VPT:SNIT:LGEMS:AVS:EXGXR, GSAGPXX:TPT:SNIT:LGEMS:VVD:RXGXS, AAPASXX:VPA:SNIT:LGEMS:VVE:AXGXR, STPPTXX:VPT:SNIT:LGEMS:VVE:SXGXR, HVPKPXX:APT:SNIT:LGEMS:VVR:SXGXH, RVPSTXX:APV:SNIT:LGEMS:VEE:XGXL, ASAAPXX:VPQ:SNIT:LGEMS:VRS:XKXS, ASASPXX:VSQ:SNIT:LGEMS:VKS:XKXS, ASASPXX:VPQ:SNIT:LGEMS:VKS:XKXS, NDEGLEX:VPT:SNIT:LGEMS:QHN:KXEXR, SSVKXQP:SRV:SNIT:LGEMS:EEH:LEXAXA, RNVQXRP:TQV:SNIT:LGEMS:EDH:LAXKXE, KIPKAXX:SRV:RSVK:KEVQV:EEH:GXGXR, GIPEPXX:SRV:RSVK:KEVQV:EEH:SXAXR, SIPKAXX:SRV:RSVK:KEVQV:EEH:GXGXR, HVTKPTX:SRV:RSVK:KEVQV:EEH:SXGXH, YVPKPXX:SRV:RSVK:KEVQV:EEH:SXGXH, TVPKPXX:SRV:RSVK:KEVQV:EEH:AXGXH, AVPKAXX:SRV:RSVK:KEVQV:EEH:AXGXH, KVGKAXX:SRV:RSVK:KEVQV:EEH:XGXR, KASKAXX:SRV:RSVK:KEVQV:EEH:EXGXR, GSAGPXX:SRV:RSVK:KEVQV:EEH:RXGXS, AAPASXX:SRV:RSVK:KEVQV:EEH:AXGXR, STPPTXX:SRV:RSVK:KEVQV:EEH:SXGXR, HVPKPXX:SRV:RSVK:KEVQV:EEH:SXGXH, RVPSTXX:SRV:RSVK:KEVQV:EEH:XGXL, ASAAPXX:SRV:RSVK:KEVQV:EEH:XKXS, ASASPXX:SRV:RSVK:KEVQV:EEH:XKXS, NDEGLEX:SRV:RSVK:KEVQV:EEH:KXEXR, NDEGLEX:SRV:RSVK:KEVQV:EEH:QXEXR, RNVQXRP:SRV:RSVK:KEVQV:EEH:LAXKXE, KIPKAXX:VPT:RSVK:KEVQV:VVE:GXGXR, GIPEPXX:VPE:RSVK:KEVQV:TVE:SXAXR, SIPKAXX:VPT:RSVK:KEVQV:VVE:GXGXR, HVTKPTX:APT:RSVK:KEVQV:VVR:SXGXH, YVPKPXX:APT:RSVK:KEVQV:VVR:SXGXH, TVPKPXX:APT:RSVK:KEVQV:VVR:AXGXH, AVPKAXX:APT:RSVK:KEVQV:VVK:AXGXH, KVGKAXX:VPT:RSVK:KEVQV:VAE:XGXR, KASKAXX:VPT:RSVK:KEVQV:AVS:EXGXR, GSAGPXX:TPT:RSVK:KEVQV:VVD:RXGXS, AAPASXX:VPA:RSVK:KEVQV:VVE:AXGXR, STPPTXX:VPT:RSVK:KEVQV:VVE:SXGXR, HVPKPXX:APT:RSVK:KEVQV:VVR:SXGXH, RVPSTXX:APV:RSVK:KEVQV:VEE:XGXL, ASAAPXX:VPQ:RSVK:KEVQV:VRS:XKXS, ASASPXX:VSQ:RSVK:KEVQV:VKS:XKXS, ASASPXX:VPQ:RSVK:KEVQV:VKS:XKXS, NDEGLEX:VPT:RSVK:KEVQV:QHN:KXEXR, NDEGLEX:VPT:RSVK:KEVQV:EHS:QXEXR, RNVQXRP:TQV:RSVK:KEVQV:EDH:LAXKXE, KIPKAXX:TQV:RPVQ:KKATV:EDH:GXGXR, GIPEPXX:TQV:RPVQ:KKATV:EDH:SXAXR, SIPKAXX:TQV:RPVQ:KKATV:EDH:GXGXR, HVTKPTX:TQV:RPVQ:KKATV:EDH:SXGXH, YVPKPXX:TQV:RPVQ:KKATV:EDH:SXGXH, TVPKPXX:TQV:RPVQ:KKATV:EDH:AXGXH, AVPKAXX:TQV:RPVQ:KKATV:EDH:AXGXH, KVGKAXX:TQV:RPVQ:KKATV:EDH:XGXR, KASKAXX:TQV:RPVQ:KKATV:EDH:EXGXR, GSAGPXX:TQV:RPVQ:KKATV:EDH:RXGXS, AAPASXX:TQV:RPVQ:KKATV:EDH:AXGXR, STPPTXX:TQV:RPVQ:KKATV:EDH:SXGXR, HVPKPXX:TQV:RPVQ:KKATV:EDH:SXGXH, RVPSTXX:TQV:RPVQ:KKATV:EDH:XGXL, ASAAPXX:TQV:RPVQ:KKATV:EDH:XKXS, ASASPXX:TQV:RPVQ:KKATV:EDH:XKXS, NDEGLEX:TQV:RPVQ:KKATV:EDH:KXEXR, NDEGLEX:TQV:RPVQ:KKATV:EDH:QXEXR, SSVKXQP:TQV:RPVQ:KKATV:EDH:LEXAXA, KIPKAXX:VPT:RPVQ:KKATV:VVE:GXGXR, GIPEPXX:VPE:RPVQ:KKATV:TVE:SXAXR, SIPKAXX:VPT:RPVQ:KKATV:VVE:GXGXR, HVTKPTX:APT:RPVQ:KKATV:VVR:SXGXH, YVPKPXX:APT:RPVQ:KKATV:VVR:SXGXH, TVPKPXX:APT:RPVQ:KKATV:VVR:AXGXH, AVPKAXX:APT:RPVQ:KKATV:VVK:AXGXH, KVGKAXX:VPT:RPVQ:KKATV:VAE:XGXR, KASKAXX:VPT:RPVQ:KKATV:AVS:EXGXR, GSAGPXX:TPT:RPVQ:KKATV:VVD:RXGXS, AAPASXX:VPA:RPVQ:KKATV:VVE:AXGXR, STPPTXX:VPT:RPVQ:KKATV:VVE:SXGXR, HVPKPXX:APT:RPVQ:KKATV:VVR:SXGXH, RVPSTXX:APV:RPVQ:KKATV:VEE:XGXL, ASAAPXX:VPQ:RPVQ:KKATV:VRS:XKXS, ASASPXX:VSQ:RPVQ:KKATV:VKS:XKXS, ASASPXX:VPQ:RPVQ:KKATV:VKS:XKXS, NDEGLEX:VPT:RPVQ:KKATV:QHN:KXEXR, NDEGLEX:VPT:RPVQ:KKATV:EHS:QXEXR and SSVKXQP:SRV:RPVQ:KKATV:EEH:LEXAXA. More particularly, the hexaplet PEP7:PEP3:PEP1:PEP2:PEP4:PEP8 is selected from the group consisting of GIPEPXX:VPT:SAIS:LKNYQ:VVE:SXAXR, HVTKPTX:VPT:SAIS:LKNYQ:VVE:SXGXH, YVPKPXX:VPT:SAIS:LKNYQ:VVE:SXGXH, TVPKPXX:VPT:SAIS:LKNYQ:VVE:AXGXH, AVPKAXX:VPT:SAIS:LKNYQ:VVE:AXGXH, KVGKAXX:VPT:SAIS:LKNYQ:VVE:XGXR, KASKAXX:VPT:SAIS:LKNYQ:VVE:EXGXR, GSAGPXX:VPT:SAIS:LKNYQ:VVE:RXGXS, AAPASXX:VPT:SAIS:LKNYQ:VVE:AXGXR, STPPTXX:VPT:SAIS:LKNYQ:VVE:SXGXR, HVPKPXX:VPT:SAIS:LKNYQ:VVE:SXGXH, RVPSTXX:VPT:SAIS:LKNYQ:VVE:XGXL, ASAAPXX:VPT:SAIS:LKNYQ:VVE:XKXS, ASASPXX:VPT:SAIS:LKNYQ:VVE:XKXS, GIPEPXX:VPE:SAIS:LKNYQ:TVE:SXAXR, HVTKPTX:APT:SAIS:LKNYQ:VVR:SXGXH, YVPKPXX:APT:SAIS:LKNYQ:VVR:SXGXH, TVPKPXX:APT:SAIS:LKNYQ:VVR:AXGXH, AVPKAXX:APT:SAIS:LKNYQ:VVK:AXGXH, KVGKAXX:VPT:SAIS:LKNYQ:VAE:XGXR, KASKAXX:VPT:SAIS:LKNYQ:AVS:EXGXR, GSAGPXX:TPT:SAIS:LKNYQ:VVD:RXGXS, AAPASXX:VPA:SAIS:LKNYQ:VVE:AXGXR, HVPKPXX:APT:SAIS:LKNYQ:VVR:SXGXH, RVPSTXX:APV:SAIS:LKNYQ:VEE:XGXL, ASAAPXX:VPQ:SAIS:LKNYQ:VRS:XKXS, ASASPXX:VSQ:SAIS:LKNYQ:VKS:XKXS, ASASPXX:VPQ:SAIS:LKNYQ:VKS:XKXS, NDEGLEX:VPT:SAIS:LKNYQ:QHN:KXEXR, NDEGLEX:VPT:SAIS:LKNYQ:EHS:QXEXR, SSVKXQP:SRV:SAIS:LKNYQ:EEH:LEXAXA, RNVQXRP:TQV:SAIS:LKNYQ:EDH:LAXKXE, KIPKAXX:VPE:SSLS:LKVYP:TVE:GXGXR, SIPKAXX:VPE:SSLS:LKVYP:TVE:GXGXR, HVTKPTX:VPE:SSLS:LKVYP:TVE:SXGXH, YVPKPXX:VPE:SSLS:LKVYP:TVE:SXGXH, TVPKPXX:VPE:SSLS:LKVYP:TVE:AXGXH, AVPKAXX:VPE:SSLS:LKVYP:TVE:AXGXH, KVGKAXX:VPE:SSLS:LKVYP:TVE:XGXR, KASKAXX:VPE:SSLS:LKVYP:TVE:EXGXR, GSAGPXX:VPE:SSLS:LKVYP:TVE:RXGXS, AAPASXX:VPE:SSLS:LKVYP:TVE:AXGXR, STPPTXX:VPE:SSLS:LKVYP:TVE:SXGXR, HVPKPXX:VPE:SSLS:LKVYP:TVE:SXGXH, RVPSTXX:VPE:SSLS:LKVYP:TVE:XGXL, ASAAPXX:VPE:SSLS:LKVYP:TVE:XKXS, ASASPXX:VPE:SSLS:LKVYP:TVE:XKXS, KIPKAXX:VPT:SSLS:LKVYP:VVE:GXGXR, SIPKAXX:VPT:SSLS:LKVYP:VVE:GXGXR, HVTKPTX:APT:SSLS:LKVYP:VVR:SXGXH, YVPKPXX:APT:SSLS:LKVYP:VVR:SXGXH, TVPKPXX:APT:SSLS:LKVYP:VVR:AXGXH, AVPKAXX:APT:SSLS:LKVYP:VVK:AXGXH, KVGKAXX:VPT:SSLS:LKVYP:VAE:XGXR, KASKAXX:VPT:SSLS:LKVYP:AVS:EXGXR, GSAGPXX:TPT:SSLS:LKVYP:VVD:RXGXS, AAPASXX:VPA:SSLS:LKVYP:VVE:AXGXR, STPPTXX:VPT:SSLS:LKVYP:VVE:SXGXR, HVPKPXX:APT:SSLS:LKVYP:VVR:SXGXH, RVPSTXX:APV:SSLS:LKVYP:VEE:XGXL, ASAAPXX:VPQ:SSLS:LKVYP:VRS:XKXS, ASASPXX:VSQ:SSLS:LKVYP:VKS:XKXS, ASASPXX:VPQ:SSLS:LKVYP:VKS:XKXS, NDEGLEX:VPT:SSLS:LKVYP:QHN:KXEXR, NDEGLEX:VPT:SSLS:LKVYP:EHS:QXEXR, SSVKXQP:SRV:SSLS:LKVYP:EEH:LEXAXA, RNVQXRP:TQV:SSLS:LKVYP:EDH:LAXKXE, KIPKAXX:APT:NAIS:LKKYR:VVR:GXGXR, GIPEPXX:APT:NAIS:LKKYR:VVR:SXAXR, SIPKAXX:APT:NAIS:LKKYR:VVR:GXGXR, AVPKAXX:APT:NAIS:LKKYR:VVR:AXGXH, KVGKAXX:APT:NAIS:LKKYR:VVR:XGXR, KASKAXX:APT:NAIS:LKKYR:VVR:EXGXR, GSAGPXX:APT:NAIS:LKKYR:VVR:RXGXS, AAPASXX:APT:NAIS:LKKYR:VVR:AXGXR, STPPTXX:APT:NAIS:LKKYR:VVR:SXGXR, RVPSTXX:APT:NAIS:LKKYR:VVR:XGXL, ASAAPXX:APT:NAIS:LKKYR:VVR:XKXS, ASASPXX:APT:NAIS:LKKYR:VVR:XKXS, KIPKAXX:VPT:NAIS:LKKYR:VVE:GXGXR, GIPEPXX:VPE:NAIS:LKKYR:TVE:SXAXR, SIPKAXX:VPT:NAIS:LKKYR:VVE:GXGXR, AVPKAXX:APT:NAIS:LKKYR:VVK:AXGXH, KVGKAXX:VPT:NAIS:LKKYR:VAE:XGXR, KASKAXX:VPT:NAIS:LKKYR:AVS:EXGXR, GSAGPXX:TPT:NAIS:LKKYR:VVD:RXGXS, AAPASXX:VPA:NAIS:LKKYR:VVE:AXGXR, STPPTXX:VPT:NAIS:LKKYR:VVE:SXGXR, RVPSTXX:APV:NAIS:LKKYR:VEE:XGXL, ASAAPXX:VPQ:NAIS:LKKYR:VRS:XKXS, ASASPXX:VSQ:NAIS:LKKYR:VKS:XKXS, ASASPXX:VPQ:NAIS:LKKYR:VKS:XKXS, NDEGLEX:VPT:NAIS:LKKYR:QHN:KXEXR, NDEGLEX:VPT:NAIS:LKKYR:EHS:QXEXR, SSVKXQP:SRV:NAIS:LKKYR:EEH:LEXAXA, RNVQXRP:TQV:NAIS:LKKYR:EDH:LAXKXE, KIPKAXX:APT:SATS:LRKHR:VVK:GXGXR, GIPEPXX:APT:SATS:LRKHR:VVK:SXAXR, SIPKAXX:APT:SATS:LRKHR:VVK:GXGXR, HVTKPTX:APT:SATS:LRKHR:VVK:SXGXH, YVPKPXX:APT:SATS:LRKHR:VVK:SXGXH, TVPKPXX:APT:SATS:LRKHR:VVK:AXGXH, KVGKAXX:APT:SATS:LRKHR:VVK:XGXR, KASKAXX:APT:SATS:LRKHR:VVK:EXGXR, GSAGPXX:APT:SATS:LRKHR:VVK:RXGXS, AAPASXX:APT:SATS:LRKHR:VVK:AXGXR, STPPTXX:APT:SATS:LRKHR:VVK:SXGXR, HVPKPXX:APT:SATS:LRKHR:VVK:SXGXH, RVPSTXX:APT:SATS:LRKHR:VVK:XGXL, ASAAPXX:APT:SATS:LRKHR:VVK:XKXS, ASASPXX:APT:SATS:LRKHR:VVK:XKXS, KIPKAXX:VPT:SATS:LRKHR:VVE:GXGXR, GIPEPXX:VPE:SATS:LRKHR:TVE:SXAXR, SIPKAXX:VPT:SATS:LRKHR:VVE:GXGXR, HVTKPTX:APT:SATS:LRKHR:VVR:SXGXH, YVPKPXX:APT:SATS:LRKHR:VVR:SXGXH, TVPKPXX:APT:SATS:LRKHR:VVR:AXGXH, KVGKAXX:VPT:SATS:LRKHR:VAE:XGXR, KASKAXX:VPT:SATS:LRKHR:AVS:EXGXR, GSAGPXX:TPT:SATS:LRKHR:VVD:RXGXS, AAPASXX:VPA:SATS:LRKHR:VVE:AXGXR, STPPTXX:VPT:SATS:LRKHR:VVE:SXGXR, HVPKPXX:APT:SATS:LRKHR:VVR:SXGXH, RVPSTXX:APV:SATS:LRKHR:VEE:XGXL, ASAAPXX:VPQ:SATS:LRKHR:VRS:XKXS, ASASPXX:VSQ:SATS:LRKHR:VKS:XKXS, ASASPXX:VPQ:SATS:LRKHR:VKS:XKXS, NDEGLEX:VPT:SATS:LRKHR:QHN:KXEXR, NDEGLEX:VPT:SATS:LRKHR:EHS:QXEXR, SSVKXQP:SRV:SATS:LRKHR:EEH:LEXAXA, RNVQXRP:TQV:SATS:LRKHR:EDH:LAXKXE, KIPKAXX:VPT:SPIS:LKYHY:VAE:GXGXR, GIPEPXX:VPT:SPIS:LKYHY:VAE:SXAXR, SIPKAXX:VPT:SPIS:LKYHY:VAE:GXGXR, HVTKPTX:VPT:SPIS:LKYHY:VAE:SXGXH, YVPKPXX:VPT:SPIS:LKYHY:VAE:SXGXH, TVPKPXX:VPT:SPIS:LKYHY:VAE:AXGXH, AVPKAXX:VPT:SPIS:LKYHY:VAE:AXGXH, KASKAXX:VPT:SPIS:LKYHY:VAE:EXGXR, GSAGPXX:VPT:SPIS:LKYHY:VAE:RXGXS, AAPASXX:VPT:SPIS:LKYHY:VAE:AXGXR, STPPTXX:VPT:SPIS:LKYHY:VAE:SXGXR, HVPKPXX:VPT:SPIS:LKYHY:VAE:SXGXH, RVPSTXX:VPT:SPIS:LKYHY:VAE:XGXL, ASAAPXX:VPT:SPIS:LKYHY:VAE:XKXS, ASASPXX:VPT:SPIS:LKYHY:VAE:XKXS, KIPKAXX:VPT:SPIS:LKYHY:VVE:GXGXR, GIPEPXX:VPE:SPIS:LKYHY:TVE:SXAXR, SIPKAXX:VPT:SPIS:LKYHY:VVE:GXGXR, HVTKPTX:APT:SPIS:LKYHY:VVR:SXGXH, YVPKPXX:APT:SPIS:LKYHY:VVR:SXGXH, TVPKPXX:APT:SPIS:LKYHY:VVR:AXGXH, AVPKAXX:APT:SPIS:LKYHY:VVK:AXGXH, KASKAXX:VPT:SPIS:LKYHY:AVS:EXGXR, GSAGPXX:TPT:SPIS:LKYHY:VVD:RXGXS, AAPASXX:VPA:SPIS:LKYHY:VVE:AXGXR, STPPTXX:VPT:SPIS:LKYHY:VVE:SXGXR, HVPKPXX:APT:SPIS:LKYHY:VVR:SXGXH, RVPSTXX:APV:SPIS:LKYHY:VEE:XGXL, ASAAPXX:VPQ:SPIS:LKYHY:VRS:XKXS, ASASPXX:VSQ:SPIS:LKYHY:VKS:XKXS, ASASPXX:VPQ:SPIS:LKYHY:VKS:XKXS, NDEGLEX:VPT:SPIS:LKYHY:QHN:KXEXR, NDEGLEX:VPT:SPIS:LKYHY:EHS:QXEXR, SSVKXQP:SRV:SPIS:LKYHY:EEH:LEXAXA, RNVQXRP:TQV:SPIS:LKYHY:EDH:LAXKXE, KIPKAXX:VPT:EPIS:KFKYE:AVS:GXGXR, GIPEPXX:VPT:EPIS:KFKYE:AVS:SXAXR, SIPKAXX:VPT:EPIS:KFKYE:AVS:GXGXR, HVTKPTX:VPT:EPIS:KFKYE:AVS:SXGXH, YVPKPXX:VPT:EPIS:KFKYE:AVS:SXGXH, TVPKPXX:VPT:EPIS:KFKYE:AVS:AXGXH, AVPKAXX:VPT:EPIS:KFKYE:AVS:AXGXH, KVGKAXX:VPT:EPIS:KFKYE:AVS:XGXR, GSAGPXX:VPT:EPIS:KFKYE:AVS:RXGXS, AAPASXX:VPT:EPIS:KFKYE:AVS:AXGXR, STPPTXX:VPT:EPIS:KFKYE:AVS:SXGXR, HVPKPXX:VPT:EPIS:KFKYE:AVS:SXGXH, RVPSTXX:VPT:EPIS:KFKYE:AVS:XGXL, ASAAPXX:VPT:EPIS:KFKYE:AVS:XKXS, ASASPXX:VPT:EPIS:KFKYE:AVS:XKXS, KIPKAXX:VPT:EPIS:KFKYE:VVE:GXGXR, GIPEPXX:VPE:EPIS:KFKYE:TVE:SXAXR, SIPKAXX:VPT:EPIS:KFKYE:VVE:GXGXR, HVTKPTX:APT:EPIS:KFKYE:VVR:SXGXH, YVPKPXX:APT:EPIS:KFKYE:VVR:SXGXH, TVPKPXX:APT:EPIS:KFKYE:VVR:AXGXH, AVPKAXX:APT:EPIS:KFKYE:VVK:AXGXH, KVGKAXX:VPT:EPIS:KFKYE:VAE:XGXR, GSAGPXX:TPT:EPIS:KFKYE:VVD:RXGXS, AAPASXX:VPA:EPIS:KFKYE:VVE:AXGXR, STPPTXX:VPT:EPIS:KFKYE:VVE:SXGXR, HVPKPXX:APT:EPIS:KFKYE:VVR:SXGXH, RVPSTXX:APV:EPIS:KFKYE:VEE:XGXL, ASAAPXX:VPQ:EPIS:KFKYE:VRS:XKXS, ASASPXX:VSQ:EPIS:KFKYE:VKS:XKXS, ASASPXX:VPQ:EPIS:KFKYE:VKS:XKXS, NDEGLEX:VPT:EPIS:KFKYE:QHN:KXEXR, NDEGLEX:VPT:EPIS:KFKYE:EHS:QXEXR, SSVKXQP:SRV:EPIS:KFKYE:EEH:LEXAXA, RNVQXRP:TQV:EPIS:KFKYE:EDH:LAXKXE, KIPKAXX:TPT:SPIN:YGKIP:VVD:GXGXR, GIPEPXX:TPT:SPIN:YGKIP:VVD:SXAXR, SIPKAXX:TPT:SPIN:YGKIP:VVD:GXGXR, HVTKPTX:TPT:SPIN:YGKIP:VVD:SXGXH, YVPKPXX:TPT:SPIN:YGKIP:VVD:SXGXH, TVPKPXX:TPT:SPIN:YGKIP:VVD:AXGXH, AVPKAXX:TPT:SPIN:YGKIP:VVD:AXGXH, KVGKAXX:TPT:SPIN:YGKIP:VVD:XGXR, KASKAXX:TPT:SPIN:YGKIP:VVD:EXGXR, AAPASXX:TPT:SPIN:YGKIP:VVD:AXGXR, STPPTXX:TPT:SPIN:YGKIP:VVD:SXGXR, HVPKPXX:TPT:SPIN:YGKIP:VVD:SXGXH, RVPSTXX:TPT:SPIN:YGKIP:VVD:XGXL, ASAAPXX:TPT:SPIN:YGKIP:VVD:XKXS, ASASPXX:TPT:SPIN:YGKIP:VVD:XKXS, KIPKAXX:VPT:SPIN:YGKIP:VVE:GXGXR, GIPEPXX:VPE:SPIN:YGKIP:TVE:SXAXR, SIPKAXX:VPT:SPIN:YGKIP:VVE:GXGXR, HVTKPTX:APT:SPIN:YGKIP:VVR:SXGXH, YVPKPXX:APT:SPIN:YGKIP:VVR:SXGXH, TVPKPXX:APT:SPIN:YGKIP:VVR:AXGXH, AVPKAXX:APT:SPIN:YGKIP:VVK:AXGXH, KVGKAXX:VPT:SPIN:YGKIP:VAE:XGXR, KASKAXX:VPT:SPIN:YGKIP:AVS:EXGXR, AAPASXX:VPA:SPIN:YGKIP:VVE:AXGXR, STPPTXX:VPT:SPIN:YGKIP:VVE:SXGXR, HVPKPXX:APT:SPIN:YGKIP:VVR:SXGXH, RVPSTXX:APV:SPIN:YGKIP:VEE:XGXL, ASAAPXX:VPQ:SPIN:YGKIP:VRS:XKXS, ASASPXX:VSQ:SPIN:YGKIP:VKS:XKXS, ASASPXX:VPQ:SPIN:YGKIP:VKS:XKXS, NDEGLEX:VPT:SPIN:YGKIP:QHN:KXEXR, NDEGLEX:VPT:SPIN:YGKIP:EHS:QXEXR, SSVKXQP:SRV:SPIN:YGKIP:EEH:LEXAXA, RNVQXRP:TQV:SPIN:YGKIP:EDH:LAXKXE, KIPKAXX:VPA:SPIS:YKQYE:VVE:GXGXR, GIPEPXX:VPA:SPIS:YKQYE:VVE:SXAXR, SIPKAXX:VPA:SPIS:YKQYE:VVE:GXGXR, HVTKPTX:VPA:SPIS:YKQYE:VVE:SXGXH, YVPKPXX:VPA:SPIS:YKQYE:VVE:SXGXH, TVPKPXX:VPA:SPIS:YKQYE:VVE:AXGXH, AVPKAXX:VPA:SPIS:YKQYE:VVE:AXGXH, KVGKAXX:VPA:SPIS:YKQYE:VVE:XGXR, KASKAXX:VPA:SPIS:YKQYE:VVE:EXGXR, GSAGPXX:VPA:SPIS:YKQYE:VVE:RXGXS, STPPTXX:VPA:SPIS:YKQYE:VVE:SXGXR, HVPKPXX:VPA:SPIS:YKQYE:VVE:SXGXH, RVPSTXX:VPA:SPIS:YKQYE:VVE:XGXL, ASAAPXX:VPA:SPIS:YKQYE:VVE:XKXS, ASASPXX:VPA:SPIS:YKQYE:VVE:XKXS, KIPKAXX:VPT:SPIS:YKQYE:VVE:GXGXR, GIPEPXX:VPE:SPIS:YKQYE:TVE:SXAXR, SIPKAXX:VPT:SPIS:YKQYE:VVE:GXGXR, HVTKPTX:APT:SPIS:YKQYE:VVR:SXGXH, YVPKPXX:APT:SPIS:YKQYE:VVR:SXGXH, TVPKPXX:APT:SPIS:YKQYE:VVR:AXGXH, AVPKAXX:APT:SPIS:YKQYE:VVK:AXGXH, KVGKAXX:VPT:SPIS:YKQYE:VAE:XGXR, KASKAXX:VPT:SPIS:YKQYE:AVS:EXGXR, GSAGPXX:TPT:SPIS:YKQYE:VVD:RXGXS, STPPTXX:VPT:SPIS:YKQYE:VVE:SXGXR, HVPKPXX:APT:SPIS:YKQYE:VVR:SXGXH, RVPSTXX:APV:SPIS:YKQYE:VEE:XGXL, ASAAPXX:VPQ:SPIS:YKQYE:VRS:XKXS, ASASPXX:VSQ:SPIS:YKQYE:VKS:XKXS, ASASPXX:VPQ:SPIS:YKQYE:VKS:XKXS, NDEGLEX:VPT:SPIS:YKQYE:QHN:KXEXR, NDEGLEX:VPT:SPIS:YKQYE:EHS:QXEXR, SSVKXQP:SRV:SPIS:YKQYE:EEH:LEXAXA, RNVQXRP:TQV:SPIS:YKQYE:EDH:LAXKXE, GIPEPXX:VPT:SPIS:YKQYE:VVE:SXAXR, HVTKPTX:VPT:SPIS:YKQYE:VVE:SXGXH, YVPKPXX:VPT:SPIS:YKQYE:VVE:SXGXH, TVPKPXX:VPT:SPIS:YKQYE:VVE:AXGXH, AVPKAXX:VPT:SPIS:YKQYE:VVE:AXGXH, KVGKAXX:VPT:SPIS:YKQYE:VVE:XGXR, KASKAXX:VPT:SPIS:YKQYE:VVE:EXGXR, GSAGPXX:VPT:SPIS:YKQYE:VVE:RXGXS, AAPASXX:VPT:SPIS:YKQYE:VVE:AXGXR, HVPKPXX:VPT:SPIS:YKQYE:VVE:SXGXH, RVPSTXX:VPT:SPIS:YKQYE:VVE:XGXL, ASAAPXX:VPT:SPIS:YKQYE:VVE:XKXS, ASASPXX:VPT:SPIS:YKQYE:VVE:XKXS, AAPASXX:VPA:SPIS:YKQYE:VVE:AXGXR, KIPKAXX:APV:KPLS:DHHKD:VEE:GXGXR, GIPEPXX:APV:KPLS:DHHKD:VEE:SXAXR, SIPKAXX:APV:KPLS:DHHKD:VEE:GXGXR, HVTKPTX:APV:KPLS:DHHKD:VEE:SXGXH, YVPKPXX:APV:KPLS:DHHKD:VEE:SXGXH, TVPKPXX:APV:KPLS:DHHKD:VEE:AXGXH, AVPKAXX:APV:KPLS:DHHKD:VEE:AXGXH, KVGKAXX:APV:KPLS:DHHKD:VEE:XGXR, KASKAXX:APV:KPLS:DHHKD:VEE:EXGXR, GSAGPXX:APV:KPLS:DHHKD:VEE:RXGXS, AAPASXX:APV:KPLS:DHHKD:VEE:AXGXR, STPPTXX:APV:KPLS:DHHKD:VEE:SXGXR, HVPKPXX:APV:KPLS:DHHKD:VEE:SXGXH, ASAAPXX:APV:KPLS:DHHKD:VEE:XKXS, ASASPXX:APV:KPLS:DHHKD:VEE:XKXS, KIPKAXX:VPT:KPLS:DHHKD:VVE:GXGXR, GIPEPXX:VPE:KPLS:DHHKD:TVE:SXAXR, SIPKAXX:VPT:KPLS:DHHKD:VVE:GXGXR, HVTKPTX:APT:KPLS:DHHKD:VVR:SXGXH, YVPKPXX:APT:KPLS:DHHKD:VVR:SXGXH, TVPKPXX:APT:KPLS:DHHKD:VVR:AXGXH, AVPKAXX:APT:KPLS:DHHKD:VVK:AXGXH, KVGKAXX:VPT:KPLS:DHHKD:VAE:XGXR, KASKAXX:VPT:KPLS:DHHKD:AVS:EXGXR, GSAGPXX:TPT:KPLS:DHHKD:VVD:RXGXS, AAPASXX:VPA:KPLS:DHHKD:VVE:AXGXR, STPPTXX:VPT:KPLS:DHHKD:VVE:SXGXR, HVPKPXX:APT:KPLS:DHHKD:VVR:SXGXH, ASAAPXX:VPQ:KPLS:DHHKD:VRS:XKXS, ASASPXX:VSQ:KPLS:DHHKD:VKS:XKXS, ASASPXX:VPQ:KPLS:DHHKD:VKS:XKXS, NDEGLEX:VPT:KPLS:DHHKD:QHN:KXEXR, NDEGLEX:VPT:KPLS:DHHKD:EHS:QXEXR, SSVKXQP:SRV:KPLS:DHHKD:EEH:LEXAXA, RNVQXRP:TQV:KPLS:DHHKD:EDH:LAXKXE, KIPKAXX:VPQ:EPLP:EQLSN:VRS:GXGXR, GIPEPXX:VPQ:EPLP:EQLSN:VRS:SXAXR, SIPKAXX:VPQ:EPLP:EQLSN:VRS:GXGXR, HVTKPTX:VPQ:EPLP:EQLSN:VRS:SXGXH, YVPKPXX:VPQ:EPLP:EQLSN:VRS:SXGXH, TVPKPXX:VPQ:EPLP:EQLSN:VRS:AXGXH, AVPKAXX:VPQ:EPLP:EQLSN:VRS:AXGXH, KVGKAXX:VPQ:EPLP:EQLSN:VRS:XGXR, KASKAXX:VPQ:EPLP:EQLSN:VRS:EXGXR, GSAGPXX:VPQ:EPLP:EQLSN:VRS:RXGXS, AAPASXX:VPQ:EPLP:EQLSN:VRS:AXGXR, STPPTXX:VPQ:EPLP:EQLSN:VRS:SXGXR, HVPKPXX:VPQ:EPLP:EQLSN:VRS:SXGXH, RVPSTXX:VPQ:EPLP:EQLSN:VRS:XGXL, ASASPXX:VPQ:EPLP:EQLSN:VRS:XKXS, KIPKAXX:VPT:EPLP:EQLSN:VVE:GXGXR, GIPEPXX:VPE:EPLP:EQLSN:TVE:SXAXR, SIPKAXX:VPT:EPLP:EQLSN:VVE:GXGXR, HVTKPTX:APT:EPLP:EQLSN:VVR:SXGXH, YVPKPXX:APT:EPLP:EQLSN:VVR:SXGXH, TVPKPXX:APT:EPLP:EQLSN:VVR:AXGXH, AVPKAXX:APT:EPLP:EQLSN:VVK:AXGXH, KVGKAXX:VPT:EPLP:EQLSN:VAE:XGXR, KASKAXX:VPT:EPLP:EQLSN:AVS:EXGXR, GSAGPXX:TPT:EPLP:EQLSN:VVD:RXGXS, AAPASXX:VPA:EPLP:EQLSN:VVE:AXGXR, STPPTXX:VPT:EPLP:EQLSN:VVE:SXGXR, HVPKPXX:APT:EPLP:EQLSN:VVR:SXGXH, RVPSTXX:APV:EPLP:EQLSN:VEE:XGXL, ASASPXX:VSQ:EPLP:EQLSN:VKS:XKXS, ASASPXX:VPQ:EPLP:EQLSN:VKS:XKXS, NDEGLEX:VPT:EPLP:EQLSN:QHN:KXEXR, NDEGLEX:VPT:EPLP:EQLSN:EHS:QXEXR, SSVKXQP:SRV:EPLP:EQLSN:EEH:LEXAXA, RNVQXRP:TQV:EPLP:EQLSN:EDH:LAXKXE, KIPKAXX:VSQ:EPLT:EQLSN:VKS:GXGXR, GIPEPXX:VSQ:EPLT:EQLSN:VKS:SXAXR, SIPKAXX:VSQ:EPLT:EQLSN:VKS:GXGXR, HVTKPTX:VSQ:EPLT:EQLSN:VKS:SXGXH, YVPKPXX:VSQ:EPLT:EQLSN:VKS:SXGXH, TVPKPXX:VSQ:EPLT:EQLSN:VKS:AXGXH, AVPKAXX:VSQ:EPLT:EQLSN:VKS:AXGXH, KVGKAXX:VSQ:EPLT:EQLSN:VKS:XGXR, KASKAXX:VSQ:EPLT:EQLSN:VKS:EXGXR, GSAGPXX:VSQ:EPLT:EQLSN:VKS:RXGXS, AAPASXX:VSQ:EPLT:EQLSN:VKS:AXGXR, STPPTXX:VSQ:EPLT:EQLSN:VKS:SXGXR, HVPKPXX:VSQ:EPLT:EQLSN:VKS:SXGXH, RVPSTXX:VSQ:EPLT:EQLSN:VKS:XGXL, ASAAPXX:VSQ:EPLT:EQLSN:VKS:XKXS, ASASPXX:VSQ:EPLT:EQLSN:VKS:XKXS, KIPKAXX:VPT:EPLT:EQLSN:VVE:GXGXR, GIPEPXX:VPE:EPLT:EQLSN:TVE:SXAXR, SIPKAXX:VPT:EPLT:EQLSN:VVE:GXGXR, HVTKPTX:APT:EPLT:EQLSN:VVR:SXGXH, YVPKPXX:APT:EPLT:EQLSN:VVR:SXGXH, TVPKPXX:APT:EPLT:EQLSN:VVR:AXGXH, AVPKAXX:APT:EPLT:EQLSN:VVK:AXGXH, KVGKAXX:VPT:EPLT:EQLSN:VAE:XGXR, KASKAXX:VPT:EPLT:EQLSN:AVS:EXGXR, GSAGPXX:TPT:EPLT:EQLSN:VVD:RXGXS, AAPASXX:VPA:EPLT:EQLSN:VVE:AXGXR, STPPTXX:VPT:EPLT:EQLSN:VVE:SXGXR, HVPKPXX:APT:EPLT:EQLSN:VVR:SXGXH, RVPSTXX:APV:EPLT:EQLSN:VEE:XGXL, ASAAPXX:VPQ:EPLT:EQLSN:VRS:XKXS, NDEGLEX:VPT:EPLT:EQLSN:QHN:KXEXR, NDEGLEX:VPT:EPLT:EQLSN:EHS:QXEXR, SSVKXQP:SRV:EPLT:EQLSN:EEH:LEXAXA, RNVQXRP:TQV:EPLT:EQLSN:EDH:LAXKXE, KIPKAXX:VPQ:EPLT:EQLSN:VKS:GXGXR, GIPEPXX:VPQ:EPLT:EQLSN:VKS:SXAXR, SIPKAXX:VPQ:EPLT:EQLSN:VKS:GXGXR, HVTKPTX:VPQ:EPLT:EQLSN:VKS:SXGXH, YVPKPXX:VPQ:EPLT:EQLSN:VKS:SXGXH, TVPKPXX:VPQ:EPLT:EQLSN:VKS:AXGXH, AVPKAXX:VPQ:EPLT:EQLSN:VKS:AXGXH, KVGKAXX:VPQ:EPLT:EQLSN:VKS:XGXR, KASKAXX:VPQ:EPLT:EQLSN:VKS:EXGXR, GSAGPXX:VPQ:EPLT:EQLSN:VKS:RXGXS, AAPASXX:VPQ:EPLT:EQLSN:VKS:AXGXR, STPPTXX:VPQ:EPLT:EQLSN:VKS:SXGXR, HVPKPXX:VPQ:EPLT:EQLSN:VKS:SXGXH, RVPSTXX:VPQ:EPLT:EQLSN:VKS:XGXL, ASAAPXX:VPQ:EPLT:EQLSN:VKS:XKXS, ASASPXX:VPQ:EPLT:EQLSN:VKS:XKXS, NDEGLEX:VPT:SNIT:IGEMS:QHN:QXEXR, KIPKAXX:VPT:SNIT:IGEMS:VVE:GXGXR, GIPEPXX:VPE:SNIT:IGEMS:TVE:SXAXR, SIPKAXX:VPT:SNIT:IGEMS:VVE:GXGXR, HVTKPTX:APT:SNIT:IGEMS:VVR:SXGXH, YVPKPXX:APT:SNIT:IGEMS:VVR:SXGXH, TVPKPXX:APT:SNIT:IGEMS:VVR:AXGXH, AVPKAXX:APT:SNIT:IGEMS:VVK:AXGXH, KVGKAXX:VPT:SNIT:IGEMS:VAE:XGXR, KASKAXX:VPT:SNIT:IGEMS:AVS:EXGXR, GSAGPXX:TPT:SNIT:IGEMS:VVD:RXGXS, AAPASXX:VPA:SNIT:IGEMS:VVE:AXGXR, STPPTXX:VPT:SNIT:IGEMS:VVE:SXGXR, HVPKPXX:APT:SN IT:IGEMS:VVR:SXGXH, RVPSTXX:APV:SN IT: IGEMS:VEE:XGXL, ASAAPXX:VPQ:SNIT:IGEMS:VRS:XKXS, ASASPXX:VSQ:SNIT:IGEMS:VKS:XKXS, ASASPXX:VPQ:SNIT:IGEMS:VKS:XKXS, NDEGLEX:VPT:SNIT:IGEMS:EHS:QXEXR, SSVKXQP:SRV:SNIT:IGEMS:EEH:LEXAXA, RNVQXRP:TQV:SNIT:IGEMS:EDH:LAXKXE, NDEGLEX:VPT:SNIT:LGEMS:EHS:KXEXR, KIPKAXX:VPT:SNIT:LGEMS:VVE:GXGXR, GIPEPXX:VPE:SNIT:LGEMS:TVE:SXAXR, SIPKAXX:VPT:SNIT:LGEMS:VVE:GXGXR, HVTKPTX:APT:SNIT:LGEMS:VVR:SXGXH, YVPKPXX:APT:SNIT:LGEMS:VVR:SXGXH, TVPKPXX:APT:SNIT:LGEMS:VVR:AXGXH, AVPKAXX:APT:SNIT:LGEMS:VVK:AXGXH, KVGKAXX:VPT:SNIT:LGEMS:VAE:XGXR, KASKAXX:VPT:SNIT:LGEMS:AVS:EXGXR, GSAGPXX:TPT:SNIT:LGEMS:VVD:RXGXS, AAPASXX:VPA:SN IT:LGEMS:VVE:AXGXR, STPPTXX:VPT:SN IT:LGEMS:VVE:SXGXR, HVPKPXX:APT:SNIT:LGEMS:VVR:SXGXH, RVPSTXX:APV:SNIT:LGEMS:VEE:XGXL, ASAAPXX:VPQ:SNIT:LGEMS:VRS:XKXS, ASASPXX:VSQ:SNIT:LGEMS:VKS:XKXS, ASASPXX:VPQ:SNIT:LGEMS:VKS:XKXS, NDEGLEX:VPT:SNIT:LGEMS:QHN:KXEXR, SSVKXQP:SRV:SNIT:LGEMS:EEH:LEXAXA, RNVQXRP:TQV:SNIT:LGEMS:EDH:LAXKXE, RNVQXRP:SRV:RSVK:KEVQV:EEH:LAXKXE, KIPKAXX:VPT:RSVK:KEVQV:VVE:GXGXR, GIPEPXX:VPE:RSVK:KEVQV:TVE:SXAXR, SIPKAXX:VPT:RSVK:KEVQV:VVE:GXGXR, HVTKPTX:APT:RSVK:KEVQV:VVR:SXGXH, YVPKPXX:APT:RSVK:KEVQV:VVR:SXGXH, TVPKPXX:APT:RSVK:KEVQV:VVR:AXGXH, AVPKAXX:APT:RSVK:KEVQV:VVK:AXGXH, KVGKAXX:VPT:RSVK:KEVQV:VAE:XGXR, KASKAXX:VPT:RSVK:KEVQV:AVS:EXGXR, GSAGPXX:TPT:RSVK:KEVQV:VVD:RXGXS, AAPASXX:VPA:RSVK:KEVQV:VVE:AXGXR, STPPTXX:VPT:RSVK:KEVQV:VVE:SXGXR, HVPKPXX:APT:RSVK:KEVQV:VVR:SXGXH, RVPSTXX:APV:RSVK:KEVQV:VEE:XGXL, ASAAPXX:VPQ:RSVK:KEVQV:VRS:XKXS, ASASPXX:VSQ:RSVK:KEVQV:VKS:XKXS, ASASPXX:VPQ:RSVK:KEVQV:VKS:XKXS, NDEGLEX:VPT:RSVK:KEVQV:QHN:KXEXR, NDEGLEX:VPT:RSVK:KEVQV:EHS:QXEXR, RNVQXRP:TQV:RSVK:KEVQV:EDH:LAXKXE, SSVKXQP:TQV:RPVQ:KKATV:EDH:LEXAXA, KIPKAXX:VPT:RPVQ:KKATV:VVE:GXGXR, GIPEPXX:VPE:RPVQ:KKATV:TVE:SXAXR, SIPKAXX:VPT:RPVQ:KKATV:VVE:GXGXR, HVTKPTX:APT:RPVQ:KKATV:VVR:SXGXH, YVPKPXX:APT:RPVQ:KKATV:VVR:SXGXH, TVPKPXX:APT:RPVQ:KKATV:VVR:AXGXH, AVPKAXX:APT:RPVQ:KKATV:VVK:AXGXH, KVGKAXX:VPT:RPVQ:KKATV:VAE:XGXR, KASKAXX:VPT:RPVQ:KKATV:AVS:EXGXR, GSAGPXX:TPT:RPVQ:KKATV:VVD:RXGXS, AAPASXX:VPA:RPVQ:KKATV:VVE:AXGXR, STPPTXX:VPT:RPVQ:KKATV:VVE:SXGXR, HVPKPXX:APT:RPVQ:KKATV:VVR:SXGXH, RVPSTXX:APV:RPVQ:KKATV:VEE:XGXL, ASAAPXX:VPQ:RPVQ:KKATV:VRS:XKXS, ASASPXX:VSQ:RPVQ:KKATV:VKS:XKXS, ASASPXX:VPQ:RPVQ:KKATV:VKS:XKXS, NDEGLEX:VPT:RPVQ:KKATV:QHN:KXEXR, NDEGLEX:VPT:RPVQ:KKATV:EHS:QXEXR and SSVKXQP:SRV:RPVQ:KKATV:EEH:LEXAXA.

In particular, in certain embodiments, the hexaplet PEP7:PEP3:PEP12:PEP2:PEP4:PEP8 is selected from the group consisting of GIPEPXX:VPT:SAIS-$AA^{17}$-LYL:LKNYQ:VVE:SXAXR, SIPKAXX:VPT:SAIS-$AA^{17}$-LYL:LKNYQ:VVE:GXGXR, HVTKPTX:VPT:SAIS-$AA^{17}$-LYL:LKNYQ:VVE:SXGXH, YVPKPXX:VPT:SAIS-$AA^{17}$-LYL:LKNYQ:VVE:SXGXH, TVPKPXX:VPT:SAIS-$AA^{17}$-LYL:LKNYQ:VVE:AXGXH, AVPKAXX:VPT:SAIS-$AA^{17}$-LYL:LKNYQ:VVE:AXGXH, KVGKAXX:VPT:SAIS-$AA^{17}$-LYL:LKNYQ:VVE:XGXR, KASKAXX:VPT:SAIS-$AA^{17}$-LYL:LKNYQ:VVE:EXGXR, GSAGPXX:VPT:SAIS-$AA^{17}$-LYL:LKNYQ:VVE:RXGXS, AAPASXX:VPT:SAIS-$AA^{17}$-LYL:LKNYQ:VVE:AXGXR, STPPTXX:VPT:SAIS-$AA^{17}$-LYL:LKNYQ:VVE:SXGXR, HVPKPXX:VPT:SAIS-$AA^{17}$-LYL:LKNYQ:VVE:SXGXH, RVPSTXX:VPT:SAIS-$AA^{17}$-LYL:LKNYQ:VVE:XGXL, ASAAPXX:VPT:SAIS-$AA^{17}$-LYL:LKNYQ:VVE:XKXS, ASASPXX:VPT:SAIS-$AA^{17}$-LYL:LKNYQ:VVE:XKXS, NDEGLEX:VPT:SAIS-$AA^{17}$-LYL:LKNYQ:VVE:KXEXR, NDEGLEX:VPT:SAIS-$AA^{17}$-LYL:LKNYQ:VVE:QXEXR, SSVKXQP:VPT:SAIS-$AA^{17}$-LYL:LKNYQ:VVE:LEXAXA, RNVQXRP:VPT:SAIS-$AA^{17}$-LYL:LKNYQ:VVE:LAXKXE, GIPEPXX:VPE:SAIS-$AA^{17}$-LYL:LKNYQ:TVE:SXAXR, HVTKPTX:APT:SAIS-$AA^{17}$-LYL:LKNYQ:VVR:SXGXH, YVPKPXX:APT:SAIS-$AA^{17}$-LYL:LKNYQ:VVR:SXGXH, TVPKPXX:APT:SAIS-$AA^{17}$-LYL:LKNYQ:VVR:AXGXH, AVPKAXX:APT:SAIS-$AA^{17}$-LYL:LKNYQ:VVK:AXGXH, KVGKAXX:VPT:SAIS-$AA^{17}$-LYL:LKNYQ:VAE:XGXR, KASKAXX:VPT:SAIS-$AA^{17}$-LYL:LKNYQ:AVS:EXGXR, GSAGPXX:TPT:SAIS-$AA^{17}$-LYL:LKNYQ:VVD:RXGXS, AAPASXX:VPA:SAIS-$AA^{17}$-LYL:LKNYQ:VVE:AXGXR, HVPKPXX:APT:SAIS-$AA^{17}$-LYL:LKNYQ:VVR:SXGXH, RVPSTXX:APV:SAIS-$AA^{17}$-LYL:LKNYQ:VEE:XGXL, ASAAPXX:VPQ:SAIS-$AA^{17}$-LYL:LKNYQ:VRS:XKXS, ASASPXX:VSQ:SAIS-$AA^{17}$-LYL:LKNYQ:VKS:XKXS, ASASPXX:VPQ:SAIS-$AA^{17}$-LYL:LKNYQ:VKS:XKXS, NDEGLEX:VPT:SAIS-$AA^{17}$-LYL:LKNYQ:QHN:KXEXR, NDEGLEX:VPT:SAIS-$AA^{17}$-LYL:LKNYQ:EHS:QXEXR, SSVKXQP:SRV:SAIS-$AA^{17}$-LYL:LKNYQ:EEH:LEXAXA, RNVQXRP:TQV:SAIS-$AA^{17}$-LYL:LKNYQ:EDH:LAXKXE, KIPKAXX:VPE:SSLS-$AA^{17}$-LFF:LKVYP:TVE:GXGXR, SIPKAXX:VPE:SSLS-$AA^{17}$-LFF:LKVYP:TVE:GXGXR, HVTKPTX:VPE:SSLS-$AA^{17}$-LFF:LKVYP:TVE:SXGXH, YVPKPXX:VPE:SSLS-$AA^{17}$-LFF:LKVYP:TVE:SXGXH, TVPKPXX:VPE:SSLS-$AA^{17}$-LFF:LKVYP:TVE:AXGXH, AVPKAXX:VPE:SSLS-$AA^{17}$-LFF:LKVYP:TVE:AXGXH, KVGKAXX:VPE:SSLS-$AA^{17}$-LFF:LKVYP:TVE:XGXR, KASKAXX:VPE:SSLS-$AA^{17}$-LFF:LKVYP:TVE:EXGXR, GSAGPXX:VPE:SSLS-$AA^{17}$-LFF:LKVYP:TVE:RXGXS, AAPASXX:VPE:SSLS-$AA^{17}$-LFF:LKVYP:TVE:AXGXR, STPPTXX:VPE:SSLS-$AA^{17}$-LFF:LKVYP:TVE:SXGXR, HVPKPXX:VPE:SSLS-$AA^{17}$-LFF:LKVYP:TVE:SXGXH, RVPSTXX:VPE:SSLS-$AA^{17}$-LFF:LKVYP:TVE:XGXL, ASAAPXX:VPE:SSLS-$AA^{17}$-LFF:LKVYP:TVE:XKXS, ASASPXX:VPE:SSLS-

$AA^{17}$-LFF:LKVYP:TVE:XKXS, NDEGLEX:VPE:SSLS-$AA^{17}$-LFF:LKVYP:TVE:KXEXR, NDEGLEX:VPE:SSLS-$AA^{17}$-LFF:LKVYP:TVE:QXEXR, SSVKXQP:VPE:SSLS-$AA^{17}$-LFF:LKVYP:TVE:LEXAXA, RNVQXRP:VPE:SSLS-$AA^{17}$-LFF:LKVYP:TVE:LAXKXE, KIPKAXX:VPT:SSLS-$AA^{17}$-LFF:LKVYP:VVE:GXGXR, SIPKAXX:VPT:SSLS-$AA^{17}$-LFF:LKVYP:VVE:GXGXR, HVTKPTX:APT:SSLS-$AA^{17}$-LFF:LKVYP:VVR:SXGXH, YVPKPXX:APT:SSLS-$AA^{17}$-LFF:LKVYP:VVR:SXGXH, TVPKPXX:APT:SSLS-$AA^{17}$-LFF:LKVYP:VVR:AXGXH, AVPKAXX:APT:SSLS-$AA^{17}$-LFF:LKVYP:VVK:AXGXH, KVGKAXX:VPT:SSLS-$AA^{17}$-LFF:LKVYP:VAE:XGXR, KASKAXX:VPT:SSLS-$AA^{17}$-LFF:LKVYP:AVS:EXGXR, GSAGPXX:TPT:SSLS-$AA^{17}$-LFF:LKVYP:VVD:RXGXS, AAPASXX:VPA:SSLS-$AA^{17}$-LFF:LKVYP:VVE:AXGXR, STPPTXX:VPT:SSLS-$AA^{17}$-LFF:LKVYP:VVE:SXGXR, HVPKPXX:APT:SSLS-$AA^{17}$-LFF:LKVYP:VVR:SXGXH, RVPSTXX:APV:SSLS-$AA^{17}$-LFF:LKVYP:VEE:XGXL, ASAAPXX:VPQ:SSLS-$AA^{17}$-LFF:LKVYP:VRS:XKXS, ASASPXX:VSQ:SSLS-$AA^{17}$-LFF:LKVYP:VKS:XKXS, ASASPXX:VPQ:SSLS-$AA^{17}$-LFF:LKVYP:VKS:XKXS, NDEGLEX:VPT:SSLS-$AA^{17}$-LFF:LKVYP:QHN:KXEXR, NDEGLEX:VPT:SSLS-$AA^{17}$-LFF:LKVYP:EHS:QXEXR, SSVKXQP:SRV:SSLS-$AA^{17}$-LFF:LKVYP:EEH:LEXAXA, RNVQXRP:TQV:SSLS-$AA^{17}$-LFF:LKVYP:EDH:LAXKXE, KIPKAXX:VPT:SAIS-$AA^{17}$-LYL:LKNYQ:VVE:GXGXR, KIPKAXX:APT:NAIS-$AA^{17}$-LYF:LKKYR:VVR:GXGXR, GIPEPXX:APT:NAIS-$AA^{17}$-LYF:LKKYR:VVR:SXAXR, SIPKAXX:APT:NAIS-$AA^{17}$-LYF:LKKYR:VVR:GXGXR, YVPKPXX:APT:NAIS-$AA^{17}$-LYF:LKKYR:VVR:SXGXH, TVPKPXX:APT:NAIS-$AA^{17}$-LYF:LKKYR:VVR:AXGXH, AVPKAXX:APT:NAIS-$AA^{17}$-LYF:LKKYR:VVR:AXGXH, KVGKAXX:APT:NAIS-$AA^{17}$-LYF:LKKYR:VVR:XGXR, KASKAXX:APT:NAIS-$AA^{17}$-LYF:LKKYR:VVR:EXGXR, GSAGPXX:APT:NAIS-$AA^{17}$-LYF:LKKYR:VVR:RXGXS, AAPASXX:APT:NAIS-$AA^{17}$-LYF:LKKYR:VVR:AXGXR, STPPTXX:APT:NAIS-$AA^{17}$-LYF:LKKYR:VVR:SXGXR, HVPKPXX:APT:NAIS-$AA^{17}$-LYF:LKKYR:VVR:SXGXH, RVPSTXX:APT:NAIS-$AA^{17}$-LYF:LKKYR:VVR:XGXL, ASAAPXX:APT:NAIS-$AA^{17}$-LYF:LKKYR:VVR:XKXS, ASASPXX:APT:NAIS-$AA^{17}$-LYF:LKKYR:VVR:XKXS, NDEGLEX:APT:NAIS-$AA^{17}$-LYF:LKKYR:VVR:KXEXR, NDEGLEX:APT:NAIS-$AA^{17}$-LYF:LKKYR:VVR:QXEXR, SSVKXQP:APT:NAIS-$AA^{17}$-LYF:LKKYR:VVR:LEXAXA, RNVQXRP:APT:NAIS-$AA^{17}$-LYF:LKKYR:VVR:LAXKXE, KIPKAXX:VPT:NAIS-$AA^{17}$-LYF:LKKYR:VVE:GXGXR, GIPEPXX:VPE:NAIS-$AA^{17}$-LYF:LKKYR:TVE:SXAXR, SIPKAXX:VPT:NAIS-$AA^{17}$-LYF:LKKYR:VVE:GXGXR, AVPKAXX:APT:NAIS-$AA^{17}$-LYF:LKKYR:VVK:AXGXH, KVGKAXX:VPT:NAIS-$AA^{17}$-LYF:LKKYR:VAE:XGXR, KASKAXX:VPT:NAIS-$AA^{17}$-LYF:LKKYR:AVS:EXGXR, GSAGPXX:TPT:NAIS-$AA^{17}$-LYF:LKKYR:VVD:RXGXS, AAPASXX:VPA:NAIS-$AA^{17}$-LYF:LKKYR:VVE:AXGXR, STPPTXX:VPT:NAIS-$AA^{17}$-LYF:LKKYR:VVE:SXGXR, RVPSTXX:APV:NAIS-$AA^{17}$-LYF:LKKYR:VEE:XGXL, ASAAPXX:VPQ:NAIS-$AA^{17}$-LYF:LKKYR:VRS:XKXS, ASASPXX:VSQ:NAIS-$AA^{17}$-LYF:LKKYR:VKS:XKXS, ASASPXX:VPQ:NAIS-$AA^{17}$-LYF:LKKYR:VKS:XKXS, NDEGLEX:VPT:NAIS-$AA^{17}$-LYF:LKKYR:QHN:KXEXR, NDEGLEX:VPT:NAIS-$AA^{17}$-LYF:LKKYR:EHS:QXEXR, SSVKXQP:SRV:NAIS-$AA^{17}$-LYF:LKKYR:EEH:LEXAXA, RNVQXRP:TQV:NAIS-$AA^{17}$-LYF:LKKYR:EDH:LAXKXE, HVTKPTX:APT:NAIS-$AA^{17}$-LYF:LKKYR:VVR:SXGXH, KIPKAXX:APT:SATS-$AA^{17}$-LYY:LRKHR:VVK:GXGXR, GIPEPXX:APT:SATS-$AA^{17}$-LYY:LRKHR:VVK:SXAXR, SIPKAXX:APT:SATS-$AA^{17}$-LYY:LRKHR:VVK:GXGXR, HVTKPTX:APT:SATS-$AA^{17}$-LYY:LRKHR:VVK:SXGXH, YVPKPXX:APT:SATS-$AA^{17}$-LYY:LRKHR:VVK:SXGXH, TVPKPXX:APT:SATS-$AA^{17}$-LYY:LRKHR:VVK:AXGXH, KVGKAXX:APT:SATS-$AA^{17}$-LYY:LRKHR:VVK:XGXR, KASKAXX:APT:SATS-$AA^{17}$-LYY:LRKHR:VVK:EXGXR, GSAGPXX:APT:SATS-$AA^{17}$-LYY:LRKHR:VVK:RXGXS, AAPASXX:APT:SATS-$AA^{17}$-LYY:LRKHR:VVK:AXGXR, STPPTXX:APT:SATS-$AA^{17}$-LYY:LRKHR:VVK:SXGXR, HVPKPXX:APT:SATS-$AA^{17}$-LYY:LRKHR:VVK:SXGXH, RVPSTXX:APT:SATS-$AA^{17}$-LYY:LRKHR:VVK:XGXL, ASAAPXX:APT:SATS-$AA^{17}$-LYY:LRKHR:VVK:XKXS, ASASPXX:APT:SATS-$AA^{17}$-LYY:LRKHR:VVK:XKXS, NDEGLEX:APT:SATS-$AA^{17}$-LYY:LRKHR:VVK:KXEXR, NDEGLEX:APT:SATS-$AA^{17}$-LYY:LRKHR:VVK:QXEXR, SSVKXQP:APT:SATS-$AA^{17}$-LYY:LRKHR:VVK:LEXAXA, RNVQXRP:APT:SATS-$AA^{17}$-LYY:LRKHR:VVK:LAXKXE, KIPKAXX:VPT:SATS-$AA^{17}$-LYY:LRKHR:VVE:GXGXR, GIPEPXX:VPE:SATS-$AA^{17}$-LYY:LRKHR:TVE:SXAXR, SIPKAXX:VPT:SATS-$AA^{17}$-LYY:LRKHR:VVE:GXGXR, HVTKPTX:APT:SATS-$AA^{17}$-LYY:LRKHR:VVR:SXGXH, YVPKPXX:APT:SATS-$AA^{17}$-LYY:LRKHR:VVR:SXGXH, TVPKPXX:APT:SATS-$AA^{17}$-LYY:LRKHR:VVR:AXGXH, KVGKAXX:VPT:SATS-$AA^{17}$-LYY:LRKHR:VAE:XGXR, KASKAXX:VPT:SATS-$AA^{17}$-LYY:LRKHR:AVS:EXGXR, GSAGPXX:TPT:SATS-$AA^{17}$-LYY:LRKHR:VVD:RXGXS, AAPASXX:VPA:SATS-$AA^{17}$-LYY:LRKHR:VVE:AXGXR, STPPTXX:VPT:SATS-$AA^{17}$-LYY:LRKHR:VVE:SXGXR, HVPKPXX:APT:SATS-$AA^{17}$-LYY:LRKHR:VVR:SXGXH, RVPSTXX:APV:SATS-$AA^{17}$-LYY:LRKHR:VEE:XGXL, ASAAPXX:VPQ:SATS-$AA^{17}$-LYY:LRKHR:VRS:XKXS, ASASPXX:VSQ:SATS-$AA^{17}$-LYY:LRKHR:VKS:XKXS, ASASPXX:VPQ:SATS-$AA^{17}$-LYY:LRKHR:VKS:XKXS, NDEGLEX:VPT:SATS-$AA^{17}$-LYY:LRKHR:QHN:KXEXR, NDEGLEX:VPT:SATS-$AA^{17}$-LYY:LRKHR:EHS:QXEXR, SSVKXQP:SRV:SATS-$AA^{17}$-LYY:LRKHR:EEH:LEXAXA, RNVQXRP:TQV:SATS-$AA^{17}$-LYY:LRKHR:EDH:LAXKXE, KIPKAXX:VPT:SPIS-$AA^{17}$-LYK:LKYHY:VAE:GXGXR, GIPEPXX:VPT:SPIS-$AA^{17}$-LYK:LKYHY:VAE:SXAXR, SIPKAXX:VPT:SPIS-$AA^{17}$-LYK:LKYHY:VAE:GXGXR, HVTKPTX:VPT:SPIS-$AA^{17}$-LYK:LKYHY:VAE:SXGXH, YVPKPXX:VPT:SPIS-$AA^{17}$-LYK:LKYHY:VAE:SXGXH, TVPKPXX:VPT:SPIS-$AA^{17}$-LYK:LKYHY:VAE:AXGXH, AVPKAXX:VPT:SPIS-$AA^{17}$-LYK:LKYHY:VAE:AXGXH, KASKAXX:VPT:SPIS-$AA^{17}$-LYK:LKYHY:VAE:EXGXR, GSAGPXX:VPT:SPIS-$AA^{17}$-LYK:LKYHY:VAE:RXGXS, AAPASXX:VPT:SPIS-$AA^{17}$-LYK:LKYHY:VAE:AXGXR, STPPTXX:VPT:SPIS-$AA^{17}$-LYK:LKYHY:VAE:SXGXR, HVPKPXX:VPT:SPIS-$AA^{17}$-LYK:LKYHY:VAE:SXGXH, RVPSTXX:VPT:SPIS-$AA^{17}$-LYK:LKYHY:VAE:XGXL, ASAAPXX:VPT:SPIS-$AA^{17}$-LYK:LKYHY:VAE:XKXS, ASASPXX:VPT:SPIS-

AA$^{17}$-LYK:LKYHY:VAE:XKXS, NDEGLEX:VPT:SPIS-AA$^{17}$-LYK:LKYHY:VAE:KXEXR, NDEGLEX:VPT:SPIS-AA$^{17}$-LYK:LKYHY:VAE:QXEXR, SSVKXQP:VPT:SPIS-AA$^{17}$-LYK:LKYHY:VAE:LEXAXA, RNVQXRP:VPT:SPIS-AA$^{17}$-LYK:LKYHY:VAE:LAXKXE, KIPKAXX:VPT:SPIS-AA$^{17}$-LYK:LKYHY:VVE:GXGXR, GIPEPXX:VPE:SPIS-AA$^{17}$-LYK:LKYHY:TVE:SXAXR, SIPKAXX:VPT:SPIS-AA$^{17}$-LYK:LKYHY:VVE:GXGXR, HVTKPTX:APT:SPIS-AA$^{17}$-LYK:LKYHY:VVR:SXGXH, YVPKPXX:APT:SPIS-AA$^{17}$-LYK:LKYHY:VVR:SXGXH, TVPKPXX:APT:SPIS-AA$^{17}$-LYK:LKYHY:VVR:AXGXH, AVPKAXX:APT:SPIS-AA$^{17}$-LYK:LKYHY:VVK:AXGXH, KASKAXX:VPT:SPIS-AA$^{17}$-LYK:LKYHY:AVS:EXGXR, GSAGPXX:TPT:SPIS-AA$^{17}$-LYK:LKYHY:VVD:RXGXS, AAPASXX:VPA:SPIS-AA$^{17}$-LYK:LKYHY:VVE:AXGXR, STPPTXX:VPT:SPIS-AA$^{17}$-LYK:LKYHY:VVE:SXGXR, HVPKPXX:APT:SPIS-AA$^{17}$-LYK:LKYHY:VVR:SXGXH, RVPSTXX:APV:SPIS-AA$^{17}$-LYK:LKYHY:VEE:XGXL, ASAAPXX:VPQ:SPIS-AA$^{17}$-LYK:LKYHY:VRS:XKXS, ASASPXX:VSQ:SPIS-AA$^{17}$-LYK:LKYHY:VKS:XKXS, ASASPXX:VPQ:SPIS-AA$^{17}$-LYK:LKYHY:VKS:XKXS, NDEGLEX:VPT:SPIS-AA$^{17}$-LYK:LKYHY:QHN:KXEXR, NDEGLEX:VPT:SPIS-AA$^{17}$-LYK:LKYHY:EHS:QXEXR, SSVKXQP:SRV:SPIS-AA$^{17}$-LYK:LKYHY:EEH:LEXAXA, RNVQXRP:TQV:SPIS-AA$^{17}$-LYK:LKYHY:EDH:LAXKXE, KIPKAXX:VPT:EPIS-AA$^{17}$-LYL:KFKYE:AVS:GXGXR, GIPEPXX:VPT:EPIS-AA$^{17}$-LYL:KFKYE:AVS:SXAXR, SIPKAXX:VPT:EPIS-AA$^{17}$-LYL:KFKYE:AVS:GXGXR, HVTKPTX:VPT:EPIS-AA$^{17}$-LYL:KFKYE:AVS:SXGXH, YVPKPXX:VPT:EPIS-AA$^{17}$-LYL:KFKYE:AVS:SXGXH, TVPKPXX:VPT:EPIS-AA$^{17}$-LYL:KFKYE:AVS:AXGXH, AVPKAXX:VPT:EPIS-AA$^{17}$-LYL:KFKYE:AVS:AXGXH, KVGKAXX:VPT:EPIS-AA$^{17}$-LYL:KFKYE:AVS:XGXR, GSAGPXXVPT:EPIS-AA$^{17}$-LYL:KFKYE:AVS:RXGXS, AAPASXX:VPT:EPIS-AA$^{17}$-LYL:KFKYE:AVS:AXGXR, STPPTXX:VPT:EPIS-AA$^{17}$-LYL:KFKYE:AVS:SXGXR, HVPKPXX:VPT:EPIS-AA$^{17}$-LYL:KFKYE:AVS:SXGXH, RVPSTXX:VPT:EPIS-AA$^{17}$-LYL:KFKYE:AVS:XGXL, ASAAPXX:VPT:EPIS-AA$^{17}$-LYL:KFKYE:AVS:XKXS, ASASPXX:VPT:EPIS-AA$^{17}$-LYL:KFKYE:AVS:XKXS, NDEGLEX:VPT:EPIS-AA$^{17}$-LYL:KFKYE:AVS:KXEXR, NDEGLEX:VPT:EPIS-AA$^{17}$-LYL:KFKYE:AVS:QXEXR, SSVKXQP:VPT:EPIS-AA$^{17}$-LYL:KFKYE:AVS:LEXAXA, RNVQXRP:VPT:EPIS-AA$^{17}$-LYL:KFKYE:AVS:LAXKXE, KIPKAXX:VPT:EPIS-AA$^{17}$-LYL:KFKYE:VVE:GXGXR, GIPEPXX:VPE:EPIS-AA$^{17}$-LYL:KFKYE:TVE:SXAXR, SIPKAXX:VPT:EPIS-AA$^{17}$-LYL:KFKYE:VVE:GXGXR, HVTKPTX:APT:EPIS-AA$^{17}$-LYL:KFKYE:VVR:SXGXH, YVPKPXX:APT:EPIS-AA$^{17}$-LYL:KFKYE:VVR:SXGXH, TVPKPXX:APT:EPIS-AA$^{17}$-LYL:KFKYE:VVR:AXGXH, AVPKAXX:APT:EPIS-AA$^{17}$-LYL:KFKYE:VVK:AXGXH, KVGKAXX:VPT:EPIS-AA$^{17}$-LYL:KFKYE:VAE:XGXR, GSAGPXX:TPT:EPIS-AA$^{17}$-LYL:KFKYE:VVD:RXGXS, AAPASXX:VPA:EPIS-AA$^{17}$-LYL:KFKYE:VVE:AXGXR, STPPTXX:VPT:EPIS-AA$^{17}$-LYL:KFKYE:VVE:SXGXR, HVPKPXX:APT:EPIS-AA$^{17}$-LYL:KFKYE:VVR:SXGXH, RVPSTXX:APV:EPIS-AA$^{17}$-LYL:KFKYE:VEE:XGXL, ASAAPXX:VPQ:EPIS-AA$^{17}$-LYL:KFKYEVRS:XKXS, ASASPXXVSQ:EPIS-AA$^{17}$-LYL:KFKYE:VKS:XKXS, ASASPXX:VPQ:EPIS-AA$^{17}$-LYL:KFKYE:VKS:XKXS, NDEGLEX:VPT:EPIS-AA$^{17}$-LYL:KFKYE:QHN:KXEXR, NDEGLEX:VPT:EPIS-AA$^{17}$-LYL:KFKYE:EHS:QXEXR, SSVKXQP:SRV:EPIS-AA$^{17}$-LYL:KFKYE:EEH:LEXAXA, RNVQXRP:TQV:EPIS-AA$^{17}$-LYL:KFKYE:EDH:LAXKXE, KIPKAXX:TPT:SPIN-AA$^{17}$-LYF:YGKIP:VVD:GXGXR, GIPEPXX:TPT:SPIN-AA$^{17}$-LYF:YGKIP:VVD:SXAXR, SIPKAXX:TPT:SPIN-AA$^{17}$-LYF:YGKIP:VVD:GXGXR, HVTKPTX:TPT:SPIN-AA$^{17}$-LYF:YGKIP:VVD:SXGXH, YVPKPXX:TPT:SPIN-AA$^{17}$-LYF:YGKIP:VVD:SXGXH, TVPKPXX:TPT:SPIN-AA$^{17}$-LYF:YGKIP:VVD:AXGXH, AVPKAXX:TPT:SPIN-AA$^{17}$-LYF:YGKIP:VVD:AXGXH, KVGKAXX:TPT:SPIN-AA$^{17}$-LYF:YGKIP:VVD:XGXR, KASKAXX:TPT:SPIN-AA$^{17}$-LYF:YGKIP:VVD:EXGXR, AAPASXX:TPT:SPIN-AA$^{17}$-LYF:YGKIP:VVD:AXGXR, STPPTXX:TPT:SPIN-AA$^{17}$-LYF:YGKIP:VVD:SXGXR, HVPKPXX:TPT:SPIN-AA$^{17}$-LYF:YGKIP:VVD:SXGXH, RVPSTXX:TPT:SPIN-AA$^{17}$-LYF:YGKIP:VVD:XGXL, ASAAPXX:TPT:SPIN-AA$^{17}$-LYF:YGKIP:VVD:XKXS, ASASPXX:TPT:SPIN-AA$^{17}$-LYF:YGKIP:VVD:XKXS, NDEGLEX:TPT:SPIN-AA$^{17}$-LYF:YGKIP:VVD:KXEXR, NDEGLEX:TPT:SPIN-AA$^{17}$-LYF:YGKIP:VVD:QXEXR, SSVKXQP:TPT:SPIN-AA$^{17}$-LYF:YGKIP:VVD:LEXAXA, RNVQXRP:TPT:SPIN-AA$^{17}$-LYF:YGKIP:VVD:LAXKXE, KIPKAXX:VPT:SPIN-AA$^{17}$-LYF:YGKIP:VVE:GXGXR, GIPEPXX:VPE:SPIN-AA$^{17}$-LYF:YGKIP:TVE:SXAXR, SIPKAXX:VPT:SPIN-AA$^{17}$-LYF:YGKIP:VVE:GXGXR, HVTKPTX:APT:SPIN-AA$^{17}$-LYF:YGKIP:VVR:SXGXH, YVPKPXX:APT:SPIN-AA$^{17}$-LYF:YGKIP:VVR:SXGXH, TVPKPXX:APT:SPIN-AA$^{17}$-LYF:YGKIP:VVR:AXGXH, AVPKAXX:APT:SPIN-AA$^{17}$-LYF:YGKIP:VVK:AXGXH, KVGKAXX:VPT:SPIN-AA$^{17}$-LYF:YGKIP:VAE:XGXR, KASKAXX:VPT:SPIN-AA$^{17}$-LYF:YGKIP:AVS:EXGXR, AAPASXX:VPA:SPIN-AA$^{17}$-LYF:YGKIP:VVE:AXGXR, STPPTXX:VPT:SPIN-AA$^{17}$-LYF:YGKIP:VVE:SXGXR, HVPKPXX:APT:SPIN-AA$^{17}$-LYF:YGKIP:VVR:SXGXH, RVPSTXX:APV:SPIN-AA$^{17}$-LYF:YGKIP:VEE:XGXL, ASAAPXX:VPQ:SPIN-AA$^{17}$-LYF:YGKIP:VRS:XKXS, ASASPXX:VSQ:SPIN-AA$^{17}$-LYF:YGKIP:VKS:XKXS, ASASPXX:VPQ:SPIN-AA$^{17}$-LYF:YGKIP:VKS:XKXS, NDEGLEX:VPT:SPIN-AA$^{17}$-LYF:YGKIP:QHN:KXEXR, NDEGLEX:VPT:SPIN-AA$^{17}$-LYF:YGKIP:EHS:QXEXR, SSVKXQP:SRV:SPIN-AA$^{17}$-LYF:YGKIP:EEH:LEXAXA, RNVQXRP:TQV:SPIN-AA$^{17}$-LYF:YGKIP:EDH:LAXKXE, KIPKAXX:VPA:SPIS-AA$^{17}$-LYI:YKQYE:VVE:GXGXR, GIPEPXX:VPA:SPIS-AA$^{17}$-LYI:YKQYE:VVE:SXAXR, SIPKAXX:VPA:SPIS-AA$^{17}$-LYI:YKQYE:VVE:GXGXR, HVTKPTX:VPA:SPIS-AA$^{17}$-LYI:YKQYE:VVE:SXGXH, YVPKPXX:VPA:SPIS-AA$^{17}$-LYI:YKQYE:VVE:SXGXH, TVPKPXX:VPA:SPIS-AA$^{17}$-LYI:YKQYE:VVE:AXGXH, AVPKAXX:VPA:SPIS-AA$^{17}$-LYI:YKQYE:VVE:AXGXH, KVGKAXX:VPA:SPIS-AA$^{17}$-LYI:YKQYE:VVE:XGXR, KASKAXX:VPA:SPIS-AA$^{17}$-LYI:YKQYE:VVE:EXGXR, GSAGPXX:VPA:SPIS-AA$^{17}$-LYI:YKQYE:VVE:RXGXS, STPPTXX:VPA:SPIS-AA$^{17}$-LYI:YKQYE:VVE:SXGXR, HVPKPXX:VPA:SPIS-AA$^{17}$-LYI:YKQYE:VVE:SXGXH, RVPSTXX:VPA:SPIS-AA$^{17}$-LYI:YKQYE:VVE:XGXL, ASAAPXX:VPA:SPIS-

AA$^{17}$-LYI:YKQYE:VVE:XKXS, ASASPXX:VPA:SPIS-AA$^{17}$-LYI:YKQYE:VVE:XKXS, NDEGLEX:VPA:SPIS-AA$^{17}$-LYI:YKQYE:VVE:KXEXR, NDEGLEX:VPA:SPIS-AA$^{17}$-LYI:YKQYE:VVE:QXEXR, SSVKXQP:VPA:SPIS-AA$^{17}$-LYI:YKQYE:VVE:LEXAXA, RNVQXRP:VPA:SPIS-AA$^{17}$-LYI:YKQYE:VVE:LAXKXE, KIPKAXX:VPT:SPIS-AA$^{17}$-LYI:YKQYE:VVE:GXGXR, GIPEPXX:VPE:SPIS-AA$^{17}$-LYI:YKQYE:TVE:SXAXR, SIPKAXX:VPT:SPIS-AA$^{17}$-LYI:YKQYE:VVE:GXGXR, HVTKPTX:APT:SPIS-AA$^{17}$-LYI:YKQYE:VVR:SXGXH, YVPKPXX:APT:SPIS-AA$^{17}$-LYI:YKQYE:VVR:SXGXH, TVPKPXX:APT:SPIS-AA$^{17}$-LYI:YKQYE:VVR:AXGXH, AVPKAXX:APT:SPIS-AA$^{17}$-LYI:YKQYE:VVK:AXGXH, KVGKAXX:VPT:SPIS-AA$^{17}$-LYI:YKQYE:VAE:XGXR, KASKAXX:VPT:SPIS-AA$^{17}$-LYI:YKQYE:AVS:EXGXR, GSAGPXX:TPT:SPIS-AA$^{17}$-LYI:YKQYE:VVD:RXGXS, STPPTXX:VPT:SPIS-AA$^{17}$-LYI:YKQYE:WE:SXGXR, HVPKPXX:APT:SPIS-AA$^{17}$-LYI:YKQYE:WR:SXGXH, RVPSTXX:APV:SPIS-AA$^{17}$-LYI:YKQYE:VEE:XGXL, ASAAPXX:VPQ:SPIS-AA$^{17}$-LYI:YKQYE:VRS:XKXS, ASASPXX:VSQ:SPIS-AA$^{17}$-LYI:YKQYE:VKS:XKXS, ASASPXX:VPQ:SPIS-AA$^{17}$-LYI:YKQYE:VKS:XKXS, NDEGLEX:VPT:SPIS-AA$^{17}$-LYI:YKQYE:QHN:KXEXR, NDEGLEX:VPT:SPIS-AA$^{17}$-LYI:YKQYE:EHS:QXEXR, SSVKXQP:SRV:SPIS-AA$^{17}$-LYI:YKQYE:EEH:LEXAXA, RNVQXRP:TQV:SPIS-AA$^{17}$-LYI:YKQYE:EDH:LAXKXE, KIPKAXX:VPT:SPIS-AA$^{17}$-LFI:YKQYE:VVE:GXGXR, GIPEPXX:VPT:SPIS-AA$^{17}$-LFI:YKQYE:VVE:SXAXR, SIPKAXX:VPT:SPIS-AA$^{17}$-LFI:YKQYE:VVE:GXGXR, HVTKPTX:VPT:SPIS-AA$^{17}$-LFI:YKQYE:VVE:SXGXH, YVPKPXX:VPT:SPIS-AA$^{17}$-LFI:YKQYE:WE:SXGXH, TVPKPXX:VPT:SPIS-AA$^{17}$-LFI:YKQYE:VVE:AXGXH, AVPKAXX:VPT:SPIS-AA$^{17}$-LFI:YKQYE:VVE:AXGXH, KVGKAXX:VPT:SPIS-AA$^{17}$-LFI:YKQYE:WE:XGXR, KASKAXX:VPT:SPIS-AA$^{17}$-LFI:YKQYE:VVE:EXGXR, GSAGPXX:VPT:SPIS-AA$^{17}$-LFI:YKQYE:VVE:RXGXS, AAPASXX:VPT:SPIS-AA$^{17}$-LFI:YKQYE:WE:AXGXR, HVPKPXX:VPT:SPIS-AA$^{17}$-LFI:YKQYE:VVE:SXGXH, RVPSTXX:VPT:SPIS-AA$^{17}$-LFI:YKQYE:VVE:XGXL, ASAAPXX:VPT:SPIS-AA$^{17}$-LFI:YKQYE:WE:XKXS, ASASPXX:VPT:SPIS-AA$^{17}$-LFI:YKQYE:WE:XKXS, NDEGLEX:VPT:SPIS-AA$^{17}$-LFI:YKQYE:VVE:KXEXR, NDEGLEX:VPT:SPIS-AA$^{17}$-LFI:YKQYE:VVE:QXEXR, SSVKXQP:VPT:SPIS-AA$^{17}$-LFI:YKQYE:VVE:LEXAXA, RNVQXRP:VPT:SPIS-AA$^{17}$-LFI:YKQYE:VVE:LAXKXE, GIPEPXX:VPE:SPIS-AA$^{17}$-LFI:YKQYE:TVE:SXAXR, HVTKPTX:APT:SPIS-AA$^{17}$-LFI:YKQYE:VVR:SXGXH, YVPKPXX:APT:SPIS-AA$^{17}$-LFI:YKQYE:VVR:SXGXH, TVPKPXX:APT:SPIS-AA$^{17}$-LFI:YKQYE:VVR:AXGXH, AVPKAXX:APT:SPIS-AA$^{17}$-LFI:YKQYE:VVK:AXGXH, KVGKAXX:VPT:SPIS-AA$^{17}$-LFI:YKQYE:VAE:XGXR, KASKAXX:VPT:SPIS-AA$^{17}$-LFI:YKQYE:AVS:EXGXR, GSAGPXX:TPT:SPIS-AA$^{17}$-LFI:YKQYE:WD:RXGXS, AAPASXX:VPA:SPIS-AA$^{17}$-LFI:YKQYE:VVE:AXGXR, HVPKPXX:APT:SPIS-AA$^{17}$-LFI:YKQYE:VVR:SXGXH, RVPSTXX:APV:SPIS-AA$^{17}$-LFI:YKQYE:VEE:XGXL, ASAAPXX:VPQ:SPIS-AA$^{17}$-LFI:YKQYE:VRS:XKXS, ASASPXX:VSQ:SPIS-AA$^{17}$-LFI:YKQYE:VKS:XKXS, ASASPXX:VPQ:SPIS-AA$^{17}$-LFI:YKQYE:VKS:XKXS, NDEGLEX:VPT:SPIS-AA$^{17}$-LFI:YKQYE:QHN:KXEXR, NDEGLEX:VPT:SPIS-AA$^{17}$-LFI:YKQYE:EHS:QXEXR, SSVKXQP:SRV:SPIS-AA$^{17}$-LFI:YKQYE:EEH:LEXAXA, RNVQXRP:TQV:SPIS-AA$^{17}$-LFI:YKQYE:EDH:LAXKXE, KIPKAXX:APV:KPLS-AA$^{17}$-LYV:DHHKD:VEE:GXGXR, GIPEPXX:APV:KPLS-AA$^{17}$-LYV:DHHKD:VEE:SXAXR, SIPKAXX:APV:KPLS-AA$^{17}$-LYV:DHHKD:VEE:GXGXR, HVTKPTX:APV:KPLS-AA$^{17}$-LYV:DHHKD:VEE:SXGXH, YVPKPXX:APV:KPLS-AA$^{17}$-LYV:DHHKD:VEE:SXGXH, TVPKPXX:APV:KPLS-AA$^{17}$-LYV:DHHKD:VEE:AXGXH, AVPKAXX:APV:KPLS-AA$^{17}$-LYV:DHHKD:VEE:AXGXH, KVGKAXX:APV:KPLS-AA$^{17}$-LYV:DHHKD:VEE:XGXR, KASKAXX:APV:KPLS-AA$^{17}$-LYV:DHHKD:VEE:EXGXR, GSAGPXX:APV:KPLS-AA$^{17}$-LYV:DHHKD:VEE:RXGXS, AAPASXX:APV:KPLS-AA$^{17}$-LYV:DHHKD:VEE:AXGXR, STPPTXX:APV:KPLS-AA$^{17}$-LYV:DHHKD:VEE:SXGXR, HVPKPXX:APV:KPLS-AA$^{17}$-LYV:DHHKD:VEE:SXGXH, ASAAPXX:APV:KPLS-AA$^{17}$-LYV:DHHKD:VEE:XKXS, ASASPXX:APV:KPLS-AA$^{17}$-LYV:DHHKD:VEE:XKXS, NDEGLEX:APV:KPLS-AA$^{17}$-LYV:DHHKD:VEE:KXEXR, NDEGLEX:APV:KPLS-AA$^{17}$-LYV:DHHKD:VEE:QXEXR, SSVKXQP:APV:KPLS-AA$^{17}$-LYV:DHHKD:VEE:LEXAXA, RNVQXRP:APV:KPLS-AA$^{17}$-LYV:DHHKD:VEE:LAXKXE, KIPKAXX:VPT:KPLS-AA$^{17}$-LYV:DHHKD:VVE:GXGXR, GIPEPXX:VPE:KPLS-AA$^{17}$-LYV:DHHKD:TVE:SXAXR, SIPKAXX:VPT:KPLS-AA$^{17}$-LYV:DHHKD:VVE:GXGXR, HVTKPTX:APT:KPLS-AA$^{17}$-LYV:DHHKD:VVR:SXGXH, YVPKPXX:APT:KPLS-AA$^{17}$-LYV:DHHKD:VVR:SXGXH, TVPKPXX:APT:KPLS-AA$^{17}$-LYV:DHHKD:VVR:AXGXH, AVPKAXX:APT:KPLS-AA$^{17}$-LYV:DHHKD:VVK:AXGXH, KVGKAXX:VPT:KPLS-AA$^{17}$-LYV:DHHKD:VAE:XGXR, KASKAXX:VPT:KPLS-AA$^{17}$-LYV:DHHKD:AVS:EXGXR, GSAGPXX:TPT:KPLS-AA$^{17}$-LYV:DHHKD:VVD:RXGXS, AAPASXX:VPA:KPLS-AA$^{17}$-LYV:DHHKD:VVE:AXGXR, STPPTXX:VPT:KPLS-AA$^{17}$-LYV:DHHKD:VVE:SXGXR, HVPKPXX:APT:KPLS-AA$^{17}$-LYV:DHHKD:VVR:SXGXH, ASAAPXX:VPQ:KPLS-AA$^{17}$-LYV:DHHKD:VRS:XKXS, ASASPXX:VSQ:KPLS-AA$^{17}$-LYV:DHHKD:VKS:XKXS, ASASPXX:VPQ:KPLS-AA$^{17}$-LYV:DHHKD:VKS:XKXS, NDEGLEX:VPT:KPLS-AA$^{17}$-LYV:DHHKD:QHN:KXEXR, NDEGLEX:VPT:KPLS-AA$^{17}$-LYV:DHHKD:EHS:QXEXR, SSVKXQP:SRV:KPLS-AA$^{17}$-LYV:DHHKD:EEH:LEXAXA, RNVQXRP:TQV:KPLS-AA$^{17}$-LYV:DHHKD:EDH:LAXKXE, KIPKAXX:VPQ:EPLP-AA$^{17}$-VYY:EQLSN:VRS:GXGXR, GIPEPXX:VPQ:EPLP-AA$^{17}$-VYY:EQLSN:VRS:SXAXR, SIPKAXX:VPQ:EPLP-AA$^{17}$-VYY:EQLSN:VRS:GXGXR, HVTKPTX:VPQ:EPLP-AA$^{17}$-VYY:EQLSN:VRS:SXGXH, YVPKPXX:VPQ:EPLP-AA$^{17}$-VYY:EQLSN:VRS:SXGXH, TVPKPXX:VPQ:EPLP-AA$^{17}$-VYY:EQLSN:VRS:AXGXH, AVPKAXX:VPQ:EPLP-AA$^{17}$-VYY:EQLSN:VRS:AXGXH, KVGKAXX:VPQ:EPLP-AA$^{17}$-VYY:EQLSN:VRS:XGXR, KASKAXX:VPQ:EPLP-AA$^{17}$-VYY:EQLSN:VRS:EXGXR, GSAGPXX:VPQ:EPLP-AA$^{17}$-VYY:EQLSN:VRS:RXGXS, AAPASXX:VPQ:EPLP-AA$^{17}$-VYY:EQLSN:VRS:AXGXR, STPPTXX:VPQ:EPLP-AA$^{17}$-VYY:EQLSN:VRS:SXGXR, HVPKPXX:VPQ:EPLP-AA$^{17}$-VYY:EQLSN:VRS:SXGXH, RVPSTXX:VPQ:EPLP-AA$^{17}$-VYY:EQLSN:VRS:XGXL, ASASPXX:VPQ:EPLP-

AA$^{17}$-VYY:EQLSN:VRS:XKXS, NDEGLEX:VPQ:EPLP-AA$^{17}$-VYY:EQLSN:VRS:KXEXR, NDEGLEX:VPQ:EPLP-
AA$^{17}$-VYY:EQLSN:VRS:QXEXR, SSVKXQP:VPQ:EPLP-AA$^{17}$-VYY:EQLSN:VRS:LEXAXA, RNVQXRP:VPQ:EPLP-
AA$^{17}$-VYY:EQLSN:VRS:LAXKXE, KIPKAXX:VPT:EPLP-AA$^{17}$-VYY:EQLSN:VVE:GXGXR, GIPEPXX:VPE:EPLP-
AA$^{17}$-VYY:EQLSN:TVE:SXAXR, SIPKAXX:VPT:EPLP-AA$^{17}$-VYY:EQLSN:VVE:GXGXR, HVTKPTX:APT:EPLP-
AA$^{17}$-VYY:EQLSN:VVR:SXGXH, YVPKPXX:APT:EPLP-AA$^{17}$-VYY:EQLSN:VVR:SXGXH, TVPKPXX:APT:EPLP-
AA$^{17}$-VYY:EQLSN:VVR:AXGXH, AVPKAXX:APT:EPLP-AA$^{17}$-VYY:EQLSN:VVK:AXGXH, KVGKAXX:VPT:EPLP-
AA$^{17}$-VYY:EQLSN:VAE:XGXR, KASKAXX:VPT:EPLP-AA$^{17}$-VYY:EQLSN:AVS:EXGXR, GSAGPXX:TPT:EPLP-
AA$^{17}$-VYY:EQLSN:VVD:RXGXS, AAPASXX:VPA:EPLP-AA$^{17}$-VYY:EQLSN:VVE:AXGXR, STPPTXX:VPT:EPLP-
AA$^{17}$-VYY:EQLSN:VVE:SXGXR, HVPKPXX:APT:EPLP-AA$^{17}$-VYY:EQLSN:VVR:SXGXH, RVPSTXX:APV:EPLP-
AA$^{17}$-VYY:EQLSN:VEE:XGXL, ASASPXX:VSQ:EPLP-AA$^{17}$-VYY:EQLSN:VKS:XKXS, ASASPXX:VPQ:EPLP-
AA$^{17}$-VYY:EQLSN:VKS:XKXS, NDEGLEX:VPT:EPLP-AA$^{17}$-VYY:EQLSN:QHN:KXEXR, NDEGLEX:VPT:EPLP-
AA$^{17}$-VYY:EQLSN:EHS:QXEXR, SSVKXQP:SRV:EPLP-AA$^{17}$-VYY:EQLSN:EEH:LEXAXA, RNVQXRP:TQV:EPLP-
AA$^{17}$-VYY:EQLSN:EDH:LAXKXE, KIPKAXX:VSQ:EPLT-AA$^{17}$-LYY:EQLSN:VKS:GXGXR, GIPEPXX:VSQ:EPLT-
AA$^{17}$-LYY:EQLSN:VKS:SXAXR, SIPKAXX:VSQ:EPLT-AA$^{17}$-LYY:EQLSN:VKS:GXGXR, HVTKPTX:VSQ:EPLT-
AA$^{17}$-LYY:EQLSN:VKS:SXGXH, YVPKPXX:VSQ:EPLT-AA$^{17}$-LYY:EQLSN:VKS:SXGXH, TVPKPXX:VSQ:EPLT-
AA$^{17}$-LYY:EQLSN:VKS:AXGXH, AVPKAXX:VSQ:EPLT-AA$^{17}$-LYY:EQLSN:VKS:AXGXH, KVGKAXX:VSQ:EPLT-
AA$^{17}$-LYY:EQLSN:VKS:XGXR, KASKAXX:VSQ:EPLT-AA$^{17}$-LYY:EQLSN:VKS:EXGXR, GSAGPXX:VSQ:EPLT-
AA$^{17}$-LYY:EQLSN:VKS:RXGXS, AAPASXX:VSQ:EPLT-AA$^{17}$-LYY:EQLSN:VKS:AXGXR, STPPTXX:VSQ:EPLT-
AA$^{17}$-LYY:EQLSN:VKS:SXGXR, HVPKPXX:VSQ:EPLT-AA$^{17}$-LYY:EQLSN:VKS:SXGXH, RVPSTXX:VSQ:EPLT-
AA$^{17}$-LYY:EQLSN:VKS:XGXL, ASAAPXX:VSQ:EPLT-AA$^{17}$-LYY:EQLSN:VKS:XKXS, ASASPXX:VSQ:EPLT-
AA$^{17}$-LYY:EQLSN:VKS:XKXS, NDEGLEX:VSQ:EPLT-AA$^{17}$-LYY:EQLSN:VKS:KXEXR, NDEGLEX:VSQ:EPLT-
AA$^{17}$-LYY:EQLSN:VKS:QXEXR, SSVKXQP:VSQ:EPLT-AA$^{17}$-LYY:EQLSN:VKS:LEXAXA, RNVQXRP:VSQ:EPLT-
AA$^{17}$-LYY:EQLSN:VKS:LAXKXE, KIPKAXX:VPT:EPLT-AA$^{17}$-LYY:EQLSN:VVE:GXGXR, GIPEPXX:VPE:EPLT-
AA$^{17}$-LYY:EQLSN:TVE:SXAXR, SIPKAXX:VPT:EPLT-AA$^{17}$-LYY:EQLSN:VVE:GXGXR, HVTKPTX:APT:EPLT-
AA$^{17}$-LYY:EQLSN:VVR:SXGXH, YVPKPXX:APT:EPLT-AA$^{17}$-LYY:EQLSN:VVR:SXGXH, TVPKPXX:APT:EPLT-
AA$^{17}$-LYY:EQLSN:VVR:AXGXH, AVPKAXX:APT:EPLT-AA$^{17}$-LYY:EQLSN:VVK:AXGXH, KVGKAXX:VPT:EPLT-
AA$^{17}$-LYY:EQLSN:VAE:XGXR, KASKAXX:VPT:EPLT-AA$^{17}$-LYY:EQLSN:AVS:EXGXR, GSAGPXX:TPT:EPLT-
AA$^{17}$-LYY:EQLSN:VVD:RXGXS, AAPASXX:VPA:EPLT-AA$^{17}$-LYY:EQLSN:VVE:AXGXR, STPPTXX:VPT:EPLT-
AA$^{17}$-LYY:EQLSN:VVE:SXGXR, HVPKPXX:APT:EPLT-AA$^{17}$-LYY:EQLSN:VVR:SXGXH, RVPSTXX:APV:EPLT-
AA$^{17}$-LYY:EQLSN:VEE:XGXL, ASAAPXX:VPQ:EPLT-AA$^{17}$-LYY:EQLSN:VRS:XKXS, ASASPXX:VPQ:EPLT-
AA$^{17}$-LYY:EQLSN:VKS:XKXS, NDEGLEX:VPT:EPLT-AA$^{17}$-LYY:EQLSN:QHN:KXEXR, NDEGLEX:VPT:EPLT-
AA$^{17}$-LYY:EQLSN:EHS:QXEXR, SSVKXQP:SRV:EPLT-AA$^{17}$-LYY:EQLSN:EEH:LEXAXA, RNVQXRP:TQV:EPLT-
AA$^{17}$-LYY:EQLSN:EDH:LAXKXE, KIPKAXX:VPQ:EPLT-AA$^{17}$-LYY:EQLSN:VKS:GXGXR, GIPEPXX:VPQ:EPLT-
AA$^{17}$-LYY:EQLSN:VKS:SXAXR, SIPKAXX:VPQ:EPLT-AA$^{17}$-LYY:EQLSN:VKS:GXGXR, HVTKPTX:VPQ:EPLT-
AA$^{17}$-LYY:EQLSN:VKS:SXGXH, YVPKPXX:VPQ:EPLT-AA$^{17}$-LYY:EQLSN:VKS:SXGXH, TVPKPXX:VPQ:EPLT-
AA$^{17}$-LYY:EQLSN:VKS:AXGXH, AVPKAXX:VPQ:EPLT-AA$^{17}$-LYY:EQLSN:VKS:AXGXH, KVGKAXX:VPQ:EPLT-
AA$^{17}$-LYY:EQLSN:VKS:XGXR, KASKAXX:VPQ:EPLT-AA$^{17}$-LYY:EQLSN:VKS:EXGXR, GSAGPXX:VPQ:EPLT-
AA$^{17}$-LYY:EQLSN:VKS:RXGXS, AAPASXX:VPQ:EPLT-AA$^{17}$-LYY:EQLSN:VKS:AXGXR, STPPTXX:VPQ:EPLT-
AA$^{17}$-LYY:EQLSN:VKS:SXGXR, HVPKPXX:VPQ:EPLT-AA$^{17}$-LYY:EQLSN:VKS:SXGXH, RVPSTXX:VPQ:EPLT-
AA$^{17}$-LYY:EQLSN:VKS:XGXL, ASAAPXX:VPQ:EPLT-AA$^{17}$-LYY:EQLSN:VKS:XKXS, NDEGLEX:VPQ:EPLT-
AA$^{17}$-LYY:EQLSN:VKS:KXEXR, NDEGLEX:VPQ:EPLT-AA$^{17}$-LYY:EQLSN:VKS:QXEXR, SSVKXQP:VPQ:EPLT-
AA$^{17}$-LYY:EQLSN:VKS:LEXAXA, RNVQXRP:VPQ:EPLT-AA$^{17}$-LYY:EQLSN:VKS:LAXKXE, KIPKAXX:VPT:SNIT-
AA$^{17}$-QIM:IGEMS:QHN:GXGXR, GIPEPXX:VPT:SNIT-AA$^{17}$-QIM:IGEMS:QHN:SXAXR, SIPKAXX:VPT:SNIT-
AA$^{17}$-QIM:IGEMS:QHN:GXGXR, HVTKPTX:VPT:SNIT-AA$^{17}$-QIM:IGEMS:QHN:SXGXH, YVPKPXX:VPT:SNIT-
AA$^{17}$-QIM:IGEMS:QHN:SXGXH, TVPKPXX:VPT:SNIT-AA$^{17}$-QIM:IGEMS:QHN:AXGXH, AVPKAXX:VPT:SNIT-
AA$^{17}$-QIM:IGEMS:QHN:AXGXH, KVGKAXX:VPT:SNIT-AA$^{17}$-QIM:IGEMS:QHN:XGXR, KASKAXX:VPT:SNIT-
AA$^{17}$-QIM:IGEMS:QHN:EXGXR, GSAGPXX:VPT:SNIT-AA$^{17}$-QIM:IGEMS:QHN:RXGXS, AAPASXX:VPT:SNIT-
AA$^{17}$-QIM:IGEMS:QHN:AXGXR, STPPTXX:VPT:SNIT-AA$^{17}$-QIM:IGEMS:QHN:SXGXR, HVPKPXX:VPT:SNIT-
AA$^{17}$-QIM:IGEMS:QHN:SXGXH, RVPSTXX:VPT:SNIT-AA$^{17}$-QIM:IGEMS:QHN:XGXL, ASAAPXXVPT:SNIT-
AA$^{17}$-QIM:IGEMS:QHN:XKXS,ASASPXXVPT:SNIT-AA$^{17}$-QIM:IGEMS:QHN:XKXS, NDEGLEX:VPT:SNIT-
AA$^{17}$-QIM:IGEMS:QHN:QXEXR, NDEGLEX:VPT:SNIT-AA$^{17}$-QIM:IGEMS:QHN:KXEXR, SSVKXQP:VPT:SNIT-
AA$^{17}$-QIM:IGEMS:QHN:LEXAXA, RNVQXRP:VPT:SNIT-AA$^{17}$-QIM:IGEMS:QHN:LAXKXE, KIPKAXX:VPT:SNIT-
AA$^{17}$-QIM:IGEMS:VVE:GXGXR, GIPEPXXVPE:SNIT-AA$^{17}$-QIM:IGEMS:TVE:SXAXR, SIPKAXX:VPT:SNIT-
AA$^{17}$-QIM:IGEMS:VVE:GXGXR, HVTKPTX:APT:SNIT-AA$^{17}$-QIM:IGEMS:VVR:SXGXH, YVPKPXX:APT:SNIT-
AA$^{17}$-QIM:IGEMS:VVR:SXGXH, TVPKPXX:APT:SNIT-AA$^{17}$-QIM:IGEMS:VVR:AXGXH, AVPKAXX:APT:SNIT-
AA$^{17}$-QIM:IGEMSVVK:AXGXH, KVGKAXX:VPT:SNIT-AA$^{17}$-QIM:IGEMS:VAE:XGXR, KASKAXX:VPT:SNIT-
AA$^{17}$-QIM:IGEMS:AVS:EXGXR, GSAGPXX:TPT:SNIT-AA$^{17}$-QIM:IGEMS:VVD:RXGXS, AAPASXX:VPA:SNIT-
AA$^{17}$-QIM:IGEMS:VVE:AXGXR, STPPTXX:VPT:SNIT-AA$^{17}$-QIM:IGEMSVVE:SXGXR, HVPKPXX:APT:SNIT-

AA$^{17}$-QIM:IGEMS:VVR:SXGXH, RVPSTXX:APV:SNIT-AA$^{17}$-QIM:IGEMS:VEE:XGXL, ASAAPXX:VPQ:SNIT-
AA$^{17}$-QIM:IGEMS:VRS:XKXS,ASASPXX:VSQ:SNIT-AA$^{17}$-QIM:IGEMS:VKS:XKXS, ASASPXX:VPQ:SNIT-
AA$^{17}$-QIM:IGEMS:VKS:XKXS, NDEGLEX:VPT:SNIT-AA$^{17}$-QIM:IGEMS:EHS:QXEXR, SSVKXQP:SRV:SNIT-
AA$^{17}$-QIM:IGEMS:EEH:LEXAXA, RNVQXRP:TQV:SNIT-AA$^{17}$-QIM:IGEMS:EDH:LAXKXE, KIPKAXX:VPT:SNIT-
AA$^{17}$-QIM:LGEMS:EHS:GXGXR, GIPEPXXVPT:SNIT-AA$^{17}$-QIM:LGEMS:EHS:SXAXR, SIPKAXX:VPT:SNIT-
AA$^{17}$-QIM:LGEMS:EHS:GXGXR, HVTKPTX:VPT:SNIT-AA$^{17}$-QIM:LGEMS:EHS:SXGXH, YVPKPXX:VPT:SNIT-
AA$^{17}$-QIM:LGEMS:EHS:SXGXH, TVPKPXX:VPT:SNIT-AA$^{17}$-QIM:LGEMS:EHS:AXGXH, AVPKAXXVPT:SNIT-
AA$^{17}$-QIM:LGEMS:EHS:AXGXH, KVGKAXX:VPT:SNIT-AA$^{17}$-QIM:LGEMS:EHS:XGXR, KASKAXX:VPT:SNIT-
AA$^{17}$-QIM:LGEMS:EHS:EXGXR, GSAGPXX:VPT:SNIT-AA$^{17}$-QIM:LGEMS:EHS:RXGXS, AAPASXX:VPT:SNIT-
AA$^{17}$-QIM:LGEMS:EHS:AXGXR, STPPTXX:VPT:SNIT-AA$^{17}$-QIM:LGEMS:EHS:SXGXR, HVPKPXX:VPT:SNIT-
AA$^{17}$-QIM:LGEMS:EHS:SXGXH, RVPSTXX:VPT:SNIT-AA$^{17}$-QIM:LGEMS:EHS:XGXL, ASAAPXX:VPT:SNIT-
AA$^{17}$-QIM:LGEMS:EHS:XKXS, ASASPXX:VPT:SNIT-AA$^{17}$-QIM:LGEMS:EHS:XKXS, NDEGLEXVPT:SNIT-
AA$^{17}$-QIM:LGEMS:EHS:KXEXR, SSVKXQP:VPT:SNIT-AA$^{17}$-QIM:LGEMS:EHS:LEXAXA, RNVQXRP:VPT:SNIT-
AA$^{17}$-QIM:LGEMS:EHS:LAXKXE, KIPKAXX:VPT:SNIT-AA$^{17}$-QIM:LGEMS:VVE:GXGXR, GIPEPXX:VPE:SNIT-
AA$^{17}$-QIM:LGEMS:TVE:SXAXR, SIPKAXX:VPT:SNIT-AA$^{17}$-QIM:LGEMS:VVE:GXGXR, HVTKPTX:APT:SNIT-
AA$^{17}$-QIM:LGEMSVVRSXGXH, YVPKPXX:APT:SNIT-AA$^{17}$-QIM:LGEMS:VVR:SXGXH, TVPKPXX:APT:SNIT-
AA$^{17}$-QIM:LGEMS:VVR:AXGXH, AVPKAXX:APT:SNIT-AA$^{17}$-QIM:LGEMS:VVK:AXGXH, KVGKAXX:VPT:SNIT-
AA$^{17}$-QIM:LGEMSVAE:XGXR, KASKAXX:VPT:SNIT-AA$^{17}$-QIM:LGEMS:AVS:EXGXR, GSAGPXX:TPT:SNIT-
AA$^{17}$-QIM:LGEMS:VVD:RXGXS, AAPASXX:VPA:SNIT-AA$^{17}$-QIM:LGEMS:VVE:AXGXR, STPPTXX:VPT:SNIT-
AA$^{17}$-QIM:LGEMS:VVE:SXGXR, HVPKPXX:APT:SNIT-AA$^{17}$-QIM:LGEMSVVRSXGXH, RVPSTXX:APV:SNIT-
AA$^{17}$-QIM:LGEMSVEE:XGXL, ASAAPXX:VPQ:SNIT-AA$^{17}$-QIM:LGEMSVRS:XKXS, ASASPXX:VSQ:SNIT-
AA$^{17}$-QIM:LGEMS:VKS:XKXS, ASASPXX:VPQ:SNIT-AA$^{17}$-QIM:LGEMS:VKS:XKXS, NDEGLEXVPT:SNIT-
AA$^{17}$-QIM:LGEMS:QHN:KXEXR, SSVKXQP:SRV:SNIT-AA$^{17}$-QIM:LGEMS:EEH:LEXAXA, RNVQXRP:TQV:SNIT-
AA$^{17}$-QIM:LGEMS:EDH:LAXKXE, KIPKAXX:SRV:RSVK-AA$^{17}$-AKV:KEVQV:EEH:GXGXR, GIPEPXX:SRV:RSVK-
AA$^{17}$-AKV:KEVQV:EEH:SXAXR, SIPKAXX:SRV:RSVK-AA$^{17}$-AKV:KEVQV:EEH:GXGXR, HVTKPTX:SRV:RSVK-
AA$^{17}$-AKV:KEVQV:EEH:SXGXH, YVPKPXX:SRV:RSVK-AA$^{17}$-AKV:KEVQV:EEH:SXGXH, TVPKPXX:SRV:RSVK-
AA$^{17}$-AKV:KEVQV:EEH:AXGXH, AVPKAXX:SRV:RSVK-AA$^{17}$-AKV:KEVQV:EEH:AXGXH, KVGKAXX:SRV:RSVK-
AA$^{17}$-AKV:KEVQV:EEH:XGXR, KASKAXX:SRV:RSVK-AA$^{17}$-AKV:KEVQV:EEH:EXGXR, GSAGPXX:SRV:RSVK-
AA$^{17}$-AKV:KEVQV:EEH:RXGXS, AAPASXX:SRV:RSVK-AA$^{17}$-AKV:KEVQV:EEH:AXGXR, STPPTXX:SRV:RSVK-
AA$^{17}$-AKV:KEVQV:EEH:SXGXR, HVPKPXX:SRV:RSVK-AA$^{17}$-AKV:KEVQV:EEH:SXGXH, RVPSTXX:SRV:RSVK-
AA$^{17}$-AKV:KEVQV:EEH:XGXL, ASAAPXX:SRV:RSVK-AA$^{17}$-AKV:KEVQV:EEH:XKXS, ASASPXX:SRV:RSVK-
AA$^{17}$-AKV:KEVQV:EEH:XKXS, NDEGLEX:SRV:RSVK-AA$^{17}$-AKV:KEVQV:EEH:KXEXR, NDEGLEX:SRV:RSVK-
AA$^{17}$-AKV:KEVQV:EEH:QXEXR, RNVQXRP:SRV:RSVK-AA$^{17}$-AKV:KEVQV:EEH:LAXKXE, KIPKAXX:VPT:RSVK-
AA$^{17}$-AKV:KEVQV:VVE:GXGXR, GIPEPXX:VPE:RSVK-AA$^{17}$-AKV:KEVQV:TVE:SXAXR, SIPKAXX:VPT:RSVK-
AA$^{17}$-AKV:KEVQV:VVE:GXGXR, HVTKPTX:APT:RSVK-AA$^{17}$-AKV:KEVQV:VVR:SXGXH, YVPKPXX:APT:RSVK-
AA$^{17}$-AKV:KEVQV:VVR:SXGXH, TVPKPXX:APT:RSVK-AA$^{17}$-AKV:KEVQV:VVR:AXGXH, AVPKAXX:APT:RSVK-
AA$^{17}$-AKV:KEVQV:VVK:AXGXH, KVGKAXX:VPT:RSVK-AA$^{17}$-AKV:KEVQV:VAE:XGXR, KASKAXX:VPT:RSVK-
AA$^{17}$-AKV:KEVQV:AVS:EXGXR, GSAGPXX:TPT:RSVK-AA$^{17}$-AKV:KEVQV:VVD:RXGXS, AAPASXX:VPA:RSVK-
AA$^{17}$-AKV:KEVQV:VVE:AXGXR, STPPTXX:VPT:RSVK-AA$^{17}$-AKV:KEVQV:VVE:SXGXR, HVPKPXX:APT:RSVK-
AA$^{17}$-AKV:KEVQV:VVR:SXGXH, RVPSTXX:APV:RSVK-AA$^{17}$-AKV:KEVQV:VEE:XGXL, ASAAPXX:VPQ:RSVK-
AA$^{17}$-AKV:KEVQV:VRS:XKXS, ASASPXX:VSQ:RSVK-AA$^{17}$-AKV:KEVQV:VKS:XKXS, ASASPXX:VPQ:RSVK-
AA$^{17}$-AKV:KEVQV:VKS:XKXS, NDEGLEX:VPT:RSVK-AA$^{17}$-AKV:KEVQV:QHN:KXEXR, NDEGLEX:VPT:RSVK-
AA$^{17}$-AKV:KEVQV:EHS:QXEXR, RNVQXRP:TQV:RSVK-AA$^{17}$-AKV:KEVQV:EDH:LAXKXE, KIPKAXX:TQV:RPVQ-
AA$^{17}$-RKI:KKATV:EDH:GXGXR, GIPEPXX:TQV:RPVQ-AA$^{17}$-RKI:KKATV:EDH:SXAXR, SIPKAXX:TQV:RPVQ-
AA$^{17}$-RKI:KKATV:EDH:GXGXR, HVTKPTX:TQV:RPVQ-AA$^{17}$-RKI:KKATV:EDH:SXGXH, YVPKPXX:TQV:RPVQ-
AA$^{17}$-RKI:KKATV:EDH:SXGXH, TVPKPXX:TQV:RPVQ-AA$^{17}$-RKI:KKATV:EDH:AXGXH, AVPKAXX:TQV:RPVQ-
AA$^{17}$-RKI:KKATV:EDH:AXGXH, KVGKAXX:TQV:RPVQ-AA$^{17}$-RKI:KKATV:EDH:XGXR, KASKAXX:TQV:RPVQ-
AA$^{17}$-RKI:KKATV:EDH:EXGXR, GSAGPXX:TQV:RPVQ-AA$^{17}$-RKI:KKATV:EDH:RXGXS, AAPASXX:TQV:RPVQ-
AA$^{17}$-RKI:KKATV:EDH:AXGXR, STPPTXX:TQV:RPVQ-AA$^{17}$-RKI:KKATV:EDH:SXGXR, HVPKPXX:TQV:RPVQ-
AA$^{17}$-RKI:KKATV:EDH:SXGXH, RVPSTXX:TQV:RPVQ-AA$^{17}$-RKI:KKATV:EDH:XGXL, ASAAPXX:TQV:RPVQ-
AA$^{17}$-RKI:KKATV:EDH:XKXS, ASASPXX:TQV:RPVQ-AA$^{17}$-RKI:KKATV:EDH:XKXS, NDEGLEX:TQV:RPVQ-
AA$^{17}$-RKI:KKATV:EDH:KXEXR, NDEGLEX:TQV:RPVQ-AA$^{17}$-RKI:KKATV:EDH:QXEXR, SSVKXQP:TQV:RPVQ-
AA$^{17}$-RKI:KKATV:EDH:LEXAXA, KIPKAXXVPT:RPVQ-AA$^{17}$-RKI:KKATV:VVE:GXGXR, GIPEPXX:VPE:RPVQ-
AA$^{17}$-RKI:KKATV:TVE:SXAXR, SIPKAXX:VPT:RPVQ-AA$^{17}$-RKI:KKATV:VVE:GXGXR, HVTKPTX:APT:RPVQ-
AA$^{17}$-RKI:KKATV:VVR:SXGXH, YVPKPXX:APT:RPVQ-AA$^{17}$-RKI:KKATV:VVR:SXGXH, TVPKPXX:APT:RPVQ-
AA$^{17}$-RKI:KKATV:VVR:AXGXH, AVPKAXX:APT:RPVQ-AA$^{17}$-RKI:KKATV:VVK:AXGXH, KVGKAXX:VPT:RPVQ-
AA$^{17}$-RKI:KKATV:VAE:XGXR, KASKAXX:VPT:RPVQ-AA$^{17}$-RKI:KKATV:AVS:EXGXR, GSAGPXX:TPT:RPVQ-
AA$^{17}$-RKI:KKATV:VVD:RXGXS, AAPASXX:VPA:RPVQ-AA$^{17}$-RKI:KKATV:VVE:AXGXR, STPPTXX:VPT:RPVQ-

AA17-RKI:KKATV:VVE:SXGXR, HVPKPXX:APT:RPVQ-AA17-RKI:KKATV:VVR:SXGXH, RVPSTXX:APV:RPVQ-AA17-RKI:KKATV:VEE:XGXL, ASAAPXX:VPQ:RPVQ-AA17-RKI:KKATV:VRS:XKXS, ASASPXX:VSQ:RPVQ-AA17-RKI:KKATV:VKS:XKXS, ASASPXX:VPQ:RPVQ-AA17-RKI:KKATV:VKS:XKXS, NDEGLEX:VPT:RPVQ-AA17-RKI:KKATV:QHN:KXEXR, NDEGLEX:VPT:RPVQ-AA17-RKI:KKATV:EHS:QXEXR and SSVKXQP:SRV:RPVQ-AA17-RKI:KKATV:EEH:LEXAXA; and wherein AA17 is selected from the group consisting of G, A, V, L, I, P, F, M, W, T and S (in particular is selected from the group consisting of M, I, L, V and T). More particularly, the hexaplet PEP7:PEP3:PEP12:PEP2:PEP4:PEP8 is selected from the group consisting of GIPEPXX:VPT:SAIS-AA17-LYL:LKNYQ:VVE:SXAXR, HVTKPTX:VPT:SAIS-AA17-LYL:LKNYQ:VVE:SXGXH, YVPKPXX:VPT:SAIS-AA17-LYL:LKNYQ:VVE:SXGXH, TVPKPXX:VPT:SAIS-AA17-LYL:LKNYQ:VVE:AXGXH, AVPKAXX:VPT:SAIS-AA17-LYL:LKNYQ:VVE:AXGXH, KVGKAXX:VPT:SAIS-AA17-LYL:LKNYQ:VVE:XGXR, KASKAXX:VPT:SAIS-AA17-LYL:LKNYQ:VVE:EXGXR, GSAGPXX:VPT:SAIS-AA17-LYL:LKNYQ:VVE:RXGXS, AAPASXX:VPT:SAIS-AA17-LYL:LKNYQ:VVE:AXGXR, STPPTXX:VPT:SAIS-AA17-LYL:LKNYQ:VVE:SXGXR, HVPKPXX:VPT:SAIS-AA17-LYL:LKNYQ:VVE:SXGXH, RVPSTXX:VPT:SAIS-AA17-LYL:LKNYQ:VVE:XGXL, ASAAPXX:VPT:SAIS-AA17-LYL:LKNYQ:VVE:XKXS, ASASPXX:VPT:SAIS-AA17-LYL:LKNYQ:VVE:XKXS, GIPEPXX:VPE:SAIS-AA17-LYL:LKNYQ:TVE:SXAXR, HVTKPTX:APT:SAIS-AA17-LYL:LKNYQ:VVR:SXGXH, YVPKPXX:APT:SAIS-AA17-LYL:LKNYQ:VVR:SXGXH, TVPKPXX:APT:SAIS-AA17-LYL:LKNYQ:VVR:AXGXH, AVPKAXX:APT:SAIS-AA17-LYL:LKNYQ:VVK:AXGXH, KVGKAXX:VPT:SAIS-AA17-LYL:LKNYQ:VAE:XGXR, KASKAXX:VPT:SAIS-AA17-LYL:LKNYQ:AVS:EXGXR, GSAGPXX:TPT:SAIS-AA17-LYL:LKNYQ:VVD:RXGXS, AAPASXX:VPA:SAIS-AA17-LYL:LKNYQ:VVE:AXGXR, HVPKPXX:APT:SAIS-AA17-LYL:LKNYQ:VVR:SXGXH, RVPSTXX:APV:SAIS-AA17-LYL:LKNYQ:VEE:XGXL, ASAAPXX:VPQ:SAIS-AA17-LYL:LKNYQ:VRS:XKXS, ASASPXX:VSQ:SAIS-AA17-LYL:LKNYQ:VKS:XKXS, ASASPXX:VPQ:SAIS-AA17-LYL:LKNYQ:VKS:XKXS, NDEGLEX:VPT:SAIS-AA17-LYL:LKNYQ:QHN:KXEXR, NDEGLEX:VPT:SAIS-AA17-LYL:LKNYQ:EHS:QXEXR, SSVKXQP:SRV:SAIS-AA17-LYL:LKNYQ:EEH:LEXAXA, RNVQXRP:TQV:SAIS-AA17-LYL:LKNYQ:EDH:LAXKXE, KIPKAXX:VPE:SSLS-AA17-LFF:LKVYP:TVE:GXGXR, SIPKAXX:VPE:SSLS-AA17-LFF:LKVYP:TVE:GXGXR, HVTKPTX:VPE:SSLS-AA17-LFF:LKVYP:TVE:SXGXH, YVPKPXX:VPE:SSLS-AA17-LFF:LKVYP:TVE:SXGXH, TVPKPXX:VPE:SSLS-AA17-LFF:LKVYP:TVE:AXGXH, AVPKAXX:VPE:SSLS-AA17-LFF:LKVYP:TVE:AXGXH, KVGKAXX:VPE:SSLS-AA17-LFF:LKVYP:TVE:XGXR, KASKAXX:VPE:SSLS-AA17-LFF:LKVYP:TVE:EXGXR, GSAGPXX:VPE:SSLS-AA17-LFF:LKVYP:TVE:RXGXS, AAPASXX:VPE:SSLS-AA17-LFF:LKVYP:TVE:AXGXR, STPPTXX:VPE:SSLS-AA17-LFF:LKVYP:TVE:SXGXR, HVPKPXX:VPE:SSLS-AA17-LFF:LKVYP:TVE:SXGXH, RVPSTXX:VPE:SSLS-AA17-LFF:LKVYP:TVE:XGXL, ASAAPXX:VPE:SSLS-AA17-LFF:LKVYP:TVE:XKXS, ASASPXX:VPE:SSLS-AA17-LFF:LKVYP:TVE:XKXS, KIPKAXX:VPT:SSLS-AA17-LFF:LKVYP:VVE:GXGXR, SIPKAXX:VPT:SSLS-AA17-LFF:LKVYP:VVE:GXGXR, HVTKPTX:APT:SSLS-AA17-LFF:LKVYP:VVR:SXGXH, YVPKPXX:APT:SSLS-AA17-LFF:LKVYP:VVR:SXGXH, TVPKPXX:APT:SSLS-AA17-LFF:LKVYP:VVR:AXGXH, AVPKAXX:APT:SSLS-AA17-LFF:LKVYP:VVK:AXGXH, KVGKAXX:VPT:SSLS-AA17-LFF:LKVYP:VAE:XGXR, KASKAXX:VPT:SSLS-AA17-LFF:LKVYP:AVS:EXGXR, GSAGPXX:TPT:SSLS-AA17-LFF:LKVYP:VVD:RXGXS, AAPASXX:VPA:SSLS-AA17-LFF:LKVYP:VVE:AXGXR, STPPTXX:VPT:SSLS-AA17-LFF:LKVYP:VVE:SXGXR, HVPKPXX:APT:SSLS-AA17-LFF:LKVYP:VVR:SXGXH, RVPSTXX:APV:SSLS-AA17-LFF:LKVYP:VEE:XGXL, ASAAPXX:VPQ:SSLS-AA17-LFF:LKVYP:VRS:XKXS, ASASPXX:VSQ:SSLS-AA17-LFF:LKVYP:VKS:XKXS, ASASPXX:VPQ:SSLS-AA17-LFF:LKVYP:VKS:XKXS, NDEGLEX:VPT:SSLS-AA17-LFF:LKVYP:QHN:KXEXR, NDEGLEX:VPT:SSLS-AA17-LFF:LKVYP:EHS:QXEXR, SSVKXQP:SRV:SSLS-AA17-LFF:LKVYP:EEH:LEXAXA, RNVQXRP:TQV:SSLS-AA17-LFF:LKVYP:EDH:LAXKXE, KIPKAXX:APT:NAIS-AA17-LYF:LKKYR:VVR:GXGXR, GIPEPXX:APT:NAIS-AA17-LYF:LKKYR:VVR:SXAXR, SIPKAXX:APT:NAIS-AA17-LYF:LKKYR:VVR:GXGXR, AVPKAXX:APT:NAIS-AA17-LYF:LKKYR:VVR:AXGXH, KVGKAXX:APT:NAIS-AA17-LYF:LKKYR:VVR:XGXR, KASKAXX:APT:NAIS-AA17-LYF:LKKYR:VVR:EXGXR, GSAGPXX:APT:NAIS-AA17-LYF:LKKYR:VVR:RXGXS, AAPASXX:APT:NAIS-AA17-LYF:LKKYR:VVR:AXGXR, STPPTXX:APT:NAIS-AA17-LYF:LKKYR:VVR:SXGXR, RVPSTXX:APT:NAIS-AA17-LYF:LKKYR:VVR:XGXL, ASAAPXX:APT:NAIS-AA17-LYF:LKKYR:VVR:XKXS, ASASPXX:APT:NAIS-AA17-LYF:LKKYR:VVR:XKXS, KIPKAXX:VPT:NAIS-AA17-LYF:LKKYR:VVE:GXGXR, GIPEPXX:VPE:NAIS-AA17-LYF:LKKYR:TVE:SXAXR, SIPKAXX:VPT:NAIS-AA17-LYF:LKKYR:VVE:GXGXR, AVPKAXX:APT:NAIS-AA17-LYF:LKKYR:VVK:AXGXH, KVGKAXX:VPT:NAIS-AA17-LYF:LKKYR:VAE:XGXR, KASKAXX:VPT:NAIS-AA17-LYF:LKKYR:AVS:EXGXR, GSAGPXX:TPT:NAIS-AA17-LYF:LKKYR:VVD:RXGXS, AAPASXX:VPA:NAIS-AA17-LYF:LKKYR:VVE:AXGXR, STPPTXX:VPT:NAIS-AA17-LYF:LKKYR:VVE:SXGXR, RVPSTXX:APV:NAIS-AA17-LYF:LKKYR:VEE:XGXL, ASAAPXX:VPQ:NAIS-AA17-LYF:LKKYR:VRS:XKXS, ASASPXX:VSQ:NAIS-AA17-LYF:LKKYR:VKS:XKXS, ASASPXX:VPQ:NAIS-AA17-LYF:LKKYR:VKS:XKXS, NDEGLEX:VPT:NAIS-AA17-LYF:LKKYR:QHN:KXEXR, NDEGLEX:VPT:NAIS-AA17-LYF:LKKYR:EHS:QXEXR, SSVKXQP:SRV:NAIS-AA17-LYF:LKKYR:EEH:LEXAXA, RNVQXRP:TQV:NAIS-AA17-LYF:LKKYR:EDH:LAXKXE, KIPKAXX:APT:SATS-AA17-LYY:LRKHR:VVK:GXGXR, GIPEPXX:APT:SATS-AA17-LYY:LRKHR:VVK:SXAXR, SIPKAXX:APT:SATS-AA17-LYY:LRKHR:VVK:GXGXR, HVTKPTX:APT:SATS-AA17-LYY:LRKHR:VVK:SXGXH, YVPKPXX:APT:SATS-AA17-LYY:LRKHR:VVK:SXGXH, TVPKPXX:APT:SATS-AA17-LYY:LRKHR:VVK:AXGXH, KVGKAXX:APT:SATS-

$AA^{17}$-LYY:LRKHR:VVK:XGXR, KASKAXX:APT:SATS-$AA^{17}$-LYY:LRKHR:VVK:EXGXR, GSAGPXX:APT:SATS-$AA^{17}$-LYY:LRKHR:VVK:RXGXS, AAPASXX:APT:SATS-$AA^{17}$-LYY:LRKHR:VVK:AXGXR, STPPTXX:APT:SATS-$AA^{17}$-LYY:LRKHR:VVK:SXGXR, HVPKPXX:APT:SATS-$AA^{17}$LYY:LRKHR:VVK:SXGXH, RVPSTXX:APT:SATS-$AA^{17}$-LYY:LRKHR:VVK:XGXL, ASAAPXX:APT:SATS-$AA^{17}$-LYY:LRKHR:VVK:XKXS, ASASPXX:APT:SATS-$AA^{17}$-LYY:LRKHR:VVK:XKXS, KIPKAXX:VPT:SATS-$AA^{17}$-LYY:LRKHR:VVE:GXGXR, GIPEPXX:VPE:SATS-$AA^{17}$-LYY:LRKHR:TVE:SXAXR, SIPKAXX:VPT:SATS-$AA^{17}$-LYY:LRKHR:VVE:GXGXR, HVTKPTX:APT:SATS-$AA^{17}$-LYY:LRKHR:VVR:SXGXH, YVPKPXX:APT:SATS-$AA^{17}$-LYY:LRKHR:VVR:SXGXH, TVPKPXX:APT:SATS-$AA^{17}$-LYY:LRKHR:VVR:AXGXH, KVGKAXX:VPT:SATS-$AA^{17}$-LYY:LRKHR:VAE:XGXR, KASKAXX:VPT:SATS-$AA^{17}$-LYY:LRKHR:AVS:EXGXR, GSAGPXX:TPT:SATS-$AA^{17}$-LYY:LRKHR:VVD:RXGXS, AAPASXX:VPA:SATS-$AA^{17}$-LYY:LRKHR:VVE:AXGXR, STPPTXX:VPT:SATS-$AA^{17}$-LYY:LRKHR:VVE:SXGXR, HVPKPXX:APT:SATS-$AA^{17}$-LYY:LRKHR:VVR:SXGXH, RVPSTXX:APV:SATS-$AA^{17}$-LYY:LRKHR:VEE:XGXL, ASAAPXX:VPQ:SATS-$AA^{17}$-LYY:LRKHR:VRS:XKXS, ASASPXX:VSQ:SATS-$AA^{17}$-LYY:LRKHR:VKS:XKXS, ASASPXX:VPQ:SATS-$AA^{17}$-LYY:LRKHR:VKS:XKXS, NDEGLEX:VPT:SATS-$AA^{17}$-LYY:LRKHR:QHN:KXEXR, NDEGLEX:VPT:SATS-$AA^{17}$-LYY:LRKHR:EHS:QXEXR, SSVKXQP:SRV:SATS-$AA^{17}$-LYY:LRKHR:EEH:LEXAXA, RNVQXRP:TQV:SATS-$AA^{17}$-LYY:LRKHR:EDH:LAXKXE, KIPKAXX:VPT:SPIS-$AA^{17}$-LYK:LKYHY:VAE:GXGXR, GIPEPXX:VPT:SPIS-$AA^{17}$-LYK:LKYHY:VAE:SXAXR, SIPKAXX:VPT:SPIS-$AA^{17}$-LYK:LKYHY:VAE:GXGXR, HVTKPTX:VPT:SPIS-$AA^{17}$-LYK:LKYHY:VAE:SXGXH, YVPKPXX:VPT:SPIS-$AA^{17}$-LYK:LKYHY:VAE:SXGXH, TVPKPXX:VPT:SPIS-$AA^{17}$-LYK:LKYHY:VAE:AXGXH, AVPKAXX:VPT:SPIS-$AA^{17}$-LYK:LKYHY:VAE:AXGXH, KASKAXX:VPT:SPIS-$AA^{17}$-LYK:LKYHY:VAE:EXGXR, GSAGPXX:VPT:SPIS-$AA^{17}$-LYK:LKYHY:VAE:RXGXS, AAPASXX:VPT:SPIS-$AA^{17}$-LYK:LKYHY:VAE:AXGXR, STPPTXX:VPT:SPIS-$AA^{17}$-LYK:LKYHY:VAE:SXGXR, HVPKPXX:VPT:SPIS-$AA^{17}$-LYK:LKYHY:VAE:SXGXH, RVPSTXX:VPT:SPIS-$AA^{17}$-LYK:LKYHY:VAE:XGXL, ASAAPXX:VPT:SPIS-$AA^{17}$-LYK:LKYHY:VAE:XKXS, ASASPXX:VPT:SPIS-$AA^{17}$-LYK:LKYHY:VAE:XKXS, KIPKAXX:VPT:SPIS-$AA^{17}$-LYK:LKYHY:VVE:GXGXR, GIPEPXX:VPE:SPIS-$AA^{17}$-LYK:LKYHY:TVE:SXAXR, SIPKAXX:VPT:SPIS-$AA^{17}$-LYK:LKYHY:VVE:GXGXR, HVTKPTX:APT:SPIS-$AA^{17}$-LYK:LKYHY:VVR:SXGXH, YVPKPXX:APT:SPIS-$AA^{17}$-LYK:LKYHY:VVR:SXGXH, TVPKPXX:APT:SPIS-$AA^{17}$-LYK:LKYHY:VVR:AXGXH, AVPKAXX:APT:SPIS-$AA^{17}$-LYK:LKYHY:VVK:AXGXH, KASKAXX:VPT:SPIS-$AA^{17}$-LYK:LKYHY:AVS:EXGXR, GSAGPXX:TPT:SPIS-$AA^{17}$-LYK:LKYHY:VVD:RXGXS, AAPASXX:VPA:SPIS-$AA^{17}$-LYK:LKYHY:VVE:AXGXR, STPPTXX:VPT:SPIS-$AA^{17}$-LYK:LKYHY:VVE:SXGXR, HVPKPXX:APT:SPIS-$AA^{17}$-LYK:LKYHY:VVR:SXGXH, RVPSTXX:APV:SPIS-$AA^{17}$-LYK:LKYHY:VEE:XGXL, ASAAPXX:VPQ:SPIS-$AA^{17}$-LYK:LKYHY:VRS:XKXS, ASASPXX:VSQ:SPIS-$AA^{17}$-LYK:LKYHY:VKS:XKXS, ASASPXX:VPQ:SPI-$AA^{17}$-LYK:LKYHY:VKS:XKXS, NDEGLEX:VPT:SPIS-$AA^{17}$-LYK:LKYHY:QHN:KXEXR, NDEGLEX:VPT:SPIS-$AA^{17}$-LYK:LKYHY:EHS:QXEXR, SSVKXQP:SRV:SPIS-$AA^{17}$-LYK:LKYHY:EEH:LEXAXA, RNVQXRP:TQV:SPIS-$AA^{17}$-LYK:LKYHY:EDH:LAXKXE, KIPKAXX:VPT:EPIS-$AA^{17}$-LYL:KFKYE:AVS:GXGXR, GIPEPXX:VPT:EPIS-$AA^{17}$-LYL:KFKYE:AVS:SXAXR, SIPKAXX:VPT:EPIS-$AA^{17}$-LYL:KFKYE:AVS:GXGXR, HVTKPTX:VPT:EPIS-$AA^{17}$-LYL:KFKYE:AVS:SXGXH, YVPKPXX:VPT:EPIS-$AA^{17}$-LYL:KFKYE:AVS:SXGXH, TVPKPXX:VPT:EPIS-$AA^{17}$-LYL:KFKYE:AVS:AXGXH, AVPKAXX:VPT:EPIS-$AA^{17}$-LYL:KFKYE:AVS:AXGXH, KVGKAXX:VPT:EPIS-$AA^{17}$-LYL:KFKYE:AVS:XGXR, GSAGPXX:VPT:EPIS-$AA^{17}$-LYL:KFKYE:AVS:RXGXS, AAPASXX:VPT:EPIS-$AA^{17}$-LYL:KFKYE:AVS:AXGXR, STPPTXX:VPT:EPIS-$AA^{17}$-LYL:KFKYE:AVS:SXGXR, HVPKPXX:VPT:EPIS-$AA^{17}$-LYL:KFKYE:AVS:SXGXH, RVPSTXX:VPT:EPIS-$AA^{17}$-LYL:KFKYE:AVS:XGXL, ASAAPXX:VPT:EPIS-$AA^{17}$-LYL:KFKYE:AVS:XKXS, ASASPXX:VPT:EPIS-$AA^{17}$-LYL:KFKYE:AVS:XKXS, KIPKAXX:VPT:EPIS-$AA^{17}$-LYL:KFKYE:VVE:GXGXR, GIPEPXX:VPE:EPIS-$AA^{17}$-LYL:KFKYE:TVE:SXAXR, SIPKAXX:VPT:EPIS-$AA^{17}$-LYL:KFKYE:VVE:GXGXR, HVTKPTX:APT:EPIS-$AA^{17}$-LYL:KFKYE:VVR:SXGXH, YVPKPXX:APT:EPIS-$AA^{17}$-LYL:KFKYE:VVR:SXGXH, TVPKPXX:APT:EPIS-$AA^{17}$-LYL:KFKYE:VVR:AXGXH, AVPKAXX:APT:EPIS-$AA^{17}$-LYL:KFKYE:VVK:AXGXH, KVGKAXX:VPT:EPIS-$AA^{17}$-LYL:KFKYE:VAE:XGXR, GSAGPXX:TPT:EPIS-$AA^{17}$-LYL:KFKYE:VVD:RXGXS, AAPASXX:VPA:EPIS-$AA^{17}$-LYL:KFKYE:VVE:AXGXR, STPPTXX:VPT:EPIS-$AA^{17}$-LYL:KFKYE:VVE:SXGXR, HVPKPXX:APT:EPIS-$AA^{17}$-LYL:KFKYE:VVR:SXGXH, RVPSTXX:APV:EPIS-$AA^{17}$-LYL:KFKYE:VEE:XGXL, ASAAPXX:VPQ:EPIS-$AA^{17}$-LYL:KFKYE:VRS:XKXS, ASASPXX:VSQ:EPIS-$AA^{17}$-LYL:KFKYE:VKS:XKXS, ASASPXX:VPQ:EPIS-$AA^{17}$-LYL:KFKYE:VKS:XKXS, NDEGLEX:VPT:EPIS-$AA^{17}$-LYL:KFKYE:QHN:KXEXR, NDEGLEX:VPT:EPIS-$AA^{17}$-LYL:KFKYE:EHS:QXEXR, SSVKXQP:SRV:EPIS-$AA^{17}$-LYL:KFKYE:EEH:LEXAXA, RNVQXRP:TQV:EPIS-$AA^{17}$-LYL:KFKYE:EDH:LAXKXE, KIPKAXX:TPT:SPIN-$AA^{17}$-LYF:YGKIP:VVD:GXGXR, GIPEPXX:TPT:SPIN-$AA^{17}$-LYF:YGKIP:VVD:SXAXR, SIPKAXX:TPT:SPIN-$AA^{17}$-LYF:YGKIP:VVD:GXGXR, HVTKPTX:TPT:SPIN-$AA^{17}$-LYF:YGKIP:VVD:SXGXH, YVPKPXX:TPT:SPIN-$AA^{17}$-LYF:YGKIP:VVD:SXGXH, TVPKPXX:TPT:SPIN-$AA^{17}$-LYF:YGKIP:VVD:AXGXH, AVPKAXX:TPT:SPIN-$AA^{17}$-LYF:YGKIP:VVD:AXGXH, KVGKAXX:TPT:SPIN-$AA^{17}$-LYF:YGKIP:VVD:XGXR, KASKAXX:TPT:SPIN-$AA^{17}$-LYF:YGKIP:VVD:EXGXR, AAPASXX:TPT:SPIN-$AA^{17}$-LYF:YGKIP:VVD:AXGXR, STPPTXX:TPT:SPIN-$AA^{17}$-LYF:YGKIP:VVD:SXGXR, HVPKPXX:TPT:SPIN-$AA^{17}$-LYF:YGKIP:VVD:SXGXH, RVPSTXX:TPT:SPIN-$AA^{17}$-LYF:YGKIP:VVD:XGXL, ASAAPXX:TPT:SPIN-$AA^{17}$-LYF:YGKIP:VVD:XKXS, ASASPXX:TPT:SPIN-$AA^{17}$-LYF:YGKIP:VVD:XKXS, KIPKAXX:VPT:SPIN-$AA^{17}$-LYF:YGKIP:VVE:GXGXR, GIPEPXX:VPE:SPIN-$AA^{17}$-LYF:YGKIP:TVE:SXAXR, SIPKAXX:VPT:SPIN-

$AA^{17}$-LYF:YGKIP:VVE:GXGXR, HVTKPTX:APT:SPIN-$AA^{17}$-LYF:YGKIP:VVR:SXGXH, YVPKPXX:APT:SPIN-$AA^{17}$-LYF:YGKIP:VVR:SXGXH, TVPKPXX:APT:SPIN-$AA^{17}$-LYF:YGKIP:VVR:AXGXH, AVPKAXX:APT:SPIN-$AA^{17}$-LYF:YGKIP:VVK:AXGXH, KVGKAXX:VPT:SPIN-$AA^{17}$-LYF:YGKIP:VAE:XGXR, KASKAXX:VPT:SPIN-$AA^{17}$-LYF:YGKIP:AVS:EXGXR, AAPASXX:VPA:SPIN-$AA^{17}$-LYF:YGKIP:VVE:AXGXR, STPPTXX:VPT:SPIN-$AA^{17}$-LYF:YGKIP:VVE:SXGXR, HVPKPXX:APT:SPIN-$AA^{17}$-LYF:YGKIP:VVR:SXGXH, RVPSTXX:APV:SPIN-$AA^{17}$-LYF:YGKIP:VEE:XGXL, ASAAPXX:VPQ:SPIN-$AA^{17}$-LYF:YGKIP:VRS:XKXS, ASASPXX:VSQ:SPIN-$AA^{17}$-LYF:YGKIP:VKS:XKXS, ASASPXX:VPQ:SPIN-$AA^{17}$-LYF:YGKIP:VKS:XKXS, NDEGLEX:VPT:SPIN-$AA^{17}$-LYF:YGKIP:QHN:KXEXR, NDEGLEX:VPT:SPIN-$AA^{17}$-LYF:YGKIP:EHS:QXEXR, SSVKXQP:SRV:SPIN-$AA^{17}$-LYF:YGKIP:EEH:LEXAXA, RNVQXRP:TQV:SPIN-$AA^{17}$-LYF:YGKIP:EDH:LAXKXE, KIPKAXX:VPA:SPIS-$AA^{17}$-LYI:YKQYE:VVE:GXGXR, GIPEPXX:VPA:SPIS-$AA^{17}$-LYI:YKQYE:VVE:SXAXR, SIPKAXX:VPA:SPIS-$AA^{17}$-LYI:YKQYE:VVE:GXGXR, HVTKPTX:VPA:SPIS-$AA^{17}$-LYI:YKQYE:VVE:SXGXH, YVPKPXX:VPA:SPIS-$AA^{17}$-LYI:YKQYE:VVE:SXGXH, TVPKPXX:VPA:SPIS-$AA^{17}$-LYI:YKQYE:VVE:AXGXH, AVPKAXX:VPA:SPIS-$AA^{17}$-LYI:YKQYE:VVE:AXGXH, KVGKAXX:VPA:SPIS-$AA^{17}$-LYI:YKQYE:VVE:XGXR, KASKAXX:VPA:SPIS-$AA^{17}$-LYI:YKQYE:VVE:EXGXR, GSAGPXX:VPA:SPIS-$AA^{17}$-LYI:YKQYE:VVE:RXGXS, STPPTXX:VPA:SPIS-$AA^{17}$-LYI:YKQYE:VVE:SXGXR, HVPKPXX:VPA:SPIS-$AA^{17}$-LYI:YKQYE:VVE:SXGXH, RVPSTXX:VPA:SPIS-$AA^{17}$-LYI:YKQYE:VVE:XGXL, ASAAPXX:VPA:SPIS-$AA^{17}$-LYI:YKQYE:VVE:XKXS, ASASPXX:VPA:SPIS-$AA^{17}$-LYI:YKQYE:VVE:XKXS, KIPKAXX:VPT:SPIS-$AA^{17}$-LYI:YKQYE:VVE:GXGXR, GIPEPXX:VPE:SPIS-$AA^{17}$-LYI:YKQYE:TVE:SXAXR, SIPKAXX:VPT:SPIS-$AA^{17}$-LYI:YKQYE:VVE:GXGXR, HVTKPTX:APT:SPIS-$AA^{17}$-LYI:YKQYE:VVR:SXGXH, YVPKPXX:APT:SPIS-$AA^{17}$-LYI:YKQYE:VVR:SXGXH, TVPKPXX:APT:SPIS-$AA^{17}$-LYI:YKQYE:VVR:AXGXH, AVPKAXX:APT:SPIS-$AA^{17}$-LYI:YKQYE:VVK:AXGXH, KVGKAXX:VPT:SPIS-$AA^{17}$-LYI:YKQYE:VAE:XGXR, KASKAXX:VPT:SPIS-$AA^{17}$-LYI:YKQYE:AVS:EXGXR, GSAGPXX:TPT:SPIS-$AA^{17}$-LYI:YKQYE:VVD:RXGXS, STPPTXX:VPT:SPIS-$AA^{17}$-LYI:YKQYE:VVE:SXGXR, HVPKPXX:APT:SPIS-$AA^{17}$-LYI:YKQYE:VVR:SXGXH, RVPSTXX:APV:SPIS-$AA^{17}$-LYI:YKQYE:VEE:XGXL, ASAAPXX:VPQ:SPIS-$AA^{17}$-LYI:YKQYE:VRS:XKXS, ASASPXX:VSQ:SPIS-$AA^{17}$-LYI:YKQYE:VKS:XKXS, ASASPXX:VPQ:SPIS-$AA^{17}$-LYI:YKQYE:VKS:XKXS, NDEGLEX:VPT:SPIS-$AA^{17}$-LYI:YKQYE:QHN:KXEXR, NDEGLEX:VPT:SPIS-$AA^{17}$-LYI:YKQYE:EHS:QXEXR, SSVKXQP:SRV:SPIS-$AA^{17}$-LYI:YKQYE:EEH:LEXAXA, RNVQXRP:TQV:SPIS-$AA^{17}$-LYI:YKQYE:EDH:LAXKXE, KIPKAXX:VPT:SPIS-$AA^{17}$-LFI:YKQYE:VVE:GXGXR, GIPEPXX:VPT:SPIS-$AA^{17}$-LFI:YKQYE:VVE:SXAXR, SIPKAXX:VPT:SPIS-$AA^{17}$-LFI:YKQYE:VVE:GXGXR, HVTKPTX:VPT:SPIS-$AA^{17}$-LFI:YKQYE:VVE:SXGXH, YVPKPXX:VPT:SPIS-$AA^{17}$-LFI:YKQYE:VVE:SXGXH, TVPKPXX:VPT:SPIS-$AA^{17}$-LFI:YKQYE:VVE:AXGXH, AVPKAXX:VPT:SPIS-$AA^{17}$-LFI:YKQYE:VVE:AXGXH, KVGKAXX:VPT:SPIS-$AA^{17}$-LFI:YKQYE:VVE:XGXR, KASKAXX:VPT:SPIS-$AA^{17}$-LFI:YKQYE:VVE:EXGXR, GSAGPXX:VPT:SPIS-$AA^{17}$-LFI:YKQYE:VVE:RXGXS, AAPASXX:VPT:SPIS-$AA^{17}$-LFI:YKQYE:VVE:AXGXR, HVPKPXX:VPT:SPIS-$AA^{17}$-LFI:YKQYE:VVE:SXGXH, RVPSTXX:VPT:SPIS-$AA^{17}$-LFI:YKQYE:VVE:XGXL, ASAAPXX:VPT:SPIS-$AA^{17}$-LFI:YKQYE:VVE:XKXS, ASASPXX:VPT:SPIS-$AA^{17}$-LFI:YKQYE:VVE:XKXS, GIPEPXX:VPE:SPIS-$AA^{17}$-LFI:YKQYE:TVE:SXAXR, HVTKPTX:APT:SPIS-$AA^{17}$-LFI:YKQYE:VVR:SXGXH, YVPKPXX:APT:SPIS-$AA^{17}$-LFI:YKQYE:VVR:SXGXH, TVPKPXX:APT:SPIS-$AA^{17}$-LFI:YKQYE:VVR:AXGXH, AVPKAXX:APT:SPIS-$AA^{17}$-LFI:YKQYE:VVK:AXGXH, KVGKAXX:VPT:SPIS-$AA^{17}$-LFI:YKQYE:VAE:XGXR, KASKAXX:VPT:SPIS-$AA^{17}$-LFI:YKQYE:AVS:EXGXR, GSAGPXX:TPT:SPIS-$AA^{17}$-LFI:YKQYE:VVD:RXGXS, AAPASXX:VPA:SPIS-$AA^{17}$-LFI:YKQYE:VVE:AXGXR, HVPKPXX:APT:SPIS-$AA^{17}$-LFI:YKQYE:VVR:SXGXH, RVPSTXX:APV:SPIS-$AA^{17}$-LFI:YKQYE:VEE:XGXL, ASAAPXX:VPQ:SPIS-$AA^{17}$-LFI:YKQYE:VRS:XKXS, ASASPXX:VSQ:SPIS-$AA^{17}$-LFI:YKQYE:VKS:XKXS, ASASPXX:VPQ:SPIS-$AA^{17}$-LFI:YKQYE:VKS:XKXS, NDEGLEX:VPT:SPIS-$AA^{17}$-LFI:YKQYE:QHN:KXEXR, NDEGLEX:VPT:SPIS-$AA^{17}$-LFI:YKQYE:EHS:QXEXR, SSVKXQP:SRV:SPIS-$AA^{17}$-LFI:YKQYE:EEH:LEXAXA, RNVQXRP:TQV:SPIS-$AA^{17}$-LFI:YKQYE:EDH:LAXKXE, KIPKAXX:APV:KPLS-$AA^{17}$-LYV:DHHKD:VEE:GXGXR, GIPEPXX:APV:KPLS-$AA^{17}$-LYV:DHHKD:VEE:SXAXR, SIPKAXX:APV:KPLS-$AA^{17}$-LYV:DHHKD:VEE:GXGXR, HVTKPTX:APV:KPLS-$AA^{17}$-LYV:DHHKD:VEE:SXGXH, YVPKPXX:APV:KPLS-$AA^{17}$-LYV:DHHKD:VEE:SXGXH, TVPKPXX:APV:KPLS-$AA^{17}$-LYV:DHHKD:VEE:AXGXH, AVPKAXX:APV:KPLS-$AA^{17}$-LYV:DHHKDVEE:AXGXH, KVGKAXX:APV:KPLS-$AA^{17}$-LYV:DHHKD:VEE:XGXR, KASKAXX:APV:KPLS-$AA^{17}$-LYV:DHHKD:VEE:EXGXR, GSAGPXX:APV:KPLS-$AA^{17}$-LYV:DHHKD:VEE:RXGXS, AAPASXX:APV:KPLS-$AA^{17}$-LYV:DHHKD:VEE:AXGXR, STPPTXX:APV:KPLS-$AA^{17}$-LYV:DHHKD:VEE:SXGXR, HVPKPXX:APV:KPLS-$AA^{17}$-LYV:DHHKD:VEE:SXGXH, ASAAPXX:APV:KPLS-$AA^{17}$-LYV:DHHKD:VEE:XKXS, ASASPXX:APV:KPLS-$AA^{17}$-LYV:DHHKD:VEE:XKXS, KIPKAXX:VPT:KPLS-$AA^{17}$-LYV:DHHKD:VVE:GXGXR, GIPEPXX:VPE:KPLS-$AA^{17}$-LYV:DHHKD:TVE:SXAXR, SIPKAXX:VPT:KPLS-$AA^{17}$-LYV:DHHKD:VVE:GXGXR, HVTKPTX:APT:KPLS-$AA^{17}$-LYV:DHHKD:VVR:SXGXH, YVPKPXX:APT:KPLS-$AA^{17}$-LYV:DHHKD:VVR:SXGXH, TVPKPXX:APT:KPLS-$AA^{17}$-LYV:DHHKD:VVR:AXGXH, AVPKAXX:APT:KPLS-$AA^{17}$-LYV:DHHKD:VVK:AXGXH, KVGKAXX:VPT:KPLS-$AA^{17}$-LYV:DHHKD:VAE:XGXR, KASKAXX:VPT:KPLS-$AA^{17}$-LYV:DHHKD:AVS:EXGXR, GSAGPXX:TPT:KPLS-$AA^{17}$-LYV:DHHKD:VVD:RXGXS, AAPASXX:VPA:KPLS-$AA^{17}$-LYV:DHHKD:VVE:AXGXR, STPPTXX:VPT:KPLS-$AA^{17}$-LYV:DHHKD:VVE:SXGXR, HVPKPXX:APT:KPLS-$AA^{17}$-LYV:DHHKD:VVR:SXGXH, ASAAPXX:VPQ:KPLS-$AA^{17}$-LYV:DHHKD:VRS:XKXS, ASASPXX:VSQ:KPLS-$AA^{17}$-LYV:DHHKD:VKS:XKXS, ASASPXX:VPQ:KPLS-

AA$^{17}$-LYV:DHHKD:VKS:XKXS, NDEGLEX:VPT:KPLS-AA$^{17}$-LYV:DHHKD:QHN:KXEXR, NDEGLEX:VPT:KPLS-
AA$^{17}$-LYV:DHHKD:EHS:QXEXR, SSVKXQP:SRV:KPLS-AA$^{17}$-LYV:DHHKD:EEH:LEXAXA, RNVQXRP:TQV:KPLS-
AA$^{17}$-LYV:DHHKD:EDH:LAXKXE, KIPKAXX:VPQ:EPLP-AA$^{17}$-VYY:EQLSN:VRS:GXGXR, GIPEPXX:VPQ:EPLP-
AA$^{17}$-VYY:EQLSN:VRS:SXAXR, SIPKAXX:VPQ:EPLP-AA$^{17}$-VYY:EQLSN:VRS:GXGXR, HVTKPTX:VPQ:EPLP-
AA$^{17}$-VYY:EQLSN:VRS:SXGXH, YVPKPXX:VPQ:EPLP-AA$^{17}$-VYY:EQLSN:VRS:SXGXH, TVPKPXX:VPQ:EPLP-
AA$^{17}$-VYY:EQLSN:VRS:AXGXH, AVPKAXX:VPQ:EPLP-AA$^{17}$-VYY:EQLSN:VRS:AXGXH, KVGKAXX:VPQ:EPLP-
AA$^{17}$-VYY:EQLSN:VRS:XGXR, KASKAXX:VPQ:EPLP-AA$^{17}$-VYY:EQLSN:VRS:EXGXR, GSAGPXX:VPQ:EPLP-
AA$^{17}$-VYY:EQLSN:VRS:RXGXS, AAPASXX:VPQ:EPLP-AA$^{17}$-VYY:EQLSN:VRS:AXGXR, STPPTXX:VPQ:EPLP-
AA$^{17}$-VYY:EQLSN:VRS:SXGXR, HVPKPXX:VPQ:EPLP-AA$^{17}$-VYY:EQLSN:VRS:SXGXH, RVPSTXX:VPQ:EPLP-
AA$^{17}$-VYY:EQLSN:VRS:XGXL, ASASPXX:VPQ:EPLP-AA$^{17}$-VYY:EQLSN:VRS:XKXS, KIPKAXX:VPT:EPLP-
AA$^{17}$-VYY:EQLSN:VVE:GXGXR, GIPEPXX:VPE:EPLP-AA$^{17}$-VYY:EQLSN:TVE:SXAXR, SIPKAXX:VPT:EPLP-
AA$^{17}$-VYY:EQLSN:VVE:GXGXR, HVTKPTX:APT:EPLP-AA$^{17}$-VYY:EQLSN:VVR:SXGXH, YVPKPXX:APT:EPLP-
AA$^{17}$-VYY:EQLSN:VVR:SXGXH, TVPKPXX:APT:EPLP-AA$^{17}$-VYY:EQLSN:VVR:AXGXH, AVPKAXX:APT:EPLP-
AA$^{17}$-VYY:EQLSN:VVK:AXGXH, KVGKAXX:VPT:EPLP-AA$^{17}$-VYY:EQLSN:VAE:XGXR, KASKAXX:VPT:EPLP-
AA$^{17}$-VYY:EQLSN:AVS:EXGXR, GSAGPXX:TPT:EPLP-AA$^{17}$-VYY:EQLSN:VVD:RXGXS, AAPASXX:VPA:EPLP-
AA$^{17}$-VYY:EQLSN:VVE:AXGXR, STPPTXX:VPT:EPLP-AA$^{17}$-VYY:EQLSN:VVE:SXGXR, HVPKPXX:APT:EPLP-
AA$^{17}$-VYY:EQLSN:VVR:SXGXH, RVPSTXX:APV:EPLP-AA$^{17}$-VYY:EQLSN:VEE:XGXL, ASASPXX:VSQ:EPLP-
AA$^{17}$-VYY:EQLSN:VKS:XKXS, ASASPXX:VPQ:EPLP-AA$^{17}$-VYY:EQLSN:VKS:XKXS, NDEGLEX:VPT:EPLP-
AA$^{17}$-VYY:EQLSN:QHN:KXEXR, NDEGLEX:VPT:EPLP-AA$^{17}$-VYY:EQLSN:EHS:QXEXR, SSVKXQP:SRV:EPLP-
AA$^{17}$-VYY:EQLSN:EEH:LEXAXA, RNVQXRP:TQV:EPLP-AA$^{17}$-VYY:EQLSN:EDH:LAXKXE, KIPKAXXVSQ:EPLT-
AA$^{17}$-LYY:EQLSN:VKS:GXGXR, GIPEPXX:VSQ:EPLT-AA$^{17}$-LYY:EQLSN:VKS:SXAXR, SIPKAXX:VSQ:EPLT-
AA$^{17}$-LYY:EQLSN:VKS:GXGXR, HVTKPTX:VSQ:EPLT-AA$^{17}$-LYY:EQLSN:VKS:SXGXH, YVPKPXX:VSQ:EPLT-
AA$^{17}$-LYY:EQLSN:VKS:SXGXH, TVPKPXX:VSQ:EPLT-AA$^{17}$-LYY:EQLSN:VKS:AXGXH, AVPKAXX:VSQ:EPLT-
AA$^{17}$-LYY:EQLSN:VKS:AXGXH, KVGKAXX:VSQ:EPLT-AA$^{17}$-LYY:EQLSN:VKS:XGXR, KASKAXX:VSQ:EPLT-
AA$^{17}$-LYY:EQLSN:VKS:EXGXR, GSAGPXX:VSQ:EPLT-AA$^{17}$-LYY:EQLSN:VKS:RXGXS, AAPASXX:VSQ:EPLT-
AA$^{17}$-LYY:EQLSN:VKS:AXGXR, STPPTXX:VSQ:EPLT-AA$^{17}$-LYY:EQLSN:VKS:SXGXR, HVPKPXX:VSQ:EPLT-
AA$^{17}$-LYY:EQLSN:VKS:SXGXH, RVPSTXX:VSQ:EPLT-AA$^{17}$-LYY:EQLSN:VKS:XGXL, ASAAPXX:VSQ:EPLT-
AA$^{17}$-LYY:EQLSN:VKS:XKXS, ASASPXX:VSQ:EPLT-AA$^{17}$-LYY:EQLSN:VKS:XKXS, KIPKAXX:VPT:EPLT-
AA$^{17}$-LYY:EQLSN:VVE:GXGXR, GIPEPXX:VPE:EPLT-AA$^{17}$-LYY:EQLSN:TVE:SXAXR, SIPKAXX:VPT:EPLT-
AA$^{17}$-LYY:EQLSN:VVE:GXGXR, HVTKPTX:APT:EPLT-AA$^{17}$-LYY:EQLSN:VVR:SXGXH, YVPKPXX:APT:EPLT-
AA$^{17}$-LYY:EQLSN:VVR:SXGXH, TVPKPXX:APT:EPLT-AA$^{17}$-LYY:EQLSN:VVR:AXGXH, AVPKAXX:APT:EPLT-
AA$^{17}$-LYY:EQLSN:VVK:AXGXH, KVGKAXX:VPT:EPLT-AA$^{17}$-LYY:EQLSN:VAE:XGXR, KASKAXXVPT:EPLT-
AA$^{17}$-LYY:EQLSN:AVS:EXGXR, GSAGPXX:TPT:EPLT-AA$^{17}$-LYY:EQLSN:VVD:RXGXS, AAPASXX:VPA:EPLT-
AA$^{17}$-LYY:EQLSN:VVE:AXGXR, STPPTXX:VPT:EPLT-AA$^{17}$-LYY:EQLSN:VVE:SXGXR, HVPKPXX:APT:EPLT-
AA$^{17}$-LYY:EQLSN:VVR:SXGXH, RVPSTXX:APV:EPLT-AA$^{17}$-LYY:EQLSN:VEE:XGXL, ASAAPXX:VPQ:EPLT-
AA$^{17}$-LYY:EQLSN:VRS:XKXS, NDEGLEX:VPT:EPLT-AA$^{17}$-LYY:EQLSN:QHN:KXEXR, NDEGLEX:VPT:EPLT-
AA$^{17}$-LYY:EQLSN:EHS:QXEXR, SSVKXQP:SRV:EPLT-AA$^{17}$-LYY:EQLSN:EEH:LEXAXA, RNVQXRP:TQV:EPLT-
AA$^{17}$-LYY:EQLSN:EDH:LAXKXE, KIPKAXX:VPQ:EPLT-AA$^{17}$-LYY:EQLSN:VKS:GXGXR, GIPEPXX:VPQ:EPLT-
AA$^{17}$-LYY:EQLSN:VKS:SXAXR, SIPKAXX:VPQ:EPLT-AA$^{17}$-LYY:EQLSN:VKS:GXGXR, HVTKPTX:VPQ:EPLT-
AA$^{17}$-LYY:EQLSN:VKS:SXGXH, YVPKPXX:VPQ:EPLT-AA$^{17}$-LYY:EQLSN:VKS:SXGXH, TVPKPXX:VPQ:EPLT-
AA$^{17}$-LYY:EQLSN:VKS:AXGXH, AVPKAXX:VPQ:EPLT-AA$^{17}$-LYY:EQLSN:VKS:AXGXH, KVGKAXX:VPQ:EPLT-
AA$^{17}$-LYY:EQLSN:VKS:XGXR, KASKAXX:VPQ:EPLT-AA$^{17}$-LYY:EQLSN:VKS:EXGXR, GSAGPXX:VPQ:EPLT-
AA$^{17}$-LYY:EQLSN:VKS:RXGXS, AAPASXX:VPQ:EPLT-AA$^{17}$-LYY:EQLSN:VKS:AXGXR, STPPTXX:VPQ:EPLT-
AA$^{17}$-LYY:EQLSN:VKS:SXGXR, HVPKPXX:VPQ:EPLT-AA$^{17}$-LYY:EQLSN:VKS:SXGXH, RVPSTXX:VPQ:EPLT-
AA$^{17}$-LYY:EQLSN:VKS:XGXL, ASAAPXX:VPQ:EPLT-AA$^{17}$-LYY:EQLSN:VKS:XKXS, ASASPXX:VPQ:EPLT-
AA$^{17}$-LYY:EQLSN:VKS:XKXS, NDEGLEX:VPT:SNIT-AA$^{17}$-QIM:IGEMS:QHN:QXEXR, KIPKAXX:VPT:SNIT-
AA$^{17}$-QIM:IGEMS:VVE:GXGXR, GIPEPXX:VPE:SNIT-AA$^{17}$-QIM:IGEMS:TVE:SXAXR, SIPKAXX:VPT:SNIT-
AA$^{17}$-QIM:IGEMS:VVE:GXGXR, HVTKPTX:APT:SNIT-AA$^{17}$-QIM:IGEMS:VVRSXGXH, YVPKPXX:APT:SNIT-
AA$^{17}$-QIM:IGEMS:VVR:SXGXH, TVPKPXX:APT:SNIT-AA$^{17}$-QIM:IGEMS:VVR:AXGXH, AVPKAXX:APT:SNIT-
AA$^{17}$-QIM:IGEMS:VVK:AXGXH, KVGKAXX:VPT:SNIT-AA$^{17}$-QIM:IGEMS:VAE:XGXR, KASKAXXVPT:SNIT-
AA$^{17}$-QIM:IGEMS:AVS:EXGXR, GSAGPXX:TPT:SNIT-AA$^{17}$-QIM:IGEMS:VVD:RXGXS, AAPASXX:VPA:SNIT-
AA$^{17}$-QIM:IGEMS:VVE:AXGXR, STPPTXX:VPT:SNIT-AA$^{17}$-QIM:IGEMS:VVE:SXGXR, HVPKPXX:APT:SNIT-
AA$^{17}$-QIM:IGEMS:VVR:SXGXH, RVPSTXX:APV:SNIT-AA$^{17}$-QIM:IGEMS:VEE:XGXL, ASAAPXX:VPQ:SNIT-
AA$^{17}$-QIM:IGEMS:VRS:XKXS, ASASPXX:VSQ:SNIT-AA$^{17}$-QIM:IGEMS:VKS:XKXS, ASASPXX:VPQ:SNIT-
AA$^{17}$-QIM:IGEMS:VKS:XKXS, NDEGLEX:VPT:SNIT-AA$^{17}$-QIM:IGEMS:EHS:QXEXR, SSVKXQP:SRV:SNIT-
AA$^{17}$-QIM:IGEMS:EEH:LEXAXA, RNVQXRP:TQV:SNIT-AA$^{17}$-QIM:IGEMS:EDH:LAXKXE, NDEGLEX:VPT:SNIT-
AA$^{17}$-QIM:LGEMS:EHS:KXEXR, KIPKAXX:VPT:SNIT-AA$^{17}$-QIM:LGEMSVVE:GXGXR, GIPEPXX:VPE:SNIT-
AA$^{17}$-QPM:LGEMS:TVE:SXAXR, SIPKAXX:VPT:SNIT-AA$^{17}$-QIM:LGEMSVVE:GXGXR, HVTKPTX:APT:SNIT-

$AA^{17}$-QIM:LGEMS:VVRSXGXH, YVPKPXX:APT:SNIT-$AA^{17}$-QIM:LGEMS:VVR:SXGXH, TVPKPXX:APT:SNIT-$AA^{17}$-QIM:LGEMSVVRAXGXH, AVPKAXX:APT:SNIT-$AA^{17}$-QIM:LGEMSVVK:AXGXH, KVGKAXX:VPT:SNIT-$AA^{17}$-QIM:LGEMS:VAE:XGXR, KASKAXX:VPT:SNIT-$AA^{17}$-QIM:LGEMS:AVS:EXGXR, GSAGPXX:TPT:SNIT-$AA^{17}$-QIM:LGEMS:VVD:RXGXS, AAPASXX:VPA:SNIT-$AA^{17}$-QIM:LGEMS:VVE:AXGXR, STPPTXX:VPT:SNIT-$AA^{17}$-QIM:LGEMSVVE:SXGXR, HVPKPXX:APT:SNIT-$AA^{17}$-QIM:LGEMS:VVRSXGXH, RVPSTXX:APV:SNIT-$AA^{17}$-QIM:LGEMS:VEE:XGXL, ASAAPXX:VPQ:SNIT-$AA^{17}$-QIM:LGEMS:VRS:XKXS, ASASPXX:VSQ:SNIT-$AA^{17}$-QIM:LGEMS:VKS:XKXS, ASASPXX:VPQ:SNIT-$AA^{17}$-QIM:LGEMSVKS:XKXS, NDEGLEX:VPT:SNIT-$AA^{17}$-QIM:LGEMS:QHN:KXEXR, SSVKXQP:SRV:SNIT-$AA^{17}$-QIM:LGEMS:EEH:LEXAXA, RNVQXRP:TQV:SNIT-$AA^{17}$-QIM:LGEMS:EDH:LAXKXE, RNVQXRP:SRV:RSVK-$AA^{17}$-AKV:KEVQV:EEH:LAXKXE, KIPKAXX:VPT:RSVK-$AA^{17}$-AKV:KEVQV:VVE:GXGXR, GIPEPXX:VPE:RSVK-$AA^{17}$-AKV:KEVQV:TVE:SXAXR, SIPKAXX:VPT:RSVK-$AA^{17}$-AKV:KEVQV:VVE:GXGXR, HVTKPTX:APT:RSVK-$AA^{17}$-AKV:KEVQV:VVR:SXGXH, YVPKPXX:APT:RSVK-$AA^{17}$-AKV:KEVQV:VVR:SXGXH, TVPKPXX:APT:RSVK-$AA^{17}$-AKV:KEVQV:VVR:AXGXH, AVPKAXX:APT:RSVK-$AA^{17}$-AKV:KEVQV:VVK:AXGXH, KVGKAXX:VPT:RSVK-$AA^{17}$-AKV:KEVQV:VAE:XGXR, KASKAXX:VPT:RSVK-$AA^{17}$-AKV:KEVQV:AVS:EXGXR, GSAGPXX:TPT:RSVK-$AA^{17}$-AKV:KEVQV:VVD:RXGXS, AAPASXX:VPA:RSVK-$AA^{17}$-AKV:KEVQV:VVE:AXGXR, STPPTXX:VPT:RSVK-$AA^{17}$-AKV:KEVQV:VVE:SXGXR, HVPKPXX:APT:RSVK-$AA^{17}$-AKV:KEVQV:VVR:SXGXH, RVPSTXX:APV:RSVK-$AA^{17}$-AKV:KEVQV:VEE:XGXL, ASAAPXX:VPQ:RSVK-$AA^{17}$-AKV:KEVQV:VRS:XKXS, ASASPXX:VSQ:RSVK-$AA^{17}$-AKV:KEVQV:VKS:XKXS, ASASPXX:VPQ:RSVK-$AA^{17}$-AKV:KEVQV:VKS:XKXS, NDEGLEX:VPT:RSVK-$AA^{17}$-AKV:KEVQV:QHN:KXEXR, NDEGLEX:VPT:RSVK-$AA^{17}$-AKV:KEVQV:EHS:QXEXR, RNVQXRP:TQV:RSVK-$AA^{17}$-AKV:KEVQV:EDH:LAXKXE, SSVKXQP:TQV:RPVQ-$AA^{17}$-RKI:KKATV:EDH:LEXAXA, KIPKAXXVPT:RPVQ-$AA^{17}$-RKI:KKATV:VVE:GXGXR, GIPEPXX:VPE:RPVQ-$AA^{17}$-RKI:KKATV:TVE:SXAXR, SIPKAXX:VPT:RPVQ-$AA^{17}$-RKI:KKATV:VVE:GXGXR, HVTKPTX:APT:RPVQ-$AA^{17}$-RKI:KKATV:VVR:SXGXH, YVPKPXX:APT:RPVQ-$AA^{17}$-RKI:KKATV:VVR:SXGXH, TVPKPXX:APT:RPVQ-$AA^{17}$-RKI:KKATV:VVR:AXGXH, AVPKAXX:APT:RPVQ-$AA^{17}$-RKI:KKATV:VVK:AXGXH, KVGKAXX:VPT:RPVQ-$AA^{17}$-RKI:KKATV:VAE:XGXR, KASKAXX:VPT:RPVQ-$AA^{17}$-RKI:KKATV:AVS:EXGXR, GSAGPXX:TPT:RPVQ-$AA^{17}$-RKI:KKATV:VVD:RXGXS, AAPASXX:VPA:RPVQ-$AA^{17}$-RKI:KKATV:VVE:AXGXR, STPPTXX:VPT:RPVQ-$AA^{17}$-RKI:KKATV:VVE:SXGXR, HVPKPXX:APT:RPVQ-$AA^{17}$-RKI:KKATV:VVR:SXGXH, RVPSTXX:APV:RPVQ-$AA^{17}$-RKI:KKATV:VEE:XGXL, ASAAPXX:VPQ:RPVQ-$AA^{17}$-RKI:KKATV:VRS:XKXS, ASASPXX:VSQ:RPVQ-$AA^{17}$-RKI:KKATV:VKS:XKXS, ASASPXX:VPQ:RPVQ-$AA^{17}$-RKI:KKATV:VKS:XKXS, NDEGLEX:VPT:RPVQ-$AA^{17}$-RKI:KKATV:QHN:KXEXR, NDEGLEX:VPT:RPVQ-$AA^{17}$-RKI:KKATV:EHS:QXEXR and SSVKXQP:SRV:RPVQ-$AA^{17}$-RKI:KKATV:EEH:LEXAXA; and wherein $AA^{17}$ is selected from the group consisting of G, A, V, L, I, P, F, M, W, T and S (in particular is selected from the group consisting of M, I, L, V and T).

In particular, in certain embodiments, the hexaplet PEP7:PEP5:PEP1:PEP2:PEP6:PEP8 is selected from the group consisting of GIPEPXX:VPTKM:SAIS:LKNYQ:NMVVE:SXAXR, SIPKAXX:VPTEL:SAIS:LKNYQ:EMVVE:GXGXR, HVTKPTX:VPTKL:SAIS:LKNYQ:NMVVE:SXGXH, YVPKPXX:VPTKL:SAIS:LKNYQ:NMVVE:SXGXH, TVPKPXX:VPTQL:SAIS:LKNYQ:NMVVE:AXGXH, AVPKAXX:VPTKL:SAIS:LKNYQ:NMVVE:AXGXH, KVGKAXX:VPTKL:SAIS:LKNYQ:EGMSVVE:XGXR, KASKAXX:VPTKL:SAIS:LKNYQ:GMVVE:EXGXR, GSAGPXX:VPTKM:SAIS:LKNYQ:GMVVE:RXGXS, AAPASXX:VPTRL:SAIS:LKNYQ:DMVVE:AXGXR, STPPTXX:VPTRL:SAIS:LKNYQ:DMVVE:SXGXR, HVPKPXX:VPTKL:SAIS:LKNYQ:NMVVE:SXGXH, RVPSTXX:VPTKT:SAIS:LKNYQ:MIVVE:XGXL, ASAAPXX:VPTAL:SAIS:LKNYQ:MIVVE:XKXS, ASASPXX:VPTDL:SAIS:LKNYQ:MIVVE:XKXS, ASASPXX:VPTDL:SAIS:LKNYQ:MVVVE:XKXS, NDEGLEX:VPTEE:SAIS:LKNYQ:FLVVE:KXEXR, NDEGLEX:VPTGQ:SAIS:LKNYQ:LEVVE:QXEXR, SSVKXQP:VPTHH:SAIS:LKNYQ:RLVVE:LEXAXA, RNVQXRP:VPTQL:SAIS:LKNYQ:TLVVE:LAXKXE, GIPEPXX:VPEKM:SAIS:LKNYQ:NMTVE:SXAXR, HVTKPTX:APTKL:SAIS:LKNYQ:NMVVR:SXGXH, YVPKPXX:APTKL:SAIS:LKNYQ:NMVVR:SXGXH, TVPKPXX:APTQL:SAIS:LKNYQ:NMVVR:AXGXH, AVPKAXX:APTKL:SAIS:LKNYQ:NMVVK:AXGXH, KVGKAXX:VPTKL:SAIS:LKNYQ:EGMSVAE:XGXR, KASKAXX:VPTKL:SAIS:LKNYQ:GMAVS:EXGXR, GSAGPXX:TPTKM:SAIS:LKNYQ:GMVVD:RXGXS, AAPASXX:VPARL:SAIS:LKNYQ:DMVVE:AXGXR, HVPKPXX:APTKL:SAIS:LKNYQ:NMVVR:SXGXH, RVPSTXX:APVKT:SAIS:LKNYQ:MIVEE:XGXL, ASAAPXX:VPQAL:SAIS:LKNYQ:MIVRS:XKXS, ASASPXX:VSQDL:SAIS:LKNYQ:MIVKS:XKXS, ASASPXX:VPQDL:SAIS:LKNYQ:MVVKS:XKXS, NDEGLEX:VPTEE:SAIS:LKNYQ:FLQHN:KXEXR, NDEGLEX:VPTGQ:SAIS:LKNYQ:LEEHS:QXEXR, SSVKXQP:SRVHH:SAIS:LKNYQ:RLEEH:LEXAXA, RNVQXRP:TQVQL:SAIS:LKNYQ:TLEDH:LAXKXE, KIPKAXX:VPEEL:SSLS:LKVYP:DMTVE:GXGXR, SIPKAXX:VPEEL:SSLS:LKVYP:EMTVE:GXGXR, HVTKPTX:VPEKL:SSLS:LKVYP:NMTVE:SXGXH, YVPKPXX:VPEKL:SSLS:LKVYP:NMTVE:SXGXH, TVPKPXX:VPEQL:SSLS:LKVYP:NMTVE:AXGXH, AVPKAXX:VPEKL:SSLS:LKVYP:NMTVE:AXGXH, KVGKAXX:VPEKL:SSLS:LKVYP:EGMSTVE:XGXR, KASKAXX:VPEKL:SSLS:LKVYP:GMTVE:EXGXR, GSAGPXX:VPEKM:SSLS:LKVYP:GMTVE:RXGXS, AAPASXX:VPERL:SSLS:LKVYP:DMTVE:AXGXR, STPPTXX:VPERL:SSLS:LKVYP:DMTVE:SXGXR, HVPKPXX:VPEKL:SSLS:LKVYP:NMTVE:SXGXH, RVPSTXX:VPEKT:SSLS:LKVYP:MITVE:XGXL, ASAAPXX:VPEAL:SSLS:LKVYP:MITVE:XKXS, ASASPXX:VPEDL:SSLS:LKVYP:MITVE:XKXS, ASASPXX:VPEDL:SSLS:LKVYP:MVTVE:XKXS, NDEGLEX:VPEEE:SSLS:LKVYP:FLTVE:KXEXR, NDEGLEX:VPEGQ:SSLS:LKVYP:LETVE:QXEXR, SSVKXQP:VPEHH:SSLS:LKVYP:RLTVE:LEXAXA, RNVQXRP:VPEQL:SSLS:LKVYP:TLTVE:LAXKXE, KIPKAXX:VPTEL:SSLS:LKVYP:DMVVE:GXGXR, SIPKAXX:VPTEL:SSLS:LKVYP:EMVVE:GXGXR, HVTKPTX:APTKL:SSLS:LKVYP:NMVVR:SXGXH, YVPKPXX:APTKL:SSLS:LKVYP:NMVVR:SXGXH, TVPKPXX:APTQL:SSLS:LKVYP:NMVVR:AXGXH, AVPKAXX:APTKL:SSLS:LKVYP:NMVVK:AXGXH, KVGKAXX:VPTKL:SSLS:LKVYP:EGMSVAE:XGXR, KASKAXX:VPTKL:SSLS:LKVYP:GMAVS:EXGXR, GSAGPXX:TPTKM:SSLS:LKVYP:GMVVD:RXGXS, AAPASXX:VPARL:SSLS:LKVYP:DMVVE:AXGXR, STPPTXX:VPTRL:SSLS:LKVYP:DMVVE:SXGXR, HVPKPXX:APTKL:SSLS:LKVYP:NMVVR:SXGXH, RVPSTXX:APVKT:SSLS:LKVYP:MIVEE:XGXL, ASAAPXX:VPQAL:SSLS:LKVYP:MIVRS:XKXS, ASASPXX:VSQDL:SSLS:LKVYP:MIVKS:XKXS, ASASPXX:VPQDL:SSLS:LKVYP:MVVKS:XKXS, NDEGLEX:VPTEE:SSLS:LKVYP:FLQHN:KXEXR, NDEGLEX:VPTGQ:SSLS:LKVYP:LEEHS:QXEXR, SSVKXQP:SRVHH:SSLS:LKVYP:RLEEH:LEXAXA, RNVQXRP:TQVQL:SSLS:LKVYP:TLEDH:LAXKXE, KIPKAXX:VPTEL:SAIS:LKNYQ:DMVVE:GXGXR, KIPKAXX:APTEL:NAIS:LKKYR:DMVVR:GXGXR, GIPEPXX:APTKM:NAIS:LKKYR:NMVVR:SXAXR, SIPKAXX:APTEL:NAIS:LKKYR:EMVVR:GXGXR, YVPKPXX:APTKL:NAIS:LKKYR:NMVVR:SXGXH, TVPKPXX:APTQL:NAIS:LKKYR:NMVVR:AXGXH, AVPKAXX:APTKL:NAIS:LKKYR:NMVVR:AXGXH, KVGKAXX:APTKL:NAIS:LKKYR:EGMSVVR:XGXR, KASKAXX:APTKL:NAIS:LKKYR:GMVVR:EXGXR, GSAGPXX:APTKM:NAIS:LKKYR:GMVVR:RXGXS, AAPASXX:APTRL:NAIS:LKKYR:DMVVR:AXGXR, STPPTXX:APTRL:NAIS:LKKYR:DMVVR:SXGXR, HVPKPXX:APTKL:NAIS:LKKYR:NMVVR:SXGXH, RVPSTXX:APTKT:NAIS:LKKYR:MIVVR:XGXL, ASAAPXX:APTAL:NAIS:LKKYR:MIVVR:XKXS, ASASPXX:APTDL:NAIS:LKKYR:MIVVR:XKXS, ASASPXX:APTDL:NAIS:LKKYR:MVVVR:XKXS, NDEGLEX:APTEE:NAIS:LKKYR:FLVVR:KXEXR, NDEGLEX:APTGQ:NAIS:LKKYR:LEVVR:QXEXR, SSVKXQP:APTHH:NAIS:LKKYR:RLVVR:LEXAXA, RNVQXRP:APTQL:NAIS:LKKYR:TLVVR:LAXKXE, KIPKAXX:VPTEL:NAIS:LKKYR:DMVVE:GXGXR, GIPEPXX:VPEKM:NAIS:LKKYR:NMTVE:SXAXR, SIPKAXX:VPTEL:NAIS:LKKYR:EMVVE:GXGXR, AVPKAXX:APTKL:NAIS:LKKYR:NMVVK:AXGXH, KVGKAXX:VPTKL:NAIS:LKKYR:EGMSVAE:XGXR, KASKAXX:VPTKL:NAIS:LKKYR:GMAVS:EXGXR, GSAGPXX:TPTKM:NAIS:LKKYR:GMVVD:RXGXS, AAPASXX:VPARL:NAIS:LKKYR:DMVVE:AXGXR, STPPTXX:VPTRL:NAIS:LKKYR:DMVVE:SXGXR, RVPSTXX:APVKT:NAIS:LKKYR:MIVEE:XGXL, ASAAPXX:VPQAL:NAIS:LKKYR:MIVRS:XKXS, ASASPXX:VSQDL:NAIS:LKKYR:MIVKS:XKXS, ASASPXX:VPQDL:NAIS:LKKYR:MVVKS:XKXS, NDEGLEX:VPTEE:NAIS:LKKYR:FLQHN:KXEXR, NDEGLEX:VPTGQ:NAIS:LKKYR:LEEHS:QXEXR, SSVKXQP:SRVHH:NAIS:LKKYR:RLEEH:LEXAXA, RNVQXRP:TQVQL:NAIS:LKKYR:TLEDH:LAXKXE, HVTKPTX:APTKL:NAIS:LKKYR:NMVVR:SXGXH, KIPKAXX:APTEL:SATS:LRKHR:DMVVK:GXGXR, GIPEPXX:APTKM:SATS:LRKHR:NMVVK:SXAXR, SIPKAXX:APTEL:SATS:LRKHR:EMVVK:GXGXR, HVTKPTX:APTKL:SATS:LRKHR:NMVVK:SXGXH, YVPKPXX:APTKL:SATS:LRKHR:NMVVK:SXGXH, TVPKPXX:APTQL:SATS:LRKHR:NMVVK:AXGXH, KVGKAXX:APTKL:SATS:LRKHR:EGMSVVK:XGXR, KASKAXX:APTKL:SATS:LRKHR:GMVVK:EXGXR, GSAGPXX:APTKM:SATS:LRKHR:GMVVK:RXGXS, AAPASXX:APTRL:SATS:LRKHR:DMVVK:AXGXR, STPPTXX:APTRL:SATS:LRKHR:DMVVK:SXGXR, HVPKPXX:APTKL:SATS:LRKHR:NMVVK:SXGXH, RVPSTXX:APTKT:SATS:LRKHR:MIVVK:XGXL, ASAAPXX:APTAL:SATS:LRKHR:MIVVK:XKXS, ASASPXX:APTDL:SATS:LRKHR:MIVVK:XKXS, ASASPXX:APTDL:SATS:LRKHR:MVVVK:XKXS, NDEGLEX:APTEE:SATS:LRKHR:FLVVK:KXEXR, NDEGLEX:APTGQ:SATS:LRKHR:LEVVK:QXEXR, SSVKXQP:APTHH:SATS:LRKHR:RLVVK:LEXAXA, RNVQXRP:APTQL:SATS:LRKHR:TLVVK:LAXKXE, KIPKAXX:VPTEL:SATS:LRKHR:DMVVE:GXGXR, GIPEPXX:VPEKM:SATS:LRKHR:NMTVE:SXAXR, SIPKAXX:VPTEL:SATS:LRKHR:EMVVE:GXGXR, HVTKPTX:APTKL:SATS:LRKHR:NMVVR:SXGXH, YVPKPXX:APTKL:SATS:LRKHR:NMVVR:SXGXH, TVPKPXX:APTQL:SATS:LRKHR:NMVVR:AXGXH, KVGKAXX:VPTKL:SATS:LRKHR:EGMSVAE:XGXR, KASKAXX:VPTKL:SATS:LRKHR:GMAVS:EXGXR, GSAGPXX:TPTKM:SATS:LRKHR:GMVVD:RXGXS, AAPASXX:VPARL:SATS:LRKHR:DMVVE:AXGXR, STPPTXX:VPTRL:SATS:LRKHR:DMVVE:SXGXR, HVPKPXX:APTKL:SATS:LRKHR:NMVVR:SXGXH, RVPSTXX:APVKT:SATS:LRKHR:MIVEE:XGXL, ASAAPXX:VPQAL:SATS:LRKHR:MIVRS:XKXS, ASASPXX:VSQDL:SATS:LRKHR:MIVKS:XKXS, ASASPXX:VPQDL:SATS:LRKHR:MVVKS:XKXS, NDEGLEX:VPTEE:SATS:LRKHR:FLQHN:KXEXR, NDEGLEX:VPTGQ:SATS:LRKHR:LEEHS:QXEXR, SSVKXQP:SRVHH:SATS:LRKHR:RLEEH:LEXAXA, RNVQXRP:TQVQL:SATS:LRKHR:TLEDH:LAXKXE, KIPKAXX:VPTEL:SPIS:LKYHY:DMVAE:GXGXR, GIPEPXX:VPTKM:SPIS:LKYHY:NMVAE:SXAXR, SIPKAXX:VPTEL:SPIS:LKYHY:EMVAE:GXGXR, HVTKPTX:VPTKL:SPIS:LKYHY:NMVAE:SXGXH, YVPKPXX:VPTKL:SPIS:LKYHY:NMVAE:SXGXH, TVPKPXX:VPTQL:SPIS:LKYHY:NMVAE:AXGXH, AVPKAXX:VPTKL:SPIS:LKYHY:NMVAE:AXGXH, KASKAXX:VPTKL:SPIS:LKYHY:GMVAE:EXGXR, GSAGPXX:VPTKM:SPIS:LKYHY:GMVAE:RXGXS, AAPASXX:VPTRL:SPIS:LKYHY:DMVAE:AXGXR, STPPTXX:VPTRL:SPIS:LKYHY:DMVAE:SXGXR, HVPKPXX:VPTKL:SPIS:LKYHY:NMVAE:SXGXH, RVPSTXX:VPTKT:SPIS:LKYHY:MIVAE:XGXL, ASAAPXX:VPTAL:SPIS:LKYHY:MIVAE:XKXS, ASASPXX:VPTDL:SPIS:LKYHY:MIVAE:XKXS, ASASPXX:VPTDL:SPIS:LKYHY:MVVAE:XKXS, NDEGLEX:VPTEE:SPIS:LKYHY:FLVAE:KXEXR, NDEGLEX:VPTGQ:SPIS:LKYHY:LEVAE:QXEXR, SSVKXQP:VPTHH:SPIS:LKYHY:RLVAE:LEXAXA, RNVQXRP:VPTQL:SPIS:LKYHY:TLVAE:LAXKXE, KIPKAXX:VPTEL:SPIS:LKYHY:DMVVE:GXGXR, GIPEPXX:VPEKM:SPIS:LKYHY:NMTVE:SXAXR, SIPKAXX:VPTEL:SPIS:LKYHY:EMVVE:GXGXR, HVTKPTX:APTKL:SPIS:LKYHY:NMVVR:SXGXH, YVPKPXX:APTKL:SPIS:LKYHY:NMVVR:SXGXH, TVPKPXX:APTQL:SPIS:LKYHY:NMVVR:AXGXH, AVPKAXX:APTKL:SPIS:LKYHY:NMVVK:AXGXH, KASKAXX:VPTKL:SPIS:LKYHY:GMAVS:EXGXR, GSAGPXX:TPTKM:SPIS:LKYHY:GMVVD:RXGXS, AAPASXX:VPARL:SPIS:LKYHY:DMVVE:AXGXR, STPPTXX:VPTRL:SPIS:LKYHY:DMVVE:SXGXR, HVPKPXX:APTKL:SPIS:LKYHY:NMVVR:SXGXH, RVPSTXX:APVKT:SPIS:LKYHY:MIVEE:XGXL, ASAAPXX:VPQAL:SPIS:LKYHY:MIVRS:XKXS, ASASPXX:VSQDL:SPIS:LKYHY:MIVKS:XKXS, ASASPXX:VPQDL:SPIS:LKYHY:MVVKS:XKXS, NDEGLEX:VPTEE:SPIS:LKYHY:FLQHN:KXEXR, NDEGLEX:VPTGQ:SPIS:LKYHY:LEEHS:QXEXR, SSVKXQP:SRVHH:SPIS:LKYHY:RLEEH:LEXAXA, RNVQXRP:TQVQL:SPIS:LKYHY:TLEDH:LAXKXE, KIPKAXX:VPTEL:EPIS:KFKYE:DMAVS:GXGXR, GIPEPXX:VPTKM:EPIS:KFKYE:NMAVS:SXAXR, SIPKAXX:VPTEL:EPIS:KFKYE:EMAVS:GXGXR, HVTKPTX:VPTKL:EPIS:KFKYE:NMAVS:SXGXH, YVPKPXX:VPTKL:EPIS:KFKYE:NMAVS:SXGXH, TVPKPXX:VPTQL:EPIS:KFKYE:NMAVS:AXGXH, AVPKAXX:VPTKL:EPIS:KFKYE:NMAVS:AXGXH, KVGKAXX:VPTKL:EPIS:KFKYE:EGMSAVS:XGXR, GSAGPXX:VPTKM:EPIS:KFKYE:GMAVS:RXGXS, AAPASXX:VPTRL:EPIS:KFKYE:DMAVS:AXGXR, STPPTXX:VPTRL:EPIS:KFKYE:DMAVS:SXGXR, HVPKPXX:VPTKL:EPIS:KFKYE:NMAVS:SXGXH, RVPSTXX:VPTKT:EPIS:KFKYE:MIAVS:XGXL, ASAAPXX:VPTAL:EPIS:KFKYE:MIAVS:XKXS, ASASPXX:VPTDL:EPIS:KFKYE:MIAVS:XKXS, ASASPXX:VPTDL:EPIS:KFKYE:MVAVS:XKXS, NDEGLEX:VPTEE:EPIS:KFKYE:FLAVS:KXEXR, NDEGLEX:VPTGQ:EPIS:KFKYE:LEAVS:QXEXR, SSVKXQP:VPTHH:EPIS:KFKYE:RLAVS:LEXAXA, RNVQXRP:VPTQL:EPIS:KFKYE:TLAVS:LAXKXE, KIPKAXX:VPTEL:EPIS:KFKYE:DMVVE:GXGXR, GIPEPXX:VPEKM:EPIS:KFKYE:NMTVE:SXAXR, SIPKAXX:VPTEL:EPIS:KFKYE:EMVVE:GXGXR, HVTKPTX:APTKL:EPIS:KFKYE:NMVVR:SXGXH, YVPKPXX:APTKL:EPIS:KFKYE:NMVVR:SXGXH, TVPKPXX:APTQL:EPIS:KFKYE:NMVVR:AXGXH, AVPKAXX:APTKL:EPIS:KFKYE:NMVVK:AXGXH, KVGKAXX:VPTKL:EPIS:KFKYE:EGMSVAE:XGXR, GSAGPXX:TPTKM:EPIS:KFKYE:GMVVD:RXGXS, AAPASXX:VPARL:EPIS:KFKYE:DMVVE:AXGXR, STPPTXX:VPTRL:EPIS:KFKYE:DMVVE:SXGXR, HVPKPXX:APTKL:EPIS:KFKYE:NMVVR:SXGXH, RVPSTXX:APVKT:EPIS:KFKYE:MIVEE:XGXL, ASAAPXX:VPQAL:EPIS:KFKYE:MIVRS:XKXS, ASASPXX:VSQDL:EPIS:KFKYE:MIVKS:XKXS, ASASPXX:VPQDL:EPIS:KFKYE:MVVKS:XKXS, NDEGLEX:VPTEE:EPIS:KFKYE:FLQHN:KXEXR, NDEGLEX:VPTGQ:EPIS:KFKYE:LEEHS:QXEXR, SSVKXQP:SRVHH:EPIS:KFKYE:RLEEH:LEXAXA, RNVQXRP:TQVQL:EPIS:KFKYE:TLEDH:LAXKXE, KIPKAXX:TPTEL:SPIN:YGKIP:DMVVD:GXGXR, GIPEPXX:TPTKM:SPIN:YGKIP:NMVVD:SXAXR, SIPKAXX:TPTEL:SPIN:YGKIP:EMVVD:GXGXR, HVTKPTX:TPTKL:SPIN:YGKIP:NMVVD:SXGXH, YVPKPXX:TPTKL:SPIN:YGKIP:NMVVD:SXGXH, TVPKPXX:TPTQL:SPIN:YGKIP:NMVVD:AXGXH, AVPKAXX:TPTKL:SPIN:YGKIP:NMVVD:AXGXH, KVGKAXX:TPTKL:SPIN:YGKIP:EGMSVVD:XGXR, KASKAXX:TPTKL:SPIN:YGKIP:GMVVD:EXGXR, AAPASXX:TPTRL:SPIN:YGKIP:DMVVD:AXGXR, STPPTXX:TPTRL:SPIN:YGKIP:DMVVD:SXGXR, HVPKPXX:TPTKL:SPIN:YGKIP:NMVVD:SXGXH, RVPSTXX:TPTKT:SPIN:YGKIP:MIVVD:XGXL, ASAAPXX:TPTAL:SPIN:YGKIP:MIVVD:XKXS, ASASPXX:TPTDL:SPIN:YGKIP:MIVVD:XKXS, ASASPXX:TPTDL:SPIN:YGKIP:MVVVD:XKXS, NDEGLEX:TPTEE:SPIN:YGKIP:FLVVD:KXEXR, NDEGLEX:TPTGQ:SPIN:YGKIP:LEVVD:QXEXR, SSVKXQP:TPTHH:SPIN:YGKIP:RLVVD:LEXAXA, RNVQXRP:TPTQL:SPIN:YGKIP:TLVVD:LAXKXE, KIPKAXX:VPTEL:SPIN:YGKIP:DMVVE:GXGXR, GIPEPXX:VPEKM:SPIN:YGKIP:NMTVE:SXAXR, SIPKAXX:VPTEL:SPIN:YGKIP:EMVVE:GXGXR, HVTKPTX:APTKL:SPIN:YGKIP:NMVVR:SXGXH, YVPKPXX:APTKL:SPIN:YGKIP:NMVVR:SXGXH, TVPKPXX:APTQL:SPIN:YGKIP:NMVVR:AXGXH, AVPKAXX:APTKL:SPIN:YGKIP:NMVVK:AXGXH, KVGKAXX:VPTKL:SPIN:YGKIP:EGMSVAE:XGXR, KASKAXX:VPTKL:SPIN:YGKIP:GMAVS:EXGXR, AAPASXX:VPARL:SPIN:YGKIP:DMVVE:AXGXR, STPPTXX:VPTRL:SPIN:YGKIP:DMVVE:SXGXR, HVPKPXX:APTKL:SPIN:YGKIP:NMVVR:SXGXH, RVPSTXX:APVKT:SPIN:YGKIP:MIVEE:XGXL, ASAAPXX:VPQAL:SPIN:YGKIP:MIVRS:XKXS, ASASPXX:VSQDL:SPIN:YGKIP:MIVKS:XKXS, ASASPXX:VPQDL:SPIN:YGKIP:MVVKS:XKXS, NDEGLEX:VPTEE:SPIN:YGKIP:FLQHN:KXEXR, NDEGLEX:VPTGQ:SPIN:YGKIP:LEEHS:QXEXR, SSVKXQP:SRVHH:SPIN:YGKIP:RLEEH:LEXAXA, RNVQXRP:TQVQL:SPIN:YGKIP:TLEDH:LAXKXE, KIPKAXX:VPAEL:SPIS:YKQYE:DMVVE:GXGXR, GIPEPXX:VPAKM:SPIS:YKQYE:NMVVE:SXAXR, SIPKAXX:VPAEL:SPIS:YKQYE:EMVVE:GXGXR, HVTKPTX:VPAKL:SPIS:YKQYE:NMVVE:SXGXH, YVPKPXX:VPAKL:SPIS:YKQYE:NMVVE:SXGXH, TVPKPXX:VPAQL:SPIS:YKQYE:NMVVE:AXGXH, AVPKAXX:VPAKL:SPIS:YKQYE:NMVVE:AXGXH, KVGKAXX:VPAKL:SPIS:YKQYE:EGMSVVE:XGXR, KASKAXX:VPAKL:SPIS:YKQYE:GMVVE:EXGXR, GSAGPXX:VPAKM:SPIS:YKQYE:GMVVE:RXGXS, STPPTXX:VPARL:SPIS:YKQYE:DMVVE:SXGXR, HVPKPXX:VPAKL:SPIS:YKQYE:NMVVE:SXGXH, RVPSTXX:VPAKT:SPIS:YKQYE:MIVVE:XGXL, ASAAPXX:VPAAL:SPIS:YKQYE:MIVVE:XKXS, ASASPXX:VPADL:SPIS:YKQYE:MIVVE:XKXS, ASASPXX:VPADL:SPIS:YKQYE:MVVVE:XKXS, NDEGLEX:VPAEE:SPIS:YKQYE:FLVVE:KXEXR, NDEGLEX:VPAGQ:SPIS:YKQYE:LEVVE:QXEXR, SSVKXQP:VPAHH:SPIS:YKQYE:RLVVE:LEXAXA,

RNVQXRP:VPAQL:SPIS:YKQYE:TLVVE:LAXKXE, KIPKAXX:VPTEL:SPIS:YKQYE:DMVVE:GXGXR, GIPEPXX:VPEKM:SPIS:YKQYE:NMTVE:SXAXR, SIPKAXX:VPTEL:SPIS:YKQYE:EMVVE:GXGXR, HVTKPTX:APTKL:SPIS:YKQYE:NMVVR:SXGXH, YVPKPXX:APTKL:SPIS:YKQYE:NMVVR:SXGXH, TVPKPXX:APTQL:SPIS:YKQYE:NMVVR:AXGXH, AVPKAXX:APTKL:SPIS:YKQYE:NMVVK:AXGXH, KVGKAXX:VPTKL:SPIS:YKQYE:EGMSVAE:XGXR, KASKAXX:VPTKL:SPIS:YKQYE:GMAVS:EXGXR, GSAGPXX:TPTKM:SPIS:YKQYE:GMVVD:RXGXS, STPPTXX:VPTRL:SPIS:YKQYE:DMVVE:SXGXR, HVPKPXX:APTKL:SPIS:YKQYE:NMVVR:SXGXH, RVPSTXX:APVKT:SPIS:YKQYE:MIVEE:XGXL, ASAAPXX:VPQAL:SPIS:YKQYE:MIVRS:XKXS, ASASPXX:VSQDL:SPIS:YKQYE:MIVKS:XKXS, ASASPXX:VPQDL:SPIS:YKQYE:MVVKS:XKXS, NDEGLEX:VPTEE:SPIS:YKQYE:FLQHN:KXEXR, NDEGLEX:VPTGQ:SPIS:YKQYE:LEEHS:QXEXR, SSVKXQP:SRVHH:SPIS:YKQYE:RLEEH:LEXAXA, RNVQXRP:TQVQL:SPIS:YKQYE:TLEDH:LAXKXE, GIPEPXX:VPTKM:SPIS:YKQYE:NMVVE:SXAXR, HVTKPTX:VPTKL:SPIS:YKQYE:NMVVE:SXGXH, YVPKPXX:VPTKL:SPIS:YKQYE:NMVVE:SXGXH, TVPKPXX:VPTQL:SPIS:YKQYE:NMVVE:AXGXH, AVPKAXX:VPTKL:SPIS:YKQYE:NMVVE:AXGXH, KVGKAXX:VPTKL:SPIS:YKQYE:EGMSVVE:XGXR, KASKAXX:VPTKL:SPIS:YKQYE:GMVVE:EXGXR, GSAGPXX:VPTKM:SPIS:YKQYE:GMVVE:RXGXS, AAPASXX:VPTRL:SPIS:YKQYE:DMVVE:AXGXR, HVPKPXX:VPTKL:SPIS:YKQYE:NMVVE:SXGXH, RVPSTXX:VPTKT:SPIS:YKQYE:MIVVE:XGXL, ASAAPXX:VPTAL:SPIS:YKQYE:MIVVE:XKXS, ASASPXX:VPTDL:SPIS:YKQYE:MIVVE:XKXS, ASASPXX:VPTDL:SPIS:YKQYE:MVVVE:XKXS, NDEGLEX:VPTEE:SPIS:YKQYE:FLVVE:KXEXR, NDEGLEX:VPTGQ:SPIS:YKQYE:LEVVE:QXEXR, SSVKXQP:VPTHH:SPIS:YKQYE:RLVVE:LEXAXA, RNVQXRP:VPTQL:SPIS:YKQYE:TLVVE:LAXKXE, AAPASXX:VPARL:SPIS:YKQYE:DMVVE:AXGXR, KIPKAXX:APVEL:KPLS:DHHKD:DMVEE:GXGXR, GIPEPXX:APVKM:KPLS:DHHKD:NMVEE:SXAXR, SIPKAXX:APVEL:KPLS:DHHKD:EMVEE:GXGXR, HVTKPTX:APVKL:KPLS:DHHKD:NMVEE:SXGXH, YVPKPXX:APVKL:KPLS:DHHKD:NMVEE:SXGXH, TVPKPXX:APVQL:KPLS:DHHKD:NMVEE:AXGXH, AVPKAXX:APVKL:KPLS:DHHKD:NMVEE:AXGXH, KVGKAXX:APVKL:KPLS:DHHKD:EGMSVEE:XGXR, KASKAXX:APVKL:KPLS:DHHKD:GMVEE:EXGXR, GSAGPXX:APVKM:KPLS:DHHKD:GMVEE:RXGXS, AAPASXX:APVRL:KPLS:DHHKD:DMVEE:AXGXR, STPPTXX:APVRL:KPLS:DHHKD:DMVEE:SXGXR, HVPKPXX:APVKL:KPLS:DHHKD:NMVEE:SXGXH, ASAAPXX:APVAL:KPLS:DHHKD:MIVEE:XKXS, ASASPXX:APVDL:KPLS:DHHKD:MIVEE:XKXS, ASASPXX:APVDL:KPLS:DHHKD:MVVEE:XKXS, NDEGLEX:APVEE:KPLS:DHHKD:FLVEE:KXEXR, NDEGLEX:APVGQ:KPLS:DHHKD:LEVEE:QXEXR, SSVKXQP:APVHH:KPLS:DHHKD:RLVEE:LEXAXA, RNVQXRP:APVQL:KPLS:DHHKD:TLVEE:LAXKXE, KIPKAXX:VPTEL:KPLS:DHHKD:DMVVE:GXGXR, GIPEPXX:VPEKM:KPLS:DHHKD:NMTVE:SXAXR, SIPKAXX:VPTEL:KPLS:DHHKD:EMVVE:GXGXR, HVTKPTX:APTKL:KPLS:DHHKD:NMVVR:SXGXH, YVPKPXX:APTKL:KPLS:DHHKD:NMVVR:SXGXH, TVPKPXX:APTQL:KPLS:DHHKD:NMVVR:AXGXH, AVPKAXX:APTKL:KPLS:DHHKD:NMVVK:AXGXH, KVGKAXX:VPTKL:KPLS:DHHKD:EGMSVAE:XGXR, KASKAXX:VPTKL:KPLS:DHHKD:GMAVS:EXGXR, GSAGPXX:TPTKM:KPLS:DHHKD:GMVVD:RXGXS, AAPASXX:VPARL:KPLS:DHHKD:DMVVE:AXGXR, STPPTXX:VPTRL:KPLS:DHHKD:DMVVE:SXGXR, HVPKPXX:APTKL:KPLS:DHHKD:NMVVR:SXGXH, ASAAPXX:VPQAL:KPLS:DHHKD:MIVRS:XKXS, ASASPXX:VSQDL:KPLS:DHHKD:MIVKS:XKXS, ASASPXX:VPQDL:KPLS:DHHKD:MVVKS:XKXS, NDEGLEX:VPTEE:KPLS:DHHKD:FLQHN:KXEXR, NDEGLEX:VPTGQ:KPLS:DHHKD:LEEHS:QXEXR, SSVKXQP:SRVHH:KPLS:DHHKD:RLEEH:LEXAXA, RNVQXRP:TQVQL:KPLS:DHHKD:TLEDH:LAXKXE, KIPKAXX:VPQEL:EPLP:EQLSN:DMVRS:GXGXR, GIPEPXX:VPQKM:EPLP:EQLSN:NMVRS:SXAXR, SIPKAXX:VPQEL:EPLP:EQLSN:EMVRS:GXGXR, HVTKPTX:VPQKL:EPLP:EQLSN:NMVRS:SXGXH, YVPKPXX:VPQKL:EPLP:EQLSN:NMVRS:SXGXH, TVPKPXX:VPQQL:EPLP:EQLSN:NMVRS:AXGXH, AVPKAXX:VPQKL:EPLP:EQLSN:NMVRS:AXGXH, KVGKAXX:VPQKL:EPLP:EQLSN:EGMSVRS:XGXR, KASKAXX:VPQKL:EPLP:EQLSN:GMVRS:EXGXR, GSAGPXX:VPQKM:EPLP:EQLSN:GMVRS:RXGXS, AAPASXX:VPQRL:EPLP:EQLSN:DMVRS:AXGXR, STPPTXX:VPQRL:EPLP:EQLSN:DMVRS:SXGXR, HVPKPXX:VPQKL:EPLP:EQLSN:NMVRS:SXGXH, RVPSTXX:VPQKT:EPLP:EQLSN:MIVRS:XGXL, ASASPXX:VPQDL:EPLP:EQLSN:MIVRS:XKXS, ASASPXX:VPQDL:EPLP:EQLSN:MVVRS:XKXS, NDEGLEX:VPQEE:EPLP:EQLSN:FLVRS:KXEXR, NDEGLEX:VPQGQ:EPLP:EQLSN:LEVRS:QXEXR, SSVKXQP:VPQHH:EPLP:EQLSN:RLVRS:LEXAXA, RNVQXRP:VPQQL:EPLP:EQLSN:TLVRS:LAXKXE, KIPKAXX:VPTEL:EPLP:EQLSN:DMVVE:GXGXR, GIPEPXX:VPEKM:EPLP:EQLSN:NMTVE:SXAXR, SIPKAXX:VPTEL:EPLP:EQLSN:EMVVE:GXGXR, HVTKPTX:APTKL:EPLP:EQLSN:NMVVR:SXGXH, YVPKPXX:APTKL:EPLP:EQLSN:NMVVR:SXGXH, TVPKPXX:APTQL:EPLP:EQLSN:NMVVR:AXGXH, AVPKAXX:APTKL:EPLP:EQLSN:NMVVK:AXGXH, KVGKAXX:VPTKL:EPLP:EQLSN:EGMSVAE:XGXR, KASKAXX:VPTKL:EPLP:EQLSN:GMAVS:EXGXR, GSAGPXX:TPTKM:EPLP:EQLSN:GMVVD:RXGXS, AAPASXX:VPARL:EPLP:EQLSN:DMVVE:AXGXR, STPPTXX:VPTRL:EPLP:EQLSN:DMVVE:SXGXR, HVPKPXX:APTKL:EPLP:EQLSN:NMVVR:SXGXH, RVPSTXX:APVKT:EPLP:EQLSN:MIVEE:XGXL, ASASPXX:VSQDL:EPLP:EQLSN:MIVKS:XKXS, ASASPXX:VPQDL:EPLP:EQLSN:MVVKS:XKXS, NDEGLEX:VPTEE:EPLP:EQLSN:FLQHN:KXEXR, NDEGLEX:VPTGQ:EPLP:EQLSN:LEEHS:QXEXR, SSVKXQP:SRVHH:EPLP:EQLSN:RLEEH:LEXAXA, RNVQXRP:TQVQL:EPLP:EQLSN:TLEDH:LAXKXE, KIPKAXX:VSQEL:EPLT:EQLSN:DMVKS:GXGXR, GIPEPXX:VSQKM:EPLT:EQLSN:NMVKS:SXAXR, SIPKAXX:VSQEL:EPLT:EQLSN:EMVKS:GXGXR, HVTKPTX:VSQKL:EPLT:EQLSN:NMVKS:SXGXH, YVPKPXX:VSQKL:EPLT:EQLSN:NMVKS:SXGXH, TVPKPXX:VSQQL:EPLT:EQLSN:NMVKS:AXGXH, AVPKAXX:VSQKL:EPLT:EQLSN:NMVKS:AXGXH, KVGKAXX:VSQKL:EPLT:EQLSN:EGMSVKS:XGXR, KASKAXX:VSQKL:EPLT:EQLSN:GMVKS:EXGXR, GSAGPXX:VSQKM:EPLT:EQLSN:GMVKS:RXGXS, AAPASXX:VSQRL:EPLT:EQLSN:DMVKS:AXGXR, STPPTXX:VSQRL:EPLT:EQLSN:DMVKS:SXGXR, HVPKPXX:VSQKL:EPLT:EQLSN:NMVKS:SXGXH, RVPSTXX:VSQKT:EPLT:EQLSN:MIVKS:XGXL, ASAAPXX:VSQAL:EPLT:EQLSN:MIVKS:XKXS, ASASPXX:VSQDL:EPLT:EQLSN:MVVKS:XKXS, NDEGLEX:VSQEE:EPLT:EQLSN:FLVKS:KXEXR, NDEGLEX:VSQGQ:EPLT:EQLSN:LEVKS:QXEXR, SSVKXQP:VSQHH:EPLT:EQLSN:RLVKS:LEXAXA, RNVQXRP:VSQQL:EPLT:EQLSN:TLVKS:LAXKXE, KIPKAXX:VPTEL:EPLT:EQLSN:DMVVE:GXGXR, GIPEPXX:VPEKM:EPLT:EQLSN:NMTVE:SXAXR, SIPKAXX:VPTEL:EPLT:EQLSN:EMVVE:GXGXR, HVTKPTX:APTKL:EPLT:EQLSN:NMVVR:SXGXH, YVPKPXX:APTKL:EPLT:EQLSN:NMVVR:SXGXH, TVPKPXX:APTQL:EPLT:EQLSN:NMVVR:AXGXH, AVPKAXX:APTKL:EPLT:EQLSN:NMVVK:AXGXH, KVGKAXX:VPTKL:EPLT:EQLSN:EGMSVAE:XGXR, KASKAXX:VPTKL:EPLT:EQLSN:GMAVS:EXGXR, GSAGPXX:TPTKM:EPLT:EQLSN:GMVVD:RXGXS, AAPASXX:VPARL:EPLT:EQLSN:DMVVE:AXGXR, STPPTXX:VPTRL:EPLT:EQLSN:DMVVE:SXGXR, HVPKPXX:APTKL:EPLT:EQLSN:NMVVR:SXGXH, RVPSTXX:APVKT:EPLT:EQLSN:MIVEE:XGXL, ASAAPXX:VPQAL:EPLT:EQLSN:MIVRS:XKXS, ASASPXX:VPQDL:EPLT:EQLSN:MVVKS:XKXS, NDEGLEX:VPTEE:EPLT:EQLSN:FLQHN:KXEXR, NDEGLEX:VPTGQ:EPLT:EQLSN:LEEHS:QXEXR, SSVKXQP:SRVHH:EPLT:EQLSN:RLEEH:LEXAXA, RNVQXRP:TQVQL:EPLT:EQLSN:TLEDH:LAXKXE, KIPKAXX:VPQEL:EPLT:EQLSN:DMVKS:GXGXR, GIPEPXX:VPQKM:EPLT:EQLSN:NMVKS:SXAXR, SIPKAXX:VPQEL:EPLT:EQLSN:EMVKS:GXGXR, HVTKPTX:VPQKL:EPLT:EQLSN:NMVKS:SXGXH, YVPKPXX:VPQKL:EPLT:EQLSN:NMVKS:SXGXH, TVPKPXX:VPQQL:EPLT:EQLSN:NMVKS:AXGXH, AVPKAXX:VPQKL:EPLT:EQLSN:NMVKS:AXGXH, KVGKAXX:VPQKL:EPLT:EQLSN:EGMSVKS:XGXR, KASKAXX:VPQKL:EPLT:EQLSN:GMVKS:EXGXR, GSAGPXX:VPQKM:EPLT:EQLSN:GMVKS:RXGXS, AAPASXX:VPQRL:EPLT:EQLSN:DMVKS:AXGXR, STPPTXX:VPQRL:EPLT:EQLSN:DMVKS:SXGXR, HVPKPXX:VPQKL:EPLT:EQLSN:NMVKS:SXGXH, RVPSTXX:VPQKT:EPLT:EQLSN:MIVKS:XGXL, ASAAPXX:VPQAL:EPLT:EQLSN:MIVKS:XKXS, ASASPXX:VPQDL:EPLT:EQLSN:MIVKS:XKXS, NDEGLEX:VPQEE:EPLT:EQLSN:FLVKS:KXEXR, NDEGLEX:VPQGQ:EPLT:EQLSN:LEVKS:QXEXR, SSVKXQP:VPQHH:EPLT:EQLSN:RLVKS:LEXAXA, RNVQXRP:VPQQL:EPLT:EQLSN:TLVKS:LAXKXE, ASASPXX:VSQDL:EPLT:EQLSN:MIVKS:XKXS, KIPKAXX:VPTEL:SNIT:IGEMS:DMQHN:GXGXR, GIPEPXX:VPTKM:SNIT:IGEMS:NMQHN:SXAXR, SIPKAXX:VPTEL:SNIT:IGEMS:EMQHN:GXGXR, HVTKPTX:VPTKL:SNIT:IGEMS:NMQHN:SXGXH, YVPKPXX:VPTKL:SNIT:IGEMS:NMQHN:SXGXH, TVPKPXX:VPTQL:SNIT:IGEMS:NMQHN:AXGXH, AVPKAXX:VPTKL:SNIT:IGEMS:NMQHN:AXGXH, KVGKAXX:VPTKL:SNIT:IGEMS:EGMSQHN:XGXR, KASKAXX:VPTKL:SNIT:IGEMS:GMQHN:EXGXR, GSAGPXX:VPTKM:SNIT:IGEMS:GMQHN:RXGXS, AAPASXX:VPTRL:SNIT:IGEMS:DMQHN:AXGXR, STPPTXX:VPTRL:SNIT:IGEMS:DMQHN:SXGXR, HVPKPXX:VPTKL:SNIT:IGEMS:NMQHN:SXGXH, RVPSTXX:VPTKT:SNIT:IGEMS:MIQHN:XGXL, ASAAPXX:VPTAL:SNIT:IGEMS:MIQHN:XKXS, ASASPXX:VPTDL:SNIT:IGEMS:MIQHN:XKXS, ASASPXX:VPTDL:SNIT:IGEMS:MVQHN:XKXS, NDEGLEX:VPTGQ:SNIT:IGEMS:LEQHN:QXEXR, NDEGLEX:VPTEE:SNIT:IGEMS:FLQHN:KXEXR, SSVKXQP:VPTHH:SNIT:IGEMS:RLQHN:LEXAXA, RNVQXRP:VPTQL:SNIT:IGEMS:TLQHN:LAXKXE, KIPKAXX:VPTEL:SNIT:IGEMS:DMVVE:GXGXR, GIPEPXX:VPEKM:SNIT:IGEMS:NMTVE:SXAXR, SIPKAXX:VPTEL:SNIT:IGEMS:EMVVE:GXGXR, HVTKPTX:APTKL:SNIT:IGEMS:NMVVR:SXGXH, YVPKPXX:APTKL:SNIT:IGEMS:NMVVR:SXGXH, TVPKPXX:APTQL:SNIT:IGEMS:NMVVR:AXGXH, AVPKAXX:APTKL:SNIT:IGEMS:NMVVK:AXGXH, KVGKAXX:VPTKL:SNIT:IGEMS:EGMSVAE:XGXR, KASKAXX:VPTKL:SNIT:IGEMS:GMAVS:EXGXR, GSAGPXX:TPTKM:SNIT:IGEMS:GMVVD:RXGXS, AAPASXX:VPARL:SNIT:IGEMS:DMVVE:AXGXR, STPPTXX:VPTRL:SNIT:IGEMS:DMVVE:SXGXR, HVPKPXX:APTKL:SNIT:IGEMS:NMVVR:SXGXH, RVPSTXX:APVKT :SNIT:IGEMS:MIVEE:XGXL, ASAAPXX:VPQAL:SNIT:IGEMS:MIVRS:XKXS, ASASPXX:VSQDL:SNIT:IGEMS:MIVKS:XKXS, ASASPXX:VPQDL:SNIT:IGEMS:MVVKS:XKXS, NDEGLEX:VPTGQ:SNIT:IGEMS:LEEHS:QXEXR, SSVKXQP:SRVHH:SNIT:IGEMS:RLEEH:LEXAXA, RNVQXRP:TQVQL:SNIT:IGEMS:TLEDH:LAXKXE, KIPKAXX:VPTEL:SNIT:LGEMS:DMEHS:GXGXR, GIPEPXX:VPTKM:SNIT:LGEMS:NMEHS:SXAXR, SIPKAXX:VPTEL:SNIT:LGEMS:EMEHS:GXGXR, HVTKPTX:VPTKL:SNIT:LGEMS:NMEHS:SXGXH, YVPKPXX:VPTKL:SNIT:LGEMS:NMEHS:SXGXH, TVPKPXX:VPTQL:SNIT:LGEMS:NMEHS:AXGXH, AVPKAXX:VPTKL:SNIT:LGEMS:NMEHS:AXGXH, KVGKAXX:VPTKL:SNIT:LGEMS:EGMSEHS:XGXR, KASKAXX:VPTKL:SNIT:LGEMS:GMEHS:EXGXR, GSAGPXX:VPTKM:SNIT:LGEMS:GMEHS:RXGXS, AAPASXX:VPTRL:SNIT:LGEMS:DMEHS:AXGXR, STPPTXX:VPTRL:SNIT:LGEMS:DMEHS:SXGXR, HVPKPXX:VPTKL:SNIT:LGEMS:NMEHS:SXGXH, RVPSTXX:VPTKT:SNIT:LGEMS:MIEHS:XGXL, ASAAPXX:VPTAL:SNIT:LGEMS:MIEHS:XKXS, ASASPXX:VPTDL:SNIT:LGEMS:MIEHS:XKXS, ASASPXX:VPTDL:SNIT:LGEMS:MVEHS:XKXS, NDEGLEX:VPTEE:SNIT:LGEMS:FLEHS:KXEXR, SSVKXQP:VPTHH:SNIT:LGEMS:RLEHS:LEXAXA, RNVQXRP:VPTQL:SNIT:LGEMS:TLEHS:LAXKXE, KIPKAXX:VPTEL:SNIT:LGEMS:DMVVE:GXGXR, GIPEPXX:VPEKM:SNIT:LGEMS:NMTVE:SXAXR, SIPKAXX:VPTEL:SNIT:LGEMS:EMVVE:GXGXR, HVTKPTX:APTKL:SNIT:LGEMS:NMVVR:SXGXH, YVPKPXX:APTKL:SNIT:LGEMS:NMVVR:SXGXH, TVPKPXX:APTQL:SNIT:LGEMS:NMVVR:AXGXH, AVPKAXX:APTKL:SNIT:LGEMS:NMVVK:AXGXH, KVGKAXX:VPTKL:SNIT:LGEMS:EGMSVAE:XGXR, KASKAXX:VPTKL:SNIT:LGEMS:GMAVS:EXGXR, GSAGPXX:TPTKM:SNIT:LGEMS:GMVVD:RXGXS, AAPASXX:VPARL:SNIT:LGEMS:DMVVE:AXGXR, STPPTXX:VPTRL:SNIT:LGEMS:DMVVE:SXGXR, HVPKPXX:APTKL:SNIT:LGEMS:NMVVR:SXGXH, RVPSTXX:APVKT:SNIT:LGEMS:MIVEE:XGXL, ASAAPXX:VPQAL:SNIT:LGEMS:MIVRS:XKXS, ASASPXX:VSQDL:SNIT:LGEMS:MIVKS:XKXS, ASASPXX:VPQDL:SNIT:LGEMS:MVVKS:XKXS, NDEGLEX:VPTEE:SNIT:LGEMS:FLQHN:KXEXR, SSVKXQP:SRVHH:SNIT:LGEMS:RLEEH:LEXAXA, RNVQXRP:TQVQL:SNIT:LGEMS:TLEDH:LAXKXE, KIPKAXX:SRVEL:RSVK:KEVQV:DMEEH:GXGXR, GIPEPXX:SRVKM:RSVK:KEVQV:NMEEH:SXAXR, SIPKAXX:SRVEL:RSVK:KEVQV:EMEEH:GXGXR, HVTKPTX:SRVKL:RSVK:KEVQV:NMEEH:SXGXH, YVPKPXX:SRVKL:RSVK:KEVQV:NMEEH:SXGXH, TVPKPXX:SRVQL:RSVK:KEVQV:NMEEH:AXGXH, AVPKAXX:SRVKL:RSVK:KEVQV:NMEEH:AXGXH, KVGKAXX:SRVKL:RSVK:KEVQV:EGMSEEH:XGXR, KASKAXX:SRVKL:RSVK:KEVQV:GMEEH:EXGXR, GSAGPXX:SRVKM:RSVK:KEVQV:GMEEH:RXGXS, AAPASXX:SRVRL:RSVK:KEVQV:DMEEH:AXGXR, STPPTXX:SRVRL:RSVK:KEVQV:DMEEH:SXGXR, HVPKPXX:SRVKL:RSVK:KEVQV:NMEEH:SXGXH, RVPSTXX:SRVKT:RSVK:KEVQV:MIEEH:XGXL, ASAAPXX:SRVAL:RSVK:KEVQV:MIEEH:XKXS, ASASPXX:SRVDL:RSVK:KEVQV:MIEEH:XKXS, ASASPXX:SRVDL:RSVK:KEVQV:MVEEH:XKXS, NDEGLEX:SRVEE:RSVK:KEVQV:FLEEH:KXEXR, NDEGLEX:SRVGQ:RSVK:KEVQV:LEEEH:QXEXR, RNVQXRP:SRVQL:RSVK:KEVQV:TLEEH:LAXKXE, KIPKAXX:VPTEL:RSVK:KEVQV:DMVVE:GXGXR, GIPEPXX:VPEKM:RSVK:KEVQV:NMTVE:SXAXR, SIPKAXX:VPTEL:RSVK:KEVQV:EMVVE:GXGXR, HVTKPTX:APTKL:RSVK:KEVQV:NMVVR:SXGXH, YVPKPXX:APTKL:RSVK:KEVQV:NMVVR:SXGXH, TVPKPXX:APTQL:RSVK:KEVQV:NMVVR:AXGXH, AVPKAXX:APTKL:RSVK:KEVQV:NMVVK:AXGXH, KVGKAXX:VPTKL:RSVK:KEVQV:EGMSVAE:XGXR, KASKAXX:VPTKL:RSVK:KEVQV:GMAVS:EXGXR, GSAGPXX:TPTKM:RSVK:KEVQV:GMVVD:RXGXS, AAPASXX:VPARL:RSVK:KEVQV:DMVVE:AXGXR, STPPTXX:VPTRL:RSVK:KEVQV:DMVVE:SXGXR, HVPKPXX:APTKL:RSVK:KEVQV:NMVVR:SXGXH, RVPSTXX:APVKT:RSVK:KEVQV:MIVEE:XGXL, ASAAPXX:VPQAL:RSVK:KEVQV:MIVRS:XKXS, ASASPXX:VSQDL:RSVK:KEVQV:MIVKS:XKXS, ASASPXX:VPQDL:RSVK:KEVQV:MVVKS:XKXS, NDEGLEX:VPTEE:RSVK:KEVQV:FLQHN:KXEXR, NDEGLEX:VPTGQ:RSVK:KEVQV:LEEHS:QXEXR, RNVQXRP:TQVQL:RSVK:KEVQV:TLEDH:LAXKXE, KIPKAXX:TQVEL:RPVQ:KKATV:DMEDH:GXGXR, GIPEPXX:TQVKM:RPVQ:KKATV:NMEDH:SXAXR, SIPKAXX:TQVEL:RPVQ:KKATV:EMEDH:GXGXR, HVTKPTX:TQVKL:RPVQ:KKATV:NMEDH:SXGXH, YVPKPXX:TQVKL:RPVQ:KKATV:NMEDH:SXGXH, TVPKPXX:TQVQL:RPVQ:KKATV:NMEDH:AXGXH, AVPKAXX:TQVKL:RPVQ:KKATV:NMEDH:AXGXH, KVGKAXX:TQVKL:RPVQ:KKATV:EGMSEDH:XGXR, KASKAXX:TQVKL:RPVQ:KKATV:GMEDH:EXGXR, GSAGPXX:TQVKM:RPVQ:KKATV:GMEDH:RXGXS, AAPASXX:TQVRL:RPVQ:KKATV:DMEDH:AXGXR, STPPTXX:TQVRL:RPVQ:KKATV:DMEDH:SXGXR, HVPKPXX:TQVKL:RPVQ:KKATV:NMEDH:SXGXH, RVPSTXX:TQVKT:RPVQ:KKATV:MIEDH:XGXL, ASAAPXX:TQVAL:RPVQ:KKATV:MIEDH:XKXS, ASASPXX:TQVDL:RPVQ:KKATV:MIEDH:XKXS, ASASPXX:TQVDL:RPVQ:KKATV:MVEDH:XKXS, NDEGLEX:TQVEE:RPVQ:KKATV:FLEDH:KXEXR, NDEGLEX:TQVGQ:RPVQ:KKATV:LEEDH:QXEXR, SSVKXQP:TQVHH:RPVQ:KKATV:RLEDH:LEXAXA, KIPKAXX:VPTEL:RPVQ:KKATV:DMVVE:GXGXR, GIPEPXX:VPEKM:RPVQ:KKATV:NMTVE:SXAXR, SIPKAXX:VPTEL:RPVQ:KKATV:EMVVE:GXGXR, HVTKPTX:APTKL:RPVQ:KKATV:NMVVR:SXGXH, YVPKPXX:APTKL:RPVQ:KKATV:NMVVR:SXGXH, TVPKPXX:APTQL:RPVQ:KKATV:NMVVR:AXGXH, AVPKAXX:APTKL:RPVQ:KKATV:NMVVK:AXGXH, KVGKAXX:VPTKL:RPVQ:KKATV:EGMSVAE:XGXR, KASKAXX:VPTKL:RPVQ:KKATV:GMAVS:EXGXR, GSAGPXX:TPTKM:RPVQ:KKATV:GMVVD:RXGXS, AAPASXX:VPARL:RPVQ:KKATV:DMVVE:AXGXR, STPPTXX:VPTRL:RPVQ:KKATV:DMVVE:SXGXR, HVPKPXX:APTKL:RPVQ:KKATV:NMVVR:SXGXH, RVPSTXX:APVKT:RPVQ:KKATV:MIVEE:XGXL, ASAAPXX:VPQAL:RPVQ:KKATV:MIVRS:XKXS, ASASPXX:VSQDL:RPVQ:KKATV:MIVKS:XKXS, ASASPXX:VPQDL:RPVQ:KKATV:MVVKS:XKXS, NDEGLEX:VPTEE:RPVQ:KKATV:FLQHN:KXEXR, NDEGLEX:VPTGQ:RPVQ:KKATV:LEEHS:QXEXR and SSVKXQP:SRVHH:RPVQ:KKATV:RLEEH:LEXAXA. More particularly, the hexaplet PEP7:PEP5:PEP1:PEP2:PEP6:PEP8 is selected from the group consisting of GIPEPXX:VPTKM:SAIS:LKNYQ:NMVVE:SXAXR, HVTKPTX:VPTKL:SAIS:LKNYQ:NMVVE:SXGXH, YVPKPXX:VPTKL:SAIS:LKNYQ:NMVVE:SXGXH, TVPKPXX:VPTQL:SAIS:LKNYQ:NMVVE:AXGXH, AVPKAXX:VPTKL:SAIS:LKNYQ:NMVVE:AXGXH, KVGKAXX:VPTKL:SAIS:LKNYQ:EGMSVVE:XGXR, KASKAXX:VPTKL:SAIS:LKNYQ:GMVVE:EXGXR, GSAGPXX:VPTKM:SAIS:LKNYQ:GMVVE:RXGXS, AAPASXX:VPTRL:SAIS:LKNYQ:DMVVE:AXGXR, STPPTXX:VPTRL:SAIS:LKNYQ:DMVVE:SXGXR, HVPKPXX:VPTKL:SAIS:LKNYQ:NMVVE:SXGXH, RVPSTXX:VPTKT:SAIS:LKNYQ:MIVVE:XGXL, ASAAPXX:VPTAL:SAIS:LKNYQ:MIVVE:XKXS, ASASPXX:VPTDL:SAIS:LKNYQ:MIVVE:XKXS, ASASPXX:VPTDL:SAIS:LKNYQ:MVVVE:XKXS, GIPEPXX:VPEKM:SAIS:LKNYQ:NMTVE:SXAXR, HVTKPTX:APTKL:SAIS:LKNYQ:NMVVR:SXGXH, YVPKPXX:APTKL:SAIS:LKNYQ:NMVVR:SXGXH, TVPKPXX:APTQL:SAIS:LKNYQ:NMVVR:AXGXH, AVPKAXX:APTKL:SAIS:LKNYQ:NMVVK:AXGXH, KVGKAXX:VPTKL:SAIS:LKNYQ:EGMSVAE:XGXR, KASKAXX:VPTKL:SAIS:LKNYQ:GMAVS:EXGXR, GSAGPXX:TPTKM:SAIS:LKNYQ:GMVVD:RXGXS, AAPASXX:VPARL:SAIS:LKNYQ:DMVVE:AXGXR, HVPKPXX:APTKL:SAIS:LKNYQ:NMVVR:SXGXH, RVPSTXX:APVKT:SAIS:LKNYQ:MIVEE:XGXL, ASAAPXX:VPQAL:SAIS:LKNYQ:MIVRS:XKXS, ASASPXX:VSQDL:SAIS:LKNYQ:MIVKS:XKXS, ASASPXX:VPQDL:SAIS:LKNYQ:MVVKS:XKXS, NDEGLEX:VPTEE:SAIS:LKNYQ:FLQHN:KXEXR, NDEGLEX:VPTGQ:SAIS:LKNYQ:LEEHS:QXEXR, SSVKXQP:SRVHH:SAIS:LKNYQ:RLEEH:LEXAXA, RNVQXRP:TQVQL:SAIS:LKNYQ:TLEDH:LAXKXE, KIPKAXX:VPEEL:SSLS:LKVYP:DMTVE:GXGXR, SIPKAXX:VPEEL:SSLS:LKVYP:EMTVE:GXGXR, HVTKPTX:VPEKL:SSLS:LKVYP:NMTVE:SXGXH, YVPKPXX:VPEKL:SSLS:LKVYP:NMTVE:SXGXH, TVPKPXX:VPEQL:SSLS:LKVYP:NMTVE:AXGXH, AVPKAXX:VPEKL:SSLS:LKVYP:NMTVE:AXGXH, KVGKAXX:VPEKL:SSLS:LKVYP:EGMSTVE:XGXR, KASKAXX:VPEKL:SSLS:LKVYP:GMTVE:EXGXR, GSAGPXX:VPEKM:SSLS:LKVYP:GMTVE:RXGXS, AAPASXX:VPERL:SSLS:LKVYP:DMTVE:AXGXR, STPPTXX:VPERL:SSLS:LKVYP:DMTVE:SXGXR, HVPKPXX:VPEKL:SSLS:LKVYP:NMTVE:SXGXH, RVPSTXX:VPEKT:SSLS:LKVYP:MITVE:XGXL, ASAAPXX:VPEAL:SSLS:LKVYP:MITVE:XKXS, ASASPXX:VPEDL:SSLS:LKVYP:MITVE:XKXS, ASASPXX:VPEDL:SSLS:LKVYP:MVTVE:XKXS, KIPKAXX:VPTEL:SSLS:LKVYP:DMVVE:GXGXR, SIPKAXX:VPTEL:SSLS:LKVYP:EMVVE:GXGXR, HVTKPTX:APTKL:SSLS:LKVYP:NMVVR:SXGXH, YVPKPXX:APTKL:SSLS:LKVYP:NMVVR:SXGXH, TVPKPXX:APTQL:SSLS:LKVYP:NMVVR:AXGXH, AVPKAXX:APTKL:SSLS:LKVYP:NMVVK:AXGXH, KVGKAXX:VPTKL:SSLS:LKVYP:EGMSVAE:XGXR, KASKAXX:VPTKL:SSLS:LKVYP:GMAVS:EXGXR, GSAGPXX:TPTKM:SSLS:LKVYP:GMVVD:RXGXS, AAPASXX:VPARL:SSLS:LKVYP:DMVVE:AXGXR, STPPTXX:VPTRL:SSLS:LKVYP:DMVVE:SXGXR, HVPKPXX:APTKL:SSLS:LKVYP:NMVVR:SXGXH, RVPSTXX:APVKT:SSLS:LKVYP:MIVEE:XGXL, ASAAPXX:VPQAL:SSLS:LKVYP:MIVRS:XKXS, ASASPXX:VSQDL:SSLS:LKVYP:MIVKS:XKXS, ASASPXX:VPQDL:SSLS:LKVYP:MVVKS:XKXS, NDEGLEX:VPTEE:SSLS:LKVYP:FLQHN:KXEXR, NDEGLEX:VPTGQ:SSLS:LKVYP:LEEHS:QXEXR, SSVKXQP:SRVHH:SSLS:LKVYP:RLEEH:LEXAXA, RNVQXRP:TQVQL:SSLS:LKVYP:TLEDH:LAXKXE, KIPKAXX:APTEL:NAIS:LKKYR:DMVVR:GXGXR, GIPEPXX:APTKM:NAIS:LKKYR:NMVVR:SXAXR, SIPKAXX:APTEL:NAIS:LKKYR:EMVVR:GXGXR, AVPKAXX:APTKL:NAIS:LKKYR:NMVVR:AXGXH, KVGKAXX:APTKL:NAIS:LKKYR:EGMSVVR:XGXR, KASKAXX:APTKL:NAIS:LKKYR:GMVVR:EXGXR, GSAGPXX:APTKM:NAIS:LKKYR:GMVVR:RXGXS, AAPASXX:APTRL:NAIS:LKKYR:DMVVR:AXGXR, STPPTXX:APTRL:NAIS:LKKYR:DMVVR:SXGXR, RVPSTXX:APTKT:NAIS:LKKYR:MIVVR:XGXL, ASAAPXX:APTAL:NAIS:LKKYR:MIVVR:XKXS, ASASPXX:APTDL:NAIS:LKKYR:MIVVR:XKXS, ASASPXX:APTDL:NAIS:LKKYR:MVVVR:XKXS, KIPKAXX:VPTEL:NAIS:LKKYR:DMVVE:GXGXR, GIPEPXX:VPEKM:NAIS:LKKYR:NMTVE:SXAXR, SIPKAXX:VPTEL:NAIS:LKKYR:EMVVE:GXGXR, AVPKAXX:APTKL:NAIS:LKKYR:NMVVK:AXGXH, KVGKAXX:VPTKL:NAIS:LKKYR:EGMSVAE:XGXR, KASKAXX:VPTKL:NAIS:LKKYR:GMAVS:EXGXR, GSAGPXX:TPTKM:NAIS:LKKYR:GMVVD:RXGXS, AAPASXX:VPARL:NAIS:LKKYR:DMVVE:AXGXR, STPPTXX:VPTRL:NAIS:LKKYR:DMVVE:SXGXR, RVPSTXX:APVKT:NAIS:LKKYR:MIVEE:XGXL, ASAAPXX:VPQAL:NAIS:LKKYR:MIVRS:XKXS, ASASPXX:VSQDL:NAIS:LKKYR:MIVKS:XKXS, ASASPXX:VPQDL:NAIS:LKKYR:MVVKS:XKXS, NDEGLEX:VPTEE:NAIS:LKKYR:FLQHN:KXEXR, NDEGLEX:VPTGQ:NAIS:LKKYR:LEEHS:QXEXR, SSVKXQP:SRVHH:NAIS:LKKYR:RLEEH:LEXAXA, RNVQXRP:TQVQL:NAIS:LKKYR:TLEDH:LAXKXE, KIPKAXX:APTEL:SATS:LRKHR:DMVVK:GXGXR, GIPEPXX:APTKM:SATS:LRKHR:NMVVK:SXAXR, SIPKAXX:APTEL:SATS:LRKHR:EMVVK:GXGXR, HVTKPTX:APTKL:SATS:LRKHR:NMVVK:SXGXH, YVPKPXX:APTKL:SATS:LRKHR:NMVVK:SXGXH, TVPKPXX:APTQL:SATS:LRKHR:NMVVK:AXGXH, KVGKAXX:APTKL:SATS:LRKHR:EGMSVVK:XGXR, KASKAXX:APTKL:SATS:LRKHR:GMVVK:EXGXR, GSAGPXX:APTKM:SATS:LRKHR:GMVVK:RXGXS, AAPASXX:APTRL:SATS:LRKHR:DMVVK:AXGXR, STPPTXX:APTRL:SATS:LRKHR:DMVVK:SXGXR, HVPKPXX:APTKL:SATS:LRKHR:NMVVK:SXGXH, RVPSTXX:APTKT:SATS:LRKHR:MIVVK:XGXL, ASAAPXX:APTAL:SATS:LRKHR:MIVVK:XKXS, ASASPXX:APTDL:SATS:LRKHR:MIVVK:XKXS, ASASPXX:APTDL:SATS:LRKHR:MVVVK:XKXS, KIPKAXX:VPTEL:SATS:LRKHR:DMVVE:GXGXR, GIPEPXX:VPEKM:SATS:LRKHR:NMTVE:SXAXR, SIPKAXX:VPTEL:SATS:LRKHR:EMVVE:GXGXR, HVTKPTX:APTKL:SATS:LRKHR:NMVVR:SXGXH, YVPKPXX:APTKL:SATS:LRKHR:NMVVR:SXGXH, TVPKPXX:APTQL:SATS:LRKHR:NMVVR:AXGXH, KVGKAXX:VPTKL:SATS:LRKHR:EGMSVAE:XGXR, KASKAXX:VPTKL:SATS:LRKHR:GMAVS:EXGXR, GSAGPXX:TPTKM:SATS:LRKHR:GMVVD:RXGXS, AAPASXX:VPARL:SATS:LRKHR:DMVVE:AXGXR, STPPTXX:VPTRL:SATS:LRKHR:DMVVE:SXGXR, HVPKPXX:APTKL:SATS:LRKHR:NMVVR:SXGXH, RVPSTXX:APVKT:SATS:LRKHR:MIVEE:XGXL, ASAAPXX:VPQAL:SATS:LRKHR:MIVRS:XKXS, ASASPXX:VSQDL:SATS:LRKHR:MIVKS:XKXS, ASASPXX:VPQDL:SATS:LRKHR:MVVKS:XKXS, NDEGLEX:VPTEE:SATS:LRKHR:FLQHN:KXEXR, NDEGLEX:VPTGQ:SATS:LRKHR:LEEHS:QXEXR, SSVKXQP:SRVHH:SATS:LRKHR:RLEEH:LEXAXA, RNVQXRP:TQVQL:SATS:LRKHR:TLEDH:LAXKXE, KIPKAXX:VPTEL:SPIS:LKYHY:DMVAE:GXGXR, GIPEPXX:VPTKM:SPIS:LKYHY:NMVAE:SXAXR, SIPKAXX:VPTEL:SPIS:LKYHY:EMVAE:GXGXR, HVTKPTX:VPTKL:SPIS:LKYHY:NMVAE:SXGXH, YVPKPXX:VPTKL:SPIS:LKYHY:NMVAE:SXGXH, TVPKPXX:VPTQL:SPIS:LKYHY:NMVAE:AXGXH, AVPKAXX:VPTKL:SPIS:LKYHY:NMVAE:AXGXH, KASKAXX:VPTKL:SPIS:LKYHY:GMVAE:EXGXR, GSAGPXX:VPTKM:SPIS:LKYHY:GMVAE:RXGXS, AAPASXX:VPTRL:SPIS:LKYHY:DMVAE:AXGXR, STPPTXX:VPTRL:SPIS:LKYHY:DMVAE:SXGXR, HVPKPXX:VPTKL:SPIS:LKYHY:NMVAE:SXGXH, RVPSTXX:VPTKT:SPIS:LKYHY:MIVAE:XGXL, ASAAPXX:VPTAL:SPIS:LKYHY:MIVAE:XKXS, ASASPXX:VPTDL:SPIS:LKYHY:MIVAE:XKXS, ASASPXX:VPTDL:SPIS:LKYHY:MVVAE:XKXS, KIPKAXX:VPTEL:SPIS:LKYHY:DMVVE:GXGXR, GIPEPXX:VPEKM:SPIS:LKYHY:NMTVE:SXAXR, SIPKAXX:VPTEL:SPIS:LKYHY:EMVVE:GXGXR, HVTKPTX:APTKL:SPIS:LKYHY:NMVVR:SXGXH, YVPKPXX:APTKL:SPIS:LKYHY:NMVVR:SXGXH, TVPKPXX:APTQL:SPIS:LKYHY:NMVVR:AXGXH, AVPKAXX:APTKL:SPIS:LKYHY:NMVVK:AXGXH, KASKAXX:VPTKL:SPIS:LKYHY:GMAVS:EXGXR, GSAGPXX:TPTKM:SPIS:LKYHY:GMVVD:RXGXS, AAPASXX:VPARL:SPIS:LKYHY:DMVVE:AXGXR, STPPTXX:VPTRL:SPIS:LKYHY:DMVVE:SXGXR, HVPKPXX:APTKL:SPIS:LKYHY:NMVVR:SXGXH, RVPSTXX:APVKT:SPIS:LKYHY:MIVEE:XGXL, ASAAPXX:VPQAL:SPIS:LKYHY:MIVRS:XKXS, ASASPXX:VSQDL:SPIS:LKYHY:MIVKS:XKXS, ASASPXX:VPQDL:SPIS:LKYHY:MVVKS:XKXS, NDEGLEX:VPTEE:SPIS:LKYHY:FLQHN:KXEXR, NDEGLEX:VPTGQ:SPIS:LKYHY:LEEHS:QXEXR, SSVKXQP:SRVHH:SPIS:LKYHY:RLEEH:LEXAXA, RNVQXRP:TQVQL:SPIS:LKYHY:TLEDH:LAXKXE, KIPKAXX:VPTEL:EPIS:KFKYE:DMAVS:GXGXR, GIPEPXX:VPTKM:EPIS:KFKYE:NMAVS:SXAXR, SIPKAXX:VPTEL:EPIS:KFKYE:EMAVS:GXGXR, HVTKPTX:VPTKL:EPIS:KFKYE:NMAVS:SXGXH, YVPKPXX:VPTKL:EPIS:KFKYE:NMAVS:SXGXH, TVPKPXX:VPTQL:EPIS:KFKYE:NMAVS:AXGXH, AVPKAXX:VPTKL:EPIS:KFKYE:NMAVS:AXGXH, KVGKAXX:VPTKL:EPIS:KFKYE:EGMSAVS:XGXR, GSAGPXX:VPTKM:EPIS:KFKYE:GMAVS:RXGXS, AAPASXX:VPTRL:EPIS:KFKYE:DMAVS:AXGXR, STPPTXX:VPTRL:EPIS:KFKYE:DMAVS:SXGXR, HVPKPXX:VPTKL:EPIS:KFKYE:NMAVS:SXGXH, RVPSTXX:VPTKT:EPIS:KFKYE:MIAVS:XGXL, ASAAPXX:VPTAL:EPIS:KFKYE:MIAVS:XKXS, ASASPXX:VPTDL:EPIS:KFKYE:MIAVS:XKXS, ASASPXX:VPTDL:EPIS:KFKYE:MVAVS:XKXS, KIPKAXX:VPTEL:EPIS:KFKYE:DMVVE:GXGXR, GIPEPXX:VPEKM:EPIS:KFKYE:NMTVE:SXAXR, SIPKAXX:VPTEL:EPIS:KFKYE:EMVVE:GXGXR, HVTKPTX:APTKL:EPIS:KFKYE:NMVVR:SXGXH, YVPKPXX:APTKL:EPIS:KFKYE:NMVVR:SXGXH, TVPKPXX:APTQL:EPIS:KFKYE:NMVVR:AXGXH, AVPKAXX:APTKL:EPIS:KFKYE:NMVVK:AXGXH, KVGKAXX:VPTKL:EPIS:KFKYE:EGMSVAE:XGXR, GSAGPXX:TPTKM:EPIS:KFKYE:GMVVD:RXGXS, AAPASXX:VPARL:EPIS:KFKYE:DMVVE:AXGXR, STPPTXX:VPTRL:EPIS:KFKYE:DMVVE:SXGXR, HVPKPXX:APTKL:EPIS:KFKYE:NMVVR:SXGXH, RVPSTXX:APVKT:EPIS:KFKYE:MIVEE:XGXL, ASAAPXX:VPQAL:EPIS:KFKYE:MIVRS:XKXS, ASASPXX:VSQDL:EPIS:KFKYE:MIVKS:XKXS, ASASPXX:VPQDL:EPIS:KFKYE:MVVKS:XKXS, NDEGLEX:VPTEE:EPIS:KFKYE:FLQHN:KXEXR, NDEGLEX:VPTGQ:EPIS:KFKYE:LEEHS:QXEXR, SSVKXQP:SRVHH:EPIS:KFKYE:RLEEH:LEXAXA, RNVQXRP:TQVQL:EPIS:KFKYE:TLEDH:LAXKXE, KIPKAXX:TPTEL:SPIN:YGKIP:DMVVD:GXGXR, GIPEPXX:TPTKM:SPIN:YGKIP:NMVVD:SXAXR, SIPKAXX:TPTEL:SPIN:YGKIP:EMVVD:GXGXR, HVTKPTX:TPTKL:SPIN:YGKIP:NMVVD:SXGXH, YVPKPXX:TPTKL:SPIN:YGKIP:NMVVD:SXGXH, TVPKPXX:TPTQL:SPIN:YGKIP:NMVVD:AXGXH, AVPKAXX:TPTKL:SPIN:YGKIP:NMVVD:AXGXH, KVGKAXX:TPTKL:SPIN:YGKIP:EGMSVVD:XGXR, KASKAXX:TPTKL:SPIN:YGKIP:GMVVD:EXGXR, AAPASXX:TPTRL:SPIN:YGKIP:DMVVD:AXGXR, STPPTXX:TPTRL:SPIN:YGKIP:DMVVD:SXGXR, HVPKPXX:TPTKL:SPIN:YGKIP:NMVVD:SXGXH, RVPSTXX:TPTKT:SPIN:YGKIP:MIVVD:XGXL, ASAAPXX:TPTAL:SPIN:YGKIP:MIVVD:XKXS, ASASPXX:TPTDL:SPIN:YGKIP:MIVVD:XKXS, ASASPXX:TPTDL:SPIN:YGKIP:MVVVD:XKXS, KIPKAXX:VPTEL:SPIN:YGKIP:DMVVE:GXGXR, GIPEPXX:VPEKM:SPIN:YGKIP:NMTVE:SXAXR, SIPKAXX:VPTEL:SPIN:YGKIP:EMVVE:GXGXR, HVTKPTX:APTKL:SPIN:YGKIP:NMVVR:SXGXH, YVPKPXX:APTKL:SPIN:YGKIP:NMVVR:SXGXH, TVPKPXX:APTQL:SPIN:YGKIP:NMVVR:AXGXH, AVPKAXX:APTKL:SPIN:YGKIP:NMVVK:AXGXH, KVGKAXX:VPTKL:SPIN:YGKIP:EGMSVAE:XGXR, KASKAXX:VPTKL:SPIN:YGKIP:GMAVS:EXGXR, AAPASXX:VPARL:SPIN:YGKIP:DMVVE:AXGXR, STPPTXX:VPTRL:SPIN:YGKIP:DMVVE:SXGXR, HVPKPXX:APTKL:SPIN:YGKIP:NMVVR:SXGXH, RVPSTXX:APVKT:SPIN:YGKIP:MIVEE:XGXL, ASAAPXX:VPQAL:SPIN:YGKIP:MIVRS:XKXS, ASASPXX:VSQDL:SPIN:YGKIP:MIVKS:XKXS, ASASPXX:VPQDL:SPIN:YGKIP:MVVKS:XKXS, NDEGLEX:VPTEE:SPIN:YGKIP:FLQHN:KXEXR, NDEGLEX:VPTGQ:SPIN:YGKIP:LEEHS:QXEXR, SSVKXQP:SRVHH:SPIN:YGKIP:RLEEH:LEXAXA, RNVQXRP:TQVQL:SPIN:YGKIP:TLEDH:LAXKXE, KIPKAXX:VPAEL:SPIS:YKQYE:DMVVE:GXGXR, GIPEPXX:VPAKM:SPIS:YKQYE:NMVVE:SXAXR, SIPKAXX:VPAEL:SPIS:YKQYE:EMVVE:GXGXR, HVTKPTX:VPAKL:SPIS:YKQYE:NMVVE:SXGXH, YVPKPXX:VPAKL:SPIS:YKQYE:NMVVE:SXGXH, TVPKPXX:VPAQL:SPIS:YKQYE:NMVVE:AXGXH, AVPKAXX:VPAKL:SPIS:YKQYE:NMVVE:AXGXH, KVGKAXX:VPAKL:SPIS:YKQYE:EGMSVVE:XGXR, KASKAXX:VPAKL:SPIS:YKQYE:GMVVE:EXGXR, GSAGPXX:VPAKM:SPIS:YKQYE:GMVVE:RXGXS, STPPTXX:VPARL:SPIS:YKQYE:DMVVE:SXGXR, HVPKPXX:VPAKL:SPIS:YKQYE:NMVVE:SXGXH, RVPSTXX:VPAKT:SPIS:YKQYE:MIVVE:XGXL, ASAAPXX:VPAAL:SPIS:YKQYE:MIVVE:XKXS, ASASPXX:VPADL:SPIS:YKQYE:MIVVE:XKXS, ASASPXX:VPADL:SPIS:YKQYE:MVVVE:XKXS, KIPKAXX:VPTEL:SPIS:YKQYE:DMVVE:GXGXR, GIPEPXX:VPEKM:SPIS:YKQYE:NMTVE:SXAXR, SIPKAXX:VPTEL:SPIS:YKQYE:EMVVE:GXGXR, HVTKPTX:APTKL:SPIS:YKQYE:NMVVR:SXGXH, YVPKPXX:APTKL:SPIS:YKQYE:NMVVR:SXGXH, TVPKPXX:APTQL:SPIS:YKQYE:NMVVR:AXGXH, AVPKAXX:APTKL:SPIS:YKQYE:NMVVK:AXGXH, KVGKAXX:VPTKL:SPIS:YKQYE:EGMSVAE:XGXR, KASKAXX:VPTKL:SPIS:YKQYE:GMAVS:EXGXR, GSAGPXX:TPTKM:SPIS:YKQYE:GMVVD:RXGXS, STPPTXX:VPTRL:SPIS:YKQYE:DMVVE:SXGXR, HVPKPXX:APTKL:SPIS:YKQYE:NMVVR:SXGXH, RVPSTXX:APVKT:SPIS:YKQYE:MIVEE:XGXL, ASAAPXX:VPQAL:SPIS:YKQYE:MIVRS:XKXS, ASASPXX:VSQDL:SPIS:YKQYE:MIVKS:XKXS, ASASPXX:VPQDL:SPIS:YKQYE:MVVKS:XKXS, NDEGLEX:VPTEE:SPIS:YKQYE:FLQHN:KXEXR, NDEGLEX:VPTGQ:SPIS:YKQYE:LEEHS:QXEXR, SSVKXQP:SRVHH:SPIS:YKQYE:RLEEH:LEXAXA, RNVQXRP:TQVQL:SPIS:YKQYE:TLEDH:LAXKXE, GIPEPXX:VPTKM:SPIS:YKQYE:NMVVE:SXAXR, HVTKPTX:VPTKL:SPIS:YKQYE:NMVVE:SXGXH, YVPKPXX:VPTKL:SPIS:YKQYE:NMVVE:SXGXH, TVPKPXX:VPTQL:SPIS:YKQYE:NMVVE:AXGXH, AVPKAXX:VPTKL:SPIS:YKQYE:NMVVE:AXGXH, KVGKAXX:VPTKL:SPIS:YKQYE:EGMSVVE:XGXR, KASKAXX:VPTKL:SPIS:YKQYE:GMVVE:EXGXR, GSAGPXX:VPTKM:SPIS:YKQYE:GMVVE:RXGXS, AAPASXX:VPTRL:SPIS:YKQYE:DMVVE:AXGXR, HVPKPXX:VPTKL:SPIS:YKQYE:NMVVE:SXGXH, RVPSTXX:VPTKT:SPIS:YKQYE:MIVVE:XGXL, ASAAPXX:VPTAL:SPIS:YKQYE:MIVVE:XKXS, ASASPXX:VPTDL:SPIS:YKQYE:MIVVE:XKXS, ASASPXX:VPTDL:SPIS:YKQYE:MVVVE:XKXS, AAPASXX:VPARL:SPIS:YKQYE:DMVVE:AXGXR, KIPKAXX:APVEL:KPLS:DHHKD:DMVEE:GXGXR, GIPEPXX:APVKM:KPLS:DHHKD:NMVEE:SXAXR, SIPKAXX:APVEL:KPLS:DHHKD:EMVEE:GXGXR, HVTKPTX:APVKL:KPLS:DHHKD:NMVEE:SXGXH, YVPKPXX:APVKL:KPLS:DHHKD:NMVEE:SXGXH, TVPKPXX:APVQL:KPLS:DHHKD:NMVEE:AXGXH, AVPKAXX:APVKL:KPLS:DHHKD:NMVEE:AXGXH, KVGKAXX:APVKL:KPLS:DHHKD:EGMSVEE:XGXR, KASKAXX:APVKL:KPLS:DHHKD:GMVEE:EXGXR, GSAGPXX:APVKM:KPLS:DHHKD:GMVEE:RXGXS, AAPASXX:APVRL:KPLS:DHHKD:DMVEE:AXGXR, STPPTXX:APVRL:KPLS:DHHKD:DMVEE:SXGXR, HVPKPXX:APVKL:KPLS:DHHKD:NMVEE:SXGXH, ASAAPXX:APVAL:KPLS:DHHKD:MIVEE:XKXS, ASASPXX:APVDL:KPLS:DHHKD:MIVEE:XKXS, ASASPXX:APVDL:KPLS:DHHKD:MVVEE:XKXS, KIPKAXX:VPTEL:KPLS:DHHKD:DMVVE:GXGXR, GIPEPXX:VPEKM:KPLS:DHHKD:NMTVE:SXAXR, SIPKAXX:VPTEL:KPLS:DHHKD:EMVVE:GXGXR, HVTKPTX:APTKL:KPLS:DHHKD:NMVVR:SXGXH, YVPKPXX:APTKL:KPLS:DHHKD:NMVVR:SXGXH, TVPKPXX:APTQL:KPLS:DHHKD:NMVVR:AXGXH, AVPKAXX:APTKL:KPLS:DHHKD:NMVVK:AXGXH, KVGKAXX:VPTKL:KPLS:DHHKD:EGMSVAE:XGXR, KASKAXX:VPTKL:KPLS:DHHKD:GMAVS:EXGXR, GSAGPXX:TPTKM:KPLS:DHHKD:GMVVD:RXGXS, AAPASXX:VPARL:KPLS:DHHKD:DMVVE:AXGXR, STPPTXX:VPTRL:KPLS:DHHKD:DMVVE:SXGXR, HVPKPXX:APTKL:KPLS:DHHKD:NMVVR:SXGXH, ASAAPXX:VPQAL:KPLS:DHHKD:MIVRS:XKXS, ASASPXX:VSQDL:KPLS:DHHKD:MIVKS:XKXS, ASASPXX:VPQDL:KPLS:DHHKD:MVVKS:XKXS, NDEGLEX:VPTEE:KPLS:DHHKD:FLQHN:KXEXR, NDEGLEX:VPTGQ:KPLS:DHHKD:LEEHS:QXEXR, SSVKXQP:SRVHH:KPLS:DHHKD:RLEEH:LEXAXA, RNVQXRP:TQVQL:KPLS:DHHKD:TLEDH:LAXKXE, KIPKAXX:VPQEL:EPLP:EQLSN:DMVRS:GXGXR, GIPEPXX:VPQKM:EPLP:EQLSN:NMVRS:SXAXR, SIPKAXX:VPQEL:EPLP:EQLSN:EMVRS:GXGXR, HVTKPTX:VPQKL:EPLP:EQLSN:NMVRS:SXGXH, YVPKPXX:VPQKL:EPLP:EQLSN:NMVRS:SXGXH, TVPKPXX:VPQQL:EPLP:EQLSN:NMVRS:AXGXH, AVPKAXX:VPQKL:EPLP:EQLSN:NMVRS:AXGXH, KVGKAXX:VPQKL:EPLP:EQLSN:EGMSVRS:XGXR, KASKAXX:VPQKL:EPLP:EQLSN:GMVRS:EXGXR, GSAGPXX:VPQKM:EPLP:EQLSN:GMVRS:RXGXS, AAPASXX:VPQRL:EPLP:EQLSN:DMVRS:AXGXR, STPPTXX:VPQRL:EPLP:EQLSN:DMVRS:SXGXR, HVPKPXX:VPQKL:EPLP:EQLSN:NMVRS:SXGXH, RVPSTXX:VPQKT:EPLP:EQLSN:MIVRS:XGXL, ASASPXX:VPQDL:EPLP:EQLSN:MIVRS:XKXS, ASASPXX:VPQDL:EPLP:EQLSN:MVVRS:XKXS, KIPKAXX:VPTEL:EPLP:EQLSN:DMVVE:GXGXR, GIPEPXX:VPEKM:EPLP:EQLSN:NMTVE:SXAXR, SIPKAXX:VPTEL:EPLP:EQLSN:EMVVE:GXGXR, HVTKPTX:APTKL:EPLP:EQLSN:NMVVR:SXGXH, YVPKPXX:APTKL:EPLP:EQLSN:NMVVR:SXGXH, TVPKPXX:APTQL:EPLP:EQLSN:NMVVR:AXGXH, AVPKAXX:APTKL:EPLP:EQLSN:NMVVK:AXGXH, KVGKAXX:VPTKL:EPLP:EQLSN:EGMSVAE:XGXR, KASKAXX:VPTKL:EPLP:EQLSN:GMAVS:EXGXR, GSAGPXX:TPTKM:EPLP:EQLSN:GMVVD:RXGXS, AAPASXX:VPARL:EPLP:EQLSN:DMVVE:AXGXR, STPPTXX:VPTRL:EPLP:EQLSN:DMVVE:SXGXR, HVPKPXX:APTKL:EPLP:EQLSN:NMVVR:SXGXH, RVPSTXX:APVKT:EPLP:EQLSN:MIVEE:XGXL, ASASPXX:VSQDL:EPLP:EQLSN:MIVKS:XKXS, ASASPXX:VPQDL:EPLP:EQLSN:MVVKS:XKXS, NDEGLEX:VPTEE:EPLP:EQLSN:FLQHN:KXEXR, NDEGLEX:VPTGQ:EPLP:EQLSN:LEEHS:QXEXR, SSVKXQP:SRVHH:EPLP:EQLSN:RLEEH:LEXAXA, RNVQXRP:TQVQL:EPLP:EQLSN:TLEDH:LAXKXE, KIPKAXX:VSQEL:EPLT:EQLSN:DMVKS:GXGXR, GIPEPXX:VSQKM:EPLT:EQLSN:NMVKS:SXAXR, SIPKAXX:VSQEL:EPLT:EQLSN:EMVKS:GXGXR, HVTKPTX:VSQKL:EPLT:EQLSN:NMVKS:SXGXH, YVPKPXX:VSQKL:EPLT:EQLSN:NMVKS:SXGXH, TVPKPXX:VSQQL:EPLT:EQLSN:NMVKS:AXGXH, AVPKAXX:VSQKL:EPLT:EQLSN:NMVKS:AXGXH, KVGKAXX:VSQKL:EPLT:EQLSN:EGMSVKS:XGXR, KASKAXX:VSQKL:EPLT:EQLSN:GMVKS:EXGXR, GSAGPXX:VSQKM:EPLT:EQLSN:GMVKS:RXGXS, AAPASXX:VSQRL:EPLT:EQLSN:DMVKS:AXGXR, STPPTXX:VSQRL:EPLT:EQLSN:DMVKS:SXGXR, HVPKPXX:VSQKL:EPLT:EQLSN:NMVKS:SXGXH, RVPSTXX:VSQKT:EPLT:EQLSN:MIVKS:XGXL, ASAAPXX:VSQAL:EPLT:EQLSN:MIVKS:XKXS, ASASPXX:VSQDL:EPLT:EQLSN:MVVKS:XKXS, KIPKAXX:VPTEL:EPLT:EQLSN:DMVVE:GXGXR, GIPEPXX:VPEKM:EPLT:EQLSN:NMTVE:SXAXR, SIPKAXX:VPTEL:EPLT:EQLSN:EMVVE:GXGXR, HVTKPTX:APTKL:EPLT:EQLSN:NMVVR:SXGXH, YVPKPXX:APTKL:EPLT:EQLSN:NMVVR:SXGXH, TVPKPXX:APTQL:EPLT:EQLSN:NMVVR:AXGXH, AVPKAXX:APTKL:EPLT:EQLSN:NMVVK:AXGXH, KVGKAXX:VPTKL:EPLT:EQLSN:EGMSVAE:XGXR, KASKAXX:VPTKL:EPLT:EQLSN:GMAVS:EXGXR, GSAGPXX:TPTKM:EPLT:EQLSN:GMVVD:RXGXS, AAPASXX:VPARL:EPLT:EQLSN:DMVVE:AXGXR, STPPTXX:VPTRL:EPLT:EQLSN:DMVVE:SXGXR, HVPKPXX:APTKL:EPLT:EQLSN:NMVVR:SXGXH, RVPSTXX:APVKT:EPLT:EQLSN:MIVEE:XGXL, ASAAPXX:VPQAL:EPLT:EQLSN:MIVRS:XKXS, NDEGLEX:VPTEE:EPLT:EQLSN:FLQHN:KXEXR, NDEGLEX:VPTGQ:EPLT:EQLSN:LEEHS:QXEXR, SSVKXQP:SRVHH:EPLT:EQLSN:RLEEH:LEXAXA, RNVQXRP:TQVQL:EPLT:EQLSN:TLEDH:LAXKXE, KIPKAXX:VPQEL:EPLT:EQLSN:DMVKS:GXGXR, GIPEPXX:VPQKM:EPLT:EQLSN:NMVKS:SXAXR, SIPKAXX:VPQEL:EPLT:EQLSN:EMVKS:GXGXR, HVTKPTX:VPQKL:EPLT:EQLSN:NMVKS:SXGXH, YVPKPXX:VPQKL:EPLT:EQLSN:NMVKS:SXGXH, TVPKPXX:VPQQL:EPLT:EQLSN:NMVKS:AXGXH, AVPKAXX:VPQKL:EPLT:EQLSN:NMVKS:AXGXH, KVGKAXX:VPQKL:EPLT:EQLSN:EGMSVKS:XGXR, KASKAXX:VPQKL:EPLT:EQLSN:GMVKS:EXGXR, GSAGPXX:VPQKM:EPLT:EQLSN:GMVKS:RXGXS, AAPASXX:VPQRL:EPLT:EQLSN:DMVKS:AXGXR, STPPTXX:VPQRL:EPLT:EQLSN:DMVKS:SXGXR, HVPKPXX:VPQKL:EPLT:EQLSN:NMVKS:SXGXH, RVPSTXX:VPQKT:EPLT:EQLSN:MIVKS:XGXL, ASAAPXX:VPQAL:EPLT:EQLSN:MIVKS:XKXS, ASASPXX:VPQDL:EPLT:EQLSN:MIVKS:XKXS, NDEGLEX:VPTGQ:SNIT:IGEMS:LEQHN:QXEXR, KIPKAXX:VPTEL:SNIT:IGEMS:DMVVE:GXGXR, GIPEPXX:VPEKM:SNIT:IGEMS:NMTVE:SXAXR, SIPKAXX:VPTEL:SNIT:IGEMS:EMVVE:GXGXR, HVTKPTX:APTKL:SNIT:IGEMS:NMVVR:SXGXH, YVPKPXX:APTKL:SNIT:IGEMS:NMVVR:SXGXH, TVPKPXX:APTQL:SNIT:IGEMS:NMVVR:AXGXH, AVPKAXX:APTKL:SNIT:IGEMS:NMVVK:AXGXH, KVGKAXX:VPTKL:SNIT:IGEMS:EGMSVAE:XGXR, KASKAXX:VPTKL:SNIT:IGEMS:GMAVS:EXGXR, GSAGPXX:TPTKM:SNIT:IGEMS:GMVVD:RXGXS, AAPASXX:VPARL:SNIT:IGEMS:DMVVE:AXGXR, STPPTXX:VPTRL:SNIT:IGEMS:DMVVE:SXGXR, HVPKPXX:APTKL:SNIT:IGEMS:NMVVR:SXGXH, RVPSTXX:APVKT:SNIT:IGEMS:MIVEE:XGXL, ASAAPXX:VPQAL:SNIT:IGEMS:MIVRS:XKXS, ASASPXX:VSQDL:SNIT:IGEMS:MIVKS:XKXS, ASASPXX:VPQDL:SNIT:IGEMS:MVVKS:XKXS, NDEGLEX:VPTGQ:SNIT:IGEMS:LEEHS:QXEXR, SSVKXQP:SRVHH:SNIT:IGEMS:RLEEH:LEXAXA, RNVQXRP:TQVQL:SNIT:IGEMS:TLEDH:LAXKXE, NDEGLEX:VPTEE:SNIT:LGEMS:FLEHS:KXEXR, KIPKAXX:VPTEL:SNIT:LGEMS:DMVVE:GXGXR, GIPEPXX:VPEKM:SNIT:LGEMS:NMTVE:SXAXR, SIPKAXX:VPTEL:SNIT:LGEMS:EMVVE:GXGXR, HVTKPTX:APTKL:SNIT:LGEMS:NMVVR:SXGXH, YVPKPXX:APTKL:SNIT:LGEMS:NMVVR:SXGXH, TVPKPXX:APTQL:SNIT:LGEMS:NMVVR:AXGXH, AVPKAXX:APTKL:SNIT:LGEMS:NMVVK:AXGXH, KVGKAXX:VPTKL:SNIT:LGEMS:EGMSVAE:XGXR, KASKAXX:VPTKL:SNIT:LGEMS:GMAVS:EXGXR, GSAGPXX:TPTKM:SNIT:LGEMS:GMVVD:RXGXS, AAPASXX:VPARL:SNIT:LGEMS:DMVVE:AXGXR, STPPTXX:VPTRL:SNIT:LGEMS:DMVVE:SXGXR, HVPKPXX:APTKL:SNIT:LGEMS:NMVVR:SXGXH, RVPSTXX:APVKT:SNIT:LGEMS:MIVEE:XGXL, ASAAPXX:VPQAL:SNIT:LGEMS:MIVRS:XKXS, ASASPXX:VSQDL:SNIT:LGEMS:MIVKS:XKXS, ASASPXX:VPQDL:SNIT:LGEMS:MVVKS:XKXS, NDEGLEX:VPTEE:SNIT:LGEMS:FLQHN:KXEXR, SSVKXQP:SRVHH:SNIT:LGEMS:RLEEH:LEXAXA, RNVQXRP:TQVQL:SNIT:LGEMS:TLEDH:LAXKXE, RNVQXRP:SRVQL:RSVK:KEVQV:TLEEH:LAXKXE, KIPKAXX:VPTEL:RSVK:KEVQV:DMVVE:GXGXR, GIPEPXX:VPEKM:RSVK:KEVQV:NMTVE:SXAXR, SIPKAXX:VPTEL:RSVK:KEVQV:EMVVE:GXGXR, HVTKPTX:APTKL:RSVK:KEVQV:NMVVR:SXGXH, YVPKPXX:APTKL:RSVK:KEVQV:NMVVR:SXGXH, TVPKPXX:APTQL:RSVK:KEVQV:NMVVR:AXGXH, AVPKAXX:APTKL:RSVK:KEVQV:NMVVK:AXGXH, KVGKAXX:VPTKL:RSVK:KEVQV:EGMSVAE:XGXR, KASKAXX:VPTKL:RSVK:KEVQV:GMAVS:EXGXR, GSAGPXX:TPTKM:RSVK:KEVQV:GMVVD:RXGXS, AAPASXX:VPARL:RSVK:KEVQV:DMVVE:AXGXR, STPPTXX:VPTRL:RSVK:KEVQV:DMVVE:SXGXR, HVPKPXX:APTKL:RSVK:KEVQV:NMVVR:SXGXH, RVPSTXX:APVKT:RSVK:KEVQV:MIVEE:XGXL, ASAAPXX:VPQAL:RSVK:KEVQV:MIVRS:XKXS, ASASPXX:VSQDL:RSVK:KEVQV:MIVKS:XKXS, ASASPXX:VPQDL:RSVK:KEVQV:MVVKS:XKXS, NDEGLEX:VPTEE:RSVK:KEVQV:FLQHN:KXEXR, NDEGLEX:VPTGQ:RSVK:KEVQV:LEEHS:QXEXR, RNVQXRP:TQVQL:RSVK:KEVQV:TLEDH:LAXKXE, SSVKXQP:TQVHH:RPVQ:KKATV:RLEDH:LEXAXA, KIPKAXX:VPTEL:RPVQ:KKATV:DMVVE:GXGXR, GIPEPXX:VPEKM:RPVQ:KKATV:NMTVE:SXAXR, SIPKAXX:VPTEL:RPVQ:KKATV:EMVVE:GXGXR, HVTKPTX:APTKL:RPVQ:KKATV:NMVVR:SXGXH, YVPKPXX:APTKL:RPVQ:KKATV:NMVVR:SXGXH, TVPKPXX:APTQL:RPVQ:KKATV:NMVVR:AXGXH, AVPKAXX:APTKL:RPVQ:KKATV:NMVVK:AXGXH, KVGKAXX:VPTKL:RPVQ:KKATV:EGMSVAE:XGXR, KASKAXX:VPTKL:RPVQ:KKATV:GMAVS:EXGXR, GSAGPXX:TPTKM:RPVQ:KKATV:GMVVD:RXGXS, AAPASXX:VPARL:RPVQ:KKATV:DMVVE:AXGXR, STPPTXX:VPTRL:RPVQ:KKATV:DMVVE:SXGXR, HVPKPXX:APTKL:RPVQ:KKATV:NMVVR:SXGXH, RVPSTXX:APVKT:RPVQ:KKATV:MIVEE:XGXL, ASAAPXX:VPQAL:RPVQ:KKATV:MIVRS:XKXS, ASASPXX:VSQDL:RPVQ:KKATV:MIVKS:XKXS, ASASPXX:VPQDL:RPVQ:KKATV:MVVKS:XKXS, NDEGLEX:VPTEE:RPVQ:KKATV:FLQHN:KXEXR, NDEGLEX:VPTGQ:RPVQ:KKATV:LEEHS:QXEXR and SSVKXQP:SRVHH:RPVQ:KKATV:RLEEH:LEXAXA.

In particular, in certain embodiments, the hexaplet PEP7:PEP5:PEP12:PEP2:PEP6:PEP8 is selected from the group consisting of GIPEPXX:VPTKM:SAIS-AA17-LYL:LKNYQ:NMVVE:SXAXR, SIPKAXX:VPTEL:SAIS-AA17-LYL:LKNYQ:EMVVE:GXGXR, HVTKPTX:VPTKL:SAIS-AA17-LYL:LKNYQ:NMVVE:SXGXH, YVPKPXX:VPTKL:SAIS-AA17-LYL:LKNYQ:NMVVE:SXGXH, TVPKPXX:VPTQL:SAIS-AA17-LYL:LKNYQ:NMVVE:AXGXH, AVPKAXX:VPTKL:SAIS-AA17-LYL:LKNYQ:NMVVE:AXGXH, KVGKAXX:VPTKL:SAIS-AA17-LYL:LKNYQ:EGMSVVE:XGXR, KASKAXX:VPTKL:SAIS-AA17-LYL:LKNYQ:GMVVE:EXGXR, GSAGPXX:VPTKM:SAIS-AA17-LYL:LKNYQ:GMVVE:RXGXS, AAPASXX:VPTRL:SAIS-AA17-LYL:LKNYQ:DMVVE:AXGXR, STPPTXX:VPTRL:SAIS-AA17-LYL:LKNYQ:DMVVE:SXGXR, HVPKPXX:VPTKL:SAIS-AA17-LYL:LKNYQ:NMVVE:SXGXH, RVPSTXX:VPTKT:SAIS-AA17-LYL:LKNYQ:MIVVE:XGXL, ASAAPXX:VPTAL:SAIS-AA17-LYL:LKNYQ:MIVVE:XKXS, ASASPXX:VPTDL:SAIS-AA17-LYL:LKNYQ:MIVVE:XKXS, ASASPXX:VPTDL:SAIS-AA17-LYL:LKNYQ:MVVVE:XKXS, NDEGLEX:VPTEE:SAIS-AA17-LYL:LKNYQ:FLVVE:KXEXR, NDEGLEX:VPTGQ:SAIS-AA17-LYL:LKNYQ:LEVVE:QXEXR, SSVKXQP:VPTHH:SAIS-AA17-LYL:LKNYQ:RLVVE:LEXAXA, RNVQXRP:VPTQL:SAIS-AA17-LYL:LKNYQ:TLVVE:LAXKXE, GIPEPXX:VPEKM:SAIS-AA17-LYL:LKNYQ:NMTVE:SXAXR, HVTKPTX:APTKL:SAIS-AA17-LYL:LKNYQ:NMVVR:SXGXH, YVPKPXX:APTKL:SAIS-AA17-LYL:LKNYQ:NMVVR:SXGXH, TVPKPXX:APTQL:SAIS-AA17-LYL:LKNYQ:NMVVR:AXGXH, AVPKAXX:APTKL:SAIS-AA17-LYL:LKNYQ:NMVVK:AXGXH, KVGKAXX:VPTKL:SAIS-AA17-LYL:LKNYQ:EGMSVAE:XGXR, KASKAXX:VPTKL:SAIS-AA17-LYL:LKNYQ:GMAVS:EXGXR, GSAGPXX:TPTKM:SAIS-AA17-LYL:LKNYQ:GMVVD:RXGXS, AAPASXX:VPARL:SAIS-AA17-LYL:LKNYQ:DMVVE:AXGXR, HVPKPXX:APTKL:SAIS-AA17-LYL:LKNYQ:NMVVR:SXGXH, RVPSTXX:APVKT:SAIS-AA17-LYL:LKNYQ:MIVEE:XGXL, ASAAPXX:VPQAL:SAIS-AA17-LYL:LKNYQ:MIVRS:XKXS, ASASPXX:VSQDL:SAIS-AA17-LYL:LKNYQ:MIVKS:XKXS, ASASPXX:VPQDL:SAIS-AA17-LYL:LKNYQ:MVVKS:XKXS, NDEGLEX:VPTEE:SAIS-AA17-LYL:LKNYQ:FLQHN:KXEXR, NDEGLEX:VPTGQ:SAIS-AA17-LYL:LKNYQ:LEEHS:QXEXR, SSVKXQP:SRVHH:SAIS-AA17-LYL:LKNYQ:RLEEH:LEXAXA, RNVQXRP:TQVQL:SAIS-AA17-LYL:LKNYQ:TLEDH:LAXKXE, KIPKAXX:VPEEL:SSLS-AA17-LFF:LKVYP:DMTVE:GXGXR, SIPKAXX:VPEEL:SSLS-AA17-LFF:LKVYP:EMTVE:GXGXR, HVTKPTX:VPEKL:SSLS-AA17-LFF:LKVYP:NMTVE:SXGXH, YVPKPXX:VPEKL:SSLS-AA17-LFF:LKVYP:NMTVE:SXGXH, TVPKPXX:VPEQL:SSLS-AA17-LFF:LKVYP:NMTVE:AXGXH, AVPKAXX:VPEKL:SSLS-AA17-LFF:LKVYP:NMTVE:AXGXH, KVGKAXX:VPEKL:SSLS-AA17-LFF:LKVYP:EGMSTVE:XGXR, KASKAXX:VPEKL:SSLS-AA17-LFF:LKVYP:GMTVE:EXGXR, GSAGPXX:VPEKM:SSLS-AA17-LFF:LKVYP:GMTVE:RXGXS, AAPASXX:VPERL:SSLS-AA17-LFF:LKVYP:DMTVE:AXGXR, STPPTXX:VPERL:SSLS-AA17-LFF:LKVYP:DMTVE:SXGXR, HVPKPXX:VPEKL:SSLS-AA17-LFF:LKVYP:NMTVE:SXGXH, RVPSTXX:VPEKT:SSLS-AA17-LFF:LKVYP:MITVE:XGXL, ASAAPXXVPEAL:SSLS-AA17-LFF:LKVYP:MITVE:XKXS, ASASPXX:VPEDL:SSLS-AA17-LFF:LKVYP:MITVE:XKXS, ASASPXX:VPEDL:SSLS-AA17-LFF:LKVYP:MVTVE:XKXS, NDEGLEX:VPEEE:SSLS-AA17-LFF:LKVYP:FLTVE:KXEXR, NDEGLEX:VPEGQ:SSLS-AA17-LFF:LKVYP:LETVE:QXEXR, SSVKXQP:VPEHH:SSLS-AA17-LFF:LKVYP:RLTVE:LEXAXA, RNVQXRP:VPEQL:SSLS-AA17-LFF:LKVYP:TLTVE:LAXKXE, KIPKAXX:VPTEL:SSLS-AA17-LFF:LKVYP:DMVVE:GXGXR, SIPKAXX:VPTEL:SSLS-AA17-LFF:LKVYP:EMVVE:GXGXR, HVTKPTX:APTKL:SSLS-AA17-LFF:LKVYP:NMVVR:SXGXH, YVPKPXX:APTKL:SSLS-AA17-LFF:LKVYP:NMVVR:SXGXH, TVPKPXX:APTQL:SSLS-AA17-LFF:LKVYP:NMVVR:AXGXH, AVPKAXX:APTKL:SSLS-AA17-LFF:LKVYP:NMVVK:AXGXH, KVGKAXX:VPTKL:SSLS-AA17-LFF:LKVYP:EGMSVAE:XGXR, KASKAXX:VPTKL:SSLS-AA17-LFF:LKVYP:GMAVS:EXGXR, GSAGPXX:TPTKM:SSLS-AA17-LFF:LKVYP:GMVVD:RXGXS, AAPASXX:VPARL:SSLS-AA17-LFF:LKVYP:DMVVE:AXGXR, STPPTXX:VPTRL:SSLS-AA17-LFF:LKVYP:DMVVE:SXGXR, HVPKPXX:APTKL:SSLS-AA17-LFF:LKVYP:NMVVR:SXGXH, RVPSTXX:APVKT:SSLS-AA17-LFF:LKVYP:MIVEE:XGXL, ASAAPXX:VPQAL:SSLS-AA17-LFF:LKVYP:MIVRS:XKXS, ASASPXX:VSQDL:SSLS-AA17-LFF:LKVYP:MIVKS:XKXS, ASASPXX:VPQDL:SSLS-AA17-LFF:LKVYP:MVVKS:XKXS, NDEGLEX:VPTEE:SSLS-AA17-LFF:LKVYP:FLQHN:KXEXR, NDEGLEX:VPTGQ:SSLS-AA17-LFF:LKVYP:LEEHS:QXEXR, SSVKXQP:SRVHH:SSLS-AA17-LFF:LKVYP:RLEEH:LEXAXA, RNVQXRP:TQVQL:SSLS-AA17-LFF:LKVYP:TLEDH:LAXKXE, KIPKAXX:VPTEL:SAIS-AA17-LYL:LKNYQ:DMVVE:GXGXR, KIPKAXX:APTEL:NAIS-AA17-LYF:LKKYR:DMVVR:GXGXR,

GIPEPXX:APTKM:NAIS-AA$^{17}$-LYF:LKKYR:NMVVR:SXAXR, SIPKAXX:APTEL:NAIS-AA$^{17}$-LYF:LKKYR:EMVVR:GXGXR, YVPKPXX:APTKL:NAIS-AA$^{17}$-LYF:LKKYR:NMVVR:SXGXH, TVPKPXX:APTQL:NAIS-AA$^{17}$-LYF:LKKYR:NMVVR:AXGXH, AVPKAXX:APTKL:NAIS-AA$^{17}$-LYF:LKKYR:NMVVR:AXGXH, KVGKAXX:APTKL:NAIS-AA$^{17}$-LYF:LKKYR:EGMSVVR:XGXR, KASKAXX:APTKL:NAIS-AA$^{17}$-LYF:LKKYR:GMVVR:EXGXR, GSAGPXX:APTKM:NAIS-AA$^{17}$-LYF:LKKYR:GMVVR:RXGXS, AAPASXX:APTRL:NAIS-AA$^{17}$-LYF:LKKYR:DMVVR:AXGXR, STPPTXX:APTRL:NAIS-AA$^{17}$-LYF:LKKYR:DMVVR:SXGXR, HVPKPXX:APTKL:NAIS-AA$^{17}$-LYF:LKKYR:NMVVR:SXGXH, RVPSTXX:APTKT:NAIS-AA$^{17}$-LYF:LKKYR:MIVVR:XGXL, ASAAPXX:APTAL:NAIS-AA$^{17}$-LYF:LKKYR:MIVVR:XKXS, ASASPXX:APTDL:NAIS-AA$^{17}$-LYF:LKKYR:MIVVR:XKXS, ASASPXX:APTDL:NAIS-AA$^{17}$-LYF:LKKYR:MVVVR:XKXS, NDEGLEX:APTEE:NAIS-AA$^{17}$-LYF:LKKYR:FLVVR:KXEXR, NDEGLEX:APTGQ:NAIS-AA$^{17}$-LYF:LKKYR:LEVVR:QXEXR, SSVKXQP:APTHH:NAIS-AA$^{17}$-LYF:LKKYR:RLVVR:LEXAXA, RNVQXRP:APTQL:NAIS-AA$^{17}$-LYF:LKKYR:TLVVR:LAXKXE, KIPKAXX:VPTEL:NAIS-AA$^{17}$-LYF:LKKYR:DMVVE:GXGXR, GIPEPXX:VPEKM:NAIS-AA$^{17}$-LYF:LKKYR:NMTVE:SXAXR, SIPKAXX:VPTEL:NAIS-AA$^{17}$-LYF:LKKYR:EMVVE:GXGXR, AVPKAXX:APTKL:NAIS-AA$^{17}$-LYF:LKKYR:NMVVK:AXGXH, KVGKAXX:VPTKL:NAIS-AA$^{17}$-LYF:LKKYR:EGMSVAE:XGXR, KASKAXX:VPTKL:NAIS-AA$^{17}$-LYF:LKKYR:GMAVS:EXGXR, GSAGPXX:TPTKM:NAIS-AA$^{17}$-LYF:LKKYR:GMVVD:RXGXS, AAPASXX:VPARL:NAIS-AA$^{17}$-LYF:LKKYR:DMVVE:AXGXR, STPPTXX:VPTRL:NAIS-AA$^{17}$-LYF:LKKYR:DMVVE:SXGXR, RVPSTXX:APVKT:NAIS-AA$^{17}$-LYF:LKKYR:MIVEE:XGXL, ASAAPXX:VPQAL:NAIS-AA$^{17}$-LYF:LKKYR:MIVRS:XKXS, ASASPXX:VSQDL:NAIS-AA$^{17}$-LYF:LKKYR:MIVKS:XKXS, ASASPXX:VPQDL:NAIS-AA$^{17}$-LYF:LKKYR:MVVKS:XKXS, NDEGLEX:VPTEE:NAIS-AA$^{17}$-LYF:LKKYR:FLQHN:KXEXR, NDEGLEX:VPTGQ:NAIS-AA$^{17}$-LYF:LKKYR:LEEHS:QXEXR, SSVKXQP:SRVHH:NAIS-AA$^{17}$-LYF:LKKYR:RLEEH:LEXAXA, RNVQXRP:TQVQL:NAIS-AA$^{17}$-LYF:LKKYR:TLEDH:LAXKXE, HVTKPTX:APTKL:NAIS-AA$^{17}$-LYF:LKKYR:NMVVR:SXGXH, KIPKAXX:APTEL:SATS-AA$^{17}$-LYY:LRKHR:DMVVK:GXGXR, GIPEPXX:APTKM:SATS-AA$^{17}$-LYY:LRKHR:NMVVK:SXAXR, SIPKAXX:APTEL:SATS-AA$^{17}$-LYY:LRKHR:EMVVK:GXGXR, HVTKPTX:APTKL:SATS-AA$^{17}$-LYY:LRKHR:NMVVK:SXGXH, YVPKPXX:APTKL:SATS-AA$^{17}$-LYY:LRKHR:NMVVK:SXGXH, TVPKPXX:APTQL:SATS-AA$^{17}$-LYY:LRKHR:NMVVK:AXGXH, KVGKAXX:APTKL:SATS-AA$^{17}$-LYY:LRKHR:EGMSVVK:XGXR, KASKAXX:APTKL:SATS-AA$^{17}$-LYY:LRKHR:GMVVK:EXGXR, GSAGPXX:APTKM:SATS-AA$^{17}$-LYY:LRKHR:GMVVK:RXGXS, AAPASXX:APTRL:SATS-AA$^{17}$-LYY:LRKHR:DMVVK:AXGXR, STPPTXX:APTRL:SATS-AA$^{17}$-LYY:LRKHR:DMVVK:SXGXR, HVPKPXX:APTKL:SATS-AA$^{17}$-LYY:LRKHR:NMVVK:SXGXH, RVPSTXX:APTKT:SATS-AA$^{17}$-LYY:LRKH R:MIVVK:XGXL, ASAAPXX:APTAL:SATS-AA$^{17}$-LYY:LRKHR:MIVVK:XKXS, ASASPXX:APTDL:SATS-AA$^{17}$-LYY:LRKHR:MIVVK:XKXS, ASASPXX:APTDL:SATS-AA$^{17}$-LYY:LRKHR:MVVVK:XKXS, NDEGLEX:APTEE:SATS-AA$^{17}$-LYY:LRKHR:FLVVK:KXEXR, NDEGLEX:APTGQ:SATS-AA$^{17}$-LYY:LRKHR:LEVVK:QXEXR, SSVKXQP:APTHH:SATS-AA$^{17}$-LYY:LRKHR:RLVVK:LEXAXA, RNVQXRP:APTQL:SATS-AA$^{17}$-LYY:LRKHR:TLVVK:LAXKXE, KIPKAXX:VPTEL:SATS-AA$^{17}$-LYY:LRKHR:DMVVE:GXGXR, GIPEPXX:VPEKM:SATS-AA$^{17}$-LYY:LRKHR:NMTVE:SXAXR, SIPKAXX:VPTEL:SATS-AA$^{17}$-LYY:LRKHR:EMVVE:GXGXR, HVTKPTX:APTKL:SATS-AA$^{17}$-LYY:LRKHR:NMVVR:SXGXH, YVPKPXX:APTKL:SATS-AA$^{17}$-LYY:LRKHR:NMVVR:SXGXH, TVPKPXX:APTQL:SATS-AA$^{17}$-LYY:LRKH R:NMVVR:AXGXH, KVGKAXX:VPTKL:SATS-AA$^{17}$-LYY:LRKHR:EGMSVAE:XGXR, KASKAXX:VPTKL:SATS-AA$^{17}$-LYY:LRKHR:GMAVS:EXGXR, GSAGPXX:TPTKM:SATS-AA$^{17}$-LYY:LRKHR:GMVVD:RXGXS, AAPASXX:VPARL:SATS-AA$^{17}$-LYY:LRKHR:DMVVE:AXGXR, STPPTXX:VPTRL:SATS-AA$^{17}$-LYY:LRKHR:DMVVE:SXGXR, HVPKPXX:APTKL:SATS-AA$^{17}$-LYY:LRKH R:NMVVR:SXGXH, RVPSTXX:APVKT:SATS-AA$^{17}$-LYY:LRKHR:MIVEE:XGXL, ASAAPXX:VPQAL:SATS-AA$^{17}$-LYY:LRKHR:MIVRS:XKXS, ASASPXX:VSQDL:SATS-AA$^{17}$-LYY:LRKHR:MIVKS:XKXS, ASASPXX:VPQDL:SATS-AA$^{17}$-LYY:LRKHR:MVVKS:XKXS, NDEGLEX:VPTEE:SATS-AA$^{17}$-LYY:LRKHR:FLQHN:KXEXR, NDEGLEX:VPTGQ:SATS-AA$^{17}$-LYY:LRKHR:LEEHS:QXEXR, SSVKXQP:SRVHH:SATS-AA$^{17}$-LYY:LRKHR:RLEEH:LEXAXA, RNVQXRP:TQVQL:SATS-AA$^{17}$-LYY:LRKHR:TLEDH:LAXKXE, KIPKAXX:VPTEL:SPIS-AA$^{17}$-LYK:LKYHY:DMVAE:GXGXR, GIPEPXX:VPTKM:SPIS-AA$^{17}$-LYK:LKYHY:NMVAE:SXAXR, SIPKAXX:VPTEL:SPIS-AA$^{17}$-LYK:LKYHY:EMVAE:GXGXR, HVTKPTX:VPTKL:SPIS-AA$^{17}$-LYK:LKYHY:NMVAE:SXGXH, YVPKPXX:VPTKL:SPIS-AA$^{17}$-LYK:LKYHY:NMVAE:SXGXH, TVPKPXX:VPTQL:SPIS-AA$^{17}$-LYK:LKYHY:NMVAE:AXGXH, AVPKAXX:VPTKL:SPIS-AA$^{17}$-LYK:LKYHY:NMVAE:AXGXH, KASKAXX:VPTKL:SPIS-AA$^{17}$-LYK:LKYHY:GMVAE:EXGXR, GSAGPXX:VPTKM:SPIS-AA$^{17}$-LYK:LKYHY:GMVAE:RXGXS, AAPASXX:VPTRL:SPIS-AA$^{17}$-LYK:LKYHY:DMVAE:AXGXR, STPPTXX:VPTRL:SPIS-AA$^{17}$-LYK:LKYHY:DMVAE:SXGXR, HVPKPXX:VPTKL:SPIS-AA$^{17}$-LYK:LKYHY:NMVAE:SXGXH, RVPSTXX:VPTKT:SPIS-AA$^{17}$-LYK:LKYHY:MIVAE:XGXL, ASAAPXXVPTAL:SPIS-AA$^{17}$-LYK:LKYHY:MIVAE:XKXS, ASASPXX:VPTDL:SPIS-AA$^{17}$-LYK:LKYHY:MIVAE:XKXS, ASASPXX:VPTDL:SPIS-AA$^{17}$-LYK:LKYHY:MVVAE:XKXS, NDEGLEX:VPTEE:SPIS-AA$^{17}$-LYK:LKYHY:FLVAE:KXEXR, NDEGLEX:VPTGQ:SPIS-AA$^{17}$-LYK:LKYHY:LEVAE:QXEXR, SSVKXQP:VPTHH:SPIS-AA$^{17}$-LYK:LKYHY:RLVAE:LEXAXA, RNVQXRP:VPTQL:SPIS-AA$^{17}$-LYK:LKYHY:TLVAE:LAXKXE, KIPKAXX:VPTEL:SPIS-AA$^{17}$-LYK:LKYHY:DMVVE:GXGXR, GIPEPXX:VPEKM:SPIS-AA$^{17}$-LYK:LKYHY:NMTVE:SXAXR, SIPKAXX:VPTEL:SPIS-AA$^{17}$-LYK:LKYHY:EMVVE:GXGXR, HVTKPTX:APTKL:SPIS-AA$^{17}$-LYK:LKYHY:NMVVR:SXGXH, YVPKPXX:APTKL:SPIS-

AA$^{17}$-LYK:LKYHY:NMVVR:SXGXH, TVPKPXX:APTQL:SPIS-AA$^{17}$-LYK:LKYHY:NMVVR:AXGXH, AVPKAXX:APTKL:SPIS-AA$^{17}$-LYK:LKYHY:NMVVK:AXGXH, KASKAXX:VPTKL:SPIS-AA$^{17}$-LYK:LKYHY:GMAVS:EXGXR, GSAGPXX:TPTKM:SPIS-AA$^{17}$-LYK:LKYHY:GMVVD:RXGXS, AAPASXX:VPARL:SPIS-AA$^{17}$-LYK:LKYHY:DMVVE:AXGXR, STPPTXX:VPTRL:SPIS-AA$^{17}$-LYK:LKYHY:DMVVE:SXGXR, HVPKPXX:APTKL:SPIS-AA$^{17}$-LYK:LKYHY:NMVVR:SXGXH, RVPSTXX:APVKT:SPIS-AA$^{17}$-LYK:LKYHY:MIVEE:XGXL, ASAAPXX:VPQAL:SPIS-AA$^{17}$-LYK:LKYHY:MIVRS:XKXS, ASASPXX:VSQDL:SPIS-AA$^{17}$-LYK:LKYHY:MIVKS:XKXS, ASASPXX:VPQDL:SPIS-AA$^{17}$-LYK:LKYHY:MVVKS:XKXS, NDEGLEX:VPTEE:SPIS-AA$^{17}$-LYK:LKYHY:FLQHN:KXEXR, NDEGLEX:VPTGQ:SPIS-AA$^{17}$-LYK:LKYHY:LEEHS:QXEXR, SSVKXQP:SRVHH:SPIS-AA$^{17}$-LYK:LKYHY:RLEEH:LEXAXA, RNVQXRP:TQVQL:SPIS-AA$^{17}$-LYK:LKYHY:TLEDH:LAXKXE, KIPKAXX:VPTEL:EPIS-AA$^{17}$-LYL:KFKYE:DMAVS:GXGXR, GIPEPXX:VPTKM:EPIS-AA$^{17}$-LYL:KFKYE:NMAVS:SXAXR, SIPKAXX:VPTEL:EPIS-AA$^{17}$-LYL:KFKYE:EMAVS:GXGXR, HVTKPTX:VPTKL:EPIS-AA$^{17}$-LYL:KFKYE:NMAVS:SXGXH, YVPKPXX:VPTKL:EPIS-AA$^{17}$-LYL:KFKYE:NMAVS:SXGXH, TVPKPXX:VPTQL:EPIS-AA$^{17}$-LYL:KFKYE:NMAVS:AXGXH, AVPKAXX:VPTKL:EPIS-AA$^{17}$-LYL:KFKYE:NMAVS:AXGXH, KVGKAXX:VPTKL:EPIS-AA$^{17}$-LYL:KFKYE:EGMSAVS:XGXR, GSAGPXX:VPTKM:EPIS-AA$^{17}$-LYL:KFKYE:GMAVS:RXGXS, AAPASXX:VPTRL:EPIS-AA$^{17}$-LYL:KFKYE:DMAVS:AXGXR, STPPTXX:VPTRL:EPIS-AA$^{17}$-LYL:KFKYE:DMAVS:SXGXR, HVPKPXX:VPTKL:EPIS-AA$^{17}$-LYL:KFKYE:NMAVS:SXGXH, RVPSTXX:VPTKT:EPIS-AA$^{17}$-LYL:KFKYE:MIAVS:XGXL, ASAAPXX:VPTAL:EPIS-AA$^{17}$-LYL:KFKYE:MIAVS:XKXS, ASASPXX:VPTDL:EPIS-AA$^{17}$-LYL:KFKYE:MIAVS:XKXS, ASASPXX:VPTDL:EPIS-AA$^{17}$-LYL:KFKYE:MVAVS:XKXS, NDEGLEX:VPTEE:EPIS-AA$^{17}$-LYL:KFKYE:FLAVS:KXEXR, NDEGLEX:VPTGQ:EPIS-AA$^{17}$-LYL:KFKYE:LEAVS:QXEXR, SSVKXQP:VPTHH:EPIS-AA$^{17}$-LYL:KFKYE:RLAVS:LEXAXA, RNVQXRP:VPTQL:EPIS-AA$^{17}$-LYL:KFKYE:TLAVS:LAXKXE, KIPKAXX:VPTEL:EPIS-AA$^{17}$-LYL:KFKYE:DMVVE:GXGXR, GIPEPXX:VPEKM:EPIS-AA$^{17}$-LYL:KFKYE:NMTVE:SXAXR, SIPKAXX:VPTEL:EPIS-AA$^{17}$-LYL:KFKYE:EMVVE:GXGXR, HVTKPTX:APTKL:EPIS-AA$^{17}$-LYL:KFKYE:NMVVR:SXGXH, YVPKPXX:APTKL:EPIS-AA$^{17}$-LYL:KFKYE:NMVVR:SXGXH, TVPKPXX:APTQL:EPIS-AA$^{17}$-LYL:KFKYE:NMVVR:AXGXH, AVPKAXX:APTKL:EPIS-AA$^{17}$-LYL:KFKYE:NMVVK:AXGXH, KVGKAXX:VPTKL:EPIS-AA$^{17}$-LYL:KFKYE:EGMSVAE:XGXR, GSAGPXX:TPTKM:EPIS-AA$^{17}$-LYL:KFKYE:GMVVD:RXGXS, AAPASXX:VPARL:EPIS-AA$^{17}$-LYL:KFKYE:DMVVE:AXGXR, STPPTXX:VPTRL:EPIS-AA$^{17}$-LYL:KFKYE:DMVVE:SXGXR, HVPKPXX:APTKL:EPIS-AA$^{17}$-LYL:KFKYE:NMVVR:SXGXH, RVPSTXX:APVKT:EPIS-AA$^{17}$-LYL:KFKYE:MIVEE:XGXL, ASAAPXX:VPQAL:EPIS-AA$^{17}$-LYL:KFKYE:MIVRS:XKXS, ASASPXX:VSQDL:EPIS-AA$^{17}$-LYL:KFKYE:MIVKS:XKXS, ASASPXX:VPQDL:EPIS-AA$^{17}$-LYL:KFKYE:MVVKS:XKXS, NDEGLEX:VPTEE:EPIS-AA$^{17}$-LYL:KFKYE:FLQHN:KXEXR, NDEGLEX:VPTGQ:EPIS-AA$^{17}$-LYL:KFKYE:LEEHS:QXEXR, SSVKXQP:SRVHH:EPIS-AA$^{17}$-LYL:KFKYE:RLEEH:LEXAXA, RNVQXRP:TQVQL:EPIS-AA$^{17}$-LYL:KFKYE:TLEDH:LAXKXE, KIPKAXX:TPTEL:SPIN-AA$^{17}$-LYF:YGKIP:DMVVD:GXGXR, GIPEPXX:TPTKM:SPIN-AA$^{17}$-LYF:YGKIP:NMVVD:SXAXR, SIPKAXX:TPTEL:SPIN-AA$^{17}$-LYF:YGKIP:EMVVD:GXGXR, HVTKPTX:TPTKL:SPIN-AA$^{17}$-LYF:YGKIP:NMVVD:SXGXH, YVPKPXX:TPTKL:SPIN-AA$^{17}$-LYF:YGKIP:NMVVD:SXGXH, TVPKPXX:TPTQL:SPIN-AA$^{17}$-LYF:YGKIP:NMVVD:AXGXH, AVPKAXX:TPTKL:SPIN-AA$^{17}$-LYF:YGKIP:NMVVD:AXGXH, KVGKAXX:TPTKL:SPIN-AA$^{17}$-LYF:YGKIP:EGMSVVD:XGXR, KASKAXX:TPTKL:SPIN-AA$^{17}$-LYF:YGKIP:GMVVD:EXGXR, AAPASXX:TPTRL:SPIN-AA$^{17}$-LYF:YGKIP:DMVVD:AXGXR, STPPTXX:TPTRL:SPIN-AA$^{17}$-LYF:YGKIP:DMVVD:SXGXR, HVPKPXX:TPTKL:SPIN-AA$^{17}$-LYF:YGKIP:NMVVD:SXGXH, RVPSTXX:TPTKT:SPIN-AA$^{17}$-LYF:YGKIP:MIVVD:XGXL, ASAAPXX:TPTAL:SPIN-AA$^{17}$-LYF:YGKIP:MIVVD:XKXS, ASASPXX:TPTDL:SPIN-AA$^{17}$-LYF:YGKIP:MIVVD:XKXS, ASASPXX:TPTDL:SPIN-AA$^{17}$-LYF:YGKIP:MVVVD:XKXS, NDEGLEX:TPTEE:SPIN-AA$^{17}$-LYF:YGKIP:FLVVD:KXEXR, NDEGLEX:TPTGQ:SPIN-AA$^{17}$-LYF:YGKIP:LEVVD:QXEXR, SSVKXQP:TPTHH:SPIN-AA$^{17}$-LYF:YGKIP:RLVVD:LEXAXA, RNVQXRP:TPTQL:SPIN-AA$^{17}$-LYF:YGKIP:TLVVD:LAXKXE, KIPKAXX:VPTEL:SPIN-AA$^{17}$-LYF:YGKIP:DMVVE:GXGXR, GIPEPXX:VPEKM:SPIN-AA$^{17}$-LYF:YGKIP:NMTVE:SXAXR, SIPKAXX:VPTEL:SPIN-AA$^{17}$-LYF:YGKIP:EMVVE:GXGXR, HVTKPTX:APTKL:SPIN-AA$^{17}$-LYF:YGKIP:NMVVR:SXGXH, YVPKPXX:APTKL:SPIN-AA$^{17}$-LYF:YGKIP:NMVVR:SXGXH, TVPKPXX:APTQL:SPIN-AA$^{17}$-LYF:YGKIP:NMVVR:AXGXH, AVPKAXX:APTKL:SPIN-AA$^{17}$-LYF:YGKIP:NMVVK:AXGXH, KVGKAXX:VPTKL:SPIN-AA$^{17}$-LYF:YGKIP:EGMSVAE:XGXR, KASKAXX:VPTKL:SPIN-AA$^{17}$-LYF:YGKIP:GMAVS:EXGXR, AAPASXX:VPARL:SPIN-AA$^{17}$-LYF:YGKIP:DMVVE:AXGXR, STPPTXX:VPTRL:SPIN-AA$^{17}$-LYF:YGKIP:DMVVE:SXGXR, HVPKPXX:APTKL:SPIN-AA$^{17}$-LYF:YGKIP:NMVVR:SXGXH, RVPSTXX:APVKT:SPIN-AA$^{17}$-LYF:YGKIP:MIVEE:XGXL, ASAAPXX:VPQAL:SPIN-AA$^{17}$-LYF:YGKIP:MIVRS:XKXS, ASASPXX:VSQDL:SPIN-AA$^{17}$-LYF:YGKIP:MIVKS:XKXS, ASASPXX:VPQDL:SPIN-AA$^{17}$-LYF:YGKIP:MVVKS:XKXS, NDEGLEX:VPTEE:SPIN-AA$^{17}$-LYF:YGKIP:FLQHN:KXEXR, NDEGLEX:VPTGQ:SPIN-AA$^{17}$-LYF:YGKIP:LEEHS:QXEXR, SSVKXQP:SRVHH:SPIN-AA$^{17}$-LYF:YGKIP:RLEEH:LEXAXA, RNVQXRP:TQVQL:SPIN-AA$^{17}$-LYF:YGKIP:TLEDH:LAXKXE, KIPKAXX:VPAEL:SPIS-AA$^{17}$-LYI:YKQYE:DMVVE:GXGXR, GIPEPXX:VPAKM:SPIS-AA$^{17}$-LYI:YKQYE:NMVVE:SXAXR, SIPKAXX:VPAEL:SPIS-AA$^{17}$-LYI:YKQYE:EMVVE:GXGXR, HVTKPTX:VPAKL:SPIS-AA$^{17}$-LYI:YKQYE:NMVVE:SXGXH, YVPKPXX:VPAKL:SPIS-AA$^{17}$-LYI:YKQYE:NMVVE:SXGXH, TVPKPXX:VPAQL:SPIS-AA$^{17}$-LYI:YKQYE:NMVVE:AXGXH, AVPKAXX:VPAKL:SPIS-AA$^{17}$-LYI:YKQYE:NMVVE:AXGXH, KVGKAXX:VPAKL:SPIS-AA$^{17}$-LYI:YKQYE:EGMSVVE:XGXR, KASKAXX:VPAKL:SPIS-AA$^{17}$-LYI:YKQYE:GMVVE:EXGXR, GSAGPXX:VPA-

KM:SPIS-AA$^{17}$-LYI:YKQYE:GMVVE:RXGXS, STPPTXX:VPARL:SPIS-AA$^{17}$-LYI:YKQYE:DMVVE:SXGXR, HVPKPXX:VPAKL:SPIS-AA$^{17}$-LYI:YKQYE:NMVVE:SXGXH, RVPSTXX:VPAKT:SPIS-AA$^{17}$-LYI:YKQYE:MIVVE:XGXL, ASAAPXX:VPAAL:SPIS-AA$^{17}$-LYI:YKQYE:MIVVE:XKXS, ASASPXX:VPADL:SPIS-AA$^{17}$-LYI:YKQYE:M IVVE:XKXS, ASASPXX:VPADL:SPIS-AA$^{17}$-LYI:YKQYE:MVVVE:XKXS, NDEGLEX:VPAEE:SPIS-AA$^{17}$-LYI:YKQYE:FLVVE:KXEXR, NDEGLEX:VPAGQ:SPIS-AA$^{17}$-LYI:YKQYE:LEVVE:QXEXR, SSVKXQP:VPAHH:SPIS-AA$^{17}$-LYI:YKQYE:RLVVE:LEXAXA, RNVQXRP:VPAQL:SPIS-AA$^{17}$-LYI:YKQYE:TLVVE:LAXKXE, KIPKAXX:VPTEL:SPIS-AA$^{17}$-LYI:YKQYE:DMVVE:GXGXR, GIPEPXX:VPEKM:SPIS-AA$^{17}$-LYI:YKQYE:NMTVE:SXAXR, SIPKAXX:VPTEL:SPIS-AA$^{17}$-LYI:YKQYE:EMVVE:GXGXR, HVTKPTX:APTKL:SPIS-AA$^{17}$-LYI:YKQYE:NMVVR:SXGXH, YVPKPXX:APTKL:SPIS-AA$^{17}$-LYI:YKQYE:NMVVR:SXGXH, TVPKPXX:APTQL:SPIS-AA$^{17}$-LYI:YKQYE:NMVVR:AXGXH, AVPKAXX:APTKL:SPIS-AA$^{17}$-LYI:YKQYE:NMVVK:AXGXH, KVGKAXX:VPTKL:SPIS-AA$^{17}$-LYI:YKQYE:EGMSVAE:XGXR, KASKAXX:VPTKL:SPIS-AA$^{17}$-LYI:YKQYE:GMAVS:EXGXR, GSAGPXX:TPTKM:SPIS-AA$^{17}$-LYIYKQYE:GMVVD:RXGXS, STPPTXX:VPTRL:SPIS-AA$^{17}$-LYI:YKQYE:DMVVE:SXGXR, HVPKPXX:APTKL:SPIS-AA$^{17}$-LYI:YKQYE:NMVVR:SXGXH, RVPSTXX:APVKT:SPIS-AA$^{17}$-LYI:YKQYE:MIVEE:XGXL, ASAAPXX:VPQAL:SPIS-AA$^{17}$-LYI:YKQYE:MIVRS:XKXS, ASASPXX:VSQDL:SPIS-AA$^{17}$-LYI:YKQYE:MIVKS:XKXS, ASASPXX:VPQDL:SPIS-AA$^{17}$-LYI:YKQYE:MVVKS:XKXS, NDEGLEX:VPTEE:SPIS-AA$^{17}$-LYI:YKQYE:FLQHN :KXEXR, NDEGLEX:VPTGQ:SPIS-AA$^{17}$-LYI:YKQYE:LEEHS:QXEXR, SSVKXQP:SRVHH:SPIS-AA$^{17}$-LYI:YKQYE:RLEEH:LEXAXA, RNVQXRP:TQVQL:SPIS-AA$^{17}$-LYI:YKQYE:TLEDH:LAXKXE, KIPKAXX:VPTEL:SPIS-AA$^{17}$-LFI:YKQYE:DMVVE:GXGXR, GIPEPXX:VPTKM:SPIS-AA$^{17}$-LFI:YKQYE:NMVVE:SXAXR, SIPKAXX:VPTEL:SPIS-AA$^{17}$-LFI:YKQYE:EMVVE:GXGXR, HVTKPTX:VPTKL:SPIS-AA$^{17}$-LFI:YKQYE:NMVVE:SXGXH, YVPKPXX:VPTKL:SPIS-AA$^{17}$-LFI:YKQYE:NMVVE:SXGXH, TVPKPXX:VPTQL:SPIS-AA$^{17}$-LFI:YKQYE:NMVVE:AXGXH, AVPKAXX:VPTKL:SPIS-AA$^{17}$-LFI:YKQYE:NMVVE:AXGXH, KVGKAXX:VPTKL:SPIS-AA$^{17}$-LFI:YKQYE:EGMSVVE:XGXR, KASKAXX:VPTKL:SPIS-AA$^{17}$-LFI:YKQYE:GMVVE:EXGXR, GSAGPXX:VPTKM:SPIS-AA$^{17}$-LFI:YKQYE:GMVVE:RXGXS, AAPASXX:VPTRL:SPIS-AA$^{17}$-LFI:YKQYE:DMVVE:AXGXR, HVPKPXX:VPTKL:SPIS-AA$^{17}$-LFI:YKQYE:NMVVE:SXGXH, RVPSTXX:VPTKT:SPIS-AA$^{17}$-LFI:YKQYE:MIVVE:XGXL, ASAAPXX:VPTAL:SPIS-AA$^{17}$-LFI:YKQYE:M IVVE:XKXS, ASASPXX:VPTDL:SPIS-AA$^{17}$-LFI:YKQYE:MIVVE:XKXS, ASASPXX:VPTDL:SPIS-AA$^{17}$-LFI:YKQYE:MVVVE:XKXS, NDEGLEX:VPTEE:SPIS-AA$^{17}$-LFI:YKQYE:FLVVE:KXEXR, NDEGLEX:VPTGQ:SPIS-AA$^{17}$-LFI:YKQYE:LEVVE:QXEXR, SSVKXQP:VPTHH:SPIS-AA$^{17}$-LFI:YKQYE:RLVVE:LEXAXA, RNVQXRP:VPTQL:SPIS-AA$^{17}$-LFI:YKQYE:TLVVE:LAXKXE, GIPEPXX:VPEKM:SPIS-AA$^{17}$-LFI:YKQYE:NMTVE:SXAXR, HVTKPTX:APTKL:SPIS-AA$^{17}$-LFI:YKQYE:NMVVR:SXGXH, YVPKPXX:APTKL:SPIS-AA$^{17}$-LFI:YKQYE:NMVVR:SXGXH, TVPKPXX:APTQL:SPIS-AA$^{17}$-LFI:YKQYE:NMVVR:AXGXH, AVPKAXX:APTKL:SPIS-AA$^{17}$-LFI:YKQYE:NMVVK:AXGXH, KVGKAXX:VPTKL:SPIS-AA$^{17}$-LFI:YKQYE:EGMSVAE:XGXR, KASKAXX:VPTKL:SPIS-AA$^{17}$-LFI:YKQYE:GMAVS:EXGXR, GSAGPXX:TPTKM:SPIS-AA$^{17}$-LFI:YKQYE:GMVVD:RXGXS, AAPASXX:VPARL:SPIS-AA$^{17}$-LFI:YKQYE:DMVVE:AXGXR, HVPKPXX:APTKL:SPIS-AA$^{17}$-LFI:YKQYE:NMVVR:SXGXH, RVPSTXX:APVKT:SPIS-AA$^{17}$-LFI:YKQYE:MIVEE:XGXL, ASAAPXXVPQAL:SPIS-AA$^{17}$-LFI:YKQYE:MIVRS:XKXS, ASASPXX:VSQDL:SPIS-AA$^{17}$-LFI:YKQYE:MIVKS:XKXS, ASASPXX:VPQDL:SPIS-AA$^{17}$-LFI:YKQYE:MVVKS:XKXS, NDEGLEX:VPTEE:SPIS-AA$^{17}$-LFI:YKQYE:FLQHN:KXEXR, NDEGLEX:VPTGQ:SPIS-AA$^{17}$-LFI:YKQYE:LEEHS:QXEXR, SSVKXQP:SRVHH:SPIS-AA$^{17}$-LFI:YKQYE:RLEEH:LEXAXA, RNVQXRP:TQVQL:SPIS-AA$^{17}$-LFI:YKQYE:TLEDH:LAXKXE, KIPKAXX:APVEL:KPLS-AA$^{17}$-LYV:DHHKD:DMVEE:GXGXR, GIPEPXX:APVKM:KPLS-AA$^{17}$-LYV:DHHKD:NMVEE:SXAXR, SIPKAXX:APVEL:KPLS-AA$^{17}$-LYV:DHHKD:EMVEE:GXGXR, HVTKPTX:APVKL:KPLS-AA$^{17}$-LYV:DHHKD:NMVEE:SXGXH, YVPKPXX:APVKL:KPLS-AA$^{17}$-LYV:DHHKD:NMVEE:SXGXH, TVPKPXX:APVQL:KPLS-AA$^{17}$-LYV:DHHKD:NMVEE:AXGXH, AVPKAXX:APVKL:KPLS-AA$^{17}$-LYV:DHHKD:NMVEE:AXGXH, KVGKAXX:APVKL:KPLS-AA$^{17}$-LYV:DHHKD:EGMSVEE:XGXR, KASKAXX:APVKL:KPLS-AA$^{17}$-LYV:DHHKD:GMVEE:EXGXR, GSAGPXX:APVKM:KPLS-AA$^{17}$-LYV:DHHKD:GMVEE:RXGXS, AAPASXX:APVRL:KPLS-AA$^{17}$-LYV:DHHKD:DMVEE:AXGXR, STPPTXX:APVRL:KPLS-AA$^{17}$-LYV:DHHKD:DMVEE:SXGXR, HVPKPXX:APVKL:KPLS-AA$^{17}$-LYV:DHHKD:NMVEE:SXGXH, ASAAPXX:APVAL:KPLS-AA$^{17}$-LYV:DHHKD:MIVEE:XKXS, ASASPXX:APVDL:KPLS-AA$^{17}$-LYV:DHHKD:MIVEE:XKXS, ASASPXX:APVDL:KPLS-AA$^{17}$-LYV:DHHKD:MVVEE:XKXS, NDEGLEX:APVEE:KPLS-AA$^{17}$-LYV:DHHKD:FLVEE:KXEXR, NDEGLEX:APVGQ:KPLS-AA$^{17}$-LYV:DHHKD:LEVEE:QXEXR, SSVKXQP:APVHH:KPLS-AA$^{17}$-LYV:DHHKD:RLVEE:LEXAXA, RNVQXRP:APVQL:KPLS-AA$^{17}$-LYV:DHHKD:TLVEE:LAXKXE, KIPKAXX:VPTEL:KPLS-AA$^{17}$-LYV:DHHKD:DMVVE:GXGXR, GIPEPXX:VPEKM:KPLS-AA$^{17}$-LYV:DHHKD:NMTVE:SXAXR, SIPKAXX:VPTEL:KPLS-AA$^{17}$-LYV:DHHKD:EMVVE:GXGXR, HVTKPTX:APTKL:KPLS-AA$^{17}$-LYV:DHHKD:NMVVR:SXGXH, YVPKPXX:APTKL:KPLS-AA$^{17}$-LYV:DHHKD:NMVVR:SXGXH, TVPKPXX:APTQL:KPLS-AA$^{17}$-LYV:DHHKD:NMVVR:AXGXH, AVPKAXX:APTKL:KPLS-AA$^{17}$-LYV:DHHKD:NMVVK:AXGXH, KVGKAXX:VPTKL:KPLS-AA$^{17}$-LYV:DHHKD:EGMSVAE:XGXR, KASKAXX:VPTKL:KPLS-AA$^{17}$-LYV:DHHKD:GMAVS:EXGXR, GSAGPXX:TPTKM:KPLS-AA$^{17}$-LYV:DHHKD:GMVVD:RXGXS, AAPASXX:VPARL:KPLS-AA$^{17}$-LYV:DHHKD:DMVVE:AXGXR, STPPTXX:VPTRL:KPLS-AA$^{17}$-LYV:DHHKD:DMVVE:SXGXR, HVPKPXX:APTKL:KPLS-AA$^{17}$-LYV:DHHKD:NMVVR:SXGXH, ASAAPXX:VPQAL:KPLS-AA$^{17}$-LYV:DHHKD:MIVRS:XKXS,

ASASPXX:VSQDL:KPLS-AA$^{17}$-LYV:DHHKD:MIVKS:XKXS, ASASPXX:VPQDL:KPLS-AA$^{17}$-LYV:DHHKD:MVVKS:XKXS, NDEGLEX:VPTEE:KPLS-AA$^{17}$-LYV:DHHKD:FLQHN:KXEXR, NDEGLEX:VPTGQ:KPLS-AA$^{17}$-LYV:DHHKD:LEEHS:QXEXR, SSVKXQP:SRVHH:KPLS-AA$^{17}$-LYV:DHHKD:RLEEH:LEXAXA, RNVQXRP:TQVQL:KPLS-AA$^{17}$-LYV:DHHKD:TLEDH:LAXKXE, KIPKAXX:VPQEL:EPLP-AA$^{17}$-VYY:EQLSN:DMVRS:GXGXR, GIPEPXX:VPQKM:EPLP-AA$^{17}$-VYY:EQLSN:NMVRS:SXAXR, SIPKAXX:VPQEL:EPLP-AA$^{17}$-VYY:EQLSN:EMVRS:GXGXR, HVTKPTX:VPQKL:EPLP-AA$^{17}$-VYY:EQLSN:NMVRS:SXGXH, YVPKPXX:VPQKL:EPLP-AA$^{17}$-VYY:EQLSN:NMVRS:SXGXH, TVPKPXX:VPQQL:EPLP-AA$^{17}$-VYY:EQLSN:NMVRS:AXGXH, AVPKAXX:VPQKL:EPLP-AA$^{17}$-VYY:EQLSN:NMVRS:AXGXH, KVGKAXX:VPQKL:EPLP-AA$^{17}$-VYY:EQLSN:EGMSVRS:XGXR, KASKAXX:VPQKL:EPLP-AA$^{17}$-VYY:EQLSN:GMVRS:EXGXR, GSAGPXX:VPQKM:EPLP-AA$^{17}$-VYY:EQLSN:GMVRS:RXGXS, AAPASXX:VPQRL:EPLP-AA$^{17}$-VYY:EQLSN:DMVRS:AXGXR, STPPTXX:VPQRL:EPLP-AA$^{17}$-VYY:EQLSN:DMVRS:SXGXR, HVPKPXX:VPQKL:EPLP-AA$^{17}$-VYY:EQLSN:NMVRS:SXGXH, RVPSTXX:VPQKT:EPLP-AA$^{17}$-VYY:EQLSN:MIVRS:XGXL, ASASPXX:VPQDL:EPLP-AA$^{17}$-VYY:EQLSN:MIVRS:XKXS, ASASPXX:VPQDL:EPLP-AA$^{17}$-VYY:EQLSN:MVVRS:XKXS, NDEGLEX:VPQEE:EPLP-AA$^{17}$-VYY:EQLSN:FLVRS:KXEXR, NDEGLEX:VPQGQ:EPLP-AA$^{17}$-VYY:EQLSN:LEVRS:QXEXR, SSVKXQP:VPQHH:EPLP-AA$^{17}$-VYY:EQLSN:RLVRS:LEXAXA, RNVQXRP:VPQQL:EPLP-AA$^{17}$-VYY:EQLSN:TLVRS:LAXKXE, KIPKAXX:VPTEL:EPLP-AA$^{17}$-VYY:EQLSN:DMVVE:GXGXR, GIPEPXX:VPEKM:EPLP-AA$^{17}$-VYY:EQLSN:NMTVE:SXAXR, SIPKAXX:VPTEL:EPLP-AA$^{17}$-VYY:EQLSN:EMVVE:GXGXR, HVTKPTX:APTKL:EPLP-AA$^{17}$-VYY:EQLSN:NMVVR:SXGXH, YVPKPXX:APTKL:EPLP-AA$^{17}$-VYY:EQLSN:NMVVR:SXGXH, TVPKPXX:APTQL:EPLP-AA$^{17}$-VYY:EQLSN:NMVVR:AXGXH, AVPKAXX:APTKL:EPLP-AA$^{17}$-VYY:EQLSN:NMVVK:AXGXH, KVGKAXX:VPTKL:EPLP-AA$^{17}$-VYY:EQLSN:EGMSVAE:XGXR, KASKAXX:VPTKL:EPLP-AA$^{17}$-VYY:EQLSN:GMAVS:EXGXR, GSAGPXX:TPTKM:EPLP-AA$^{17}$-VYY:EQLSN:GMVVD:RXGXS, AAPASXX:VPARL:EPLP-AA$^{17}$-VYY:EQLSN:DMVVE:AXGXR, STPPTXX:VPTRL:EPLP-AA$^{17}$-VYY:EQLSN:DMVVE:SXGXR, HVPKPXX:APTKL:EPLP-AA$^{17}$-VYY:EQLSN:NMVVR:SXGXH, RVPSTXX:APVKT:EPLP-AA$^{17}$-VYY:EQLSN:MIVEE:XGXL, ASASPXX:VSQDL:EPLP-AA$^{17}$-VYY:EQLSN:MIVKS:XKXS, ASASPXX:VPQDL:EPLP-AA$^{17}$-VYY:EQLSN:MVVKS:XKXS, NDEGLEX:VPTEE:EPLP-AA$^{17}$-VYY:EQLSN:FLQHN:KXEXR, NDEGLEX:VPTGQ:EPLP-AA$^{17}$-VYY:EQLSN:LEEHS:QXEXR, SSVKXQP:SRVHH:EPLP-AA$^{17}$-VYY:EQLSN:RLEEH:LEXAXA, RNVQXRP:TQVQL:EPLP-AA$^{17}$-VYY:EQLSN:TLEDH:LAXKXE, KIPKAXX:VSQEL:EPLT-AA$^{17}$-LYY:EQLSN:DMVKS:GXGXR, GIPEPXX:VSQKM:EPLT-AA$^{17}$-LYY:EQLSN:NMVKS:SXAXR, SIPKAXX:VSQEL:EPLT-AA$^{17}$-LYY:EQLSN:EMVKS:GXGXR, HVTKPTX:VSQKL:EPLT-AA$^{17}$-LYY:EQLSN:NMVKS:SXGXH, YVPKPXX:VSQKL:EPLT-AA$^{17}$-LYY:EQLSN:NMVKS:SXGXH, TVPKPXX:VSQQL:EPLT-AA$^{17}$-LYY:EQLSN:NMVKS:AXGXH, AVPKAXX:VSQKL:EPLT-AA$^{17}$-LYY:EQLSN:NMVKS:AXGXH, KVGKAXX:VSQKL:EPLT-AA$^{17}$-LYY:EQLSN:EGMSVKS:XGXR, KASKAXXVSQKL:EPLT-AA$^{17}$-LYY:EQLSN:GMVKS:EXGXR, GSAGPXX:VSQKM:EPLT-AA$^{17}$-LYY:EQLSN:GMVKS:RXGXS, AAPASXX:VSQRL:EPLT-AA$^{17}$-LYY:EQLSN:DMVKS:AXGXR, STPPTXX:VSQRL:EPLT-AA$^{17}$-LYY:EQLSN:DMVKS:SXGXR, HVPKPXX:VSQKL:EPLT-AA$^{17}$-LYY:EQLSN:NMVKS:SXGXH, RVPSTXX:VSQKT:EPLT-AA$^{17}$-LYY:EQLSN:MIVKS:XGXL, ASAAPXX:VSQAL:EPLT-AA$^{17}$-LYY:EQLSN:MIVKS:XKXS, ASASPXX:VSQDL:EPLT-AA$^{17}$-LYY:EQLSN:MVVKS:XKXS, NDEGLEX:VSQEE:EPLT-AA$^{17}$-LYY:EQLSN:FLVKS:KXEXR, NDEGLEX:VSQGQ:EPLT-AA$^{17}$-LYY:EQLSN:LEVKS:QXEXR, SSVKXQP:VSQHH:EPLT-AA$^{17}$-LYY:EQLSN:RLVKS:LEXAXA, RNVQXRP:VSQQL:EPLT-AA$^{17}$-LYY:EQLSN:TLVKS:LAXKXE, KIPKAXX:VPTEL:EPLT-AA$^{17}$-LYY:EQLSN:DMVVE:GXGXR, GIPEPXX:VPEKM:EPLT-AA$^{17}$-LYY:EQLSN:NMTVE:SXAXR, SIPKAXX:VPTEL:EPLT-AA$^{17}$-LYY:EQLSN:EMVVE:GXGXR, HVTKPTX:APTKL:EPLT-AA$^{17}$-LYY:EQLSN:NMVVR:SXGXH, YVPKPXX:APTKL:EPLT-AA$^{17}$-LYY:EQLSN:NMVVR:SXGXH, TVPKPXX:APTQL:EPLT-AA$^{17}$-LYY:EQLSN:NMVVR:AXGXH, AVPKAXX:APTKL:EPLT-AA$^{17}$-LYY:EQLSN:NMVVK:AXGXH, KVGKAXX:VPTKL:EPLT-AA$^{17}$-LYY:EQLSN:EGMSVAE:XGXR, KASKAXX:VPTKL:EPLT-AA$^{17}$-LYY:EQLSN:GMAVS:EXGXR, GSAGPXX:TPTKM:EPLT-AA$^{17}$-LYY:EQLSN:GMVVD:RXGXS, AAPASXX:VPARL:EPLT-AA$^{17}$-LYY:EQLSN:DMVVE:AXGXR, STPPTXX:VPTRL:EPLT-AA$^{17}$-LYY:EQLSN:DMVVE:SXGXR, HVPKPXX:APTKL:EPLT-AA$^{17}$-LYY:EQLSN:NMVVR:SXGXH, RVPSTXX:APVKT:EPLT-AA$^{17}$-LYY:EQLSN:MIVEE:XGXL, ASAAPXX:VPQAL:EPLT-AA$^{17}$-LYY:EQLSN:MIVRS:XKXS, ASASPXX:VPQDL:EPLT-AA$^{17}$-LYY:EQLSN:MVVKS:XKXS, NDEGLEX:VPTEE:EPLT-AA$^{17}$-LYY:EQLSN:FLQHN:KXEXR, NDEGLEX:VPTGQ:EPLT-AA$^{17}$-LYY:EQLSN:LEEHS:QXEXR, SSVKXQP:SRVHH:EPLT-AA$^{17}$-LYY:EQLSN:RLEEH:LEXAXA, RNVQXRP:TQVQL:EPLT-AA$^{17}$-LYY:EQLSN:TLEDH:LAXKXE, KIPKAXX:VPQEL:EPLT-AA$^{17}$-LYY:EQLSN:DMVKS:GXGXR, GIPEPXX:VPQKM:EPLT-AA$^{17}$-LYY:EQLSN:NMVKS:SXAXR, SIPKAXX:VPQEL:EPLT-AA$^{17}$-LYY:EQLSN:EMVKS:GXGXR, HVTKPTX:VPQKL:EPLT-AA$^{17}$-LYY:EQLSN:NMVKS:SXGXH, YVPKPXX:VPQKL:EPLT-AA$^{17}$-LYY:EQLSN:NMVKS:SXGXH, TVPKPXX:VPQQL:EPLT-AA$^{17}$-LYY:EQLSN:NMVKS:AXGXH, AVPKAXX:VPQKL:EPLT-AA$^{17}$-LYY:EQLSN:NMVKS:AXGXH, KVGKAXX:VPQKL:EPLT-AA$^{17}$-LYY:EQLSN:EGMSVKS:XGXR, KASKAXX:VPQKL:EPLT-AA$^{17}$-LYY:EQLSN:GMVKS:EXGXR, GSAGPXX:VPQKM:EPLT-AA$^{17}$-LYY:EQLSN:GMVKS:RXGXS, AAPASXX:VPQRL:EPLT-AA$^{17}$-LYY:EQLSN:DMVKS:AXGXR, STPPTXX:VPQRL:EPLT-AA$^{17}$-LYY:EQLSN:DMVKS:SXGXR, HVPKPXX:VPQKL:EPLT-AA$^{17}$-LYY:EQLSN:NMVKS:SXGXH, RVPSTXX:VPQKT:EPLT-AA$^{17}$-LYY:EQLSN:MIVKS:XGXL,

ASAAPXX:VPQAL:EPLT-AA$^{17}$-LYY:EQLSN:MIVKS:XKXS, ASASPXX:VPQDL:EPLT-AA$^{17}$-LYY:EQLSN:MIVKS:XKXS, NDEGLEX:VPQEE:EPLT-AA$^{17}$-LYY:EQLSN:FLVKS:KXEXR, NDEGLEX:VPQGQ:EPLT-AA$^{17}$-LYY:EQLSN:LEVKS:QXEXR, SSVKXQP:VPQHH:EPLT-AA$^{17}$-LYY:EQLSN:RLVKS:LEXAXA, RNVQXRP:VPQQL:EPLT-AA$^{17}$-LYY:EQLSN:TLVKS:LAXKXE, ASASPXX:VSQDL:EPLT-AA$^{17}$-LYY:EQLSN:MIVKS:XKXS, KIPKAXX:VPTEL:SNIT-AA$^{17}$-QIM:IGEMS:DMQHN:GXGXR, GIPEPXX:VPTKM:SNIT-AA$^{17}$-QIM:IGEMS:NMQHN:SXAXR, SIPKAXX:VPTEL:SNIT-AA$^{17}$-QIM:IGEMS:EMQHN:GXGXR, HVTKPTX:VPTKL:SNIT-AA$^{17}$-QIM:IGEMS:NMQHN:SXGXH, YVPKPXX:VPTKL:SNIT-AA$^{17}$-QIM:IGEMS:NMQHN:SXGXH, TVPKPXX:VPTQL:SNIT-AA$^{17}$-QIM:IGEMS:NMQHN:AXGXH, AVPKAXX:VPTKL:SNIT-AA$^{17}$-QIM:IGEMS:NMQHN:AXGXH, KVGKAXXVPTKL:SNIT-AA$^{17}$-QIM:IGEMS:EGMSQHN:XGXR, KASKAXX:VPTKL:SNIT-AA$^{17}$-QIM:IGEMS:GMQHN:EXGXR, GSAGPXX:VPTKM:SNIT-AA$^{17}$-QIM:IGEMS:GMQHN:RXGXS, AAPASXX:VPTRL:SNIT-AA$^{17}$-QIM:IGEMS:DMQHN:AXGXR, STPPTXX:VPTRL:SNIT-AA$^{17}$-QIM:IGEMS:DMQHN:SXGXR, HVPKPXX:VPTKL:SNIT-AA$^{17}$-QIM:IGEMS:NMQHN:SXGXH, RVPSTXX:VPTKT:SNIT-AA$^{17}$-QIM:IGEMS:MIQHN:XGXL, ASAAPXX:VPTAL:SNIT-AA$^{17}$-QIM:IGEMS:MIQHN:XKXS, ASASPXX:VPTDL:SNIT-AA$^{17}$-QIM:IGEMS:MIQHN:XKXS, ASASPXX:VPTDL:SNIT-AA$^{17}$-QIM:IGEMS:MVQHN:XKXS, NDEGLEX:VPTGQ:SNIT-AA$^{17}$-QIM:IGEMS:LEQHN:QXEXR, NDEGLEX:VPTEE:SNIT-AA$^{17}$-QIM:IGEMS:FLQHN:KXEXR, SSVKXQP:VPTHH:SNIT-AA$^{17}$-QIM:IGEMS:RLQHN:LEXAXA, RNVQXRP:VPTQL:SNIT-AA$^{17}$-QIM:IGEMS:TLQHN:LAXKXE, KIPKAXX:VPTEL:SNIT-AA$^{17}$-QIM:IGEMS:DMVVE:GXGXR, GIPEPXX:VPEKM:SNIT-AA$^{17}$-QIM:IGEMS:NMTVE:SXAXR, SIPKAXX:VPTEL:SNIT-AA$^{17}$-QIM:IGEMS:EMVVE:GXGXR, HVTKPTX:APTKL:SNIT-AA$^{17}$-QIM:IGEMS:NMVVR:SXGXH, YVPKPXX:APTKL:SNIT-AA$^{17}$-QIM:IGEMS:NMVVR:SXGXH, TVPKPXX:APTQL:SNIT-AA$^{17}$-QIM:IGEMS:NMVVR:AXGXH, AVPKAXX:APTKL:SNIT-AA$^{17}$-QIM:IGEMS:NMVVK:AXGXH, KVGKAXX:VPTKL:SNIT-AA$^{17}$-QIM:IGEMS:EGMSVAE:XGXR, KASKAXX:VPTKL:SNIT-AA$^{17}$-QIM:IGEMS:GMAVS:EXGXR, GSAGPXX:TPTKM:SNIT-AA$^{17}$-QIM:IGEMS:GMVVD:RXGXS, AAPASXX:VPARL:SNIT-AA$^{17}$-QIM:IGEMS:DMVVE:AXGXR, STPPTXX:VPTRL:SNIT-AA$^{17}$-QIM:IGEMS:DMVVE:SXGXR, HVPKPXX:APTKL:SNIT-AA$^{17}$-QIM:IGEMS:NMVVR:SXGXH, RVPSTXX:APVKT:SNIT-AA$^{17}$-QIM:IGEMS:MIVEE:XGXL, ASAAPXX:VPQAL:SNIT-AA$^{17}$-QIM:IGEMS:MIVRS:XKXS, ASASPXX:VSQDL:SNIT-AA$^{17}$-QIM:IGEMS:MIVKS:XKXS, ASASPXX:VPQDL:SNIT-AA$^{17}$-QIM:IGEMS:MVVKS:XKXS, NDEGLEX:VPTGQ:SNIT-AA$^{17}$-QIM:IGEMS:LEEHS:QXEXR, SSVKXQP:SRVHH:SNIT-AA$^{17}$-QIM:IGEMS:RLEEH:LEXAXA, RNVQXRP:TQVQL:SNIT-AA$^{17}$-QIM:IGEMS:TLEDH:LAXKXE, KIPKAXX:VPTEL:SNIT-AA$^{17}$-QIM:LGEMS:DMEHS:GXGXR, GIPEPXX:VPTKM:SNIT-AA$^{17}$-QIM:LGEMS:NMEHS:SXAXR, SIPKAXX:VPTEL:SNIT-AA$^{17}$-QIM:LGEMS:EMEHS:GXGXR, HVTKPTX:VPTKL:SNIT-AA$^{17}$-QIM:LGEMS:NMEHS:SXGXH, YVPKPXX:VPTKL:SNIT-AA$^{17}$-QIM:LGEMS:NMEHS:SXGXH, TVPKPXX:VPTQL:SNIT-AA$^{17}$-QIM:LGEMS:NMEHS:AXGXH, AVPKAXX:VPTKL:SNIT-AA$^{17}$-QIM:LGEMS:NMEHS:AXGXH, KVGKAXX:VPTKL:SNIT-AA$^{17}$-QIM:LGEMS:EGMSEHS:XGXR, KASKAXXVPTKL:SNIT-AA$^{17}$-QIM:LGEMS:GMEHS:EXGXR, GSAGPXX:VPTKM:SNIT-AA$^{17}$-QIM:LGEMS:GMEHS:RXGXS, AAPASXX:VPTRL:SNIT-AA$^{17}$-QIM:LGEMS:DMEHS:AXGXR, STPPTXX:VPTRL:SNIT-AA$^{17}$-QIM:LGEMS:DMEHS:SXGXR, HVPKPXX:VPTKL:SNIT-AA$^{17}$-QIM:LGEMS:NMEHS:SXGXH, RVPSTXX:VPTKT:SNIT-AA$^{17}$-QIM:LGEMS:MIEHS:XGXL, ASAAPXX:VPTAL:SNIT-AA$^{17}$-QIM:LGEMS:MIEHS:XKXS, ASASPXXVPTDL:SNIT-AA$^{17}$-QIM:LGEMS:MIEHS:XKXS, ASASPXX:VPTDL:SNIT-AA$^{17}$-QIM:LGEMS:MVEHS:XKXS, NDEGLEX:VPTEE:SNIT-AA$^{17}$-QIM:LGEMS:FLEHS:KXEXR, SSVKXQP:VPTHH:SNIT-AA$^{17}$-QIM:LGEMS:RLEHS:LEXAXA, RNVQXRP:VPTQL:SNIT-AA$^{17}$-QIM:LGEMS:TLEHS:LAXKXE, KIPKAXX:VPTEL:SNIT-AA$^{17}$-QIM:LGEMS:DMVVE:GXGXR, GIPEPXXVPEKM:SNIT-AA$^{17}$-QIM:LGEMS:NMTVE:SXAXR, SIPKAXX:VPTEL:SNIT-AA$^{17}$-QIM:LGEMS:EMVVE:GXGXR, HVTKPTX:APTKL:SNIT-AA$^{17}$-QIM:LGEMS:NMVVR:SXGXH, YVPKPXX:APTKL:SNIT-AA$^{17}$-QIM:LGEMS:NMVVRSXGXH, TVPKPXX:APTQL:SNIT-AA$^{17}$-QIM:LGEMS:NMVVR:AXGXH, AVPKAXX:APTKL:SNIT-AA$^{17}$-QIM:LGEMS:NMVVK:AXGXH, KVGKAXX:VPTKL:SNIT-AA$^{17}$-QIM:LGEMS:EGMSVAE:XGXR, KASKAXX:VPTKL:SNIT-AA$^{17}$-QIM:LGEMS:GMAVS:EXGXR, GSAGPXX:TPTKM:SNIT-AA$^{17}$-QIM:LGEMS:GMVVD:RXGXS, AAPASXX:VPARL:SNIT-AA$^{17}$-QIM:LGEMS:DMVVE:AXGXR, STPPTXX:VPTRL:SNIT-AA$^{17}$-QIM:LGEMS:DMVVE:SXGXR, HVPKPXX:APTKL:SNIT-AA$^{17}$-QIM:LGEMS:NMVVR:SXGXH, RVPSTXX:APVKT:SNIT-AA$^{17}$-QIM:LGEMS:MIVEE:XGXL, ASAAPXX:VPQAL:SNIT-AA$^{17}$-QIM:LGEMS:MIVRS:XKXS, ASASPXX:VSQDL:SNIT-AA$^{17}$-QIM:LGEMS:MIVKS:XKXS, ASASPXX:VPQDL:SNIT-AA$^{17}$-QIM:LGEMS:MVVKS:XKXS, NDEGLEX:VPTEE:SNIT-AA$^{17}$-QIM:LGEMS:FLQHN:KXEXR, SSVKXQP:SRVHH:SNIT-AA$^{17}$-QIM:LGEMS:RLEEH:LEXAXA, RNVQXRP:TQVQL:SNIT-AA$^{17}$-QIM:LGEMS:TLEDH:LAXKXE, KIPKAXX:SRVEL:RSVK-AA$^{17}$-AKV:KEVQV:DMEEH:GXGXR, GIPEPXX:SRVKM:RSVK-AA$^{17}$-AKV:KEVQV:NMEEH:SXAXR, SIPKAXX:SRVEL:RSVK-AA$^{17}$-AKV:KEVQV:EMEEH:GXGXR, HVTKPTX:SRVKL:RSVK-AA$^{17}$-AKV:KEVQV:NMEEH:SXGXH, YVPKPXX:SRVKL:RSVK-AA$^{17}$-AKV:KEVQV:NMEEH:SXGXH, TVPKPXX:SRVQL:RSVK-AA$^{17}$-AKV:KEVQV:NMEEH:AXGXH, AVPKAXX:SRVKL:RSVK-AA$^{17}$-AKV:KEVQV:NMEEH:AXGXH, KVGKAXX:SRVKL:RSVK-AA$^{17}$-AKV:KEVQV:EGMSEEH:XGXR, KASKAXX:SRVKL:RSVK-AA$^{17}$-AKV:KEVQV:GMEEH:EXGXR, GSAGPXX:SRVKM:RSVK-AA$^{17}$-AKV:KEVQV:GMEEH:RXGXS, AAPASXX:SRVRL:RSVK-AA$^{17}$-AKV:KEVQV:DMEEH:AXGXR, STPP-

TXX:SRVRL:RSVK-AA$^{17}$-AKV:KEVQV:DMEEH:SXGXR, HVPKPXX:SRVKL:RSVK-AA$^{17}$-AKV:KEVQV:NMEEH:SXGXH, RVPSTXX:SRVKT:RSVK-AA$^{17}$-AKV:KEVQV:MIEEH:XGXL, ASAAPXX:SRVAL:RSVK-AA$^{17}$-AKV:KEVQV:MIEEH:XKXS, ASASPXX:SRVDL:RSVK-AA$^{17}$-AKV:KEVQV:MIEEH:XKXS, ASASPXX:SRVDL:RSVK-AA$^{17}$-AKV:KEVQV:MVEEH:XKXS, NDEGLEX:SRVEE:RSVK-AA$^{17}$-AKV:KEVQV:FLEEH:KXEXR, NDEGLEX:SRVGQ:RSVK-AA$^{17}$-AKV:KEVQV:LEEEH:QXEXR, RNVQXRP:SRVQL:RSVK-AA$^{17}$-AKV:KEVQV:TLEEH:LAXKXE, KIPKAXX:VPTEL:RSVK-AA$^{17}$-AKV:KEVQV:DMVVE:GXGXR, GIPEPXX:VPEKM:RSVK-AA$^{17}$-AKV:KEVQV:NMTVE:SXAXR, SIPKAXX:VPTEL:RSVK-AA$^{17}$-AKV:KEVQV:EMVVE:GXGXR, HVTKPTX:APTKL:RSVK-AA$^{17}$-AKV:KEVQV:NMVVR:SXGXH, YVPKPXX:APTKL:RSVK-AA$^{17}$-AKV:KEVQV:NMVVR:SXGXH, TVPKPXX:APTQL:RSVK-AA$^{17}$-AKV:KEVQV:NMVVR:AXGXH, AVPKAXX:APTKL:RSVK-AA$^{17}$-AKV:KEVQV:NMVVK:AXGXH, KVGKAXX:VPTKL:RSVK-AA$^{17}$-AKV:KEVQV:EGMSVAE:XGXR, KASKAXX:VPTKL:RSVK-AA$^{17}$-AKV:KEVQV:GMAVS:EXGXR, GSAGPXX:TPTKM:RSVK-AA$^{17}$-AKV:KEVQV:GMVVD:RXGXS, AAPASXX:VPARL:RSVK-AA$^{17}$-AKV:KEVQV:DMVVE:AXGXR, STPPTXX:VPTRL:RSVK-AA$^{17}$-AKV:KEVQV:DMVVE:SXGXR, HVPKPXX:APTKL:RSVK-AA$^{17}$-AKV:KEVQV:NMVVR:SXGXH, RVPSTXX:APVKT:RSVK-AA$^{17}$-AKV:KEVQV:MIVEE:XGXL, ASAAPXX:VPQAL:RSVK-AA$^{17}$-AKV:KEVQV:MIVRS:XKXS, ASASPXX:VSQDL:RSVK-AA$^{17}$-AKV:KEVQV:MIVKS:XKXS, ASASPXX:VPQDL:RSVK-AA$^{17}$-AKV:KEVQV:MVVKS:XKXS, NDEGLEX:VPTEE:RSVK-AA$^{17}$-AKV:KEVQV:FLQHN:KXEXR, NDEGLEX:VPTGQ:RSVK-AA$^{17}$-AKV:KEVQV:LEEHS:QXEXR, RNVQXRP:TQVQL:RSVK-AA$^{17}$-AKV:KEVQV:TLEDH:LAXKXE, KIPKAXX:TQVEL:RPVQ-AA$^{17}$-RKI:KKATV:DMEDH:GXGXR, GIPEPXX:TQVKM:RPVQ-AA$^{17}$-RKI:KKATV:NMEDH:SXAXR, SIPKAXX:TQVEL:RPVQ-AA$^{17}$-RKI:KKATV:EMEDH:GXGXR, HVTKPTX:TQVKL:RPVQ-AA$^{17}$-RKI:KKATV:NMEDH:SXGXH, YVPKPXX:TQVKL:RPVQ-AA$^{17}$-RKI:KKATV:NMEDH:SXGXH, TVPKPXX:TQVQL:RPVQ-AA$^{17}$-RKI:KKATV:NMEDH:AXGXH, AVPKAXX:TQVKL:RPVQ-AA$^{17}$-RKI:KKATV:NMEDH:AXGXH, KVGKAXX:TQVKL:RPVQ-AA$^{17}$-RKI:KKATV:EGMSEDH:XGXR, KASKAXX:TQVKL:RPVQ-AA$^{17}$-RKI:KKATV:GMEDH:EXGXR, GSAGPXX:TQVKM:RPVQ-AA$^{17}$-RKI:KKATV:GMEDH:RXGXS, AAPASXX:TQVRL:RPVQ-AA$^{17}$-RKI:KKATV:DMEDH:AXGXR, STPPTXX:TQVRL:RPVQ-AA$^{17}$-RKI:KKATV:DMEDH:SXGXR, HVPKPXX:TQVKL:RPVQ-AA$^{17}$-RKI:KKATV:NMEDH:SXGXH, RVPSTXX:TQVKT:RPVQ-AA$^{17}$-RKI:KKATV:MIEDH:XGXL, ASAAPXX:TQVAL:RPVQ-AA$^{17}$-RKI:KKATV:MIEDH:XKXS, ASASPXX:TQVDL:RPVQ-AA$^{17}$-RKI:KKATV:MIEDH:XKXS, ASASPXX:TQVDL:RPVQ-AA$^{17}$-RKI:KKATV:MVEDH:XKXS, NDEGLEX:TQVEE:RPVQ-AA$^{17}$-RKI:KKATV:FLEDH:KXEXR, NDEGLEX:TQVGQ:RPVQ-AA$^{17}$-RKI:KKATV:LEEDH:QXEXR, SSVKXQP:TQVHH:RPVQ-AA$^{17}$-RKI:KKATV:RLEDH:LEXAXA, KIPKAXX:VPTEL:RPVQ-AA$^{17}$-RKI:KKATV:DMVVE:GXGXR, GIPEPXX:VPEKM:RPVQ-AA$^{17}$-RKI:KKATV:NMTVE:SXAXR, SIPKAXX:VPTEL:RPVQ-AA$^{17}$-RKI:KKATV:EMVVE:GXGXR, HVTKPTX:APTKL:RPVQ-AA$^{17}$-RKI:KKATV:NMVVR:SXGXH, YVPKPXX:APTKL:RPVQ-AA$^{17}$-RKI:KKATV:NMVVR:SXGXH, TVPKPXX:APTQL:RPVQ-AA$^{17}$-RKI:KKATV:NMVVR:AXGXH, AVPKAXX:APTKL:RPVQ-AA$^{17}$-RKI:KKATV:NMVVK:AXGXH, KVGKAXX:VPTKL:RPVQ-AA$^{17}$-RKI:KKATV:EGMSVAE:XGXR, KASKAXX:VPTKL:RPVQ-AA$^{17}$-RKI:KKATV:GMAVS:EXGXR, GSAGPXX:TPTKM:RPVQ-AA$^{17}$-RKI:KKATV:GMVVD:RXGXS, AAPASXX:VPARL:RPVQ-AA$^{17}$-RKI:KKATV:DMVVE:AXGXR, STPPTXX:VPTRL:RPVQ-AA$^{17}$-RKI:KKATV:DMVVE:SXGXR, HVPKPXX:APTKL:RPVQ-AA$^{17}$-RKI:KKATV:NMVVR:SXGXH, RVPSTXX:APVKT:RPVQ-AA$^{17}$-RKI:KKATV:MIVEE:XGXL, ASAAPXX:VPQAL:RPVQ-AA$^{17}$-RKI:KKATV:MIVRS:XKXS, ASASPXX:VSQDL:RPVQ-AA$^{17}$-RKI:KKATV:MIVKS:XKXS, ASASPXX:VPQDL:RPVQ-AA$^{17}$-RKI:KKATV:MVVKS:XKXS, NDEGLEX:VPTEE:RPVQ-AA$^{17}$-RKI:KKATV:FLQHN:KXEXR, NDEGLEX:VPTGQ:RPVQ-AA$^{17}$-RKI:KKATV:LEEHS:QXEXR and SSVKXQP:SRVHH:RPVQ-AA$^{17}$-RKI:KKATV:RLEEH:LEXAXA; and wherein AA$^{17}$ is selected from the group consisting of G, A, V, L, I, P, F, M, W, T and S (in particular is selected from the group consisting of M, I, L, V and T). More particularly, the hexaplet PEP7:PEP5:PEP12:PEP2:PEP6:PEP8 is selected from the group consisting of GIPEPXX:VPTKM:SAIS-AA17-LYL:LKNYQ:NMVVE:SXAXR, HVTKPTX:VPTKL:SAIS-AA17-LYL:LKNYQ:NMVVE:SXGXH, YVPKPXX:VPTKL:SAIS-AA17-LYL:LKNYQ:NMVVE:SXGXH, TVPKPXX:VPTQL:SAIS-AA17-LYL:LKNYQ:NMVVE:AXGXH, AVPKAXX:VPTKL:SAIS-AA17-LYL:LKNYQ:NMVVE:AXGXH, KVGKAXX:VPTKL:SAIS-AA17-LYL:LKNYQ:EGMSVVE:XGXR, KASKAXX:VPTKL:SAIS-AA17-LYL:LKNYQ:GMVVE:EXGXR, GSAGPXX:VPTKM:SAIS-AA17-LYL:LKNYQ:GMVVE:RXGXS, AAPASXX:VPTRL:SAIS-AA17-LYL:LKNYQ:DMVVE:AXGXR, STPPTXX:VPTRL:SAIS-AA17-LYL:LKNYQ:DMVVE:SXGXR, HVPKPXX:VPTKL:SAIS-AA17-LYL:LKNYQ:NMVVE:SXGXH, RVPSTXX:VPTKT:SAIS-AA17-LYL:LKNYQ:MIVVE:XGXL, ASAAPXX:VPTAL:SAIS-AA17-LYL:LKNYQ:MIVVE:XKXS, ASASPXX:VPTDL:SAIS-AA17-LYL:LKNYQ:MIVVE:XKXS, ASASPXX:VPTDL:SAIS-AA17-LYL:LKNYQ:MVVVE:XKXS, GIPEPXX:VPEKM:SAIS-AA17-LYL:LKNYQ:NMTVE:SXAXR, HVTKPTX:APTKL:SAIS-AA17-LYL:LKNYQ:NMVVR:SXGXH, YVPKPXX:APTKL:SAIS-AA17-LYL:LKNYQ:NMVVR:SXGXH, TVPKPXX:APTQL:SAIS-AA17-LYL:LKNYQ:NMVVR:AXGXH, AVPKAXX:APTKL:SAIS-AA17-LYL:LKNYQ:NMVVK:AXGXH, KVGKAXX:VPTKL:SAIS-AA17-LYL:LKNYQ:EGMSVAE:XGXR, KASKAXX:VPTKL:SAIS-AA17-LYL:LKNYQ:GMAVS:EXGXR, GSAGPXX:TPTKM:SAIS-AA17-LYL:LKNYQ:GMVVD:RXGXS, AAPASXX:VPARL:SAIS-AA17-LYL:LKNYQ:DMVVE:AXGXR, HVPKPXX:APTKL:SAIS-AA17-LYL:LKNYQ:NMVVR:SXGXH, RVPSTXX:APVKT:SAIS-AA17-LYL:LKNYQ:MIVEE:XGXL, ASAAPXX:VPQAL:SAIS-AA17-LYL:LKNYQ:MIVRS:XKXS, ASASPXX:VSQDL:SAIS-AA17-LYL:LKNYQ:MIVKS:XKXS, ASASPXX:VPQDL:SAIS-AA17-LYL:LKNYQ:MVVKS:XKXS, NDEGLEX:VPTEE:SAIS-AA17-LYL:LKNYQ:FLQHN:KXEXR, NDEGLEX:VPTGQ:SAIS-AA17-LYL:LKNYQ:LEEHS:QXEXR, SSVKXQP:SRVHH:SAIS-AA17-LYL:LKNYQ:RLEEH:LEXAXA, RNVQXRP:TQVQL:SAIS-AA17-LYL:LKNYQ:TLEDH:LAXKXE, KIPKAXX:VPEEL:SSLS-AA17-LFF:LKVYP:DMTVE:GXGXR, SIPKAXX:VPEEL:SSLS-AA17-LFF:LKVYP:EMTVE:GXGXR, HVTKPTX:VPEKL:SSLS-AA17-LFF:LKVYP:NMTVE:SXGXH, YVPKPXX:VPEKL:SSLS-AA17-LFF:LKVYP:NMTVE:SXGXH, TVPKPXX:VPEQL:SSLS-AA17-LFF:LKVYP:NMTVE:AXGXH, AVPKAXX:VPEKL:SSLS-AA17-LFF:LKVYP:NMTVE:AXGXH, KVGKAXX:VPEKL:SSLS-AA17-LFF:LKVYP:EGMSTVE:XGXR, KASKAXX:VPEKL:SSLS-AA17-LFF:LKVYP:GMTVE:EXGXR, GSAGPXX:VPEKM:SSLS-AA17-LFF:LKVYP:GMTVE:RXGXS, AAPASXX:VPERL:SSLS-AA17-LFF:LKVYP:DMTVE:AXGXR, STPPTXX:VPERL:SSLS-AA17-LFF:LKVYP:DMTVE:SXGXR, HVPKPXX:VPEKL:SSLS-AA17-LFF:LKVYP:NMTVE:SXGXH, RVPSTXX:VPEKT:SSLS-AA17-LFF:LKVYP:MITVE:XGXL, ASAAPXX:VPEAL:SSLS-AA17-LFF:LKVYP:MITVE:XKXS, ASASPXX:VPEDL:SSLS-AA17-LFF:LKVYP:MITVE:XKXS, ASASPXX:VPEDL:SSLS-AA17-LFF:LKVYP:MVTVE:XKXS, KIPKAXX:VPTEL:SSLS-AA17-LFF:LKVYP:DMVVE:GXGXR, SIPKAXX:VPTEL:SSLS-AA17-LFF:LKVYP:EMVVE:GXGXR, HVTKPTX:APTKL:SSLS-AA17-LFF:LKVYP:NMVVR:SXGXH, YVPKPXX:APTKL:SSLS-AA17-LFF:LKVYP:NMVVR:SXGXH, TVPKPXX:APTQL:SSLS-AA17-LFF:LKVYP:NMVVR:AXGXH, AVPKAXX:APTKL:SSLS-AA17-LFF:LKVYP:NMVVK:AXGXH, KVGKAXX:VPTKL:SSLS-AA17-LFF:LKVYP:EGMSVAE:XGXR, KASKAXX:VPTKL:SSLS-AA17-LFF:LKVYP:GMAVS:EXGXR, GSAGPXX:TPTKM:SSLS-AA17-LFF:LKVYP:GMVVD:RXGXS, AAPASXX:VPARL:SSLS-AA17-LFF:LKVYP:DMVVE:AXGXR, STPPTXX:VPTRL:SSLS-AA17-LFF:LKVYP:DMVVE:SXGXR, HVPKPXX:APTKL:SSLS-AA17-LFF:LKVYP:NMVVR:SXGXH, RVPSTXX:APVKT:SSLS-AA17-LFF:LKVYP:MIVEE:XGXL, ASAAPXX:VPQAL:SSLS-AA17-LFF:LKVYP:MIVRS:XKXS, ASASPXX:VSQDL:SSLS-AA17-LFF:LKVYP:MIVKS:XKXS, ASASPXX:VPQDL:SSLS-AA17-LFF:LKVYP:MVVKS:XKXS, NDEGLEX:VPTEE:SSLS-AA17-LFF:LKVYP:FLQHN:KXEXR, NDEGLEX:VPTGQ:SSLS-AA17-LFF:LKVYP:LEEHS:QXEXR, SSVKXQP:SRVHH:SSLS-AA17-LFF:LKVYP:RLEEH:LEXAXA, RNVQXRP:TQVQL:SSLS-AA17-LFF:LKVYP:TLEDH:LAXKXE, KIPKAXX:APTEL:NAIS-AA17-LYF:LKKYR:DMVVR:GXGXR, GIPEPXX:APTKM:NAIS-AA17-LYF:LKKYR:NMVVR:SXAXR, SIPKAXX:APTEL:NAIS-AA17-LYF:LKKYR:EMVVR:GXGXR, AVPKAXX:APTKL:NAIS-AA17-LYF:LKKYR:NMVVR:AXGXH, KVGKAXX:APTKL:NAIS-AA17-LYF:LKKYR:EGMSVVR:XGXR, KASKAXX:APTKL:NAIS-AA17-LYF:LKKYR:GMVVR:EXGXR, GSAGPXX:APTKM:NAIS-AA17-LYF:LKKYR:GMVVR:RXGXS, AAPASXX:APTRL:NAIS-AA17-LYF:LKKYR:DMVVR:AXGXR, STPPTXX:APTRL:NAIS-AA17-LYF:LKKYR:DMVVR:SXGXR, RVPSTXX:APTKT:NAIS-AA17-LYF:LKKYR:MIVVR:XGXL, ASAAPXX:APTAL:NAIS-AA17-LYF:LKKYR:MIVVR:XKXS, ASASPXX:APTDL:NAIS-AA17-LYF:LKKYR:MIVVR:XKXS, ASASPXX:APTDL:NAIS-AA17-LYF:LKKYR:MVVVR:XKXS, KIPKAXX:VPTEL:NAIS-AA17-LYF:LKKYR:DMVVE:GXGXR, GIPEPXX:VPEKM:NAIS-AA17-LYF:LKKYR:NMTVE:SXAXR, SIPKAXX:VPTEL:NAIS-AA17-LYF:LKKYR:EMVVE:GXGXR, AVPKAXX:APTKL:NAIS-AA17-LYF:LKKYR:NMVVK:AXGXH, KVGKAXX:VPTKL:NAIS-AA17-LYF:LKKYR:EGMSVAE:XGXR, KASKAXX:VPTKL:NAIS-AA17-LYF:LKKYR:GMAVS:EXGXR, GSAGPXX:TPTKM:NAIS-AA17-LYF:LKKYR:GMVVD:RXGXS, AAPASXX:VPARL:NAIS-AA17-LYF:LKKYR:DMVVE:AXGXR, STPPTXX:VPTRL:NAIS-AA17-LYF:LKKYR:DMVVE:SXGXR, RVPSTXX:APVKT:NAIS-AA17-LYF:LKKYR:MIVEE:XGXL, ASAAPXX:VPQAL:NAIS-AA17-LYF:LKKYR:MIVRS:XKXS, ASASPXX:VSQDL:NAIS-AA17-LYF:LKKYR:MIVKS:XKXS, ASASPXX:VPQDL:NAIS-AA17-LYF:LKKYR:MVVKS:XKXS, NDEGLEX:VPTEE:NAIS-AA17-LYF:LKKYR:FLQHN:KXEXR, NDEGLEX:VPTGQ:NAIS-AA17-LYF:LKKYR:LEEHS:QXEXR, SSVKXQP:SRVHH:NAIS-AA17-LYF:LKKYR:RLEEH:LEXAXA, RNVQXRP:TQVQL:NAIS-AA17-LYF:LKKYR:TLEDH:LAXKXE, KIPKAXX:APTEL:SATS-AA17-LYY:LRKHR:DMVVK:GXGXR, GIPEPXX:APTKM:SATS-AA17-LYY:LRKHR:NMVVK:SXAXR, SIPKAXX:APTEL:SATS-AA17-LYY:LRKHR:EMVVK:GXGXR, HVTKPTX:APTKL:SATS-AA17-LYY:LRKHR:NMVVK:SXGXH, YVPKPXX:APTKL:SATS-AA17-LYY:LRKHR:NMVVK:SXGXH, TVPKPXX:APTQL:SATS-AA17-LYY:LRKHR:NMVVK:AXGXH, KVGKAXX:APTKL:SATS-AA17-LYY:LRKHR:EGMSVVK:XGXR, KASKAXX:APTKL:SATS-AA17-LYY:LRKHR:GMVVK:EXGXR, GSAGPXX:APTKM:SATS-AA17-LYY:LRKHR:GMVVK:RXGXS, AAPASXX:APTRL:SATS-AA17-LYY:LRKHR:DMVVK:AXGXR, STPPTXX:APTRL:SATS-AA17-LYY:LRKHR:DMVVK:SXGXR, HVPKPXX:APTKL:SATS-AA17-LYY:LRKHR:NMVVK:SXGXH, RVPSTXX:APTKT:SATS-AA17-LYY:LRKHR:MIVVK:XGXL, ASAAPXX:APTAL:SATS-AA17-LYY:LRKHR:MIVVK:XKXS, ASASPXX:APTDL:SATS-AA17-LYY:LRKHR:MIVVK:XKXS, ASASPXX:APTDL:SATS-AA17-LYY:LRKHR:MVVVK:XKXS, KIPKAXX:VPTEL:SATS-AA17-LYY:LRKHR:DMVVE:GXGXR, GIPEPXX:VPEKM:SATS-AA17-LYY:LRKHR:NMTVE:SXAXR, SIPKAXX:VPTEL:SATS-AA17-LYY:LRKHR:EMVVE:GXGXR, HVTKPTX:APTKL:SATS-AA17-LYY:LRKHR:NMVVR:SXGXH, YVPKPXX:APTKL:SATS-AA17-LYY:LRKHR:NMVVR:SXGXH, TVPKPXX:APTQL:SATS-AA17-LYY:LRKHR:NMVVR:AXGXH, KVGKAXX:VPTKL:SATS-AA17-LYY:LRKHR:EGMSVAE:XGXR, KASKAXX:VPTKL:SATS-AA17-LYY:LRKHR:GMAVS:EXGXR, GSAGPXX:TPTKM:SATS-AA17-LYY:LRKHR:GMVVD:RXGXS, AAPASXX:VPARL:SATS-AA17-LYY:LRKHR:DMVVE:AXGXR, STPPTXX:VPTRL:SATS-AA17-LYY:LRKHR:DMVVE:SXGXR, HVPKPXX:APTKL:SATS-AA17-LYY:LRKHR:NMVVR:SXGXH, RVPSTXX:APVKT:SATS-AA17-LYY:LRKHR:MIVEE:XGXL, ASAAPXX:VPQAL:SATS-AA17-LYY:LRKHR:MIVRS:XKXS, ASASPXX:VSQDL:SATS-AA17-LYY:LRKHR:MIVKS:XKXS, ASASPXX:VPQDL:SATS-AA17-LYY:LRKHR:MVVKS:XKXS, NDEGLEX:VPTEE:SATS-AA17-LYY:LRKHR:FLQHN:KXEXR, NDEGLEX:VPTGQ:SATS-AA17-LYY:LRKHR:LEEHS:QXEXR, SSVKXQP:SRVHH:SATS-AA17-LYY:LRKHR:RLEEH:LEXAXA, RNVQXRP:TQVQL:SATS-AA17-LYY:LRKHR:TLEDH:LAXKXE, KIPKAXX:VPTEL:SPIS-AA17-LYK:LKYHY:DMVAE:GXGXR, GIPEPXX:VPTKM:SPIS-AA17-LYK:LKYHY:NMVAE:SXAXR, SIPKAXX:VPTEL:SPIS-AA17-LYK:LKYHY:EMVAE:GXGXR, HVTKPTX:VPTKL:SPIS-AA17-LYK:LKYHY:NMVAE:SXGXH, YVPKPXX:VPTKL:SPIS-AA17-LYK:LKYHY:NMVAE:SXGXH, TVPKPXX:VPTQL:SPIS-AA17-LYK:LKYHY:NMVAE:AXGXH, AVPKAXX:VPTKL:SPIS-AA17-LYK:LKYHY:NMVAE:AXGXH, KASKAXX:VPTKL:SPIS-AA17-LYK:LKYHY:GMVAE:EXGXR, GSAGPXX:VPTKM:SPIS-AA17-LYK:LKYHY:GMVAE:RXGXS, AAPASXX:VPTRL:SPIS-AA17-LYK:LKYHY:DMVAE:AXGXR, STPPTXX:VPTRL:SPIS-AA17-LYK:LKYHY:DMVAE:SXGXR, HVPKPXX:VPTKL:SPIS-AA17-LYK:LKYHY:NMVAE:SXGXH, RVPSTXX:VPTKT:SPIS-AA17-LYK:LKYHY:MIVAE:XGXL, ASAAPXX:VPTAL:SPIS-AA17-LYK:LKYHY:MIVAE:XKXS, ASASPXX:VPTDL:SPIS-AA17-LYK:LKYHY:MIVAE:XKXS, ASASPXX:VPTDL:SPIS-AA17-LYK:LKYHY:MVVAE:XKXS, KIPKAXX:VPTEL:SPIS-AA17-LYK:LKYHY:DMVVE:GXGXR, GIPEPXX:VPEKM:SPIS-AA17-LYK:LKYHY:NMTVE:SXAXR, SIPKAXX:VPTEL:SPIS-AA17-LYK:LKYHY:EMVVE:GXGXR, HVTKPTX:APTKL:SPIS-AA17-LYK:LKYHY:NMVVR:SXGXH, YVPKPXX:APTKL:SPIS-AA17-LYK:LKYHY:NMVVR:SXGXH, TVPKPXX:APTQL:SPIS-AA17-LYK:LKYHY:NMVVR:AXGXH, AVPKAXX:APTKL:SPIS-AA17-LYK:LKYHY:NMVVK:AXGXH, KASKAXX:VPTKL:SPIS-AA17-LYK:LKYHY:GMAVS:EXGXR, GSAGPXX:TPTKM:SPIS-AA17-LYK:LKYHY:GMVVD:RXGXS, AAPASXX:VPARL:SPIS-AA17-LYK:LKYHY:DMVVE:AXGXR, STPPTXX:VPTRL:SPIS-AA17-LYK:LKYHY:DMVVE:SXGXR, HVPKPXX:APTKL:SPIS-AA17-LYK:LKYHY:NMVVR:SXGXH, RVPSTXX:APVKT:SPIS-AA17-LYK:LKYHY:MIVEE:XGXL, ASAAPXX:VPQAL:SPIS-AA17-LYK:LKYHY:MIVRS:XKXS, ASASPXX:VSQDL:SPIS-AA17-LYK:LKYHY:MIVKS:XKXS, ASASPXX:VPQDL:SPIS-AA17-LYK:LKYHY:MVVKS:XKXS, NDEGLEX:VPTEE:SPIS-AA17-LYK:LKYHY:FLQHN:KXEXR, NDEGLEX:VPTGQ:SPIS-AA17-LYK:LKYHY:LEEHS:QXEXR, SSVKXQP:SRVHH:SPIS-AA17-LYK:LKYHY:RLEEH:LEXAXA, RNVQXRP:TQVQL:SPIS-AA17-LYK:LKYHY:TLEDH:LAXKXE, KIPKAXX:VPTEL:EPIS-AA17-LYL:KFKYE:DMAVS:GXGXR, GIPEPXX:VPTKM:EPIS-AA17-LYL:KFKYE:NMAVS:SXAXR, SIPKAXX:VPTEL:EPIS-AA17-LYL:KFKYE:EMAVS:GXGXR, HVTKPTX:VPTKL:EPIS-AA17-LYL:KFKYE:NMAVS:SXGXH, YVPKPXX:VPTKL:EPIS-AA17-LYL:KFKYE:NMAVS:SXGXH, TVPKPXX:VPTQL:EPIS-AA17-LYL:KFKYE:NMAVS:AXGXH, AVPKAXX:VPTKL:EPIS-AA17-LYL:KFKYE:NMAVS:AXGXH, KVGKAXX:VPTKL:EPIS-AA17-LYL:KFKYE:EGMSAVS:XGXR, GSAGPXX:VPTKM:EPIS-AA17-LYL:KFKYE:GMAVS:RXGXS, AAPASXX:VPTRL:EPIS-AA17-LYL:KFKYE:DMAVS:AXGXR, STPPTXX:VPTRL:EPIS-AA17-LYL:KFKYE:DMAVS:SXGXR, HVPKPXX:VPTKL:EPIS-AA17-LYL:KFKYE:NMAVS:SXGXH, RVPSTXX:VPTKT:EPIS-AA17-LYL:KFKYE:MIAVS:XGXL, ASAAPXX:VPTAL:EPIS-AA17-LYL:KFKYE:MIAVS:XKXS, ASASPXX:VPTDL:EPIS-AA17-LYL:KFKYE:MIAVS:XKXS, ASASPXX:VPTDL:EPIS-AA17-LYL:KFKYE:MVAVS:XKXS, KIPKAXX:VPTEL:EPIS-AA17-LYL:KFKYE:DMVVE:GXGXR, GIPEPXX:VPEKM:EPIS-AA17-LYL:KFKYE:NMTVE:SXAXR, SIPKAXX:VPTEL:EPIS-AA17-LYL:KFKYE:EMVVE:GXGXR, HVTKPTX:APTKL:EPIS-AA17-LYL:KFKYE:NMVVR:SXGXH, YVPKPXX:APTKL:EPIS-AA17-LYL:KFKYE:NMVVR:SXGXH, TVPKPXX:APTQL:EPIS-AA17-LYL:KFKYE:NMVVR:AXGXH, AVPKAXX:APTKL:EPIS-AA17-LYL:KFKYE:NMVVK:AXGXH, KVGKAXX:VPTKL:EPIS-AA17-LYL:KFKYE:EGMSVAE:XGXR, GSAGPXX:TPTKM:EPIS-AA17-LYL:KFKYE:GMVVD:RXGXS, AAPASXX:VPARL:EPIS-AA17-LYL:KFKYE:DMVVE:AXGXR, STPPTXX:VPTRL:EPIS-AA17-LYL:KFKYE:DMVVE:SXGXR, HVPKPXX:APTKL:EPIS-AA17-LYL:KFKYE:NMVVR:SXGXH, RVPSTXX:APVKT:EPIS-AA17-LYL:KFKYE:MIVEE:XGXL, ASAAPXX:VPQAL:EPIS-AA17-LYL:KFKYE:MIVRS:XKXS, ASASPXX:VSQDL:EPIS-AA17-LYL:KFKYE:MIVKS:XKXS, ASASPXX:VPQDL:EPIS-AA17-LYL:KFKYE:MVVKS:XKXS, NDEGLEX:VPTEE:EPIS-AA17-LYL:KFKYE:FLQHN:KXEXR, NDEGLEX:VPTGQ:EPIS-AA17-LYL:KFKYE:LEEHS:QXEXR, SSVKXQP:SRVHH:EPIS-AA17-LYL:KFKYE:RLEEH:LEXAXA, RNVQXRP:TQVQL:EPIS-AA17-LYL:KFKYE:TLEDH:LAXKXE, KIPKAXX:TPTEL:SPIN-AA17-LYF:YGKIP:DMVVD:GXGXR, GIPEPXX:TPTKM:SPIN-AA17-LYF:YGKIP:NMVVD:SXAXR, SIPKAXX:TPTEL:SPIN-AA17-LYF:YGKIP:EMVVD:GXGXR, HVTKPTX:TPTKL:SPIN-AA17-LYF:YGKIP:NMVVD:SXGXH, YVPKPXX:TPTKL:SPIN-AA17-LYF:YGKIP:NMVVD:SXGXH, TVPKPXX:TPTQL:SPIN-AA17-LYF:YGKIP:NMVVD:AXGXH, AVPKAXX:TPTKL:SPIN-AA17-LYF:YGKIP:NMVVD:AXGXH, KVGKAXX:TPTKL:SPIN-AA17-LYF:YGKIP:EGMSVVD:XGXR, KASKAXX:TPTKL:SPIN-AA17-LYF:YGKIP:GMVVD:EXGXR, AAPASXX:TPTRL:SPIN-AA17-LYF:YGKIP:DMVVD:AXGXR, STPPTXX:TPTRL:SPIN-AA17-LYF:YGKIP:DMVVD:SXGXR, HVPKPXX:TPTKL:SPIN-AA17-LYF:YGKIP:NMVVD:SXGXH, RVPSTXX:TPTKT:SPIN-AA17-LYF:YGKIP:MIVVD:XGXL, ASAAPXX:TPTAL:SPIN-AA17-LYF:YGKIP:MIVVD:XKXS, ASASPXX:TPTDL:SPIN-AA17-LYF:YGKIP:MIVVD:XKXS, ASASPXX:TPTDL:SPIN-AA17-LYF:YGKIP:MVVVD:XKXS, KIPKAXX:VPTEL:SPIN-AA17-LYF:YGKIP:DMVVE:GXGXR, GIPEPXX:VPEKM:SPIN-AA17-LYF:YGKIP:NMTVE:SXAXR, SIPKAXX:VPTEL:SPIN-AA17-LYF:YGKIP:EMVVE:GXGXR, HVTKPTX:APTKL:SPIN-AA17-LYF:YGKIP:NMVVR:SXGXH, YVPKPXX:APTKL:SPIN-AA17-LYF:YGKIP:NMVVR:SXGXH, TVPKPXX:APTQL:SPIN-AA17-LYF:YGKIP:NMVVR:AXGXH, AVPKAXX:APTKL:SPIN-AA17-LYF:YGKIP:NMVVK:AXGXH, KVGKAXX:VPTKL:SPIN-AA17-LYF:YGKIP:EGMSVAE:XGXR, KASKAXX:VPTKL:SPIN-AA17-LYF:YGKIP:GMAVS:EXGXR, AAPASXX:VPARL:SPIN-AA17-LYF:YGKIP:DMVVE:AXGXR, STPPTXX:VPTRL:SPIN-AA17-LYF:YGKIP:DMVVE:SXGXR, HVPKPXX:APTKL:SPIN-AA17-LYF:YGKIP:NMVVR:SXGXH, RVPSTXX:APVKT:SPIN-AA17-LYF:YGKIP:MIVEE:XGXL, ASAAPXX:VPQAL:SPIN-AA17-LYF:YGKIP:MIVRS:XKXS, ASASPXX:VSQDL:SPIN-AA17-LYF:YGKIP:MIVKS:XKXS, ASASPXX:VPQDL:SPIN-AA17-LYF:YGKIP:MVVKS:XKXS, NDEGLEX:VPTEE:SPIN-AA17-LYF:YGKIP:FLQHN:KXEXR, NDEGLEX:VPTGQ:SPIN-AA17-LYF:YGKIP:LEEHS:QXEXR, SSVKXQP:SRVHH:SPIN-AA17-LYF:YGKIP:RLEEH:LEXAXA, RNVQXRP:TQVQL:SPIN-AA17-LYF:YGKIP:TLEDH:LAXKXE, KIPKAXX:VPAEL:SPIS-AA17-LYI:YKQYE:DMVVE:GXGXR, GIPEPXX:VPAKM:SPIS-AA17-LYI:YKQYE:NMVVE:SXAXR, SIPKAXX:VPAEL:SPIS-AA17-LYI:YKQYE:EMVVE:GXGXR, HVTKPTX:VPAKL:SPIS-AA17-LYI:YKQYE:NMVVE:SXGXH, YVPKPXX:VPAKL:SPIS-AA17-LYI:YKQYE:NMVVE:SXGXH, TVPKPXX:VPAQL:SPIS-AA17-LYI:YKQYE:NMVVE:AXGXH, AVPKAXX:VPAKL:SPIS-AA17-LYI:YKQYE:NMVVE:AXGXH, KVGKAXX:VPAKL:SPIS-AA17-LYI:YKQYE:EGMSVVE:XGXR, KASKAXX:VPAKL:SPIS-AA17-LYI:YKQYE:GMVVE:EXGXR, GSAGPXX:VPAKM:SPIS-AA17-LYI:YKQYE:GMVVE:RXGXS, STPPTXX:VPARL:SPIS-AA17-LYI:YKQYE:DMVVE:SXGXR, HVPKPXX:VPAKL:SPIS-AA17-LYI:YKQYE:NMVVE:SXGXH, RVPSTXX:VPAKT:SPIS-AA17-LYI:YKQYE:MIVVE:XGXL, ASAAPXX:VPAAL:SPIS-AA17-LYI:YKQYE:MIVVE:XKXS, ASASPXX:VPADL:SPIS-AA17-LYI:YKQYE:MIVVE:XKXS, ASASPXX:VPADL:SPIS-AA17-LYI:YKQYE:MVVVE:XKXS, KIPKAXX:VPTEL:SPIS-AA17-LYI:YKQYE:DMVVE:GXGXR, GIPEPXX:VPEKM:SPIS-AA17-LYI:YKQYE:NMTVE:SXAXR, SIPKAXX:VPTEL:SPIS-AA17-LYI:YKQYE:EMVVE:GXGXR, HVTKPTX:APTKL:SPIS-AA17-LYI:YKQYE:NMVVR:SXGXH, YVPKPXX:APTKL:SPIS-AA17-LYI:YKQYE:NMVVR:SXGXH, TVPKPXX:APTQL:SPIS-AA17-LYI:YKQYE:NMVVR:AXGXH, AVPKAXX:APTKL:SPIS-AA17-LYI:YKQYE:NMVVK:AXGXH, KVGKAXX:VPTKL:SPIS-AA17-LYI:YKQYE:EGMSVAE:XGXR, KASKAXX:VPTKL:SPIS-AA17-LYI:YKQYE:GMAVS:EXGXR, GSAGPXX:TPTKM:SPIS-AA17-LYI:YKQYE:GMVVD:RXGXS, STPPTXX:VPTRL:SPIS-AA17-LYI:YKQYE:DMVVE:SXGXR, HVPKPXX:APTKL:SPIS-AA17-LYI:YKQYE:NMVVR:SXGXH, RVPSTXX:APVKT:SPIS-AA17-LYI:YKQYE:MIVEE:XGXL, ASAAPXX:VPQAL:SPIS-AA17-LYI:YKQYE:MIVRS:XKXS, ASASPXX:VSQDL:SPIS-AA17-LYI:YKQYE:MIVKS:XKXS, ASASPXX:VPQDL:SPIS-AA17-LYI:YKQYE:MVVKS:XKXS, NDEGLEX:VPTEE:SPIS-AA17-LYI:YKQYE:FLQHN:KXEXR, NDEGLEX:VPTGQ:SPIS-AA17-LYI:YKQYE:LEEHS:QXEXR, SSVKXQP:SRVHH:SPIS-AA17-LYI:YKQYE:RLEEH:LEXAXA, RNVQXRP:TQVQL:SPIS-AA17-LYI:YKQYE:TLEDH:LAXKXE, KIPKAXX:VPTEL:SPIS-AA17-LFI:YKQYE:DMVVE:GXGXR, GIPEPXX:VPTKM:SPIS-AA17-LFI:YKQYE:NMVVE:SXAXR, SIPKAXX:VPTEL:SPIS-AA17-LFI:YKQYE:EMVVE:GXGXR, HVTKPTX:VPTKL:SPIS-AA17-LFI:YKQYE:NMVVE:SXGXH, YVPKPXX:VPTKL:SPIS-AA17-LFI:YKQYE:NMVVE:SXGXH, TVPKPXX:VPTQL:SPIS-AA17-LFI:YKQYE:NMVVE:AXGXH, AVPKAXX:VPTKL:SPIS-AA17-LFI:YKQYE:NMVVE:AXGXH, KVGKAXX:VPTKL:SPIS-AA17-LFI:YKQYE:EGMSVVE:XGXR, KASKAXX:VPTKL:SPIS-AA17-LFI:YKQYE:GMVVE:EXGXR, GSAGPXX:VPTKM:SPIS-AA17-LFI:YKQYE:GMVVE:RXGXS, AAPASXX:VPTRL:SPIS-AA17-LFI:YKQYE:DMVVE:AXGXR, HVPKPXX:VPTKL:SPIS-AA17-LFI:YKQYE:NMVVE:SXGXH, RVPSTXX:VPTKT:SPIS-AA17-LFI:YKQYE:MIVVE:XGXL, ASAAPXX:VPTAL:SPIS-AA17-LFI:YKQYE:MIVVE:XKXS, ASASPXX:VPTDL:SPIS-AA17-LFI:YKQYE:MIVVE:XKXS, ASASPXX:VPTDL:SPIS-AA17-LFI:YKQYE:MVVVE:XKXS, GIPEPXX:VPEKM:SPIS-AA17-LFI:YKQYE:NMTVE:SXAXR, HVTKPTX:APTKL:SPIS-AA17-LFI:YKQYE:NMVVR:SXGXH, YVPKPXX:APTKL:SPIS-AA17-LFI:YKQYE:NMVVR:SXGXH, TVPKPXX:APTQL:SPIS-AA17-LFI:YKQYE:NMVVR:AXGXH, AVPKAXX:APTKL:SPIS-AA17-LFI:YKQYE:NMVVK:AXGXH, KVGKAXX:VPTKL:SPIS-AA17-LFI:YKQYE:EGMSVAE:XGXR, KASKAXX:VPTKL:SPIS-AA17-LFI:YKQYE:GMAVS:EXGXR, GSAGPXX:TPTKM:SPIS-AA17-LFI:YKQYE:GMVVD:RXGXS, AAPASXX:VPARL:SPIS-AA17-LFI:YKQYE:DMVVE:AXGXR, HVPKPXX:APTKL:SPIS-AA17-LFI:YKQYE:NMVVR:SXGXH, RVPSTXX:APVKT:SPIS-AA17-LFI:YKQYE:MIVEE:XGXL, ASAAPXX:VPQAL:SPIS-AA17-LFI:YKQYE:MIVRS:XKXS, ASASPXX:VSQDL:SPIS-AA17-LFI:YKQYE:MIVKS:XKXS, ASASPXX:VPQDL:SPIS-AA17-LFI:YKQYE:MVVKS:XKXS, NDEGLEX:VPTEE:SPIS-AA17-LFI:YKQYE:FLQHN:KXEXR, NDEGLEX:VPTGQ:SPIS-AA17-LFI:YKQYE:LEEHS:QXEXR, SSVKXQP:SRVHH:SPIS-AA17-LFI:YKQYE:RLEEH:LEXAXA, RNVQXRP:TQVQL:SPIS-AA17-LFI:YKQYE:TLEDH:LAXKXE, KIPKAXX:APVEL:KPLS-AA17-LYV:DHHKD:DMVEE:GXGXR, GIPEPXX:APVKM:KPLS-AA17-LYV:DHHKD:NMVEE:SXAXR, SIPKAXX:APVEL:KPLS-AA17-LYV:DHHKD:EMVEE:GXGXR, HVTKPTX:APVKL:KPLS-AA17-LYV:DHHKD:NMVEE:SXGXH, YVPKPXX:APVKL:KPLS-AA17-LYV:DHHKD:NMVEE:SXGXH, TVPKPXX:APVQL:KPLS-AA17-LYV:DHHKD:NMVEE:AXGXH, AVPKAXX:APVKL:KPLS-AA17-LYV:DHHKD:NMVEE:AXGXH, KVGKAXX:APVKL:KPLS-AA17-LYV:DHHKD:EGMSVEE:XGXR, KASKAXX:APVKL:KPLS-AA17-LYV:DHHKD:GMVEE:EXGXR, GSAGPXX:APVKM:KPLS-AA17-LYV:DHHKD:GMVEE:RXGXS, AAPASXX:APVRL:KPLS-AA17-LYV:DHHKD:DMVEE:AXGXR, STPPTXX:APVRL:KPLS-AA17-LYV:DHHKD:DMVEE:SXGXR, HVPKPXX:APVKL:KPLS-AA17-LYV:DHHKD:NMVEE:SXGXH, ASAAPXX:APVAL:KPLS-AA17-LYV:DHHKD:MIVEE:XKXS, ASASPXX:APVDL:KPLS-AA17-LYV:DHHKD:MIVEE:XKXS, ASASPXX:APVDL:KPLS-AA17-LYV:DHHKD:MVVEE:XKXS, KIPKAXX:VPTEL:KPLS-AA17-LYV:DHHKD:DMVVE:GXGXR, GIPEPXX:VPEKM:KPLS-AA17-LYV:DHHKD:NMTVE:SXAXR, SIPKAXX:VPTEL:KPLS-AA17-LYV:DHHKD:EMVVE:GXGXR, HVTKPTX:APTKL:KPLS-AA17-LYV:DHHKD:NMVVR:SXGXH, YVPKPXX:APTKL:KPLS-AA17-LYV:DHHKD:NMVVR:SXGXH, TVPKPXX:APTQL:KPLS-AA17-LYV:DHHKD:NMVVR:AXGXH, AVPKAXX:APTKL:KPLS-AA17-LYV:DHHKD:NMVVK:AXGXH, KVGKAXX:VPTKL:KPLS-AA17-LYV:DHHKD:EGMSVAE:XGXR, KASKAXX:VPTKL:KPLS-AA17-LYV:DHHKD:GMAVS:EXGXR, GSAGPXX:TPTKM:KPLS-AA17-LYV:DHHKD:GMVVD:RXGXS, AAPASXX:VPARL:KPLS-AA17-LYV:DHHKD:DMVVE:AXGXR, STPPTXX:VPTRL:KPLS-AA17-LYV:DHHKD:DMVVE:SXGXR, HVPKPXX:APTKL:KPLS-AA17-LYV:DHHKD:NMVVR:SXGXH, ASAAPXX:VPQAL:KPLS-AA17-LYV:DHHKD:MIVRS:XKXS, ASASPXX:VSQDL:KPLS-AA17-LYV:DHHKD:MIVKS:XKXS, ASASPXX:VPQDL:KPLS-AA17-LYV:DHHKD:MVVKS:XKXS, NDEGLEX:VPTEE:KPLS-AA17-LYV:DHHKD:FLQHN:KXEXR, NDEGLEX:VPTGQ:KPLS-AA17-LYV:DHHKD:LEEHS:QXEXR, SSVKXQP:SRVHH:KPLS-AA17-LYV:DHHKD:RLEEH:LEXAXA, RNVQXRP:TQVQL:KPLS-AA17-LYV:DHHKD:TLEDH:LAXKXE, KIPKAXX:VPQEL:EPLP-AA17-VYY:EQLSN:DMVRS:GXGXR, GIPEPXX:VPQKM:EPLP-AA17-VYY:EQLSN:NMVRS:SXAXR, SIPKAXX:VPQEL:EPLP-AA17-VYY:EQLSN:EMVRS:GXGXR, HVTKPTX:VPQKL:EPLP-AA17-VYY:EQLSN:NMVRS:SXGXH, YVPKPXX:VPQKL:EPLP-AA17-VYY:EQLSN:NMVRS:SXGXH, TVPKPXX:VPQQL:EPLP-AA17-VYY:EQLSN:NMVRS:AXGXH, AVPKAXX:VPQKL:EPLP-AA17-VYY:EQLSN:NMVRS:AXGXH, KVGKAXX:VPQKL:EPLP-AA17-VYY:EQLSN:EGMSVRS:XGXR, KASKAXX:VPQKL:EPLP-AA17-VYY:EQLSN:GMVRS:EXGXR, GSAGPXX:VPQKM:EPLP-AA17-VYY:EQLSN:GMVRS:RXGXS, AAPASXX:VPQRL:EPLP-AA17-VYY:EQLSN:DMVRS:AXGXR, STPPTXX:VPQRL:EPLP-AA17-VYY:EQLSN:DMVRS:SXGXR, HVPKPXX:VPQKL:EPLP-AA17-VYY:EQLSN:NMVRS:SXGXH, RVPSTXX:VPQKT:EPLP-AA17-VYY:EQLSN:MIVRS:XGXL, ASASPXX:VPQDL:EPLP-AA17-VYY:EQLSN:MIVRS:XKXS, ASASPXX:VPQDL:EPLP-AA17-VYY:EQLSN:MVVRS:XKXS, KIPKAXX:VPTEL:EPLP-AA17-VYY:EQLSN:DMVVE:GXGXR, GIPEPXX:VPEKM:EPLP-AA17-VYY:EQLSN:NMTVE:SXAXR, SIPKAXX:VPTEL:EPLP-AA17-VYY:EQLSN:EMVVE:GXGXR, HVTKPTX:APTKL:EPLP-AA17-VYY:EQLSN:NMVVR:SXGXH, YVPKPXX:APTKL:EPLP-AA17-VYY:EQLSN:NMVVR:SXGXH, TVPKPXX:APTQL:EPLP-AA17-VYY:EQLSN:NMVVR:AXGXH, AVPKAXX:APTKL:EPLP-AA17-VYY:EQLSN:NMVVK:AXGXH, KVGKAXX:VPTKL:EPLP-AA17-VYY:EQLSN:EGMSVAE:XGXR, KASKAXX:VPTKL:EPLP-AA17-VYY:EQLSN:GMAVS:EXGXR, GSAGPXX:TPTKM:EPLP-AA17-VYY:EQLSN:GMVVD:RXGXS, AAPASXX:VPARL:EPLP-AA17-VYY:EQLSN:DMVVE:AXGXR, STPPTXX:VPTRL:EPLP-AA17-VYY:EQLSN:DMVVE:SXGXR, HVPKPXX:APTKL:EPLP-AA17-VYY:EQLSN:NMVVR:SXGXH, RVPSTXX:APVKT:EPLP-AA17-VYY:EQLSN:MIVEE:XGXL, ASASPXX:VSQDL:EPLP-AA17-VYY:EQLSN:MIVKS:XKXS, ASASPXX:VPQDL:EPLP-AA17-VYY:EQLSN:MVVKS:XKXS, NDEGLEX:VPTEE:EPLP-AA17-VYY:EQLSN:FLQHN:KXEXR, NDEGLEX:VPTGQ:EPLP-AA17-VYY:EQLSN:LEEHS:QXEXR, SSVKXQP:SRVHH:EPLP-AA17-VYY:EQLSN:RLEEH:LEXAXA, RNVQXRP:TQVQL:EPLP-AA17-VYY:EQLSN:TLEDH:LAXKXE, KIPKAXX:VSQEL:EPLT-AA17-LYY:EQLSN:DMVKS:GXGXR, GIPEPXX:VSQKM:EPLT-AA17-LYY:EQLSN:NMVKS:SXAXR, SIPKAXX:VSQEL:EPLT-AA17-LYY:EQLSN:EMVKS:GXGXR, HVTKPTX:VSQKL:EPLT-AA17-LYY:EQLSN:NMVKS:SXGXH, YVPKPXX:VSQKL:EPLT-AA17-LYY:EQLSN:NMVKS:SXGXH, TVPKPXX:VSQQL:EPLT-AA17-LYY:EQLSN:NMVKS:AXGXH, AVPKAXX:VSQKL:EPLT-AA17-LYY:EQLSN:NMVKS:AXGXH, KVGKAXX:VSQKL:EPLT-AA17-LYY:EQLSN:EGMSVKS:XGXR, KASKAXX:VSQKL:EPLT-AA17-LYY:EQLSN:GMVKS:EXGXR, GSAGPXX:VSQKM:EPLT-AA17-LYY:EQLSN:GMVKS:RXGXS, AAPASXX:VSQRL:EPLT-AA17-LYY:EQLSN:DMVKS:AXGXR, STPPTXX:VSQRL:EPLT-AA17-LYY:EQLSN:DMVKS:SXGXR, HVPKPXX:VSQKL:EPLT-AA17-LYY:EQLSN:NMVKS:SXGXH, RVPSTXX:VSQKT:EPLT-AA17-LYY:EQLSN:MIVKS:XGXL, ASAAPXX:VSQAL:EPLT-AA17-LYY:EQLSN:MIVKS:XKXS, ASASPXX:VSQDL:EPLT-AA17-LYY:EQLSN:MVVKS:XKXS, KIPKAXX:VPTEL:EPLT-AA17-LYY:EQLSN:DMVVE:GXGXR, GIPEPXX:VPEKM:EPLT-AA17-LYY:EQLSN:NMTVE:SXAXR, SIPKAXX:VPTEL:EPLT-AA17-LYY:EQLSN:EMVVE:GXGXR, HVTKPTX:APTKL:EPLT-AA17-LYY:EQLSN:NMVVR:SXGXH, YVPKPXX:APTKL:EPLT-AA17-LYY:EQLSN:NMVVR:SXGXH, TVPKPXX:APTQL:EPLT-AA17-LYY:EQLSN:NMVVR:AXGXH, AVPKAXX:APTKL:EPLT-AA17-LYY:EQLSN:NMVVK:AXGXH, KVGKAXX:VPTKL:EPLT-AA17-LYY:EQLSN:EGMSVAE:XGXR, KASKAXX:VPTKL:EPLT-AA17-LYY:EQLSN:GMAVS:EXGXR, GSAGPXX:TPTKM:EPLT-AA17-LYY:EQLSN:GMVVD:RXGXS, AAPASXX:VPARL:EPLT-AA17-LYY:EQLSN:DMVVE:AXGXR, STPPTXX:VPTRL:EPLT-AA17-LYY:EQLSN:DMVVE:SXGXR, HVPKPXX:APTKL:EPLT-AA17-LYY:EQLSN:NMVVR:SXGXH, RVPSTXX:APVKT:EPLT-AA17-LYY:EQLSN:MIVEE:XGXL, ASAAPXX:VPQAL:EPLT-AA17-LYY:EQLSN:MIVRS:XKXS, NDEGLEX:VPTEE:EPLT-AA17-LYY:EQLSN:FLQHN:KXEXR, NDEGLEX:VPTGQ:EPLT-AA17-LYY:EQLSN:LEEHS:QXEXR, SSVKXQP:SRVHH:EPLT-AA17-LYY:EQLSN:RLEEH:LEXAXA, RNVQXRP:TQVQL:EPLT-AA17-LYY:EQLSN:TLEDH:LAXKXE, KIPKAXX:VPQEL:EPLT-AA17-LYY:EQLSN:DMVKS:GXGXR, GIPEPXX:VPQKM:EPLT-AA17-LYY:EQLSN:NMVKS:SXAXR, SIPKAXX:VPQEL:EPLT-AA17-LYY:EQLSN:EMVKS:GXGXR, HVTKPTX:VPQKL:EPLT-AA17-LYY:EQLSN:NMVKS:SXGXH, YVPKPXX:VPQKL:EPLT-AA17-LYY:EQLSN:NMVKS:SXGXH, TVPKPXX:VPQQL:EPLT-AA17-LYY:EQLSN:NMVKS:AXGXH, AVPKAXX:VPQKL:EPLT-AA17-LYY:EQLSN:NMVKS:AXGXH, KVGKAXX:VPQKL:EPLT-AA17-LYY:EQLSN:EGMSVKS:XGXR, KASKAXX:VPQKL:EPLT-AA17-LYY:EQLSN:GMVKS:EXGXR, GSAGPXX:VPQKM:EPLT-AA17-LYY:EQLSN:GMVKS:RXGXS, AAPASXX:VPQRL:EPLT-AA17-LYY:EQLSN:DMVKS:AXGXR, STPPTXX:VPQRL:EPLT-AA17-LYY:EQLSN:DMVKS:SXGXR, HVPKPXX:VPQKL:EPLT-AA17-LYY:EQLSN:NMVKS:SXGXH, RVPSTXX:VPQKT:EPLT-AA17-LYY:EQLSN:MIVKS:XGXL, ASAAPXX:VPQAL:EPLT-AA17-LYY:EQLSN:MIVKS:XKXS, ASASPXX:VPQDL:EPLT-AA17-LYY:EQLSN:MIVKS:XKXS, NDEGLEX:VPTGQ:SNIT-AA17-QIM:IGEMS:LEQHN:QXEXR, KIPKAXX:VPTEL:SNIT-AA17-QIM:IGEMS:DMVVE:GXGXR, GIPEPXX:VPEKM:SNIT-AA17-QIM:IGEMS:NMTVE:SXAXR, SIPKAXX:VPTEL:SNIT-AA17-QIM:IGEMS:EMVVE:GXGXR, HVTKPTX:APTKL:SNIT-AA17-QIM:IGEMS:NMVVR:SXGXH, YVPKPXX:APTKL:SNIT-AA17-QIM:IGEMS:NMVVR:SXGXH, TVPKPXX:APTQL:SNIT-AA17-QIM:IGEMS:NMVVR:AXGXH, AVPKAXX:APTKL:SNIT-AA17-QIM:IGEMS:NMVVK:AXGXH, KVGKAXX:VPTKL:SNIT-AA17-QIM:IGEMS:EGMSVAE:XGXR, KASKAXX:VPTKL:SNIT-AA17-QIM:IGEMS:GMAVS:EXGXR, GSAGPXX:TPTKM:SNIT-AA17-QIM:IGEMS:GMVVD:RXGXS, AAPASXX:VPARL:SNIT-AA17-QIM:IGEMS:DMVVE:AXGXR, STPPTXX:VPTRL:SNIT-AA17-QIM:IGEMS:DMVVE:SXGXR, HVPKPXX:APTKL:SNIT-AA17-QIM:IGEMS:NMVVR:SXGXH, RVPSTXX:APVKT:SNIT-AA17-QIM:IGEMS:MIVEE:XGXL, ASAAPXX:VPQAL:SNIT-AA17-QIM:IGEMS:MIVRS:XKXS, ASASPXX:VSQDL:SNIT-AA17-QIM:IGEMS:MIVKS:XKXS, ASASPXX:VPQDL:SNIT-AA17-QIM:IGEMS:MVVKS:XKXS, NDEGLEX:VPTGQ:SNIT-AA17-QIM:IGEMS:LEEHS:QXEXR, SSVKXQP:SRVHH:SNIT-AA17-QIM:IGEMS:RLEEH:LEXAXA, RNVQXRP:TQVQL:SNIT-AA17-QIM:IGEMS:TLEDH:LAXKXE, NDEGLEX:VPTEE:SNIT-AA17-QIM:LGEMS:FLEHS:KXEXR, KIPKAXX:VPTEL:SNIT-AA17-QIM:LGEMS:DMVVE:GXGXR, GIPEPXX:VPEKM:SNIT-AA17-QIM:LGEMS:NMTVE:SXAXR, SIPKAXX:VPTEL:SNIT-AA17-QIM:LGEMS:EMVVE:GXGXR, HVTKPTX:APTKL:SNIT-AA17-QIM:LGEMS:NMVVR:SXGXH, YVPKPXX:APTKL:SNIT-AA17-QIM:LGEMS:NMVVR:SXGXH, TVPKPXX:APTQL:SNIT-AA17-QIM:LGEMS:NMVVR:AXGXH, AVPKAXX:APTKL:SNIT-AA17-QIM:LGEMS:NMVVK:AXGXH, KVGKAXX:VPTKL:SNIT-AA17-QIM:LGEMS:EGMSVAE:XGXR, KASKAXX:VPTKL:SNIT-AA17-QIM:LGEMS:GMAVS:EXGXR, GSAGPXX:TPTKM:SNIT-AA17-QIM:LGEMS:GMVVD:RXGXS, AAPASXX:VPARL:SNIT-AA17-QIM:LGEMS:DMVVE:AXGXR, STPPTXX:VPTRL:SNIT-AA17-QIM:LGEMS:DMVVE:SXGXR, HVPKPXX:APTKL:SNIT-AA17-QIM:LGEMS:NMVVR:SXGXH, RVPSTXX:APVKT:SNIT-AA17-QIM:LGEMS:MIVEE:XGXL, ASAAPXX:VPQAL:SNIT-AA17-QIM:LGEMS:MIVRS:XKXS, ASASPXX:VSQDL:SNIT-AA17-QIM:LGEMS:MIVKS:XKXS, ASASPXX:VPQDL:SNIT-AA17-QIM:LGEMS:MVVKS:XKXS, NDEGLEX:VPTEE:SNIT-AA17-QIM:LGEMS:FLQHN:KXEXR, SSVKXQP:SRVHH:SNIT-AA17-QIM:LGEMS:RLEEH:LEXAXA, RNVQXRP:TQVQL:SNIT-AA17-QIM:LGEMS:TLEDH:LAXKXE, RNVQXRP:SRVQL:RSVK-AA17-AKV:KEVQV:TLEEH:LAXKXE, KIPKAXX:VPTEL:RSVK-AA17-AKV:KEVQV:DMVVE:GXGXR, GIPEPXX:VPEKM:RSVK-AA17-AKV:KEVQV:NMTVE:SXAXR, SIPKAXX:VPTEL:RSVK-AA17-AKV:KEVQV:EMVVE:GXGXR, HVTKPTX:APTKL:RSVK-AA17-AKV:KEVQV:NMVVR:SXGXH, YVPKPXX:APTKL:RSVK-AA17-AKV:KEVQV:NMVVR:SXGXH, TVPKPXX:APTQL:RSVK-AA17-AKV:KEVQV:NMVVR:AXGXH, AVPKAXX:APTKL:RSVK-AA7-AKV:KEVQV:NMVVK:AXGXH, KVGKAXX:VPTKL:RSVK-AA17-AKV:KEVQV:EGMSVAE:XGXR, KASKAXX:VPTKL:RSVK-AA17-AKV:KEVQV:GMAVS:EXGXR, GSAGPXX:TPTKM:RSVK-AA17-AKV:KEVQV:GMVVD:RXGXS, AAPASXX:VPARL:RSVK-AA17-AKV:KEVQV:DMVVE:AXGXR, STPPTXX:VPTRL:RSVK-AA17-AKV:KEVQV:DMVVE:SXGXR, HVPKPXX:APTKL:RSVK-AA17-AKV:KEVQV:NMVVR:SXGXH, RVPSTXX:APVKT:RSVK-AA17-AKV:KEVQV:MIVEE:XGXL, ASAAPXX:VPQAL:RSVK-AA17-AKV:KEVQV:MIVRS:XKXS, ASASPXX:VSQDL:RSVK-AA17-AKV:KEVQV:MIVKS:XKXS, ASASPXX:VPQDL:RSVK-AA17-AKV:KEVQV:MVVKS:XKXS, NDEGLEX:VPTEE:RSVK-AA17-AKV:KEVQV:FLQHN:KXEXR, NDEGLEX:VPTGQ:RSVK-AA17-AKV:KEVQV:LEEHS:QXEXR, RNVQXRP:TQVQL:RSVK-AA17-AKV:KEVQV:TLEDH:LAXKXE, SSVKXQP:TQVHH:RPVQ-AA17-RKI:KKATV:RLEDH:LEXAXA, KIPKAXX:VPTEL:RPVQ-AA17-RKI:KKATV:DMVVE:GXGXR, GIPEPXX:VPEKM:RPVQ-AA17-RKI:KKATV:NMTVE:SXAXR, SIPKAXX:VPTEL:RPVQ-AA17-RKI:KKATV:EMVVE:GXGXR, HVTKPTX:APTKL:RPVQ-AA17-RKI:KKATV:NMVVR:SXGXH, YVPKPXX:APTKL:RPVQ-AA17-RKI:KKATV:NMVVR:SXGXH, TVPKPXX:APTQL:RPVQ-AA17-RKI:KKATV:NMVVR:AXGXH, AVPKAXX:APTKL:RPVQ-AA17-RKI:KKATV:NMVVK:AXGXH, KVGKAXX:VPTKL:RPVQ-AA17-RKI:KKATV:EGMSVAE:XGXR, KASKAXX:VPTKL:RPVQ-AA17-RKI:KKATV:GMAVS:EXGXR, GSAGPXX:TPTKM:RPVQ-AA17-RKI:KKATV:GMVVD:RXGXS, AAPASXX:VPARL:RPVQ-AA17-RKI:KKATV:DMVVE:AXGXR, STPPTXX:VPTRL:RPVQ-AA17-RKI:KKATV:DMVVE:SXGXR, HVPKPXX:APTKL:RPVQ-AA17-RKI:KKATV:NMVVR:SXGXH, RVPSTXX:APVKT:RPVQ-AA17-RKI:KKATV:MIVEE:XGXL, ASAAPXX:VPQAL:RPVQ-AA17-RKI:KKATV:MIVRS:XKXS, ASASPXX:VSQDL:RPVQ-AA17-RKI:KKATV:MIVKS:XKXS, ASASPXX:VPQDL:RPVQ-AA17-RKI:KKATV:MVVKS:XKXS, NDEGLEX:VPTEE:RPVQ-AA17-RKI:KKATV:FLQHN:KXEXR, NDEGLEX:VPTGQ:RPVQ-AA17-RKI:KKATV:LEEHS:QXEXR and SSVKXQP:SRVHH:RPVQ-AA17-RKI:KKATV:RLEEH:LEXAXA; and wherein AA$^{17}$ is selected from the group consisting of G, A, V, L, I, P, F, M, W, T and S (in particular is selected from the group consisting of M, I, L, V and T).

In particular, in certain embodiments, the heptuplet PEP7:PEP3:PEP1:LINKER:PEP2:PEP4:PEP8 is selected from the group consisting of GIPEPXX:VPT:SAIS:DENKNVV:LKNYQ:VVE:SXAXR, SIPKAXX:VPT:SAIS:DEYDKVV:LKNYQ:VVE:GXGXR, HVTKPTX:VPT:SAIS:DDSSNVI:LKNYQ:VVE:SXGXH, YVPKPXX:VPT:SAIS:DDSSNVI:LKNYQ:VVE:SXGXH, TVPKPXX:VPT:SAIS:DDSSNVI:LKNYQ:VVE:AXGXH, AVPKAXX:VPT:SAIS:DSSNNVI:LKNYQ:VVE:AXGXH, KVGKAXX:VPT:SAIS:DDMGVPT:LKNYQ:VVE:XGXR, KASKAXX:VPT:SAIS:DKGVVTY:LKNYQ:VVE:EXGXR, GSAGPXX:VPT:SAIS:NDKQQII:LKNYQ:VVE:RXGXS, AAPASXX:VPT:SAIS:DAANNVV:LKNYQ:VVE:AXGXR, STPPTXX:VPT:SAIS:DSANNVV:LKNYQ:VVE:SXGXR, HVPKPXX:VPT:SAIS:DDSSNVI:LKNYQ:VVE:SXGXH, RVPSTXX:VPT:SAIS:DNGRVLL:LKNYQ:VVE:XGXL, ASAAPXX:VPT:SAIS:VGRKPKV:LKNYQ:VVE:XKXS, ASASPXX:VPT:SAIS:IGKTPKI:LKNYQ:VVE:XKXS, ASASPXX:VPT:SAIS:VGRTPKV:LKNYQ:VVE:XKXS, NDEGLEX:VPT:SAIS:RIKPHQGQH:LKNYQ:VVE:KXEXR, NDEGLEX:VPT:SAIS:RIKPHQGQH:LKNYQ:VVE:QXEXR, SSVKXQP:VPT:SAIS:EYVRKKPKL:LKNYQ:VVE:LEXAXA, RNVQXRP:VPT:SAIS:EIVRKKPIF:LKNYQ:VVE:LAXKXE, GIPEPXX:VPE:SAIS:DENKNVV:LKNYQ:TVE:SXAXR, HVTKPTX:APT:SAIS:DDSSNVI:LKNYQ:VVR:SXGXH, YVPKPXX:APT:SAIS:DDSSNVI:LKNYQ:VVR:SXGXH, TVPKPXX:APT:SAIS:DDSSNVI:LKNYQ:VVR:AXGXH, AVPKAXX:APT:SAIS:DSSNNVI:LKNYQ:VVK:AXGXH, KVGKAXX:VPT:SAIS:DDMGVPT:LKNYQ:VAE:XGXR, KASKAXX:VPT:SAIS:DKGVVTY:LKNYQ:AVS:EXGXR, GSAGPXX:TPT:SAIS:NDKQQII:LKNYQ:VVD:RXGXS, AAPASXX:VPA:SAIS:DAANNVV:LKNYQ:VVE:AXGXR, HVPKPXX:APT:SAIS:DDSSNVI:LKNYQ:VVR:SXGXH, RVPSTXX:APV:SAIS:DNGRVLL:LKNYQ:VEE:XGXL, ASAAPXX:VPQ:SAIS:VGRKPKV:LKNYQ:VRS:XKXS, ASASPXX:VSQ:SAIS:IGKTPKI:LKNYQ:VKS:XKXS, ASASPXX:VPQ:SAIS:VGRTPKV:LKNYQ:VKS:XKXS, NDEGLEX:VPT:SAIS:RIKPHQGQH:LKNYQ:QHN:KXEXR, NDEGLEX:VPT:SAIS:RIKPHQGQH:LKNYQ:EHS:QXEXR, SSVKXQP:SRV:SAIS:EYVRKKPKL:LKNYQ:EEH:LEXAXA, RNVQXRP:TQV:SAIS:EIVRKKPIF:LKNYQ:EDH:LAXKXE, KIPKAXX:VPE:SSLS:DENEKVV:LKVYP:TVE:GXGXR, SIPKAXX:VPE:SSLS:DEYDKVV:LKVYP:TVE:GXGXR, HVTKPTX:VPE:SSLS:DDSSNVI:LKVYP:TVE:SXGXH, YVPKPXX:VPE:SSLS:DDSSNVI:LKVYP:TVE:SXGXH, TVPKPXX:VPE:SSLS:DDSSNVI:LKVYP:TVE:AXGXH, AVPKAXX:VPE:SSLS:DSSNNVI:LKVYP:TVE:AXGXH, KVGKAXX:VPE:SSLS:DDMGVPT:LKVYP:TVE:XGXR, KASKAXX:VPE:SSLS:DKGVVTY:LKVYP:TVE:EXGXR, GSAGPXX:VPE:SSLS:NDKQQII:LKVYP:TVE:RXGXS, AAPASXX:VPE:SSLS:DAANNVV:LKVYP:TVE:AXGXR, STPPTXX:VPE:SSLS:DSANNVV:LKVYP:TVE:SXGXR, HVPKPXX:VPE:SSLS:DDSSNVI:LKVYP:TVE:SXGXH, RVPSTXX:VPE:SSLS:DNGRVLL:LKVYP:TVE:XGXL, ASAAPXX:VPE:SSLS:VGRKPKV:LKVYP:TVE:XKXS, ASASPXX:VPE:SSLS:IGKTPKI:LKVYP:TVE:XKXS, ASASPXX:VPE:SSLS:VGRTPKV:LKVYP:TVE:XKXS, NDEGLEX:VPE:SSLS:RIKPHQGQH:LKVYP:TVE:KXEXR, NDEGLEX:VPE:SSLS:RIKPHQGQH:LKVYP:TVE:QXEXR, SSVKXQP:VPE:SSLS:EYVRKKPKL:LKVYP:TVE:LEXAXA, RNVQXRP:VPE:SSLS:EIVRKKPIF:LKVYP:TVE:LAXKXE, KIPKAXX:VPT:SSLS:DENEKVV:LKVYP:VVE:GXGXR, SIPKAXX:VPT:SSLS:DEYDKVV:LKVYP:VVE:GXGXR, HVTKPTX:APT:SSLS:DDSSNVI:LKVYP:VVR:SXGXH, YVPKPXX:APT:SSLS:DDSSNVI:LKVYP:VVR:SXGXH, TVPKPXX:APT:SSLS:DDSSNVI:LKVYP:VVR:AXGXH, AVPKAXX:APT:SSLS:DSSNNVI:LKVYP:VVK:AXGXH, KVGKAXX:VPT:SSLS:DDMGVPT:LKVYP:VAE:XGXR, KASKAXX:VPT:SSLS:DKGVVTY:LKVYP:AVS:EXGXR, GSAGPXX:TPT:SSLS:NDKQQII:LKVYP:VVD:RXGXS, AAPASXX:VPA:SSLS:DAANNVV:LKVYP:VVE:AXGXR, STPPTXX:VPT:SSLS:DSANNVV:LKVYP:VVE:SXGXR, HVPKPXX:APT:SSLS:DDSSNVI:LKVYP:VVR:SXGXH, RVPSTXX:APV:SSLS:DNGRVLL:LKVYP:VEE:XGXL, ASAAPXX:VPQ:SSLS:VGRKPKV:LKVYP:VRS:XKXS, ASASPXX:VSQ:SSLS:IGKTPKI:LKVYP:VKS:XKXS, ASASPXX:VPQ:SSLS:VGRTPKV:LKVYP:VKS:XKXS, NDEGLEX:VPT:SSLS:RIKPHQGQH:LKVYP:QHN:KXEXR, NDEGLEX:VPT:SSLS:RIKPHQGQH:LKVYP:EHS:QXEXR, SSVKXQP:SRV:SSLS:EYVRKKPKL:LKVYP:EEH:LEXAXA, RNVQXRP:TQV:SSLS:EIVRKKPIF:LKVYP:EDH:LAXKXE, KIPKAXX:VPT:SAIS:DENEKVV:LKNYQ:VVE:GXGXR, KIPKAXX:APT:NAIS:DENEKVV:LKKYR:VVR:GXGXR, GIPEPXX:APT:NAIS:DENKNVV:LKKYR:VVR:SXAXR, SIPKAXX:APT:NAIS:DEYDKVV:LKKYR:VVR:GXGXR, YVPKPXX:APT:NAIS:DDSSNVI:LKKYR:VVR:SXGXH, TVPKPXX:APT:NAIS:DDSSNVI:LKKYR:VVR:AXGXH, AVPKAXX:APT:NAIS:DSSNNVI:LKKYR:VVR:AXGXH, KVGKAXX:APT:NAIS:DDMGVPT:LKKYR:VVR:XGXR, KASKAXX:APT:NAIS:DKGVVTY:LKKYR:VVR:EXGXR, GSAGPXX:APT:NAIS:NDKQQII:LKKYR:VVR:RXGXS, AAPASXX:APT:NAIS:DAANNVV:LKKYR:VVR:AXGXR, STPPTXX:APT:NAIS:DSANNVV:LKKYR:VVR:SXGXR, HVPKPXX:APT:NAIS:DDSSNVI:LKKYR:VVR:SXGXH, RVPSTXX:APT:NAIS:DNGRVLL:LKKYR:VVR:XGXL, ASAAPXX:APT:NAIS:VGRKPKV:LKKYR:VVR:XKXS, ASASPXX:APT:NAIS:IGKTPKI:LKKYR:VVR:XKXS, ASASPXX:APT:NAIS:VGRTPKV:LKKYR:VVR:XKXS, NDEGLEX:APT:NAIS:RIKPHQGQH:LKKYR:VVR:KXEXR, NDEGLEX:APT:NAIS:RIKPHQGQH:LKKYR:VVR:QXEXR, SSVKXQP:APT:NAIS:EYVRKKPKL:LKKYR:VVR:LEXAXA, RNVQXRP:APT:NAIS:EIVRKKPIF:LKKYR:VVR:LAXKXE, KIPKAXX:VPT:NAIS:DENEKVV:LKKYR:VVE:GXGXR, GIPEPXX:VPE:NAIS:DENKNVV:LKKYR:TVE:SXAXR, SIPKAXX:VPT:NAIS:DEYDKVV:LKKYR:VVE:GXGXR, AVPKAXX:APT:NAIS:DSSNNVI:LKKYR:VVK:AXGXH, KVGKAXX:VPT:NAIS:DDMGVPT:LKKYR:VAE:XGXR, KASKAXX:VPT:NAIS:DKGVVTY:LKKYR:AVS:EXGXR, GSAGPXX:TPT:NAIS:NDKQQII:LKKYR:VVD:RXGXS, AAPASXX:VPA:NAIS:DAANNVV:LKKYR:VVE:AXGXR, STPPTXX:VPT:NAIS:DSANNVV:LKKYR:VVE:SXGXR, RVPSTXX:APV:NAIS:DNGRVLL:LKKYR:VEE:XGXL, ASAAPXX:VPQ:NAIS:VGRKPKV:LKKYR:VRS:XKXS, ASASPXX:VSQ:NAIS:IGKTPKI:LKKYR:VKS:XKXS, ASASPXX:VPQ:NAIS:VGRTPKV:LKKYR:VKS:XKXS, NDEGLEX:VPT:NAIS:RIKPHQGQH:LKKYR:QHN:KXEXR, NDEGLEX:VPT:NAIS:RIKPHQGQH:LKKYR:EHS:QXEXR, SSVKXQP:SRV:NAIS:EYVRKKPKL:LKKYR:EEH:LEXAXA, RNVQXRP:TQV:NAIS:EIVRKKPIF:LKKYR:EDH:LAXKXE, HVTKPTX:APT:NAIS:DDSSNVI:LKKYR:VVR:SXGXH, KIPKAXX:APT:SATS:DENEKVV:LRKHR:VVK:GXGXR, GIPEPXX:APT:SATS:DENKNVV:LRKHR:VVK:SXAXR, SIPKAXX:APT:SATS:DEYDKVV:LRKHR:VVK:GXGXR, HVTKPTX:APT:SATS:DDSSNVI:LRKHR:VVK:SXGXH, YVPKPXX:APT:SATS:DDSSNVI:LRKHR:VVK:SXGXH, TVPKPXX:APT:SATS:DDSSNVI:LRKHR:VVK:AXGXH, KVGKAXX:APT:SATS:DDMGVPT:LRKHR:VVK:XGXR, KASKAXX:APT:SATS:DKGVVTY:LRKHR:VVK:EXGXR, GSAGPXX:APT:SATS:NDKQQII:LRKHR:VVK:RXGXS, AAPASXX:APT:SATS:DAANNVV:LRKHR:VVK:AXGXR, STPPTXX:APT:SATS:DSANNVV:LRKHR:VVK:SXGXR, HVPKPXX:APT:SATS:DDSSNVI:LRKHR:VVK:SXGXH, RVPSTXX:APT:SATS:DNGRVLL:LRKHR:VVK:XGXL, ASAAPXX:APT:SATS:VGRKPKV:LRKHR:VVK:XKXS, ASASPXX:APT:SATS:IGKTPKI:LRKHR:VVK:XKXS, ASASPXX:APT:SATS:VGRTPKV:LRKHR:VVK:XKXS, NDEGLEX:APT:SATS:RIKPHQGQH:LRKHR:VVK:KXEXR, NDEGLEX:APT:SATS:RIKPHQGQH:LRKHR:VVK:QXEXR, SSVKXQP:APT:SATS:EYVRKKPKL:LRKHR:VVK:LEXAXA, RNVQXRP:APT:SATS:EIVRKKPIF:LRKHR:VVK:LAXKXE, KIPKAXX:VPT:SATS:DENEKVV:LRKHR:VVE:GXGXR, GIPEPXX:VPE:SATS:DENKNVV:LRKHR:TVE:SXAXR, SIPKAXX:VPT:SATS:DEYDKVV:LRKHR:VVE:GXGXR, HVTKPTX:APT:SATS:DDSSNVI:LRKHR:VVR:SXGXH, YVPKPXX:APT:SATS:DDSSNVI:LRKHR:VVR:SXGXH, TVPKPXX:APT:SATS:DDSSNVI:LRKHR:VVR:AXGXH, KVGKAXX:VPT:SATS:DDMGVPT:LRKHR:VAE:XGXR, KASKAXX:VPT:SATS:DKGVVTY:LRKHR:AVS:EXGXR, GSAGPXX:TPT:SATS:NDKQQII:LRKHR:VVD:RXGXS, AAPASXX:VPA:SATS:DAANNVV:LRKHR:VVE:AXGXR, STPPTXX:VPT:SATS:DSANNVV:LRKHR:VVE:SXGXR, HVPKPXX:APT:SATS:DDSSNVI:LRKHR:VVR:SXGXH, RVPSTXX:APV:SATS:DNGRVLL:LRKHR:VEE:XGXL, ASAAPXX:VPQ:SATS:VGRKPKV:LRKHR:VRS:XKXS, ASASPXX:VSQ:SATS:IGKTPKI:LRKHR:VKS:XKXS, ASASPXX:VPQ:SATS:VGRTPKV:LRKHR:VKS:XKXS, NDEGLEX:VPT:SATS:RIKPHQGQH:LRKHR:QHN:KXEXR, NDEGLEX:VPT:SATS:RIKPHQGQH:LRKHR:EHS:QXEXR, SSVKXQP:SRV:SATS:EYVRKKPKL:LRKHR:EEH:LEXAXA, RNVQXRP:TQV:SATS:EIVRKKPIF:LRKHR:EDH:LAXKXE, KIPKAXX:VPT:SPIS:DENEKVV:LKYHY:VAE:GXGXR, GIPEPXX:VPT:SPIS:DENKNVV:LKYHY:VAE:SXAXR, SIPKAXX:VPT:SPIS:DEYDKVV:LKYHY:VAE:GXGXR, HVTKPTX:VPT:SPIS:DDSSNVI:LKYHY:VAE:SXGXH, YVPKPXX:VPT:SPIS:DDSSNVI:LKYHY:VAE:SXGXH, TVPKPXX:VPT:SPIS:DDSSNVI:LKYHY:VAE:AXGXH, AVPKAXX:VPT:SPIS:DSSNNVI:LKYHY:VAE:AXGXH, KASKAXX:VPT:SPIS:DKGVVTY:LKYHY:VAE:EXGXR, GSAGPXX:VPT:SPIS:NDKQQII:LKYHY:VAE:RXGXS, AAPASXX:VPT:SPIS:DAANNVV:LKYHY:VAE:AXGXR, STPPTXX:VPT:SPIS:DSANNVV:LKYHY:VAE:SXGXR, HVPKPXX:VPT:SPIS:DDSSNVI:LKYHY:VAE:SXGXH, RVPSTXX:VPT:SPIS:DNGRVLL:LKYHY:VAE:XGXL, ASAAPXX:VPT:SPIS:VGRKPKV:LKYHY:VAE:XKXS, ASASPXX:VPT:SPIS:IGKTPKI:LKYHY:VAE:XKXS, ASASPXX:VPT:SPIS:VGRTPKV:LKYHY:VAE:XKXS, NDEGLEX:VPT:SPIS:RIKPHQGQH:LKYHY:VAE:KXEXR, NDEGLEX:VPT:SPIS:RIKPHQGQH:LKYHY:VAE:QXEXR, SSVKXQP:VPT:SPIS:EYVRKKPKL:LKYHY:VAE:LEXAXA, RNVQXRP:VPT:SPIS:EIVRKKPIF:LKYHY:VAE:LAXKXE, KIPKAXX:VPT:SPIS:DENEKVV:LKYHY:VVE:GXGXR, GIPEPXX:VPE:SPIS:DENKNVV:LKYHY:TVE:SXAXR, SIPKAXX:VPT:SPIS:DEYDKVV:LKYHY:VVE:GXGXR, HVTKPTX:APT:SPIS:DDSSNVI:LKYHY:VVR:SXGXH, YVPKPXX:APT:SPIS:DDSSNVI:LKYHY:VVR:SXGXH, TVPKPXX:APT:SPIS:DDSSNVI:LKYHY:VVR:AXGXH, AVPKAXX:APT:SPIS:DSSNNVI:LKYHY:VVK:AXGXH, KASKAXX:VPT:SPIS:DKGVVTY:LKYHY:AVS:EXGXR, GSAGPXX:TPT:SPIS:NDKQQII:LKYHY:VVD:RXGXS, AAPASXX:VPA:SPIS:DAANNVV:LKYHY:VVE:AXGXR, STPPTXX:VPT:SPIS:DSANNVV:LKYHY:VVE:SXGXR, HVPKPXX:APT:SPIS:DDSSNVI:LKYHY:VVR:SXGXH, RVPSTXX:APV:SPIS:DNGRVLL:LKYHY:VEE:XGXL, ASAAPXX:VPQ:SPIS:VGRKPKV:LKYHY:VRS:XKXS, ASASPXX:VSQ:SPIS:IGKTPKI:LKYHY:VKS:XKXS, ASASPXX:VPQ:SPIS:VGRTPKV:LKYHY:VKS:XKXS, NDEGLEX:VPT:SPIS:RIKPHQGQH:LKYHY:QHN:KXEXR, NDEGLEX:VPT:SPIS:RIKPHQGQH:LKYHY:EHS:QXEXR, SSVKXQP:SRV:SPIS:EYVRKKPKL:LKYHY:EEH:LEXAXA, RNVQXRP:TQV:SPIS:EIVRKKPIF:LKYHY:EDH:LAXKXE, KIPKAXX:VPT:EPIS:DENEKVV:KFKYE:AVS:GXGXR, GIPEPXX:VPT:EPIS:DENKNVV:KFKYE:AVS:SXAXR, SIPKAXX:VPT:EPIS:DEYDKVV:KFKYE:AVS:GXGXR, HVTKPTX:VPT:EPIS:DDSSNVI:KFKYE:AVS:SXGXH, YVPKPXX:VPT:EPIS:DDSSNVI:KFKYE:AVS:SXGXH, TVPKPXX:VPT:EPIS:DDSSNVI:KFKYE:AVS:AXGXH, AVPKAXX:VPT:EPIS:DSSNNVI:KFKYE:AVS:AXGXH, KVGKAXX:VPT:EPIS:DDMGVPT:KFKYE:AVS:XGXR, GSAGPXX:VPT:EPIS:NDKQQII:KFKYE:AVS:RXGXS, AAPASXX:VPT:EPIS:DAANNVV:KFKYE:AVS:AXGXR, STPPTXX:VPT:EPIS:DSANNVV:KFKYE:AVS:SXGXR, HVPKPXX:VPT:EPIS:DDSSNVI:KFKYE:AVS:SXGXH, RVPSTXX:VPT:EPIS:DNGRVLL:KFKYE:AVS:XGXL, ASAAPXX:VPT:EPIS:VGRKPKV:KFKYE:AVS:XKXS, ASASPXX:VPT:EPIS:IGKTPKI:KFKYE:AVS:XKXS, ASASPXX:VPT:EPIS:VGRTPKV:KFKYE:AVS:XKXS, NDEGLEX:VPT:EPIS:RIKPHQGQH:KFKYE:AVS:KXEXR, NDEGLEX:VPT:EPIS:RIKPHQGQH:KFKYE:AVS:QXEXR, SSVKXQP:VPT:EPIS:EYVRKKPKL:KFKYE:AVS:LEXAXA, RNVQXRP:VPT:EPIS:EIVRKKPIF:KFKYE:AVS:LAXKXE, KIPKAXX:VPT:EPIS:DENEKVV:KFKYE:VVE:GXGXR, GIPEPXX:VPE:EPIS:DENKNVV:KFKYE:TVE:SXAXR, SIPKAXX:VPT:EPIS:DEYDKVV:KFKYE:VVE:GXGXR, HVTKPTX:APT:EPIS:DDSSNVI:KFKYE:VVR:SXGXH, YVPKPXX:APT:EPIS:DDSSNVI:KFKYE:VVR:SXGXH, TVPKPXX:APT:EPIS:DDSSNVI:KFKYE:VVR:AXGXH, AVPKAXX:APT:EPIS:DSSNNVI:KFKYE:VVK:AXGXH, KVGKAXX:VPT:EPIS:DDMGVPT:KFKYE:VAE:XGXR, GSAGPXX:TPT:EPIS:NDKQQII:KFKYE:VVD:RXGXS, AAPASXX:VPA:EPIS:DAANNVV:KFKYE:VVE:AXGXR, STPPTXX:VPT:EPIS:DSANNVV:KFKYE:VVE:SXGXR, HVPKPXX:APT:EPIS:DDSSNVI:KFKYE:VVR:SXGXH, RVPSTXX:APV:EPIS:DNGRVLL:KFKYE:VEE:XGXL, ASAAPXX:VPQ:EPIS:VGRKPKV:KFKYE:VRS:XKXS, ASASPXX:VSQ:EPIS:IGKTPKI:KFKYE:VKS:XKXS, ASASPXX:VPQ:EPIS:VGRTPKV:KFKYE:VKS:XKXS, NDEGLEX:VPT:EPIS:RIKPHQGQH:KFKYE:QHN:KXEXR, NDEGLEX:VPT:EPIS:RIKPHQGQH:KFKYE:EHS:QXEXR, SSVKXQP:SRV:EPIS:EYVRKKPKL:KFKYE:EEH:LEXAXA, RNVQXRP:TQV:EPIS:EIVRKKPIF:KFKYE:EDH:LAXKXE, KIPKAXX:TPT:SPIN:DENEKVV:YGKIP:VVD:GXGXR, GIPEPXX:TPT:SPIN:DENKNVV:YGKIP:VVD:SXAXR, SIPKAXX:TPT:SPIN:DEYDKVV:YGKIP:VVD:GXGXR, HVTKPTX:TPT:SPIN:DDSSNVI:YGKIP:VVD:SXGXH, YVPKPXX:TPT:SPIN:DDSSNVI:YGKIP:VVD:SXGXH, TVPKPXX:TPT:SPIN:DDSSNVI:YGKIP:VVD:AXGXH, AVPKAXX:TPT:SPIN:DSSNNVI:YGKIP:VVD:AXGXH, KVGKAXX:TPT:SPIN:DDMGVPT:YGKIP:VVD:XGXR, KASKAXX:TPT:SPIN:DKGVVTY:YGKIP:VVD:EXGXR, AAPASXX:TPT:SPIN:DAANNVV:YGKIP:VVD:AXGXR, STPPTXX:TPT:SPIN:DSANNVV:YGKIP:VVD:SXGXR, HVPKPXX:TPT:SPIN:DDSSNVI:YGKIP:VVD:SXGXH, RVPSTXX:TPT:SPIN:DNGRVLL:YGKIP:VVD:XGXL, ASAAPXX:TPT:SPIN:VGRKPKV:YGKIP:VVD:XKXS, ASASPXX:TPT:SPIN:IGKTPKI:YGKIP:VVD:XKXS, ASASPXX:TPT:SPIN:VGRTPKV:YGKIP:VVD:XKXS, NDEGLEX:TPT:SPIN:RIKPHQGQH:YGKIP:VVD:KXEXR, NDEGLEX:TPT:SPIN:RIKPHQGQH:YGKIP:VVD:QXEXR, SSVKXQP:TPT:SPIN:EYVRKKPKL:YGKIP:VVD:LEXAXA, RNVQXRP:TPT:SPIN:EIVRKKPIF:YGKIP:VVD:LAXKXE, KIPKAXX:VPT:SPIN:DENEKVV:YGKIP:VVE:GXGXR, GIPEPXX:VPE:SPIN:DENKNVV:YGKIP:TVE:SXAXR, SIPKAXX:VPT:SPIN:DEYDKVV:YGKIP:VVE:GXGXR, HVTKPTX:APT:SPIN:DDSSNVI:YGKIP:VVR:SXGXH, YVPKPXX:APT:SPIN:DDSSNVI:YGKIP:VVR:SXGXH, TVPKPXX:APT:SPIN:DDSSNVI:YGKIP:VVR:AXGXH, AVPKAXX:APT:SPIN:DSSNNVI:YGKIP:VVK:AXGXH, KVGKAXX:VPT:SPIN:DDMGVPT:YGKIP:VAE:XGXR, KASKAXX:VPT:SPIN:DKGVVTY:YGKIP:AVS:EXGXR, AAPASXX:VPA:SPIN:DAANNVV:YGKIP:VVE:AXGXR, STPPTXX:VPT:SPIN:DSANNVV:YGKIP:VVE:SXGXR, HVPKPXX:APT:SPIN:DDSSNVI:YGKIP:VVR:SXGXH, RVPSTXX:APV:SPIN:DNGRVLL:YGKIP:VEE:XGXL, ASAAPXX:VPQ:SPIN:VGRKPKV:YGKIP:VRS:XKXS, ASASPXX:VSQ:SPIN:IGKTPKI:YGKIP:VKS:XKXS, ASASPXX:VPQ:SPIN:VGRTPKV:YGKIP:VKS:XKXS, NDEGLEX:VPT:SPIN:RIKPHQGQH:YGKIP:QHN:KXEXR, NDEGLEX:VPT:SPIN:RIKPHQGQH:YGKIP:EHS:QXEXR, SSVKXQP:SRV:SPIN:EYVRKKPKL:YGKIP:EEH:LEXAXA, RNVQXRP:TQV:SPIN:EIVRKKPIF:YGKIP:EDH:LAXKXE, KIPKAXX:VPA:SPIS:DENEKVV:YKQYE:VVE:GXGXR, GIPEPXX:VPA:SPIS:DENKNVV:YKQYE:VVE:SXAXR, SIPKAXX:VPA:SPIS:DEYDKVV:YKQYE:VVE:GXGXR, HVTKPTX:VPA:SPIS:DDSSNVI:YKQYE:VVE:SXGXH, YVPKPXX:VPA:SPIS:DDSSNVI:YKQYE:VVE:SXGXH, TVPKPXX:VPA:SPIS:DDSSNVI:YKQYE:VVE:AXGXH, AVPKAXX:VPA:SPIS:DSSNNVI:YKQYE:VVE:AXGXH, KVGKAXX:VPA:SPIS:DDMGVPT:YKQYE:VVE:XGXR, KASKAXX:VPA:SPIS:DKGVVTY:YKQYE:VVE:EXGXR, GSAGPXX:VPA:SPIS:NDKQQII:YKQYE:VVE:RXGXS, STPPTXX:VPA:SPIS:DSANNVV:YKQYE:VVE:SXGXR, HVPKPXX:VPA:SPIS:DDSSNVI:YKQYE:VVE:SXGXH, RVPSTXX:VPA:SPIS:DNGRVLL:YKQYE:VVE:XGXL, ASAAPXX:VPA:SPIS:VGRKPKV:YKQYE:VVE:XKXS, ASASPXX:VPA:SPIS:IGKTPKI:YKQYE:VVE:XKXS, ASASPXX:VPA:SPIS:VGRTPKV:YKQYE:VVE:XKXS, NDEGLEX:VPA:SPIS:RIKPHQGQH:YKQYE:VVE:KXEXR, NDEGLEX:VPA:SPIS:RIKPHQGQH:YKQYE:VVE:QXEXR, SSVKXQP:VPA:SPIS:EYVRKKPKL:YKQYE:VVE:LEXAXA, RNVQXRP:VPA:SPIS:EIVRKKPIF:YKQYE:VVE:LAXKXE, KIPKAXX:VPT:SPIS:DENEKVV:YKQYE:VVE:GXGXR, GIPEPXX:VPE:SPIS:DENKNW:YKQYE:TVE:SXAXR, SIPKAXX:VPT:SPIS:DEYDKVV:YKQYE:VVE:GXGXR, HVTKPTX:APT:SPIS:DDSSNVI:YKQYE:VVR:SXGXH, YVPKPXX:APT:SPIS:DDSSNVI:YKQYE:VVR:SXGXH, TVPKPXX:APT:SPIS:DDSSNVI:YKQYE:VVR:AXGXH, AVPKAXX:APT:SPIS:DSSNNVI:YKQYE:VVK:AXGXH, KVGKAXX:VPT:SPIS:DDMGVPT:YKQYE:VAE:XGXR, KASKAXX:VPT:SPIS:DKGVVTY:YKQYE:AVS:EXGXR, GSAGPXX:TPT:SPIS:NDKQQII:YKQYE:VVD:RXGXS, STPPTXX:VPT:SPIS:DSANNVV:YKQYE:VVE:SXGXR, HVPKPXX:APT:SPIS:DDSSNVI:YKQYE:VVR:SXGXH, RVPSTXX:APV:SPIS:DNGRVLL:YKQYE:VEE:XGXL, ASAAPXX:VPQ:SPIS:VGRKPKV:YKQYE:VRS:XKXS, ASASPXX:VSQ:SPIS:IGKTPKI:YKQYE:VKS:XKXS, ASASPXX:VPQ:SPIS:VGRTPKV:YKQYE:VKS:XKXS, NDEGLEX:VPT:SPIS:RIKPHQGQH:YKQYE:QHN:KXEXR, NDEGLEX:VPT:SPIS:RIKPHQGQH:YKQYE:EHS:QXEXR, SSVKXQP:SRV:SPIS:EYVRKKPKL:YKQYE:EEH:LEXAXA, RNVQXRP:TQV:SPIS:EIVRKKPIF:YKQYE:EDH:LAXKXE, GIPEPXX:VPT :SPIS:DENKNW:YKQYE:WE:SXAXR, HVTKPTX:VPT:SPIS:DDSSNVI:YKQYE:VVE:SXGXH, YVPKPXX:VPT:SPIS:DDSSNVI:YKQYE:VVE:SXGXH, TVPKPXX:VPT:SPIS:DDSSNVI:YKQYE:VVE:AXGXH, AVPKAXX:VPT:SPIS:DSSNNVI:YKQYE:VVE:AXGXH, KVGKAXX:VPT:SPIS:DDMGVPT:YKQYE:VVE:XGXR, KASKAXX:VPT:SPIS:DKGVVTY:YKQYE:VVE:EXGXR, GSAGPXX:VPT:SPIS:NDKQQII:YKQYE:VVE:RXGXS, AAPASXX:VPT:SPIS:DAANNVV:YKQYE:VVE:AXGXR, HVPKPXX:VPT:SPIS:DDSSNVI:YKQYE:VVE:SXGXH, RVPSTXX:VPT:SPIS:DNGRVLL:YKQYE:VVE:XGXL, ASAAPXX:VPT:SPIS:VGRKPKV:YKQYE:VVE:XKXS, ASASPXX:VPT:SPIS:IGKTPKI:YKQYE:VVE:XKXS, ASASPXX:VPT:SPIS:VGRTPKV:YKQYE:VVE:XKXS, NDEGLEX:VPT:SPIS:RIKPHQGQH:YKQYE:VVE:KXEXR, NDEGLEX:VPT:SPIS:RIKPHQGQH:YKQYE:VVE:QXEXR, SSVKXQP:VPT:SPIS:EYVRKKPKL:YKQYE:VVE:LEXAXA, RNVQXRP:VPT:SPIS:EIVRKKPIF:YKQYE:VVE:LAXKXE, AAPASXX:VPA:SPIS:DAANNVV:YKQYE:VVE:AXGXR, KIPKAXX:APV:KPLS:DENEKVV:DHHKD:VEE:GXGXR, GIPEPXX:APV:KPLS:DENKNVV:DHHKD:VEE:SXAXR, SIPKAXX:APV:KPLS:DEYDKVV:DHHKD:VEE:GXGXR, HVTKPTX:APV:KPLS:DDSSNVI:DHHKD:VEE:SXGXH, YVPKPXX:APV:KPLS:DDSSNVI:DHHKD:VEE:SXGXH, TVPKPXX:APV:KPLS:DDSSNVI:DHHKD:VEE:AXGXH, AVPKAXX:APV:KPLS:DSSNNVI:DHHKD:VEE:AXGXH, KVGKAXX:APV:KPLS:DDMGVPT:DHHKD:VEE:XGXR, KASKAXX:APV:KPLS:DKGVVTY:DHHKD:VEE:EXGXR, GSAGPXX:APV:KPLS:NDKQQII:DHHKD:VEE:RXGXS, AAPASXX:APV:KPLS:DAANNVV:DHHKD:VEE:AXGXR, STPPTXX:APV:KPLS:DSANNVV:DHHKD:VEE:SXGXR, HVPKPXX:APV:KPLS:DDSSNVI:DHHKD:VEE:SXGXH, ASAAPXX:APV:KPLS:VGRKPKV:DHHKD:VEE:XKXS, ASASPXX:APV:KPLS:IGKTPKI:DHHKD:VEE:XKXS, ASASPXX:APV:KPLS:VGRTPKV:DHHKD:VEE:XKXS, NDEGLEX:APV:KPLS:RIKPHQGQH:DHHKD:VEE:KXEXR, NDEGLEX:APV:KPLS:RIKPHQGQH:DHHKD:VEE:QXEXR, SSVKXQP:APV:KPLS:EYVRKKPKL:DHHKD:VEE:LEXAXA, RNVQXRP:APV:KPLS:EIVRKKPIF:DHHKD:VEE:LAXKXE, KIPKAXX:VPT :KPLS:DENEKVV:DHHKD:VVE:GXGXR, GIPEPXX:VPE:KPLS:DENKNVV:DHHKD:TVE:SXAXR, SIPKAXX:VPT:KPLS:DEYDKVV:DHHKD:VVE:GXGXR, HVTKPTX:APT:KPLS:DDSSNVI:DHHKD:VVR:SXGXH, YVPKPXX:APT:KPLS:DDSSNVI:DHHKD:VVR:SXGXH, TVPKPXX:APT:KPLS:DDSSNVI:DHHKD:VVR:AXGXH, AVPKAXX:APT:KPLS:DSSNNVI:DHHKD:VVK:AXGXH, KVGKAXX:VPT:KPLS:D DMGVPT:DHHKD:VAE:XGXR, KASKAXX:VPT:KPLS:DKGVVTY:DHHKD:AVS:EXGXR, GSAGPXX:TPT:KPLS:NDKQQII:DHHKD:VVD:RXGXS, AAPASXX:VPA:KPLS:DAANNVV:DHHKD:VVE:AXGXR, STPPTXX:VPT:KPLS:DSANNVV:DHHKD:VVE:SXGXR, HVPKPXX:APT:KPLS:DDSSNVI:DHHKD:VVR:SXGXH, ASAAPXX:VPQ:KPLS:VGRKPKV:DHHKD:VRS:XKXS, ASASPXX:VSQ:KPLS:IGKTPKI:DHHKD:VKS:XKXS, ASASPXX:VPQ:KPLS:VGRTPKV:DHHKD:VKS:XKXS, NDEGLEX:VPT:KPLS:RIKPHQGQH:DHHKD:QHN:KXEXR, NDEGLEX:VPT:KPLS:RIKPHQGQH:DHHKD:EHS:QXEXR, SSVKXQP:SRV:KPLS:EYVRKKPKL:DHHKD:EEH:LEXAXA, RNVQXRP:TQV:KPLS:EIVRKKPIF:DHHKD:EDH:LAXKXE, KIPKAXX:VPQ:EPLP:DENEKVV:EQLSN:VRS:GXGXR, GIPEPXX:VPQ:EPLP:DENKNVV:EQLSN:VRS:SXAXR, SIPKAXX:VPQ:EPLP:DEYDKVV:EQLSN:VRS:GXGXR, HVTKPTX:VPQ:EPLP:DDSSNVI:EQLSN:VRS:SXGXH, YVPKPXX:VPQ:EPLP:DDSSNVI:EQLSN:VRS:SXGXH, TVPKPXX:VPQ:EPLP:DDSSNVI:EQLSN:VRS:AXGXH, AVPKAXX:VPQ:EPLP:DSSNNVI:EQLSN:VRS:AXGXH, KVGKAXX:VPQ:EPLP:DDMGVPT:EQLSN:VRS:XGXR, KASKAXX:VPQ:EPLP:DKGVVTY:EQLSN:VRS:EXGXR, GSAGPXX:VPQ:EPLP:NDKQQII:EQLSN:VRS:RXGXS, AAPASXX:VPQ:EPLP:DAANNVV:EQLSN:VRS:AXGXR, STPPTXX:VPQ:EPLP:DSANNVV:EQLSN:VRS:SXGXR, HVPKPXX:VPQ:EPLP:DDSSNVI:EQLSN:VRS:SXGXH, RVPSTXX:VPQ:EPLP:DNGRVLL:EQLSN:VRS:XGXL, ASASPXX:VPQ:EPLP:IGKTPKI:EQLSN:VRS:XKXS, ASASPXX:VPQ:EPLP:VGRTPKV:EQLSN:VRS:XKXS, NDEGLEX:VPQ:EPLP:RIKPHQGQH:EQLSN:VRS:KXEXR, NDEGLEX:VPQ:EPLP:RIKPHQGQH:EQLSN:VRS:QXEXR, SSVKXQP:VPQ:EPLP:EYVRKKPKL:EQLSN:VRS:LEXAXA, RNVQXRP:VPQ:EPLP:EIVRKKPIF:EQLSN:VRS:LAXKXE, KIPKAXX:VPT:EPLP:DENEKVV:EQLSN:VVE:GXGXR, GIPEPXX:VPE:EPLP:DENKNVV:EQLSN:TVE:SXAXR, SIPKAXX:VPT:EPLP:DEYDKVV:EQLSN:VVE:GXGXR, HVTKPTX:APT:EPLP:DDSSNVI:EQLSN:VVR:SXGXH, YVPKPXX:APT:EPLP:DDSSNVI:EQLSN:VVR:SXGXH, TVPKPXX:APT:EPLP:DDSSNVI:EQLSN:VVR:AXGXH, AVPKAXX:APT:EPLP:DSSNNVI:EQLSN:VVK:AXGXH, KVGKAXX:VPT:EPLP:DDMGVPT:EQLSN:VAE:XGXR, KASKAXX:VPT:EPLP:DKGVVTY:EQLSN:AVS:EXGXR, GSAGPXX:TPT:EPLP:NDKQQII:EQLSN:VVD:RXGXS, AAPASXX:VPA:EPLP:DAANNVV:EQLSN:VVE:AXGXR, STPPTXX:VPT:EPLP:DSANNVV:EQLSN:VVE:SXGXR, HVPKPXX:APT:EPLP:DDSSNVI:EQLSN:VVR:SXGXH, RVPSTXX:APV:EPLP:DNGRVLL:EQLSN:VEE:XGXL, ASASPXX:VSQ:EPLP:IGKTPKI:EQLSN:VKS:XKXS, ASASPXX:VPQ:EPLP:VGRTPKV:EQLSN:VKS:XKXS, NDEGLEX:VPT:EPLP:RIKPHQGQH:EQLSN:QHN:KXEXR, NDEGLEX:VPT:EPLP:RIKPHQGQH:EQLSN:EHS:QXEXR, SSVKXQP:SRV:EPLP:EYVRKKPKL:EQLSN:EEH:LEXAXA, RNVQXRP:TQV:EPLP:EIVRKKPIF:EQLSN:EDH:LAXKXE, KIPKAXX:VSQ:EPLT:DENEKVV:EQLSN:VKS:GXGXR, GIPEPXX:VSQ:EPLT:DENKNVV:EQLSN:VKS:SXAXR, SIPKAXX:VSQ:EPLT:DEYDKVV:EQLSN:VKS:GXGXR, HVTKPTX:VSQ:EPLT:DDSSNVI:EQLSN:VKS:SXGXH, YVPKPXX:VSQ:EPLT:DDSSNVI:EQLSN:VKS:SXGXH, TVPKPXX:VSQ:EPLT:DDSSNVI:EQLSN:VKS:AXGXH, AVPKAXX:VSQ:EPLT:DSSNNVI:EQLSN:VKS:AXGXH, KVGKAXX:VSQ:EPLT:DDMGVPT:EQLSN:VKS:XGXR, KASKAXX:VSQ:EPLT:DKGVVTY:EQLSN:VKS:EXGXR, GSAGPXX:VSQ:EPLT:NDKQQII:EQLSN:VKS:RXGXS, AAPASXX:VSQ:EPLT:DAANNVV:EQLSN:VKS:AXGXR, STPPTXX:VSQ:EPLT:DSANNVV:EQLSN:VKS:SXGXR, HVPKPXX:VSQ:EPLT:DDSSNVI:EQLSN:VKS:SXGXH, RVPSTXX:VSQ:EPLT:DNGRVLL:EQLSN:VKS:XGXL, ASAAPXX:VSQ:EPLT:VGRKPKV:EQLSN:VKS:XKXS, ASASPXX:VSQ:EPLT:VGRTPKV:EQLSN:VKS:XKXS, NDEGLEX:VSQ:EPLT:RIKPHQGQH:EQLSN:VKS:KXEXR, NDEGLEX:VSQ:EPLT:RIKPHQGQH:EQLSN:VKS:QXEXR, SSVKXQP:VSQ:EPLT:EYVRKKPKL:EQLSN:VKS:LEXAXA, RNVQXRP:VSQ:EPLT:EIVRKKPIF:EQLSN:VKS:LAXKXE, KIPKAXX:VPT:EPLT:DENEKVV:EQLSN:VVE:GXGXR, GIPEPXX:VPE:EPLT:DENKNVV:EQLSN:TVE:SXAXR, SIPKAXX:VPT:EPLT:DEYDKVV:EQLSN:VVE:GXGXR, HVTKPTX:APT:EPLT:DDSSNVI:EQLSN:VVR:SXGXH, YVPKPXX:APT:EPLT:DDSSNVI:EQLSN:VVR:SXGXH, TVPKPXX:APT:EPLT:DDSSNVI:EQLSN:VVR:AXGXH, AVPKAXX:APT:EPLT:DSSNNVI:EQLSN:VVK:AXGXH, KVGKAXX:VPT:EPLT:DDMGVPT:EQLSN:VAE:XGXR, KASKAXX:VPT:EPLT:DKGVVTY:EQLSN:AVS:EXGXR, GSAGPXX:TPT:EPLT:NDKQQII:EQLSN:VVD:RXGXS, AAPASXX:VPA:EPLT:DAANNVV:EQLSN:VVE:AXGXR, STPPTXX:VPT:EPLT:DSANNVV:EQLSN:VVE:SXGXR, HVPKPXX:APT:EPLT:DDSSNVI:EQLSN:VVR:SXGXH, RVPSTXX:APV:EPLT:DNGRVLL:EQLSN:VEE:XGXL, ASAAPXX:VPQ:EPLT:VGRKPKV:EQLSN:VRS:XKXS, ASASPXX:VPQ:EPLT:VGRTPKV:EQLSN:VKS:XKXS, NDEGLEX:VPT:EPLT:RIKPHQGQH:EQLSN:QHN:KXEXR, NDEGLEX:VPT:EPLT:RIKPHQGQH:EQLSN:EHS:QXEXR, SSVKXQP:SRV:EPLT:EYVRKKPKL:EQLSN:EEH:LEXAXA, RNVQXRP:TQV:EPLT:EIVRKKPIF:EQLSN:EDH:LAXKXE, KIPKAXX:VPQ:EPLT:DENEKVV:EQLSN:VKS:GXGXR, GIPEPXX:VPQ:EPLT:DENKNVV:EQLSN:VKS:SXAXR, SIPKAXX:VPQ:EPLT:DEYDKVV:EQLSN:VKS:GXGXR, HVTKPTX:VPQ:EPLT:DDSSNVI:EQLSN:VKS:SXGXH, YVPKPXX:VPQ:EPLT:DDSSNVI:EQLSN:VKS:SXGXH, TVPKPXX:VPQ:EPLT:DDSSNVI:EQLSN:VKS:AXGXH, AVPKAXX:VPQ:EPLT:DSSNNVI:EQLSN:VKS:AXGXH, KVGKAXX:VPQ:EPLT:DDMGVPT:EQLSN:VKS:XGXR, KASKAXX:VPQ:EPLT:DKGVVTY:EQLSN:VKS:EXGXR, GSAGPXX:VPQ:EPLT:NDKQQII:EQLSN:VKS:RXGXS, AAPASXX:VPQ:EPLT:DAANNVV:EQLSN:VKS:AXGXR, STPPTXX:VPQ:EPLT:DSANNVV:EQLSN:VKS:SXGXR, HVPKPXX:VPQ:EPLT:DDSSNVI:EQLSN:VKS:SXGXH, RVPSTXX:VPQ:EPLT:DNGRVLL:EQLSN:VKS:XGXL, ASAAPXX:VPQ:EPLT:VGRKPKV:EQLSN:VKS:XKXS, ASASPXX:VPQ:EPLT:IGKTPKI:EQLSN:VKS:XKXS, NDEGLEX:VPQ:EPLT:RIKPHQGQH:EQLSN:VKS:KXEXR, NDEGLEX:VPQ:EPLT:RIKPHQGQH:EQLSN:VKS:QXEXR, SSVKXQP:VPQ:EPLT:EYVRKKPKL:EQLSN:VKS:LEXAXA, RNVQXRP:VPQ:EPLT:EIVRKKPIF:EQLSN:VKS:LAXKXE, ASASPXX:VSQ:EPLT:IGKTPKI:EQLSN:VKS:XKXS, KIPKAXX:VPT:SNIT:DENEKVV:IGEMS:QHN:GXGXR, GIPEPXX:VPT:SNIT:DENKNVV:IGEMS:QHN:SXAXR, SIPKAXX:VPT:SNIT:DEYDKVV:IGEMS:QHN:GXGXR, HVTKPTX:VPT:SNIT:DDSSNVI:IGEMS:QHN:SXGXH, YVPKPXX:VPT:SNIT:DDSSNVI:IGEMS:QHN:SXGXH, TVPKPXX:VPT:SNIT:DDSSNVI:IGEMS:QHN:AXGXH, AVPKAXXVPT:SNIT:DSSNNVI:IGEMS:QHN:AXGXH, KVGKAXX:VPT:SNIT:DDMGVPT:IGEMS:QHN:XGXR, KASKAXX:VPT:SNIT:DKGVVTY:IGEMS:QHN:EXGXR, GSAGPXX:VPT:SNIT :NDKQQII:IGEMS:QHN:RXGXS, AAPASXX:VPT:SNIT:DAANNVV:IGEMS:QHN:AXGXR, STPPTXX:VPT:SNIT:DSANNVV:IGEMS:QHN:SXGXR, HVPKPXX:VPT:SNIT :DDSSNVI:IGEMS:QHN:SXGXH, RVPSTXX:VPT:SNIT:DNGRVLL:IGEMS:QHN:XGXL, ASAAPXX:VPT:SNIT:VGRKPKV:IGEMS:QHN:XKXS, ASASPXX:VPT:SNIT:IGKTPKI:IGEMS:QHN:XKXS, ASASPXX:VPT:SNIT :VGRTPKV:IGEMS:QHN:XKXS, NDEGLEX:VPT:SNIT:RIKPHQGQH:IGEMS:QHN:QXEXR, NDEGLEX:VPT:SNIT:RIKPHQGQH:IGEMS:QHN:KXEXR, SSVKXQP:VPT:SNIT:EYVRKKPKL:IGEMS:QHN:LEXAXA, RNVQXRP:VPT:SNIT:EIVRKKPIF:IGEMS:QHN:LAXKXE, KIPKAXX:VPT:SNIT:DENEKVV:IGEMS:VVE:GXGXR, GIPEPXX:VPE:SNIT:DENKNVV:IGEMS:TVE:SXAXR, SIPKAXX:VPT :SNIT:DEYDKVV:IGEMS:VVE:GXGXR, HVTKPTX:APT:SNIT:DDSSNVI:IGEMS:VVR:SXGXH, YVPKPXX:APT:SN IT:DDSSNVI:IGEMS:VVR:SXGXH, TVPKPXX:APT:SN IT: DDSSNVI: IG EMS:VVR:AXGXH, AVPKAXX:APT:SNIT:DSSNNVI:IGEMS:VVK:AXGXH, KVGKAXX:VPT:SNIT:DDMGVPT:IGEMS:VAE:XGXR, KASKAXX:VPT:SNIT:DKGVVTY:IGEMS:AVS:EXGXR, GSAGPXX:TPT:SNIT:NDKQQII:IGEMS:VVD:RXGXS, AAPASXX:VPA:SNIT:DAANNVV:IGEMS:VVE:AXGXR, STPPTXXVPT:SNIT:DSANNVV:IGEMS:VVE:SXGXR, HVPKPXX:APT:SNIT:DDSSNVI:IGEMS:VVR:SXGXH, RVPSTXX:APV:SNIT:DNGRVLL:IGEMS:VEE:XGXL, ASAAPXX:VPQ:SNIT:VGRKPKV:IGEMS:VRS:XKXS, ASASPXX:VSQ:SNIT:IGKTPKI:IGEMS:VKS:XKXS, ASASPXX:VPQ:SNIT:VGRTPKV:IGEMS:VKS:XKXS, NDEGLEX:VPT:SNIT:RIKPHQGQH:IGEMS:EHS:QXEXR, SSVKXQP:SRV:SNIT:EYVRKKPKL:IGEMS:EEH:LEXAXA, RNVQXRP:TQV:SNIT:EIVRKKPIF:IGEMS:EDH:LAXKXE, KIPKAXX:VPT:SNIT:DENEKVV:LGEMS:EHS:GXGXR, GIPEPXX:VPT:SNIT:DENKNVV:LGEMS:EHS:SXAXR, SIPKAXX:VPT:SNIT:DEYDKVV:LGEMS:EHS:GXGXR, HVTKPTX:VPT:SNIT:DDSSNVI:LGEMS:EHS:SXGXH, YVPKPXXVPT:SNIT:DDSSNVI:LGEMS:EHS:SXGXH, TVPKPXX:VPT:SNIT:DDSSNVI:LGEMS:EHS:AXGXH, AVPKAXXVPT:SNIT:DSSNNVI:LGEMS:EHS:AXGXH, KVGKAXX:VPT:SNIT:DDMGVPT:LGEMS:EHS:XGXR, KASKAXX:VPT:SNIT:DKGVVTY:LGEMS:EHS:EXGXR, GSAGPXXVPT:SNIT:NDKQQII:LGEMS:EHS:RXGXS, AAPASXX:VPT:SNIT:DAANNVV:LGEMS:EHS:AXGXR, STPPTXX:VPT:SNIT:DSANNVV:LGEMS:EHS:SXGXR, HVPKPXX:VPT:SNIT:DDSSNVI:LGEMS:EHS:SXGXH, RVPSTXX:VPT:SNIT:DNGRVLL:LGEMS:EHS:XGXL, ASAAPXX:VPT:SNIT:VGRKPKV:LGEMS:EHS:XKXS, ASASPXXVPT:SNIT:IGKTPKI:LGEMS:EHS:XKXS, ASASPXX:VPT:SNIT:VGRTPKV:LGEMS:EHS:XKXS, NDEGLEX:VPT:SNIT:RIKPHQGQH:LGEMS:EHS:KXEXR, SSVKXQP:VPT:SNIT:EYVRKKPKL:LGEMS:EHS:LEXAXA, RNVQXRP:VPT:SNIT:EIVRKKPIF:LGEMS:EHS:LAXKXE, KIPKAXX:VPT:SNIT:DENEKVV:LGEMS:VVE:GXGXR, GIPEPXX:VPE:SNIT:DENKNVV:LGEMS:TVE:SXAXR, SIPKAXX:VPT:SNIT:DEYDKVV:LGEMS:VVE:GXGXR, HVTKPTX:APT:SNIT:DDSSNVI:LGEMS:VVR:SXGXH, YVPKPXX:APT:SNIT:DDSSNVI:LGEMS:VVR:SXGXH, TVPKPXX:APT:SNIT:DDSSNVI:LGEMS:VVR:AXGXH, AVPKAXX:APT:SNIT:DSSNNVI:LGEMS:VVK:AXGXH, KVGKAXX:VPT:SNIT:DDMGVPT:LGEMS:VAE:XGXR, KASKAXX:VPT:SNIT:DKGVVTY:LGEMS:AVS:EXGXR, GSAGPXX:TPT:SNIT:NDKQQII:LGEMS:VVD:RXGXS, AAPASXX:VPA:SNIT:DAANNVV:LGEMS:VVE:AXGXR, STPPTXX:VPT:SNIT:DSANNVV:LGEMS:VVE:SXGXR, HVPKPXX:APT:SNIT:DDSSNVI:LGEMS:VVR:SXGXH, RVPSTXX:APV:SNIT:DNGRVLL:LGEMS:VEE:XGXL, ASAAPXX:VPQ:SNIT:VGRKPKV:LGEMS:VRS:XKXS, ASASPXX:VSQ:SNIT:IGKTPKI:LGEMS:VKS:XKXS, ASASPXX:VPQ:SNIT:VGRTPKV:LGEMS:VKS:XKXS, NDEGLEX:VPT:SNIT:RIKPHQGQH:LGEMS:QHN:KXEXR, SSVKXQP:SRV:SNIT:EYVRKKPKL:LGEMS:EEH:LEXAXA, RNVQXRP:TQV:SNIT:EIVRKKPIF:LGEMS:EDH:LAXKXE, KIPKAXX:SRV:RSVK:DENEKVV:KEVQV:EEH:GXGXR, GIPEPXX:SRV:RSVK:DENKNVV:KEVQV:EEH:SXAXR, SIPKAXX:SRV:RSVK:DEYDKVV:KEVQV:EEH:GXGXR, HVTKPTX:SRV:RSVK:DDSSNVI:KEVQV:EEH:SXGXH, YVPKPXX:SRV:RSVK:DDSSNVI:KEVQV:EEH:SXGXH, TVPKPXX:SRV:RSVK:DDSSNVI:KEVQV:EEH:AXGXH, AVPKAXX:SRV:RSVK:DSSNNVI:KEVQV:EEH:AXGXH, KVGKAXX:SRV:RSVK:DDMGVPT:KEVQV:EEH:XGXR, KASKAXX:SRV:RSVK:DKGVVTY:KEVQV:EEH:EXGXR, GSAGPXX:SRV:RSVK:NDKQQII:KEVQV:EEH:RXGXS, AAPASXX:SRV:RSVK:DAANNVV:KEVQV:EEH:AXGXR, STPPTXX:SRV:RSVK:DSANNVV:KEVQV:EEH:SXGXR, HVPKPXX:SRV:RSVK:DDSSNVI:KEVQV:EEH:SXGXH, RVPSTXX:SRV:RSVK:DNGRVLL:KEVQV:EEH:XGXL, ASAAPXX:SRV:RSVK:VGRKPKV:KEVQV:EEH:XKXS, ASASPXX:SRV:RSVK:IGKTPKI:KEVQV:EEH:XKXS, ASASPXX:SRV:RSVK:VGRTPKV:KEVQV:EEH:XKXS, NDEGLEX:SRV:RSVK:RIKPHQGQH:KEVQV:EEH:KXEXR, NDEGLEX:SRV:RSVK:RIKPHQGQH:KEVQV:EEH:QXEXR, RNVQXRP:SRV:RSVK:EIVRKKPIF:KEVQV:EEH:LAXKXE, KIPKAXX:VPT:RSVK:DENEKVV:KEVQV:VVE:GXGXR, GIPEPXX:VPE:RSVK:DENKNVV:KEVQV:TVE:SXAXR, SIPKAXX:VPT:RSVK:DEYDKVV:KEVQV:VVE:GXGXR, HVTKPTX:APT:RSVK:DDSSNVI:KEVQV:VVR:SXGXH, YVPKPXX:APT:RSVK:DDSSNVI:KEVQV:VVR:SXGXH, TVPKPXX:APT:RSVK:DDSSNVI:KEVQV:VVR:AXGXH, AVPKAXX:APT:RSVK:DSSNNVI:KEVQV:VVK:AXGXH, KVGKAXX:VPT:RSVK:DDMGVPT:KEVQV:VAE:XGXR, KASKAXX:VPT:RSVK:DKGVVTY:KEVQV:AVS:EXGXR, GSAGPXX:TPT:RSVK:NDKQQII:KEVQV:VVD:RXGXS, AAPASXX:VPA:RSVK:DAANNVV:KEVQV:VVE:AXGXR, STPPTXX:VPT:RSVK:DSANNVV:KEVQV:VVE:SXGXR, HVPKPXX:APT:RSVK:DDSSNVI:KEVQV:VVR:SXGXH, RVPSTXX:APV:RSVK:DNGRVLL:KEVQV:VEE:XGXL, ASAAPXX:VPQ:RSVK:VGRKPKV:KEVQV:VRS:XKXS, ASASPXX:VSQ:RSVK:IGKTPKI:KEVQV:VKS:XKXS, ASASPXX:VPQ:RSVK:VGRTPKV:KEVQV:VKS:XKXS, NDEGLEX:VPT:RSVK:RIKPHQGQH:KEVQV:QHN:KXEXR, NDEGLEX:VPT:RSVK:RIKPHQGQH:KEVQV:EHS:QXEXR, RNVQXRP:TQV:RSVK:EIVRKKPIF:KEVQV:EDH:LAXKXE, KIPKAXX:TQV:RPVQ:DENEKVV:KKATV:EDH:GXGXR, GIPEPXX:TQV:RPVQ:DENKNVV:KKATV:EDH:SXAXR, SIPKAXX:TQV:RPVQ:DEYDKVV:KKATV:EDH:GXGXR, HVTKPTX:TQV:RPVQ:DDSSNVI:KKATV:EDH:SXGXH, YVPKPXX:TQV:RPVQ:DDSSNVI:KKATV:EDH:SXGXH, TVPKPXX:TQV:RPVQ:DDSSNVI:KKATV:EDH:AXGXH, AVPKAXX:TQV:RPVQ:DSSNNVI:KKATV:EDH:AXGXH, KVGKAXX:TQV:RPVQ:DDMGVPT:KKATV:EDH:XGXR, KASKAXX:TQV:RPVQ:DKGVVTY:KKATV:EDH:EXGXR, GSAGPXX:TQV:RPVQ:NDKQQII:KKATV:EDH:RXGXS, AAPASXX:TQV:RPVQ:DAANNVV:KKATV:EDH:AXGXR, STPPTXX:TQV:RPVQ:DSANNVV:KKATV:EDH:SXGXR, HVPKPXX:TQV:RPVQ:DDSSNVI:KKATV:EDH:SXGXH, RVPSTXX:TQV:RPVQ:DNGRVLL:KKATV:EDH:XGXL, ASAAPXX:TQV:RPVQ:VGRKPKV:KKATV:EDH:XKXS, ASASPXX:TQV:RPVQ:IGKTPKI:KKATV:EDH:XKXS, ASASPXX:TQV:RPVQ:VGRTPKV:KKATV:EDH:XKXS, NDEGLEX:TQV:RPVQ:RIKPHQGQH:KKATV:EDH:KXEXR, NDEGLEX:TQV:RPVQ:RIKPHQGQH:KKATV:EDH:QXEXR, SSVKXQP:TQV:RPVQ:EYVRKKPKL:KKATV:EDH:LEXAXA, KIPKAXX:VPT:RPVQ:DENEKVV:KKATV:VVE:GXGXR, GIPEPXX:VPE:RPVQ:DENKNVV:KKATV:TVE:SXAXR, SIPKAXX:VPT:RPVQ:DEYDKVV:KKATV:VVE:GXGXR, HVTKPTX:APT:RPVQ:DDSSNVI:KKATV:VVR:SXGXH, YVPKPXX:APT:RPVQ:DDSSNVI:KKATV:VVR:SXGXH, TVPKPXX:APT:RPVQ:DDSSNVI:KKATV:VVR:AXGXH, AVPKAXX:APT:RPVQ:DSSNNVI:KKATV:VVK:AXGXH, KVGKAXX:VPT:RPVQ:DDMGVPT:KKATV:VAE:XGXR, KASKAXX:VPT:RPVQ:DKGVVTY:KKATV:AVS:EXGXR, GSAGPXX:TPT:RPVQ:NDKQQII:KKATV:VVD:RXGXS, AAPASXX:VPA:RPVQ:DAANNVV:KKATV:VVE:AXGXR, STPPTXX:VPT:RPVQ:DSANNVV:KKATV:VVE:SXGXR, HVPKPXX:APT:RPVQ:DDSSNVI:KKATV:VVR:SXGXH, RVPSTXX:APV:RPVQ:DNGRVLL:KKATV:VEE:XGXL, ASAAPXX:VPQ:RPVQ:VGRKPKV:KKATV:VRS:XKXS, ASASPXX:VSQ:RPVQ:IGKTPKI:KKATV:VKS:XKXS, ASASPXX:VPQ:RPVQ:VGRTPKV:KKATV:VKS:XKXS, NDEGLEX:VPT:RPVQ:RIKPHQGQH:KKATV:QHN:KXEXR, NDEGLEX:VPT:RPVQ:RIKPHQGQH:KKATV:EHS:QXEXR and SSVKXQP:SRV:RPVQ:EYVRKKPKL:KKATV:EEH:LEXAXA. More particularly, in certain embodiments, the heptuplet PEP7:PEP3:PEP1:LINKER:PEP2:PEP4:PEP8 is selected from the group consisting of GIPEPXX:VPT:SAIS:DENKNVV:LKNYQ:VVE:SXAXR, HVTKPTX:VPT:SAIS:DDSSNVI:LKNYQ:VVE:SXGXH, YVPKPXX:VPT:SAIS:DDSSNVI:LKNYQ:VVE:SXGXH, TVPKPXX:VPT:SAIS:DDSSNVI:LKNYQ:VVE:AXGXH, AVPKAXX:VPT:SAIS:DSSNNVI:LKNYQ:VVE:AXGXH, KVGKAXX:VPT:SAIS:DDMGVPT:LKNYQ:VVE:XGXR, KASKAXX:VPT:SAIS:DKGVVTY:LKNYQ:VVE:EXGXR, GSAGPXX:VPT:SAIS:NDKQQII:LKNYQ:VVE:RXGXS, AAPASXX:VPT:SAIS:DAANNVV:LKNYQ:VVE:AXGXR, STPPTXX:VPT:SAIS:DSANNVV:LKNYQ:VVE:SXGXR, HVPKPXX:VPT:SAIS:DDSSNVI:LKNYQ:VVE:SXGXH, RVPSTXX:VPT:SAIS:DNGRVLL:LKNYQ:VVE:XGXL, ASAAPXX:VPT:SAIS:VGRKPKV:LKNYQ:VVE:XKXS,ASASPXX:VPT:SAIS:IGKTPKI:LKNYQ:VVE:XKXS, ASASPXX:VPT:SAIS:VGRTPKV:LKNYQ:VVE:XKXS, GIPEPXX:VPE:SAIS:DENKNVV:LKNYQ:TVE:SXAXR, HVTKPTX:APT:SAIS:DDSSNVI:LKNYQ:VVR:SXGXH, YVPKPXX:APT:SAIS:DDSSNVI:LKNYQ:VVR:SXGXH, TVPKPXX:APT:SAIS:DDSSNVI:LKNYQ:VVR:AXGXH, AVPKAXX:APT:SAIS:DSSNNVI:LKNYQ:VVK:AXGXH, KVGKAXX:VPT:SAIS:DDMGVPT:LKNYQ:VAE:XGXR, KASKAXX:VPT:SAIS:DKGVVTY:LKNYQ:AVS:EXGXR, GSAGPXX:TPT:SAIS:NDKQQII:LKNYQ:VVD:RXGXS, AAPASXX:VPA:SAIS:DAANNVV:LKNYQ:VVE:AXGXR, HVPKPXX:APT:SAIS:DDSSNVI:LKNYQ:VVR:SXGXH, RVPSTXX:APV:SAIS:DNGRVLL:LKNYQ:VEE:XGXL, ASAAPXX:VPQ:SAIS:VGRKPKV:LKNYQ:VRS:XKXS, ASASPXX:VSQ:SAIS:IGKTPKI:LKNYQ:VKS:XKXS, ASASPXX:VPQ:SAIS:VGRTPKV:LKNYQ:VKS:XKXS, NDEGLEX:VPT:SAIS:RIKPHQGQH:LKNYQ:QHN:KXEXR, NDEGLEX:VPT:SAIS:RIKPHQGQH:LKNYQ:EHS:QXEXR, SSVKXQP:SRV:SAIS:EYVRKKPKL:LKNYQ:EEH:LEXAXA, RNVQXRP:TQV:SAIS:EIVRKKPIF:LKNYQ:EDH:LAXKXE, KIPKAXX:VPE:SSLS:DENEKVV:LKVYP:TVE:GXGXR, SIPKAXX:VPE:SSLS:DEYDKVV:LKVYP:TVE:GXGXR, HVTKPTX:VPE:SSLS:DDSSNVI:LKVYP:TVE:SXGXH, YVPKPXX:VPE:SSLS:DDSSNVI:LKVYP:TVE:SXGXH, TVPKPXX:VPE:SSLS:DDSSNVI:LKVYP:TVE:AXGXH, AVPKAXX:VPE:SSLS:DSSNNVI:LKVYP:TVE:AXGXH, KVGKAXX:VPE:SSLS:DDMGVPT:LKVYP:TVE:XGXR, KASKAXX:VPE:SSLS:DKGVVTY:LKVYP:TVE:EXGXR, GSAGPXX:VPE:SSLS:NDKQQII:LKVYP:TVE:RXGXS, AAPASXX:VPE:SSLS:DAANNVV:LKVYP:TVE:AXGXR, STPPTXX:VPE:SSLS:DSANNVV:LKVYP:TVE:SXGXR, HVPKPXX:VPE:SSLS:DDSSNVI:LKVYP:TVE:SXGXH, RVPSTXX:VPE:SSLS:DNGRVLL:LKVYP:TVE:XGXL, ASAAPXX:VPE:SSLS:VGRKPKV:LKVYP:TVE:XKXS, ASASPXX:VPE:SSLS:IGKTPKI:LKVYP:TVE:XKXS, ASASPXX:VPE:SSLS:VGRTPKV:LKVYP:TVE:XKXS, KIPKAXX:VPT:SSLS:DENEKVV:LKVYP:VVE:GXGXR, SIPKAXX:VPT:SSLS:DEYDKVV:LKVYP:VVE:GXGXR, HVTKPTX:APT:SSLS:DDSSNVI:LKVYP:VVR:SXGXH, YVPKPXX:APT:SSLS:DDSSNVI:LKVYP:VVR:SXGXH, TVPKPXX:APT:SSLS:DDSSNVI:LKVYP:VVR:AXGXH, AVPKAXX:APT:SSLS:DSSNNVI:LKVYP:VVK:AXGXH, KVGKAXX:VPT:SSLS:DDMGVPT:LKVYP:VAE:XGXR, KASKAXX:VPT:SSLS:DKGVVTY:LKVYP:AVS:EXGXR,

GSAGPXX:TPT:SSLS:NDKQQII:LKVYP:VVD:RXGXS, AAPASXX:VPA:SSLS:DAANNVV:LKVYP:VVE:AXGXR, STPPTXX:VPT:SSLS:DSANNVV:LKVYP:VVE:SXGXR, HVPKPXX:APT:SSLS:DDSSNVI:LKVYP:VVR:SXGXH, RVPSTXX:APV:SSLS:DNGRVLL:LKVYP:VEE:XGXL, ASAAPXX:VPQ:SSLS:VGRKPKV:LKVYP:VRS:XKXS, ASASPXX:VSQ:SSLS:IGKTPKI:LKVYP:VKS:XKXS, ASASPXX:VPQ:SSLS:VGRTPKV:LKVYP:VKS:XKXS, NDEGLEX:VPT:SSLS:RIKPHQGQH:LKVYP:QHN:KXEXR, NDEGLEX:VPT:SSLS:RIKPHQGQH:LKVYP:EHS:QXEXR, SSVKXQP:SRV:SSLS:EYVRKKPKL:LKVYP:EEH:LEXAXA, RNVQXRP:TQV:SSLS:EIVRKKPIF:LKVYP:EDH:LAXKXE, KIPKAXX:APT:NAIS:DENEKVV:LKKYR:VVR:GXGXR, GIPEPXX:APT:NAIS:DENKNVV:LKKYR:VVR:SXAXR, SIPKAXX:APT:NAIS:DEYDKVV:LKKYR:VVR:GXGXR, AVPKAXX:APT:NAIS:DSSNNVI:LKKYR:VVR:AXGXH, KVGKAXX:APT:NAIS:DDMGVPT:LKKYR:VVR:XGXR, KASKAXX:APT:NAIS:DKGVVTY:LKKYR:VVR:EXGXR, GSAGPXX:APT:NAIS:NDKQQII:LKKYR:VVR:RXGXS, AAPASXX:APT:NAIS:DAANNVV:LKKYR:VVR:AXGXR, STPPTXX:APT:NAIS:DSANNVV:LKKYR:VVR:SXGXR, RVPSTXX:APT:NAIS:DNGRVLL:LKKYR:VVR:XGXL, ASAAPXX:APT:NAIS:VGRKPKV:LKKYR:VVR:XKXS, ASASPXX:APT:NAIS:IGKTPKI:LKKYR:VVR:XKXS, ASASPXX:APT:NAIS:VGRTPKV:LKKYR:VVR:XKXS, KIPKAXX:VPT:NAIS:DENEKVV:LKKYR:VVE:GXGXR, GIPEPXX:VPE:NAIS:DENKNVV:LKKYR:TVE:SXAXR, SIPKAXX:VPT:NAIS:DEYDKVV:LKKYR:VVE:GXGXR, AVPKAXX:APT:NAIS:DSSNNVI:LKKYR:VVK:AXGXH, KVGKAXX:VPT:NAIS:DDMGVPT:LKKYR:VAE:XGXR, KASKAXX:VPT:NAIS:DKGVVTY:LKKYR:AVS:EXGXR, GSAGPXX:TPT:NAIS:NDKQQII:LKKYR:VVD:RXGXS, AAPASXX:VPA:NAIS:DAANNVV:LKKYR:VVE:AXGXR, STPPTXX:VPT:NAIS:DSANNVV:LKKYR:VVE:SXGXR, RVPSTXX:APV:NAIS:DNGRVLL:LKKYR:VEE:XGXL, ASAAPXX:VPQ:NAIS:VGRKPKV:LKKYR:VRS:XKXS, ASASPXX:VSQ:NAIS:IGKTPKI:LKKYR:VKS:XKXS, ASASPXX:VPQ: NAIS:VGRTPKV:LKKYR:VKS:XKXS, NDEGLEX:VPT:NAIS:RIKPHQGQH:LKKYR:QHN:KXEXR, NDEGLEX:VPT:NAIS:RIKPHQGQH:LKKYR:EHS:QXEXR, SSVKXQP:SRV:NAIS:EYVRKKPKL:LKKYR:EEH:LEXAXA, RNVQXRP:TQV:NAIS:EIVRKKPIF:LKKYR:EDH:LAXKXE, KIPKAXX:APT:SATS:DENEKVV:LRKHR:VVK:GXGXR, GIPEPXX:APT:SATS:DENKNVV:LRKHR:VVK:SXAXR, SIPKAXX:APT:SATS:DEYDKVV:LRKHR:VVK:GXGXR, HVTKPTX:APT:SATS:DDSSNVI:LRKHR:VVK:SXGXH, YVPKPXX:APT:SATS:DDSSNVI:LRKHR:VVK:SXGXH, TVPKPXX:APT:SATS:DDSSNVI:LRKHR:VVK:AXGXH, KVGKAXX:APT:SATS:DDMGVPT:LRKHR:VVK:XGXR, KASKAXX:APT:SATS:DKGVVTY:LRKHR:VVK:EXGXR, GSAGPXX:APT:SATS:NDKQQII:LRKHR:VVK:RXGXS, AAPASXX:APT:SATS:DAANNVV:LRKHR:VVK:AXGXR, STPPTXX:APT:SATS:DSANNVV:LRKHR:VVK:SXGXR, HVPKPXX:APT:SATS:DDSSNVI:LRKHR:VVK:SXGXH, RVPSTXX:APT:SATS:DNGRVLL:LRKHR:VVK:XGXL, ASAAPXX:APT:SATS:VGRKPKV:LRKHR:VVK:XKXS, ASASPXX:APT:SATS:IGKTPKI:LRKHR:VVK:XKXS, ASASPXX:APT:SATS:VGRTPKV:LRKHR:VVK:XKXS, KIPKAXX:VPT:SATS:DENEKVV:LRKHR:VVE:GXGXR, GIPEPXX:VPE:SATS:DENKNVV:LRKHR:TVE:SXAXR, SIPKAXX:VPT:SATS:DEYDKVV:LRKHR:VVE:GXGXR, HVTKPTX:APT:SATS:DDSSNVI:LRKHR:VVR:SXGXH, YVPKPXX:APT:SATS:DDSSNVI:LRKHR:VVR:SXGXH, TVPKPXX:APT:SATS:DDSSNVI:LRKHR:VVR:AXGXH, KVGKAXX:VPT:SATS:DDMGVPT:LRKHR:VAE:XGXR, KASKAXX:VPT:SATS:DKGVVTY:LRKHR:AVS:EXGXR, GSAGPXX:TPT:SATS:NDKQQII:LRKHR:VVD:RXGXS, AAPASXX:VPA:SATS:DAANNVV:LRKHR:VVE:AXGXR, STPPTXX:VPT:SATS:DSANNVV:LRKHR:VVE:SXGXR, HVPKPXX:APT:SATS:DDSSNVI:LRKHR:VVR:SXGXH, RVPSTXX:APV:SATS:DNGRVLL:LRKHR:VEE:XGXL, ASAAPXX:VPQ:SATS:VGRKPKV:LRKHR:VRS:XKXS, ASASPXX:VSQ:SATS:IGKTPKI:LRKHR:VKS:XKXS, ASASPXX:VPQ:SATS:VGRTPKV:LRKHR:VKS:XKXS, NDEGLEX:VPT:SATS:RIKPHQGQH:LRKHR:QHN:KXEXR, NDEGLEX:VPT:SATS:RIKPHQGQH:LRKHR:EHS:QXEXR, SSVKXQP:SRV:SATS:EYVRKKPKL:LRKHR:EEH:LEXAXA, RNVQXRP:TQV:SATS:EIVRKKPIF:LRKHR:EDH:LAXKXE, KIPKAXX:VPT:SPIS:DENEKVV:LKYHY:VAE:GXGXR, GIPEPXX:VPT:SPIS:DENKNVV:LKYHY:VAE:SXAXR, SIPKAXX:VPT:SPIS:DEYDKVV:LKYHY:VAE:GXGXR, HVTKPTX:VPT:SPIS:DDSSNVI:LKYHY:VAE:SXGXH, YVPKPXX:VPT:SPIS:DDSSNVI:LKYHY:VAE:SXGXH, TVPKPXX:VPT:SPIS:DDSSNVI:LKYHY:VAE:AXGXH, AVPKAXX:VPT:SPIS:DSSNNVI:LKYHY:VAE:AXGXH, KASKAXX:VPT:SPIS:DKGVVTY:LKYHY:VAE:EXGXR, GSAGPXX:VPT:SPIS:NDKQQII:LKYHY:VAE:RXGXS, AAPASXX:VPT:SPIS:DAANNVV:LKYHY:VAE:AXGXR, STPPTXX:VPT:SPIS:DSANNVV:LKYHY:VAE:SXGXR, HVPKPXX:VPT:SPIS:DDSSNVI:LKYHY:VAE:SXGXH, RVPSTXX:VPT:SPIS:DNGRVLL:LKYHY:VAE:XGXL, ASAAPXX:VPT:SPIS:VGRKPKV:LKYHY:VAE:XKXS, ASASPXX:VPT:SPIS:IGKTPKI:LKYHY:VAE:XKXS, ASASPXX:VPT:SPIS:VGRTPKV:LKYHY:VAE:XKXS, KIPKAXX:VPT:SPIS:DENEKVV:LKYHY:VVE:GXGXR, GIPEPXX:VPE:SPIS:DENKNVV:LKYHY:TVE:SXAXR, SIPKAXX:VPT:SPIS:DEYDKVV:LKYHY:VVE:GXGXR, HVTKPTX:APT:SPIS:DDSSNVI:LKYHY:VVR:SXGXH, YVPKPXX:APT:SPIS:DDSSNVI: LKYHY:VVR:SXGXH, TVPKPXX:APT:SPIS:DDSSNVI:LKYHY:VVR:AXGXH, AVPKAXX:APT:SPIS:DSSNNVI:LKYHY:VVK:AXGXH, KASKAXX:VPT:SPIS:DKGVVTY:LKYHY:AVS:EXGXR, GSAGPXX:TPT:SPIS:NDKQQII:LKYHY:VVD:RXGXS, AAPASXX:VPA:SPIS:DAANNVV:LKYHY:VVE:AXGXR, STPPTXX:VPT:SPIS: DSANNVV:LKYHY:VVE:SXGXR, HVPKPXX:APT:SPIS:DDSSNVI:LKYHY:VVR:SXGXH, RVPSTXX:APV:SPIS:DNGRVLL:LKYHY:VEE:XGXL, ASAAPXX:VPQ:SPIS:VGRKPKV:LKYHY:VRS:XKXS, ASASPXX:VSQ:SPIS:IGKTPKI:LKYHY:VKS:XKXS, ASASPXX:VPQ:SPIS:VGRTPKV:LKYHY:VKS:XKXS, NDEGLEX:VPT:SPIS:RIKPHQGQH:LKYHY:QHN:KXEXR, NDEGLEX:VPT:SPIS:RIKPHQGQH:LKYHY:EHS:QXEXR, SSVKXQP:SRV:SPIS:EYVRKKPKL:LKYHY:EEH:LEXAXA, RNVQXRP:TQV:SPIS:EIVRKKPIF:LKYHY:EDH:LAXKXE, KIPKAXX:VPT:EPIS:DENEKVV:KFKYE:AVS:GXGXR,

GIPEPXX:VPT:EPIS:DENKNVV:KFKYE:AVS:SXAXR, SIPKAXX:VPT:EPIS:DEYDKVV:KFKYE:AVS:GXGXR, HVTKPTX:VPT:EPIS:DDSSNVI:KFKYE:AVS:SXGXH, YVPKPXX:VPT:EPIS:DDSSNVI:KFKYE:AVS:SXGXH, TVPKPXX:VPT:EPIS:DDSSNVI:KFKYE:AVS:AXGXH, AVPKAXX:VPT:EPIS:DSSNNVI:KFKYE:AVS:AXGXH, KVGKAXX:VPT:EPIS:DDMGVPT:KFKYE:AVS:XGXR, GSAGPXX:VPT:EPIS:NDKQQII:KFKYE:AVS:RXGXS, AAPASXX:VPT:EPIS:DAANNVV:KFKYE:AVS:AXGXR, STPPTXX:VPT:EPIS:DSANNVV:KFKYE:AVS:SXGXR, HVPKPXX:VPT:EPIS:DDSSNVI:KFKYE:AVS:SXGXH, RVPSTXX:VPT:EPIS:DNGRVLL:KFKYE:AVS:XGXL, ASAAPXX:VPT:EPIS:VGRKPKV:KFKYE:AVS:XKXS, ASASPXX:VPT:EPIS:IGKTPKI:KFKYE:AVS:XKXS, ASASPXX:VPT:EPIS:VGRTPKV:KFKYE:AVS:XKXS, KIPKAXX:VPT:EPIS:DENEKVV:KFKYE:VVE:GXGXR, GIPEPXX:VPE:EPIS:DENKNVV:KFKYE:TVE:SXAXR, SIPKAXX:VPT:EPIS:DEYDKVV:KFKYE:VVE:GXGXR, HVTKPTX:APT:EPIS:DDSSNVI:KFKYE:VVR:SXGXH, YVPKPXX:APT:EPIS:DDSSNVI:KFKYE:VVR:SXGXH, TVPKPXX:APT:EPIS:DDSSNVI:KFKYE:VVR:AXGXH, AVPKAXX:APT:EPIS:DSSNNVI:KFKYE:VVK:AXGXH, KVGKAXX:VPT:EPIS:DDMGVPT:KFKYE:VAE:XGXR, GSAGPXX:TPT:EPIS:NDKQQII:KFKYE:VVD:RXGXS, AAPASXX:VPA:EPIS:DAANNVV:KFKYE:VVE:AXGXR, STPPTXX:VPT:EPIS:DSANNVV:KFKYE:VVE:SXGXR, HVPKPXX:APT:EPIS:DDSSNVI:KFKYE:VVR:SXGXH, RVPSTXX:APV:EPIS:DNGRVLL:KFKYE:VEE:XGXL, ASAAPXX:VPQ:EPIS:VGRKPKV:KFKYE:VRS:XKXS, ASASPXX:VSQ:EPIS:IGKTPKI:KFKYE:VKS:XKXS, ASASPXX:VPQ:EPIS:VGRTPKV:KFKYE:VKS:XKXS, NDEGLEX:VPT:EPIS:RIKPHQGQH:KFKYE:QHN:KXEXR, NDEGLEX:VPT:EPIS:RIKPHQGQH:KFKYE:EHS:QXEXR, SSVKXQP:SRV:EPIS:EYVRKKPKL:KFKYE:EEH:LEXAXA, RNVQXRP:TQV:EPIS:EIVRKKPIF:KFKYE:EDH:LAXKXE, KIPKAXX:TPT:SPIN:DENEKVV:YGKIP:VVD:GXGXR, GIPEPXX:TPT:SPIN:DENKNVV:YGKIP:VVD:SXAXR, SIPKAXX:TPT:SPIN:DEYDKVV:YGKIP:VVD:GXGXR, HVTKPTX:TPT:SPIN:DDSSNVI:YGKIP:VVD:SXGXH, YVPKPXX:TPT:SPIN:DDSSNVI:YGKIP:VVD:SXGXH, TVPKPXX:TPT:SPIN:DDSSNVI:YGKIP:VVD:AXGXH, AVPKAXX:TPT:SPIN:DSSNNVI:YGKIP:VVD:AXGXH, KVGKAXX:TPT:SPIN:DDMGVPT:YGKIP:VVD:XGXR, KASKAXX:TPT:SPIN:DKGVVTY:YGKIP:VVD:EXGXR, AAPASXX:TPT:SPIN:DAANNVV:YGKIP:VVD:AXGXR, STPPTXX:TPT:SPIN:DSANNVV:YGKIP:VVD:SXGXR, HVPKPXX:TPT:SPIN:DDSSNVI:YGKIP:VVD:SXGXH, RVPSTXX:TPT:SPIN:DNGRVLL:YGKIP:VVD:XGXL, ASAAPXX:TPT:SPIN:VGRKPKV:YGKIP:VVD:XKXS, ASASPXX:TPT:SPIN:IGKTPKI:YGKIP:VVD:XKXS, ASASPXX:TPT:SPIN:VGRTPKV:YGKIP:VVD:XKXS, KIPKAXX:VPT:SPIN:DENEKVV:YGKIP:VVE:GXGXR, GIPEPXX:VPE:SPIN:DENKNVV:YGKIP:TVE:SXAXR, SIPKAXX:VPT:SPIN:DEYDKVV:YGKIP:VVE:GXGXR, HVTKPTX:APT:SPIN:DDSSNVI:YGKIP:VVR:SXGXH, YVPKPXX:APT:SPIN:DDSSNVI:YGKIP:VVR:SXGXH, TVPKPXX:APT:SPIN:DDSSNVI:YGKIP:VVR:AXGXH, AVPKAXX:APT:SPIN:DSSNNVI:YGKIP:VVK:AXGXH, KVGKAXX:VPT:SPIN:DDMGVPT:YGKIP:VAE:XGXR, KASKAXX:VPT:SPIN:DKGVVTY:YGKIP:AVS:EXGXR, AAPASXX:VPA:SPIN:DAANNVV:YGKIP:VVE:AXGXR, STPPTXX:VPT:SPIN:DSANNVV:YGKIP:VVE:SXGXR, HVPKPXX:APT:SPIN:DDSSNVI:YGKIP:VVR:SXGXH, RVPSTXX:APV:SPIN:DNGRVLL:YGKIP:VEE:XGXL, ASAAPXX:VPQ:SPIN:VGRKPKV:YGKIP:VRS:XKXS, ASASPXX:VSQ:SPIN:IGKTPKI:YGKIP:VKS:XKXS, ASASPXX:VPQ:SPIN:VGRTPKV:YGKIP:VKS:XKXS, NDEGLEX:VPT:SPIN:RIKPHQGQH:YGKIP:QHN:KXEXR, NDEGLEX:VPT:SPIN:RIKPHQGQH:YGKIP:EHS:QXEXR, SSVKXQP:SRV:SPIN:EYVRKKPKL:YGKIP:EEH:LEXAXA, RNVQXRP:TQV:SPIN:EIVRKKPIF:YGKIP:EDH:LAXKXE, KIPKAXX:VPA:SPIS:DENEKVV:YKQYE:VVE:GXGXR, GIPEPXX:VPA:SPIS:DENKNVV:YKQYE:VVE:SXAXR, SIPKAXX:VPA:SPIS:DEYDKVV:YKQYE:VVE:GXGXR, HVTKPTX:VPA:SPIS:DDSSNVI:YKQYE:VVE:SXGXH, YVPKPXX:VPA:SPIS:DDSSNVI:YKQYE:VVE:SXGXH, TVPKPXX:VPA:SPIS:DDSSNVI:YKQYE:VVE:AXGXH, AVPKAXX:VPA:SPIS:DSSNNVI:YKQYE:VVE:AXGXH, KVGKAXX:VPA:SPIS:DDMGVPT:YKQYE:VVE:XGXR, KASKAXX:VPA:SPIS:DKGVVTY:YKQYE:VVE:EXGXR, GSAGPXX:VPA:SPIS:NDKQQII:YKQYE:VVE:RXGXS, STPPTXX:VPA:SPIS:DSANNVV:YKQYE:VVE:SXGXR, HVPKPXX:VPA:SPIS:DDSSNVI:YKQYE:VVE:SXGXH, RVPSTXX:VPA:SPIS:DNGRVLL:YKQYE:VVE:XGXL, ASAAPXX:VPA:SPIS:VGRKPKV:YKQYE:VVE:XKXS, ASASPXX:VPA:SPIS:IGKTPKI:YKQYE:VVE:XKXS, ASASPXX:VPA:SPIS:VGRTPKV:YKQYE:VVE:XKXS, KIPKAXX:VPT:SPIS:DENEKVV:YKQYE:VVE:GXGXR, GIPEPXX:VPE:SPIS:DENKNVV:YKQYE:TVE:SXAXR, SIPKAXX:VPT:SPIS:DEYDKVV:YKQYE:VVE:GXGXR, HVTKPTX:APT:SPIS:DDSSNVI:YKQYE:VVR:SXGXH, YVPKPXX:APT:SPIS:DDSSNVI:YKQYE:VVR:SXGXH, TVPKPXX:APT:SPIS:DDSSNVI:YKQYE:VVR:AXGXH, AVPKAXX:APT:SPIS:DSSNNVI:YKQYE:VVK:AXGXH, KVGKAXX:VPT:SPIS:DDMGVPT:YKQYE:VAE:XGXR, KASKAXX:VPT:SPIS:DKGVVTY:YKQYE:AVS:EXGXR, GSAGPXX:TPT:SPIS:NDKQQII:YKQYE:VVD:RXGXS, STPPTXX:VPT:SPIS: DSANNVV:YKQYE:VVE:SXGXR, HVPKPXX:APT:SPIS:DDSSNVI:YKQYE:VVR:SXGXH, RVPSTXX:APV:SPIS:DNGRVLL:YKQYE:VEE:XGXL, ASAAPXX:VPQ:SPIS:VGRKPKV:YKQYE:VRS:XKXS, ASASPXX:VSQ:SPIS:IGKTPKI:YKQYE:VKS:XKXS, ASASPXX:VPQ:SPIS:VGRTPKV:YKQYE:VKS:XKXS, NDEGLEX:VPT:SPIS:RIKPHQGQH:YKQYE:QHN:KXEXR, NDEGLEX:VPT:SPIS:RIKPHQGQH:YKQYE:EHS:QXEXR, SSVKXQP:SRV:SPIS:EYVRKKPKL:YKQYE:EEH:LEXAXA, RNVQXRP:TQV:SPIS:EIVRKKPIF:YKQYE:EDH:LAXKXE, GIPEPXX:VPT :SPIS:DENKNVV:YKQYE:VVE:SXAXR, HVTKPTX:VPT:SPIS:DDSSNVI:YKQYE:VVE:SXGXH, YVPKPXX:VPT:SPIS:DDSSNVI:YKQYE:VVE:SXGXH, TVPKPXX:VPT:SPIS:DDSSNVI:YKQYE:VVE:AXGXH, AVPKAXX:VPT:SPIS:DSSNNVI:YKQYE:VVE:AXGXH, KVGKAXX:VPT:SPIS:DDMGVPT:YKQYE:VVE:XGXR, KASKAXX:VPT:SPIS:DKGVVTY:YKQYE:VVE:EXGXR, GSAGPXX:VPT:SPIS:NDKQQII:YKQYE:VVE:RXGXS, AAPASXX:VPT:SPIS:DAANNVV:YKQYE:VVE:AXGXR, HVPKPXX:VPT:SPIS:DDSSNVI:YKQYE:VVE:SXGXH, RVPSTXX:VPT:SPIS:DNGRVLL:YKQYE:VVE:XGXL, ASAAPXX:VPT:SPIS:VGRKPKV:YKQYE:VVE:XKXS, ASASPXX:VPT:SPIS:IGKTPKI:YKQYE:VVE:XKXS, ASASPXX:VPT:SPIS:VGRTPKV:YKQYE:VVE:XKXS, AAPASXX:VPA:SPIS:DAANNVV:YKQYE:VVE:AXGXR, KIPKAXX:APV:KPLS:DENEKVV:DHHKD:VEE:GXGXR, GIPEPXX:APV:KPLS:DENKNVV:DHHKD:VEE:SXAXR, SIPKAXX:APV:KPLS:DEYDKVV:DHHKD:VEE:GXGXR, HVTKPTX:APV:KPLS:DDSSNVI:DHHKD:VEE:SXGXH, YVPKPXX:APV:KPLS:DDSSNVI:DHHKD:VEE:SXGXH, TVPKPXX:APV:KPLS:DDSSNVI:DHHKD:VEE:AXGXH, AVPKAXX:APV:KPLS:DSSNNVI:DHHKD:VEE:AXGXH, KVGKAXX:APV:KPLS:DDMGVPT:DHHKD:VEE:XGXR, KASKAXX:APV:KPLS:DKGVVTY:DHHKD:VEE:EXGXR, GSAGPXX:APV:KPLS:NDKQQII:DHHKD:VEE:RXGXS, AAPASXX:APV:KPLS:DAANNVV:DHHKD:VEE:AXGXR, STPPTXX:APV:KPLS:DSANNVV:DHHKD:VEE:SXGXR, HVPKPXX:APV:KPLS:DDSSNVI:DHHKD:VEE:SXGXH, ASAAPXX:APV:KPLS:VGRKPKV:DHHKD:VEE:XKXS, ASASPXX:APV:KPLS:IGKTPKI:DHHKD:VEE:XKXS, ASASPXX:APV:KPLS:VGRTPKV:DHHKD:VEE:XKXS, KIPKAXX:VPT :KPLS:DENEKVV:DHHKD:VVE:GXGXR, GIPEPXX:VPE:KPLS:DENKNVV:DHHKD:TVE:SXAXR, SIPKAXX:VPT :KPLS:DEYDKVV:DHHKD:VVE:GXGXR, HVTKPTX:APT:KPLS:DDSSNVI:DHHKD:VVR:SXGXH, YVPKPXX:APT:KPLS:DDSSNVI:DHHKD:VVR:SXGXH, TVPKPXX:APT:KPLS:DDSSNVI:DHHKD:VVR:AXGXH, AVPKAXX:APT:KPLS:DSSNNVI:DHHKD:VVK:AXGXH, KVGKAXX:VPT:KPLS:DDMGVPT:DHHKD:VAE:XGXR, KASKAXX:VPT:KPLS:DKGVVTY:DHHKD:AVS:EXGXR, GSAGPXX:TPT:KPLS:NDKQQII:DHHKD:VVD:RXGXS, AAPASXX:VPA:KPLS:DAANNVV:DHHKD:VVE:AXGXR, STPPTXX:VPT:KPLS:DSANNVV:DHHKD:VVE:SXGXR, HVPKPXX:APT:KPLS:DDSSNVI:DHHKD:VVR:SXGXH, ASAAPXX:VPQ:KPLS:VGRKPKV:DHHKD:VRS:XKXS, ASASPXX:VSQ:KPLS:IGKTPKI:DHHKD:VKS:XKXS, ASASPXX:VPQ:KPLS:VGRTPKV:DHHKD:VKS:XKXS, NDEGLEX:VPT:KPLS:RIKPHQGQH:DHHKD:QHN:KXEXR, NDEGLEX:VPT:KPLS:RIKPHQGQH:DHHKD:EHS:QXEXR, SSVKXQP:SRV:KPLS:EYVRKKPKL:DHHKD:EEH:LEXAXA, RNVQXRP:TQV:KPLS:EIVRKKPIF:DHHKD:EDH:LAXKXE, KIPKAXX:VPQ:EPLP:DENEKVV:EQLSN:VRS:GXGXR, GIPEPXX:VPQ:EPLP:DENKNVV:EQLSN:VRS:SXAXR, SIPKAXX:VPQ:EPLP:DEYDKVV:EQLSN:VRS:GXGXR, HVTKPTX:VPQ:EPLP:DDSSNVI:EQLSN:VRS:SXGXH, YVPKPXX:VPQ:EPLP:DDSSNVI:EQLSN:VRS:SXGXH, TVPKPXX:VPQ:EPLP:DDSSNVI:EQLSN:VRS:AXGXH, AVPKAXX:VPQ:EPLP:DSSNNVI:EQLSN:VRS:AXGXH, KVGKAXX:VPQ:EPLP:DDMGVPT:EQLSN:VRS:XGXR, KASKAXX:VPQ:EPLP:DKGVVTY:EQLSN:VRS:EXGXR, GSAGPXX:VPQ:EPLP:NDKQQII:EQLSN:VRS:RXGXS, AAPASXX:VPQ:EPLP:DAANNVV:EQLSN:VRS:AXGXR, STPPTXX:VPQ:EPLP:DSANNVV:EQLSN:VRS:SXGXR, HVPKPXX:VPQ:EPLP:DDSSNVI:EQLSN:VRS:SXGXH, RVPSTXX:VPQ:EPLP:DNGRVLL:EQLSN:VRS:XGXL, ASASPXX:VPQ:EPLP:IGKTPKI:EQLSN:VRS:XKXS, ASASPXX:VPQ: EPLP:VGRTPKV:EQLSN:VRS:XKXS, KIPKAXX:VPT:EPLP:DENEKVV:EQLSN:VVE:GXGXR, GIPEPXX:VPE:EPLP:DENKNVV:EQLSN:TVE:SXAXR, SIPKAXX:VPT:EPLP:DEYDKVV:EQLSN:VVE:GXGXR, HVTKPTX:APT:EPLP:DDSSNVI:EQLSN:VVR:SXGXH, YVPKPXX:APT:EPLP:DDSSNVI:EQLSN:VVR:SXGXH, TVPKPXX:APT:EPLP:DDSSNVI:EQLSN:VVR:AXGXH, AVPKAXX:APT:EPLP:DSSNNVI:EQLSN:VVK:AXGXH, KVGKAXX:VPT:EPLP:DDMGVPT:EQLSN:VAE:XGXR, KASKAXX:VPT:EPLP:DKGVVTY:EQLSN:AVS:EXGXR, GSAGPXX:TPT:EPLP:NDKQQII:EQLSN:VVD:RXGXS, AAPASXX:VPA:EPLP:DAANNVV:EQLSN:VVE:AXGXR, STPPTXX:VPT:EPLP:DSANNVV:EQLSN:VVE:SXGXR, HVPKPXX:APT:EPLP:DDSSNVI:EQLSN:VVR:SXGXH, RVPSTXX:APV:EPLP:DNGRVLL:EQLSN:VEE:XGXL, ASASPXX:VSQ:EPLP:IGKTPKI:EQLSN:VKS:XKXS, ASASPXX:VPQ:EPLP:VGRTPKV:EQLSN:VKS:XKXS, NDEGLEX:VPT:EPLP:RIKPHQGQH:EQLSN:QHN:KXEXR, NDEGLEX:VPT:EPLP:RIKPHQGQH:EQLSN:EHS:QXEXR, SSVKXQP:SRV:EPLP:EYVRKKPKL:EQLSN:EEH:LEXAXA, RNVQXRP:TQV:EPLP:EIVRKKPIF:EQLSN:EDH:LAXKXE, KIPKAXX:VSQ:EPLT:DENEKVV:EQLSN:VKS:GXGXR, GIPEPXX:VSQ:EPLT:DENKNVV:EQLSN:VKS:SXAXR, SIPKAXX:VSQ:EPLT:DEYDKVV:EQLSN:VKS:GXGXR, HVTKPTX:VSQ:EPLT:DDSSNVI:EQLSN:VKS:SXGXH, YVPKPXX:VSQ:EPLT:DDSSNVI:EQLSN:VKS:SXGXH, TVPKPXX:VSQ:EPLT:DDSSNVI:EQLSN:VKS:AXGXH, AVPKAXX:VSQ:EPLT:DSSNNVI:EQLSN:VKS:AXGXH, KVGKAXX:VSQ:EPLT:DDMGVPT:EQLSN:VKS:XGXR, KASKAXX:VSQ:EPLT:DKGVVTY:EQLSN:VKS:EXGXR, GSAGPXX:VSQ:EPLT:NDKQQII:EQLSN:VKS:RXGXS, AAPASXX:VSQ:EPLT:DAANNVV:EQLSN:VKS:AXGXR, STPPTXX:VSQ:EPLT:DSANNVV:EQLSN:VKS:SXGXR, HVPKPXX:VSQ:EPLT:DDSSNVI:EQLSN:VKS:SXGXH, RVPSTXX:VSQ:EPLT:DNGRVLL:EQLSN:VKS:XGXL, ASAAPXX:VSQ:EPLT:VGRKPKV:EQLSN:VKS:XKXS, ASASPXX:VSQ:EPLT:VGRTPKV:EQLSN:VKS:XKXS, KIPKAXX:VPT:EPLT:DENEKVV:EQLSN:VVE:GXGXR, GIPEPXX:VPE:EPLT:DENKNVV:EQLSN:TVE:SXAXR, SIPKAXX:VPT:EPLT:DEYDKVV:EQLSN:VVE:GXGXR, HVTKPTX:APT:EPLT:DDSSNVI:EQLSN:VVR:SXGXH, YVPKPXX:APT:EPLT:DDSSNVI:EQLSN:VVR:SXGXH, TVPKPXX:APT:EPLT:DDSSNVI:EQLSN:VVR:AXGXH, AVPKAXX:APT:EPLT:DSSNNVI:EQLSN:VVK:AXGXH, KVGKAXX:VPT:EPLT:DDMGVPT:EQLSN:VAE:XGXR, KASKAXX:VPT:EPLT:DKGVVTY:EQLSN:AVS:EXGXR, GSAGPXX:TPT:EPLT:NDKQQII:EQLSN:VVD:RXGXS, AAPASXX:VPA:EPLT:DAANNVV:EQLSN:VVE:AXGXR, STPPTXX:VPT:EPLT:DSANNVV:EQLSN:VVE:SXGXR, HVPKPXX:APT:EPLT:DDSSNVI:EQLSN:VVR:SXGXH, RVPSTXX:APV:EPLT:DNGRVLL:EQLSN:VEE:XGXL, ASAAPXX:VPQ:EPLT:VGRKPKV:EQLSN:VRS:XKXS, NDEGLEX:VPT:EPLT:RIKPHQGQH:EQLSN:QHN:KXEXR, NDEGLEX:VPT:EPLT:RIKPHQGQH:EQLSN:EHS:QXEXR, SSVKXQP:SRV:EPLT:EYVRKKPKL:EQLSN:EEH:LEXAXA, RNVQXRP:TQV:EPLT:EIVRKKPIF:EQLSN:EDH:LAXKXE, KIPKAXX:VPQ:EPLT:DENEKVV:EQLSN:VKS:GXGXR, GIPEPXX:VPQ:EPLT:DENKNVV:EQLSN:VKS:SXAXR, SIPKAXX:VPQ:EPLT:DEYDKVV:EQLSN:VKS:GXGXR, HVTKPTX:VPQ:EPLT:DDSSNVI:EQLSN:VKS:SXGXH, YVPKPXX:VPQ:EPLT:DDSSNVI:EQLSN:VKS:SXGXH, TVPKPXX:VPQ:EPLT:DDSSNVI:EQLSN:VKS:AXGXH, AVPKAXX:VPQ:EPLT:DSSNNVI:EQLSN:VKS:AXGXH, KVGKAXX:VPQ:EPLT:DDMGVPT:EQLSN:VKS:XGXR, KASKAXX:VPQ:EPLT:DKGVVTY:EQLSN:VKS:EXGXR, GSAGPXX:VPQ:EPLT:NDKQQII:EQLSN:VKS:RXGXS, AAPASXX:VPQ:EPLT:DAANNVV:EQLSN:VKS:AXGXR, STPPTXX:VPQ:EPLT:DSANNVV:EQLSN:VKS:SXGXR, HVPKPXX:VPQ:EPLT:DDSSNVI:EQLSN:VKS:SXGXH, RVPSTXX:VPQ:EPLT:DNGRVLL:EQLSN:VKS:XGXL, ASAAPXX:VPQ:EPLT:VGRKPKV:EQLSN:VKS:XKXS, ASASPXX:VPQ:EPLT:IGKTPKI:EQLSN:VKS:XKXS, NDEGLEX:VPT:SNIT:RIKPHQGQH:IGEMS:QHN:QXEXR, KIPKAXX:VPT:SNIT:DENEKVV:IGEMS:VVE:GXGXR, GIPEPXX:VPE:SNIT:DENKNVV:IGEMS:TVE:SXAXR, SIPKAXX:VPT:SNIT:DEYDKVV:IGEMS:VVE:GXGXR, HVTKPTX:APT:SNIT:DDSSNVI:IGEMS:VVR:SXGXH, YVPKPXX:APT:SNIT:DDSSNVI:IGEMS:VVR:SXGXH, TVPKPXX:APT:SNIT:DDSSNVI:IGEMS:VVR:AXGXH, AVPKAXX:APT:SNIT:DSSNNVI:IGEMS:VVK:AXGXH, KVGKAXX:VPT:SNIT:DDMGVPT:IGEMS:VAE:XGXR, KASKAXX:VPT:SNIT:DKGVVTY:IGEMS:AVS:EXGXR, GSAGPXX:TPT:SNIT:NDKQQII:IGEMS:VVD:RXGXS, AAPASXX:VPA:SNIT:DAANNVV:IGEMS:VVE:AXGXR, STPPTXX:VPT:SNIT:DSANNVV:IGEMS:VVE:SXGXR, HVPKPXX:APT:SNIT:DDSSNVI:IGEMS:VVR:SXGXH, RVPSTXX:APV:SNIT:DNGRVLL:IGEMS:VEE:XGXL, ASAAPXX:VPQ:SNIT:VGRKPKV:IGEMS:VRS:XKXS, ASASPXX:VSQ:SNIT:IGKTPKI:IGEMS:VKS:XKXS, ASASPXX:VPQ:SNIT:VGRTPKV:IGEMS:VKS:XKXS, NDEGLEX:VPT:SNIT:RIKPHQGQH:IGEMS:EHS:QXEXR, SSVKXQP:SRV:SNIT:EYVRKKPKL:IGEMS:EEH:LEXAXA, RNVQXRP:TQV:SNIT:EIVRKKPIF:IGEMS:EDH:LAXKXE, NDEGLEX:VPT:SNIT:RIKPHQGQH:LGEMS:EHS:KXEXR, KIPKAXX:VPT:SNIT:DENEKVV:LGEMS:VVE:GXGXR, GIPEPXX:VPE:SNIT:DENKNVV:LGEMS:TVE:SXAXR, SIPKAXX:VPT:SNIT:DEYDKVV:LGEMS:VVE:GXGXR, HVTKPTX:APT:SNIT:DDSSNVI:LGEMS:VVR:SXGXH, YVPKPXX:APT:SNIT:DDSSNVI:LGEMS:VVR:SXGXH, TVPKPXX:APT:SNIT:DDSSNVI:LGEMS:VVR:AXGXH, AVPKAXX:APT:SNIT :DSSNNVI:LGEMS:VVK:AXGXH, KVGKAXX:VPT :SNIT:DDMGVPT:LGEMS:VAE:XGXR, KASKAXX:VPT:SN IT:DKGVVTY:LGEMS:A VS :EXGXR, GSAGPXX:TPT:SNIT:NDKQQII:LGEMS:VVD:RXGXS, AAPASXX:VPA:SNIT :DAANNVV:LGEMS:VVE:AXGXR, STPPTXX:VPT:SNIT:DSANNVV:LGEMS:VVE :SXGXR, HVPKPXX:APT:SN IT:D DSSNVI:LG EMS:VVR:SXGXH, RVPSTXX:APV:SN IT:D NGRVLL: LGEMS:VEE:XGXL, ASAAPXX:VPQ:SN IT:VGRKPKV:LGEMS:VRS:XKXS, ASASPXX:VSQ:SN IT:IGKTPKI:LGEMS:VKS:XKXS, ASASPXX:VPQ:SN IT:VGRTPKV:LGEMS:VKS:XKXS, N DEGLEX:VPT:SN IT: RIKPHQGQH: LG EMS :QH N: KXEXR, SSVKXQP:SRV:SNIT:EYVRKKPKL: LGEMS:EEH :LEXAXA, RNVQXRP:TQV:SN IT:E IVRKKPI F: LGEMS: EDH:LAXKXE, RNVQXRP:SRV:RSVK:EIVRKKPIF:KEVQV:EEH:LAXKXE, KIPKAXX:VPT:RSVK:D EN EKVV:KEVQV:VVE :GXGXR, GIPEPXX:VPE :RSVK:DEN KNVV:KEVQV:TVE:SXAXR, SIPKAXX:VPT:RSVK:DEYDKVV:KEVQV:VVE:GXGXR, HVTKPTX:APT:RSVK:DDSSNVI :KEVQV:VVR:SXGXH, YVPKPXX:APT:RSVK:DDSSNVI:KEVQV:VVR:SXGXH, TVPKPXX:APT:RSVK:DDSSNVI :KEVQV:VVR:AXGXH, AVPKAXX:APT: RSVK:DSSNNVI: KEVQV:VVK:AXGXH, KVGKAXX:VPT:RSVK:DDMGVPT:KEVQV:VAE:XGXR, KASKAXX:VPT:RSVK:DKGVVTY:KEVQV:AVS:EXGXR, GSAGPXX:TPT:RSVK:NDKQQII:KEVQV:VVD:RXGXS, AAPASXX:VPA:RSVK:DAANNVV:KEVQV:VVE:AXGXR, STPPTXX:VPT:RSVK:DSANNVV:KEVQV:VVE:SXGXR, HVPKPXX:APT: RSVK:DDSSNVI:KEVQV:VVR:SXGXH, RVPSTXX:APV:RSVK:DNGRVLL:KEVQV:VEE:XGXL, ASAAPXX:VPQ:RSVK:VGRKPKV:KEVQV:VRS:XKXS, ASASPXX:VSQ:RSVK:IGKTPKI: KEVQV:VKS :XKXS, ASASPXX:VPQ: RSVK:VGRTPKV:KEVQV:VKS :XKXS, NDEGLEX:VPT:RSVK:RIKPHQGQH:KEVQV:QHN:KXEXR, NDEGLEX:VPT:RSVK:RIKPHQGQH:KEVQV:EHS:QXEXR, RNVQXRP:TQV:RSVK:EIVRKKPIF:KEVQV:EDH:LAXKXE, SSVKXQP:TQV:RPVQ:EYVRKKPKL:KKATV:EDH:LEXAXA, KIPKAXX:VPT:RPVQ:DENEKVV:KKATV:VVE:GXGXR, GIPEPXX:VPE:RPVQ:DENKNVV:KKATV:TVE:SXAXR, SIPKAXX:VPT:RPVQ:DEYDKVV:KKATV:VVE:GXGXR, HVTKPTX:APT:RPVQ:DDSSNVI:KKATV:VVR:SXGXH, YVPKPXX:APT:RPVQ:DDSSNVI:KKATV:VVR:SXGXH, TVPKPXX:APT:RPVQ:DDSSNVI: KKATV:VVR:AXGXH, AVPKAXX:APT:RPVQ:DSSNNVI:KKATV:VVK:AXGXH, KVGKAXX:VPT:RPVQ:DDMGVPT:KKATV:VAE:XGXR, KASKAXX:VPT:RPVQ:DKGVVTY:KKATV:AVS:EXGXR, GSAGPXX:TPT:RPVQ:NDKQQII:KKATV:VVD:RXGXS, AAPASXX:VPA:RPVQ:DAANNVV:KKATV:VVE:AXGXR, STPPTXX:VPT:RPVQ:DSANNVV:KKATV:VVE:SXGXR, HVPKPXX:APT:RPVQ:DDSSNVI:KKATV:VVR:SXGXH, RVPSTXX:APV:RPVQ:DNGRVLL:KKATV:VEE:XGXL, ASAAPXX:VPQ:RPVQ:VGRKPKV:KKATV:VRS:XKXS, ASASPXX:VSQ:RPVQ:IGKTPKI:KKATV:VKS:XKXS, ASASPXX:VPQ:RPVQ:VGRTPKV:KKATV:VKS:XKXS, NDEGLEX:VPT:RPVQ:RIKPHQGQH:KKATV:QHN:KXEXR, NDEGLEX:VPT:RPVQ:RIKPHQGQH:KKATV:EHS:QXEXR and SSVKXQP:SRV:RPVQ:EYVRKKPKL:KKATV:EEH:LEXAXA.

In particular, in certain embodiments, the heptuplet PEP7:PEP3:PEP12:LINKER:PEP2:PEP4:PEP8 is selected from the group consisting of GIPEPXX:VPT:SAIS-AA$^{17}$-LYL:DENKNVV:LKNYQ:VVE:SXAXR, SIPKAXX:VPT:SAIS-AA$^{17}$-LYL:DEYDKVV:LKNYQ:VVE:GXGXR, HVTKPTX:VPT:SAIS-AA$^{17}$-LYL:DDSSNVI:LKNYQ:VVE:SXGXH, YVPKPXX:VPT:SAIS-AA$^{17}$-LYL:DDSSNVI:LKNYQ:VVE:SXGXH, TVPKPXX:VPT:SAIS-AA$^{17}$-LYL:DDSSNVI:LKNYQ:VVE:AXGXH,AVPKAXX:VPT:SAIS-AA$^{17}$-LYL:DSSNNVI:LKNYQ:VVE:AXGXH, KVGKAXX:VPT:SAIS-AA$^{17}$-LYL:DDMGVPT:LKNYQ:VVE:XGXR, KASKAXX:VPT:SAIS-AA$^{17}$-LYL:DKGVVTY:LKNYQ:VVE:EXGXR, GSAGPXX:VPT:SAIS-AA$^{17}$-LYL:NDKQQII:LKNYQ:VVE:RXGXS, AAPASXX:VPT:SAIS-AA$^{17}$-LYL:DAAN-

NVV:LKNYQ:VVE:AXGXR, STPPTXX:VPT:SAIS-AA17-LYL:DSANNVV:LKNYQ:VVE:SXGXR, HVPKPXX:VPT:SAIS-AA17-LYL:DDSSNVI:LKNYQ:VVE:SXGXH, RVPSTXX:VPT:SAIS-AA17-LYL:DNGRVLL:LKNYQ:VVE:XGXL, ASAAPXX:VPT:SAIS-AA17-LYL:VGRKPKV:LKNYQ:VVE:XKXS,ASASPXX:VPT:SAIS-AA17-LYL:IGKTP-KI:LKNYQ:VVE:XKXS, ASASPXX:VPT:SAIS-AA17-LYL:VGRTPKV:LKNYQ:VVE:XKXS, NDEGLEX:VPT:SAIS-AA17-LYL:RIKPHQGQH:LKNYQ:VVE:KXEXR, NDEGLEX:VPT:SAIS-AA17-LYL:RIKPHQGQH:LKNYQ:VVE:QXEXR, SSVKXQP:VPT:SAIS-AA17-LYL:EYVRKKPKL:LKNYQ:VVE:LEXAXA, RNVQXRP:VPT:SAIS-AA17-LYL:EIVRKKPIF:LKNYQ:VVE:LAXKXE, GIPEPXX:VPE:SAIS-AA17-LYL:DENKNVV:LKNYQ:TVE:SXAXR, HVTKPTX:APT:SAIS-AA17-LYL:DDSSNVI:LKNYQ:VVR:SXGXH, YVPKPXX:APT:SAIS-AA17-LYL:DDSSN-VI:LKNYQ:VVR:SXGXH, TVPKPXX:APT:SAIS-AA17-LYL:DDSSNVI:LKNYQ:VVR:AXGXH, AVPKAXX:APT:SAIS-AA17-LYL:DSSNNVI:LKNYQ:VVK:AXGXH,KVGKAXX:VPT:SAIS-AA17-LYL:DDM-GVPT:LKNYQ:VAE:XGXR,KASKAXX:VPT:SAIS-AA17-LYL:DKGVVTY:LKNYQ:AVS:EXGXR, GSAGPXX:TPT:SAIS-AA17-LYL:NDKQQII:LKNYQ:VVD:RXGXS,AAPASXX:VPA:SAIS-AA17-LYL:DAANNVV:LKNYQ:VVE:AXGXR,HVPK-PXX:APT:SAIS-AA17-LYL:DDSSNVI:LKNYQ:VVR:SXGXH, RVPSTXX:APV:SAIS-AA17-LYL:DNGRV-LL:LKNYQ:VEE:XGXL,ASAAPXX:VPQ:SAIS-AA17-LYL:VGRKPKV:LKNYQ:VRS:XKXS, ASASPXX:VSQ:SAIS-AA17-LYL:IGKTPKI:LKNYQ:VKS:XKXS, ASASPXX:VPQ:SAIS-AA17-LYL:VGRTPKV:LKNYQ:VKS:XKXS, NDE-GLEX:VPT:SAIS-AA17- LYL:RIKPHQGQH:LKNYQ:QHN:KXEXR, NDEGLEX:VPT:SAIS-AA17-LYL:RIK-PHQGQH:LKNYQ:EHS:QXEXR, SSVKXQP:SRV:SAIS-AA17-LYL:EYVRKKPKL:LKNYQ:EEH:LEXAXA, RNVQXRP:TQV:SAIS-AA17-LYL:EIVRKKPIF:LKNYQ:EDH:LAXKXE, KIPKAXX:VPE:SSLS-AA17-LFF:DENEKVV:LKVYP:TVE:GXGXR, SIPKAXX:VPE:SSLS-AA17-LFF:DEYDKVV:LKVYP:TVE:GXGXR, HVTKPTX:VPE:SSLS-AA17-LFF:DDSSNVI:LKVYP:TVE:SXGXH, YVPKPXX:VPE:SSLS-AA17-LFF:DDSSNVI:LKV-YP:TVE:SXGXH, TVPKPXX:VPE:SSLS-AA17-LFF:DDSSNVI:LKVYP:TVE:AXGXH, AVPKAXX:VPE:SSLS-AA17-LFF:DSSNNVI:LKVYP:TVE:AXGXH, KVGKAXX:VPE:SSLS-AA17-LFF:DDMGVPT:LKVYP:TVE:XGXR, KASKAXX:VPE:SSLS-AA17-LFF:DKGVVTY:LKVYP:TVE:EXGXR, GSAGPXX:VPE:SSLS-AA17-LFF:NDKQQII:LKV-YP:TVE:RXGXS, AAPASXX:VPE:SSLS-AA17-LFF:DAANNVV:LKVYP:TVE:AXGXR,STPPTXX:VPE:SSLS-AA17-LFF:DSANNVV:LKVYP:TVE:SXGXR, HVPKPXX:VPE:SSLS-AA17-LFF:DDSSNVI:LKVYP:TVE:SXGXH,RVP-STXX:VPE:SSLS-AA17-LFF:DNGRVLL:LKVYP:TVE:XGXL, ASAAPXX:VPE:SSLS-AA17-LFF:VGRKPKV:LKV-YP:TVE:XKXS, ASASPXX:VPE:SSLS-AA17-LFF:IGKTPKI:LKVYP:TVE:XKXS, ASASPXX:VPE:SSLS-AA17-LFF:VGRTPKV:LKVYP:TVE:XKXS, NDEGLEX:VPE:SSLS-AA17-LFF:RIKPHQGQH:LKVYP:TVE:KXEXR, NDE-GLEX:VPE:SSLS-AA17-LFF:RIKPHQGQH:LKVYP:TVE:QXEXR,SSVKXQP:VPE:SSLS-AA17-LFF:EYVRKKPKL:LKV-YP:TVE:LEXAXA, RNVQXRP:VPE:SSLS-AA17-LFF:EIVRKKPIF:LKVYP:TVE:LAXKXE, KIPKAXX:VPT:SSLS-AA17-LFF:DENEKVV:LKVYP:VVE:GXGXR, SIPKAXX:VPT:SSLS-AA17-LFF:DEYDKVV:LKVYP:VVE:GXGXR, HVTKPTX:APT:SSLS-AA17-LFF:DDSSNVI:LKVYP:VVR:SXGXH, YVPKPXX:APT:SSLS-AA17-LFF:DDSSNVI:LKV-YP:VVR:SXGXH, TVPKPXX:APT:SSLS-AA17-LFF:DDSSNVI:LKVYP:VVR:AXGXH, AVPKAXX:APT:SSLS-AA17-LFF:DSSNNVI:LKVYP:VVK:AXGXH, KVGKAXX:VPT:SSLS-AA17-LFF:DDMGVPT:LKVYP:VAE:XGXR, KASKAXX:VPT:SSLS-AA17-LFF:DKGVVTY:LKVYP:AVS:EXGXR, GSAGPXX:TPT:SSLS-AA17-LFF:NDKQQII:LKV-YP:VVD:RXGXS,AAPASXX:VPA:SSLS-AA17-LFF:DAANNVV:LKVYP:VVE:AXGXR, STPPTXX:VPT:SSLS-AA17-LFF:DSANNVV:LKVYP:VVE:SXGXR, HVPKPXX:APT:SSLS-AA17-LFF:DDSSNVI:LKVYP:VVR:SXGXH, RVP-STXX:APV:SSLS-AA17-LFF:DNGRVLL:LKVYP:VEE:XGXL, ASAAPXX:VPQ:SSLS-AA17-LFF:VGRKPKV:LKV-YP:VRS:XKXS, ASASPXX:VSQ:SSLS-AA17-LFF:IGKTPKI:LKVYP:VKS:XKXS, ASASPXX:VPQ:SSLS-AA17-LFF:VGRTPKV:LKVYP:VKS:XKXS, NDEGLEX:VPT:SSLS-AA17-LFF:RIKPHQGQH:LKVYP:QHN:KXEXR, NDE-GLEX:VPT:SSLS-AA17-LFF:RIKPHQGQH:LKVYP:EHS:QXEXR, SSVKXQP:SRV:SSLS-AA17-LFF:EYVRKKP-KL:LKVYP:EEH:LEXAXA, RNVQXRP:TQV:SSLS-AA17-LFF:EIVRKKPIF:LKVYP:EDH:LAXKXE, KIPKAXX:VPT:SAIS-AA17-LYL:DENEKVV:LKNYQ:VVE:GXGXR, KIPKAXX:APT:NAIS-AA17-LYF:DENEKVV:LKKYR:VVR:GXGXR,GIPEPXX:APT:NAIS-AA17-LYF:DENKNVV:LKKYR:VVR:SXAXR, SIP-KAXX:APT:NAIS-AA17-LYF:DEYDKVV:LKKYR:VVR:GXGXR, YVPKPXX:APT:NAIS-AA17-LYF:DDSSN-VI:LKKYR:VVR:SXGXH, TVPKPXX:APT:NAIS-AA17-LYF:DDSSNVI:LKKYR:VVR:AXGXH,AVPKAXX:APT:NAIS-AA17-LYF:DSSNNVI:LKKYR:VVR:AXGXH, KVGKAXX:APT:NAIS-AA17-LYF:DDMGVPT:LKKYR:VVR:XGXR, KASKAXX:APT:NAIS-AA17-LYF:DKGVVTY:LKKYR:VVR:EXGXR, GSAGPXX:APT:NAIS-AA17-LYF:NDKQ-QII:LKKYR:VVR:RXGXS, AAPASXX:APT:NAIS-AA17-LYF:DAANNVV:LKKYR:VVR:AXGXR, STPPTXX:APT:NAIS-AA17-LYF:DSANNVV:LKKYR:VVR:SXGXR, HVPKPXX:APT:NAIS-AA17-LYF:DDSSNVI:LKKYR:VVR:SXGXH, RVP-STXX:APT:NAIS-AA17-LYF:DNGRVLL:LKKYR:VVR:XGXL, ASAAPXX:APT:NAIS-AA17-LYF:VGRKP-KV:LKKYR:VVR:XKXS, ASASPXX:APT:NAIS-AA17-LYF:IGKTPKI:LKKYR:VVR:XKXS, ASASPXX:APT:NAIS-AA17-LYF:VGRTPKV:LKKYR:VVR:XKXS, NDEGLEX:APT:NAIS-AA17-LYF:RIKPHQGQH:LKKYR:VVR:KXEXR, NDE-GLEX:APT:NAIS-AA17-LYF:RIKPHQGQH:LKKYR:VVR:QXEXR, SSVKXQP:APT:NAIS-AA17-LYF:EYVRKKP-KL:LKKYR:VVR:LEXAXA, RNVQXRP:APT:NAIS-AA17-LYF:EIVRKKPIF:LKKYR:VVR:LAXKXE, KIPKAXX:VPT:NAIS-AA17-LYF:DENEKVV:LKKYR:VVE:GXGXR, GIPEPXX:VPE:NAIS-AA17-LYF:DENKNVV:LKKYR:TVE:SXAXR, SIP-KAXX:VPT:NAIS-AA17-LYF:DEYDKVV:LKKYR:VVE:GXGXR, AVPKAXX:APT:NAIS-AA17-LYF:DSSNN-VI:LKKYR:VVK:AXGXH, KVGKAXX:VPT:NAIS-AA17-LYF:DDMGVPT:LKKYR:VAE:XGXR, KASKAXX:VPT:NAIS-

AA$^{17}$-LYF:DKGVVTY:LKKYR:AVS:EXGXR, GSAGPXX:TPT:NAIS-AA$^{17}$-LYF:NDKQQII:LKKYR:VVD:RXGXS, AAPASXX:VPA:NAIS-AA$^{17}$-LYF:DAANNVV:LKKYR:VVE:AXGXR, STPPTXX:VPT:NAIS-AA$^{17}$-LYF:DSANNVV:LKKYR:VVE:SXGXR, RVPSTXX:APV:NAIS-AA$^{17}$-LYF:DNGRVLL:LKKYR:VEE:XGXL, ASAAPXX:VPQ:NAIS-AA$^{17}$-LYF:VGRKPKV:LKKYR:VRS:XKXS, ASASPXX:VSQ:NAIS-AA$^{17}$-LYF:IGKTPKI:LKKYR:VKS:XKXS, ASASPXX:VPQ:NAIS-AA$^{17}$-LYF:VGRTPKV:LKKYR:VKS:XKXS, NDEGLEX:VPT:NAIS-AA$^{17}$-LYF:RIKPHQGQH:LKKYR:QHN:KXEXR, NDEGLEX:VPT:NAIS-AA$^{17}$-LYF:RIKPHQGQH:LKKYR:EHS:QXEXR, SSVKXQP:SRV:NAIS-AA$^{17}$-LYF:EYVRKKPKL:LKKYR:EEH:LEXAXA, RNVQXRP:TQV:NAIS-AA$^{17}$-LYF:EIVRKKPIF:LKKYR:EDH:LAXKXE, HVTKPTX:APT:NAIS-AA$^{17}$-LYF:DDSSNVI:LKKYR:VVR:SXGXH, KIPKAXX:APT:SATS-AA$^{17}$-LYY:DENEKVV:LRKHR:VVK:GXGXR, GIPEPXX:APT:SATS-AA$^{17}$-LYY:DENKNVV:LRKHR:VVK:SXAXR, SIPKAXX:APT:SATS-AA$^{17}$-LYY:DEYDKVV:LRKHR:VVK:GXGXR, HVTKPTX:APT:SATS-AA$^{17}$-LYY:DDSSNVI:LRKHR:VVK:SXGXH, YVPKPXX:APT:SATS-AA$^{17}$-LYY:DDSSNVI:LRKHR:VVK:SXGXH, TVPKPXX:APT:SATS-AA$^{17}$-LYY:DDSSNVI:LRKHR:VVK:AXGXH, KVGKAXX:APT:SATS-AA$^{17}$-LYY:DDMGVPT:LRKHR:VVK:XGXR, KASKAXX:APT:SATS-AA$^{17}$-LYY:DKGVVTY:LRKHR:VVK:EXGXR, GSAGPXX:APT:SATS-AA$^{17}$-LYY:NDKQQII:LRKHR:VVK:RXGXS, AAPASXX:APT:SATS-AA$^{17}$-LYY:DAANNVV:LRKHR:VVK:AXGXR, STPPTXX:APT:SATS-AA$^{17}$-LYY:DSANNVV:LRKHR:VVK:SXGXR, HVPKPXX:APT:SATS-AA$^{17}$-LYY:DDSSNVI:LRKHR:VVK:SXGXH, RVPSTXX:APT:SATS-AA$^{17}$-LYY:DNGRVLL:LRKHR:VVK:XGXL, ASAAPXX:APT:SATS-AA$^{17}$-LYY:VGRKPKV:LRKHR:VVK:XKXS, ASASPXX:APT:SATS-AA$^{17}$-LYY:IGKTPKI:LRKHR:VVK:XKXS, ASASPXX:APT:SATS-AA$^{17}$-LYY:VGRTPKV:LRKHR:VVK:XKXS, NDEGLEX:APT:SATS-AA$^{17}$-LYY:RIKPHQGQH:LRKHR:VVK:KXEXR, NDEGLEX:APT:SATS-AA$^{17}$-LYY:RIKPHQGQH:LRKHR:VVK:QXEXR, SSVKXQP:APT:SATS-AA$^{17}$-LYY:EYVRKKPKL:LRKHR:VVK:LEXAXA, RNVQXRP:APT:SATS-AA$^{17}$-LYY:EIVRKKPIF:LRKHR:VVK:LAXKXE, KIPKAXX:VPT:SATS-AA$^{17}$-LYY:DENEKVV:LRKHR:VVE:GXGXR, GIPEPXX:VPE:SATS-AA$^{17}$-LYY:DENKNVV:LRKHR:TVE:SXAXR, SIPKAXX:VPT:SATS-AA$^{17}$-LYY:DEYDKVV:LRKHR:VVE:GXGXR, HVTKPTX:APT:SATS-AA$^{17}$-LYY:DDSSNVI:LRKHR:VVR:SXGXH, YVPKPXX:APT:SATS-AA$^{17}$-LYY:DDSSNVI:LRKHR:VVR:SXGXH, TVPKPXX:APT:SATS-AA$^{17}$-LYY:DDSSNVI:LRKHR:VVR:AXGXH, KVGKAXX:VPT:SATS-AA$^{17}$-LYY:DDMGVPT:LRKHR:VAE:XGXR, KASKAXX:VPT:SATS-AA$^{17}$-LYY:DKGVVTY:LRKHR:AVS:EXGXR, GSAGPXX:TPT:SATS-AA$^{17}$-LYY:NDKQQII:LRKHR:VVD:RXGXS, AAPASXX:VPA:SATS-AA$^{17}$-LYY:DAANNVV:LRKHR:VVE:AXGXR, STPPTXX:VPT:SATS-AA$^{17}$-LYY:DSANNVV:LRKHR:VVE:SXGXR, HVPKPXX:APT:SATS-AA$^{17}$-LYY:DDSSNVI:LRKHR:VVR:SXGXH, RVPSTXX:APV:SATS-AA$^{17}$-LYY:DNGRVLL:LRKHR:VEE:XGXL, ASAAPXX:VPQ:SATS-AA$^{17}$-LYY:VGRKPKV:LRKHR:VRS:XKXS, ASASPXX:VSQ:SATS-AA$^{17}$-LYY:IGKTPKI:LRKHR:VKS:XKXS, ASASPXX:VPQ:SATS-AA$^{17}$-LYY:VGRTPKV:LRKHR:VKS:XKXS, NDEGLEX:VPT:SATS-AA$^{17}$-LYY:RIKPHQGQH:LRKHR:QHN:KXEXR, NDEGLEX:VPT:SATS-AA$^{17}$-LYY:RIKPHQGQH:LRKHR:EHS:QXEXR, SSVKXQP:SRV:SATS-AA$^{17}$-LYY:EYVRKKPKL:LRKHR:EEH:LEXAXA, RNVQXRP:TQV:SATS-AA$^{17}$-LYY:EIVRKKPIF:LRKHR:EDH:LAXKXE, KIPKAXX:VPT:SPIS-AA$^{17}$-LYK:DENEKVV:LKYHY:VAE:GXGXR, GIPEPXX:VPT:SPIS-AA$^{17}$-LYK:DENKNVV:LKYHY:VAE:SXAXR, SIPKAXX:VPT:SPIS-AA$^{17}$-LYK:DEYDKVV:LKYHY:VAE:GXGXR, HVTKPTX:VPT:SPIS-AA$^{17}$-LYK:DDSSNVI:LKYHY:VAE:SXGXH, YVPKPXX:VPT:SPIS-AA$^{17}$-LYK:DDSSNVI:LKYHY:VAE:SXGXH, TVPKPXX:VPT:SPIS-AA$^{17}$-LYK:DDSSNVI:LKYHY:VAE:AXGXH, AVPKAXX:VPT:SPIS-AA$^{17}$-LYK:DSSNNVI:LKYHY:VAE:AXGXH, KASKAXX:VPT:SPIS-AA$^{17}$-LYK:DKGVVTY:LKYHY:VAE:EXGXR, GSAGPXX:VPT:SPIS-AA$^{17}$-LYK:NDKQQII:LKYHY:VAE:RXGXS, AAPASXX:VPT:SPIS-AA$^{17}$-LYK:DAANNVV:LKYHY:VAE:AXGXR, STPPTXX:VPT:SPIS-AA$^{17}$-LYK:DSANNVV:LKYHY:VAE:SXGXR, HVPKPXX:VPT:SPIS-AA$^{17}$-LYK:DDSSNVI:LKYHY:VAE:SXGXH, RVPSTXX:VPT:SPIS-AA$^{17}$-LYK:DNGRVLL:LKYHY:VAE:XGXL, ASAAPXX:VPT:SPIS-AA$^{17}$-LYK:VGRKPKV:LKYHY:VAE:XKXS, ASASPXX:VPT:SPIS-AA$^{17}$-LYK:IGKTPKI:LKYHY:VAE:XKXS, ASASPXX:VPT:SPIS-AA$^{17}$-LYK:VGRTPKV:LKYHY:VAE:XKXS, NDEGLEX:VPT:SPIS-AA$^{17}$-LYK:RIKPHQGQH:LKYHY:VAE:KXEXR, NDEGLEX:VPT:SPIS-AA$^{17}$-LYK:RIKPHQGQH:LKYHY:VAE:QXEXR, SSVKXQP:VPT:SPIS-AA$^{17}$-LYK:EYVRKKPKL:LKYHY:VAE:LEXAXA, RNVQXRP:VPT:SPIS-AA$^{17}$-LYK:EIVRKKPIF:LKYHY:VAE:LAXKXE, KIPKAXX:VPT:SPIS-AA$^{17}$-LYK:DENEKVV:LKYHY:VVE:GXGXR, GIPEPXX:VPE:SPIS-AA$^{17}$-LYK:DENKNVV:LKYHY:TVE:SXAXR, SIPKAXX:VPT:SPIS-AA$^{17}$-LYK:DEYDKVV:LKYHY:VVE:GXGXR, HVTKPTX:APT:SPIS-AA$^{17}$-LYK:DDSSNVI:LKYHY:VVR:SXGXH, YVPKPXX:APT:SPIS-AA$^{17}$-LYK:DDSSNVI:LKYHY:VVR:SXGXH, TVPKPXX:APT:SPIS-AA$^{17}$-LYK:DDSSNVI:LKYHY:VVR:AXGXH, AVPKAXX:APT:SPIS-AA$^{17}$-LYK:DSSNNVI:LKYHY:VVK:AXGXH, KASKAXX:VPT:SPIS-AA$^{17}$-LYK:DKGVVTY:LKYHY:AVS:EXGXR, GSAGPXX:TPT:SPIS-AA$^{17}$-LYK:NDKQQII:LKYHY:VVD:RXGXS, AAPASXX:VPA:SPIS-AA$^{17}$-LYK:DAANNVV:LKYHY:VVE:AXGXR, STPPTXX:VPT:SPIS-AA$^{17}$-LYK:DSANNVV:LKYHY:VVE:SXGXR, HVPKPXX:APT:SPIS-AA$^{17}$-LYK:DDSSNVI:LKYHY:VVR:SXGXH, RVPSTXX:APV:SPIS-AA$^{17}$-LYK:DNGRVLL:LKYHY:VEE:XGXL, ASAAPXX:VPQ:SPIS-AA$^{17}$-LYK:VGRKPKV:LKYHY:VRS:XKXS, ASASPXX:VSQ:SPIS-AA$^{17}$-LYK:IGKTPKI:LKYHY:VKS:XKXS, ASASPXX:VPQ:SPIS-AA$^{17}$-LYK:VGRTPKV:LKYHY:VKS:XKXS, NDEGLEX:VPT:SPIS-AA$^{17}$-LYK:RIKPHQGQH:LKYHY:QHN:KXEXR, NDEGLEX:VPT:SPIS-AA$^{17}$-LYK:RIKPHQGQH:LKYHY:EHS:QXEXR, SSVKXQP:SRV:SPIS-AA$^{17}$-LYK:EYVRKKPKL:LKYHY:EEH:LEXAXA, RNVQXRP:TQV:SPIS-AA$^{17}$-LYK:EIVRKKPIF:LKYHY:EDH:LAXKXE, KIPKAXX:VPT:EPIS-AA$^{17}$-LYL:DENEKVV:KFKYE:AVS:GXGXR, GIPEPXX:VPT:EPIS-AA$^{17}$-LYL:DENKN-

VV:KFKYE:AVS:SXAXR, SIPKAXX:VPT:EPIS-AA$^{17}$-LYL:DEYDKVV:KFKYE:AVS:GXGXR, HVTKPTX:VPT:EPIS-AA$^{17}$-LYL:DDSSNVI:KFKYE:AVS:SXGXH, YVPKPXX:VPT:EPIS-AA$^{17}$-LYL:DDSSNVI:KFKYE:AVS:SXGXH, TVPKPXX:VPT:EPIS-AA$^{17}$-LYL:DDSSNVI:KFKYE:AVS:AXGXH, AVPKAXX:VPT:EPIS-AA$^{17}$-LYL:DSSNNVI:KFKYE:AVS:AXGXH, KVGKAXX:VPT:EPIS-AA$^{17}$-LYL:DDMGVPT:KFKYE:AVS:XGXR, GSAGPXX:VPT:EPIS-AA$^{17}$-LYL:NDKQQII:KFKYE:AVS:RXGXS, AAPASXX:VPT:EPIS-AA$^{17}$-LYL:DAANNVV:KFKYE:AVS:AXGXR, STPPTXX:VPT:EPIS-AA$^{17}$-LYL:DSANNVV:KFKYE:AVS:SXGXR, HVPKPXX:VPT:EPIS-AA$^{17}$-LYL:DDSSNVI:KFKYE:AVS:SXGXH, RVPSTXX:VPT:EPIS-AA$^{17}$-LYL:DNGRVLL:KFKYE:AVS:XGXL, ASAAPXX:VPT:EPIS-AA$^{17}$-LYL:VGRKPKV:KFKYE:AVS:XKXS, ASASPXX:VPT:EPIS-AA$^{17}$-LYL:IGKTPKI:KFKYE:AVS:XKXS, ASASPXX:VPT:EPIS-AA$^{17}$-LYL:VGRTPKV:KFKYE:AVS:XKXS, NDEGLEX:VPT:EPIS-AA$^{17}$-LYL:RIKPHQGQH:KFKYE:AVS:KXEXR, NDEGLEX:VPT:EPIS-AA$^{17}$-LYL:RIKPHQGQH:KFKYE:AVS:QXEXR, SSVKXQP:VPT:EPIS-AA$^{17}$-LYL:EYVRKKPKL:KFKYE:AVS:LEXAXA, RNVQXRP:VPT:EPIS-AA$^{17}$-LYL:EIVRKKPIF:KFKYE:AVS:LAXKXE, KIPKAXX:VPT:EPIS-AA$^{17}$-LYL:DENEKVV:KFKYE:VVE:GXGXR, GIPEPXX:VPE:EPIS-AA$^{17}$-LYL:DENKNVV:KFKYE:TVE:SXAXR, SIPKAXX:VPT:EPIS-AA$^{17}$-LYL:DEYDKVV:KFKYE:VVE:GXGXR, HVTKPTX:APT:EPIS-AA$^{17}$-LYL:DDSSNVI:KFKYE:VVR:SXGXH, YVPKPXX:APT:EPIS-AA$^{17}$-LYL:DDSSNVI:KFKYE:VVR:SXGXH, TVPKPXX:APT:EPIS-AA$^{17}$-LYL:DDSSNVI:KFKYE:VVR:AXGXH, AVPKAXX:APT:EPIS-AA$^{17}$-LYL:DSSNNVI:KFKYE:VVK:AXGXH, KVGKAXX:VPT:EPIS-AA$^{17}$-LYL:DDMGVPT:KFKYE:VAE:XGXR, GSAGPXX:TPT:EPIS-AA$^{17}$-LYL:NDKQQII:KFKYE:VVD:RXGXS, AAPASXX:VPA:EPIS-AA$^{17}$-LYL:DAANNVV:KFKYE:VVE:AXGXR, STPPTXX:VPT:EPIS-AA$^{17}$-LYL:DSANNVV:KFKYE:VVE:SXGXR, HVPKPXX:APT:EPIS-AA$^{17}$-LYL:DDSSNVI:KFKYE:VVR:SXGXH, RVPSTXX:APV:EPIS-AA$^{17}$-LYL:DNGRVLL:KFKYE:VEE:XGXL, ASAAPXX:VPQ:EPIS-AA$^{17}$-LYL:VGRKPKV:KFKYE:VRS:XKXS, ASASPXX:VSQ:EPIS-AA$^{17}$-LYL:IGKTPKI:KFKYE:VKS:XKXS, ASASPXX:VPQ:EPIS-AA$^{17}$-LYL:VGRTPKV:KFKYE:VKS:XKXS, NDEGLEX:VPT:EPIS-AA$^{17}$-LYL:RIKPHQGQH:KFKYE:QHN:KXEXR, NDEGLEX:VPT:EPIS-AA$^{17}$-LYL:RIKPHQGQH:KFKYE:EHS:QXEXR, SSVKXQP:SRV:EPIS-AA$^{17}$-LYL:EYVRKKPKL:KFKYE:EEH:LEXAXA, RNVQXRP:TQV:EPIS-AA$^{17}$-LYL:EIVRKKPIF:KFKYE:EDH:LAXKXE, KIPKAXX:TPT:SPIN-AA$^{17}$-LYF:DENEKVV:YGKIP:VVD:GXGXR, GIPEPXX:TPT:SPIN-AA$^{17}$-LYF:DENKNVV:YGKIP:VVD:SXAXR, SIPKAXX:TPT:SPIN-AA$^{17}$-LYF:DEYDKVV:YGKIP:VVD:GXGXR, HVTKPTX:TPT:SPIN-AA$^{17}$-LYF:DDSSNVI:YGKIP:VVD:SXGXH, YVPKPXX:TPT:SPIN-AA$^{17}$-LYF:DDSSNVI:YGKIP:VVD:SXGXH, TVPKPXX:TPT:SPIN-AA$^{17}$-LYF:DDSSNVI:YGKIP:VVD:AXGXH, AVPKAXX:TPT:SPIN-AA$^{17}$-LYF:DSSNNVI:YGKIP:VVD:AXGXH, KVGKAXX:TPT:SPIN-AA$^{17}$-LYF:DDMGVPT:YGKIP:VVD:XGXR, KASKAXX:TPT:SPIN-AA$^{17}$-LYF:DKGVVTY:YGKIP:VVD:EXGXR, AAPASXX:TPT:SPIN-AA$^{17}$-LYF:DAANNVV:YGKIP:VVD:AXGXR, STPPTXX:TPT:SPIN-AA$^{17}$-LYF:DSANNVV:YGKIP:VVD:SXGXR, HVPKPXX:TPT:SPIN-AA$^{17}$-LYF:DDSSNVI:YGKIP:VVD:SXGXH, RVPSTXX:TPT:SPIN-AA$^{17}$-LYF:DNGRVLL:YGKIP:VVD:XGXL, ASAAPXX:TPT:SPIN-AA$^{17}$-LYF:VGRKPKV:YGKIP:VVD:XKXS, ASASPXX:TPT:SPIN-AA$^{17}$-LYF:IGKTPKI:YGKIP:VVD:XKXS, ASASPXX:TPT:SPIN-AA$^{17}$-LYF:VGRTPKV:YGKIP:VVD:XKXS, NDEGLEX:TPT:SPIN-AA$^{17}$-LYF:RIKPHQGQH:YGKIP:VVD:KXEXR, NDEGLEX:TPT:SPIN-AA$^{17}$-LYF:RIKPHQGQH:YGKIP:VVD:QXEXR, SSVKXQP:TPT:SPIN-AA$^{17}$-LYF:EYVRKKPKL:YGKIP:VVD:LEXAXA, RNVQXRP:TPT:SPIN-AA$^{17}$-LYF:EIVRKKPIF:YGKIP:VVD:LAXKXE, KIPKAXX:VPT:SPIN-AA$^{17}$-LYF:DENEKVV:YGKIP:VVE:GXGXR, GIPEPXX:VPE:SPIN-AA$^{17}$-LYF:DENKNVV:YGKIP:TVE:SXAXR, SIPKAXX:VPT:SPIN-AA$^{17}$-LYF:DEYDKVV:YGKIP:VVE:GXGXR, HVTKPTX:APT:SPIN-AA$^{17}$-LYF:DDSSNVI:YGKIP:VVR:SXGXH, YVPKPXX:APT:SPIN-AA$^{17}$-LYF:DDSSNVI:YGKIP:VVR:SXGXH, TVPKPXX:APT:SPIN-AA$^{17}$-LYF:DDSSNVI:YGKIP:VVR:AXGXH, AVPKAXX:APT:SPIN-AA$^{17}$-LYF:DSSNNVI:YGKIP:VVK:AXGXH, KVGKAXX:VPT:SPIN-AA$^{17}$-LYF:DDMGVPT:YGKIP:VAE:XGXR, KASKAXX:VPT:SPIN-AA$^{17}$-LYF:DKGVVTY:YGKIP:AVS:EXGXR, AAPASXX:VPA:SPIN-AA$^{17}$-LYF:DAANNVV:YGKIP:VVE:AXGXR, STPPTXX:VPT:SPIN-AA$^{17}$-LYF:DSANNVV:YGKIP:VVE:SXGXR, HVPKPXX:APT:SPIN-AA$^{17}$-LYF:DDSSNVI:YGKIP:VVR:SXGXH, RVPSTXX:APV:SPIN-AA$^{17}$-LYF:DNGRVLL:YGKIP:VEE:XGXL, ASAAPXX:VPQ:SPIN-AA$^{17}$-LYF:VGRKPKV:YGKIP:VRS:XKXS, ASASPXX:VSQ:SPIN-AA$^{17}$-LYF:IGKTPKI:YGKIP:VKS:XKXS, ASASPXX:VPQ:SPIN-AA$^{17}$-LYF:VGRTPKV:YGKIP:VKS:XKXS, NDEGLEX:VPT:SPIN-AA$^{17}$-LYF:RIKPHQGQH:YGKIP:QHN:KXEXR, NDEGLEX:VPT:SPIN-AA$^{17}$-LYF:RIKPHQGQH:YGKIP:EHS:QXEXR, SSVKXQP:SRV:SPIN-AA$^{17}$-LYF:EYVRKKPKL:YGKIP:EEH:LEXAXA, RNVQXRP:TQV:SPIN-AA$^{17}$-LYF:EIVRKKPIF:YGKIP:EDH:LAXKXE, KIPKAXX:VPA:SPIS-AA$^{17}$-LYI:DENEKVV:YKQYE:VVE:GXGXR, GIPEPXX:VPA:SPIS-AA$^{17}$-LYI:DENKNVV:YKQYE:VVE:SXAXR, SIPKAXX:VPA:SPIS-AA$^{17}$-LYI:DEYDKVV:YKQYE:VVE:GXGXR, HVTKPTX:VPA:SPIS-AA$^{17}$-LYI:DDSSNVI:YKQYE:VVE:SXGXH, YVPKPXX:VPA:SPIS-AA$^{17}$-LYI:DDSSNVI:YKQYE:VVE:SXGXH, TVPKPXX:VPA:SPIS-AA$^{17}$-LYI:DDSSNVI:YKQYE:VVE:AXGXH, AVPKAXX:VPA:SPIS-AA$^{17}$-LYI:DSSNNVI:YKQYE:VVE:AXGXH, KVGKAXX:VPA:SPIS-AA$^{17}$-LYI:DDMGVPT:YKQYE:VVE:XGXR, KASKAXX:VPA:SPIS-AA$^{17}$-LYI:DKGVVTY:YKQYE:VVE:EXGXR, GSAGPXX:VPA:SPIS-AA$^{17}$-LYI:NDKQQII:YKQYE:VVE:RXGXS, STPPTXX:VPA:SPIS-AA$^{17}$-LYI:DSANNVV:YKQYE:VVE:SXGXR, HVPKPXX:VPA:SPIS-AA$^{17}$-LYI:DDSSNVI:YKQYE:VVE:SXGXH, RVPSTXX:VPA:SPIS-AA$^{17}$-LYI:DNGRVLL:YKQYE:VVE:XGXL, ASAAPXX:VPA:SPIS-AA$^{17}$-LYI:VGRKPKV:YKQYE:VVE:XKXS, ASASPXX:VPA:SPIS-AA$^{17}$-LYI:IGKTPKI:YKQYE:VVE:XKXS, ASASPXX:VPA:SPIS-AA$^{17}$-LYI:VGRTPKV:YKQYE:VVE:XKXS, NDEGLEX:VPA:SPIS-AA$^{17}$-LYI:RIKPHQGQH:YKQYE:VVE:KXEXR, NDE-

GLEX:VPA:SPIS-AA$^{17}$-LYI:RIKPHQGQH:YKQYE:VVE:QXEXR, SSVKXQP:VPA:SPIS-AA$^{17}$-LYI:EYVRKKPKL:YKQYE:VVE:LEXAXA, RNVQXRP:VPA:SPIS-AA$^{17}$-LYI:EIVRKKPIF:YKQYE:VVE:LAXKXE, KIPKAXX:VPT:SPIS-AA$^{17}$-LYI:DENEKVV:YKQYE:VVE:GXGXR, GIPEPXX:VPE:SPIS-AA$^{17}$-LYI:DENKNVV:YKQYE:TVE:SXAXR, SIPKAXX:VPT:SPIS-AA$^{17}$-LYI:DEYDKVV:YKQYE:VVE:GXGXR, HVTKPTX:APT:SPIS-AA$^{17}$-LYI:DDSSNVI:YKQYE:VVR:SXGXH, YVPKPXX:APT:SPIS-AA$^{17}$-LYI:DDSSNVI:YKQYE:VVR:SXGXH, TVPKPXX:APT:SPIS-AA$^{17}$-LYI:DDSSNVI:YKQYE:VVR:AXGXH, AVPKAXX:APT:SPIS-AA$^{17}$-LYI:DSSNNVI:YKQYE:VVK:AXGXH, KVGKAXX:VPT:SPIS-AA$^{17}$-LYI:DDMGVPT:YKQYE:VAE:XGXR, KASKAXX:VPT:SPIS-AA$^{17}$-LYI:DKGVVTY:YKQYE:AVS:EXGXR, GSAGPXX:TPT:SPIS-AA$^{17}$-LYI:NDKQQII:YKQYE:VVD:RXGXS, STPPTXX:VPT:SPIS-AA$^{17}$-LYI:DSANNVV:YKQYE:VVE:SXGXR, HVPKPXX:APT:SPIS-AA$^{17}$-LYI:DDSSNVI:YKQYE:VVR:SXGXH, RVPSTXX:APV:SPIS-AA$^{17}$-LYI:DNGRVLL:YKQYE:VEE:XGXL, ASAAPXX:VPQ:SPIS-AA$^{17}$-LYI:VGRKPKV:YKQYE:VRS:XKXS, ASASPXX:VSQ:SPIS-AA$^{17}$-LYI:IGKTPKI:YKQYE:VKS:XKXS, ASASPXX:VPQ:SPIS-AA$^{17}$-LYI:VGRTPKV:YKQYE:VKS:XKXS, NDEGLEX:VPT:SPIS-AA$^{17}$-LYI:RIKPHQGQH:YKQYE:QHN:KXEXR, NDEGLEX:VPT:SPIS-AA$^{17}$-LYI:RIKPHQGQH:YKQYE:EHS:QXEXR, SSVKXQP:SRV:SPIS-AA$^{17}$-LYI:EYVRKKPKL:YKQYE:EEH:LEXAXA, RNVQXRP:TQV:SPIS-AA$^{17}$-LYI:EIVRKKPIF:YKQYE:EDH:LAXKXE, KIPKAXX:VPT:SPIS-AA$^{17}$-LFI:DENEKVV:YKQYE:VVE:GXGXR, GIPEPXX:VPT:SPIS-AA$^{17}$-LFI:DENKNVV:YKQYE:VVE:SXAXR, SIPKAXX:VPT:SPIS-AA$^{17}$-LFI:DEYDKVV:YKQYE:VVE:GXGXR, HVTKPTX:VPT:SPIS-AA$^{17}$-LFI:DDSSNVI:YKQYE:VVE:SXGXH, YVPKPXX:VPT:SPIS-AA$^{17}$-LFI:DDSSNVI:YKQYE:VVE:SXGXH, TVPKPXX:VPT:SPIS-AA$^{17}$-LFI:DDSSNVI:YKQYE:VVE:AXGXH, AVPKAXX:VPT:SPIS-AA$^{17}$-LFI:DSSNNVI:YKQYE:VVE:AXGXH, KVGKAXX:VPT:SPIS-AA$^{17}$-LFI:DDMGVPT:YKQYE:VVE:XGXR, KASKAXX:VPT:SPIS-AA$^{17}$-LFI:DKGVVTY:YKQYE:VVE:EXGXR, GSAGPXX:VPT:SPIS-AA$^{17}$-LFI:NDKQQII:YKQYE:WE:RXGXS, AAPASXX:VPT:SPIS-AA$^{17}$-LFI:DAANNVV:YKQYE:VVE:AXGXR, HVPKPXX:VPT:SPIS-AA$^{17}$-LFI:DDSSNVI:YKQYE:VVE:SXGXH, RVPSTXX:VPT:SPIS-AA$^{17}$-LFI:DNGRVLL:YKQYE:WE:XGXL, ASAAPXX:VPT:SPIS-AA$^{17}$-LFI:VGRKPKV:YKQYE:VVE:XKXS, ASASPXX:VPT:SPIS-AA$^{17}$-LFI:IGKTPKI:YKQYE:VVE:XKXS, ASASPXX:VPT:SPIS-AA$^{17}$-LFI:VGRTPKV:YKQYE:VVE:XKXS, NDEGLEX:VPT:SPIS-AA$^{17}$-LFI:RIKPHQGQH:YKQYE:VVE:KXEXR, NDEGLEX:VPT:SPIS-AA$^{17}$-LFI:RIKPHQGQH:YKQYE:VVE:QXEXR, SSVKXQP:VPT:SPIS-AA$^{17}$-LFI:EYVRKKPKL:YKQYE:VVE:LEXAXA, RNVQXRP:VPT:SPIS-AA$^{17}$-LFI:EIVRKKPIF:YKQYE:VVE:LAXKXE, GIPEPXX:VPE:SPIS-AA$^{17}$-LFI:DENKNVV:YKQYE:TVE:SXAXR, HVTKPTX:APT:SPIS-AA$^{17}$-LFI:DDSSNVI:YKQYE:VVR:SXGXH, YVPKPXX:APT:SPIS-AA$^{17}$-LFI:DDSSNVI:YKQYE:VVR:SXGXH, TVPKPXX:APT:SPIS-AA$^{17}$-LFI:DDSSNVI:YKQYE:VVR:AXGXH, AVPKAXX:APT:SPIS-AA$^{17}$-LFI:DSSNNVI:YKQYE:VVK:AXGXH, KVGKAXX:VPT:SPIS-AA$^{17}$-LFI:DDMGVPT:YKQYE:VAE:XGXR, KASKAXX:VPT:SPIS-AA$^{17}$-LFI:DKGVVTY:YKQYE:AVS:EXGXR, GSAGPXX:TPT:SPIS-AA$^{17}$-LFI:NDKQQII:YKQYE:VVD:RXGXS, AAPASXX:VPA:SPIS-AA$^{17}$-LFI:DAANNVV:YKQYE:VVE:AXGXR, HVPKPXX:APT:SPIS-AA$^{17}$-LFI:DDSSNVI:YKQYE:VVR:SXGXH, RVPSTXX:APV:SPIS-AA$^{17}$-LFI:DNGRVLL:YKQYE:VEE:XGXL, ASAAPXX:VPQ:SPIS-AA$^{17}$-LFI:VGRKPKV:YKQYE:VRS:XKXS, ASASPXX:VSQ:SPIS-AA$^{17}$-LFI:IGKTPKI:YKQYE:VKS:XKXS, ASASPXX:VPQ:SPIS-AA$^{17}$-LFI:VGRTPKV:YKQYE:VKS:XKXS, NDEGLEXVPT:SPIS-AA$^{17}$-LFI:RIKPHQGQH:YKQYE:QHN:KXEXR, NDEGLEX:VPT:SPIS-AA$^{17}$-LFI:RIKPHQGQH:YKQYE:EHS:QXEXR, SSVKXQP:SRV:SPIS-AA$^{17}$-LFI:EYVRKKPKL:YKQYE:EEH:LEXAXA, RNVQXRP:TQV:SPIS-AA$^{17}$-LFI:EIVRKKPIF:YKQYE:EDH:LAXKXE, KIPKAXX:APV:KPLS-AA$^{17}$-LYV:DENEKVV:DHHKD:VEE:GXGXR, GIPEPXX:APV:KPLS-AA$^{17}$-LYV:DENKNVV:DHHKD:VEE:SXAXR, SIPKAXX:APV:KPLS-AA$^{17}$-LYV:DEYDKVV:DHHKD:VEE:GXGXR, HVTKPTX:APV:KPLS-AA$^{17}$-LYV:DDSSNVI:DHHKD:VEE:SXGXH, YVPKPXX:APV:KPLS-AA$^{17}$-LYV:DDSSNVI:DHHKD:VEE:SXGXH, TVPKPXX:APV:KPLS-AA$^{17}$-LYV:DDSSNVI:DHHKD:VEE:AXGXH, AVPKAXX:APV:KPLS-AA$^{17}$-LYV:DSSNNVI:DHHKD:VEE:AXGXH, KVGKAXX:APV:KPLS-AA$^{17}$-LYV:DDMGVPT:DHHKD:VEE:XGXR, KASKAXX:APV:KPLS-AA$^{17}$-LYV:DKGVVTY:DHHKD:VEE:EXGXR, GSAGPXX:APV:KPLS-AA$^{17}$-LYV:NDKQQII:DHHKD:VEE:RXGXS, AAPASXX:APV:KPLS-AA$^{17}$-LYV:DAANNVV:DHHKD:VEE:AXGXR, STPPTXX:APV:KPLS-AA$^{17}$-LYV:DSANNVV:DHHKD:VEE:SXGXR, HVPKPXX:APV:KPLS-AA$^{17}$-LYV:DDSSNVI:DHHKD:VEE:SXGXH, ASAAPXX:APV:KPLS-AA$^{17}$-LYV:VGRKPKV:DHHKD:VEE:XKXS, ASASPXX:APV:KPLS-AA$^{17}$-LYV:IGKTPKI:DHHKD:VEE:XKXS, ASASPXX:APV:KPLS-AA$^{17}$-LYV:VGRTPKV:DHHKD:VEE:XKXS, NDEGLEX:APV:KPLS-AA$^{17}$-LYV:RIKPHQGQH:DHHKD:VEE:KXEXR, NDEGLEX:APV:KPLS-AA$^{17}$-LYV:RIKPHQGQH:DHHKD:VEE:QXEXR, SSVKXQP:APV:KPLS-AA$^{17}$-LYV:EYVRKKPKL:DHHKD:VEE:LEXAXA, RNVQXRP:APV:KPLS-AA$^{17}$-LYV:EIVRKKPIF:DHHKD:VEE:LAXKXE, KIPKAXX:VPT:KPLS-AA$^{17}$-LYV:DENEKVV:DHHKD:VVE:GXGXR, GIPEPXX:VPE:KPLS-AA$^{17}$-LYV:DENKNVV:DHHKD:TVE:SXAXR, SIPKAXX:VPT:KPLS-AA$^{17}$-LYV:DEYDKVV:DHHKD:VVE:GXGXR, HVTKPTX:APT:KPLS-AA$^{17}$-LYV:DDSSNVI:DHHKD:VVR:SXGXH, YVPKPXX:APT:KPLS-AA$^{17}$-LYV:DDSSNVI:DHHKD:VVR:SXGXH, TVPKPXX:APT:KPLS-AA$^{17}$-LYV:DDSSNVI:DHHKD:VVR:AXGXH, AVPKAXX:APT:KPLS-AA$^{17}$-LYV:DSSNNVI:DHHKD:VVK:AXGXH, KVGKAXX:VPT:KPLS-AA$^{17}$-LYV:DDMGVPT:DHHKD:VAE:XGXR, KASKAXX:VPT:KPLS-AA$^{17}$-LYV:DKGVVTY:DHHKD:AVS:EXGXR, GSAGPXX:TPT:KPLS-AA$^{17}$-LYV:NDKQQII:DHHKD:VVD:RXGXS, AAPASXX:VPA:KPLS-AA$^{17}$-LYV:DAANNVV:DHHKD:VVE:AXGXR, STPPTXX:VPT:KPLS-AA$^{17}$-LYV:DSANNVV:DHHKD:VVE:SXGXR, HVPKPXX:APT:KPLS-AA$^{17}$-LYV:DDSSNVI:DHHKD:VVR:SXGXH, ASAAPXX:VPQ:KPLS-AA$^{17}$-LYV:VGRKPKV:DHHKD:VRS:XKXS, ASASPXX:VSQ:KPLS-AA$^{17}$-LYV:IGKTPKI:DHHKD:VKS:XKXS, ASASPXX:VPQ:KPLS-

AA17-LYV:VGRTPKV:DHHKD:VKS:XKXS, NDEGLEX:VPT:KPLS-AA17-LYV:RIKPHQGQH:DHHKD:QHN:KXEXR, NDEGLEX:VPT:KPLS-AA17-LYV:RIKPHQGQH:DHHKD:EHS:QXEXR, SSVKXQP:SRV:KPLS-AA17-LYV:EYVRKKP-KL:DHHKD:EEH:LEXAXA, RNVQXRP:TQV:KPLS-AA17-LYV:EIVRKKPIF:DHHKD:EDH:LAXKXE, KIP-KAXX:VPQ:EPLP-AA17-VYY:DENEKVV:EQLSN:VRS:GXGXR, GIPEPXX:VPQ:EPLP-AA17-VYY:DENKNVV:EQL-SN:VRS:SXAXR, SIPKAXX:VPQ:EPLP-AA17-VYY:DEYDKVV:EQLSN:VRS:GXGXR, HVTKPTX:VPQ:EPLP-AA17-VYY:DDSSNVI:EQLSN:VRS:SXGXH, YVPKPXX:VPQ:EPLP-AA17-VYY:DDSSNVI:EQLSN:VRS:SXGXH, TVP-KPXX:VPQ:EPLP-AA17-VYY:DDSSNVI:EQLSN:VRS:AXGXH, AVPKAXX:VPQ:EPLP-AA17-VYY:DSSNNVI:EQL-SN:VRS:AXGXH, KVGKAXX:VPQ:EPLP-AA17-VYY:DDMGVPT:EQLSN:VRS:XGXR, KASKAXX:VPQ:EPLP-AA17-VYY:DKGVVTY:EQLSN:VRS:EXGXR, GSAGPXX:VPQ:EPLP-AA17-VYY:NDKQQII:EQLSN:VRS:RXGXS, AA-PASXX:VPQ:EPLP-AA17-VYY:DAANNVV:EQLSN:VRS:AXGXR, STPPTXX:VPQ:EPLP-AA17-VYY:DSANNVV:EQL-SN:VRS:SXGXR, HVPKPXX:VPQ:EPLP-AA17-VYY:DDSSNVI:EQLSN:VRS:SXGXH, RVPSTXX:VPQ:EPLP-AA17-VYY:DNGRVLL:EQLSN:VRS:XGXL, ASASPXX:VPQ:EPLP-AA17-VYY:IGKTPKI:EQLSN:VRS:XKXS, ASAS-PXX:VPQ:EPLP-AA17-VYY:VGRTPKV:EQLSN:VRS:XKXS, NDEGLEX:VPQ:EPLP-AA17-VYY:RIKPHQGQH:EQL-SN:VRS:KXEXR, NDEGLEX:VPQ:EPLP-AA17-VYY:RIKPHQGQH:EQLSN:VRS:QXEXR, SSVKXQP:VPQ:EPLP-AA17-VYY:EYVRKKPKL:EQLSN:VRS:LEXAXA, RNVQXRP:VPQ:EPLP-AA17-VYY:EIVRKKPIF:EQL-SN:VRS:LAXKXE, KIPKAXX:VPT:EPLP-AA17-VYY:DENEKVV:EQLSN:VVE:GXGXR, GIPEPXX:VPE:EPLP-AA17-VYY:DENKNVV:EQLSN:TVE:SXAXR, SIPKAXX:VPT:EPLP-AA17-VYY:DEYDKVV:EQLSN:VVE:GXGXR, HVTKPTX:APT:EPLP-AA17-VYY:DDSSNVI:EQLSN:VVR:SXGXH, YVPKPXX:APT:EPLP-AA17-VYY:DDSSNVI:EQL-SN:VVR:SXGXH, TVPKPXX:APT:EPLP-AA17-VYY:DDSSNVI:EQLSN:VVR:AXGXH, AVPKAXX:APT:EPLP-AA17-VYY:DSSNNVI:EQLSN:VVK:AXGXH, KVGKAXX:VPT:EPLP-AA17-VYY:DDMGVPT:EQLSN:VAE:XGXR, KASKAXX:VPT:EPLP-AA17-VYY:DKGVVTY:EQLSN:AVS:EXGXR, GSAGPXX:TPT:EPLP-AA17-VYY:NDKQQII:EQL-SN:VVD:RXGXS, AAPASXX:VPA:EPLP-AA17-VYY:DAANNVV:EQLSN:VVE:AXGXR, STPPTXX:VPT:EPLP-AA17-VYY:DSANNVV:EQLSN:VVE:SXGXR, HVPKPXX:APT:EPLP-AA17-VYY:DDSSNVI:EQLSN:VVR:SXGXH, RVP-STXX:APV:EPLP-AA17-VYY:DNGRVLL:EQLSN:VEE:XGXL, ASASPXX:VSQ:EPLP-AA17-VYY:IGKTPKI:EQL-SN:VKS:XKXS, ASASPXX:VPQ:EPLP-AA17-VYY:VGRTPKV:EQLSN:VKS:XKXS, NDEGLEX:VPT:EPLP-AA17-VYY:RIKPHQGQH:EQLSN:QHN:KXEXR, NDEGLEX:VPT:EPLP-AA17-VYY:RIKPHQGQH:EQLSN:EHS:QX-EXR, SSVKXQP:SRV:EPLP-AA17-VYY:EYVRKKPKL:EQLSN:EEH:LEXAXA, RNVQXRP:TQV:EPLP-AA17-VYY:EIVRKKPIF:EQLSN:EDH:LAXKXE, KIPKAXX:VSQ:EPLT-AA17-LYY:DENEKVV:EQLSN:VKS:GXGXR, GIPEPXX:VSQ:EPLT-AA17-LYY:DENKNVV:EQLSN:VKS:SXAXR, SIPKAXX:VSQ:EPLT-AA17-LYY:DEYDKVV:EQL-SN:VKS:GXGXR, HVTKPTX:VSQ:EPLT-AA17-LYY:DDSSNVI:EQLSN:VKS:SXGXH, YVPKPXX:VSQ:EPLT-AA17-LYY:DDSSNVI:EQLSN:VKS:SXGXH, TVPKPXX:VSQ:EPLT-AA17-LYY:DDSSNVI:EQLSN:VKS:AXGXH, AVP-KAXX:VSQ:EPLT-AA17-LYY:DSSNNVI:EQLSN:VKS:AXGXH, KVGKAXX:VSQ:EPLT-AA17-LYY:DDMGVPT:EQL-SN:VKS:XGXR, KASKAXX:VSQ:EPLT-AA17-LYY:DKGVVTY:EQLSN:VKS:EXGXR, GSAGPXX:VSQ:EPLT-AA17-LYY:NDKQQII:EQLSN:VKS:RXGXS, AAPASXX:VSQ:EPLT-AA17-LYY:DAANNVV:EQLSN:VKS:AXGXR, STPP-TXX:VSQ:EPLT-AA17-LYY:DSANNVV:EQLSN:VKS:SXGXR, HVPKPXX:VSQ:EPLT-AA17-LYY:DDSSNVI:EQL-SN:VKS:SXGXH, RVPSTXX:VSQ:EPLT-AA17-LYY:DNGRVLL:EQLSN:VKS:XGXL, ASAAPXX:VSQ:EPLT-AA17-LYY:VGRKPKV:EQLSN:VKS:XKXS, ASASPXX:VSQ:EPLT-AA17-LYY:VGRTPKV:EQLSN:VKS:XKXS, NDE-GLEX:VSQ:EPLT-AA17-LYY:RIKPHQGQH:EQLSN:VKS:KXEXR, NDEGLEX:VSQ:EPLT-AA17-LYY:RIK-PHQGQH:EQLSN:VKS:QXEXR, SSVKXQP:VSQ:EPLT-AA17-LYY:EYVRKKPKL:EQLSN:VKS:LEXAXA, RNVQXRP:VSQ:EPLT-AA17-LYY:EIVRKKPIF:EQLSN:VKS:LAXKXE, KIPKAXX:VPT:EPLT-AA17-LYY:DENEKVV:EQLSN:VVE:GXGXR, GIPEPXX:VPE:EPLT-AA17-LYY:DENKNVV:EQLSN:TVE:SXAXR, SIP-KAXX:VPT:EPLT-AA17-LYY:DEYDKVV:EQLSN:VVE:GXGXR, HVTKPTX:APT:EPLT-AA17-LYY:DDSSNVI:EQL-SN:VVR:SXGXH, YVPKPXX:APT:EPLT-AA17-LYY:DDSSNVI:EQLSN:VVR:SXGXH, TVPKPXX:APT:EPLT-AA17-LYY:DDSSNVI:EQLSN:VVR:AXGXH, AVPKAXX:APT:EPLT-AA17-LYY:DSSNNVI:EQLSN:VVK:AXGXH, KVG-KAXX:VPT:EPLT-AA17-LYY:DDMGVPT:EQLSN:VAE:XGXR, KASKAXX:VPT:EPLT-AA17-LYY:DKGVVTY:EQL-SN:AVS:EXGXR, GSAGPXX:TPT:EPLT-AA17-LYY:NDKQQII:EQLSN:VVD:RXGXS, AAPASXX:VPA:EPLT-AA17-LYY:DAANNVV:EQLSN:VVE:AXGXR, STPPTXX:VPT:EPLT-AA17-LYY:DSANNVV:EQLSN:VVE:SXGXR, HVP-KPXX:APT:EPLT-AA17-LYY:DDSSNVI:EQLSN:VVR:SXGXH, RVPSTXX:APV:EPLT-AA17-LYY:DNGRVLL:EQL-SN:VEE:XGXL, ASAAPXX:VPQ:EPLT-AA17-LYY:VGRKPKV:EQLSN:VRS:XKXS, ASASPXX:VPQ:EPLT-AA17-LYY:VGRTPKV:EQLSN:VKS:XKXS, NDEGLEX:VPT:EPLT-AA17-LYY:RIKPHQGQH:EQLSN:QHN:KXEXR, NDEGLEX:VPT:EPLT-AA17-LYY:RIKPHQGQH:EQLSN:EHS:QXEXR, SSVKXQP:SRV:EPLT-AA17-LYY:EYVRKKP-KL:EQLSN:EEH:LEXAXA, RNVQXRP:TQV:EPLT-AA17-LYY:EIVRKKPIF:EQLSN:EDH:LAXKXE, KIP-KAXX:VPQ:EPLT-AA17-LYY:DENEKVV:EQLSN:VKS:GXGXR, GIPEPXX:VPQ:EPLT-AA17-LYY:DENKNVV:EQL-SN:VKS:SXAXR, SIPKAXX:VPQ:EPLT-AA17-LYY:DEYDKVV:EQLSN:VKS:GXGXR, HVTKPTX:VPQ:EPLT-AA17-LYY:DDSSNVI:EQLSN:VKS:SXGXH, YVPKPXX:VPQ:EPLT-AA17-LYY:DDSSNVI:EQLSN:VKS:SXGXH, TVPK-PXX:VPQ:EPLT-AA17-LYY:DDSSNVI:EQLSN:VKS:AXGXH, AVPKAXX:VPQ:EPLT-AA17-LYY:DSSNNVI:EQL-SN:VKS:AXGXH, KVGKAXX:VPQ:EPLT-AA17-LYY:DDMGVPT:EQLSN:VKS:XGXR, KASKAXX:VPQ:EPLT-AA17-LYY:DKGVVTY:EQLSN:VKS:EXGXR, GSAGPXX:VPQ:EPLT-AA17-LYY:NDKQQII:EQLSN:VKS:RXGXS, AA-

PASXX:VPQ:EPLT-AA$^{17}$-LYY:DAANNVV:EQLSN:VKS:AXGXR, STPPTXX:VPQ:EPLT-AA$^{17}$-LYY:DSANNVV:EQLSN:VKS:SXGXR, HVPKPXX:VPQ:EPLT-AA$^{17}$-LYY:DDSSNVI:EQLSN:VKS:SXGXH, RVPSTXX:VPQ:EPLT-AA$^{17}$-LYY:DNGRVLL:EQLSN:VKS:XGXL, ASAAPXX:VPQ:EPLT-AA$^{17}$-LYY:VGRKPKV:EQLSN:VKS:XKXS, ASASPXX:VPQ:EPLT-AA$^{17}$-LYY:IGKTPKI:EQLSN:VKS:XKXS, NDEGLEX:VPQ:EPLT-AA$^{17}$-LYY:RIKPHQGQH:EQLSN:VKS:KXEXR, NDEGLEX:VPQ:EPLT-AA$^{17}$-LYY:RIKPHQGQH:EQLSN:VKS:QXEXR, SSVKXQP:VPQ:EPLT-AA$^{17}$-LYY:EYVRKKPKL:EQLSN:VKS:LEXAXA, RNVQXRP:VPQ:EPLT-AA$^{17}$-LYY:EIVRKKPIF:EQLSN:VKS:LAXKXE, ASASPXX:VSQ:EPLT-AA$^{17}$-LYY:IGKTPKI:EQLSN:VKS:XKXS, KIPKAXX:VPT:SNIT-AA$^{17}$-QIM:DENEKVV:IGEMS:QHN:GXGXR, GIPEPXX:VPT:SNIT-AA$^{17}$-QIM:DENKNVV:IGEMS:QHN:SXAXR, SIPKAXX:VPT:SNIT-AA$^{17}$-QIM:DEYDKVV:IGEMS:QHN:GXGXR, HVTKPTX:VPT:SNIT-AA$^{17}$-QIM:DDSSNVI:IGEMS:QHN:SXGXH, YVPKPXX:VPT:SNIT-AA$^{17}$-QIM:DDSSNVI:IGEMS:QHN:SXGXH, TVPKPXX:VPT:SNIT-AA$^{17}$-QIM:DDSSNVI:IGEMS:QHN:AXGXH, AVPKAXX:VPT:SNIT-AA$^{17}$-QIM:DSSNNVI:IGEMS:QHN:AXGXH, KVGKAXX:VPT:SNIT-AA$^{17}$-QIM:DDMGVPT:IGEMS:QHN:XGXR, KASKAXX:VPT:SNIT-AA$^{17}$-QIM:DKGVVTY:IGEMS:QHN:EXGXR, GSAGPXX:VPT:SNIT-AA$^{17}$-QIM:NDKQQII:IGEMS:QHN:RXGXS, AAPASXX:VPT:SNIT-AA$^{17}$-QIM:DAANNVV:IGEMS:QHN:AXGXR, STPPTXX:VPT:SNIT-AA$^{17}$-QIM:DSANNVV:IGEMS:QHN:SXGXR, HVPKPXX:VPT:SNIT-AA$^{17}$-QIM:DDSSNVI:IGEMS:QHN:SXGXH, RVPSTXX:VPT:SNIT-AA$^{17}$-QIM:DNGRVLL:IGEMS:QHN:XGXL, ASAAPXX:VPT:SNIT-AA$^{17}$-QIM:VGRKPKV:IGEMS:QHN:XKXS, ASASPXX:VPT:SNIT-AA$^{17}$-QIM:IGKTPKI:IGEMS:QHN:XKXS, ASASPXX:VPT:SNIT-AA$^{17}$-QIM:VGRTPKV:IGEMS:QHN:XKXS, NDEGLEX:VPT:SNIT-AA$^{17}$-QIM:RIKPHQGQH:IGEMS:QHN:QXEXR, NDEGLEX:VPT:SNIT-AA$^{17}$-QIM:RIKPHQGQH:IGEMS:QHN:KXEXR, SSVKXQP:VPT:SNIT-AA$^{17}$-QIM:EYVRKKPKL:IGEMS:QHN:LEXAXA, RNVQXRP:VPT:SNIT-AA$^{17}$-QIM:EIVRKKPIF:IGEMS:QHN:LAXKXE, KIPKAXX:VPT:SNIT-AA$^{17}$-QIM:DENEKVV:IGEMS:VVE:GXGXR, GIPEPXX:VPE:SNIT-AA$^{17}$-QIM:DENKNVV:IGEMS:TVE:SXAXR, SIPKAXX:VPT:SNIT-AA$^{17}$-QIM:DEYDKVV:IGEMS:VVE:GXGXR, HVTKPTX:APT:SNIT-AA$^{17}$-QIM:DDSSNVI:IGEMS:VVR:SXGXH, YVPKPXX:APT:SNIT-AA$^{17}$-QIM:DDSSNVI:IGEMS:VVR:SXGXH, TVPKPXX:APT:SNIT-AA$^{17}$-QIM:DDSSNVI:IGEMS:VVR:AXGXH, AVPKAXX:APT:SNIT-AA$^{17}$-QIM:DSSNNVI:IGEMS:VVK:AXGXH, KVGKAXX:VPT:SNIT-AA$^{17}$-QIM:DDMGVPT:IGEMS:VAE:XGXR, KASKAXX:VPT:SNIT-AA$^{17}$-QIM:DKGVVTY:IGEMS:AVS:EXGXR, GSAGPXX:TPT:SNIT-AA$^{17}$-QIM:NDKQQII:IGEMS:VVD:RXGXS, AAPASXX:VPA:SNIT-AA$^{17}$-QIM:DAANNVV:IGEMS:VVE:AXGXR, STPPTXX:VPT:SNIT-AA$^{17}$-QIM:DSANNVV:IGEMS:VVE:SXGXR, HVPKPXX:APT:SNIT-AA$^{17}$-QIM:DDSSNVI:IGEMS:VVR:SXGXH, RVPSTXX:APV:SNIT-AA$^{17}$-QIM:DNGRVLL:IGEMS:VEE:XGXL, ASAAPXX:VPQ:SNIT-AA$^{17}$-QIM:VGRKPKV:IGEMS:VRS:XKXS, ASASPXX:VSQ:SNIT-AA$^{17}$-QIM:IGKTPKI:IGEMS:VKS:XKXS, ASASPXX:VPQ:SNIT-AA$^{17}$-QIM:VGRTPKV:IGEMS:VKS:XKXS, NDEGLEX:VPT:SNIT-AA$^{17}$-QIM:RIKPHQGQH:IGEMS:EHS:QXEXR, SSVKXQP:SRV:SNIT-AA$^{17}$-QIM:EYVRKKPKL:IGEMS:EEH:LEXAXA, RNVQXRP:TQV:SNIT-AA$^{17}$-QIM:EIVRKKPIF:IGEMS:EDH:LAXKXE, KIPKAXX:VPT:SNIT-AA$^{17}$-QIM:DENEKVV:LGEMS:EHS:GXGXR, GIPEPXX:VPT:SNIT-AA$^{17}$-QIM:DENKNVV:LGEMS:EHS:SXAXR, SIPKAXX:VPT:SNIT-AA$^{17}$-QIM:DEYDKVV:LGEMS:EHS:GXGXR, HVTKPTX:VPT:SNIT-AA$^{17}$-QIM:DDSSNVI:LGEMS:EHS:SXGXH, YVPKPXX:VPT:SNIT-AA$^{17}$-QIM:DDSSNVI:LGEMS:EHS:SXGXH, TVPKPXX:VPT:SNIT-AA$^{17}$-QIM:DDSSNVI:LGEMS:EHS:AXGXH, AVPKAXX:VPT:SNIT-AA$^{17}$-QIM:DSSNNVI:LGEMS:EHS:AXGXH, KVGKAXX:VPT:SNIT-AA$^{17}$-QIM:DDMGVPT:LGEMS:EHS:XGXR, KASKAXX:VPT:SNIT-AA$^{17}$-QIM:DKGVVTY:LGEMS:EHS:EXGXR, GSAGPXX:VPT:SNIT-AA$^{17}$-QIM:NDKQQII:LGEMS:EHS:RXGXS, AAPASXX:VPT:SNIT-AA$^{17}$-QIM:DAANNVV:LGEMS:EHS:AXGXR, STPPTXX:VPT:SNIT-AA$^{17}$-QIM:DSANNVV:LGEMS:EHS:SXGXR, HVPKPXX:VPT:SNIT-AA$^{17}$-QIM:DDSSNVI:LGEMS:EHS:SXGXH, RVPSTXX:VPT:SNIT-AA$^{17}$-QIM:DNGRVLL:LGEMS:EHS:XGXL, ASAAPXX:VPT:SNIT-AA$^{17}$-QIM:VGRKPKV:LGEMS:EHS:XKXS, ASASPXX:VPT:SNIT-AA$^{17}$-QIM:IGKTPKI:LGEMS:EHS:XKXS, ASASPXX:VPT:SNIT-AA$^{17}$-QIM:VGRTPKV:LGEMS:EHS:XKXS, NDEGLEX:VPT:SNIT-AA$^{17}$-QIM:RIKPHQGQH:LGEMS:EHS:KXEXR, SSVKXQP:VPT:SNIT-AA$^{17}$-QIM:EYVRKKPKL:LGEMS:EHS:LEXAXA, RNVQXRP:VPT:SNIT-AA$^{17}$-QIM:EIVRKKPIF:LGEMS:EHS:LAXKXE, KIPKAXX:VPT:SNIT-AA$^{17}$-QIM:DENEKVV:LGEMS:VVE:GXGXR, GIPEPXX:VPE:SNIT-AA$^{17}$-QIM:DENKNVV:LGEMS:TVE:SXAXR, SIPKAXX:VPT:SNIT-AA$^{17}$-QIM:DEYDKVV:LGEMS:VVE:GXGXR, HVTKPTX:APT:SNIT-AA$^{17}$-QIM:DDSSNVI:LGEMS:VVR:SXGXH, YVPKPXX:APT:SNIT-AA$^{17}$-QIM:DDSSNVI:LGEMS:VVR:SXGXH, TVPKPXX:APT:SNIT-AA$^{17}$-QIM:DDSSNVI:LGEMS:VVR:AXGXH, AVPKAXX:APT:SNIT-AA$^{17}$-QIM:DSSNNVI:LGEMS:VVK:AXGXH, KVGKAXX:VPT:SNIT-AA$^{17}$-QIM:DDMGVPT:LGEMS:VAE:XGXR, KASKAXX:VPT:SNIT-AA$^{17}$-QIM:DKGVVTY:LGEMS:AVS:EXGXR, GSAGPXX:TPT:SNIT-AA$^{17}$-QIM:NDKQQII:LGEMS:VVD:RXGXS, AAPASXX:VPA:SNIT-AA$^{17}$-QIM:DAANNVV:LGEMS:VVE:AXGXR, STPPTXX:VPT:SNIT-AA$^{17}$-QIM:DSANNVV:LGEMS:VVE:SXGXR, HVPKPXX:APT:SNIT-AA$^{17}$-QIM:DDSSNVI:LGEMS:VVR:SXGXH, RVPSTXX:APV:SNIT-AA$^{17}$-QIM:DNGRVLL:LGEMS:VEE:XGXL, ASAAPXX:VPQ:SNIT-AA$^{17}$-QIM:VGRKPKV:LGEMS:VRS:XKXS, ASASPXX:VSQ:SNIT-AA$^{17}$-QIM:IGKTPKI:LGEMS:VKS:XKXS, ASASPXX:VPQ:SNIT-AA$^{17}$-QIM:VGRTPKV:LGEMS:VKS:XKXS, NDEGLEX:VPT:SNIT-AA$^{17}$-QIM:RIKPHQGQH:LGEMS:QHN:KXEXR, SSVKXQP:SRV:SNIT-AA$^{17}$-QIM:EYVRKKPKL:LGEMS:EEH:LEXAXA, RNVQXRP:TQV:SNIT-AA$^{17}$-QIM:EIVRKKPIF:LGEMS:EDH:LAXKXE, KIPKAXX:SRV:RSVK-AA$^{17}$-AKV:DENEKVV:KEVQV:EEH:GXGXR, GIPEPXX:SRV:RSVK-AA$^{17}$-AKV:DENKNVV:KEVQV:EEH:SXAXR, SIPKAXX:SRV:RSVK-AA$^{17}$-AKV:DEYDKVV:KEVQV:EEH:GXGXR, HVTKPTX:SRV:RSVK-AA$^{17}$-AKV:DDSSN-

VI:KEVQV:EEH:SXGXH, YVPKPXX:SRV:RSVK-AA$^{17}$-AKV:DDSSNVI:KEVQV:EEH:SXGXH, TVPKPXX:SRV:RSVK-AA$^{17}$-AKV:DDSSNVI:KEVQV:EEH:AXGXH, AVPKAXX:SRV:RSVK-AA$^{17}$-AKV:DSSNNVI:KEVQV:EEH:AXGXH, KVGKAXX:SRV:RSVK-AA$^{17}$-AKV:DDMGVPT:KEVQV:EEH:XGXR, KASKAXX:SRV:RSVK-AA$^{17}$-AKV:DKGVVTY:KEVQV:EEH:EXGXR, GSAGPXX:SRV:RSVK-AA$^{17}$-AKV:NDKQQII:KEVQV:EEH:RXGXS, AAPASXX:SRV:RSVK-AA$^{17}$-AKV:DAANNVV:KEVQV:EEH:AXGXR, STPPTXX:SRV:RSVK-AA$^{17}$-AKV:DSANNVV:KEVQV:EEH:SXGXR, HVPKPXX:SRV:RSVK-AA$^{17}$-AKV:DDSSNVI:KEVQV:EEH:SXGXH, RVPSTXX:SRV:RSVK-AA$^{17}$-AKV:DNGRVLL:KEVQV:EEH:XGXL, ASAAPXX:SRV:RSVK-AA$^{17}$-AKV:VGRKPKV:KEVQV:EEH:XKXS, ASASPXX:SRV:RSVK-AA$^{17}$-AKV:IGKTPKI:KEVQV:EEH:XKXS, ASASPXX:SRV:RSVK-AA$^{17}$-AKV:VGRTPKV:KEVQV:EEH:XKXS, NDEGLEX:SRV:RSVK-AA$^{17}$-AKV:RIKPHQGQH:KEVQV:EEH:KXEXR, NDEGLEX:SRV:RSVK-AA$^{17}$-AKV:RIKPHQGQH:KEVQV:EEH:QXEXR, RNVQXRP:SRV:RSVK-AA$^{17}$-AKV:EIVRKKPIF:KEVQV:EEH:LAXKXE, KIPKAXX:VPT:RSVK-AA$^{17}$-AKV:DENEKVV:KEVQV:VVE:GXGXR, GIPEPXX:VPE:RSVK-AA$^{17}$-AKV:DENKNVV:KEVQV:TVE:SXAXR, SIPKAXX:VPT:RSVK-AA$^{17}$-AKV:DEYDKVV:KEVQV:VVE:GXGXR, HVTKPTX:APT:RSVK-AA$^{17}$-AKV:DDSSNVI:KEVQV:VVR:SXGXH, YVPKPXX:APT:RSVK-AA$^{17}$-AKV:DDSSNVI:KEVQV:VVR:SXGXH, TVPKPXX:APT:RSVK-AA$^{17}$-AKV:DDSSNVI:KEVQV:VVR:AXGXH, AVPKAXX:APT:RSVK-AA$^{17}$-AKV:DSSNNVI:KEVQV:VVK:AXGXH, KVGKAXX:VPT:RSVK-AA$^{17}$-AKV:DDMGVPT:KEVQV:VAE:XGXR, KASKAXX:VPT:RSVK-AA$^{17}$-AKV:DKGVVTY:KEVQV:AVS:EXGXR, GSAGPXX:TPT:RSVK-AA$^{17}$-AKV:NDKQQII:KEVQV:VVD:RXGXS, AAPASXX:VPA:RSVK-AA$^{17}$-AKV:DAANNVV:KEVQV:VVE:AXGXR, STPPTXX:VPT:RSVK-AA$^{17}$-AKV:DSANNVV:KEVQV:VVE:SXGXR, HVPKPXX:APT:RSVK-AA$^{17}$-AKV:DDSSNVI:KEVQV:VVR:SXGXH, RVPSTXX:APV:RSVK-AA$^{17}$-AKV:DNGRVLL:KEVQV:VEE:XGXL, ASAAPXX:VPQ:RSVK-AA$^{17}$-AKV:VGRKPKV:KEVQV:VRS:XKXS, ASASPXX:VSQ:RSVK-AA$^{17}$-AKV:IGKTPKI:KEVQV:VKS:XKXS, ASASPXX:VPQ:RSVK-AA$^{17}$-AKV:VGRTPKV:KEVQV:VKS:XKXS, NDEGLEX:VPT:RSVK-AA$^{17}$-AKV:RIKPHQGQH:KEVQV:QHN:KXEXR, NDEGLEX:VPT:RSVK-AA$^{17}$-AKV:RIKPHQGQH:KEVQV:EHS:QXEXR, RNVQXRP:TQV:RSVK-AA$^{17}$-AKV:EIVRKKPIF:KEVQV:EDH:LAXKXE, KIPKAXX:TQV:RPVQ-AA$^{17}$-RKI:DENEKVV:KKATV:EDH:GXGXR, GIPEPXX:TQV:RPVQ-AA$^{17}$-RKI:DENKNVV:KKATV:EDH:SXAXR, SIPKAXX:TQV:RPVQ-AA$^{17}$-RKI:DEYDKVV:KKATV:EDH:GXGXR, HVTKPTX:TQV:RPVQ-AA$^{17}$-RKI:DDSSNVI:KKATV:EDH:SXGXH, YVPKPXX:TQV:RPVQ-AA$^{17}$-RKI:DDSSNVI:KKATV:EDH:SXGXH, TVPKPXX:TQV:RPVQ-AA$^{17}$-RKI:DDSSNVI:KKATV:EDH:AXGXH, AVPKAXX:TQV:RPVQ-AA$^{17}$-RKI:DSSNNVI:KKATV:EDH:AXGXH, KVGKAXX:TQV:RPVQ-AA$^{17}$-RKI:DDMGVPT:KKATV:EDH:XGXR, KASKAXX:TQV:RPVQ-AA$^{17}$-RKI:DKGVVTY:KKATV:EDH:EXGXR, GSAGPXX:TQV:RPVQ-AA$^{17}$-RKI:NDKQQII:KKATV:EDH:RXGXS, AAPASXX:TQV:RPVQ-AA$^{17}$-RKI:DAANNVV:KKATV:EDH:AXGXR, STPPTXX:TQV:RPVQ-AA$^{17}$-RKI:DSANNVV:KKATV:EDH:SXGXR, HVPKPXX:TQV:RPVQ-AA$^{17}$-RKI:DDSSNVI:KKATV:EDH:SXGXH, RVPSTXX:TQV:RPVQ-AA$^{17}$-RKI:DNGRVLL:KKATV:EDH:XGXL, ASAAPXX:TQV:RPVQ-AA$^{17}$-RKI:VGRKPKV:KKATV:EDH:XKXS, ASASPXX:TQV:RPVQ-AA$^{17}$-RKI:IGKTPKI:KKATV:EDH:XKXS, ASASPXX:TQV:RPVQ-AA$^{17}$-RKI:VGRTPKV:KKATV:EDH:XKXS, NDEGLEX:TQV:RPVQ-AA$^{17}$-RKI:RIKPHQGQH:KKATV:EDH:KXEXR, NDEGLEX:TQV:RPVQ-AA$^{17}$-RKI:RIKPHQGQH:KKATV:EDH:QXEXR, SSVKXQP:TQV:RPVQ-AA$^{17}$-RKI:EYVRKKPKL:KKATV:EDH:LEXAXA, KIPKAXX:VPT:RPVQ-AA$^{17}$-RKI:DENEKVV:KKATV:VVE:GXGXR, GIPEPXX:VPE:RPVQ-AA$^{17}$-RKI:DENKNVV:KKATV:TVE:SXAXR, SIPKAXX:VPT:RPVQ-AA$^{17}$-RKI:DEYDKVV:KKATV:VVE:GXGXR, HVTKPTX:APT:RPVQ-AA$^{17}$-RKI:DDSSNVI:KKATV:VVR:SXGXH, YVPKPXX:APT:RPVQ-AA$^{17}$-RKI:DDSSNVI:KKATV:VVR:SXGXH, TVPKPXX:APT:RPVQ-AA$^{17}$-RKI:DDSSNVI:KKATV:VVR:AXGXH, AVPKAXX:APT:RPVQ-AA$^{17}$-RKI:DSSNNVI:KKATV:VVK:AXGXH, KVGKAXX:VPT:RPVQ-AA$^{17}$-RKI:DDMGVPT:KKATV:VAE:XGXR, KASKAXX:VPT:RPVQ-AA$^{17}$-RKI:DKGVVTY:KKATV:AVS:EXGXR, GSAGPXX:TPT:RPVQ-AA$^{17}$-RKI:NDKQQII:KKATV:VVD:RXGXS, AAPASXX:VPA:RPVQ-AA$^{17}$-RKI:DAANNVV:KKATV:VVE:AXGXR, STPPTXX:VPT:RPVQ-AA$^{17}$-RKI:DSANNVV:KKATV:VVE:SXGXR, HVPKPXX:APT:RPVQ-AA$^{17}$-RKI:DDSSNVI:KKATV:VVR:SXGXH, RVPSTXX:APV:RPVQ-AA$^{17}$-RKI:DNGRVLL:KKATV:VEE:XGXL, ASAAPXX:VPQ:RPVQ-AA$^{17}$-RKI:VGRKPKV:KKATV:VRS:XKXS, ASASPXX:VSQ:RPVQ-AA$^{17}$-RKI:IGKTPKI:KKATV:VKS:XKXS, ASASPXX:VPQ:RPVQ-AA$^{17}$-RKI:VGRTPKV:KKATV:VKS:XKXS, NDEGLEX:VPT:RPVQ-AA$^{17}$-RKI:RIKPHQGQH:KKATV:QHN:KXEXR, NDEGLEX:VPT:RPVQ-AA$^{17}$-RKI:RIKPHQGQH:KKATV:EHS:QXEXR and SSVKXQP:SRV:RPVQ-AA$^{17}$-RKI:EYVRKKPKL:KKATV:EEH:LEXAXA; and wherein AA$^{17}$ is selected from the group consisting of G, A, V, L, I, P, F, M, W, T and S (in particular is selected from the group consisting of M, I, L, V and T). More particularly, the heptuplet PEP7:PEP3:PEP12:LINKER:PEP2:PEP4:PEP8 is selected from the group consisting of GIPEPXX:VPT:SAIS-AA$^{17}$-LYL:DENKNVV:LKNYQ:VVE:SXAXR, HVTKPTX:VPT:SAIS-AA$^{17}$-LYL:DDSSNVI:LKNYQ:VVE:SXGXH, YVPKPXX:VPT:SAIS-AA$^{17}$-LYL:DDSSNVI:LKNYQ:VVE:SXGXH, TVPKPXX:VPT:SAIS-AA$^{17}$-LYL:DDSSNVI:LKNYQ:VVE:AXGXH, AVPKAXX:VPT:SAIS-AA$^{17}$-LYL:DSSNNVI:LKNYQ:VVE:AXGXH, KVGKAXX:VPT:SAIS-AA$^{17}$-LYL:DDMGVPT:LKNYQ:VVE:XGXR, KASKAXX:VPT:SAIS-AA$^{17}$-LYL:DKGVVTY:LKNYQ:VVE:EXGXR, GSAGPXX:VPT:SAIS-AA$^{17}$-LYL:NDKQQII:LKNYQ:VVE:RXGXS, AAPASXX:VPT:SAIS-AA$^{17}$-LYL:DAANNVV:LKNYQ:VVE:AXGXR, STPPTXX:VPT:SAIS-AA$^{17}$-LYL:DSANNVV:LKNYQ:VVE:SXGXR, HVPKPXX:VPT:SAIS-AA$^{17}$-LYL:DDSSNVI:LKNYQ:VVE:SXGXH, RVPSTXX:VPT:SAIS-AA$^{17}$-LYL:DNGRVLL:LKNYQ:VVE:XGXL, ASAAPXX:VPT:SAIS-AA$^{17}$-LYL:VGRKPKV:LKNYQ:VVE:XKXS, ASASPXX:VPT:SAIS-AA$^{17}$-LYL:IGKTP-

KI:LKNYQ:VVE:XKXS, ASASPXX:VPT:SAIS-AA$^{17}$-LYL:VGRTPKV:LKNYQ:VVE:XKXS, GIPEPXX:VPE:SAIS-AA$^{17}$-LYL:DENKNVV:LKNYQ:TVE:SXAXR, HVTKPTX:APT:SAIS-AA$^{17}$-LYL:DDSSNVI:LKNYQ:VVR:SXGXH, YVPKPXX:APT:SAIS-AA$^{17}$-LYL:DDSSNVI:LKNYQ:VVR:SXGXH, TVPKPXX:APT:SAIS-AA$^{17}$-LYL:DDSSNVI:LKNYQ:VVR:AXGXH, AVPKAXX:APT:SAIS-AA$^{17}$-LYL:DSSNNVI:LKNYQ:VVK:AXGXH, KVGKAXX:VPT:SAIS-AA$^{17}$-LYL:DDMGVPT:LKNYQ:VAE:XGXR, KASKAXX:VPT:SAIS-AA$^{17}$-LYL:DKGVVTY:LKNYQ:AVS:EXGXR, GSAGPXX:TPT:SAIS-AA$^{17}$-LYL:NDKQQII:LKNYQ:VVD:RXGXS, AAPASXX:VPA:SAIS-AA$^{17}$-LYL:DAANNVV:LKNYQ:VVE:AXGXR, HVPKPXX:APT:SAIS-AA$^{17}$-LYL:DDSSNVI:LKNYQ:VVR:SXGXH, RVPSTXX:APV:SAIS-AA$^{17}$-LYL:DNGRVLL:LKNYQ:VEE:XGXL, ASAAPXX:VPQ:SAIS-AA$^{17}$-LYL:VGRKPKV:LKNYQ:VRS:XKXS, ASASPXX:VSQ:SAIS-AA$^{17}$-LYL:IGKTPKI:LKNYQ:VKS:XKXS, ASASPXX:VPQ:SAIS-AA$^{17}$-LYL:VGRTPKV:LKNYQ:VKS:XKXS, NDEGLEX:VPT:SAIS-AA$^{17}$-LYL:RIKPHQGQH:LKNYQ:QHN:KXEXR, NDEGLEX:VPT :SAIS-AA$^{17}$-LYL:RIKPHQGQH:LKNYQ:EHS:QXEXR, SSVKXQP:SRV:SAIS-AA$^{17}$-LYL:EYVRKKPKL:LKNYQ:EEH:LEXAXA, RNVQXRP:TQV:SAIS-AA$^{17}$-LYL:EIVRKKPIF:LKNYQ:EDH:LAXKXE, KIPKAXX:VPE:SSLS-AA$^{17}$-LFF:DENEKVV:LKVYP:TVE:GXGXR, SIPKAXX:VPE:SSLS-AA$^{17}$-LFF:DEYDKVV:LKVYP:TVE:GXGXR, HVTKPTX:VPE:SSLS-AA$^{17}$-LFF:DDSSNVI:LKVYP:TVE:SXGXH, YVPKPXX:VPE:SSLS-AA$^{17}$-LFF:DDSSNVI:LKVYP:TVE:SXGXH, TVPKPXX:VPE:SSLS-AA$^{17}$-LFF:DDSSNVI:LKVYP:TVE:AXGXH, AVPKAXX:VPE:SSLS-AA$^{17}$-LFF:DSSNNVI:LKVYP:TVE:AXGXH, KVGKAXX:VPE:SSLS-AA$^{17}$-LFF:DDMGVPT:LKVYP:TVE:XGXR, KASKAXX:VPE:SSLS-AA$^{17}$-LFF:DKGVVTY:LKVYP:TVE:EXGXR, GSAGPXX:VPE:SSLS-AA$^{17}$-LFF:NDKQQII:LKVYP:TVE:RXGXS, AAPASXX:VPE:SSLS-AA$^{17}$-LFF:DAANNVV:LKVYP:TVE:AXGXR, STPPTXX:VPE:SSLS-AA$^{17}$-LFF:DSANNVV:LKVYP:TVE:SXGXR, HVPKPXX:VPE:SSLS-AA$^{17}$-LFF:DDSSNVI:LKVYP:TVE:SXGXH, RVPSTXX:VPE:SSLS-AA$^{17}$-LFF:DNGRVLL:LKVYP:TVE:XGXL, ASAAPXX:VPE:SSLS-AA$^{17}$-LFF:VGRKPKV:LKVYP:TVE:XKXS, ASASPXX:VPE:SSLS-AA$^{17}$-LFF:IGKTPKI:LKVYP:TVE:XKXS, ASASPXX:VPE:SSLS-AA$^{17}$-LFF:VGRTPKV:LKVYP:TVE:XKXS, KIPKAXX:VPT:SSLS-AA$^{17}$-LFF:DENEKVV:LKVYP:VVE:GXGXR, SIPKAXX:VPT:SSLS-AA$^{17}$-LFF:DEYDKVV:LKVYP:VVE:GXGXR, HVTKPTX:APT:SSLS-AA$^{17}$-LFF:DDSSNVI:LKVYP:VVR:SXGXH, YVPKPXX:APT:SSLS-AA$^{17}$-LFF:DDSSNVI:LKVYP:VVR:SXGXH, TVPKPXX:APT:SSLS-AA$^{17}$-LFF:DDSSNVI:LKVYP:VVR:AXGXH, AVPKAXX:APT:SSLS-AA$^{17}$-LFF:DSSNNVI:LKVYP:VVK:AXGXH, KVGKAXX:VPT:SSLS-AA$^{17}$-LFF:DDMGVPT:LKVYP:VAE:XGXR, KASKAXX:VPT:SSLS-AA$^{17}$-LFF:DKGVVTY:LKVYP:AVS:EXGXR, GSAGPXX:TPT:SSLS-AA$^{17}$-LFF:NDKQQII:LKVYP:VVD:RXGXS, AAPASXX:VPA:SSLS-AA$^{17}$-LFF:DAANNVV:LKVYP:VVE:AXGXR, STPPTXX:VPT:SSLS-AA$^{17}$-LFF:DSANNVV:LKVYP:VVE:SXGXR, HVPKPXX:APT:SSLS-AA$^{17}$-LFF:DDSSNVI:LKVYP:VVR:SXGXH, RVPSTXX:APV:SSLS-AA$^{17}$-LFF:DNGRVLL:LKVYP:VEE:XGXL, ASAAPXX:VPQ:SSLS-AA$^{17}$-LFF:VGRKPKV:LKVYP:VRS:XKXS, ASASPXX:VSQ:SSLS-AA$^{17}$-LFF:IGKTPKI:LKVYP:VKS:XKXS, ASASPXX:VPQ:SSLS-AA$^{17}$-LFF:VGRTPKV:LKVYP:VKS:XKXS, NDEGLEX:VPT:SSLS-AA$^{17}$-LFF:RIKPHQGQH:LKVYP:QHN:KXEXR, NDEGLEX:VPT:SSLS-AA$^{17}$-LFF:RIKPHQGQH:LKVYP:EHS:QXEXR, SSVKXQP:SRV:SSLS-AA$^{17}$-LFF:EYVRKKPKL:LKVYP:EEH:LEXAXA, RNVQXRP:TQV:SSLS-AA$^{17}$-LFF:EIVRKKPIF:LKVYP:EDH:LAXKXE, KIPKAXX:APT:NAIS-AA$^{17}$-LYF:DENEKVV:LKKYR:VVR:GXGXR, GIPEPXX:APT:NAIS-AA$^{17}$-LYF:DENKNVV:LKKYR:VVR:SXAXR, SIPKAXX:APT:NAIS-AA$^{17}$-LYF:DEYDKVV:LKKYR:VVR:GXGXR, AVPKAXX:APT:NAIS-AA$^{17}$-LYF:DSSNNVI:LKKYR:VVR:AXGXH, KVGKAXX:APT:NAIS-AA$^{17}$-LYF:DDMGVPT:LKKYR:VVR:XGXR, KASKAXX:APT:NAIS-AA$^{17}$-LYF:DKGVVTY:LKKYR:VVR:EXGXR, GSAGPXX:APT:NAIS-AA$^{17}$-LYF:NDKQQII:LKKYR:VVR:RXGXS, AAPASXX:APT:NAIS-AA$^{17}$-LYF:DAANNVV:LKKYR:VVR:AXGXR, STPPTXX:APT:NAIS-AA$^{17}$-LYF:DSANNVV:LKKYR:VVR:SXGXR, RVPSTXX:APT:NAIS-AA$^{17}$-LYF:DNGRVLL:LKKYR:VVR:XGXL, ASAAPXX:APT:NAIS-AA$^{17}$-LYF:VGRKPKV:LKKYR:VVR:XKXS, ASASPXX:APT:NAIS-AA$^{17}$-LYF:IGKTPKI:LKKYR:VVR:XKXS, ASASPXX:APT:NAIS-AA$^{17}$-LYF:VGRTPKV:LKKYR:VVR:XKXS, KIPKAXX:VPT:NAIS-AA$^{17}$-LYF:DENEKVV:LKKYR:VVE:GXGXR, GIPEPXX:VPE:NAIS-AA$^{17}$-LYF:DENKNVV:LKKYR:TVE:SXAXR, SIPKAXX:VPT:NAIS-AA$^{17}$-LYF:DEYDKVV:LKKYR:VVE:GXGXR, AVPKAXX:APT:NAIS-AA$^{17}$-LYF:DSSNNVI:LKKYR:VVK:AXGXH, KVGKAXX:VPT:NAIS-AA$^{17}$-LYF:DDMGVPT:LKKYR:VAE:XGXR, KASKAXX:VPT:NAIS-AA$^{17}$-LYF:DKGVVTY:LKKYR:AVS:EXGXR, GSAGPXX:TPT:NAIS-AA$^{17}$-LYF:NDKQQII:LKKYR:VVD:RXGXS, AAPASXX:VPA:NAIS-AA$^{17}$-LYF:DAANNVV:LKKYR:VVE:AXGXR, STPPTXX:VPT:NAIS-AA$^{17}$-LYF:DSANNVV:LKKYR:VVE:SXGXR, RVPSTXX:APV:NAIS-AA$^{17}$-LYF:DNGRVLL:LKKYR:VEE:XGXL, ASAAPXX:VPQ:NAIS-AA$^{17}$-LYF:VGRKPKV:LKKYR:VRS:XKXS, ASASPXX:VSQ:NAIS-AA$^{17}$-LYF:IGKTPKI:LKKYR:VKS:XKXS, ASASPXX:VPQ:NAIS-AA$^{17}$-LYF:VGRTPKV:LKKYR:VKS:XKXS, NDEGLEX:VPT:NAIS-AA$^{17}$-LYF:RIKPHQGQH:LKKYR:QHN:KXEXR, NDEGLEX:VPT:NAIS-AA$^{17}$-LYF:RIKPHQGQH:LKKYR:EHS:QXEXR, SSVKXQP:SRV:NAIS-AA$^{17}$-LYF:EYVRKKPKL:LKKYR:EEH:LEXAXA, RNVQXRP:TQV:NAIS-AA$^{17}$-LYF:EIVRKKPIF:LKKYR:EDH:LAXKXE, KIPKAXX:APT:SATS-AA$^{17}$-LYY:DENEKVV:LRKHR:VVK:GXGXR, GIPEPXX:APT:SATS-AA$^{17}$-LYY:DENKNVV:LRKHR:VVK:SXAXR, SIPKAXX:APT:SATS-AA$^{17}$-LYY:DEYDKVV:LRKHR:VVK:GXGXR, HVTKPTX:APT:SATS-AA$^{17}$-LYY:DDSSNVI:LRKHR:VVK:SXGXH, YVPKPXX:APT:SATS-AA$^{17}$-LYY:DDSSNVI:LRKHR:VVK:SXGXH, TVPKPXX:APT:SATS-AA$^{17}$-LYY:DDSSNVI:LRKHR:VVK:AXGXH, KVGKAXX:APT:SATS-AA$^{17}$-LYY:DDMGVPT:LRKHR:VVK:XGXR, KASKAXX:APT:SATS-AA$^{17}$-LYY:DKGVVTY:LRKHR:VVK:EXGXR, GSAGPXX:APT:SATS-AA$^{17}$-LYY:NDKQQII:LRKHR:VVK:RXGXS, AAPASXX:APT:SATS-AA$^{17}$-LYY:DAANNVV:LRKHR:VVK:AXGXR, STPPTXX:APT:SATS-

AA$^{17}$-LYY:DSANNVV:LRKHR:VVK:SXGXR, HVPKPXX:APT:SATS-AA$^{17}$-LYY:DDSSNVI:LRKHR:VVK:SXGXH, RVPSTXX:APT:SATS-AA$^{17}$-LYY:DNGRVLL:LRKHR:VVK:XGXL, ASAAPXX:APT:SATS-AA$^{17}$-LYY:VGRKPKV:LRKHR:VVK:XKXS, ASASPXX:APT:SATS-AA$^{17}$-LYY:IGKTPKI:LRKHR:VVK:XKXS, ASASPXX:APT:SATS-AA$^{17}$-LYY:VGRTPKV:LRKHR:VVK:XKXS, KIPKAXX:VPT:SATS-AA$^{17}$-LYY:DENEKVV:LRKHR:VVE:GXGXR, GIPEPXX:VPE:SATS-AA$^{17}$-LYY:DENKNVV:LRKHR:TVE:SXAXR, SIPKAXX:VPT:SATS-AA$^{17}$-LYY:DEYDKVV:LRKHR:VVE:GXGXR, HVTKPTX:APT:SATS-AA$^{17}$-LYY:DDSSNVI:LRKHR:VVR:SXGXH, YVPKPXX:APT:SATS-AA$^{17}$-LYY:DDSSNVI:LRKHR:VVR:SXGXH, TVPKPXX:APT:SATS-AA$^{17}$-LYY:DDSSNVI:LRKHR:VVR:AXGXH, KVGKAXX:VPT:SATS-AA$^{17}$-LYY:DDMGVPT:LRKHR:VAE:XGXR, KASKAXX:VPT:SATS-AA$^{17}$-LYY:DKGVVTY:LRKHR:AVS:EXGXR, GSAGPXX:TPT:SATS-AA$^{17}$-LYY:NDKQQII:LRKHR:VVD:RXGXS, AAPASXX:VPA:SATS-AA$^{17}$-LYY:DAANNVV:LRKHR:VVE:AXGXR, STPPTXX:VPT:SATS-AA$^{17}$-LYY:DSANNVV:LRKHR:VVE:SXGXR, HVPKPXX:APT:SATS-AA$^{17}$-LYY:DDSSNVI:LRKHR:VVR:SXGXH, RVPSTXX:APV:SATS-AA$^{17}$-LYY:DNGRVLL:LRKHR:VEE:XGXL, ASAAPXX:VPQ:SATS-AA$^{17}$-LYY:VGRKPKV:LRKHR:VRS:XKXS, ASASPXX:VSQ:SATS-AA$^{17}$-LYY:IGKTPKI:LRKHR:VKS:XKXS, ASASPXX:VPQ:SATS-AA$^{17}$-LYY:VGRTPKV:LRKHR:VKS:XKXS, NDEGLEX:VPT:SATS-AA$^{17}$-LYY:RIKPHQGQH:LRKHR:QHN:KXEXR, NDEGLEX:VPT:SATS-AA$^{17}$-LYY:RIKPHQGQH:LRKHR:EHS:QXEXR, SSVKXQP:SRV:SATS-AA$^{17}$-LYY:EYVRKKPKL:LRKHR:EEH:LEXAXA, RNVQXRP:TQV:SATS-AA$^{17}$-LYY:EIVRKKPIF:LRKHR:EDH:LAXKXE, KIPKAXX:VPT:SPIS-AA$^{17}$-LYK:DENEKVV:LKYHY:VAE:GXGXR, GIPEPXX:VPT:SPIS-AA$^{17}$-LYK:DENKNVV:LKYHY:VAE:SXAXR, SIPKAXX:VPT:SPIS-AA$^{17}$-LYK:DEYDKVV:LKYHY:VAE:GXGXR, HVTKPTX:VPT:SPIS-AA$^{17}$-LYK:DDSSNVI:LKYHY:VAE:SXGXH, YVPKPXX:VPT:SPIS-AA$^{17}$-LYK:DDSSNVI:LKYHY:VAE:SXGXH, TVPKPXX:VPT:SPIS-AA$^{17}$-LYK:DDSSNVI:LKYHY:VAE:AXGXH, AVPKAXX:VPT:SPIS-AA$^{17}$-LYK:DSSNNVI:LKYHY:VAE:AXGXH, KASKAXX:VPT:SPIS-AA$^{17}$-LYK:DKGVVTY:LKYHY:VAE:EXGXR, GSAGPXX:VPT:SPIS-AA$^{17}$-LYK:NDKQQII:LKYHY:VAE:RXGXS, AAPASXX:VPT:SPIS-AA$^{17}$-LYK:DAANNVV:LKYHY:VAE:AXGXR, STPPTXX:VPT:SPIS-AA$^{17}$-LYK:DSANNVV:LKYHY:VAE:SXGXR, HVPKPXX:VPT:SPIS-AA$^{17}$-LYK:DDSSNVI:LKYHY:VAE:SXGXH, RVPSTXX:VPT:SPIS-AA$^{17}$-LYK:DNGRVLL:LKYHY:VAE:XGXL, ASAAPXX:VPT:SPIS-AA$^{17}$-LYK:VGRKPKV:LKYHY:VAE:XKXS, ASASPXX:VPT:SPIS-AA$^{17}$-LYK:IGKTPKI:LKYHY:VAE:XKXS, ASASPXX:VPT:SPIS-AA$^{17}$-LYK:VGRTPKV:LKYHY:VAE:XKXS, KIPKAXX:VPT:SPIS-AA$^{17}$-LYK:DENEKVV:LKYHY:VVE:GXGXR, GIPEPXX:VPE:SPIS-AA$^{17}$-LYK:DENKNVV:LKYHY:TVE:SXAXR, SIPKAXX:VPT:SPIS-AA$^{17}$-LYK:DEYDKVV:LKYHY:VVE:GXGXR, HVTKPTX:APT:SPIS-AA$^{17}$-LYK:DDSSNVI:LKYHY:VVR:SXGXH, YVPKPXX:APT:SPIS-AA$^{17}$-LYK:DDSSNVI:LKYHY:VVR:SXGXH, TVPKPXX:APT:SPIS-AA$^{17}$-LYK:DDSSNVI:LKYHY:VVR:AXGXH, AVPKAXX:APT:SPIS-AA$^{17}$-LYK:DSSNNVI:LKYHY:VVK:AXGXH, KASKAXX:VPT:SPIS-AA$^{17}$-LYK:DKGVVTY:LKYHY:AVS:EXGXR, GSAGPXX:TPT:SPIS-AA$^{17}$-LYK:NDKQQII:LKYHY:VVD:RXGXS, AAPASXX:VPA:SPIS-AA$^{17}$-LYK:DAANNVV:LKYHY:VVE:AXGXR, STPPTXX:VPT:SPIS-AA$^{17}$-LYK:DSANNVV:LKYHY:VVE:SXGXR, HVPKPXX:APT:SPIS-AA$^{17}$-LYK:DDSSNVI:LKYHY:VVR:SXGXH, RVPSTXX:APV:SPIS-AA$^{17}$-LYK:DNGRVLL:LKYHY:VEE:XGXL, ASAAPXX:VPQ:SPIS-AA$^{17}$-LYK:VGRKPKV:LKYHY:VRS:XKXS, ASASPXX:VSQ:SPIS-AA$^{17}$-LYK:IGKTPKI:LKYHY:VKS:XKXS, ASASPXX:VPQ:SPIS-AA$^{17}$-LYK:VGRTPKV:LKYHY:VKS:XKXS, NDEGLEX:VPT:SPIS-AA$^{17}$-LYK:RIKPHQGQH:LKYHY:QHN:KXEXR, NDEGLEX:VPT:SPIS-AA$^{17}$-LYK:RIKPHQGQH:LKYHY:EHS:QXEXR, SSVKXQP:SRV:SPIS-AA$^{17}$-LYK:EYVRKKPKL:LKYHY:EEH:LEXAXA, RNVQXRP:TQV:SPIS-AA$^{17}$-LYK:EIVRKKPIF:LKYHY:EDH:LAXKXE, KIPKAXX:VPT:EPIS-AA$^{17}$-LYL:DENEKVV:KFKYE:AVS:GXGXR, GIPEPXX:VPT:EPIS-AA$^{17}$-LYL:DENKNVV:KFKYE:AVS:SXAXR, SIPKAXX:VPT:EPIS-AA$^{17}$-LYL:DEYDKVV:KFKYE:AVS:GXGXR, HVTKPTX:VPT:EPIS-AA$^{17}$-LYL:DDSSNVI:KFKYE:AVS:SXGXH, YVPKPXX:VPT:EPIS-AA$^{17}$-LYL:DDSSNVI:KFKYE:AVS:SXGXH, TVPKPXX:VPT:EPIS-AA$^{17}$-LYL:DDSSNVI:KFKYE:AVS:AXGXH, AVPKAXX:VPT:EPIS-AA$^{17}$-LYL:DSSNNVI:KFKYE:AVS:AXGXH, KVGKAXX:VPT:EPIS-AA$^{17}$-LYL:DDMGVPT:KFKYE:AVS:XGXR, GSAGPXX:VPT:EPIS-AA$^{17}$-LYL:NDKQQII:KFKYE:AVS:RXGXS, AAPASXX:VPT:EPIS-AA$^{17}$-LYL:DAANNVV:KFKYE:AVS:AXGXR, STPPTXX:VPT:EPIS-AA$^{17}$-LYL:DSANNVV:KFKYE:AVS:SXGXR, HVPKPXX:VPT:EPIS-AA$^{17}$-LYL:DDSSNVI:KFKYE:AVS:SXGXH, RVPSTXX:VPT:EPIS-AA$^{17}$-LYL:DNGRVLL:KFKYE:AVS:XGXL, ASAAPXX:VPT:EPIS-AA$^{17}$-LYL:VGRKPKV:KFKYE:AVS:XKXS, ASASPXX:VPT:EPIS-AA$^{17}$-LYL:IGKTPKI:KFKYE:AVS:XKXS, ASASPXX:VPT:EPIS-AA$^{17}$-LYL:VGRTPKV:KFKYE:AVS:XKXS, KIPKAXX:VPT:EPIS-AA$^{17}$-LYL:DENEKVV:KFKYE:VVE:GXGXR, GIPEPXX:VPE:EPIS-AA$^{17}$-LYL:DENKNVV:KFKYE:TVE:SXAXR, SIPKAXX:VPT:EPIS-AA$^{17}$-LYL:DEYDKVV:KFKYE:VVE:GXGXR, HVTKPTX:APT:EPIS-AA$^{17}$-LYL:DDSSNVI:KFKYE:VVR:SXGXH, YVPKPXX:APT:EPIS-AA$^{17}$-LYL:DDSSNVI:KFKYE:VVR:SXGXH, TVPKPXX:APT:EPIS-AA$^{17}$-LYL:DDSSNVI:KFKYE:VVR:AXGXH, AVPKAXX:APT:EPIS-AA$^{17}$-LYL:DSSNNVI:KFKYE:VVK:AXGXH, KVGKAXX:VPT:EPIS-AA$^{17}$-LYL:DDMGVPT:KFKYE:VAE:XGXR, GSAGPXX:TPT:EPIS-AA$^{17}$-LYL:NDKQQII:KFKYE:VVD:RXGXS, AAPASXX:VPA:EPIS-AA$^{17}$-LYL:DAANNVV:KFKYE:VVE:AXGXR, STPPTXX:VPT:EPIS-AA$^{17}$-LYL:DSANNVV:KFKYE:VVE:SXGXR, HVPKPXX:APT:EPIS-AA$^{17}$-LYL:DDSSNVI:KFKYE:VVR:SXGXH, RVPSTXX:APV:EPIS-AA$^{17}$-LYL:DNGRVLL:KFKYE:VEE:XGXL, ASAAPXX:VPQ:EPIS-AA$^{17}$-LYL:VGRKPKV:KFKYE:VRS:XKXS, ASASPXX:VSQ:EPIS-AA$^{17}$-LYL:IGKTPKI:KFKYE:VKS:XKXS, ASASPXX:VPQ:EPIS-AA$^{17}$-LYL:VGRTPKV:KFKYE:VKS:XKXS, NDEGLEX:VPT:EPIS-AA$^{17}$-LYL:RIKPHQGQH:KFKYE:QHN:KXEXR, NDEGLEX:VPT:EPIS-AA$^{17}$-LYL:RIKPHQGQH:KFKYE:EHS:QXEXR, SSVKX-

QP:SRV:EPIS-AA$^{17}$-LYL:EYVRKKPKL:KFKYE:EEH:LEXAXA, RNVQXRP:TQV:EPIS-AA$^{17}$-LYL:EIVRKKPIF:KFKYE:EDH:LAXKXE, KIPKAXX:TPT:SPIN-AA$^{17}$-LYF:DENEKVV:YGKIP:VVD:GXGXR, GIPEPXX:TPT:SPIN-AA$^{17}$-LYF:DENKNVV:YGKIP:VVD:SXAXR, SIPKAXX:TPT:SPIN-AA$^{17}$-LYF:DEYDKVV:YGKIP:VVD:GXGXR, HVTKPTX:TPT:SPIN-AA$^{17}$-LYF:DDSSNVI:YGKIP:VVD:SXGXH, YVPKPXX:TPT:SPIN-AA$^{17}$-LYF:DDSSNVI:YGKIP:VVD:SXGXH, TVPKPXX:TPT:SPIN-AA$^{17}$-LYF:DDSSNVI:YGKIP:VVD:AXGXH, AVPKAXX:TPT:SPIN-AA$^{17}$-LYF:DSSNNVI:YGKIP:VVD:AXGXH, KVGKAXX:TPT:SPIN-AA$^{17}$-LYF:DDMGVPT:YGKIP:VVD:XGXR, KASKAXX:TPT:SPIN-AA$^{17}$-LYF:DKGVVTY:YGKIP:VVD:EXGXR, AAPASXX:TPT:SPIN-AA$^{17}$-LYF:DAANNVV:YGKIP:VVD:AXGXR, STPPTXX:TPT:SPIN-AA$^{17}$-LYF:DSANNVV:YGKIP:VVD:SXGXR, HVPKPXX:TPT:SPIN-AA$^{17}$-LYF:DDSSNVI:YGKIP:VVD:SXGXH, RVPSTXX:TPT:SPIN-AA$^{17}$-LYF:DNGRVLL:YGKIP:VVD:XGXL, ASAAPXX:TPT:SPIN-AA$^{17}$-LYF:VGRKPKV:YGKIP:VVD:XKXS, ASASPXX:TPT:SPIN-AA$^{17}$-LYF:IGKTPKI:YGKIP:VVD:XKXS, ASASPXX:TPT:SPIN-AA$^{17}$-LYF:VGRTPKV:YGKIP:VVD:XKXS, KIPKAXX:VPT:SPIN-AA$^{17}$-LYF:DENEKVV:YGKIP:VVE:GXGXR, GIPEPXX:VPE:SPIN-AA$^{17}$-LYF:DENKNVV:YGKIP:TVE:SXAXR, SIPKAXX:VPT:SPIN-AA$^{17}$-LYF:DEYDKVV:YGKIP:VVE:GXGXR, HVTKPTX:APT:SPIN-AA$^{17}$-LYF:DDSSNVI:YGKIP:VVR:SXGXH, YVPKPXX:APT:SPIN-AA$^{17}$-LYF:DDSSNVI:YGKIP:VVR:SXGXH, TVPKPXX:APT:SPIN-AA$^{17}$-LYF:DDSSNVI:YGKIP:VVR:AXGXH, AVPKAXX:APT:SPIN-AA$^{17}$-LYF:DSSNNVI:YGKIP:VVK:AXGXH, KVGKAXX:VPT:SPIN-AA$^{17}$-LYF:DDMGVPT:YGKIP:VAE:XGXR, KASKAXX:VPT:SPIN-AA$^{17}$-LYF:DKGVVTY:YGKIP:AVS:EXGXR, AAPASXX:VPA:SPIN-AA$^{17}$-LYF:DAANNVV:YGKIP:VVE:AXGXR, STPPTXX:VPT:SPIN-AA$^{17}$-LYF:DSANNVV:YGKIP:VVE:SXGXR, HVPKPXX:APT:SPIN-AA$^{17}$-LYF:DDSSNVI:YGKIP:VVR:SXGXH, RVPSTXX:APV:SPIN-AA$^{17}$-LYF:DNGRVLL:YGKIP:VEE:XGXL, ASAAPXX:VPQ:SPIN-AA$^{17}$-LYF:VGRKPKV:YGKIP:VRS:XKXS, ASASPXX:VSQ:SPIN-AA$^{17}$-LYF:IGKTPKI:YGKIP:VKS:XKXS, ASASPXX:VPQ:SPIN-AA$^{17}$-LYF:VGRTPKV:YGKIP:VKS:XKXS, NDEGLEX:VPT:SPIN-AA$^{17}$-LYF:RIKPHQGQH:YGKIP:QHN:KXEXR, NDEGLEX:VPT:SPIN-AA$^{17}$-LYF:RIKPHQGQH:YGKIP:EHS:QXEXR, SSVKXQP:SRV:SPIN-AA$^{17}$-LYF:EYVRKKPKL:YGKIP:EEH:LEXAXA, RNVQXRP:TQV:SPIN-AA$^{17}$-LYF:EIVRKKPIF:YGKIP:EDH:LAXKXE, KIPKAXX:VPA:SPIS-AA$^{17}$-LYI:DENEKVV:YKQYE:VVE:GXGXR, GIPEPXX:VPA:SPIS-AA$^{17}$-LYI:DENKNVV:YKQYE:VVE:SXAXR, SIPKAXX:VPA:SPIS-AA$^{17}$-LYI:DEYDKVV:YKQYE:VVE:GXGXR, HVTKPTX:VPA:SPIS-AA$^{17}$-LYI:DDSSNVI:YKQYE:VVE:SXGXH, YVPKPXX:VPA:SPIS-AA$^{17}$-LYI:DDSSNVI:YKQYE:VVE:SXGXH, TVPKPXX:VPA:SPIS-AA$^{17}$-LYI:DDSSNVI:YKQYE:VVE:AXGXH, AVPKAXX:VPA:SPIS-AA$^{17}$-LYI:DSSNNVI:YKQYE:VVE:AXGXH, KVGKAXX:VPA:SPIS-AA$^{17}$-LYI:DDMGVPT:YKQYE:VVE:XGXR, KASKAXX:VPA:SPIS-AA$^{17}$-LYI:DKGVVTY:YKQYE:VVE:EXGXR, GSAGPXX:VPA:SPIS-AA$^{17}$-LYI:NDKQQII:YKQYE:VVE:RXGXS, STPPTXX:VPA:SPIS-AA$^{17}$-LYI:DSANNVV:YKQYE:VVE:SXGXR, HVPKPXX:VPA:SPIS-AA$^{17}$-LYI:DDSSNVI:YKQYE:VVE:SXGXH, RVPSTXX:VPA:SPIS-AA$^{17}$-LYI:DNGRVLL:YKQYE:VVE:XGXL, ASAAPXX:VPA:SPIS-AA$^{17}$-LYI:VGRKPKV:YKQYE:VVE:XKXS, ASASPXX:VPA:SPIS-AA$^{17}$-LYI:IGKTPKI:YKQYE:VVE:XKXS, ASASPXX:VPA:SPIS-AA$^{17}$-LYI:VGRTPKV:YKQYE:VVE:XKXS, KIPKAXX:VPT:SPIS-AA$^{17}$-LYI:DENEKVV:YKQYE:VVE:GXGXR, GIPEPXX:VPE:SPIS-AA$^{17}$-LYI:DENKNVV:YKQYE:TVE:SXAXR, SIPKAXX:VPT:SPIS-AA$^{17}$-LYI:DEYDKVV:YKQYE:VVE:GXGXR, HVTKPTX:APT:SPIS-AA$^{17}$-LYI:DDSSNVI:YKQYE:VVR:SXGXH, YVPKPXX:APT:SPIS-AA$^{17}$-LYI:DDSSNVI:YKQYE:VVR:SXGXH, TVPKPXX:APT:SPIS-AA$^{17}$-LYI:DDSSNVI:YKQYE:VVR:AXGXH, AVPKAXX:APT:SPIS-AA$^{17}$-LYI:DSSNNVI:YKQYE:VVK:AXGXH, KVGKAXX:VPT:SPIS-AA$^{17}$-LYI:DDMGVPT:YKQYE:VAE:XGXR, KASKAXX:VPT:SPIS-AA$^{17}$-LYI:DKGVVTY:YKQYE:AVS:EXGXR, GSAGPXX:TPT:SPIS-AA$^{17}$-LYI:NDKQQII:YKQYE:VVD:RXGXS, STPPTXX:VPT:SPIS-AA$^{17}$-LYI:DSANNVV:YKQYE:VVE:SXGXR, HVPKPXX:APT:SPIS-AA$^{17}$-LYI:DDSSNVI:YKQYE:VVR:SXGXH, RVPSTXX:APV:SPIS-AA$^{17}$-LYI:DNGRVLL:YKQYE:VEE:XGXL, ASAAPXX:VPQ:SPIS-AA$^{17}$-LYI:VGRKPKV:YKQYE:VRS:XKXS, ASASPXX:VSQ:SPIS-AA$^{17}$-LYI:IGKTPKI:YKQYE:VKS:XKXS, ASASPXX:VPQ:SPIS-AA$^{17}$-LYI:VGRTPKV:YKQYE:VKS:XKXS, NDEGLEX:VPT:SPIS-AA$^{17}$-LYI:RIKPHQGQH:YKQYE:QHN:KXEXR, NDEGLEX:VPT:SPIS-AA$^{17}$-LYI:RIKPHQGQH:YKQYE:EHS:QXEXR, SSVKXQP:SRV:SPIS-AA$^{17}$-LYI:EYVRKKPKL:YKQYE:EEH:LEXAXA, RNVQXRP:TQV:SPIS-AA$^{17}$-LYI:EIVRKKPIF:YKQYE:EDH:LAXKXE, KIPKAXX:VPT:SPIS-AA$^{17}$-LFI:DENEKVV:YKQYE:VVE:GXGXR, GIPEPXX:VPT:SPIS-AA$^{17}$-LFI:DENKNVV:YKQYE:VVE:SXAXR, SIPKAXX:VPT:SPIS-AA$^{17}$-LFI:DEYDKVV:YKQYE:VVE:GXGXR, HVTKPTX:VPT:SPIS-AA$^{17}$-LFI:DDSSNVI:YKQYE:VVE:SXGXH, YVPKPXX:VPT:SPIS-AA$^{17}$-LFI:DDSSNVI:YKQYE:VVE:SXGXH, TVPKPXX:VPT:SPIS-AA$^{17}$-LFI:DDSSNVI:YKQYE:VVE:AXGXH, AVPKAXX:VPT:SPIS-AA$^{17}$-LFI:DSSNNVI:YKQYE:VVE:AXGXH, KVGKAXX:VPT:SPIS-AA$^{17}$-LFI:DDMGVPT:YKQYE:VVE:XGXR, KASKAXX:VPT:SPIS-AA$^{17}$-LFI:DKGVVTY:YKQYE:VVE:EXGXR, GSAGPXX:VPT:SPIS-AA$^{17}$-LFI:NDKQQII:YKQYE:VVE:RXGXS, AAPASXX:VPT:SPIS-AA$^{17}$-LFI:DAANNVV:YKQYE:VVE:AXGXR, HVPKPXX:VPT:SPIS-AA$^{17}$-LFI:DDSSNVI:YKQYE:VVE:SXGXH, RVPSTXX:VPT:SPIS-AA$^{17}$-LFI:DNGRVLL:YKQYE:VVE:XGXL, ASAAPXX:VPT:SPIS-AA$^{17}$-LFI:VGRKPKV:YKQYE:VVE:XKXS, ASASPXX:VPT:SPIS-AA$^{17}$-LFI:IGKTPKI:YKQYE:VVE:XKXS, ASASPXX:VPT:SPIS-AA$^{17}$-LFI:VGRTPKV:YKQYE:VVE:XKXS, GIPEPXX:VPE:SPIS-AA$^{17}$-LFI:DENKNVV:YKQYE:TVE:SXAXR, HVTKPTX:APT:SPIS-AA$^{17}$-LFI:DDSSNVI:YKQYE:VVR:SXGXH, YVPKPXX:APT:SPIS-AA$^{17}$-LFI:DDSSNVI:YKQYE:VVR:SXGXH, TVPKPXX:APT:SPIS-AA$^{17}$-LFI:DDSSNVI:YKQYE:VVR:AXGXH, AVPKAXX:APT:SPIS-AA$^{17}$-LFI:DSSNNVI:YKQYE:VVK:AXGXH, KVGKAXX:VPT:SPIS-AA$^{17}$-LFI:DDMGVPT:YKQYE:VAE:XGXR,

KASKAXX:VPT:SPIS-AA$^{17}$-LFI:DKGVVTY:YKQYE:AVS:EXGXR, GSAGPXX:TPT:SPIS-AA$^{17}$-LFI:NDKQ-QII:YKQYE:VVD:RXGXS, AAPASXX:VPA:SPIS-AA$^{17}$-LFI:DAANNVV:YKQYE:VVE:AXGXR, HVPKPXX:APT:SPIS-AA$^{17}$-LFI:DDSSNVI:YKQYE:VVR:SXGXH, RVPSTXX:APV:SPIS-AA$^{17}$-LFI:DNGRVLL:YKQYE:VEE:XGXL, ASAAPXX:VPQ:SPIS-AA$^{17}$-LFI:VGRKPKV:YKQYE:VRS:XKXS, ASASPXX:VSQ:SPIS-AA$^{17}$-LFI:IGKTP-KI:YKQYE:VKS:XKXS, ASASPXX:VPQ:SPIS-AA$^{17}$-LFI:VGRTPKV:YKQYE:VKS:XKXS, NDEGLEX:VPT:SPIS-AA$^{17}$-LFI:RIKPHQGQH:YKQYE:QHN:KXEXR, NDEGLEX:VPT:SPIS-AA$^{17}$-LFI:RIKPHQGQH:YKQYE:EHS:QXEXR, SSVKXQP:SRV:SPIS-AA$^{17}$-LFI:EYVRKKPKL:YKQYE:EEH:LEXAXA, RNVQXRP:TQV:SPIS-AA$^{17}$-LFI:EIVRKKPIF:YKQYE:EDH:LAXKXE, KIPKAXX:APV:KPLS-AA$^{17}$-LYV:DENEKVV:DHHKD:VEE:GXGXR, GIPEPXX:APV:KPLS-AA$^{17}$-LYV:DENKNW:DHHKD:VEE:SXAXR, SIPKAXX:APV:KPLS-AA$^{17}$-LYV:DEYDKVV:DH-HKD:VEE:GXGXR, HVTKPTX:APV:KPLS-AA$^{17}$-LYV:DDSSNVI:DHHKD:VEE:SXGXH, YVPKPXX:APV:KPLS-AA$^{17}$-LYV:DDSSNVI:DHHKD:VEE:SXGXH, TVPKPXX:APV:KPLS-AA$^{17}$-LYV:DDSSNVI:DHHKD:VEE:AXGXH, AVP-KAXX:APV:KPLS-AA$^{17}$-LYV:DSSNNVI:DHHKD:VEE:AXGXH, KVGKAXX:APV:KPLS-AA$^{17}$-LYV:DDMGVPT:DH-HKD:VEE:XGXR, KASKAXX:APV:KPLS-AA$^{17}$-LYV:DKGVVTY:DHHKD:VEE:EXGXR, GSAGPXX:APV:KPLS-AA$^{17}$-LYV:NDKQQII:DHHKD:VEE:RXGXS, AAPASXX:APV:KPLS-AA$^{17}$-LYV:DAANNVV:DHHKD:VEE:AXGXR, STPPTXX:APV:KPLS-AA$^{17}$-LYV:DSANNVV:DHHKD:VEE:SXGXR, HVPKPXX:APV:KPLS-AA$^{17}$-LYV:DDSSNVI:DH-HKD:VEE:SXGXH, ASAAPXX:APV:KPLS-AA$^{17}$-LYV:VGRKPKV:DHHKD:VEE:XKXS, ASASPXX:APV:KPLS-AA$^{17}$-LYV:IGKTPKI:DHHKD:VEE:XKXS, ASASPXX:APV:KPLS-AA$^{17}$-LYV:VGRTPKV:DHHKD:VEE:XKXS, KIP-KAXX:VPT:KPLS-AA$^{17}$-LYV:DENEKVV:DHHKD:VVE:GXGXR, GIPEPXX:VPE:KPLS-AA$^{17}$-LYV:DENKNVV:DH-HKD:TVE:SXAXR, SIPKAXX:VPT:KPLS-AA$^{17}$-LYV:DEYDKVV:DHHKD:VVE:GXGXR, HVTKPTX:APT:KPLS-AA$^{17}$-LYV:DDSSNVI:DHHKD:VVR:SXGXH, YVPKPXX:APT:KPLS-AA$^{17}$-LYV:DDSSNVI:DHHKD:VVR:SXGXH, TVPK-PXX:APT:KPLS-AA$^{17}$-LYV:DDSSNVI:DHHKD:VVR:AXGXH, AVPKAXX:APT:KPLS-AA$^{17}$-LYV:DSSNNVI:DH-HKD:VVK:AXGXH, KVGKAXX:VPT:KPLS-AA$^{17}$-LYV:DDMGVPT:DHHKD:VAE:XGXR, KASKAXX:VPT:KPLS-AA$^{17}$-LYV:DKGVVTY:DHHKD:AVS:EXGXR, GSAGPXX:TPT:KPLS-AA$^{17}$-LYV:NDKQQII:DHHKD:VVD:RXGXS, AA-PASXX:VPA:KPLS-AA$^{17}$-LYV:DAANNVV:DHHKD:VVE:AXGXR, STPPTXX:VPT:KPLS-AA$^{17}$-LYV:DSANNVV:DH-HKD:VVE:SXGXR, HVPKPXX:APT:KPLS-AA$^{17}$-LYV:DDSSNVI:DHHKD:VVR:SXGXH, ASAAPXX:VPQ:KPLS-AA$^{17}$-LYV:VGRKPKV:DHHKD:VRS:XKXS, ASASPXX:VSQ:KPLS-AA$^{17}$-LYV:IGKTPKI:DHHKD:VKS:XKXS, ASAS-PXX:VPQ:KPLS-AA$^{17}$-LYV:VGRTPKV:DHHKD:VKS:XKXS, NDEGLEX:VPT:KPLS-AA$^{17}$-LYV:RIKPHQGQH:DH-HKD:QHN:KXEXR, NDEGLEX:VPT:KPLS-AA$^{17}$-LYV:RIKPHQGQH:DHHKD:EHS:QXEXR, SSVKXQP:SRV:KPLS-AA$^{17}$-LYV:EYVRKKPKL:DHHKD:EEH:LEXAXA, RNVQXRP:TQV:KPLS-AA$^{17}$-LYV:EIVRKKPIF:DH-HKD:EDH:LAXKXE, KIPKAXX:VPQ:EPLP-AA$^{17}$-VYY:DENEKVV:EQLSN:VRS:GXGXR, GIPEPXX:VPQ:EPLP-AA$^{17}$-VYY:DENKNVV:EQLSN:VRS:SXAXR, SIPKAXX:VPQ:EPLP-AA$^{17}$-VYY:DEYDKVV:EQLSN:VRS:GXGXR, HVTKPTX:VPQ:EPLP-AA$^{17}$-VYY:DDSSNVI:EQLSN:VRS:SXGXH, YVPKPXX:VPQ:EPLP-AA$^{17}$-VYY:DDSSNVI:EQL-SN:VRS:SXGXH, TVPKPXX:VPQ:EPLP-AA$^{17}$-VYY:DDSSNVI:EQLSN:VRS:AXGXH, AVPKAXX:VPQ:EPLP-AA$^{17}$-VYY:DSSNNVI:EQLSN:VRS:AXGXH, KVGKAXX:VPQ:EPLP-AA$^{17}$-VYY:DDMGVPT:EQLSN:VRS:XGXR, KASKAXX:VPQ:EPLP-AA$^{17}$-VYY:DKGVVTY:EQLSN:VRS:EXGXR, GSAGPXX:VPQ:EPLP-AA$^{17}$-VYY:NDKQ-QII:EQLSN:VRS:RXGXS, AAPASXX:VPQ:EPLP-AA$^{17}$-VYY:DAANNVV:EQLSN:VRS:AXGXR, STPPTXX:VPQ:EPLP-AA$^{17}$-VYY:DSANNVV:EQLSN:VRS:SXGXR, HVPKPXX:VPQ:EPLP-AA$^{17}$-VYY:DDSSNVI:EQLSN:VRS:SXGXH, RVP-STXX:VPQ:EPLP-AA$^{17}$-VYY:DNGRVLL:EQLSN:VRS:XGXL, ASASPXX:VPQ:EPLP-AA$^{17}$-VYY:IGKTPKI:EQL-SN:VRS:XKXS, ASASPXX:VPQ:EPLP-AA$^{17}$-VYY:VGRTPKV:EQLSN:VRS:XKXS, KIPKAXX:VPT:EPLP-AA$^{17}$-VYY:DENEKVV:EQLSN:VVE:GXGXR, GIPEPXX:VPE:EPLP-AA$^{17}$-VYY:DENKNVV:EQLSN:TVE:SXAXR, SIP-KAXX:VPT:EPLP-AA$^{17}$-VYY:DEYDKVV:EQLSN:VVE:GXGXR, HVTKPTX:APT:EPLP-AA$^{17}$-VYY:DDSSNVI:EQL-SN:VVR:SXGXH, YVPKPXX:APT:EPLP-AA$^{17}$-VYY:DDSSNVI:EQLSN:VVR:SXGXH, TVPKPXX:APT:EPLP-AA$^{17}$-VYY:DDSSNVI:EQLSN:VVR:AXGXH, AVPKAXX:APT:EPLP-AA$^{17}$-VYY:DSSNNVI:EQLSN:VVK:AXGXH, KVG-KAXX:VPT:EPLP-AA$^{17}$-VYY:DDMGVPT:EQLSN:VAE:XGXR, KASKAXX:VPT:EPLP-AA$^{17}$-VYY:DKGVVTY:EQL-SN:AVS:EXGXR, GSAGPXX:TPT:EPLP-AA$^{17}$-VYY:NDKQQII:EQLSN:VVD:RXGXS, AAPASXX:VPA:EPLP-AA$^{17}$-VYY:DAANNVV:EQLSN:VVE:AXGXR, STPPTXX:VPT:EPLP-AA$^{17}$-VYY:DSANNVV:EQLSN:VVE:SXGXR, HVP-KPXX:APT:EPLP-AA$^{17}$-VYY:DDSSNVI:EQLSN:VVR:SXGXH, RVPSTXX:APV:EPLP-AA$^{17}$-VYY:DNGRVLL:EQL-SN:VEE:XGXL, ASASPXX:VSQ:EPLP-AA$^{17}$-VYY:IGKTPKI:EQLSN:VKS:XKXS, ASASPXX:VPQ:EPLP-AA$^{17}$-VYY:VGRTPKV:EQLSN:VKS:XKXS, NDEGLEX:VPT:EPLP-AA$^{17}$-VYY:RIKPHQGQH:EQLSN:QHN:KXEXR, NDEGLEX:VPT:EPLP-AA$^{17}$-VYY:RIKPHQGQH:EQLSN:EHS:QXEXR, SSVKXQP:SRV:EPLP-AA$^{17}$-VYY:EYVRKKP-KL:EQLSN:EEH:LEXAXA, RNVQXRP:TQV:EPLP-AA$^{17}$-VYY:EIVRKKPIF:EQLSN:EDH:LAXKXE, KIP-KAXX:VSQ:EPLT-AA$^{17}$-LYY:DENEKVV:EQLSN:VKS:GXGXR, GIPEPXX:VSQ:EPLT-AA$^{17}$-LYY:DENKNVV:EQL-SN:VKS:SXAXR, SIPKAXX:VSQ:EPLT-AA$^{17}$-LYY:DEYDKVV:EQLSN:VKS:GXGXR, HVTKPTX:VSQ:EPLT-AA$^{17}$-LYY:DDSSNVI:EQLSN:VKS:SXGXH, YVPKPXX:VSQ:EPLT-AA$^{17}$-LYY:DDSSNVI:EQLSN:VKS:SXGXH, TVPK-PXX:VSQ:EPLT-AA$^{17}$-LYY:DDSSNVI:EQLSN:VKS:AXGXH, AVPKAXX:VSQ:EPLT-AA$^{17}$-LYY:DSSNNVI:EQL-SN:VKS:AXGXH, KVGKAXX:VSQ:EPLT-AA$^{17}$-LYY:DDMGVPT:EQLSN:VKS:XGXR, KASKAXX:VSQ:EPLT-AA$^{17}$-LYY:DKGVVTY:EQLSN:VKS:EXGXR, GSAGPXX:VSQ:EPLT-AA$^{17}$-LYY:NDKQQII:EQLSN:VKS:RXGXS, AA-PASXX:VSQ:EPLT-AA$^{17}$-LYY:DAANNVV:EQLSN:VKS:AXGXR, STPPTXX:VSQ:EPLT-AA$^{17}$-LYY:DSANNVV:EQL-

SN:VKS:SXGXR, HVPKPXX:VSQ:EPLT-AA$^{17}$-LYY:DDSSNVI:EQLSN:VKS:SXGXH, RVPSTXX:VSQ:EPLT-AA$^{17}$-LYY:DNGRVLL:EQLSN:VKS:XGXL, ASAAPXX:VSQ:EPLT-AA$^{17}$-LYY:VGRKPKV:EQLSN:VKS:XKXS, ASASPXX:VSQ:EPLT-AA$^{17}$-LYY:VGRTPKV:EQLSN:VKS:XKXS, KIPKAXX:VPT:EPLT-AA$^{17}$-LYY:DENEKVV:EQLSN:VVE:GXGXR, GIPEPXX:VPE:EPLT-AA$^{17}$-LYY:DENKNVV:EQLSN:TVE:SXAXR, SIPKAXX:VPT:EPLT-AA$^{17}$-LYY:DEYDKVV:EQLSN:VVE:GXGXR, HVTKPTX:APT:EPLT-AA$^{17}$-LYY:DDSSNVI:EQLSN:VVR:SXGXH, YVPKPXX:APT:EPLT-AA$^{17}$-LYY:DDSSNVI:EQLSN:VVR:SXGXH, TVPKPXX:APT:EPLT-AA$^{17}$-LYY:DDSSNVI:EQLSN:VVR:AXGXH, AVPKAXX:APT:EPLT-AA$^{17}$-LYY:DSSNNVI:EQLSN:VVK:AXGXH, KVGKAXX:VPT:EPLT-AA$^{17}$-LYY:DDMGVPT:EQLSN:VAE:XGXR, KASKAXX:VPT:EPLT-AA$^{17}$-LYY:DKGVVTY:EQLSN:AVS:EXGXR, GSAGPXX:TPT:EPLT-AA$^{17}$-LYY:NDKQQII:EQLSN:VVD:RXGXS, AAPASXX:VPA:EPLT-AA$^{17}$-LYY:DAANNVV:EQLSN:VVE:AXGXR, STPPTXX:VPT:EPLT-AA$^{17}$-LYY:DSANNVV:EQLSN:VVE:SXGXR, HVPKPXX:APT:EPLT-AA$^{17}$-LYY:DDSSNVI:EQLSN:VVR:SXGXH, RVPSTXX:APV:EPLT-AA$^{17}$-LYY:DNGRVLL:EQLSN:VEE:XGXL, ASAAPXX:VPQ:EPLT-AA$^{17}$-LYY:VGRKPKV:EQLSN:VRS:XKXS, NDEGLEX:VPT:EPLT-AA$^{17}$-LYY:RIKPHQGQH:EQLSN:QHN:KXEXR, NDEGLEX:VPT:EPLT-AA$^{17}$-LYY:RIKPHQGQH:EQLSN:EHS:QXEXR, SSVKXQP:SRV:EPLT-AA$^{17}$-LYY:EYVRKKPKL:EQLSN:EEH:LEXAXA, RNVQXRP:TQV:EPLT-AA$^{17}$-LYY:EIVRKKPIF:EQLSN:EDH:LAXKXE, KIPKAXX:VPQ:EPLT-AA$^{17}$-LYY:DENEKVV:EQLSN:VKS:GXGXR, GIPEPXX:VPQ:EPLT-AA$^{17}$-LYY:DENKNVV:EQLSN:VKS:SXAXR, SIPKAXX:VPQ:EPLT-AA$^{17}$-LYY:DEYDKVV:EQLSN:VKS:GXGXR, HVTKPTX:VPQ:EPLT-AA$^{17}$-LYY:DDSSNVI:EQLSN:VKS:SXGXH, YVPKPXX:VPQ:EPLT-AA$^{17}$-LYY:DDSSNVI:EQLSN:VKS:SXGXH, TVPKPXX:VPQ:EPLT-AA$^{17}$-LYY:DDSSNVI:EQLSN:VKS:AXGXH, AVPKAXX:VPQ:EPLT-AA$^{17}$-LYY:DSSNNVI:EQLSN:VKS:AXGXH, KVGKAXX:VPQ:EPLT-AA$^{17}$-LYY:DDMGVPT:EQLSN:VKS:XGXR, KASKAXX:VPQ:EPLT-AA$^{17}$-LYY:DKGVVTY:EQLSN:VKS:EXGXR, GSAGPXX:VPQ:EPLT-AA$^{17}$-LYY:NDKQQII:EQLSN:VKS:RXGXS, AAPASXX:VPQ:EPLT-AA$^{17}$-LYY:DAANNVV:EQLSN:VKS:AXGXR, STPPTXX:VPQ:EPLT-AA$^{17}$-LYY:DSANNVV:EQLSN:VKS:SXGXR, HVPKPXX:VPQ:EPLT-AA$^{17}$-LYY:DDSSNVI:EQLSN:VKS:SXGXH, RVPSTXX:VPQ:EPLT-AA$^{17}$-LYY:DNGRVLL:EQLSN:VKS:XGXL, ASAAPXX:VPQ:EPLT-AA$^{17}$-LYY:VGRKPKV:EQLSN:VKS:XKXS, ASASPXX:VPQ:EPLT-AA$^{17}$-LYY:IGKTPKI:EQLSN:VKS:XKXS, NDEGLEX:VPT:SNIT-AA$^{17}$-QIM:RIKPHQGQH:IGEMS:QHN:QXEXR, KIPKAXX:VPT:SNIT-AA$^{17}$-QIM:DENEKVV:IGEMS:VVE:GXGXR, GIPEPXX:VPE:SNIT-AA$^{17}$-QIM:DENKNVV:IGEMS:TVE:SXAXR, SIPKAXX:VPT:SNIT-AA$^{17}$-QIM:DEYDKVV:IGEMS:VVE:GXGXR, HVTKPTX:APT:SNIT-AA$^{17}$-QIM:DDSSNVI:IGEMS:VVRSXGXH, YVPKPXX:APT:SNIT-AA$^{17}$-QIM:DDSSNVI:IGEMS:VVR:SXGXH, TVPKPXX:APT:SNIT-AA$^{17}$-QIM:DDSSNVI:IGEMS:VVRAXGXH, AVPKAXX:APT:SNIT-AA$^{17}$-QIM:DSSNNVI:IGEMS:VVK:AXGXH, KVGKAXX:VPT:SNIT-AA$^{17}$-QIM:DDMGVPT:IGEMS:VAE:XGXR, KASKAXX:VPT:SNIT-AA$^{17}$-QIM:DKGVVTY:IGEMS:AVS:EXGXR, GSAGPXX:TPT:SNIT-AA$^{17}$-QIM:NDKQQII:IGEMS:VVD:RXGXS, AAPASXX:VPA:SNIT-AA$^{17}$-QIM:DAANNVV:IGEMS:VVE:AXGXR, STPPTXX:VPT:SNIT-AA$^{17}$-QIM:DSANNVV:IGEMS:VVE:SXGXR, HVPKPXX:APT:SNIT-AA$^{17}$-QIM:DDSSNVI:IGEMS:VVR:SXGXH, RVPSTXX:APV:SNIT-AA$^{17}$-QIM:DNGRVLL:IGEMS:VEE:XGXL, ASAAPXX:VPQ:SNIT-AA$^{17}$-QIM:VGRKPKV:IGEMS:VRS:XKXS, ASASPXX:VSQ:SNIT-AA$^{17}$-QIM:IGKTPKI:IGEMS:VKS:XKXS, ASASPXX:VPQ:SNIT-AA$^{17}$-QIM:VGRTPKV:IGEMS:VKS:XKXS, NDEGLEX:VPT:SNIT-AA$^{17}$-QIM:RIKPHQGQH:IGEMS:EHS:QXEXR, SSVKXQP:SRV:SNIT-AA$^{17}$-QIM:EYVRKKPKL:IGEMS:EEH:LEXAXA, RNVQXRP:TQV:SNIT-AA$^{17}$-QIM:EIVRKKPIF:IGEMS:EDH:LAXKXE, NDEGLEX:VPT:SNIT-AA$^{17}$-QIM:RIKPHQGQH:LGEMS:EHS:KXEXR, KIPKAXX:VPT:SNIT-AA$^{17}$-QIM:DENEKVV:LGEMS:VVE:GXGXR, GIPEPXX:VPE:SNIT-AA$^{17}$-QIM:DENKNVV:LGEMS:TVE:SXAXR, SIPKAXX:VPT:SNIT-AA$^{17}$-QIM:DEYDKVV:LGEMS:VVE:GXGXR, HVTKPTX:APT:SNIT-AA$^{17}$-QIM:DDSSNVI:LGEMS:VVR:SXGXH, YVPKPXX:APT:SNIT-AA$^{17}$-QIM:DDSSNVI:LGEMS:VVR:SXGXH, TVPKPXX:APT:SNIT-AA$^{17}$-QIM:DDSSNVI:LGEMS:VVR:AXGXH, AVPKAXX:APT:SNIT-AA$^{17}$-QIM:DSSNNVI:LGEMS:VVK:AXGXH, KVGKAXX:VPT:SNIT-AA$^{17}$-QIM:DDMGVPT:LGEMS:VAE:XGXR, KASKAXX:VPT:SNIT-AA$^{17}$-QIM:DKGVVTY:LGEMS:AVS:EXGXR, GSAGPXX:TPT:SNIT-AA$^{17}$-QIM:NDKQQII:LGEMS:VVD:RXGXS, AAPASXX:VPASNIT-AA$^{17}$-QIM:DAANNVV:LGEMS:VVE:AXGXR, STPPTXX:VPT:SNIT-AA$^{17}$-QIM:DSANNVV:LGEMS:VVE:SXGXR, HVPKPXX:APT:SNIT-AA$^{17}$-QIM:DDSSNVI:LGEMS:VVR:SXGXH, RVPSTXX:APV:SNIT-AA$^{17}$-QIM:DNGRVLL:LGEMS:VEE:XGXL, ASAAPXX:VPQ:SNIT-AA$^{17}$-QIM:VGRKPKV:LGEMS:VRS:XKXS, ASASPXX:VSQ:SNIT-AA$^{17}$-QIM:IGKTPKI:LGEMS:VKS:XKXS, ASASPXX:VPQ:SNIT-AA$^{17}$-QIM:VGRTPKV:LGEMS:VKS:XKXS, NDEGLEX:VPT:SNIT-AA$^{17}$-QIM:RIKPHQGQH:LGEMS:QHN:KXEXR, SSVKXQP:SRV:SNIT-AA$^{17}$-QIM:EYVRKKPKL:LGEMS:EEH:LEXAXA, RNVQXRP:TQV:SNIT-AA$^{17}$-QIM:EIVRKKPIF:LGEMS:EDH:LAXKXE, RNVQXRP:SRV:RSVK-AA$^{17}$-AKV:EIVRKKPIF:KEVQV:EEH:LAXKXE, KIPKAXX:VPT:RSVK-AA$^{17}$-AKV:DENEKVV:KEVQV:VVE:GXGXR, GIPEPXX:VPE:RSVK-AA$^{17}$-AKV:DENKNVV:KEVQV:TVE:SXAXR, SIPKAXX:VPT:RSVK-AA$^{17}$-AKV:DEYDKVV:KEVQV:VVE:GXGXR, HVTKPTX:APT:RSVK-AA$^{17}$-AKV:DDSSNVI:KEVQV:VVR:SXGXH, YVPKPXX:APT:RSVK-AA$^{17}$-AKV:DDSSNVI:KEVQV:VVR:SXGXH, TVPKPXX:APT:RSVK-AA$^{17}$-AKV:DDSSNVI:KEVQV:VVR:AXGXH, AVPKAXX:APT:RSVK-AA$^{17}$-AKV:DSSNNVI:KEVQV:VVK:AXGXH, KVGKAXX:VPT:RSVK-AA$^{17}$-AKV:DDMGVPT:KEVQV:VAE:XGXR, KASKAXX:VPT:RSVK-AA$^{17}$-AKV:DKGVVTY:KEVQV:AVS:EXGXR, GSAGPXX:TPT:RSVK-AA$^{17}$-AKV:NDKQQII:KEVQV:VVD:RXGXS, AAPASXX:VPA:RSVK-AA$^{17}$-AKV:DAANNVV:KEVQV:VVE:AXGXR, STPPTXX:VPT:RSVK-AA$^{17}$-AKV:DSANNVV:KEVQV:VVE:SXGXR, HVPKPXX:APT:RSVK-AA$^{17}$-AKV:DDSSN-

VI:KEVQV:VVR:SXGXH, RVPSTXX:APV:RSVK-AA$^{17}$-AKV:DNGRVLL:KEVQV:VEE:XGXL, ASAAPXX:VPQ:RSVK-AA$^{17}$-AKV:VGRKPKV:KEVQV:VRS:XKXS, ASASPXX:VSQ:RSVK-AA$^{17}$-AKV:IGKTPKI:KEVQV:VKS:XKXS, ASASPXX:VPQ:RSVK-AA$^{17}$-AKV:VGRTPKV:KEVQV:VKS:XKXS, NDEGLEX:VPT:RSVK-AA$^{17}$-AKV:RIKPHQGQH:KEVQV:QHN:KXEXR, NDEGLEX:VPT:RSVK-AA$^{17}$-AKV:RIKPHQGQH:KEVQV:EHS:QXEXR, RNVQXRP:TQV:RSVK-AA$^{17}$-AKV:EIVRKKPIF:KEVQV:EDH:LAXKXE, SSVKXQP:TQV:RPVQ-AA$^{17}$-RKI:EYVRKKPKL:KKATV:EDH:LEXAXA, KIPKAXX:VPT:RPVQ-AA$^{17}$-RKI:DENEKVV:KKATV:VVE:GXGXR, GIPEPXX:VPE:RPVQ-AA$^{17}$-RKI:DENKNVV:KKATV:TVE:SXAXR, SIPKAXX:VPT:RPVQ-AA$^{17}$-RKI:DEYDKVV:KKATV:VVE:GXGXR, HVTKPTX:APT:RPVQ-AA$^{17}$-RKI:DDSSNVI:KKATV:VVR:SXGXH, YVPKPXX:APT:RPVQ-AA$^{17}$-RKI:DDSSNVI:KKATV:VVR:SXGXH, TVPKPXX:APT:RPVQ-AA$^{17}$-RKI:DDSSNVI:KKATV:VVR:AXGXH, AVPKAXX:APT:RPVQ-AA$^{17}$-RKI:DSSNNVI:KKATV:VVK:AXGXH, KVGKAXX:VPT:RPVQ-AA$^{17}$-RKI:DDMGVPT:KKATV:VAE:XGXR, KASKAXX:VPT:RPVQ-AA$^{17}$-RKI:DKGVVTY:KKATV:AVS:EXGXR, GSAGPXX:TPT:RPVQ-AA$^{17}$-RKI:NDKQQII:KKATV:VVD:RXGXS, AAPASXX:VPA:RPVQ-AA$^{17}$-RKI:DAANNVV:KKATV:VVE:AXGXR, STPPTXX:VPT:RPVQ-AA$^{17}$-RKI:DSANNVV:KKATV:VVE:SXGXR, HVPKPXX:APT:RPVQ-AA$^{17}$-RKI:DDSSNVI:KKATV:VVR:SXGXH, RVPSTXX:APV:RPVQ-AA$^{17}$-RKI:DNGRVLL:KKATV:VEE:XGXL, ASAAPXX:VPQ:RPVQ-AA$^{17}$-RKI:VGRKPKV:KKATV:VRS:XKXS, ASASPXX:VSQ:RPVQ-AA$^{17}$-RKI:IGKTPKI:KKATV:VKS:XKXS, ASASPXX:VPQ:RPVQ-AA$^{17}$-RKI:VGRTPKV:KKATV:VKS:XKXS, NDEGLEX:VPT:RPVQ-AA$^{17}$-RKI:RIKPHQGQH:KKATV:QHN:KXEXR, NDEGLEX:VPT:RPVQ-AA$^{17}$-RKI:RIKPHQGQH:KKATV:EHS:QXEXR and SSVKXQP:SRV:RPVQ-AA$^{17}$-RKI:EYVRKKPKL:KKATV:EEH:LEXAXA; and wherein AA$^{17}$ is selected from the group consisting of G, A, V, L, I, P, F, M, W, T and S (in particular is selected from the group consisting of M, I, L, V and T).

In particular, in certain embodiments, the heptuplet PEP7:PEP5:PEP1:LINKER:PEP2:PEP6:PEP8 is selected from the group consisting of GIPEPXX:VPTKM:SAIS:DENKNVV:LKNYQ:NMVVE:SXAXR, SIPKAXX:VPTEL:SAIS:DEYDKVV:LKNYQ:EMVVE:GXGXR, HVTKPTX:VPTKL:SAIS:DDSSNVI:LKNYQ:NMVVE:SXGXH, YVPKPXX:VPTKL:SAIS:DDSSNVI:LKNYQ:NMVVE:SXGXH, TVPKPXX:VPTQL:SAIS:DDSSNVI:LKNYQ:NMVVE:AXGXH, AVPKAXX:VPTKL:SAIS:DSSNNVI:LKNYQ:NMVVE:AXGXH, KVGKAXX:VPTKL:SAIS:DDMGVPT:LKNYQ:EGMSVVE:XGXR, KASKAXX:VPTKL:SAIS:DKGVVTY:LKNYQ:GMVVE:EXGXR, GSAGPXX:VPTKM:SAIS:NDKQQII:LKNYQ:GMVVE:RXGXS, AAPASXX:VPTRL:SAIS:DAANNVV:LKNYQ:DMVVE:AXGXR, STPPTXX:VPTRL:SAIS:DSANNVV:LKNYQ:DMVVE:SXGXR, HVPKPXX:VPTKL:SAIS:DDSSNVI:LKNYQ:NMVVE:SXGXH, RVPSTXX:VPTKT:SAIS:DNGRVLL:LKNYQ:MIVVE:XGXL, ASAAPXX:VPTAL:SAIS:VGRKPKV:LKNYQ:MIVVE:XKXS, ASASPXX:VPTDL:SAIS:IGKTPKI:LKNYQ:MIVVE:XKXS, ASASPXX:VPTDL:SAIS:VGRTPKV:LKNYQ:MVVVE:XKXS, NDEGLXS:VPTEE:SAIS:RIKPHQGQH:LKNYQ:FLVVE:KXEXR, NDEGLXS:VPTGQ:SAIS:RIKPHQGQH:LKNYQ:LEVVE:QXEXR, SSVKXQP:VPTHH:SAIS:EYVRKKPKL:LKNYQ:RLVVE:LEXAXA, RNVQXRP:VPTQL:SAIS:EIVRKKPIF:LKNYQ:TLVVE:LAXKXE, GIPEPXX:VPEKM:SAIS:DENKNVV:LKNYQ:NMTVE:SXAXR, HVTKPTX:APTKL:SAIS:DDSSNVI:LKNYQ:NMVVR:SXGXH, YVPKPXX:APTKL:SAIS:DDSSNVI:LKNYQ:NMVVR:SXGXH, TVPKPXX:APTQL:SAIS:DDSSNVI:LKNYQ:NMVVR:AXGXH, AVPKAXX:APTKL:SAIS:DSSNNVI:LKNYQ:NMVVK:AXGXH, KVGKAXX:VPTKL:SAIS:DDMGVPT:LKNYQ:EGMSVAE:XGXR, KASKAXX:VPTKL:SAIS:DKGVVTY:LKNYQ:GMAVS:EXGXR, GSAGPXX:TPTKM:SAIS:NDKQQII:LKNYQ:GMVVD:RXGXS, AAPASXX:VPARL:SAIS:DAANNVV:LKNYQ:DMVVE:AXGXR, HVPKPXX:APTKL:SAIS:DDSSNVI:LKNYQ:NMVVR:SXGXH, RVPSTXX:APVKT:SAIS:DNGRVLL:LKNYQ:MIVEE:XGXL, ASAAPXX:VPQAL:SAIS:VGRKPKV:LKNYQ:MIVRS:XKXS, ASASPXX:VSQDL:SAIS:IGKTPKI:LKNYQ:MIVKS:XKXS, ASASPXX:VPQDL:SAIS:VGRTPKV:LKNYQ:MVVKS:XKXS, NDEGLXS:VPTEE:SAIS:RIKPHQGQH:LKNYQ:FLQHN:KXEXR, NDEGLXS:VPTGQ:SAIS:RIKPHQGQH:LKNYQ:LEEHS:QXEXR, SSVKXQP:SRVHH:SAIS:EYVRKKPKL:LKNYQ:RLEEH :LEXAXA, RNVQXRP:TQVQL:SAIS:EIVRKKPIF:LKNYQ:TLEDH:LAXKXE, KIPKAXX:VPEEL:SSLS:DENEKVV:LKVYP:DMTVE:GXGXR, SIPKAXX:VPEEL:SSLS:DEYDKVV:LKVYP:EMTVE:GXGXR, HVTKPTX:VPEKL:SSLS:DDSSNVI:LKVYP:NMTVE:SXGXH, YVPKPXX:VPEKL:SSLS:DDSSNVI:LKVYP:NMTVE:SXGXH, TVPKPXX:VPEQL:SSLS:DDSSNVI:LKVYP:NMTVE:AXGXH, AVPKAXX:VPEKL:SSLS:DSSNNVI:LKVYP:NMTVE:AXGXH, KVGKAXX:VPEKL:SSLS:DDMGVPT:LKVYP:EGMSTVE:XGXR, KASKAXX:VPEKL:SSLS:DKGVVTY:LKVYP:GMTVE:EXGXR, GSAGPXX:VPEKM:SSLS:NDKQQII:LKVYP:GMTVE:RXGXS, AAPASXX:VPERL:SSLS:DAANNVV:LKVYP:DMTVE:AXGXR, STPPTXX:VPERL:SSLS:DSANNVV:LKVYP:DMTVE:SXGXR, HVPKPXX:VPEKL:SSLS:DDSSNVI:LKVYP:NMTVE:SXGXH, RVPSTXX:VPEKT:SSLS:DNGRVLL:LKVYP:MITVE:XGXL, ASAAPXX:VPEAL:SSLS:VGRKPKV:LKVYP:MITVE:XKXS, ASASPXX:VPEDL:SSLS:IGKTPKI:LKVYP:MITVE:XKXS, ASASPXX:VPEDL:SSLS:VGRTPKV:LKVYP:MVTVE:XKXS, NDEGLXS:VPEEE:SSLS:RIKPHQGQH:LKVYP:FLTVE:KXEXR, NDEGLXS:VPEGQ:SSLS:RIKPHQGQH:LKVYP:LETVE:QXEXR, SSVKXQP:VPEHH:SSLS:EYVRKKPKL:LKVYP:RLTVE:LEXAXA, RNVQXRP:VPEQL:SSLS:EIVRKKPIF:LKVYP:TLTVE:LAXKXE, KIPKAXX:VPTEL:SSLS:DENEKVV:LKVYP:DMVVE:GXGXR, SIPKAXX:VPTEL:SSLS:DEYDKVV:LKVYP:EMVVE:GXGXR, HVTKPTX:APTKL:SSLS:DDSSNVI:LKVYP:NMVVR:SXGXH, YVPKPXX:APTKL:SSLS:DDSSNVI:LKVYP:NMVVR:SXGXH, TVPKPXX:APTQL:SSLS:DDSSNVI:LKVYP:NMVVR:AXGXH, AVPKAXX:APTKL:SSLS:DSSNNVI:LKVYP:NMVVK:AXGXH, KVGKAXX:VPTKL:SSLS:DDMGVPT:LKVYP:EGMSVAE:XGXR,

KASKAXX:VPTKL:SSLS:DKGVVTY:LKVYP:GMAVS:EXGXR, GSAGPXX:TPTKM:SSLS:NDKQQII:LKVYP:GMVVD:RXGXS, AAPASXX:VPARL:SSLS:DAANNVV:LKVYP:DMVVE:AXGXR, STPPTXX:VPTRL:SSLS:DSANNVV:LKVYP:DMVVE:SXGXR, HVPKPXX:APTKL:SSLS:DDSSNVI:LKVYP:NMVVR:SXGXH, RVPSTXX:APVKT:SSLS:DNGRVLL:LKVYP:MIVEE:XGXL, ASAAPXX:VPQAL:SSLS:VGRKPKV:LKVYP:MIVRS:XKXS, ASASPXX:VSQDL:SSLS:IGKTPKI:LKVYP:MIVKS:XKXS, ASASPXX:VPQDL:SSLS:VGRTPKV:LKVYP:MVVKS:XKXS, NDEGLXS:VPTEE:SSLS:RIKPHQGQH:LKVYP:FLQHN:KXEXR, NDEGLXS:VPTGQ:SSLS:RIKPHQGQH:LKVYP:LEEHS:QXEXR, SSVKXQP:SRVHH:SSLS:EYVRKKPKL:LKVYP:RLEEH:LEXAXA, RNVQXRP:TQVQL:SSLS:EIVRKKPIF:LKVYP:TLEDH:LAXKXE, KIPKAXX:VPTEL:SAIS:DENEKVV:LKNYQ:DMVVE:GXGXR, KIPKAXX:APTEL:NAIS:DENEKVV:LKKYR:DMVVR:GXGXR, GIPEPXX:APTKM:NAIS:DENKNVV:LKKYR:NMVVR:SXAXR, SIPKAXX:APTEL:NAIS:DEYDKVV:LKKYR:EMVVR:GXGXR, YVPKPXX:APTKL:NAIS:DDSSNVI:LKKYR:NMVVR:SXGXH, TVPKPXX:APTQL:NAIS:DDSSNVI:LKKYR:NMVVR:AXGXH, AVPKAXX:APTKL:NAIS:DSSNNVI:LKKYR:NMVVR:AXGXH, KVGKAXX:APTKL:NAIS:DDMGVPT:LKKYR:EGMSVVR:XGXR, KASKAXX:APTKL:NAIS:DKGVVTY:LKKYR:GMVVR:EXGXR, GSAGPXX:APTKM:NAIS:NDKQQII:LKKYR:GMVVR:RXGXS, AAPASXX:APTRL:NAIS:DAANNVV:LKKYR:DMVVR:AXGXR, STPPTXX:APTRL:NAIS:DSANNVV:LKKYR:DMVVR:SXGXR, HVPKPXX:APTKL:NAIS:DDSSNVI:LKKYR:NMVVR:SXGXH, RVPSTXX:APTKT:NAIS:DNGRVLL:LKKYR:MIVVR:XGXL, ASAAPXX:APTAL:NAIS:VGRKPKV:LKKYR:MIVVR:XKXS, ASASPXX:APTDL:NAIS:IGKTPKI:LKKYR:MIVVR:XKXS, ASASPXX:APTDL:NAIS:VGRTPKV:LKKYR:MVVVR:XKXS, NDEGLXS:APTEE:NAIS:RIKPHQGQH:LKKYR:FLVVR:KXEXR, NDEGLXS:APTGQ:NAIS:RIKPHQGQH:LKKYR:LEVVR:QXEXR, SSVKXQP:APTHH:NAIS:EYVRKKPKL:LKKYR:RLVVR:LEXAXA, RNVQXRP:APTQL:NAIS:EIVRKKPIF:LKKYR:TLVVR:LAXKXE, KIPKAXX:VPTEL:NAIS:DENEKVV:LKKYR:DMVVE:GXGXR, GIPEPXX:VPEKM:NAIS:DENKNVV:LKKYR:NMTVE:SXAXR, SIPKAXX:VPTEL:NAIS:DEYDKVV:LKKYR:EMVVE:GXGXR, AVPKAXX:APTKL:NAIS:DSSNNVI:LKKYR:NMVVK:AXGXH, KVGKAXX:VPTKL:NAIS:DDMGVPT:LKKYR:EGMSVAE:XGXR, KASKAXX:VPTKL:NAIS:DKGVVTY:LKKYR:GMAVS:EXGXR, GSAGPXX:TPTKM:NAIS:NDKQQII:LKKYR:GMVVD:RXGXS, AAPASXX:VPARL:NAIS:DAANNVV:LKKYR:DMVVE:AXGXR, STPPTXX:VPTRL:NAIS:DSANNVV:LKKYR:DMVVE:SXGXR, RVPSTXX:APVKT:NAIS:DNGRVLL:LKKYR:MIVEE:XGXL, ASAAPXX:VPQAL:NAIS:VGRKPKV:LKKYR:MIVRS:XKXS, ASASPXX:VSQDL:NAIS:IGKTPKI:LKKYR:MIVKS:XKXS, ASASPXX:VPQDL:NAIS:VGRTPKV:LKKYR:MVVKS:XKXS, NDEGLXS:VPTEE:NAIS:RIKPHQGQH:LKKYR:FLQHN:KXEXR, NDEGLXS:VPTGQ:NAIS:RIKPHQGQH:LKKYR:LEEHS:QXEXR, SSVKXQP:SRVHH:NAIS:EYVRKKPKL:LKKYR:RLEEH:LEXAXA, RNVQXRP:TQVQL:NAIS:EIVRKKPIF:LKKYR:TLEDH:LAXKXE, HVTKPTX:APTKL:NAIS:DDSSNVI:LKKYR:NMVVR:SXGXH, KIPKAXX:APTEL:SATS:DENEKVV:LRKHR:DMVVK:GXGXR, GIPEPXX:APTKM:SATS:DENKNVV:LRKHR:NMVVK:SXAXR, SIPKAXX:APTEL:SATS:DEYDKVV:LRKHR:EMVVK:GXGXR, HVTKPTX:APTKL:SATS:DDSSNVI:LRKHR:NMVVK:SXGXH, YVPKPXX:APTKL:SATS:DDSSNVI:LRKHR:NMVVK:SXGXH, TVPKPXX:APTQL:SATS:DDSSNVI:LRKHR:NMVVK:AXGXH, KVGKAXX:APTKL:SATS:DDMGVPT:LRKHR:EGMSVVK:XGXR, KASKAXX:APTKL:SATS:DKGVVTY:LRKHR:GMVVK:EXGXR, GSAGPXX:APTKM:SATS:NDKQQII:LRKHR:GMVVK:RXGXS, AAPASXX:APTRL:SATS:DAANNVV:LRKHR:DMVVK:AXGXR, STPPTXX:APTRL:SATS:DSANNVV:LRKHR:DMVVK:SXGXR, HVPKPXX:APTKL:SATS:DDSSNVI:LRKHR:NMVVK:SXGXH, RVPSTXX:APTKT:SATS:DNGRVLL:LRKHR:MIVVK:XGXL, ASAAPXX:APTAL:SATS:VGRKPKV:LRKHR:MIVVK:XKXS, ASASPXX:APTDL:SATS:IGKTPKI:LRKHR:MIVVK:XKXS, ASASPXX:APTDL:SATS:VGRTPKV:LRKHR:MVVVK:XKXS, NDEGLXS:APTEE:SATS:RIKPHQGQH:LRKHR:FLVVK:KXEXR, NDEGLXS:APTGQ:SATS:RIKPHQGQH:LRKHR:LEVVK:QXEXR, SSVKXQP:APTHH:SATS:EYVRKKPKL:LRKHR:RLVVK:LEXAXA, RNVQXRP:APTQL:SATS:EIVRKKPIF:LRKHR:TLVVK:LAXKXE, KIPKAXX:VPTEL:SATS:DENEKVV:LRKHR:DMVVE:GXGXR, GIPEPXX:VPEKM:SATS:DENKNVV:LRKHR:NMTVE:SXAXR, SIPKAXX:VPTEL:SATS:DEYDKVV:LRKHR:EMVVE:GXGXR, HVTKPTX:APTKL:SATS:DDSSNVI:LRKHR:NMVVR:SXGXH, YVPKPXX:APTKL:SATS:DDSSNVI:LRKHR:NMVVR:SXGXH, TVPKPXX:APTQL:SATS:DDSSNVI:LRKHR:NMVVR:AXGXH, KVGKAXX:VPTKL:SATS:DDMGVPT:LRKHR:EGMSVAE:XGXR, KASKAXX:VPTKL:SATS:DKGVVTY:LRKHR:GMAVS:EXGXR, GSAGPXX:TPTKM:SATS:NDKQQII:LRKHR:GMVVD:RXGXS, AAPASXX:VPARL:SATS:DAANNVV:LRKHR:DMVVE:AXGXR, STPPTXX:VPTRL:SATS:DSANNVV:LRKHR:DMVVE:SXGXR, HVPKPXX:APTKL:SATS:DDSSNVI:LRKHR:NMVVR:SXGXH, RVPSTXX:APVKT:SATS:DNGRVLL:LRKHR:MIVEE:XGXL, ASAAPXX:VPQAL:SATS:VGRKPKV:LRKHR:MIVRS:XKXS, ASASPXX:VSQDL:SATS:IGKTPKI:LRKHR:MIVKS:XKXS, ASASPXX:VPQDL:SATS:VGRTPKV:LRKHR:MVVKS:XKXS, NDEGLXS:VPTEE:SATS:RIKPHQGQH:LRKHR:FLQHN:KXEXR, NDEGLXS:VPTGQ:SATS:RIKPHQGQH:LRKHR:LEEHS:QXEXR, SSVKXQP:SRVHH:SATS:EYVRKKPKL:LRKHR:RLEEH:LEXAXA, RNVQXRP:TQVQL:SATS:EIVRKKPIF:LRKHR:TLEDH:LAXKXE, KIPKAXX:VPTEL:SPIS:DENEKVV:LKYHY:DMVAE:GXGXR, GIPEPXX:VPTKM:SPIS:DENKNVV:LKYHY:NMVAE:SXAXR, SIPKAXX:VPTEL:SPIS:DEYDKVV:LKYHY:EMVAE:GXGXR, HVTKPTX:VPTKL:SPIS:DDSSNVI:LKYHY:NMVAE:SXGXH, YVPKPXX:VPTKL:SPIS:DDSSNVI:LKYHY:NMVAE:SXGXH, TVPKPXX:VPTQL:SPIS:DDSSNVI:LKYHY:NMVAE:AXGXH, AVPKAXX:VPTKL:SPIS:DSSNNVI:LKYHY:NMVAE:AXGXH, KASKAXX:VPTKL:SPIS:DKGVVTY:LKYHY:GMVAE:EXGXR, GSAGPXX:VPTKM:SPIS:NDKQQII:LKYHY:GMVAE:RXGXS, AAPASXX:VPTRL:SPIS:DAANNVV:LKYHY:DMVAE:AXGXR, STPPTXX:VPTRL:SPIS:DSANNVV:LKYHY:DMVAE:SXGXR, HVPKPXX:VPTKL:SPIS:DDSSNVI:LKYHY:NMVAE:SXGXH, RVPSTXX:VPTKT:SPIS:DNGRVLL:LKYHY:MIVAE:XGXL, ASAAPXX:VPTAL:SPIS:VGRKPKV:LKYHY:M IVAE:XKXS, ASASPXX:VPTDL:SPIS:IGKTPKI:LKYHY:MIVAE:XKXS, ASASPXX:VPTDL:SPIS:VGRTPKV:LKYHY:MVVAE:XKXS, NDEGLXS:VPTEE:SPIS:RIKPHQGQH:LKYHY:FLVAE:KXEXR, NDEGLXS:VPTGQ:SPIS:RIKPHQGQH:LKYHY:LEVAE:QXEXR, SSVKXQP:VPTHH:SPIS:EYVRKKPKL:LKYHY:RLVAE:LEXAXA, RNVQXRP:VPTQL:SPIS:EIVRKKPIF:LKYHY:TLVAE:LAXKXE, KIPKAXX:VPTEL:SPIS:DENEKVV:LKYHY:DMVVE:GXGXR, GIPEPXX:VPEKM:SPIS:DENKNVV:LKYHY:NMTVE:SXAXR, SIPKAXX:VPTEL:SPIS:DEYDKVV:LKYHY:EMVVE:GXGXR, HVTKPTX:APTKL:SPIS:DDSSNVI:LKYHY:NMVVR:SXGXH, YVPKPXX:APTKL:SPIS:DDSSNVI:LKYHY:NMVVR:SXGXH, TVPKPXX:APTQL:SPIS:DDSSNVI:LKYHY:NMVVR:AXGXH, AVPKAXX:APTKL:SPIS:DSSNNVI:LKYHY:NMVVK:AXGXH, KASKAXX:VPTKL:SPIS:DKGVVTY:LKYHY:GMAVS:EXGXR, GSAGPXX:TPTKM:SPIS:NDKQQII:LKYHY:GMVVD:RXGXS, AAPASXX:VPARL:SPIS:DAANNVV:LKYHY:DMVVE:AXGXR, STPPTXX:VPTRL:SPIS:DSANNVV:LKYHY:DMVVE:SXGXR, HVPKPXX:APTKL:SPIS:DDSSNVI:LKYHY:NMVVR:SXGXH, RVPSTXX:APVKT:SPIS:DNGRVLL:LKYHY:MIVEE:XGXL, ASAAPXX:VPQAL:SPIS:VGRKPKV:LKYHY:MIVRS:XKXS, ASASPXX:VSQDL:SPIS:IGKTPKI:LKYHY:MIVKS :XKXS, ASASPXX:VPQDL:SPIS:VGRTPKV:LKYHY:MVVKS:XKXS, NDEGLXS:VPTEE:SPIS:RIKPHQGQH:LKYHY:FLQHN:KXEXR, NDEGLXS:VPTGQ:SPIS:RIKPHQGQH:LKYHY:LEEHS:QXEXR, SSVKXQP:SRVHH:SPIS:EYVRKKPKL:LKYHY:RLEEH:LEXAXA, RNVQXRP:TQVQL:SPIS:EIVRKKPIF:LKYHY:TLEDH:LAXKXE, KIPKAXX:VPTEL:EPIS:DENEKVV:KFKYE:DMAVS:GXGXR, GIPEPXX:VPTKM:EPIS:DENKNVV:KFKYE:NMAVS:SXAXR, SIPKAXX:VPTEL:EPIS:DEYDKVV:KFKYE:EMAVS:GXGXR, HVTKPTX:VPTKL:EPIS:DDSSNVI:KFKYE:NMAVS:SXGXH, YVPKPXX:VPTKL:EPIS:DDSSNVI:KFKYE:NMAVS:SXGXH, TVPKPXX:VPTQL:EPIS:DDSSNVI:KFKYE:NMAVS:AXGXH, AVPKAXX:VPTKL:EPIS:DSSNNVI:KFKYE:NMAVS:AXGXH, KVGKAXX:VPTKL:EPIS:DDMGVPT:KFKYE:EGMSAVS:XGXR, GSAGPXX:VPTKM:EPIS:NDKQQII:KFKYE:GMAVS:RXGXS, AAPASXX:VPTRL:EPIS:DAANNVV:KFKYE:DMAVS:AXGXR, STPPTXX:VPTRL:EPIS:DSANNVV:KFKYE:DMAVS:SXGXR, HVPKPXX:VPTKL:EPIS:DDSSNVI:KFKYE:NMAVS:SXGXH, RVPSTXX:VPTKT:EPIS:DNGRVLL:KFKYE:MIAVS:XGXL, ASAAPXX:VPTAL:EPIS:VGRKPKV:KFKYE:MIAVS:XKXS, ASASPXX:VPTDL:EPIS:IGKTPKI:KFKYE:MIAVS:XKXS, ASASPXX:VPTDL:EPIS:VGRTPKV:KFKYE:MVAVS:XKXS, NDEGLXS:VPTEE:EPIS:RIKPHQGQH:KFKYE:FLAVS:KXEXR, NDEGLXS:VPTGQ:EPIS:RIKPHQGQH:KFKYE:LEAVS:QXEXR, SSVKXQP:VPTHH:EPIS:EYVRKKPKL:KFKYE:RLAVS:LEXAXA, RNVQXRP:VPTQL:EPIS:EIVRKKPIF:KFKYE:TLAVS:LAXKXE, KIPKAXX:VPTEL:EPIS:DENEKVV:KFKYE:DMVVE:GXGXR, GIPEPXX:VPEKM:EPIS:DENKNVV:KFKYE:NMTVE:SXAXR, SIPKAXX:VPTEL:EPIS:DEYDKVV:KFKYE:EMVVE:GXGXR, HVTKPTX:APTKL:EPIS:DDSSNVI:KFKYE:NMVVR:SXGXH, YVPKPXX:APTKL:EPIS:DDSSNVI:KFKYE:NMVVR:SXGXH, TVPKPXX:APTQL:EPIS:DDSSNVI:KFKYE:NMVVR:AXGXH, AVPKAXX:APTKL:EPIS:DSSNNVI:KFKYE:NMVVK:AXGXH, KVGKAXX:VPTKL:EPIS:DDMGVPT:KFKYE:EGMSVAE :XGXR, GSAGPXX:TPTKM:EPIS:NDKQQII:KFKYE:GMVVD:RXGXS, AAPASXX:VPARL:EPIS:DAANNVV:KFKYE:DMVVE:AXGXR, STPPTXX:VPTRL:EPIS:DSANNVV:KFKYE:DMVVE:SXGXR, HVPKPXX:APTKL:EPIS:DDSSNVI:KFKYE:NMVVR:SXGXH, RVPSTXX:APVKT:EPIS:DNGRVLL:KFKYE:MIVEE:XGXL, ASAAPXX:VPQAL:EPIS:VGRKPKV:KFKYE:MIVRS:XKXS, ASASPXX:VSQDL:EPIS:IGKTPKI:KFKYE:MIVKS:XKXS, ASASPXX:VPQDL:EPIS:VGRTPKV:KFKYE:MVVKS:XKXS, NDEGLXS:VPTEE:EPIS:RIKPHQGQH:KFKYE:FLQHN:KXEXR, NDEGLXS:VPTGQ:EPIS:RIKPHQGQH:KFKYE:LEEHS:QXEXR, SSVKXQP:SRVHH:EPIS:EYVRKKPKL:KFKYE:RLEEH:LEXAXA, RNVQXRP:TQVQL:EPIS:EIVRKKPIF:KFKYE:TLEDH:LAXKXE, KIPKAXX:TPTEL:SPIN:DENEKVV:YGKIP:DMVVD:GXGXR, GIPEPXX:TPTKM:SPIN:DENKNVV:YGKIP:NMVVD:SXAXR, SIPKAXX:TPTEL:SPIN:DEYDKVV:YGKIP:EMVVD:GXGXR, HVTKPTX:TPTKL:SPIN:DDSSNVI:YGKIP:NMVVD:SXGXH, YVPKPXX:TPTKL:SPIN:DDSSNVI:YGKIP:NMVVD:SXGXH, TVPKPXX:TPTQL:SPIN:DDSSNVI:YGKIP:NMVVD:AXGXH, AVPKAXX:TPTKL:SPIN:DSSNNVI:YGKIP:NMVVD:AXGXH, KVGKAXX:TPTKL:SPIN:DDMGVPT:YGKIP:EGMSVVD:XGXR, KASKAXX:TPTKL:SPIN:DKGVVTY:YGKIP:GMVVD:EXGXR, AAPASXX:TPTRL:SPIN:DAANNVV:YGKIP:DMVVD:AXGXR, STPPTXX:TPTRL:SPIN:DSANNVV:YGKIP:DMVVD:SXGXR, HVPKPXX:TPTKL:SPIN:DDSSNVI:YGKIP:NMVVD:SXGXH, RVPSTXX:TPTKT:SPIN:DNGRVLL:YGKIP:MIVVD:XGXL, ASAAPXX:TPTAL:SPIN:VGRKPKV:YGKIP:MIVVD:XKXS, ASASPXX:TPTDL:SPIN:IGKTPKI:YGKIP:MIVVD:XKXS, ASASPXX:TPTDL:SPIN:VGRTPKV:YGKIP:MVVVD:XKXS, NDEGLXS:TPTEE:SPIN:RIKPHQGQH:YGKIP:FLVVD:KXEXR, NDEGLXS:TPTGQ:SPIN:RIKPHQGQH:YGKIP:LEVVD:QXEXR, SSVKXQP:TPTHH:SPIN:EYVRKKPKL:YGKIP:RLVVD:LEXAXA, RNVQXRP:TPTQL:SPIN:EIVRKKPIF:YGKIP:TLVVD:LAXKXE, KIPKAXX:VPTEL:SPIN:DENEKVV:YGKIP:DMVVE:GXGXR, GIPEPXX:VPEKM:SPIN:DENKNVV:YGKIP:NMTVE:SXAXR, SIPKAXX:VPTEL:SPIN:DEYDKVV:YGKIP:EMVVE:GXGXR, HVTKPTX:APTKL:SPIN:DDSSNVI:YGKIP:NMVVR:SXGXH, YVPKPXX:APTKL:SPIN:DDSSNVI:YGKIP:NMVVR:SXGXH, TVPKPXX:APTQL:SPIN:DDSSNVI:YGKIP:NMVVR:AXGXH, AVPKAXX:APTKL:SPIN:DSSNNVI:YGKIP:NMVVK:AXGXH, KVGKAXX:VPTKL:SPIN:DDMGVPT:YGKIP:EGMSVAE:XGXR, KASKAXX:VPTKL:SPIN:DKGVVTY:YGKIP:GMAVS:EXGXR, AAPASXX:VPARL:SPIN:DAANNVV:YGKIP:DMVVE:AXGXR, STPPTXX:VPTRL:SPIN:DSANNVV:YGKIP:DMVVE:SXGXR, HVPKPXX:APTKL:SPIN:DDSSNVI:YGKIP:NMVVR:SXGXH, RVPSTXX:APVKT :SPIN:DNGRVLL:YGKIP:MIVEE:XGXL, ASAAPXX:VPQAL:SPIN:VGRKPKV:YGKIP:MIVRS:XKXS, ASASPXX:VSQDL:SPIN:IGKTPKI:YGKIP:MIVKS:XKXS, ASASPXX:VPQDL:SPIN:VGRTPKV:YGKIP:MVVKS:XKXS, NDEGLXS:VPTEE:SPIN:RIKPHQGQH:YGKIP:FLQHN:KXEXR, NDEGLXS:VPTGQ:SPIN:RIKPHQGQH:YGKIP:LEEHS:QXEXR, SSVKXQP:SRVHH:SPIN:EYVRKKPKL:YGKIP:RLEEH:LEXAXA, RNVQXRP:TQVQL:SPIN:EIVRKKPIF:YGKIP:TLEDH:LAXKXE, KIPKAXX:VPAEL:SPIS:DENEKVV:YKQYE:DMVVE:GXGXR, GIPEPXX:VPAKM:SPIS:DENKNVV:YKQYE:NMVVE:SXAXR, SIPKAXX:VPAEL:SPIS:DEYDKVV:YKQYE:EMVVE:GXGXR, HVTKPTX:VPAKL:SPIS:DDSSNVI:YKQYE:NMVVE:SXGXH, YVPKPXX:VPAKL:SPIS:DDSSNVI:YKQYE:NMVVE:SXGXH, TVPKPXX:VPAQL:SPIS:DDSSNVI:YKQYE:NMVVE:AXGXH, AVPKAXX:VPAKL:SPIS:DSSNNVI:YKQYE:NMVVE:AXGXH, KVGKAXX:VPAKL:SPIS:DDMGVPT:YKQYE:EGMSVVE :XGXR, KASKAXX:VPAKL:SPIS:DKGVVTY:YKQYE:GMVVE:EXGXR, GSAGPXX:VPAKM:SPIS:NDKQQII:YKQYE:GMVVE:RXGXS, STPPTXX:VPARL:SPIS:DSANNVV:YKQYE:DMVVE:SXGXR, HVPKPXX:VPAKL:SPIS:DDSSNVI:YKQYE:NMVVE:SXGXH, RVPSTXX:VPAKT:SPIS:DNGRVLL:YKQYE:MIVVE:XGXL, ASAAPXX:VPAAL:SPIS:VGRKPKV:YKQYE:MIVVE:XKXS, ASASPXX:VPADL:SPIS:IGKTPKI:YKQYE:MIVVE:XKXS, ASASPXX:VPADL:SPIS:VGRTPKV:YKQYE:MVVVE:XKXS, NDEGLXS:VPAEE:SPIS:RIKPHQGQH:YKQYE:FLVVE:KXEXR, NDEGLXS:VPAGQ:SPIS:RIKPHQGQH:YKQYE:LEVVE:QXEXR, SSVKXQP:VPAHH:SPIS:EYVRKKPKL:YKQYE:RLVVE:LEXAXA, RNVQXRP:VPAQL:SPIS:EIVRKKPIF:YKQYE:TLVVE:LAXKXE, KIPKAXX:VPTEL:SPIS:DENEKVV:YKQYE:DMVVE:GXGXR, GIPEPXX:VPEKM:SPIS:DENKNVV:YKQYE:NMTVE:SXAXR, SIPKAXX:VPTEL:SPIS:DEYDKVV:YKQYE:EMVVE:GXGXR, HVTKPTX:APTKL:SPIS:DDSSNVI:YKQYE:NMVVR:SXGXH, YVPKPXX:APTKL:SPIS:DDSSNVI:YKQYE:NMVVR:SXGXH, TVPKPXX:APTQL:SPIS:DDSSNVI:YKQYE:NMVVR:AXGXH, AVPKAXX:APTKL:SPIS:DSSNNVI:YKQYE:NMVVK:AXGXH, KVGKAXX:VPTKL:SPIS:DDMGVPT:YKQYE:EGMSVAE :XGXR, KASKAXX:VPTKL:SPIS:DKGVVTY:YKQYE:GMAVS:EXGXR, GSAGPXX:TPTKM:SPIS:NDKQQII:YKQYE:GMVVD:RXGXS, STPPTXX:VPTRL:SPIS:DSANNVV:YKQYE:DMVVE:SXGXR, HVPKPXX:APTKL:SPIS:DDSSNVI:YKQYE:NMVVR:SXGXH, RVPSTXX:APVKT:SPIS:DNGRVLL:YKQYE:MIVEE:XGXL, ASAAPXX:VPQAL:SPIS:VGRKPKV:YKQYE:MIVRS:XKXS, ASASPXX:VSQDL:SPIS:IGKTPKI:YKQYE:MIVKS:XKXS, ASASPXX:VPQDL:SPIS:VGRTPKV:YKQYE:MVVKS:XKXS, NDEGLXS:VPTEE:SPIS:RIKPHQGQH:YKQYE:FLQHN:KXEXR, NDEGLXS:VPTGQ:SPIS:RIKPHQGQH:YKQYE:LEEHS:QXEXR, SSVKXQP:SRVHH:SPIS:EYVRKKPKL:YKQYE:RLEEH:LEXAXA, RNVQXRP:TQVQL:SPIS:EIVRKKPIF:YKQYE:TLEDH:LAXKXE, GIPEPXX:VPTKM:SPIS:DENKNVV:YKQYE:NMVVE:SXAXR, HVTKPTX:VPTKL:SPIS:DDSSNVI:YKQYE:NMVVE:SXGXH, YVPKPXX:VPTKL:SPIS:DDSSNVI:YKQYE:NMVVE:SXGXH, TVPKPXX:VPTQL:SPIS:DDSSNVI:YKQYE:NMVVE:AXGXH, AVPKAXX:VPTKL:SPIS:DSSNNVI:YKQYE:NMVVE:AXGXH, KVGKAXX:VPTKL:SPIS:DDMGVPT:YKQYE:EGMSVVE:XGXR, KASKAXX:VPTKL:SPIS:DKGVVTY:YKQYE:GMVVE:EXGXR, GSAGPXX:VPTKM:SPIS:NDKQQII:YKQYE:GMVVE:RXGXS, AAPASXX:VPTRL:SPIS:DAANNVV:YKQYE:DMVVE:AXGXR, HVPKPXX:VPTKL:SPIS:DDSSNVI:YKQYE:NMVVE:SXGXH, RVPSTXX:VPTKT:SPIS:DNGRVLL:YKQYE:MIVVE:XGXL, ASAAPXX:VPTAL:SPIS:VGRKPKV:YKQYE:MIVVE:XKXS, ASASPXX:VPTDL:SPIS:IGKTPKI:YKQYE:MIVVE:XKXS, ASASPXX:VPTDL:SPIS:VGRTPKV:YKQYE:MVVVE:XKXS, NDEGLXS:VPTEE:SPIS:RIKPHQGQH:YKQYE:FLVVE:KXEXR, NDEGLXS:VPTGQ:SPIS:RIKPHQGQH:YKQYE:LEVVE:QXEXR, SSVKXQP:VPTHH:SPIS:EYVRKKPKL:YKQYE:RLVVE:LEXAXA, RNVQXRP:VPTQL:SPIS:EIVRKKPIF:YKQYE:TLVVE:LAXKXE, AAPASXX:VPARL:SPIS:DAANNVV:YKQYE:DMVVE:AXGXR, KIPKAXX:APVEL:KPLS:DENEKVV:DHHKD:DMVEE:GXGXR, GIPEPXX:APVKM:KPLS:DENKNVV:DHHKD:NMVEE:SXAXR, SIPKAXX:APVEL:KPLS:DEYDKVV:DHHKD:EMVEE:GXGXR, HVTKPTX:APVKL:KPLS:DDSSNVI:DHHKD:NMVEE:SXGXH, YVPKPXX:APVKL:KPLS:DDSSNVI:DHHKD:NMVEE:SXGXH, TVPKPXX:APVQL:KPLS:DDSSNVI:DHHKD:NMVEE:AXGXH, AVPKAXX:APVKL:KPLS:DSSNNVI:DHHKD:NMVEE:AXGXH, KVGKAXX:APVKL:KPLS:DDMGVPT:DHHKD:EGMSVEE:XGXR, KASKAXX:APVKL:KPLS:DKGVVTY:DHHKD:GMVEE:EXGXR, GSAGPXX:APVKM:KPLS:NDKQQII:DHHKD:GMVEE:RXGXS, AAPASXX:APVRL:KPLS:DAANNVV:DHHKD:DMVEE:AXGXR, STPPTXX:APVRL:KPLS:DSANNVV:DHHKD:DMVEE:SXGXR, HVPKPXX:APVKL:KPLS:DDSSNVI:DHHKD:NMVEE:SXGXH, ASAAPXX:APVAL:KPLS:VGRKPKV:DHHKD:MIVEE:XKXS, ASASPXX:APVDL:KPLS:IGKTPKI:DHHKD:MIVEE:XKXS, ASASPXX:APVDL:KPLS:VGRTPKV:DHHKD:MVVEE:XKXS, NDEGLXS:APVEE:KPLS:RIKPHQGQH:DHHKD:FLVEE:KXEXR, NDEGLXS:APVGQ:KPLS:RIKPHQGQH:DHHKD:LEVEE:QXEXR, SSVKXQP:APVHH:KPLS:EYVRKKPKL:DHHKD:RLVEE:LEXAXA, RNVQXRP:APVQL:KPLS:EIVRKKPIF:DHHKD:TLVEE:LAXKXE, KIPKAXX:VPTEL:KPLS:DENEKVV:DHHKD:DMVVE:GXGXR, GIPEPXX:VPEKM:KPLS:DENKNVV:DHHKD:NMTVE:SXAXR, SIPKAXX:VPTEL:KPLS:DEYDKVV:DHHKD:EMVVE:GXGXR, HVTKPTX:APTKL:KPLS:DDSSNVI:DHHKD:NMVVR:SXGXH, YVPKPXX:APTKL:KPLS:DDSSNVI:DHHKD:NMVVR:SXGXH, TVPKPXX:APTQL:KPLS:DDSSNVI:DHHKD:NMVVR:AXGXH, AVPKAXX:APTKL:KPLS:DSSNNVI:DHHKD:NMVVK:AXGXH, KVGKAXX:VPTKL:KPLS:DDMGVPT:DHHKD:EGMSVAE:XGXR, KASKAXX:VPTKL:KPLS:DKGVVTY:DHHKD:GMAVS:EXGXR, GSAGPXX:TPTKM:KPLS:NDKQQII:DHHKD:GMVVD:RXGXS, AAPASXX:VPARL:KPLS:DAANNVV:DHHKD:DMVVE:AXGXR, STPPTXX:VPTRL:KPLS:DSANNVV:DHHKD:DMVVE:SXGXR, HVPKPXX:APTKL:KPLS:DDSSNVI:DHHKD:NMVVR:SXGXH, ASAAPXX:VPQAL:KPLS:VGRKPKV:DHHKD:MIVRS:XKXS, ASASPXX:VSQDL:KPLS:IGKTPKI:DHHKD:MIVKS:XKXS, ASASPXX:VPQDL:KPLS:VGRTPKV:DHHKD:MVVKS:XKXS, NDEGLXS:VPTEE:KPLS:RIKPHQGQH:DHHKD:FLQHN:KXEXR, NDEGLXS:VPTGQ:KPLS:RIKPHQGQH:DHHKD:LEEHS:QXEXR, SSVKXQP:SRVHH:KPLS:EYVRKKPKL:DHHKD:RLEEH:LEXAXA, RNVQXRP:TQVQL:KPLS:EIVRKKPIF:DHHKD:TLEDH:LAXKXE, KIPKAXX:VPQEL:EPLP:DENEKVV:EQLSN:DMVRS:GXGXR, GIPEPXX:VPQKM:EPLP:DENKNVV:EQLSN:NMVRS:SXAXR, SIPKAXX:VPQEL:EPLP:DEYDKVV:EQLSN:EMVRS:GXGXR, HVTKPTX:VPQKL:EPLP:DDSSNVI:EQLSN:NMVRS:SXGXH, YVPKPXX:VPQKL:EPLP:DDSSNVI:EQLSN:NMVRS:SXGXH, TVPKPXX:VPQQL:EPLP:DDSSNVI:EQLSN:NMVRS:AXGXH, AVPKAXX:VPQKL:EPLP:DSSNNVI:EQLSN:NMVRS:AXGXH, KVGKAXX:VPQKL:EPLP:DDMGVPT:EQLSN:EGMSVRS:XGXR, KASKAXX:VPQKL:EPLP:DKGVVTY:EQLSN:GMVRS:EXGXR, GSAGPXX:VPQKM:EPLP:NDKQQII:EQLSN:GMVRS:RXGXS, AAPASXX:VPQRL:EPLP:DAANNVV:EQLSN:DMVRS:AXGXR, STPPTXX:VPQRL:EPLP:DSANNVV:EQLSN:DMVRS:SXGXR, HVPKPXX:VPQKL:EPLP:DDSSNVI:EQLSN:NMVRS:SXGXH, RVPSTXX:VPQKT:EPLP:DNGRVLL:EQLSN:MIVRS:XGXL, ASASPXX:VPQDL:EPLP:IGKTPKI:EQLSN:MIVRS:XKXS, ASASPXX:VPQDL:EPLP:VGRTPKV:EQLSN:MVVRS:XKXS, NDEGLXS:VPQEE:EPLP:RIKPHQGQH:EQLSN:FLVRS:KXEXR, NDEGLXS:VPQGQ:EPLP:RIKPHQGQH:EQLSN:LEVRS:QXEXR, SSVKXQP:VPQHH:EPLP:EYVRKKPKL:EQLSN:RLVRS:LEXAXA, RNVQXRP:VPQQL:EPLP:EIVRKKPIF:EQLSN:TLVRS:LAXKXE, KIPKAXX:VPTEL:EPLP:DENEKVV:EQLSN:DMVVE:GXGXR, GIPEPXX:VPEKM:EPLP:DENKNVV:EQLSN:NMTVE:SXAXR, SIPKAXX:VPTEL:EPLP:DEYDKVV:EQLSN:EMVVE:GXGXR, HVTKPTX:APTKL:EPLP:DDSSNVI:EQLSN:NMVVR:SXGXH, YVPKPXX:APTKL:EPLP:DDSSNVI:EQLSN:NMVVR:SXGXH, TVPKPXX:APTQL:EPLP:DDSSNVI:EQLSN:NMVVR:AXGXH, AVPKAXX:APTKL:EPLP:DSSNNVI:EQLSN:NMVVK:AXGXH, KVGKAXX:VPTKL:EPLP:DDMGVPT:EQLSN:EGMSVAE:XGXR, KASKAXX:VPTKL:EPLP:DKGVVTY:EQLSN:GMAVS:EXGXR, GSAGPXX:TPTKM:EPLP:NDKQQII:EQLSN:GMVVD:RXGXS, AAPASXX:VPARL:EPLP:DAANNVV:EQLSN:DMVVE:AXGXR, STPPTXX:VPTRL:EPLP:DSANNVV:EQLSN:DMVVE:SXGXR, HVPKPXX:APTKL:EPLP:DDSSNVI:EQLSN:NMVVR:SXGXH, RVPSTXX:APVKT:EPLP:DNGRVLL:EQLSN:MIVEE:XGXL, ASASPXX:VSQDL:EPLP:IGKTPKI:EQLSN:MIVKS:XKXS, ASASPXX:VPQDL:EPLP:VGRTPKV:EQLSN:MVVKS:XKXS, NDEGLXS:VPTEE:EPLP:RIKPHQGQH:EQLSN:FLQHN:KXEXR, NDEGLXS:VPTGQ:EPLP:RIKPHQGQH:EQLSN:LEEHS:QXEXR, SSVKXQP:SRVHH:EPLP:EYVRKKPKL:EQLSN:RLEEH:LEXAXA, RNVQXRP:TQVQL:EPLP:EIVRKKPIF:EQLSN:TLEDH:LAXKXE, KIPKAXX:VSQEL:EPLT:DENEKVV:EQLSN:DMVKS:GXGXR, GIPEPXX:VSQKM:EPLT:DENKNVV:EQLSN:NMVKS:SXAXR, SIPKAXX:VSQEL:EPLT:DEYDKVV:EQLSN:EMVKS:GXGXR, HVTKPTX:VSQKL:EPLT:DDSSNVI:EQLSN:NMVKS:SXGXH, YVPKPXX:VSQKL:EPLT:DDSSNVI:EQLSN:NMVKS:SXGXH, TVPKPXX:VSQQL:EPLT:DDSSNVI:EQLSN:NMVKS:AXGXH, AVPKAXX:VSQKL:EPLT:DSSNNVI:EQLSN:NMVKS:AXGXH, KVGKAXX:VSQKL:EPLT:DDMGVPT:EQLSN:EGMSVKS:XGXR, KASKAXX:VSQKL:EPLT:DKGVVTY:EQLSN:GMVKS:EXGXR, GSAGPXX:VSQKM:EPLT:NDKQQII:EQLSN:GMVKS:RXGXS, AAPASXX:VSQRL:EPLT:DAANNVV:EQLSN:DMVKS:AXGXR, STPPTXX:VSQRL:EPLT:DSANNVV:EQLSN:DMVKS:SXGXR, HVPKPXX:VSQKL:EPLT:DDSSNVI:EQLSN:NMVKS:SXGXH, RVPSTXX:VSQKT:EPLT:DNGRVLL:EQLSN:MIVKS:XGXL, ASAAPXX:VSQAL:EPLT :VGRKPKV:EQLSN:MIVKS:XKXS, ASASPXX:VSQDL:EPLT:VGRTPKV:EQLSN:MVVKS:XKXS, NDEGLXS:VSQEE:EPLT:RIKPHQGQH:EQLSN:FLVKS:KXEXR, NDEGLXS:VSQGQ:EPLT:RIKPHQGQH:EQLSN:LEVKS:QXEXR, SSVKXQP:VSQHH:EPLT:EYVRKKPKL:EQLSN:RLVKS:LEXAXA, RNVQXRP:VSQQL:EPLT:EIVRKKPIF:EQLSN:TLVKS:LAXKXE, KIPKAXX:VPTEL:EPLT:DENEKVV:EQLSN:DMVVE:GXGXR, GIPEPXX:VPEKM:EPLT:DENKNVV:EQLSN:NMTVE:SXAXR, SIPKAXX:VPTEL:EPLT:DEYDKVV:EQLSN:EMVVE:GXGXR, HVTKPTX:APTKL:EPLT:DDSSNVI:EQLSN:NMVVR:SXGXH, YVPKPXX:APTKL:EPLT:DDSSNVI:EQLSN:NMVVR:SXGXH, TVPKPXX:APTQL:EPLT:DDSSNVI:EQLSN:NMVVR:AXGXH, AVPKAXX:APTKL:EPLT:DSSNNVI:EQLSN:NMVVK:AXGXH, KVGKAXX:VPTKL:EPLT:DDMGVPT:EQLSN:EGMSVAE:XGXR, KASKAXX:VPTKL:EPLT:DKGVVTY:EQLSN:GMAVS:EXGXR, GSAGPXX:TPTKM:EPLT:NDKQQII:EQLSN:GMVVD:RXGXS, AAPASXX:VPARL:EPLT:DAANNVV:EQLSN:DMVVE:AXGXR, STPPTXX:VPTRL:EPLT:DSANNVV:EQLSN:DMVVE:SXGXR, HVPKPXX:APTKL:EPLT:DDSSNVI:EQLSN:NMVVR:SXGXH, RVPSTXX:APVKT:EPLT:DNGRVLL:EQLSN:MIVEE:XGXL, ASAAPXX:VPQAL:EPLT:VGRKPKV:EQLSN:MIVRS:XKXS, ASASPXX:VPQDL:EPLT:VGRTPKV:EQLSN:MVVKS:XKXS, NDEGLXS:VPTEE:EPLT:RIKPHQGQH:EQLSN:FLQHN:KXEXR, NDEGLXS:VPTGQ:EPLT:RIKPHQGQH:EQLSN:LEEHS:QXEXR, SSVKXQP:SRVHH:EPLT:EYVRKKPKL:EQLSN:RLEEH:LEXAXA, RNVQXRP:TQVQL:EPLT:EIVRKKPIF:EQLSN:TLEDH:LAXKXE, KIPKAXX:VPQEL:EPLT:DENEKVV:EQLSN:DMVKS:GXGXR, GIPEPXX:VPQKM:EPLT:DENKNVV:EQLSN:NMVKS:SXAXR, SIPKAXX:VPQEL:EPLT:DEYDKVV:EQLSN:EMVKS:GXGXR, HVTKPTX:VPQKL:EPLT:DDSSNVI:EQLSN:NMVKS:SXGXH, YVPKPXX:VPQKL:EPLT:DDSSNVI:EQLSN:NMVKS:SXGXH, TVPKPXX:VPQQL:EPLT:DDSSNVI:EQLSN:NMVKS:AXGXH, AVPKAXX:VPQKL:EPLT:DSSNNVI:EQLSN:NMVKS:AXGXH, KVGKAXX:VPQKL:EPLT:DDMGVPT:EQLSN:EGMSVKS:XGXR, KASKAXX:VPQKL:EPLT:DKGVVTY:EQLSN:GMVKS:EXGXR, GSAGPXX:VPQKM:EPLT:NDKQQII:EQLSN:GMVKS:RXGXS, AAPASXX:VPQRL:EPLT:DAANNVV:EQLSN:DMVKS:AXGXR, STPPTXX:VPQRL:EPLT:DSANNVV:EQLSN:DMVKS:SXGXR, HVPKPXX:VPQKL:EPLT:DDSSNVI:EQLSN:NMVKS:SXGXH, RVPSTXX:VPQKT:EPLT:DNGRVLL:EQLSN:MIVKS:XGXL, ASAAPXX:VPQAL:EPLT:VGRKPKV:EQLSN:MIVKS:XKXS, ASASPXX:VPQDL:EPLT:IGKTPKI:EQLSN:MIVKS:XKXS, NDEGLXS:VPQEE:EPLT:RIKPHQGQH:EQLSN:FLVKS:KXEXR, NDEGLXS:VPQGQ:EPLT:RIKPHQGQH:EQLSN:LEVKS:QXEXR, SSVKXQP:VPQHH:EPLT:EYVRKKPKL:EQLSN:RLVKS:LEXAXA, RNVQXRP:VPQQL:EPLT:EIVRKKPIF:EQLSN:TLVKS:LAXKXE, ASASPXX:VSQDL:EPLT:IGKTPKI:EQLSN:MIVKS:XKXS, KIPKAXX:VPTEL:SNIT:DENEKVV:IGEMS:DMQHN:GXGXR, GIPEPXX:VPTKM:SNIT:DENKNVV:IGEMS:NMQHN:SXAXR, SIPKAXX:VPTEL:SNIT:DEYDKVV:IGEMS:EMQHN:GXGXR, HVTKPTX:VPTKL:SNIT:DDSSNVI:IGEMS:NMQHN:SXGXH, YVPKPXX:VPTKL:SNIT:DDSSNVI:IGEMS:NMQHN:SXGXH, TVPKPXX:VPTQL:SNIT:DDSSNVI:IGEMS:NMQHN:AXGXH, AVPKAXX:VPTKL:SNIT:DSSNNVI:IGEMS:NMQHN:AXGXH, KVGKAXX:VPTKL:SNIT:DDMGVPT:IGEMS:EGMSQHN:XGXR, KASKAXX:VPTKL:SNIT:DKGVVTY:IGEMS:GMQHN:EXGXR, GSAGPXX:VPTKM:SNIT:NDKQQII:IGEMS:GMQHN:RXGXS, AAPASXX:VPTRL:SNIT:DAANNVV:IGEMS:DMQHN:AXGXR, STPPTXX:VPTRL:SNIT:DSANNVV:IGEMS:DMQHN:SXGXR, HVPKPXX:VPTKL:SNIT:DDSSNVI:IGEMS:NMQHN:SXGXH, RVPSTXX:VPTKT:SNIT:DNGRVLL:IGEMS:MIQHN:XGXL, ASAAPXXVPTAL:SNITVGRKPKV:IGEMS:MIQHN:XKXS, ASASPXX:VPTDL:SNIT:IGKTPKI:IGEMS:MIQHN:XKXS, ASASPXX:VPTDL:SNIT:VGRTPKV:IGEMS:MVQHN:XKXS, NDEGLXS:VPTGQ:SNIT:RIKPHQGQH:IGEMS:LEQHN:QXEXR, NDEGLXS:VPTEE:SNIT:RIKPHQGQH:IGEMS:FLQHN:KXEXR, SSVKXQP:VPTHH:SNIT:EYVRKKPKL:IGEMS:RLQHN:LEXAXA, RNVQXRP:VPTQL:SNIT:EIVRKKPIF:IGEMS:TLQHN:LAXKXE, KIPKAXX:VPTEL:SNIT:DENEKVV:IGEMS:DMVVE:GXGXR, GIPEPXX:VPEKM:SNIT:DENKNVV:IGEMS:NMTVE:SXAXR, SIPKAXX:VPTEL:SNIT:DEYDKVV:IGEMS:EMVVE:GXGXR, HVTKPTX:APTKL:SNIT:DDSSNVI:IGEMS:NMVVR:SXGXH, YVPKPXX:APTKL:SNIT:DDSSNVI:IGEMS:NMVVR:SXGXH, TVPKPXX:APTQL:SNIT:DDSSNVI:IGEMS:NMVVR:AXGXH, AVPKAXX:APTKL:SNIT:DSSNNVI:IGEMS:NMVVK:AXGXH, KVGKAXX:VPTKL:SNIT:DDMGVPT:IGEMS:EGMSVAE:XGXR, KASKAXX:VPTKL:SNIT:DKGVVTY:IGEMS:GMAVS:EXGXR, GSAGPXX:TPTKM:SNIT:NDKQQII:IGEMS:GMVVD:RXGXS, AAPASXX:VPARL:SNIT:DAANNVV:IGEMS:DMVVE:AXGXR, STPPTXX:VPTRL:SNIT:DSANNVV:IGEMS:DMVVE:SXGXR, HVPKPXX:APTKL:SNIT:DDSSNVI:IGEMS:NMVVR:SXGXH, RVPSTXX:APVKT:SNIT:DNGRVLL:IGEMS:MIVEE:XGXL, ASAAPXX:VPQAL:SNIT:VGRKPKV:IGEMS:MIVRS:XKXS, ASASPXX:VSQDL:SNIT:IGKTPKI:IGEMS:MIVKS:XKXS, ASASPXX:VPQDL:SNIT:VGRTPKV:IGEMS:MVVKS:XKXS, NDEGLXS:VPTGQ:SNIT:RIKPHQGQH:IGEMS:LEEHS:QXEXR, SSVKXQP:SRVHH:SNIT:EYVRKKPKL:IGEMS:RLEEH:LEXAXA, RNVQXRP:TQVQL:SNIT:EIVRKKPIF:IGEMS:TLEDH:LAXKXE, KIPKAXX:VPTEL:SNIT:DENEKVV:LGEMS:DMEHS:GXGXR, GIPEPXX:VPTKM:SNIT:DENKNVV:LGEMS:NMEHS:SXAXR, SIPKAXX:VPTEL:SNIT:DEYDKVV:LGEMS:EMEHS:GXGXR, HVTKPTX:VPTKL:SNIT:DDSSNVI:LGEMS:NMEHS:SXGXH, YVPKPXX:VPTKL:SNIT:DDSSNVI:LGEMS:NMEHS:SXGXH, TVPKPXX:VPTQL:SNIT:DDSSNVI:LGEMS:NMEHS:AXGXH, AVPKAXX:VPTKL:SNIT:DSSNNVI:LGEMS:NMEHS:AXGXH, KVGKAXX:VPTKL:SNIT:DDMGVPT:LGEMS:EGMSEHS:XGXR, KASKAXX:VPTKL:SNIT:DKGVVTY:LGEMS:GMEHS:EXGXR, GSAGPXX:VPTKM:SNIT:NDKQQII:LGEMS:GMEHS:RXGXS, AAPASXX:VPTRL:SNIT:DAANNVV:LGEMS:DMEHS:AXGXR, STPPTXX:VPTRL:SNIT:DSANNVV:LGEMS:DMEHS:SXGXR, HVPKPXX:VPTKL:SNIT:DDSSNVI:LGEMS:NMEHS:SXGXH, RVPSTXX:VPTKT:SNIT:DNGRVLL:LGEMS:MIEHS:XGXL, ASAAPXX:VPTAL:SNIT:VGRKPKV:LGEMS:MIEHS:XKXS, ASASPXXVPTDL:SNIT:IGKTPKI:LGEMS:MIEHS:XKXS, ASASPXX:VPTDL:SNIT:VGRTPKV:LGEMS:MVEHS:XKXS, NDEGLXS:VPTEE:SNIT:RIKPHQGQH:LGEMS:FLEHS:KXEXR, SSVKXQP:VPTHH:SNIT:EYVRKKPKL:LGEMS:RLEHS:LEXAXA, RNVQXRP:VPTQL:SNIT:EIVRKKPIF:LGEMS:TLEHS:LAXKXE, KIPKAXX:VPTEL:SNIT:DENEKVV:LGEMS:DMVVE:GXGXR, GIPEPXX:VPEKM:SNIT:DENKNVV:LGEMS:NMTVE:SXAXR, SIPKAXX:VPTEL:SNIT:DEYDKVV:LGEMS:EMVVE:GXGXR, HVTKPTX:APTKL:SNIT:DDSSNVI:LGEMS:NMVVR:SXGXH, YVPKPXX:APTKL:SNIT:DDSSNVI:LGEMS:NMVVR:SXGXH, TVPKPXX:APTQL:SNIT:DDSSNVI:LGEMS:NMVVR:AXGXH, AVPKAXX:APTKL:SNIT:DSSNNVI:LGEMS:NMVVK:AXGXH, KVGKAXX:VPTKL:SNIT:DDMGVPT:LGEMS:EGMSVAE:XGXR, KASKAXX:VPTKL:SNIT:DKGVVTY:LGEMS:GMAVS:EXGXR, GSAGPXX:TPTKM:SNIT:NDKQQII:LGEMS:GMVVD:RXGXS, AAPASXX:VPARL:SNIT:DAANNVV:LGEMS:DMVVE:AXGXR, STPPTXX:VPTRL:SNIT:DSANNVV:LGEMS:DMVVE:SXGXR, HVPKPXX:APTKL:SNIT:DDSSNVI:LGEMS:NMVVR:SXGXH, RVPSTXX:APVKT:SNIT:DNGRVLL:LGEMS:MIVEE:XGXL, ASAAPXX:VPQAL:SNIT:VGRKPKV:LGEMS:MIVRS:XKXS, ASASPXX:VSQDL:SNIT:IGKTPKI:LGEMS:MIVKS:XKXS, ASASPXX:VPQDL:SNIT:VGRTPKV:LGEMS:MVVKS:XKXS, NDEGLXS:VPTEE:SNIT:RIKPHQGQH:LGEMS:FLQHN:KXEXR, SSVKXQP:SRVHH:SNIT:EYVRKKPKL:LGEMS:RLEEH:LEXAXA, RNVQXRP:TQVQL:SNIT:EIVRKKPIF:LGEMS:TLEDH:LAXKXE, KIPKAXX:SRVEL:RSVK:DENEKVV:KEVQV:DMEEH:GXGXR, GIPEPXX:SRVKM:RSVK:DENKNVV:KEVQV:NMEEH:SXAXR, SIPKAXX:SRVEL:RSVK:DEYDKVV:KEVQV:EMEEH:GXGXR, HVTKPTX:SRVKL:RSVK:DDSSNVI:KEVQV:NMEEH:SXGXH, YVPKPXX:SRVKL:RSVK:DDSSNVI:KEVQV:NMEEH:SXGXH, TVPKPXX:SRVQL:RSVK:DDSSNVI:KEVQV:NMEEH:AXGXH, AVPKAXX:SRVKL:RSVK:DSSNNVI:KEVQV:NMEEH:AXGXH, KVGKAXX:SRVKL:RSVK:DDMGVPT:KEVQV:EGMSEEH:XGXR, KASKAXX:SRVKL:RSVK:DKGVVTY:KEVQV:GMEEH:EXGXR, GSAGPXX:SRVKM:RSVK:NDKQQII:KEVQV:GMEEH:RXGXS, AAPASXX:SRVRL:RSVK:DAANNVV:KEVQV:DMEEH:AXGXR, STPPTXX:SRVRL:RSVK:DSANNVV:KEVQV:DMEEH:SXGXR, HVPKPXX:SRVKL:RSVK:DDSSNVI:KEVQV:NMEEH:SXGXH, RVPSTXX:SRVKT:RSVK:DNGRVLL:KEVQV:MIEEH:XGXL, ASAAPXX:SRVAL:RSVK:VGRKPKV:KEVQV:MIEEH:XKXS, ASASPXX:SRVDL:RSVK:IGKTPKI:KEVQV:MIEEH:XKXS, ASASPXX:SRVDL:RSVK:VGRTPKV:KEVQV:MVEEH:XKXS, NDEGLXS:SRVEE:RSVK:RIKPHQGQH:KEVQV:FLEEH:KXEXR, NDEGLXS:SRVGQ:RSVK:RIKPHQGQH:KEVQV:LEEEH:QXEXR, RNVQXRP:SRVQL:RSVK:EIVRKKPIF:KEVQV:TLEEH:LAXKXE, KIPKAXX:VPTEL:RSVK:DENEKVV:KEVQV:DMVVE:GXGXR, GIPEPXX:VPEKM:RSVK:DENKNVV:KEVQV:NMTVE:SXAXR, SIPKAXX:VPTEL:RSVK:DEYDKVV:KEVQV:EMVVE:GXGXR, HVTKPTX:APTKL:RSVK:DDSSNVI:KEVQV:NMVVR:SXGXH, YVPKPXX:APTKL:RSVK:DDSSNVI:KEVQV:NMVVR:SXGXH, TVPKPXX:APTQL:RSVK:DDSSNVI:KEVQV:NMVVR:AXGXH, AVPKAXX:APTKL:RSVK:DSSNNVI:KEVQV:NMVVK:AXGXH, KVGKAXX:VPTKL:RSVK:DDMGVPT:KEVQV:EGMSVAE:XGXR, KASKAXX:VPTKL:RSVK:DKGVVTY:KEVQV:GMAVS:EXGXR, GSAGPXX:TPTKM:RSVK:NDKQQII:KEVQV:GMVVD:RXGXS, AAPASXX:VPARL:RSVK:DAANNVV:KEVQV:DMVVE:AXGXR, STPPTXX:VPTRL:RSVK:DSANNVV:KEVQV:DMVVE:SXGXR, HVPKPXX:APTKL:RSVK:DDSSNVI:KEVQV:NMVVR:SXGXH, RVPSTXX:APVKT:RSVK:DNGRVLL:KEVQV:MIVEE:XGXL, ASAAPXX:VPQAL:RSVK:VGRKPKV:KEVQV:MIVRS:XKXS, ASASPXX:VSQDL:RSVK:IGKTPKI:KEVQV:MIVKS:XKXS, ASASPXX:VPQDL:RSVK:VGRTPKV:KEVQV:MVVKS:XKXS, NDEGLXS:VPTEE:RSVK:RIKPHQGQH:KEVQV:FLQHN:KXEXR, NDEGLXS:VPTGQ:RSVK:RIKPHQGQH:KEVQV:LEEHS:QXEXR, RNVQXRP:TQVQL:RSVK:EIVRKKPIF:KEVQV:TLEDH:LAXKXE, KIPKAXX:TQVEL:RPVQ:DENEKVV:KKATV:DMEDH:GXGXR, GIPEPXX:TQVKM:RPVQ:DENKNVV:KKATV:NMEDH:SXAXR, SIPKAXX:TQVEL:RPVQ:DEYDKVV:KKATV:EMEDH:GXGXR, HVTKPTX:TQVKL:RPVQ:DDSSNVI:KKATV:NMEDH:SXGXH, YVPKPXX:TQVKL:RPVQ:DDSSNVI:KKATV:NMEDH:SXGXH, TVPKPXX:TQVQL:RPVQ:DDSSNVI:KKATV:NMEDH:AXGXH, AVPKAXX:TQVKL:RPVQ:DSSNNVI:KKATV:NMEDH:AXGXH, KVGKAXX:TQVKL:RPVQ:DDMGVPT:KKATV:EGMSEDH:XGXR, KASKAXX:TQVKL:RPVQ:DKGVVTY:KKATV:GMEDH:EXGXR, GSAGPXX:TQVKM:RPVQ:NDKQQII:KKATV:GMEDH:RXGXS, AAPASXX:TQVRL:RPVQ:DAANNVV:KKATV:DMEDH:AXGXR, STPPTXX:TQVRL:RPVQ:DSANNVV:KKATV:DMEDH:SXGXR, HVPKPXX:TQVKL:RPVQ:DDSSNVI:KKATV:NMEDH:SXGXH, RVPSTXX:TQVKT:RPVQ:DNGRVLL:KKATV:MIEDH:XGXL, ASAAPXX:TQVAL:RPVQ:VGRKPKV:KKATV:MIEDH:XKXS, ASASPXX:TQVDL:RPVQ:IGKTPKI:KKATV:MIEDH:XKXS, ASASPXX:TQVDL:RPVQ:VGRTPKV:KKA TV:MVEDH:XKXS, NDEGLXS:TQVEE:RPVQ:RIKPHQGQH:KKATV:FLEDH:KXEXR, NDEGLXS:TQVGQ:RPVQ:RIKPHQGQH:KKATV:LEEDH:QXEXR, SSVKXQP:TQVHH:RPVQ:EYVRKKPKL:KKATV:RLEDH:LEXAXA, KIPKAXX:VPTEL:RPVQ:DENEKVV:KKATV:DMVVE:GXGXR, GIPEPXX:VPEKM:RPVQ:DENKNVV:KKATV:NMTVE:SXAXR, SIPKAXX:VPTEL:RPVQ:DEYDKVV:KKATV:EMVVE:GXGXR, HVTKPTX:APTKL:RPVQ:DDSSNVI:KKATV:NMVVR:SXGXH, YVPKPXX:APTKL:RPVQ:DDSSNVI:KKATV:NMVVR:SXGXH, TVPKPXX:APTQL:RPVQ:DDSSNVI:KKATV:NMVVR:AXGXH, AVPKAXX:APTKL:RPVQ:DSSNNVI:KKATV:NMVVK:AXGXH, KVGKAXX:VPTKL:RPVQ:DDMGVPT:KKATV:EGMSVAE :XGXR, KASKAXX:VPTKL:RPVQ:DKGVVTY:KKATV:GMAVS:EXGXR, GSAGPXX:TPTKM:RPVQ:NDKQQII:KKATV:GMVVD:RXGXS, AAPASXX:VPARL:RPVQ:DAANNVV:KKATV:DMVVE:AXGXR, STPPTXX:VPTRL:RPVQ:DSANNVV:KKATV:DMVVE:SXGXR, HVPKPXX:APTKL:RPVQ:DDSSNVI:KKATV:NMVVR:SXGXH, RVPSTXX:APVKT:RPVQ:DNGRVLL:KKATV:MIVEE:XGXL, ASAAPXX:VPQAL:RPVQ:VGRKPKV:KKATV:MIVRS:XKXS, ASASPXX:VSQDL:RPVQ:IGKTPKI:KKATV:MIVKS:XKXS, ASASPXX:VPQDL:RPVQ:VGRTPKV:KKATV:MVVKS:XKXS, NDEGLXS:VPTEE:RPVQ:RIKPHQGQH:KKATV:FLQHN:KXEXR, NDEGLXS:VPTGQ:RPVQ:RIKPHQGQH:KKATV:LEEHS:QXEXR, SSVKXQP:SRVHH:RPVQ:EYVRKKPKL:KKATV:RLEEH:LEXAXA. More particularly, the heptuplet PEP7:PEP5:PEP1:LINKER:PEP2:PEP6:PEP8 is selected from the group consisting of GIPEPXX:VPTKM:SAIS:DENKNVV:LKNYQ:NMVVE:SXAXR, HVTKPTX:VPTKL:SAIS:DDSSNVI:LKNYQ:NMVVE:SXGXH, YVPKPXX:VPTKL:SAIS:DDSSNVI:LKNYQ:NMVVE:SXGXH, TVPKPXX:VPTQL:SAIS:DDSSNVI:LKNYQ:NMVVE:AXGXH, AVPKAXX:VPTKL:SAIS:DSSNNVI:LKNYQ:NMVVE:AXGXH, KVGKAXX:VPTKL:SAIS:DDMGVPT:LKNYQ:EGMSVVE:XGXR, KASKAXX:VPTKL:SAIS:DKGVVTY:LKNYQ:GMVVE:EXGXR, GSAGPXX:VPTKM:SAIS:NDKQQII:LKNYQ:GMVVE:RXGXS, AAPASXX:VPTRL:SAIS:DAANNVV:LKNYQ:DMVVE:AXGXR, STPPTXX:VPTRL:SAIS:DSANNVV:LKNYQ:DMVVE:SXGXR, HVPKPXX:VPTKL:SAIS:DDSSNVI:LKNYQ:NMVVE:SXGXH, RVPSTXX:VPTKT:SAIS:DNGRVLL:LKNYQ:MIVVE:XGXL, ASAAPXX:VPTAL:SAIS:VGRKPKV:LKNYQ:MIVVE:XKXS, ASASPXX:VPTDL:SAIS:IGKTPKI:LKNYQ:MIVVE:XKXS, ASASPXX:VPTDL:SAIS:VGRTPKV:LKNYQ:MVVVE:XKXS, GIPEPXX:VPEKM:SAIS:DENKNVV:LKNYQ:NMTVE:SXAXR, HVTKPTX:APTKL:SAIS:DDSSNVI:LKNYQ:NMVVR:SXGXH, YVPKPXX:APTKL:SAIS:DDSSNVI:LKNYQ:NMVVR:SXGXH, TVPKPXX:APTQL:SAIS:DDSSNVI:LKNYQ:NMVVR:AXGXH, AVPKAXX:APTKL:SAIS:DSSNNVI:LKNYQ:NMVVK:AXGXH, KVGKAXX:VPTKL:SAIS:DDMGVPT:LKNYQ:EGMSVAE:XGXR, KASKAXX:VPTKL:SAIS:DKGVVTY:LKNYQ:GMAVS:EXGXR, GSAGPXX:TPTKM:SAIS:NDKQQII:LKNYQ:GMVVD:RXGXS, AAPASXX:VPARL:SAIS:DAANNVV:LKNYQ:DMVVE:AXGXR, HVPKPXX:APTKL:SAIS:DDSSNVI:LKNYQ:NMVVR:SXGXH, RVPSTXX:APVKT:SAIS:DNGRVLL:LKNYQ:MIVEE:XGXL, ASAAPXX:VPQAL:SAIS:VGRKPKV:LKNYQ:MIVRS:XKXS, ASASPXX:VSQDL:SAIS:IGKTPKI:LKNYQ:MIVKS:XKXS, ASASPXX:VPQDL:SAIS:VGRTPKV:LKNYQ:MVVKS:XKXS, NDEGLEX:VPTEE:SAIS:RIKPHQGQH:LKNYQ:FLQHN:KXEXR, NDEGLEX:VPTGQ:SAIS:RIKPHQGQH:LKNYQ:LEEHS:QXEXR, SSVKXQP:SRVHH:SAIS:EYVRKKPKL:LKNYQ:RLEEH:LEXAXA, RNVQXRP:TQVQL:SAIS:EIVRKKPIF:LKNYQ:TLEDH:LAXKXE, KIPKAXX:VPEEL:SSLS:DENEKVV:LKVYP:DMTVE:GXGXR, SIPKAXX:VPEEL:SSLS:DEYDKVV:LKVYP:EMTVE:GXGXR, HVTKPTX:VPEKL:SSLS:DDSSNVI:LKVYP:NMTVE:SXGXH, YVPKPXX:VPEKL:SSLS:DDSSNVI:LKVYP:NMTVE:SXGXH, TVPKPXX:VPEQL:SSLS:DDSSNVI:LKVYP:NMTVE:AXGXH, AVPKAXX:VPEKL:SSLS:DSSNNVI:LKVYP:NMTVE:AXGXH, KVGKAXX:VPEKL:SSLS:DDMGVPT:LKVYP:EGMSTVE:XGXR, KASKAXX:VPEKL:SSLS:DKGVVTY:LKVYP:GMTVE:EXGXR, GSAGPXX:VPEKM:SSLS:NDKQQII:LKVYP:GMTVE:RXGXS, AAPASXX:VPERL:SSLS:DAANNVV:LKVYP:DMTVE:AXGXR, STPPTXX:VPERL:SSLS:DSANNVV:LKVYP:DMTVE:SXGXR, HVPKPXX:VPEKL:SSLS:DDSSNVI:LKVYP:NMTVE:SXGXH, RVPSTXX:VPEKT:SSLS:DNGRVLL:LKVYP:MITVE:XGXL, ASAAPXX:VPEAL:SSLS:VGRKPKV:LKVYP:MITVE:XKXS, ASASPXX:VPEDL:SSLS:IGKTPKI:LKVYP:MITVE:XKXS, ASASPXX:VPEDL:SSLS:VGRTPKV:LKVYP:MVTVE:XKXS, KIPKAXX:VPTEL:SSLS:DENEKVV:LKVYP:DMVVE:GXGXR, SIPKAXX:VPTEL:SSLS:DEYDKVV:LKVYP:EMVVE:GXGXR, HVTKPTX:APTKL:SSLS:DDSSNVI:LKVYP:NMVVR:SXGXH, YVPKPXX:APTKL:SSLS:DDSSNVI:LKVYP:NMVVR:SXGXH, TVPKPXX:APTQL:SSLS:DDSSNVI:LKVYP:NMVVR:AXGXH, AVPKAXX:APTKL:SSLS:DSSNNVI:LKVYP:NMVVK:AXGXH, KVGKAXX:VPTKL:SSLS:DDMGVPT:LKVYP:EGMSVAE:XGXR, KASKAXX:VPTKL:SSLS:DKGVVTY:LKVYP:GMAVS:EXGXR, GSAGPXX:TPTKM:SSLS:NDKQQII:LKVYP:GMVVD:RXGXS, AAPASXX:VPARL:SSLS:DAANNVV:LKVYP:DMVVE:AXGXR, STPPTXX:VPTRL:SSLS:DSANNVV:LKVYP:DMVVE:SXGXR, HVPKPXX:APTKL:SSLS:DDSSNVI:LKVYP:NMVVR:SXGXH, RVPSTXX:APVKT:SSLS:DNGRVLL:LKVYP:MIVEE:XGXL, ASAAPXX:VPQAL:SSLS:VGRKPKV:LKVYP:MIVRS:XKXS, ASASPXX:VSQDL:SSLS:IGKTPKI:LKVYP:MIVKS:XKXS, ASASPXX:VPQDL:SSLS:VGRTPKV:LKVYP:MVVKS:XKXS, NDEGLEX:VPTEE:SSLS:RIKPHQGQH:LKVYP:FLQHN:KXEXR, NDEGLEX:VPTGQ:SSLS:RIKPHQGQH:LKVYP:LEEHS:QXEXR, SSVKXQP:SRVHH:SSLS:EYVRKKPKL:LKVYP:RLEEH:LEXAXA, RNVQXRP:TQVQL:SSLS:EIVRKKPIF:LKVYP:TLEDH:LAXKXE, KIPKAXX:APTEL:NAIS:DENEKVV:LKKYR:DMVVR:GXGXR, GIPEPXX:APTKM:NAIS:DENKNVV:LKKYR:NMVVR:SXAXR, SIPKAXX:APTEL:NAIS:DEYDKVV:LKKYR:EMVVR:GXGXR, AVPKAXX:APTKL:NAIS:DSSNNVI:LKKYR:NMVVR:AXGXH, KVGKAXX:APTKL:NAIS:DDMGVPT:LKKYR:EGMSVVR:XGXR, KASKAXX:APTKL:NAIS:DKGVVTY:LKKYR:GMVVR:EXGXR, GSAGPXX:APTKM:NAIS:NDKQQII:LKKYR:GMVVR:RXGXS, AAPASXX:APTRL:NAIS:DAANNVV:LKKYR:DMVVR:AXGXR, STPPTXX:APTRL:NAIS:DSANNVV:LKKYR:DMVVR:SXGXR, RVPSTXX:APTKT:NAIS:DNGRVLL:LKKYR:MIVVR:XGXL, ASAAPXX:APTAL:NAIS:VGRKPKV:LKKYR:MIVVR:XKXS, ASASPXX:APTDL:NAIS:IGKTPKI:LKKYR:MIVVR:XKXS, ASASPXX:APTDL:NAIS:VGRTPKV:LKKYR:MVVVR:XKXS, KIPKAXX:VPTEL:NAIS:DENEKVV:LKKYR:DMVVE:GXGXR, GIPEPXX:VPEKM:NAIS:DENKNVV:LKKYR:NMTVE:SXAXR, SIPKAXX:VPTEL:NAIS:DEYDKVV:LKKYR:EMVVE:GXGXR, AVPKAXX:APTKL:NAIS:DSSNNVI:LKKYR:NMVVK:AXGXH, KVGKAXX:VPTKL:NAIS:DDMGVPT:LKKYR:EGMSVAE:XGXR, KASKAXX:VPTKL:NAIS:DKGVVTY:LKKYR:GMAVS:EXGXR, GSAGPXX:TPTKM:NAIS:NDKQQII:LKKYR:GMVVD:RXGXS, AAPASXX:VPARL:NAIS:DAANNVV:LKKYR:DMVVE:AXGXR, STPPTXX:VPTRL:NAIS:DSANNVV:LKKYR:DMVVE:SXGXR, RVPSTXX:APVKT:NAIS:DNGRVLL:LKKYR:MIVEE:XGXL, ASAAPXX:VPQAL:NAIS:VGRKPKV:LKKYR:MIVRS:XKXS, ASASPXX:VSQDL:NAIS:IGKTPKI:LKKYR:MIVKS:XKXS, ASASPXX:VPQDL:NAIS:VGRTPKV:LKKYR:MVVKS:XKXS, NDEGLEX:VPTEE:NAIS:RIKPHQGQH:LKKYR:FLQHN:KXEXR, NDEGLEX:VPTGQ:NAIS:RIKPHQGQH:LKKYR:LEEHS:QXEXR, SSVKXQP:SRVHH:NAIS:EYVRKKPKL:LKKYR:RLEEH:LEXAXA, RNVQXRP:TQVQL:NAIS:EIVRKKPIF:LKKYR:TLEDH:LAXKXE, KIPKAXX:APTEL:SATS:DENEKVV:LRKHR:DMVVK:GXGXR, GIPEPXX:APTKM:SATS:DENKNVV:LRKHR:NMVVK:SXAXR, SIPKAXX:APTEL:SATS:DEYDKVV:LRKHR:EMVVK:GXGXR, HVTKPTX:APTKL:SATS:DDSSNVI:LRKHR:NMVVK:SXGXH, YVPKPXX:APTKL:SATS:DDSSNVI:LRKHR:NMVVK:SXGXH, TVPKPXX:APTQL:SATS:DDSSNVI:LRKHR:NMVVK:AXGXH, KVGKAXX:APTKL:SATS:DDMGVPT:LRKHR:EGMSVVK:XGXR, KASKAXX:APTKL:SATS:DKGVVTY:LRKHR:GMVVK:EXGXR, GSAGPXX:APTKM:SATS:NDKQQII:LRKHR:GMVVK:RXGXS, AAPASXX:APTRL:SATS:DAANNVV:LRKHR:DMVVK:AXGXR, STPPTXX:APTRL:SATS:DSANNVV:LRKHR:DMVVK:SXGXR, HVPKPXX:APTKL:SATS:DDSSNVI:LRKHR:NMVVK:SXGXH, RVPSTXX:APTKT:SATS:DNGRVLL:LRKHR:MIVVK:XGXL, ASAAPXX:APTAL:SATS:VGRKPKV:LRKHR:MIVVK:XKXS, ASASPXX:APTDL:SATS:IGKTPKI:LRKHR:MIVVK:XKXS, ASASPXX:APTDL:SATS:VGRTPKV:LRKHR:MVVVK:XKXS, KIPKAXX:VPTEL:SATS:DENEKVV:LRKHR:DMVVE:GXGXR, GIPEPXX:VPEKM:SATS:DENKNVV:LRKHR:NMTVE:SXAXR, SIPKAXX:VPTEL:SATS:DEYDKVV:LRKHR:EMVVE:GXGXR, HVTKPTX:APTKL:SATS:DDSSNVI:LRKHR:NMVVR:SXGXH, YVPKPXX:APTKL:SATS:DDSSNVI:LRKHR:NMVVR:SXGXH, TVPKPXX:APTQL:SATS:DDSSNVI:LRKHR:NMVVR:AXGXH, KVGKAXX:VPTKL:SATS:DDMGVPT:LRKHR:EGMSVAE:XGXR, KASKAXX:VPTKL:SATS:DKGVVTY:LRKHR:GMAVS:EXGXR, GSAGPXX:TPTKM:SATS:NDKQQII:LRKHR:GMVVD:RXGXS, AAPASXX:VPARL:SATS:DAANNVV:LRKHR:DMVVE:AXGXR, STPPTXX:VPTRL:SATS:DSANNVV:LRKHR:DMVVE:SXGXR, HVPKPXX:APTKL:SATS:DDSSNVI:LRKHR:NMVVR:SXGXH, RVPSTXX:APVKT:SATS:DNGRVLL:LRKHR:MIVEE:XGXL, ASAAPXX:VPQAL:SATS:VGRKPKV:LRKHR:MIVRS:XKXS, ASASPXX:VSQDL:SATS:IGKTPKI:LRKHR:MIVKS:XKXS, ASASPXX:VPQDL:SATS:VGRTPKV:LRKHR:MVVKS:XKXS, NDEGLEX:VPTEE:SATS:RIKPHQGQH:LRKHR:FLQHN:KXEXR, NDEGLEX:VPTGQ:SATS:RIKPHQGQH:LRKHR:LEEHS:QXEXR, SSVKXQP:SRVHH:SATS:EYVRKKPKL:LRKHR:RLEEH:LEXAXA, RNVQXRP:TQVQL:SATS:EIVRKKPIF:LRKHR:TLEDH:LAXKXE, KIPKAXX:VPTEL:SPIS:DENEKVV:LKYHY:DMVAE:GXGXR, GIPEPXX:VPTKM:SPIS:DENKNVV:LKYHY:NMVAE:SXAXR, SIPKAXX:VPTEL:SPIS:DEYDKVV:LKYHY:EMVAE:GXGXR, HVTKPTX:VPTKL:SPIS:DDSSNVI:LKYHY:NMVAE:SXGXH, YVPKPXX:VPTKL:SPIS:DDSSNVI:LKYHY:NMVAE:SXGXH, TVPKPXX:VPTQL:SPIS:DDSSNVI:LKYHY:NMVAE:AXGXH, AVPKAXX:VPTKL:SPIS:DSSNNVI:LKYHY:NMVAE:AXGXH, KASKAXX:VPTKL:SPIS:DKGVVTY:LKYHY:GMVAE:EXGXR, GSAGPXX:VPTKM:SPIS:NDKQQII:LKYHY:GMVAE:RXGXS, AAPASXX:VPTRL:SPIS:DAANNVV:LKYHY:DMVAE:AXGXR, STPPTXX:VPTRL:SPIS:DSANNVV:LKYHY:DMVAE:SXGXR, HVPKPXX:VPTKL:SPIS:DDSSNVI:LKYHY:NMVAE:SXGXH, RVPSTXX:VPTKT:SPIS:DNGRVLL:LKYHY:MIVAE:XGXL, ASAAPXX:VPTAL:SPIS:VGRKPKV:LKYHY:MIVAE:XKXS, ASASPXX:VPTDL:SPIS:IGKTPKI:LKYHY:MIVAE:XKXS, ASASPXX:VPTDL:SPIS:VGRTPKV:LKYHY:MVVAE :XKXS, KIPKAXX:VPTEL:SPIS:DENEKVV:LKYHY:DMVVE:GXGXR, GIPEPXX:VPEKM:SPIS:DENKNVV:LKYHY:NMTVE:SXAXR, SIPKAXX:VPTEL:SPIS:DEYDKVV:LKYHY:EMVVE:GXGXR, HVTKPTX:APTKL:SPIS:DDSSNVI: LKYHY:NMVVR:SXGXH, YVPKPXX:APTKL:SPIS:DDSSNVI: LKYHY:NMVVR:SXGXH, TVPKPXX:APTQL:SPIS:DDSSNVI :LKYHY:NMVVR:AXGXH, AVPKAXX:APTKL:SPIS:DSSNNVI:LKYHY:NMVVK:AXGXH, KASKAXX:VPTKL:SPIS :DKGVVTY:LKYHY:GMAVS:EXGXR, GSAGPXX:TPTKM:SPIS:NDKQQII:LKYHY:GMVVD:RXGXS, AAPASXX:VPARL:SPIS:DAANNVV:LKYHY:DMVVE:AXGXR, STPPTXX:VPTRL:SPIS:DSANNVV: LKYHY:DMVVE:SXGXR, HVPKPXX:APTKL:SPIS:DDSSNVI :LKYHY:NMVVR:SXGXH, RVPSTXX:APVKT:SPIS:DNGRVLL:LKYHY:MIVEE:XGXL, ASAAPXX:VPQAL:SPIS:VGRKPKV:LKYHY:MIVRS:XKXS, ASASPXX:VSQDL:SPIS:IGKTPKI:LKYHY:MIVKS:XKXS, ASASPXX:VPQDL:SPIS:VGRTPKV:LKYHY:MVVKS:XKXS, NDEGLEX:VPTEE:SPIS:RIKPHQGQH:LKYHY:FLQHN:KXEXR, NDEGLEX:VPTGQ:SPIS:RIKPHQGQH:LKYHY:LEEHS:QXEXR, SSVKXQP:SRVHH:SPIS:EYVRKKPKL:LKYHY:RLEEH:LEXAXA, RNVQXRP:TQVQL:SPIS:EIVRKKPIF:LKYHY:TLEDH:LAXKXE, KIPKAXX:VPTEL:EPIS:DENEKVV:KFKYE:DMAVS:GXGXR, GIPEPXX:VPTKM:EPIS:DENKNVV:KFKYE:NMAVS:SXAXR, SIPKAXX:VPTEL:EPIS:DEYDKVV:KFKYE:EMAVS:GXGXR, HVTKPTX:VPTKL:EPIS:DDSSNVI: KFKYE:NMAVS:SXGXH, YVPKPXX:VPTKL:EPIS:DDSSNVI:KFKYE:NMAVS:SXGXH, TVPKPXX:VPTQL:EPIS:DDSSNVI:KFKYE:NMAVS:AXGXH, AVPKAXX:VPTKL:EPIS:DSSNNVI:KFKYE:NMAVS:AXGXH, KVGKAXX:VPTKL:EPIS:DDMGVPT:KFKYE:EGMSAVS:XGXR, GSAGPXX:VPTKM:EPIS:NDKQQII:KFKYE:GMAVS:RXGXS, AAPASXX:VPTRL:EPIS:DAANNVV:KFKYE:DMAVS:AXGXR, STPPTXX:VPTRL:EPIS:DSANNVV:KFKYE:DMAVS:SXGXR, HVPKPXX:VPTKL:EPIS:DDSSNVI:KFKYE:NMAVS:SXGXH, RVPSTXX:VPTKT:EPIS:DNGRVLL:KFKYE:MIAVS:XGXL, ASAAPXX:VPTAL:EPIS:VGRKPKV:KFKYE:MIAVS:XKXS, ASASPXX:VPTDL:EPIS:IGKTPKI:KFKYE:MIAVS:XKXS, ASASPXX:VPTDL:EPIS:VGRTPKV:KFKYE:MVAVS:XKXS, KIPKAXX:VPTEL:EPIS:DENEKVV:KFKYE:DMVVE:GXGXR, GIPEPXX:VPEKM:EPIS:DENKNVV:KFKYE:NMTVE:SXAXR, SIPKAXX:VPTEL:EPIS:DEYDKVV:KFKYE:EMVVE:GXGXR, HVTKPTX:APTKL:EPIS:DDSSNVI:KFKYE:NMVVR:SXGXH, YVPKPXX:APTKL:EPIS:DDSSNVI:KFKYE:NMVVR:SXGXH, TVPKPXX:APTQL:EPIS:DDSSNVI:KFKYE:NMVVR:AXGXH, AVPKAXX:APTKL:EPIS:DSSNNVI:KFKYE:NMVVK:AXGXH, KVGKAXX:VPTKL:EPIS:DDMGVPT:KFKYE:EGMSVAE:XGXR, GSAGPXX:TPTKM:EPIS:NDKQQII:KFKYE:GMVVD:RXGXS, AAPASXX:VPARL:EPIS:DAANNVV:KFKYE:DMVVE:AXGXR, STPPTXX:VPTRL:EPIS:DSANNVV:KFKYE:DMVVE:SXGXR, HVPKPXX:APTKL:EPIS:DDSSNVI:KFKYE:NMVVR:SXGXH, RVPSTXX:APVKT:EPIS:DNGRVLL:KFKYE:MIVEE:XGXL, ASAAPXX:VPQAL:EPIS:VGRKPKV:KFKYE:MIVRS:XKXS, ASASPXX:VSQDL:EPIS:IGKTPKI:KFKYE:MIVKS:XKXS, ASASPXX:VPQDL:EPIS:VGRTPKV:KFKYE:MVVKS:XKXS, NDEGLEX:VPTEE:EPIS:RIKPHQGQH:KFKYE:FLQHN:KXEXR, NDEGLEX:VPTGQ:EPIS:RIKPHQGQH:KFKYE:LEEHS:QXEXR, SSVKXQP:SRVHH:EPIS:EYVRKKPKL:KFKYE:RLEEH:LEXAXA, RNVQXRP:TQVQL:EPIS:EIVRKKPIF:KFKYE:TLEDH:LAXKXE, KIPKAXX:TPTEL:SPIN:DENEKVV:YGKIP:DMVVD:GXGXR, GIPEPXX:TPTKM:SPIN:DENKNVV:YGKIP:NMVVD:SXAXR, SIPKAXX:TPTEL:SPIN:DEYDKVV:YGKIP:EMVVD:GXGXR, HVTKPTX:TPTKL:SPIN:DDSSNVI:YGKIP:NMVVD:SXGXH, YVPKPXX:TPTKL:SPIN:DDSSNVI:YGKIP:NMVVD:SXGXH, TVPKPXX:TPTQL:SPIN:DDSSNVI:YGKIP:NMVVD:AXGXH, AVPKAXX:TPTKL:SPIN:DSSNNVI:YGKIP:NMVVD:AXGXH, KVGKAXX:TPTKL:SPIN:DDMGVPT:YGKIP:EGMSVVD:XGXR, KASKAXX:TPTKL:SPIN:DKGVVTY:YGKIP:GMVVD:EXGXR, AAPASXX:TPTRL:SPIN:DAANNVV:YGKIP:DMVVD:AXGXR, STPPTXX:TPTRL:SPIN:DSANNVV:YGKIP:DMVVD:SXGXR, HVPKPXX:TPTKL:SPIN:DDSSNVI:YGKIP:NMVVD:SXGXH, RVPSTXX:TPTKT:SPIN:DNGRVLL:YGKIP:MIVVD:XGXL, ASAAPXX:TPTAL:SPIN:VGRKPKV:YGKIP:MIVVD:XKXS, ASASPXX:TPTDL:SPIN:IGKTPKI:YGKIP:MIVVD:XKXS, ASASPXX:TPTDL:SPIN:VGRTPKV:YGKIP:MVVVD:XKXS, KIPKAXX:VPTEL:SPIN:DENEKVV:YGKIP:DMVVE:GXGXR, GIPEPXX:VPEKM:SPIN:DENKNVV:YGKIP:NMTVE:SXAXR, SIPKAXX:VPTEL:SPIN:DEYDKVV:YGKIP:EMVVE:GXGXR, HVTKPTX:APTKL:SPIN:DDSSNVI:YGKIP:NMVVR:SXGXH, YVPKPXX:APTKL:SPIN:DDSSNVI:YGKIP:NMVVR:SXGXH, TVPKPXX:APTQL:SPIN:DDSSNVI:YGKIP:NMVVR:AXGXH, AVPKAXX:APTKL:SPIN:DSSNNVI:YGKIP:NMVVK:AXGXH, KVGKAXX:VPTKL:SPIN:DDMGVPT:YGKIP:EGMSVAE:XGXR, KASKAXX:VPTKL:SPIN:DKGVVTY:YGKIP:GMAVS:EXGXR, AAPASXX:VPARL:SPIN:DAANNVV:YGKIP:DMVVE:AXGXR, STPPTXX:VPTRL:SPIN:DSANNVV:YGKIP:DMVVE:SXGXR, HVPKPXX:APTKL:SPIN:DDSSNVI:YGKIP:NMVVR:SXGXH, RVPSTXX:APVKT:SPIN:DNGRVLL:YGKIP:MIVEE:XGXL, ASAAPXX:VPQAL:SPIN:VGRKPKV:YGKIP:MIVRS:XKXS, ASASPXX:VSQDL:SPIN:IGKTPKI:YGKIP:MIVKS:XKXS, ASASPXX:VPQDL:SPIN:VGRTPKV:YGKIP:MVVKS:XKXS, NDEGLEX:VPTEE:SPIN:RIKPHQGQH:YGKIP:FLQHN:KXEXR, NDEGLEX:VPTGQ:SPIN:RIKPHQGQH:YGKIP:LEEHS:QXEXR, SSVKXQP:SRVHH:SPIN:EYVRKKPKL:YGKIP:RLEEH:LEXAXA, RNVQXRP:TQVQL:SPIN:EIVRKKPIF:YGKIP:TLEDH:LAXKXE, KIPKAXX:VPAEL:SPIS:DENEKVV:YKQYE:DMVVE:GXGXR, GIPEPXX:VPAKM:SPIS:DENKNVV:YKQYE:NMVVE:SXAXR, SIPKAXX:VPAEL:SPIS:DEYDKVV:YKQYE:EMVVE:GXGXR, HVTKPTX:VPAKL:SPIS:DDSSNVI:YKQYE:NMVVE:SXGXH, YVPKPXX:VPAKL:SPIS:DDSSNVI:YKQYE:NMVVE:SXGXH, TVPKPXX:VPAQL:SPIS:DDSSNVI:YKQYE:NMVVE:AXGXH, AVPKAXX:VPAKL:SPIS:DSSNNVI:YKQYE:NMVVE:AXGXH, KVGKAXX:VPAKL:SPIS:DDMGVPT:YKQYE:EGMSVVE:XGXR, KASKAXX:VPAKL:SPIS:DKGVVTY:YKQYE:GMVVE:EXGXR, GSAGPXX:VPAKM:SPIS:NDKQQII:YKQYE:GMVVE:RXGXS, STPPTXX:VPARL:SPIS:DSANNVV:YKQYE:DMVVE:SXGXR, HVPKPXX:VPAKL:SPIS:DDSSNVI:YKQYE:NMVVE:SXGXH, RVPSTXX:VPAKT:SPIS:DNGRVLL:YKQYE:MIVVE:XGXL, ASAAPXX:VPAAL:SPIS:VGRKPKV:YKQYE:MIVVE:XKXS, ASASPXX:VPADL:SPIS:IGKTPKI:YKQYE:MIVVE:XKXS, ASASPXX:VPADL:SPIS:VGRTPKV:YKQYE:MVVVE:XKXS, KIPKAXX:VPTEL:SPIS:DENEKVV:YKQYE:DMVVE:GXGXR, GIPEPXX:VPEKM:SPIS:DENKNVV:YKQYE:NMTVE:SXAXR, SIPKAXX:VPTEL:SPIS:DEYDKVV:YKQYE:EMVVE:GXGXR, HVTKPTX:APTKL:SPIS:DDSSNVI:YKQYE:NMVVR:SXGXH, YVPKPXX:APTKL:SPIS:DDSSNVI:YKQYE:NMVVR:SXGXH, TVPKPXX:APTQL:SPIS:DDSSNVI:YKQYE:NMVVR:AXGXH, AVPKAXX:APTKL:SPIS:DSSNNVI:YKQYE:NMVVK:AXGXH, KVGKAXX:VPTKL:SPIS:DDMGVPT:YKQYE:EGMSVAE:XGXR, KASKAXX:VPTKL:SPIS :DKGVVTY:YKQYE:GMAVS:EXGXR, GSAGPXX:TPTKM:SPIS:NDKQQII:YKQYE:GMVVD:RXGXS, STPPTXX:VPTRL:SPIS:DSANNVV:YKQYE:DMVVE:SXGXR, HVPKPXX:APTKL:SPIS:DDSSNVI :YKQYE:NMVVR:SXGXH, RVPSTXX:APVKT:SPIS:DNGRVLL:YKQYE:MIVEE:XGXL, ASAAPXX:VPQAL:SPIS:VGRKPKV:YKQYE:MIVRS:XKXS, ASASPXX:VSQDL:SPIS:IGKTPKI:YKQYE:MIVKS:XKXS, ASASPXX:VPQDL:SPIS:VGRTPKV:YKQYE:MVVKS:XKXS, NDEGLEX:VPTEE:SPIS:RIKPHQGQH:YKQYE:FLQHN:KXEXR, NDEGLEX:VPTGQ:SPIS:RIKPHQGQH:YKQYE:LEEHS:QXEXR, SSVKXQP:SRVHH:SPIS:EYVRKKPKL:YKQYE:RLEEH:LEXAXA,
RNVQXRP:TQVQL:SPIS:EIVRKKPIF:YKQYE:TLEDH:LAXKXE, GIPEPXX:VPTKM:SPIS:DENKNVV:YKQYE:NMVVE:SXAXR, HVTKPTX:VPTKL:SPIS:DDSSNVI:YKQYE:NMVVE:SXGXH, YVPKPXX:VPTKL:SPIS:DDSSNVI:YKQYE:NMVVE:SXGXH, TVPKPXX:VPTQL:SPIS:DDSSNVI :YKQYE:NMVVE:AXGXH, AVPKAXX:VPTKL:SPIS:DSSNNVI:YKQYE:NMVVE:AXGXH, KVGKAXX:VPTKL:SPIS:DDMGVPT:YKQYE:EGMSVVE:XGXR, KASKAXX:VPTKL:SPIS :DKGVVTY:YKQYE:GMVVE:EXGXR, GSAGPXX:VPTKM:SPIS:NDKQQII:YKQYE:GMVVE:RXGXS, AAPASXX:VPTRL:SPIS:DAANNVV:YKQYE:DMVVE:AXGXR, HVPKPXX:VPTKL:SPIS:DDSSNVI :YKQYE:NMVVE:SXGXH, RVPSTXX:VPTKT:SPIS:DNGRVLL:YKQYE:MIVVE:XGXL, ASAAPXX:VPTAL:SPIS:VGRKPKV:YKQYE:MIVVE:XKXS, ASASPXX:VPTDL:SPIS:IGKTPKI:YKQYE:MIVVE:XKXS, ASASPXX:VPTDL:SPIS:VGRTPKV:YKQYE:MVVVE:XKXS, AAPASXX:VPARL:SPIS:DAANNVV:YKQYE:DMVVE:AXGXR, KIPKAXX:APVEL:KPLS:DENEKVV:DHHKD:DMVEE:GXGXR, GIPEPXX:APVKM:KPLS:DENKNVV:DHHKD:NMVEE:SXAXR, SIPKAXX:APVEL:KPLS:DEYDKVV:DHHKD:EMVEE:GXGXR, HVTKPTX:APVKL:KPLS:DDSSNVI:DHHKD:NMVEE:SXGXH, YVPKPXX:APVKL:KPLS:DDSSNVI:DHHKD:NMVEE:SXGXH,
TVPKPXX:APVQL:KPLS:DDSSNVI:DHHKD:NMVEE:AXGXH, AVPKAXX:APVKL:KPLS:DSSNNVI:DHHKD:NMVEE:AXGXH, KVGKAXX:APVKL:KPLS:DDMGVPT:DHHKD:EGMSVEE:XGXR, KASKAXX:APVKL:KPLS:DKGVVTY:DHHKD:GMVEE:EXGXR, GSAGPXX:APVKM:KPLS:NDKQQII:DHHKD:GMVEE:RXGXS, AAPASXX:APVRL:KPLS:DAANNVV:DHHKD:DMVEE:AXGXR, STPPTXX:APVRL:KPLS:DSANNVV:DHHKD:DMVEE:SXGXR, HVPKPXX:APVKL:KPLS:DDSSNVI:DHHKD:NMVEE:SXGXH, ASAAPXX:APVAL:KPLS:VGRKPKV:DHHKD:MIVEE:XKXS, ASASPXX:APVDL:KPLS:IGKTPKI:DHHKD:MIVEE:XKXS, ASASPXX:APVDL:KPLS:VGRTPKV:DHHKD:MVVEE:XKXS, KIPKAXX:VPTEL:KPLS:DENEKVV:DHHKD:DMVVE:GXGXR, GIPEPXX:VPEKM:KPLS:DENKNVV:DHHKD:NMTVE:SXAXR, SIPKAXX:VPTEL:KPLS:DEYDKVV:DHHKD:EMVVE:GXGXR, HVTKPTX:APTKL:KPLS:DDSSNVI:DHHKD:NMVVR:SXGXH, YVPKPXX:APTKL:KPLS:DDSSNVI:DHHKD:NMVVR:SXGXH, TVPKPXX:APTQL:KPLS:DDSSNVI:DHHKD:NMVVR:AXGXH, AVPKAXX:APTKL:KPLS:DSSNNVI:DHHKD:NMVVK:AXGXH, KVGKAXX:VPTKL:KPLS:DDMGVPT:DHHKD:EGMSVAE:XGXR, KASKAXX:VPTKL:KPLS:DKGVVTY:DHHKD:GMAVS:EXGXR, GSAGPXX:TPTKM:KPLS:NDKQQII:DHHKD:GMVVD:RXGXS, AAPASXX:VPARL:KPLS:DAANNVV:DHHKD:DMVVE:AXGXR, STPPTXX:VPTRL:KPLS:DSANNVV:DHHKD:DMVVE:SXGXR, HVPKPXX:APTKL:KPLS:DDSSNVI:DHHKD:NMVVR:SXGXH, ASAAPXX:VPQAL:KPLS:VGRKPKV:DHHKD:MIVRS:XKXS, ASASPXX:VSQDL:KPLS:IGKTPKI:DHHKD:MIVKS:XKXS, ASASPXX:VPQDL:KPLS:VGRTPKV:DHHKD:MVVKS:XKXS, NDEGLEX:VPTEE:KPLS:RIKPHQGQH:DHHKD:FLQHN:KXEXR, NDEGLEX:VPTGQ:KPLS:RIKPHQGQH:DHHKD:LEEHS:QXEXR, SSVKXQP:SRVHH:KPLS:EYVRKKPKL:DHHKD:RLEEH:LEXAXA, RNVQXRP:TQVQL:KPLS:EIVRKKPIF:DHHKD:TLEDH:LAXKXE, KIPKAXX:VPQEL:EPLP:DENEKVV:EQLSN:DMVRS:GXGXR, GIPEPXX:VPQKM:EPLP:DENKNVV:EQLSN:NMVRS:SXAXR, SIPKAXX:VPQEL:EPLP:DEYDKVV:EQLSN:EMVRS:GXGXR, HVTKPTX:VPQKL:EPLP:DDSSNVI:EQLSN:NMVRS:SXGXH, YVPKPXX:VPQKL:EPLP:DDSSNVI:EQLSN:NMVRS:SXGXH, TVPKPXX:VPQQL:EPLP:DDSSNVI:EQLSN:NMVRS:AXGXH, AVPKAXX:VPQKL:EPLP:DSSNNVI:EQLSN:NMVRS:AXGXH, KVGKAXX:VPQKL:EPLP:DDMGVPT:EQLSN:EGMSVRS:XGXR, KASKAXX:VPQKL:EPLP:DKGVVTY:EQLSN:GMVRS:EXGXR, GSAGPXX:VPQKM:EPLP:NDKQQII:EQLSN:GMVRS:RXGXS, AAPASXX:VPQRL:EPLP:DAANNVV:EQLSN:DMVRS:AXGXR, STPPTXX:VPQRL:EPLP:DSANNVV:EQLSN:DMVRS:SXGXR, HVPKPXX:VPQKL:EPLP:DDSSNVI:EQLSN:NMVRS:SXGXH, RVPSTXX:VPQKT:EPLP:DNGRVLL:EQLSN:MIVRS:XGXL, ASASPXX:VPQDL:EPLP:IGKTPKI:EQLSN:MIVRS:XKXS, ASASPXX:VPQDL:EPLP:VGRTPKV:EQLSN:MVVRS:XKXS, KIPKAXX:VPTEL:EPLP:DENEKVV:EQLSN:DMVVE:GXGXR, GIPEPXX:VPEKM:EPLP:DENKNVV:EQLSN:NMTVE:SXAXR, SIPKAXX:VPTEL:EPLP:DEYDKVV:EQLSN:EMVVE:GXGXR, HVTKPTX:APTKL:EPLP:DDSSNVI:EQLSN:NMVVR:SXGXH, YVPKPXX:APTKL:EPLP:DDSSNVI:EQLSN:NMVVR:SXGXH, TVPKPXX:APTQL:EPLP:DDSSNVI:EQLSN:NMVVR:AXGXH, AVPKAXX:APTKL:EPLP:DSSNNVI:EQLSN:NMVVK:AXGXH, KVGKAXX:VPTKL:EPLP:DDMGVPT:EQLSN:EGMSVAE:XGXR, KASKAXX:VPTKL:EPLP:DKGVVTY:EQLSN:GMAVS:EXGXR, GSAGPXX:TPTKM:EPLP:NDKQQII:EQLSN:GMVVD:RXGXS, AAPASXX:VPARL:EPLP:DAANNVV:EQLSN:DMVVE:AXGXR, STPPTXX:VPTRL:EPLP:DSANNVV:EQLSN:DMVVE:SXGXR, HVPKPXX:APTKL:EPLP:DDSSNVI:EQLSN:NMVVR:SXGXH, RVPSTXX:APVKT :EPLP:DNGRVLL:EQLSN:MIVEE:XGXL, ASASPXX:VSQDL:EPLP:IGKTPKI:EQLSN:MIVKS:XKXS, ASASPXX:VPQDL:EPLP:VGRTPKV:EQLSN:MVVKS:XKXS, NDEGLEX:VPTEE:EPLP:RIKPHQGQH:EQLSN:FLQHN:KXEXR, NDEGLEX:VPTGQ:EPLP:RIKPHQGQH:EQLSN:LEEHS:QXEXR, SSVKXQP:SRVHH:EPLP:EYVRKKPKL:EQLSN:RLEEH:LEXAXA, RNVQXRP:TQVQL:EPLP:EIVRKKPIF:EQLSN:TLEDH:LAXKXE, KIPKAXX:VSQEL:EPLT:DENEKVV:EQLSN:DMVKS:GXGXR, GIPEPXX:VSQKM:EPLT:DENKNVV:EQLSN:NMVKS:SXAXR, SIPKAXX:VSQEL:EPLT:DEYDKVV:EQLSN:EMVKS:GXGXR, HVTKPTX:VSQKL:EPLT:DDSSNVI:EQLSN:NMVKS:SXGXH, YVPKPXX:VSQKL:EPLT:DDSSNVI:EQLSN:NMVKS:SXGXH, TVPKPXX:VSQQL:EPLT:DDSSNVI:EQLSN:NMVKS:AXGXH, AVPKAXX:VSQKL:EPLT:DSSNNVI:EQLSN:NMVKS:AXGXH, KVGKAXX:VSQKL:EPLT:DDMGVPT:EQLSN:EGMSVKS:XGXR, KASKAXX:VSQKL:EPLT:DKGVVTY:EQLSN:GMVKS:EXGXR, GSAGPXX:VSQKM:EPLT:NDKQQII:EQLSN:GMVKS:RXGXS, AAPASXX:VSQRL:EPLT:DAANNVV:EQLSN:DMVKS:AXGXR, STPPTXX:VSQRL:EPLT:DSANNVV:EQLSN:DMVKS:SXGXR, HVPKPXX:VSQKL:EPLT:DDSSNVI:EQLSN:NMVKS:SXGXH, RVPSTXX:VSQKT:EPLT:DNGRVLL:EQLSN:MIVKS:XGXL, ASAAPXX:VSQAL:EPLT:VGRKPKV:EQLSN:MIVKS:XKXS, ASASPXX:VSQDL:EPLT:VGRTPKV:EQLSN:MVVKS:XKXS, KIPKAXX:VPTEL:EPLT:DENEKVV:EQLSN:DMVVE:GXGXR, GIPEPXX:VPEKM:EPLT:DENKNVV:EQLSN:NMTVE:SXAXR, SIPKAXX:VPTEL:EPLT:DEYDKVV:EQLSN:EMVVE:GXGXR, HVTKPTX:APTKL:EPLT:DDSSNVI:EQLSN:NMVVR:SXGXH, YVPKPXX:APTKL:EPLT:DDSSNVI:EQLSN:NMVVR:SXGXH, TVPKPXX:APTQL:EPLT:DDSSNVI:EQLSN:NMVVR:AXGXH, AVPKAXX:APTKL:EPLT:DSSNNVI:EQLSN:NMVVK:AXGXH, KVGKAXX:VPTKL:EPLT:DDMGVPT:EQLSN:EGMSVAE:XGXR, KASKAXX:VPTKL:EPLT:DKGVVTY:EQLSN:GMAVS:EXGXR, GSAGPXX:TPTKM:EPLT:NDKQQII:EQLSN:GMVVD:RXGXS, AAPASXX:VPARL:EPLT:DAANNVV:EQLSN:DMVVE:AXGXR, STPPTXX:VPTRL:EPLT:DSANNVV:EQLSN:DMVVE:SXGXR, HVPKPXX:APTKL:EPLT:DDSSNVI:EQLSN:NMVVR:SXGXH, RVPSTXX:APVKT:EPLT:DNGRVLL:EQLSN:MIVEE:XGXL, ASAAPXX:VPQAL:EPLT:VGRKPKV:EQLSN:MIVRS:XKXS, NDEGLEX:VPTEE:EPLT:RIKPHQGQH:EQLSN:FLQHN:KXEXR, NDEGLEX:VPTGQ:EPLT:RIKPHQGQH:EQLSN:LEEHS:QXEXR, SSVKXQP:SRVHH:EPLT:EYVRKKPKL:EQLSN:RLEEH:LEXAXA, RNVQXRP:TQVQL:EPLT:EIVRKKPIF:EQLSN:TLEDH:LAXKXE, KIPKAXX:VPQEL:EPLT:DENEKVV:EQLSN:DMVKS:GXGXR, GIPEPXX:VPQKM:EPLT:DENKNVV:EQLSN:NMVKS:SXAXR, SIPKAXX:VPQEL:EPLT:DEYDKVV:EQLSN:EMVKS:GXGXR, HVTKPTX:VPQKL:EPLT:DDSSNVI:EQLSN:NMVKS:SXGXH, YVPKPXX:VPQKL:EPLT:DDSSNVI:EQLSN:NMVKS:SXGXH, TVPKPXX:VPQQL:EPLT:DDSSNVI:EQLSN:NMVKS:AXGXH, AVPKAXX:VPQKL:EPLT:DSSNNVI:EQLSN:NMVKS:AXGXH, KVGKAXX:VPQKL:EPLT:DDMGVPT:EQLSN:EGMSVKS:XGXR, KASKAXX:VPQKL:EPLT:DKGVVTY:EQLSN:GMVKS:EXGXR, GSAGPXX:VPQKM:EPLT:NDKQQII:EQLSN:GMVKS:RXGXS, AAPASXX:VPQRL:EPLT:DAANNVV:EQLSN:DMVKS:AXGXR, STPPTXX:VPQRL:EPLT:DSANNVV:EQLSN:DMVKS:SXGXR,

HVPKPXX:VPQKL:EPLT:DDSSNVI:EQLSN:NMVKS:SXGXH, RVPSTXX:VPQKT:EPLT:DNGRVLL:EQLSN:MIVKS:XGXL, ASAAPXX:VPQAL:EPLT:VGRKPKV:EQLSN:MIVKS:XKXS, ASASPXX:VPQDL:EPLT:IGKTPKI:EQLSN:MIVKS:XKXS, NDEGLEX:VPTGQ:SNIT:RIKPHQGQH:IGEMS:LEQHN:QXEXR, KIPKAXX:VPTEL:SNIT:DENEKVV:IGEMS:DMVVE:GXGXR, GIPEPXX:VPEKM:SNIT:DENKNVV:IGEMS:NMTVE:SXAXR, SIPKAXX:VPTEL:SNIT:DEYDKVV:IGEMS:EMVVE:GXGXR, HVTKPTX:APTKL:SNIT:DDSSNVI: IGEMS:NMVVR:SXGXH, YVPKPXX:APTKL:SN IT:DDSSNVI:IGEMS:NMVVR:SXGXH, TVPKPXX:APTQL:SNIT:DDSSNVI:IGEMS:NMVVR:AXGXH, AVPKAXX:APTKL:SNIT:DSSNNVI:IGEMS:NMVVK:AXGXH, KVGKAXX:VPTKL:SNIT:DDMGVPT:IGEMS:EGMSVAE:XGXR, KASKAXX:VPTKL:SNIT:DKGVVTY:IGEMS:GMAVS:EXGXR, GSAGPXX:TPTKM:SNIT:NDKQQII:IGEMS:GMVVD:RXGXS, AAPASXX:VPARL:SNIT:DAANNVV:IGEMS:DMVVE:AXGXR, STPPTXX:VPTRL:SNIT:DSANNVV:IGEMS:DMVVE:SXGXR, HVPKPXX:APTKL:SNIT:DDSSNVI:IGEMS:NMVVR:SXGXH, RVPSTXX:APVKT:SNIT:DNGRVLL:IGEMS:MIVEE:XGXL, ASAAPXX:VPQAL:SNIT:VGRKPKV:IGEMS:MIVRS:XKXS, ASASPXX:VSQDL:SNIT:IGKTPKI:IGEMS:MIVKS:XKXS, ASASPXX:VPQDL:SNIT:VGRTPKV:IGEMS:MVVKS:XKXS, NDEGLEX:VPTGQ:SNIT:RIKPHQGQH:IGEMS:LEEHS:QXEXR, SSVKXQP:SRVHH:SNIT:EYVRKKPKL:IGEMS:RLEEH:LEXAXA, RNVQXRP:TQVQL:SNIT:EIVRKKPIF:IGEMS:TLEDH:LAXKXE, NDEGLEX:VPTEE:SNIT:RIKPHQGQH:LGEMS:FLEHS:KXEXR, KIPKAXX:VPTEL:SNIT:DENEKVV:LGEMS:DMVVE:GXGXR, GIPEPXX:VPEKM:SNIT:DENKNVV:LGEMS:NMTVE:SXAXR, SIPKAXX:VPTEL:SNIT:DEYDKVV:LGEMS:EMVVE:GXGXR, HVTKPTX:APTKL:SNIT:DDSSNVI: LGEMS:NMVVR:SXGXH, YVPKPXX:APTKL:SN IT:DDSSNVI:LGEMS:NMVVR:SXGXH, TVPKPXX:APTQL:SNIT:DDSSNVI:LGEMS:NMVVR:AXGXH, AVPKAXX:APTKL:SNIT:DSSNNVI:LGEMS:NMVVK:AXGXH, KVGKAXX:VPTKL:SNIT:DDMGVPT:LGEMS:EGMSVAE:XGXR, KASKAXX:VPTKL:SNIT:DKGVVTY:LGEMS:GMAVS:EXGXR, GSAGPXX:TPTKM:SNIT:NDKQQII:LGEMS:GMVVD:RXGXS, AAPASXX:VPARL:SNIT:DAANNVV:LGEMS:DMVVE:AXGXR, STPPTXX:VPTRL:SNIT:DSANNVV: LGEMS:DMVVE:SXGXR, HVPKPXX:APTKL:SNIT:DDSSNVI:LGEMS:NMVVR:SXGXH, RVPSTXX:APVKT:SNIT:DNGRVLL:LGEMS:MIVEE:XGXL, ASAAPXX:VPQAL:SNIT:VGRKPKV:LGEMS:MIVRS:XKXS, ASASPXX:VSQDL:SNIT:IGKTPKI:LGEMS:MIVKS:XKXS, ASASPXX:VPQDL:SNIT:VGRTPKV:LGEMS:MVVKS:XKXS, NDEGLEX:VPTEE:SNIT:RIKPHQGQH:LGEMS:FLQHN:KXEXR, SSVKXQP:SRVHH:SNIT:EYVRKKPKL:LGEMS:RLEEH:LEXAXA, RNVQXRP:TQVQL:SNIT:EIVRKKPIF:LGEMS:TLEDH:LAXKXE, RNVQXRP:SRVQL:RSVK:EIVRKKPIF:KEVQV:TLEEH:LAXKXE, KIPKAXX:VPTEL:RSVK:DENEKVV:KEVQV:DMVVE:GXGXR, GIPEPXX:VPEKM:RSVK:DENKNVV:KEVQV:NMTVE:SXAXR, SIPKAXX:VPTEL:RSVK:DEYDKVV:KEVQV:EMVVE:GXGXR, HVTKPTX:APTKL:RSVK:DDSSNVI:KEVQV:NMVVR:SXGXH, YVPKPXX:APTKL:RSVK:DDSSNVI:KEVQV:NMVVR:SXGXH, TVPKPXX:APTQL:RSVK:DDSSNVI:KEVQV:NMVVR:AXGXH, AVPKAXX:APTKL:RSVK:DSSNNVI:KEVQV:NMVVK:AXGXH, KVGKAXX:VPTKL:RSVK:DDMGVPT:KEVQV:EGMSVAE:XGXR, KASKAXX:VPTKL:RSVK:DKGVVTY:KEVQV:GMAVS:EXGXR, GSAGPXX:TPTKM:RSVK:NDKQQII:KEVQV:GMVVD:RXGXS, AAPASXX:VPARL:RSVK:DAANNVV:KEVQV:DMVVE:AXGXR, STPPTXX:VPTRL:RSVK:DSANNVV:KEVQV:DMVVE:SXGXR, HVPKPXX:APTKL:RSVK:DDSSNVI:KEVQV:NMVVR:SXGXH, RVPSTXX:APVKT:RSVK:DNGRVLL:KEVQV:MIVEE:XGXL, ASAAPXX:VPQAL:RSVK:VGRKPKV:KEVQV:MIVRS:XKXS, ASASPXX:VSQDL:RSVK:IGKTPKI:KEVQV:MIVKS:XKXS, ASASPXX:VPQDL:RSVK:VGRTPKV:KEVQV:MVVKS:XKXS, NDEGLEX:VPTEE:RSVK:RIKPHQGQH:KEVQV:FLQHN:KXEXR, NDEGLEX:VPTGQ:RSVK:RIKPHQGQH:KEVQV:LEEHS:QXEXR, RNVQXRP:TQVQL:RSVK:EIVRKKPIF:KEVQV:TLEDH:LAXKXE, SSVKXQP:TQVHH:RPVQ:EYVRKKPKL:KKATV:RLEDH:LEXAXA, KIPKAXX:VPTEL:RPVQ:DENEKVV:KKATV:DMVVE:GXGXR, GIPEPXX:VPEKM:RPVQ:DENKNVV:KKATV:NMTVE:SXAXR, SIPKAXX:VPTEL:RPVQ:DEYDKVV:KKATV:EMVVE:GXGXR, HVTKPTX:APTKL:RPVQ:DDSSNVI: KKATV:NMVVR:SXGXH, YVPKPXX:APTKL:RPVQ:DDSSNVI:KKATV:NMVVR:SXGXH, TVPKPXX:APTQL:RPVQ:DDSSNVI:KKATV:NMVVR:AXGXH, AVPKAXX:APTKL:RPVQ:DSSNNVI:KKATV:NMVVK:AXGXH, KVGKAXX:VPTKL:RPVQ:DDMGVPT:KKATV:EGMSVAE:XGXR, KASKAXX:VPTKL:RPVQ:DKGVVTY:KKATV:GMAVS:EXGXR, GSAGPXX:TPTKM:RPVQ:NDKQQII:KKATV:GMVVD:RXGXS, AAPASXX:VPARL:RPVQ:DAANNVV:KKATV:DMVVE:AXGXR, STPPTXX:VPTRL:RPVQ:DSANNVV: KKATV:DMVVE:SXGXR, HVPKPXX:APTKL:RPVQ:DDSSNVI:KKATV:NMVVR:SXGXH, RVPSTXX:APVKT:RPVQ:DNGRVLL:KKATV:MIVEE:XGXL, ASAAPXX:VPQAL:RPVQ:VGRKPKV:KKATV:MIVRS:XKXS, ASASPXX:VSQDL:RPVQ:IGKTPKI:KKATV:MIVKS:XKXS, ASASPXX:VPQDL:RPVQ:VGRTPKV:KKATV:MVVKS:XKXS, NDEGLEX:VPTEE:RPVQ:RIKPHQGQH:KKATV:FLQHN:KXEXR, NDEGLEX:VPTGQ:RPVQ:RIKPHQGQH:KKATV:LEEHS:QXEXR and SSVKXQP:SRVHH:RPVQ:EYVRKKPKL:KKATV:RLEEH:LEXAXA.

In particular, in certain embodiments, the heptuplet PEP7:PEP5:PEP12:LINKER:PEP2:PEP6:PEP8 is selected from the group consisting of GIPEPXX:VPTKM:SAIS-AA$^{17}$-LYL:DENKNVV:LKNYQ:NMVVE:SXAXR, SIPKAXX:VPTEL:SAIS-AA$^{17}$-LYL:DEYDKVV:LKNYQ:EMVVE:GXGXR, HVTKPTX:VPTKL:SAIS-AA$^{17}$-LYL:DDSSNVI:LKNYQ:NMVVE:SXGXH, YVPKPXX:VPTKL:SAIS-AA$^{17}$-LYL:DDSSNVI:LKNYQ:NMVVE:SXGXH, TVPKPXX:VPTQL:SAIS-AA$^{17}$-LYL:DDSSNVI:LKNYQ:NMVVE:AXGXH, AVPKAXX:VPTKL:SAIS-AA$^{17}$-LYL:DSSNN-

VI:LKNYQ:NMVVE:AXGXH, KVGKAXX:VPTKL:SAIS-AA$^{17}$-LYL:DDMGVPT:LKNYQ:EGMSVVE:XGXR, KASKAXX:VPTKL:SAIS-AA$^{17}$-LYL:DKGVVTY:LKNYQ:GMVVE:EXGXR, GSAGPXX:VPTKM:SAIS-AA$^{17}$-LYL:NDKQQII:LKNYQ:GMVVE:RXGXS, AAPASXX:VPTRL:SAIS-AA$^{17}$-LYL:DAANNVV:LKNYQ:DMVVE:AXGXR, STPPTXX:VPTRL:SAIS-AA$^{17}$-LYL:DSANNVV:LKNYQ:DMVVE:SXGXR, HVPKPXX:VPTKL:SAIS-AA$^{17}$-LYL:DDSSNVI:LKNYQ:NMVVE:SXGXH, RVPSTXX:VPTKT:SAIS-AA$^{17}$-LYL:DNGRVLL:LKNYQ:MIVVE:XGXL, ASAAPXX:VPTAL:SAIS-AA$^{17}$-LYL:VGRKPKV:LKNYQ:MIVVE:XKXS, ASASPXX:VPTDL:SAIS-AA$^{17}$-LYL:IGKTPKI:LKNYQ:MIVVE:XKXS, ASASPXX:VPTDL:SAIS-AA$^{17}$-LYL:VGRTPKV:LKNYQ:MVVVE:XKXS, NDEGLEX:VPTEE:SAIS-AA$^{17}$-LYL:RIKPHQGQH:LKNYQ:FLVVE:KXEXR, NDEGLEX:VPTGQ:SAIS-AA$^{17}$-LYL:RIKPHQGQH:LKNYQ:LEVVE:QXEXR, SSVKXQP:VPTHH:SAIS-AA$^{17}$-LYL:EYVRKKPKL:LKNYQ:RLVVE:LEXAXA, RNVQXRP:VPTQL:SAIS-AA$^{17}$-LYL:EIVRKKPIF:LKNYQ:TLVVE:LAXKXE, GIPEPXX:VPEKM:SAIS-AA$^{17}$-LYL:DENKNVV:LKNYQ:NMTVE:SXAXR, HVTKPTX:APTKL:SAIS-AA$^{17}$-LYL:DDSSNVI:LKNYQ:NMVVR:SXGXH, YVPKPXX:APTKL:SAIS-AA$^{17}$-LYL:DDSSNVI:LKNYQ:NMVVR:SXGXH, TVPKPXX:APTQL:SAIS-AA$^{17}$-LYL:DDSSNVI:LKNYQ:NMVVR:AXGXH, AVPKAXX:APTKL:SAIS-AA$^{17}$-LYL:DSSNNVI:LKNYQ:NMVVK:AXGXH, KVGKAXX:VPTKL:SAIS-AA$^{17}$-LYL:DDMGVPT:LKNYQ:EGMSVAE:XGXR, KASKAXX:VPTKL:SAIS-AA$^{17}$-LYL:DKGVVTY:LKNYQ:GMAVS:EXGXR, GSAGPXX:TPTKM:SAIS-AA$^{17}$-LYL:NDKQQII:LKNYQ:GMVVD:RXGXS, AAPASXX:VPARL:SAIS-AA$^{17}$-LYL:DAANNVV:LKNYQ:DMVVE:AXGXR, HVPKPXX:APTKL:SAIS-AA$^{17}$-LYL:DDSSNVI:LKNYQ:NMVVR:SXGXH, RVPSTXX:APVKT:SAIS-AA$^{17}$-LYL:DNGRVLL:LKNYQ:MIVEE:XGXL, ASAAPXX:VPQAL:SAIS-AA$^{17}$-LYL:VGRKPKV:LKNYQ:MIVRS:XKXS, ASASPXX:VSQDL:SAIS-AA$^{17}$-LYL:IGKTPKI:LKNYQ:MIVKS:XKXS, ASASPXX:VPQDL:SAIS-AA$^{17}$-LYL:VGRTPKV:LKNYQ:MVVKS:XKXS, NDEGLEX:VPTEE:SAIS-AA$^{17}$-LYL:RIKPHQGQH:LKNYQ:FLQHN:KXEXR, NDEGLEX:VPTGQ:SAIS-AA$^{17}$-LYL:RIKPHQGQH:LKNYQ:LEEHS:QXEXR, SSVKXQP:SRVHH:SAIS-AA$^{17}$-LYL:EYVRKKPKL:LKNYQ:RLEEH:LEXAXA, RNVQXRP:TQVQL:SAIS-AA$^{17}$-LYL:EIVRKKPIF:LKNYQ:TLEDH:LAXKXE, KIPKAXX:VPEEL:SSLS-AA$^{17}$-LFF:DENEKVV:LKVYP:DMTVE:GXGXR, SIPKAXX:VPEEL:SSLS-AA$^{17}$-LFF:DEYDKVV:LKVYP:EMTVE:GXGXR, HVTKPTX:VPEKL:SSLS-AA$^{17}$-LFF:DDSSNVI:LKVYP:NMTVE:SXGXH, YVPKPXX:VPEKL:SSLS-AA$^{17}$-LFF:DDSSNVI:LKVYP:NMTVE:SXGXH, TVPKPXX:VPEQL:SSLS-AA$^{17}$-LFF:DDSSNVI:LKVYP:NMTVE:AXGXH, AVPKAXXVPEKL:SSLS-AA$^{17}$-LFF:DSSNNVI:LKVYP:NMTVE:AXGXH, KVGKAXX:VPEKL:SSLS-AA$^{17}$-LFF:DDMGVPT:LKVYP:EGMSTVE:XGXR, KASKAXX:VPEKL:SSLS-AA$^{17}$-LFF:DKGVVTY:LKVYP:GMTVE:EXGXR, GSAGPXX:VPEKM:SSLS-AA$^{17}$-LFF:NDKQQII:LKVYP:GMTVE:RXGXS, AAPASXX:VPERL:SSLS-AA$^{17}$-LFF:DAANNVV:LKVYP:DMTVE:AXGXR, STPPTXX:VPERL:SSLS-AA$^{17}$-LFF:DSANNVV:LKVYP:DMTVE:SXGXR, HVPKPXX:VPEKL:SSLS-AA$^{17}$-LFF:DDSSNVI:LKVYP:NMTVE:SXGXH, RVPSTXX:VPEKT:SSLS-AA$^{17}$-LFF:DNGRVLL:LKVYP:MITVE:XGXL, ASAAPXX:VPEAL:SSLS-AA$^{17}$-LFF:VGRKPKV:LKVYP:MITVE:XKXS, ASASPXX:VPEDL:SSLS-AA$^{17}$-LFF:IGKTPKI:LKVYP:MITVE:XKXS, ASASPXX:VPEDL:SSLS-AA$^{17}$-LFF:VGRTPKV:LKVYP:MVTVE:XKXS, NDEGLEX:VPEEE:SSLS-AA$^{17}$-LFF:RIKPHQGQH:LKVYP:FLTVE:KXEXR, NDEGLEX:VPEGQ:SSLS-AA$^{17}$-LFF:RIKPHQGQH:LKVYP:LETVE:QXEXR, SSVKXQPVPEHH:SSLS-AA$^{17}$-LFF:EYVRKKPKL:LKVYP:RLTVE:LEXAXA, RNVQXRP:VPEQL:SSLS-AA$^{17}$-LFF:EIVRKKPIF:LKVYP:TLTVE:LAXKXE, KIPKAXX:VPTEL:SSLS-AA$^{17}$-LFF:DENEKVV:LKVYP:DMVVE:GXGXR, SIPKAXX:VPTEL:SSLS-AA$^{17}$-LFF:DEYDKVV:LKVYP:EMVVE:GXGXR, HVTKPTX:APTKL:SSLS-AA$^{17}$-LFF:DDSSNVI:LKVYP:NMVVR:SXGXH, YVPKPXX:APTKL:SSLS-AA$^{17}$-LFF:DDSSNVI:LKVYP:NMVVR:SXGXH, TVPKPXX:APTQL:SSLS-AA$^{17}$-LFF:DDSSNVI:LKVYP:NMVVR:AXGXH, AVPKAXX:APTKL:SSLS-AA$^{17}$-LFF:DSSNNVI:LKVYP:NMVVK:AXGXH, KVGKAXX:VPTKL:SSLS-AA$^{17}$-LFF:DDMGVPT:LKVYP:EGMSVAE:XGXR, KASKAXX:VPTKL:SSLS-AA$^{17}$-LFF:DKGVVTY:LKVYP:GMAVS:EXGXR, GSAGPXX:TPTKM:SSLS-AA$^{17}$-LFF:NDKQQII:LKVYP:GMVVD:RXGXS, AAPASXX:VPARL:SSLS-AA$^{17}$-LFF:DAANNVV:LKVYP:DMVVE:AXGXR, STPPTXX:VPTRL:SSLS-AA$^{17}$-LFF:DSANNVV:LKVYP:DMVVE:SXGXR, HVPKPXX:APTKL:SSLS-AA$^{17}$-LFF:DDSSNVI:LKVYP:NMVVR:SXGXH, RVPSTXX:APVKT:SSLS-AA$^{17}$-LFF:DNGRVLL:LKVYP:MIVEE:XGXL, ASAAPXX:VPQAL:SSLS-AA$^{17}$-LFF:VGRKPKV:LKVYP:MIVRS:XKXS, ASASPXX:VSQDL:SSLS-AA$^{17}$-LFF:IGKTPKI:LKVYP:MIVKS:XKXS, ASASPXX:VPQDL:SSLS-AA$^{17}$-LFF:VGRTPKV:LKVYP:MVVKS:XKXS, NDEGLEX:VPTEE:SSLS-AA$^{17}$-LFF:RIKPHQGQH:LKVYP:FLQHN:KXEXR, NDEGLEX:VPTGQ:SSLS-AA$^{17}$-LFF:RIKPHQGQH:LKVYP:LEEHS:QXEXR, SSVKXQP:SRVHH:SSLS-AA$^{17}$-LFF:EYVRKKPKL:LKVYP:RLEEH:LEXAXA, RNVQXRP:TQVQL:SSLS-AA$^{17}$-LFF:EIVRKKPIF:LKVYP:TLEDH:LAXKXE, KIPKAXX:VPTEL:SAIS-AA$^{17}$-LYL:DENEKVV:LKNYQ:DMVVE:GXGXR, KIPKAXX:APTEL:NAIS-AA$^{17}$-LYF:DENEKVV:LKKYR:DMVVR:GXGXR, GIPEPXX:APTKM:NAIS-AA$^{17}$-LYF:DENKNVV:LKKYR:NMVVR:SXAXR, SIPKAXX:APTEL:NAIS-AA$^{17}$-LYF:DEYDKVV:LKKYR:EMVVR:GXGXR, YVPKPXX:APTKL:NAIS-AA$^{17}$-LYF:DDSSNVI:LKKYR:NMVVR:SXGXH, TVPKPXX:APTQL:NAIS-AA$^{17}$-LYF:DDSSNVI:LKKYR:NMVVR:AXGXH, AVPKAXX:APTKL:NAIS-AA$^{17}$-LYF:DSSNNVI:LKKYR:NMVVR:AXGXH, KVGKAXX:APTKL:NAIS-AA$^{17}$-LYF:DDMGVPT:LKKYR:EGMSVVR:XGXR, KASKAXX:APTKL:NAIS-AA$^{17}$-LYF:DKGVVTY:LKKYR:GMVVR:EXGXR, GSAGPXX:APTKM:NAIS-AA$^{17}$-LYF:NDKQQII:LKKYR:GMVVR:RXGXS, AAPASXX:APTRL:NAIS-AA$^{17}$-LYF:DAANNVV:LKKYR:DMVVR:AXGXR, STPPTXX:APTRL:NAIS-AA$^{17}$-LYF:DSANNVV:LKKYR:DMVVR:SXGXR, HVPKPXX:APTKL:NAIS-AA$^{17}$-LYF:DDSSNVI:LKKYR:NMVVR:SXGXH, RVPSTXX:APTKT:NAIS-AA$^{17}$-LYF:DNGRVLL:LKKYR:MIVVR:XGXL,

ASAAPXX:APTAL:NAIS-AA$^{17}$-LYF:VGRKPKV:LKKYR:MIVVR:XKXS, ASASPXX:APTDL:NAIS-AA$^{17}$-LYF:IGKTPKI:LKKYR:MIVVR:XKXS, ASASPXX:APTDL:NAIS-AA$^{17}$-LYF:VGRTPKV:LKKYR:MVVVR:XKXS, NDEGLEX:APTEE:NAIS-AA$^{17}$-LYF:RIKPHQGQH:LKKYR:FLVVR:KXEXR, NDEGLEX:APTGQ:NAIS-AA$^{17}$-LYF:RIKPHQGQH:LKKYR:LEVVR:QXEXR, SSVKXQP:APTHH:NAIS-AA$^{17}$-LYF:EYVRKKPKL:LKKYR:RLVVR:LEXAXA, RNVQXRP:APTQL:NAIS-AA$^{17}$-LYF:EIVRKKPIF:LKKYR:TLVVR:LAXKXE, KIPKAXX:VPTEL:NAIS-AA$^{17}$-LYF:DENEKVV:LKKYR:DMVVE:GXGXR, GIPEPXX:VPEKM:NAIS-AA$^{17}$-LYF:DENKNVV:LKKYR:NMTVE:SXAXR, SIPKAXX:VPTEL:NAIS-AA$^{17}$-LYF:DEYDKVV:LKKYR:EMVVE:GXGXR, AVPKAXX:APTKL:NAIS-AA$^{17}$-LYF:DSSNNVI:LKKYR:NMVVK:AXGXH, KVGKAXX:VPTKL:NAIS-AA$^{17}$-LYF:DDMGVPT:LKKYR:EGMSVAE:XGXR, KASKAXX:VPTKL:NAIS-AA$^{17}$-LYF:DKGVVTY:LKKYR:GMAVS:EXGXR, GSAGPXX:TPTKM:NAIS-AA$^{17}$-LYF:NDKQQII:LKKYR:GMVVD:RXGXS, AAPASXX:VPARL:NAIS-AA$^{17}$-LYF:DAANNVV:LKKYR:DMVVE:AXGXR, STPPTXX:VPTRL:NAIS-AA$^{17}$-LYF:DSANNVV:LKKYR:DMVVE:SXGXR, RVPSTXX:APVKT:NAIS-AA$^{17}$-LYF:DNGRVLL:LKKYR:MIVEE:XGXL, ASAAPXX:VPQAL:NAIS-AA$^{17}$-LYF:VGRKPKV:LKKYR:MIVRS:XKXS, ASASPXX:VSQDL:NAIS-AA$^{17}$-LYF:IGKTPKI:LKKYR:MIVKS:XKXS, ASASPXX:VPQDL:NAIS-AA$^{17}$-LYF:VGRTPKV:LKKYR:MVVKS:XKXS, NDEGLEX:VPTEE:NAIS-AA$^{17}$-LYF:RIKPHQGQH:LKKYR:FLQHN:KXEXR, NDEGLEXVPTGQ:NAIS-AA$^{17}$-LYF:RIKPHQGQH:LKKYR:LEEHS:QXEXR, SSVKXQP:SRVHH:NAIS-AA$^{17}$-LYF:EYVRKKPKL:LKKYR:RLEEH:LEXAXA, RNVQXRP:TQVQL:NAIS-AA$^{17}$-LYF:EIVRKKPIF:LKKYR:TLEDH:LAXKXE, HVTKPTX:APTKL:NAIS-AA$^{17}$-LYF:DDSSNVI:LKKYR:NMVVR:SXGXH, KIPKAXX:APTEL:SATS-AA$^{17}$-LYY:DENEKVV:LRKHR:DMVVK:GXGXR, GIPEPXX:APTKM:SATS-AA$^{17}$-LYY:DENKNVV:LRKHR:NMVVK:SXAXR, SIPKAXX:APTEL:SATS-AA$^{17}$-LYY:DEYDKVV:LRKHR:EMVVK:GXGXR, HVTKPTX:APTKL:SATS-AA$^{17}$-LYY:DDSSNVI:LRKHR:NMVVK:SXGXH, YVPKPXX:APTKL:SATS-AA$^{17}$-LYY:DDSSNVI:LRKHR:NMVVK:SXGXH, TVPKPXX:APTQL:SATS-AA$^{17}$-LYY:DDSSNVI:LRKHR:NMVVK:AXGXH, KVGKAXX:APTKL:SATS-AA$^{17}$-LYY:DDMGVPT:LRKHR:EGMSVVK:XGXR, KASKAXX:APTKL:SATS-AA$^{17}$-LYY:DKGVVTY:LRKHR:GMVVK:EXGXR, GSAGPXX:APTKM:SATS-AA$^{17}$-LYY:NDKQQII:LRKHR:GMVVK:RXGXS, AAPASXX:APTRL:SATS-AA$^{17}$-LYY:DAANNVV:LRKHR:DMVVK:AXGXR, STPPTXX:APTRL:SATS-AA$^{17}$-LYY:DSANNVV:LRKHR:DMVVK:SXGXR, HVPKPXX:APTKL:SATS-AA$^{17}$-LYY:DDSSNVI:LRKHR:NMVVK:SXGXH, RVPSTXX:APTKT:SATS-AA$^{17}$-LYY:DNGRVLL:LRKHR:MIVVK:XGXL, ASAAPXX:APTAL:SATS-AA$^{17}$-LYY:VGRKPKV:LRKHR:MIVVK:XKXS, ASASPXX:APTDL:SATS-AA$^{17}$-LYY:IGKTPKI:LRKHR:MIVVK:XKXS, ASASPXX:APTDL:SATS-AA$^{17}$-LYY:VGRTPKV:LRKHR:MVVVK:XKXS, NDEGLEX:APTEE:SATS-AA$^{17}$-LYY:RIKPHQGQH:LRKHR:FLVVK:KXEXR, NDEGLEX:APTGQ:SATS-AA$^{17}$-LYY:RIKPHQGQH:LRKHR:LEVVK:QXEXR, SSVKXQP:APTHH:SATS-AA$^{17}$-LYY:EYVRKKPKL:LRKHR:RLVVK:LEXAXA, RNVQXRP:APTQL:SATS-AA$^{17}$-LYY:EIVRKKPIF:LRKHR:TLVVK:LAXKXE, KIPKAXX:VPTEL:SATS-AA$^{17}$-LYY:DENEKVV:LRKHR:DMVVE:GXGXR, GIPEPXX:VPEKM:SATS-AA$^{17}$-LYY:DENKNVV:LRKHR:NMTVE:SXAXR, SIPKAXX:VPTEL:SATS-AA$^{17}$-LYY:DEYDKVV:LRKHR:EMVVE:GXGXR, HVTKPTX:APTKL:SATS-AA$^{17}$-LYY:DDSSNVI:LRKHR:NMVVR:SXGXH, YVPKPXX:APTKL:SATS-AA$^{17}$-LYY:DDSSNVI:LRKHR:NMVVR:SXGXH, TVPKPXX:APTQL:SATS-AA$^{17}$-LYY:DDSSNVI:LRKHR:NMVVR:AXGXH, KVGKAXX:VPTKL:SATS-AA$^{17}$-LYY:DDMGVPT:LRKHR:EGMSVAE:XGXR, KASKAXX:VPTKL:SATS-AA$^{17}$-LYY:DKGVVTY:LRKHR:GMAVS:EXGXR, GSAGPXX:TPTKM:SATS-AA$^{17}$-LYY:NDKQQII:LRKHR:GMVVD:RXGXS, AAPASXX:VPARL:SATS-AA$^{17}$-LYY:DAANNVV:LRKHR:DMVVE:AXGXR, STPPTXXVPTRL:SATS-AA$^{17}$-LYY:DSANNVV:LRKHR:DMVVE:SXGXR, HVPKPXX:APTKL:SATS-AA$^{17}$-LYY:DDSSNVI:LRKHR:NMVVR:SXGXH, RVPSTXX:APVKT:SATS-AA$^{17}$-LYY:DNGRVLL:LRKHR:MIVEE:XGXL, ASAAPXX:VPQAL:SATS-AA$^{17}$-LYY:VGRKPKV:LRKHR:MIVRS:XKXS, ASASPXX:VSQDL:SATS-AA$^{17}$-LYY:IGKTPKI:LRKHR:MIVKS:XKXS, ASASPXX:VPQDL:SATS-AA$^{17}$-LYY:VGRTPKV:LRKHR:MVVKS:XKXS, NDEGLEX:VPTEE:SATS-AA$^{17}$-LYY:RIKPHQGQH:LRKHR:FLQHN:KXEXR, NDEGLEX:VPTGQ:SATS-AA$^{17}$-LYY:RIKPHQGQH:LRKHR:LEEHS:QXEXR, SSVKXQP:SRVHH:SATS-AA$^{17}$-LYY:EYVRKKPKL:LRKHR:RLEEH:LEXAXA, RNVQXRP:TQVQL:SATS-AA$^{17}$-LYY:EIVRKKPIF:LRKHR:TLEDH:LAXKXE, KIPKAXX:VPTEL:SPIS-AA$^{17}$-LYK:DENEKVV:LKYHY:DMVAE:GXGXR, GIPEPXX:VPTKM:SPIS-AA$^{17}$-LYK:DENKNVV:LKYHY:NMVAE:SXAXR, SIPKAXX:VPTEL:SPIS-AA$^{17}$-LYK:DEYDKVV:LKYHY:EMVAE:GXGXR, HVTKPTX:VPTKL:SPIS-AA$^{17}$-LYK:DDSSNVI:LKYHY:N MVAE:SXGXH, YVPKPXX:VPTKL:SPIS-AA$^{17}$-LYK:DDSSNVI:LKYHY:NMVAE:SXGXH, TVPKPXX:VPTQL:SPIS-AA$^{17}$-LYK:DDSSNVI:LKYHY:N MVAE:AXGXH, AVPKAXX:VPTKL:SPIS-AA$^{17}$-LYK:DSSNNVI:LKYHY:NMVAE:AXGXH, KASKAXX:VPTKL:SPIS-AA$^{17}$-LYK:DKGVVTY:LKYHY:GMVAE:EXGXR, GSAGPXX:VPTKM:SPIS-AA$^{17}$-LYK:NDKQQII:LKYHY:GMVAE:RXGXS, AAPASXX:VPTRL:SPIS-AA$^{17}$-LYK:DAANNVV:LKYHY:DMVAE:AXGXR, STPPTXX:VPTRL:SPIS-AA$^{17}$-LYK:DSANNVV:LKYHY:DMVAE:SXGXR, HVPKPXX:VPTKL:SPIS-AA$^{17}$-LYK:DDSSNVI:LKYHY:N MVAE:SXGXH, RVPSTXX:VPTKT:SPIS-AA$^{17}$-LYK:DNGRVLL:LKYHY:MIVAE:XGXL, ASAAPXX:VPTAL:SPIS-AA$^{17}$-LYK:VGRKPKV:LKYHY:MIVAE:XKXS, ASASPXX:VPTDL:SPIS-AA$^{17}$-LYK:IGKTPKI:LKYHY:MIVAE:XKXS, ASASPXX:VPTDL:SPIS-AA$^{17}$-LYK:VGRTPKV:LKYHY:MVVAE:XKXS, NDEGLEX:VPTEE:SPIS-AA$^{17}$-LYK:RIKPHQGQH:LKYHY:FLVAE:KXEXR, NDEGLEX:VPTGQ:SPIS-AA$^{17}$-LYK:RIKPHQGQH:LKYHY:LEVAE:QXEXR, SSVKXQP:VPTHH:SPIS-AA$^{17}$-LYK:EYVRKKPKL:LKYHY:RLVAE:LEXAXA, RNVQXRP:VPTQL:SPIS-AA$^{17}$-LYK:EIVRKKPIF:LKYHY:TLVAE:LAXKXE, KIPKAXX:VPTEL:SPIS-AA$^{17}$-LYK:DENEKVV:LKYHY:DMVVE:GXGXR, GIPEPXX:VPEKM:SPIS-AA$^{17}$-LYK:DENKNVV:LKYHY:NMTVE:SXAXR, SIPKAXX:VP-

TEL:SPIS-AA$^{17}$-LYK:DEYDKVV:LKYHY:EMVVE:GXGXR, HVTKPTX:APTKL:SPIS-AA$^{17}$-LYK:DDSSNVI:LKYHY:NMVVR:SXGXH, YVPKPXX:APTKL:SPIS-AA$^{17}$-LYK:DDSSNVI:LKYHY:N MVVR:SXGXH, TVPKPXX:APTQL:SPIS-AA$^{17}$-LYK:DDSSNVI:LKYHY:N MVVR:AXGXH, AVPKAXX:APTKL:SPIS-AA$^{17}$-LYK:DSSNNVI:LKYHY:NMVVK:AXGXH, KASKAXX:VPTKL:SPIS-AA$^{17}$-LYK:DKGVVTY:LKYHY:GMAVS:EXGXR, GSAGPXX:TPTKM:SPIS-AA$^{17}$-LYK:NDKQQII:LKYHY:GMVVD:RXGXS, AAPASXX:VPARL:SPIS-AA$^{17}$-LYK:DAANNVV:LKYHY:DMVVE:AXGXR, STPPTXX:VPTRL:SPIS-AA$^{17}$-LYK:DSANNVV:LKYHY:DMVVE:SXGXR, HVPKPXX:APTKL:SPIS-AA$^{17}$-LYK:DDSSNVI:LKYHY:N MVVR:SXGXH, RVPSTXX:APVKT:SPIS-AA$^{17}$-LYK:DNGRVLL:LKYHY:MIVEE:XGXL, ASAAPXX:VPQAL:SPIS-AA$^{17}$-LYK:VGRKPKV:LKYHY:MIVRS:XKXS, ASASPXX:VSQDL:SPIS-AA$^{17}$-LYK:IGKTPKI:LKYHY:MIVKS:XKXS, ASASPXX:VPQDL:SPIS-AA$^{17}$-LYK:VGRTPKV:LKYHY:MVVKS:XKXS, NDEGLEX:VPTEE:SPIS-AA$^{17}$-LYK:RIKPHQGQH:LKYHY:FLQHN:KXEXR, NDEGLEX:VPTGQ:SPIS-AA$^{17}$-LYK:RIKPHQGQH:LKYHY:LEEHS:QXEXR, SSVKXQP:SRVHH:SPIS-AA$^{17}$-LYK:EYVRKKPKL:LKYHY:RLEEH:LEXAXA, RNVQXRP:TQVQL:SPIS-AA$^{17}$-LYK:EIVRKKPIF:LKYHY:TLEDH:LAXKXE, KIPKAXX:VPTEL:EPIS-AA$^{17}$-LYL:DENEKVV:KFKYE:DMAVS:GXGXR, GIPEPXX:VPTKM:EPIS-AA$^{17}$-LYL:DENKNVV:KFKYE:NMAVS:SXAXR, SIPKAXX:VPTEL:EPIS-AA$^{17}$-LYL:DEYDKVV:KFKYE:EMAVS:GXGXR, HVTKPTX:VPTKL:EPIS-AA$^{17}$-LYL:DDSSNVI:KFKYE:NMAVS:SXGXH, YVPKPXX:VPTKL:EPIS-AA$^{17}$-LYL:DDSSNVI:KFKYE:NMAVS:SXGXH, TVPKPXX:VPTQL:EPIS-AA$^{17}$-LYL:DDSSNVI:KFKYE:NMAVS:AXGXH, AVPKAXX:VPTKL:EPIS-AA$^{17}$-LYL:DSSNNVI:KFKYE:NMAVS:AXGXH, KVGKAXX:VPTKL:EPIS-AA$^{17}$-LYL:DDMGVPT:KFKYE:EGMSAVS:XGXR, GSAGPXX:VPTKM:EPIS-AA$^{17}$-LYL:NDKQQII:KFKYE:GMAVS:RXGXS, AAPASXX:VPTRL:EPIS-AA$^{17}$-LYL:DAANNVV:KFKYE:DMAVS:AXGXR, STPPTXX:VPTRL:EPIS-AA$^{17}$-LYL:DSANNVV:KFKYE:DMAVS:SXGXR, HVPKPXX:VPTKL:EPIS-AA$^{17}$-LYL:DDSSNVI:KFKYE:NMAVS:SXGXH, RVPSTXX:VPTKT:EPIS-AA$^{17}$-LYL:DNGRVLL:KFKYE:MIAVS:XGXL, ASAAPXX:VPTAL:EPIS-AA$^{17}$-LYL:VGRKPKV:KFKYE:MIAVS:XKXS, ASASPXX:VPTDL:EPIS-AA$^{17}$-LYL:IGKTPKI:KFKYE:MIAVS:XKXS, ASASPXX:VPTDL:EPIS-AA$^{17}$-LYL:VGRTPKV:KFKYE:MVAVS:XKXS, NDEGLEX:VPTEE:EPIS-AA$^{17}$-LYL:RIKPHQGQH:KFKYE:FLAVS:KXEXR, NDEGLEX:VPTGQ:EPIS-AA$^{17}$-LYL:RIKPHQGQH:KFKYE:LEAVS:QXEXR, SSVKXQP:VPTHH:EPIS-AA$^{17}$-LYL:EYVRKKPKL:KFKYE:RLAVS:LEXAXA, RNVQXRP:VPTQL:EPIS-AA$^{17}$-LYL:EIVRKKPIF:KFKYE:TLAVS:LAXKXE, KIPKAXX:VPTEL:EPIS-AA$^{17}$-LYL:DENEKVV:KFKYE:DMVVE:GXGXR, GIPEPXX:VPEKM:EPIS-AA$^{17}$-LYL:DENKNVV:KFKYE:NMTVE:SXAXR, SIPKAXX:VPTEL:EPIS-AA$^{17}$-LYL:DEYDKVV:KFKYE:EMVVE:GXGXR, HVTKPTX:APTKL:EPIS-AA$^{17}$-LYL:DDSSNVI:KFKYE:NMVVR:SXGXH, YVPKPXX:APTKL:EPIS-AA$^{17}$-LYL:DDSSNVI:KFKYE:NMVVR:SXGXH, TVPKPXX:APTQL:EPIS-AA$^{17}$-LYL:DDSSNVI:KFKYE:NMVVR:AXGXH, AVPKAXX:APTKL:EPIS-AA$^{17}$-LYL:DSSNNVI:KFKYE:NMVVK:AXGXH, KVGKAXX:VPTKL:EPIS-AA$^{17}$-LYL:DDMGVPT:KFKYE:EGMSVAE:XGXR, GSAGPXX:TPTKM:EPIS-AA$^{17}$-LYL:NDKQQII:KFKYE:GMVVD:RXGXS, AAPASXX:VPARL:EPIS-AA$^{17}$-LYL:DAANNVV:KFKYE:DMVVE:AXGXR, STPPTXX:VPTRL:EPIS-AA$^{17}$-LYL:DSANNVV:KFKYE:DMVVE:SXGXR, HVPKPXX:APTKL:EPIS-AA$^{17}$-LYL:DDSSNVI:KFKYE:NMVVR:SXGXH, RVPSTXX:APVKT:EPIS-AA$^{17}$-LYL:DNGRVLL:KFKYE:MIVEE:XGXL, ASAAPXX:VPQAL:EPIS-AA$^{17}$-LYL:VGRKPKV:KFKYE:MIVRS:XKXS, ASASPXX:VSQDL:EPIS-AA$^{17}$-LYL:IGKTPKI:KFKYE:MIVKS:XKXS, ASASPXX:VPQDL:EPIS-AA$^{17}$-LYL:VGRTPKV:KFKYE:MVVKS:XKXS, NDEGLEX:VPTEE:EPIS-AA$^{17}$-LYL:RIKPHQGQH:KFKYE:FLQHN:KXEXR, NDEGLEX:VPTGQ:EPIS-AA$^{17}$-LYL:RIKPHQGQH:KFKYE:LEEHS:QXEXR, SSVKXQP:SRVHH:EPIS-AA$^{17}$-LYL:EYVRKKPKL:KFKYE:RLEEH:LEXAXA, RNVQXRP:TQVQL:EPIS-AA$^{17}$-LYL:EIVRKKPIF:KFKYE:TLEDH:LAXKXE, KIPKAXX:TPTEL:SPIN-AA$^{17}$-LYF:DENEKVV:YGKIP:DMVVD:GXGXR, GIPEPXX:TPTKM:SPIN-AA$^{17}$-LYF:DENKNVV:YGKIP:NMVVD:SXAXR, SIPKAXX:TPTEL:SPIN-AA$^{17}$-LYF:DEYDKVV:YGKIP:EMVVD:GXGXR, HVTKPTX:TPTKL:SPIN-AA$^{17}$-LYF:DDSSNVI:YGKIP:NMVVD:SXGXH, YVPKPXX:TPTKL:SPIN-AA$^{17}$-LYF:DDSSNVI:YGKIP:NMVVD:SXGXH, TVPKPXX:TPTQL:SPIN-AA$^{17}$-LYF:DDSSNVI:YGKIP:NMVVD:AXGXH, AVPKAXX:TPTKL:SPIN-AA$^{17}$-LYF:DSSNNVI:YGKIP:NMVVD:AXGXH, KVGKAXX:TPTKL:SPIN-AA$^{17}$-LYF:DDMGVPT:YGKIP:EGMSVVD:XGXR, KASKAXX:TPTKL:SPIN-AA$^{17}$-LYF:DKGVVTY:YGKIP:GMVVD:EXGXR, AAPASXX:TPTRL:SPIN-AA$^{17}$-LYF:DAANNVV:YGKIP:DMVVD:AXGXR, STPPTXX:TPTRL:SPIN-AA$^{17}$-LYF:DSANNVV:YGKIP:DMVVD:SXGXR, HVPKPXX:TPTKL:SPIN-AA$^{17}$-LYF:DDSSNVI:YGKIP:NMVVD:SXGXH, RVPSTXX:TPTKT:SPIN-AA$^{17}$-LYF:DNGRVLL:YGKIP:MIVVD:XGXL, ASAAPXX:TPTAL:SPIN-AA$^{17}$-LYF:VGRKPKV:YGKIP:MIVVD:XKXS, ASASPXX:TPTDL:SPIN-AA$^{17}$-LYF:IGKTPKI:YGKIP:MIVVD:XKXS, ASASPXX:TPTDL:SPIN-AA$^{17}$-LYF:VGRTPKV:YGKIP:MVVVD:XKXS, NDEGLEX:TPTEE:SPIN-AA$^{17}$-LYF:RIKPHQGQH:YGKIP:FLVVD:KXEXR, NDEGLEX:TPTGQ:SPIN-AA$^{17}$-LYF:RIKPHQGQH:YGKIP:LEVVD:QXEXR, SSVKXQP:TPTHH:SPIN-AA$^{17}$-LYF:EYVRKKPKL:YGKIP:RLVVD:LEXAXA, RNVQXRP:TPTQL:SPIN-AA$^{17}$-LYF:EIVRKKPIF:YGKIP:TLVVD:LAXKXE, KIPKAXX:VPTEL:SPIN-AA$^{17}$-LYF:DENEKVV:YGKIP:DMVVE:GXGXR, GIPEPXX:VPEKM:SPIN-AA$^{17}$-LYF:DENKNVV:YGKIP:NMTVE:SXAXR, SIPKAXX:VPTEL:SPIN-AA$^{17}$-LYF:DEYDKVV:YGKIP:EMVVE:GXGXR, HVTKPTX:APTKL:SPIN-AA$^{17}$-LYF:DDSSNVI:YGKIP:NMVVR:SXGXH, YVPKPXX:APTKL:SPIN-AA$^{17}$-LYF:DDSSNVI:YGKIP:NMVVR:SXGXH, TVPKPXX:APTQL:SPIN-AA$^{17}$-LYF:DDSSNVI:YGKIP:NMVVR:AXGXH, AVPKAXX:APTKL:SPIN-AA$^{17}$-LYF:DSSNNVI:YGKIP:NMVVK:AXGXH, KVGKAXX:VPTKL:SPIN-AA$^{17}$-LYF:DDMGVPT:YGKIP:EGMSVAE:XGXR, KASKAXX:VPTKL:SPIN-AA$^{17}$-LYF:DKGVVTY:YGKIP:GMAVS:EXGXR, AAPASXX:VPARL:SPIN-AA$^{17}$-LYF:DAAN-

NVV:YGKIP:DMVVE:AXGXR, STPPTXX:VPTRL:SPIN-AA$^{17}$-LYF:DSANNVV:YGKIP:DMVVE:SXGXR, HVPKPXX:APTKL:SPIN-AA$^{17}$-LYF:DDSSNVI:YGKIP:NMVVR:SXGXH, RVPSTXX:APVKT:SPIN-AA$^{17}$-LYF:DNGRVLL:YGKIP:MIVEE:XGXL, ASAAPXX:VPQAL:SPIN-AA$^{17}$-LYF:VGRKPKV:YGKIP:MIVRS:XKXS, ASASPXX:VSQDL:SPIN-AA$^{17}$-LYF:IGKTPKI:YGKIP:MIVKS:XKXS, ASASPXX:VPQDL:SPIN-AA$^{17}$-LYF:VGRTPKV:YGKIP:MVVKS:XKXS, NDEGLEX:VPTEE:SPIN-AA$^{17}$-LYF:RIKPHQGQH:YGKIP:FLQHN:KXEXR, NDEGLEXVPTGQ:SPIN-AA$^{17}$-LYF:RIKPHQGQH:YGKIP:LEEHS:QXEXR, SSVKXQP:SRVHH:SPIN-AA$^{17}$-LYF:EYVRKKPKL:YGKIP:RLEEH:LEXAXA, RNVQXRP:TQVQL:SPIN-AA$^{17}$-LYF:EIVRKKPIF:YGKIP:TLEDH:LAXKXE, KIPKAXX:VPAEL:SPIS-AA$^{17}$-LYI:DENEKVV:YKQYE:DMVVE:GXGXR, GIPEPXX:VPAKM:SPIS-AA$^{17}$-LYI:DENKNVV:YKQYE:NMVVE:SXAXR, SIPKAXX:VPAEL:SPIS-AA$^{17}$-LYI:DEYDKVV:YKQYE:EMVVE:GXGXR, HVTKPTX:VPAKL:SPIS-AA$^{17}$-LYI:DDSSNVI:YKQYE:NMVVE:SXGXH, YVPKPXX:VPAKL:SPIS-AA$^{17}$-LYI:DDSSNVI:YKQYE:NMVVE:SXGXH, TVPKPXX:VPAQL:SPIS-AA$^{17}$-LYI:DDSSNVI:YKQYE:NMVVE:AXGXH, AVPKAXX:VPAKL:SPIS-AA$^{17}$-LYI:DSSNNVI:YKQYE:NMVVE:AXGXH, KVGKAXX:VPAKL:SPIS-AA$^{17}$-LYI:DDMGVPT:YKQYE:EGMSVVE:XGXR, KASKAXX:VPAKL:SPIS-AA$^{17}$-LYI:DKGVVTY:YKQYE:GMVVE:EXGXR, GSAGPXX:VPAKM:SPIS-AA$^{17}$-LYI:NDKQQII:YKQYE:GMVVE:RXGXS, STPPTXX:VPARL:SPIS-AA$^{17}$-LYI:DSANNVV:YKQYE:DMVVE:SXGXR, HVPKPXX:VPAKL:SPIS-AA$^{17}$-LYI:DDSSNVI:YKQYE:NMVVE:SXGXH, RVPSTXX:VPAKT:SPIS-AA$^{17}$-LYI:DNGRVLL:YKQYE:MIVVE:XGXL, ASAAPXX:VPAAL:SPIS-AA$^{17}$-LYI:VGRKPKV:YKQYE:MIVVE:XKXS, ASASPXX:VPADL:SPIS-AA$^{17}$-LYI:IGKTPKI:YKQYE:MIVVE:XKXS, ASASPXX:VPADL:SPIS-AA$^{17}$ -LYI:VGRTPKV:YKQYE:MVVVE:XKXS, NDEGLEX:VPAEE:SPIS-AA$^{17}$-LYI:RIKPHQGQH:YKQYE:FLVVE:KXEXR, NDEGLEX:VPAGQ:SPIS-AA$^{17}$-LYI:RIKPHQGQH:YKQYE:LEVVE:QXEXR, SSVKXQP:VPAHH:SPIS-AA$^{17}$-LYI:EYVRKKPKL:YKQYE:RLVVE:LEXAXA, RNVQXRP:VPAQL:SPIS-AA$^{17}$-LYI:EIVRKKPIF:YKQYE:TLVVE:LAXKXE, KIPKAXX:VPTEL:SPIS-AA$^{17}$-LYI:DENEKVV:YKQYE:DMVVE:GXGXR, GIPEPXX:VPEKM:SPIS-AA$^{17}$-LYI:DENKNVV:YKQYE:NMTVE:SXAXR, SIPKAXX:VPTEL:SPIS-AA$^{17}$-LYI:DEYDKVV:YKQYE:EMVVE:GXGXR, HVTKPTX:APTKL:SPIS-AA$^{17}$-LYI:DDSSNVI:YKQYE:NMVVR:SXGXH, YVPKPXX:APTKL:SPIS-AA$^{17}$-LYI:DDSSNVI:YKQYE:NMVVR:SXGXH, TVPKPXX:APTQL:SPIS-AA$^{17}$-LYI:DDSSNVI:YKQYE:NMVVR:AXGXH, AVPKAXX:APTKL:SPIS-AA$^{17}$-LYI:DSSNNVI:YKQYE:NMVVK:AXGXH, KVGKAXX:VPTKL:SPIS-AA$^{17}$-LYI:DDMGVPT:YKQYE:EGMSVAE:XGXR, KASKAXX:VPTKL:SPIS-AA$^{17}$-LYI:DKGVVTY:YKQYE:GMAVS:EXGXR, GSAGPXX:TPTKM:SPIS-AA$^{17}$-LYI:NDKQQII:YKQYE:GMVVD:RXGXS, STPPTXX:VPTRL:SPIS-AA$^{17}$-LYI:DSANNVV:YKQYE:DMVVE:SXGXR, HVPKPXX:APTKL:SPIS-AA$^{17}$-LYI:DDSSNVI:YKQYE:NMVVR:SXGXH, RVPSTXX:APVKT:SPIS-AA$^{17}$-LYI:DNGRVLL:YKQYE:MIVEE:XGXL, ASAAPXX:VPQAL:SPIS-AA$^{17}$-LYI:VGRKPKV:YKQYE:MIVRS:XKXS, ASASPXX:VSQDL:SPIS-AA$^{17}$-LYI:IGKTPKI:YKQYE:MIVKS:XKXS, ASASPXX:VPQDL:SPIS-AA$^{17}$-LYI:VGRTPKV:YKQYE:MVVKS:XKXS, NDEGLEX:VPTEE:SPIS-AA$^{17}$-LYI:RIKPHQGQH:YKQYE:FLQHN:KXEXR, NDEGLEX:VPTGQ:SPIS-AA$^{17}$-LYI:RIKPHQGQH:YKQYE:LEEHS:QXEXR, SSVKXQP:SRVHH:SPIS-AA$^{17}$-LYI:EYVRKKPKL:YKQYE:RLEEH:LEXAXA, RNVQXRP:TQVQL:SPIS-AA$^{17}$-LYI:EIVRKKPIF:YKQYE:TLEDH:LAXKXE, KIPKAXX:VPTEL:SPIS-AA$^{17}$-LFI:DENEKVV:YKQYE:DMVVE:GXGXR, GIPEPXX:VPTKM:SPIS-AA$^{17}$-LFI:DENKNVV:YKQYE:NMVVE:SXAXR, SIPKAXX:VPTEL:SPIS-AA$^{17}$-LFI:DEYDKVV:YKQYE:EMVVE:GXGXR, HVTKPTX:VPTKL:SPIS-AA$^{17}$-LFI:DDSSNVI:YKQYE:NMVVE:SXGXH, YVPKPXX:VPTKL:SPIS-AA$^{17}$-LFI:DDSSNVI:YKQYE:NMVVE:SXGXH, TVPKPXX:VPTQL:SPIS-AA$^{17}$-LFI:DDSSNVI:YKQYE:NMVVE:AXGXH, AVPKAXXVPTKL:SPIS-AA$^{17}$-LFI:DSSNNVI:YKQYE:NMVVE:AXGXH, KVGKAXX:VPTKL:SPIS-AA$^{17}$-LFI:DDMGVPT:YKQYE:EGMSVVE:XGXR, KASKAXX:VPTKL:SPIS-AA$^{17}$-LFI:DKGVVTY:YKQYE:GMVVE:EXGXR, GSAGPXX:VPTKM:SPIS-AA$^{17}$-LFI:NDKQQII:YKQYE:GMVVE:RXGXS, AAPASXX:VPTRL:SPIS-AA$^{17}$-LFI:DAANNVV:YKQYE:DMVVE:AXGXR, HVPKPXX:VPTKL:SPIS-AA$^{17}$-LFI:DDSSNVI:YKQYE:NMVVE:SXGXH, RVPSTXX:VPTKT:SPIS-AA$^{17}$-LFI:DNGRVLL:YKQYE:MIVVE:XGXL, ASAAPXX:VPTAL:SPIS-AA$^{17}$-LFI:VGRKPKV:YKQYE:MIVVE:XKXS, ASASPXX:VPTDL:SPIS-AA$^{17}$-LFI:IGKTPKI:YKQYE:MIVVE:XKXS, ASASPXX:VPTDL:SPIS-AA$^{17}$-LFI:VGRTPKV:YKQYE:MVVVE:XKXS, NDEGLEX:VPTEE:SPIS-AA$^{17}$-LFI:RIKPHQGQH:YKQYE:FLVVE:KXEXR, NDEGLEX:VPTGQ:SPIS-AA$^{17}$-LFI:RIKPHQGQH:YKQYE:LEVVE:QXEXR, SSVKXQP:VPTHH:SPIS-AA$^{17}$-LFI:EYVRKKPKL:YKQYE:RLVVE:LEXAXA, RNVQXRP:VPTQL:SPIS-AA$^{17}$-LFI:EIVRKKPIF:YKQYE:TLVVE:LAXKXE, GIPEPXX:VPEKM:SPIS-AA$^{17}$-LFI:DENKNVV:YKQYE:NMTVE:SXAXR, HVTKPTX:APTKL:SPIS-AA$^{17}$-LFI:DDSSNVI:YKQYE:NMVVR:SXGXH, YVPKPXX:APTKL:SPIS-AA$^{17}$-LFI:DDSSNVI:YKQYE:NMVVR:SXGXH, TVPKPXX:APTQL:SPIS-AA$^{17}$-LFI:DDSSNVI:YKQYE:NMVVR:AXGXH, AVPKAXX:APTKL:SPIS-AA$^{17}$-LFI:DSSNNVI:YKQYE:NMVVK:AXGXH, KVGKAXX:VPTKL:SPIS-AA$^{17}$-LFI:DDMGVPT:YKQYE:EGMSVAE:XGXR, KASKAXX:VPTKL:SPIS-AA$^{17}$-LFI:DKGVVTY:YKQYE:GMAVS:EXGXR, GSAGPXX:TPTKM:SPIS-AA$^{17}$-LFI:NDKQQII:YKQYE:GMVVD:RXGXS, AAPASXX:VPARL:SPIS-AA$^{17}$-LFI:DAANNVV:YKQYE:DMVVE:AXGXR, HVPKPXX:APTKL:SPIS-AA$^{17}$-LFI:DDSSNVI:YKQYE:NMVVR:SXGXH, RVPSTXX:APVKT:SPIS-AA$^{17}$-LFI:DNGRVLL:YKQYE:MIVEE:XGXL, ASAAPXX:VPQAL:SPIS-AA$^{17}$-LFI:VGRKPKV:YKQYE:MIVRS:XKXS, ASASPXX:VSQDL:SPIS-AA$^{17}$-LFI:IGKTPKI:YKQYE:MIVKS:XKXS, ASASPXX:VPQDL:SPIS-AA$^{17}$-LFI:VGRTPKV:YKQYE:MVVKS:XKXS, NDEGLEX:VPTEE:SPIS-AA$^{17}$-LFI:RIKPHQGQH:YKQYE:FLQHN:KXEXR, NDEGLEX:VPTGQ:SPIS-AA$^{17}$-LFI:RIKPHQGQH:YKQYE:LEEHS:QXEXR, SSVKXQP:SRVHH:SPIS-AA$^{17}$-LFI:EYVRKKPKL:YKQYE:RLEEH:LEXAXA,

RNVQXRP:TQVQL:SPIS-AA$^{17}$-LFI:EIVRKKPIF:YKQYE:TLEDH:LAXKXE, KIPKAXX:APVEL:KPLS-AA$^{17}$-LYV:DENEKVV:DHHKD:DMVEE:GXGXR, GIPEPXX:APVKM:KPLS-AA$^{17}$-LYV:DENKNVV:DHHKD:NMVEE:SXAXR, SIPKAXX:APVEL:KPLS-AA$^{17}$-LYV:DEYDKVV:DHHKD:EMVEE:GXGXR, HVTKPTX:APVKL:KPLS-AA$^{17}$-LYV:DDSSNVI:DHHKD:NMVEE:SXGXH, YVPKPXX:APVKL:KPLS-AA$^{17}$-LYV:DDSSNVI:DHHKD:NMVEE:SXGXH, TVPKPXX:APVQL:KPLS-AA$^{17}$-LYV:DDSSNVI:DHHKD:NMVEE:AXGXH, AVPKAXX:APVKL:KPLS-AA$^{17}$-LYV:DSSNNVI:DHHKD:NMVEE:AXGXH, KVGKAXX:APVKL:KPLS-AA$^{17}$-LYV:DDMGVPT:DHHKD:EGMSVEE:XGXR, KASKAXX:APVKL:KPLS-AA$^{17}$-LYV:DKGVVTY:DHHKD:GMVEE:EXGXR, GSAGPXX:APVKM:KPLS-AA$^{17}$-LYV:NDKQQII:DHHKD:GMVEE:RXGXS, AAPASXX:APVRL:KPLS-AA$^{17}$-LYV:DAANNVV:DHHKD:DMVEE:AXGXR, STPPTXX:APVRL:KPLS-AA$^{17}$-LYV:DSANNVV:DHHKD:DMVEE:SXGXR, HVPKPXX:APVKL:KPLS-AA$^{17}$-LYV:DDSSNVI:DHHKD:NMVEE:SXGXH, ASAAPXX:APVAL:KPLS-AA$^{17}$-LYV:VGRKPKV:DHHKD:MIVEE:XKXS, ASASPXX:APVDL:KPLS-AA$^{17}$-LYV:IGKTPKI:DHHKD:MIVEE:XKXS, ASASPXX:APVDL:KPLS-AA$^{17}$-LYV:VGRTPKV:DHHKD:MVVEE:XKXS, NDEGLEX:APVEE:KPLS-AA$^{17}$-LYV:RIKPHQGQH:DHHKD:FLVEE:KXEXR, NDEGLEX:APVGQ:KPLS-AA$^{17}$-LYV:RIKPHQGQH:DHHKD:LEVEE:QXEXR, SSVKXQP:APVHH:KPLS-AA$^{17}$-LYV:EYVRKKPKL:DHHKD:RLVEE:LEXAXA, RNVQXRP:APVQL:KPLS-AA$^{17}$-LYV:EIVRKKPIF:DHHKD:TLVEE:LAXKXE, KIPKAXX:VPTEL:KPLS-AA$^{17}$-LYV:DENEKVV:DHHKD:DMVVE:GXGXR, GIPEPXX:VPEKM:KPLS-AA$^{17}$-LYV:DENKNVV:DHHKD:NMTVE:SXAXR, SIPKAXX:VPTEL:KPLS-AA$^{17}$-LYV:DEYDKVV:DHHKD:EMVVE:GXGXR, HVTKPTX:APTKL:KPLS-AA$^{17}$-LYV:DDSSNVI:DHHKD:NMVVR:SXGXH, YVPKPXX:APTKL:KPLS-AA$^{17}$-LYV:DDSSNVI:DHHKD:NMVVR:SXGXH, TVPKPXX:APTQL:KPLS-AA$^{17}$-LYV:DDSSNVI:DHHKD:NMVVR:AXGXH, AVPKAXX:APTKL:KPLS-AA$^{17}$-LYV:DSSNNVI:DHHKD:NMVVK:AXGXH, KVGKAXX:VPTKL:KPLS-AA$^{17}$-LYV:DDMGVPT:DHHKD:EGMSVAE:XGXR, KASKAXX:VPTKL:KPLS-AA$^{17}$-LYV:DKGVVTY:DHHKD:GMAVS:EXGXR, GSAGPXX:TPTKM:KPLS-AA$^{17}$-LYV:NDKQQII:DHHKD:GMVVD:RXGXS, AAPASXX:VPARL:KPLS-AA$^{17}$-LYV:DAANNVV:DHHKD:DMVVE:AXGXR, STPPTXX:VPTRL:KPLS-AA$^{17}$-LYV:DSANNVV:DHHKD:DMVVE:SXGXR, HVPKPXX:APTKL:KPLS-AA$^{17}$-LYV:DDSSNVI:DHHKD:NMVVR:SXGXH, ASAAPXX:VPQAL:KPLS-AA$^{17}$-LYV:VGRKPKV:DHHKD:MIVRS:XKXS, ASASPXX:VSQDL:KPLS-AA$^{17}$-LYV:IGKTPKI:DHHKD:MIVKS:XKXS, ASASPXX:VPQDL:KPLS-AA$^{17}$-LYV:VGRTPKV:DHHKD:MVVKS:XKXS, NDEGLEXVPTEE:KPLS-AA$^{17}$-LYV:RIKPHQGQH:DHHKD:FLQHN:KXEXR, NDEGLEX:VPTGQ:KPLS-AA$^{17}$-LYV:RIKPHQGQH:DHHKD:LEEHS:QXEXR, SSVKXQP:SRVHH:KPLS-AA$^{17}$-LYV:EYVRKKPKL:DHHKD:RLEEH:LEXAXA, RNVQXRP:TQVQL:KPLS-AA$^{17}$-LYV:EIVRKKPIF:DHHKD:TLEDH:LAXKXE, KIPKAXX:VPQEL:EPLP-AA$^{17}$-VYY:DENEKVV:EQLSN:DMVRS:GXGXR, GIPEPXX:VPQKM:EPLP-AA$^{17}$-VYY:DENKNVV:EQLSN:NMVRS:SXAXR, SIPKAXX:VPQEL:EPLP-AA$^{17}$-VYY:DEYDKVV:EQLSN:EMVRS:GXGXR, HVTKPTX:VPQKL:EPLP-AA$^{17}$-VYY:DDSSNVI:EQLSN:NMVRS:SXGXH, YVPKPXX:VPQKL:EPLP-AA$^{17}$-VYY:DDSSNVI:EQLSN:NMVRS:SXGXH, TVPKPXX:VPQQL:EPLP-AA$^{17}$-VYY:DDSSNVI:EQLSN:NMVRS:AXGXH, AVPKAXX:VPQKL:EPLP-AA$^{17}$-VYY:DSSNNVI:EQLSN:NMVRS:AXGXH, KVGKAXX:VPQKL:EPLP-AA$^{17}$-VYY:DDMGVPT:EQLSN:EGMSVRS:XGXR, KASKAXX:VPQKL:EPLP-AA$^{17}$-VYY:DKGVVTY:EQLSN:GMVRS:EXGXR, GSAGPXX:VPQKM:EPLP-AA$^{17}$-VYY:NDKQQII:EQLSN:GMVRS:RXGXS, AAPASXX:VPQRL:EPLP-AA$^{17}$-VYY:DAANNVV:EQLSN:DMVRS:AXGXR, STPPTXX:VPQRL:EPLP-AA$^{17}$-VYY:DSANNVV:EQLSN:DMVRS:SXGXR, HVPKPXX:VPQKL:EPLP-AA$^{17}$-VYY:DDSSNVI:EQLSN:NMVRS:SXGXH, RVPSTXX:VPQKT:EPLP-AA$^{17}$-VYY:DNGRVLL:EQLSN:MIVRS:XGXL, ASASPXX:VPQDL:EPLP-AA$^{17}$-VYY:IGKTPKI:EQLSN:MIVRS:XKXS, ASASPXX:VPQDL:EPLP-AA$^{17}$-VYY:VGRTPKV:EQLSN:MVVRS:XKXS, NDEGLEX:VPQEE:EPLP-AA$^{17}$-VYY:RIKPHQGQH:EQLSN:FLVRS:KXEXR, NDEGLEX:VPQGQ:EPLP-AA$^{17}$-VYY:RIKPHQGQH:EQLSN:LEVRS:QXEXR, SSVKXQP:VPQHH:EPLP-AA$^{17}$-VYY:EYVRKKPKL:EQLSN:RLVRS:LEXAXA, RNVQXRP:VPQQL:EPLP-AA$^{17}$-VYY:EIVRKKPIF:EQLSN:TLVRS:LAXKXE, KIPKAXX:VPTEL:EPLP-AA$^{17}$-VYY:DENEKVV:EQLSN:DMVVE:GXGXR, GIPEPXX:VPEKM:EPLP-AA$^{17}$-VYY:DENKNVV:EQLSN:NMTVE:SXAXR, SIPKAXX:VPTEL:EPLP-AA$^{17}$-VYY:DEYDKVV:EQLSN:EMVVE:GXGXR, HVTKPTX:APTKL:EPLP-AA$^{17}$-VYY:DDSSNVI:EQLSN:NMVVR:SXGXH, YVPKPXX:APTKL:EPLP-AA$^{17}$-VYY:DDSSNVI:EQLSN:NMVVR:SXGXH, TVPKPXX:APTQL:EPLP-AA$^{17}$-VYY:DDSSNVI:EQLSN:NMVVR:AXGXH, AVPKAXX:APTKL:EPLP-AA$^{17}$-VYY:DSSNNVI:EQLSN:NMVVK:AXGXH, KVGKAXX:VPTKL:EPLP-AA$^{17}$-VYY:DDMGVPT:EQLSN:EGMSVAE:XGXR, KASKAXX:VPTKL:EPLP-AA$^{17}$-VYY:DKGVVTY:EQLSN:GMAVS:EXGXR, GSAGPXX:TPTKM:EPLP-AA$^{17}$-VYY:NDKQQII:EQLSN:GMVVD:RXGXS, AAPASXX:VPARL:EPLP-AA$^{17}$-VYY:DAANNVV:EQLSN:DMVVE:AXGXR, STPPTXX:VPTRL:EPLP-AA$^{17}$-VYY:DSANNVV:EQLSN:DMVVE:SXGXR, HVPKPXX:APTKL:EPLP-AA$^{17}$-VYY:DDSSNVI:EQLSN:NMVVR:SXGXH, RVPSTXX:APVKT:EPLP-AA$^{17}$-VYY:DNGRVLL:EQLSN:MIVEE:XGXL, ASASPXX:VSQDL:EPLP-AA$^{17}$-VYY:IGKTPKI:EQLSN:MIVKS:XKXS, ASASPXX:VPQDL:EPLP-AA$^{17}$-VYY:VGRTPKV:EQLSN:MVVKS:XKXS, NDEGLEX:VPTEE:EPLP-AA$^{17}$-VYY:RIKPHQGQH:EQLSN:FLQHN:KXEXR, NDEGLEX:VPTGQ:EPLP-AA$^{17}$-VYY:RIKPHQGQH:EQLSN:LEEHS:QXEXR, SSVKXQP:SRVHH:EPLP-AA$^{17}$-VYY:EYVRKKPKL:EQLSN:RLEEH:LEXAXA, RNVQXRP:TQVQL:EPLP-AA$^{17}$-VYY:EIVRKKPIF:EQLSN:TLEDH:LAXKXE, KIPKAXX:VSQEL:EPLT-AA$^{17}$-LYY:DENEKVV:EQLSN:DMVKS:GXGXR, GIPEPXX:VSQKM:EPLT-AA$^{17}$-LYY:DENKNVV:EQLSN:NMVKS:SXAXR, SIPKAXX:VSQEL:EPLT-AA$^{17}$-LYY:DEYDKVV:EQLSN:EMVKS:GXGXR, HVTKPTX:VSQKL:EPLT-AA$^{17}$-LYY:DDSSNVI:EQLSN:NMVKS:SXGXH, YVPKPXX:VSQKL:EPLT-AA$^{17}$-LYY:DDSSNVI:EQL-

SN:NMVKS:SXGXH, TVPKPXX:VSQQL:EPLT-AA$^{17}$-LYY:DDSSNVI:EQLSN:NMVKS:AXGXH, AVPKAXX:VSQKL:EPLT-AA$^{17}$-LYY:DSSNNVI:EQLSN:NMVKS:AXGXH, KVGKAXX:VSQKL:EPLT-AA$^{17}$-LYY:DDMGVPT:EQLSN:EGMSVKS:XGXR, KASKAXX:VSQKL:EPLT-AA$^{17}$-LYY:DKGVVTY:EQLSN:GMVKS:EXGXR, GSAGPXX:VSQKM:EPLT-AA$^{17}$-LYY:NDKQQII:EQLSN:GMVKS:RXGXS, AAPASXX:VSQRL:EPL T-AA$^{17}$-LYY:DAANNVV:EQLSN:DMVKS:AXGXR, STPPTXX:VSQRL:EPLT-AA$^{17}$-LYY:DSANNVV:EQLSN:DMVKS:SXGXR, HVPKPXX:VSQKL:EPLT-AA$^{17}$-LYY:DDSSNVI:EQLSN:NMVKS:SXGXH, RVPSTXX:VSQKT:EPLT-AA$^{17}$-LYY:DNGRVLL:EQLSN:MIVKS:XGXL, ASAAPXX:VSQAL:EPLT-AA$^{17}$-LYY:VGRKPKV:EQLSN:MIVKS:XKXS, ASASPXX:VSQDL:EPLT-AA$^{17}$-LYY:VGRTPKV:EQLSN:MVVKS:XKXS, NDEGLEX:VSQEE:EPLT-AA$^{17}$-LYY:RIKPHQGQH:EQLSN:FLVKS:KXEXR, NDEGLEX:VSQGQ:EPLT-AA$^{17}$-LYY:RIKPHQGQH:EQLSN:LEVKS:QXEXR, SSVKXQP:VSQHH:EPLT-AA$^{17}$-LYY:EYVRKKPKL:EQLSN:RLVKS:LEXAXA, RNVQXRP:VSQQL:EPLT-AA$^{17}$-LYY:EIVRKKPIF:EQLSN:TLVKS:LAXKXE, KIPKAXX:VPTEL:EPLT-AA$^{17}$-LYY:DENEKVV:EQLSN:DMVVE:GXGXR, GIPEPXX:VPEKM:EPLT-AA$^{17}$-LYY:DENKNVV:EQLSN:NMTVE:SXAXR, SIPKAXX:VPTEL:EPLT-AA$^{17}$-LYY:DEYDKVV:EQLSN:EMVVE:GXGXR, HVTKPTX:APTKL:EPLT-AA$^{17}$-LYY:DDSSNVI:EQLSN:NMVVR:SXGXH, YVPKPXX:APTKL:EPLT-AA$^{17}$-LYY:DDSSNVI:EQLSN:NMVVR:SXGXH, TVPKPXX:APTQL:EPLT-AA$^{17}$-LYY:DDSSNVI:EQLSN:NMVVR:AXGXH, AVPKAXX:APTKL:EPLT-AA$^{17}$-LYY:DSSNNVI:EQLSN:NMVVK:AXGXH, KVGKAXX:VPTKL:EPLT-AA$^{17}$-LYY:DDMGVPT:EQLSN:EGMSVAE:XGXR, KASKAXX:VPTKL:EPLT-AA$^{17}$-LYY:DKGVVTY:EQLSN:GMAVS:EXGXR, GSAGPXX:TPTKM:EPLT-AA$^{17}$-LYY:NDKQQII:EQLSN:GMVVD:RXGXS, AAPASXX:VPARL:EPLT-AA$^{17}$-LYY:DAANNVV:EQLSN:DMVVE:AXGXR, STPPTXX:VPTRL:EPLT-AA$^{17}$-LYY:DSANNVV:EQLSN:DMVVE:SXGXR, HVPKPXX:APTKL:EPLT-AA$^{17}$-LYY:DDSSNVI:EQLSN:NMVVR:SXGXH, RVPSTXX:APVKT:EPLT-AA$^{17}$-LYY:DNGRVLL:EQLSN:MIVEE:XGXL, ASAAPXX:VPQAL:EPLT-AA$^{17}$-LYY:VGRKPKV:EQLSN:MIVRS:XKXS, ASASPXX:VPQDL:EPLT-AA$^{17}$-LYY:VGRTPKV:EQLSN:MVVKS:XKXS, NDEGLEX:VPTEE:EPLT-AA$^{17}$-LYY:RIKPHQGQH:EQLSN:FLQHN:KXEXR, NDEGLEX:VPTGQ:EPLT-AA$^{17}$-LYY:RIKPHQGQH:EQLSN:LEEHS:QXEXR, SSVKXQP:SRVHH:EPLT-AA$^{17}$-LYY:EYVRKKPKL:EQLSN:RLEEH:LEXAXA, RNVQXRP:TQVQL:EPLT-AA$^{17}$-LYY:EIVRKKPIF:EQLSN:TLEDH:LAXKXE, KIPKAXX:VPQEL:EPLT-AA$^{17}$-LYY:DENEKVV:EQLSN:DMVKS:GXGXR, GIPEPXX:VPQKM:EPLT-AA$^{17}$-LYY:DENKNVV:EQLSN:NMVKS:SXAXR, SIPKAXX:VPQEL:EPLT-AA$^{17}$-LYY:DEYDKVV:EQLSN:EMVKS:GXGXR, HVTKPTX:VPQKL:EPLT-AA$^{17}$-LYY:DDSSNVI:EQLSN:NMVKS:SXGXH, YVPKPXX:VPQKL:EPLT-AA$^{17}$-LYY:DDSSNVI:EQLSN:NMVKS:SXGXH, TVPKPXX:VPQQL:EPLT-AA$^{17}$-LYY:DDSSNVI:EQLSN:NMVKS:AXGXH, AVPKAXX:VPQKL:EPLT-AA$^{17}$-LYY:DSSNNVI:EQLSN:NMVKS:AXGXH, KVGKAXX:VPQKL:EPLT-AA$^{17}$-LYY:DDMGVPT:EQLSN:EGMSVKS:XGXR, KASKAXX:VPQKL:EPLT-AA$^{17}$-LYY:DKGVVTY:EQLSN:GMVKS:EXGXR, GSAGPXX:VPQKM:EPLT-AA$^{17}$-LYY:NDKQQII:EQLSN:GMVKS:RXGXS, AAPASXX:VPQRL:EPLT-AA$^{17}$-LYY:DAANNVV:EQLSN:DMVKS:AXGXR, STPPTXX:VPQRL:EPLT-AA$^{17}$-LYY:DSANNVV:EQLSN:DMVKS:SXGXR, HVPKPXX:VPQKL:EPLT-AA$^{17}$-LYY:DDSSNVI:EQLSN:NMVKS:SXGXH, RVPSTXX:VPQKT:EPLT-AA$^{17}$-LYY:DNGRVLL:EQLSN:MIVKS:XGXL, ASAAPXX:VPQAL:EPLT-AA$^{17}$-LYY:VGRKPKV:EQLSN:MIVKS:XKXS, ASASPXX:VPQDL:EPLT-AA$^{17}$-LYY:IGKTPKI:EQLSN:MIVKS:XKXS, NDEGLEX:VPQEE:EPLT-AA$^{17}$-LYY:RIKPHQGQH:EQLSN:FLVKS:KXEXR, NDEGLEX:VPQGQ:EPLT-AA$^{17}$-LYY:RIKPHQGQH:EQLSN:LEVKS:QXEXR, SSVKXQP:VPQHH:EPLT-AA$^{17}$-LYY:EYVRKKPKL:EQLSN:RLVKS:LEXAXA, RNVQXRP:VPQQL:EPLT-AA$^{17}$-LYY:EIVRKKPIF:EQLSN:TLVKS:LAXKXE, ASASPXX:VSQDL:EPLT-AA$^{17}$-LYY:IGKTPKI:EQLSN:MIVKS:XKXS, KIPKAXX:VPTEL:SNIT-AA$^{17}$-QIM:DENEKVV:IGEMS:DMQHN:GXGXR, GIPEPXX:VPTKM:SNIT-AA$^{17}$-QIM:DENKNVV:IGEMS:NMQHN:SXAXR, SIPKAXX:VPTEL:SNIT-AA$^{17}$-QIM:DEYDKVV:IGEMS:EMQHN:GXGXR, HVTKPTX:VPTKL:SNIT-AA$^{17}$-QIM:DDSSNVI:IGEMS:NMQHN:SXGXH, YVPKPXX:VPTKL:SNIT-AA$^{17}$-QIM:DDSSNVI:IGEMS:NMQHN:SXGXH, TVPKPXX:VPTQL:SNIT-AA$^{17}$-QIM:DDSSNVI:IGEMS:NMQHN:AXGXH, AVPKAXX:VPTKL:SNIT-AA$^{17}$-QIM:DSSNNVI:IGEMS:NMQHN:AXGXH, KVGKAXX:VPTKL:SNIT-AA$^{17}$-QIM:DDMGVPT:IGEMS:EGMSQHN:XGXR, KASKAXX:VPTKL:SNIT-AA$^{17}$-QIM:DKGVVTY:IGEMS:GMQHN:EXGXR, GSAGPXX:VPTKM:SNIT-AA$^{17}$-QIM:NDKQQII:IGEMS:GMQHN:RXGXS, AAPASXX:VPTRL:SNIT-AA$^{17}$-QIM:DAANNVV:IGEMS:DMQHN:AXGXR, STPPTXX:VPTRL:SNIT-AA$^{17}$-QIM:DSANNVV:IGEMS:DMQHN:SXGXR, HVPKPXX:VPTKL:SNIT-AA$^{17}$-QIM:DDSSNVI:IGEMS:NMQHN:SXGXH, RVPSTXXVPTKT:SNIT-AA$^{17}$-QIM:DNGRVLL:IGEMS:MIQHN:XGXL, ASAAPXX:VPTAL:SNIT-AA$^{17}$-QIM:VGRKPKV:IGEMS:MIQHN:XKXS, ASASPXX:VPTDL:SNIT-AA$^{17}$-QIM:IGKTPKI:IGEMS:MIQHN:XKXS, ASASPXX:VPTDL:SNIT-AA$^{17}$-QIM:VGRTPKV:IGEMS:MVQHN:XKXS, NDEGLEX:VPTGQ:SNIT-AA$^{17}$-QIM:RIKPHQGQH:IGEMS:LEQHN:QXEXR, NDEGLEX:VPTEE:SNIT-AA$^{17}$-QIM:RIKPHQGQH:IGEMS:FLQHN:KXEXR, SSVKXQP:VPTHH:SNIT-AA$^{17}$-QIM:EYVRKKPKL:IGEMS:RLQHN:LEXAXA, RNVQXRP:VPTQL:SNIT-AA$^{17}$-QIM:EIVRKKPIF:IGEMS:TLQHN:LAXKXE, KIPKAXX:VPTEL:SNIT-AA$^{17}$-QIM:DENEKVV:IGEMS:DMVVE:GXGXR, GIPEPXX:VPEKM:SNIT-AA$^{17}$-QIM:DENKNVV:IGEMS:NMTVE:SXAXR, SIPKAXX:VPTEL:SNIT-AA$^{17}$-QIM:DEYDKVV:IGEMS:EMVVE:GXGXR, HVTKPTX:APTKL:SNIT-AA$^{17}$-QIM:DDSSNVI:IGEMS:NMVVR:SXGXH, YVPKPXX:APTKL:SNIT-AA$^{17}$-QIM:DDSSNVI:IGEMS:NMVVR:SXGXH, TVPKPXX:APTQL:SNIT-AA$^{17}$-QIM:DDSSNVI:IGEMS:NMVVR:AXGXH, AVPKAXX:APTKL:SNIT-AA$^{17}$-QIM:DSSNNVI:IGEMS:NMVVK:AXGXH, KVGKAXX:VPTKL:SNIT-AA$^{17}$-QIM:DDMGVPT:IGEMS:EGMSVAE:XGXR, KASKAXX:VPTKL:SNIT-AA$^{17}$-QIM:DKGVVTY:IGEMS:GMAVS:EXGXR,

GSAGPXX:TPTKM:SNIT-$AA^{17}$-QIM:NDKQQII:IGEMS:GMVVD:RXGXS, AAPASXX:VPARL:SNIT-$AA^{17}$-QIM:DAANNVV:IGEMS:DMVVE:AXGXR, STPPTXX:VPTRL:SNIT-$AA^{17}$-QIM:DSANNVV:IGEMS:DMVVE:SXGXR, HVPKPXX:APTKL:SNIT-$AA^{17}$-QIM:DDSSNVI:IGEMS:NMVVR:SXGXH, RVPSTXX:APVKT:SNIT-$AA^{17}$-QIM:DNGRVLL:IGEMS:MIVEE:XGXL, ASAAPXX:VPQAL:SNIT-$AA^{17}$-QIM:VGRKPKV:IGEMS:MIVRS:XKXS, ASASPXX:VSQDL:SNIT-$AA^{17}$-QIM:IGKTPKI:IGEMS:MIVKS:XKXS, ASASPXX:VPQDL:SNIT-$AA^{17}$-QIM:VGRTPKV:IGEMS:MVVKS:XKXS, NDEGLEX:VPTGQ:SNIT-$AA^{17}$-QIM:RIKPHQGQH:IGEMS:LEEHS:QXEXR, SSVKXQP:SRVHH:SNIT-$AA^{17}$-QIM:EYVRKKPKL:IGEMS:RLEEH:LEXAXA, RNVQXRP:TQVQL:SNIT-$AA^{17}$-QIM:EIVRKKPIF:IGEMS:TLEDH:LAXKXE, KIPKAXX:VPTEL:SNIT-$AA^{17}$-QIM:DENEKVV:LGEMS:DMEHS:GXGXR, GIPEPXX:VPTKM:SNIT-$AA^{17}$-QIM:DENKNVV:LGEMS:NMEHS:SXAXR, SIPKAXX:VPTEL:SNIT-$AA^{17}$-QIM:DEYDKVV:LGEMS:EMEHS:GXGXR, HVTKPTX:VPTKL:SNIT-$AA^{17}$-QIM:DDSSNVI:LGEMS:NMEHS:SXGXH, YVPKPXX:VPTKL:SNIT-$AA^{17}$-QIM:DDSSNVI:LGEMS:NMEHS:SXGXH, TVPKPXX:VPTQL:SNIT-$AA^{17}$-QIM:DDSSNVI:LGEMS:NMEHS:AXGXH, AVPKAXX:VPTKL:SNIT-$AA^{17}$-QIM:DSSNNVI:LGEMS:NMEHS:AXGXH, KVGKAXX:VPTKL:SNIT-$AA^{17}$-QIM:DDMGVPT:LGEMS:EGMSEHS:XGXR, KASKAXXVPTKL:SNIT-$AA^{17}$-QIM:DKGVVTY:LGEMS:GMEHS:EXGXR, GSAGPXX:VPTKM:SNIT-$AA^{17}$-QIM:NDKQQII:LGEMS:GMEHS:RXGXS, AAPASXXVPTRL:SNIT-$AA^{17}$-QIM:DAANNVV:LGEMS:DMEHS:AXGXR, STPPTXX:VPTRL:SNIT-$AA^{17}$-QIM:DSANNVV:LGEMS:DMEHS:SXGXR, HVPKPXX:VPTKL:SNIT-$AA^{17}$-QIM:DDSSNVI:LGEMS:NMEHS:SXGXH, RVPSTXX:VPTKT:SNIT-$AA^{17}$-QIM:DNGRVLL:LGEMS:MIEHS:XGXL, ASAAPXX:VPTAL:SNIT-$AA^{17}$-QIM:VGRKPKV:LGEMS:MIEHS:XKXS, ASASPXXVPTDL:SNIT-$AA^{17}$-QIM:IGKTPKI:LGEMS:MIEHS:XKXS, ASASPXX:VPTDL:SNIT-$AA^{17}$-QIM:VGRTPKV:LGEMS:MVEHS:XKXS, NDEGLEX:VPTEE:SNIT-$AA^{17}$-QIM:RIKPHQGQH:LGEMS:FLEHS:KXEXR, SSVKXQP:VPTHH:SNIT-$AA^{17}$-QIM:EYVRKKPKL:LGEMS:RLEHS:LEXAXA, RNVQXRP:VPTQL:SNIT-$AA^{17}$-QIM:EIVRKKPIF:LGEMS:TLEHS:LAXKXE, KIPKAXX:VPTEL:SNIT-$AA^{17}$-QIM:DENEKVV:LGEMS:DMVVE:GXGXR, GIPEPXX:VPEKM:SNIT-$AA^{17}$-QIM:DENKNVV:LGEMS:NMTVE:SXAXR, SIPKAXX:VPTEL:SNIT-$AA^{17}$-QIM:DEYDKVV:LGEMS:EMVVE:GXGXR, HVTKPTX:APTKL:SNIT-$AA^{17}$-QIM:DDSSNVI:LGEMS:NMVVR:SXGXH, YVPKPXX:APTKL:SNIT-$AA^{17}$-QIM:DDSSNVI:LGEMS:NMVVR:SXGXH, TVPKPXX:APTQL:SNIT-$AA^{17}$-QIM:DDSSNVI:LGEMS:NMVVR:AXGXH, AVPKAXX:APTKL:SNIT-$AA^{17}$-QIM:DSSNNVI:LGEMS:NMVVK:AXGXH, KVGKAXX:VPTKL:SNIT-$AA^{17}$-QIM:DDMGVPT:LGEMS:EGMSVAE:XGXR, KASKAXXVPTKL:SNIT-$AA^{17}$-QIM:DKGVVTY:LGEMS:GMAVS:EXGXR, GSAGPXX:TPTKM:SNIT-$AA^{17}$-QIM:NDKQQII:LGEMS:GMVVD:RXGXS, AAPASXX:VPARL:SNIT-$AA^{17}$-QIM:DAANNVV:LGEMS:DMVVE:AXGXR, STPPTXX:VPTRL:SNIT-$AA^{17}$-QIM:DSANNVV:LGEMS:DMVVE:SXGXR, HVPKPXX:APTKL:SNIT-$AA^{17}$-QIM:DDSSNVI:LGEMS:NMVVR:SXGXH, RVPSTXX:APVKT:SNIT-$AA^{17}$-QIM:DNGRVLL:LGEMS:MIVEE:XGXL, ASAAPXX:VPQAL:SNIT-$AA^{17}$-QIM:VGRKPKV:LGEMS:MIVRS:XKXS, ASASPXX:VSQDL:SNIT-$AA^{17}$-QIM:IGKTPKI:LGEMS:MIVKS:XKXS, ASASPXX:VPQDL:SNIT-$AA^{17}$-QIM:VGRTPKV:LGEMS:MVVKS:XKXS, NDEGLEX:VPTEE:SNIT-$AA^{17}$-QIM:RIKPHQGQH:LGEMS:FLQHN:KXEXR, SSVKXQP:SRVHH:SNIT-$AA^{17}$-QIM:EYVRKKPKL:LGEMS:RLEEH:LEXAXA, RNVQXRP:TQVQL:SNIT-$AA^{17}$-QIM:EIVRKKPIF:LGEMS:TLEDH:LAXKXE, KIPKAXX:SRVEL:RSVK-$AA^{17}$-AKV:DENEKVV:KEVQV:DMEEH:GXGXR, GIPEPXX:SRVKM:RSVK-$AA^{17}$-AKV:DENKNVV:KEVQV:NMEEH:SXAXR, SIPKAXX:SRVEL:RSVK-$AA^{17}$-AKV:DEYDKVV:KEVQV:EMEEH:GXGXR, HVTKPTX:SRVKL:RSVK-$AA^{17}$-AKV:DDSSNVI:KEVQV:NMEEH:SXGXH, YVPKPXX:SRVKL:RSVK-$AA^{17}$-AKV:DDSSNVI:KEVQV:NMEEH:SXGXH, TVPKPXX:SRVQL:RSVK-$AA^{17}$-AKV:DDSSNVI:KEVQV:NMEEH:AXGXH, AVPKAXX:SRVKL:RSVK-$AA^{17}$-AKV:DSSNNVI:KEVQV:NMEEH:AXGXH, KVGKAXX:SRVKL:RSVK-$AA^{17}$-AKV:DDMGVPT:KEVQV:EGMSEEH:XGXR, KASKAXX:SRVKL:RSVK-$AA^{17}$-AKV:DKGVVTY:KEVQV:GMEEH:EXGXR, GSAGPXX:SRVKM:RSVK-$AA^{17}$-AKV:NDKQQII:KEVQV:GMEEH:RXGXS, AAPASXX:SRVRL:RSVK-$AA^{17}$-AKV:DAANNVV:KEVQV:DMEEH:AXGXR, STPPTXX:SRVRL:RSVK-$AA^{17}$-AKV:DSANNVV:KEVQV:DMEEH:SXGXR, HVPKPXX:SRVKL:RSVK-$AA^{17}$-AKV:DDSSNVI:KEVQV:NMEEH:SXGXH, RVPSTXX:SRVKT:RSVK-$AA^{17}$-AKV:DNGRVLL:KEVQV:MIEEH:XGXL, ASAAPXX:SRVAL:RSVK-$AA^{17}$-AKV:VGRKPKV:KEVQV:MIEEH:XKXS, ASASPXX:SRVDL:RSVK-$AA^{17}$-AKV:IGKTPKI:KEVQV:MIEEH:XKXS, ASASPXX:SRVDL:RSVK-$AA^{17}$-AKV:VGRTPKV:KEVQV:MVEEH:XKXS, NDEGLEX:SRVEE:RSVK-$AA^{17}$-AKV:RIKPHQGQH:KEVQV:FLEEH:KXEXR, NDEGLEX:SRVGQ:RSVK-$AA^{17}$-AKV:RIKPHQGQH:KEVQV:LEEEH:QXEXR, RNVQXRP:SRVQL:RSVK-$AA^{17}$-AKV:EIVRKKPIF:KEVQV:TLEEH:LAXKXE, KIPKAXX:VPTEL:RSVK-$AA^{17}$-AKV:DENEKVV:KEVQV:DMVVE:GXGXR, GIPEPXX:VPEKM:RSVK-$AA^{17}$-AKV:DENKNVV:KEVQV:NMTVE:SXAXR, SIPKAXX:VPTEL:RSVK-$AA^{17}$-AKV:DEYDKVV:KEVQV:EMVVE:GXGXR, HVTKPTX:APTKL:RSVK-$AA^{17}$-AKV:DDSSNVI:KEVQV:NMVVR:SXGXH, YVPKPXX:APTKL:RSVK-$AA^{17}$-AKV:DDSSNVI:KEVQV:NMVVR:SXGXH, TVPKPXX:APTQL:RSVK-$AA^{17}$-AKV:DDSSNVI:KEVQV:NMVVR:AXGXH, AVPKAXX:APTKL:RSVK-$AA^{17}$-AKV:DSSNNVI:KEVQV:NMVVK:AXGXH, KVGKAXX:VPTKL:RSVK-$AA^{17}$-AKV:DDMGVPT:KEVQV:EGMSVAE:XGXR, KASKAXX:VPTKL:RSVK-$AA^{17}$-AKV:DKGVVTY:KEVQV:GMAVS:EXGXR, GSAGPXX:TPTKM:RSVK-$AA^{17}$-AKV:NDKQQII:KEVQV:GMVVD:RXGXS, AAPASXX:VPARL:RSVK-$AA^{17}$-AKV:DAANNVV:KEVQV:DMVVE:AXGXR, STPPTXX:VPTRL:RSVK-$AA^{17}$-AKV:DSANNVV:KEVQV:DMVVE:SXGXR, HVPKPXX:APTKL:RSVK-$AA^{17}$-AKV:DDSSNVI:KEVQV:NMVVR:SXGXH, RVPSTXX:APVKT:RSVK-$AA^{17}$-AKV:DNGRVLL:KEVQV:MIVEE:XGXL, ASAAPXX:VPQAL:RSVK-

AA$^{17}$-AKV:VGRKPKV:KEVQV:MIVRS:XKXS, ASASPXX:VSQDL:RSVK-AA$^{17}$-AKV:IGKTPKI:KEVQV:MIVKS:XKXS, ASASPXX:VPQDL:RSVK-AA$^{17}$-AKV:VGRTPKV:KEVQV:MVVKS:XKXS, NDEGLEX:VPTEE:RSVK-AA$^{17}$-AKV:RIKPHQGQH:KEVQV:FLQHN:KXEXR, NDEGLEX:VPTGQ:RSVK-AA$^{17}$-AKV:RIKPHQGQH:KEVQV:LEEHS:QXEXR, RNVQXRP:TQVQL:RSVK-AA$^{17}$-AKV:EIVRKKPIF:KEVQV:TLEDH:LAXKXE, KIPKAXX:TQVEL:RPVQ-AA$^{17}$-RKI:DENEKVV:KKATV:DMEDH:GXGXR, GIPEPXX:TQVKM:RPVQ-AA$^{17}$-RKI:DENKNVV:KKATV:NMEDH:SXAXR, SIPKAXX:TQVEL:RPVQ-AA$^{17}$-RKI:DEYDKVV:KKATV:EMEDH:GXGXR, HVTKPTX:TQVKL:RPVQ-AA$^{17}$-RKI:DDSSNVI:KKATV:NMEDH:SXGXH, YVPKPXX:TQVKL:RPVQ-AA$^{17}$-RKI:DDSSNVI:KKATV:NMEDH:SXGXH, TVPKPXX:TQVQL:RPVQ-AA$^{17}$-RKI:DDSSNVI:KKATV:NMEDH:AXGXH, AVPKAXX:TQVKL:RPVQ-AA$^{17}$-RKI:DSSNNVI:KKATV:NMEDH:AXGXH, KVGKAXX:TQVKL:RPVQ-AA$^{17}$-RKI:DDMGVPT:KKATV:EGMSEDH:XGXR, KASKAXX:TQVKL:RPVQ-AA$^{17}$-RKI:DKGVVTY:KKATV:GMEDH:EXGXR, GSAGPXX:TQVKM:RPVQ-AA$^{17}$-RKI:NDKQQII:KKATV:GMEDH:RXGXS, AAPASXX:TQVRL:RPVQ-AA$^{17}$-RKI:DAANNVV:KKATV:DMEDH:AXGXR, STPPTXX:TQVRL:RPVQ-AA$^{17}$-RKI:DSANNVV:KKATV:DMEDH:SXGXR, HVPKPXX:TQVKL:RPVQ-AA$^{17}$-RKI:DDSSNVI:KKATV:NMEDH:SXGXH, RVPSTXX:TQVKT:RPVQ-AA$^{17}$-RKI:DNGRVLL:KKATV:MIEDH:XGXL, ASAAPXX:TQVAL:RPVQ-AA$^{17}$-RKI:VGRKPKV:KKATV:MIEDH:XKXS, ASASPXX:TQVDL:RPVQ-AA$^{17}$-RKI:IGKTPKI:KKATV:MIEDH:XKXS, ASASPXX:TQVDL:RPVQ-AA$^{17}$-RKI:VGRTPKV:KKATV:MVEDH:XKXS, NDEGLEX:TQVEE:RPVQ-AA$^{17}$-RKI:RIKPHQGQH:KKATV:FLEDH:KXEXR, NDEGLEX:TQVGQ:RPVQ-AA$^{17}$-RKI:RIKPHQGQH:KKATV:LEEDH:QXEXR, SSVKXQP:TQVHH:RPVQ-AA$^{17}$-RKI:EYVRKKPKL:KKATV:RLEDH:LEXAXA, KIPKAXX:VPTEL:RPVQ-AA$^{17}$-RKI:DENEKVV:KKATV:DMVVE:GXGXR, GIPEPXX:VPEKM:RPVQ-AA$^{17}$-RKI:DENKNVV:KKATV:NMTVE:SXAXR, SIPKAXX:VPTEL:RPVQ-AA$^{17}$-RKI:DEYDKVV:KKATV:EMVVE:GXGXR, HVTKPTX:APTKL:RPVQ-AA$^{17}$-RKI:DDSSNVI:KKATV:NMVVR:SXGXH, YVPKPXX:APTKL:RPVQ-AA$^{17}$-RKI:DDSSNVI:KKATV:NMVVR:SXGXH, TVPKPXX:APTQL:RPVQ-AA$^{17}$-RKI:DDSSNVI:KKATV:NMVVR:AXGXH, AVPKAXX:APTKL:RPVQ-AA$^{17}$-RKI:DSSNNVI:KKATV:NMVVK:AXGXH, KVGKAXX:VPTKL:RPVQ-AA$^{17}$-RKI:DDMGVPT:KKATV:EGMSVAE:XGXR, KASKAXX:VPTKL:RPVQ-AA$^{17}$-RKI:DKGVVTY:KKATV:GMAVS:EXGXR, GSAGPXX:TPTKM:RPVQ-AA$^{17}$-RKI:NDKQQII:KKATV:GMVVD:RXGXS, AAPASXX:VPARL:RPVQ-AA$^{17}$-RKI:DAANNVV:KKATV:DMVVE:AXGXR, STPPTXX:VPTRL:RPVQ-AA$^{17}$-RKI:DSANNVV:KKATV:DMVVE:SXGXR, HVPKPXX:APTKL:RPVQ-AA$^{17}$-RKI:DDSSNVI:KKATV:NMVVR:SXGXH, RVPSTXX:APVKT:RPVQ-AA$^{17}$-RKI:DNGRVLL:KKATV:MIVEE:XGXL, ASAAPXX:VPQAL:RPVQ-AA$^{17}$-RKI:VGRKPKV:KKATV:MIVRS:XKXS, ASASPXX:VSQDL:RPVQ-AA$^{17}$-RKI:IGKTPKI:KKATV:MIVKS:XKXS, ASASPXX:VPQDL:RPVQ-AA$^{17}$-RKI:VGRTPKV:KKATV:MVVKS:XKXS, NDEGLEX:VPTEE:RPVQ-AA$^{17}$-RKI:RIKPHQGQH:KKATV:FLQHN:KXEXR, NDEGLEX:VPTGQ:RPVQ-AA$^{17}$-RKI:RIKPHQGQH:KKATV:LEEHS:QXEXR and SSVKXQP:SRVHH:RPVQ-AA$^{17}$-RKI:EYVRKKPKL:KKATV:RLEEH:LEXAXA; and wherein AA$^{17}$ is selected from the group consisting of G, A, V, L, I, P, F, M, W, T and S (in particular is selected from the group consisting of M, I, L, V and T). More particularly, the heptuplet PEP7:PEP5:PEP12:LINKER:PEP2:PEP6:PEP8 is selected from the group consisting of GIPEPXX:VPTKM:SAIS_AA$^{17}$-LYL:DENKNVV:LKNYQ:NMVVE:SXAXR, HVTKPTX:VPTKL:SAIS-AA$^{17}$-LYL:DDSSNVI:LKNYQ:NMVVE:SXGXH, YVPKPXX:VPTKL:SAIS-AA$^{17}$-LYL:DDSSNVI:LKNYQ:NMVVE:SXGXH, TVPKPXX:VPTQL:SAIS-AA$^{17}$-LYL:DDSSNVI:LKNYQ:NMVVE:AXGXH, AVPKAXX:VPTKL:SAIS-AA$^{17}$-LYL:DSSNNVI:LKNYQ:NMVVE:AXGXH, KVGKAXX:VPTKL:SAIS-AA$^{17}$-LYL:DDMGVPT:LKNYQ:EGMSVVE:XGXR, KASKAXX:VPTKL:SAIS-AA$^{17}$-LYL:DKGVVTY:LKNYQ:GMVVE:EXGXR, GSAGPXX:VPTKM:SAIS-AA$^{17}$-LYL:NDKQQII:LKNYQ:GMVVE:RXGXS, AAPASXX:VPTRL:SAIS-AA$^{17}$-LYL:DAANNVV:LKNYQ:DMVVE:AXGXR, STPPTXX:VPTRL:SAIS-AA$^{17}$-LYL:DSANNVV:LKNYQ:DMVVE:SXGXR, HVPKPXX:VPTKL:SAIS-AA$^{17}$-LYL:DDSSNVI:LKNYQ:NMVVE:SXGXH, RVPSTXX:VPTKT:SAIS-AA$^{17}$-LYL:DNGRVLL:LKNYQ:MIVVE:XGXL, ASAAPXX:VPTAL:SAIS-AA$^{17}$-LYL:VGRKPKV:LKNYQ:MIVVE:XKXS, ASASPXX:VPTDL:SAIS-AA$^{17}$-LYL:IGKTPKI:LKNYQ:MIVVE:XKXS, ASASPXX:VPTDL:SAIS-AA$^{17}$-LYL:VGRTPKV:LKNYQ:MVVVE:XKXS, GIPEPXX:VPEKM:SAIS-AA$^{17}$-LYL:DENKNVV:LKNYQ:NMTVE:SXAXR, HVTKPTX:APTKL:SAIS-AA$^{17}$-LYL:DDSSNVI:LKNYQ:NMVVR:SXGXH, YVPKPXX:APTKL:SAIS-AA$^{17}$-LYL:DDSSNVI:LKNYQ:NMVVR:SXGXH, TVPKPXX:APTQL:SAIS-AA$^{17}$-LYL:DDSSNVI:LKNYQ:NMVVR:AXGXH, AVPKAXX:APTKL:SAIS-AA$^{17}$-LYL:DSSNNVI:LKNYQ:NMVVK:AXGXH, KVGKAXX:VPTKL:SAIS-AA$^{17}$-LYL:DDMGVPT:LKNYQ:EGMSVAE:XGXR, KASKAXX:VPTKL:SAIS-AA$^{17}$-LYL:DKGVVTY:LKNYQ:GMAVS:EXGXR, GSAGPXX:TPTKM:SAIS-AA$^{17}$-LYL:NDKQQII:LKNYQ:GMVVD:RXGXS, AAPASXX:VPARL:SAIS-AA$^{17}$-LYL:DAANNVV:LKNYQ:DMVVE:AXGXR, HVPKPXX:APTKL:SAIS-AA$^{17}$-LYL:DDSSNVI:LKNYQ:NMVVR:SXGXH, RVPSTXX:APVKT:SAIS-AA$^{17}$-LYL:DNGRVLL:LKNYQ:MIVEE:XGXL, ASAAPXX:VPQAL:SAIS-AA$^{17}$-LYL:VGRKPKV:LKNYQ:MIVRS:XKXS, ASASPXX:VSQDL:SAIS-AA$^{17}$-LYL:IGKTPKI:LKNYQ:MIVKS:XKXS, ASASPXX:VPQDL:SAIS-AA$^{17}$-LYL:VGRTPKV:LKNYQ:MVVKS:XKXS, NDEGLEX:VPTEE:SAIS-AA$^{17}$-LYL:RIKPHQGQH:LKNYQ:FLQHN:KXEXR, NDEGLEX:VPTGQ:SAIS-AA$^{17}$-LYL:RIKPHQGQH:LKNYQ:LEEHS:QXEXR, SSVKXQP:SRVHH:SAIS-AA$^{17}$-LYL:EYVRKKPKL:LKNYQ:RLEEH:LEXAXA, RNVQXRP:TQVQL:SAIS-AA$^{17}$-LYL:EIVRKKPIF:LKNYQ:TLEDH:LAXKXE, KIPKAXX:VPEEL:SSLS-AA$^{17}$-LFF:DENEKVV:LKVYP:DMTVE:GXGXR, SIPKAXX:VPEEL:SSLS-AA$^{17}$-LFF:DEYDKVV:LKVYP:EMTVE:GXGXR, HVTKPTX:VPEKL:SSLS-AA$^{17}$-LFF:DDSSNVI:LKVYP:NMTVE:SXGXH, YVPKPXX:VPEKL:SSLS-AA$^{17}$-LFF:DDSSNVI:LKVYP:NMTVE:SXGXH, TVPKPXX:VPEQL:SSLS-AA$^{17}$-LFF:DDSSN-

VI:LKVYP:NMTVE:AXGXH, AVPKAXXVPEKL:SSLS-AA$^{17}$-LFF:DSSNNVI:LKVYP:NMTVE:AXGXH, KVGKAXX:VPEKL:SSLS-AA$^{17}$-LFF:DDMGVPT:LKVYP:EGMSTVE:XGXR, KASKAXX:VPEKL:SSLS-AA$^{17}$-LFF:DKGVVTY:LKVYP:GMTVE:EXGXR, GSAGPXX:VPEKM:SSLS-AA$^{17}$-LFF:NDKQQII:LKVYP:GMTVE:RXGXS, AAPASXX:VPERL:SSLS-AA$^{17}$-LFF:DAANNVV:LKVYP:DMTVE:AXGXR, STPPTXX:VPERL:SSLS-AA$^{17}$-LFF:DSANNVV:LKVYP:DMTVE:SXGXR, HVPKPXX:VPEKL:SSLS-AA$^{17}$-LFF:DDSSNVI:LKVYP:NMTVE:SXGXH, RVPSTXX:VPEKT:SSLS-AA$^{17}$-LFF:DNGRVLL:LKVYP:MITVE:XGXL, ASAAPXX:VPEAL:SSLS-AA$^{17}$-LFF:VGRKPKV:LKVYP:MITVE:XKXS, ASASPXX:VPEDL:SSLS-AA$^{17}$-LFF:IGKTPKI:LKVYP:MITVE:XKXS, ASASPXX:VPEDL:SSLS-AA$^{17}$-LFF:VGRTPKV:LKVYP:MVTVE:XKXS, KIPKAXX:VPTEL:SSLS-AA$^{17}$-LFF:DENEKVV:LKVYP:DMVVE:GXGXR, SIPKAXX:VPTEL:SSLS-AA$^{17}$-LFF:DEYDKVV:LKVYP:EMVVE:GXGXR, HVTKPTX:APTKL:SSLS-AA$^{17}$-LFF:DDSSNVI:LKVYP:NMVVR:SXGXH, YVPKPXX:APTKL:SSLS-AA$^{17}$-LFF:DDSSNVI:LKVYP:NMVVR:SXGXH, TVPKPXX:APTQL:SSLS-AA$^{17}$-LFF:DDSSNVI:LKVYP:NMVVR:AXGXH, AVPKAXX:APTKL:SSLS-AA$^{17}$-LFF:DSSNNVI:LKVYP:NMVVK:AXGXH, KVGKAXX:VPTKL:SSLS-AA$^{17}$-LFF:DDMGVPT:LKVYP:EGMSVAE:XGXR, KASKAXX:VPTKL:SSLS-AA$^{17}$-LFF:DKGVVTY:LKVYP:GMAVS:EXGXR, GSAGPXX:TPTKM:SSLS-AA$^{17}$-LFF:NDKQQII:LKVYP:GMVVD:RXGXS, AAPASXX:VPARL:SSLS-AA$^{17}$-LFF:DAANNVV:LKVYP:DMVVE:AXGXR, STPPTXX:VPTRL:SSLS-AA$^{17}$-LFF:DSANNVV:LKVYP:DMVVE:SXGXR, HVPKPXX:APTKL:SSLS-AA$^{17}$-LFF:DDSSNVI:LKVYP:NMVVR:SXGXH, RVPSTXX:APVKT:SSLS-AA$^{17}$-LFF:DNGRVLL:LKVYP:MIVEE:XGXL, ASAAPXX:VPQAL:SSLS-AA$^{17}$-LFF:VGRKPKV:LKVYP:MIVRS:XKXS, ASASPXX:VSQDL:SSLS-AA$^{17}$-LFF:IGKTPKI:LKVYP:MIVKS:XKXS, ASASPXX:VPQDL:SSLS-AA$^{17}$-LFF:VGRTPKV:LKVYP:MVVKS:XKXS, NDEGLEX:VPTEE:SSLS-AA$^{17}$-LFF:RIKPHQGQH:LKVYP:FLQHN:KXEXR, NDEGLEX:VPTGQ:SSLS-AA$^{17}$-LFF:RIKPHQGQH:LKVYP:LEEHS:QXEXR, SSVKXQP:SRVHH:SSLS-AA$^{17}$-LFF:EYVRKKPKL:LKVYP:RLEEH:LEXAXA, RNVQXRP:TQVQL:SSLS-AA$^{17}$-LFF:EIVRKKPIF:LKVYP:TLEDH:LAXKXE, KIPKAXX:APTEL:NAIS-AA$^{17}$-LYF:DENEKVV:LKKYR:DMVVR:GXGXR, GIPEPXX:APTKM:NAIS-AA$^{17}$-LYF:DENKNVV:LKKYR:NMVVR:SXAXR, SIPKAXX:APTEL:NAIS-AA$^{17}$-LYF:DEYDKVV:LKKYR:EMVVR:GXGXR, AVPKAXX:APTKL:NAIS-AA$^{17}$-LYF:DSSNNVI:LKKYR:NMVVR:AXGXH, KVGKAXX:APTKL:NAIS-AA$^{17}$-LYF:DDMGVPT:LKKYR:EGMSVVR:XGXR, KASKAXX:APTKL:NAIS-AA$^{17}$-LYF:DKGVVTY:LKKYR:GMVVR:EXGXR, GSAGPXX:APTKM:NAIS-AA$^{17}$-LYF:NDKQQII:LKKYR:GMVVR:RXGXS, AAPASXX:APTRL:NAIS-AA$^{17}$-LYF:DAANNVV:LKKYR:DMVVR:AXGXR, STPPTXX:APTRL:NAIS-AA$^{17}$-LYF:DSANNVV:LKKYR:DMVVR:SXGXR, RVPSTXX:APTKT:NAIS-AA$^{17}$-LYF:DNGRVLL:LKKYR:MIVVR:XGXL, ASAAPXX:APTAL:NAIS-AA$^{17}$-LYF:VGRKPKV:LKKYR:MIVVR:XKXS, ASASPXX:APTDL:NAIS-AA$^{17}$-LYF:IGKTPKI:LKKYR:MIVVR:XKXS, ASASPXX:APTDL:NAIS-AA$^{17}$-LYF:VGRTPKV:LKKYR:MVVVR:XKXS, KIPKAXX:VPTEL:NAIS-AA$^{17}$-LYF:DENEKVV:LKKYR:DMVVE:GXGXR, GIPEPXX:VPEKM:NAIS-AA$^{17}$-LYF:DENKNVV:LKKYR:NMTVE:SXAXR, SIPKAXX:VPTEL:NAIS-AA$^{17}$-LYF:DEYDKVV:LKKYR:EMVVE:GXGXR, AVPKAXX:APTKL:NAIS-AA$^{17}$-LYF:DSSNNVI:LKKYR:NMVVK:AXGXH, KVGKAXX:VPTKL:NAIS-AA$^{17}$-LYF:DDMGVPT:LKKYR:EGMSVAE:XGXR, KASKAXX:VPTKL:NAIS-AA$^{17}$-LYF:DKGVVTY:LKKYR:GMAVS:EXGXR, GSAGPXX:TPTKM:NAIS-AA$^{17}$-LYF:NDKQQII:LKKYR:GMVVD:RXGXS, AAPASXX:VPARL:NAIS-AA$^{17}$-LYF:DAANNVV:LKKYR:DMVVE:AXGXR, STPPTXX:VPTRL:NAIS-AA$^{17}$-LYF:DSANNVV:LKKYR:DMVVE:SXGXR, RVPSTXX:APVKT:NAIS-AA$^{17}$-LYF:DNGRVLL:LKKYR:MIVEE:XGXL, ASAAPXX:VPQAL:NAIS-AA$^{17}$-LYF:VGRKPKV:LKKYR:MIVRS:XKXS, ASASPXX:VSQDL:NAIS-AA$^{17}$-LYF:IGKTPKI:LKKYR:MIVKS:XKXS, ASASPXX:VPQDL:NAIS-AA$^{17}$-LYF:VGRTPKV:LKKYR:MVVKS:XKXS, NDEGLEX:VPTEE:NAIS-AA$^{17}$-LYF:RIKPHQGQH:LKKYR:FLQHN:KXEXR, NDEGLEX:VPTGQ:NAIS-AA$^{17}$-LYF:RIKPHQGQH:LKKYR:LEEHS:QXEXR, SSVKXQP:SRVHH:NAIS-AA$^{17}$-LYF:EYVRKKPKL:LKKYR:RLEEH:LEXAXA, RNVQXRP:TQVQL:NAIS-AA$^{17}$-LYF:EIVRKKPIF:LKKYR:TLEDH:LAXKXE, KIPKAXX:APTEL:SATS-AA$^{17}$-LYY:DENEKVV:LRKHR:DMVVK:GXGXR, GIPEPXX:APTKM:SATS-AA$^{17}$-LYY:DENKNVV:LRKHR:NMVVK:SXAXR, SIPKAXX:APTEL:SATS-AA$^{17}$-LYY:DEYDKVV:LRKHR:EMVVK:GXGXR, HVTKPTX:APTKL:SATS-AA$^{17}$-LYY:DDSSNVI:LRKHR:NMVVK:SXGXH, YVPKPXX:APTKL:SATS-AA$^{17}$-LYY:DDSSNVI:LRKHR:NMVVK:SXGXH, TVPKPXX:APTQL:SATS-AA$^{17}$-LYY:DDSSNVI:LRKHR:NMVVK:AXGXH, KVGKAXX:APTKL:SATS-AA$^{17}$-LYY:DDMGVPT:LRKHR:EGMSVVK:XGXR, KASKAXX:APTKL:SATS-AA$^{17}$-LYY:DKGVVTY:LRKHR:GMVVK:EXGXR, GSAGPXX:APTKM:SATS-AA$^{17}$-LYY:NDKQQII:LRKHR:GMVVK:RXGXS, AAPASXX:APTRL:SATS-AA$^{17}$-LYY:DAANNVV:LRKHR:DMVVK:AXGXR, STPPTXX:APTRL:SATS-AA$^{17}$-LYY:DSANNVV:LRKHR:DMVVK:SXGXR, HVPKPXX:APTKL:SATS-AA$^{17}$-LYY:DDSSNVI:LRKHR:NMVVK:SXGXH, RVPSTXX:APTKT:SATS-AA$^{17}$-LYY:DNGRVLL:LRKHR:MIVVK:XGXL, ASAAPXX:APTAL:SATS-AA$^{17}$-LYY:VGRKPKV:LRKHR:MIVVK:XKXS, ASASPXX:APTDL:SATS-AA$^{17}$-LYY:IGKTPKI:LRKHR:MIVVK:XKXS, ASASPXX:APTDL:SATS-AA$^{17}$-LYY:VGRTPKV:LRKHR:MVVVK:XKXS, KIPKAXX:VPTEL:SATS-AA$^{17}$-LYY:DENEKVV:LRKHR:DMVVE:GXGXR, GIPEPXX:VPEKM:SATS-AA$^{17}$-LYY:DENKNVV:LRKHR:NMTVE:SXAXR, SIPKAXX:VPTEL:SATS-AA$^{17}$-LYY:DEYDKVV:LRKHR:EMVVE:GXGXR, HVTKPTX:APTKL:SATS-AA$^{17}$-LYY:DDSSNVI:LRKHR:NMVVR:SXGXH, YVPKPXX:APTKL:SATS-AA$^{17}$-LYY:DDSSNVI:LRKHR:NMVVR:SXGXH, TVPKPXX:APTQL:SATS-AA$^{17}$-LYY:DDSSNVI:LRKHR:NMVVR:AXGXH, KVGKAXX:VPTKL:SATS-AA$^{17}$-LYY:DDMGVPT:LRKHR:EGMSVAE:XGXR, KASKAXX:VPTKL:SATS-AA$^{17}$-LYY:DKGVVTY:LRKHR:GMAVS:EXGXR, GSAGPXX:TPTKM:SATS-AA$^{17}$-LYY:NDKQQII:LRKHR:GMVVD:RXGXS, AAPASXX:VPARL:SATS-AA$^{17}$-LYY:DAANNVV:LRKHR:DM-

VVE:AXGXR, STPPTXXVPTRL:SATS-AA$^{17}$-LYY:DSANNVV:LRKHR:DMVVE:SXGXR, HVPKPXX:APTKL:SATS-AA$^{17}$-LYY:DDSSNVI:LRKHR:NMVVR:SXGXH, RVPSTXX:APVKT:SATS-AA$^{17}$-LYY:DNGRVLL:LRKHR:MIVEE:XGXL, ASAAPXX:VPQAL:SATS-AA$^{17}$-LYY:VGRKPKV:LRKHR:MIVRS:XKXS, ASASPXX:VSQDL:SATS-AA$^{17}$-LYY:IGKTPKI:LRKHR:MIVKS:XKXS, ASASPXX:VPQDL:SATS-AA$^{17}$-LYY:VGRTPKV:LRKHR:MVVKS:XKXS, NDEGLEX:VPTEE:SATS-AA$^{17}$-LYY:RIKPHQGQH:LRKHR:FLQHN:KXEXR, NDEGLEX:VPTGQ:SATS-AA$^{17}$-LYY:RIKPHQGQH:LRKHR:LEEHS:QXEXR, SSVKXQP:SRVHH:SATS-AA$^{17}$-LYY:EYVRKKPKL:LRKHR:RLEEH:LEXAXA, RNVQXRP:TQVQL:SATS-AA$^{17}$-LYY:EIVRKKPIF:LRKHR:TLEDH:LAXKXE, KIPKAXX:VPTEL:SPIS-AA$^{17}$-LYK:DENEKVV:LKYHY:DMVAE:GXGXR, GIPEPXX:VPTKM:SPIS-AA$^{17}$-LYK:DENKNVV:LKYHY:NMVAE:SXAXR, SIPKAXX:VPTEL:SPIS-AA$^{17}$-LYK:DEYDKVV:LKYHY:EMVAE:GXGXR, HVTKPTX:VPTKL:SPIS-AA$^{17}$-LYK:DDSSNVI:LKYHY:N MVAE:SXGXH, YVPKPXX:VPTKL:SPIS-AA$^{17}$-LYK:DDSSNVI:LKYHY:NMVAE:SXGXH, TVPKPXX:VPTQL:SPIS-AA$^{17}$-LYK:DDSSNVI:LKYHY:NMVAE:AXGXH, AVPKAXX:VPTKL:SPIS-AA$^{17}$-LYK:DSSNNVI:LKYHY:NMVAE:AXGXH, KASKAXX:VPTKL:SPIS-AA$^{17}$-LYK:DKGVVTY:LKYHY:GMVAE:EXGXR, GSAGPXX:VPTKM:SPIS-AA$^{17}$-LYK:NDKQQII:LKYHY:GMVAE:RXGXS, AAPASXX:VPTRL:SPIS-AA$^{17}$-LYK:DAANNVV:LKYHY:DMVAE:AXGXR, STPPTXX:VPTRL:SPIS-AA$^{17}$-LYK:DSANNVV:LKYHY:DMVAE:SXGXR, HVPKPXX:VPTKL:SPIS-AA$^{17}$-LYK:DDSSNVI:LKYHY:NMVAE:SXGXH, RVPSTXX:VPTKT:SPIS-AA$^{17}$-LYK:DNGRVLL:LKYHY:MIVAE:XGXL, ASAAPXX:VPTAL:SPIS-AA$^{17}$-LYK:VGRKPKV:LKYHY:MIVAE:XKXS, ASASPXX:VPTDL:SPIS-AA$^{17}$-LYK:IGKTPKI:LKYHY:MIVAE:XKXS, ASASPXX:VPTDL:SPIS-AA$^{17}$-LYK:VGRTPKV:LKYHY:MVVAE:XKXS, KIPKAXX:VPTEL:SPIS-AA$^{17}$-LYK:DENEKVV:LKYHY:DMVVE:GXGXR, GIPEPXX:VPEKM:SPIS-AA$^{17}$-LYK:DENKNVV:LKYHY:NMTVE:SXAXR, SIPKAXX:VPTEL:SPIS-AA$^{17}$-LYK:DEYDKVV:LKYHY:EMVVE:GXGXR, HVTKPTX:APTKL:SPIS-AA$^{17}$-LYK:DDSSNVI:LKYHY:N MVVR:SXGXH, YVPKPXX:APTKL:SPIS-AA$^{17}$-LYK:DDSSNVI:LKYHY:N MVVR:SXGXH, TVPKPXX:APTQL:SPIS-AA$^{17}$-LYK:DDSSNVI:LKYHY:N MVVR:AXGXH, AVPKAXX:APTKL:SPIS-AA$^{17}$-LYK:DSSNNVI:LKYHY:NMVVK:AXGXH, KASKAXX:VPTKL:SPIS-AA$^{17}$-LYK:DKGVVTY:LKYHY:GMAVS:EXGXR, GSAGPXX:TPTKM:SPIS-AA$^{17}$-LYK:NDKQQII:LKYHY:GMVVD:RXGXS, AAPASXX:VPARL:SPIS-AA$^{17}$-LYK:DAANNVV:LKYHY:DMVVE:AXGXR, STPPTXX:VPTRL:SPIS-AA$^{17}$-LYK:DSANNVV:LKYHY:DMVVE:SXGXR, HVPKPXX:APTKL:SPIS-AA$^{17}$-LYK:DDSSNVI:LKYHY:N MVVR:SXGXH, RVPSTXX:APVKT:SPIS-AA$^{17}$-LYK:DNGRVLL:LKYHY:MIVEE:XGXL, ASAAPXX:VPQAL:SPIS-AA$^{17}$-LYK:VGRKPKV:LKYHY:MIVRS:XKXS, ASASPXX:VSQDL:SPIS-AA$^{17}$-LYK:IGKTPKI:LKYHY:MIVKS:XKXS, ASASPXX:VPQDL:SPIS-AA$^{17}$-LYK:VGRTPKV:LKYHY:MVVKS:XKXS, NDEGLEX:VPTEE:SPIS-AA$^{17}$-LYK:RIKPHQGQH:LKYHY:FLQHN:KXEXR, NDEGLEX:VPTGQ:SPIS-AA$^{17}$-LYK:RIKPHQGQH:LKYHY:LEEHS:QXEXR, SSVKXQP:SRVHH:SPIS-AA$^{17}$-LYK:EYVRKKPKL:LKYHY:RLEEH:LEXAXA, RNVQXRP:TQVQL:SPIS-AA$^{17}$-LYK:EIVRKKPIF:LKYHY:TLEDH:LAXKXE, KIPKAXX:VPTEL:EPIS-AA$^{17}$-LYL:DENEKVV:KFKYE:DMAVS:GXGXR, GIPEPXX:VPTKM:EPIS-AA$^{17}$-LYL:DENKNVV:KFKYE:NMAVS:SXAXR, SIPKAXX:VPTEL:EPIS-AA$^{17}$-LYL:DEYDKVV:KFKYE:EMAVS:GXGXR, HVTKPTX:VPTKL:EPIS-AA$^{17}$-LYL:DDSSNVI:KFKYE:NMAVS:SXGXH, YVPKPXX:VPTKL:EPIS-AA$^{17}$-LYL:DDSSNVI:KFKYE:NMAVS:SXGXH, TVPKPXX:VPTQL:EPIS-AA$^{17}$-LYL:DDSSNVI:KFKYE:NMAVS:AXGXH, AVPKAXX:VPTKL:EPIS-AA$^{17}$-LYL:DSSNNVI:KFKYE:NMAVS:AXGXH, KVGKAXX:VPTKL:EPIS-AA$^{17}$-LYL:DDMGVPT:KFKYE:EGMSAVS:XGXR, GSAGPXX:VPTKM:EPIS-AA$^{17}$-LYL:NDKQQII:KFKYE:GMAVS:RXGXS, AAPASXX:VPTRL:EPIS-AA$^{17}$-LYL:DAANNVV:KFKYE:DMAVS:AXGXR, STPPTXX:VPTRL:EPIS-AA$^{17}$-LYL:DSANNVV:KFKYE:DMAVS:SXGXR, HVPKPXX:VPTKL:EPIS-AA$^{17}$-LYL:DDSSNVI:KFKYE:NMAVS:SXGXH, RVPSTXX:VPTKT:EPIS-AA$^{17}$-LYL:DNGRVLL:KFKYE:MIAVS:XGXL, ASAAPXX:VPTAL:EPIS-AA$^{17}$-LYL:VGRKPKV:KFKYE:MIAVS:XKXS, ASASPXX:VPTDL:EPIS-AA$^{17}$-LYL:IGKTPKI:KFKYE:MIAVS:XKXS, ASASPXX:VPTDL:EPIS-AA$^{17}$-LYL:VGRTPKV:KFKYE:MVAVS:XKXS, KIPKAXX:VPTEL:EPIS-AA$^{17}$-LYL:DENEKVV:KFKYE:DMVVE:GXGXR, GIPEPXX:VPEKM:EPIS-AA$^{17}$-LYL:DENKNVV:KFKYE:NMTVE:SXAXR, SIPKAXX:VPTEL:EPIS-AA$^{17}$-LYL:DEYDKVV:KFKYE:EMVVE:GXGXR, HVTKPTX:APTKL:EPIS-AA$^{17}$-LYL:DDSSNVI:KFKYE:NMVVR:SXGXH, YVPKPXX:APTKL:EPIS-AA$^{17}$-LYL:DDSSNVI:KFKYE:NMVVR:SXGXH, TVPKPXX:APTQL:EPIS-AA$^{17}$-LYL:DDSSNVI:KFKYE:NMVVR:AXGXH, AVPKAXX:APTKL:EPIS-AA$^{17}$-LYL:DSSNNVI:KFKYE:NMVVK:AXGXH, KVGKAXX:VPTKL:EPIS-AA$^{17}$-LYL:DDMGVPT:KFKYE:EGMSVAE:XGXR, GSAGPXX:TPTKM:EPIS-AA$^{17}$-LYL:NDKQQII:KFKYE:GMVVD:RXGXS, AAPASXX:VPARL:EPIS-AA$^{17}$-LYL:DAANNVV:KFKYE:DMVVE:AXGXR, STPPTXX:VPTRL:EPIS-AA$^{17}$-LYL:DSANNVV:KFKYE:DMVVE:SXGXR, HVPKPXX:APTKL:EPIS-AA$^{17}$-LYL:DDSSNVI:KFKYE:NMVVR:SXGXH, RVPSTXX:APVKT:EPIS-AA$^{17}$-LYL:DNGRVLL:KFKYE:MIVEE:XGXL, ASAAPXX:VPQAL:EPIS-AA$^{17}$-LYL:VGRKPKV:KFKYE:MIVRS:XKXS, ASASPXX:VSQDL:EPIS-AA$^{17}$-LYL:IGKTPKI:KFKYE:MIVKS:XKXS, ASASPXX:VPQDL:EPIS-AA$^{17}$-LYL:VGRTPKV:KFKYE:MVVKS:XKXS, NDEGLEX:VPTEE:EPIS-AA$^{17}$-LYL:RIKPHQGQH:KFKYE:FLQHN:KXEXR, NDEGLEX:VPTGQ:EPIS-AA$^{17}$-LYL:RIKPHQGQH:KFKYE:LEEHS:QXEXR, SSVKXQP:SRVHH:EPIS-AA$^{17}$-LYL:EYVRKKPKL:KFKYE:RLEEH:LEXAXA, RNVQXRP:TQVQL:EPIS-AA$^{17}$-LYL:EIVRKKPIF:KFKYE:TLEDH:LAXKXE, KIPKAXX:TPTEL:SPIN-AA$^{17}$-LYF:DENEKVV:YGKIP:DMVVD:GXGXR, GIPEPXX:TPTKM:SPIN-AA$^{17}$-LYF:DENKNVV:YGKIP:NMVVD:SXAXR, SIPKAXX:TPTEL:SPIN-AA$^{17}$-LYF:DEYDKVV:YGKIP:EMVVD:GXGXR, HVTKPTX:TPTKL:SPIN-AA$^{17}$-LYF:DDSSNVI:YGKIP:NMVVD:SXGXH, YVPKPXX:TPTKL:SPIN-AA$^{17}$-LYF:DDSSNVI:YGKIP:NMVVD:SXGXH,

TVPKPXX:TPTQL:SPIN-AA$^{17}$-LYF:DDSSNVI:YGKIP:NMVVD:AXGXH, AVPKAXX:TPTKL:SPIN-AA$^{17}$-LYF:DSSNNVI:YGKIP:NMVVD:AXGXH, KVGKAXX:TPTKL:SPIN-AA$^{17}$-LYF:DDMGVPT:YGKIP:EGMSVVD:XGXR, KASKAXX:TPTKL:SPIN-AA$^{17}$-LYF:DKGVVTY:YGKIP:GMVVD:EXGXR, AAPASXX:TPTRL:SPIN-AA$^{17}$-LYF:DAANNVV:YGKIP:DMVVD:AXGXR, STPPTXX:TPTRL:SPIN-AA$^{17}$-LYF:DSANNVV:YGKIP:DMVVD:SXGXR, HVPKPXX:TPTKL:SPIN-AA$^{17}$-LYF:DDSSNVI:YGKIP:NMVVD:SXGXH, RVPSTXX:TPTKT:SPIN-AA$^{17}$-LYF:DNGRVLL:YGKIP:MIVVD:XGXL, ASAAPXX:TPTAL:SPIN-AA$^{17}$-LYF:VGRKPKV:YGKIP:MIVVD:XKXS, ASASPXX:TPTDL:SPIN-AA$^{17}$-LYF:IGKTPKI:YGKIP:MIVVD:XKXS, ASASPXX:TPTDL:SPIN-AA$^{17}$-LYF:VGRTPKV:YGKIP:MVVVD:XKXS, KIPKAXX:VPTEL:SPIN-AA$^{17}$-LYF:DENEKVV:YGKIP:DMVVE:GXGXR, GIPEPXX:VPEKM:SPIN-AA$^{17}$-LYF:DENKNVV:YGKIP:NMTVE:SXAXR, SIPKAXX:VPTEL:SPIN-AA$^{17}$-LYF:DEYDKVV:YGKIP:EMVVE:GXGXR, HVTKPTX:APTKL:SPIN-AA$^{17}$-LYF:DDSSNVI:YGKIP:NMVVR:SXGXH, YVPKPXX:APTKL:SPIN-AA$^{17}$-LYF:DDSSNVI:YGKIP:NMVVR:SXGXH, TVPKPXX:APTQL:SPIN-AA$^{17}$-LYF:DDSSNVI:YGKIP:NMVVR:AXGXH, AVPKAXX:APTKL:SPIN-AA$^{17}$-LYF:DSSNNVI:YGKIP:NMVVK:AXGXH, KVGKAXX:VPTKL:SPIN-AA$^{17}$-LYF:DDMGVPT:YGKIP:EGMSVAE:XGXR, KASKAXX:VPTKL:SPIN-AA$^{17}$-LYF:DKGVVTY:YGKIP:GMAVS:EXGXR, AAPASXX:VPARL:SPIN-AA$^{17}$-LYF:DAANNVV:YGKIP:DMVVE:AXGXR, STPPTXX:VPTRL:SPIN-AA$^{17}$-LYF:DSANNVV:YGKIP:DMVVE:SXGXR, HVPKPXX:APTKL:SPIN-AA$^{17}$-LYF:DDSSNVI:YGKIP:NMVVR:SXGXH, RVPSTXX:APVKT:SPIN-AA$^{17}$-LYF:DNGRVLL:YGKIP:MIVEE:XGXL, ASAAPXX:VPQAL:SPIN-AA$^{17}$-LYF:VGRKPKV:YGKIP:MIVRS:XKXS, ASASPXX:VSQDL:SPIN-AA$^{17}$-LYF:IGKTPKI:YGKIP:MIVKS:XKXS, ASASPXX:VPQDL:SPIN-AA$^{17}$-LYF:VGRTPKV:YGKIP:MVVKS:XKXS, NDEGLEX:VPTEE:SPIN-AA$^{17}$-LYF:RIKPHQGQH:YGKIP:FLQHN:KXEXR, NDEGLEXVPTGQ:SPIN-AA$^{17}$-LYF:RIKPHQGQH:YGKIP:LEEHS:QXEXR, SSVKXQP:SRVHH:SPIN-AA$^{17}$-LYF:EYVRKKPKL:YGKIP:RLEEH:LEXAXA, RNVQXRP:TQVQL:SPIN-AA$^{17}$-LYF:EIVRKKPIF:YGKIP:TLEDH:LAXKXE, KIPKAXX:VPAEL:SPIS-AA$^{17}$-LYI:DENEKVV:YKQYE:DMVVE:GXGXR, GIPEPXX:VPAKM:SPIS-AA$^{17}$-LYI:DENKNVV:YKQYE:NMVVE:SXAXR, SIPKAXX:VPAEL:SPIS-AA$^{17}$-LYI:DEYDKVV:YKQYE:EMVVE:GXGXR, HVTKPTX:VPAKL:SPIS-AA$^{17}$-LYI:DDSSNVI:YKQYE:NMVVE:SXGXH, YVPKPXX:VPAKL:SPIS-AA$^{17}$-LYI:DDSSNVI:YKQYE:NMVVE:SXGXH, TVPKPXX:VPAQL:SPIS-AA$^{17}$-LYI:DDSSNVI:YKQYE:NMVVE:AXGXH, AVPKAXX:VPAKL:SPIS-AA$^{17}$-LYI:DSSNNVI:YKQYE:NMVVE:AXGXH, KVGKAXX:VPAKL:SPIS-AA$^{17}$-LYI:DDMGVPT:YKQYE:EGMSVVE:XGXR, KASKAXX:VPAKL:SPIS-AA$^{17}$-LYI:DKGVVTY:YKQYE:GMVVE:EXGXR, GSAGPXX:VPAKM:SPIS-AA$^{17}$-LYI:NDKQQII:YKQYE:GMVVE:RXGXS, STPPTXX:VPARL:SPIS-AA$^{17}$-LYI:DSANNVV:YKQYE:DMVVE:SXGXR, HVPKPXX:VPAKL:SPIS-AA$^{17}$-LYI:DDSSNVI:YKQYE:NMVVE:SXGXH, RVPSTXX:VPAKT:SPIS-AA$^{17}$-LYI:DNGRVLL:YKQYE:MIVVE:XGXL, ASAAPXX:VPAAL:SPIS-AA$^{17}$-LYI:VGRKPKV:YKQYE:MIVVE:XKXS, ASASPXX:VPADL:SPIS-AA$^{17}$-LYI:IGKTPKI:YKQYE:MIVVE:XKXS, ASASPXX:VPADL:SPIS-AA$^{17}$-LYI:VGRTPKV:YKQYE:MVVVE:XKXS, KIPKAXX:VPTEL:SPIS-AA$^{17}$-LYI:DENEKVV:YKQYE:DMVVE:GXGXR, GIPEPXX:VPEKM:SPIS-AA$^{17}$-LYI:DENKNVV:YKQYE:NMTVE:SXAXR, SIPKAXX:VPTEL:SPIS-AA$^{17}$-LYI:DEYDKVV:YKQYE:EMVVE:GXGXR, HVTKPTX:APTKL:SPIS-AA$^{17}$-LYI:DDSSNVI:YKQYE:NMVVR:SXGXH, YVPKPXX:APTKL:SPIS-AA$^{17}$-LYI:DDSSNVI:YKQYE:NMVVR:SXGXH, TVPKPXX:APTQL:SPIS-AA$^{17}$-LYI:DDSSNVI:YKQYE:NMVVR:AXGXH, AVPKAXX:APTKL:SPIS-AA$^{17}$-LYI:DSSNNVI:YKQYE:NMVVK:AXGXH, KVGKAXX:VPTKL:SPIS-AA$^{17}$-LYI:DDMGVPT:YKQYE:EGMSVAE:XGXR, KASKAXX:VPTKL:SPIS-AA$^{17}$-LYI:DKGVVTY:YKQYE:GMAVS:EXGXR, GSAGPXX:TPTKM:SPIS-AA$^{17}$-LYI:NDKQQII:YKQYE:GMVVD:RXGXS, STPPTXX:VPTRL:SPIS-AA$^{17}$-LYI:DSANNVV:YKQYE:DMVVE:SXGXR, HVPKPXX:APTKL:SPIS-AA$^{17}$-LYI:DDSSNVI:YKQYE:NMVVR:SXGXH, RVPSTXX:APVKT:SPIS-AA$^{17}$-LYI:DNGRVLL:YKQYE:MIVEE:XGXL, ASAAPXX:VPQAL:SPIS-AA$^{17}$-LYI:VGRKPKV:YKQYE:MIVRS:XKXS, ASASPXX:VSQDL:SPIS-AA$^{17}$-LYI:IGKTPKI:YKQYE:MIVKS:XKXS, ASASPXX:VPQDL:SPIS-AA$^{17}$-LYI:VGRTPKV:YKQYE:MVVKS:XKXS, NDEGLEX:VPTEE:SPIS-AA$^{17}$-LYI:RIKPHQGQH:YKQYE:FLQHN:KXEXR, NDEGLEX:VPTGQ:SPIS-AA$^{17}$-LYI:RIKPHQGQH:YKQYE:LEEHS:QXEXR, SSVKXQP:SRVHH:SPIS-AA$^{17}$-LYI:EYVRKKPKL:YKQYE:RLEEH:LEXAXA, RNVQXRP:TQVQL:SPIS-AA$^{17}$-LYI:EIVRKKPIF:YKQYE:TLEDH:LAXKXE, KIPKAXX:VPTEL:SPIS-AA$^{17}$-LFI:DENEKVV:YKQYE:DMVVE:GXGXR, GIPEPXX:VPTKM:SPIS-AA$^{17}$-LFI:DENKNVV:YKQYE:NMVVE:SXAXR, SIPKAXX:VPTEL:SPIS-AA$^{17}$-LFI:DEYDKVV:YKQYE:EMVVE:GXGXR, HVTKPTX:VPTKL:SPIS-AA$^{17}$-LFI:DDSSNVI:YKQYE:NMVVE:SXGXH, YVPKPXX:VPTKL:SPIS-AA$^{17}$-LFI:DDSSNVI:YKQYE:NMVVE:SXGXH, TVPKPXX:VPTQL:SPIS-AA$^{17}$-LFI:DDSSNVI:YKQYE:NMVVE:AXGXH, AVPKAXXVPTKL:SPIS-AA$^{17}$-LFI:DSSNNVI:YKQYE:NMVVE:AXGXH, KVGKAXX:VPTKL:SPIS-AA$^{17}$-LFI:DDMGVPT:YKQYE:EGMSVVE:XGXR, KASKAXX:VPTKL:SPIS-AA$^{17}$-LFI:DKGVVTY:YKQYE:GMVVE:EXGXR, GSAGPXX:VPTKM:SPIS-AA$^{17}$-LFI:NDKQQII:YKQYE:GMVVE:RXGXS, AAPASXX:VPTRL:SPIS-AA$^{17}$-LFI:DAANNVV:YKQYE:DMVVE:AXGXR, HVPKPXX:VPTKL:SPIS-AA$^{17}$-LFI:DDSSNVI:YKQYE:NMVVE:SXGXH, RVPSTXX:VPTKT:SPIS-AA$^{17}$-LFI:DNGRVLL:YKQYE:MIVVE:XGXL, ASAAPXX:VPTAL:SPIS-AA$^{17}$-LFI:VGRKPKV:YKQYE:MIVVE:XKXS, ASASPXX:VPTDL:SPIS-AA$^{17}$-LFI:IGKTPKI:YKQYE:MIVVE:XKXS, ASASPXX:VPTDL:SPIS-AA$^{17}$-LFI:VGRTPKV:YKQYE:MVVVE:XKXS, GIPEPXX:VPEKM:SPIS-AA$^{17}$-LFI:DENKNVV:YKQYE:NMTVE:SXAXR, HVTKPTX:APTKL:SPIS-AA$^{17}$-LFI:DDSSNVI:YKQYE:NMVVR:SXGXH, YVPKPXX:APTKL:SPIS-AA$^{17}$-LFI:DDSSNVI:YKQYE:NMVVR:SXGXH, TVPKPXX:APTQL:SPIS-AA$^{17}$-LFI:DDSSNVI:YKQYE:NMVVR:AXGXH, AVPKAXX:APTKL:SPIS-AA$^{17}$-LFI:DSSNN-

VI:YKQYE:NMVVK:AXGXH, KVGKAXX:VPTKL:SPIS-AA$^{17}$-LFI:DDMGVPT:YKQYE:EGMSVAE:XGXR, KASKAXX:VPTKL:SPIS-AA$^{17}$-LFI:DKGVVTY:YKQYE:GMAVS:EXGXR, GSAGPXX:TPTKM:SPIS-AA$^{17}$-LFI:NDKQQII:YKQYE:GMVVD:RXGXS, AAPASXX:VPARL:SPIS-AA$^{17}$-LFI:DAANNVV:YKQYE:DMVVE:AXGXR, HVPKPXX:APTKL:SPIS-AA$^{17}$-LFI:DDSSNVI:YKQYE:NMVVR:SXGXH, RVPSTXX:APVKT:SPIS-AA$^{17}$-LFI:DNGRVLL:YKQYE:MIVEE:XGXL, ASAAPXX:VPQAL:SPIS-AA$^{17}$-LFI:VGRKPKV:YKQYE:MIVRS:XKXS, ASASPXX:VSQDL:SPIS-AA$^{17}$-LFI:IGKTPKI:YKQYE:MIVKS:XKXS, ASASPXX:VPQDL:SPIS-AA$^{17}$-LFI:VGRTPKV:YKQYE:MVVKS:XKXS, NDEGLEX:VPTEE:SPIS-AA$^{17}$-LFI:RIKPHQGQH:YKQYE:FLQHN:KXEXR, NDEGLEX:VPTGQ:SPIS-AA$^{17}$-LFI:RIKPHQGQH:YKQYE:LEEHS:QXEXR, SSVKXQP:SRVHH:SPIS-AA$^{17}$-LFI:EYVRKKPKL:YKQYE:RLEEH:LEXAXA, RNVQXRP:TQVQL:SPIS-AA$^{17}$-LFI:EIVRKKPIF:YKQYE:TLEDH:LAXKXE, KIPKAXX:APVEL:KPLS-AA$^{17}$-LYV:DENEKVV:DHHKD:DMVEE:GXGXR, GIPEPXX:APVKM:KPLS-AA$^{17}$-LYV:DENKNVV:DHHKD:NMVEE:SXAXR, SIPKAXX:APVEL:KPLS-AA$^{17}$-LYV:DEYDKVV:DHHKD:EMVEE:GXGXR, HVTKPTX:APVKL:KPLS-AA$^{17}$-LYV:DDSSNVI:DHHKD:NMVEE:SXGXH, YVPKPXX:APVKL:KPLS-AA$^{17}$-LYV:DDSSNVI:DHHKD:NMVEE:SXGXH, TVPKPXX:APVQL:KPLS-AA$^{17}$-LYV:DDSSNVI:DHHKD:NMVEE:AXGXH, AVPKAXX:APVKL:KPLS-AA$^{17}$-LYV:DSSNNVI:DHHKD:NMVEE:AXGXH, KVGKAXX:APVKL:KPLS-AA$^{17}$-LYV:DDMGVPT:DHHKD:EGMSVEE:XGXR, KASKAXX:APVKL:KPLS-AA$^{17}$-LYV:DKGVVTY:DHHKD:GMVEE:EXGXR, GSAGPXX:APVKM:KPLS-AA$^{17}$-LYV:NDKQQII:DHHKD:GMVEE:RXGXS, AAPASXX:APVRL:KPLS-AA$^{17}$-LYV:DAANNVV:DHHKD:DMVEE:AXGXR, STPPTXX:APVRL:KPLS-AA$^{17}$-LYV:DSANNVV:DHHKD:DMVEE:SXGXR, HVPKPXX:APVKL:KPLS-AA$^{17}$-LYV:DDSSNVI:DHHKD:NMVEE:SXGXH, ASAAPXX:APVAL:KPLS-AA$^{17}$-LYV:VGRKPKV:DHHKD:MIVEE:XKXS, ASASPXX:APVDL:KPLS-AA$^{17}$-LYV:IGKTPKI:DHHKD:MIVEE:XKXS, ASASPXX:APVDL:KPLS-AA$^{17}$-LYV:VGRTPKV:DHHKD:MVVEE:XKXS, KIPKAXX:VPTEL:KPLS-AA$^{17}$-LYV:DENEKVV:DHHKD:DMVVE:GXGXR, GIPEPXX:VPEKM:KPLS-AA$^{17}$-LYV:DENKNVV:DHHKD:NMTVE:SXAXR, SIPKAXX:VPTEL:KPLS-AA$^{17}$-LYV:DEYDKVV:DHHKD:EMVVE:GXGXR, HVTKPTX:APTKL:KPLS-AA$^{17}$-LYV:DDSSNVI:DHHKD:NMVVR:SXGXH, YVPKPXX:APTKL:KPLS-AA$^{17}$-LYV:DDSSNVI:DHHKD:NMVVR:SXGXH, TVPKPXX:APTQL:KPLS-AA$^{17}$-LYV:DDSSNVI:DHHKD:NMVVR:AXGXH, AVPKAXX:APTKL:KPLS-AA$^{17}$-LYV:DSSNNVI:DHHKD:NMVVK:AXGXH, KVGKAXX:VPTKL:KPLS-AA$^{17}$-LYV:DDMGVPT:DHHKD:EGMSVAE:XGXR, KASKAXX:VPTKL:KPLS-AA$^{17}$-LYV:DKGVVTY:DHHKD:GMAVS:EXGXR, GSAGPXX:TPTKM:KPLS-AA$^{17}$-LYV:NDKQQII:DHHKD:GMVVD:RXGXS, AAPASXX:VPARL:KPLS-AA$^{17}$-LYV:DAANNVV:DHHKD:DMVVE:AXGXR, STPPTXX:VPTRL:KPLS-AA$^{17}$-LYV:DSANNVV:DHHKD:DMVVE:SXGXR, HVPKPXX:APTKL:KPLS-AA$^{17}$-LYV:DDSSNVI:DHHKD:NMVVR:SXGXH, ASAAPXX:VPQAL:KPLS-AA$^{17}$-LYV:VGRKPKV:DHHKD:MIVRS:XKXS, ASASPXX:VSQDL:KPLS-AA$^{17}$-LYV:IGKTPKI:DHHKD:MIVKS:XKXS, ASASPXX:VPQDL:KPLS-AA$^{17}$-LYV:VGRTPKV:DHHKD:MVVKS:XKXS, NDEGLEX:VPTEE:KPLS-AA$^{17}$-LYV:RIKPHQGQH:DHHKD:FLQHN:KXEXR, NDEGLEX:VPTGQ:KPLS-AA$^{17}$-LYV:RIKPHQGQH:DHHKD:LEEHS:QXEXR, SSVKXQP:SRVHH:KPLS-AA$^{17}$-LYV:EYVRKKPKL:DHHKD:RLEEH:LEXAXA, RNVQXRP:TQVQL:KPLS-AA$^{17}$-LYV:EIVRKKPIF:DHHKD:TLEDH:LAXKXE, KIPKAXX:VPQEL:EPLP-AA$^{17}$-VYY:DENEKVV:EQLSN:DMVRS:GXGXR, GIPEPXX:VPQKM:EPLP-AA$^{17}$-VYY:DENKNVV:EQLSN:NMVRS:SXAXR, SIPKAXX:VPQEL:EPLP-AA$^{17}$-VYY:DEYDKVV:EQLSN:EMVRS:GXGXR, HVTKPTX:VPQKL:EPLP-AA$^{17}$-VYY:DDSSNVI:EQLSN:NMVRS:SXGXH, YVPKPXX:VPQKL:EPLP-AA$^{17}$-VYY:DDSSNVI:EQLSN:NMVRS:SXGXH, TVPKPXX:VPQQL:EPLP-AA$^{17}$-VYY:DDSSNVI:EQLSN:NMVRS:AXGXH, AVPKAXX:VPQKL:EPLP-AA$^{17}$-VYY:DSSNNVI:EQLSN:NMVRS:AXGXH, KVGKAXX:VPQKL:EPLP-AA$^{17}$-VYY:DDMGVPT:EQLSN:EGMSVRS:XGXR, KASKAXX:VPQKL:EPLP-AA$^{17}$-VYY:DKGVVTY:EQLSN:GMVRS:EXGXR, GSAGPXXVPQKM:EPLP-AA$^{17}$-VYY:NDKQQII:EQLSN:GMVRS:RXGXS, AAPASXX:VPQRL:EPLP-AA$^{17}$-VYY:DAANNVV:EQLSN:DMVRS:AXGXR, STPPTXX:VPQRL:EPLP-AA$^{17}$-VYY:DSANNVV:EQLSN:DMVRS:SXGXR, HVPKPXX:VPQKL:EPLP-AA$^{17}$-VYY:DDSSNVI:EQLSN:NMVRS:SXGXH, RVPSTXX:VPQKT:EPLP-AA$^{17}$-VYY:DNGRVLL:EQLSN:MIVRS:XGXL, ASASPXX:VPQDL:EPLP-AA$^{17}$-VYY:IGKTPKI:EQLSN:MIVRS:XKXS, ASASPXX:VPQDL:EPLP-AA$^{17}$-VYY:VGRTPKV:EQLSN:MVVRS:XKXS, KIPKAXX:VPTEL:EPLP-AA$^{17}$-VYY:DENEKVV:EQLSN:DMVVE:GXGXR, GIPEPXX:VPEKM:EPLP-AA$^{17}$-VYY:DENKNVV:EQLSN:NMTVE:SXAXR, SIPKAXX:VPTEL:EPLP-AA$^{17}$-VYY:DEYDKVV:EQLSN:EMVVE:GXGXR, HVTKPTX:APTKL:EPLP-AA$^{17}$-VYY:DDSSNVI:EQLSN:NMVVR:SXGXH, YVPKPXX:APTKL:EPLP-AA$^{17}$-VYY:DDSSNVI:EQLSN:NMVVR:SXGXH, TVPKPXX:APTQL:EPLP-AA$^{17}$-VYY:DDSSNVI:EQLSN:NMVVR:AXGXH, AVPKAXX:APTKL:EPLP-AA$^{17}$-VYY:DSSNNVI:EQLSN:NMVVK:AXGXH, KVGKAXX:VPTKL:EPLP-AA$^{17}$-VYY:DDMGVPT:EQLSN:EGMSVAE:XGXR, KASKAXX:VPTKL:EPLP-AA$^{17}$-VYY:DKGVVTY:EQLSN:GMAVS:EXGXR, GSAGPXX:TPTKM:EPLP-AA$^{17}$-VYY:NDKQQII:EQLSN:GMVVD:RXGXS, AAPASXX:VPARL:EPLP-AA$^{17}$-VYY:DAANNVV:EQLSN:DMVVE:AXGXR, STPPTXX:VPTRL:EPLP-AA$^{17}$-VYY:DSANNVV:EQLSN:DMVVE:SXGXR, HVPKPXX:APTKL:EPLP-AA$^{17}$-VYY:DDSSNVI:EQLSN:NMVVR:SXGXH, RVPSTXX:APVKT:EPLP-AA$^{17}$-VYY:DNGRVLL:EQLSN:MIVEE:XGXL, ASASPXX:VSQDL:EPLP-AA$^{17}$-VYY:IGKTPKI:EQLSN:MIVKS:XKXS, ASASPXX:VPQDL:EPLP-AA$^{17}$-VYY:VGRTPKV:EQLSN:MVVKS:XKXS, NDEGLEX:VPTEE:EPLP-AA$^{17}$-VYY:RIKPHQGQH:EQLSN:FLQHN:KXEXR, NDEGLEX:VPTGQ:EPLP-AA$^{17}$-VYY:RIKPHQGQH:EQLSN:LEEHS:QXEXR, SSVKXQP:SRVHH:EPLP-AA$^{17}$-VYY:EYVRKKPKL:EQLSN:RLEEH:LEXAXA, RNVQXRP:TQVQL:EPLP-AA$^{17}$-VYY:EIVRKKPIF:EQLSN:TLEDH:LAXKXE, KIPKAXX:VSQEL:EPLT-AA$^{17}$-LYY:DENEKVV:EQLSN:DM-

VKS:GXGXR, GIPEPXX:VSQKM:EPLT-AA17-LYY:DENKNVV:EQLSN:NMVKS:SXAXR, SIPKAXX:VSQEL:EPLT-AA17-LYY:DEYDKVV:EQLSN:EMVKS:GXGXR, HVTKPTX:VSQKL:EPLT-AA17-LYY:DDSSNVI:EQLSN:NMVKS:SXGXH, YVPKPXX:VSQKL:EPLT-AA17-LYY:DDSSNVI:EQLSN:NMVKS:SXGXH, TVPKPXX:VSQQL:EPLT-AA17-LYY:DDSSNVI:EQLSN:NMVKS:AXGXH, AVPKAXX:VSQKL:EPLT-AA17-LYY:DSSNNVI:EQLSN:NMVKS:AXGXH, KVGKAXX:VSQKL:EPLT-AA17-LYY:DDMGVPT:EQLSN:EGMSVKS:XGXR, KASKAXX:VSQKL:EPLT-AA17-LYY:DKGVVTY:EQLSN:GMVKS:EXGXR, GSAGPXX:VSQKM:EPLT-AA17-LYY:NDKQQII:EQLSN:GMVKS:RXGXS, AAPASXX:VSQRL:EPLT-AA17-LYY:DAANNVV:EQLSN:DMVKS:AXGXR, STPPTXX:VSQRL:EPLT-AA17-LYY:DSANNVV:EQLSN:DMVKS:SXGXR, HVPKPXX:VSQKL:EPLT-AA17-LYY:DDSSNVI:EQLSN:NMVKS:SXGXH, RVPSTXX:VSQKT:EPLT-AA17-LYY:DNGRVLL:EQLSN:MIVKS:XGXL, ASAAPXX:VSQAL:EPLT-AA17-LYY:VGRKPKV:EQLSN:MIVKS:XKXS, ASASPXX:VSQDL:EPLT-AA17-LYY:VGRTPKV:EQLSN:MVVKS:XKXS, KIPKAXX:VPTEL:EPLT-AA17-LYY:DENEKVV:EQLSN:DMVVE:GXGXR, GIPEPXX:VPEKM:EPLT-AA17-LYY:DENKNVV:EQLSN:NMTVE:SXAXR, SIPKAXX:VPTEL:EPLT-AA17-LYY:DEYDKVV:EQLSN:EMVVE:GXGXR, HVTKPTX:APTKL:EPLT-AA17-LYY:DDSSNVI:EQLSN:NMVVR:SXGXH, YVPKPXX:APTKL:EPLT-AA17-LYY:DDSSNVI:EQLSN:NMVVR:SXGXH, TVPKPXX:APTQL:EPLT-AA17-LYY:DDSSNVI:EQLSN:NMVVR:AXGXH, AVPKAXX:APTKL:EPLT-AA17-LYY:DSSNNVI:EQLSN:NMVVK:AXGXH, KVGKAXX:VPTKL:EPLT-AA17-LYY:DDMGVPT:EQLSN:EGMSVAE:XGXR, KASKAXX:VPTKL:EPLT-AA17-LYY:DKGVVTY:EQLSN:GMAVS:EXGXR, GSAGPXX:TPTKM:EPLT-AA17-LYY:NDKQQII:EQLSN:GMVVD:RXGXS, AAPASXX:VPARL:EPLT-AA17-LYY:DAANNVV:EQLSN:DMVVE:AXGXR, STPPTXX:VPTRL:EPLT-AA17-LYY:DSANNVV:EQLSN:DMVVE:SXGXR, HVPKPXX:APTKL:EPLT-AA17-LYY:DDSSNVI:EQLSN:NMVVR:SXGXH, RVPSTXX:APVKT:EPLT-AA17-LYY:DNGRVLL:EQLSN:MIVEE:XGXL, ASAAPXX:VPQAL:EPLT-AA17-LYY:VGRKPKV:EQLSN:MIVRS:XKXS, NDEGLEX:VPTEE:EPLT-AA17-LYY:RIKPHQGQH:EQLSN:FLQHN:KXEXR, NDEGLEX:VPTGQ:EPLT-AA17-LYY:RIKPHQGQH:EQLSN:LEEHS:QXEXR, SSVKXQP:SRVHH:EPLT-AA17-LYY:EYVRKKPKL:EQLSN:RLEEH:LEXAXA, RNVQXRP:TQVQL:EPLT-AA17-LYY:EIVRKKPIF:EQLSN:TLEDH:LAXKXE, KIPKAXX:VPQEL:EPLT-AA17-LYY:DENEKVV:EQLSN:DMVKS:GXGXR, GIPEPXX:VPQKM:EPLT-AA17-LYY:DENKNVV:EQLSN:NMVKS:SXAXR, SIPKAXX:VPQEL:EPLT-AA17-LYY:DEYDKVV:EQLSN:EMVKS:GXGXR, HVTKPTX:VPQKL:EPLT-AA17-LYY:DDSSNVI:EQLSN:NMVKS:SXGXH, YVPKPXX:VPQKL:EPLT-AA17-LYY:DDSSNVI:EQLSN:NMVKS:SXGXH, TVPKPXX:VPQQL:EPLT-AA17-LYY:DDSSNVI:EQLSN:NMVKS:AXGXH, AVPKAXX:VPQKL:EPLT-AA17-LYY:DSSNNVI:EQLSN:NMVKS:AXGXH, KVGKAXX:VPQKL:EPLT-AA17-LYY:DDMGVPT:EQLSN:EGMSVKS:XGXR, KASKAXX:VPQKL:EPLT-AA17-LYY:DKGVVTY:EQLSN:GMVKS:EXGXR, GSAGPXX:VPQKM:EPLT-AA17-LYY:NDKQQII:EQLSN:GMVKS:RXGXS, AAPASXX:VPQRL:EPLT-AA17-LYY:DAANNVV:EQLSN:DMVKS:AXGXR, STPPTXX:VPQRL:EPLT-AA17-LYY:DSANNVV:EQLSN:DMVKS:SXGXR, HVPKPXX:VPQKL:EPLT-AA17-LYY:DDSSNVI:EQLSN:NMVKS:SXGXH, RVPSTXX:VPQKT:EPLT-AA17-LYY:DNGRVLL:EQLSN:MIVKS:XGXL, ASAAPXX:VPQAL:EPLT-AA17-LYY:VGRKPKV:EQLSN:MIVKS:XKXS, ASASPXX:VPQDL:EPLT-AA17-LYY:IGKTPKI:EQLSN:MIVKS:XKXS, NDEGLEX:VPTGQ:SNIT-AA17-QIM:RIKPHQGQH:IGEMS:LEQHN:QXEXR, KIPKAXX:VPTEL:SNIT-AA17-QIM:DENEKVV:IGEMS:DMVVE:GXGXR, GIPEPXX:VPEKM:SNIT-AA17-QIM:DENKNVV:IGEMS:NMTVE:SXAXR, SIPKAXX:VPTEL:SNIT-AA17-QIM:DEYDKVV:IGEMS:EMVVE:GXGXR, HVTKPTX:APTKL:SNIT-AA17-QIM:DDSSNVI:IGEMS:NMVVR:SXGXH, YVPKPXX:APTKL:SNIT-AA17-QIM:DDSSNVI:IGEMS:NMVVR:SXGXH, TVPKPXX:APTQL:SNIT-AA17-QIM:DDSSNVI:IGEMS:NMVVR:AXGXH, AVPKAXX:APTKL:SNIT-AA17-QIM:DSSNNVI:IGEMS:NMVVK:AXGXH, KVGKAXX:VPTKL:SNIT-AA17-QIM:DDMGVPT:IGEMS:EGMSVAE:XGXR, KASKAXX:VPTKL:SNIT-AA17-QIM:DKGVVTY:IGEMS:GMAVS:EXGXR, GSAGPXX:TPTKM:SNIT-AA17-QIM:NDKQQII:IGEMS:GMVVD:RXGXS, AAPASXX:VPARL:SNIT-AA17-QIM:DAANNVV:IGEMS:DMVVE:AXGXR, STPPTXX:VPTRL:SNIT-AA17-QIM:DSANNVV:IGEMS:DMVVE:SXGXR, HVPKPXX:APTKL:SNIT-AA17-QIM:DDSSNVI:IGEMS:NMVVR:SXGXH, RVPSTXX:APVKT:SNIT-AA17-QIM:DNGRVLL:IGEMS:MIVEE:XGXL, ASAAPXXVPQAL:SNIT-AA17-QIM:VGRKPKV:IGEMS:MIVRS:XKXS, ASASPXX:VSQDL:SNIT-AA17-QIM:IGKTPKI:IGEMS:MIVKS:XKXS, ASASPXX:VPQDL:SNIT-AA17-QIM:VGRTPKV:IGEMS:MVVKS:XKXS, NDEGLEX:VPTGQ:SNIT-AA17-QIM:RIKPHQGQH:IGEMS:LEEHS:QXEXR, SSVKXQP:SRVHH:SNIT-AA17-QIM:EYVRKKPKL:IGEMS:RLEEH:LEXAXA, RNVQXRP:TQVQL:SNIT-AA17-QIM:EIVRKKPIF:IGEMS:TLEDH:LAXKXE, NDEGLEX:VPTEE:SNIT-AA17-QIM:RIKPHQGQH:LGEMS:FLEHS:KXEXR, KIPKAXX:VPTEL:SNIT-AA17-QIM:DENEKVV:LGEMS:DMVVE:GXGXR, GIPEPXX:VPEKM:SNIT-AA17-QIM:DENKNVV:LGEMS:NMTVE:SXAXR, SIPKAXX:VPTEL:SNIT-AA17-QIM:DEYDKVV:LGEMS:EMVVE:GXGXR, HVTKPTX:APTKL:SNIT-AA17-QIM:DDSSNVI:LGEMS:NMVVR:SXGXH, YVPKPXX:APTKL:SNIT-AA17-QIM:DDSSNVI:LGEMS:NMVVR:SXGXH, TVPKPXX:APTQL:SNIT-AA17-QIM:DDSSNVI:LGEMS:NMVVR:AXGXH, AVPKAXX:APTKL:SNIT-AA17-QIM:DSSNNVI:LGEMS:NMVVK:AXGXH, KVGKAXX:VPTKL:SNIT-AA17-QIM:DDMGVPT:LGEMS:EGMSVAE:XGXR, KASKAXXVPTKL:SNIT-AA17-QIM:DKGVVTY:LGEMS:GMAVS:EXGXR, GSAGPXX:TPTKM:SNIT-AA17-QIM:NDKQQII:LGEMS:GMVVD:RXGXS, AAPASXX:VPARL:SNIT-AA17-QIM:DAANNVV:LGEMS:DMVVE:AXGXR, STPPTXX:VPTRL:SNIT-AA17-QIM:DSANNVV:LGEMS:DMVVE:SXGXR, HVPKPXX:APTKL:SNIT-AA17-QIM:DDSSNVI:LGEMS:NMVVR:SXGXH, RVPSTXX:APVKT:SNIT-AA17-QIM:DNGRVLL:LGEMS:MIVEE:XGXL, ASAAPXX:VPQAL:SNIT-AA17-QIM:VGRKP-

KV:LGEMS:MIVRS:XKXS, ASASPXX:VSQDL:SNIT-AA$^{17}$-QIM:IGKTPKI:LGEMS:MIVKS:XKXS, ASASPXXVPQDL:SNIT-AA$^{17}$-QIM:VGRTPKV:LGEMS:MVVKS:XKXS, NDEGLEX:VPTEE:SNIT-AA$^{17}$-QIM:RIKPHQGQH:LGEMS:FLQHN:KXEXR, SSVKXQP:SRVHH:SNIT-AA$^{17}$-QIM:EYVRKKPKL:LGEMS:RLEEH:LEXAXA, RNVQXRP:TQVQL:SNIT-AA$^{17}$-QIM:EIVRKKPIF:LGEMS:TLEDH:LAXKXE, RNVQXRP:SRVQL:RSVK-AA$^{17}$-AKV:EIVRKKPIF:KEVQV:TLEEH:LAXKXE, KIPKAXX:VPTEL:RSVK-AA$^{17}$-AKV:DENEKVV:KEVQV:DMVVE:GXGXR, GIPEPXX:VPEKM:RSVK-AA$^{17}$-AKV:DENKNVV:KEVQV:NMTVE:SXAXR, SIPKAXX:VPTEL:RSVK-AA$^{17}$-AKV:DEYDKVV:KEVQV:EMVVE:GXGXR, HVTKPTX:APTKL:RSVK-AA$^{17}$-AKV:DDSSNVI:KEVQV:NMVVR:SXGXH, YVPKPXX:APTKL:RSVK-AA$^{17}$-AKV:DDSSNVI:KEVQV:NMVVR:SXGXH, TVPKPXX:APTQL:RSVK-AA$^{17}$-AKV:DDSSNVI:KEVQV:NMVVR:AXGXH, AVPKAXX:APTKL:RSVK-AA$^{17}$-AKV:DSSNNVI:KEVQV:NMVVK:AXGXH, KVGKAXX:VPTKL:RSVK-AA$^{17}$-AKV:DDMGVPT:KEVQV:EGMSVAE:XGXR, KASKAXX:VPTKL:RSVK-AA$^{17}$-AKV:DKGVVTY:KEVQV:GMAVS:EXGXR, GSAGPXX:TPTKM:RSVK-AA$^{17}$-AKV:NDKQQII:KEVQV:GMVVD:RXGXS, AAPASXX:VPARL:RSVK-AA$^{17}$-AKV:DAANNVV:KEVQV:DMVVE:AXGXR, STPPTXX:VPTRL:RSVK-AA$^{17}$-AKV:DSANNVV:KEVQV:DMVVE:SXGXR, HVPKPXX:APTKL:RSVK-AA$^{17}$-AKV:DDSSNVI:KEVQV:NMVVR:SXGXH, RVPSTXX:APVKT:RSVK-AA$^{17}$-AKV:DNGRVLL:KEVQV:MIVEE:XGXL, ASAAPXX:VPQAL:RSVK-AA$^{17}$-AKV:VGRKPKV:KEVQV:MIVRS:XKXS, ASASPXX:VSQDL:RSVK-AA$^{17}$-AKV:IGKTPKI:KEVQV:MIVKS:XKXS, ASASPXX:VPQDL:RSVK-AA$^{17}$-AKV:VGRTPKV:KEVQV:MVVKS:XKXS, NDEGLEX:VPTEE:RSVK-AA$^{17}$-AKV:RIKPHQGQH:KEVQV:FLQHN:KXEXR, NDEGLEX:VPTGQ:RSVK-AA$^{17}$-AKV:RIKPHQGQH:KEVQV:LEEHS:QXEXR, RNVQXRP:TQVQL:RSVK-AA$^{17}$-AKV:EIVRKKPIF:KEVQV:TLEDH:LAXKXE, SSVKXQP:TQVHH:RPVQ-AA$^{17}$-RKI:EYVRKKPKL:KKATV:RLEDH:LEXAXA, KIPKAXX:VPTEL:RPVQ-AA$^{17}$-RKI:DENEKVV:KKATV:DMVVE:GXGXR, GIPEPXXVPEKM:RPVQ-AA$^{17}$-RKI:DENKNVV:KKATV:NMTVE:SXAXR, SIPKAXX:VPTEL:RPVQ-AA$^{17}$-RKI:DEYDKVV:KKATV:EMVVE:GXGXR, HVTKPTX:APTKL:RPVQ-AA$^{17}$-RKI:DDSSNVI:KKATV:NMVVR:SXGXH, YVPKPXX:APTKL:RPVQ-AA$^{17}$-RKI:DDSSNVI:KKATV:NMVVR:SXGXH, TVPKPXX:APTQL:RPVQ-AA$^{17}$-RKI:DDSSNVI:KKATV:NMVVR:AXGXH, AVPKAXX:APTKL:RPVQ-AA$^{17}$-RKI:DSSNNVI:KKATV:NMVVK:AXGXH, KVGKAXX:VPTKL:RPVQ-AA$^{17}$-RKI:DDMGVPT:KKATV:EGMSVAE:XGXR, KASKAXX:VPTKL:RPVQ-AA$^{17}$-RKI:DKGVVTY:KKATV:GMAVS:EXGXR, GSAGPXX:TPTKM:RPVQ-AA$^{17}$-RKI:NDKQQII:KKATV:GMVVD:RXGXS, AAPASXX:VPARL:RPVQ-AA$^{17}$-RKI:DAANNVV:KKATV:DMVVE:AXGXR, STPPTXX:VPTRL:RPVQ-AA$^{17}$-RKI:DSANNVV:KKATV:DMVVE:SXGXR, HVPKPXX:APTKL:RPVQ-AA$^{17}$-RKI:DDSSNVI:KKATV:NMVVR:SXGXH, RVPSTXX:APVKT:RPVQ-AA$^{17}$-RKI:DNGRVLL:KKATV:MIVEE:XGXL, ASAAPXX:VPQAL:RPVQ-AA$^{17}$-RKI:VGRKPKV:KKATV:MIVRS:XKXS, ASASPXX:VSQDL:RPVQ-AA$^{17}$-RKI:IGKTPKI:KKATV:MIVKS:XKXS, ASASPXX:VPQDL:RPVQ-AA$^{17}$-RKI:VGRTPKV:KKATV:MVVKS:XKXS, NDEGLEX:VPTEE:RPVQ-AA$^{17}$-RKI:RIKPHQGQH:KKATV:FLQHN:KXEXR, NDEGLEX:VPTGQ:RPVQ-AA$^{17}$-RKI:RIKPHQGQH:KKATV:LEEHS:QXEXR and SSVKXQP:SRVHH:RPVQ-AA$^{17}$-RKI:EYVRKKPKL:KKATV:RLEEH:LEXAXA; and wherein AA$^{17}$ is selected from the group consisting of G, A, V, L, I, P, F, M, W, T and S (in particular is selected from the group consisting of M, I, L, V and T).

In one most particular example, the RMSD value of the three dimensional (3D) atomic coordinates of said GFR-binding compound as defined herein with respect to PEPREF is 2.45Å (Angstroms) or less, in particular is 2Å or less, and more particularly is 1.79Å or less, and wherein PEPREF is the set of 3D atomic coordinates already defined herein (hereinafter may be referred to as "wherein the RMSD is 2.45Å or less" for the sake of conciseness).

In one example, said GFR-binding compound is a synthetic molecule as defined herein in the definition section.

In one particular example, said GFR-binding compound is a synthetic peptide, or a variant or analog thereof, or a peptidomimetic.

In one most particular example, said GFR-binding compound is a non-cyclic synthetic peptide.

In one example, a length of said GFR-binding compound, in solution, such as in a physiologically acceptable solvent such as water or PBS, is comprised between about 6 and about 20 nm, preferably between about 6 and about 16 nm, as determined using the standard « 3D » procedure described above.

In one particular example, said GFR-binding compounds may be any one or a plurality of of peptides of SEQ ID NO: 1 to 38199.

*LINKER suitable for implementing non-cyclic GFR-compounds' embodiments*

In one particular example, said LINKER has a Mw comprised between about 28 and about 2,000 Da. In one particular example, said LINKER has a Mw comprised between about 75 and about 2,000 Da. In one particular example, said LINKER has a Mw comprised between about 150 and about 2,000 Da. In one particular example, said LINKER has a Mw comprised between about 150 and about 1,500 Da. In one most particular example, said LINKER has a Mw comprised between about 300 and about 1,000 Da.

The chemical nature of said LINKER is not meant to be particularly limited and may be any organic molecule capable of covalently connecting two peptide fragments such as PEP(A) and PEP(B) or PEP(C)-PEP12 and PEP2-PEP(D) so long as LINKER provides sufficient spacing between the two elements connected therethrough for these two connected elements to provide or conserve the required recoding activity. LINKER may thus be a peptide, or variant,

analog or peptidomimetic thereof, a polysaccharide, a polynucleotide, a saturated or unsaturated hydrocarbon chain, or a mixture thereof.

For example, in certain embodiments, LINKER is a peptide with 2 to 16 amino acids. In one particular example, LINKER is a peptide with 2 to 10 amino acids. In one particular example, LINKER is a peptide with 2 to 9 amino acids. In one most particular example, LINKER is a peptide with 5 to 9 amino acids.

Thus, in one particular example, said GFR-binding compound is a peptide, a variant or analog thereof as defined herein, with (exclusively consisting of, or constituted of) between 20 and 60 (in particular between 20 and 50, and more particularly, between 20 and 45) amino acids, of general formula (Ia); wherein one end of LINKER interacts covalently with one end of PEP(A); wherein another end of LINKER interacts covalently with one end of PEP(B); wherein PEP(A) comprises PEP1 or PEP12; wherein PEP(B) comprises PEP2; wherein LINKER is a peptide comprising 2 to 16 amino acids (in particular 2 to 10, 2 to 9, or 5 to 9 amino acids); and, optionally, wherein the RMSD is 2.45Å or less.

Thus, in one particular example, said GFR-binding compound is a peptide, a variant or analog thereof as defined herein, with (exclusively consisting of, or constituted of) between 25 and 60 (in particular between 25 and 50, and more particularly, between 25 and 45) amino acids, of general formula (IIa); wherein LINKER is a peptide comprising 2 to 16 amino acids (in particular 2 to 10, 2 to 9, or 5 to 9 amino acids); wherein PEP12 is a peptide with 8 amino acids of formula PEP1-$AA^{17}$-PEP11 as already defined herein; wherein PEP1, PEP2, PEP11, PEP(C) and PEP(D) are as already defined herein; wherein $AA^{13}$ and $AA^{21}$ may be both N-terminal amino acids or both C-terminal amino acids; wherein $AA^{25}$ and $AA^{20}$ may be both N-terminal amino acids or both C-terminal amino acids; wherein one end of LINKER interacts covalently with one end of PEP12 via $AA^{20}$; wherein another end of LINKER interacts covalently with PEP2 via $AA^{21}$; wherein one end of PEP(C) interacts covalently with PEP12 via $AA^{13}$; wherein one end of PEP(D) interacts covalently with PEP2 via $AA^{25}$; and, optionally, wherein the RMSD is 2.45Å or less.

In certain embodiments, said GFR-binding compound is a peptidomimetic as defined herein, comprising (consecutively or non consecutively) between 25 and 60 (in particular between 25 and 50, and more particularly, between 25 and 45) amino acids, of general formula (IIa); wherein LINKER is not a peptide but may comprise amino acids or peptides in covalent or non-covalent (preferably covalent) association with other groups or residues other than amino acids or peptides.

In one particular example, LINKER comprises (or is) a peptide of general formula (XXXV):

$$*AA^{201}\text{-}AA^{202}\text{-}AA^{203}\text{-}AA^{204}\text{-}AA^{205}\text{-}AA^{206}\text{-}AA^{207}\text{-}AA^{208}\text{-}AA^{209}** \qquad (XXXV)$$

wherein said peptide of formula (XXXV) may be selected from the group consisting of *$AA^{III}$-$AA^{I}$-$AA^{I}$-$AA^{II}$-$AA^{VII}$-$AA^{XII}$-$AA^{XII}$- $AA^{XIII}$-$AA^{XIII}$**, *$AA^{III}$-$AA^{I}$-$A^{VI}$-$AA^{II}$-$AA^{VII}$-$AA^{XII}$-$AA^{XII}$-$AA^{XIII}$-$AA^{XIII}$**, *$AA^{III}$-$AA^{I}$-$AA^{I}$-$AA^{II}$-$AA^{II}$-$AA^{XII}$-$AA^{XII}$-$AA^{XIII}$-$AA^{XIII}$**, *$AA^{III}$-$AA^{I}$-$AA^{II}$-$AA^{II}$-$AA^{VII}$-$AA^{XII}$-$AA^{XII}$-$AA^{XIII}$-$AA^{XIII}$**, *$AA^{III}$-$AA^{I}$-$AA^{II}$-$AA^{IV}$-$AA^{IV}$-$AA^{XII}$-$AA^{XII}$-$AA^{XIII}$-$AA^{XIII}$**, *$AA^{II}$-$AA^{I}$-$AA^{II}$-$AA^{II}$-$AA^{II}$-$AA^{XII}$-$AA^{XII}$-$AA^{XIII}$-$AAX^{III}$**, *$AA^{III}$-$AA^{III}$-$AA^{II}$-$AA^{VII}$-$AA^{II}$-$AA^{XII}$-$AA^{XII}$-$AA^{XIII}$-$AA^{XIII}$**, *$AA^{III}$-$AA^{I}$-$AA^{I}$-$AA^{II}$-$AA^{II}$-$AA^{XII}$-$AA^{XII}$-$AA^{XIII}$-$AA^{XIII}$**, *$AA^{V}$-$AA^{V}$-$AA^{VII}$-$AA^{VII}$-$AA^{XII}$-$AA^{XII}$-$AA^{XIII}$-$AA^{XIII}$**, *$AA^{VIII}$-$AA^{V}$-$AA^{VII}$-$AA^{II}$-$AA^{XII}$-$AA^{XII}$-$AA^{XIII}$-$AA^{XII}$** and *$AA^{V}$-$AA^{V}$-$AA^{VII}$-$AA^{II}$-$AA^{XII}$-$AA^{XII}$-$AA^{XIII}$-$AA^{XIII}$**; wherein $AA^{I}$ is any amino acid as defined herein, $AA^{II}$ is any polar amino acid as defined herein, $AA^{III}$ is any acidic amino acid as defined herein, $AA^{IV}$ is any aliphatic amino acid as defined herein, $AA^{V}$ is any apolar amino acid as defined herein, $AA^{VI}$ is any aromatic amino acid as defined herein, $AA^{VII}$ is any basic amino acid as defined herein, $AA^{VIII}$ is L or I as defined herein, $AA^{XII}$ is an amino acid selected from the group consisting of G, A, V, L, I, P, M, K, R, H, Y and E, wherein $AA^{XIII}$ is absent, $AA^{II}$ or $AA^{VII}$, preferably absent; and wherein any one of the fragment $AA^{201}$, $AA^{201}$-$AA^{202}$, $AA^{201}$-$AA^{202}$-$AA^{203}$, $AA^{201}$-$AA^{202}$-$AA^{203}$-$AA^{204}$, $AA^{201}$-$AA^{202}$-$AA^{203}$-$AA^{204}$-$AA^{205}$, $AA^{201}$-$AA^{202}$-$AA^{203}$-$AA^{204}$-$AA^{205}$-$AA^{206}$, $AA^{201}$-$AA^{202}$-$AA^{203}$-$AA^{204}$-$AA^{205}$-$AA^{206}$-$AA^{207}$, $AA^{203}$-$AA^{204}$-$AA^{205}$-$AA^{206}$-$AA^{207}$-$AA^{208}$-$AA^{209}$, $AA^{204}$-$AA^{205}$-$AA^{206}$-$AA^{207}$-$AA^{208}$-$AA^{209}$, $AA^{205}$-$AA^{206}$-$AA^{207}$-$AA^{208}$-$AA^{209}$, $AA^{206}$-$AA^{207}$-$AA^{208}$-$AA^{209}$, $AA^{207}$-$AA^{208}$-$AA^{209}$, $AA^{208}$-$AA^{209}$ or $AA^{209}$, may be absent. In the peptides of formula (XXXV), any one of the amino acid labelled "*" or the amino acid labelled "**" is an N-terminal amino acid and the other is a C-terminal amino acid.

For instance, in the peptide of formula *$AA^{III}$-$AA^{I}$-$AA^{I}$-$AA^{II}$-$AA^{VII}$-$AA^{XII}$-$AA^{XII}$-$AA^{XIII}$-$AA^{XIII}$** (XXXV-1), $AA^{III}$ occupies position $AA^{201}$, *$AA^{I}$ occupies position $AA^{202}$, $AA^{I}$ occupies position $AA^{203}$, $AA^{II}$ occupies position $AA^{204}$, $AA^{VII}$ occupies position $AA^{205}$, $AA^{XII}$ occupies position $AA^{206}$, $AA^{XII}$ occupies position $AA^{207}$, $AA^{XIII}$ occupies position $AA^{208}$ and $AA^{XIII}$** occupies position $AA^{209}$.

Likewise, in the peptide of formula *$AA^{V}$-$AA^{V}$-$AA^{VII}$-$AA^{II}$-$AA^{XII}$-$AA^{XII}$-$AA^{XIII}$-$AA^{XIII}$** (XXXV-1) $AA^{V}$ occupies position $AA^{201}$, $AA^{V}$ occupies position $AA^{202}$, $AA^{VII}$ occupies position $AA^{203}$, $AA^{II}$ occupies position $AA^{204}$, $AA^{XII}$ occupies position $AA^{205}$, $AA^{XII}$ occupies position $AA^{206}$, $AA^{XIII}$ occupies position $AA^{207}$, $AA^{XIII}$ occupies position $AA^{208}$ and position $AA^{209}$ is vacant, thus $AA^{208}$ would be the amino acid which interacts, in certain embodiments, with PEP2 via $AA^{21}$.

In one example, LINKER may thus be any one of the following peptides: *$AA^{201}$-$AA^{202}$-$AA^{203}$-$AA^{204}$-$AA^{205}$-$AA^{206}$-$AA^{207}$-$AA^{208}$** (XXXV-1); *$AA^{201}$-$AA^{202}$-$AA^{203}$-$AA^{204}$-$AA^{205}$-$AA^{206}$-$AA^{207}$** (XXXV-2); *$AA^{201}$-$AA^{202}$-$AA^{203}$-$AA^{204}$-$AA^{205}$-$AA^{206}$** (XXXV-3); *$AA^{201}$-$AA^{202}$-$AA^{203}$-$AA^{204}$-$AA^{205}$** (XXXV-4);

*$AA^{201}$-$AA^{202}$-$AA^{203}$-$AA^{204}$** (XXXV-5); *$AA^{201}$-$AA^{202}$-$AA^{203}$** (XXXV-6); *$AA^{201}$-$AA^{202}$** (XXXV-7); *$AA^{202}$-$AA^{203}$-$AA^{204}$-$AA^{205}$-$AA^{206}$-$AA^{207}$-$AA^{208}$-$AA^{209}$** (XXXV-8); *$AA^{203}$-$AA^{204}$-$AA^{205}$-$AA^{206}$-$AA^{207}$-$AA^{208}$-$AA^{209}$** (XXXV-9); *$AA^{204}$-$AA^{205}$-$AA^{206}$-$AA^{207}$-$AA^{208}$-$AA^{209}$** (XXXV-10); *$AA^{205}$-$AA^{206}$-$AA^{207}$-$AA^{208}$-$AA^{209}$** (XXXV-11); *$AA^{206}$-$AA^{207}$-$AA^{208}$-$AA^{209}$** (XXXV-12); *$AA^{207}$-$AA^{208}$-$AA^{209}$** (XXXV-13); *$AA^{208}$-$AA^{209}$** (XXXV-14); wherein, for instance, said peptide of formulae (XXXV-1) may thus be selected from the group consisting of *$AA^{III}$-$AA^{I}$-$AA^{I}$-$AA^{II}$-$AA^{VII}$-$AA^{XII}$-$AA^{XII}$-$AA^{XIII}$**, *$AA^{III}$-$AA^{I}$-$AA^{VI}$-$AA^{II}$-$AA^{VII}$-$AA^{XII}$-$AA^{XII}$-$AA^{XIII}$**, *$AA^{III}$-$AA^{I}$-$AA^{I}$-$AA^{II}$-$AA^{II}$-$AA^{XII}$-$AA^{XII}$-$AA^{XIII}$**, *$AA^{III}$-$AA^{I}$-$AA^{II}$-$AA^{II}$-$AA^{VII}$-$AA^{XII}$-$AA^{XII}$-$AA^{XIII}$**, *$AA^{III}$-$AA^{I}$-$AA^{II}$-$AA^{IV}$-$AA^{IV}$-$AA^{XII}$-$AA^{XII}$-$AA^{XIII}$**, *$AA^{II}$-$AA^{I}$-$AA^{II}$-$AA^{II}$-$AA^{II}$-$AA^{XII}$-$AA^{XII}$-$AA^{XIII}$**, *$AA^{III}$-$AA^{III}$-$AA^{II}$-$AA^{VII}$-$AA^{II}$-$AA^{XII}$-$AA^{XII}$-$AA^{XIII}$**, *$AA^{III}$-$AA^{I}$-$AA^{I}$-$AA^{II}$-$AA^{II}$-$AA^{XII}$-$AA^{XII}$-$AA^{XIII}$**, *$AA^{V}$-$AA^{V}$-$AA^{VII}$-$AA^{VII}$-$AA^{XII}$-$AA^{XII}$-$AA^{XIII}$**, *$AA^{VIII}$-$AA^{V}$-$AA^{VII}$-$AA^{II}$-$AA^{XII}$-$AA^{XII}$-$AA^{XIII}$** and *$AA^{V}$-$AA^{V}$-$AA^{VII}$-$AA^{II}$-$AA^{XII}$-$AA^{XII}$-$AA^{XIII}$**.

In one particular example, LINKER comprises a peptide of formula (XXXV), (XXXV-2) or (XXXV-4).

In one example, LINKER comprises (or is) a poly-(aliphatic amino acid) peptide such as poly-alanine peptide $(A)_n$, or a poly-glycine $(G)_n$, n being an integer comprised between 2 and 16, preferably between 2 to 9, such as A-A-A-A-A-A-A-A-A, A-A-A-A-A-A-A-A, A-A-A-A-A, G-G-G-G-G-G-G-G-G, G-G-G-G-G-G-G or G-G-G-G-G.

In one particular example, LINKER comprises (or is) a peptide of general formulae (XXXV) to (XXXV-14), more particularly (XXXV), (XXXV-2) or (XXXV-4), or a poly-(aliphatic amino acid)$_n$ peptide as defined herein.

For example, in certain embodiments, LINKER is a polysaccharide comprising 2 to 16 saccharides. In one particular example, LINKER is a polysaccharide comprising 2 to 10 saccharides. In one particular example, LINKER is a polysaccharide comprising 2 to 9 saccharides. In one most particular example, LINKER is a polysaccharide comprising 5 to 9 saccharides. Suitable monosaccharides include, but are not limited to, glucose (dextrose), fructose (levulose) and galactose. Monosaccharides are the building blocks of disaccharides (such as sucrose) and polysaccharides (such as celluloses, chitosans, ulvanes and starches). Further, each carbon atom that supports a hydroxyl group (except for the first and last) is chiral, giving rise to a number of isomeric forms all with the same chemical formula. A large number of biologically important modified monosaccharides exists e.g. amino sugars such as Galactosamine, Glucosamine, Sialic acid, N-Acetylglucosamine, and sulfosugars such as Sulfoquinovose. All of these monosaccharide and polysaccharide derivatives may be used as LINKER in the present invention.

Thus, in one particular example, said GFR-binding compound is a hydride peptide/polysaccharide peptidomimetic of between 2,000 and 8,000 Da and of general formula (Ia) defined herein; wherein one end of LINKER interacts covalently with one end of PEP(A); wherein another end of LINKER interacts covalently with one end of PEP(B); wherein PEP(A) comprises PEP1 or PEP12; wherein PEP(B) comprises PEP2; wherein LINKER is a polysaccharide comprising 2 to 16 saccharides (in particular 2 to 10, 2 to 9, or 5 to 9 saccharides); and, optionally, wherein the RMSD is 2.45Å or less.

Thus, in one particular example, said GFR-binding compound is a hydride peptide/polysaccharide peptidomimetic of between 2,000 and 8,000 Da and of general formula (IIa); wherein LINKER is a polysaccharide comprising 2 to 16 saccharides (in particular 2 to 10, 2 to 9, or 5 to 9 saccharides); wherein PEP12 is a peptide with 8 amino acids of formula PEP1-$AA^{17}$-PEP11 as already defined herein; wherein PEP1, PEP2, PEP11, PEP(C) and PEP(D) are as already defined herein; wherein $AA^{13}$ and $AA^{21}$ may be both N-terminal amino acids or both C-terminal amino acids; wherein $AA^{25}$ and $AA^{20}$ may be both N-terminal amino acids or both C-terminal amino acids; wherein one end of LINKER interacts covalently with one end of PEP12 via $AA^{20}$; wherein another end of LINKER interacts covalently with PEP2 via $AA^{21}$; wherein one end of PEP(C) interacts covalently with PEP12 via $AA^{13}$; wherein one end of PEP(D) interacts covalently with PEP2 via $AA^{25}$; and, optionally, wherein the RMSD is 2.45Å or less.

For example, in certain embodiments, LINKER is a polynucleotide comprising 2 to 16 nucleotides. In one particular example, LINKER is a polynucleotide comprising 2 to 10 nucleotides. In one particular example, LINKER is a polynucleotide comprising 2 to 9 nucleotides. In one most particular example, LINKER is a polynucleotide comprising 5 to 9 nucleotides. Suitable nucleotides include adenine (A), guanine (G), thymine (T), cytosine (C), uracil (U) and derivatives, analogues and/or mimetic thereof.

Thus, in one particular example, said GFR-binding compound is a hydride peptide/ polynucleotide peptidomimetic of between 2,000 and 8,000 Da and of general formula (Ia) defined herein; wherein one end of LINKER interacts covalently with one end of PEP(A); wherein another end of LINKER interacts covalently with one end of PEP(B); wherein PEP(A) comprises PEP1 or PEP12; wherein PEP(B) comprises PEP2; wherein LINKER is a polynucleotide comprising 2 to 16 nucleotides (in particular 2 to 10, 2 to 9, or 5 to 9 nucleotides); and, optionally, wherein the RMSD is 2.45Å or less.

Thus, in one particular example, said GFR-binding compound is a hydride peptide/ polynucleotide peptidomimetic of between 2,000 and 8,000 Da and of general formula (IIa); wherein LINKER is a polynucleotide comprising 2 to 16 nucleotides (in particular 2 to 10, 2 to 9, or 5 to 9 nucleotides); wherein PEP12 is a peptide with 8 amino acids of formula PEP1-$AA^{17}$-PEP11 as already defined herein; wherein PEP1, PEP2, PEP11, PEP(C) and PEP(D) are as already defined herein; wherein $AA^{13}$ and $AA^{21}$ may be both N-terminal amino acids or both C-terminal amino acids; wherein $AA^{25}$ and $AA^{20}$ may be both N-terminal amino acids or both C-terminal amino acids; wherein one end of LINKER interacts covalently with one end of PEP12 via $AA^{20}$; wherein another end of LINKER interacts covalently with PEP2 via $AA^{21}$;

wherein one end of PEP(C) interacts covalently with PEP12 via AA13; wherein one end of PEP(D) interacts covalently with PEP2 via AA25; and, optionally, wherein the RMSD is 2.45Å or less.

For example, in certain embodiments, LINKER is a saturated or unsaturated hydrocarbon chain comprising between 1 and 22, between 1 and 16, between 1 and 12, between 1 and 8 or between 1 and 6 carbon atoms, wherein said hydrocarbon chain is optionally interrupted by one or more non-carbon atom, preferably between 1 and 16, between 1 and 12 or between 1 and 8 non-carbon atoms as appropriate, wherein said non-carbon atom is selected from the group consisting of -O-, -S-, -C(=O), - $SO_2$-, -N(Ri)(C=O)-, and -N(Ri)-, wherein Ri is selected from the group consisting of a hydrogen atom, a C1-C6 alkyl group and an aryl group, and wherein said hydrocarbon chain is non-substituted or substituted by at least one radical selected from the group consisting of a halogen, a monosaccharide, a poly(1-6)saccharide, a nucleotide, a poly(1-6)nucleotide, a C1-C10 alkyl group and an aryl group.

Thus, in one particular example, said GFR-binding compound is a hydride peptide/ hydrocarbon chain peptidomimetic of between 2,000 and 8,000 Da and of general formula (Ia) defined herein; wherein one end of LINKER interacts covalently with one end of PEP(A); wherein another end of LINKER interacts covalently with one end of PEP(B); wherein PEP(A) comprises PEP1 or PEP12; wherein PEP(B) comprises PEP2; wherein LINKER is a saturated or unsaturated hydrocarbon chain comprising between 1 and 22 as defined herein; and, optionally, wherein the RMSD is 2.45Å or less.

Thus, in one particular example, said GFR-binding compound is a hydride peptide/ hydrocarbon chain peptidomimetic of between 2,000 and 8,000 Da and of general formula (IIa); wherein LINKER is a saturated or unsaturated hydrocarbon chain comprising between 1 and 22 as defined herein; wherein PEP12 is a peptide with 8 amino acids of formula PEP1-AA17-PEP11 as already defined herein; wherein PEP1, PEP2, PEP11, PEP(C) and PEP(D) are as already defined herein; wherein AA13 and AA21 may be both N-terminal amino acids or both C-terminal amino acids; wherein AA25 and AA20 may be both N-terminal amino acids or both C-terminal amino acids; wherein one end of LINKER interacts covalently with one end of PEP12 via AA20; wherein another end of LINKER interacts covalently with PEP2 via AA21; wherein one end of PEP(C) interacts covalently with PEP12 via AA13; wherein one end of PEP(D) interacts covalently with PEP2 via AA25; and, optionally, wherein the RMSD is 2.45Å or less.

In one example, LINKER is a saturated or unsaturated hydrocarbon chain of at most 10 nanometres (nm) in length, preferably at most 8 nm, 6nm, 4 nm or 2 nm as determined using the standard « 2D » procedure described above.

In one example, such saturated or unsaturated hydrocarbon chains include polyethylene glycol (PEG) or any one of its derivatives.

More particularly, LINKER is a pentapeptide (five amino acids). More particularly, LINKER is a hexapeptide (six amino acids). More particularly, LINKER is a heptapeptide (seven amino acids). More particularly, LINKER is an octapeptide (eight amino acids). More particularly, LINKER is a nonapeptide (nine amino acids).

In one particular example, LINKER is a peptide selected from the group consisting of DENEKVV, DENKNVV, DEYDKVV, DDSSNVI, DSSNNVI, DDMGVPT, DKGVVTY, NDKQQII, DAANNVV, DSANNVV, DDSSNVI, DNGRVLL, VGRKPKV, IGKTPKI, VGRTPKV, RIKPHQGQH, EYVRKKPKL, EIVRKKPIF, EYVRKKP, EIVRKKP, polyalanine ($A_{1-9}$) and polyglycine ($G_{1-9}$).

For example, in certain embodiments, the covalent interaction between an end (i.e. one end or the other end) of LINKER or PEP(A) or PEP12 and an N-terminal amino acid, such as AA13 or AA21, may be created through the chemical reaction between the free amine moiety of the N-terminal amino acid (-$NH_2$ or -$NH_3$X, X generally being a halide anion selected from the group consisting of $F^-$, $Cl^-$ and $Br^-$), typically acting as a nucleophile, and an electrophile moiety of LINKER. Such an electrophile moiety includes, but is not limited to, alkyl halides (-$CR_2$-X), alcohols (-$CR_2$-OH), acid chlorides (-C(=O)X), esters, (-C(=O)OR), phosphate (-$OP(OR)_3$), phosphinate (-$OP(OR)R_2$), phosphonates (-$OP(OR)_2R$), phosphonite (-$P(OR)_2R$) or sulfonic esters (-$SO_2OR$). More particularly, this covalent interaction is an amide bond (in particular a peptide bond) formed through conventional peptide synthesis using conventional coupling reagents as already defined herein.

For example, in certain embodiments, the covalent interaction between an end (i.e. one end or the other end) of LINKER or PEP(B) or PEP2 and a C-terminal amino acid such as AA25 or AA20, may be created through the chemical reaction between the free carboxylic acid moiety of the C-terminal amino acid (-$CO_2H$ or -$CO_2X$, X generally being an inorganic cation such as alkaline cations (e.g. $Li^+$, $Na^+$ or $K^+$) or an organic cation such as ammonium cations, typically acting as an electrophile, and a nucleophile moiety of LINKER. Such a nucleophile moiety includes, but is not limited to, alcohols (-OH), amines (-$NH_2$), phosphines (-$PR_3$), thiols (-SH). More particularly, this covalent interaction is a peptide bond formed through conventional peptide synthesis using conventional coupling reagents as already defined herein.

### 11.2. Cyclic GFR-binding compounds

In one example, said cyclic GFR-binding compound has a molecular weight of less than 5,000 Daltons. In one particular example, said cyclic GFR-binding compound has a molecular weight of less than 4,000 Daltons. In one particular example, said cyclic GFR-binding compound has a molecular weight comprised between 1,500 and 5,000 Daltons. In one particular example, said cyclic GFR-binding compound has a molecular weight comprised between 1,500

and 4,000 Daltons.

In one example, said cyclic GFR-binding compound has a molecular weight of less than 7,000 Daltons. In one example, said cyclic GFR-binding compound has a molecular weight of less than 6,000 Daltons. In one example, said cyclic GFR-binding compound has a molecular weight of less than 5,000 Daltons. In one particular example, said cyclic GFR-binding compound has a molecular weight comprised between 1,500 and 7,000 Daltons. In one particular example, said cyclic GFR-binding compound has a molecular weight comprised between 1,500 and 6,000 Daltons. In one particular example, said cyclic GFR-binding compound has a molecular weight comprised between 2,000 and 7,000 Daltons. In one particular example, said cyclic GFR-binding compound has a molecular weight comprised between 3,000 and 7,000 Daltons. In one particular example, said cyclic GFR-binding compound has a molecular weight comprised between 4,000 and 7,000 Daltons. In one particular example, said cyclic GFR-binding compound has a molecular weight comprised between 2,000 and 6,000 Daltons. In one particular example, said cyclic GFR-binding compound has a molecular weight comprised between 3,000 and 6,000 Daltons. In one particular example, said cyclic GFR-binding compound has a molecular weight comprised between 4,000 and 6,000 Daltons.

In one particular example, the growth factor receptor involved in the interaction with said cyclic GFR-binding compound is an epidermal growth factor receptor. In one particular example, the growth factor receptor involved in the interaction with said cyclic GFR-binding compound is a fibroblast growth factor receptor. In one particular example, the growth factor receptor involved in the interaction with said cyclic GFR-binding compound is a vascular endothelial growth factor receptor. In one particular example, the growth factor receptor involved in the interaction with said cyclic GFR-binding compound is a nerve growth factor receptor. In one particular example, the growth factor receptor involved in the interaction with said cyclic GFR-binding compound is a hepatocyte growth factor receptor. In one particular example, the growth factor receptor involved in the interaction with said cyclic GFR-binding compound is a somatomedin or insulin-like growth factor receptor. In one particular example, the growth factor receptor involved in the interaction with said cyclic GFR-binding compound is a platelet-derived growth factor receptor. In one particular example, the growth factor receptor involved in the interaction with said cyclic GFR-binding compound is a protein from the transforming growth factor beta (TGF-$\beta$) superfamily.

In one particular example, the growth factor receptor(s) involved in the interaction with said cyclic GFR-binding compound is (are) preferably selected from epidermal growth factor receptors, fibroblast growth factor receptors, vascular endothelial growth factor receptors, nerve growth factor receptors, hepatocyte growth factor receptors, somatomedin or insulin-like growth factor receptors, platelet-derived growth factor receptors, and transforming growth factor beta (TGF-$\beta$) superfamily proteins.

In one particular example, said cyclic GFR-binding compound is a peptide, or a variant or analog thereof, having growth factor receptor-binding capability or capabilities, with (exclusively consisting of, or constituted of) between 15-60 amino acids, in particular between 15-55 amino acids, more particularly between 19-60 amino acids, and even more particularly between 19-55 amino acids, or between 15-35 amino acids, in particular between 15-30 amino acids, more particularly between 19-35 amino acids, and even more particularly between 19-30 amino acids.

In one particular example, said cyclic GFR-binding compound is a cyclic peptidomimetic as defined herein, having growth factor receptor-binding capability or capabilities, comprising (consecutively or non consecutively) between 15-60 amino acids, in particular between 15-55 amino acids, more particularly between 19-60 amino acids, and even more particularly between 19-55 amino acids, or between 15-60 amino acids, in particular between 15-55 amino acids, more particularly between 19-60 amino acids, and even more particularly between 19-55 amino acids, or between 15-35 amino acids, in particular between 15-30 amino acids, more particularly between 19-35 amino acids, and even more particularly between 19-30 amino acids; wherein said cyclic GFR-binding compound has a molecular weight comprised between 1,500 and 7,000 Daltons.

In one particular example, said cyclic GFR-binding compound is a cyclic peptidomimetic as defined herein, having growth factor receptor-binding capability or capabilities, comprising (consecutively or non consecutively) between 15-60 amino acids, in particular between 15-55 amino acids, more particularly between 19-60 amino acids, and even more particularly between 19-55 amino acids, or between 15-35 amino acids, in particular between 15-30 amino acids, more particularly between 19-35 amino acids, and even more particularly between 19-30 amino acids; and containing at least one peptide portion or fragment with between 5-20 amino acids (in particular containing two peptide portions or fragments, each of which with between 5-20 amino acids); wherein said cyclic GFR-binding compound has a molecular weight comprised between 1,500 and 7,000 Daltons.

In one aspect, the present disclosure provides a cyclic GFR-binding compound, wherein said cyclic GFR-binding compound is a cyclic peptide, a variant or analog thereof, or a cyclic peptidomimetic as defined herein, having growth factor receptor-binding capability or capabilities, having a molecular weight of less than 5,000 Da, in particular of between 1,500 and 7,000 Da.

In one aspect, the present disclosure provides a cyclic GFR-binding compound, wherein said cyclic GFR-binding compound is a cyclic peptide, a variant or analog thereof, or a cyclic peptidomimetic as defined herein, having growth factor receptor-binding capability or capabilities, with between 15-60 (in particular between 15-55, more particularly

between 19-60, and even more particularly between 19-55) amino acids, or between 15-35 (in particular between 15-30, more particularly between 19-35, and even more particularly between 19-30) amino acids, comprising a peptide with four amino acids (PEP1) and a peptide with five amino acids (PEP2).

In one aspect, the present disclosure provides a cyclic GFR-binding compound, wherein said cyclic GFR-binding compound is a cyclic peptide, a variant or analog thereof, or a cyclic peptidomimetic as defined herein, with between 15-60 (in particular between 15-55, more particularly between 19-60, and even more particularly between 19-55) amino acids, or between 15-35 (in particular between 15-30, more particularly between 19-35, and even more particularly between 19-30) amino acids, comprising a peptide with eight amino acids (PEP12) and a peptide with five amino acids (PEP2).

In one aspect, the present disclosure provides a cyclic GFR-binding compound, wherein said cyclic GFR-binding compound is a cyclic peptide, a variant or analog thereof, or a cyclic peptidomimetic as defined herein, with between 15-60 (in particular between 15-55, more particularly between 19-60, and even more particularly between 19-55) amino acids, or between 15-35 (in particular between 15-30, more particularly between 19-35, and even more particularly between 19-30) amino acids, comprising a peptide with four amino acids (PEP1) and a peptide with five amino acids (PEP2); wherein said cyclic GFR-binding compound further comprises a peptide with three amino acids (PEP3) and a peptide with three amino acids (PEP4).

In one aspect, the present disclosure provides a cyclic GFR-binding compound, wherein said cyclic GFR-binding compound is a cyclic peptide, a variant or analog thereof, or a cyclic peptidomimetic as defined herein, with between 15-60 (in particular between 15-55, more particularly between 19-60, and even more particularly between 19-55) amino acids, or between 15-35 (in particular between 15-30, more particularly between 19-35, and even more particularly between 19-30) amino acids, comprising a peptide with eight amino acids (PEP12) and a peptide with five amino acids (PEP2); wherein said cyclic GFR-binding compound further comprises a peptide with three amino acids (PEP3) and a peptide with three amino acids (PEP4).

In one aspect, the present disclosure provides a cyclic GFR-binding compound, wherein said cyclic GFR-binding compound is a cyclic peptide, a variant or analog thereof, or a cyclic peptidomimetic as defined herein, with between 15-60 (in particular between 15-55, more particularly between 19-60, and even more particularly between 19-55) amino acids, or between 15-35 (in particular between 15-30, more particularly between 19-35, and even more particularly between 19-30) amino acids, comprising a peptide with four amino acids (PEP1) and a peptide with five amino acids (PEP2); wherein said cyclic GFR-binding compound further comprises a peptide with five amino acids (PEP5) and a peptide with between five and seven amino acids (PEP6).

In one aspect, the present disclosure provides a cyclic GFR-binding compound, wherein said cyclic GFR-binding compound is a cyclic peptide, a variant or analog thereof, or a cyclic peptidomimetic as defined herein, with between 15-60 (in particular between 15-55, more particularly between 19-60, and even more particularly between 19-55) amino acids, or between 15-35 (in particular between 15-30, more particularly between 19-35, and even more particularly between 19-30) amino acids, comprising a peptide with eight amino acids (PEP12) and a peptide with five amino acids (PEP2); wherein said cyclic GFR-binding compound further comprises a peptide with five amino acids (PEP5) and a peptide with between five and seven amino acids (PEP6).

In one aspect, the present disclosure provides a cyclic GFR-binding compound, wherein said cyclic GFR-binding compound is a cyclic peptide, a variant or analog thereof, or a cyclic peptidomimetic as defined herein, with between 15-60 (in particular between 15-55, more particularly between 19-60, and even more particularly between 19-55) amino acids, or between 15-35 (in particular between 15-30, more particularly between 19-35, and even more particularly between 19-30) amino acids, comprising a peptide with four amino acids (PEP1) and a peptide with five amino acids (PEP2); wherein said cyclic GFR-binding compound further comprises a peptide with between six and twelve amino acids (PEP9) and a peptide with between six and eleven amino acids (PEP10).

In one aspect, the present disclosure provides a cyclic GFR-binding compound, wherein said cyclic GFR-binding compound is a cyclic peptide, a variant or analog thereof, or a cyclic peptidomimetic as defined herein, with between 15-60 (in particular between 15-55, more particularly between 19-60, and even more particularly between 19-55) amino acids, or between 15-35 (in particular between 15-30, more particularly between 19-35, and even more particularly between 19-30) amino acids, comprising a peptide with eight amino acids (PEP12) and a peptide with five amino acids (PEP2); wherein said cyclic GFR-binding compound further comprises a peptide with between six and twelve amino acids (PEP9) and a peptide with between six and eleven amino acids (PEP10).

In one aspect, the present disclosure provides a cyclic GFR-binding compound, wherein said cyclic GFR-binding compound is a cyclic peptide, a variant or analog thereof, or a cyclic peptidomimetic as defined herein, with between 15-60 (in particular between 15-55, more particularly between 19-60, and even more particularly between 19-55) amino acids, or between 15-35 (in particular between 15-30, more particularly between 19-35, and even more particularly between 19-30) amino acids, comprising a peptide with four amino acids (PEP1) and a peptide with five amino acids (PEP2); wherein said cyclic GFR-binding compound further comprises a peptide with three amino acids (PEP3) and a peptide with between five and seven amino acids (PEP6).

In one aspect, the present disclosure provides a cyclic GFR-binding compound, wherein said cyclic GFR-binding compound is a cyclic peptide, a variant or analog thereof, or a cyclic peptidomimetic as defined herein, with between 15-60 (in particular between 15-55, more particularly between 19-60, and even more particularly between 19-55) amino acids, or between 15-35 (in particular between 15-30, more particularly between 19-35, and even more particularly between 19-30) amino acids, comprising a peptide with eight amino acids (PEP12) and a peptide with five amino acids (PEP2); wherein said cyclic GFR-binding compound further comprises a peptide with three amino acids (PEP3) and a peptide with between five and seven amino acids (PEP6).

In one aspect, the present disclosure provides a cyclic GFR-binding compound, wherein said cyclic GFR-binding compound is a cyclic peptide, a variant or analog thereof, or a cyclic peptidomimetic as defined herein, with between 15-60 (in particular between 15-55, more particularly between 19-60, and even more particularly between 19-55) amino acids, or between 15-35 (in particular between 15-30, more particularly between 19-35, and even more particularly between 19-30) amino acids, comprising a peptide with four amino acids (PEP1) and a peptide with five amino acids (PEP2); wherein said cyclic GFR-binding compound further comprises a peptide with three amino acids (PEP3) and a peptide with between six and eleven amino acids (PEP10).

In one aspect, the present disclosure provides a cyclic GFR-binding compound, wherein said cyclic GFR-binding compound is a cyclic peptide, a variant or analog thereof, or a cyclic peptidomimetic as defined herein, with between 15-60 (in particular between 15-55, more particularly between 19-60, and even more particularly between 19-55) amino acids, or between 15-35 (in particular between 15-30, more particularly between 19-35, and even more particularly between 19-30) amino acids, comprising a peptide with eight amino acids (PEP12) and a peptide with five amino acids (PEP2); wherein said cyclic GFR-binding compound further comprises a peptide with three amino acids (PEP3) and a peptide with between six and eleven amino acids (PEP10).

In one aspect, the present disclosure provides a cyclic GFR-binding compound, wherein said cyclic GFR-binding compound is a cyclic peptide, a variant or analog thereof, or a cyclic peptidomimetic as defined herein, with between 15-60 (in particular between 15-55, more particularly between 19-60, and even more particularly between 19-55) amino acids, or between 15-35 (in particular between 15-30, more particularly between 19-35, and even more particularly between 19-30) amino acids, comprising a peptide with four amino acids (PEP1) and a peptide with five amino acids (PEP2); wherein said cyclic GFR-binding compound further comprises a peptide with five amino acids (PEP5) and a peptide with three amino acids (PEP4).

In one aspect, the present disclosure provides a cyclic GFR-binding compound, wherein said cyclic GFR-binding compound is a cyclic peptide, a variant or analog thereof, or a cyclic peptidomimetic as defined herein, with between 15-60 (in particular between 15-55, more particularly between 19-60, and even more particularly between 19-55) amino acids, or between 15-35 (in particular between 15-30, more particularly between 19-35, and even more particularly between 19-30) amino acids, comprising a peptide with four amino acids (PEP12) and a peptide with five amino acids (PEP2); wherein said cyclic GFR-binding compound further comprises a peptide with five amino acids (PEP5) and a peptide with three amino acids (PEP4).

In one aspect, the present disclosure provides a cyclic GFR-binding compound, wherein said cyclic GFR-binding compound is a cyclic peptide, a variant or analog thereof, or a cyclic peptidomimetic as defined herein, with between 15-60 (in particular between 15-55, more particularly between 19-60, and even more particularly between 19-55) amino acids, or between 15-35 (in particular between 15-30, more particularly between 19-35, and even more particularly between 19-30) amino acids, comprising a peptide with four amino acids (PEP1) and a peptide with five amino acids (PEP2); wherein said cyclic GFR-binding compound further comprises a peptide with between six and twelve amino acids (PEP9) and a peptide with three amino acids (PEP4).

In one aspect, the present disclosure provides a cyclic GFR-binding compound, wherein said cyclic GFR-binding compound is a cyclic peptide, a variant or analog thereof, or a cyclic peptidomimetic as defined herein, with between 15-60 (in particular between 15-55, more particularly between 19-60, and even more particularly between 19-55) amino acids, or between 15-35 (in particular between 15-30, more particularly between 19-35, and even more particularly between 19-30) amino acids, comprising a peptide with four amino acids (PEP12) and a peptide with five amino acids (PEP2); wherein said cyclic GFR-binding compound further comprises a peptide with between six and twelve amino acids (PEP9) and a peptide with three amino acids (PEP4).

In one aspect, the present disclosure provides a cyclic GFR-binding compound, wherein said cyclic GFR-binding compound is a cyclic peptide, a variant or analog thereof, or a cyclic peptidomimetic as defined herein, with between 15-60 (in particular between 15-55, more particularly between 19-60, and even more particularly between 19-55) amino acids, or between 15-35 (in particular between 15-30, more particularly between 19-35, and even more particularly between 19-30) amino acids, comprising a peptide with four amino acids (PEP1) and a peptide with five amino acids (PEP2); wherein said cyclic GFR-binding compound further comprises a peptide with five amino acids (PEP5) and a peptide with between six and eleven amino acids (PEP10).

In one aspect, the present disclosure provides a cyclic GFR-binding compound, wherein said cyclic GFR-binding compound is a cyclic peptide, a variant or analog thereof, or a cyclic peptidomimetic as defined herein, with

between 15-60 (in particular between 15-55, more particularly between 19-60, and even more particularly between 19-55) amino acids, or between 15-35 (in particular between 15-30, more particularly between 19-35, and even more particularly between 19-30) amino acids, comprising a peptide with four amino acids (PEP12) and a peptide with five amino acids (PEP2); wherein said cyclic GFR-binding compound further comprises a peptide with five amino acids (PEP5) and a peptide with between six and eleven amino acids (PEP10).

In one aspect, the present disclosure provides a cyclic GFR-binding compound, wherein said cyclic GFR-binding compound is a cyclic peptide, a variant or analog thereof, or a cyclic peptidomimetic as defined herein, with between 15-60 (in particular between 15-55, more particularly between 19-60, and even more particularly between 19-55) amino acids, or between 15-35 (in particular between 15-30, more particularly between 19-35, and even more particularly between 19-30) amino acids, comprising a peptide with four amino acids (PEP1) and a peptide with five amino acids (PEP2); wherein said cyclic GFR-binding compound further comprises a peptide with between six and twelve amino acids (PEP9) and a peptide with between five and seven amino acids (PEP6).

In one aspect, the present disclosure provides a cyclic GFR-binding compound, wherein said cyclic GFR-binding compound is a cyclic peptide, a variant or analog thereof, or a cyclic peptidomimetic as defined herein, with between 15-60 (in particular between 15-55, more particularly between 19-60, and even more particularly between 19-55) amino acids, or between 15-35 (in particular between 15-30, more particularly between 19-35, and even more particularly between 19-30) amino acids, comprising a peptide with four amino acids (PEP12) and a peptide with five amino acids (PEP2); wherein said cyclic GFR-binding compound further comprises a peptide with between six and twelve amino acids (PEP9) and a peptide with between five and seven amino acids (PEP6).

In one aspect, the present disclosure provides a cyclic GFR-binding compound, wherein said cyclic GFR-binding compound is a cyclic peptide, a variant or analog thereof, or a cyclic peptidomimetic as defined herein, with between 15-60 (in particular between 15-55, more particularly between 19-60, and even more particularly between 19-55) amino acids, or between 15-35 (in particular between 15-30, more particularly between 19-35, and even more particularly between 19-30) amino acids, comprising a peptide with four amino acids (PEP1) and a peptide with five amino acids (PEP2); wherein said cyclic GFR-binding compound further comprises a peptide with three amino acids (PEP3), an amino acid or a peptide with between two and seven amino acids (PEP7), and a peptide with three amino acids (PEP4).

In one aspect, the present disclosure provides a cyclic GFR-binding compound, wherein said cyclic GFR-binding compound is a cyclic peptide, a variant or analog thereof, or a cyclic peptidomimetic as defined herein, with between 15-60 (in particular between 15-55, more particularly between 19-60, and even more particularly between 19-55) amino acids, or between 15-35 (in particular between 15-30, more particularly between 19-35, and even more particularly between 19-30) amino acids, comprising a peptide with four amino acids (PEP12) and a peptide with five amino acids (PEP2); wherein said cyclic GFR-binding compound further comprises a peptide with three amino acids (PEP3), an amino acid or a peptide with between two and seven amino acids (PEP7), and a peptide with three amino acids (PEP4).

In one aspect, the present disclosure provides a cyclic GFR-binding compound, wherein said cyclic GFR-binding compound is a cyclic peptide, a variant or analog thereof, or a cyclic peptidomimetic as defined herein, with between 15-60 (in particular between 15-55, more particularly between 19-60, and even more particularly between 19-55) amino acids, or between 15-35 (in particular between 15-30, more particularly between 19-35, and even more particularly between 19-30) amino acids, comprising a peptide with four amino acids (PEP1) and a peptide with five amino acids (PEP2); wherein said cyclic GFR-binding compound further comprises a peptide with three amino acids (PEP3), an amino acid or a peptide with between two and seven amino acids (PEP7), and a peptide with between five and seven amino acids (PEP6).

In one aspect, the present disclosure provides a cyclic GFR-binding compound, wherein said cyclic GFR-binding compound is a cyclic peptide, a variant or analog thereof, or a cyclic peptidomimetic as defined herein, with between 15-60 (in particular between 15-55, more particularly between 19-60, and even more particularly between 19-55) amino acids, or between 15-35 (in particular between 15-30, more particularly between 19-35, and even more particularly between 19-30) amino acids, comprising a peptide with four amino acids (PEP12) and a peptide with five amino acids (PEP2); wherein said cyclic GFR-binding compound further comprises a peptide with three amino acids (PEP3), an amino acid or a peptide with between two and seven amino acids (PEP7), and a peptide with between five and seven amino acids (PEP6).

In one aspect, the present disclosure provides a cyclic GFR-binding compound, wherein said cyclic GFR-binding compound is a cyclic peptide, a variant or analog thereof, or a cyclic peptidomimetic as defined herein, with between 15-60 (in particular between 15-55, more particularly between 19-60, and even more particularly between 19-55) amino acids, or between 15-35 (in particular between 15-30, more particularly between 19-35, and even more particularly between 19-30) amino acids, comprising a peptide with four amino acids (PEP1) and a peptide with five amino acids (PEP2); wherein said cyclic GFR-binding compound further comprises a peptide with three amino acids (PEP3), an amino acid or a peptide with between two and seven amino acids (PEP7), and a peptide with between six

and eleven amino acids (PEP10).

In one aspect, the present disclosure provides a cyclic GFR-binding compound, wherein said cyclic GFR-binding compound is a cyclic peptide, a variant or analog thereof, or a cyclic peptidomimetic as defined herein, with between 15-60 (in particular between 15-55, more particularly between 19-60, and even more particularly between 19-55) amino acids, or between 15-35 (in particular between 15-30, more particularly between 19-35, and even more particularly between 19-30) amino acids, comprising a peptide with four amino acids (PEP12) and a peptide with five amino acids (PEP2); wherein said cyclic GFR-binding compound further comprises a peptide with three amino acids (PEP3), an amino acid or a peptide with between two and seven amino acids (PEP7), and a peptide with between six and eleven amino acids (PEP10).

In one aspect, the present disclosure provides a cyclic GFR-binding compound, wherein said cyclic GFR-binding compound is a cyclic peptide, a variant or analog thereof, or a cyclic peptidomimetic as defined herein, with between 15-60 (in particular between 15-55, more particularly between 19-60, and even more particularly between 19-55) amino acids, or between 15-35 (in particular between 15-30, more particularly between 19-35, and even more particularly between 19-30) amino acids, comprising a peptide with four amino acids (PEP1) and a peptide with five amino acids (PEP2); wherein said cyclic GFR-binding compound further comprises a peptide with three amino acids (PEP3), an amino acid or a peptide with between two and seven amino acids (PEP7), a peptide with three amino acids (PEP4), and an amino acid or a peptide with between two and six amino acids (PEP8).

In one aspect, the present disclosure provides a cyclic GFR-binding compound, wherein said cyclic GFR-binding compound is a cyclic peptide, a variant or analog thereof, or a cyclic peptidomimetic as defined herein, with between 15-60 (in particular between 15-55, more particularly between 19-60, and even more particularly between 19-55) amino acids, or between 15-35 (in particular between 15-30, more particularly between 19-35, and even more particularly between 19-30) amino acids, comprising a peptide with four amino acids (PEP12) and a peptide with five amino acids (PEP2); wherein said cyclic GFR-binding compound further comprises a peptide with three amino acids (PEP3), an amino acid or a peptide with between two and seven amino acids (PEP7), a peptide with three amino acids (PEP4), and an amino acid or a peptide with between two and six amino acids (PEP8).

In one aspect, the present disclosure provides a cyclic GFR-binding compound, wherein said cyclic GFR-binding compound is a cyclic peptide, a variant or analog thereof, or a cyclic peptidomimetic as defined herein, with between 15-60 (in particular between 15-55, more particularly between 19-60, and even more particularly between 19-55) amino acids, or between 15-35 (in particular between 15-30, more particularly between 19-35, and even more particularly between 19-30) amino acids, comprising a peptide with four amino acids (PEP1) and a peptide with five amino acids (PEP2); wherein said cyclic GFR-binding compound further comprises a peptide with three amino acids (PEP3), an amino acid or a peptide with between two and seven amino acids (PEP7), a peptide with between five and seven amino acids (PEP6), and an amino acid or a peptide with between two and six amino acids (PEP8).

In one aspect, the present disclosure provides a cyclic GFR-binding compound, wherein said cyclic GFR-binding compound is a cyclic peptide, a variant or analog thereof, or a cyclic peptidomimetic as defined herein, with between 15-60 (in particular between 15-55, more particularly between 19-60, and even more particularly between 19-55) amino acids, or between 15-35 (in particular between 15-30, more particularly between 19-35, and even more particularly between 19-30) amino acids, comprising a peptide with four amino acids (PEP12) and a peptide with five amino acids (PEP2); wherein said cyclic GFR-binding compound further comprises a peptide with three amino acids (PEP3), an amino acid or a peptide with between two and seven amino acids (PEP7), a peptide with between five and seven amino acids (PEP6), and an amino acid or a peptide with between two and six amino acids (PEP8).

In one aspect, the present disclosure provides a cyclic GFR-binding compound, wherein said cyclic GFR-binding compound is a cyclic peptide, a variant or analog thereof, or a cyclic peptidomimetic as defined herein, with between 15-60 (in particular between 15-55, more particularly between 19-60, and even more particularly between 19-55) amino acids, or between 15-35 (in particular between 15-30, more particularly between 19-35, and even more particularly between 19-30) amino acids, comprising a peptide with four amino acids (PEP1) and a peptide with five amino acids (PEP2); wherein said cyclic GFR-binding compound further comprises a peptide with five amino acids (PEP5), an amino acid or a peptide with between two and seven amino acids (PEP7), and a peptide with three amino acids (PEP4).

In one aspect, the present disclosure provides a cyclic GFR-binding compound, wherein said cyclic GFR-binding compound is a cyclic peptide, a variant or analog thereof, or a cyclic peptidomimetic as defined herein, with between 15-60 (in particular between 15-55, more particularly between 19-60, and even more particularly between 19-55) amino acids, or between 15-35 (in particular between 15-30, more particularly between 19-35, and even more particularly between 19-30) amino acids, comprising a peptide with four amino acids (PEP12) and a peptide with five amino acids (PEP2); wherein said cyclic GFR-binding compound further comprises a peptide with five amino acids (PEP5), an amino acid or a peptide with between two and seven amino acids (PEP7), and a peptide with three amino acids (PEP4).

In one aspect, the present disclosure provides a cyclic GFR-binding compound, wherein said cyclic GFR-binding compound is a cyclic peptide, a variant or analog thereof, or a cyclic peptidomimetic as defined herein, with between 15-60 (in particular between 15-55, more particularly between 19-60, and even more particularly between 19-55) amino acids, or between 15-35 (in particular between 15-30, more particularly between 19-35, and even more particularly between 19-30) amino acids, comprising a peptide with four amino acids (PEP1) and a peptide with five amino acids (PEP2); wherein said cyclic GFR-binding compound further comprises a peptide with five amino acids (PEP5), an amino acid or a peptide with between two and seven amino acids (PEP7), and a peptide with between five and seven amino acids (PEP6).

In one aspect, the present disclosure provides a cyclic GFR-binding compound, wherein said cyclic GFR-binding compound is a cyclic peptide, a variant or analog thereof, or a cyclic peptidomimetic as defined herein, with between 15-60 (in particular between 15-55, more particularly between 19-60, and even more particularly between 19-55) amino acids, or between 15-35 (in particular between 15-30, more particularly between 19-35, and even more particularly between 19-30) amino acids, comprising a peptide with four amino acids (PEP12) and a peptide with five amino acids (PEP2); wherein said cyclic GFR-binding compound further comprises a peptide with five amino acids (PEP5), an amino acid or a peptide with between two and seven amino acids (PEP7), and a peptide with between five and seven amino acids (PEP6).

In one aspect, the present disclosure provides a cyclic GFR-binding compound, wherein said cyclic GFR-binding compound is a cyclic peptide, a variant or analog thereof, or a cyclic peptidomimetic as defined herein, with between 15-60 (in particular between 15-55, more particularly between 19-60, and even more particularly between 19-55) amino acids, or between 15-35 (in particular between 15-30, more particularly between 19-35, and even more particularly between 19-30) amino acids, comprising a peptide with four amino acids (PEP1) and a peptide with five amino acids (PEP2); wherein said cyclic GFR-binding compound further comprises a peptide with five amino acids (PEP5), an amino acid or a peptide with between two and seven amino acids (PEP7), and a peptide with between six and eleven amino acids (PEP10).

In one aspect, the present disclosure provides a cyclic GFR-binding compound, wherein said cyclic GFR-binding compound is a cyclic peptide, a variant or analog thereof, or a cyclic peptidomimetic as defined herein, with between 15-60 (in particular between 15-55, more particularly between 19-60, and even more particularly between 19-55) amino acids, or between 15-35 (in particular between 15-30, more particularly between 19-35, and even more particularly between 19-30) amino acids, comprising a peptide with four amino acids (PEP12) and a peptide with five amino acids (PEP2); wherein said cyclic GFR-binding compound further comprises a peptide with five amino acids (PEP5), an amino acid or a peptide with between two and seven amino acids (PEP7), and a peptide with between six and eleven amino acids (PEP10).

In one aspect, the present disclosure provides a cyclic GFR-binding compound, wherein said cyclic GFR-binding compound is a cyclic peptide, a variant or analog thereof, or a cyclic peptidomimetic as defined herein, with between 15-60 (in particular between 15-55, more particularly between 19-60, and even more particularly between 19-55) amino acids, or between 15-35 (in particular between 15-30, more particularly between 19-35, and even more particularly between 19-30) amino acids, comprising a peptide with four amino acids (PEP1) and a peptide with five amino acids (PEP2); wherein said cyclic GFR-binding compound further comprises a peptide with five amino acids (PEP5), an amino acid or a peptide with between two and seven amino acids (PEP7), a peptide with three amino acids (PEP4), and an amino acid or a peptide with between two and six amino acids (PEP8).

In one aspect, the present disclosure provides a cyclic GFR-binding compound, wherein said cyclic GFR-binding compound is a cyclic peptide, a variant or analog thereof, or a cyclic peptidomimetic as defined herein, with between 15-60 (in particular between 15-55, more particularly between 19-60, and even more particularly between 19-55) amino acids, or between 15-35 (in particular between 15-30, more particularly between 19-35, and even more particularly between 19-30) amino acids, comprising a peptide with four amino acids (PEP12) and a peptide with five amino acids (PEP2); wherein said cyclic GFR-binding compound further comprises a peptide with five amino acids (PEP5), an amino acid or a peptide with between two and seven amino acids (PEP7), a peptide with three amino acids (PEP4), and an amino acid or a peptide with between two and six amino acids (PEP8).

In one aspect, the present disclosure provides a cyclic GFR-binding compound, wherein said cyclic GFR-binding compound is a cyclic peptide, a variant or analog thereof, or a cyclic peptidomimetic as defined herein, with between 15-60 (in particular between 15-55, more particularly between 19-60, and even more particularly between 19-55) amino acids, or between 15-35 (in particular between 15-30, more particularly between 19-35, and even more particularly between 19-30) amino acids, comprising a peptide with four amino acids (PEP1) and a peptide with five amino acids (PEP2); wherein said cyclic GFR-binding compound further comprises a peptide with five amino acids (PEP5), an amino acid or a peptide with between two and seven amino acids (PEP7), a peptide with between five and seven amino acids (PEP6), and an amino acid or a peptide with between two and six amino acids (PEP8).

In one aspect, the present disclosure provides a cyclic GFR-binding compound, wherein said cyclic GFR-binding compound is a cyclic peptide, a variant or analog thereof, or a cyclic peptidomimetic as defined herein, with between 15-60 (in particular between 15-55, more particularly between 19-60, and even more particularly between

19-55) amino acids, or between 15-35 (in particular between 15-30, more particularly between 19-35, and even more particularly between 19-30) amino acids, comprising a peptide with four amino acids (PEP12) and a peptide with five amino acids (PEP2); wherein said cyclic GFR-binding compound further comprises a peptide with five amino acids (PEP5), an amino acid or a peptide with between two and seven amino acids (PEP7), a peptide with between five and seven amino acids (PEP6), and an amino acid or a peptide with between two and six amino acids (PEP8).

In one aspect, the present disclosure provides a cyclic GFR-binding compound, wherein said cyclic GFR-binding compound is a cyclic peptide, a variant or analog thereof, or a cyclic peptidomimetic as defined herein, with between 15-60 (in particular between 15-55, more particularly between 19-60, and even more particularly between 19-55) amino acids, or between 15-35 (in particular between 15-30, more particularly between 19-35, and even more particularly between 19-30) amino acids, comprising a peptide with four amino acids (PEP1) and a peptide with five amino acids (PEP2); wherein said cyclic GFR-binding compound further comprises a peptide with between six and twelve amino acids (PEP9), a peptide with three amino acids (PEP4), and an amino acid or a peptide with between two and six amino acids (PEP8).

In one aspect, the present disclosure provides a cyclic GFR-binding compound, wherein said cyclic GFR-binding compound is a cyclic peptide, a variant or analog thereof, or a cyclic peptidomimetic as defined herein, with between 15-60 (in particular between 15-55, more particularly between 19-60, and even more particularly between 19-55) amino acids, or between 15-35 (in particular between 15-30, more particularly between 19-35, and even more particularly between 19-30) amino acids, comprising a peptide with four amino acids (PEP12) and a peptide with five amino acids (PEP2); wherein said cyclic GFR-binding compound further comprises a peptide with between six and twelve amino acids (PEP9), a peptide with three amino acids (PEP4), and an amino acid or a peptide with between two and six amino acids (PEP8).

In one aspect, the present disclosure provides a cyclic GFR-binding compound, wherein said cyclic GFR-binding compound is a cyclic peptide, a variant or analog thereof, or a cyclic peptidomimetic as defined herein, with between 15-60 (in particular between 15-55, more particularly between 19-60, and even more particularly between 19-55) amino acids, or between 15-35 (in particular between 15-30, more particularly between 19-35, and even more particularly between 19-30) amino acids, comprising a peptide with four amino acids (PEP1) and a peptide with five amino acids (PEP2); wherein said cyclic GFR-binding compound further comprises a peptide with between six and twelve amino acids (PEP9), a peptide with between five and seven amino acids (PEP6), and an amino acid or a peptide with between two and six amino acids (PEP8).

In one aspect, the present disclosure provides a cyclic GFR-binding compound, wherein said cyclic GFR-binding compound is a cyclic peptide, a variant or analog thereof, or a cyclic peptidomimetic as defined herein, with between 15-60 (in particular between 15-55, more particularly between 19-60, and even more particularly between 19-55) amino acids, or between 15-35 (in particular between 15-30, more particularly between 19-35, and even more particularly between 19-30) amino acids, comprising a peptide with four amino acids (PEP12) and a peptide with five amino acids (PEP2); wherein said cyclic GFR-binding compound further comprises a peptide with between six and twelve amino acids (PEP9), a peptide with between five and seven amino acids (PEP6), and an amino acid or a peptide with between two and six amino acids (PEP8).

In one aspect, the present disclosure provides a cyclic GFR-binding compound, wherein said cyclic GFR-binding compound is a cyclic peptide, a variant or analog thereof, or a cyclic peptidomimetic as defined herein, with between 15-60 (in particular between 15-55, more particularly between 19-60, and even more particularly between 19-55) amino acids, or between 15-35 (in particular between 15-30, more particularly between 19-35, and even more particularly between 19-30) amino acids, comprising a peptide, a variant or analog thereof, or a peptidomimetic having the following general formula (Ia) (hereinafter may also be referred to as compound (Ia) or peptide (Ia)):

PEP(A)-LINKER-PEP(B) (Ia)

wherein one end of LINKER interacts covalently with one end of PEP(A); wherein another end of LINKER interacts covalently with one end of PEP(B); wherein PEP(A) comprises PEP1; wherein PEP(B) comprises PEP2; wherein LINKER is a linear or branched organic divalent radical, moiety or compound having a molecular weight (Mw) comprised between about 28 and about 2,000 Da, in particular, between about 300 and about 1000 Da, more particularly between about 300 and about 800 Da.

In one aspect, the present disclosure provides a cyclic GFR-binding compound, wherein said cyclic GFR-binding compound is a cyclic peptide, a variant or analog thereof, or a cyclic peptidomimetic as defined herein, with between 15-60 (in particular between 15-55, more particularly between 19-60, and even more particularly between 19-55) amino acids, or between 15-35 (in particular between 15-30, more particularly between 19-35, and even more particularly between 19-30) amino acids, comprising a peptide, a variant or analog thereof, or a peptidomimetic having the following general formula (Ib) (hereinafter may also be referred to as compound (Ib) or peptide (Ib)):

PEP(A)-LINKER-PEP(B)-LINKER (Ib)

wherein one end of a first LINKER interacts covalently with one end of PEP(A); wherein another end of a first LINKER interacts covalently with one end of PEP(B); wherein one end of a second LINKER interacts covalently with another end of PEP(B); wherein another end of a second LINKER interacts covalently with another end of PEP(A); wherein PEP(A) comprises PEP1; wherein PEP(B) comprises PEP2; wherein LINKER are independently a linear or branched organic divalent radical, moiety or compound having a molecular weight (Mw) comprised between about 28 and about 2,000 Da, in particular, between about 300 and about 1000 Da, more particularly between about 300 and about 800 Da.

In the present description, the molecular weight of LINKER refer to the calculated molecular weight prior to being connected to / reacted with any of the elements it is configured to connect to or react with e.g. PEP(A), PEP(B) or any other groups defined herein.

15-60 (in particular between 15-55, more particularly between 19-60, and even more particularly between 19-55) amino acids, or between 15-35 (in particular between 15-30, In one aspect, the present disclosure provides a cyclic GFR-binding compound comprising compounds (Ia) or (Ib), wherein PEP(A) comprises PEP1; wherein PEP(B) comprises PEP2; wherein PEP(A) further comprises PEP3; wherein PEP(B) further comprises PEP4.

In one aspect, the present disclosure provides a cyclic GFR-binding compound comprising compounds (Ia) or (Ib), wherein PEP(A) comprises PEP12; wherein PEP(B) comprises PEP2; wherein PEP(A) further comprises PEP3; wherein PEP(B) further comprises PEP4.

In one aspect, the present disclosure provides a cyclic GFR-binding compound comprising compounds (Ia) or (Ib), wherein PEP(A) comprises PEP1; wherein PEP(B) comprises PEP2; wherein PEP(A) further comprises PEP5; wherein PEP(B) further comprises PEP6.

In one aspect, the present disclosure provides a cyclic GFR-binding compound comprising compounds (Ia) or (Ib), wherein PEP(A) comprises PEP12; wherein PEP(B) comprises PEP2; wherein PEP(A) further comprises PEP5; wherein PEP(B) further comprises PEP6.

In one aspect, the present disclosure provides a cyclic GFR-binding compound comprising compounds (Ia) or (Ib), wherein PEP(A) comprises PEP1; wherein PEP(B) comprises PEP2; wherein PEP(A) further comprises PEP9; wherein PEP(B) further comprises PEP10.

In one aspect, the present disclosure provides a cyclic GFR-binding compound comprising compounds (Ia) or (Ib), wherein PEP(A) comprises PEP12; wherein PEP(B) comprises PEP2; wherein PEP(A) further comprises PEP9; wherein PEP(B) further comprises PEP10.

In one aspect, the present disclosure provides a cyclic GFR-binding compound comprising compounds (Ia) or (Ib), wherein PEP(A) comprises PEP1; wherein PEP(B) comprises PEP2; wherein PEP(A) further comprises PEP3; wherein PEP(B) further comprises PEP6.

In one aspect, the present disclosure provides a cyclic GFR-binding compound comprising compounds (Ia) or (Ib), wherein PEP(A) comprises PEP12; wherein PEP(B) comprises PEP2; wherein PEP(A) further comprises PEP3; wherein PEP(B) further comprises PEP6.

In one aspect, the present disclosure provides a cyclic GFR-binding compound comprising compounds (Ia) or (Ib), wherein PEP(A) comprises PEP1; wherein PEP(B) comprises PEP2; wherein PEP(A) further comprises PEP3; wherein PEP(B) further comprises PEP10.

In one aspect, the present disclosure provides a cyclic GFR-binding compound comprising compounds (Ia) or (Ib), wherein PEP(A) comprises PEP12; wherein PEP(B) comprises PEP2; wherein PEP(A) further comprises PEP3; wherein PEP(B) further comprises PEP10.

In one aspect, the present disclosure provides a cyclic GFR-binding compound comprising compounds (Ia) or (Ib), wherein PEP(A) comprises PEP1; wherein PEP(B) comprises PEP2; wherein PEP(A) further comprises PEP5; wherein PEP(B) further comprises PEP4.

In one aspect, the present disclosure provides a cyclic GFR-binding compound comprising compounds (Ia) or (Ib), wherein PEP(A) comprises PEP12; wherein PEP(B) comprises PEP2; wherein PEP(A) further comprises PEP5; wherein PEP(B) further comprises PEP4.

In one aspect, the present disclosure provides a cyclic GFR-binding compound comprising compounds (Ia) or (Ib), wherein PEP(A) comprises PEP1; wherein PEP(B) comprises PEP2; wherein PEP(A) further comprises PEP9; wherein PEP(B) further comprises PEP4.

In one aspect, the present disclosure provides a cyclic GFR-binding compound comprising compounds (Ia) or (Ib), wherein PEP(A) comprises PEP12; wherein PEP(B) comprises PEP2; wherein PEP(A) further comprises PEP9; wherein PEP(B) further comprises PEP4.

In one aspect, the present disclosure provides a cyclic GFR-binding compound comprising compounds (Ia) or (Ib), wherein PEP(A) comprises PEP1; wherein PEP(B) comprises PEP2; wherein PEP(A) further comprises PEP5; wherein PEP(B) further comprises PEP10.

In one aspect, the present disclosure provides a cyclic GFR-binding compound comprising compounds (Ia) or

(Ib), wherein PEP(A) comprises PEP12; wherein PEP(B) comprises PEP2; wherein PEP(A) further comprises PEP5; wherein PEP(B) further comprises PEP10.

In one aspect, the present disclosure provides a cyclic GFR-binding compound comprising compounds (Ia) or (Ib), wherein PEP(A) comprises PEP1; wherein PEP(B) comprises PEP2; wherein PEP(A) further comprises PEP9; wherein PEP(B) further comprises PEP6.

In one aspect, the present disclosure provides a cyclic GFR-binding compound comprising compounds (Ia) or (Ib), wherein PEP(A) comprises PEP12; wherein PEP(B) comprises PEP2; wherein PEP(A) further comprises PEP9; wherein PEP(B) further comprises PEP6.

In one aspect, the present disclosure provides a cyclic GFR-binding compound comprising compounds (Ia) or (Ib), wherein PEP(A) comprises PEP1; wherein PEP(B) comprises PEP2; wherein PEP(A) further comprises PEP3 and PEP7; wherein PEP(B) further comprises PEP4.

In one aspect, the present disclosure provides a cyclic GFR-binding compound comprising compounds (Ia) or (Ib), wherein PEP(A) comprises PEP12; wherein PEP(B) comprises PEP2; wherein PEP(A) further comprises PEP3 and PEP7; wherein PEP(B) further comprises PEP4.

In one aspect, the present disclosure provides a cyclic GFR-binding compound comprising compounds (Ia) or (Ib), wherein PEP(A) comprises PEP1; wherein PEP(B) comprises PEP2; wherein PEP(A) further comprises PEP3 and PEP7; wherein PEP(B) further comprises PEP6.

In one aspect, the present disclosure provides a cyclic GFR-binding compound comprising compounds (Ia) or (Ib), wherein PEP(A) comprises PEP12; wherein PEP(B) comprises PEP2; wherein PEP(A) further comprises PEP3 and PEP7; wherein PEP(B) further comprises PEP6.

In one aspect, the present disclosure provides a cyclic GFR-binding compound comprising compounds (Ia) or (Ib), wherein PEP(A) comprises PEP1; wherein PEP(B) comprises PEP2; wherein PEP(A) further comprises PEP3 and PEP7; wherein PEP(B) further comprises PEP10.

In one aspect, the present disclosure provides a cyclic GFR-binding compound comprising compounds (Ia) or (Ib), wherein PEP(A) comprises PEP12; wherein PEP(B) comprises PEP2; wherein PEP(A) further comprises PEP3 and PEP7; wherein PEP(B) further comprises PEP10.

In one aspect, the present disclosure provides a cyclic GFR-binding compound comprising compounds (Ia) or (Ib), wherein PEP(A) comprises PEP1; wherein PEP(B) comprises PEP2; wherein PEP(A) further comprises PEP7; wherein PEP(B) further comprises PEP4 and PEP8.

In one aspect, the present disclosure provides a cyclic GFR-binding compound comprising compounds (Ia) or (Ib), wherein PEP(A) comprises PEP12; wherein PEP(B) comprises PEP2; wherein PEP(A) further comprises PEP7; wherein PEP(B) further comprises PEP4 and PEP8.

In one aspect, the present disclosure provides a cyclic GFR-binding compound comprising compounds (Ia) or (Ib), wherein PEP(A) comprises PEP1; wherein PEP(B) comprises PEP2; wherein PEP(A) further comprises PEP7; wherein PEP(B) further comprises PEP6 and PEP8.

In one aspect, the present disclosure provides a cyclic GFR-binding compound comprising compounds (Ia) or (Ib), wherein PEP(A) comprises PEP12; wherein PEP(B) comprises PEP2; wherein PEP(A) further comprises PEP7; wherein PEP(B) further comprises PEP6 and PEP8.

In one aspect, the present disclosure provides a cyclic GFR-binding compound comprising compounds (Ia) or (Ib), wherein PEP(A) comprises PEP1; wherein PEP(B) comprises PEP2; wherein PEP(A) further comprises PEP5 and PEP7; wherein PEP(B) further comprises PEP4.

In one aspect, the present disclosure provides a cyclic GFR-binding compound comprising compounds (Ia) or (Ib), wherein PEP(A) comprises PEP12; wherein PEP(B) comprises PEP2; wherein PEP(A) further comprises PEP5 and PEP7; wherein PEP(B) further comprises PEP4.

In one aspect, the present disclosure provides a cyclic GFR-binding compound comprising compounds (Ia) or (Ib), wherein PEP(A) comprises PEP1; wherein PEP(B) comprises PEP2; wherein PEP(A) further comprises PEP5 and PEP7; wherein PEP(B) further comprises PEP6.

In one aspect, the present disclosure provides a cyclic GFR-binding compound comprising compounds (Ia) or (Ib), wherein PEP(A) comprises PEP12; wherein PEP(B) comprises PEP2; wherein PEP(A) further comprises PEP5 and PEP7; wherein PEP(B) further comprises PEP6.

In one aspect, the present disclosure provides a cyclic GFR-binding compound comprising compounds (Ia) or (Ib), wherein PEP(A) comprises PEP1; wherein PEP(B) comprises PEP2; wherein PEP(A) further comprises PEP5 and PEP7; wherein PEP(B) further comprises PEP10.

In one aspect, the present disclosure provides a cyclic GFR-binding compound comprising compounds (Ia) or (Ib), wherein PEP(A) comprises PEP12; wherein PEP(B) comprises PEP2; wherein PEP(A) further comprises PEP5 and PEP7; wherein PEP(B) further comprises PEP10.

In one aspect, the present disclosure provides a cyclic GFR-binding compound comprising compounds (Ia) or (Ib), wherein PEP(A) comprises PEP1; wherein PEP(B) comprises PEP2; wherein PEP(A) further comprises PEP5 and

PEP7; wherein PEP(B) further comprises PEP4 and PEP8.

In one aspect, the present disclosure provides a cyclic GFR-binding compound comprising compounds (Ia) or (Ib), wherein PEP(A) comprises PEP12; wherein PEP(B) comprises PEP2; wherein PEP(A) further comprises PEP5 and PEP7; wherein PEP(B) further comprises PEP4 and PEP8.

In one aspect, the present disclosure provides a cyclic GFR-binding compound comprising compounds (Ia) or (Ib), wherein PEP(A) comprises PEP1; wherein PEP(B) comprises PEP2; wherein PEP(A) further comprises PEP5 and PEP7; wherein PEP(B) further comprises PEP6 and PEP8.

In one aspect, the present disclosure provides a cyclic GFR-binding compound comprising compounds (Ia) or (Ib), wherein PEP(A) comprises PEP12; wherein PEP(B) comprises PEP2; wherein PEP(A) further comprises PEP5 and PEP7; wherein PEP(B) further comprises PEP6 and PEP8.

In one aspect, the present disclosure provides a cyclic GFR-binding compound comprising compounds (Ia) or (Ib), wherein PEP(A) comprises PEP1; wherein PEP(B) comprises PEP2; wherein PEP(A) further comprises PEP9; wherein PEP(B) further comprises PEP4 and PEP8.

In one aspect, the present disclosure provides a cyclic GFR-binding compound comprising compounds (Ia) or (Ib), wherein PEP(A) comprises PEP12; wherein PEP(B) comprises PEP2; wherein PEP(A) further comprises PEP9; wherein PEP(B) further comprises PEP4 and PEP8.

In one aspect, the present disclosure provides a cyclic GFR-binding compound comprising compounds (Ia) or (Ib), wherein PEP(A) comprises PEP1; wherein PEP(B) comprises PEP2; wherein PEP(A) further comprises PEP9; wherein PEP(B) further comprises PEP6 and PEP8.

In one aspect, the present disclosure provides a cyclic GFR-binding compound comprising compounds (Ia) or (Ib), wherein PEP(A) comprises PEP12; wherein PEP(B) comprises PEP2; wherein PEP(A) further comprises PEP9; wherein PEP(B) further comprises PEP6 and PEP8.

In one aspect, the present disclosure provides a cyclic GFR-binding compound, wherein said cyclic GFR-binding compound is a cyclic peptide, a variant or analog thereof, or a cyclic peptidomimetic as defined herein, with between 15-60 (in particular between 15-55, more particularly between 19-60, and even more particularly between 19-55) amino acids, or between 15-35 (in particular between 15-30, more particularly between 19-35, and even more particularly between 19-30) amino acids, comprising a peptide, a variant or analog thereof, or a peptidomimetic having the following general formula (IIa) (hereinafter may also be referred to as compound (IIa) or peptide (IIa)):

PEP(C)-PEP12-LINKER-PEP2-PEP(D) (IIa)

wherein LINKER is a linear or branched organic divalent radical, moiety or compound having a molecular weight (Mw) comprised between about 28 and about 2,000 Da, in particular, between about 300 and about 1000 Da, more particularly between about 300 and about 800 Da; wherein PEP12 is a peptide with 8 amino acids of formula PEP1-$AA^{17}$-PEP11 as defined herein; wherein PEP2 is a peptide with five amino acids as already defined herein; wherein one end of PEP(C) interacts covalently with PEP12 via one end of PEP1; wherein one end of PEP(D) interacts covalently with one end of PEP2; wherein one end of LINKER interacts covalently with one end of PEP12 via one end of PEP11; wherein another end of LINKER interacts covalently with another end of PEP2; wherein PEP(C) is a peptide with at least 5 amino acids, in particular a peptide with between 5 and 12 amino acids; wherein PEP(D) is a peptide with at least 5 amino acids, in particular a peptide with between 5 and 11 amino acids.

In one aspect, the present disclosure provides a cyclic GFR-binding compound, wherein said cyclic GFR-binding compound is a cyclic peptide, a variant or analog thereof, or a cyclic peptidomimetic as defined herein, with between 15-60 (in particular between 15-55, more particularly between 19-60, and even more particularly between 19-55) amino acids, or between 15-35 (in particular between 15-30, more particularly between 19-35, and even more particularly between 19-30) amino acids, comprising a peptide, a variant or analog thereof, or a peptidomimetic having the following general formula (IIb) (hereinafter may also be referred to as compound (IIb) or peptide (IIb)):

PEP(C)-PEP12-LINKER-PEP2-PEP(D)-LINKER (IIb)

wherein LINKER are independently a linear or branched organic divalent radical, moiety or compound having a molecular weight (Mw) comprised between about 28 and about 2,000 Da, in particular, between about 300 and about 1000 Da, more particularly between about 300 and about 800 Da; wherein PEP12 is a peptide with 8 amino acids of formula PEP1-$AA^{17}$-PEP11 as defined herein; wherein PEP2 is a peptide with five amino acids as already defined herein; wherein one end of PEP(C) interacts covalently with PEP12 via one end of PEP1; wherein one end of PEP(D) interacts covalently with one end of PEP2; wherein one end of a first LINKER interacts covalently with one end of PEP12 via one end of PEP11; wherein another end of a first LINKER interacts covalently with another end of PEP2; wherein one end of a second LINKER interacts covalently with another end of PEP(D); wherein another end of a second LINKER interacts covalently with another end of PEP(C); wherein PEP(C) is a peptide with at least 5 amino acids, in particular a peptide

with between 5 and 12 amino acids; wherein PEP(D) is a peptide with at least 5 amino acids, in particular a peptide with between 5 and 11 amino acids.

In one aspect, the present disclosure provides a cyclic GFR-binding compound comprising compounds (IIa) or (IIb), wherein PEP(C) comprises PEP3; wherein PEP(D) comprises PEP4.

In one aspect, the present disclosure provides a cyclic GFR-binding compound comprising compounds (IIa) or (IIb), wherein PEP(C) comprises PEP5; wherein PEP(D) comprises PEP6. In one particular example, PEP(C) is PEP5; and PEP(D) is PEP6.

In one aspect, the present disclosure provides a cyclic GFR-binding compound comprising compounds (IIa) or (IIb), wherein PEP(C) comprises PEP9; wherein PEP(D) comprises PEP10. In one particular example, PEP(C) is PEP9; and PEP(D) is PEP10.

In one aspect, the present disclosure provides a cyclic GFR-binding compound comprising compounds (IIa) or (IIb), wherein PEP(C) comprises PEP3; wherein PEP(D) comprises PEP6. In one particular example, PEP(C) comprises PEP3 and PEP(D) is PEP6.

In one aspect, the present disclosure provides a cyclic GFR-binding compound comprising compounds (IIa) or (IIb), wherein PEP(C) comprises PEP3; wherein PEP(D) comprises PEP10. In one particular example, PEP(C) comprises PEP3 and PEP(D) is PEP10.

In one aspect, the present disclosure provides a cyclic GFR-binding compound comprising compounds (IIa) or (IIb), wherein PEP(C) comprises PEP5; wherein PEP(D) comprises PEP4. In one particular example, PEP(C) is PEP5 and PEP(D) comprises PEP4.

In one aspect, the present disclosure provides a cyclic GFR-binding compound comprising compounds (IIa) or (IIb), wherein PEP(C) comprises PEP9; wherein PEP(D) comprises PEP4. In one particular example, PEP(C) is PEP9 and PEP(D) comprises PEP4.

In one aspect, the present disclosure provides a cyclic GFR-binding compound comprising compounds (IIa) or (IIb), wherein PEP(C) comprises PEP5; wherein PEP(D) comprises PEP10. In one particular example, PEP(C) is PEP5 and PEP(D) is PEP10.

In one aspect, the present disclosure provides a cyclic GFR-binding compound comprising compounds (IIa) or (IIb), wherein PEP(C) comprises PEP9; wherein PEP(D) comprises PEP6. In one particular example, PEP(C) is PEP9 and PEP(D) is PEP6.

In one aspect, the present disclosure provides a cyclic GFR-binding compound comprising compounds (IIa) or (IIb), wherein PEP(C) comprises PEP3 and PEP7; wherein PEP(D) comprises PEP4.

In one aspect, the present disclosure provides a cyclic GFR-binding compound comprising compounds (IIa) or (IIb), wherein PEP(C) comprises PEP3 and PEP7; wherein PEP(D) comprises PEP6. In one particular example, PEP(C) comprises PEP3 and PEP7, and PEP(D) is PEP6.

In one aspect, the present disclosure provides a cyclic GFR-binding compound comprising compounds (IIa) or (IIb), wherein PEP(C) comprises PEP3 and PEP7; wherein PEP(D) comprises PEP10. In one particular example, PEP(C) comprises PEP3 and PEP7, and PEP(D) is PEP10.

In one aspect, the present disclosure provides a cyclic GFR-binding compound comprising compounds (IIa) or (IIb), wherein PEP(C) comprises PEP7; wherein PEP(D) comprises PEP4 and PEP8.

In one aspect, the present disclosure provides a cyclic GFR-binding compound comprising compounds (IIa) or (IIb), wherein PEP(C) comprises PEP7; wherein PEP(D) comprises PEP6 and PEP8.

In one aspect, the present disclosure provides a cyclic GFR-binding compound comprising compounds (IIa) or (IIb), wherein PEP(C) comprises PEP5 and PEP7; wherein PEP(D) comprises PEP4.

In one aspect, the present disclosure provides a cyclic GFR-binding compound comprising compounds (IIa) or (IIb), wherein PEP(C) comprises PEP5 and PEP7; wherein PEP(D) comprises PEP6.

In one aspect, the present disclosure provides a cyclic GFR-binding compound comprising compounds (IIa) or (IIb), wherein PEP(C) comprises PEP5 and PEP7; wherein PEP(D) comprises PEP10.

In one aspect, the present disclosure provides a cyclic GFR-binding compound comprising compounds (IIa) or (IIb), wherein PEP(C) comprises PEP5 and PEP7; wherein PEP(D) comprises PEP4 and PEP8.

In one aspect, the present disclosure provides a cyclic GFR-binding compound comprising compounds (IIa) or (IIb), wherein PEP(C) comprises PEP5 and PEP7; wherein PEP(D) comprises PEP6 and PEP8.

In one aspect, the present disclosure provides a cyclic GFR-binding compound comprising compounds (IIa) or (IIb), wherein PEP(C) comprises PEP9; wherein PEP(D) comprises PEP4 and PEP8.

In one aspect, the present disclosure provides a cyclic GFR-binding compound comprising compounds (IIa) or (IIb), wherein PEP(C) comprises PEP9; wherein PEP(D) comprises PEP6 and PEP8.

In one aspect, the present disclosure provides a cyclic GFR-binding compound comprising compounds (IIa) or (IIb), wherein PEP(C) is PEP5 or PEP9 and wherein PEP(D) is PEP6 or PEP10.

In one aspect, the present disclosure provides a cyclic GFR-binding compound, wherein said cyclic GFR-binding compound is a cyclic peptide, a variant or analog thereof, or a cyclic peptidomimetic as defined herein, with

between 15-60 (in particular between 15-55, more particularly between 19-60, and even more particularly between 19-55) amino acids, or between 15-35 (in particular between 15-30, more particularly between 19-35, and even more particularly between 19-30) amino acids, comprising a peptide, a variant or analog thereof, or a peptidomimetic having the following general formula (IIIa) (hereinafter may also be referred to as compound (IIIa) or peptide (IIIa)):

PEP7-PEP5-PEP12-LINKER-PEP2-PEP6-PEP8 (IIIa)

wherein LINKER is a linear or branched organic divalent radical, moiety or compound having a molecular weight (Mw) comprised between about 28 and about 2,000 Da, in particular, between about 300 and about 1000 Da, more particularly between about 300 and about 800 Da; wherein PEP12 is a peptide with 8 amino acids of formula PEP1-$AA^{17}$-PEP11 as defined herein; wherein PEP2 is a peptide with five amino acids as already defined herein; wherein PEP5 is a peptide with five amino acids as already defined herein; wherein PEP6 is a peptide with between five and seven amino acids as already defined herein; wherein PEP7 an amino acid or a peptide with between two and seven amino acids as already defined herein; wherein PEP8 an amino acid or a peptide with between two and six amino acids as already defined herein; wherein one end of LINKER interacts covalently with one end of PEP12 via $AA^{20}$; wherein another end of LINKER interacts covalently with one end of PEP2 via $AA^{21}$; wherein one end of PEP5 interacts covalently with another end of PEP12 via $AA^{12}$; wherein another end of PEP5 interacts covalently with one end of PEP7 via $AA^{8}$; wherein one end of PEP6 interacts covalently with another end of PEP2 via $AA^{26}$; wherein another end of PEP6 interacts covalently with one end of PEP8 via $AA^{32}$.

In one aspect, the present disclosure provides a cyclic GFR-binding compound, wherein said cyclic GFR-binding compound is a cyclic peptide, a variant or analog thereof, or a cyclic peptidomimetic as defined herein, with between 15-60 (in particular between 15-55, more particularly between 19-60, and even more particularly between 19-55) amino acids, or between 15-35 (in particular between 15-30, more particularly between 19-35, and even more particularly between 19-30) amino acids, comprising a peptide, a variant or analog thereof, or a peptidomimetic having the following general formula (IIIb) (hereinafter may also be referred to as compound (IIIb) or peptide (IIIb)):

PEP7-PEP5-PEP12-LINKER-PEP2-PEP6-PEP8-LINKER (IIIb)

wherein LINKER are independently a linear or branched organic divalent radical, moiety or compound having a molecular weight (Mw) comprised between about 28 and about 2,000 Da, in particular, between about 300 and about 1000 Da, more particularly between about 300 and about 800 Da; wherein PEP12 is a peptide with 8 amino acids of formula PEP1-$AA^{17}$-PEP11 as defined herein; wherein PEP2 is a peptide with five amino acids as already defined herein; wherein PEP5 is a peptide with five amino acids as already defined herein; wherein PEP6 is a peptide with between five and seven amino acids as already defined herein; wherein PEP7 an amino acid or a peptide with between two and seven amino acids as already defined herein; wherein PEP8 an amino acid or a peptide with between two and six amino acids as already defined herein; wherein one end of a first LINKER interacts covalently with one end of PEP12 via $AA^{20}$; wherein another end of a first LINKER interacts covalently with one end of PEP2 via $AA^{21}$; wherein one end of a second LINKER interacts covalently with another end of PEP8; wherein another end of a second LINKER interacts covalently with another end of PEP7; wherein one end of PEP5 interacts covalently with another end of PEP12 via $AA^{12}$; wherein another end of PEP5 interacts covalently with one end of PEP7 via $AA^{8}$; wherein one end of PEP6 interacts covalently with another end of PEP2 via $AA^{26}$; wherein another end of PEP6 interacts covalently with one end of PEP8 via $AA^{32}$.

In one aspect, the present disclosure provides a cyclic GFR-binding compound, wherein said cyclic GFR-binding compound is a cyclic peptide, a variant or analog thereof, or a cyclic peptidomimetic as defined herein, with between 15-60 (in particular between 15-55, more particularly between 19-60, and even more particularly between 19-55) amino acids, or between 15-35 (in particular between 15-30, more particularly between 19-35, and even more particularly between 19-30) amino acids, comprising a peptide, a variant or analog thereof, or a peptidomimetic having the following general formula (IVa) (hereinafter may also be referred to as compound (IVa) or peptide (IVa)):

$AA^{1}$-$AA^{2}$-$AA^{3}$-$AA^{4}$-$AA^{5}$-$AA^{6}$-$AA^{7}$-$AA^{8}$-$AA^{9}$-$AA^{10}$-$AA^{11}$-$AA^{12}$-$AA^{13}$-$AA^{14}$-$AA^{15}$-$AA^{16}$-$AA^{17}$-$AA^{18}$-$AA^{19}$-$AA^{20}$-LINKER-$AA^{21}$-$AA^{22}$-$AA^{23}$-$AA^{24}$-$AA^{25}$-$AA^{26}$-$AA^{27}$-$AA^{28}$-$AA^{29}$-$AA^{30}$-$AA^{31}$-$AA^{32}$-$AA^{33}$-$AA^{34}$-$AA^{35}$-$AA^{36}$-$AA^{37}$-$AA^{38}$ (IVa)

wherein LINKER is a linear or branched organic divalent radical, moiety or compound having a molecular weight (Mw) comprised between about 28 and about 2,000 Da, in particular, between about 300 and about 1000 Da, more particularly between about 300 and about 800 Da; wherein $AA^{1}$-$AA^{2}$-$AA^{3}$-$AA^{4}$-$AA^{5}$-$AA^{6}$-$AA^{7}$ is PEP7 as defined herein; wherein $AA^{13}$-$AA^{14}$-$AA^{15}$-$AA^{16}$-$AA^{17}$-$AA^{18}$-$AA^{19}$-$AA^{20}$ is PEP12 as defined herein; wherein $AA^{21}$-$AA^{22}$-$AA^{23}$-$AA^{24}$-$AA^{25}$ is PEP2 as already defined herein; wherein $AA^{8}$-$AA^{9}$-$AA^{10}$ is PEP3 as defined herein; wherein $AA^{30}$-$AA^{31}$-$AA^{32}$ is PEP4 as

defined herein; wherein $AA^{33}$-$AA^{34}$-$AA^{35}$-$AA^{36}$-$AA^{37}$-$AA^{38}$ is PEP8 as defined herein; wherein $AA^{11}$, $AA^{12}$, $AA^{26}$, $AA^{27}$, $AA^{28}$, and $AA^{29}$ are as defined herein; wherein one end of LINKER interacts covalently with $AA^{20}$; wherein another end of LINKER interacts covalently with $AA^{21}$; wherein $AA^{1}$ and $AA^{21}$ may be both N-terminal amino acids or both C-terminal amino acids; wherein $AA^{38}$ and $AA^{20}$ may be both N-terminal amino acids or both C-terminal amino acids.

In one aspect, the present disclosure provides a cyclic GFR-binding compound, wherein said cyclic GFR-binding compound is a cyclic peptide, a variant or analog thereof, or a cyclic peptidomimetic as defined herein, with between 15-60 (in particular between 15-55, more particularly between 19-60, and even more particularly between 19-55) amino acids, or between 15-35 (in particular between 15-30, more particularly between 19-35, and even more particularly between 19-30) amino acids, comprising a peptide, a variant or analog thereof, or a peptidomimetic having the following general formula (IVb) (hereinafter may also be referred to as compound (IVb) or peptide (IVb)):

$AA^{1}$-$AA^{2}$-$AA^{3}$-$AA^{4}$-$AA^{5}$-$AA^{6}$-$AA^{7}$-$AA^{8}$-$AA^{9}$-$AA^{10}$-$AA^{11}$-$AA^{12}$-$AA^{13}$-$AA^{14}$-$AA^{15}$-$AA^{16}$-$AA^{17}$-$AA^{18}$-$AA^{19}$-$AA^{20}$-LINKER-$AA^{21}$-$AA^{22}$-$AA^{23}$-$AA^{24}$-$AA^{25}$-$AA^{26}$-$AA^{27}$-$AA^{28}$-$AA^{29}$-$AA^{30}$-$AA^{31}$-$AA^{32}$-$AA^{33}$-$AA^{34}$-$AA^{35}$-$AA^{36}$-$AA^{37}$-$AA^{38}$-LINKER (IVb)

wherein LINKER are independently a linear or branched organic divalent radical, moiety or compound having a molecular weight (Mw) comprised between about 28 and about 2,000 Da, in particular, between about 300 and about 1000 Da, more particularly between about 300 and about 800 Da; wherein $AA^{1}$-$AA^{2}$-$AA^{3}$-$AA^{4}$-$AA^{5}$-$AA^{6}$-$AA^{7}$ is PEP7 as defined herein; wherein $AA^{13}$-$AA^{14}$-$AA^{15}$-$AA^{16}$-$AA^{17}$-$AA^{18}$-$AA^{19}$-$AA^{20}$ is PEP12 as defined herein; wherein $AA^{21}$-$AA^{22}$-$AA^{23}$-$AA^{24}$-$AA^{25}$ is PEP2 as already defined herein; wherein $AA^{8}$-$AA^{9}$-$AA^{10}$ is PEP3 as defined herein; wherein $AA^{30}$-$AA^{31}$-$AA^{32}$ is PEP4 as defined herein; wherein $AA^{33}$-$AA^{34}$-$AA^{35}$-$AA^{36}$-$AA^{37}$-$AA^{38}$ is PEP8 as defined herein; wherein $AA^{11}$, $AA^{12}$, $AA^{26}$, $AA^{27}$, $AA^{28}$, and $AA^{29}$ are as defined herein; wherein one end of a first LINKER interacts covalently with $AA^{20}$; wherein another end of a first LINKER interacts covalently with $AA^{21}$; wherein one end of a second LINKER interacts covalently with $AA^{38}$; wherein another end of a second LINKER interacts covalently with $AA^{1}$; wherein $AA^{1}$ may be an N-terminal amino acids or a C-terminal amino acid.

In one aspect, the present disclosure provides a cyclic GFR-binding compound, wherein said cyclic GFR-binding compound is a cyclic peptide, a variant or analog thereof, or a cyclic peptidomimetic as defined herein, with between 15-60 (in particular between 15-55, more particularly between 19-60, and even more particularly between 19-55) amino acids, or between 15-35 (in particular between 15-30, more particularly between 19-35, and even more particularly between 19-30) amino acids, comprising two LINKERs.

In one aspect, the present disclosure provides a cyclic GFR-binding compound, wherein said cyclic GFR-binding compound is a cyclic peptide, a variant or analog thereof, or a cyclic peptidomimetic as defined herein, with between 15-60 (in particular between 15-55, more particularly between 19-60, and even more particularly between 19-55) amino acids, or between 15-35 (in particular between 15-30, more particularly between 19-35, and even more particularly between 19-30) amino acids, having any one of the following schematic general formulae (V) to (XXVIII) (hereinafter may also be referred to as compounds (V) to (XXVIII) or peptides (V) to (XXVIII)):

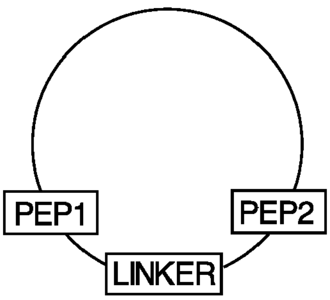

(V)

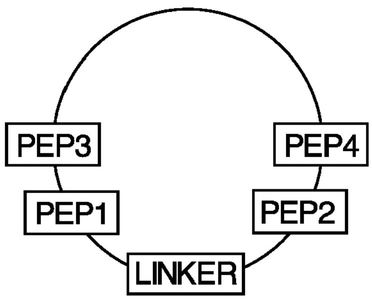

(VI)

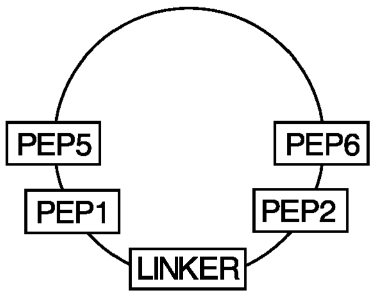

(VII)

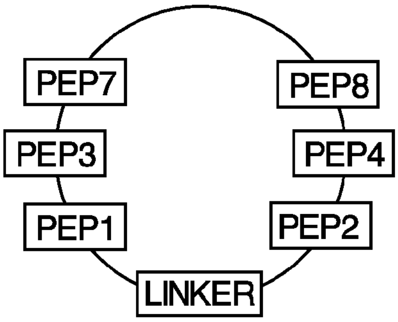


(VIII)

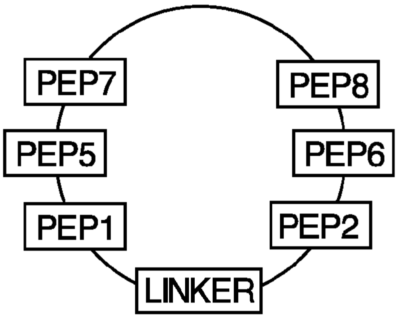


(IX)

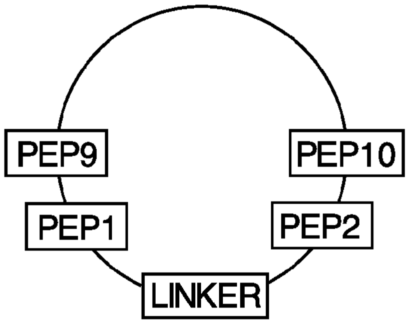


(X)

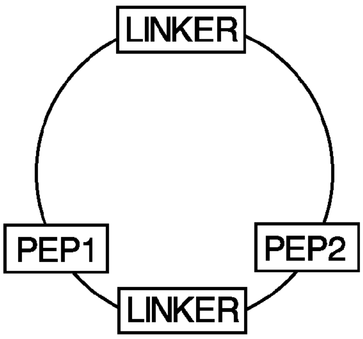


(XI)

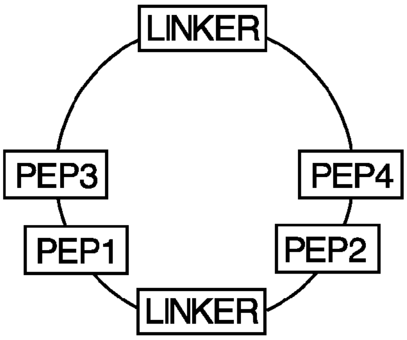


(XII)

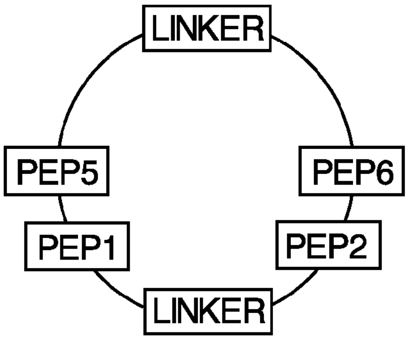


(XIII)

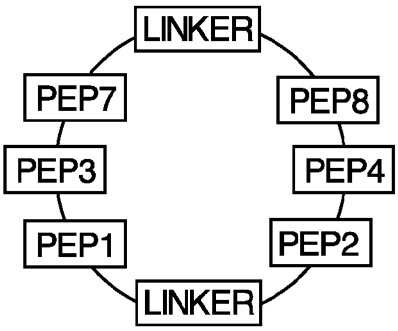


(XIV)

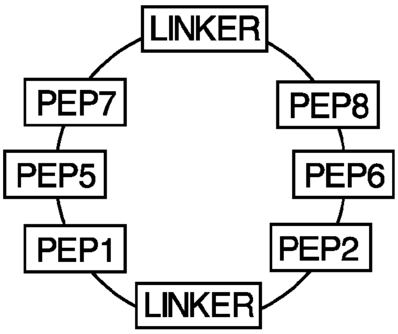


(XV)

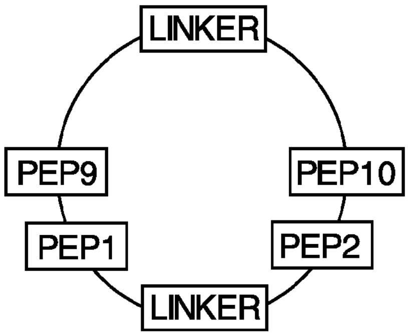


(XVI)

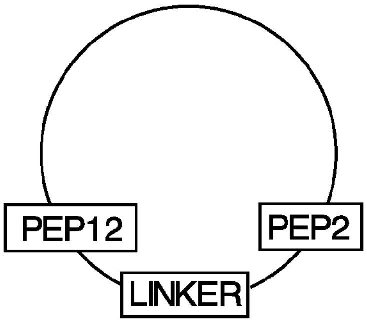


(XVII)

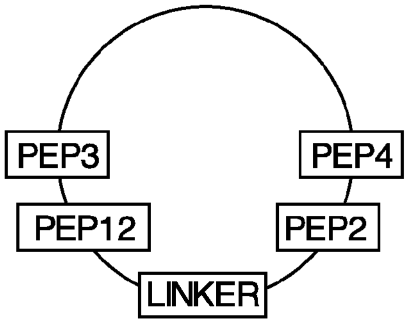


(XVIII)

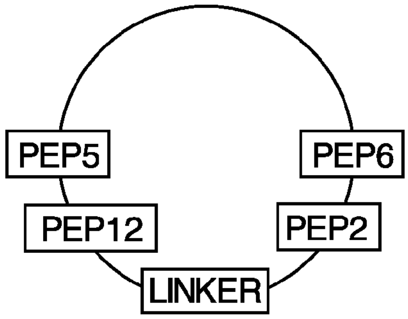


(XIX)

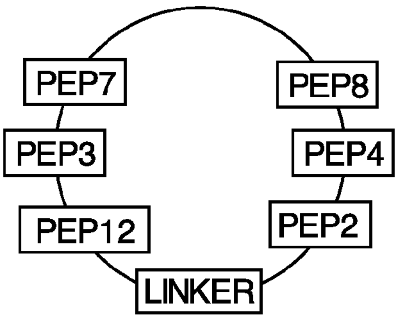


(XX)

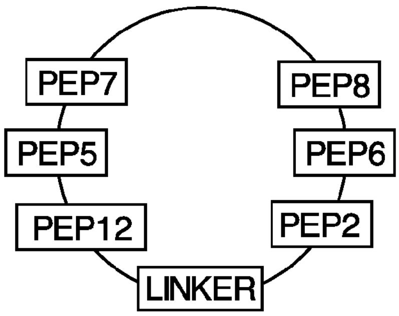


(XXI)

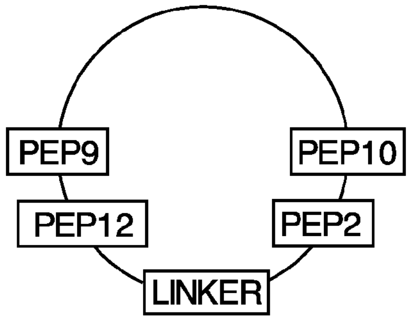


(XXII)

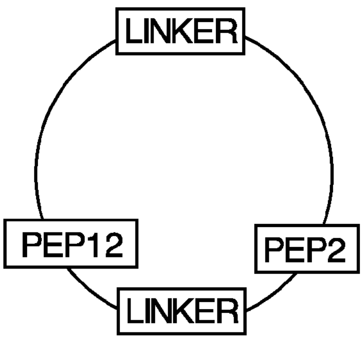


(XXIII)

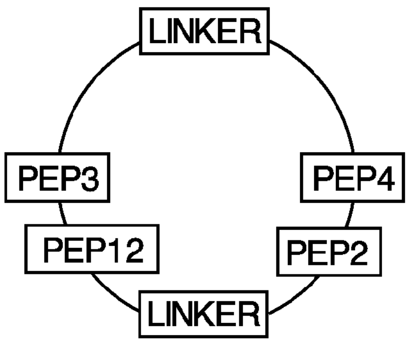


(XXIV)

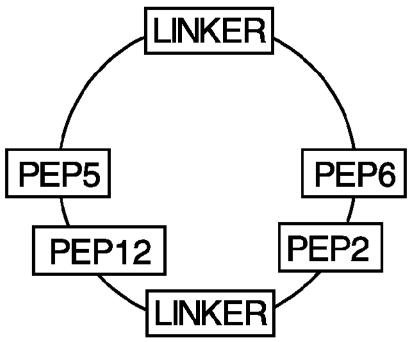


(XXV)

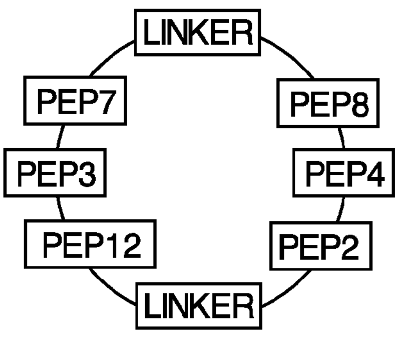


(XXVI)

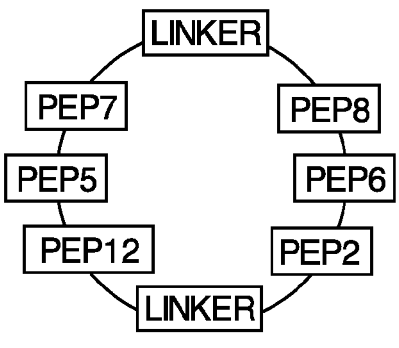


(XXVII)

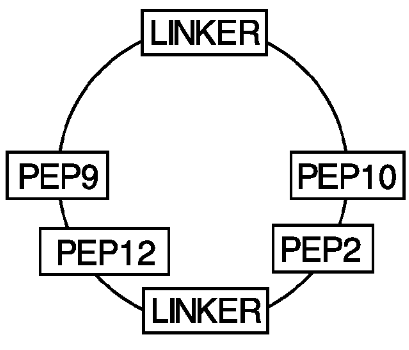


(XXVIII)

wherein LINKER is a linear or branched organic divalent radical, moiety or compound having a molecular weight (Mw) comprised between about 28 and about 2,000 Da, in particular, between about 300 and about 1000 Da, more particularly between about 300 and about 800 Da; wherein PEP12 is a peptide with 8 amino acids of formula PEP1-$AA^{17}$-PEP11 as defined herein; wherein PEP2 is a peptide with five amino acids as already defined herein; wherein PEP5 is a peptide with five amino acids as already defined herein; wherein PEP6 is a peptide with between five and seven amino acids as already defined herein; wherein PEP7 an amino acid or a peptide with between two and seven amino acids as already defined herein; wherein PEP8 an amino acid or a peptide with between two and six amino acids as already defined herein; wherein PEP9 is a peptide with between six and twelve amino acids; wherein PEP10 is a peptide with between six and eleven amino acids; wherein curved lines represents covalent bonds between LINKERs and PEP1 to PEP12 "boxes". Curved lines' lengths may not be representative of the actual relative distance between the LINKERs and PEP1 to PEP12.

In one aspect, the present disclosure provides a cyclic GFR-binding compound, wherein said cyclic GFR-binding compound is a cyclic peptide, a variant or analog thereof, or a cyclic peptidomimetic as defined herein, with between 15-60 (in particular between 15-55, more particularly between 19-60, and even more particularly between 19-55) amino acids, or between 15-35 (in particular between 15-30, more particularly between 19-35, and even more particularly between 19-30) amino acids, having any one of the following schematic general formulae (XXIX) to (XXXIV) (hereinafter may also be referred to as compounds (XXIX) to (XXXIV) or peptides (XXIX) to (XXXIV)):

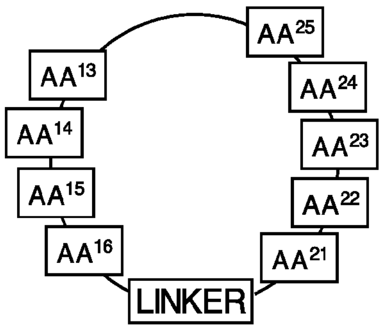


(XXIX)

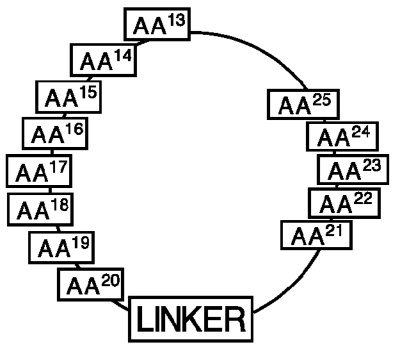


(XXX)

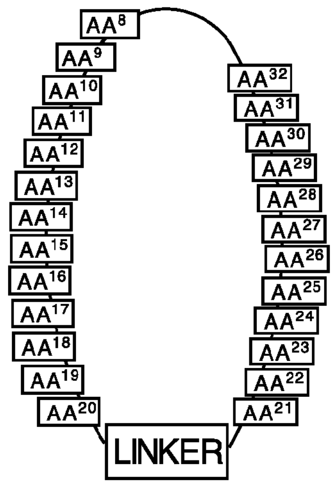


(XXXI)

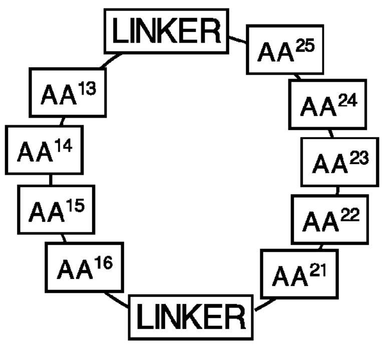


(XXXII)

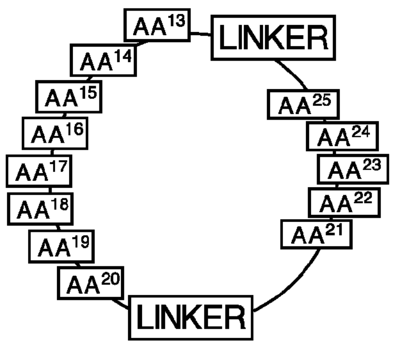


(XXXIII)

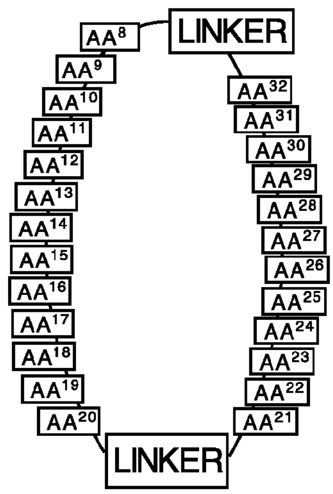


(XXXIV)

wherein LINKER is a linear or branched organic divalent radical, moiety or compound having a molecular weight (Mw) comprised between about 28 and about 2,000 Da, in particular, between about 300 and about 1000 Da, more particularly

between about 300 and about 800 Da; wherein $AA^{13}$-$AA^{14}$-$AA^{15}$-$AA^{16}$-$AA^{17}$-$AA^{18}$-$AA^{19}$-$AA^{20}$ is PEP12 as defined herein; wherein $AA^{21}$-$AA^{22}$-$AA^{23}$-$AA^{24}$-$AA^{25}$ is PEP2 as already defined herein; wherein $AA^{8}$-$AA^{9}$-$AA^{10}$ is PEP3 as defined herein; wherein $AA^{30}$-$AA^{31}$-$AA^{32}$ is PEP4 as defined herein; wherein $AA^{11}$, $AA^{12}$, $AA^{26}$, $AA^{27}$, $AA^{28}$, and $AA^{29}$ are as defined herein; wherein one end of LINKER interacts covalently with $AA^{16}$ or $AA^{20}$; wherein another end of LINKER interacts covalently with $AA^{21}$; and wherein curved lines represents covalent bonds between LINKERs and AAs "boxes". Curved lines' lengths may not be representative of the actual relative distance between the LINKERs and AAs.

Each and every definitions of PEP1, PEP2, PEP3, PEP4, PEP5, PEP6, PEP7, PEP8, PEP9, PEP10, PEP11, PEP12, $AA^{17}$, "PEP" pairs, quadruplets, hexaplets and heptuplet e.g. PEP1:PEP2, PEP12:PEP2, or PEP7:PEP5:PEP12:LINKER:PEP2:PEP6:PEP8, as already defined herein with respect to non-cyclic GFR-binding compounds also apply, without any specific restrictions unless specified otherwise or contradictory in context, to the cyclic GFR-binding compounds as defined herein.

In one example, said cyclic GFR-binding compound is a synthetic molecule as defined herein in the definition section.

In one particular example, said cyclic GFR-binding compound is a synthetic peptide, or a variant or analog thereof, or a cyclic peptidomimetic.

In one most particular example, said cyclic GFR-binding compound is a synthetic cyclic peptide.

In one example, a length of said cyclic GFR-binding compound, in solution, such as in a physiologically acceptable solvent such as water or PBS, is comprised between about 6 and about 20 nm, preferably between about 6 and about 16 nm, as determined using the standard « 3D » procedure described above.

In one particular example, said cyclic GFR-binding compounds may be any one of peptides of SEQ ID NO: 38200 to 100371.

*LINKER suitable for implementing cyclic GFR-compounds' embodiments*

In one particular example, said LINKER has a Mw comprised between about 28 and about 2,000 Da. In one particular example, said LINKER has a Mw comprised between about 75 and about 2,000 Da. In one particular example, said LINKER has a Mw comprised between about 150 and about 2,000 Da. In one particular example, said LINKER has a Mw comprised between about 150 and about 1,500 Da. In one most particular example, said LINKER has a Mw comprised between about 300 and about 1,000 Da. In one even more particular example, said LINKER has a Mw comprised between abour 300 and about 800 Da.

The chemical nature of said LINKER is not meant to be particularly limited and may be any organic molecule capable of covalently connecting two ends of a peptide or a peptidomimetic such as PEP(A)-LINKER-PEP(B) or PEP(C)-PEP12-LINKER-PEP2-PEP(D) so as to form a cyclic compound, so long as LINKER provides sufficient spacing between the two elements connected therethrough and adequate cycle stability for these two connected elements to provide or conserve the required recoding activity. LINKER may thus be, for example, in certain embodiments, a peptide, or variant, analog or peptidomimetic thereof, a polysaccharide, a polynucleotide, a saturated or unsaturated hydrocarbon chain, or a mixture thereof.

For example, in certain embodiments, LINKER is a peptide with 2 to 16 amino acids. In one particular example, LINKER is a peptide with 2 to 10 amino acids. In one particular example, LINKER is a peptide with 2 to 9 amino acids. In one most particular example, LINKER is a peptide with 3 to 9 amino acids.

Thus, in one particular example, said cyclic GFR-binding compound is a peptide, a variant or analog thereof as defined herein, with between 15-60 (in particular between 15-55, more particularly between 19-60, and even more particularly between 19-55) amino acids, or between 15-35 (in particular between 15-30, more particularly between 19-35, and even more particularly between 19-30) amino acids, comprising a peptide, a variant or analog thereof of general formula (I); wherein one end of LINKER interacts covalently with one end of PEP(A); wherein another end of LINKER interacts covalently with one end of PEP(B); wherein PEP(A) comprises PEP1 or PEP12; wherein PEP(B) comprises PEP2; wherein LINKER is a peptide comprising 2 to 16 amino acids (in particular 2 to 10, 2 to 9, or 3 to 9 amino acids).

Thus, in one particular example, said cyclic GFR-binding compound is a peptide, a variant or analog thereof as defined herein, with between 15-60 (in particular between 15-55, more particularly between 19-60, and even more particularly between 19-55) amino acids, or between 15-35 (in particular between 15-30, more particularly between 19-35, and even more particularly between 19-30) amino acids, comprising a peptide, a variant or analog thereof of general formula (II); wherein LINKER is a peptide comprising 2 to 16 amino acids (in particular 2 to 10, 2 to 9, or 3 to 9 amino acids); wherein PEP12 is a peptide with 8 amino acids of formula PEP1-$AA^{17}$-PEP11 as already defined herein; wherein PEP1, PEP2, PEP11, PEP(C) and PEP(D) are as already defined herein; wherein $AA^{13}$ and $AA^{21}$ may be both N-terminal amino acids or both C-terminal amino acids; wherein $AA^{25}$ and $AA^{20}$ may be both N-terminal amino acids or both C-terminal amino acids; wherein one end of LINKER interacts covalently with one end of PEP12 via $AA^{20}$; wherein another end of LINKER interacts covalently with PEP2 via $AA^{21}$; wherein one end of PEP(C) interacts covalently with

PEP12 via $AA^{13}$; wherein one end of PEP(D) interacts covalently with PEP2 via $AA^{25}$.

Thus, in one particular example, said cyclic GFR-binding compound is a peptide, a variant or analog thereof as defined herein, with between 15-60 (in particular between 15-55, more particularly between 19-60, and even more particularly between 19-55) amino acids, or between 15-35 (in particular between 15-30, more particularly between 19-35, and even more particularly between 19-30) amino acids, having any one of the general formula (V) to (XXXIV); wherein LINKER is a peptide comprising 2 to 16 amino acids (in particular 2 to 10, 2 to 9, or 3 to 9 amino acids).

In certain embodiments, said cyclic GFR-binding compound is a cyclic peptidomimetic as defined herein, with between 15-60 (in particular between 15-55, more particularly between 19-60, and even more particularly between 19-55) amino acids, or between 15-35 (in particular between 15-30, more particularly between 19-35, and even more particularly between 19-30) amino acids, of general formula (II); wherein LINKER is not a peptide but may comprise amino acids or peptides in covalent or non-covalent (preferably covalent) association with other groups or residues other than amino acids or peptides.

In one particular example, LINKER comprises (or is) a peptide of general formula (XXXV):

$$*AA^{201}\text{-}AA^{202}\text{-}AA^{203}\text{-}AA^{204}\text{-}AA^{205}\text{-}AA^{206}\text{-}AA^{207}\text{-}AA^{208}\text{-}AA^{209}** \qquad (XXXV)$$

wherein said peptide of formula (XXXV) may be selected from the group consisting of $*AA^{III}\text{-}AA^{I}\text{-}AA^{I}\text{-}AA^{II}\text{-}AA^{VII}\text{-}AA^{XII}\text{-}AA^{XII}\text{-}AA^{XIII}\text{-}AA^{XIII}**$,$*AA^{III}\text{-}AA^{I}\text{-}AA^{VI}AA^{II}\text{-}AA^{VII}\text{-}AA^{XII}\text{-}AA^{XIII}\text{-}AA^{XIII}**$, $AA^{III}\text{-}AA^{I}\text{-}AA^{I}\text{-}AA^{II}\text{-}AA^{II}\text{-}AA^{XII}\text{-}AA^{XII}\text{-}AA^{XIII}\text{-}AA^{XIII}**$, $*AA^{III}\text{-}AA^{I}\text{-}AA^{II}\text{-}AA^{II}\text{-}AA^{VII}\text{-}AA^{XII}\text{-}AA^{XII}\text{-}AA^{XIII}\text{-}AA^{XIII}**$, $*AA^{III}\text{-}AA^{I}\text{-}AA^{II}\text{-}AA^{IV}\text{-}AA^{IV}\text{-}AA^{XII}\text{-}AA^{XII}\text{-}AA^{XIII}\text{-}AA^{XIII}**$, $*AA^{II}\text{-}AA^{I}\text{-}AA^{II}\text{-}AA^{II}\text{-}AA^{II}\text{-}AA^{XII}\text{-}AA^{XII}\text{-}AA^{XIII}\text{-}AA^{XIII}**$, $*AA^{III}\text{-}AA^{III}\text{-}AA^{II}\text{-}AA^{VII}\text{-}AA^{II}\text{-}AA^{XII}\text{-}AA^{XII}\text{-}AA^{XIII}\text{-}AA^{XIII}**$, $*AA^{III}\text{-}AA^{I}\text{-}AA^{I}\text{-}AA^{II}\text{-}AA^{II}\text{-}AA^{XII}\text{-}AA^{XII}\text{-}AA^{XIII}\text{-}AA^{XIII}**$, $*AA^{V}\text{-}AA^{V}\text{-}AA^{VII}\text{-}AA^{VII}\text{-}AA^{XII}\text{-}AA^{XII}\text{-}AA^{XIII}\text{-}AA^{XIII}**$, $*AA^{VIII}\text{-}AA^{V}\text{-}AA^{VII}\text{-}AA^{II}\text{-}AA^{XII}\text{-}AA^{XII}\text{-}AA^{XIII}\text{-}AA^{XIII}**$ and $*AA^{V}\text{-}AA^{V}\text{-}AA^{VII}\text{-}AA^{II}\text{-}AA^{XII}\text{-}AA^{XII}\text{-}AA^{XIII}\text{-}AA^{XIII}**$; wherein $AA^{I}$ is any amino acid as defined herein, $AA^{II}$ is any polar amino acid as defined herein, $AA^{III}$ is any acidic amino acid as defined herein, $AA^{IV}$ is any aliphatic amino acid as defined herein, $AA^{V}$ is any apolar amino acid as defined herein, $AA^{VI}$ is any aromatic amino acid as defined herein, $AA^{VII}$ is any basic amino acid as defined herein, $AA^{VIII}$ is L or I as defined herein, $AA^{XII}$ is an amino acid selected from the group consisting of G, A, V, L, I, P, M, K, R, H, Y and E, wherein $AA^{XIII}$ is absent, $AA^{II}$ or $AA^{VII}$, preferably absent; and wherein any one of the fragment $AA^{201}$, $AA^{201}\text{-}AA^{202}$, $AA^{201}\text{-}AA^{202}\text{-}AA^{203}$, $AA^{201}\text{-}AA^{202}\text{-}AA^{203}\text{-}AA^{204}$, $AA^{201}\text{-}AA^{202}\text{-}AA^{203}\text{-}AA^{204}\text{-}AA^{205}$, $AA^{201}\text{-}AA^{202}\text{-}AA^{203}\text{-}AA^{204}\text{-}AA^{205}\text{-}AA^{206}$, $AA^{201}\text{-}AA^{202}\text{-}AA^{203}\text{-}AA^{204}\text{-}AA^{205}\text{-}AA^{206}\text{-}AA^{207}$, $AA^{203}\text{-}AA^{204}\text{-}AA^{205}\text{-}AA^{206}\text{-}AA^{207}\text{-}AA^{208}\text{-}AA^{209}$, $AA^{204}\text{-}AA^{205}\text{-}AA^{206}\text{-}AA^{207}\text{-}AA^{208}\text{-}AA^{209}$, $AA^{205}\text{-}AA^{206}\text{-}AA^{207}\text{-}AA^{208}\text{-}AA^{209}$, $AA^{206}\text{-}AA^{207}\text{-}AA^{208}\text{-}AA^{209}$, $AA^{207}\text{-}AA^{208}\text{-}AA^{209}$, $AA^{208}\text{-}AA^{209}$ or $AA^{209}$, may be absent. In the peptides of formula (XXXV), any one of the amino acid labelled "*" or the amino acid labelled "**" is an N-terminal amino acid and the other is a C-terminal amino acid.

For instance, in the peptide of formula $*AA^{III}\text{-}AA^{I}\text{-}AA^{I}\text{-}AA^{II}\text{-}AA^{VII}\text{-}AA^{XII}\text{-}AA^{XII}\text{-}AA^{XIII}\text{-}AA^{XIII}**$ (XXXV-1), $AA^{III}$ occupies position $AA^{201}$, $*AA^{I}$ occupies position $AA^{202}$, $AA^{I}$ occupies position $AA^{203}$, $AA^{II}$ occupies position $AA^{204}$, $AA^{VII}$ occupies position $AA^{205}$, $AA^{XII}$ occupies position $AA^{206}$, $AA^{XII}$ occupies position $AA^{207}$, $AA^{XIII}$ occupies position $AA^{208}$ and $AA^{XIII}**$ occupies position $AA^{209}$.

Likewise, in the peptide of formula $*AA^{V}\text{-}AA^{V}\text{-}AA^{VII}\text{-}AA^{II}\text{-}AA^{XII}\text{-}AA^{XII}\text{-}AA^{XIII}\text{-}AA^{XIII}**$ (XXXV-1) $AA^{V}$ occupies position $AA^{201}$, $AA^{V}$ occupies position $AA^{202}$, $AA^{VII}$ occupies position $AA^{203}$, $AA^{II}$ occupies position $AA^{204}$, $AA^{XII}$ occupies position $AA^{205}$, $AA^{XII}$ occupies position $AA^{206}$, $AA^{XIII}$ occupies position $AA^{207}$, $AA^{XIII}$ occupies position $AA^{208}$ and position $AA^{209}$ is vacant, thus $AA^{208}$ would be the amino acid which interacts, in certain embodiments, with PEP2 via $AA^{21}$.

In one example, LINKER may thus be any one of the following peptides: $*AA^{201}\text{-}AA^{202}\text{-}AA^{203}\text{-}AA^{204}\text{-}AA^{205}\text{-}AA^{206}\text{-}AA^{207}\text{-}AA^{208}**$ (XXXV-1); $*AA^{201}\text{-}AA^{202}\text{-}AA^{203}\text{-}AA^{204}\text{-}AA^{205}\text{-}AA^{206}\text{-}AA^{207}**$ (XXXV-2); $*AA^{201}\text{-}AA^{202}\text{-}AA^{203}\text{-}AA^{204}\text{-}AA^{205}\text{-}AA^{206}**$ (XXXV-3); $*AA^{201}\text{-}AA^{202}\text{-}AA^{203}\text{-}AA^{204}\text{-}AA^{205}**$ (XXXV-4); $*AA^{201}\text{-}AA^{202}\text{-}AA^{203}\text{-}AA^{204}**$ (XXXV-5); $*AA^{201}\text{-}AA^{202}\text{-}AA^{203}**$ (XXXV-6); $*AA^{201}\text{-}AA^{202}**$ (XXXV-7); $*AA^{202}\text{-}AA^{203}\text{-}AA^{204}\text{-}AA^{205}\text{-}AA^{206}\text{-}AA^{207}\text{-}AA^{208}\text{-}AA^{209}**$ (XXXV-8); $*AA^{203}\text{-}AA^{204}\text{-}AA^{205}\text{-}AA^{206}\text{-}AA^{207}\text{-}AA^{208}\text{-}AA^{209}**$ (XXXV-9); $*AA^{204}\text{-}AA^{205}\text{-}AA^{206}\text{-}AA^{207}\text{-}AA^{208}\text{-}AA^{209}**$ (XXXV-10); $*AA^{205}\text{-}AA^{206}\text{-}AA^{207}\text{-}AA^{208}\text{-}AA^{209}**$ (XXXV-11); $*AA^{206}\text{-}AA^{207}\text{-}AA^{208}\text{-}AA^{209}**$ (XXXV-12); $*AA^{207}\text{-}AA^{208}\text{-}AA^{209}**$ (XXXV-13); $*AA^{208}\text{-}AA^{209}**$ (XXXV-14); wherein, for instance, said peptide of formulae (XXXV-1) may thus be selected from the group consisting of $*AA^{III}\text{-}AA^{I}\text{-}AA^{I}\text{-}AA^{II}\text{-}AA^{VII}\text{-}AA^{XII}\text{-}AA^{XII}\text{-}AA^{XIII}**$, $*AA^{III}\text{-}AA^{I}\text{-}AA^{VI}\text{-}AA^{II}\text{-}AA^{VII}\text{-}AA^{XII}\text{-}AA^{XII}\text{-}AA^{XIII}**$, $*AA^{III}\text{-}AA^{I}\text{-}AA^{I}\text{-}AA^{II}\text{-}AA^{II}\text{-}AA^{XII}\text{-}AA^{XII}\text{-}AA^{XIII}**$, $*AA^{III}\text{-}AA^{I}\text{-}AA^{II}\text{-}AA^{II}\text{-}AA^{VII}\text{-}AA^{XII}\text{-}AA^{XII}\text{-}AA^{XIII}**$, $*AA^{III}\text{-}AA^{I}\text{-}AA^{II}\text{-}AA^{IV}\text{-}AA^{IV}\text{-}AA^{XII}\text{-}AA^{XII}\text{-}AA^{XIII}**$, $*AA^{II}\text{-}AA^{I}\text{-}AA^{II}\text{-}AA^{II}\text{-}AA^{II}\text{-}AA^{XII}\text{-}AA^{XII}\text{-}AA^{XIII}**$, $*AA^{III}\text{-}AA^{III}\text{-}AA^{II}\text{-}AA^{VII}\text{-}AA^{II}\text{-}AA^{XII}\text{-}AA^{XII}\text{-}AA^{XIII}**$, $*AA^{III}\text{-}AA^{I}\text{-}AA^{I}\text{-}AA^{II}\text{-}AA^{II}\text{-}AA^{XII}\text{-}AA^{XII}\text{-}AA^{XIII}**$, $*AA^{V}\text{-}AA^{V}\text{-}AA^{VII}\text{-}AA^{VII}\text{-}AA^{XII}\text{-}AA^{XII}\text{-}AA^{XIII}**$, $*AA^{VII}\text{-}AA^{V}\text{-}AA^{VII}\text{-}AA^{II}\text{-}AA^{XII}\text{-}AA^{XII}\text{-}AA^{XIII}**$ and $*AA^{V}\text{-}AA^{V}\text{-}AA^{VII}\text{-}AA^{II}\text{-}AA^{XII}\text{-}AA^{XII}\text{-}AA^{XIII}**$.

In one particular example, LINKER comprises a peptide of formula (XXXV), (XXXV-2) or (XXXV-4).

In one example, LINKER comprises (or is) a poly-(aliphatic amino acid) peptide such as poly-alanine peptide $(A)_n$, or a poly-glycine $(G)_n$, n being an integer comprised between 2 and 16, preferably between 2 to 9, such as A-A-A-A-A-A-A-A-A, A-A-A-A-A-A-A-A, A-A-A-A-A, G-G-G-G-G-G-G-G-G, G-G-G-G-G-G-G or G-G-G-G-G.

In one particular example, LINKER comprises (or is) a peptide of general formulae (XXXV) to (XXXV-14), more particularly (XXXV), (XXXV-2) or (XXXV-4), or a poly-(aliphatic amino acid)$_n$ peptide as defined herein.

For example, in certain embodiments, LINKER is a polysaccharide comprising 2 to 16 saccharides. In one particular example, LINKER is a polysaccharide comprising 2 to 10 saccharides. In one particular example, LINKER is a polysaccharide comprising 2 to 9 saccharides. In one most particular example, LINKER is a polysaccharide comprising 3 to 9 saccharides. Suitable monosaccharides include, but are not limited to, glucose (dextrose), fructose (levulose) and galactose. Monosaccharides are the building blocks of disaccharides (such as sucrose) and polysaccharides (such as celluloses, chitosans, ulvanes and starches). Further, each carbon atom that supports a hydroxyl group (except for the first and last) is chiral, giving rise to a number of isomeric forms all with the same chemical formula. A large number of biologically important modified monosaccharides exists e.g. amino sugars such as Galactosamine, Glucosamine, Sialic acid, N-Acetylglucosamine, and sulfosugars such as Sulfoquinovose. All of these monosaccharide and polysaccharide derivatives may be used as LINKER in the present invention.

Thus, in one particular example, said cyclic GFR-binding compound is a hydride peptide/polysaccharide peptidomimetic of between 1,500 and 7,000 or between 1,500 and 5,000 Da and comprising compound (Ia) as defined herein; wherein one end of LINKER interacts covalently with one end of PEP(A); wherein another end of LINKER interacts covalently with one end of PEP(B); wherein PEP(A) comprises PEP1 or PEP12; wherein PEP(B) comprises PEP2; and wherein LINKER is a polysaccharide comprising 2 to 16 saccharides (in particular 2 to 10, 2 to 9, or 3 to 9 saccharides).

Thus, in one particular example, said cyclic GFR-binding compound is a hydride peptide/polysaccharide peptidomimetic of between 1,500 and 7,000 or between 1,500 and 5,000 Da and comprising compound (Ib) as defined herein; wherein one end of a first LINKER interacts covalently with one end of PEP(A); wherein another end of a first LINKER interacts covalently with one end of PEP(B); wherein one end of a second LINKER interacts covalently with another end of PEP(B); wherein another end of a second LINKER interacts covalently with another end of PEP(A); wherein PEP(A) comprises PEP1; wherein PEP(B) comprises PEP2; and wherein LINKER are independently a polysaccharide comprising 2 to 16 saccharides (in particular 2 to 10, 2 to 9, or 3 to 9 saccharides).

Thus, in one particular example, said cyclic GFR-binding compound is a hydride peptide/polysaccharide peptidomimetic of between 1,500 and 7,000 or between 1,500 and 5,000 Da and comprising compound (IIa) as defined herein; wherein LINKER is a polysaccharide comprising 2 to 16 saccharides (in particular 2 to 10, 2 to 9, or 3 to 9 saccharides); wherein PEP12 is a peptide with 8 amino acids of formula PEP1-$AA^{17}$-PEP11 as already defined herein; wherein PEP1, PEP2, PEP11, PEP(C) and PEP(D) are as already defined herein; wherein $AA^{13}$ and $AA^{21}$ may be both N-terminal amino acids or both C-terminal amino acids; wherein $AA^{25}$ and $AA^{20}$ may be both N-terminal amino acids or both C-terminal amino acids; wherein one end of LINKER interacts covalently with one end of PEP12 via $AA^{20}$; wherein another end of LINKER interacts covalently with PEP2 via $AA^{21}$; wherein one end of PEP(C) interacts covalently with PEP12 via $AA^{13}$; and wherein one end of PEP(D) interacts covalently with PEP2 via $AA^{25}$.

Thus, in one particular example, said cyclic GFR-binding compound is a hydride peptide/polysaccharide peptidomimetic of between 1,500 and 7,000 or between 1,500 and 5,000 Da and comprising compound (IIb) as defined herein; wherein PEP12 is a peptide with 8 amino acids of formula PEP1-$AA^{17}$-PEP11 as defined herein; wherein PEP2 is a peptide with five amino acids as already defined herein; wherein one end of PEP(C) interacts covalently with PEP12 via one end of PEP1; wherein one end of PEP(D) interacts covalently with one end of PEP2; wherein one end of a first LINKER interacts covalently with one end of PEP12 via one end of PEP11; wherein another end of a first LINKER interacts covalently with another end of PEP2; wherein one end of a second LINKER interacts covalently with another end of PEP(D); wherein another end of a second LINKER interacts covalently with another end of PEP(C); wherein PEP(C) is a peptide with at least 5 amino acids, in particular a peptide with between 5 and 12 amino acids; wherein PEP(D) is a peptide with at least 5 amino acids, in particular a peptide with between 5 and 11 amino acids; and wherein LINKER are independently a polysaccharide comprising 2 to 16 saccharides (in particular 2 to 10, 2 to 9, or 3 to 9 saccharides).

For example, in certain embodiments, LINKER is a polynucleotide comprising 2 to 16 nucleotides. In one particular example, LINKER is a polynucleotide comprising 2 to 10 nucleotides. In one particular example, LINKER is a polynucleotide comprising 2 to 9 nucleotides. In one most particular example, LINKER is a polynucleotide comprising 3 to 9 nucleotides. Suitable nucleotides include adenine (A), guanine (G), thymine (T), cytosine (C), uracil (U) and derivatives, analogues and/or mimetic thereof.

Thus, in one particular example, said cyclic GFR-binding compound is a hydride peptide/ polynucleotide peptidomimetic of between 1,500 and 7,000 or between 1,500 and 5,000 Da and comprising compound (Ia) as defined herein; wherein one end of LINKER interacts covalently with one end of PEP(A); wherein another end of LINKER interacts covalently with one end of PEP(B); wherein PEP(A) comprises PEP1 or PEP12; and wherein PEP(B) comprises PEP2; wherein LINKER is a polynucleotide comprising 2 to 16 nucleotides (in particular 2 to 10, 2 to 9, or 3 to 9 nucleotides).

Thus, in one particular example, said cyclic GFR-binding compound is a hydride peptide/ polynucleotide peptidomimetic of between 1,500 and 7,000 or between 1,500 and 5,000 Da and comprising compound (IIa) as defined

herein; wherein LINKER is a polynucleotide comprising 2 to 16 nucleotides (in particular 2 to 10, 2 to 9, or 3 to 9 nucleotides); wherein PEP12 is a peptide with 8 amino acids of formula PEP1-$AA^{17}$-PEP11 as already defined herein; wherein PEP1, PEP2, PEP11, PEP(C) and PEP(D) are as already defined herein; wherein $AA^{13}$ and $AA^{21}$ may be both N-terminal amino acids or both C-terminal amino acids; wherein $AA^{25}$ and $AA^{20}$ may be both N-terminal amino acids or both C-terminal amino acids; wherein one end of LINKER interacts covalently with one end of PEP12 via $AA^{20}$; wherein another end of LINKER interacts covalently with PEP2 via $AA^{21}$; wherein one end of PEP(C) interacts covalently with PEP12 via $AA^{13}$; and wherein one end of PEP(D) interacts covalently with PEP2 via $AA^{25}$.

For example, in certain embodiments, LINKER is a saturated or unsaturated hydrocarbon chain comprising between 1 and 22, between 1 and 16, between 1 and 12, between 1 and 8 or between 1 and 6 carbon atoms, wherein said hydrocarbon chain is optionally interrupted by one or more non-carbon atom, preferably between 1 and 16, between 1 and 12 or between 1 and 8 non-carbon atoms as appropriate, wherein said non-carbon atom is selected from the group consisting of -O-, -S-, -C(=O), - $SO_2$-, -N(Ri)(C=O)-, and -N(Ri)-, wherein Ri is selected from the group consisting of a hydrogen atom, a C1-C6 alkyl group and an aryl group, and wherein said hydrocarbon chain is non-substituted or substituted by at least one radical selected from the group consisting of a halogen, a monosaccharide, a poly(1-6)saccharide, a nucleotide, a poly(1-6)nucleotide, a C1-C10 alkyl group and an aryl group.

Thus, in one particular example, said cyclic GFR-binding compound is a hydride peptide/ hydrocarbon chain peptidomimetic of between 1,500 and 7,000 or between 1,500 and 5,000 Da and comprising compound (Ia) as defined herein; wherein one end of LINKER interacts covalently with one end of PEP(A); wherein another end of LINKER interacts covalently with one end of PEP(B); wherein PEP(A) comprises PEP1 or PEP12; wherein PEP(B) comprises PEP2; and wherein LINKER is a saturated or unsaturated hydrocarbon chain comprising between 1 and 22 as defined herein.

Thus, in one particular example, said cyclic GFR-binding compound is a hydride peptide/ hydrocarbon chain peptidomimetic of between 1,500 and 7,000 or between 1,500 and 5,000 Da and comprising compound (IIa) as defined herein; wherein LINKER is a saturated or unsaturated hydrocarbon chain comprising between 1 and 22 as defined herein; wherein PEP12 is a peptide with 8 amino acids of formula PEPL-$AA^{17}$-PEP11 as already defined herein; wherein PEP1, PEP2, PEP11, PEP(C) and PEP(D) are as already defined herein; wherein $AA^{13}$ and $AA^{21}$ may be both N-terminal amino acids or both C-terminal amino acids; wherein $AA^{25}$ and $AA^{20}$ may be both N-terminal amino acids or both C-terminal amino acids; wherein one end of LINKER interacts covalently with one end of PEP12 via $AA^{20}$; wherein another end of LINKER interacts covalently with PEP2 via $AA^{21}$; wherein one end of PEP(C) interacts covalently with PEP12 via $AA^{13}$; and wherein one end of PEP(D) interacts covalently with PEP2 via $AA^{25}$.

In one example, LINKER is a saturated or unsaturated hydrocarbon chain of at most 10 nanometres (nm) in length, preferably at most 8 nm, 6nm, 4 nm or 2 nm as determined using the standard « 2D » procedure described above.

In one example, such saturated or unsaturated hydrocarbon chains include polyethylene glycol (PEG) or any one of its derivatives.

More particularly, LINKER is a tripeptide (three amino acids). More particularly, LINKER is a tetrapeptide (four amino acids). More particularly, LINKER is a pentapeptide (five amino acids). More particularly, LINKER is a hexapeptide (six amino acids). More particularly, LINKER is a heptapeptide (seven amino acids). More particularly, LINKER is an octapeptide (eight amino acids). More particularly, LINKER is a nonapeptide (nine amino acids).

In one particular example, LINKER is a peptide selected from the group consisting of DENEKVV, DENKNVV, DEYDKVV, DDSSNVI, DSSNNVI, DDMGVPT, DKGVVTY, NDKQQII, DAANNVV, DSANNVV, DDSSNVI, DNGRVLL, VGRKPKV, IGKTPKI, VGRTPKV, RIKPHQGQH, EYVRKKPKL, EIVRKKPIF, EYVRKKP, EIVRKKP, polyalanine ($A_{1-9}$) (preferably $A_{2-5}$) and polyglycine ($G_{1-9}$) (preferably $G_{2-5}$).

For example, in certain embodiments, to synthetise cyclic GFR-binding compounds of the present disclosure, the covalent bonds between e.g. LINKER, PEP(A) to (D) or PEP1 to PEP12, may be created through the chemical reaction between a free amine moiety e.g. of a N-terminal amino acid (-$NH_2$ or - $NH_3X$, X generally being a halide anion selected from the group consisting of $F^-$, $Cl^-$ and $Br^-$), typically acting as a nucleophile, and an electrophile moiety of e.g. a C-terminal amino acid. Such an electrophile moiety includes, but is not limited to, alkyl halides (-$CR_2$-X), alcohols (-$CR_2$-OH), acid chlorides (-C(=O)X), esters, (-C(=O)OR), phosphate (-$OP(OR)_3$), phosphinate (-$OP(OR)R_2$), phosphonates (-$OP(OR)_2R$), phosphonite (-$P(OR)_2R$) or sulfonic esters (-$SO_2OR$). More particularly, this covalent bond is an amide bond (in particular a peptide bond) formed through conventional peptide synthesis using conventional coupling reagents as already defined herein.

For example, in certain embodiments, to synthetise cyclic GFR-binding compounds of the present disclosure, the covalent bonds between e.g. LINKER, PEP(A) to (D) or PEP1 to PEP12, may be created through the chemical reaction between a free carboxylic acid moiety e.g. of a C-terminal amino acid (-$CO_2H$ or -$CO_2X$, X generally being an inorganic cation such as alkaline cations (e.g. $Li^+$, $Na^+$ or $K^+$) or an organic cation such as ammonium cations), typically acting as an electrophile, and a nucleophile moiety of e.g. an N-terminal amino acid. Such a nucleophile moiety includes, but is not limited to, alcohols (-OH), amines (-$NH_2$), phosphines (-$PR_3$), thiols (-SH). More particularly, this covalent interaction is a peptide bond formed through conventional peptide synthesis using conventional coupling reagents as already defined herein.

Cyclisation of a cyclic GFR-binding compound of the present disclosure may be carried out as described above using conventional peptide bond formation procedures, click chemistry, formation of disulphide bonds, etc.

The present disclosure provides pharmaceutical associations, combinations and compositions, methods and uses for converting or recoding any neoplastic cell into a non-neoplastic cell (in particular, a functional and healthy cell) of any type. In some cases, the cell type of the converted or recoded non-neoplastic cell may be selected/chosen by *e.g.* a person providing the treatment to a subject.

In one example, said pharmaceutical association, combination or composition induces the conversion of a neoplastic cell into a physiologically functional and/or healthy cell of any cell lineage including, but not limited to, bone cell, chondrocytic cell, neuron cell, fibroblast, vascular cell, ligament cell, tendon cell, epithelial cell, retina photoreceptor cell, muscle cell, glandular cell, myoepithelial cell, subepithelial interstitial cell, smooth muscle cell, blood cell, gastrointestinal cell, adipocyte, Sertoli cells, Leydig cell, Germ cell and renal cell lineages. Such cells include any progenitor or precursor cells or any partially or fully differentiated cells of these lineages. More generally, the present disclosure includes the treatment of any neoplastic cell of any type and cell lineage using the associations, combinations, compositions, methods and uses as defined herein.

For example, in certain embodiments, a neoplastic cell of a bone tissue (i.e. a neoplastic cell from the bone cell lineage) has been, may be or will be the cause of the development of a neoplastic bone disease (e.g. bone cancer). Inducing the conversion of this neoplastic bone cell into a physiologically functional and/or healthy cell (any physiologically functional and/or healthy cell) of the bone cell lineage may protect a subject/patient carrying this cell from bone cancer. In some cases, it may be preferable and/or satisfying and/or more practical to induce the conversion of a neoplastic bone cell into a functional and/or healthy cell of a different cell lineage i.e. not a cell from a bone lineage, such as, for example, in certain embodiments, a cell from a chondrocytic cell lineage, a fibroblast lineage or a ligament/tendon cell lineage.

In one example, a neoplastic cell of a soft tissue such as a muscle, vascular, skin or kidney tissue (i.e. a neoplastic cell from the muscle, vascular, skin or kidney cell lineage, respectively) has been, may be or will be the cause of the development of a neoplastic muscle, vascular, skin or kidney disease (e.g. Rhabdomyosarcoma, Hemangiosarcoma, basal cell carcinoma or Squamous cell carcinoma, respectively). Inducing the conversion of any of these neoplastic cells into a physiologically functional and/or healthy cell (any physiologically functional and/or healthy cell) of the muscle, vascular, skin or kidney cell lineage, respectively, may protect a subject carrying any of these cells from the diseases mentioned above. In some cases, it may be preferable and/or satisfying and/or more practical to induce the conversion of, e.g. a neoplastic muscle, vascular, skin or kidney cell into a functional and/or healthy cell of a different cell lineage i.e. not a cell from a muscle, vascular, skin or kidney lineage, such as, for example, in certain embodiments, a cell from a bone cell lineage so that a medical practitioner such as a surgeon, may be able to surgically remove the newly converted bone cells more conveniently. The surgery may be carried out purely for aesthetic reasons and/or because of a discomfort and/or to avoid the possibility of e.g. infections, complications, etc.

For example, in certain embodiments, a neoplastic cell of an adipose tissue (i.e. a neoplastic cell from the adipocyte lineage) has been, may be or will be the cause of the development of a neoplastic adipose tissue disease (e.g. adipose tissue cancer or lipoma). Several treatment routes may be envisaged to induce the conversion of this neoplastic adipocyte into a physiologically functional and/or healthy cell and thus protect a subject/patient carrying this cell from a lipoma. In some cases, it may be preferable and/or satisfying and/or more practical to induce the conversion of the neoplastic adipocyte into a functional and/or healthy cell of a bone cell lineage. Once the treatment has been completed i.e. after the conversion of the neoplastic adipocyte into a non-neoplastic bone cell (e.g. an osteocyte) has taken place, surgical removal of the newly converted bone cells may be performed for e.g. aesthetic reasons and/or because of a discomfort and/or to avoid the possibility of e.g. infections, complications, etc. In other cases, it may be preferable and/or satisfying and/or more practical to induce the conversion of the neoplastic adipocyte into a functional and/or healthy cell of a muscle cell lineage. Because the conversion of the neoplastic adipocyte into a non-neoplastic muscle cell (e.g. a myocyte) does not yield hard tissues such as osteocytes, the patient would generally not feel any substantial discomfort and therefore, the surgical removal of the newly converted muscle cells may be avoided, although it may still be performed if the patient were to feel even the slight discomfort. The necessity of performing surgery following the presently defined recoding treatment would generally be left to the appreciation of the patient upon supervision of a medical practitioner.

The present disclosure thus provides methods to convert or recode a neoplastic cell of a specific tissue/cell type (e.g. bone, cartilage, neuron, prostate, ovary, muscle, skin, vascular, ligament, tendon, eye retina, kidney, head, neck, blood, gastrointestinal, lung and adipose tissues) into a non-neoplastic cell of any tissue/cell type (e.g. bone, cartilage, neuron, prostate, ovary, muscle, skin, vascular, ligament, tendon, eye retina and kidney).

11.3. Induction into a cell of a specific cell lineage

*Bone cell lineage*

Certain embodiments of the invention are particularly useful for inducing the conversion of a neoplastic cell into a cell (any cell) of a bone cell lineage.

In certain embodiments useful for inducing the conversion of a neoplastic cell into a cell (any cell) of a bone cell lineage, PEP1 is selected from the group consisting of SAIS, NAIS, SATS and SPIS.

In certain embodiments useful for inducing the conversion of a neoplastic cell into a cell (any cell) of a bone cell lineage, PEP2 is selected from the group consisting of LKNYQ, LKKYR, LRKHR and LKYHY.

In certain embodiments useful for inducing the conversion of a neoplastic cell into a cell (any cell) of a bone cell lineage, PEP3 is selected from the group consisting of VPT, APT, VPQ, VSQ and TQV.

In certain embodiments useful for inducing the conversion of a neoplastic cell into a cell (any cell) of a bone cell lineage, PEP4 is selected from the group consisting of VVE, VVR, VVK, VAE, VRS, VKS and EDH.

In certain embodiments useful for inducing the conversion of a neoplastic cell into a cell (any cell) of a bone cell lineage, PEP5 is a peptide of general formula PEP3-$AA^{11}$-$AA^{12}$; wherein PEP3 is selected from the group consisting of VPT, APT, VPQ, VSQ and TQV; wherein $AA^{11}$ is selected from the group consisting of E, K, Q, R, A, D, G and H, in particular E, K, Q, A and D; wherein $AA^{12}$ is selected from the group consisting of L, M, T, E, Q and H, in particular L. In one particular example, PEP5 is selected from the group consisting of VPTEL, APTKL, APTQL, VPTKL, VPQAL, VSQDL, VPQDL and TQVQL.

In certain embodiments useful for inducing the conversion of a neoplastic cell into a cell (any cell) of a bone cell lineage, PEP6 is a peptide of general formula $AA^{26}$-$AA^{27}$-$AA^{28}$-$AA^{29}$-PEP4; wherein PEP4 is selected from the group consisting of VVE, VVR, VVK, VAE, VRS, VKS and EDH; wherein $AA^{26}$ is absent or selected from the group consisting of $AA^{III}$ amino acids, preferably is absent or E; wherein $AA^{27}$ and $AA^{28}$ are independently selected from the group consisting of $AA^{III}$ and $AA^{V}$ amino acids, preferably $AA^{27}$ is selected from the group consisting of D, E, N, G, M and T, preferably $AA^{28}$ is selected from the group consisting of M, I, V and L; wherein $AA^{29}$ is absent or selected from the group consisting of $AA^{II}$ amino acids, preferably absent or S. In one particular example, PEP6 is selected from the group consisting of DMVVE, EMVVE, NMVVR, NMVVK, EGMSVAE, MIVRS, MIVKS, MVVKS and TLEDH.

In certain embodiments useful for inducing the conversion of a neoplastic cell into a cell (any cell) of a bone cell lineage, PEP7 is an amino acid or a peptide with between two and seven amino acids of general formula $AA^1$-$AA^2$-$AA^3$-$AA^4$-$AA^5$-$AA^6$-$AA^7$; wherein $AA^1$, $AA^2$, $AA^3$, $AA^4$, and $AA^5$ are independently absent or $AA^I$ as defined herein; wherein $AA^6$ is absent or selected from the group consisting of S, T, C, E, Q, P and R, preferably C, S, T or R; wherein $AA^7$ is absent or is selected from the group consisting of S, T, C, E, Q, P and R, preferably is selected from the group consisting of C, S and P; and wherein at least one of $AA^1$, $AA^2$, $AA^3$, $AA^4$, $AA^5$, $AA^6$ or $AA^7$ is not absent. In one particular example, PEP7 is selected from the group consisting of KIPKAXX, SIPKAXX, HVTKPTX, YVPKPXX, TVPKPXX, AVPKAXX, KVGKAXX, ASAAPXX, ASASPXX and RNVQXRP.

In certain embodiments useful for inducing the conversion of a neoplastic cell into a cell (any cell) of a bone cell lineage, PEP8 is an amino acid or a peptide with between two and six amino acids of general formula $AA^{33}$-$AA^{34}$-$AA^{35}$-$AA^{36}$-$AA^{37}$-$AA^{38}$; wherein $AA^{33}$ is absent or is selected from the group consisting of $AA^I$ amino acids at the exception of $AA^{VI}$ amino acids; $AA^{34}$ is absent or is selected from the group consisting of $AA^{III}$ and $AA^{IV}$ amino acids; wherein $AA^{35}$ is absent or is selected from the group consisting of $AA^{II}$ amino acids, preferably is S or C; wherein $AA^{36}$ is absent or is selected from the group consisting of $AA^{III}$ and $AA^{IV}$ amino acids; wherein $AA^{37}$ is absent or is selected from the group consisting of $AA^{II}$ amino acids, preferably is S or C; wherein $AA^{38}$ is absent or is selected from the group consisting of $AA^I$; and wherein at least one of $AA^{33}$, $AA^{34}$, $AA^{35}$, $AA^{36}$, $AA^{37}$ or $AA^{38}$ is not absent. In one particular example, PEP8 is selected from the group consisting of GXGXR, SXGXH, AXGXH, XGXR, XKXS and LAXKXE.

In certain embodiments useful for inducing the conversion of a neoplastic cell into a cell (any cell) of a bone cell lineage, PEP9 is a peptide of general formula $AA^1$-$AA^2$-$AA^3$-$AA^4$-$AA^5$-$AA^6$-$AA^7$-PEP5; wherein PEP5 is a peptide of formula PEP3-$AA^{11}$-$AA^{12}$; wherein PEP3 is selected from the group consisting of VPT, APT, VPQ, VSQ and TQV; wherein $AA^{11}$ is selected from the group consisting of E, K, Q, R, A, D, G and H, in particular E, K, Q, A and D; wherein $AA^{12}$ is selected from the group consisting of L, M, T, E, Q and H, in particular L; wherein $AA^1$, $AA^2$, $AA^3$, $AA^4$, and $AA^5$ are independently absent or $AA^I$ as defined herein; wherein $AA^6$ is absent or selected from the group consisting of S, T, C, E, Q, P and R, preferably C, S, T or R; wherein $AA^7$ is selected from the group consisting of S, T, C, E, Q, P and R, preferably is selected from the group consisting of C, S and P. In one particular example, PEP9 is selected from the group consisting of KIPKAXXVPTEL, SIPKAXXVPTEL, HVTKPTXAPTKL, YVPKPXXAPTKL, TVPKPXXAPTQL, AVPKAXXAPTKL, KVGKAXXVPTKL, ASAAPXXVPQAL, ASASPXXVSQDL, ASASPXXVPQDL and RNVQXRPTQVQL.

In certain embodiments useful for inducing the conversion of a neoplastic cell into a cell (any cell) of a bone cell lineage, PEP10 is a peptide of general formula PEP6-$AA^{33}$-$AA^{34}$-$AA^{35}$-$AA^{36}$-$AA^{37}$-$AA^{38}$; wherein PEP6 is a peptide of formula $AA^{26}$-$AA^{27}$-$AA^{28}$-$AA^{29}$-PEP4; wherein PEP4 is selected from the group consisting of VVE, VVR, VVK, VAE,

VRS, VKS and EDH; wherein $AA^{26}$ is absent or selected from the group consisting of $AA^{III}$ amino acids, preferably E; wherein $AA^{27}$ and $AA^{28}$ are independently selected from the group consisting of $AA^{III}$ and $AA^{V}$ amino acids, preferably $AA^{27}$ is selected from the group consisting of D, E, N, G, M and T, preferably $AA^{28}$ is selected from the group consisting of M, I, V and L; wherein $AA^{29}$ is absent or selected from the group consisting of $AA^{II}$ amino acids, preferably absent or S; wherein $AA^{33}$ is absent or is selected from the group consisting of $AA^{I}$ amino acids at the exception of $AA^{VI}$ amino acids; $AA^{34}$ is absent or is selected from the group consisting of $AA^{III}$ and $AA^{IV}$ amino acids; wherein $AA^{35}$ is absent or is selected from the group consisting of $AA^{II}$ amino acids, preferably is S or C; wherein $AA^{36}$ is absent or is selected from the group consisting of $AA^{III}$ and $AA^{IV}$ amino acids; wherein $AA^{37}$ is absent or is selected from the group consisting of $AA^{II}$ amino acids, preferably is S or C; wherein $AA^{38}$ is absent or is selected from the group consisting of $AA^{I}$. In one particular example, PEP10 is selected from the group consisting of DMVVEGXGXR, EMVVEGXGXR, NMVVRSXGXH, NMVVRAXGXH, NMVVKAXGXH, EGMSVAEXGXR, MIVRSXKXS, MIVKSXKXS, MVVKSXKXS and TLEDHLAXKXE.

In certain embodiments useful for inducing the conversion of a neoplastic cell into a cell (any cell) of a bone cell lineage, PEP12 is a peptide of general formula PEP1-$AA^{17}$-PEP11; wherein $AA^{17}$ is selected from the group consisting of G, A, V, L, I, P, F, M, W, T and S (in particular is selected from the group consisting of M, I, L, V and T); wherein PEP1 is selected from the group consisting of SAIS, NAIS, SATS and SPIS.

In certain embodiments useful for inducing the conversion of a neoplastic cell into a cell (any cell) of a bone cell lineage, PEP11 is a peptide with 3 amino acids of general formula $AA^{18}$-$AA^{19}$-$AA^{20}$; wherein $AA^{18}$ is selected from the group consisting of L, V, Q, A and R, in particular is L; wherein $AA^{19}$ is selected from the group consisting of F, W, H and Y (in particular is an aromatic, polar amino acid such as Y); wherein $AA^{20}$ is selected from the group consisting of L, F, Y, K, I, V and M, in particular is selected from the group consisting of L, F, Y, and K. In one particular example, PEP11 is selected from the group consisting of LYL, LYF, LYY and LYK.

The definitions of "PEP" pairs, quadruplets, hexaplets and heptuplet e.g. PEP1:PEP2, PEP12:PEP2, or PEP7:PEP5:PEP12:LINKER:PEP2:PEP6:PEP8, most particularly useful for inducing the conversion of a neoplastic cell into a cell (any cell) of a bone cell lineage, are as already defined herein to the extent that PEP1 to PEP12 are particularly useful for these applications as defined in the present bone section. For instances:

In certain embodiments, useful for inducing the conversion of a neoplastic cell into a cell (any cell) of a bone cell lineage, the pair PEP1:PEP2 is selected from the group consisting of SAIS:LKNYQ, NAIS:LKKYR, SATS:LRKHR and SPIS:LKYHY.

In certain embodiments, useful for inducing the conversion of a neoplastic cell into a cell (any cell) of a bone cell lineage, the pair PEP12:PEP2 is selected from the group consisting of SAIS-$AA^{17}$-LYL:LKNYQ, NAIS-$AA^{17}$-LYF:LKKYR, SATS-$AA^{17}$-LYY:LRKHR and SPIS-$AA^{17}$-LYK:LKYHY; wherein $AA^{17}$ is selected from the group consisting of G, A, V, L, I, P, F, M, W, T and S (in particular is selected from the group consisting of M, V and T).

In certain embodiments useful for inducing the conversion of a neoplastic cell into a cell (any cell) of a bone cell lineage, PEP3 is selected from the group consisting of VPT, APT, VPQ, VSQ and TQV; PEP4 is selected from the group consisting of VVE, VVR, VVK, VAE, VRS, VKS and EDH; and wherein the pair PEP3:PEP4 is selected from the group consisting of VPT:VVE, APT:VVR, APT:VVK, VPT:VAE, VPQ:VRS, VSQ:VKS, VPQ:VKS and TQV:EDH.

In certain embodiments useful for inducing the conversion of a neoplastic cell into a cell (any cell) of a bone cell lineage, PEP5 is selected from the group consisting of VPTEL, APTKL, APTQL, VPTKL, VPQAL, VSQDL, VPQDL and TQVQL; PEP6 is selected from the group consisting of DMVVE, EMVVE, NMVVR, NMVVK, EGMSVAE, MIVRS, MIVKS, MVVKS and TLEDH; and wherein the pair PEP5:PEP6 is selected from the group consisting of VPTEL:DMVVE, VPTEL:EMVVE, APTKL:NMVVR, APTQL:NMVVR, APTKL:NMVVK, VPTKL:EGMSVAE, VPQAL:MIVRS, VSQDL:MIVKS, VPQDL:MVVKS and TQVQL:TLEDH.

In certain embodiments useful for inducing the conversion of a neoplastic cell into a cell (any cell) of a bone cell lineage, PEP7 is selected from the group consisting of KIPKAXX, SIPKAXX, HVTKPTX, YVPKPXX, TVPKPXX, AVPKAXX, KVGKAXX, ASAAPXX, ASASPXX and RNVQXRP; wherein PEP8 is selected from the group consisting of GXGXR, SXGXH, AXGXH, XGXR, XKXS and LAXKXE; and wherein the pair PEP7:PEP8 is selected from the group consisting of KIPKAXX:GXGXR, SIPKAXX:GXGXR, HVTKPTX:SXGXH, YVPKPXX:SXGXH, KVGKAXX:XGXR, ASAAPXX:XKXS, ASASPXX:XKXS and RNVQXRP:LAXKXE.

In certain embodiments useful for inducing the conversion of a neoplastic cell into a cell (any cell) of a bone cell lineage, PEP9 is selected from the group consisting of KIPKAXXVPTEL, SIPKAXXVPTEL, HVTKPTXAPTKL, YVPKPXXAPTKL, TVPKPXXAPTQL, AVPKAXXAPTKL, KVGKAXXVPTKL, ASAAPXXVPQAL, ASASPXXVSQDL, ASASPXXVPQDL and RNVQXRPTQVQL; PEP10 is selected from the group consisting of DMVVEGXGXR, EMVVEGXGXR, NMVVRSXGXH, NMVVRAXGXH, NMVVKAXGXH, EGMSVAEXGXR, MIVRSXKXS, MIVKSXKXS, MVVKSXKXS and TLEDHLAXKXE; and wherein the pair PEP9:PEP10 is selected from the group consisting of KIPKAXXVPTEL:DMVVEGXGXR, SIPKAXXVPTEL:EMVVEGXGXR, HVTKPTXAPTKL:NMVVRSXGXH, YVPKPXXAPTKL:NMVVRSXGXH, KVGKAXXVPTKL:EGMSVAEXGXR, ASAAPXXVPQAL:MIVRSXKXS, ASASPXXVSQDL:MIVKSXKXS, ASASPXXVPQDL:MVVKSXKXS and RNVQXRPTQVQL:TLEDHLAXKXE.

In certain embodiments useful for inducing the conversion of a neoplastic cell into a cell (any cell) of a bone

cell lineage, PEP5 is selected from the group consisting of VPTEL, APTKL, APTQL, VPTKL, VPQAL, VSQDL, VPQDL and TQVQL; PEP6 is selected from the group consisting of DMVVE, EMVVE, NMVVR, NMVVK, EGMSVAE, MIVRS, MIVKS, MVVKS and TLEDH; and wherein the pair PEP5:PEP6 is selected from the group consisting of VPTEL:DMVVE, VPTEL:EMVVE, APTKL:NMVVR, APTQL:NMVVR, APTKL:NMVVK, VPTKL:EGMSVAE, VPQAL:MIVRS, VSQDL:MIVKS, VPQDL:MVVKS and TQVQL:TLEDH; and wherein the pair PEP5:PEP6 is not VPTEL:DMVVE when PEP1 is SAIS and PEP2 is LKNYQ; wherein the pair PEP5:PEP6 is not VPTEL:EMVVE when PEP1 is SAIS and PEP2 is LKNYQ; wherein the pair PEP5:PEP6 is not APTKL:NMVVR when PEP1 is NAIS and PEP2 is LKKYR; wherein the pair PEP5:PEP6 is not APTQL:NMVVR when PEP1 is NAIS and PEP2 is LKKYR; wherein the pair PEP5:PEP6 is not APTKL:NMVVK when PEP1 is SATS and PEP2 is LRKHR; wherein the pair PEP5:PEP6 is not VPTKL:EGMSVAE when PEP1 is SPIS and PEP2 is LKYHY.

In certain embodiments useful for inducing the conversion of a neoplastic cell into a cell (any cell) of a bone cell lineage, PEP9 is selected from the group consisting of KIPKAXXVPTEL, SIPKAXXVPTEL, HVTKPTXAPTKL, YVPKPXXAPTKL, TVPKPXXAPTQL, AVPKAXXAPTKL, KVGKAXXVPTKL, ASAAPXXVPQAL, ASASPXXVSQDL, ASASPXXVPQDL and RNVQXRPTQVQL; PEP10 is selected from the group consisting of DMVVEGXGXR, EMVVEGXGXR, NMVVRSXGXH, NMVVRAXGXH, NMVVKAXGXH, EGMSVAEXGXR, MIVRSXKXS, MIVKSXKXS, MVVKSXKXS and TLEDHLAXKXE; and wherein the pair PEP9:PEP10 is selected from the group consisting of KIPKAXXVPTEL:DMVVEGXGXR, SIPKAXXVPTEL:EMVVEGXGXR, HVTKPTXAPTKL:NMVVRSXGXH, YVPKPXXAPTKL:NMVVRSXGXH, KVGKAXXVPTKL:EGMSVAEXGXR, ASAAPXXVPQAL:MIVRSXKXS, ASASPXXVSQDL:MIVKSXKXS, ASASPXXVPQDL:MVVKSXKXS and RNVQXRPTQVQL:TLEDHLAXKXE; and wherein PEP9:PEP10 is not KIPKAXXVPTEL:DMVVEGXGXR when PEP1 is SAIS and PEP2 is LKNYQ ; wherein PEP9:PEP10 is not SIPKAXXVPTEL:EMVVEGXGXR when PEP1 is SAIS and PEP2 is LKNYQ; wherein PEP9:PEP10 is not HVTKPTXAPTKL:NMVVRSXGXH when PEP1 is NAIS and PEP2 is LKKYR; wherein PEP9:PEP10 is not YVPKPXXAPTKL:NMVVRSXGXH when PEP1 is NAIS and PEP2 is LKKYR; wherein PEP9:PEP10 is not TVPKPXXAPTQL:NMVVRAXGXH when PEP1 is NAIS and PEP2 is LKKYR; wherein PEP9:PEP10 is not AVPKAXXAPTKL:NMVVKAXGXH when PEP1 is SATS and PEP2 is LRKHR; wherein PEP9:PEP10 is not KVGKAXXVPTKL:EGMSVAEXGXR when PEP1 is SPIS and PEP2 is LKYHY.

In certain embodiments useful for inducing the conversion of a neoplastic cell into a cell (any cell) of a bone cell lineage, PEP1 is selected from the group consisting of SAIS, NAIS, SATS and SPIS; PEP11 is selected from the group consisting of LYL, LFF, LYF, LYY, LYK, LYI, LFI, LYV, VYY, QIM, AKV and RKI; and the pair PEP1:PEP11 is selected from the group consisting of SAIS:LYL, NAIS:LYF, SATS:LYY and SPIS:LYK.

In one aspect, the present disclosure provides a GFR-binding compound, wherein said GFR-binding compound is a peptide, a variant or analog thereof, or a peptidomimetic as defined herein, with between 20 and 60 (in particular between 20 and 50, and more particularly, between 20 and 45) amino acids, comprising a peptide with four amino acids (PEP1) or a peptide with eight amino acids (PEP12), a peptide with five amino acids (PEP2), and optionally a pair of peptides selected form the group consisting of PEP3:PEP4, PEP3:PEP6, PEP3:PEP10, PEP5:PEP4, PEP9:PEP4, PEP5:PEP6, PEP5:PEP10, PEP9:PEP6 and PEP9:PEP10; wherein said GFR-binding compound does not comprise the pair of peptides VPTEL:DMVVE when PEP1 is SAIS and PEP2 is LKNYQ; wherein said GFR-binding compound does not comprise the pair of peptides VPTEL:EMVVE when PEP1 is SAIS and PEP2 is LKNYQ; wherein said GFR-binding compound does not comprise the pair of peptides APTKL:NMVVR when PEP1 is NAIS and PEP2 is LKKYR; wherein said GFR-binding compound does not comprise the pair of peptides APTQL:NMVVR when PEP1 is NAIS and PEP2 is LKKYR; wherein said GFR-binding compound does not comprise the pair of peptides APTKL:NMVVK when PEP1 is SATS and PEP2 is LRKHR; wherein said GFR-binding compound does not comprise the pair of peptides VPTKL:EGMSVAE when PEP1 is SPIS and PEP2 is LKYHY; and, optionally, wherein the RMSD is 2.45Å or less.

In one aspect, the present disclosure provides a GFR-binding compound, wherein said GFR-binding compound is a peptide, a variant or analog thereof, or a peptidomimetic as defined herein, with between 20 and 60 (in particular between 20 and 50, and more particularly, between 20 and 45) amino acids, comprising a peptide with four amino acids (PEP1) or a peptide with eight amino acids (PEP12), a peptide with five amino acids (PEP2), and optionally a pair of peptides selected form the group consisting of PEP3:PEP4, PEP3:PEP6, PEP3:PEP10, PEP5:PEP4, PEP9:PEP4, PEP5:PEP6, PEP5:PEP10, PEP9:PEP6 and PEP9:PEP10; wherein said GFR-binding compound does not comprise the pair of peptides KIPKAXXVPTEL:DMVVEGXGXR when PEP1 is SAIS and PEP2 is LKNYQ ; wherein said GFR-binding compound does not comprise the pair of peptides SIPKAXXVPTEL:EMVVEGXGXR when PEP1 is SAIS and PEP2 is LKNYQ; wherein said GFR-binding compound does not comprise the pair of peptides HVTKPTXAPTKL:NMVVRSXGXH when PEP1 is NAIS and PEP2 is LKKYR; wherein said GFR-binding compound does not comprise the pair of peptides YVPKPXXAPTKL:NMVVRSXGXH when PEP1 is NAIS and PEP2 is LKKYR; wherein said GFR-binding compound does not comprise the pair of peptides TVPKPXXAPTQL:NMVVRAXGXH when PEP1 is NAIS and PEP2 is LKKYR; wherein said GFR-binding compound does not comprise the pair of peptides AVPKAXXAPTKL:NMVVKAXGXH when PEP1 is SATS and PEP2 is LRKHR; wherein said GFR-binding compound does not comprise the pair of peptides KVGKAXXVPTKL:EGMSVAEXGXR when PEP1 is SPIS and PEP2 is LKYHY; and, optionally, wherein the RMSD is

2.45Å or less.

In certain embodiments useful for inducing the conversion of a neoplastic cell into a cell (any cell) of a bone cell lineage, said GFR-binding compound is a synthetic molecule as defined herein in the definition section.

In certain embodiments useful for inducing the conversion of a neoplastic cell into a cell (any cell) of a bone cell lineage, said GFR-binding compound is a synthetic peptide, or a variant or analog thereof, or a peptidomimetic.

*Chondrocytic cell lineage*

Certain embodiments of the invention are particularly useful for inducing the conversion of a neoplastic cell into a cell (any cell) of a chondrocytic cell lineage.

In certain embodiments useful for inducing the conversion of a neoplastic cell into a cell (any cell) of a chondrocytic cell lineage, PEP1 is selected from the group consisting of SAIS, NAIS, SPIS, EPLP and EPLT.

In certain embodiments useful for inducing the conversion of a neoplastic cell into a cell (any cell) of a chondrocytic cell lineage, PEP2 is selected from the group consisting of LKNYQ, LKKYR, YKQYE and EQLSN.

In certain embodiments useful for inducing the conversion of a neoplastic cell into a cell (any cell) of a chondrocytic cell lineage, PEP3 is selected from the group consisting of VPT, APT, VPQ and VSQ.

In certain embodiments useful for inducing the conversion of a neoplastic cell into a cell (any cell) of a chondrocytic cell lineage, PEP4 is selected from the group consisting of VVE, VVR, VRS and VKS.

In certain embodiments useful for inducing the conversion of a neoplastic cell into a cell (any cell) of a chondrocytic cell lineage, PEP5 is a peptide of general formula PEP3-$AA^{11}$-$AA^{12}$; wherein PEP3 is selected from the group consisting of VPT, APT, VPQ and VSQ; wherein $AA^{11}$ is selected from the group consisting of E, K, Q, R, A, D, G and H, in particular E, K, Q, R, A and D; wherein $AA^{12}$ is selected from the group consisting of L, M, T, E, Q and H, in particular is L. In one particular example, PEP5 is selected from the group consisting of VPTEL, APTKL, APTQL, VPTRL, VPQAL, VSQDL and VPQDL.

In certain embodiments useful for inducing the conversion of a neoplastic cell into a cell (any cell) of a chondrocytic cell lineage, PEP6 is a peptide of general formula $AA^{26}$-$AA^{27}$-$AA^{28}$-$AA^{29}$-PEP4; wherein PEP4 is selected from the group consisting of VVE, VVR, VRS and VKS; wherein $AA^{26}$ is absent or selected from the group consisting of $AA^{III}$ amino acids, preferably is absent; wherein $AA^{27}$ and $AA^{28}$ are independently selected from the group consisting of $AA^{III}$ and $AA^{V}$ amino acids, preferably $AA^{27}$ is selected from the group consisting of D, E, N and M, preferably $AA^{28}$ is selected from the group consisting of M, I and V; wherein $AA^{29}$ is absent or selected from the group consisting of $AA^{II}$ amino acids, preferably absent. In one particular example, PEP6 is selected from the group consisting of DMVVE, EMVVE, NMVVR, DMVVE, MIVRS, MIVKS and MVVKS.

In certain embodiments useful for inducing the conversion of a neoplastic cell into a cell (any cell) of a chondrocytic cell lineage, PEP7 is an amino acid or a peptide with between two and seven amino acids of general formula $AA^1$-$AA^2$-$AA^3$-$AA^4$-$AA^5$-$AA^6$-$AA^7$; wherein $AA^1$, $AA^2$, $AA^3$, $AA^4$, and $AA^5$ are independently absent or $AA^I$ as defined herein; wherein $AA^6$ is absent or selected from the group consisting of S, T, C, E, Q, P and R, preferably is S, C or T; wherein $AA^7$ is absent or is selected from the group consisting of S, T, C, E, Q, P and R, preferably is S or C; and wherein at least one of $AA^1$, $AA^2$, $AA^3$, $AA^4$, $AA^5$, $AA^6$ or $AA^7$ is not absent. In one particular example, PEP7 is selected from the group consisting of KIPKAXX, SIPKAXX, HVTKPTX, YVPKPXX, TVPKPXX, STPPTXX, ASAAPXX and ASASPXX.

In certain embodiments useful for inducing the conversion of a neoplastic cell into a cell (any cell) of a chondrocytic cell lineage, PEP8 is an amino acid or a peptide with between two and six amino acids of general formula $AA^{33}$-$AA^{34}$-$AA^{35}$-$AA^{36}$-$AA^{37}$-$AA^{38}$; wherein $AA^{33}$ is absent or is selected from the group consisting of $AA^I$ amino acids at the exception of $AA^{VI}$ amino acids, preferably absent, G, S or A; $AA^{34}$ is absent or is selected from the group consisting of $AA^{III}$ and $AA^{IV}$ amino acids, preferably absent; wherein $AA^{35}$ is absent or is selected from the group consisting of $AA^{II}$ amino acids, preferably is S or C; wherein $AA^{36}$ is absent or is selected from the group consisting of $AA^{III}$ and $AA^{IV}$ amino acids, preferably is G or K; wherein $AA^{37}$ is absent or is selected from the group consisting of $AA^{II}$ amino acids, preferably is S or C; wherein $AA^{38}$ is absent or is selected from the group consisting of $AA^I$; and wherein at least one of $AA^{33}$, $AA^{34}$, $AA^{35}$, $AA^{36}$, $AA^{37}$ or $AA^{38}$ is not absent. In one particular example, PEP8 is selected from the group consisting of GXGXR, SXGXH, AXGXH, SXGXR and XKXS.

In certain embodiments useful for inducing the conversion of a neoplastic cell into a cell (any cell) of a chondrocytic cell lineage, PEP9 is a peptide of general formula $AA^1$-$AA^2$-$AA^3$-$AA^4$-$AA^5$-$AA^6$-$AA^7$-PEP5; wherein PEP5 is a peptide of formula PEP3-$AA^{11}$-$AA^{12}$; wherein PEP3 is selected from the group consisting of VPT, APT, VPQ and VSQ; wherein $AA^{11}$ is selected from the group consisting of E, K, Q, R, A, D, G and H, in particular E, K, Q, R, A and D; wherein $AA^{12}$ is selected from the group consisting of L, M, T, E, Q and H, in particular is L; wherein $AA^1$, $AA^2$, $AA^3$, $AA^4$, and $AA^5$ are independently absent or $AA^I$ as defined herein; wherein $AA^6$ is absent or selected from the group consisting of S, T, C, E, Q, P and R, preferably is S, C or T; wherein $AA^7$ is selected from the group consisting of S, T, C, E, Q, P and R, preferably is S or C. In one particular example, PEP9 is selected from the group consisting of KIPKAXXVPTEL, SIPKAXXVPTEL, HVTKPTXAPTKL, YVPKPXXAPTKL, TVPKPXXAPTQL, STPPTXXVPTRL, ASAAPXXVPQAL,

ASASPXXVSQDL and ASASPXXVPQDL.

In certain embodiments useful for inducing the conversion of a neoplastic cell into a cell (any cell) of a chondrocytic cell lineage, PEP10 is a peptide of general formula PEP6-$AA^{33}$-$AA^{34}$-$AA^{35}$-$AA^{36}$-$AA^{37}$-$AA^{38}$; wherein PEP6 is a peptide of formula $AA^{26}$-$AA^{27}$-$AA^{28}$-$AA^{29}$-PEP4; wherein PEP4 is selected from the group consisting of VVE, VVR, VRS and VKS; wherein $AA^{26}$ is absent or selected from the group consisting of $AA^{III}$ amino acids, preferably absent; wherein $AA^{27}$ and $AA^{28}$ are independently selected from the group consisting of $AA^{III}$ and $AA^{V}$ amino acids, preferably $AA^{27}$ is selected from the group consisting of D, E, N and M, preferably $AA^{28}$ is selected from the group consisting of M, I and V; wherein $AA^{29}$ is absent or selected from the group consisting of $AA^{II}$ amino acids, preferably absent; wherein $AA^{33}$ is absent or is selected from the group consisting of $AA^{I}$ amino acids at the exception of $AA^{VI}$ amino acids, preferably absent, G, S or A; $AA^{34}$ is absent or is selected from the group consisting of $AA^{III}$ and $AA^{IV}$ amino acids, preferably absent; wherein $AA^{35}$ is absent or is selected from the group consisting of $AA^{II}$ amino acids, preferably is S or C; wherein $AA^{36}$ is absent or is selected from the group consisting of $AA^{III}$ and $AA^{IV}$ amino acids, preferably is G or K; wherein $AA^{37}$ is absent or is selected from the group consisting of $AA^{II}$ amino acids, preferably is S or C; wherein $AA^{38}$ is absent or is selected from the group consisting of $AA^{I}$. In one particular example, PEP10 is selected from the group consisting of DMVVEGXGXR, EMVVEGXGXR, NMVVRSXGXH, NMVVRAXGXH, DMVVESXGXR, MIVRSXKXS, MIVKSXKXS and MVVKSXKXS.

In certain embodiments useful for inducing the conversion of a neoplastic cell into a cell (any cell) of a chondrocytic cell lineage, PEP12 is a peptide of general formula PEPL-$AA^{17}$-PEP11; wherein $AA^{17}$ is selected from the group consisting of G, A, V, L, I, P, F, M, W, T and S (in particular is selected from the group consisting of M, I, L, V and T); wherein PEP1 is selected from the group consisting of SAIS, NAIS, SPIS, EPLP and EPLT.

In certain embodiments useful for inducing the conversion of a neoplastic cell into a cell (any cell) of a chondrocytic cell lineage, PEP11 is a peptide with 3 amino acids of general formula $AA^{18}$-$AA^{19}$-$AA^{20}$; wherein $AA^{18}$ is selected from the group consisting of L, V, Q, A and R, in particular is L or V; wherein $AA^{19}$ is selected from the group consisting of F, W, H and Y, in particular is Y or F; wherein $AA^{20}$ is selected from the group consisting of L, F, Y and I. In one particular example, PEP11 is selected from the group consisting of LYL, LYF, LFI, VYY and LYY.

The definitions of "PEP" pairs, quadruplets, hexaplets and heptuplet e.g. PEP1:PEP2, PEP12:PEP2, or PEP7:PEP5:PEP12:LINKER:PEP2:PEP6:PEP8, most particularly useful for inducing the conversion of a neoplastic cell into a cell (any cell) of a chondrocytic cell lineage, are as already defined herein to the extent that PEP1 to PEP12 are particularly useful for these applications. For instances:

In certain embodiments useful for inducing the conversion of a neoplastic cell into a cell (any cell) of a chondrocytic cell lineage, the pair PEP1:PEP2 is selected from the group consisting of SAIS:LKNYQ, NAIS:LKKYR, SPIS:YKQYE, EPLP:EQLSN and EPLT:EQLSN.

In certain embodiments useful for inducing the conversion of a neoplastic cell into a cell (any cell) of a chondrocytic cell lineage, the pair PEP12:PEP2 is selected from the group consisting of SAIS-$AA^{17}$-LYL:LKNYQ, NAIS-$AA^{17}$-LYF:LKKYR, SPIS-$AA^{17}$-LFI:YKQYE, EPLP-$AA^{17}$-VYY:EQLSN and EPLT-$AA^{17}$-LYY:EQLSN; wherein $AA^{17}$ is selected from the group consisting of G, A, V, L, I, P, F, M, W, T and S (in particular is selected from the group consisting of M, I, V and T).

In certain embodiments useful for inducing the conversion of a neoplastic cell into a cell (any cell) of a chondrocytic cell lineage, PEP3 is selected from the group consisting of VPT, APT, VPQ and VSQ; PEP4 is selected from the group consisting of VVE, VVR, VRS and VKS; and wherein the pair PEP3:PEP4 is selected from the group consisting of VPT:VVE, APT:VVR, VPQ:VRS, VSQ:VKS and VPQ:VKS.

In certain embodiments useful for inducing the conversion of a neoplastic cell into a cell (any cell) of a chondrocytic cell lineage, PEP5 is selected from the group consisting of VPTEL, APTKL, APTQL, VPTRL, VPQAL, VSQDL and VPQDL; PEP6 is selected from the group consisting of DMVVE, EMVVE, NMVVR, DMVVE, MIVRS, MIVKS and MVVKS; and wherein the pair PEP5:PEP6 is selected from the group consisting of VPTEL:DMVVE, VPTEL:EMVVE, APTKL:NMVVR, APTQL:NMVVR, VPTRL:DMVVE, VPQAL:MIVRS, VSQDL:MIVKS and VPQDL:MVVKS.

In certain embodiments useful for inducing the conversion of a neoplastic cell into a cell (any cell) of a chondrocytic cell lineage, PEP7 is selected from the group consisting of KIPKAXX, SIPKAXX, HVTKPTX, YVPKPXX, TVPKPXX, STPPTXX, ASAAPXX and ASASPXX; wherein PEP8 is selected from the group consisting of GXGXR, SXGXH, AXGXH, SXGXR and XKXS; and wherein the pair PEP7:PEP8 is selected from the group consisting of KIPKAXX: GXGXR, SIPKAXX:GXGXR, HVTKPTX:SXGXH, YVPKPXX:SXGXH, TVPKPXX:AXGXH, STPPTXX:SXGSR, ASAAPXX:XKXS and ASASPXX:XKXS.

In certain embodiments useful for inducing the conversion of a neoplastic cell into a cell (any cell) of a chondrocytic cell lineage, PEP9 is selected from the group consisting of KIPKAXXVPTEL, SIPKAXXVPTEL, HVTKPTXAPTKL, YVPKPXXAPTKL, TVPKPXXAPTQL, STPPTXXVPTRL, ASAAPXXVPQAL, ASASPXXVSQDL and ASASPXXVPQDL; PEP10 is selected from the group consisting of DMVVEGXGXR, EMVVEGXGXR, NMVVRSXGXH, NMVVRAXGXH, DMVVESXGXR, MIVRSXKXS, MIVKSXKXS and MVVKSXKXS; and wherein the pair PEP9:PEP10 is selected from the group consisting of KIPKAXXVPTEL:DMVVEGXGXR, SIPKAXXVPTEL:EMVVEGXGXR, HVTKPTXAPTKL:NMVVRSXGXH, YVPKPXXAPTKL:NMVVRSXGXH, TVPKPXXAPTQL:NMVVRAXGXH, STPPTXXVPTRLDMVVESXGSR, ASAAPXXVPQAL:MIVRSXKXS, ASASPXXVSQDL:MIVKSXKXS and ASASPXXVPQDL:MVVKSXKXS.

In certain embodiments useful for inducing the conversion of a neoplastic cell into a cell (any cell) of a chondrocytic cell lineage, PEP5 is selected from the group consisting of VPTEL, APTKL, APTQL, VPTRL, VPQAL, VSQDL and VPQDL; PEP6 is selected from the group consisting of DMVVE, EMVVE, NMVVR, DMVVE, MIVRS, MIVKS and MVVKS; and wherein the pair PEP5:PEP6 is selected from the group consisting of VPTEL:DMVVE, VPTEL:EMVVE, APTKL:NMVVR, APTQL:NMVVR, VPTRL:DMVVE, VPQAL:MIVRS, VSQDL:MIVKS and VPQDL:MVVKS; wherein the pair PEP5:PEP6 is not VPTEL:DMVVE when PEP1 is SAIS and PEP2 is LKNYQ; wherein the pair PEP5:PEP6 is not VPTEL:EMVVE when PEP1 is SAIS and PEP2 is LKNYQ; wherein the pair PEP5:PEP6 is not APTKL:NMVVR when PEP1 is NAIS and PEP2 is LKKYR; wherein the pair PEP5:PEP6 is not APTQL:NMVVR when PEP1 is NAIS and PEP2 is LKKYR; wherein the pair PEP5:PEP6 is not VPTRL:DMVVE when PEP1 is SPIS and PEP2 is YKQYE; wherein the pair PEP5:PEP6 is not VPQAL:MIVRS when PEP1 is EPLP and PEP2 is EQLSN; wherein the pair PEP5:PEP6 is not VSQDL:MIVKS when PEP1 is EPLT and PEP2 is EQLSN; wherein the pair PEP5:PEP6 is not VPQDL:MVVKS when PEP1 is EPLT and PEP2 is EQLSN.

In certain embodiments useful for inducing the conversion of a neoplastic cell into a cell (any cell) of a chondrocytic cell lineage, PEP9 is selected from the group consisting of KIPKAXXVPTEL, SIPKAXXVPTEL, HVTKPTXAPTKL, YVPKPXXAPTKL, TVPKPXXAPTQL, STPPTXXVPTRL, ASAAPXXVPQAL, ASASPXXVSQDL and ASASPXXVPQDL; PEP10 is selected from the group consisting of DMVVEGXGXR, EMVVEGXGXR, NMVVRSXGXH, NMVVRAXGXH, DMVVESXGXR, MIVRSXKXS, MIVKSXKXS and MVVKSXKXS; and wherein the pair PEP9:PEP10 is selected from the group consisting of KIPKAXXVPTEL:DMVVEGXGXR, SIPKAXXVPTEL:EMVVEGXGXR, HVTKPTXAPTKL:NMVVRSXGXH, YVPKPXXAPTKL:NMVVRSXGXH, TVPKPXXAPTQL:NMVVRAXGXH, STPPTXXVPTRL:DMVVESXGSR, ASAAPXXVPQAL:MIVRSXKXS, ASASPXXVSQDL:MIVKSXKXS and ASASPXXVPQDL:MVVKSXKXS; wherein PEP9:PEP10 is not KIPKAXXVPTEL:DMVVEGXGXR when PEP1 is SAIS and PEP2 is LKNYQ; wherein PEP9:PEP10 is not SIPKAXXVPTEL:EMVVEGXGXR when PEP1 is SAIS and PEP2 is LKNYQ; wherein PEP9:PEP10 is not HVTKPTXAPTKL:NMVVRSXGXH when PEP1 is NAIS and PEP2 is LKKYR; wherein PEP9:PEP10 is not YVPKPXXAPTKL:NMVVRSXGXH when PEP1 is NAIS and PEP2 is LKKYR; wherein PEP9:PEP10 is not TVPKPXXAPTQL:NMVVRAXGXH when PEP1 is NAIS and PEP2 is LKKYR; wherein PEP9:PEP10 is not STPPTXXVPTRL:DMVVESXGSR when PEP1 is SPIS; wherein PEP9:PEP10 is not ASAAPXXVPQAL:MIVRSXKXS when PEP1 is EPLP and PEP2 is EQLSN; wherein PEP9:PEP10 is not ASASPXXVSQDL:MIVKSXKXS when PEP1 is EPLT and PEP2 is EQLSN; wherein PEP9:PEP10 is not ASASPXXVPQDL:MVVKSXKXS when PEP1 is EPLT and PEP2 is EQLSN.

In certain embodiments useful for inducing the conversion of a neoplastic cell into a cell (any cell) of a chondrocytic cell lineage, PEP1 is selected from the group consisting of SAIS, NAIS, SPIS, EPLP and EPLT; PEP11 is selected from the group consisting of LYL, LYF, LFI, VYY and LYY; and the pair PEP1:PEP11 is selected from the group consisting of SAIS:LYL, NAIS:LYF, SPIS:LFI, EPLP:VYY and EPLT:LYY.

In one aspect, the present disclosure provides a GFR-binding compound, wherein said GFR-binding compound is a peptide, a variant or analog thereof, or a peptidomimetic as defined herein, with between 20 and 60 (in particular between 20 and 50, and more particularly, between 20 and 45) amino acids, comprising a peptide with four amino acids (PEP1) or a peptide with eight amino acids (PEP12), a peptide with five amino acids (PEP2), and optionally a pair of peptides selected form the group consisting of PEP3:PEP4, PEP3:PEP6, PEP3:PEP10, PEP5:PEP4, PEP9:PEP4, PEP5:PEP6, PEP5:PEP10, PEP9:PEP6 and PEP9:PEP10; wherein said GFR-binding compound does not comprise the pair of peptides VPTEL:DMVVE when PEP1 is SAIS and PEP2 is LKNYQ; wherein said GFR-binding compound does not comprise the pair of peptides VPTEL:EMVVE when PEP1 is SAIS and PEP2 is LKNYQ; wherein said GFR-binding compound does not comprise the pair of peptides APTKL:NMVVR when PEP1 is NAIS and PEP2 is LKKYR; wherein said GFR-binding compound does not comprise the pair of peptides APTQL:NMVVR when PEP1 is NAIS and PEP2 is LKKYR; wherein said GFR-binding compound does not comprise the pair of peptides VPTRL:DMVVE when PEP1 is SPIS and PEP2 is YKQYE; wherein said GFR-binding compound does not comprise the pair of peptides VPQAL:MIVRS when PEP1 is EPLP and PEP2 is EQLSN; wherein said GFR-binding compound does not comprise the pair of peptides VSQDL:MIVKS when PEP1 is EPLT and PEP2 is EQLSN; wherein said GFR-binding compound does not comprise the pair of peptides VPQDL:MVVKS when PEP1 is EPLT and PEP2 is EQLSN; and, optionally, wherein the RMSD is 2.45Å or less.

In one aspect, the present disclosure provides a GFR-binding compound, wherein said GFR-binding compound is a peptide, a variant or analog thereof, or a peptidomimetic as defined herein, with between 20 and 60 (in particular between 20 and 50, and more particularly, between 20 and 45) amino acids, comprising a peptide with four amino acids (PEP1) or a peptide with eight amino acids (PEP12), a peptide with five amino acids (PEP2), and optionally a pair of peptides selected form the group consisting of PEP3:PEP4, PEP3:PEP6, PEP3:PEP10, PEP5:PEP4, PEP9:PEP4, PEP5:PEP6, PEP5:PEP10, PEP9:PEP6 and PEP9:PEP10; wherein said GFR-binding compound does not comprise the pair of peptides KIPKAXXVPTEL:DMVVEGXGXR when PEP1 is SAIS and PEP2 is LKNYQ; wherein said GFR-binding compound does not comprise the pair of peptides SIPKAXXVPTEL:EMVVEGXGXR when PEP1 is SAIS and

PEP2 is LKNYQ; wherein said GFR-binding compound does not comprise the pair of peptides HVTKPTXAPTKL:NMVVRSXGXH when PEP1 is NAIS and PEP2 is LKKYR; wherein said GFR-binding compound does not comprise the pair of peptides YVPKPXXAPTKL:NMVVRSXGXH when PEP1 is NAIS and PEP2 is LKKYR; wherein said GFR-binding compound does not comprise the pair of peptides TVPKPXXAPTQL:NMVVRAXGXH when PEP1 is NAIS and PEP2 is LKKYR; wherein said GFR-binding compound does not comprise the pair of peptides STPPTXXVPTRL:DMVVESXGSR when PEP1 is SPIS; wherein said GFR-binding compound does not comprise the pair of peptides ASAAPXXVPQAL:MIVRSXKXS when PEP1 is EPLP and PEP2 is EQLSN; wherein said GFR-binding compound does not comprise the pair of peptides ASASPXXVSQDL:MIVKSXKXS when PEP1 is EPLT and PEP2 is EQLSN; wherein said GFR-binding compound does not comprise the pair of peptides ASASPXXVPQDL:MVVKSXKXS when PEP1 is EPLT and PEP2 is EQLSN; and, optionally, wherein the RMSD is 2.45Å or less.

In certain embodiments useful for inducing the conversion of a neoplastic cell into a cell (any cell) of a chondrocytic cell lineage, said GFR-binding compound is a synthetic molecule as defined herein in the definition section.

In certain embodiments useful for inducing the conversion of a neoplastic cell into a cell (any cell) of a chondrocytic cell lineage, said GFR-binding compound is a synthetic peptide, or a variant or analog thereof, or a peptidomimetic.

*Vascular cell lineage*

Certain embodiments of the invention are particularly useful for inducing the conversion of a neoplastic cell into a cell (any cell) of a vascular cell lineage.

In other embodiments useful for inducing the conversion of a neoplastic cell into a cell (any cell) of a vascular cell lineage, PEP1 is selected from the group consisting of SNIT, RPVQ and RSVK.

In other embodiments useful for inducing the conversion of a neoplastic cell into a cell (any cell) of a vascular cell lineage, PEP2 is selected from the group consisting of IGEMS, LGEMS, KEVQV and KKATV.

In other embodiments useful for inducing the conversion of a neoplastic cell into a cell (any cell) of a vascular cell lineage, PEP3 is selected from the group consisting of VPT, SRV and TQV.

In other embodiments useful for inducing the conversion of a neoplastic cell into a cell (any cell) of a vascular cell lineage, PEP4 is selected from the group consisting of QHN, EHS, EEH and EDH.

In other embodiments useful for inducing the conversion of a neoplastic cell into a cell (any cell) of a vascular cell lineage, PEP5 is a peptide of general formula PEP3-$AA^{11}$-$AA^{12}$; wherein PEP3 is selected from the group consisting of VPT, SRV and TQV; wherein $AA^{11}$ is selected from the group consisting of E, K, Q, R, A, D, G and H, in particular is E, G, H and Q; wherein $AA^{12}$ is selected from the group consisting of L, M, T, E, Q and H, in particular is selected from the group consisting of E, Q, H and L. In one particular example, PEP5 is selected from the group consisting of VPTGQ, VPTEE, SRVHH and TQVQL.

In other embodiments useful for inducing the conversion of a neoplastic cell into a cell (any cell) of a vascular cell lineage, PEP6 is a peptide of general formula $AA^{26}$-$AA^{27}$-$AA^{28}$-$AA^{29}$-PEP4; wherein PEP4 is selected from the group consisting of QHN, EHS, EEH and EDH; wherein $AA^{26}$ is absent or selected from the group consisting of $AA^{III}$ amino acids, preferably is absent; wherein $AA^{27}$ and $AA^{28}$ are independently selected from the group consisting of $AA^{III}$ and $AA^{V}$ amino acids, preferably $AA^{27}$ is selected from the group consisting of F, L, R and T, preferably $AA^{28}$ is L or E; wherein $AA^{29}$ is absent or selected from the group consisting of $AA^{II}$ amino acids, preferably is absent. In one particular example, PEP6 is selected from the group consisting of LEEHS, FLQHN, RLEEH and TLEDH.

In other embodiments useful for inducing the conversion of a neoplastic cell into a cell (any cell) of a vascular cell lineage, PEP7 is an amino acid or a peptide with between two and seven amino acids of general formula $AA^{1}$-$AA^{2}$-$AA^{3}$-$AA^{4}$-$AA^{5}$-$AA^{6}$-$AA^{7}$; wherein $AA^{1}$, $AA^{2}$, $AA^{3}$, $AA^{4}$, and $AA^{5}$ are independently absent or $AA^{I}$ as defined herein; wherein $AA^{6}$ is absent or selected from the group consisting of S, T, C, E, Q, P and R, preferably is selected from the group consisting of E, Q and R; wherein $AA^{7}$ is absent or is selected from the group consisting of S, T, C, E, Q, P and R, preferably is selected from the group consisting of S, C and P; and wherein at least one of $AA^{1}$, $AA^{2}$, $AA^{3}$, $AA^{4}$, $AA^{5}$, $AA^{6}$ or $AA^{7}$ is not absent. In one particular example, PEP7 is selected from the group consisting of NDEGLEX, SSVKXQP and RNVQXRP.

In other embodiments useful for inducing the conversion of a neoplastic cell into a cell (any cell) of a vascular cell lineage, PEP8 is an amino acid or a peptide with between two and six amino acids of general formula $AA^{33}$-$AA^{34}$-$AA^{35}$-$AA^{36}$-$AA^{37}$-$AA^{38}$; wherein $AA^{33}$ is absent or is selected from the group consisting of $AA^{I}$ amino acids at the exception of $AA^{VI}$ amino acids, in particular is selected from the group consisting of K, Q and L; $AA^{34}$ is absent or is selected from the group consisting of $AA^{III}$ and $AA^{IV}$ amino acids, in particular is absent, E or A; wherein $AA^{35}$ is absent or is selected from the group consisting of $AA^{11}$ amino acids, preferably is S or C; wherein $AA^{36}$ is absent or is selected from the group consisting of $AA^{III}$ and $AA^{IV}$ amino acids, in particular is selected from the group consisting of E, A and K; wherein $AA^{37}$ is absent or is selected from the group consisting of $AA^{II}$ amino acids, preferably is S or C; wherein $AA^{38}$ is absent or is selected from the group consisting of $AA^{1}$, in particular is selected from the group consisting of R, A and E; and wherein at least one of $AA^{33}$, $AA^{34}$, $AA^{35}$, $AA^{36}$, $AA^{37}$ or $AA^{38}$ is not absent. In one particular example,

PEP8 is selected from the group consisting of KXEXR, QXEXR, LEXAXA and LAXKXE.

In other embodiments useful for inducing the conversion of a neoplastic cell into a cell (any cell) of a vascular cell lineage, PEP9 is a peptide of general formula $AA^1$-$AA^2$-$AA^3$-$AA^4$-$AA^5$-$AA^6$-$AA^7$-PEP5; wherein PEP5 is a peptide of formula PEP3-$AA^{11}$-$AA^{12}$; wherein PEP3 is selected from the group consisting of VPT, SRV and TQV; wherein $AA^{11}$ is selected from the group consisting of E, K, Q, R, A, D, G and H, in particular is E, G, H and Q; wherein $AA^{12}$ is selected from the group consisting of L, M, T, E, Q and H, in particular is selected from the group consisting of E, Q, H and L; wherein $AA^1$, $AA^2$, $AA^3$, $AA^4$, and $AA^5$ are independently absent or $AA^I$ as defined herein; wherein $AA^6$ is absent or selected from the group consisting of S, T, C, E, Q, P and R, preferably is selected from the group consisting of E, Q and R; wherein $AA^7$ is selected from the group consisting of S, T, C, E, Q, P and R, preferably is selected from the group consisting of S, C and P. In one particular example, PEP9 is selected from the group consisting of NDEGLEXVPTEE, NDEGLEXVPTGQ, SSVKXQPSRVHH and RNVQXRPTQVQL.

In other embodiments useful for inducing the conversion of a neoplastic cell into a cell (any cell) of a vascular cell lineage, PEP10 is a peptide of general formula PEP6-$AA^{33}$-$AA^{34}$-$AA^{35}$-$AA^{36}$-$AA^{37}$-$AA^{38}$; wherein PEP6 is a peptide of formula $AA^{26}$-$AA^{27}$-$AA^{28}$-$AA^{29}$-PEP4; wherein PEP4 is selected from the group consisting of QHN, EHS, EEH and EDH; wherein $AA^{26}$ is absent or selected from the group consisting of $AA^{III}$ amino acids, preferably is absent; wherein $AA^{27}$ and $AA^{28}$ are independently selected from the group consisting of $AA^{III}$ and $AA^V$ amino acids, preferably $AA^{27}$ is selected from the group consisting of F, L, R and T, preferably $AA^{28}$ is L or E; wherein $AA^{29}$ is absent or selected from the group consisting of $AA^{II}$ amino acids, preferably is absent; wherein $AA^{33}$ is absent or is selected from the group consisting of $AA^I$ amino acids at the exception of $AA^{VI}$ amino acids, in particular is selected from the group consisting of K, Q and L; $AA^{34}$ is absent or is selected from the group consisting of $AA^{III}$ and $AA^{IV}$ amino acids, in particular is absent, E or A; wherein $AA^{35}$ is absent or is selected from the group consisting of $AA^{11}$ amino acids, preferably is S or C; wherein $AA^{36}$ is absent or is selected from the group consisting of $AA^{III}$ and $AA^{IV}$ amino acids, in particular is selected from the group consisting of E, A and K; wherein $AA^{37}$ is absent or is selected from the group consisting of $AA^{II}$ amino acids, preferably is S or C; wherein $AA^{38}$ is absent or is selected from the group consisting of $AA^1$, in particular is selected from the group consisting of R, A and E. In one particular example, PEP10 is selected from the group consisting of FLQHNKXEXR, LEEHSQXEXR, RLEEHLEXAXA and TLEDHLAXKXE.

In other embodiments useful for inducing the conversion of a neoplastic cell into a cell (any cell) of a vascular cell lineage, PEP12 is a peptide of general formula PEP1-$AA^{17}$-PEP11; wherein $AA^{17}$ is selected from the group consisting of G, A, V, L, I, P, F, M, W, T and S (in particular is selected from the group consisting of M, I, L, V and T); wherein PEP1 is selected from the group consisting of SNIT, RPVQ and RSVK.

In other embodiments useful for inducing the conversion of a neoplastic cell into a cell (any cell) of a vascular cell lineage, PEP11 is a peptide with 3 amino acids of general formula $AA^{18}$-$AA^{19}$-$AA^{20}$; wherein $AA^{18}$ is selected from the group consisting of L, V, Q, A and R, in particular is selected from the group consisting of Q, A and R; wherein $AA^{19}$ is selected from the group consisting of F, W, H, Y, I and K, in particular is I or K; wherein $AA^{20}$ is selected from the group consisting of L, F, Y, K, I, V and M, in particular is selected from the group consisting of M, V and I. In one particular example, PEP11 is selected from the group consisting of QIM, AKV and RKI.

The definitions of "PEP" pairs, quadruplets, hexaplets and heptuplet e.g. PEP1:PEP2, PEP12:PEP2, or PEP7:PEP5:PEP12:LINKER:PEP2:PEP6:PEP8, most particularly useful for inducing the conversion of a neoplastic cell into a cell (any cell) of a vascular cell lineage, are as already defined herein to the extent that PEP1 to PEP12 are particularly useful for these applications. For instances:

In other embodiments useful for inducing the conversion of a neoplastic cell into a cell (any cell) of a vascular cell lineage, the pair PEP1:PEP2 is selected from the group consisting of SNIT:IGEMS, SNIT:LGEMS, RSVK:KEVQV and RPVQ:KKATV.

In other embodiments useful for inducing the conversion of a neoplastic cell into a cell (any cell) of a vascular cell lineage, the pair PEP12:PEP2 is selected from the group consisting of SNIT-$AA^{17}$-QIM:IGEMS, SNIT-$AA^{17}$-QIM:LGEMS, RSVK-$AA^{17}$-AKV:KEVQV and RPVQ-$AA^{17}$-RKI:KKATV ; wherein $AA^{17}$ is selected from the group consisting of G, A, V, L, I, P, F, M, W, T and S (in particular is selected from the group consisting of M, V and T).

In other embodiments useful for inducing the conversion of a neoplastic cell into a cell (any cell) of a vascular cell lineage, PEP3 is selected from the group consisting of VPT, SRV and TQV; PEP4 is selected from the group consisting of QHN, EHS, EEH and EDH; and wherein the pair PEP3:PEP4 is selected from the group consisting of VPT:QHN, VPT:EHS, SRV:EEH and TQV:EDH.

In other embodiments useful for inducing the conversion of a neoplastic cell into a cell (any cell) of a vascular cell lineage, PEP5 is selected from the group consisting of VPTGQ, VPTEE, SRVHH and TQVQL; PEP6 is selected from the group consisting of LEEHS, FLQHN, RLEEH and TLEDH; and wherein the pair PEP5:PEP6 is selected from the group consisting of VPTGQ:LEEHS, VPTEE:FLQHN, SRVHH:RLEEH and TQVQL:TLEDH.

In other embodiments useful for inducing the conversion of a neoplastic cell into a cell (any cell) of a vascular cell lineage, PEP7 is selected from the group consisting of NDEGLEX, SSVKXQP and RNVQXRP; wherein PEP8 is selected from the group consisting of KXEXR, QXEXR, LEXAXA and LAXKXE; and wherein the pair PEP7:PEP8 is

selected from the group consisting of NDEGLEX:KXEXR, NDEGLEX:QXEXR, SSVKXQP:LEXAXA and RNVQXRP:LAXKXE.

In other embodiments useful for inducing the conversion of a neoplastic cell into a cell (any cell) of a vascular cell lineage, PEP9 is selected from the group consisting of NDEGLEXVPTEE, NDEGLEXVPTGQ, SSVKXQPSRVHH and RNVQXRPTQVQL; PEP10 is selected from the group consisting of FLQHNKXEXR, LEEHSQXEXR, RLEEHLEXAXA and TLEDHLAXKXE; and wherein the pair PEP9:PEP10 is selected from the group consisting of NDEGLEXVPTEE:FLQHNKXEXR, NDEGLEXVPTGQ:LEEHSQXEXR, SSVKXQPSRVHH:RLEEHLEXAXA and RNVQXRPTQVQL:TLEDHLAXKXE.

In other embodiments useful for inducing the conversion of a neoplastic cell into a cell (any cell) of a vascular cell lineage, PEP5 is selected from the group consisting of VPTGQ, VPTEE, SRVHH and TQVQL; PEP6 is selected from the group consisting of LEEHS, FLQHN, RLEEH and TLEDH; and wherein the pair PEP5:PEP6 is selected from the group consisting of VPTGQ:LEEHS, VPTEE:FLQHN, SRVHH:RLEEH and TQVQL:TLEDH; wherein the pair PEP5:PEP6 is not VPTEE:FLQHN when PEP1 is SNIT and PEP2 is IGEMS; wherein the pair PEP5:PEP6 is not VPTGQ:LEEHS when PEP1 is SNIT and PEP2 is LGEMS; wherein the pair PEP5:PEP6 is not SRVHH:RLEEH when PEP1 is RSVK and PEP2 is KEVQV; and wherein the pair PEP5:PEP6 is not TQVQL:TLEDH when PEP1 is RPVQ and PEP2 is KKATV.

In other embodiments useful for inducing the conversion of a neoplastic cell into a cell (any cell) of a vascular cell lineage, PEP9 is selected from the group consisting of NDEGLEXVPTEE, NDEGLEXVPTGQ, SSVKXQPSRVHH and RNVQXRPTQVQL; PEP10 is selected from the group consisting of FLQHNKXEXR, LEEHSQXEXR, RLEEHLEXAXA and TLEDHLAXKXE; and wherein the pair PEP9:PEP10 is selected from the group consisting of NDEGLEXVPTEE:FLQHNKXEXR, NDEGLEXVPTGQ:LEEHSQXEXR, SSVKXQPSRVHH:RLEEHLEXAXA and RNVQXRPTQVQL:TLEDHLAXKXE; wherein the pair PEP9:PEP10 is not NDEGLEXVPTEE:FLQHNKXEXR when PEP1 is SNIT and PEP2 is IGEMS; wherein the pair PEP9:PEP10 is not NDEGLEXVPTGQ:LEEHSQXEXR when PEP1 is SNIT and PEP2 is LGEMS; wherein the pair PEP9:PEP10 is not SSVKXQPSRVHH:RLEEHLEXAXA when PEP1 is RSVK and PEP2 is KEVQV; and wherein the pair PEP9:PEP10 is not RNVQXRPTQVQL:TLEDHLAXKXE when PEP1 is RPVQ and PEP2 is KKATV.

In other embodiments useful for inducing the conversion of a neoplastic cell into a cell (any cell) of a vascular cell lineage, PEP1 is selected from the group consisting of SNIT, RPVQ and RSVK; PEP11 is selected from the group consisting of QIM, AKV and RKI; and the pair PEP1:PEP11 is selected from the group consisting of SNIT:QIM, RSVK:KEVQV and RPVQ:KKATV.

In one aspect, the present disclosure provides a GFR-binding compound, wherein said GFR-binding compound is a peptide, a variant or analog thereof, or a peptidomimetic as defined herein, with between 20 and 60 (in particular between 20 and 50, and more particularly, between 20 and 45) amino acids, comprising a peptide with four amino acids (PEP1) or a peptide with eight amino acids (PEP12), a peptide with five amino acids (PEP2), and optionally a pair of peptides selected form the group consisting of PEP3:PEP4, PEP3:PEP6, PEP3:PEP10, PEP5:PEP4, PEP9:PEP4, PEP5:PEP6, PEP5:PEP10, PEP9:PEP6 and PEP9:PEP10; wherein said GFR-binding compound does not comprise the pair of peptides VPTEE:FLQHN when PEP1 is SNIT and PEP2 is IGEMS; wherein said GFR-binding compound does not comprise the pair of peptides VPTGQ:LEEHS when PEP1 is SNIT and PEP2 is LGEMS; wherein said GFR-binding compound does not comprise the pair of peptides SRVHH:RLEEH when PEP1 is RSVK and PEP2 is KEVQV; and wherein said GFR-binding compound does not comprise the pair of peptides TQVQL:TLEDH when PEP1 is RPVQ and PEP2 is KKATV; and, optionally, wherein the RMSD is 2.45Å or less.

In one aspect, the present disclosure provides a GFR-binding compound, wherein said GFR-binding compound is a peptide, a variant or analog thereof, or a peptidomimetic as defined herein, with between 20 and 60 (in particular between 20 and 50, and more particularly, between 20 and 45) amino acids, comprising a peptide with four amino acids (PEP1) or a peptide with eight amino acids (PEP12), a peptide with five amino acids (PEP2), and optionally a pair of peptides selected form the group consisting of PEP3:PEP4, PEP3:PEP6, PEP3:PEP10, PEP5:PEP4, PEP9:PEP4, PEP5:PEP6, PEP5:PEP10, PEP9:PEP6 and PEP9:PEP10; wherein said GFR-binding compound does not comprise the pair of peptides NDEGLEXVPTEE:FLQHNKXEXR when PEP1 is SNIT and PEP2 is IGEMS; wherein said GFR-binding compound does not comprise the pair of peptides NDEGLEXVPTGQ:LEEHSQXEXR when PEP1 is SNIT and PEP2 is LGEMS; wherein said GFR-binding compound does not comprise the pair of peptides SSVKXQPSRVHH:RLEEHLEXAXA when PEP1 is RSVK and PEP2 is KEVQV; and wherein said GFR-binding compound does not comprise the pair of peptides RNVQXRPTQVQL:TLEDHLAXKXE when PEP1 is RPVQ and PEP2 is KKATV; and, optionally, wherein the RMSD is 2.45Å or less.

In other embodiments useful for inducing the conversion of a neoplastic cell into a cell (any cell) of a vascular cell lineage, said GFR-binding compound is a synthetic molecule as defined herein in the definition section.

In other embodiments useful for inducing the conversion of a neoplastic cell into a cell (any cell) of a vascular cell lineage, said GFR-binding compound is a synthetic peptide, or a variant or analog thereof, or a peptidomimetic.

*Neuron lineage*

Certain embodiments of the invention are particularly useful for inducing the conversion of a neoplastic cell into a cell (any cell) of a neuron lineage.

In certain embodiments useful for inducing the conversion of a neoplastic cell into a cell (any cell) of a neuron lineage, PEP1 is selected from the group consisting of NAIS, SPIS and EPIS.

In certain embodiments useful for inducing the conversion of a neoplastic cell into a cell (any cell) of a neuron lineage, PEP2 is selected from the group consisting of LKKYR, LKYHY, YKQYE and KFKYE.

In certain embodiments useful for inducing the conversion of a neoplastic cell into a cell (any cell) of a neuron lineage, PEP3 is selected from the group consisting of VPT, APT, VPA, VPQ and VSQ.

In certain embodiments useful for inducing the conversion of a neoplastic cell into a cell (any cell) of a neuron lineage, PEP4 is selected from the group consisting of VVE, VVR, VVK, VAE, AVS, VRS and VKS.

In certain embodiments useful for inducing the conversion of a neoplastic cell into a cell (any cell) of a neuron lineage, PEP5 is a peptide of general formula PEP3-$AA^{11}$-$AA^{12}$; wherein PEP3 is selected from the group consisting of VPT, APT, VPA, VPQ and VSQ; wherein $AA^{11}$ is selected from the group consisting of E, K, Q, R, A, D, G and H, in particular E, K, Q, R, A and D; wherein $AA^{12}$ is selected from the group consisting of L, M, T, E, Q and H, in particular L. In one particular example, PEP5 is selected from the group consisting of VPTEL, APTKL, APTQL, VPTKL, VPARL, VPQAL, VSQDL and VPQDL.

In certain embodiments useful for inducing the conversion of a neoplastic cell into a cell (any cell) of a neuron lineage, PEP6 is a peptide of general formula $AA^{26}$-$AA^{27}$-$AA^{28}$-$AA^{29}$-PEP4; wherein PEP4 is selected from the group consisting of VVE, VVR, VVK, VAE, AVS, VRS and VKS; wherein $AA^{26}$ is absent or selected from the group consisting of $AA^{III}$ amino acids, preferably absent or E; wherein $AA^{27}$ and $AA^{28}$ are independently selected from the group consisting of $AA^{III}$ and $AA^{V}$ amino acids, preferably $AA^{27}$ is selected from the group consisting of D, E, N, G and M, preferably $AA^{28}$ is selected from the group consisting of M, I and V; wherein $AA^{29}$ is absent or selected from the group consisting of $AA^{II}$ amino acids, preferably absent or S. In one particular example, PEP6 is selected from the group consisting of DMVVE, EMVVE, NMVVR, NMVVK, EGMSVAE, GMAVS, DMVVE, MIVRS, MIVKS and MVVKS.

In certain embodiments useful for inducing the conversion of a neoplastic cell into a cell (any cell) of a neuron lineage, PEP7 is an amino acid or a peptide with between two and seven amino acids of general formula $AA^1$-$AA^2$-$AA^3$-$AA^4$-$AA^5$-$AA^6$-$AA^7$; wherein $AA^1$, $AA^2$, $AA^3$, $AA^4$, and $AA^5$ are independently absent or $AA^I$ as defined herein; wherein $AA^6$ is absent or selected from the group consisting of S, T, C, E, Q, P and R, preferably S or C; wherein $AA^7$ is absent or is selected from the group consisting of S, T, C, E, Q, P and R, preferably is S or C; and wherein at least one of $AA^1$, $AA^2$, $AA^3$, $AA^4$, $AA^5$, $AA^6$ or $AA^7$ is not absent. In one particular example, PEP7 is selected from the group consisting of KIPKAXX, SIPKAXX, HVTKPTX, YVPKPXX, TVPKPXX, AVPKAXX, KVGKAXX, ASAAPXX, ASASPXX and RNVQXRP.

In certain embodiments useful for inducing the conversion of a neoplastic cell into a cell (any cell) of a neuron lineage, PEP8 is an amino acid or a peptide with between two and six amino acids of general formula $AA^{33}$-$AA^{34}$-$AA^{35}$-$AA^{36}$-$AA^{37}$-$AA^{38}$; wherein $AA^{33}$ is absent or is selected from the group consisting of $AA^I$ amino acids at the exception of $AA^{VI}$ amino acids, in particular is absent or is selected from the group consisting of G, S, A and E; $AA^{34}$ is absent or is selected from the group consisting of $AA^{III}$ and $AA^{IV}$ amino acids, in particular is absent; wherein $AA^{35}$ is absent or is selected from the group consisting of $AA^{II}$ amino acids, preferably is S or C; wherein $AA^{36}$ is absent or is selected from the group consisting of $AA^{III}$ and $AA^{IV}$ amino acids, in particular is G or K; wherein $AA^{37}$ is absent or is selected from the group consisting of $AA^{II}$ amino acids, preferably is S or C; wherein $AA^{38}$ is absent or is selected from the group consisting of $AA^I$, in particular is selected from the group consisting of R, H and S; and wherein at least one of $AA^{33}$, $AA^{34}$, $AA^{35}$, $AA^{36}$, $AA^{37}$ or $AA^{38}$ is not absent. In one particular example, PEP8 is selected from the group consisting of GXGXR, SXGXH, AXGXH, XGXR, XKXS and LAXKXE.

In certain embodiments useful for inducing the conversion of a neoplastic cell into a cell (any cell) of a neuron lineage, PEP9 is a peptide of general formula $AA^1$-$AA^2$-$AA^3$-$AA^4$-$AA^5$-$AA^6$-$AA^7$-PEP5; wherein PEP5 is a peptide of formula PEP3-$AA^{11}$-$AA^{12}$; wherein PEP3 is selected from the group consisting of VPT, APT, VPA, VPQ and VSQ; wherein $AA^{11}$ is selected from the group consisting of E, K, Q, R, A, D, G and H, in particular E, K, Q, R, A and D; wherein $AA^{12}$ is selected from the group consisting of L, M, T, E, Q and H, in particular L; wherein $AA^1$, $AA^2$, $AA^3$, $AA^4$, and $AA^5$ are independently absent or $AA^I$ as defined herein; wherein $AA^6$ is absent or selected from the group consisting of S, T, C, E, Q, P and R, preferably S or C; wherein $AA^7$ is selected from the group consisting of S, T, C, E, Q, P and R, preferably is S or C. In one particular example, PEP9 is selected from the group consisting of KIPKAXXVPTEL, SIPKAXXVPTEL, HVTKPTXAPTKL, YVPKPXXAPTKL, TVPKPXXAPTQL, AVPKAXXAPTKL, KVGKAXXVPTKL, ASAAPXXVPQAL, ASASPXXVSQDL, ASASPXXVPQDL and RNVQXRPTQVQL.

In certain embodiments useful for inducing the conversion of a neoplastic cell into a cell (any cell) of a neuron lineage, PEP10 is a peptide of general formula PEP6-$AA^{33}$-$AA^{34}$-$AA^{35}$-$AA^{36}$-$AA^{37}$-$AA^{38}$; wherein PEP6 is a peptide of formula $AA^{26}$-$AA^{27}$-$AA^{28}$-$AA^{29}$-PEP4; wherein PEP4 is selected from the group consisting of VVE, VVR, VVK, VAE,

AVS, VRS and VKS; wherein $AA^{26}$ is absent or selected from the group consisting of $AA^{III}$ amino acids, preferably absent or E; wherein $AA^{27}$ and $AA^{28}$ are independently selected from the group consisting of $AA^{III}$ and $AA^{V}$ amino acids, preferably $AA^{27}$ is selected from the group consisting of D, E, N, G and M, preferably $AA^{28}$ is selected from the group consisting of M, I and V; wherein $AA^{29}$ is absent or selected from the group consisting of $AA^{II}$ amino acids, preferably absent or S; wherein $AA^{33}$ is absent or is selected from the group consisting of $AA^{I}$ amino acids at the exception of $AA^{VI}$ amino acids, in particular is absent or is selected from the group consisting of G, S, A and E; $AA^{34}$ is absent or is selected from the group consisting of $AA^{III}$ and $AA^{IV}$ amino acids, in particular is absent; wherein $AA^{35}$ is absent or is selected from the group consisting of $AA^{II}$ amino acids, preferably is S or C; wherein $AA^{36}$ is absent or is selected from the group consisting of $AA^{III}$ and $AA^{IV}$ amino acids, in particular is G or K; wherein $AA^{37}$ is absent or is selected from the group consisting of $AA^{II}$ amino acids, preferably is S or C; wherein $AA^{38}$ is absent or is selected from the group consisting of $AA^{I}$, in particular is selected from the group consisting of R, H and S. In one particular example, PEP10 is selected from the group consisting of DMVVEGXGXR, EMVVEGXGXR, NMVVRSXGXH, NMVVRAXGXH, NMVVKAXGXH, EGMSVAEXGXR, MIVRSXKXS, MIVKSXKXS, MVVKSXKXS and TLEDHLAXKXE.

In certain embodiments useful for inducing the conversion of a neoplastic cell into a cell (any cell) of a neuron lineage, PEP12 is a peptide of general formula PEP1-$AA^{17}$-PEP11; wherein $AA^{17}$ is selected from the group consisting of G, A, V, L, I, P, F, M, W, T and S (in particular is selected from the group consisting of M, I, L, V and T); wherein PEP1 is selected from the group consisting of NAIS, SPIS and EPIS.

In certain embodiments useful for inducing the conversion of a neoplastic cell into a cell (any cell) of a neuron lineage, PEP11 is a peptide with 3 amino acids of general formula $AA^{18}$-$AA^{19}$-$AA^{20}$; wherein $AA^{18}$ is selected from the group consisting of L, V, Q, A and R, in particular is L; wherein $AA^{19}$ is selected from the group consisting of F, W, H and Y (in particular is an aromatic, polar amino acid such as Y); wherein $AA^{20}$ is selected from the group consisting of L, F, Y, K, I, V and M, in particular is selected from the group consisting of L, F, I, and K. In one particular example, PEP11 is selected from the group consisting of LYL, LYF, LYI and LYK.

The definitions of "PEP" pairs, quadruplets, hexaplets and heptuplet e.g. PEP1:PEP2, PEP12:PEP2, or PEP7:PEP5:PEP12:LINKER:PEP2:PEP6:PEP8, most particularly useful for inducing the conversion of a neoplastic cell into a cell (any cell) of a neuron lineage, are as already defined herein to the extent that PEP1 to PEP12 are particularly useful for these applications. For instances:

In certain embodiments useful for inducing the conversion of a neoplastic cell into a cell (any cell) of a neuron lineage, the pair PEP1:PEP2 is selected from the group consisting of NAIS:LKKYR, SPIS:LKYHY, EPIS:KFKYE and SPIS:YKQYE.

In certain embodiments useful for inducing the conversion of a neoplastic cell into a cell (any cell) of a neuron lineage, the pair PEP12:PEP2 is selected from the group consisting of NAIS-$AA^{17}$-LYF:LKKYR, SPIS-$AA^{17}$-LYK:LKYHY, EPIS-$AA^{17}$-LYL:KFKYE and SPIS-$AA^{17}$-LYI:YKQYE; wherein $AA^{17}$ is selected from the group consisting of G, A, V, L, I, P, F, M, W, T and S (in particular is selected from the group consisting of V, I and T).

In certain embodiments useful for inducing the conversion of a neoplastic cell into a cell (any cell) of a neuron lineage, PEP3 is selected from the group consisting of VPT, APT, VPA, VPQ and VSQ; PEP4 is selected from the group consisting of VVE, VVR, VVK, VAE, AVS, VRS and VKS; and wherein the pair PEP3:PEP4 is selected from the group consisting of VPT:VVE, APT:VVR, APT:VVK, VPT:VAE, VPT:AVS, VPA:VVE, VPQ:VRS, VSQ:VKS and VPQ:VKS.

In certain embodiments useful for inducing the conversion of a neoplastic cell into a cell (any cell) of a neuron lineage, PEP5 is selected from the group consisting of VPTEL, APTKL, APTQL, VPTKL, VPARL, VPQAL, VSQDL and VPQDL; PEP6 is selected from the group consisting of DMVVE, EMVVE, NMVVR, NMVVK, EGMSVAE, GMAVS, DMVVE, MIVRS, MIVKS and MVVKS; and wherein the pair PEP5:PEP6 is selected from the group consisting of VPTEL:DMVVE, VPTEL:EMVVE, APTKL:NMVVR, APTQL:NMVV, APTKL:NMVVK, VPTKL:EGMSVAE, VPTKL:EGMAVS, VPARL:DMVVE, VPQAL:MIVRS, VSQDL:MIVKS and VPQDL:MVVKS.

In certain embodiments useful for inducing the conversion of a neoplastic cell into a cell (any cell) of a neuron lineage, PEP7 is selected from the group consisting of KIPKAXX, SIPKAXX, HVTKPTX, YVPKPXX, TVPKPXX, AVPKAXX, KVGKAXX, ASAAPXX, ASASPXX and RNVQXRP; wherein PEP8 is selected from the group consisting of GXGXR, SXGXH, AXGXH, XGXR, XKXS and LAXKXE; and wherein the pair PEP7:PEP8 is selected from the group consisting of KIPKAXX:GXGXR, SIPKAXX:GXGXR, HVTKPTX:SXGXH, YVPKPXX:SXGXH, TVPKPXX:AXGXH, AVPKAXX:AXGXH, KVGKAXX:XGXR, KASKAXX:EXGXR, AAPASXX:AXGXR, ASAAPXX:XKXS, ASASPXX:XKXS and ASASPXX:XKXS.

In certain embodiments useful for inducing the conversion of a neoplastic cell into a cell (any cell) of a neuron lineage, PEP9 is selected from the group consisting of KIPKAXXVPTEL, SIPKAXXVPTEL, HVTKPTXAPTKL, YVPKPXXAPTKL, TVPKPXXAPTQL, AVPKAXXAPTKL, KVGKAXXVPTKL, ASAAPXXVPQAL, ASASPXXVSQDL, ASASPXXVPQDL and RNVQXRPTQVQL; PEP10 is selected from the group consisting of DMVVEGXGXR, EMVVEGXGXR, NMVVRSXGXH, NMVVRAXGXH, NMVVKAXGXH, EGMSVAEXGXR, MIVRSXKXS, MIVKSXKXS, MVVKSXKXS and TLEDHLAXKXE; and wherein the pair PEP9:PEP10 is selected from the group consisting of KIPKAXXVPTEL:DMVVEGXGXR, SIPKAXXVPTEL:EMVVEGXGXR, HVTKPTXAPTKL:NMVVRSXGXH, YVPKPXXAPTKL:NMVVRSXGXH, TVPKPXXAPTQL:NMVVRAXGXH, AVPKAXXAPTKL:NMVVKAXGXH, KVGKAXXVPTKL:EGMSVAEXGXR, KASKAXXVPTKL:GMAVSEXGXR, AAPASXXVPARL:DMVVEAXGXR, ASAAPXXVPQAL:MIVRSXKXS, ASASPXXVSQDL:MIVKSXKXS and ASASPXXVPQDL:MVVKSXKXS.

In certain embodiments useful for inducing the conversion of a neoplastic cell into a cell (any cell) of a neuron lineage, PEP5 is selected from the group consisting of VPTEL, APTKL, APTQL, VPTKL, VPARL, VPQAL, VSQDL and VPQDL; PEP6 is selected from the group consisting of DMVVE, EMVVE, NMVVR, NMVVK, EGMSVAE, GMAVS, DMVVE, MIVRS, MIVKS and MVVKS; and wherein the pair PEP5:PEP6 is selected from the group consisting of VPTEL:DMVVE, VPTEL:EMVVE, APTKL:NMVVR, APTQL:NMVV, APTKL:NMVVK, VPTKL:EGMSVAE, VPTKL:EGMAVS, VPARL:DMVVE, VPQAL:MIVRS, VSQDL:MIVKS and VPQDL:MVVKS; wherein the pair PEP5:PEP6 is not APTQL:NMVVR when PEP1 is NAIS and PEP2 is LKKYR; wherein the pair PEP5:PEP6 is not VPTKL:EGMSVAE when PEP1 is SPIS and PEP2 is LKYHY; wherein the pair PEP5:PEP6 is not VPTKL:GMAVS when PEP1 is EPIS and PEP2 is KFKYE; and wherein the pair PEP5:PEP6 is not VPARL:DMVVE when PEP1 is SPIS and PEP2 is YKQYE.

In certain embodiments useful for inducing the conversion of a neoplastic cell into a cell (any cell) of a neuron lineage, PEP9 is selected from the group consisting of KIPKAXXVPTEL, SIPKAXXVPTEL, HVTKPTXAPTKL, YVPKPXXAPTKL, TVPKPXXAPTQL, AVPKAXXAPTKL, KVGKAXXVPTKL, ASAAPXXVPQAL, ASASPXXVSQDL, ASASPXXVPQDL and RNVQXRPTQVQL; PEP10 is selected from the group consisting of DMVVEGXGXR, EMVVEGXGXR, NMVVRSXGXH, NMVVRAXGXH, NMVVKAXGXH, EGMSVAEXGXR, MIVRSXKXS, MIVKSXKXS, MVVKSXKXS and TLEDHLAXKXE; and wherein the pair PEP9:PEP10 is selected from the group consisting of KIPKAXXVPTEL:DMVVEGXGXR, SIPKAXXVPTEL:EMVVEGXGXR, HVTKPTXAPTKL:NMVVRSXGXH, YVPKPXXAPTKL:NMVVRSXGXH, TVPKPXXAPTQL:NMVVRAXGXH, AVPKAXXAPTKL:NMVVKAXGXH, KVGKAXXVPTKL:EGMSVAEXGXR, KASKAXXVPTKL:GMAVSEXGXR, AAPASXXVPARL:DMVVEAXGXR, ASAAPXXVPQAL:MIVRSXKXS, ASASPXXVSQDL:MIVKSXKXS and ASASPXXVPQDL:MVVKSXKXS; wherein PEP9:PEP10 is not TVPKPXXAPTQL:NMVVRAXGXH when PEP1 is NAIS and PEP2 is LKKYR ; wherein PEP9:PEP10 is not KVGKAXXVPTKL:EGMSVAEXGXR when PEP1 is SPIS and PEP2 is LKYHY ; wherein PEP9:PEP10 is not KASKAXXVPTKL:GMAVSEXGXR when PEP1 is EPIS and PEP2 is KFKYE ; wherein PEP9:PEP10 is not AAPASXXVPARL:DMVVEAXGXR when PEP1 is SPIS.

In certain embodiments useful for inducing the conversion of a neoplastic cell into a cell (any cell) of a neuron lineage, PEP1 is selected from the group consisting of NAIS, SPIS and EPIS; PEP11 is selected from the group consisting of LYF, LYK, LYL and LYI; and the pair PEP1:PEP11 is selected from the group consisting of NAIS:LYF, SPIS:LYK, EPIS:LYL and SPIS:LYI.

In one aspect, the present disclosure provides a GFR-binding compound, wherein said GFR-binding compound is a peptide, a variant or analog thereof, or a peptidomimetic as defined herein, with between 20 and 60 (in particular between 20 and 50, and more particularly, between 20 and 45) amino acids, comprising a peptide with four amino acids (PEP1) or a peptide with eight amino acids (PEP12), a peptide with five amino acids (PEP2), and optionally a pair of peptides selected form the group consisting of PEP3:PEP4, PEP3:PEP6, PEP3:PEP10, PEP5:PEP4, PEP9:PEP4, PEP5:PEP6, PEP5:PEP10, PEP9:PEP6 and PEP9:PEP10; wherein said GFR-binding compound does not comprise the pair of peptides APTQL:NMVVR when PEP1 is NAIS and PEP2 is LKKYR; wherein said GFR-binding compound does not comprise the pair of peptides VPTKL:EGMSVAE when PEP1 is SPIS and PEP2 is LKYHY; wherein said GFR-binding compound does not comprise the pair of peptides VPTKL:GMAVS when PEP1 is EPIS and PEP2 is KFKYE; and wherein said GFR-binding compound does not comprise the pair of peptides VPARL:DMVVE when PEP1 is SPIS and PEP2 is YKQYE; and, optionally, wherein the RMSD is 2.45Å or less.

In one aspect, the present disclosure provides a GFR-binding compound, wherein said GFR-binding compound is a peptide, a variant or analog thereof, or a peptidomimetic as defined herein, with between 20 and 60 (in particular between 20 and 50, and more particularly, between 20 and 45) amino acids, comprising a peptide with four amino acids (PEP1) or a peptide with eight amino acids (PEP12), a peptide with five amino acids (PEP2), and optionally a pair of peptides selected form the group consisting of PEP3:PEP4, PEP3:PEP6, PEP3:PEP10, PEP5:PEP4, PEP9:PEP4, PEP5:PEP6, PEP5:PEP10, PEP9:PEP6 and PEP9:PEP10; wherein said GFR-binding compound does not comprise the pair of peptides TVPKPXXAPTQL:NMVVRAXGXH when PEP1 is NAIS and PEP2 is LKKYR ; wherein said GFR-binding compound does not comprise the pair of peptides KVGKAXXVPTKL:EGMSVAEXGXR when PEP1 is SPIS and PEP2 is LKYHY ; wherein said GFR-binding compound does not comprise the pair of peptides KASKAXXVPTKL:GMAVSEXGXR when PEP1 is EPIS and PEP2 is KFKYE ; wherein said GFR-binding compound does not comprise the pair of peptides AAPASXXVPARL:DMVVEAXGXR when PEP1 is SPIS; and, optionally, wherein the RMSD is 2.45Å or less.

In certain embodiments useful for inducing the conversion of a neoplastic cell into a cell (any cell) of a neuron lineage, said GFR-binding compound is a synthetic molecule as defined herein in the definition section.

In certain embodiments useful for inducing the conversion of a neoplastic cell into a cell (any cell) of a neuron lineage, said GFR-binding compound is a synthetic peptide, or a variant or analog thereof, or a peptidomimetic.

*Eye retina cell lineage*

Certain embodiments of the invention are particularly useful for inducing the conversion of a neoplastic cell into a cell (any cell) of an eye retina cell lineage.

In certain embodiments useful for inducing the conversion of a neoplastic cell into a cell (any cell) of an eye retina cell lineage, PEP1 is SPIN.

In certain embodiments useful for inducing the conversion of a neoplastic cell into a cell (any cell) of an eye retina cell lineage, PEP2 is YGKIP.

In certain embodiments useful for inducing the conversion of a neoplastic cell into a cell (any cell) of an eye retina cell lineage, PEP3 is selected from the group consisting of VPT, APT, TPT, VPA and APV.

In certain embodiments useful for inducing the conversion of a neoplastic cell into a cell (any cell) of an eye retina cell lineage, PEP4 is selected from the group consisting of VVE, VVR, VVK, VAE, AVS, VVD and VEE.

In certain embodiments useful for inducing the conversion of a neoplastic cell into a cell (any cell) of an eye retina cell lineage, PEP5 is a peptide of general formula PEP3-$AA^{11}$-$AA^{12}$; wherein PEP3 is selected from the group consisting of VPT, APT, TPT, VPA and APV; wherein $AA^{11}$ is selected from the group consisting of E, K, Q, R, A, D, G and H, in particular is E, K, Q and R; wherein $AA^{12}$ is selected from the group consisting of L, M, T, E, Q and H, in particular is L, M or T. In one particular example, PEP5 is selected from the group consisting of VPTEL, APTKL, APTQL, VPTKL, TPTKM, VPARL, VPTRL and APVKT.

In certain embodiments useful for inducing the conversion of a neoplastic cell into a cell (any cell) of an eye retina cell lineage, PEP6 is a peptide of general formula $AA^{26}$-$AA^{27}$-$AA^{28}$-$AA^{29}$-PEP4; wherein PEP4 is selected from the group consisting of VVE, VVR, VVK, VAE, AVS, VVD and VEE; wherein $AA^{26}$ is absent or selected from the group consisting of $AA^{III}$ amino acids, preferably absent or E; wherein $AA^{27}$ and $AA^{28}$ are independently selected from the group consisting of $AA^{III}$ and $AA^{V}$ amino acids, preferably $AA^{27}$ is selected from the group consisting of D, E, N, G and M, preferably $AA^{28}$ is M or I; wherein $AA^{29}$ is absent or selected from the group consisting of $AA^{II}$ amino acids, preferably absent or S. In one particular example, PEP6 is selected from the group consisting of DMVVE, EMVVE, NMVVR, NMVVK, EGMSVAE, EGMAVS, GMVVD and DMIVEE.

In certain embodiments useful for inducing the conversion of a neoplastic cell into a cell (any cell) of an eye retina cell lineage, PEP7 is an amino acid or a peptide with between two and seven amino acids of general formula $AA^{1}$-$AA^{2}$-$AA^{3}$-$AA^{4}$-$AA^{5}$-$AA^{6}$-$AA^{7}$; wherein $AA^{1}$, $AA^{2}$, $AA^{3}$, $AA^{4}$, and $AA^{5}$ are independently absent or $AA^{I}$ as defined herein; wherein $AA^{6}$ is absent or selected from the group consisting of S, T, C, E, Q, P and R, preferably is S or C; wherein $AA^{7}$ is absent or is selected from the group consisting of S, T, C, E, Q, P and R, preferably is S or C; and wherein at least one of $AA^{1}$, $AA^{2}$, $AA^{3}$, $AA^{4}$, $AA^{5}$, $AA^{6}$ or $AA^{7}$ is not absent. In one particular example, PEP7 is selected from the group consisting of KIPKAXX, SIPKAXX, HVTKPTX, YVPKPXX, TVPKPXX, AVPKAXX, KVGKAXX, KASKAXX, GSAGPXX, AAPAXXS, STPPTXX, HVPKPXX and RVPSTXX.

In certain embodiments useful for inducing the conversion of a neoplastic cell into a cell (any cell) of an eye retina cell lineage, PEP8 is an amino acid or a peptide with between two and six amino acids of general formula $AA^{33}$-$AA^{34}$-$AA^{35}$-$AA^{36}$-$AA^{37}$-$AA^{38}$; wherein $AA^{33}$ is absent or is selected from the group consisting of $AA^{I}$ amino acids at the exception of $AA^{VI}$ amino acids, in particular is absent or is selected from the group consisting of G, S, A, E and R; $AA^{34}$ is absent or is selected from the group consisting of $AA^{III}$ and $AA^{IV}$ amino acids, in particular is absent; wherein $AA^{35}$ is absent or is selected from the group consisting of $AA^{II}$ amino acids, preferably is S or C; wherein $AA^{36}$ is absent or is selected from the group consisting of $AA^{III}$ and $AA^{IV}$ amino acids; wherein $AA^{37}$ is absent or is selected from the group consisting of $AA^{II}$ amino acids, preferably is S or C; wherein $AA^{38}$ is absent or is selected from the group consisting of $AA^{I}$, in particular is selected from the group consisting of R, H, S and L; and wherein at least one of $AA^{33}$, $AA^{34}$, $AA^{35}$, $AA^{36}$, $AA^{37}$ or $AA^{38}$ is not absent. In one particular example, PEP8 is selected from the group consisting of GXGXR, SXGXH, AXGXH, XGXR, EXGXR, RXGXS, AXGXR, SXGXR and XGXL.

In certain embodiments useful for inducing the conversion of a neoplastic cell into a cell (any cell) of an eye retina cell lineage, PEP9 is a peptide of general formula $AA^{1}$-$AA^{2}$-$AA^{3}$-$AA^{4}$-$AA^{5}$-$AA^{6}$-$AA^{7}$-PEP5; wherein PEP5 is a peptide of formula PEP3-$AA^{11}$-$AA^{12}$; wherein PEP3 is selected from the group consisting of VPT, APT, TPT, VPA and APV; wherein $AA^{11}$ is selected from the group consisting of E, K, Q, R, A, D, G and H, in particular is E, K, Q and R; wherein $AA^{12}$ is selected from the group consisting of L, M, T, E, Q and H, in particular is L, M or T; wherein $AA^{1}$, $AA^{2}$, $AA^{3}$, $AA^{4}$, and $AA^{5}$ are independently absent or $AA^{I}$ as defined herein; wherein $AA^{6}$ is absent or selected from the group consisting of S, T, C, E, Q, P and R, preferably is S or C; wherein $AA^{7}$ is selected from the group consisting of S, T, C, E, Q, P and R, preferably is S or C. In one particular example, PEP9 is selected from the group consisting of KIPKAXXVPTEL, SIPKAXXVPTEL, HVTKPTXAPTKL, YVPKPXXAPTKL, TVPKPXXAPTQL, AVPKAXXAPTKL, KVGKAXXVPTKL, KASKAXXVPTKL, GSAGPXXTPTKL, AAPASXXVPARL, STPPTXXVPTRL, HVPKPXXAPTKL and RVPSTXXAPVKT.

In certain embodiments useful for inducing the conversion of a neoplastic cell into a cell (any cell) of an eye retina cell lineage, PEP10 is a peptide of general formula PEP6-$AA^{33}$-$AA^{34}$-$AA^{35}$-$AA^{36}$-$AA^{37}$-$AA^{38}$; wherein PEP6 is a peptide of formula $AA^{26}$-$AA^{27}$-$AA^{28}$-$AA^{29}$-PEP4; wherein PEP4 is selected from the group consisting of VVE, VVR, VVK,

VAE, AVS, VVD and VEE; wherein $AA^{26}$ is absent or selected from the group consisting of $AA^{III}$ amino acids, preferably absent or E; wherein $AA^{27}$ and $AA^{28}$ are independently selected from the group consisting of $AA^{III}$ and $AA^{V}$ amino acids, preferably $AA^{27}$ is selected from the group consisting of D, E, N, G and M, preferably $AA^{28}$ is M or I; wherein $AA^{29}$ is absent or selected from the group consisting of $AA^{II}$ amino acids, preferably absent or S; wherein $AA^{33}$ is absent or is selected from the group consisting of $AA^{I}$ amino acids at the exception of $AA^{VI}$ amino acids, in particular is absent or is selected from the group consisting of G, S, A, E and R; $AA^{34}$ is absent or is selected from the group consisting of $AA^{III}$ and $AA^{IV}$ amino acids, in particular is absent; wherein $AA^{35}$ is absent or is selected from the group consisting of $AA^{II}$ amino acids, preferably is S or C; wherein $AA^{36}$ is absent or is selected from the group consisting of $AA^{III}$ and $AA^{IV}$ amino acids; wherein $AA^{37}$ is absent or is selected from the group consisting of $AA^{II}$ amino acids, preferably is S or C; wherein $AA^{38}$ is absent or is selected from the group consisting of $AA^{I}$, in particular is selected from the group consisting of R, H, S and L. In one particular example, PEP10 is selected from the group consisting of DMVVEGXGXR, EMVVEGXGXR, NMVVRSXGXH, NMVVRAXGXH, NMVVKAXGXH, EGMSVAEXGXR, GMAVSEXGXR, GMVVDRXGXS, DMVVEAXGXR, DMVVESXGXR, NMVVRSXGXH and MIVEEXGXL.

In certain embodiments useful for inducing the conversion of a neoplastic cell into a cell (any cell) of an eye retina cell lineage, PEP12 is a peptide of general formula PEP1-$AA^{17}$-PEP11; wherein $AA^{17}$ is selected from the group consisting of G, A, V, L, I, P, F, M, W, T and S (in particular is selected from the group consisting of M, I, L, V and T); wherein PEP1 is SPIN.

In certain embodiments useful for inducing the conversion of a neoplastic cell into a cell (any cell) of an eye retina cell lineage, PEP11 is a peptide with 3 amino acids of general formula $AA^{18}$-$AA^{19}$-$AA^{20}$; wherein $AA^{18}$ is selected from the group consisting of L, V, Q, A and R, in particular is L; wherein $AA^{19}$ is selected from the group consisting of F, W, H and Y, in particular is Y or F; wherein $AA^{20}$ is selected from the group consisting of L, F, Y, K, I, V and M, in particular is selected from the group consisting of L, F, Y, K, I and V. In one particular example, PEP11 is LYF.

The definitions of "PEP" pairs, quadruplets, hexaplets and heptuplet e.g. PEP1:PEP2, PEP12:PEP2, or PEP7:PEP5:PEP12:LINKER:PEP2:PEP6:PEP8, most particularly useful for inducing the conversion of a neoplastic cell into a cell (any cell) of an eye retina cell lineage, are as already defined herein to the extent that PEP1 to PEP12 are particularly useful for these applications. For instances:

In certain embodiments useful for inducing the conversion of a neoplastic cell into a cell (any cell) of an eye retina cell lineage, PEP3 is selected from the group consisting of VPT, APT, TPT, VPA and APV; PEP4 is selected from the group consisting of VVE, VVR, VVK, VAE, AVS, VVD and VEE; and wherein the pair PEP3:PEP4 is selected from the group consisting of VPT:VVE, APT:VVR, APT:VVK, VPT:VAE, VPT:AVS, TPT:VVD, VPA:VVE and APV:VEE.

In certain embodiments useful for inducing the conversion of a neoplastic cell into a cell (any cell) of an eye retina cell lineage, PEP5 is selected from the group consisting of VPTEL, APTKL, APTQL, VPTKL, TPTKM, VPARL, VPTRL and APVKT; PEP6 is selected from the group consisting of DMVVE, EMVVE, NMVVR, NMVVK, EGMSVAE, EGMAVS, GMVVD and DMIVEE; and wherein the pair PEP5:PEP6 is selected from the group consisting of VPTEL:DMVVE, VPTEL:EMVVE, APTKL:NMVVR, APTQL:NMVVR, APTKL:NMVVK, VPTKL:EGMSVAE, VPTKL:EGMAVS, TPTKM:GMVVD, VPARL:DMVVE, VPTRL:DMVVE, APTKL:NMVVR and APVKT:DMIVEE.

In certain embodiments useful for inducing the conversion of a neoplastic cell into a cell (any cell) of an eye retina cell lineage, PEP7 is selected from the group consisting of KIPKAXX, SIPKAXX, HVTKPTX, YVPKPXX, TVPKPXX, AVPKAXX, KVGKAXX, KASKAXX, GSAGPXX, AAPAXXS, STPPTXX, HVPKPXX and RVPSTXX; wherein PEP8 is selected from the group consisting of GXGXR, SXGXH, AXGXH, XGXR, EXGXR, RXGXS, AXGXR, SXGXR and XGXL; and wherein the pair PEP7:PEP8 is selected from the group consisting of KIPKAXX:GXGXR, SIPKAXX:GXGXR, HVTKPTX:SXGXH, YVPKPXX:SXGSH, TVPKPXX:AXGXH, AVPKAXX:AXGXH, KVGKAXX:XGXR, KASKAXX:EXGXR, GSAGPXX:RXGXS, AAPASXX:AXGXR, STPPTXX:SXGXR, HVPKPXX:SXGXH and RVPSTXX:XGXL.

In certain embodiments useful for inducing the conversion of a neoplastic cell into a cell (any cell) of an eye retina cell lineage, PEP9 is selected from the group consisting of KIPKAXXVPTEL, SIPKAXXVPTEL, HVTKPTXAPTKL, YVPKPXXAPTKL, TVPKPXXAPTQL, AVPKAXXAPTKL, KVGKAXXVPTKL, KASKAXXVPTKL, GSAGPXXTPTKL, AAPASXXVPARL, STPPTXXVPTRL, HVPKPXXAPTKL and RVPSTXXAPVKT; PEP10 is selected from the group consisting of DMVVEGXGXR, EMVVEGXGXR, NMVVRSXGXH, NMVVRAXGXH, NMVVKAXGXH, EGMSVAEXGXR, GMAVSEXGXR, GMVVDRXGXS, DMVVEAXGXR, DMVVESXGXR, NMVVRSXGXH and MIVEEXGXL; and wherein the pair PEP9:PEP10 is selected from the group consisting of KIPKAXXVPTEL:DMVVEGXGXR, SIPKAXXVPTEL:EMVVEGXGXR, HVTKPTXAPTKL:NMVVRSXGXH, YVPKPXXAPTKL:NMVVRSXGXH, TVPKPXXAPTQL:NMVVRAXGXH, AVPKAXXAPTKL:NMVVKAXGXH, KVGKAXXVPTKL:EGMSVAEXGXR, KASKAXXVPTKL:GMAVSEXGXR, GSAGPXXTPTKM:GMVVDRXGXS, AAPASXXVPARL:DMVVEAXGXR, STPPTXXVPTRL:DMVVESXGXR, HVPKPXXAPTKL:NMVVRSXGXH and RVPSTXXAPVKT:MIVEEXGXL.

In certain embodiments useful for inducing the conversion of a neoplastic cell into a cell (any cell) of an eye retina cell lineage, PEP5 is selected from the group consisting of VPTEL, APTKL, APTQL, VPTKL, VPARL, VPQAL, VSQDL and VPQDL; PEP6 is selected from the group consisting of DMVVE, EMVVE, NMVVR, NMVVK, EGMSVAE, GMAVS, DMVVE, MIVRS, MIVKS and MVVKS; and wherein the pair PEP5:PEP6 is selected from the group consisting

of VPTEL:DMVVE, VPTEL:EMVVE, APTKL:NMVVR, APTQL:NMVVR, APTKL:NMVVK, VPTKL:EGMSVAE, VPTKL:EGMAVS, TPTKM:GMVVD, VPARL:DMVVE, VPTRL:DMVVE, APTKL:NMVVR and APVKT:DMIVEE; and wherein the pair PEP5:PEP6 is not TPTKM:GMVVD when PEP1 is SPIN and PEP2 is YGKIP.

In certain embodiments useful for inducing the conversion of a neoplastic cell into a cell (any cell) of an eye retina cell lineage, PEP9 is selected from the group consisting of KIPKAXXVPTEL, SIPKAXXVPTEL, HVTKPTXAPTKL, YVPKPXXAPTKL, TVPKPXXAPTQL, AVPKAXXAPTKL, KVGKAXXVPTKL, KASKAXXVPTKL, GSAGPXXTPTKL, AAPASXXVPARL, STPPTXXVPTRL, HVPKPXXAPTKL and RVPSTXXAPVKT; PEP10 is selected from the group consisting of DMVVEGXGXR, EMVVEGXGXR, NMVVRSXGXH, NMVVRAXGXH, NMVVKAXGXH, EGMSVAEXGXR, GMAVSEXGXR, GMVVDRXGXS, DMVVEAXGXR, DMVVESXGXR, NMVVRSXGXH and MIVEEXGXL; and wherein the pair PEP9:PEP10 is selected from the group consisting of KIPKAXXVPTEL:DMVVEGXGXR, SIPKAXXVPTEL:EMVVEGXGXR, HVTKPTXAPTKL:NMVVRSXGXH, YVPKPXXAPTKL:NMVVRSXGXH, TVPKPXXAPTQL:NMVVRAXGXH, AVPKAXXAPTKL:NMVVKAXGXH, KVGKAXXVPTKL:EGMSVAEXGXR, KASKAXXVPTKL:GMAVSEXGXR, GSAGPXXTPTKM:GMVVDRXGXS, AAPASXXVPARL:DMVVEAXGXR, STPPTXXVPTRL:DMVVESXGXR, HVPKPXXAPTKL:NMVVRSXGXH and RVPSTXXAPVKT:MIVEEXGXL; and wherein PEP9:PEP10 is not GSAGPXXTPTKM:GMVVDRXGXS when PEP1 is SPIN and PEP2 is YGKIP.

In certain embodiments useful for inducing the conversion of a neoplastic cell into a cell (any cell) of an eye retina cell lineage, PEP1 is SPIN and PEP11 is LYF.

In one aspect, the present disclosure provides a GFR-binding compound, wherein said GFR-binding compound is a peptide, a variant or analog thereof, or a peptidomimetic as defined herein, with between 20 and 60 (in particular between 20 and 50, and more particularly, between 20 and 45) amino acids, comprising a peptide with four amino acids (PEP1) or a peptide with eight amino acids (PEP12), a peptide with five amino acids (PEP2), and optionally a pair of peptides selected form the group consisting of PEP3:PEP4, PEP3:PEP6, PEP3:PEP10, PEP5:PEP4, PEP9:PEP4, PEP5:PEP6, PEP5:PEP10, PEP9:PEP6 and PEP9:PEP10; wherein said GFR-binding compound does not comprise the pair of peptides TPTKM:GMVVD when PEP1 is SPIN and PEP2 is YGKIP; and, optionally, wherein the RMSD is 2.45Å or less.

In one aspect, the present disclosure provides a GFR-binding compound, wherein said GFR-binding compound is a peptide, a variant or analog thereof, or a peptidomimetic as defined herein, with between 20 and 60 (in particular between 20 and 50, and more particularly, between 20 and 45) amino acids, comprising a peptide with four amino acids (PEP1) or a peptide with eight amino acids (PEP12), a peptide with five amino acids (PEP2), and optionally a pair of peptides selected form the group consisting of PEP3:PEP4, PEP3:PEP6, PEP3:PEP10, PEP5:PEP4, PEP9:PEP4, PEP5:PEP6, PEP5:PEP10, PEP9:PEP6 and PEP9:PEP10; wherein said GFR-binding compound does not comprise the pair of peptides GSAGPXXTPTKM:GMVVDRXGXS when PEP1 is SPIN and PEP2 is YGKIP; and, optionally, wherein the RMSD is 2.45Å or less.

In certain embodiments useful for inducing the conversion of a neoplastic cell into a cell (any cell) of an eye retina cell lineage, said GFR-binding compound is a synthetic molecule as defined herein in the definition section.

In certain embodiments useful for inducing the conversion of a neoplastic cell into a cell (any cell) of an eye retina cell lineage, said GFR-binding compound is a synthetic peptide, or a variant or analog thereof, or a peptidomimetic.

*Renal cell lineage*

Certain embodiments of the invention are particularly useful for inducing the conversion of a neoplastic cell into a cell (any cell) of a renal cell lineage.

In certain embodiments useful for inducing the conversion of a neoplastic cell into a cell (any cell) of a renal cell lineage, PEP1 is SPIN.

In certain embodiments useful for inducing the conversion of a neoplastic cell into a cell (any cell) of a renal cell lineage, PEP2 is YGKIP.

In certain embodiments useful for inducing the conversion of a neoplastic cell into a cell (any cell) of a renal cell lineage, PEP3 is selected from the group consisting of VPT, APT, TPT, VPA and APV.

In certain embodiments useful for inducing the conversion of a neoplastic cell into a cell (any cell) of a renal cell lineage, PEP4 is selected from the group consisting of VVE, VVR, VVK, VAE, AVS, VVD and VEE.

In certain embodiments useful for inducing the conversion of a neoplastic cell into a cell (any cell) of a renal cell lineage, PEP5 is a peptide of general formula PEP3-$AA^{11}$-$AA^{12}$; wherein PEP3 is selected from the group consisting of VPT, APT, TPT, VPA and APV; wherein $AA^{11}$ is selected from the group consisting of E, K, Q, R, A, D, G and H, in particular is E, K, Q and R; wherein $AA^{12}$ is selected from the group consisting of L, M, T, E, Q and H, in particular is L, M or T. In one particular example, PEP5 is selected from the group consisting of VPTEL, APTKL, APTQL, VPTKL, TPTKM, VPARL, VPTRL and APVKT.

In certain embodiments useful for inducing the conversion of a neoplastic cell into a cell (any cell) of a renal cell lineage, PEP6 is a peptide of general formula $AA^{26}$-$AA^{27}$-$AA^{28}$-$AA^{29}$-PEP4; wherein PEP4 is selected from the

group consisting of VVE, VVR, VVK, VAE, AVS, VVD and VEE; wherein $AA^{26}$ is absent or selected from the group consisting of $AA^{III}$ amino acids, preferably absent or E; wherein $AA^{27}$ and $AA^{28}$ are independently selected from the group consisting of $AA^{III}$ and $AA^{V}$ amino acids, preferably $AA^{27}$ is selected from the group consisting of D, E, N, G and M, preferably $AA^{28}$ is M or I; wherein $AA^{29}$ is absent or selected from the group consisting of $AA^{II}$ amino acids, preferably absent or S. In one particular example, PEP6 is selected from the group consisting of DMVVE, EMVVE, NMVVR, NMVVK, EGMSVAE, EGMAVS, GMVVD and DMIVEE.

In certain embodiments useful for inducing the conversion of a neoplastic cell into a cell (any cell) of a renal cell lineage, PEP7 is an amino acid or a peptide with between two and seven amino acids of general formula $AA^1$-$AA^2$-$AA^3$-$AA^4$-$AA^5$-$AA^6$-$AA^7$; wherein $AA^1$, $AA^2$, $AA^3$, $AA^4$, and $AA^5$ are independently absent or $AA^I$ as defined herein; wherein $AA^6$ is absent or selected from the group consisting of S, T, C, E, Q, P and R, preferably is S or C; wherein $AA^7$ is absent or is selected from the group consisting of S, T, C, E, Q, P and R, preferably is S or C; and wherein at least one of $AA^1$, $AA^2$, $AA^3$, $AA^4$, $AA^5$, $AA^6$ or $AA^7$ is not absent. In one particular example, PEP7 is selected from the group consisting of KIPKAXX, SIPKAXX, HVTKPTX, YVPKPXX, TVPKPXX, AVPKAXX, KVGKAXX, KASKAXX, GSAGPXX, AAPAXXS, STPPTXX, HVPKPXX and RVPSTXX.

In certain embodiments useful for inducing the conversion of a neoplastic cell into a cell (any cell) of a renal cell lineage, PEP8 is an amino acid or a peptide with between two and six amino acids of general formula $AA^{33}$-$AA^{34}$-$AA^{35}$-$AA^{36}$-$AA^{37}$-$AA^{38}$; wherein $AA^{33}$ is absent or is selected from the group consisting of $AA^I$ amino acids at the exception of $AA^{VI}$ amino acids, in particular is absent or is selected from the group consisting of G, S, A, E and R; $AA^{34}$ is absent or is selected from the group consisting of $AA^{III}$ and $AA^{IV}$ amino acids, in particular is absent; wherein $AA^{35}$ is absent or is selected from the group consisting of $AA^{II}$ amino acids, preferably is S or C; wherein $AA^{36}$ is absent or is selected from the group consisting of $AA^{III}$ and $AA^{IV}$ amino acids; wherein $AA^{37}$ is absent or is selected from the group consisting of $AA^{II}$ amino acids, preferably is S or C; wherein $AA^{38}$ is absent or is selected from the group consisting of $AA^I$, in particular is selected from the group consisting of R, H, S and L; and wherein at least one of $AA^{33}$, $AA^{34}$, $AA^{35}$, $AA^{36}$, $AA^{37}$ or $AA^{38}$ is not absent. In one particular example, PEP8 is selected from the group consisting of GXGXR, SXGXH, AXGXH, XGXR, EXGXR, RXGXS, AXGXR, SXGXR and XGXL.

In certain embodiments useful for inducing the conversion of a neoplastic cell into a cell (any cell) of a renal cell lineage, PEP9 is a peptide of general formula $AA^1$-$AA^2$-$AA^3$-$AA^4$-$AA^5$-$AA^6$-$AA^7$-PEP5; wherein PEP5 is a peptide of formula PEP3-$AA^{11}$-$AA^{12}$; wherein PEP3 is selected from the group consisting of VPT, APT, TPT, VPA and APV; wherein $AA^{11}$ is selected from the group consisting of E, K, Q, R, A, D, G and H, in particular is E, K, Q and R; wherein $AA^{12}$ is selected from the group consisting of L, M, T, E, Q and H, in particular is L, M or T; wherein $AA^1$, $AA^2$, $AA^3$, $AA^4$, and $AA^5$ are independently absent or $AA^I$ as defined herein; wherein $AA^6$ is absent or selected from the group consisting of S, T, C, E, Q, P and R, preferably is S or C; wherein $AA^7$ is selected from the group consisting of S, T, C, E, Q, P and R, preferably is S or C. In one particular example, PEP9 is selected from the group consisting of KIPKAXXVPTEL, SIPKAXXVPTEL, HVTKPTXAPTKL, YVPKPXXAPTKL, TVPKPXXAPTQL, AVPKAXXAPTKL, KVGKAXXVPTKL, KASKAXXVPTKL, GSAGPXXTPTKL, AAPASXXVPARL, STPPTXXVPTRL, HVPKPXXAPTKL and RVPSTXXAPVKT.

In certain embodiments useful for inducing the conversion of a neoplastic cell into a cell (any cell) of a renal cell lineage, PEP10 is a peptide of general formula PEP6-$AA^{33}$-$AA^{34}$-$AA^{35}$-$AA^{36}$-$AA^{37}$-$AA^{38}$; wherein PEP6 is a peptide of formula $AA^{26}$-$AA^{27}$-$AA^{28}$-$AA^{29}$-PEP4; wherein PEP4 is selected from the group consisting of VVE, VVR, VVK, VAE, AVS, VVD and VEE; wherein $AA^{26}$ is absent or selected from the group consisting of $AA^{III}$ amino acids, preferably absent or E; wherein $AA^{27}$ and $AA^{28}$ are independently selected from the group consisting of $AA^{III}$ and $AA^{V}$ amino acids, preferably $AA^{27}$ is selected from the group consisting of D, E, N, G and M, preferably $AA^{28}$ is M or I; wherein $AA^{29}$ is absent or selected from the group consisting of $AA^{II}$ amino acids, preferably absent or S; wherein $AA^{33}$ is absent or is selected from the group consisting of $AA^I$ amino acids at the exception of $AA^{VI}$ amino acids, in particular is absent or is selected from the group consisting of G, S, A, E and R; $AA^{34}$ is absent or is selected from the group consisting of $AA^{III}$ and $AA^{IV}$ amino acids, in particular is absent; wherein $AA^{35}$ is absent or is selected from the group consisting of $AA^{II}$ amino acids, preferably is S or C; wherein $AA^{36}$ is absent or is selected from the group consisting of $AA^{III}$ and $AA^{IV}$ amino acids; wherein $AA^{37}$ is absent or is selected from the group consisting of $AA^{II}$ amino acids, preferably is S or C; wherein $AA^{38}$ is absent or is selected from the group consisting of $AA^I$, in particular is selected from the group consisting of R, H, S and L. In one particular example, PEP10 is selected from the group consisting of DMVVEGXGXR, EMVVEGXGXR, NMVVRSXGXH, NMVVRAXGXH, NMVVKAXGXH, EGMSVAEXGXR, GMAVSEXGXR, GMVVDRXGXS, DMVVEAXGXR, DMVVESXGXR, NMVVRSXGXH and MIVEEXGXL.

In certain embodiments useful for inducing the conversion of a neoplastic cell into a cell (any cell) of a renal cell lineage, PEP12 is a peptide of general formula PEP1-$AA^{17}$-PEP11; wherein $AA^{17}$ is selected from the group consisting of G, A, V, L, I, P, F, M, W, T and S (in particular is selected from the group consisting of M, I, L, V and T); wherein PEP1 is SPIN.

In certain embodiments useful for inducing the conversion of a neoplastic cell into a cell (any cell) of a renal cell lineage, PEP11 is a peptide with 3 amino acids of general formula $AA^{18}$-$AA^{19}$-$AA^{20}$; wherein $AA^{18}$ is selected from the group consisting of L, V, Q, A and R, in particular is L; wherein $AA^{19}$ is selected from the group consisting of F, W,

H and Y, in particular is Y or F; wherein $AA^{20}$ is selected from the group consisting of L, F, Y, K, I, V and M, in particular is selected from the group consisting of L, F, Y, K, I and V. In one particular example, PEP11 is LYF.

The definitions of "PEP" pairs, quadruplets, hexaplets and heptuplet e.g. PEP1:PEP2, PEP12:PEP2, or PEP7:PEP5:PEP12:LINKER:PEP2:PEP6:PEP8, most particularly useful for inducing the conversion of a neoplastic cell into a cell (any cell) of a renal cell lineage, are as already defined herein to the extent that PEP1 to PEP12 are particularly useful for these applications. For instances:

In certain embodiments useful for inducing the conversion of a neoplastic cell into a cell (any cell) of a renal cell lineage, PEP3 is selected from the group consisting of VPT, APT, TPT, VPA and APV; PEP4 is selected from the group consisting of VVE, VVR, VVK, VAE, AVS, VVD and VEE; and wherein the pair PEP3:PEP4 is selected from the group consisting of VPT:VVE, APT:VVR, APT:VVK, VPT:VAE, VPT:AVS, TPT:VVD, VPA:VVE and APV:VEE.

In certain embodiments useful for inducing the conversion of a neoplastic cell into a cell (any cell) of a renal cell lineage, PEP5 is selected from the group consisting of VPTEL, APTKL, APTQL, VPTKL, TPTKM, VPARL, VPTRL and APVKT; PEP6 is selected from the group consisting of DMVVE, EMVVE, NMVVR, NMVVK, EGMSVAE, EGMAVS, GMVVD and DMIVEE; and wherein the pair PEP5:PEP6 is selected from the group consisting of VPTEL:DMVVE, VPTEL:EMVVE, APTKL:NMVVR, APTQL:NMVVR, APTKL:NMVVK, VPTKL:EGMSVAE, VPTKL:EGMAVS, TPTKM:GMVVD, VPARL:DMVVE, VPTRL:DMVVE, APTKL:NMVVR and APVKT:DMIVEE.

In certain embodiments useful for inducing the conversion of a neoplastic cell into a cell (any cell) of a renal cell lineage, PEP7 is selected from the group consisting of KIPKAXX, SIPKAXX, HVTKPTX, YVPKPXX, TVPKPXX, AVPKAXX, KVGKAXX, KASKAXX, GSAGPXX, AAPAXXS, STPPTXX, HVPKPXX and RVPSTXX; wherein PEP8 is selected from the group consisting of GXGXR, SXGXH, AXGXH, XGXR, EXGXR, RXGXS, AXGXR, SXGXR and XGXL; and wherein the pair PEP7:PEP8 is selected from the group consisting of KIPKAXX:GXGXR, SIPKAXX:GXGXR, HVTKPTX:SXGXH, YVPKPXX:SXGXH, TVPKPXX:AXGXH, AVPKAXX:AXGXH, KVGKAXX:XGXR, KASKAXX:EXGXR, GSAGPXX:RXGXS, AAPASXX:AXGXR, STPPTXX:SXGXR, HVPKPXX:SXGXH and RVPSTXX:XGXL.

In certain embodiments useful for inducing the conversion of a neoplastic cell into a cell (any cell) of a renal cell lineage, PEP9 is selected from the group consisting of KIPKAXXVPTEL, SIPKAXXVPTEL, HVTKPTXAPTKL, YVPKPXXAPTKL, TVPKPXXAPTQL, AVPKAXXAPTKL, KVGKAXXVPTKL, KASKAXXVPTKL, GSAGPXXTPTKL, AAPASXXVPARL, STPPTXXVPTRL, HVPKPXXAPTKL and RVPSTXXAPVKT; PEP10 is selected from the group consisting of DMVVEGXGXR, EMVVEGXGXR, NMVVRSXGXH, NMVVRAXGXH, NMVVKAXGXH, EGMSVAEXGXR, GMAVSEXGXR, GMVVDRXGXS, DMVVEAXGXR, DMVVESXGXR, NMVVRSXGXH and MIVEEXGXL; and wherein the pair PEP9:PEP10 is selected from the group consisting of KIPKAXXVPTEL:DMVVEGXGXR, SIPKAXXVPTEL:EMVVEGXGXR, HVTKPTXAPTKL:NMVVRSXGXH, YVPKPXXAPTKL:NMVVRSXGXH, TVPKPXXAPTQL:NMVVRAXGXH, AVPKAXXAPTKL:NMVVKAXGXH, KVGKAXXVPTKL:EGMSVAEXGXR, KASKAXXVPTKL:GMAVSEXGXR, GSAGPXXTPTKM:GMVVDRXGXS, AAPASXXVPARL:DMVVEAXGXR, STPPTXXVPTRL:DMVVESXGXR, HVPKPXXAPTKL:NMVVRSXGXH and RVPSTXXAPVKT:MIVEEXGXL.

In certain embodiments useful for inducing the conversion of a neoplastic cell into a cell (any cell) of a renal cell lineage, PEP5 is selected from the group consisting of VPTEL, APTKL, APTQL, VPTKL, VPARL, VPQAL, VSQDL and VPQDL; PEP6 is selected from the group consisting of DMVVE, EMVVE, NMVVR, NMVVK, EGMSVAE, GMAVS, DMVVE, MIVRS, MIVKS and MVVKS; and wherein the pair PEP5:PEP6 is selected from the group consisting of VPTEL:DMVVE, VPTEL:EMVVE, APTKL:NMVVR, APTQL:NMVVR, APTKL:NMVVK, VPTKL:EGMSVAE, VPTKL:EGMAVS, TPTKM:GMVVD, VPARL:DMVVE, VPTRL:DMVVE, APTKL:NMVVR and APVKT:DMIVEE; and wherein the pair PEP5:PEP6 is not TPTKM:GMVVD when PEP1 is SPIN and PEP2 is YGKIP.

In certain embodiments useful for inducing the conversion of a neoplastic cell into a cell (any cell) of a renal cell lineage, PEP9 is selected from the group consisting of KIPKAXXVPTEL, SIPKAXXVPTEL, HVTKPTXAPTKL, YVPKPXXAPTKL, TVPKPXXAPTQL, AVPKAXXAPTKL, KVGKAXXVPTKL, KASKAXXVPTKL, GSAGPXXTPTKL, AAPASXXVPARL, STPPTXXVPTRL, HVPKPXXAPTKL and RVPSTXXAPVKT; PEP10 is selected from the group consisting of DMVVEGXGXR, EMVVEGXGXR, NMVVRSXGXH, NMVVRAXGXH, NMVVKAXGXH, EGMSVAEXGXR, GMAVSEXGXR, GMVVDRXGXS, DMVVEAXGXR, DMVVESXGXR, NMVVRSXGXH and MIVEEXGXL; and wherein the pair PEP9:PEP10 is selected from the group consisting of KIPKAXXVPTEL:DMVVEGXGXR, SIPKAXXVPTEL:EMVVEGXGXR, HVTKPTXAPTKL:NMVVRSXGXH, YVPKPXXAPTKL:NMVVRSXGXH, TVPKPXXAPTQL:NMVVRAXGXH, AVPKAXXAPTKL:NMVVKAXGXH, KVGKAXXVPTKL:EGMSVAEXGXR, KASKAXXVPTKL:GMAVSEXGXR, GSAGPXXTPTKM:GMVVDRXGXS, AAPASXXVPARL:DMVVEAXGXR, STPPTXXVPTRL:DMVVESXGXR, HVPKPXXAPTKL:NMVVRSXGXH and RVPSTXXAPVKT:MIVEEXGXL; and wherein PEP9:PEP10 is not GSAGPXXTPTKM:GMVVDRXGXS when PEP1 is SPIN and PEP2 is YGKIP.

In certain embodiments useful for inducing the conversion of a neoplastic cell into a cell (any cell) of a renal cell lineage, PEP1 is SPIN and PEP11 is LYF.

In one aspect, the present disclosure provides a GFR-binding compound, wherein said GFR-binding compound is a peptide, a variant or analog thereof, or a peptidomimetic as defined herein, with between 20 and 60 (in particular between 20 and 50, and more particularly, between 20 and 45) amino acids, comprising a peptide with four amino acids

(PEP1) or a peptide with eight amino acids (PEP12), a peptide with five amino acids (PEP2), and optionally a pair of peptides selected form the group consisting of PEP3:PEP4, PEP3:PEP6, PEP3:PEP10, PEP5:PEP4, PEP9:PEP4, PEP5:PEP6, PEP5:PEP10, PEP9:PEP6 and PEP9:PEP10; wherein said GFR-binding compound does not comprise the pair of peptides TPTKM:GMVVD when PEP1 is SPIN and PEP2 is YGKIP; and, optionally, wherein the RMSD is 2.45Å or less.

In one aspect, the present disclosure provides a GFR-binding compound, wherein said GFR-binding compound is a peptide, a variant or analog thereof, or a peptidomimetic as defined herein, with between 20 and 60 (in particular between 20 and 50, and more particularly, between 20 and 45) amino acids, comprising a peptide with four amino acids (PEP1) or a peptide with eight amino acids (PEP12), a peptide with five amino acids (PEP2), and optionally a pair of peptides selected form the group consisting of PEP3:PEP4, PEP3:PEP6, PEP3:PEP10, PEP5:PEP4, PEP9:PEP4, PEP5:PEP6, PEP5:PEP10, PEP9:PEP6 and PEP9:PEP10; wherein said GFR-binding compound does not comprise the pair of peptides GSAGPXXTPTKM:GMVVDRXGXS when PEP1 is SPIN and PEP2 is YGKIP; and, optionally, wherein the RMSD is 2.45Å or less.

In certain embodiments useful for inducing the conversion of a neoplastic cell into a cell (any cell) of a renal cell lineage, said GFR-binding compound is a synthetic molecule as defined herein in the definition section.

In certain embodiments useful for inducing the conversion of a neoplastic cell into a cell (any cell) of a renal cell lineage, said GFR-binding compound is a synthetic peptide, or a variant or analog thereof, or a peptidomimetic.

*Ligament and Tendon cell lineage*

Certain embodiments of the invention are particularly useful for inducing the conversion of a neoplastic cell into a cell (any cell) of the ligament and tendon cell lineage.

In certain embodiments useful for inducing the conversion of a neoplastic cell into a cell (any cell) of the ligament and tendon cell lineage, PEP1 is selected from the group consisting of NAIS, SPIS, EPLP and EPLT.

In certain embodiments useful for inducing the conversion of a neoplastic cell into a cell (any cell) of the ligament and tendon cell lineage, PEP2 is selected from the group consisting of LKKYR, YKQYE and EQLSN.

In certain embodiments useful for inducing the conversion of a neoplastic cell into a cell (any cell) of the ligament and tendon cell lineage, PEP3 is selected from the group consisting of VPT, APT, VPQ and VSQ.

In certain embodiments useful for inducing the conversion of a neoplastic cell into a cell (any cell) of the ligament and tendon cell lineage, PEP4 is selected from the group consisting of VVE, VVR, VRS, VKS and VVK.

In certain embodiments useful for inducing the conversion of a neoplastic cell into a cell (any cell) of the ligament and tendon cell lineage, PEP5 is a peptide of general formula PEP3-$AA^{11}$-$AA^{12}$; wherein PEP3 is selected from the group consisting of VPT, APT, VPQ and VSQ; wherein $AA^{11}$ is selected from the group consisting of E, K, Q, R, A, D, G and H, in particular is E, K, Q, R, A and D; wherein $AA^{12}$ is selected from the group consisting of L, M, T, E, Q and H, in particular is L. In one particular example, PEP5 is selected from the group consisting of VPTEL, APTKL, APTQL, VPTRL, VPQAL, VSQDL and VPQDL.

In certain embodiments useful for inducing the conversion of a neoplastic cell into a cell (any cell) of the ligament and tendon cell lineage, PEP6 is a peptide of general formula $AA^{26}$-$AA^{27}$-$AA^{28}$-$AA^{29}$-PEP4; wherein PEP4 is selected from the group consisting of VVE, VVR, VRS, VKS and VVK; wherein $AA^{26}$ is absent or selected from the group consisting of $AA^{III}$ amino acids, preferably is absent; wherein $AA^{27}$ and $AA^{28}$ are independently selected from the group consisting of $AA^{III}$ and $AA^{V}$ amino acids, preferably $AA^{27}$ is selected from the group consisting of D, E, N and M, preferably $AA^{28}$ is selected from the group consisting of M, I and V; wherein $AA^{29}$ is absent or selected from the group consisting of $AA^{II}$ amino acids, preferably is absent. In one particular example, PEP6 is selected from the group consisting of DMVVE, EMVVE, NMVVR, DMVVE, MIVRS, MIVKS and NMVVK.

In certain embodiments useful for inducing the conversion of a neoplastic cell into a cell (any cell) of the ligament and tendon cell lineage, PEP7 is an amino acid or a peptide with between two and seven amino acids of general formula $AA^{1}$-$AA^{2}$-$AA^{3}$-$AA^{4}$-$AA^{5}$-$AA^{6}$-$AA^{7}$; wherein $AA^{1}$, $AA^{2}$, $AA^{3}$, $AA^{4}$, and $AA^{5}$ are independently absent or $AA^{I}$ as defined herein; wherein $AA^{6}$ is absent or selected from the group consisting of S, T, C, E, Q, P and R, preferably is selected from the group consisting of T, S and C; wherein $AA^{7}$ is absent or is selected from the group consisting of S, T, C, E, Q, P and R, preferably S or C; and wherein at least one of $AA^{1}$, $AA^{2}$, $AA^{3}$, $AA^{4}$, $AA^{5}$, $AA^{6}$ or $AA^{7}$ is not absent. In one particular example, PEP7 is selected from the group consisting of KIPKAXX, SIPKAXX, HVTKPTX, YVPKPXX, TVPKPXX, STPPTXX, ASAAPXX and ASASPXX.

In certain embodiments useful for inducing the conversion of a neoplastic cell into a cell (any cell) of the ligament and tendon cell lineage, PEP8 is an amino acid or a peptide with between two and six amino acids of general formula $AA^{33}$-$AA^{34}$-$AA^{35}$-$AA^{36}$-$AA^{37}$-$AA^{38}$; wherein $AA^{33}$ is absent or is selected from the group consisting of $AA^{I}$ amino acids at the exception of $AA^{VI}$ amino acids, in particular is absent or is selected from the group consisting of G, S and A; $AA^{34}$ is absent or is selected from the group consisting of $AA^{III}$ and $AA^{IV}$ amino acids, in particular is absent; wherein $AA^{35}$ is absent or is selected from the group consisting of $AA^{II}$ amino acids, preferably is S or C; wherein $AA^{36}$ is absent or is

selected from the group consisting of $AA^{III}$ and $AA^{IV}$ amino acids, in particular is G or K; wherein $AA^{37}$ is absent or is selected from the group consisting of $AA^{II}$ amino acids, preferably is S or C; wherein $AA^{38}$ is absent or is selected from the group consisting of $AA^{I}$, in particular is selected from the group consisting of R, H and S; and wherein at least one of $AA^{33}$, $AA^{34}$, $AA^{35}$, $AA^{36}$, $AA^{37}$ or $AA^{38}$ is not absent. In one particular example, PEP8 is selected from the group consisting of GXGXR, SXGXH, AXGXH, SXGXR, XKXS and SXKXS.

In certain embodiments useful for inducing the conversion of a neoplastic cell into a cell (any cell) of the ligament and tendon cell lineage, PEP9 is a peptide of general formula $AA^{1}$-$AA^{2}$-$AA^{3}$-$AA^{4}$-$AA^{5}$-$AA^{6}$-$AA^{7}$-PEP5; wherein PEP5 is a peptide of formula PEP3-$AA^{11}$-$AA^{12}$; wherein PEP3 is selected from the group consisting of VPT, APT, VPQ and VSQ; wherein $AA^{11}$ is selected from the group consisting of E, K, Q, R, A, D, G and H, in particular is E, K, Q, R, A and D; wherein $AA^{12}$ is selected from the group consisting of L, M, T, E, Q and H, in particular is L; wherein $AA^{1}$, $AA^{2}$, $AA^{3}$, $AA^{4}$, and $AA^{5}$ are independently absent or $AA^{I}$ as defined herein; wherein $AA^{6}$ is absent or selected from the group consisting of S, T, C, E, Q, P and R, preferably is selected from the group consisting of T, S and C; wherein $AA^{7}$ is selected from the group consisting of S, T, C, E, Q, P and R, preferably S or C. In one particular example, PEP9 is selected from the group consisting of KIPKAXXVPTEL, SIPKAXXVPTEL, HVTKPTXAPTKL, YVPKPXXAPTKL, TVPKPXXAPTQL, STPPTXXVPTRL, ASAAPXXVPQAL, ASASPXXVSQDL and ASASPXXVPQDL.

In certain embodiments useful for inducing the conversion of a neoplastic cell into a cell (any cell) of the ligament and tendon cell lineage, PEP10 is a peptide of general formula PEP6-$AA^{33}$-$AA^{34}$-$AA^{35}$-$AA^{36}$-$AA^{37}$-$AA^{38}$; wherein PEP6 is a peptide of formula $AA^{26}$-$AA^{27}$-$AA^{28}$-$AA^{29}$-PEP4; wherein PEP4 is selected from the group consisting of VVE, VVR, VRS, VKS and VVK; wherein $AA^{26}$ is absent or selected from the group consisting of $AA^{III}$ amino acids, preferably is absent; wherein $AA^{27}$ and $AA^{28}$ are independently selected from the group consisting of $AA^{III}$ and $AA^{V}$ amino acids, preferably $AA^{27}$ is selected from the group consisting of D, E, N and M, preferably $AA^{28}$ is selected from the group consisting of M, I and V; wherein $AA^{29}$ is absent or selected from the group consisting of $AA^{II}$ amino acids, preferably is absent; wherein $AA^{33}$ is absent or is selected from the group consisting of $AA^{I}$ amino acids at the exception of $AA^{VI}$ amino acids, in particular is absent or is selected from the group consisting of G, S and A; $AA^{34}$ is absent or is selected from the group consisting of $AA^{III}$ and $AA^{IV}$ amino acids, in particular is absent; wherein $AA^{35}$ is absent or is selected from the group consisting of $AA^{II}$ amino acids, preferably is S or C; wherein $AA^{36}$ is absent or is selected from the group consisting of $AA^{III}$ and $AA^{IV}$ amino acids, in particular is G or K; wherein $AA^{37}$ is absent or is selected from the group consisting of $AA^{II}$ amino acids, preferably is S or C; wherein $AA^{38}$ is absent or is selected from the group consisting of $AA^{I}$, in particular is selected from the group consisting of R, H and S. In one particular example, PEP10 is selected from the group consisting of DMVVEGXGXR, EMVVEGXGXR, NMVVRSXGXH, NMVVRAXGXH, DMVVESXGXR, MIVRSXKXS, MIVKSXKXS and MVVKSXKXS.

In certain embodiments useful for inducing the conversion of a neoplastic cell into a cell (any cell) of the ligament and tendon cell lineage, PEP12 is a peptide of general formula PEP1-$AA^{17}$-PEP11; wherein $AA^{17}$ is selected from the group consisting of G, A, V, L, I, P, F, M, W, T and S (in particular is selected from the group consisting of M, I, L, V and T); wherein PEP1 is selected from the group consisting of NAIS, SPIS, EPLP and EPLT.

In certain embodiments useful for inducing the conversion of a neoplastic cell into a cell (any cell) of the ligament and tendon cell lineage, PEP11 is a peptide with 3 amino acids of general formula $AA^{18}$-$AA^{19}$-$AA^{20}$; wherein $AA^{18}$ is selected from the group consisting of L, V, Q, A and R, in particular is L or V; wherein $AA^{19}$ is selected from the group consisting of F, W, H and Y, in particular is Y or F; wherein $AA^{20}$ is selected from the group consisting of L, F, Y, K, I, V and M, in particular is selected from the group consisting of F, I and Y. In one particular example, PEP11 is selected from the group consisting of LYF, LFI, VYY and LYY.

The definitions of "PEP" pairs, quadruplets, hexaplets and heptuplet e.g. PEP1:PEP2, PEP12:PEP2, or PEP7:PEP5:PEP12:LINKER:PEP2:PEP6:PEP8, most particularly useful for inducing the conversion of a neoplastic cell into a cell (any cell) of the ligament and tendon cell lineage, are as already defined herein to the extent that PEP1 to PEP12 are particularly useful for these applications. For instances:

In certain embodiments useful for inducing the conversion of a neoplastic cell into a cell (any cell) of the ligament and tendon cell lineage, the pair PEP1:PEP2 is selected from the group consisting of NAIS:LKKYR, SPIS:YKQYE, EPLP:EQLSN and EPLT:EQLSN.

In certain embodiments useful for inducing the conversion of a neoplastic cell into a cell (any cell) of the ligament and tendon cell lineage, the pair PEP12:PEP2 is selected from the group consisting of NAIS-$AA^{17}$-LYF:LKKYR, SPIS-$AA^{17}$-LFI:YKQYE, EPLP-$AA^{17}$-VYY:EQLSN and EPLT-$AA^{17}$-LYY:EQLSN; wherein $AA^{17}$ is selected from the group consisting of G, A, V, L, I, P, F, M, W, T and S (in particular is selected from the group consisting of V, I and T).

In certain embodiments useful for inducing the conversion of a neoplastic cell into a cell (any cell) of the ligament and tendon cell lineage, PEP3 is selected from the group consisting of VPT, APT, VPQ and VSQ; PEP4 is selected from the group consisting of VVE, VVR, VRS, VKS and VVK; and wherein the pair PEP3:PEP4 is selected from the group consisting of VPT:VVE, APT:VVR, VPT:VVE, VPQ:VRS, VSQ:VKS and VPQ:VVK.

In certain embodiments useful for inducing the conversion of a neoplastic cell into a cell (any cell) of the ligament and tendon cell lineage, PEP5 is selected from the group consisting of VPTEL, APTKL, APTQL, VPTRL, VPQAL, VSQDL

and VPQDL; PEP6 is selected from the group consisting of DMVVE, EMVVE, NMVVR, DMVVE, MIVRS, MIVKS and NMVVK; and wherein the pair PEP5:PEP6 is selected from the group consisting of VPTEL:DMVVE, VPTEL:EMVVE, APTKL:NMVVR, APTQL:NMVVR, VPTRL:DMVVE, VPQAL:MIVRS, VSQDL:MIVKS and VPQDL:NMVVK.

In certain embodiments useful for inducing the conversion of a neoplastic cell into a cell (any cell) of the ligament and tendon cell lineage, PEP7 is selected from the group consisting of KIPKAXX, SIPKAXX, HVTKPTX, YVPKPXX, TVPKPXX, STPPTXX, ASAAPXX and ASASPXX; wherein PEP8 is selected from the group consisting of GXGXR, SXGXH, AXGXH, SXGXR, XKXS and SXKXS; and wherein the pair PEP7:PEP8 is selected from the group consisting of KIPKAXX:GXGXR, SIPKAXX:GXGXR, HVTKPTX:SXGXH, YVPKPXX:SXGXH, TVPKPXX:AXGXH, STPPTXX:SXGXR, ASAAPXX:XKXS and ASASPXX:XKXS.

In certain embodiments useful for inducing the conversion of a neoplastic cell into a cell (any cell) of the ligament and tendon cell lineage, PEP9 is selected from the group consisting of KIPKAXXVPTEL, SIPKAXXVPTEL, HVTKPTXAPTKL, YVPKPXXAPTKL, TVPKPXXAPTQL, STPPTXXVPTRL, ASAAPXXVPQAL, ASASPXXVSQDL and ASASPXXVPQDL; PEP10 is selected from the group consisting of DMVVEGXGXR, EMVVEGXGXR, NMVVRSXGXH, NMVVRAXGXH, DMVVESXGXR, MIVRSXKXS, MIVKSXKXS and MVVKSXKXS; and wherein the pair PEP9:PEP10 is selected from the group consisting of KIPKAXXVPTEL:DMVVEGXGXR, SIPKAXXVPTEL:EMVVEGXGXR, HVTKPTXAPTKL:NMVVRSXGXH, YVPKPXXAPTKL:NMVVRSXGXH, TVPKPXXAPTQL:NMVVRAXGXH, STPPTXXVPTRL:DMVVESXGXR, ASAAPXXVPQAL:MIVRSXKXS, ASASPXXVSQDL:MIVKSXKXS and ASASPXXVPQDL:MVVKSXKXS.

In certain embodiments useful for inducing the conversion of a neoplastic cell into a cell (any cell) of the ligament and tendon cell lineage, PEP5 is selected from the group consisting of VPTEL, APTKL, APTQL, VPTRL, VPQAL, VSQDL and VPQDL; PEP6 is selected from the group consisting of DMVVE, EMVVE, NMVVR, DMVVE, MIVRS, MIVKS and NMVVK; and wherein the pair PEP5:PEP6 is selected from the group consisting of VPTEL:DMVVE, VPTEL:EMVVE, APTKL:NMVVR, APTQL:NMVVR, VPTRL:DMVVE, VPQAL:MIVRS, VSQDL:MIVKS and VPQDL:NMVVK; wherein the pair PEP5:PEP6 is not APTQL:NMVVR when PEP1 is NAIS and PEP2 is LKKYR; wherein the pair PEP5:PEP6 is not VPTRL:DMVVE when PEP1 is SPIS and PEP2 is YKQYE; wherein the pair PEP5:PEP6 is not VPQAL:MIVRS when PEP1 is EPLP and PEP2 is EQLSN; wherein the pair PEP5:PEP6 is not VSQDL:MIVKS when PEP1 is EPLT and PEP2 is EQLSN; and wherein the pair PEP5:PEP6 is not VPQDL:NMVVK when PEP1 is EPLT and PEP2 is EQLSN.

In certain embodiments useful for inducing the conversion of a neoplastic cell into a cell (any cell) of the ligament and tendon cell lineage, PEP9 is selected from the group consisting of KIPKAXXVPTEL, SIPKAXXVPTEL, HVTKPTXAPTKL, YVPKPXXAPTKL, TVPKPXXAPTQL, STPPTXXVPTRL, ASAAPXXVPQAL, ASASPXXVSQDL and ASASPXXVPQDL; PEP10 is selected from the group consisting of DMVVEGXGXR, EMVVEGXGXR, NMVVRSXGXH, NMVVRAXGXH, DMVVESXGXR, MIVRSXKXS, MIVKSXKXS and MVVKSXKXS; and wherein the pair PEP9:PEP10 is selected from the group consisting of KIPKAXXVPTEL:DMVVEGXGXR, SIPKAXXVPTEL:EMVVEGXGXR, HVTKPTXAPTKL:NMVVRSXGXH, YVPKPXXAPTKL:NMVVRSXGXH, TVPKPXXAPTQL:NMVVRAXGXH, STPPTXXVPTRL:DMVVESXGXR, ASAAPXXVPQAL:MIVRSXKXS, ASASPXXVSQDL:MIVKSXKXS and ASASPXXVPQDL:MVVKSXKXS; wherein the pair PEP9:PEP10 is not TVPKPXXAPTQL:NMVVRAXGXH when PEP1 is NAIS and PEP2 is LKKYR; wherein the pair PEP9:PEP10 is not STPPTXXVPTRL:DMVVESXGXR when PEP1 is SPIS and PEP2 is YKQYE; wherein the pair PEP9:PEP10 is not ASAAPXXVPQAL:MIVRSXKXS when PEP1 is EPLP and PEP2 is EQLSN; wherein the pair PEP9:PEP10 is not ASASPXXVSQDL:MIVKSXKXS when PEP1 is EPLT and PEP2 is EQLSN; and wherein the pair PEP9:PEP10 is not ASASPXXVPQDL:MVVKSXKXS when PEP1 is EPLT and PEP2 is EQLSN.

In certain embodiments useful for inducing the conversion of a neoplastic cell into a cell (any cell) of the ligament and tendon cell lineage, PEP1 is selected from the group consisting of NAIS, SPIS, EPLP and EPLT ; PEP11 is selected from the group consisting of LYF, LFI, VYY and LYY; and the pair PEP1 :PEP11 is selected from the group consisting of NAIS:LYF, SPIS:LFI, EPLP:VYY and EPLT:LYY.

In one aspect, the present disclosure provides a GFR-binding compound, wherein said GFR-binding compound is a peptide, a variant or analog thereof, or a peptidomimetic as defined herein, with between 20 and 60 (in particular between 20 and 50, and more particularly, between 20 and 45) amino acids, comprising a peptide with four amino acids (PEP1) or a peptide with eight amino acids (PEP12), a peptide with five amino acids (PEP2), and optionally a pair of peptides selected form the group consisting of PEP3:PEP4, PEP3:PEP6, PEP3:PEP10, PEP5:PEP4, PEP9:PEP4, PEP5:PEP6, PEP5:PEP10, PEP9:PEP6 and PEP9:PEP10; wherein said GFR-binding compound does not comprise the pair of peptides APTQL:NMVVR when PEP1 is NAIS and PEP2 is LKKYR; wherein said GFR-binding compound does not comprise the pair of peptides VPTRL:DMVVE when PEP1 is SPIS and PEP2 is YKQYE; wherein said GFR-binding compound does not comprise the pair of peptides VPQAL:MIVRS when PEP1 is EPLP and PEP2 is EQLSN; wherein said GFR-binding compound does not comprise the pair of peptides VSQDL:MIVKS when PEP1 is EPLT and PEP2 is EQLSN; and wherein said GFR-binding compound does not comprise the pair of peptides VPQDL:NMVVK when PEP1 is EPLT and PEP2 is EQLSN; and, optionally, wherein the RMSD is 2.45Å or less.

In one aspect, the present disclosure provides a GFR-binding compound, wherein said GFR-binding compound is a peptide, a variant or analog thereof, or a peptidomimetic as defined herein, with between 20 and 60 (in particular between 20 and 50, and more particularly, between 20 and 45) amino acids, comprising a peptide with four amino acids

(PEP1) or a peptide with eight amino acids (PEP12), a peptide with five amino acids (PEP2), and optionally a pair of peptides selected form the group consisting of PEP3:PEP4, PEP3:PEP6, PEP3:PEP10, PEP5:PEP4, PEP9:PEP4, PEP5:PEP6, PEP5:PEP10, PEP9:PEP6 and PEP9:PEP10; wherein said GFR-binding compound does not comprise the pair of peptides TVPKPXXAPTQL:NMVVRAXGXH when PEP1 is NAIS and PEP2 is LKKYR; wherein said GFR-binding compound does not comprise the pair of peptides STPPTXXVPTRL:DMVVESXGXR when PEP1 is SPIS and PEP2 is YKQYE; wherein said GFR-binding compound does not comprise the pair of peptides ASAAPXXVPQAL:MIVRSXKXS when PEP1 is EPLP and PEP2 is EQLSN; wherein said GFR-binding compound does not comprise the pair of peptides ASASPXXVSQDL:MIVKSXKXS when PEP1 is EPLT and PEP2 is EQLSN; and wherein said GFR-binding compound does not comprise the pair of peptides ASASPXXVPQDL:MVVKSXKXS when PEP1 is EPLT and PEP2 is EQLSN; and, optionally, wherein the RMSD is 2.45Å or less.

In certain embodiments useful for inducing the conversion of a neoplastic cell into a cell (any cell) of the ligament and tendon cell lineage, said GFR-binding compound is a synthetic molecule as defined herein in the definition section.

In certain embodiments useful for inducing the conversion of a neoplastic cell into a cell (any cell) of the ligament and tendon cell lineage, said GFR-binding compound is a synthetic peptide, or a variant or analog thereof, or a peptidomimetic.

In other embodiments also useful for inducing the conversion of a neoplastic cell into a cell (any cell) of the ligament and tendon cell lineage, PEP1 is SPIS.

In other embodiments also useful for inducing the conversion of a neoplastic cell into a cell (any cell) of the ligament and tendon cell lineage, PEP2 is YKQYE.

In other embodiments also useful for inducing the conversion of a neoplastic cell into a cell (any cell) of the ligament and tendon cell lineage, PEP3 is selected from the group consisting of VPT, APT, TPT, VPA and APV.

In other embodiments also useful for inducing the conversion of a neoplastic cell into a cell (any cell) of the ligament and tendon cell lineage, PEP4 is selected from the group consisting of VVE, VVR, VVK, VAE, AVS, VVD and VEE.

In other embodiments also useful for inducing the conversion of a neoplastic cell into a cell (any cell) of the ligament and tendon cell lineage, PEP5 is a peptide of general formula PEP3-$AA^{11}$-$AA^{12}$; wherein PEP3 is selected from the group consisting of VPT, APT, TPT, VPA and APV; wherein $AA^{11}$ is selected from the group consisting of E, K, Q, R, A, D, G and H, in particular is selected from the group consisting of E, K, Q and R; wherein $AA^{12}$ is selected from the group consisting of L, M, T, E, Q and H, in particular is selected from the group consisting of L, M and T. In one particular example, PEP5 is selected from the group consisting of VPTEL, APTKL, APTQL, VPTKL, TPTKM, VPARL, VPTRL and APVKT.

In other embodiments also useful for inducing the conversion of a neoplastic cell into a cell (any cell) of the ligament and tendon cell lineage, PEP6 is a peptide of general formula $AA^{26}$-$AA^{27}$-$AA^{28}$-$AA^{29}$-PEP4; wherein PEP4 is selected from the group consisting of VVE, VVR, VVK, VAE, AVS, VVD and VEE; wherein $AA^{26}$ is absent or selected from the group consisting of $AA^{III}$ amino acids, preferably absent or E; wherein $AA^{27}$ and $AA^{28}$ are independently selected from the group consisting of $AA^{III}$ and $AA^{V}$ amino acids, preferably $AA^{27}$ is selected from the group consisting of D, E, N, G and M, preferably $AA^{28}$ is M or I; wherein $AA^{29}$ is absent or selected from the group consisting of $AA^{II}$ amino acids, preferably absent or S. In one particular example, PEP6 is selected from the group consisting of DMVVE, EMVVE, NMVVR, NMVVK, EGMSVAE, EGMAVS, GMVVD and DMIVEE.

In other embodiments also useful for inducing the conversion of a neoplastic cell into a cell (any cell) of the ligament and tendon cell lineage, PEP7 is an amino acid or a peptide with between two and seven amino acids of general formula $AA^1$-$AA^2$-$AA^3$-$AA^4$-$AA^5$-$AA^6$-$AA^7$; wherein $AA^1$, $AA^2$, $AA^3$, $AA^4$, and $AA^5$ are independently absent or $AA^I$ as defined herein; wherein $AA^6$ is absent or selected from the group consisting of S, T, C, E, Q, P and R, preferably is S or C; wherein $AA^7$ is absent or is selected from the group consisting of S, T, C, E, Q, P and R, preferably is S or C; and wherein at least one of $AA^1$, $AA^2$, $AA^3$, $AA^4$, $AA^5$, $AA^6$ or $AA^7$ is not absent. In one particular example, PEP7 is selected from the group consisting of KIPKAXX, SIPKAXX, HVTKPTX, YVPKPXX, TVPKPXX, AVPKAXX, KVGKAXX, KASKAXX, GSAGPXX, AAPAXXS, STPPTXX, HVPKPXX and RVPSTXX.

In other embodiments also useful for inducing the conversion of a neoplastic cell into a cell (any cell) of the ligament and tendon cell lineage, PEP8 is an amino acid or a peptide with between two and six amino acids of general formula $AA^{33}$-$AA^{34}$-$AA^{35}$-$AA^{36}$-$AA^{37}$-$AA^{38}$; wherein $AA^{33}$ is absent or is selected from the group consisting of $AA^I$ amino acids at the exception of $AA^{VI}$ amino acids, in particular is absent or is selected from the group consisting of G, S, A, E and R; $AA^{34}$ is absent or is selected from the group consisting of $AA^{III}$ and $AA^{IV}$ amino acids, in particular is absent; wherein $AA^{35}$ is absent or is selected from the group consisting of $AA^{II}$ amino acids, preferably is S or C; wherein $AA^{36}$ is absent or is selected from the group consisting of $AA^{III}$ and $AA^{IV}$ amino acids; wherein $AA^{37}$ is absent or is selected from the group consisting of $AA^{II}$ amino acids, preferably is S or C; wherein $AA^{38}$ is absent or is selected from the group consisting of $AA^I$, in particular is selected from the group consisting of R, H, S and L; and wherein at least one of $AA^{33}$, $AA^{34}$, $AA^{31}$, $AA^{36}$, $AA^{37}$ or $AA^{38}$ is not absent. In one particular example, PEP8 is selected from the group consisting of GXGXR, SXGXH, AXGXH, XGXR, EXGXR, RXGXS, AXGXR, SXGXR and XGXL.

In other embodiments also useful for inducing the conversion of a neoplastic cell into a cell (any cell) of the

ligament and tendon cell lineage, PEP9 is a peptide of general formula $AA^1$-$AA^2$-$AA^3$-$AA^4$-$AA^5$-$AA^6$-$AA^7$-PEP5; wherein PEP5 is a peptide of formula PEP3-$AA^{11}$-$AA^{12}$; wherein PEP3 is selected from the group consisting of VPT, APT, TPT, VPA and APV; wherein $AA^{11}$ is selected from the group consisting of E, K, Q, R, A, D, G and H, in particular is E, K, Q and R; wherein $AA^{12}$ is selected from the group consisting of L, M, T, E, Q and H, in particular is L, M or T; wherein $AA^1$, $AA^2$, $AA^3$, $AA^4$, and $AA^5$ are independently absent or $AA^I$ as defined herein; wherein $AA^6$ is absent or selected from the group consisting of S, T, C, E, Q, P and R, preferably is S or C; wherein $AA^7$ is selected from the group consisting of S, T, C, E, Q, P and R, preferably is S or C. In one particular example, PEP9 is selected from the group consisting of KIPKAXXVPTEL, SIPKAXXVPTEL, HVTKPTXAPTKL, YVPKPXXAPTKL, TVPKPXXAPTQL, AVPKAXXAPTKL, KVGKAXXVPTKL, KASKAXXVPTKL, GSAGPXXTPTKL, AAPASXXVPARL, STPPTXXVPTRL, HVPKPXXAPTKL and RVPSTXXAPVKT.

In other embodiments also useful for inducing the conversion of a neoplastic cell into a cell (any cell) of the ligament and tendon cell lineage, PEP10 is a peptide of general formula PEP6-$AA^{33}$-$AA^{34}$-$AA^{35}$-$AA^{36}$-$AA^{37}$-$AA^{38}$; wherein PEP6 is a peptide of formula $AA^{26}$-$AA^{27}$-$AA^{28}$-$AA^{29}$-PEP4; wherein PEP4 is selected from the group consisting of VVE, VVR, VVK, VAE, AVS, VVD and VEE; wherein $AA^{26}$ is absent or selected from the group consisting of $AA^{III}$ amino acids, preferably absent or E; wherein $AA^{27}$ and $AA^{28}$ are independently selected from the group consisting of $AA^{III}$ and $AA^V$ amino acids, preferably $AA^{27}$ is selected from the group consisting of D, E, N, G and M, preferably $AA^{28}$ is M or I; wherein $AA^{29}$ is absent or selected from the group consisting of $AA^{II}$ amino acids, preferably absent or S; wherein $AA^{33}$ is absent or is selected from the group consisting of $AA^I$ amino acids at the exception of $AA^{VI}$ amino acids, in particular is absent or is selected from the group consisting of G, S, A, E and R; $AA^{34}$ is absent or is selected from the group consisting of $AA^{III}$ and $AA^{IV}$ amino acids, in particular is absent; wherein $AA^{35}$ is absent or is selected from the group consisting of $AA^{II}$ amino acids, preferably is S or C; wherein $AA^{36}$ is absent or is selected from the group consisting of $AA^{III}$ and $AA^{IV}$ amino acids; wherein $AA^{37}$ is absent or is selected from the group consisting of $AA^{II}$ amino acids, preferably is S or C; wherein $AA^{38}$ is absent or is selected from the group consisting of $AA^I$, in particular is selected from the group consisting of R, H, S and L. In one particular example, PEP10 is selected from the group consisting of DMVVEGXGXR, EMVVEGXGXR, NMVVRSXGXH, NMVVRAXGXH, NMVVKAXGXH, EGMSVAEXGXR, GMAVSEXGXR, GMVVDRXGXS, DMVVEAXGXR, DMVVESXGXR, NMVVRSXGXH and MIVEEXGXL.

In other embodiments also useful for inducing the conversion of a neoplastic cell into a cell (any cell) of the ligament and tendon cell lineage, PEP12 is a peptide of general formula PEP1-$AA^{17}$-PEP11; wherein $AA^{17}$ is selected from the group consisting of G, A, V, L, I, P, F, M, W, T and S (in particular is selected from the group consisting of M, I, L, V and T); wherein PEP1 is SPIS.

In other embodiments also useful for inducing the conversion of a neoplastic cell into a cell (any cell) of the ligament and tendon cell lineage, PEP11 is a peptide with 3 amino acids of general formula $AA^{18}$-$AA^{19}$-$AA^{20}$; wherein $AA^{18}$ is selected from the group consisting of L, V, Q, A and R, in particular is L; wherein $AA^{19}$ is selected from the group consisting of F, W, H and Y, in particular is a polar aromatic amino acid such as Y; wherein $AA^{20}$ is selected from the group consisting of L, F, Y, K, I, V and M, in particular is I. In one particular example, PEP11 is LYI.

The definitions of "PEP" pairs, quadruplets, hexaplets and heptuplet e.g. PEP1:PEP2, PEP12:PEP2, or PEP7:PEP5:PEP12:LINKER:PEP2:PEP6:PEP8, also useful for inducing the conversion of a neoplastic cell into a cell (any cell) of the ligament and tendon cell lineage, are as already defined herein to the extent that PEP1 to PEP12 are particularly useful for these applications. For instances:

In other embodiments also useful for inducing the conversion of a neoplastic cell into a cell (any cell) of the ligament and tendon cell lineage, PEP3 is selected from the group consisting of VPT, APT, TPT, VPA and APV; PEP4 is selected from the group consisting of VVE, VVR, VVK, VAE, AVS, VVD and VEE; and wherein the pair PEP3:PEP4 is selected from the group consisting of VPT:VVE, APT:VVR, APT:VVK, VPT:VAE, VPT:AVS, TPT:VVD, VPA:VVE and APV:VEE.

In other embodiments also useful for inducing the conversion of a neoplastic cell into a cell (any cell) of the ligament and tendon cell lineage, PEP5 is selected from the group consisting of VPTEL, APTKL, APTQL, VPTKL, TPTKM, VPARL, VPTRL and APVKT; PEP6 is selected from the group consisting of DMVVE, EMVVE, NMVVR, NMVVK, EGMSVAE, EGMAVS, GMVVD and DMIVEE; and wherein the pair PEP5:PEP6 is selected from the group consisting of VPTEL:DMVVE, VPTEL:EMVVE, APTKL:NMVVR, APTQL:NMVVR, APTKL:NMVVK, VPTKL:EGMSVAE, VPTKL:EGMAVS, TPTKM:GMVVD, VPARL:DMVVE, VPTRL:DMVVE, APTKL:NMVVR and APVKT:DMIVEE.

In other embodiments also useful for inducing the conversion of a neoplastic cell into a cell (any cell) of the ligament and tendon cell lineage, PEP7 is selected from the group consisting of KIPKAXX, SIPKAXX, HVTKPTX, YVPKPXX, TVPKPXX, AVPKAXX, KVGKAXX, KASKAXX, GSAGPXX, AAPAXXS, STPPTXX, HVPKPXX and RVPSTXX; wherein PEP8 is selected from the group consisting of GXGXR, SXGXH, AXGXH, XGXR, EXGXR, RXGXS, AXGXR, SXGXR and XGXL; and wherein the pair PEP7:PEP8 is selected from the group consisting of KIPKAXX:GXGXR, SIPKAXX:GXGXR, HVTKPTX:SXGXH, YVPKPXX:SXGXH, TVPKPXX:AXGXH, AVPKAXX:AXGXH, KVGKAXX:XGXR, KASKAXX:EXGXR, GSAGPXX:RXGXS, AAPASXX:AXGXR, STPPTXX:SXGXR, HVPKPXX:SXGXH and RVPSTXX:XGXL.

In other embodiments also useful for inducing the conversion of a neoplastic cell into a cell (any cell) of the

ligament and tendon cell lineage, PEP9 is selected from the group consisting of KIPKAXXVPTEL, SIPKAXXVPTEL, HVTKPTXAPTKL, YVPKPXXAPTKL, TVPKPXXAPTQL, AVPKAXXAPTKL, KVGKAXXVPTKL, KASKAXXVPTKL, GSAGPXXTPTKL, AAPASXXVPARL, STPPTXXVPTRL, HVPKPXXAPTKL and RVPSTXXAPVKT; PEP10 is selected from the group consisting of DMVVEGXGXR, EMVVEGXGXR, NMVVRSXGXH, NMVVRAXGXH, NMVVKAXGXH, EGMSVAEXGXR, GMAVSEXGXR, GMVVDRXGXS, DMVVEAXGXR, DMVVESXGXR, NMVVRSXGXH and MIVEEXGXL; and wherein the pair PEP9:PEP10 is selected from the group consisting of KIPKAXXVPTEL:DMVVEGXGXR, SIPKAXXVPTEL:EMVVEGXGXR, HVTKPTXAPTKL:NMVVRSXGXH, YVPKPXXAPTKL:NMVVRSXGXH, TVPKPXXAPTQL:NMVVRAXGXH, AVPKAXXAPTKL:NMVVKAXGXH, KVGKAXXVPTKL:EGMSVAEXGXR, KASKAXXVPTKL:GMAVSEXGXR, GSAGPXXTPTKM:GMVVDRXGXS, AAPASXXVPARL:DMVVEAXGXR, STPPTXXVPTRL:DMVVESXGXR, HVPKPXXAPTKL:NMVVRSXGXH and RVPSTXXAPVKT:MIVEEXGXL.

In other embodiments also useful for inducing the conversion of a neoplastic cell into a cell (any cell) of the ligament and tendon cell lineage, PEP5 is selected from the group consisting of VPTEL, APTKL, APTQL, VPTKL, VPARL, VPQAL, VSQDL and VPQDL; PEP6 is selected from the group consisting of DMVVE, EMVVE, NMVVR, NMVVK, EGMSVAE, GMAVS, DMVVE, MIVRS, MIVKS and MVVKS; and wherein the pair PEP5:PEP6 is selected from the group consisting of VPTEL:DMVVE, VPTEL:EMVVE, APTKL:NMVVR, APTQL:NMVVR, APTKL:NMVVK, VPTKL:EGMSVAE, VPTKL:EGMAVS, TPTKM:GMVVD, VPARL:DMVVE, VPTRL:DMVVE, APTKL:NMVVR and APVKT:DMIVEE; and wherein the pair PEP5:PEP6 is not VPARL:DMVVE when PEP1 is SPIS and PEP2 is YKQYE.

In other embodiments also useful for inducing the conversion of a neoplastic cell into a cell (any cell) of the ligament and tendon cell lineage, PEP9 is selected from the group consisting of KIPKAXXVPTEL, SIPKAXXVPTEL, HVTKPTXAPTKL, YVPKPXXAPTKL, TVPKPXXAPTQL, AVPKAXXAPTKL, KVGKAXXVPTKL, KASKAXXVPTKL, GSAGPXXTPTKL, AAPASXXVPARL, STPPTXXVPTRL, HVPKPXXAPTKL and RVPSTXXAPVKT; PEP10 is selected from the group consisting of DMVVEGXGXR, EMVVEGXGXR, NMVVRSXGXH, NMVVRAXGXH, NMVVKAXGXH, EGMSVAEXGXR, GMAVSEXGXR, GMVVDRXGXS, DMVVEAXGXR, DMVVESXGXR, NMVVRSXGXH and MIVEEXGXL; and wherein the pair PEP9:PEP10 is selected from the group consisting of KIPKAXXVPTEL:DMVVEGXGXR, SIPKAXXVPTEL:EMVVEGXGXR, HVTKPTXAPTKL:NMVVRSXGXH, YVPKPXXAPTKL:NMVVRSXGXH, TVPKPXXAPTQL:NMVVRAXGXH, AVPKAXXAPTKL:NMVVKAXGXH, KVGKAXXVPTKL:EGMSVAEXGXR, KASKAXXVPTKL:GMAVSEXGXR, GSAGPXXTPTKM:GMVVDRXGXS, AAPASXXVPARL:DMVVEAXGXR, STPPTXXVPTRL:DMVVESXGXR, HVPKPXXAPTKL:NMVVRSXGXH and RVPSTXXAPVKT:MIVEEXGXL ; and wherein PEP9:PEP10 is not AAPASXXVPARL:DMVVEAXGXR when PEP1 is SPIS and PEP2 is YKQYE.

In other embodiments also useful for inducing the conversion of a neoplastic cell into a cell (any cell) of the ligament and tendon cell lineage, PEP1 is SPIS and PEP11 is LYI.

In one aspect, the present disclosure provides a GFR-binding compound, wherein said GFR-binding compound is a peptide, a variant or analog thereof, or a peptidomimetic as defined herein, with between 20 and 60 (in particular between 20 and 50, and more particularly, between 20 and 45) amino acids, comprising a peptide with four amino acids (PEP1) or a peptide with eight amino acids (PEP12), a peptide with five amino acids (PEP2), and optionally a pair of peptides selected form the group consisting of PEP3:PEP4, PEP3:PEP6, PEP3:PEP10, PEP5:PEP4, PEP9:PEP4, PEP5:PEP6, PEP5:PEP10, PEP9:PEP6 and PEP9:PEP10; wherein said GFR-binding compound does not comprise the pair of peptides VPARL:DMVVE when PEP1 is SPIS and PEP2 is YKQYE; and, optionally, wherein the RMSD is 2.45Å or less.

In one aspect, the present disclosure provides a GFR-binding compound, wherein said GFR-binding compound is a peptide, a variant or analog thereof, or a peptidomimetic as defined herein, with between 20 and 60 (in particular between 20 and 50, and more particularly, between 20 and 45) amino acids, comprising a peptide with four amino acids (PEP1) or a peptide with eight amino acids (PEP12), a peptide with five amino acids (PEP2), and optionally a pair of peptides selected form the group consisting of PEP3:PEP4, PEP3:PEP6, PEP3:PEP10, PEP5:PEP4, PEP9:PEP4, PEP5:PEP6, PEP5:PEP10, PEP9:PEP6 and PEP9:PEP10; wherein said GFR-binding compound does not comprise the pair of peptides AAPASXXVPARL:DMVVEAXGXR when PEP1 is SPIS and PEP2 is YKQYE; and, optionally, wherein the RMSD is 2.45Å or less.

In other embodiments also useful for inducing the conversion of a neoplastic cell into a cell (any cell) of the ligament and tendon cell lineage, said GFR-binding compound is a synthetic molecule as defined herein in the definition section.

In other embodiments also useful for inducing the conversion of a neoplastic cell into a cell (any cell) of the ligament and tendon cell lineage, said GFR-binding compound is a synthetic peptide, or a variant or analog thereof, or a peptidomimetic.

*Fibroblast lineage*

Certain embodiments of the invention are particularly useful for inducing the conversion of a neoplastic cell into a cell (any cell) of a fibroblast lineage.

In certain embodiments useful for inducing the conversion of a neoplastic cell into a cell (any cell) of a fibroblast lineage, PEP1 is selected from the group consisting of EPLP, EPLT, SNIT, RSVK and RPVQ.

In certain embodiments useful for inducing the conversion of a neoplastic cell into a cell (any cell) of a fibroblast lineage, PEP2 is selected from the group consisting of EQLSN, IGEMS, KEVQV and KKATV.

In certain embodiments useful for inducing the conversion of a neoplastic cell into a cell (any cell) of a fibroblast lineage, PEP3 is selected from the group consisting of VPT, APT, VPQ, VSQ, SRV and TQV.

In certain embodiments useful for inducing the conversion of a neoplastic cell into a cell (any cell) of a fibroblast lineage, PEP4 is selected from the group consisting of VVE, VVR, VRS, VKS, VVK, QHN, EEH and EDH.

In certain embodiments useful for inducing the conversion of a neoplastic cell into a cell (any cell) of a fibroblast lineage, PEP5 is a peptide of general formula PEP3-$AA^{11}$-$AA^{12}$; wherein PEP3 is selected from the group consisting of VPT, APT, VPQ, VSQ, SRV and TQV; wherein $AA^{11}$ is selected from the group consisting of E, K, Q, R, A, D, G and H, in particular is selected from the group consisting of E, K, Q, A, D and H; wherein $AA^{12}$ is selected from the group consisting of L, M, T, E, Q and H, in particular is selected from the group consisting of L, E and H. In one particular example, PEP5 is selected from the group consisting of VPTEL, APTKL, APTQL, VPQAL, VSQDL, VPQDL, VPTEE, SRVHH and TQVQL.

In certain embodiments useful for inducing the conversion of a neoplastic cell into a cell (any cell) of a fibroblast lineage, PEP6 is a peptide of general formula $AA^{26}$-$AA^{27}$-$AA^{28}$-$AA^{29}$-PEP4; wherein PEP4 is selected from the group consisting of VVE, VVR, VRS, VKS, VVK, QHN, EEH and EDH; wherein $AA^{26}$ is absent or selected from the group consisting of $AA^{III}$ amino acids, preferably is absent; wherein $AA^{27}$ and $AA^{28}$ are independently selected from the group consisting of $AA^{III}$ and $AA^{V}$ amino acids, preferably $AA^{27}$ is selected from the group consisting of D, N, E, M, F, T and R, preferably $AA^{28}$ is selected from the group consisting of M, I, V and L; wherein $AA^{29}$ is absent or selected from the group consisting of $AA^{II}$ amino acids, preferably is absent. In one particular example, PEP6 is selected from the group consisting of DMVVE, EMVVE, NMVVR, MIVRS, MIVKS, NMVVK, FLQHN, RLEEH and TLEDH.

In certain embodiments useful for inducing the conversion of a neoplastic cell into a cell (any cell) of a fibroblast lineage, PEP7 is an amino acid or a peptide with between two and seven amino acids of general formula $AA^{1}$-$AA^{2}$-$AA^{3}$-$AA^{4}$-$AA^{5}$-$AA^{6}$-$AA^{7}$; wherein $AA^{1}$, $AA^{2}$, $AA^{3}$, $AA^{4}$, and $AA^{5}$ are independently absent or $AA^{I}$ as defined herein; wherein $AA^{6}$ is absent or selected from the group consisting of S, T, C, E, Q, P and R, preferably is selected from the group consisting of C, S, T, E, R and Q; wherein $AA^{7}$ is absent or is selected from the group consisting of S, T, C, E, Q, P and R, preferably is selected from the group consisting of S, C and P; and wherein at least one of $AA^{1}$, $AA^{2}$, $AA^{3}$, $AA^{4}$, $AA^{5}$, $AA^{6}$ or $AA^{7}$ is not absent. In one particular example, PEP7 is selected from the group consisting of KIPKAXX, SIPKAXX, HVTKPTX, YVPKPXX, TVPKPXX, ASAAPXX, ASASPXX, NDEGLEX, SSVKXQP and RNVQXRP.

In certain embodiments useful for inducing the conversion of a neoplastic cell into a cell (any cell) of a fibroblast lineage, PEP8 is an amino acid or a peptide with between two and six amino acids of general formula $AA^{33}$-$AA^{34}$-$AA^{35}$-$AA^{36}$-$AA^{37}$-$AA^{38}$; wherein $AA^{33}$ is absent or is selected from the group consisting of $AA^{I}$ amino acids at the exception of $AA^{VI}$ amino acids, in particular is absent or is selected from the group consisting of G, S, A, K and L; $AA^{34}$ is absent or is selected from the group consisting of $AA^{III}$ and $AA^{IV}$ amino acids, in particular is absent, A or E; wherein $AA^{35}$ is absent or is selected from the group consisting of $AA^{II}$ amino acids, preferably is S or C; wherein $AA^{36}$ is absent or is selected from the group consisting of $AA^{III}$ and $AA^{IV}$ amino acids, in particular is selected from the group consisting of G, A, K and E; wherein $AA^{37}$ is absent or is selected from the group consisting of $AA^{II}$ amino acids, preferably is S or C; wherein $AA^{38}$ is absent or is selected from the group consisting of $AA^{I}$, in particular is selected from the group consisting of R, H, S, E and A; and wherein at least one of $AA^{33}$, $AA^{34}$, $AA^{35}$, $AA^{36}$, $AA^{37}$ or $AA^{38}$ is not absent. In one particular example, PEP8 is selected from the group consisting of GXGXR, SXGXH, AXGXH, XKXS, KXEXR, LEXAXA and LAXKXE.

In certain embodiments useful for inducing the conversion of a neoplastic cell into a cell (any cell) of a fibroblast lineage, PEP9 is a peptide of general formula $AA^{1}$-$AA^{2}$-$AA^{3}$-$AA^{4}$-$AA^{5}$-$AA^{6}$-$AA^{7}$-PEP5; wherein PEP5 is a peptide of formula PEP3-$AA^{11}$-$AA^{12}$; wherein PEP3 is selected from the group consisting of VPT, APT, VPQ, VSQ, SRV and TQV; wherein $AA^{11}$ is selected from the group consisting of E, K, Q, R, A, D, G and H, in particular is selected from the group consisting of E, K, Q, A, D and H; wherein $AA^{12}$ is selected from the group consisting of L, M, T, E, Q and H, in particular is selected from the group consisting of L, E and H; wherein $AA^{1}$, $AA^{2}$, $AA^{3}$, $AA^{4}$, and $AA^{5}$ are independently absent or $AA^{I}$ as defined herein; wherein $AA^{6}$ is absent or selected from the group consisting of S, T, C, E, Q, P and R, preferably is selected from the group consisting of C, S, T, E, R and Q; wherein $AA^{7}$ is selected from the group consisting of S, T, C, E, Q, P and R, preferably is selected from the group consisting of S, C and P. In one particular example, PEP9 is selected from the group consisting of KIPKAXXVPTEL, SIPKAXXVPTEL, HVTKPTXAPTKL, YVPKPXXAPTKL, TVPKPXXAPTQL, ASAAPXXVPQAL, ASASPXXVSQDL, ASASPXXVPQDL, NDEGLEXVPTEE, SSVKXQPSRVHH and RNVQXRPTQVQL.

In certain embodiments useful for inducing the conversion of a neoplastic cell into a cell (any cell) of a fibroblast lineage, PEP10 is a peptide of general formula PEP6-$AA^{33}$-$AA^{34}$-$AA^{35}$-$AA^{36}$-$AA^{37}$-$AA^{38}$; wherein PEP6 is a peptide of

formula $AA^{26}$-$AA^{27}$-$AA^{28}$-$AA^{29}$-PEP4; wherein PEP4 is selected from the group consisting of VVE, VVR, VRS, VKS, VVK, QHN, EEH and EDH; wherein $AA^{26}$ is absent or selected from the group consisting of $AA^{III}$ amino acids, preferably is absent; wherein $AA^{27}$ and $AA^{28}$ are independently selected from the group consisting of $AA^{III}$ and $AA^{V}$ amino acids, preferably $AA^{27}$ is selected from the group consisting of D, N, E, M, F, T and R, preferably $AA^{28}$ is selected from the group consisting of M, I, V and L; wherein $AA^{29}$ is absent or selected from the group consisting of $AA^{II}$ amino acids, preferably is absent; wherein $AA^{33}$ is absent or is selected from the group consisting of $AA^{I}$ amino acids at the exception of $AA^{VI}$ amino acids, in particular is absent or is selected from the group consisting of G, S, A, K and L; $AA^{34}$ is absent or is selected from the group consisting of $AA^{III}$ and $AA^{IV}$ amino acids, in particular is absent, A or E; wherein $AA^{35}$ is absent or is selected from the group consisting of $AA^{II}$ amino acids, preferably is S or C; wherein $AA^{36}$ is absent or is selected from the group consisting of $AA^{III}$ and $AA^{IV}$ amino acids, in particular is selected from the group consisting of G, A, K and E; wherein $AA^{37}$ is absent or is selected from the group consisting of $AA^{II}$ amino acids, preferably is S or C; wherein $AA^{38}$ is absent or is selected from the group consisting of $AA^{I}$, in particular is selected from the group consisting of R, H, S, E and A. In one particular example, PEP10 is selected from the group consisting of DMVVEGXGXR, EMVVEGXGXR, NMVVRSXGXH, NMVVRAXGXH, MIVRSXKXS, MIVKSXKXS, MVVKSXKXS, FLQHNKXEXR, RLEEHLEXAXA and TLEDHLAXKXE.

In certain embodiments useful for inducing the conversion of a neoplastic cell into a cell (any cell) of a fibroblast lineage, PEP12 is a peptide of general formula PEP1-$AA^{17}$-PEP11; wherein $AA^{17}$ is selected from the group consisting of G, A, V, L, I, P, F, M, W, T and S (in particular is selected from the group consisting of M, I, L, V and T); wherein PEP1 is selected from the group consisting of EPLP, EPLT, SNIT, RSVK and RPVQ.

In certain embodiments useful for inducing the conversion of a neoplastic cell into a cell (any cell) of a fibroblast lineage, PEP11 is a peptide with 3 amino acids of general formula $AA^{18}$-$AA^{19}$-$AA^{20}$; wherein $AA^{18}$ is selected from the group consisting of L, V, Q, A and R; wherein $AA^{19}$ is selected from the group consisting of F, W, H, Y, I and K, in particular is selected from the group consisting of Y, I and K; wherein $AA^{20}$ is selected from the group consisting of L, F, Y, K, I, V and M, in particular is selected from the group consisting of Y, M, V and I. In one particular example, PEP11 is selected from the group consisting of VYY, LYY, QIM, AKV and RKI.

The definitions of "PEP" pairs, quadruplets, hexaplets and heptuplet e.g. PEP1:PEP2, PEP12:PEP2, or PEP7:PEP5:PEP12:LINKER:PEP2:PEP6:PEP8, useful for inducing the conversion of a neoplastic cell into a cell (any cell) of a fibroblast lineage, are as already defined herein to the extent that PEP1 to PEP12 are particularly useful for these applications. For instances:

In certain embodiments useful for inducing the conversion of a neoplastic cell into a cell (any cell) of a fibroblast lineage, the pair PEP1:PEP2 is selected from the group consisting of EPLP:EQLSN, EPLT:EQLSN and SNIT:IGEMS.

In certain embodiments useful for inducing the conversion of a neoplastic cell into a cell (any cell) of a fibroblast lineage, the pair PEP12:PEP2 is selected from the group consisting of EPLP-$AA^{17}$- VYY:EQLSN, EPLT-$AA^{17}$-LYY:EQLSN and SNIT-$AA^{17}$-QIM:IGEMS; wherein $AA^{17}$ is selected from the group consisting of G, A, V, L, I, P, F, M, W, T and S (in particular is selected from the group consisting of M, I, V and T).

In certain embodiments useful for inducing the conversion of a neoplastic cell into a cell (any cell) of a fibroblast lineage, PEP3 is selected from the group consisting of VPT, APT, VPQ, VSQ, SRV and TQV; PEP4 is selected from the group consisting of VVE, VVR, VRS, VKS, VVK, QHN, EEH and EDH; and wherein the pair PEP3:PEP4 is selected from the group consisting of VPT:VVE, APT:VVR, VPQ:VRS, VSQ:VKS, VPQ:VVK, VPT:QHN, SRV:EEH and TQV:EDH.

In certain embodiments useful for inducing the conversion of a neoplastic cell into a cell (any cell) of a fibroblast lineage, PEP5 is selected from the group consisting of VPTEL, APTKL, APTQL, VPQAL, VSQDL, VPQDL, VPTEE, SRVHH and TQVQL; PEP6 is selected from the group consisting of DMVVE, EMVVE, NMVVR, MIVRS, MIVKS, NMVVK, FLQHN, RLEEH and TLEDH; and wherein the pair PEP5:PEP6 is selected from the group consisting of VPTEL:DMVVE, VPTEL:EMVVE, APTKL:NMVVR, APTQL:NMVVR, VPQAL:MIVRS, VSQDL:MIVKS, VPQDL:NMVVK, VPTEE:FLQHN, SRVHH:RLEEH and TQVQL:TLEDH.

In certain embodiments useful for inducing the conversion of a neoplastic cell into a cell (any cell) of a fibroblast lineage, PEP7 is selected from the group consisting of KIPKAXX, SIPKAXX, HVTKPTX, YVPKPXX, TVPKPXX, ASAAPXX, ASASPXX, NDEGLEX, SSVKXQP and RNVQXRP; wherein PEP8 is selected from the group consisting of GXGXR, SXGXH, AXGXH, XKXS, KXEXR, LEXAXA and LAXKXE; and wherein the pair PEP7:PEP8 is selected from the group consisting of KIPKAXX:GXGXR, SIPKAXX:GXGXR, HVTKPTX:SXGXH, YVPKPXX:SXGXH, TVPKPXX:AXGXH, ASAAPXX:XKXS, ASASPXX:XKXS, NDEGLEX:KXEXR, SSVKXQP:LEXAXA and RNVQXRP:LAXKXE.

In certain embodiments useful for inducing the conversion of a neoplastic cell into a cell (any cell) of a fibroblast lineage, PEP9 is selected from the group consisting of KIPKAXXVPTEL, SIPKAXXVPTEL, HVTKPTXAPTKL, YVPKPXXAPTKL, TVPKPXXAPTQL, ASAAPXXVPQAL, ASASPXXVSQDL, ASASPXXVPQDL, NDEGLEXVPTEE, SSVKXQPSRVHH and RNVQXRPTQVQL; PEP10 is selected from the group consisting of DMVVEGXGXR, EMVVEGXGXR, NMVVRSXGXH, NMVVRAXGXH, MIVRSXKXS, MIVKSXKXS, MVVKSXKXS, FLQHNKXEXR, RLEEHLEXAXA and TLEDHLAXKXE; and wherein the pair PEP9:PEP10 is selected from the group consisting of KIPKAXXVPTEL:DMVVEGXGXR, SIPKAXXVPTEL:EMVVEGXGXR, HVTKPTXAPTKL:NMVVRSXGXH, YVPKPXXAPTKL:NMVVRSXGXH, TVPKPXXAPTQL:NMVVRAXGXH, ASAAPXXVPQAL:MIVRSXKXS, ASASPXXVSQDL:MIVKSXKXS, ASASPXXVPQDL:MVVKSXKXS, NDEGLEXVPTEE:FLQHNKXEXR, SSVKXQPSRVHH:RLEEHLEXAXA and RNVQXRPTQVQL:TLEDHLAXKXE.

In certain embodiments useful for inducing the conversion of a neoplastic cell into a cell (any cell) of a fibroblast lineage, PEP5 is selected from the group consisting of VPTEL, APTKL, APTQL, VPQAL, VSQDL, VPQDL, VPTEE, SRVHH and TQVQL; PEP6 is selected from the group consisting of DMVVE, EMVVE, NMVVR, MIVRS, MIVKS, NMVVK, FLQHN, RLEEH and TLEDH; and wherein the pair PEP5:PEP6 is selected from the group consisting of VPTEL:DMVVE, VPTEL:EMVVE, APTKL:NMVVR, APTQL:NMVVR, VPQAL:MIVRS, VSQDL:MIVKS, VPQDL:NMVVK, VPTEE:FLQHN, SRVHH:RLEEH and TQVQL:TLEDH; wherein the pair PEP5:PEP6 is not VPQAL:MIVRS when PEP1 is EPLP and PEP2 is EQLSN; wherein the pair PEP5:PEP6 is not VSQDL:MIVKS when PEP1 is EPLT and PEP2 is EQLSN; wherein the pair PEP5:PEP6 is not VPQDL:MVVVK when PEP1 is EPLT and PEP2 is EQLSN; wherein the pair PEP5:PEP6 is not VPTEE:FLQHN when PEP1 is SNIT and PEP2 is IGEMS; wherein the pair PEP5:PEP6 is not SRVHH:RLEEH when PEP1 is RSVK and PEP2 is KEVQV; and wherein the pair PEP5:PEP6 is not TQVQL:TLEDH when PEP1 is RPVQ and PEP2 is KKATV.

In certain embodiments useful for inducing the conversion of a neoplastic cell into a cell (any cell) of a fibroblast lineage, PEP9 is selected from the group consisting of KIPKAXXVPTEL, SIPKAXXVPTEL, HVTKPTXAPTKL, YVPKPXXAPTKL, TVPKPXXAPTQL, ASAAPXXVPQAL, ASASPXXVSQDL, ASASPXXVPQDL, NDEGLEXVPTEE, SSVKXQPSRVHH and RNVQXRPTQVQL; PEP10 is selected from the group consisting of DMVVEGXGXR, EMVVEGXGXR, NMVVRSXGXH, NMVVRAXGXH, MIVRSXKXS, MIVKSXKXS, MVVKSXKXS, FLQHNKXEXR, RLEEHLEXAXA and TLEDHLAXKXE; and wherein the pair PEP9:PEP10 is selected from the group consisting of KIPKAXXVPTEL:DMVVEGXGXR, SIPKAXXVPTEL:EMVVEGXGXR, HVTKPTXAPTKL:NMVVRSXGXH, YVPKPXXAPTKL:NMVVRSXGXH, TVPKPXXAPTQL:NMVVRAXGXH, ASAAPXXVPQAL:MIVRSXKXS, ASASPXXVSQDL:MIVKSXKXS, ASASPXXVPQDL:MVVKSXKXS, NDEGLEXVPTEE:FLQHNKXEXR, SSVKXQPSRVHH:RLEEHLEXAXA and RNVQXRPTQVQL:TLEDHLAXKXE; wherein the pair PEP9:PEP10 is not ASAAPXXVPQAL:MIVRSXKXS when PEP1 is EPLP and PEP2 is EQLSN; wherein the pair PEP9:PEP10 is not ASASPXXVSQDL:MIVKSXKXS when PEP1 is EPLT and PEP2 is EQLSN; wherein the pair PEP9:PEP10 is not ASASPXXVPQDL:MVVKSXKXS when PEP1 is EPLT and PEP2 is EQLSN; wherein the pair PEP9:PEP10 is not NDEGLEXVPTEE:FLQHNKXEXR when PEP1 is SNIT and PEP2 is IGEMS; wherein the pair PEP9:PEP10 is not SSVKXQPSRVHH:RLEEHLEXAXA when PEP1 is RSVK and PEP2 is KEVQV; and wherein the pair PEP9:PEP10 is not RNVQXRPTQVQL:TLEDHLAXKXE when PEP1 is RPVQ and PEP2 is KKATV.

In certain embodiments useful for inducing the conversion of a neoplastic cell into a cell (any cell) of a fibroblast lineage, PEP1 is selected from the group consisting of EPLP, EPLT, SNIT, RSVK and RPVQ; PEP11 is selected from the group consisting of VYY, LYY, QIM, AKV and RKI; ; and the pair PEP1:PEP11 is selected from the group consisting of EPLP:VYY, EPLT:LYY, SNIT:QIM, RSVK:KEVQV and RPVQ:KKATV.

In one aspect, the present disclosure provides a GFR-binding compound, wherein said GFR-binding compound is a peptide, a variant or analog thereof, or a peptidomimetic as defined herein, with between 20 and 60 (in particular between 20 and 50, and more particularly, between 20 and 45) amino acids, comprising a peptide with four amino acids (PEP1) or a peptide with eight amino acids (PEP12), a peptide with five amino acids (PEP2), and optionally a pair of peptides selected form the group consisting of PEP3:PEP4, PEP3:PEP6, PEP3:PEP10, PEP5:PEP4, PEP9:PEP4, PEP5:PEP6, PEP5:PEP10, PEP9:PEP6 and PEP9:PEP10; wherein said GFR-binding compound does not comprise the pair of peptides VPQAL:MIVRS when PEP1 is EPLP and PEP2 is EQLSN; wherein said GFR-binding compound does not comprise the pair of peptides VSQDL:MIVKS when PEP1 is EPLT and PEP2 is EQLSN; wherein said GFR-binding compound does not comprise the pair of peptides VPQDL:MVVVK when PEP1 is EPLT and PEP2 is EQLSN; wherein said GFR-binding compound does not comprise the pair of peptides VPTEE:FLQHN when PEP1 is SNIT and PEP2 is IGEMS; wherein said GFR-binding compound does not comprise the pair of peptides SRVHH:RLEEH when PEP1 is RSVK and PEP2 is KEVQV; and wherein said GFR-binding compound does not comprise the pair of peptides TQVQL:TLEDH when PEP1 is RPVQ and PEP2 is KKATV; and, optionally, wherein the RMSD is 2.45Å or less.

In one aspect, the present disclosure provides a GFR-binding compound, wherein said GFR-binding compound is a peptide, a variant or analog thereof, or a peptidomimetic as defined herein, with between 20 and 60 (in particular between 20 and 50, and more particularly, between 20 and 45) amino acids, comprising a peptide with four amino acids (PEP1) or a peptide with eight amino acids (PEP12), a peptide with five amino acids (PEP2), and optionally a pair of peptides selected form the group consisting of PEP3:PEP4, PEP3:PEP6, PEP3:PEP10, PEP5:PEP4, PEP9:PEP4, PEP5:PEP6, PEP5:PEP10, PEP9:PEP6 and PEP9:PEP10; wherein said GFR-binding compound does not comprise the pair of peptides ASAAPXXVPQAL:MIVRSXKXS when PEP1 is EPLP and PEP2 is EQLSN; wherein said GFR-binding compound does not comprise the pair of peptides ASASPXXVSQDL:MIVKSXKXS when PEP1 is EPLT and PEP2 is EQLSN; wherein said GFR-binding compound does not comprise the pair of peptides ASASPXXVPQDL:MVVKSXKXS when PEP1 is EPLT and PEP2 is EQLSN; wherein said GFR-binding compound does not comprise the pair of peptides NDEGLEXVPTEE:FLQHNKXEXR when PEP1 is SNIT and PEP2 is IGEMS; wherein said GFR-binding

compound does not comprise the pair of peptides SSVKXQPSRVHH:RLEEHLEXAXA when PEP1 is RSVK and PEP2 is KEVQV; and wherein said GFR-binding compound does not comprise the pair of peptides RNVQXRPTQVQL:TLEDHLAXKXE when PEP1 is RPVQ and PEP2 is KKATV; and, optionally, wherein the RMSD is 2.45Å or less.

In certain embodiments useful for inducing the conversion of a neoplastic cell into a cell (any cell) of a fibroblast lineage, said GFR-binding compound is a synthetic molecule as defined herein in the definition section.

In certain embodiments useful for inducing the conversion of a neoplastic cell into a cell (any cell) of a fibroblast lineage, said GFR-binding compound is a synthetic peptide, or a variant or analog thereof, or a peptidomimetic.

*Reproduction system lineage*

Certain embodiments of the invention are particularly useful for inducing the conversion of a neoplastic cell into a cell (any cell) of the reproduction system lineage.

In certain embodiments useful for inducing the conversion of a neoplastic cell into a cell (any cell) of the reproduction system lineage, PEP1 is NAIS.

In certain embodiments useful for inducing the conversion of a neoplastic cell into a cell (any cell) of the reproduction system lineage, PEP2 is LKKYR.

In certain embodiments useful for inducing the conversion of a neoplastic cell into a cell (any cell) of the reproduction system lineage, PEP3 is selected from the group consisting of VPT, APT, TPT, VPA and APV.

In certain embodiments useful for inducing the conversion of a neoplastic cell into a cell (any cell) of the reproduction system lineage, PEP4 is selected from the group consisting of VVE, VVR, VVK, VAE, AVS, VVD and VEE.

In certain embodiments useful for inducing the conversion of a neoplastic cell into a cell (any cell) of the reproduction system lineage, PEP5 is a peptide of general formula PEP3-$AA^{11}$-$AA^{12}$; wherein PEP3 is selected from the group consisting of VPT, APT, TPT, VPA and APV; wherein $AA^{11}$ is selected from the group consisting of E, K, Q, R, A, D, G and H, in particular is selected from the group consisting of E, K, Q and R; wherein $AA^{12}$ is selected from the group consisting of L, M, T, E, Q and H, in particular is selected from the group consisting of L, M and T. In one particular example, PEP5 is selected from the group consisting of VPTEL, APTKL, APTQL, VPTKL, TPTKM, VPARL, VPTRL and APVKT.

In certain embodiments useful for inducing the conversion of a neoplastic cell into a cell (any cell) of the reproduction system lineage, PEP6 is a peptide of general formula $AA^{26}$-$AA^{27}$-$AA^{28}$-$AA^{29}$-PEP4; wherein PEP4 is selected from the group consisting of VVE, VVR, VRS, VKS, VVK, QHN, EEH and EDH; wherein $AA^{26}$ is absent or selected from the group consisting of $AA^{III}$ amino acids, preferably is absent or E; wherein $AA^{27}$ and $AA^{28}$ are independently selected from the group consisting of $AA^{III}$ and $AA^{V}$ amino acids, preferably $AA^{27}$ is selected from the group consisting of D, N, E, M and G, preferably $AA^{28}$ is M or I; wherein $AA^{29}$ is absent or selected from the group consisting of $AA^{II}$ amino acids, preferably is absent or S. In one particular example, PEP6 is selected from the group consisting of DMVVE, EMVVE, NMVVR, NMVVK, EGMSVAE, GMAVS, GMVVD and MIVEE.

In certain embodiments useful for inducing the conversion of a neoplastic cell into a cell (any cell) of the reproduction system lineage, PEP7 is an amino acid or a peptide with between two and seven amino acids of general formula $AA^{1}$-$AA^{2}$-$AA^{3}$-$AA^{4}$-$AA^{5}$-$AA^{6}$-$AA^{7}$; wherein $AA^{1}$, $AA^{2}$, $AA^{3}$, $AA^{4}$, and $AA^{5}$ are independently absent or $AA^{I}$ as defined herein; wherein $AA^{6}$ is absent or selected from the group consisting of S, T, C, E, Q, P and R, preferably is selected from the group consisting of C, S and T; wherein $AA^{7}$ is absent or is selected from the group consisting of S, T, C, E, Q, P and R, preferably is selected from the group consisting of S and C; and wherein at least one of $AA^{1}$, $AA^{2}$, $AA^{3}$, $AA^{4}$, $AA^{5}$, $AA^{6}$ or $AA^{7}$ is not absent. In one particular example, PEP7 is selected from the group consisting of KIPKAXX, SIPKAXX, HVTKPTX, YVPKPXX, TVPKPXX, AVPKAXX, KVGKAXX, KASKAXX, GSAGPXX, AAPASXX, STPPTXX, HVPKPXX and RVPSTXX.

In certain embodiments useful for inducing the conversion of a neoplastic cell into a cell (any cell) of the reproduction system lineage, PEP8 is an amino acid or a peptide with between two and six amino acids of general formula $AA^{33}$-$AA^{34}$-$AA^{35}$-$AA^{36}$-$AA^{37}$-$AA^{38}$; wherein $AA^{33}$ is absent or is selected from the group consisting of $AA^{I}$ amino acids at the exception of $AA^{VI}$ amino acids, in particular is absent or is selected from the group consisting of G, S, A, E and R; $AA^{34}$ is absent or is selected from the group consisting of $AA^{III}$ and $AA^{IV}$ amino acids, in particular is absent; wherein $AA^{35}$ is absent or is selected from the group consisting of $AA^{II}$ amino acids, preferably is S or C; wherein $AA^{36}$ is absent or is selected from the group consisting of $AA^{III}$ and $AA^{IV}$ amino acids, in particular is G; wherein $AA^{37}$ is absent or is selected from the group consisting of $AA^{II}$ amino acids, preferably is S or C; wherein $AA^{38}$ is absent or is selected from the group consisting of $AA^{I}$, in particular is selected from the group consisting of R, H, S and L; and wherein at least one of $AA^{33}$, $AA^{34}$, $AA^{35}$, $AA^{36}$, $AA^{37}$ or $AA^{38}$ is not absent. In one particular example, PEP8 is selected from the group consisting of GXGXR, SXGXH, AXGXH, XGXR, EXGXR, RXGXS, AXGXR, SXGXR and XGXL.

In certain embodiments useful for inducing the conversion of a neoplastic cell into a cell (any cell) of the reproduction system lineage, PEP9 is a peptide of general formula $AA^{1}$-$AA^{2}$-$AA^{3}$-$AA^{4}$-$AA^{5}$-$AA^{6}$-$AA^{7}$-PEP5; wherein PEP5 is a peptide of formula PEP3-$AA^{11}$-$AA^{12}$; wherein PEP3 is selected from the group consisting of VPT, APT, TPT,

VPA and APV; wherein $AA^{11}$ is selected from the group consisting of E, K, Q, R, A, D, G and H, in particular is selected from the group consisting of E, K, Q and R; wherein $AA^{12}$ is selected from the group consisting of L, M, T, E, Q and H, in particular is selected from the group consisting of L, M and T; wherein $AA^1$, $AA^2$, $AA^3$, $AA^4$, and $AA^5$ are independently absent or $AA^I$ as defined herein; wherein $AA^6$ is absent or selected from the group consisting of S, T, C, E, Q, P and R, preferably is selected from the group consisting of C, S and T; wherein $AA^7$ is selected from the group consisting of S, T, C, E, Q, P and R, preferably is selected from the group consisting of S and C. In one particular example, PEP9 is selected from the group consisting of KIPKAXXVPTEL, SIPKAXXVPTEL, HVTKPTXAPTKL, YVPKPXXAPTKL, TVPKPXXAPTQL, AVPKAXXAPTKL, KVGKAXXVPTKL, KASKAXXVPTKL, GSAGPXXTPTKM, AAPASXXVPARL, STPPTXXVPTRL, HVPKPXXAPTKL and RVPSTXXAPVKT.

In certain embodiments useful for inducing the conversion of a neoplastic cell into a cell (any cell) of the reproduction system lineage, PEP10 is a peptide of general formula PEP6-$AA^{33}$-$AA^{34}$-$AA^{35}$-$AA^{36}$-$AA^{37}$-$AA^{38}$; wherein PEP6 is a peptide of formula $AA^{26}$-$AA^{27}$-$AA^{28}$-$AA^{29}$-PEP4; wherein PEP4 is selected from the group consisting of VVE, VVR, VVK, VAE, AVS, VVD and VEE; wherein $AA^{26}$ is absent or selected from the group consisting of $AA^{III}$ amino acids, preferably is absent or E; wherein $AA^{27}$ and $AA^{28}$ are independently selected from the group consisting of $AA^{III}$ and $AA^V$ amino acids, preferably $AA^{27}$ is selected from the group consisting of D, N, E, M and G, preferably $AA^{28}$ is M or I; wherein $AA^{29}$ is absent or selected from the group consisting of $AA^{II}$ amino acids, preferably is absent or is S; wherein $AA^{33}$ is absent or is selected from the group consisting of $AA^I$ amino acids at the exception of $AA^{VI}$ amino acids, in particular is absent or is selected from the group consisting of G, S, A, E and R; $AA^{34}$ is absent or is selected from the group consisting of $AA^{III}$ and $AA^{IV}$ amino acids, in particular is absent; wherein $AA^{35}$ is absent or is selected from the group consisting of $AA^{II}$ amino acids, preferably is S or C; wherein $AA^{36}$ is absent or is selected from the group consisting of $AA^{III}$ and $AA^{IV}$ amino acids, in particular is G; wherein $AA^{37}$ is absent or is selected from the group consisting of $AA^{II}$ amino acids, preferably is S or C; wherein $AA^{38}$ is absent or is selected from the group consisting of $AA^I$, in particular is selected from the group consisting of R, H, S and L. In one particular example, PEP10 is selected from the group consisting of DMVVEGXGXR, EMVVEGXGXR, NMVVRSXGXH, NMVVRSXGXH, NMVVRAXGXH, NMVVKAXGXH, EGMSVAEXGXR, GMAVSEXGXR, GMVVDRXGXS, DMVVEAXGXR, DMVVESXGXR, NMVVRSXGXH and MIVEEXGXL.

In certain embodiments useful for inducing the conversion of a neoplastic cell into a cell (any cell) of the reproduction system lineage, PEP12 is a peptide of general formula PEP1-$AA^{17}$-PEP11; wherein $AA^{17}$ is selected from the group consisting of G, A, V, L, I, P, F, M, W, T and S (in particular is selected from the group consisting of M, I, L, V and T); wherein PEP1 is NAIS.

In certain embodiments useful for inducing the conversion of a neoplastic cell into a cell (any cell) of the reproduction system lineage, PEP11 is a peptide with 3 amino acids of general formula $AA^{18}$-$AA^{19}$-$AA^{20}$; wherein $AA^{18}$ is selected from the group consisting of L, V, Q, A and R, in particular is L; wherein $AA^{19}$ is selected from the group consisting of F, W, H, Y, I and K, in particular is Y; wherein $AA^{20}$ is selected from the group consisting of L, F, Y, K, I, V and M, in particular is F. In one particular example, PEP11 is LYF.

The definitions of "PEP" pairs, quadruplets, hexaplets and heptuplet e.g. PEP1:PEP2, PEP12:PEP2, or PEP7:PEP5:PEP12:LINKER:PEP2:PEP6:PEP8, useful for inducing the conversion of a neoplastic cell into a cell (any cell) of the reproduction system lineage, are as already defined herein to the extent that PEP1 to PEP12 are particularly useful for these applications. For instances:

In certain embodiments useful for inducing the conversion of a neoplastic cell into a cell (any cell) of the reproduction system lineage, PEP3 is selected from the group consisting of VPT, APT, TPT, VPA and APV; PEP4 is selected from the group consisting of VVE, VVR, VVK, VAE, AVS, VVD and VEE; and wherein the pair PEP3:PEP4 is selected from the group consisting of VPT:VVE, APT:VVR, APT:VVK, VPT:VAE, VPT:AVS, TPT:VVD, VPA:VVE and APV:VEE.

In certain embodiments useful for inducing the conversion of a neoplastic cell into a cell (any cell) of the reproduction system lineage, PEP5 is selected from the group consisting of VPTEL, APTKL, APTQL, VPTKL, TPTKM, VPARL, VPTRL and APVKT; PEP6 is selected from the group consisting of DMVVE, EMVVE, NMVVR, NMVVK, EGMSVAE, GMAVS, GMVVD and MIVEE; and wherein the pair PEP5:PEP6 is selected from the group consisting of VPTEL:DMVVE, VPTEL:EMVVE, APTKL:NMVVR, APTQL:NMVVR, APTKL:NMVVK, VPTKL:EGMSVAE, VPTKL:GMAVS, TPTKM:GMVVD, VPARL:DMVVE, VPTRL:DMVVE and APVKT:MIVEE.

In certain embodiments useful for inducing the conversion of a neoplastic cell into a cell (any cell) of the reproduction system lineage, PEP7 is selected from the group consisting of KIPKAXX, SIPKAXX, HVTKPTX, YVPKPXX, TVPKPXX, AVPKAXX, KVGKAXX, KASKAXX, GSAGPXX, AAPASXX, STPPTXX, HVPKPXX and RVPSTXX; wherein PEP8 is selected from the group consisting of GXGXR, SXGXH, AXGXH, XGXR, EXGXR, RXGXS, AXGXR, SXGXR and XGXL; and wherein the pair PEP7:PEP8 is selected from the group consisting of KIPKAXX:GXGXR, SIPKAXX:GXGXR, HVTKPTX:SXGXH, YVPKPXX:SXGXH, TVPKPXX:AXGXH, AVPKAXX:AXGXH, KVGKAXX:XGXR, KASKAXX:EXGXR, GSAGPXX:RXGXS, AAPASXX:AXGXR, STPPTXX:SXGXR, HVPKPXX:SXGXH and RVPSTXX:XGXL.

In certain embodiments useful for inducing the conversion of a neoplastic cell into a cell (any cell) of the reproduction system lineage, PEP9 is selected from the group consisting of KIPKAXXVPTEL, SIPKAXXVPTEL, HVTKPTXAPTKL, YVPKPXXAPTKL, TVPKPXXAPTQL, AVPKAXXAPTKL, KVGKAXXVPTKL, KASKAXXVPTKL, GSAGPXXTPTKM, AAPASXXVPARL, STPPTXXVPTRL, HVPKPXXAPTKL and RVPSTXXAPVKT; PEP10 is selected from the group consisting of DMVVEGXGXR, EMVVEGXGXR, NMVVRSXGXH, NMVVRSXGXH, NMVVRAXGXH, NMVVKAXGXH, EGMSVAEXGXR, GMAVSEXGXR, GMVVDRXGXS, DMVVEAXGXR, DMVVESXGXR, NMVVRSXGXH and MIVEEXGXL; and wherein the pair PEP9:PEP10 is selected from the group consisting of KIPKAXXVPTEL:DMVVEGXGXR, SIPKAXXVPTEL:EMVVEGXGXR, HVTKPTXAPTKL:NMVVRSXGXH, YVPKPXXAPTKL:NMVVRSXGXH, TVPKPXXAPTQL:NMVVRAXGXH, AVPKAXXAPTKL:NMVVKAXGXH, KVGKAXXVPTKL:EGMSVAEXGXR, KASKAXXVPTKL:GMAVSEXGXR, GSAGPXXTPTKM:GMVVDRXGXS, AAPASXXVPARL:DMVVEAXGXR, STPPTXXVPTRL:DMVVESXGXR, HVPKPXXAPTKL:NMVVRSXGXH and RVPSTXXAPVKT:MIVEEXGXL.

In certain embodiments useful for inducing the conversion of a neoplastic cell into a cell (any cell) of the reproduction system lineage, PEP5 is selected from the group consisting of VPTEL, APTKL, APTQL, VPTKL, TPTKM, VPARL, VPTRL and APVKT; PEP6 is selected from the group consisting of DMVVE, EMVVE, NMVVR, NMVVK, EGMSVAE, GMAVS, GMVVD and MIVEE; and wherein the pair PEP5:PEP6 is selected from the group consisting of VPTEL:DMVVE, VPTEL:EMVVE, APTKL:NMVVR, APTQL:NMVVR, APTKL:NMVVK, VPTKL:EGMSVAE, VPTKL:GMAVS, TPTKM:GMVVD, VPARL:DMVVE, VPTRL:DMVVE and APVKT:MIVEE; and wherein the pair PEP5:PEP6 is not APTKL:NMVVR when PEP1 is NAIS and PEP2 is LKKYR.

In certain embodiments useful for inducing the conversion of a neoplastic cell into a cell (any cell) of the reproduction system lineage, PEP9 is selected from the group consisting of KIPKAXXVPTEL, SIPKAXXVPTEL, HVTKPTXAPTKL, YVPKPXXAPTKL, TVPKPXXAPTQL, AVPKAXXAPTKL, KVGKAXXVPTKL, KASKAXXVPTKL, GSAGPXXTPTKM, AAPASXXVPARL, STPPTXXVPTRL, HVPKPXXAPTKL and RVPSTXXAPVKT; PEP10 is selected from the group consisting of DMVVEGXGXR, EMVVEGXGXR, NMVVRSXGXH, NMVVRSXGXH, NMVVRAXGXH, NMVVKAXGXH, EGMSVAEXGXR, GMAVSEXGXR, GMVVDRXGXS, DMVVEAXGXR, DMVVESXGXR, NMVVRSXGXH and MIVEEXGXL; and wherein the pair PEP9:PEP10 is selected from the group consisting of KIPKAXXVPTEL:DMVVEGXGXR, SIPKAXXVPTEL:EMVVEGXGXR, HVTKPTXAPTKL:NMVVRSXGXH, YVPKPXXAPTKL:NMVVRSXGXH, TVPKPXXAPTQL:NMVVRAXGXH, AVPKAXXAPTKL:NMVVKAXGXH, KVGKAXXVPTKL:EGMSVAEXGXR, KASKAXXVPTKL:GMAVSEXGXR, GSAGPXXTPTKM:GMVVDRXGXS, AAPASXXVPARL:DMVVEAXGXR, STPPTXXVPTRL:DMVVESXGXR, HVPKPXXAPTKL:NMVVRSXGXH and RVPSTXXAPVKT:MIVEEXGXL; and wherein the pair PEP9:PEP10 is not HVPKPXXAPTKL:NMVVRSXGXH when PEP1 is NAIS and PEP2 is LKKYR.

In certain embodiments useful for inducing the conversion of a neoplastic cell into a cell (any cell) of the reproduction system lineage, PEP1 is NAIS and PEP11 is LYF.

In one aspect, the present disclosure provides a GFR-binding compound, wherein said GFR-binding compound is a peptide, a variant or analog thereof, or a peptidomimetic as defined herein, with between 20 and 60 (in particular between 20 and 50, and more particularly, between 20 and 45) amino acids, comprising a peptide with four amino acids (PEP1) or a peptide with eight amino acids (PEP12), a peptide with five amino acids (PEP2), and optionally a pair of peptides selected form the group consisting of PEP3:PEP4, PEP3:PEP6, PEP3:PEP10, PEP5:PEP4, PEP9:PEP4, PEP5:PEP6, PEP5:PEP10, PEP9:PEP6 and PEP9:PEP10; wherein said GFR-binding compound does not comprise the pair of peptides APTKL:NMVVR when PEP1 is NAIS and PEP2 is LKKYR; and, optionally, wherein the RMSD is 2.45Å or less.

In one aspect, the present disclosure provides a GFR-binding compound, wherein said GFR-binding compound is a peptide, a variant or analog thereof, or a peptidomimetic as defined herein, with between 20 and 60 (in particular between 20 and 50, and more particularly, between 20 and 45) amino acids, comprising a peptide with four amino acids (PEP1) or a peptide with eight amino acids (PEP12), a peptide with five amino acids (PEP2), and optionally a pair of peptides selected form the group consisting of PEP3:PEP4, PEP3:PEP6, PEP3:PEP10, PEP5:PEP4, PEP9:PEP4, PEP5:PEP6, PEP5:PEP10, PEP9:PEP6 and PEP9:PEP10; wherein said GFR-binding compound does not comprise the pair of peptides HVPKPXXAPTKL:NMVVRSXGXH when PEP1 is NAIS and PEP2 is LKKYR; and, optionally, wherein the RMSD is 2.45Å or less.

In certain embodiments useful for inducing the conversion of a neoplastic cell into a cell (any cell) of the reproduction system lineage, said GFR-binding compound is a synthetic molecule as defined herein in the definition section.

In certain embodiments useful for inducing the conversion of a neoplastic cell into a cell (any cell) of the reproduction system lineage, said GFR-binding compound is a synthetic peptide, or a variant or analog thereof, or a peptidomimetic.

*Lung cell lineage*

Certain embodiments of the invention are particularly useful for inducing the conversion of a neoplastic cell into a cell (any cell) of the lung cell lineage.

In certain embodiments useful for inducing the conversion of a neoplastic cell into a cell (any cell) of the lung

cell lineage, PEP1 is selected from the group consisting of NAIS, SATS, SPIS, EPIS and SPIN.

In certain embodiments useful for inducing the conversion of a neoplastic cell into a cell (any cell) of the lung cell lineage, PEP2 is selected from the group consisting of LKKYR, LRKHR, LKYHY, KFKYE and YGKIP.

In certain embodiments useful for inducing the conversion of a neoplastic cell into a cell (any cell) of the lung cell lineage, PEP3 is selected from the group consisting of VPT, VPE, APT, TPT, VPA, APV, VPQ and VSQ.

In certain embodiments useful for inducing the conversion of a neoplastic cell into a cell (any cell) of the lung cell lineage, PEP4 is selected from the group consisting of VVE, TVE, VVR, VVK, VAE, AVS, VVD, VEE, VRS and VKS.

In certain embodiments useful for inducing the conversion of a neoplastic cell into a cell (any cell) of the lung cell lineage, PEP5 is a peptide of general formula PEP3-$AA^{11}$-$AA^{12}$; wherein PEP3 is selected from the group consisting of VPT, VPE, APT, TPT, VPA, APV, VPQ and VSQ; wherein $AA^{11}$ is selected from the group consisting of E, K, Q, R, A, D, G and H, in particular is selected from the group consisting of E, K, Q, R, A and D; wherein $AA^{12}$ is selected from the group consisting of L, M, T, E, Q and H, in particular selected from the group consisting of L, M and T. In one particular example, PEP5 is selected from the group consisting of VPTEL, VPEKM, APTKL, APTQL, VPTKL, TPTKM, VPARL, VPTRL, APVKT, VPQAL, VSQDL and VPQDL.

In certain embodiments useful for inducing the conversion of a neoplastic cell into a cell (any cell) of the lung cell lineage, PEP6 is a peptide of general formula $AA^{26}$-$AA^{27}$-$AA^{28}$-$AA^{29}$-PEP4; wherein PEP4 is selected from the group consisting of VVE, TVE, VVR, VVK, VAE, AVS, VVD, VEE, VRS and VKS; wherein $AA^{26}$ is absent or selected from the group consisting of $AA^{III}$ amino acids, preferably is absent or E; wherein $AA^{27}$ and $AA^{28}$ are independently selected from the group consisting of $AA^{III}$ and $AA^{V}$ amino acids, preferably $AA^{27}$ is selected from the group consisting of D, E, N, G and M, preferably $AA^{28}$ is selected from the group consisting of M, I and V; wherein $AA^{29}$ is absent or selected from the group consisting of $AA^{II}$ amino acids, preferably absent or S. In one particular example, PEP6 is selected from the group consisting of DMVVE, NMTVE, EMVVE, NMVVR, NMVVK, EGMSVAE, GMAVS, GMVVD, DMVVE, MIVEE, MIVRS, MIVKS and MVVKS.

In certain embodiments useful for inducing the conversion of a neoplastic cell into a cell (any cell) of the lung cell lineage, PEP7 is an amino acid or a peptide with between two and seven amino acids of general formula $AA^{1}$-$AA^{2}$-$AA^{3}$-$AA^{4}$-$AA^{5}$-$AA^{6}$-$AA^{7}$; wherein $AA^{1}$, $AA^{2}$, $AA^{3}$, $AA^{4}$, and $AA^{5}$ are independently absent or $AA^{I}$ as defined herein; wherein $AA^{6}$ is absent or selected from the group consisting of S, T, C, E, Q, P and R, preferably is selected from the group consisting of C, S and T; wherein $AA^{7}$ is absent or is selected from the group consisting of S, T, C, E, Q, P and R, preferably is C or S; and wherein at least one of $AA^{1}$, $AA^{2}$, $AA^{3}$, $AA^{4}$, $AA^{5}$, $AA^{6}$ or $AA^{7}$ is not absent. In one particular example, PEP7 is selected from the group consisting of KIPKAXX, GIPEPXX, SIPKAXX, HVTKPTX, YVPKPXX, TVPKPXX, AVPKAXX, KVGKAXX, KASKAXX, GSAGPXX, AAPASXX, STPPTXX, HVPKPXX, RVPSTXX, ASAAPXX and ASASPXX.

In certain embodiments useful for inducing the conversion of a neoplastic cell into a cell (any cell) of the lung cell lineage, PEP8 is an amino acid or a peptide with between two and six amino acids of general formula $AA^{33}$-$AA^{34}$-$AA^{35}$-$AA^{36}$-$AA^{37}$-$AA^{38}$; wherein $AA^{33}$ is absent or is selected from the group consisting of $AA^{I}$ amino acids at the exception of $AA^{VI}$ amino acids; $AA^{34}$ is absent or is selected from the group consisting of $AA^{III}$ and $AA^{IV}$ amino acids; wherein $AA^{35}$ is absent or is selected from the group consisting of $AA^{II}$ amino acids, preferably is S or C; wherein $AA^{36}$ is absent or is selected from the group consisting of $AA^{III}$ and $AA^{IV}$ amino acids; wherein $AA^{37}$ is absent or is selected from the group consisting of $AA^{II}$ amino acids, preferably is S or C; wherein $AA^{38}$ is absent or is selected from the group consisting of $AA^{I}$; and wherein at least one of $AA^{33}$, $AA^{34}$, $AA^{35}$, $AA^{36}$, $AA^{37}$ or $AA^{38}$ is not absent. In one particular example, PEP8 is selected from the group consisting of GXGXR, SXAXR, SXGXH, AXGXH, XGXR, EXGXR, RXGXS, AXGXR, SXGXR, XGXL and XKXS.

In certain embodiments useful for inducing the conversion of a neoplastic cell into a cell (any cell) of the lung cell lineage, PEP9 is a peptide of general formula $AA^{1}$-$AA^{2}$-$AA^{3}$-$AA^{4}$-$AA^{5}$-$AA^{6}$-$AA^{7}$-PEP5; wherein PEP5 is a peptide of formula PEP3-$AA^{11}$-$AA^{12}$; wherein PEP3 is selected from the group consisting of VPT, VPE, APT, TPT, VPA, APV, VPQ and VSQ; wherein $AA^{11}$ is selected from the group consisting of E, K, Q, R, A, D, G and H, in particular E, K, Q, R, A and D; wherein $AA^{12}$ is selected from the group consisting of L, M, T, E, Q and H, in particular selected from the group consisting of L, M and T; wherein $AA^{1}$, $AA^{2}$, $AA^{3}$, $AA^{4}$, and $AA^{5}$ are independently absent or $AA^{I}$ as defined herein; wherein $AA^{6}$ is absent or selected from the group consisting of S, T, C, E, Q, P and R, preferably is selected from the group consisting of C, S and T; wherein $AA^{7}$ is selected from the group consisting of S, T, C, E, Q, P and R, preferably is C or S. In one particular example, PEP9 is selected from the group consisting of KIPKAXXVPTEL, GIPEPXXVPEKM, SIPKAXXVPTEL, HVTKPTXAPTKL, YVPKPXXAPTKL, TVPKPXXAPTQL, AVPKAXXAPTKL, KVGKAXXVPTKL, KASKAXXVPTKL, GSAGPXXTPTKM, AAPASXXVPARL, STPPTXXVPTRL, HVPKPXXAPTKL, RVPSTXXAPVKT, ASAAPXXVPQAL, ASASPXXVSQDL and ASASPXXVPQDL.

In certain embodiments useful for inducing the conversion of a neoplastic cell into a cell (any cell) of the lung cell lineage, PEP10 is a peptide of general formula PEP6-$AA^{33}$-$AA^{34}$-$AA^{35}$-$AA^{36}$-$AA^{37}$-$AA^{38}$; wherein PEP6 is a peptide of formula $AA^{26}$-$AA^{27}$-$AA^{28}$-$AA^{29}$-PEP4; wherein PEP4 is selected from the group consisting of VVE, TVE, VVR, VVK, VAE, AVS, VVD, VEE, VRS and VKS; wherein $AA^{26}$ is absent or selected from the group consisting of $AA^{III}$ amino acids,

preferably is absent or E; wherein $AA^{27}$ and $AA^{28}$ are independently selected from the group consisting of $AA^{III}$ and $AA^{V}$ amino acids, preferably $AA^{27}$ is selected from the group consisting of D, E, N, G and M, preferably $AA^{28}$ is selected from the group consisting of M, I and V; wherein $AA^{29}$ is absent or selected from the group consisting of $AA^{II}$ amino acids, preferably absent or S; wherein $AA^{33}$ is absent or is selected from the group consisting of $AA^{I}$ amino acids at the exception of $AA^{VI}$ amino acids; $AA^{34}$ is absent or is selected from the group consisting of $AA^{III}$ and $AA^{IV}$ amino acids; wherein $AA^{35}$ is absent or is selected from the group consisting of $AA^{II}$ amino acids, preferably is S or C; wherein $AA^{36}$ is absent or is selected from the group consisting of $AA^{III}$ and $AA^{IV}$ amino acids; wherein $AA^{37}$ is absent or is selected from the group consisting of $AA^{II}$ amino acids, preferably is S or C; wherein $AA^{38}$ is absent or is selected from the group consisting of $AA^{I}$. In one particular example, PEP10 is selected from the group consisting of DMVVEGXGXR, NMTVESXAXR, EMVVEGXGXR, NMVVRSXGXH, NMVVRSXGXH, NMVVRAXGXH, NMVVKAXGXH, EGMSVAEXGXR, GMAVSEXGXR, GMVVDRXGXS, DMVVEAXGXR, DMVVESXGXR, NMVVRSXGXH, MIVEEXGXL, MIVRSXKXS, MIVKSXKXS and MVVKSXKXS.

In certain embodiments useful for inducing the conversion of a neoplastic cell into a cell (any cell) of the lung cell lineage, PEP12 is a peptide of general formula PEP1-$AA^{17}$-PEP11; wherein $AA^{17}$ is selected from the group consisting of G, A, V, L, I, P, F, M, W, T and S (in particular is selected from the group consisting of M, I, L, V and T); wherein PEP1 is selected from the group consisting of NAIS, SATS, SPIS, EPIS and SPIN.

In certain embodiments useful for inducing the conversion of a neoplastic cell into a cell (any cell) of the lung cell lineage, PEP11 is a peptide with 3 amino acids of general formula $AA^{18}$-$AA^{19}$-$AA^{20}$; wherein $AA^{18}$ is selected from the group consisting of L, V, Q, A and R, in particular is L; wherein $AA^{19}$ is selected from the group consisting of F, W, H and Y (in particular is a polar aromatic amino acid such as Y); wherein $AA^{20}$ is selected from the group consisting of L, F, Y, K, I, V and M, in particular is selected from the group consisting of L, F, Y, and K. In one particular example, PEP11 is selected from the group consisting of LYF, LYY, LYK and LYL.

The definitions of "PEP" pairs, quadruplets, hexaplets and heptuplet e.g. PEP1:PEP2, PEP12:PEP2, or PEP7:PEP5:PEP12:LINKER:PEP2:PEP6:PEP8, useful for inducing the conversion of a neoplastic cell into a cell (any cell) of the lung cell lineage, are as already defined herein to the extent that PEP1 to PEP12 are particularly useful for these applications. For instances:

In certain embodiments useful for inducing the conversion of a neoplastic cell into a cell (any cell) of the lung cell lineage, the pair PEP1:PEP2 is selected from the group consisting of NAIS:LKKYR, SATS:LRKHR, SPIS:LKYHY, EPIS:KFKYE and SPIN:YGKIP.

In certain embodiments useful for inducing the conversion of a neoplastic cell into a cell (any cell) of the lung cell lineage, the pair PEP12:PEP2 is selected from the group consisting of NAIS-$AA^{17}$-LYF:LKKYR, SATS-$AA^{17}$-LYY:LRKHR, SPIS-$AA^{17}$-LYK:LKYHY, EPIS-$AA^{17}$-LYL:KFKYE and SPIN-$AA^{17}$-LYF:YGKIP; wherein $AA^{17}$ is selected from the group consisting of G, A, V, L, I, P, F, M, W, T and S (in particular is selected from the group consisting of M, I, V and T).

In certain embodiments useful for inducing the conversion of a neoplastic cell into a cell (any cell) of the lung cell lineage, PEP3 is selected from the group consisting of VPT, VPE, APT, TPT, VPA, APV, VPQ and VSQ; PEP4 is selected from the group consisting of VVE, TVE, VVR, VVK, VAE, AVS, VVD, VEE, VRS and VKS; and wherein the pair PEP3:PEP4 is selected from the group consisting of VPT:VVE, VPE:TVE, APT:VVR, APT:VVK, VPT:VAE, VPT:AVS, TPT:VVD, VPA:VVE, APV:VEE, VPQ:VRS, VSQ:VKS and VPQ:VKS.

In certain embodiments useful for inducing the conversion of a neoplastic cell into a cell (any cell) of the lung cell lineage, PEP5 is selected from the group consisting of VPTEL, VPEKM, APTKL, APTQL, VPTKL, TPTKM, VPARL, VPTRL, APVKT, VPQAL, VSQDL and VPQDL; PEP6 is selected from the group consisting of DMVVE, NMTVE, EMVVE, NMVVR, NMVVK, EGMSVAE, GMAVS, GMVVD, DMVVE, MIVEE, MIVRS, MIVKS and MVVKS; and wherein the pair PEP5:PEP6 is selected from the group consisting of VPTEL:DMVVE, VPEKM:NMTVE, VPTEL:EMVVE, APTKL:NMVVR, APTQL:NMVVR, APTKL:NMVVK, VPTKL:EGMSVAE, VPTKL:GMAVS, TPTKM:GMVVD, VPARL:DMVVE, VPTRL:DMVVE, APVKT:MIVEE, VPQAL:MIVRS, VSQDL:MIVKS and VPQDL:MVVKS.

In certain embodiments useful for inducing the conversion of a neoplastic cell into a cell (any cell) of the lung cell lineage, PEP7 is selected from the group consisting of KIPKAXX, GIPEPXX, SIPKAXX, HVTKPTX, YVPKPXX, TVPKPXX, AVPKAXX, KVGKAXX, KASKAXX, GSAGPXX, AAPASXX, STPPTXX, HVPKPXX, RVPSTXX, ASAAPXX and ASASPXX; wherein PEP8 is selected from the group consisting of GXGXR, SXAXR, SXGXH, AXGXH, XGXR, EXGXR, RXGXS, AXGXR, SXGXR, XGXL and XKXS; and wherein the pair PEP7:PEP8 is selected from the group consisting of KIPKAXX:GXGXR, GIPEPXX:SXAXR, SIPKAXX:GXGXR, HVTKPTX:SXGXH, YVPKPXX:SXGXH, TVPKPXX:AXGXH, AVPKAXX:AXGXH, KVGKAXX:XGXR, KASKAXX:EXGXR, GSAGPXX:RXGXS, AAPASXX:AXGXR, STPPTXX:SXGXR, HVPKPXX:SXGXH, RVPSTXX:XGXL, ASAAPXX:XKXS and ASASPXX:XKXS.

In certain embodiments useful for inducing the conversion of a neoplastic cell into a cell (any cell) of the lung cell lineage, PEP9 is selected from the group consisting of KIPKAXXVPTEL, GIPEPXXVPEKM, SIPKAXXVPTEL, HVTKPTXAPTKL, YVPKPXXAPTKL, TVPKPXXAPTQL, AVPKAXXAPTKL, KVGKAXXVPTKL, KASKAXXVPTKL, GSAGPXXTPTKM, AAPASXXVPARL, STPPTXXVPTRL, HVPKPXXAPTKL, RVPSTXXAPVKT, ASAAPXXVPQAL,

ASASPXXVSQDL and ASASPXXVPQDL; PEP10 is selected from the group consisting of DMVVEGXGXR, NMTVESXAXR, EMVVEGXGXR, NMVVRSXGXH, NMVVRSXGXH, NMVVRAXGXH, NMVVKAXGXH, EGMSVAEXGXR, GMAVSEXGXR, GMVVDRXGXS, DMVVEAXGXR, DMVVESXGXR, NMVVRSXGXH, MIVEEXGXL, MIVRSXKXS, MIVKSXKXS and MVVKSXKXS; and wherein the pair PEP9:PEP10 is selected from the group consisting of KIPKAXXVPTEL:DMVVEGXGXR, GIPEPXXVPEKM:NMTVESXAXR, SIPKAXXVPTEL:EMVVEGXGXR, HVTKPTXAPTKL:NMVVRSXGXH, YVPKPXXAPTKL:NMVVRSXGXH, TVPKPXXAPTQL:NMVVRAXGXH, AVPKAXXAPTKL:NMVVKAXGXH, KVGKAXXVPTKL:EGMSVAEXGXR, KASKAXXVPTKL:GMAVSEXGXR, GSAGPXXTPTKM:GMVVDRXGXS, AAPASXXVPARL:DMVVEAXGXR, STPPTXXVPTRL:DMVVESXGXR, HVPKPXXAPTKL:NMVVRSXGXH, RVPSTXXAPVKT:MIVEEXGXL, ASAAPXXVPQAL:MIVRSXKXS, ASASPXXVSQDL:MIVKSXKXS and ASASPXXVPQDL:MVVKSXKXS.

In certain embodiments useful for inducing the conversion of a neoplastic cell into a cell (any cell) of the lung cell lineage, PEP5 is selected from the group consisting of VPTEL, VPEKM, APTKL, APTQL, VPTKL, TPTKM, VPARL, VPTRL, APVKT, VPQAL, VSQDL and VPQDL; PEP6 is selected from the group consisting of DMVVE, NMTVE, EMVVE, NMVVR, NMVVK, EGMSVAE, GMAVS, GMVVD, DMVVE, MIVEE, MIVRS, MIVKS and MVVKS; and wherein the pair PEP5:PEP6 is selected from the group consisting of VPTEL:DMVVE, VPEKM:NMTVE, VPTEL:EMVVE, APTKL:NMVVR, APTQL:NMVVR, APTKL:NMVVK, VPTKL:EGMSVAE, VPTKL:GMAVS, TPTKM:GMVVD, VPARL:DMVVE, VPTRL:DMVVE, APVKT:MIVEE, VPQAL:MIVRS, VSQDL:MIVKS and VPQDL:MVVKS; wherein the pair PEP5:PEP6 is not APTKL:NMVVR when PEP1 is NAIS and PEP2 is LKKYR; wherein the pair PEP5:PEP6 is not APTQL:NMVVR when PEP1 is NAIS and PEP2 is LKKYR; wherein the pair PEP5:PEP6 is not APTKL:NMVVK when PEP1 is SATS and PEP2 is LRKHR; wherein the pair PEP5:PEP6 is not VPTKL:EGMSVAE when PEP1 is SPIS and PEP2 is LKYHY; wherein the pair PEP5:PEP6 is not VPTKL:GMAVS when PEP1 is EPIS and PEP2 is KFKYE; and wherein the pair PEP5:PEP6 is not TPTKM:GMVVD when PEP1 is SPIN and PEP2 is YGKIP.

In certain embodiments useful for inducing the conversion of a neoplastic cell into a cell (any cell) of the lung cell lineage, PEP9 is selected from the group consisting of KIPKAXXVPTEL, GIPEPXXVPEKM, SIPKAXXVPTEL, HVTKPTXAPTKL, YVPKPXXAPTKL, TVPKPXXAPTQL, AVPKAXXAPTKL, KVGKAXXVPTKL, KASKAXXVPTKL, GSAGPXXTPTKM, AAPASXXVPARL, STPPTXXVPTRL, HVPKPXXAPTKL, RVPSTXXAPVKT, ASAAPXXVPQAL, ASASPXXVSQDL and ASASPXXVPQDL; PEP10 is selected from the group consisting of DMVVEGXGXR, NMTVESXAXR, EMVVEGXGXR, NMVVRSXGXH, NMVVRSXGXH, NMVVRAXGXH, NMVVKAXGXH, EGMSVAEXGXR, GMAVSEXGXR, GMVVDRXGXS, DMVVEAXGXR, DMVVESXGXR, NMVVRSXGXH, MIVEEXGXL, MIVRSXKXS, MIVKSXKXS and MVVKSXKXS; and wherein the pair PEP9:PEP10 is selected from the group consisting of KIPKAXXVPTEL:DMVVEGXGXR, GIPEPXXVPEKM:NMTVESXAXR, SIPKAXXVPTEL:EMVVEGXGXR, HVTKPTXAPTKL:NMVVRSXGXH, YVPKPXXAPTKL:NMVVRSXGXH, TVPKPXXAPTQL:NMVVRAXGXH, AVPKAXXAPTKL:NMVVKAXGXH, KVGKAXXVPTKL:EGMSVAEXGXR, KASKAXXVPTKL:GMAVSEXGXR, GSAGPXXTPTKM:GMVVDRXGXS, AAPASXXVPARL:DMVVEAXGXR, STPPTXXVPTRL:DMVVESXGXR, HVPKPXXAPTKL:NMVVRSXGXH, RVPSTXXAPVKT:MIVEEXGXL, ASAAPXXVPQAL:MIVRSXKXS, ASASPXXVSQDL:MIVKSXKXS and ASASPXXVPQDL:MVVKSXKXS; wherein the pair PEP9:PEP10 is not YVPKPXXAPTKL:NMVVRSXGXH when PEP1 is NAIS and PEP2 is LKKYR; wherein the pair PEP9:PEP10 is not TVPKPXXAPTQL:NMVVRAXGXH when PEP1 is NAIS and PEP2 is LKKYR; wherein the pair PEP9:PEP10 is not AVPKAXXAPTKL:NMVVKAXGXH when PEP1 is SATS and PEP2 is LRKHR; wherein the pair PEP9:PEP10 is not KVGKAXXVPTKL:EGMSVAEXGXR when PEP1 is SPIS and PEP2 is LKYHY; wherein the pair PEP9:PEP10 is not KASKAXXVPTKL:GMAVSEXGXR when PEP1 is EPIS and PEP2 is KFKYE; and wherein the pair PEP9:PEP10 is not GSAGPXXTPTKM:GMVVDRXGXS when PEP1 is SPIN and PEP2 is YGKIP.

In certain embodiments useful for inducing the conversion of a neoplastic cell into a cell (any cell) of the lung cell lineage, PEP1 is selected from the group consisting of NAIS, SATS, SPIS, EPIS and SPIN; PEP11 is selected from the group consisting of LYF, LYY, LYK and LYL; and the pair PEP1:PEP11 is selected from the group consisting of NAIS:LYF, SATS:LYY, SPIS:LYK, EPIS:LYL and SPIN:LYF.

In one aspect, the present disclosure provides a GFR-binding compound, wherein said GFR-binding compound is a peptide, a variant or analog thereof, or a peptidomimetic as defined herein, with between 20 and 60 (in particular between 20 and 50, and more particularly, between 20 and 45) amino acids, comprising a peptide with four amino acids (PEP1) or a peptide with eight amino acids (PEP12), a peptide with five amino acids (PEP2), and optionally a pair of peptides selected form the group consisting of PEP3:PEP4, PEP3:PEP6, PEP3:PEP10, PEP5:PEP4, PEP9:PEP4, PEP5:PEP6, PEP5:PEP10, PEP9:PEP6 and PEP9:PEP10; wherein said GFR-binding compound does not comprise the pair of peptides APTKL:NMVVR when PEP1 is NAIS and PEP2 is LKKYR; wherein said GFR-binding compound does not comprise the pair of peptides APTQL:NMVVR when PEP1 is NAIS and PEP2 is LKKYR; wherein said GFR-binding compound does not comprise the pair of peptides APTKL:NMVVK when PEP1 is SATS and PEP2 is LRKHR; wherein said GFR-binding compound does not comprise the pair of peptides VPTKL:EGMSVAE when PEP1 is SPIS and PEP2 is LKYHY; wherein said GFR-binding compound does not comprise the pair of peptides VPTKL:GMAVS when PEP1 is EPIS and PEP2 is KFKYE; and wherein said GFR-binding compound does not comprise the pair of peptides

TPTKM:GMVVD when PEP1 is SPIN and PEP2 is YGKIP; and, optionally, wherein the RMSD is 2.45Å or less.

In one aspect, the present disclosure provides a GFR-binding compound, wherein said GFR-binding compound is a peptide, a variant or analog thereof, or a peptidomimetic as defined herein, with between 20 and 60 (in particular between 20 and 50, and more particularly, between 20 and 45) amino acids, comprising a peptide with four amino acids (PEP1) or a peptide with eight amino acids (PEP12), a peptide with five amino acids (PEP2), and optionally a pair of peptides selected form the group consisting of PEP3:PEP4, PEP3:PEP6, PEP3:PEP10, PEP5:PEP4, PEP9:PEP4, PEP5:PEP6, PEP5:PEP10, PEP9:PEP6 and PEP9:PEP10; wherein said GFR-binding compound does not comprise the pair of peptides YVPKPXXAPTKL:NMVVRSXGXH when PEP1 is NAIS and PEP2 is LKKYR; wherein said GFR-binding compound does not comprise the pair of peptides TVPKPXXAPTQL:NMVVRAXGXH when PEP1 is NAIS and PEP2 is LKKYR; wherein said GFR-binding compound does not comprise the pair of peptides AVPKAXXAPTKL:NMVVKAXGXH when PEP1 is SATS and PEP2 is LRKHR; wherein said GFR-binding compound does not comprise the pair of peptides KVGKAXXVPTKL:EGMSVAEXGXR when PEP1 is SPIS and PEP2 is LKYHY; wherein said GFR-binding compound does not comprise the pair of peptides KASKAXXVPTKL:GMAVSEXGXR when PEP1 is EPIS and PEP2 is KFKYE; and wherein said GFR-binding compound does not comprise the pair of peptides GSAGPXXTPTKM:GMVVDRXGXS when PEP1 is SPIN and PEP2 is YGKIP; and, optionally, wherein the RMSD is 2.45Å or less.

In certain embodiments useful for inducing the conversion of a neoplastic cell into a cell (any cell) of the lung cell lineage, said GFR-binding compound is a synthetic molecule as defined herein in the definition section.

In certain embodiments useful for inducing the conversion of a neoplastic cell into a cell (any cell) of the lung cell lineage, said GFR-binding compound is a synthetic peptide, or a variant or analog thereof, or a peptidomimetic.

*Muscle cell lineage*

Certain embodiments of the invention are particularly useful for inducing the conversion of a neoplastic cell into a cell (any cell) of the muscle cell lineage.

In certain embodiments useful for inducing the conversion of a neoplastic cell into a cell (any cell) of the muscle cell lineage, PEP1 is RSVK or RPVQ.

In certain embodiments useful for inducing the conversion of a neoplastic cell into a cell (any cell) of the muscle cell lineage, PEP2 is KEVQV or KKATV.

In certain embodiments useful for inducing the conversion of a neoplastic cell into a cell (any cell) of the muscle cell lineage, PEP3 is selected from the group consisting of VPQ, VSQ, VPT, SRV and TQV.

In certain embodiments useful for inducing the conversion of a neoplastic cell into a cell (any cell) of the muscle cell lineage, PEP4 is selected from the group consisting of VRS, VKS, QHN, EHS, EEH and EDH.

In certain embodiments useful for inducing the conversion of a neoplastic cell into a cell (any cell) of the muscle cell lineage, PEP5 is a peptide of general formula PEP3-$AA^{11}$-$AA^{12}$; wherein PEP3 is selected from the group consisting of VPQ, VSQ, VPT, SRV and TQV; wherein $AA^{11}$ is selected from the group consisting of E, K, Q, R, A, D, G and H, in particular A, D, E, H, Q and G; wherein $AA^{12}$ is selected from the group consisting of L, M, T, E, Q and H, in particular L, E, H and Q. In one particular example, PEP5 is selected from the group consisting of VPQAL, VSQDL, VPQDL, VPTEE, VPTGQ, SRVHH and TQVQL.

In certain embodiments useful for inducing the conversion of a neoplastic cell into a cell (any cell) of the muscle cell lineage, PEP6 is a peptide of general formula $AA^{26}$-$AA^{27}$-$AA^{28}$-$AA^{29}$-PEP4; wherein PEP4 is selected from the group consisting of VRS, VKS, QHN, EHS, EEH and EDH; wherein $AA^{26}$ is absent or selected from the group consisting of $AA^{III}$ amino acids, preferably is absent; wherein $AA^{27}$ and $AA^{28}$ are independently selected from the group consisting of $AA^{III}$ and $AA^{V}$ amino acids, preferably $AA^{27}$ is selected from the group consisting of M, F, R, T and L, preferably $AA^{28}$ is selected from the group consisting of I, V, L and E; wherein $AA^{29}$ is absent or selected from the group consisting of $AA^{II}$ amino acids, preferably absent. In one particular example, PEP6 is selected from the group consisting of MIVRS, MIVKS, MVVKS, FLQHN, LEEHS, RLEEH and TLEDH.

In certain embodiments useful for inducing the conversion of a neoplastic cell into a cell (any cell) of the muscle cell lineage, PEP7 is an amino acid or a peptide with between two and seven amino acids of general formula $AA^{1}$-$AA^{2}$-$AA^{3}$-$AA^{4}$-$AA^{5}$-$AA^{6}$-$AA^{7}$; wherein $AA^{1}$, $AA^{2}$, $AA^{3}$, $AA^{4}$, and $AA^{5}$ are independently absent or $AA^{I}$ as defined herein; wherein $AA^{6}$ is absent or selected from the group consisting of S, T, C, E, Q, P and R, preferably C, S, Q, R or E; wherein $AA^{7}$ is absent or is selected from the group consisting of S, T, C, E, Q, P and R, preferably is S, P or C; and wherein at least one of $AA^{1}$, $AA^{2}$, $AA^{3}$, $AA^{4}$, $AA^{5}$, $AA^{6}$ or $AA^{7}$ is not absent. In one particular example, PEP7 is selected from the group consisting of ASAAPXX, ASASPXX, NDEGLEX, SSVKXQP and RNVQXRP.

In certain embodiments useful for inducing the conversion of a neoplastic cell into a cell (any cell) of the muscle cell lineage, PEP8 is an amino acid or a peptide with between two and six amino acids of general formula $AA^{33}$-$AA^{34}$-$AA^{35}$-$AA^{36}$-$AA^{37}$-$AA^{38}$; wherein $AA^{33}$ is absent or is selected from the group consisting of $AA^{I}$ amino acids at the exception of $AA^{VI}$ amino acids; $AA^{34}$ is absent or is selected from the group consisting of $AA^{III}$ and $AA^{IV}$ amino acids; wherein $AA^{35}$ is absent or is selected from the group consisting of $AA^{II}$ amino acids, preferably is S or C; wherein

$AA^{36}$ is absent or is selected from the group consisting of $AA^{III}$ and $AA^{IV}$ amino acids; wherein $AA^{37}$ is absent or is selected from the group consisting of $AA^{II}$ amino acids, preferably is S or C; wherein $AA^{38}$ is absent or is selected from the group consisting of $AA^{I}$; and wherein at least one of $AA^{33}$, $AA^{34}$, $AA^{35}$, $AA^{36}$, $AA^{37}$ or $AA^{38}$ is not absent. In one particular example, PEP8 is selected from the group consisting of XKXS, KXEXR, QXEXR, LEXAXA and LAXKXE.

In certain embodiments useful for inducing the conversion of a neoplastic cell into a cell (any cell) of the muscle cell lineage, PEP9 is a peptide of general formula $AA^1$-$AA^2$-$AA^3$-$AA^4$-$AA^5$-$AA^6$-$AA^7$-PEP5; wherein PEP5 is a peptide of formula PEP3-$AA^{11}$-$AA^{12}$; wherein PEP3 is selected from the group consisting of VPQ, VSQ, VPT, SRV and TQV; wherein $AA^{11}$ is selected from the group consisting of E, K, Q, R, A, D, G and H, in particular A, D, E, H, Q and G; wherein $AA^{12}$ is selected from the group consisting of L, M, T, E, Q and H, in particular L, E, H and Q; wherein $AA^1$, $AA^2$, $AA^3$, $AA^4$, and $AA^5$ are independently absent or $AA^I$ as defined herein; wherein $AA^6$ is absent or selected from the group consisting of S, T, C, E, Q, P and R, preferably C, S, Q, R or E; wherein $AA^7$ is absent or is selected from the group consisting of S, T, C, E, Q, P and R, preferably is S, P or C. In one particular example, PEP9 is selected from the group consisting of ASAAPXXVPQAL, ASASPXXVSQDL, ASASPXXVPQDL, NDEGLEXVPTEE, NDEGLEXVPTGQ, SSVKXQPSRVHH and RNVQXRPTQVQL.

In certain embodiments useful for inducing the conversion of a neoplastic cell into a cell (any cell) of the muscle cell lineage, PEP10 is a peptide of general formula PEP6-$AA^{33}$-$AA^{34}$-$AA^{35}$-$AA^{36}$-$AA^{37}$-$AA^{38}$; wherein PEP6 is a peptide of formula $AA^{26}$-$AA^{27}$-$AA^{28}$-$AA^{29}$-PEP4; wherein PEP4 is selected from the group consisting of VRS, VKS, QHN, EHS, EEH and EDH; wherein $AA^{26}$ is absent or selected from the group consisting of $AA^{III}$ amino acids, preferably is absent; wherein $AA^{27}$ and $AA^{28}$ are independently selected from the group consisting of $AA^{III}$ and $AA^{V}$ amino acids, preferably $AA^{27}$ is selected from the group consisting of M, F, R, T and L, preferably $AA^{28}$ is selected from the group consisting of I, V, L and E; wherein $AA^{29}$ is absent or selected from the group consisting of $AA^{II}$ amino acids, preferably absent; wherein $AA^{33}$ is absent or is selected from the group consisting of $AA^{I}$ amino acids at the exception of $AA^{VI}$ amino acids; $AA^{34}$ is absent or is selected from the group consisting of $AA^{III}$ and $AA^{IV}$ amino acids; wherein $AA^{35}$ is absent or is selected from the group consisting of $AA^{II}$ amino acids, preferably is S or C; wherein $AA^{36}$ is absent or is selected from the group consisting of $AA^{III}$ and $AA^{IV}$ amino acids; wherein $AA^{37}$ is absent or is selected from the group consisting of $AA^{II}$ amino acids, preferably is S or C; wherein $AA^{38}$ is absent or is selected from the group consisting of $AA^{I}$. In one particular example, PEP10 is selected from the group consisting of MIVRSXKXS, MIVKSXKXS, MVVKSXKXS, FLQHNKXEXR, LEEHSQXEXR, RLEEHLEXAXA and TLEDHLAXKXE.

In certain embodiments useful for inducing the conversion of a neoplastic cell into a cell (any cell) of the muscle cell lineage, PEP12 is a peptide of general formula PEP1-$AA^{17}$-PEP11; wherein $AA^{17}$ is selected from the group consisting of G, A, V, L, I, P, F, M, W, T and S (in particular is I or M); wherein PEP1 is RSVK or RPVQ.

In certain embodiments useful for inducing the conversion of a neoplastic cell into a cell (any cell) of the muscle cell lineage, PEP11 is a peptide with 3 amino acids of general formula $AA^{18}$-$AA^{19}$-$AA^{20}$; wherein $AA^{18}$ is selected from the group consisting of L, V, Q, A and R, in particular is A or R; wherein $AA^{19}$ is selected from the group consisting of $AA^{VII}$ amino acids (in particular is K); wherein $AA^{20}$ is selected from the group consisting of L, F, Y, K, I, V and M, in particular is V or I. In one particular example, PEP11 is AKV or RKI.

The definitions of "PEP" pairs, quadruplets, hexaplets and heptuplet e.g. PEP1:PEP2, PEP12:PEP2, or PEP7:PEP5:PEP12:LINKER:PEP2:PEP6:PEP8, useful for inducing the conversion of a neoplastic cell into a cell (any cell) of the muscle cell lineage, are as already defined herein to the extent that PEP1 to PEP12 are particularly useful for these applications. For instances:

In certain embodiments useful for inducing the conversion of a neoplastic cell into a cell (any cell) of the muscle cell lineage, the pair PEP1:PEP2 is RSVK:KEVQV or RPVQ:KKATV.

In certain embodiments, useful for inducing the conversion of a neoplastic cell into a cell (any cell) of the muscle cell lineage, the pair PEP12:PEP2 is RSVK-$AA^{17}$-AKV:KEVQV or RPVQ-$AA^{17}$-RKI:KKATV; wherein $AA^{17}$ is selected from the group consisting of G, A, V, L, I, P, F, M, W, T and S (in particular is I or M).

In certain embodiments useful for inducing the conversion of a neoplastic cell into a cell (any cell) of the muscle cell lineage, PEP3 is selected from the group consisting of VPQ, VSQ, VPT, SRV and TQV; PEP4 is selected from the group consisting of VRS, VKS, QHN, EHS, EEH and EDH; and wherein the pair PEP3:PEP4 is selected from the group consisting of VPQ:VRS, VSQ:VKS, VPQ:VKS, VPT:QHN, VPT:EHS, SRV:EEH and TQV:EDH.

In certain embodiments useful for inducing the conversion of a neoplastic cell into a cell (any cell) of the muscle cell lineage, PEP5 is selected from the group consisting of VPQAL, VSQDL, VPQDL, VPTEE, VPTGQ, SRVHH and TQVQL; PEP6 is selected from the group consisting of MIVRS, MIVKS, MVVKS, FLQHN, LEEHS, RLEEH and TLEDH; and wherein the pair PEP5:PEP6 is selected from the group consisting of VPQAL:MIVRS, VSQDL:MIVKS, VPQDL:MVVKS, VPTEE:FLQHN, VPTGQ:LEEHS and SRVHH:RLEEH and TQVQL:TLEDH.

In certain embodiments useful for inducing the conversion of a neoplastic cell into a cell (any cell) of the muscle cell lineage, PEP7 is selected from the group consisting of ASAAPXX, ASASPXX, NDEGLEX, SSVKXQP and RNVQXRP; wherein PEP8 is selected from the group consisting of XKXS, KXEXR, QXEXR, LEXAXA and LAXKXE; and wherein the pair PEP7:PEP8 is selected from the group consisting of ASAAPXX:XKXS, ASASPXX:XKXS, NDEGLEX:KXEXR,

NDEGLEX:QXEXR, SSVKXQP:LEXAXA and RNVQXRP:LAXKXE.

In certain embodiments useful for inducing the conversion of a neoplastic cell into a cell (any cell) of the muscle cell lineage, PEP9 is selected from the group consisting of ASAAPXXVPQAL, ASASPXXVSQDL, ASASPXXVPQDL, NDEGLEXVPTEE, NDEGLEXVPTGQ, SSVKXQPSRVHH and RNVQXRPTQVQL; PEP10 is selected from the group consisting of MIVRSXKXS, MIVKSXKXS, MVVKSXKXS, FLQHNKXEXR, LEEHSQXEXR, RLEEHLEXAXA and TLEDHLAXKXE; and wherein the pair PEP9:PEP10 is selected from the group consisting of ASAAPXXVPQAL:MIVRSXKXS, ASASPXXVSQDL:MIVKSXKXS, ASASPXXVPQDL:MVVKSXKXS, NDEGLEXVPTEE:FLQHNKXEXR, NDEGLEXVPTGQ:LEEHSQXEXR, SSVKXQPSRVHH:RLEEHLEXAXA and RNVQXRPTQVQL:TLEDHLAXKXE.

In certain embodiments useful for inducing the conversion of a neoplastic cell into a cell (any cell) of the muscle cell lineage, PEP1 is RSVK or RPVQ; PEP11 is is AKV or RKI; and the pair PEP1:PEP11 is RSVK:AKV or RPVQ:RKI.

In certain embodiments useful for inducing the conversion of a neoplastic cell into a cell (any cell) of the muscle cell lineage, said GFR-binding compound is a synthetic molecule as defined herein in the definition section.

In certain embodiments useful for inducing the conversion of a neoplastic cell into a cell (any cell) of the muscle cell lineage, said GFR-binding compound is a synthetic peptide, or a variant or analog thereof, or a peptidomimetic.

*Blood cell lineage*

Certain embodiments of the invention are particularly useful for inducing the conversion of a neoplastic cell into a cell (any cell) of the blood cell lineage.

In certain embodiments useful for inducing the conversion of a neoplastic cell into a cell (any cell) of the blood cell lineage, PEP1 is SNIT.

In certain embodiments useful for inducing the conversion of a neoplastic cell into a cell (any cell) of the blood cell lineage, PEP2 is IGEMS or LGEMS.

In certain embodiments useful for inducing the conversion of a neoplastic cell into a cell (any cell) of the blood cell lineage, PEP3 is selected from the group consisting of TPT, VPA, VPT, APT, APV, VPQ, VSQ, SRV and TQV.

In certain embodiments useful for inducing the conversion of a neoplastic cell into a cell (any cell) of the blood cell lineage, PEP4 is selected from the group consisting of VVD, VVE, VVR, VEE, VRS, VKS, QHN, EHS, EEH and EDH.

In certain embodiments useful for inducing the conversion of a neoplastic cell into a cell (any cell) of the blood cell lineage, PEP5 is a peptide of general formula PEP3-$AA^{11}$-$AA^{12}$; wherein PEP3 is selected from the group consisting of TPT, VPA, VPT, APT, APV, VPQ, VSQ, SRV and TQV; wherein $AA^{11}$ is selected from the group consisting of E, K, Q, R, A, D, G and H; wherein $AA^{12}$ is selected from the group consisting of L, M, T, E, Q and H. In one particular example, PEP5 is selected from the group consisting of TPTKM, VPARL, VPTRL, APTKL, APVKT, VPQAL, VSQDL, VPQDL, VPTEE, VPTGQ, SRVHH and TQVQL.

In certain embodiments useful for inducing the conversion of a neoplastic cell into a cell (any cell) of the blood cell lineage, PEP6 is a peptide of general formula $AA^{26}$-$AA^{27}$-$AA^{28}$-$AA^{29}$-PEP4; wherein PEP4 is selected from the group consisting of VVD, VVE, VVR, VEE, VRS, VKS, QHN, EHS, EEH and EDH; wherein $AA^{26}$ is absent or selected from the group consisting of $AA^{III}$ amino acids, preferably is absent; wherein $AA^{27}$ and $AA^{28}$ are independently selected from the group consisting of $AA^{III}$ and $AA^{V}$ amino acids, preferably $AA^{27}$ is selected from the group consisting of G, D, N, M, R, F, L and T, preferably $AA^{28}$ is selected from the group consisting of M, I, V, E and L; wherein $AA^{29}$ is absent or selected from the group consisting of $AA^{II}$ amino acids, preferably is absent. In one particular example, PEP6 is selected from the group consisting of GMVVD, DMVVE, NMVVR, MIVEE, MIVRS, MIVKS, MVVKS, FLQHN, LEEHS, RLEEH and TLEDH.

In certain embodiments useful for inducing the conversion of a neoplastic cell into a cell (any cell) of the blood cell lineage, PEP7 is an amino acid or a peptide with between two and seven amino acids of general formula $AA^{1}$-$AA^{2}$-$AA^{3}$-$AA^{4}$-$AA^{5}$-$AA^{6}$-$AA^{7}$; wherein $AA^{1}$, $AA^{2}$, $AA^{3}$, $AA^{4}$, and $AA^{5}$ are independently absent or $AA^{I}$ as defined herein; wherein $AA^{6}$ is absent or selected from the group consisting of S, T, C, E, Q, P and R, preferably is selected from the group consisting of S, C, E, Q and R; wherein $AA^{7}$ is absent or is selected from the group consisting of S, T, C, E, Q, P and R, preferably is selected from the group consisting of C, S and P; and wherein at least one of $AA^{1}$, $AA^{2}$, $AA^{3}$, $AA^{4}$, $AA^{5}$, $AA^{6}$ or $AA^{7}$ is not absent. In one particular example, PEP7 is selected from the group consisting of GSAGPXX, AAPASXX, STPPTXX, HVPKPXX, RVPSTXX, ASAAPXX, ASASPXX, NDEGLEX, SSVKXQP and RNVQXRP.

In certain embodiments useful for inducing the conversion of a neoplastic cell into a cell (any cell) of the blood cell lineage, PEP8 is an amino acid or a peptide with between two and six amino acids of general formula $AA^{33}$-$AA^{34}$-$AA^{35}$-$AA^{36}$-$AA^{37}$-$AA^{38}$; wherein $AA^{33}$ is absent or is selected from the group consisting of $AA^{I}$ amino acids at the exception of $AA^{VI}$ amino acids; $AA^{34}$ is absent or is selected from the group consisting of $AA^{III}$ and $AA^{IV}$ amino acids; wherein $AA^{35}$ is absent or is selected from the group consisting of $AA^{II}$ amino acids, preferably is S or C; wherein $AA^{36}$ is absent or is selected from the group consisting of $AA^{III}$ and $AA^{IV}$ amino acids; wherein $AA^{37}$ is absent or is selected from the group consisting of $AA^{II}$ amino acids, preferably is S or C; wherein $AA^{38}$ is absent or is selected from

the group consisting of $AA^I$; and wherein at least one of $AA^{33}$, $AA^{34}$, $AA^{35}$, $AA^{36}$, $AA^{37}$ or $AA^{38}$ is not absent. In one particular example, PEP8 is selected from the group consisting of RXGXS, AXGXR, SXGXR, SXGXH, XGXL, XKXS, KXEXR, QXEXR, LEXAXA and LAXKXE.

In certain embodiments useful for inducing the conversion of a neoplastic cell into a cell (any cell) of the blood cell lineage, PEP9 is a peptide of general formula $AA^1$-$AA^2$-$AA^3$-$AA^4$-$AA^5$-$AA^6$-$AA^7$-PEPS; wherein PEP5 is a peptide of formula PEP3-$AA^{11}$-$AA^{12}$; wherein PEP3 is selected from the group consisting of TPT, VPA, VPT, APT, APV, VPQ, VSQ, SRV and TQV; wherein $AA^{11}$ is selected from the group consisting of E, K, Q, R, A, D, G and H; wherein $AA^{12}$ is selected from the group consisting of L, M, T, E, Q and H; wherein $AA^1$, $AA^2$, $AA^3$, $AA^4$, and $AA^5$ are independently absent or $AA^I$ as defined herein; wherein $AA^6$ is absent or selected from the group consisting of S, T, C, E, Q, P and R, preferably is selected from the group consisting of S, C, E, Q and R; wherein $AA^7$ is absent or is selected from the group consisting of S, T, C, E, Q, P and R, preferably is selected from the group consisting of C, S and P. In one particular example, PEP9 is selected from the group consisting of GSAGPXXTPTKM, AAPASXXVPARL, STPPTXXVPTRL, HVPKPXXAPTKL, RVPSTXXAPVKT, ASAAPXXVPQAL, ASASPXXVSQDL, ASASPXXVPQDL, NDEGLEXVPTEE, NDEGLEXVPTGQ, SSVKXQPSRVHH and RNVQXRPTQVQL.

In certain embodiments useful for inducing the conversion of a neoplastic cell into a cell (any cell) of the blood cell lineage, PEP10 is a peptide of general formula PEP6-$AA^{33}$-$AA^{34}$-$AA^{35}$-$AA^{36}$-$AA^{37}$-$AA^{38}$; wherein PEP6 is a peptide of formula $AA^{26}$-$AA^{27}$-$AA^{28}$-$AA^{29}$-PEP4; wherein PEP4 is selected from the group consisting of VVD, VVE, VVR, VEE, VRS, VKS, QHN, EHS, EEH and EDH; wherein $AA^{26}$ is absent or selected from the group consisting of $AA^{III}$ amino acids, preferably is absent; wherein $AA^{27}$ and $AA^{28}$ are independently selected from the group consisting of $AA^{III}$ and $AA^V$ amino acids, preferably $AA^{27}$ is selected from the group consisting of G, D, N, M, R, F, L and T, preferably $AA^{28}$ is selected from the group consisting of M, I, V, E and L; wherein $AA^{29}$ is absent or selected from the group consisting of $AA^{II}$ amino acids, preferably is absent; wherein $AA^{33}$ is absent or is selected from the group consisting of $AA^I$ amino acids at the exception of $AA^{VI}$ amino acids; $AA^{34}$ is absent or is selected from the group consisting of $AA^{III}$ and $AA^{IV}$ amino acids; wherein $AA^{35}$ is absent or is selected from the group consisting of $AA^{II}$ amino acids, preferably is S or C; wherein $AA^{36}$ is absent or is selected from the group consisting of $AA^{III}$ and $AA^{IV}$ amino acids; wherein $AA^{37}$ is absent or is selected from the group consisting of $AA^{II}$ amino acids, preferably is S or C; wherein $AA^{38}$ is absent or is selected from the group consisting of $AA^I$. In one particular example, PEP10 is selected from the group consisting of GMVVDRXGXS, DMVVEAXGXR, DMVVESXGXR, NMVVRSXGXH, MIVEEXGXL, MIVRSXKXS, MIVKSXKXS, MVVKSXKXS, FLQHNKXEXR, LEEHSQXEXR, RLEEHLEXAXA and TLEDHLAXKXE.

In certain embodiments useful for inducing the conversion of a neoplastic cell into a cell (any cell) of the blood cell lineage, PEP12 is a peptide of general formula PEPL-$AA^{17}$-PEP11; wherein $AA^{17}$ is selected from the group consisting of G, A, V, L, I, P, F, M, W, T and S (in particular is selected from the group consisting of M, I, V and T); wherein PEP1 is SNIT.

In certain embodiments useful for inducing the conversion of a neoplastic cell into a cell (any cell) of the blood cell lineage, PEP11 is a peptide with 3 amino acids of general formula $AA^{18}$-$AA^{19}$-$AA^{21}$; wherein $AA^{18}$ is selected from the group consisting of L, V, Q, A and R, in particular is Q; wherein $AA^{19}$ is selected from the group consisting of F, W, H, I and Y (in particular is I); wherein $AA^{20}$ is selected from the group consisting of L, F, Y, K, I, V and M, in particular is M. In one particular example, PEP11 is QIM.

In certain embodiments useful for inducing the conversion of a neoplastic cell into a cell (any cell) of the blood cell lineage, the pair PEP1:PEP2 is SNIT:IGEMS or SNIT:LGEMS.

In certain embodiments useful for inducing the conversion of a neoplastic cell into a cell (any cell) of the blood cell lineage, the pair PEP12:PEP2 is SNIT-$AA^{17}$-QIM:IGEMS or SNIT-$AA^{17}$-QIM:LGEMS; wherein $AA^{17}$ is selected from the group consisting of G, A, V, L, I, P, F, M, W, T and S (in particular is selected from the group consisting of M, I, V and T).

The definitions of "PEP" pairs, quadruplets, hexaplets and heptuplet e.g. PEP1:PEP2, PEP12:PEP2, or PEP7:PEP5:PEP12:LINKER:PEP2:PEP6:PEP8, useful for inducing the conversion of a neoplastic cell into a cell (any cell) of the blood cell lineage, are as already defined herein to the extent that PEP1 to PEP12 are particularly useful for these applications. For instances:

In certain embodiments useful for inducing the conversion of a neoplastic cell into a cell (any cell) of the blood cell lineage, PEP3 is selected from the group consisting of TPT, VPA, VPT, APT, APV, VPQ, VSQ, SRV and TQV; PEP4 is selected from the group consisting of VVD, VVE, VVR, VEE, VRS, VKS, QHN, EHS, EEH and EDH; and wherein the pair PEP3:PEP4 is selected from the consisting of TPT:VVD, VPA:VVE, VPT:VVE, APT:VVR, APV:VEE, VPQ:VRS, VSQ:VKS, VPQ:VKS, VPT:QHN, VPT:EHS, SRV:EEH and TQV:EDH.

In certain embodiments useful for inducing the conversion of a neoplastic cell into a cell (any cell) of the blood cell lineage, PEP5 is selected from the group consisting of TPTKM, VPARL, VPTRL, APTKL, APVKT, VPQAL, VSQDL, VPQDL, VPTEE, VPTGQ, SRVHH and TQVQL; PEP6 is selected from the group consisting of GMVVD, DMVVE, NMVVR, MIVEE, MIVRS, MIVKS, MVVKS, FLQHN, LEEHS, RLEEH and TLEDH; and wherein the pair PEP5:PEP6 is selected from the group consisting of TPTKM:GMVVD, VPARL:DMVVE, VPTRL:DMVVE, APTKL:NMVVR, APVKT:MIVEE, VPQAL:MIVRS, VSQDL:MIVKS, VPQDL:MVVKS, VPTEE:FLQHN, VPTGQ:LEEHS, SRVHH:RLEEH and TQVQL:TLEDH.

In certain embodiments useful for inducing the conversion of a neoplastic cell into a cell (any cell) of the blood cell lineage, PEP7 is selected from the group consisting of GSAGPXX, AAPASXX, STPPTXX, HVPKPXX, RVPSTXX, ASAAPXX, ASASPXX, NDEGLEX, SSVKXQP and RNVQXRP; wherein PEP8 is selected from the group consisting of RXGXS, AXGXR, SXGXR, SXGXH, XGXL, XKXS, KXEXR, QXEXR, LEXAXA and LAXKXE; and wherein the pair PEP7:PEP8 is selected from the group consisting of GSAGPXX:RXGXS, AAPASXX:AXGXR, STPPTXX:SXGXR, HVPKPXX:SXGXH, RVPSTXX:XGXL, ASAAPXX:XKXS, ASASPXX:XKXS, NDEGLEX:KXEXR, NDEGLEX:QXEXR, SSVKXQP:LEXAXA and RNVQXRP:LAXKXE.

In certain embodiments useful for inducing the conversion of a neoplastic cell into a cell (any cell) of the blood cell lineage, PEP9 is selected from the group consisting of GSAGPXXTPTKM, AAPASXXVPARL, STPPTXXVPTRL, HVPKPXXAPTKL, RVPSTXXAPVKT, ASAAPXXVPQAL, ASASPXXVSQDL, ASASPXXVPQDL, NDEGLEXVPTEE, NDEGLEXVPTGQ, SSVKXQPSRVHH and RNVQXRPTQVQL; PEP10 is selected from the group consisting of GMVVDRXGXS, DMVVEAXGXR, DMVVESXGXR, NMVVRSXGXH, MIVEEXGXL, MIVRSXKXS, MIVKSXKXS, MVVKSXKXS, FLQHNKXEXR, LEEHSQXEXR, RLEEHLEXAXA and TLEDHLAXKXE; and wherein the pair PEP9:PEP10 is selected from the group consisting of GSAGPXXTPTKM:GMVVDRXGXS, AAPASXXVPARL:DMVVEAXGXR, STPPTXXVPTRL:DMVVESXGXR, HVPKPXXAPTKL:NMVVRSXGXH, RVPSTXXAPVKT:MIVEEXGXL, ASAAPXXVPQAL:MIVRSXKXS, ASASPXXVSQDL:MIVKSXKXS, ASASPXXVPQDL:MVVKSXKXS, NDEGLEXVPTEE:FLQHNKXEXR, NDEGLEXVPTGQ:LEEHSQXEXR, SSVKXQPSRVHH:RLEEHLEXAXA and RNVQXRPTQVQL:TLEDHLAXKXE.

In certain embodiments useful for inducing the conversion of a neoplastic cell into a cell (any cell) of the blood cell lineage, PEP1 is SNIT and PEP11 is QIM.

In one aspect, the present disclosure provides a GFR-binding compound, wherein said GFR-binding compound is a peptide, a variant or analog thereof, or a peptidomimetic as defined herein, with between 20 and 60 (in particular between 20 and 50, and more particularly, between 20 and 45) amino acids, comprising a peptide with four amino acids (PEP1) or a peptide with eight amino acids (PEP12), a peptide with five amino acids (PEP2); optionally a pair of peptides selected form the group consisting of PEP3:PEP4, PEP3:PEP6, PEP3:PEP10, PEP5:PEP4, PEP9:PEP4, PEP5:PEP6, PEP5:PEP10, PEP9:PEP6 and PEP9:PEP10; and, optionally, wherein the RMSD is 2.45Å or less.

In certain embodiments useful for inducing the conversion of a neoplastic cell into a cell (any cell) of the blood cell lineage, said GFR-binding compound is a synthetic molecule as defined herein in the definition section.

In certain embodiments useful for inducing the conversion of a neoplastic cell into a cell (any cell) of the blood cell lineage, said GFR-binding compound is a synthetic peptide, or a variant or analog thereof, or a peptidomimetic.

*Adipocyte cell lineage*

Certain embodiments of the invention are particularly useful for inducing the conversion of a neoplastic cell into a cell (any cell) of the adipocyte lineage.

In certain embodiments useful for inducing the conversion of a neoplastic cell into a cell (any cell) of the adipocyte lineage, PEP1 is SAIS or NAIS.

In certain embodiments useful for inducing the conversion of a neoplastic cell into a cell (any cell) of the adipocyte lineage, PEP2 is LKNYQ or LKKYR.

In certain embodiments useful for inducing the conversion of a neoplastic cell into a cell (any cell) of the adipocyte lineage, PEP3 is selected from the group consisting of VPT, VPE, APT, TPT, VPA, APV, VPQ and VSQ.

In certain embodiments useful for inducing the conversion of a neoplastic cell into a cell (any cell) of the adipocyte lineage, PEP4 is selected from the group consisting of VVE, TVE, VVR, VVK, VAE, AVS, VVD, VEE, VRS and VKS.

In certain embodiments useful for inducing the conversion of a neoplastic cell into a cell (any cell) of the adipocyte lineage, PEP5 is a peptide of general formula PEP3-$AA^{11}$-$AA^{12}$; wherein PEP3 is selected from the group consisting of VPT, VPE, APT, TPT, VPA, APV, VPQ and VSQ; wherein $AA^{11}$ is selected from the group consisting of E, K, Q, R, A, D, G and H, in particular is selected from the group consisting of E, K, Q, R, A and D; wherein $AA^{12}$ is selected from the group consisting of L, M, T, E, Q and H, in particular selected from the group consisting of L, M and T. In one particular example, PEP5 is selected from the group consisting of VPTEL, VPEKM, APTKL, APTQL, VPTKL, TPTKM, VPARL, VPTRL, APVKT, VPQAL, VSQDL and VPQDL.

In certain embodiments useful for inducing the conversion of a neoplastic cell into a cell (any cell) of the adipocyte lineage, PEP6 is a peptide of general formula $AA^{26}$-$AA^{27}$-$AA^{28}$-$AA^{29}$-PEP4; wherein PEP4 is selected from the group consisting of VVE, TVE, VVR, VVK, VAE, AVS, VVD, VEE, VRS and VKS; wherein $AA^{26}$ is absent or selected from the group consisting of $AA^{III}$ amino acids, preferably is absent or E; wherein $AA^{27}$ and $AA^{28}$ are independently selected from the group consisting of $AA^{III}$ and $AA^{V}$ amino acids, preferably $AA^{27}$ is selected from the group consisting of D, E, N, G and M, preferably $AA^{28}$ is selected from the group consisting of M, I and V; wherein $AA^{29}$ is absent or selected from the group consisting of $AA^{II}$ amino acids, preferably absent or S. In one particular example, PEP6 is selected from the group consisting of DMVVE, NMTVE, EMVVE, NMVVR, NMVVK, EGMSVAE, GMAVS, GMVVD, DMVVE, MIVEE, MIVRS,

MIVKS and MVVKS.

In certain embodiments useful for inducing the conversion of a neoplastic cell into a cell (any cell) of the adipocyte lineage, PEP7 is an amino acid or a peptide with between two and seven amino acids of general formula $AA^1$-$AA^2$-$AA^3$-$AA^4$-$AA^5$-$AA^6$-$AA^7$; wherein $AA^1$, $AA^2$, $AA^3$, $AA^4$, and $AA^5$ are independently absent or $AA^I$ as defined herein; wherein $AA^6$ is absent or selected from the group consisting of S, T, C, E, Q, P and R, preferably is selected from the group consisting of C, S and T; wherein $AA^7$ is absent or is selected from the group consisting of S, T, C, E, Q, P and R, preferably is C or S; and wherein at least one of $AA^1$, $AA^2$, $AA^3$, $AA^4$, $AA^5$, $AA^6$ or $AA^7$ is not absent. In one particular example, PEP7 is selected from the group consisting of KIPKAXX, GIPEPXX, SIPKAXX, HVTKPTX, YVPKPXX, TVPKPXX, AVPKAXX, KVGKAXX, KASKAXX, GSAGPXX, AAPASXX, STPPTXX, HVPKPXX, RVPSTXX, ASAAPXX and ASASPXX.

In certain embodiments useful for inducing the conversion of a neoplastic cell into a cell (any cell) of the adipocyte lineage, PEP8 is an amino acid or a peptide with between two and six amino acids of general formula $AA^{33}$-$AA^{34}$-$AA^{35}$-$AA^{36}$-$AA^{37}$-$AA^{38}$; wherein $AA^{33}$ is absent or is selected from the group consisting of $AA^I$ amino acids at the exception of $AA^{VI}$ amino acids; $AA^{34}$ is absent or is selected from the group consisting of $AA^{III}$ and $AA^{IV}$ amino acids; wherein $AA^{35}$ is absent or is selected from the group consisting of $AA^{II}$ amino acids, preferably is S or C; wherein $AA^{36}$ is absent or is selected from the group consisting of $AA^{III}$ and $AA^{IV}$ amino acids; wherein $AA^{37}$ is absent or is selected from the group consisting of $AA^{II}$ amino acids, preferably is S or C; wherein $AA^{38}$ is absent or is selected from the group consisting of $AA^I$; and wherein at least one of $AA^{33}$, $AA^{34}$, $AA^{35}$, $AA^{36}$, $AA^{37}$ or $AA^{38}$ is not absent. In one particular example, PEP8 is selected from the group consisting of GXGXR, SXAXR, SXGXH, AXGXH, XGXR, EXGXR, RXGXS, AXGXR, SXGXR, XGXL and XKXS.

In certain embodiments useful for inducing the conversion of a neoplastic cell into a cell (any cell) of the adipocyte lineage, PEP9 is a peptide of general formula $AA^1$-$AA^2$-$AA^3$-$AA^4$-$AA^5$-$AA^6$-$AA^7$-PEP5; wherein PEP5 is a peptide of formula PEP3-$AA^{11}$-$AA^{12}$; wherein PEP3 is selected from the group consisting of VPT, VPE, APT, TPT, VPA, APV, VPQ and VSQ; wherein $AA^{11}$ is selected from the group consisting of E, K, Q, R, A, D, G and H, in particular E, K, Q, R, A and D; wherein $AA^{12}$ is selected from the group consisting of L, M, T, E, Q and H, in particular selected from the group consisting of L, M and T; wherein $AA^1$, $AA^2$, $AA^3$, $AA^4$, and $AA^5$ are independently absent or $AA^I$ as defined herein; wherein $AA^6$ is absent or selected from the group consisting of S, T, C, E, Q, P and R, preferably is selected from the group consisting of C, S and T; wherein $AA^7$ is selected from the group consisting of S, T, C, E, Q, P and R, preferably is C or S. In one particular example, PEP9 is selected from the group consisting of KIPKAXXVPTEL, GIPEPXXVPEKM, SIPKAXXVPTEL, HVTKPTXAPTKL, YVPKPXXAPTKL, TVPKPXXAPTQL, AVPKAXXAPTKL, KVGKAXXVPTKL, KASKAXXVPTKL, GSAGPXXTPTKM, AAPASXXVPARL, STPPTXXVPTRL, HVPKPXXAPTKL, RVPSTXXAPVKT, ASAAPXXVPQAL, ASASPXXVSQDL and ASASPXXVPQDL.

In certain embodiments useful for inducing the conversion of a neoplastic cell into a cell (any cell) of the adipocyte lineage, PEP10 is a peptide of general formula PEP6-$AA^{33}$-$AA^{34}$-$AA^{35}$-$AA^{36}$-$AA^{37}$-$AA^{38}$; wherein PEP6 is a peptide of formula $AA^{26}$-$AA^{27}$-$AA^{28}$-$AA^{29}$-PEP4; wherein PEP4 is selected from the group consisting of VVE, TVE, VVR, VVK, VAE, AVS, VVD, VEE, VRS and VKS; wherein $AA^{26}$ is absent or selected from the group consisting of $AA^{III}$ amino acids, preferably is absent or E; wherein $AA^{27}$ and $AA^{28}$ are independently selected from the group consisting of $AA^{III}$ and $AA^V$ amino acids, preferably $AA^{27}$ is selected from the group consisting of D, E, N, G and M, preferably $AA^{28}$ is selected from the group consisting of M, I and V; wherein $AA^{29}$ is absent or selected from the group consisting of $AA^{II}$ amino acids, preferably absent or S; wherein $AA^{33}$ is absent or is selected from the group consisting of $AA^I$ amino acids at the exception of $AA^{VI}$ amino acids; $AA^{34}$ is absent or is selected from the group consisting of $AA^{III}$ and $AA^{IV}$ amino acids; wherein $AA^{35}$ is absent or is selected from the group consisting of $AA^{II}$ amino acids, preferably is S or C; wherein $AA^{36}$ is absent or is selected from the group consisting of $AA^{III}$ and $AA^{IV}$ amino acids; wherein $AA^{37}$ is absent or is selected from the group consisting of $AA^{II}$ amino acids, preferably is S or C; wherein $AA^{38}$ is absent or is selected from the group consisting of $AA^I$. In one particular example, PEP10 is selected from the group consisting of DMVVEGXGXR, NMTVESXAXR, EMVVEGXGXR, NMVVRSXGXH, NMVVRSXGXH, NMVVRAXGXH, NMVVKAXGXH, EGMSVAEXGXR, GMAVSEXGXR, GMVVDRXGXS, DMVVEAXGXR, DMVVESXGXR, NMVVRSXGXH, MIVEEXGXL, MIVRSXKXS, MIVKSXKXS and MVVKSXKXS.

In certain embodiments useful for inducing the conversion of a neoplastic cell into a cell (any cell) of the adipocyte lineage, PEP12 is a peptide of general formula PEP1-$AA^{17}$-PEP11; wherein $AA^{17}$ is selected from the group consisting of G, A, V, L, I, P, F, M, W, T and S (in particular is selected from the group consisting of M, V and T); wherein PEP1 is SAIS or NAIS.

In certain embodiments useful for inducing the conversion of a neoplastic cell into a cell (any cell) of the adipocyte lineage, PEP11 is a peptide with 3 amino acids of general formula $AA^{18}$-$AA^{19}$-$AA^{20}$; wherein $AA^{18}$ is selected from the group consisting of L, V, Q, A and R, in particular is L; wherein $AA^{19}$ is selected from the group consisting of F, W, H and Y (in particular is a polar aromatic amino acid such as Y); wherein $AA^{20}$ is selected from the group consisting of L, F, Y, K, I, V and M, in particular is L or F. In one particular example, PEP11 is LYL or LYF.

The definitions of "PEP" pairs, quadruplets, hexaplets and heptuplet e.g. PEP1:PEP2, PEP12:PEP2, or

PEP7:PEP5:PEP12:LINKER:PEP2:PEP6:PEP8, useful for inducing the conversion of a neoplastic cell into a cell (any cell) of the adipocyte lineage, are as already defined herein to the extent that PEP1 to PEP12 are particularly useful for these applications. For instances:

In certain embodiments useful for inducing the conversion of a neoplastic cell into a cell (any cell) of the adipocyte lineage, the pair PEP1:PEP2 is SAIS:LKNYQ or NAIS:LKKYR.

In certain embodiments useful for inducing the conversion of a neoplastic cell into a cell (any cell) of the adipocyte lineage, the pair PEP12:PEP2 is SAIS-$AA^{17}$-LYL:LKNYQ or NAIS-$AA^{17}$-LYF:LKKYR; wherein $AA^{17}$ is selected from the group consisting of G, A, V, L, I, P, F, M, W, T and S (in particular is selected from the group consisting of M, V and T).

In certain embodiments useful for inducing the conversion of a neoplastic cell into a cell (any cell) of the adipocyte lineage, PEP3 is selected from the group consisting of VPT, VPE, APT, TPT, VPA, APV, VPQ and VSQ; PEP4 is selected from the group consisting of VVE, TVE, VVR, VVK, VAE, AVS, VVD, VEE, VRS and VKS; and wherein the pair PEP3:PEP4 is selected from the group consisting of VPT:VVE, VPE:TVE, APT:VVR, APT:VVK, VPT:VAE, VPT:AVS, TPT:VVD, VPA:VVE, APV:VEE, VPQ:VRS, VSQ:VKS and VPQ:VKS.

In certain embodiments useful for inducing the conversion of a neoplastic cell into a cell (any cell) of the adipocyte lineage, PEP5 is selected from the group consisting of VPTEL, VPEKM, APTKL, APTQL, VPTKL, TPTKM, VPARL, VPTRL, APVKT, VPQAL, VSQDL and VPQDL; PEP6 is selected from the group consisting of DMVVE, NMTVE, EMVVE, NMVVR, NMVVK, EGMSVAE, GMAVS, GMVVD, DMVVE, MIVEE, MIVRS, MIVKS and MVVKS; and wherein the pair PEP5:PEP6 is selected from the group consisting of VPTEL:DMVVE, VPEKM:NMTVE, VPTEL:EMVVE, APTKL:NMVVR, APTQL:NMVVR, APTKL:NMVVK, VPTKL:EGMSVAE, VPTKL:GMAVS, TPTKM:GMVVD, VPARL:DMVVE, VPTRL:DMVVE, APVKT:MIVEE, VPQAL:MIVRS, VSQDL:MIVKS and VPQDL:MVVKS.

In certain embodiments useful for inducing the conversion of a neoplastic cell into a cell (any cell) of the adipocyte lineage, PEP7 is selected from the group consisting of KIPKAXX, GIPEPXX, SIPKAXX, HVTKPTX, YVPKPXX, TVPKPXX, AVPKAXX, KVGKAXX, KASKAXX, GSAGPXX, AAPASXX, STPPTXX, HVPKPXX, RVPSTXX, ASAAPXX and ASASPXX; wherein PEP8 is selected from the group consisting of GXGXR, SXAXR, SXGXH, AXGXH, XGXR, EXGXR, RXGXS, AXGXR, SXGXR, XGXL and XKXS; and wherein the pair PEP7:PEP8 is selected from the group consisting of KIPKAXX:GXGXR, GIPEPXX:SXAXR, SIPKAXX:GXGXR, HVTKPTX:SXGXH, YVPKPXX:SXGXH, TVPKPXX:AXGXH, AVPKAXX:AXGXH, KVGKAXX:XGXR, KASKAXX:EXGXR, GSAGPXX:RXGXS, AAPASXX:AXGXR, STPPTXX:SXGXR, HVPKPXX:SXGXH, RVPSTXX:XGXL, ASAAPXX:XKXS and ASASPXX:XKXS.

In certain embodiments useful for inducing the conversion of a neoplastic cell into a cell (any cell) of the adipocyte lineage, PEP9 is selected from the group consisting of KIPKAXXVPTEL, GIPEPXXVPEKM, SIPKAXXVPTEL, HVTKPTXAPTKL, YVPKPXXAPTKL, TVPKPXXAPTQL, AVPKAXXAPTKL, KVGKAXXVPTKL, KASKAXXVPTKL, GSAGPXXTPTKM, AAPASXXVPARL, STPPTXXVPTRL, HVPKPXXAPTKL, RVPSTXXAPVKT, ASAAPXXVPQAL, ASASPXXVSQDL and ASASPXXVPQDL; PEP10 is selected from the group consisting of DMVVEGXGXR, NMTVESXAXR, EMVVEGXGXR, NMVVRSXGXH, NMVVRSXGXH, NMVVRAXGXH, NMVVKAXGXH, EGMSVAEXGXR, GMAVSEXGXR, GMVVDRXGXS, DMVVEAXGXR, DMVVESXGXR, NMVVRSXGXH, MIVEEXGXL, MIVRSXKXS, MIVKSXKXS and MVVKSXKXS; and wherein the pair PEP9:PEP10 is selected from the group consisting of KIPKAXXVPTEL:DMVVEGXGXR, GIPEPXXVPEKM:NMTVESXAXR, SIPKAXXVPTEL:EMVVEGXGXR, HVTKPTXAPTKL:NMVVRSXGXH, YVPKPXXAPTKL:NMVVRSXGXH, TVPKPXXAPTQL:NMVVRAXGXH, AVPKAXXAPTKL:NMVVKAXGXH, KVGKAXXVPTKL:EGMSVAEXGXR, KASKAXXVPTKL:GMAVSEXGXR, GSAGPXXTPTKM:GMVVDRXGXS, AAPASXXVPARL:DMVVEAXGXR, STPPTXXVPTRL:DMVVESXGXR, HVPKPXXAPTKL:NMVVRSXGXH, RVPSTXXAPVKT:MIVEEXGXL, ASAAPXXVPQAL:MIVRSXKXS, ASASPXXVSQDL:MIVKSXKXS and ASASPXXVPQDL:MVVKSXKXS.

In certain embodiments useful for inducing the conversion of a neoplastic cell into a cell (any cell) of the adipocyte lineage, PEP5 is selected from the group consisting of VPTEL, VPEKM, APTKL, APTQL, VPTKL, TPTKM, VPARL, VPTRL, APVKT, VPQAL, VSQDL and VPQDL; PEP6 is selected from the group consisting of DMVVE, NMTVE, EMVVE, NMVVR, NMVVK, EGMSVAE, GMAVS, GMVVD, DMVVE, MIVEE, MIVRS, MIVKS and MVVKS; and wherein the pair PEP5:PEP6 is selected from the group consisting of VPTEL:DMVVE, VPEKM:NMTVE, VPTEL:EMVVE, APTKL:NMVVR, APTQL:NMVVR, APTKL:NMVVK, VPTKL:EGMSVAE, VPTKL:GMAVS, TPTKM:GMVVD, VPARL:DMVVE, VPTRL:DMVVE, APVKT:MIVEE, VPQAL:MIVRS, VSQDL:MIVKS and VPQDL:MVVKS; wherein the pair PEP5:PEP6 is not VPTEL:DMVVE when PEP1 is SAIS and PEP2 is LKNYQ; wherein the pair PEP5:PEP6 is not VPTEL:EMVVE when PEP1 is SAIS and PEP2 is LKNYQ; wherein the pair PEP5:PEP6 is not APTQL:NMVVR when PEP1 is NAIS and PEP2 is LKKYR; and wherein the pair PEP5:PEP6 is not APTKL:NMVVR when PEP1 is NAIS and PEP2 is LKKYR.

In certain embodiments useful for inducing the conversion of a neoplastic cell into a cell (any cell) of the adipocyte lineage, PEP9 is selected from the group consisting of KIPKAXXVPTEL, GIPEPXXVPEKM, SIPKAXXVPTEL, HVTKPTXAPTKL, YVPKPXXAPTKL, TVPKPXXAPTQL, AVPKAXXAPTKL, KVGKAXXVPTKL, KASKAXXVPTKL, GSAGPXXTPTKM, AAPASXXVPARL, STPPTXXVPTRL, HVPKPXXAPTKL, RVPSTXXAPVKT, ASAAPXXVPQAL, ASASPXXVSQDL and ASASPXXVPQDL; PEP10 is selected from the group consisting of DMVVEGXGXR, NMTVESXAXR, EMVVEGXGXR, NMVVRSXGXH, NMVVRSXGXH, NMVVRAXGXH, NMVVKAXGXH, EGMSVAEXGXR, GMAVSEXGXR, GMVVDRXGXS, DMVVEAXGXR, DMVVESXGXR, NMVVRSXGXH, MIVEEXGXL, MIVRSXKXS, MIVKSXKXS and MVVKSXKXS; and wherein the pair PEP9:PEP10 is selected from the group consisting of KIPKAXXVPTEL:DMVVEGXGXR, GIPEPXXVPEKM:NMTVESXAXR, SIPKAXXVPTEL:EMVVEGXGXR, HVTKPTXAPTKL:NMVVRSXGXH, YVPKPXXAPTKL:NMVVRSXGXH, TVPKPXXAPTQL:NMVVRAXGXH, AVPKAXXAPTKL:NMVVKAXGXH, KVGKAXXVPTKL:EGMSVAEXGXR, KASKAXXVPTKL:GMAVSEXGXR, GSAGPXXTPTKM:GMVVDRXGXS, AAPASXXVPARL:DMVVEAXGXR, STPPTXXVPTRL:DMVVESXGXR, HVPKPXXAPTKL:NMVVRSXGXH, RVPSTXXAPVKT:MIVEEXGXL, ASAAPXXVPQAL:MIVRSXKXS, ASASPXXVSQDL:MIVKSXKXS and ASASPXXVPQDL:MVVKSXKXS; wherein the pair PEP9:PEP10 is not KIPKAXXVPTEL:DMVVEGXGXR when PEP1 is SAIS and PEP2 is LKNYQ; wherein the pair PEP9:PEP10 is not SIPKAXXVPTEL:EMVVEGXGXR when PEP1 is SAIS and PEP2 is LKNYQ; wherein the pair PEP9:PEP10 is not HVTKPTXAPTKL:NMVVRSXGXH when PEP1 is NAIS and PEP2 is LKKYR; wherein the pair PEP9:PEP10 is not YVPKPXXAPTKL:NMVVRSXGXH when PEP1 is NAIS and PEP2 is LKKYR; wherein the pair PEP9:PEP10 is not TVPKPXXAPTQL:NMVVRAXGXH when PEP1 is NAIS and PEP2 is LKKYR; and wherein the pair PEP9:PEP10 is not HVPKPXXAPTKL:NMVVRSXGXH when PEP1 is NAIS and PEP2 is LKKYR.

In certain embodiments useful for inducing the conversion of a neoplastic cell into a cell (any cell) of the adipocyte lineage, PEP1 is SAIS or NAIS; PEP11 is LYL or LYF; and the pair PEP1:PEP11 is SAIS:LYL or NAIS:LYF.

In one aspect, the present disclosure provides a GFR-binding compound, wherein said GFR-binding compound is a peptide, a variant or analog thereof, or a peptidomimetic as defined herein, with between 20 and 60 (in particular between 20 and 50, and more particularly, between 20 and 45) amino acids, comprising a peptide with four amino acids (PEP1) or a peptide with eight amino acids (PEP12), a peptide with five amino acids (PEP2), and optionally a pair of peptides selected form the group consisting of PEP3:PEP4, PEP3:PEP6, PEP3:PEP10, PEP5:PEP4, PEP9:PEP4, PEP5:PEP6, PEP5:PEP10, PEP9:PEP6 and PEP9:PEP10; wherein said GFR-binding compound does not comprise the pair of peptides VPTEL:DMVVE when PEP1 is SAIS and PEP2 is LKNYQ; wherein said GFR-binding compound does not comprise the pair of peptides VPTEL:EMVVE when PEP1 is SAIS and PEP2 is LKNYQ; wherein said GFR-binding compound does not comprise the pair of peptides APTQL:NMVVR when PEP1 is NAIS and PEP2 is LKKYR; and wherein said GFR-binding compound does not comprise the pair of peptides APTKL:NMVVR when PEP1 is NAIS and PEP2 is LKKYR; and, optionally, wherein the RMSD is 2.45Å or less.

In one aspect, the present disclosure provides a GFR-binding compound, wherein said GFR-binding compound is a peptide, a variant or analog thereof, or a peptidomimetic as defined herein, with between 20 and 60 (in particular between 20 and 50, and more particularly, between 20 and 45) amino acids, comprising a peptide with four amino acids (PEP1) or a peptide with eight amino acids (PEP12), a peptide with five amino acids (PEP2), and optionally a pair of peptides selected form the group consisting of PEP3:PEP4, PEP3:PEP6, PEP3:PEP10, PEP5:PEP4, PEP9:PEP4, PEP5:PEP6, PEP5:PEP10, PEP9:PEP6 and PEP9:PEP10; wherein said GFR-binding compound does not comprise the pair of peptides KIPKAXXVPTEL:DMVVEGXGXR when PEP1 is SAIS and PEP2 is LKNYQ; wherein said GFR-binding compound does not comprise the pair of peptides SIPKAXXVPTEL:EMVVEGXGXR when PEP1 is SAIS and PEP2 is LKNYQ; wherein said GFR-binding compound does not comprise the pair of peptides HVTKPTXAPTKL:NMVVRSXGXH when PEP1 is NAIS and PEP2 is LKKYR; wherein said GFR-binding compound does not comprise the pair of peptides YVPKPXXAPTKL:NMVVRSXGXH when PEP1 is NAIS and PEP2 is LKKYR; wherein said GFR-binding compound does not comprise the pair of peptides TVPKPXXAPTQL:NMVVRAXGXH when PEP1 is NAIS and PEP2 is LKKYR; and wherein said GFR-binding compound does not comprise the pair of peptides HVPKPXXAPTKL:NMVVRSXGXH when PEP1 is NAIS and PEP2 is LKKYR; and, optionally, wherein the RMSD is 2.45Å or less.

In certain embodiments useful for inducing the conversion of a neoplastic cell into a cell (any cell) of the adipocyte lineage, said GFR-binding compound is a synthetic molecule as defined herein in the definition section.

In certain embodiments useful for inducing the conversion of a neoplastic cell into a cell (any cell) of the adipocyte lineage, said GFR-binding compound is a synthetic peptide, or a variant or analog thereof, or a peptidomimetic.

**II.4. Interactions**

GFR-binding compounds may form at least one intramolecular non-covalent interaction. Such interactions may be one of electrostatic interactions, $\pi$-interactions, van der Waals forces, hydrophobic interactions or any combinations thereof. Such interactions may be formed between 2 or more amino acids' side chains, between 2 or more amino acids' backbones or between amino acids' side chains and backbones. Such interactions may increase the stability of the GFR-binding compounds in vitro and/or in vivo thus reducing or preventing physiological degradation. In one example, GFR-binding compounds may form at least one, preferably more than one, of the following intramolecular non-covalent interactions: $AA^{20}$ /// $AA^{206}$, $AA^{19}$ /// $AA^{207}$, $AA^{18}$ /// $AA^{21}$, $AA^{17}$ /// $AA^{22}$, $AA^{16}$ /// $AA^{23}$, $AA^{15}$ /// $AA^{24}$, $AA^{15}$ /// $AA^{25}$, wherein /// represents a non-covalent interaction.

## III. Adhesion protein inhibitor

The present invention achieves its intended therapeutic action(s) i.e. the treatment of a neoplastic disease via extracellular, non-mutagenic, recoding or conversion of neoplastic cells, by providing an association (or combination) of at least two bioactive substances, namely, at least one GFR-binding compound as described herein and at least one adhesion protein inhibitor or adhesion molecule inhibitor.

In one aspect, the present disclosure thus provides at least one adhesion protein inhibitor, as part of a pharmaceutical association or combination as defined herein, as an active principle for use in methods and uses described herein.

In one example, said GFR-binding compound and said adhesion protein inhibitor may be operably covalently or non-covalently associated, combined, linked or connected as defined herein.

Various methods known in the art may be used to assess the deactivation or inhibitory activity or potency of a given adhesion protein inhibitor on a given adhesion protein such an integrin or a syndecan. However, for the avoidance of doubts and for the purpose of the present disclosure, the assessment of the inhibition or deactivation of adhesion protein(s) by a given adhesion protein inhibitor as defined herein (alone or within a pharmaceutical association, combination or composition as defined herein) is performed using fluorescence resonance energy transfer (FRET). This method is able to measure with high precision the degree of protein conformational changes that are associated with the action of a specific adhesion protein inhibitor on a given adhesion protein and thus quantify the inhibitory effect of the adhesion protein inhibitor. FRET is a well-known and recognised analytical method, but for the avoidance of doubts and for the purpose of the present disclosure, the experimental procedures, parameters and setup are those described in Chigaev, A., Buranda, T., Dwyer, D. C., Prossnitz, E. R. and Sklar, L. A. (2003), "FRET detection of cellular α4-integrin conformational activation", Biophys., J. 85, 3951-3962, which is hereby incorporated by reference in its entirety.

As used herein, unless stated otherwise or contradictory in context, the terms "inhibition", "down-regulation", "deactivation" or "inactivation", when used in the context of cell adhesion, refers to a diminution or reduction in activity, function, gene expression and/or protein expression of a molecule, protein or gene, observed when a pharmaceutical association, combination or composition of the present disclosure is provided or administered to a cell or to a subject carrying this cell, using one of the methods defined herein, in comparison with the activity, function, gene expression and/or protein expression of a molecule, protein or gene in a neoplastic cell in the absence of such provision or administration, of more than 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99%, in particular of more than 20%. In one particular example, said inhibition is more than 30%. In one particular example, said inhibition is more than 40%. In one particular example, said inhibition is more than 50%. In one particular example, said inhibition is more than 60%. In one particular example, said inhibition is more than 70%. In one particular example, said inhibition is more than 80%.

In certain embodiments, the inhibition of cell adhesion comprises (or is exclusively constituted of, or exclusively consists of) the inhibition, deactivation or down-regulation of the gene or protein expression of one or more integrins, syndecans, selectins, dystroglycans or any combinations thereof.

In one aspect, the present disclosure also provides a use of an adhesion protein inhibitor as defined herein in the manufacture of a medicament comprising a GFR-binding compound as defined herein, for use in the diagnostic, prevention and/or treatment of a neoplastic disease.

One particular biological checkpoint that the present invention is able to re-establish, repair or restore is the cell adhesion checkpoint. The cell adhesion checkpoint is known to be in charge of monitoring the cells attachments to the extracellular matrix (ECM). In normal conditions, when a cell does not detect a "correct" attachment it should halt further advance through the cell cycle and enter the G0 phase.

Cell adhesion is the binding of a cell to a surface or substrate, such as the extracellular matrix (ECM). Adhesion occurs from the action (or engagement) of proteins, called cell adhesion proteins or molecules. Examples of such cell adhesion proteins are cell transmembrane adhesion proteins, such as the integrins, syndecans, selectins and dystroglycans. The most important among these proteins are the integrins, which assemble as alpha-beta heterodimers.

### III.1. Integrins

The integrins belong to a superfamily of cell adhesion receptors that bind to extracellular matrix ligands, cell-surface ligands, and soluble ligands. They are transmembrane αβ heterodimers and at least 18 α and 8 β subunits are known in humans, generating 24 heterodimers such as α1β1, α2β1, α3β1, α4β1, α5β1, α6β1, α7β1, αLβ2, αMβ2, αIIbβ3, αVβ1, αVβ3, αVβ5, αVβ6, αVβ8, and α6β4. Members of this family have been found in mammals, chicken and zebrafish, as well as lower eukaryotes. The α and β subunits have distinct domain structures, with extracellular domains from each

subunit contributing to the ligand-binding site of the heterodimer. The sequence arginine-glycine-aspartic acid (RGD) was identified as a general integrin-binding motif, but individual integrins are also specific for particular protein ligands. In addition to physically linking the cell to the extracellular matrix, the binding of the extracellular integrin domains to specific components of the ECM activates the integrins and allows binding of different signalling molecules to their intracellular domain. This activates various signalling pathways that mediate signals to the adhesion checkpoint.

The activation of an integrin is associated with structural conformational changes of the protein. Integrins are known to adopt three major conformational states: « inactive » (low affinity), « primed » or « active » (high affinity), and ligand occupied (which may be activated or not depending on the nature of the ligand). The active form of the protein is considered as having an « extended » conformation while the inactive form is considered as « bent » with bending of the ligand-binding headpiece of its extracellular domain. Upon activation the integrin proceeds with an unbending of this domain to acquire the extended conformation, which is able to bind the ECM components. This process initiates further conformational changes, which activate, through the intracellular domain several signaling pathway cascades.

In the present disclosure, the pharmaceutical association, combination or (therapeutic, dermatologic, ophthalmologic, diagnostic, etc.) composition defined herein is thus suitable for treating a neoplastic cell or a neoplastic disease (i) by activating one or more growth factor receptors and (ii) by, simultaneously or non-simultaneously, modulating or regulating the adhesion of that cell to its ECM microenvironment by (partially or completely) deactivating or inhibiting at least one integrin expressed on the cell membrane of said neoplastic cell. In certain embodiments, and without wishing to be bound to any specific theory, the deactivation or inhibition of an integrin may be the result of, for example, integrin's inactivation, integrin's dimerisation inhibition, and/or integrin's gene expression inhibition.

In certain embodiments, the adhesion protein inhibitor as defined herein comprises (or is) an integrin deactivator or inhibitor which binds to/interacts with at least one integrin and/or to at least one integrin dimer. In one example, said integrin deactivator or inhibitor is a receptor competitive antagonist. In one example, said integrin deactivator or inhibitor is a receptor non-competitive antagonist. In one example, said integrin deactivator or inhibitor is a receptor uncompetitive antagonist. In one example, said integrin deactivator or inhibitor is a receptor silent antagonist. In one example, said integrin deactivator or inhibitor is a receptor partial agonist. In one example, said integrin deactivator or inhibitor is a receptor inverse agonist.

In one aspect, the present disclosure thus provides a pharmaceutical association or combination or (therapeutic, dermatologic, ophthalmologic, diagnostic, etc.) composition as defined herein comprising at least one GFR-binding compound as defined herein and at least one inhibitor of integrin engagement with the ECM.

In certain embodiments, said at least one inhibitor of integrin engagement with the ECM is an anti-integrin antibody. One aspect of the present disclosure thus also provides a pharmaceutical association or combination or (therapeutic, dermatologic, ophthalmologic, diagnostic, etc.) composition as defined herein comprising at least one GFR-binding compound as defined herein and at least one integrin antibody.

In one example, said integrin antibody is selected from the group consisting of, Integrin α1 (A-9), Integrin α1 (F-19), Integrin α1 (HM alpha 1), Integrin α1 (R-164), Integrin α1 (R-19), Integrin α1 (TS2/7.1.1), Integrin α2 (H-293), Integrin α2 (N-19), Integrin α2 (C-9), Integrin α2 (P1H5), Integrin α2 (P1E6), Integrin α2 (HAS-4), Integrin α2 (P4B4), Integrin α2 (HAS-3), Integrin α2 (2), Integrin α3 (A-3), Integrin α3 (A-6), Integrin α3 (I-19), Integrin α3 (C-18), Integrin α3 (E-8), Integrin α3 (P1B5), Integrin α3 (H-43), Integrin α3 (Ralph 3.2), Integrin α3 (N-19), Integrin α3 (VM-2), Integrin α3 (IA3), Integrin α3 (F35 177-1), Integrin α4 (A-7), Integrin α4 (C-2), Integrin α4 (H-210), Integrin α4 (B-2), Integrin α4 (C-20), Integrin α4 (N-19), Integrin α4 (9F10), Integrin α4 (Y-18), Integrin α4 (PS/2), Integrin α5 (H-104), Integrin α5 (P-19), Integrin α5 (C-9), Integrin α5 (A-11), Integrin α5 (B-4), Integrin α5 (D-9), Integrin α5 (HMα5-1), Integrin α5 (P1D6), Integrin α5 (MFR5/5H10), Integrin α5 (JBS5), Integrin α5 (SAM-1), Integrin α5 (1), Integrin α6 (F-6), Integrin α6 (H-87), Integrin α6 (N-19), Integrin α6 (BQ16), Integrin α6 (GOH3), Integrin α6 (C-18), Integrin α6 (4F10), Integrin α6 (541A11), Integrin α6 (mAB-5A), Integrin α6 (450-30A), Integrin α6 (NKI-GOH3), Integrin α6 (3H1512), Integrin α6 (2Q959), Integrin α6 (1.BB.460), Integrin α7 (L-17), Integrin α7 (H-40), Integrin α7 (C-15), Integrin α7 (012-Z), Integrin α8 (F-11), Integrin α8 (F-19), Integrin α8 (T-20), Integrin α8 (H-180), Integrin α8 (S-16), Integrin α9 (N-19), Integrin α9 (H-198), Integrin α10 (E-15), Integrin α10 (S-14), Integrin α11 (H-242), Integrin α11 (Y-16), Integrin α11 (F-5), Integrin α11 (E-20), Integrin αIIb (B-10), Integrin αIIb (B-9), Integrin αIIb (H-160), Integrin αIIb (C-20), Integrin αIIb (A-7), Integrin αIIb (K-18), Integrin αIIb (96-2C1), Integrin αIIb (MWReg30), Integrin αIIb (SZ.22), Integrin αIIb (M-148), Integrin αIIIa (158A3), Integrin αIIIa (29A3), Integrin αIIIb (54B3), Integrin αVIa (1A10), Integrin αVIb (6B4), Integrin αIIIb/VIb (PB36), Integrin αD (T-17), Integrin αE (H-260), Integrin αE (N-19), Integrin αE (Ber-ACT8), Integrin αE (R-15), Integrin αE (OX62), Integrin αE (BP6), Integrin αL (E-1), Integrin αL (C-11), Integrin αL (N-18), Integrin αL (Y-17), Integrin αL (H-300), Integrin αL (C-17), Integrin αL (F-11), Integrin αL (38), Integrin αL (16B8), Integrin αL (HI111), Integrin αL (I21/7), Integrin αL (CRIS-3), Integrin αL (27), Integrin αM (M-19), Integrin αM (H-61), Integrin αM (2LPM19c), Integrin αM (44), Integrin αM (1B6e), Integrin αM (C-19), Integrin αM (OX42), Integrin αM (VIM12), Integrin αM (LM2/1), Integrin αM (ICRF44), Integrin αM (M1/70), Integrin αM (CBRM1/5), Integrin αM (M1/70.15.11.5.HL), Integrin αM (LM2/1.6.11), Integrin αM (2B2.38), Integrin αM (CC125), Integrin αM (CC104), Integrin αM (Bear-1), Integrin αM (6A248), Integrin αM (2Q913), Integrin αM (3A33), Integrin αM (MEM-174), Integrin αV (H-2), Integrin αV (Q-20)-R, Integrin αV (P2W7), Integrin αV (H-75),

Integrin αV (T-20), Integrin αV (N-19), Integrin αV (2Q888), Integrin αV (13C2), Integrin αV (NKI-M9), Integrin αX (B-6), Integrin αX (BU15), Integrin αX (D-8), Integrin αX (G-3), Integrin αX (KB90), Integrin αX (3.9), Integrin αX (B-ly6), Integrin αX (HC1/1), Integrin αX (2Q862), Integrin αX (2B2.36), Integrin αX (2Q865), Integrin αX (F-20), Integrin αX (H-68), Integrin αX (N-19), Integrin αX (M-50), Integrin αX (C-20), Integrin αX (M-20), Integrin αX (N418), Integrin αX (R-113), Integrin αX (3H986), Integrin β1 (A-4), Integrin β1 (E-11), Integrin β1 (N-20), Integrin β1 (K-20), Integrin β1 (M-106), Integrin β1 (P5D2), Integrin β1 (L-16), Integrin β1 (HMβ1-1), Integrin β1 (4B7R), Integrin β1 (P4G11), Integrin β1 (JB1B), Integrin β1 (12G10), Integrin β1 (102DF5), Integrin β1 (TS2/16), Integrin α9/β1 (Y9A2), Integrin β1 (MEM-101A), Integrin β1 (2Q837), Integrin β1 (3H1192), Integrin α2/β1 (16B4), Integrin α9/β1 (2Q954), Integrin α2/β1 (2B2.29), Integrin α2/β1 (3H1472), Integrin β2 (CTB104), Integrin β2 (F-3), Integrin β2 (N-19), Integrin β2 (M18/2), Integrin β2 (H-120), Integrin β2 (H-7), Integrin β2 (C-20), Integrin β2 (K-19), Integrin β2 (C-4), Integrin β2 (GAME-46), Integrin β2 (P4H9), Integrin β2 (C71/16), Integrin β2 (M18/2.a.12.7), Integrin β2 (1.BB.246), Integrin β2 (IB4), Integrin aL/M/X/(32 (24), Integrin β2 (3H1041), Integrin β2 (2B2.45), Integrin β2 (YTS 213.1), Integrin β2 (6G2), Integrin β2 (2Q822), Integrin β2 (L-13), Integrin β2 (6A21), Integrin β2 (MEM-48), Integrin β2 (MEM-148), Integrin β2 (IVA35), Integrin β3 (D-11), Integrin β3 (H-96), Integrin β3 (C-20), Integrin β3 (N-20), Integrin β3 (B-7), Integrin αIIb/β3 (A2A9/6), Integrin αV/β3 (23C6), Integrin β3 (Y2/51), Integrin β3 (F-11), Integrin β3 (SAP), Integrin β3 (2C9.G2), Integrin αIIb/β3 (P256), Integrin β3 (BV4), Integrin β3 (NaM28-7D6), Integrin β3 (MHF4), Integrin β3 (5K291), Integrin β3 (PM6/13), Integrin αIIb/β3 (474), Integrin αV/β3 (BV3), Integrin αIIb/β3 (IVA30), Integrin β4 (H-101), Integrin β4 (C-20), Integrin β4 (B-4), Integrin β4 (G-7), Integrin β4 (F-7), Integrin β4 (N-20), Integrin β4 (H-1), Integrin β4 (A9), Integrin β4 (7), Integrin β5 (B-10), Integrin β5 (H-96), Integrin β5 (B-1), Integrin β5 (E-18), Integrin β5 (E-19), Integrin β5 (F-5), Integrin αV/β5 (P1F76), Integrin αV/β5 (P1F6), Integrin β5 (4AK), Integrin β6 (H-110), Integrin β6 (C-19), Integrin β6 (N-20), Integrin β7 (C-2), Integrin β7 (C-20), Integrin β7 (N-18), Integrin β7 (FIB504), Integrin β7 (H-120), Integrin β7 (E-19), Integrin α4/β7 (DATK32), Integrin β8 (E-6), Integrin β7 (LS722), Integrin β8 (G-17), Integrin β8 (H-160), Integrin β8 (C-19), Integrin βId (2B1), Integrin βId (1G2), Integrin βL1 (F-21), and Integrin βL1 (N-15). Conveniently, the integrin antibody or antibodies is/are the antibody or antibodies which is/are predominantly expressed on the surface of a given neoplastic cell and will thus vary according to the type and nature of the neoplastic cell to be treated using the recoding therapy as defined herein.

In certain embodiments, said at least one inhibitor of integrin engagement with the ECM is a Arginylglycylaspartic acid (RGD) peptide. One aspect of the present disclosure thus also provides a pharmaceutical association or combination or (therapeutic, dermatologic, ophthalmologic, diagnostic, etc.) composition as defined herein comprising at least one GFR-binding compound as defined herein and at least one RGD peptide.

In certain embodiments, the adhesion protein inhibitor as defined herein comprises (or is) an integrin deactivator or inhibitor which inhibits or down-regulates the gene or protein expression of at least one integrin by e.g. alteration of the chromatin structure (e.g. through histone modifications), regulation of gene transcription (e.g. by acting on specificity factors, repressors, general transcription factors, activators, enhancers and/or silencers), post-transcriptional regulation, and/or regulation of translation.

In one aspect, the present disclosure thus provides a pharmaceutical association or combination or (therapeutic, dermatologic, ophthalmologic, diagnostic, etc.) composition defined herein comprising at least one GFR-binding compound as defined herein and at least one small-interfering RNA (si-RNA) which interferes with or inhibit the gene or protein expression of at least one integrin.

Integrins expressed on the cell membrane of neoplastic cells may be identified using various methods. However, for the avoidance of doubts and for the purpose of the present disclosure, identification of such integrin(s) is performed using e.g. RT-PCR or western blot. Because, the type of integrins expressed on the cell membrane of a given cell (e.g. a neoplastic cell) may differ from the integrins expressed in the cell membrane of another (neoplastic) cell, prior identification of the integrins expressed on the cell membrane of a cell to be treated using a method such as RT-PCR, allows for a more tailored, personalised and/or efficient treatment to be provided.

In certain embodiments, the modulation or regulation of a neoplastic cell adhesion thus comprises (or is exclusively constituted of, or exclusively consists of) the inhibition, deactivation or down-regulation of the gene or protein expression of one or more identified integrins or dimers of integrins expressed by the neoplastic cell to be treated using, for example, conventional techniques known in the art such as gene silencing using small-interfering RNAs, microRNAs or shRNAs, integrin extracellular antibodies and/or integrin antagonists. The identification of said specific one or more integrins or dimers of integrins may be carried for each specific neoplastic cell using conventional methods in the art such as the one described herein.

Various methods known in the art may be used to qualify and/or quantify the degree of deactivation or inhibitory activity of a given adhesion protein inhibitor on the gene expression of integrins. However, for the avoidance of doubts and for the purpose of the present disclosure, assessment of integrins gene expression of a given cell by an adhesion protein inhibitor as defined herein is performed using RT-PCR as already defined herein.

Various methods known in the art may be used to qualify and/or quantify the degree of deactivation or inhibitory activity of a given adhesion protein inhibitor on the protein expression of integrins. However, for the avoidance of doubts and for the purpose of the present disclosure, assessment of integrins protein expression of a given cell by an adhesion

protein inhibitor as defined herein is performed using the western blot method as already defined herein.

Various methods known in the art may be used to qualify and/or quantify the degree of activation or inactivation/inhibition state of integrins. However, for the avoidance of doubts and for the purpose of the present disclosure, assessment of the integrins activation or inactivation state of a given cell by an adhesion protein inhibitor as defined herein is performed using FRET as already defined herein.

Also provided is a method of treating a neoplastic cell or a neoplastic disease by (partially or completely) deactivating or inhibiting at least one integrin or dimer of integrins expressed on the cell membrane of said neoplastic cell, comprising administering to a subject in need thereof an effective amount of a pharmaceutical association, combination or (therapeutic, dermatologic, ophthalmologic, diagnostic, etc.) composition as defined herein.

Also provided is a method of treating a neoplastic cell or a neoplastic disease comprising identifying at least one integrin or dimer of integrin expressed on the membrane of said neoplastic cell, and (partially or completely) deactivating or inhibiting said at least one integrin or dimer of integrin by administering to a subject in need thereof an effective amount of a pharmaceutical association, combination or (therapeutic, dermatologic, ophthalmologic, diagnostic, etc.) composition as defined herein.

### III.2. Syndecans

As used herein, unless indicated otherwise or contradictory in context, the term "syndecans" refers to single transmembrane domain proteins that are thought to act as co-receptors, especially for G protein-coupled receptors. These core proteins carry three to five heparan sulfate and chondroitin sulfate chains, which allow for interaction with a large variety of ligands including fibroblast growth factors, vascular endothelial growth factor, transforming growth factor-beta, fibronectin and antithrombin-1. Interactions between fibronectin and some syndecans can be modulated by the extracellular matrix protein tenascin C. The syndecan protein family has four members. Syndecans 1 and 3 and syndecans 2 and 4, making up separate subfamilies, arose by gene duplication and divergent evolution from a single ancestral gene. The syndecan numbers reflect the order in which the cDNAs for each family member were cloned. All syndecans have an N-terminal signal peptide, an ectodomain, a single hydrophobic transmembrane domain, and a short C-terminal cytoplasmic domain. All syndecans are anchored to plasma membrane via a 24-25 amino acid long hydrophobic transmembrane domain. In mammalian cells, syndecans are expressed by unique genes located on different chromosomes. All members of the syndecan family have 5 exons. The difference in size of the syndecans is credited to the variable length of exon 3, which encodes a spacer domain. In humans, the amino acid length of syndecan 1, 2, 3 and 4 is 310, 201, 346 and 198 respectively. Glycosaminoglycan chains, a member of the heparan sulfate group, are an important component of syndecans and are responsible for a diverse set of syndecan functions. The addition of glycosaminoglycans to syndecan is controlled by a series of post- translational events.

In the present disclosure, the pharmaceutical association, combination or (therapeutic, dermatologic, ophthalmologic, diagnostic, etc.) composition defined herein is thus suitable for treating a neoplastic cell or a neoplastic disease (i) by activating one or more growth factor receptors and (ii) by, simultaneously or non-simultaneously, modulating or regulating the adhesion of that cell with its ECM microenvironment by (partially or completely) deactivating or inhibiting at least one syndecan expressed on the cell membrane of said neoplastic cell. In certain embodiments, and without wishing to be bound to any specific theory, the deactivation or inhibition of a syndecan may be the result of, for example, syndecan's inactivation, syndecan's dimerisation inhibition, and/or syndecan's gene expression inhibition.

In certain embodiments, the adhesion protein inhibitor as defined herein comprises (or is) an syndecan deactivator or inhibitor which binds to/interacts with at least one syndecan and/or to at least one syndecan dimer. In one example, said syndecan deactivator or inhibitor is a receptor competitive antagonist. In one example, said syndecan deactivator or inhibitor is a receptor non-competitive antagonist. In one example, said syndecan deactivator or inhibitor is a receptor uncompetitive antagonist. In one example, said syndecan deactivator or inhibitor is a receptor silent antagonist. In one example, said syndecan deactivator or inhibitor is a receptor partial agonist. In one example, said syndecan deactivator or inhibitor is a receptor inverse agonist.

In one aspect, the present disclosure thus provides a pharmaceutical association or combination or (therapeutic, dermatologic, ophthalmologic, diagnostic, etc.) composition as defined herein comprising at least one GFR-binding compound as defined herein and at least one inhibitor of syndecan engagement.

In one aspect, the present disclosure thus provides a pharmaceutical association or combination or (therapeutic, dermatologic, ophthalmologic, diagnostic, etc.) composition defined herein comprising at least one GFR-binding compound as defined herein and at least one syndecan antibody.

In certain embodiments, the adhesion protein inhibitor as defined herein comprises (or is) a syndecan deactivator or inhibitor which inhibits or down-regulates the gene or protein expression of at least one syndecan by e.g. alteration of the chromatin structure (e.g. through histone modifications), regulation of gene transcription (e.g. by acting on specificity factors, repressors, general transcription factors, activators, enhancers and/or silencers), post-transcriptional regulation, and/or regulation of translation.

In one aspect, the present disclosure thus provides a pharmaceutical association or combination or (therapeutic, dermatologic, ophthalmologic, diagnostic, etc.) composition defined herein comprising at least one GFR-binding compound as defined herein and at least one small-interfering RNA (si-RNA) which interferes with or inhibit the gene or protein expression of at least one syndecan.

Syndecans expressed on the cell membrane of neoplastic cells may be identified using various methods. However, for the avoidance of doubts and for the purpose of the present disclosure, identification of such syndecan is performed using e.g. RT-PCR or western blot. Because, the type of syndecans expressed on the cell membrane of a given cell (e.g. a neoplastic cell) may differ from the syndecans expressed in the cell membrane of another (neoplastic) cell, prior identification of the syndecans expressed on the cell membrane of a cell to be treated using a method such as RT-PCR, allows for a more tailored, personalised and/or efficient treatment to be provided.

In certain embodiments, the modulation or regulation of a neoplastic cell adhesion thus comprises (or is exclusively constituted of, or exclusively consists of) the inhibition, deactivation or down-regulation of the gene or protein expression of one or more identified syndecans or dimers of syndecans expressed by the neoplastic cell to be treated using, for example, conventional techniques known in the art such as gene silencing using small-interfering RNAs, microRNAs, or shRNAs, syndecan extracellular antibodies and/or syndecan antagonists. The identification of said specific one or more syndecans or dimers of syndecans may be carried for each specific neoplastic cell using conventional methods in the art such as the one described herein.

Various methods known in the art may be used to qualify and/or quantify the degree of deactivation or inhibitory activity of a given adhesion protein inhibitor on the gene expression of syndecans. However, for the avoidance of doubts and for the purpose of the present disclosure, assessment of syndecans gene expression of a given cell by an adhesion protein inhibitor as defined herein is performed using RT-PCR as already defined herein.

Various methods known in the art may be used to qualify and/or quantify the degree of deactivation or inhibitory activity of a given adhesion protein inhibitor on the protein expression of syndecans. However, for the avoidance of doubts and for the purpose of the present disclosure, assessment of syndecans protein expression of a given cell by an adhesion protein inhibitor as defined herein is performed using the western blot method as already defined herein.

Various methods known in the art may be used to qualify and/or quantify the degree of activation or inactivation/inhibition state of syndecans. However, for the avoidance of doubts and for the purpose of the present disclosure, assessment of the syndecans activation or inactivation state of a given cell by an adhesion protein inhibitor as defined herein is performed using FRET as already defined herein.

Also provided is a method of treating a neoplastic cell or a neoplastic disease by (partially or completely) deactivating or inhibiting at least one syndecan or dimer of syndecans expressed on the cell membrane of said neoplastic cell, comprising administering to a subject in need thereof an effective amount of a pharmaceutical association, combination or (therapeutic, dermatologic, ophthalmologic, diagnostic, etc.) composition as defined herein.

Also provided is a method of treating a neoplastic cell or a neoplastic disease comprising identifying at least one syndecan or dimer of syndecan expressed on the membrane of said neoplastic cell, and (partially or completely) deactivating or inhibiting said at least one syndecan or dimer of syndecan by administering to a subject in need thereof an effective amount of a pharmaceutical association, combination or (therapeutic, dermatologic, ophthalmologic, diagnostic, etc.) composition as defined herein.

### 111.3. Selectins

The selectins are a family of cell adhesion molecules. All selectins are single-chain transmembrane glycoproteins. Selectins bind to sugar moieties and so are considered to be a type of lectin, cell adhesion proteins that bind sugar polymers. All three known members of the selectin family (L-, E-, and P- selectin) share a similar cassette structure: an N-terminal, calcium-dependent lectin domain, an epidermal growth factor (EGF)-like domain, a variable number of consensus repeat units (2, 6, and 9 for L-, E-, and P-selectin, respectively), a transmembrane domain (TM) and an intracellular cytoplasmic tail (cyto). Selectins have hinge domains, allowing them to undergo rapid conformational changes in the nanosecond range between 'open' and 'closed' conformations. Shear stress on the selectin molecule causes it to favor the 'open' conformation.

In the present disclosure, the pharmaceutical association, combination or (therapeutic, dermatologic, ophthalmologic, diagnostic, etc.) composition defined herein is thus suitable for treating a neoplastic cell or a neoplastic disease (i) by activating one or more growth factor receptors and (ii) by, simultaneously or non-simultaneously, modulating or regulating the adhesion of that cell to its ECM microenvironment by (partially or completely) deactivating or inhibiting at least one selectin expressed on the cell membrane of said neoplastic cell. In certain embodiments, and without wishing to be bound to any specific theory, the deactivation or inhibition of a selectin may be the result of, for example, selectin's inactivation, selectin's dimerisation inhibition, and/or selectin's gene expression inhibition.

In certain embodiments, the adhesion protein inhibitor as defined herein comprises (or is) an selectin deactivator or inhibitor which binds to/interacts with at least one selectin and/or to at least one selectin dimer. In one example, said

selectin deactivator or inhibitor is a receptor competitive antagonist. In one example, said selectin deactivator or inhibitor is a receptor non-competitive antagonist. In one example, said selectin deactivator or inhibitor is a receptor uncompetitive antagonist. In one example, said selectin deactivator or inhibitor is a receptor silent antagonist. In one example, said selectin deactivator or inhibitor is a receptor partial agonist. In one example, said selectin deactivator or inhibitor is a receptor inverse agonist.

In one aspect, the present disclosure thus provides a pharmaceutical association or combination or (therapeutic, dermatologic, ophthalmologic, diagnostic, etc.) composition as defined herein comprising at least one GFR-binding compound as defined herein and at least one inhibitor of selectin engagement.

In one aspect, the present disclosure thus provides a pharmaceutical association or combination or (therapeutic, dermatologic, ophthalmologic, diagnostic, etc.) composition defined herein comprising at least one GFR-binding compound as defined herein and at least one selectin antibody.

In one example, said selectin antibody is selected from the group consisting of E-Selectin (H-300), E-Selectin (D-7), E-Selectin (CTB202), E-Selectin (A-10), E-Selectin (C-20), E-Selectin (P2H3), E-Selectin (M-20), E-Selectin (HAE-If), E-Selectin (1.2B6), E-Selectin (UZ5), E-Selectin (UZ6), E-Selectin (2Q780), E-Selectin (UZ4), L-Selectin (lam1-116), L-Selectin (N-18), L-Selectin (H-149), L-Selectin (FMC46), L-Selectin (DREG56), L-Selectin (B-8), L-Selectin (DREG55), L-Selectin (OX85), L-Selectin (3H1611), P-Selectin (CTB201), P-Selectin (H-150), P-Selectin (C-20), P-Selectin (M-20), P-Selectin (AK4), P-Selectin (1 E 3), P-Selectin (H-2), P-Selectin (P8G6), P-Selectin (Psel.KO.2.9), P-Selectin (Psel.KO.2.7) and P-Selectin (CLB/thromb/6). Conveniently, the selectin antibody or antibodies is/are the antibody or antibodies which is/are predominantly expressed on the surface of a given neoplastic cell and will thus vary according to the type and nature of the neoplastic cell to be treated using the recoding therapy as defined herein.

In certain embodiments, the adhesion protein inhibitor as defined herein comprises (or is) a selectin deactivator or inhibitor which inhibits or down-regulates the gene or protein expression of at least one selectin by e.g. alteration of the chromatin structure (e.g. through histone modifications), regulation of gene transcription (e.g. by acting on specificity factors, repressors, general transcription factors, activators, enhancers and/or silencers), post-transcriptional regulation, and/or regulation of translation.

In one aspect, the present disclosure thus provides a pharmaceutical association or combination or (therapeutic, dermatologic, ophthalmologic, diagnostic, etc.) composition defined herein comprising at least one GFR-binding compound as defined herein and at least one small-interfering RNA (si-RNA) which interferes with or inhibit the gene or protein expression of at least one selectin.

Selectins expressed on the cell membrane of neoplastic cells may be identified using various methods. However, for the avoidance of doubts and for the purpose of the present disclosure, identification of such selectin(s) is performed using e.g. RT-PCR or western blot. Because, the type of selectins expressed on the cell membrane of a given cell (e.g. a neoplastic cell) may differ from the selectins expressed in the cell membrane of another (neoplastic) cell, prior identification of the selectins expressed on the cell membrane of a cell to be treated using a method such as RT-PCR, allows for a more tailored, personalised and/or efficient treatment to be provided.

In certain embodiments, the modulation or regulation of a neoplastic cell adhesion thus comprises (or is exclusively constituted of, or exclusively consists of) the inhibition, deactivation or down-regulation of the gene or protein expression of one or more identified selectins or dimers of selectins expressed by the neoplastic cell to be treated using, for example, conventional techniques known in the art such as gene silencing using small-interfering RNAs, microRNAs, or shRNAs, selectin extracellular antibodies and/or selectin antagonists. The identification of said specific one or more selectins or dimers of selectins may be carried for each specific neoplastic cell using conventional methods in the art such as the one described herein.

Various methods known in the art may be used to qualify and/or quantify the degree of deactivation or inhibitory activity of a given adhesion protein inhibitor on the gene expression of selectins. However, for the avoidance of doubts and for the purpose of the present disclosure, assessment of selectins gene expression of a given cell by an adhesion protein inhibitor as defined herein is performed using RT-PCR as already defined herein.

Various methods known in the art may be used to qualify and/or quantify the degree of deactivation or inhibitory activity of a given adhesion protein inhibitor on the protein expression of selectins. However, for the avoidance of doubts and for the purpose of the present disclosure, assessment of selectins protein expression of a given cell by an adhesion protein inhibitor as defined herein is performed using the western blot method as already defined herein.

Various methods known in the art may be used to qualify and/or quantify the degree of activation or inactivation/inhibition state of selectins. However, for the avoidance of doubts and for the purpose of the present disclosure, assessment of the selectins activation or inactivation state of a given cell by an adhesion protein inhibitor as defined herein is performed using FRET as already defined herein.

Also provided is a method of treating a neoplastic cell or a neoplastic disease by (partially or completely) deactivating or inhibiting at least one selectin or dimer of selectins expressed on the cell membrane of said neoplastic cell, comprising administering to a subject in need thereof an effective amount of a pharmaceutical association, combination or (therapeutic, dermatologic, ophthalmologic, diagnostic, etc.) composition as defined herein.

Also provided is a method of treating a neoplastic cell or a neoplastic disease comprising identifying at least one selectin or dimer of selectin expressed on the membrane of said neoplastic cell, and (partially or completely) deactivating or inhibiting said at least one selectin or dimer of selectin by administering to a subject in need thereof an effective amount of a pharmaceutical association, combination or (therapeutic, dermatologic, ophthalmologic, diagnostic, etc.) composition as defined herein.

### 111.4. Dystroglycans

Dystroglycan is a protein that in humans is encoded by the *DAG1* gene. Dystroglycan is one of the dystrophin-associated glycoproteins, which is encoded by a 5.5 kb transcript in *Homo sapiens* on chromosome 3. There are two exons that are separated by a large intron. The spliced exons code for a protein product that is finally cleaved into two non-covalently associated subunits, [alpha] (N-terminal) and [beta] (C-terminal). The dystroglycan complex works as a transmembrane linkage between the extracellular matrix and the cytoskeleton. [alpha]-dystroglycan is extracellular and binds to merosin [alpha]-2 laminin in the basement membrane, while [beta]-dystroglycan is a transmembrane protein and binds to dystrophin, which is a large rod-like cytoskeletal protein. Dystrophin binds to intracellular actin fibers. In this way, the dystroglycan complex, which links the extracellular matrix to the intracellular actin fibers, is thought to provide structural integrity in muscle tissues.

In the present disclosure, the pharmaceutical association, combination or (therapeutic, dermatologic, ophthalmologic, diagnostic, etc.) composition defined herein is thus suitable for treating a neoplastic cell or a neoplastic disease (i) by activating one or more growth factor receptors and (ii) by, simultaneously or non-simultaneously, modulating or regulating the adhesion of that cell to its ECM microenvironment by (partially or completely) deactivating or inhibiting at least one dystroglycan expressed on the cell membrane of said neoplastic cell. In certain embodiments, and without wishing to be bound to any specific theory, the deactivation or inhibition of a dystroglycan may be the result of, for example, dystroglycan's inactivation and/or dystroglycan's gene expression inhibition.

In certain embodiments, the adhesion protein inhibitor as defined herein comprises (or is) an dystroglycan deactivator or inhibitor which binds to/interacts with at least one dystroglycan and/or to at least one dystroglycan dimer. In one example, said dystroglycan deactivator or inhibitor is a receptor competitive antagonist. In one example, said dystroglycan deactivator or inhibitor is a receptor non-competitive antagonist. In one example, said dystroglycan deactivator or inhibitor is a receptor uncompetitive antagonist. In one example, said dystroglycan deactivator or inhibitor is a receptor silent antagonist. In one example, said dystroglycan deactivator or inhibitor is a receptor partial agonist. In one example, said dystroglycan deactivator or inhibitor is a receptor inverse agonist.

In one aspect, the present disclosure thus provides a pharmaceutical association or combination or (therapeutic, dermatologic, ophthalmologic, diagnostic, etc.) composition as defined herein comprising at least one GFR-binding compound as defined herein and at least one inhibitor of dystroglycan engagement.

In one aspect, the present disclosure thus provides a pharmaceutical association or combination or (therapeutic, dermatologic, ophthalmologic, diagnostic, etc.) composition defined herein comprising at least one GFR-binding compound as defined herein and at least one dystroglycan antibody.

In certain embodiments, the adhesion protein inhibitor as defined herein comprises (or is) a dystroglycan deactivator or inhibitor which inhibits or down-regulates the gene or protein expression of at least one dystroglycan by e.g. alteration of the chromatin structure (e.g. through histone modifications), regulation of gene transcription (e.g. by acting on specificity factors, repressors, general transcription factors, activators, enhancers and/or silencers), post-transcriptional regulation, and/or regulation of translation.

In one aspect, the present disclosure thus provides a pharmaceutical association or combination or (therapeutic, dermatologic, ophthalmologic, diagnostic, etc.) composition defined herein comprising at least one GFR-binding compound as defined herein and at least one small-interfering RNA (si-RNA) which interferes with or inhibit the gene or protein expression of at least one dystroglycan.

Dystroglycans expressed on the cell membrane of neoplastic cells may be identified using various methods. However, for the avoidance of doubts and for the purpose of the present disclosure, identification of such dystroglycan(s) is performed using e.g. RT-PCR or western blot. Because, the type of dystroglycans expressed on the cell membrane of a given cell (e.g. a neoplastic cell) may differ from the dystroglycans expressed in the cell membrane of another (neoplastic) cell, prior identification of the dystroglycans expressed on the cell membrane of a cell to be treated using a method such as RT-PCR, allows for a more tailored, personalised and/or efficient treatment to be provided.

In certain embodiments, the modulation or regulation of a neoplastic cell adhesion thus comprises (or is exclusively constituted of, or exclusively consists of) the inhibition, deactivation or down-regulation of the gene or protein expression of one or more identified dystroglycans expressed by the neoplastic cell to be treated using, for example, conventional techniques known in the art such as gene silencing using small-interfering RNAs, microRNAs, or shRNAs, dystroglycan extracellular antibodies and/or dystroglycan antagonists. The identification of said specific one or more dystroglycans may be carried for each specific neoplastic cell using conventional methods in the art such as the one

described herein.

Various methods known in the art may be used to qualify and/or quantify the degree of deactivation or inhibitory activity of a given adhesion protein inhibitor on the gene expression of dystroglycans. However, for the avoidance of doubts and for the purpose of the present disclosure, assessment of dystroglycans gene expression of a given cell by an adhesion protein inhibitor as defined herein is performed using RT-PCR as already defined herein.

Various methods known in the art may be used to qualify and/or quantify the degree of deactivation or inhibitory activity of a given adhesion protein inhibitor on the protein expression of dystroglycans. However, for the avoidance of doubts and for the purpose of the present disclosure, assessment of dystroglycans protein expression of a given cell by an adhesion protein inhibitor as defined herein is performed using the western blot method as already defined herein.

Various methods known in the art may be used to qualify and/or quantify the degree of activation or inactivation/inhibition state of dystroglycans. However, for the avoidance of doubts and for the purpose of the present disclosure, assessment of the dystroglycans activation or inactivation state of a given cell by an adhesion protein inhibitor as defined herein is performed using FRET as already defined herein.

Also provided is a method of treating a neoplastic cell or a neoplastic disease by (partially or completely) deactivating or inhibiting at least one dystroglycan expressed on the cell membrane of said neoplastic cell, comprising administering to a subject in need thereof an effective amount of a pharmaceutical association, combination or (therapeutic, dermatologic, ophthalmologic, diagnostic, etc.) composition as defined herein.

Also provided is a method of treating a neoplastic cell or a neoplastic disease comprising identifying at least one dystroglycan expressed on the membrane of said neoplastic cell, and (partially or completely) deactivating or inhibiting said at least one dystroglycan by administering to a subject in need thereof an effective amount of a pharmaceutical association, combination or (therapeutic, dermatologic, ophthalmologic, diagnostic, etc.) composition as defined herein.

## IV. Pharmaceutical associations or combinations

In one aspect, the present disclosure provides a pharmaceutical association or combination which may be used for converting or recoding, in-vitro, ex-vivo or in-vivo, a neoplastic cell into a non-neoplastic cell comprising at least one GFR-binding compound and at least one adhesion protein inhibitor, both as defined herein. In one example, said adhesion protein inhibitor is an integrin inhibitor inhibiting or preventing the activation of at least one integrin expressed on the surface of said neoplastic cell. In one example, said adhesion protein inhibitor comprises a syndecan inhibitor inhibiting or preventing the activation of at least one syndecan expressed on the surface of said neoplastic cell. In one example, said adhesion protein inhibitor comprises an integrin inhibitor and a syndecan inhibitor inhibiting or preventing the activation of at least one integrin and at least one syndecan expressed on the surface of said neoplastic cell.

The present pharmaceutical associations or combinations may thus also be used for protecting a subject carrying a neoplastic cell from a neoplastic disease.

In one aspect, the present disclosure provides a pharmaceutical association or combination for the uses disclosed herein, substantially free from any cell adhesion promoter. As used herein, the term "substantially free", as applied to a given component such as a cell adhesion promoter, means that the amount of such a component is less than 20%, 15%, 10%, 5%, 1%, 0.5%, 0.1% or less in mole with respect to the mole content of GFR-binding compound unless otherwise indicated, self-evident or contradictory in context.

In one aspect, the present disclosure provides a pharmaceutical association as defined herein further comprising (another) anti-cancer agent thus forming a pharmaceutical composition of the invention. In one example, said pharmaceutical composition further comprises at least one pharmaceutically acceptable excipient. In one example, said further anti-cancer agent is functionally associated with said GFR-binding compound and/or said adhesion protein inhibitor. Suitable further anti-cancer agents include but are not limited to, agents that inhibit the synthesis of DNA molecule building blocks, agents that directly damage DNA in the cell nucleus, agents that affect the synthesis or breakdown of mitotic spindles, and agents that inhibit kinase proteins by interacting with the kinase active site. Preferred examples of agents that inhibit the synthesis of DNA molecule building blocks include, but are not limited to, methotrexate (Abitrexate®), fluorouracil (Adrucil®), gemcitabine (Gemzar®), arabinosylcytosine (araC), hydroxyurea (Hydrea®), and mercaptopurine (Purinethol®). Preferred examples of agents that directly damage DNA in the cell nucleus include, but are not limited to, carboplatin (Paraplatin® and paraplatin-AQ®), cisplatin (Platinol®) and antibiotics such as daunorubicin (Cerubidine®), doxorubicin (Adriamycin®), and etoposide (VePesid®). Preferred examples of agents that affect the synthesis or breakdown of mitotic spindles include, but are not limited to, miotic disrupters such as Vinblastine (Velban@), Vincristine (Oncovin®) and Pacitaxel (Taxol®). Preferred examples of agents that inhibit kinase proteins include, but are not limited to, Afatinib®, Axitinib®, Bosulif®, Bosutinib®, Cabozantinib®, Caprelsa®, Cometriq®, Crizotinib®, Dasatinib®, Erlotinib®, Gilotrif®, Gleevec®, Ibrutinib®, Iclusig®, Imatinib®, Imbruvica®, Inlyta®, Lapatinib®, Nexavar®, Nilotinib®, Pazopanib®, Ponatinib®, Regorafenib®, Sorafenib®, Sprycel®, Stivarga®, Sunitinib®, Sutent®, Tarceva®, Tasigna®, Tivopath®, Tivozanib®, Tykerb®, Vandetanib®, Votrient®, Xalkori®, Zaltrap®, and ziv-aflibercept®.

In one example, the L-asparaginase enzyme may also be used as a further agent in combination with the pharmaceutical association or composition as defined herein. L-asparaginase enzyme has been reported e.g. in L-Asparaginase: A Promising Enzyme for Treatment of Acute Lymphoblastic Leukiemia, People's Journal of Scientific Research, Vol. 5(1), Jan. 2012, which is incorporated herein by reference in its entirety, to act by depriving cancer cells (such as leukemia cells) of asparagine thus inducing their death.

Other anti-cancer agents may also be used such as nitrogen mustards, ethylenimes, alkylsulfonates, triazenes, piperazines, nitrosureas and antibiotics such as anthracyclines, dactinomycin, bleomycin, adriamycin, or mithramycin.

In one example, said pharmaceutical association or combination comprises one GFR-binding compound. In one example, said pharmaceutical association or combination comprises two or more distinct GFR-binding compounds. In one example, said pharmaceutical association or combination comprises three or more distinct GFR-binding compounds. In one example, said pharmaceutical association or combination comprises four or more distinct GFR-binding compounds.

In one aspect, the present disclosure provides a process or method for manufacturing a neoplastic disease medicament, said process or method comprising associating or combining an adhesion protein inhibitor with a GFR-binding compound both as defined herein. Said step of associating or combining may be carried out using any suitable method known in the art.

In one aspect, the present disclosure provides a process or method for manufacturing a neoplastic disease medicament precursor, said process or method comprising providing a GFR-binding compound and/or an adhesion protein inhibitor both as defined herein, wherein providing said adhesion protein inhibitor and/or said GFR-binding compound manufactures said neoplastic disease medicament.

In certain aspects, said pharmaceutical association or composition substantially down-regulates, reduces, inhibits or suppresses the gene and/or protein expression of at least one of cyclin-D1, cyclin-D2 or cyclin-D3. Because cyclins D1, D2 and D3 regulate the activity of Cyclin-dependent kinases (CDKs) 4 and 6 by forming cyclin-CDK protein complexes including Cyclin D1-CDK4 complex, Cyclin D1-CDK6 complex, Cyclin D2-CDK4 complex, Cyclin D2-CDK6 complex, Cyclin D3-CDK4 complex and Cyclin D3-CDK6 complex, said pharmaceutical association or composition was also observed to substantially reduce, inhibit, suppress or destabilise the formation of any one of such complexes.

There are many ways to test, measure and represent the inhibitory effect of a given substance on the gene or protein expression of cyclins D, but for the purpose of the present disclosure, and for the avoidance of any doubts, the inhibition values of a given pharmaceutical association or composition as defined herein is a measure of the gene expression of cyclins D as provided by RT-PCR. It is to be understood that the values of the gene expression of cyclins D disclosed herein correspond to the total gene expression of all cyclins D present in the tested cell i.e. D1, D2 and D3 as known at the date of the present disclosure.

As already stated above, pharmaceutical associations or compositions suitable for implementing embodiments of the present invention reduce the gene expression of cyclins D by at least 20% during at least one part of the G1 phase of the cell cycle as compared to the wild-type expression. The absolute and relative duration of each cell cycle phase (in other words, the cell cycle duration profile) usually varies (some phases such as the Gap phases may "shrink") amongst healthy cells of different types (e.g. bone cells, skin cells, etc...) and between healthy cells and neoplastic cells of the same type (e.g. healthy bone cells and neoplastic bone cells). Typical average cell cycle duration is commonly accepted to be about 24 hours. Typically accepted phase durations for a healthy cell are 11 to 14 hours for the G1 phase, 5 to 12 hours for the S phase, 3 to 12 hours for the G2 phase and about 1 hour for the M phase. In neoplastic cells, such as in cancer cells, it is commonly admitted that, although the duration of the S and M phases may generally be conserved, the length of the G1 and G2 phases is generally shortened in order to increase cell division. Cell cycle phase's duration may thus vary significantly from one cell type to another. Consequently, conventionally and for the purpose of facilitating the comparative representation of the inhibition of the gene expression of cyclins D for different cell types, the gene expression is represented as a function of the cell cycle progression (starting from G1 and finishing with M) and not as a direct function of time.

**Active or bioactive principles or ingredients:** In the present description and unless otherwise indicated or contradictory in context, the term "(bio)active principle" or "(bio)active ingredient" generally refers to a molecule, compound or substance which is responsible for providing the desired biological effect. Without said active ingredient, the formulation or composition containing it, would not provide the desired biological effect. For example, in certain embodiments, formulation excipients are not considered as active ingredients in the pharmaceutical composition as defined herein.

In the present disclosure, said GFR-binding compound and adhesion protein inhibitor or antagonist are both active principles/ingredients.

**Neoplastic disease medicament:** In the present description and unless otherwise indicated or contradictory in context, the term "neoplastic disease medicament" means a substance, compound, pharmaceutical association, combination, composition or formulation which is suitable for treating or preventing a neoplastic disease in a subject.

**Subject carrying a neoplastic cell:** In the present description and unless otherwise indicated or contradictory

in context, the term "subject carrying a neoplastic cell" means that at least one cell constitutive of the subject is a neoplastic cell as defined herein.

In one particular example, said pharmaceutical association or combination comprises at least one GFR-binding compound selected from the group consisting of peptides of SEQ ID NO: 1 to 100371.

The present disclosure provides a pharmaceutical association or combination comprising a GFR-binding compound, wherein all of PEP1, PEP2, PEP3, PEP4, PEP5, PEP6, PEP9, PEP10, PEP11, PEP12, $AA^{17}$, pairs, quadruplets, hexaplets and heptuplets thereof, disclaimers and provisos, are as already defined herein.

In one example, an adhesion protein inhibitor and a GFR-binding compound are covalently or non-covalently associated or combined using a method comprising, or exclusively consisting of, contacting an adhesion protein inhibitor and a GFR-binding compound under suitable reacting conditions thereby forming a covalent or non-covalent association between said adhesion protein inhibitor and said GFR-binding compound.

In one example, said pharmaceutical association, combination or composition comprises at least 1 part of said adhesion protein inhibitor for 100 parts of GFR-binding compound. In one example, said pharmaceutical association, combination or composition comprises at least 5 parts of said adhesion protein inhibitor for 100 parts of GFR-binding compound. In one example, said pharmaceutical association, combination or composition comprises at least 10 parts of said adhesion protein inhibitor for 100 parts of GFR-binding compound. In one example, said pharmaceutical association, combination or composition comprises at least 15 parts of said adhesion protein inhibitor for 100 parts of GFR-binding compound. In one example, said pharmaceutical association, combination or composition comprises at least 20 parts of said adhesion protein inhibitor for 100 parts of GFR-binding compound. In one example, said pharmaceutical association, combination or composition comprises at least 25 parts of said adhesion protein inhibitor for 100 parts of GFR-binding compound. In one example, said pharmaceutical association, combination or composition comprises at least 30 parts of said adhesion protein inhibitor for 100 parts of GFR-binding compound. In one example, said pharmaceutical association, combination or composition comprises at least 35 parts of said adhesion protein inhibitor for 100 parts of GFR-binding compound. In one example, said pharmaceutical association, combination or composition comprises at least 40 parts of said adhesion protein inhibitor for 100 parts of GFR-binding compound. In one example, said pharmaceutical association, combination or composition comprises at least 45 parts of said adhesion protein inhibitor for 100 parts of GFR-binding compound. In one example, said pharmaceutical association, combination or composition comprises at least 50 parts of said adhesion protein inhibitor for 100 parts of GFR-binding compound. In one example, said pharmaceutical association, combination or composition comprises at least 55 parts of said adhesion protein inhibitor for 100 parts of GFR-binding compound. In one example, said pharmaceutical association, combination or composition comprises at least 60 parts of said adhesion protein inhibitor for 100 parts of GFR-binding compound. In one example, said pharmaceutical association, combination or composition comprises at least 65 parts of said adhesion protein inhibitor for 100 parts of GFR-binding compound. In one example, said pharmaceutical association, combination or composition comprises at least 70 parts of said adhesion protein inhibitor for 100 parts of GFR-binding compound. In one example, said pharmaceutical association, combination or composition comprises at least 75 parts of said adhesion protein inhibitor for 100 parts of GFR-binding compound. In one example, said pharmaceutical association, combination or composition comprises at least 80 parts of said adhesion protein inhibitor for 100 parts of GFR-binding compound. In one example, said pharmaceutical association, combination or composition comprises at least 85 parts of said adhesion protein inhibitor for 100 parts of GFR-binding compound. In one example, said pharmaceutical association, combination or composition comprises at least 90 parts of said adhesion protein inhibitor for 100 parts of GFR-binding compound. In one example, said pharmaceutical association, combination or composition comprises at least 95 parts of said adhesion protein inhibitor for 100 parts of GFR-binding compound. In one example, said pharmaceutical association, combination or composition comprises at least 100 parts of said adhesion protein inhibitor for 100 parts of GFR-binding compound.

## V. Polynucleotides

The extracellular biological action of the herein-defined pharmaceutical association or combination may also be conveyed via expression, by a cell, of the appropriate polynucleotide sequence engineered to encode a particular GFR-binding peptide of interest and/or a particular adhesion protein inhibitor or antagonist of interest. One may thus inject or administer, to a mammal subject, at least one GFR-binding-peptide-encoding polynucleotide or at least one adhesion protein inhibitor- or antagonist-encoding polynucleotide (such as a messenger RNA), wherein said polynucleotide would enable the intracellular production of the encoded GFR-binding peptide or adhesion protein inhibitor or antagonist, which may, once released outside the host cell, exert its extracellular action on the host cell and/or on neighbouring cells and/or distant cells in combination with its complementary extracellular biologically active partner (i.e. a GFR-binding-peptide or an adhesion protein inhibitor or antagonist, as the case may be). In other words, GFR-binding peptides or adhesion protein inhibitors or antagonists may be produced ex-vivo (e.g. using a peptide synthesizer or recombinantly produced) or in-vivo (e.g. via cell expression of encoding polynucleotides), and in all cases have an extracellular combined biological action of (i) activation of growth factor receptors and (ii) inhibition, deactivation, or

disengagement action of at least one adhesion protein, to induce cell conversion or recoding of a neoplastic cell into a non-neoplastic cell.

Alternatively, one may also inject or administer, to a mammal subject, at least one GFR-binding-peptide-encoding polynucleotide and at least one adhesion protein inhibitor- or antagonist-encoding polynucleotide (such as a messenger RNA), wherein said polynucleotides would enable the intracellular production of both, the encoded GFR-binding peptide and the adhesion protein inhibitor or antagonist, which may, once released outside the host cell, exert their combined extracellular action on the host cell and/or on neighbouring cells and/or distant cells. In other words, GFR-binding peptides and adhesion protein inhibitors or antagonists may all be produced ex-vivo (e.g. using a peptide synthesizer or recombinantly produced) or in-vivo (e.g. via cell expression of encoding polynucleotides), and in all cases have an extracellular combined biological action of (i) activation of growth factor receptors and (ii) inhibition, deactivation, or disengagement action of at least one adhesion protein, to induce cell conversion or recoding of a neoplastic cell into a non-neoplastic cell.

Thus, in one aspect, the present disclosure provides a pharmaceutical composition comprising at least one polynucleotide encoding a GFR-binding peptide as disclosed herein and at least one polypeptide encoding an adhesion protein inhibitor or antagonist.

Thus, in one aspect, the present disclosure also provides a pharmaceutical composition comprising at least one polynucleotide encoding a GFR-binding peptide as disclosed herein and at least one polypeptide encoding an adhesion protein inhibitor or antagonist having at least one, more than one, or all of, the features described herein with respect to the pharmaceutical association or combination comprising at least one GFR-binding peptide and at least one adhesion protein inhibitor or antagonist.

In one particular example, said polynucleotide is a messenger RNA or a primary construct thereof. Said messenger RNA may additionally have a 5’ cap structure chosen from the group consisting of $m^7G(5')ppp(5')A$,G(5’)ppp(5’)A and G(5’)ppp(5’)G. In one example, the messenger RNA additionally has a poly-A tail of from about 10 to 200 adenosine nucleotides. In one example, the messenger RNA additionally has a poly-C tail of from about 10 to 200 cytosine nucleotides. In one example, the messenger RNA additionally codes a tag for purification chosen from the group consisting of a hexahistidine tag (HIS tag, polyhistidine tag), a streptavidin tag (Strep tag), an SBP tag (streptavidin-binding tag) or a GST (glutathione S-transferase) tag, or codes for a tag for purification via an antibody epitope chosen from the group consisting of antibody-binding tags, a Myc tag, a Swal 1 epitope, a FLAG tag or an HA tag. In one example, the messenger RNA additionally codes a signal peptide and/or a localization sequence, in particular a secretion sequence. In one example, said polynucleotide is a complementary DNA of said messenger RNA or a primary construct thereof.

In one aspect, the present disclosure also provides a vector comprising a polynucleotide as defined in the present disclosure.

In one aspect, the present disclosure also provides a cultured cell (or transfected cell) comprising a vector as defined in the present disclosure.

In one aspect, the present disclosure also provides a method of expressing a peptide of interest, variant or analog thereof, in a mammalian cell, said method comprising: (i) providing an mRNA as defined in the present disclosure; and (ii) introducing said mRNA to a mammalian cell under conditions that permit the expression of the peptide of interest by the mammalian cell.

In one aspect, the present disclosure also provides a method of expressing a pharmaceutical association or combination of interest, in a mammalian cell, said method comprising: (i) providing mRNAs for every components of a pharmaceutical association or combination as defined in the present disclosure; and (ii) introducing said mRNAs to a mammalian cell under conditions that permit the expression of all the components of the pharmaceutical association or combination by the mammalian cell.

In one aspect, the present disclosure also provides a pharmaceutical composition comprising mRNA molecules as disclosed herein for use in a medical treatment or prophylactic method. In one example, said medical treatment method is a therapeutic, surgical, or diagnostic method. In one particular example, said method is a method for converting or recoding a neoplastic cell into a non-neoplastic cell as defined in the present disclosure. In one particular example, said method is a method for protecting from cancer as disclosed herein.

In one aspect, the present disclosure also provides a use of mRNA molecules as defined herein, for the preparation of a pharmaceutical composition for the treatment or prevention of a neoplastic disease as defined herein.

In one aspect, the present disclosure also provides a medical composition comprising one or more polynucleotide(s), one or more vector(s), or one or more transfected cell(s), all as defined in the present disclosure, and a medically acceptable excipient or carrier.

**Expression:** As used herein, "expression" of a nucleic acid sequence refers to one or more of the following events: (1) production of an RNA template from a DNA sequence (e.g., by transcription); (2) processing of an RNA transcript (e.g., by splicing, editing, 5’ cap formation, and/or 3’ end processing); (3) translation of an RNA into a polypeptide or protein; and (4) post-translational modification of a polypeptide or protein.

**mRNA:** As used herein, the term "mRNA" refers to messenger RNA. Traditionally, the basic components of

an mRNA molecule include at least a coding region, a 5'UTR, a 3'UTR, a 5'cap and a poly-A tail. Whereas the 5'UTR, 3'UTR, 5'cap and the poly-A tail are usually required to improve e.g. stability, translation and/or recognition by the ribosome, it is the coding region which comprises the sequence encoding the protein(s), polypeptide(s), or peptide(s) of (therapeutic) interest. Therefore, when the mRNA molecule as disclosed herein is conventionally described with reference to its coding region, any mRNA molecule also comprising at least one of a 5'UTR, a 3'UTR, a 5'cap or a poly-A tail forms an integral part of the present disclosure.

**Coding region:** As used herein, the term "coding region" or "coding sequence" refers to a portion of a polynucleotide that codes for a peptide or peptides of interest.

**Primary RNA construct or transcript:** As used herein, unless indicated otherwise or contradictory in context, the term "Primary RNA construct" or "Primary RNA transcript" refers to any precursor RNA molecule from a mature and functional (i.e. translatable) RNA molecule may be obtained. For instance, a precursor messenger RNA (pre-mRNA) is a type of primary transcript that becomes a messenger RNA (mRNA) after processing. Newly synthesized primary transcripts are modified in several ways to yield their mature form before they can be translated into a protein of interest. Such modifications include, but are not limited to, excision of introns, splicing of exons, addition of 5'cap and poly-A tail. Therefore, when reference is made to an RNA molecule, it shall be understood that it aims to cover all RNA molecules including, but not limited to, primary RNA transcripts or constructs at any stage of the modification process leading to a mature and functional RNA molecule e.g. with or without introns, exons, 5'cap, poly-A tail and/or any other conventional modifications, insofar as the RNA molecule contains a coding region or a precursor thereof allowing for a peptide of interest encoded by said coding region or precursor thereof to be expressed.

**5' Capping:** As used herein, unless indicated otherwise or contradictory in context, the term "5' Capping" ou "5' Cap" refers to a 5' cap structure of an mRNA that is involved in nuclear export, increasing mRNA stability and binds the mRNA Cap Binding Protein (CBP), which is responsible for mRNA stability in the cell and translation competency through the association of CBP with poly(A) binding protein to form the mature cyclic mRNA species. The cap further assists the removal of 5' proximal introns removal during mRNA splicing. Endogenous mRNA molecules may be 5 '-end capped generating a 5'-ppp-5'-triphosphate linkage between a terminal guanosine cap residue and the 5 '-terminal transcribed sense nucleotide of the mRNA molecule. This 5'-guanylate cap may then be methylated to generate an N7-methyl-guanylate residue.

**Poly-A tails:** During RNA processing, a long chain of adenine nucleotides (poly-A tail) may be added to a polynucleotide such as an mRNA molecules in order to increase stability. Immediately after transcription, the 3' end of the transcript may be cleaved to free a 3' hydroxyl. Then poly-A polymerase adds a chain of adenine nucleotides to the RNA. The process, called polyadenylation, adds a poly-A tail that can be between, for example, approximately 100 and 250 residues long.

**Untranslated regions:** As used herein, the term "Untranslated regions" or UTRs of a gene refers to regions that are transcribed but not translated. The 5'UTR starts at the transcription start site and continues to the start codon but does not include the start codon; whereas, the 3'UTR starts immediately following the stop codon and continues until the transcriptional termination signal. The regulatory features of a UTR can be incorporated into the polynucleotides, primary constructs and/or mRNA of the present disclosure to enhance the stability of the molecule.

**3'UTR:** As used herein, unless indicated otherwise or contradictory in context, the term "3'UTR" or "three prime untranslated region" refers to the section of messenger RNA that immediately follows the translation termination codon. The 3'-UTR often contains regulatory regions that post-transcriptionally influence gene expression.

**5'UTR:** As used herein, unless indicated otherwise or contradictory in context, the term "5'UTR" or "five prime untranslated region" refers to the section of mRNA that starts at the transcription start site and continues to the start codon but does not include the start codon. There is growing body of evidence about the regulatory roles played by the UTRs in terms of stability of the nucleic acid molecule and translation. Natural 5'UTRs bear features which play roles in for translation initiation. 5 'UTR also have been known to form secondary structures which are involved in elongation factor binding.

**Complementary DNA:** As used herein, the term "complementary DNA" or "cDNA" refers to a DNA molecule containing an eukaryote gene which has been tailored or engineered to be expressed in a prokaryote host cell. cDNA is also called "intron-free" DNA as it lacks the gene regions encoding introns, its transcription yielding an intron-free mRNA molecule.

**Vector:** As used herein, unless indicated otherwise or contradictory in context, the term "vector" is used in its most general meaning and refers to any intermediary vehicle for a nucleic acid which enables said nucleic acid, for example, to be introduced into prokaryotic and/or eukaryotic cells and, where appropriate, to be integrated into a genome. Vectors of this kind are preferably replicated and/or expressed in the cells. Vectors may comprise plasmids, phagemids, bacteriophages or viral genomes.

**Plasmid:** As used herein, unless indicated otherwise or contradictory in context, the term "plasmid" refers to a double-stranded (which may be circular) DNA sequence that is capable of automatically replicating in a host cell.

In one aspect, the present disclosure provides methods and uses for converting or recoding a neoplastic cell

into a non-neoplastic cell and protecting a patient from neoplastic diseases, conditions, disorders or pathologies, using a medical combination of (i) a polynucleotide encoding at least one peptide, variant or analog thereof, having growth factor receptor-binding and activating capability or capabilities, as defined herein, a vector comprising such a polynucleotide, or a transfected cell comprising such a vector, and (ii) at least one adhesion protein inhibitor or antagonist, as defined herein.

In one aspect, the present disclosure provides methods and uses for converting or recoding a neoplastic cell into a non-neoplastic cell and protecting a patient from neoplastic diseases, conditions, disorders or pathologies, using a medical combination of (i) a polynucleotide encoding at least one peptide, variant or analog thereof, having growth factor receptor-binding and activating capability or capabilities, as defined herein, a vector comprising such a polynucleotide, or a transfected cell comprising such a vector, and (ii) a polynucleotide encoding at least one adhesion protein inhibitor or antagonist, as defined herein, a vector comprising such a polynucleotide, or a transfected cell comprising such a vector.

## VI. Pharmaceutical compositions

In one aspect, the present disclosure provides a composition such as a pharmaceutical, prophylactic, surgical, diagnostic, or imaging composition (hereinafter shorten as pharmaceutical or medical composition) for the uses and methods already disclosed herein comprising at least one pharmaceutical association or combination as defined herein and further comprising at least one pharmaceutically acceptable excipient, carriers and/or vehicles.

Formulations of the pharmaceutical compositions described herein may be prepared by any method known or hereafter developed in the art of pharmacology. Generally, such methods of preparation include the step of bringing the active ingredient(s) into association with an excipient and/or one or more other accessory ingredients, and then, if necessary and/or desirable, shaping and/or packaging the product into a desired single- or multi-dose unit.

For example, in certain embodiments, a pharmaceutical composition as defined herein may contain between 0.01% and 100% by weight (over the total weight of the pharmaceutical composition) of a GFR-binding compound or a pharmaceutical association or combination, both as defined herein, as a pharmaceutically effective amount. The pharmaceutical composition particularly comprises between 0.01% and 95%, between 0.01% and 90%, between 0.01% and 85%, between 0.01% and 80%, between 0.01% and 75%, between 0.01% and 70%, between 0.01% and 65%, between 0.01% and 60%, between 0.01% and 55%, between 0.01% and 50%, between 0.01% and 45%, between 0.01% and 40%, between 0.01% and 35%, between 0.01% and 30%, between 0.01% and 25%, between 0.01% and 20%, between 0.01% and 15%, between 0.01% and 10%, between 0.01% and 5%, between 0.1% and 100%, between 0.1% and 95%, between 0.1% and 90%, between 0.1% and 85%, between 0.1% and 80%, between 0.1% and 75%, between 0.1% and 70%, between 0.1% and 65%, between 0.1% and 60%, between 0.1% and 55%, between 0.1% and 50%, between 0.1% and 45%, between 0.1% and 40%, between 0.1% and 35%, between 0.1% and 30%, between 0.1% and 25%, between 0.1% and 20%, between 0.1% and 15%, between 0.1% and 10%, and between 0.1% and 5% by weight (over the total weight of the pharmaceutical composition) of any one of a GFR-binding compound or an adhesion protein inhibitor or both.

Generally, the GFR-binding compounds or pharmaceutical associations or combinations as defined herein may thus be administered as such or as part of a formulation in association with one or more pharmaceutically acceptable excipients, carriers and/or vehicles so as to form what is generally referred to as a pharmaceutical composition or pharmaceutical formulation.

**Pharmaceutical effective amount:** As used herein, unless indicated otherwise or contradictory in context, the term "pharmaceutical effective amount" or "therapeutically effective amount" refers to an amount of an agent to be delivered (e.g., nucleic acid, protein, peptide, drug, therapeutic agent, diagnostic agent, prophylactic agent, etc.) that is sufficient, when administered to a subject suffering from or susceptible to an infection, disease, disorder, condition and/or pathology, to produce/provide a therapeutically effective outcome. Thus, a "pharmaceutical effective amount" depends upon the context in which it is being applied. A pharmaceutical effective amount of a composition is provided based, at least in part, on the target tissue, target cell type, means of administration, physical characteristics of the pharmaceutical association or composition (e.g., size, 3D shape, etc.), and other determinants. For example, in certain embodiments, in the context of administering an agent that treats cancer, a pharmaceutical effective amount of an agent is, for example, in certain embodiments, an amount sufficient to achieve treatment, as defined herein, of cancer, as compared to the response obtained without administration of the agent. For example, in certain embodiments, a therapeutically effective amount as used herein is any of the herein disclosed weight or molar amounts, ratios or ranges of the GFR-binding compound, the adhesion protein inhibitor or the association/combination thereof.

**Therapeutically effective outcome:** As used herein, unless indicated otherwise or contradictory in context, the term "therapeutically effective outcome" refers to an outcome that is sufficient in a subject suffering from or susceptible to an infection, disease, disorder, condition and/or pathology, to treat, improve symptoms of, diagnose, prevent, and/or delay the onset of the infection, disease, disorder, condition and/or pathology.

**Therapeutic Agent:** As used herein, unless indicated otherwise or contradictory in context, the term "therapeutic agent" refers to any agent that, when administered to a subject/patient/individual, has a therapeutic, diagnostic, and/or prophylactic effect and/or elicits a desired biological and/or pharmacological effect.

**Pharmaceutically acceptable:** As used herein, unless indicated otherwise or contradictory in context, the term "pharmaceutically acceptable" refers to those compounds, materials, compositions, and/or dosage forms which are, within the ambit of sound medical judgment, suitable for use in contact with the tissues of human beings and animals without excessive toxicity, irritation, allergic response, or other problem or complication, commensurate with a reasonable benefit/risk ratio.

**Pharmaceutically acceptable excipients:** As used herein, unless indicated otherwise or contradictory in context, the term "pharmaceutically acceptable excipient" refers to any ingredient other than the compounds described herein (i.e. GFR-binding compounds, adhesion protein inhibitor as defined herein or any further active principles such as additional anti-cancer agents or anti-inflammatory agents) and satisfying to the herein defined definition of pharmaceutically acceptable for a patient. Excipients may include, for example: inert diluents, dispersing and/or granulating agents, surface active agents and/or emulsifiers, disintegrating agents, binding agents, preservatives, buffering agents, lubricating agents, oils, printing inks, sweeteners, and/or waters of hydration. The choice of excipient(s) will largely depend on factors such as the particular mode of administration, the effect of the excipient(s) on solubility and stability, and the nature of the dosage form. In one embodiment, the pharmaceutically acceptable excipient is not a naturally occurring excipient.

**Diluents:** As used herein, unless indicated otherwise or contradictory in context, diluents include, but are not limited to, calcium carbonate, sodium carbonate, calcium phosphate, dicalcium phosphate, calcium sulfate, calcium hydrogen phosphate, sodium phosphate lactose, sucrose, cellulose, microcrystalline cellulose, kaolin, mannitol, sorbitol, inositol, sodium chloride, dry starch, powdered sugar and/or any combinations thereof.

**Buffering agents:** As used herein, unless indicated otherwise or contradictory in context, buffering agents include, but are not limited to, citrate buffer solutions, acetate buffer solutions, phosphate buffer solutions, ammonium chloride, potassium acetate, potassium chloride, monobasic potassium phosphate, calcium carbonate, calcium chloride, calcium citrate, calcium gluconate, calcium lactate, propanoic acid, calcium levulinate, pentanoic acid, phosphoric acid, calcium hydroxide phosphate, sodium acetate, sodium bicarbonate, sodium chloride, sodium citrate, sodium lactate, magnesium hydroxide, aluminum hydroxide, alginic acid, pyrogen-free water, isotonic saline, Ringer's solution, ethyl alcohol and any combinations thereof.

**Granulating and/or dispersing agents:** As used herein, unless indicated otherwise or contradictory in context, granulating and/or dispersing agents include, but are not limited to, potato starch, corn starch, tapioca starch, sodium starch glycolate, clays, alginic acid, guar gum, citrus pulp, agar, bentonite, cellulose and wood products, natural sponge, cation-exchange resins, calcium carbonate, silicates, sodium carbonate, cross-linked poly(vinyl-pyrrolidone), sodium carboxymethyl starch, carboxymethyl cellulose, cross-linked sodium carboxymethyl cellulose, methylcellulose, pregelatinized starch, microcrystalline starch, water insoluble starch, calcium carboxymethyl cellulose, magnesium aluminum silicate, sodium lauryl sulfate, quaternary ammonium compounds and/or any combinations thereof.

**Surface active agents and/or emulsifiers:** As used herein, unless indicated otherwise or contradictory in context, surface active agents and/or emulsifiers include, but are not limited to, colloidal clays (such as aluminum silicates and magnesium aluminum silicates), natural emulsifiers (such as acacia, agar, sodium alginate, cholesterol, xanthan, pectin, gelatin, egg yolk, casein, cholesterol, wax, and lecithin), long chain amino acid derivatives, high molecular weight alcohols (such as stearyl, cetyl and oleyl alcohols, triacetin monostearate, ethylene glycol distearate and glyceryl monostearate), carbomers (such as carboxy polymethylene, polyacrylic acid, acrylic acid polymer, and carboxyvinyl polymer), diethylene glycol monolaurate, triethanolamine oleate, sodium oleate, potassium oleate, ethyl oleate, oleic acid, ethyl laurate, sodium lauryl sulfate, cetrimonium bromide, cetylpyridinium chloride, benzalkonium chloride, docusate sodium, carrageenan, cellulosic derivatives (such as carboxymethylcellulose sodium, hydroxymethyl cellulose, hydroxypropyl methylcellulose and methylcellulose), sorbitan fatty acid esters (such as polyoxyethylene sorbitan monolaurate, polyoxyethylene sorbitan, polyoxyethylene sorbitan monooleate, sorbitan monopalmitate and glyceryl monooleate), polyoxyethylene esters, sucrose fatty acid esters, polyethylene glycol fatty acid esters, polyoxyethylene ethers, poly(vinylpyrrolidone), and any combinations thereof.

**Binding agents:** As used herein, unless indicated otherwise or contradictory in context, binding agents include, but are not limited to, natural and synthetic gums (such as acacia, sodium alginate, carboxymethylcellulose, methylcellulose, ethylcellulose, hydroxyethylcellulose, hydroxypropyl cellulose, hydroxypropyl methylcellulose, microcrystalline cellulose, cellulose acetate and poly(vinyl-pyrrolidone), gelatin, starch, sugars (such as sucrose, dextrose, glucose, dextrin, lactose, and mannitol), alignates, magnesium aluminum silicates, polyethylene glycol, polyethylene oxide, inorganic calcium salts, water, alcohol, silicic acid, waxes, and any combinations thereof.

**Preservatives:** As used herein, unless indicated otherwise or contradictory in context, preservatives include, but are not limited to, antioxidants, chelating agents, antifungal preservatives, antimicrobial preservatives, acidic preservatives, and alcohol preservatives.

**Antioxidants:** As used herein, unless indicated otherwise or contradictory in context, antioxidants include, but are not limited to, alpha tocopherol, ascorbic acid, acorbyl palmitate, butylated hydroxyanisole, propionic acid, potassium metabisulfite, propyl gallate, sodium metabisulfite, sodium ascorbate, and sodium sulfite.

**Chelating agents:** As used herein, unless indicated otherwise or contradictory in context, chelating agents include ethylenediaminetetraacetic acid (EDTA), fumaric acid, malic acid, phosphoric acid, citric acid monohydrate and tartaric acid.

**Antimicrobial preservatives:** As used herein, unless indicated otherwise or contradictory in context, antimicrobial preservatives include, but are not limited to, benzalkonium chloride, benzethonium chloride, chlorhexidine, chlorobutanol, chlorocresol, chloroxylenol, benzyl alcohol, bronopol, cetylpyridinium chloride, cresol, ethyl alcohol, glycerin, hexetidine, imidurea, phenoxyethanol, phenylmercuric nitrate, phenylethyl alcohol, phenol, and propylene glycol.

**Antifungal preservatives:** As used herein, unless indicated otherwise or contradictory in context, antifungal preservatives include, but are not limited to, benzoic acid, hydroxybenzoic acid, butyl paraben, methyl paraben, ethyl paraben, propyl paraben, potassium benzoate, sodium propionate, potassium sorbate, and/or sorbic acid.

**Alcohol preservatives:** As used herein, unless indicated otherwise or contradictory in context, alcohol preservatives include, but are not limited to, phenol, phenolic compounds, bisphenol, ethanol, polyethylene glycol, chlorobutanol and hydroxybenzoate.

**Acidic preservatives:** As used herein, unless indicated otherwise or contradictory in context, acidic preservatives include, but are not limited to, vitamin A, vitamin C, vitamin E, beta-carotene, acetic acid, citric acid, dehydroacetic acid, and sorbic acid.

**Lubricating agents:** As used herein, unless indicated otherwise or contradictory in context, lubricating agents include, but are not limited to, magnesium stearate, calcium stearate, stearic acid, sodium benzoate, sodium acetate, sodium chloride, silica, talc, malt, glyceryl behanate, hydrogenated vegetable oils, polyethylene glycol, magnesium lauryl sulphate and any combinations thereof.

**Sweeteners:** As used herein, unless indicated otherwise or contradictory in context, sweeteners include, but are not limited to, any natural or synthetic sugar substitutes. Natural sugar substitutes include, but are not limited to, brazzein, curculin, erythritol, glycyrrhizin, glycerol, hydrogenated starch hydrolysates, inulin, isomalt, lactitol, mogroside mix, mabinlin, maltitol, malto-oligosaccharide, mannitol, miraculin, monatin, monellin, osladin, pentadin, sorbitol, stevia, tagatose, thaumatin, and xylitol. Synthetic sugar substitutes include, but are not limited to, acesulfame potassium, advantame, alitame, aspartame, salt of aspartame-acesulfame, sodium cyclamate, dulcin, glucin, neohesperidin dihydrochalcone, neotame, P-4000, saccharin, Sucralose.

Exemplary excipients include, but are not limited to: butylated hydroxytoluene (BHT), calcium carbonate, calcium phosphate (dibasic), calcium stearate, croscarmellose, crosslinked polyvinyl pyrrolidone, citric acid, crospovidone, cysteine, ethylcellulose, gelatin, hydroxypropyl cellulose, hydroxypropyl methylcellulose, lactose, magnesium stearate, maltitol, mannitol, methionine, methylcellulose, methyl paraben, microcrystalline cellulose, polyethylene glycol, polyvinyl pyrrolidone, povidone, pregelatinized starch, propyl paraben, retinyl palmitate, shellac, silicon dioxide, sodium carboxymethyl cellulose, sodium citrate, sodium starch glycolate, sorbitol, starch, stearic acid, sucrose, talc, titanium dioxide, vitamin A, vitamin E, vitamin C, and xylitol. Suitable excipients for use in the present invention also include, but are not limited to, water, phosphate buffered saline (PBS), Ringer's solution, dextrose solution, serumcontaining solutions, Hank's solution, other aqueous physiologically balanced solutions, oils, esters and glycols. Aqueous excipients can contain suitable auxiliary substances required to approximate the physiological conditions of the recipient, for example, in certain embodiments, by enhancing chemical stability and isotonicity.

**Pharmaceutically/medically acceptable carriers:** As used herein, unless indicated otherwise or contradictory in context, the term "pharmaceutically acceptable carriers", "medically acceptable carrier" or "carriers" refers to pharmaceutically acceptable excipients and/or delivery vehicles suitable for delivering a pharmaceutical or therapeutic composition useful in a therapeutic method and uses of the present invention to a suitable in-vivo or ex-vivo site. Preferred pharmaceutically acceptable carriers are capable of maintaining a composition containing an active combination or association of a GFR-binding compound and an adhesion protein inhibitor as defined herein, in a form that, upon arrival of the combination to a target cell, site or tissue, the active combination is capable of performing one or more biological functions thereof the protein at the cell or tissue site. One type of pharmaceutically acceptable carrier includes a controlled release formulation that is capable of slowly releasing a composition or combination into an animal. In one example, a controlled release formulation comprises an active combination or association as defined herein in a controlled release vehicle. Suitable controlled release vehicles include, but are not limited to, microparticles, biocompatible polymers, other polymeric matrices, capsules, microcapsules, osmotic pumps, bolus preparations, diffusion devices, liposomes, lipospheres, and transdermal delivery systems. Such suitable controlled release vehicle may be combined with at least one targeting moiety. In one embodiment, the pharmaceutically acceptable carrier is not a naturally occurring carrier. When polynucleotides such as mRNAs are used, it is preferable to use pharmaceutically acceptable carriers that would also effect/help transfection into the cells. Calcium Phosphate Transfection, Cationic Lipid Transfection, DEAE-Dextran Transfection, Electroporation, or viral transfection, are examples of transfection methods using pharmaceutically acceptable

carriers that may also be used for implementing certain embodiments of the present disclosure.

**Targeting Moieties:** In one example, the pharmaceutical association or combination disclosed herein includes at least one binding partner which functions to target the cell to a specific tissue space or to interact with a specific moiety, either in-vivo, ex-vivo or in-vitro. Suitable binding partners include antibodies and functional fragments thereof, scaffold proteins, or peptides.

In one example, said excipients, carriers or vehicles are compatible with the GFR-binding compounds or pharmaceutical association or combinations defined herein so that they do not disrupt, tamper, modify, de-organise, decombine or de-associate said the GFR-binding compounds or pharmaceutical association or combinations. In contrast, said excipients, carriers or vehicles preserves, maintains or reinforces the stability of the GFR-binding compounds or pharmaceutical association or combinations so as to preserve their biological activity.

In one example, the present pharmaceutical compositions also include pharmaceutically acceptable salts and/or solvates and/or prodrugs and/or isotopically-labelled derivatives of the substances and compounds described herein such as the GFR-binding compounds or any other active principles.

**Pharmaceutically acceptable salts:** As used herein, unless indicated otherwise or contradictory in context, the term "pharmaceutically acceptable salts" refers to derivatives of the disclosed substances and compounds wherein the parent substance or compound is modified by converting an existing acid or base moiety to its salt form (e.g., by reacting the free base group with a suitable organic acid). The degree of ionization in the salt may vary from completely ionized to almost non-ionized. Examples of pharmaceutically acceptable salts include, but are not limited to, mineral or organic acid salts of basic residues such as amines; alkali or organic salts of acidic residues such as carboxylic acids; and the like. Representative acid addition salts include acetate, adipate, alginate, ascorbate, aspartate, benzenesulfonate, benzoate, bisulfate, borate, butyrate, camphorate, camphorsulfonate, citrate, cyclopentanepropionate, digluconate, dodecylsulfate, ethanesulfonate, fumarate, glucoheptonate, glycerophosphate, hemisulfate, heptonate, hexanoate, hydrobromide, hydrochloride, hydroiodide, 2-hydroxy-ethanesulfonate, lactobionate, lactate, laurate, lauryl sulfate, malate, maleate, malonate, methanesulfonate, 2-naphthalenesulfonate, nicotinate, nitrate, oleate, oxalate, palmitate, pamoate, pectinate, persulfate, 3-phenylpropionate, phosphate, picrate, pivalate, propionate, stearate, succinate, sulfate, tartrate, thiocyanate, toluenesulfonate, undecanoate, valerate salts, and the like. Representative alkali or alkaline earth metal salts include sodium, lithium, potassium, calcium, magnesium, and the like, as well as nontoxic ammonium, quaternary ammonium, and amine cations, including, but not limited to ammonium, tetramethylammonium, tetraethylammonium, methylamine, dimethylamine, trimethylamine, triethylamine, ethylamine, and the like. The pharmaceutically acceptable salts of the present disclosure include the conventional non-toxic salts of the parent compound formed, for example, in certain embodiments, from non- toxic inorganic or organic acids. The pharmaceutically acceptable salts of the present disclosure can be synthesized from the parent compound which contains a basic or acidic moiety by conventional chemical methods. Generally, such salts can be prepared by reacting the free acid or base forms of these compounds with a stoichiometric amount of the appropriate base or acid in water or in an organic solvent, or in a mixture of the two; generally, non-aqueous media like ether, ethyl acetate, ethanol, isopropanol, or acetonitrile are preferred. Lists of suitable salts are generally found in Remington's Pharmaceutical Sciences, 17th ed., Mack Publishing Company, Easton, Pa., 1985, p. 1418 and in Pharmaceutical Salts: Properties, Selection, and Use, P.H. Stahl and C.G. Wermuth (eds.), Wiley-VCH, 2008, each of which being incorporated herein by reference in its entirety. In one embodiment, the pharmaceutically acceptable salt is not a naturally occurring salt.

**Pharmaceutically acceptable solvate:** As used herein, unless indicated otherwise or contradictory in context, the term "pharmaceutically acceptable solvate," refers to a compound, substance, association or combination wherein molecules of a suitable solvent are incorporated in the crystal lattice. A suitable solvent is physiologically tolerable at the dosage administered. For example, in certain embodiments, solvates may be prepared by crystallization, recrystallization, or precipitation from a solution that includes organic solvents, water, or a mixture thereof. Examples of suitable solvents are ethanol, water (For example, in certain embodiments, mono-, di-, and tri-hydrates), N-methylpyrrolidinone (NMP), dimethyl sulfoxide (DMSO), dimethylformamide (DMF), [Nu],[Nu]'-dimethylacetamide (DMAC), 1,3-dimethyl-2-imidazolidinone (DMEU), 1,3-dimethyl-3,4,5,6-tetrahydro-2-(1H)-pyrimidinone (DMPU), acetonitrile (ACN), propylene glycol, ethyl acetate, benzyl alcohol, 2-pyrrolidone, benzyl benzoate, and the like. When water is the solvent, the solvate is referred to as a "hydrate". In one embodiment, the pharmaceutically acceptable solvate is not a naturally occurring solvate.

**Pharmaceutically acceptable isotopically-labelled compounds:** In one example, the present invention also includes all pharmaceutically acceptable isotopically-labelled derivatives, which are identical to the compounds, substances, combinations or associations described herein but wherein one or more atoms are replaced by atoms having an atomic mass or mass number different from the atomic mass or mass number usually found in nature. Examples of isotopes that may be incorporated into GFR-binding compound(s) as defined herein include isotopes of hydrogen, carbon, chlorine, fluorine, iodine, nitrogen, oxygen, and sulfur, such as $^{2}H$, $^{3}H$, $^{11}C$, $^{13}C$, $^{14}C$, $^{36}Cl$, $^{18}F$, $^{123}I$, $^{13}N$, $^{15}N$, $^{17}O$, $^{18}O$, and $^{35}S$, respectively. It should be understood that compounds, substances, combinations, associations, prodrugs, and pharmaceutical acceptable salts thereof described herein which contain the aforementioned isotopes

and/or other isotopes of other atoms are within the scope of the invention. Certain isotopically labeled of the compounds, substances, combinations, associations, prodrugs, and salts thereof such as, for example, in certain embodiments, those incorporating a radioactive isotope such as $^3H$ and $^{14}C$, are useful in drug and/or substrate tissue distribution studies. Tritium, i.e. $^3H$, and carbon-14, i.e. $^{14}C$, are particularly preferred due to their ease of preparation and detection. Further, substitution with heavier isotopes such as deuterium, i.e. $^2H$, can afford certain therapeutic advantages resulting from greater metabolic stability, for example, in certain embodiments, increased in vivo half-life or reduced dosage requirements, and hence may be preferred in some circumstances. Isotopically labeled compounds, substances, combinations, associations, prodrugs, and salts thereof can generally be prepared by carrying out the procedures disclosed in the Schemes and/or in the Examples by substituting a readily available non-isotopically labeled reagent for an isotopically labeled reagent.

**Prodrugs:** As used herein, unless indicated otherwise or contradictory in context, the term "prodrug" refers to a compound, substance, combination or association that is transformed in vivo to yield a compound, substance, combination or association as defined herein or a pharmaceutically acceptable salt or solvate thereof. The transformation may occur by various mechanisms, such as via hydrolysis in blood. A prodrug of a compound, substance, combination or association defined herein may be formed in a conventional manner with one or more functional groups in the compound, such as an amino, hydroxyl or carboxyl group. For example, in certain embodiments, if a compound defined herein contains a carboxylic acid functional group, a prodrug can comprise: (1) an ester formed by the replacement of a hydrogen of the acid group with a group such as (C1-C6)alkyl or (C6-C10) aryl; (2) an activated ester formed by the replacement of the hydrogen of the acid group with groups such as -($CR^2$)COOR', where $CR^2$ is a spacer and R can be groups such as H or methyl and R' can be groups such as (C1-C6)alkyl or (C6-C10) aryl; and/or (3) a carbonate formed by the replacement of the hydrogen of the acid with groups such as CHROCOOR' where R can be groups such as H or methyl and R' can be groups such as (C1-C6)alkyl or (C6-C10)aryl. Similarly, if a compound defined herein contains an alcohol functional group, a prodrug can be formed via the replacement of the hydrogen of the alcohol with groups such as (C1-C6)alkanoyloxymethyl or (C1-C6)alkanoyloxyaryl or by forming an ester via condensation with, for example, in certain embodiments, an amino acid. Where a compound defined herein contains a primary or secondary amino group, a prodrug may comprise, for example, in certain embodiments, an amide formed by the replacement of one or both of the hydrogen atoms of the amino group with (C1-C10)alkanoyl or (C6-C10)aroyl. Other prodrugs of amines are well known to those skilled in the art. Alternatively, certain compounds defined herein may themselves act as prodrugs of other compounds defined herein. Discussions regarding prodrugs and their use can be found in, for example, in certain embodiments, "Prodrugs as Novel Delivery Systems," T. Higuchi and W. Stella, Vol. 14 of the ACS Symposium Series, and Bioreversible Carriers in Drug Design, Pergamon Press, 1987 (ed. E B Roche, American Pharmaceutical Association). Examples of other prodrug types may be found in the aforementioned reference which is hereby incorporated by reference.

In one particular example, alginate may be used as a carrier or matrice for the non-cyclic or cyclic GFR-binding compounds in association with the adhesion-protein-inhibitor, both as defined herein.

## VII. Administration routes and procedures

Compounds, substances, pharmaceutical associations or combinations to be delivered and/or pharmaceutical, dermatological, prophylactic, diagnostic, or imaging compositions or formulations thereof in accordance with the present disclosure may be administered by any route of administration effective for preventing, treating, diagnosing, or imaging a disease, disorder, and/or condition and/or treating or alleviating at least one symptoms thereof.

Suitable administration protocols include any in-vitro, in-vivo or ex-vivo administration protocol. The preferred types and routes of administration will be apparent to those of skill in the art, depending on the type of condition or disease to be prevented or treated; whether the composition is nucleic acid based, protein based, cell based or combinations or mixtures thereof; and/or the target cell/tissue.

Cells, tissues or organs can be contacted ex vivo or in vitro with a pharmaceutical association or combination for uses and methods of the invention by any suitable method, including mixing or the use of a delivery vehicle. Effective in vitro or ex vivo culture conditions include, but are not limited to, effective media, bioreactor, temperature, pH and oxygen conditions that permit cell culture. An effective medium refers to any medium in which a given host cell or tissue is typically cultured. Such medium typically comprises an aqueous medium having assimilable carbon, nitrogen and phosphate sources, and appropriate salts, minerals, metals and other nutrients, such as vitamins. Cells can be cultured in conventional fermentation bioreactors, shake flasks, test tubes, microtiter dishes, and petri plates. Culturing can be carried out at a temperature, pH and oxygen content appropriate for a cell or tissue. Such culturing conditions are within the expertise of one of ordinary skill in the art.

In one aspect, the present disclosure thus also provides a method for converting a neoplastic cell into a non-neoplastic cell, in-vitro or ex-vivo as defined herein, said method comprising the administration to a neoplastic cell of an effective amount of a pharmaceutical association, combination or composition as defined herein.

**Ex-vivo administration:** As used herein, unless indicated otherwise or contradictory in context, the term "ex-vivo administration" refers to performing the regulatory step outside of the subject/patient, such as administering a pharmaceutical association, combination or composition as defined herein to a population of cells (e.g., neoplastic cells) removed from a subject/patient for e.g. diagnostic, analysis and/or academic purposes.

**In-vivo administration:** In one example, pharmaceutical, prophylactic, diagnostic, or imaging associations, combinations or compositions are administered by one or more of a variety of routes, including oral, intravenous, intramuscular, intra-arterial, intramedullary, rectal, intravaginal, intrathecal, subcutaneous, intraventricular, transdermal, intradermal, intraperitoneal, topical (e.g. by ointments, creams, powders, lotions, gels, and/or drops), buccal, enteral, mucosal, nasal, vitreal, intratumoral, sublingual, by intra-tracheal instillation, bronchial instillation, and/or inhalation, as an oral spray, nasal spray, and/or aerosol, and/or through a portal vein catheter. In one example, pharmaceutical, prophylactic, diagnostic, or imaging associations, combinations or compositions are administered by systemic intravenous injection. In one example, pharmaceutical, prophylactic, diagnostic, or imaging associations, combinations or compositions may be administered in a way which allows them to cross the blood-brain barrier, vascular barrier, or other epithelial barrier. In one most particular example, pharmaceutical, prophylactic, diagnostic, or imaging associations, combinations or compositions may be administered locally by intratumoral administration or near tumor sites. Local administration may be performed e.g. using patches, syringes, or insertion of permeable macroscopic or microscopic vesicles containing the active principle(s).

**Delivery:** As used herein, unless indicated otherwise or contradictory in context, the term "delivery" refers to the act or manner of delivering a compound, substance, association, combination, composition, entity, moiety, cargo or payload.

**Delivery Agent:** As used herein, unless indicated otherwise or contradictory in context, the term "delivery agent" refers to any substance which facilitates, at least in part, the in vivo delivery of a pharmaceutical association, combination or composition defined herein to targeted cells.

**Forms suitable for oral administration:** A pharmaceutical association, combination or composition for uses and methods of the invention, for example, in certain embodiments, includes forms suitable for oral administration as a tablet, capsule, pill, powder, sustained release formulations, solution, suspension, or for parenteral injection as a sterile solution, suspension or emulsion. Pharmaceutical compositions suitable for the delivery of pharmaceutical associations or combinations defined herein and methods for their preparation will be readily apparent to those skilled in the art. Such compositions and methods for their preparation may be found, for example, in certain embodiments, in 'Remington's Pharmaceutical Sciences', 19th Edition (Mack Publishing Company, 1995), which is hereby incorporated by reference in its entirety. Oral administration may involve swallowing, so that the compounds or associations enters the gastrointestinal tract, or buccal or sublingual administration may be employed by which the compound enters the blood stream directly from the mouth. Formulations suitable for oral administration include solid formulations, such as tablets, capsules containing particulates, liquids, or powders; lozenges (including liquid-filled), chews; multi- and nano-particulates; gels, solid solution, liposome, films (including mucoadhesive), ovules, sprays and liquid formulations. Liquid formulations include suspensions, solutions, syrups and elixirs. Such formulations may be employed as fillers in soft or hard capsules and typically comprise a carrier, for example, in certain embodiments, water, ethanol, polyethylene glycol, propylene glycol, methylcellulose, or a suitable oil, and one or more emulsifying agents and/or suspending agents. Liquid formulations may also be prepared by the reconstitution of a solid, for example, in certain embodiments, from a sachet. The pharmaceutical associations defined herein may also be used in fast-dissolving, fast-disintegrating dosage forms such as those described in the art.

**Forms suitable for parenteral administration:** In one example, the pharmaceutical association, combination or composition for uses and methods of the invention may be administered by parenteral injection. Exemplary parenteral administration forms include sterile solutions, suspensions or emulsions of the pharmaceutical association defined herein in sterile aqueous media, for example, in certain embodiments, aqueous propylene glycol or dextrose. In another embodiment, the parenteral administration form is a solution. Such parenteral dosage forms can be suitably buffered, if desired. Preferred sterile solutions include sodium chloride, 0.9%, UPS solution. Injectable formulations can be sterilized, for example, in certain embodiments, by filtration through a bacterial- retaining filter, and/or by incorporating sterilizing agents in the form of sterile solid compositions which can be dissolved or dispersed in sterile water or other sterile injectable medium prior to use.

**Forms suitable for rectal and vaginal administration:** Compositions for rectal or vaginal administration are typically suppositories which can be prepared by mixing compositions with suitable non-irritating excipients such as cocoa butter, polyethylene glycol or a suppository wax which are solid at ambient temperature but liquid at body temperature and therefore melt in the rectum or vaginal cavity and release the active ingredient.

**Forms suitable for topical and/or transdermal administration:** Dosage forms for topical and/or transdermal administration of a composition may include ointments, pastes, creams, lotions, gels, powders, solutions, sprays, inhalants and/or patches. Generally, an active ingredient is admixed under sterile conditions with a pharmaceutically acceptable excipient and/or any needed preservatives and/or buffers as may be required.

**Forms suitable for pulmonary administration:** Dosage forms for pulmonary administration via the buccal cavity may comprise dry particles which comprise the active ingredient (e.g. the pharmaceutical association defined herein) and which have a diameter in the range from about 0.5 nm to about 7 nm. Such compositions are conveniently in the form of dry powders for administration using a device comprising a dry powder reservoir to which a stream of propellant may be directed to disperse the powder and/or using a self-propelling solvent/powder dispensing container such as a device comprising the active ingredient dissolved and/or suspended in a low-boiling propellant in a sealed container. Pharmaceutical compositions formulated for pulmonary delivery may provide an active ingredient in the form of droplets of a solution and/or suspension. Such formulations may be prepared, packaged, and/or sold as aqueous and/or dilute alcoholic solutions and/or suspensions, optionally sterile, comprising active ingredient, and may conveniently be administered using any nebulization and/or atomization device. Such formulations may further comprise one or more additional ingredients including, but not limited to, a flavoring agent such as saccharin sodium, a volatile oil, a buffering agent, a surface active agent, and/or a preservative such as methylhydroxybenzoate.

**Forms suitable for nasal administration:** Formulations described herein as being useful for pulmonary delivery are also useful for intranasal delivery of a pharmaceutical composition. Formulations suitable for nasal administration may, for example, in certain embodiments, comprise from about as little as 0.1% (w/w) and as much as 100% (w/w) of active ingredient (e.g. the pharmaceutical association defined herein), and may comprise one or more of the additional ingredients described herein. A pharmaceutical composition may be prepared, packaged, and/or sold in a formulation suitable for buccal administration. Such formulations may, for example, in certain embodiments, be in the form of tablets and/or lozenges made using conventional methods, and may, for example, in certain embodiments, 0.1 % to 20% (w/w) active ingredient, the balance comprising an orally dissolvable and/or degradable composition and, optionally, one or more of the additional ingredients described herein. Alternately, formulations suitable for buccal administration may comprise a powder and/or an aerosolized and/or atomized solution and/or suspension comprising active ingredient. Such powdered, aerosolized, and/or aerosolized formulations, when dispersed, may have an average particle and/or droplet size in the range from about 0.1 nm to about 200 nm, and may further comprise one or more of any additional ingredients described herein.

**Forms suitable for ophthalmic administration:** Dosage forms for ophthalmic administration include, for example, in certain embodiments, eye drops including, for example, in certain embodiments, a 0.1/1.0% (w/w) solution and/or suspension of the active ingredient (e.g. the pharmaceutical association defined herein) in an aqueous or oily liquid excipient. Such drops may further comprise buffering agents, salts, and/or one or more other of any additional ingredients described herein. Other opthalmically-administrable formulations which are useful include those which comprise the active ingredient in microcrystalline form and/or in a liposomal preparation. Ear drops and/or eye drops are contemplated as being within the scope of this present disclosure.

**Direct injection:** One preferred administration method for delivering a pharmaceutical association, combination or composition as defined herein is by local administration, in particular, by direct injection. Direct injection techniques are particularly useful for administering a composition to a cell or tissue that is accessible by surgery, and particularly, on or near the surface of the body. Administration of a composition locally within the area of a target cell refers to injecting the composition centimeters and preferably, millimeters from the target cell or tissue.

**Dosage regimens:** The dosage regimen of the pharmaceutical associations or combinations and/or pharmaceutical compositions as defined herein may be adjusted to provide the optimum desired response. For example, in certain embodiments, a single bolus may be administered, several divided doses may be administered over time or the dose may be proportionally reduced or increased as indicated by the exigencies of the therapeutic situation. The appropriate dosing regimen, the amount of each dose administered and/or the intervals between doses will depend upon the pharmaceutical association being used, the type of pharmaceutical composition, the characteristics of the subject in need of treatment and the severity of the condition being treated. Thus, the skilled artisan would appreciate, based upon the disclosure provided herein, that the dose and dosing regimen is adjusted in accordance with methods well-known in the therapeutic arts. That is, the maximum tolerable dose can be readily established, and the effective amount providing a detectable therapeutic benefit to a patient may also be determined, as can the temporal requirements for administering each agent to provide a detectable therapeutic benefit to the patient. Accordingly, while certain dose and administration regimens are exemplified herein, these examples in no way limit the dose and administration regimen that may be provided to a patient in practicing the present invention. In general, pharmaceutical compositions in accordance with the present disclosure may be administered at dosage levels sufficient to deliver from about 0.0001 mg/kg to about 100 mg/kg, from about 0.01 mg/kg to about 50 mg/kg, from about 0.1 mg/kg to about 40 mg/kg, from about 0.5 mg/kg to about 30 mg/kg, from about 0.01 mg/kg to about 10 mg/kg, from about 0.1 mg/kg to about 10 mg/kg, or from about 1 mg/kg to about 25 mg/kg, of subject body weight per day, one or more times a day, to obtain the desired therapeutic, diagnostic, prophylactic, or imaging effect. The desired dosage may be delivered three times a day, two times a day, once a day, every other day, every third day, every week, every two weeks, every three weeks, or every four weeks. In certain embodiments, the desired dosage may be delivered using multiple administrations (e.g., two, three, four, five, six, seven, eight, nine, ten, eleven, twelve, thirteen, fourteen, or more administrations). It is to be further understood that

for any particular subject, specific dosage regimens should be adjusted over time according to the individual need and the professional judgment of the person administering or supervising the administration of the compositions, and that dosage ranges set forth herein are exemplary only and are not intended to limit the scope or practice of the present invention. For example, in certain embodiments, doses may be adjusted based on pharmacokinetic or pharmacodynamic parameters, which may include clinical effects such as toxic effects and/or laboratory values. Thus, the present invention encompasses intra-patient dose-escalation as determined by the skilled artisan. Determining appropriate dosages and regiments for administration of the chemotherapeutic agent are well-known in the relevant art and would be understood to be encompassed by the skilled artisan once provided the teachings disclosed herein.

**Effective dose parameters:** The dosage regimen of the pharmaceutical associations or combinations and/or pharmaceutical compositions as defined herein may be adjusted to obtain effective dose parameters. Effective dose parameters can be determined using methods standard in the art for a particular disease or condition. In particular, the effectiveness of dose parameters of a therapeutic composition as defined herein when treating cancer can be determined by assessing response rates. Such response rates refer to the percentage of treated patients in a population of patients that respond with either partial or complete remission. Remission can be determined by, for example, in certain embodiments, measuring tumor size or microscopic examination for the presence of cancer cells in a tissue sample.

A pharmaceutical composition as defined herein may be prepared, packaged, or sold in bulk, as a single unit dose, or as a plurality of single unit doses.

**Unit dose:** As used herein, unless indicated otherwise or contradictory in context, the term "unit dose" refers to a discrete amount of the pharmaceutical composition comprising a predetermined amount of the active ingredient. The amount of the active ingredient is generally equal to the dosage of the active ingredient which would be administered to a subject or a convenient fraction of such a dosage such as, for example, in certain embodiments, one-half or one-third of such a dosage.

**Single unit dose:** As used herein, unless indicated otherwise or contradictory in context, the term "single unit dose" refers to a dose of any therapeutic association or composition administered in one dose/at one time/single route/single point of contact, i.e., single administration event.

**Split dose:** As used herein, unless indicated otherwise or contradictory in context, the term "split dose" refers to the division of single unit dose or total daily dose into two or more doses.

**Total daily dose:** As used herein, unless indicated otherwise or contradictory in context, the term "total daily dose" refers to an amount given or prescribed in 24hr period. It may be administered as a single unit dose.

The relative amounts of the active ingredient(s), the pharmaceutically acceptable excipients, carriers or vehicles, and any additional ingredients in a pharmaceutical composition defined herein will vary, depending upon the identity, size, and condition of the subject treated and further depending upon the route by which the composition is to be administered. In addition to the active ingredient, a pharmaceutical composition of the invention may further comprise one or more additional pharmaceutically active agents.

**Combination therapy:** Compounds, associations, compositions or formulations defined herein may be used in combination with one or more other therapeutic, prophylactic, diagnostic, or imaging agents. As used herein, the term "in combination with" is not intended to imply that the agents must be administered at the same time and/or formulated for delivery together, although these methods of delivery are within the scope of the present disclosure. Compounds, associations, compositions or formulations can be administered concurrently with, prior to, or subsequent to, one or more other desired therapeutics or medical procedures. In some embodiments, they are administered within about 90, 60, 30, 15, 10, 5, or 1 minute of one another. In some embodiments, the administrations of the agents are spaced sufficiently closely together such that a combinatorial (e.g., a synergistic) effect is achieved. In general, each agent will be administered at a dose and/or on a time schedule determined for that agent. In one example, the present disclosure encompasses the delivery of pharmaceutical, prophylactic, diagnostic, or imaging compositions in combination with agents that improve their bioavailability, reduce and/or modify their metabolism, inhibit their excretion, and/or modify their distribution within the body. It will further be appreciated that therapeutically, prophylactically, diagnostically, or imaging active agents used in combination may be administered together in a single composition or administered separately in different compositions. In general, it is expected that agents used in combination with be used at levels that do not exceed the levels at which they are used individually. In one example, the levels used in combination will be lower than those utilized individually. The particular combination of therapies to employ in a combination regimen will take into account compatibility of the desired therapeutics and/or procedures and the desired therapeutic effect to be achieved. It will also be appreciated that the therapies employed may achieve a desired effect for the same disorder (For example, in certain embodiments, a composition useful for treating cancer in accordance with the present disclosure may be administered concurrently with a chemotherapeutic agent), or they may achieve different effects (e.g., control of any adverse effects).

In certain embodiments, said adhesion protein inhibitor may be, separately from said GFR-binding compound, administered locally to the tumor or cancer site using appropriate means such as by injection or by incision, using one administration technique and said GFR-binding compound may then be administered locally (immediately or not) using

another administration technique to the same tumor or cancer site.

In one aspect, the present disclosure thus provides at least one GFR-binding compound as defined herein for use in the prevention, diagnostic and/or treatment of a neoplastic disease in association or combination with at least one adhesion protein or molecule inhibitor as defined herein.

Also provided is a method of treating a neoplastic disease, comprising administering to a subject in need thereof an effective amount of at least one GFR-binding compound as defined herein in association or combination with at least one adhesion protein or molecule inhibitor as defined herein.

Although the descriptions of pharmaceutical associations, combinations or compositions provided herein are principally directed to pharmaceutical associations, combinations or compositions which are suitable for administration to mammals, in particular humans, it will be understood by the skilled artisan that such compositions are generally suitable for administration to animals of all sorts, in particular to any member of the Vertebrate class. Modification of pharmaceutical compositions suitable for administration to humans in order to render the compositions suitable for administration to various animals is well understood, and the ordinarily skilled veterinary pharmacologist can design and/or perform such modification with merely ordinary, if any, experimentation. Subjects to which administration of the pharmaceutical compositions is contemplated include, but are not limited to, humans and/or other primates; mammals, including commercially relevant mammals such as cattle, pigs, horses, sheep, cats, dogs, mice, and/or rats; and/or birds, including commercially relevant birds such as chickens, ducks, geese, and/or turkeys.

## VIII. Dermatological applications

When the neoplastic disease to be prevented or treated involves neoplastic cells belonging to the skin cell lineage (mainly to the fibroblast lineage), the pharmaceutical composition defined herein may be a dermatological composition comprising a pharmaceutical association or combination of a GFR-binding compound and an adhesion protein inhibitor as all defined herein and at least one dermatologically acceptable excipient.

For example, in certain embodiments, a dermatological composition for the uses of the invention may contain between 0.01% and 100% by weight (over the total weight of the dermatological composition) of a GFR-binding compound and/or an adhesion protein inhibitor, both as defined herein, as a dermatological effective amount. The dermatological composition particularly comprises between 0.01% and 95%, between 0.01% and 90%, between 0.01% and 85%, between 0.01% and 80%, between 0.01% and 75%, between 0.01% and 70%, between 0.01% and 65%, between 0.01% and 60%, between 0.01% and 55%, between 0.01% and 50%, between 0.01% and 45%, between 0.01% and 40%, between 0.01% and 35%, between 0.01% and 30%, between 0.01% and 25%, between 0.01% and 20%, between 0.01% and 15%, between 0.01% and 10%, between 0.01% and 5%, between 0.1% and 100%, between 0.1% and 95%, between 0.1% and 90%, between 0.1% and 85%, between 0.1% and 80%, between 0.1% and 75%, between 0.1% and 70%, between 0.1% and 65%, between 0.1% and 60%, between 0.1% and 55%, between 0.1% and 50%, between 0.1% and 45%, between 0.1% and 40%, between 0.1% and 35%, between 0.1% and 30%, between 0.1% and 25%, between 0.1% and 20%, between 0.1% and 15%, between 0.1% and 10%, and between 0.1% and 5% by weight (over the total weight of the dermatological composition) of any one of a GFR-binding compound or an adhesion protein inhibitor or both.

**Dermatologically acceptable:** As used herein, unless indicated otherwise or contradictory in context, the term "dermatologically acceptable" means that the compound(s) or pharmaceutical association(s) used are adapted for use in contact with human skin without undue toxicity, incompatibility, instability, allergic response, or their equivalents.

**Dermatological formulations:** Suitable formulation for implementing dermatological embodiments of the invention include an aqueous or oil-based solution, a water-based cream or gel or an oily gel, usually in a jar or a tube, particularly a shower gel, shampoo, milk, emulsion, microemulsion or nanoemulsion, particularly oil-in-water or water-in-oil or multiple of silicone-based; a lotion, particularly in a glass or plastic bottle of a spray or aerosol bottle, a blister-pack, liquid soap, a dermatological bar of soap, a pomade, mousse, an anhydrous product, preferably liquid, cream or solid, for example in the form of a stick, particularly in the form of lipstick, a cataplasm or a patch.

Preferred administration routes include, but are not limited to, topical, intradermal and intra-tumoral as already defined herein.

**Dermatologically acceptable excipients:** Suitable dermatologically acceptable excipients for implementing embodiments of the invention include, but are not limited to, preservatives, emollients, emulsifiers, surfactants, moisturizers, thickeners, conditioners, mattifying agents, stabilizers, antioxidants, texturizing agents, shine agents, filmogenic agents, solubilizers, pigments, colorants, perfumes, and solar filters. These excipients are preferably chosen from among the group consisting of amino acids and their derivatives, polyglycerols, esters, polymers and cellulose derivatives, lanoline derivatives, phospholipids, lactoferrins, lactoperoxidases, sucrose-based stabilizers, vitamin E and its derivatives, natural and synthetic waxes, vegetable oils, triglycerides, insaponifiables, phytosterols, plant esters, silicones and their derivatives, protein hydrolysates, jojoba oil and its derivatives, lipo/hydrosoluble esters, betaines, aminoxides, saccharose ester plant extracts, titanium dioxides, glycines, parabens, and even more preferably from among the group consisting of butylene glycol, glycol-15 stearyl ether, cetearyl alcohol, phenoxyethanol, methylparaben, propylparaben,

butylparaben, butylenes glycol, natural tocopherols, glycerine, dihydroxycetyl sodium phosphate, isopropyl hydroxycetyl ether, le glycol stearate, triisononanoin, octyl cocoate, polyacrylamide, isoparaffin, laureth-7, carbomer, propylene glycol, glycerol, bisabolol, dimethicone, sodium hydroxide, PEG 30-dipolyhydroxysterate, capric/caprylic triglycerides, cetearyl octanoate, dibutyl adipate, grapeseed oil, jojoba oil, magnesium sulfate, EDTA, cyclomethicone, xanthan gum, citric acid, sodium lauryl sulfate, mineral waxes and oils, isostearyl isostearate, dipelargonate of propylene glycol, isostearate of propylene glycol, PEG 8, beeswax, glyceride of hydrogenated palm kernel oil, lanolin oil, sesame oil, cetyl lactate, lanolin alcohol, titanium dioxide, lactose, saccharose, low-density polyethylene, and isotonic salt solution.

In one example, the dermatological composition as defined herein may contain at least one other active agents and/or excipients and/or additives of pharmaceutical, especially dermatological, interest such as agents with the following properties:

- wound-healing properties; such as panthenol and derivatives thereof, for example ethyl panthenol, aloe vera, pantothenic acid and derivatives thereof, allantoin, bisabolol, and dipotassium glycyrrhizinate;
- anti-inflammatory properties: such as steroidal and non-steroidal antiinflammatories, in particular Inhibitors of the production of cytokines and chemokines, of cyclooxygenase, of nitric oxide (NO) and nitric oxide synthase (NOS). As an example of anti-inflammatory products, mention may be made of extracts of Ginkgo biloba, trilactone terpenes such as ginkgolides, especially ginkgolide B and bilobalide known for their platelet- activating factor (PAF) antagonist properties.

The CTFA Cosmetic Ingredient Handbook, Second Edition (1992), which is hereby incorporated by reference in its entirety, describes different cosmetic and pharmaceutical ingredients currently used in the cosmetic and pharmaceutical industry that are particularly adapted to topical use and which may be used in a dermatological composition of the invention. Examples of these types of ingredients include but are not limited to the following compounds: abrasives, absorbent compounds, compounds with aesthetic purposes such as perfumes, pigments, colorants, essential oils, astringents, etc. (for example: clove oil, menthol, camphor, eucalyptus oil, eugenol, menthyl lactate, and hamelis distillate), anti-acne agents, anti-flocculant agents, anti-foaming agents, anti-microbial agents (for example iodopropyl butylcarbamate), les antioxidants, bonding agents, biological additives, tampon agents, swelling agents, chelatants, additives, biocidal agents, denaturants, external analgesics, film-forming materials, polymers, opacifying agents, pH adjusters, reducing agents, depigmenting or lightening agents (for example: hydroquinone, kojic acid, ascorbic acid, magnesium ascorbyl phosphate, ascorbyl glucosamine), conditioning agents (for example: humectants), calming agents for the skin and/or scarring agents (for example: panthenol and its derivatives, for example ethyl panthenol), aloe vera, pantothenic acid and its derivatives, allantoin, bisabolol and dipotassium glycyrrhizinate), thickeners, vitamins, and the derivatives or equivalents of these.

In one aspect, the present disclosure provides a pharmaceutical association or combination as defined herein or a dermatological composition as defined herein for use in converting a neoplastic skin cell (i.e. any cell belonging to the fibroblast lineage as defined herein) into a non-neoplastic skin cell, in particular into a functional and/or healthy skin cell.

In one aspect, the present disclosure provides a pharmaceutical association or combination as defined herein or a dermatological composition as defined herein for use in protecting (i.e. preventing and/or treating) a subject or patient from a skin neoplastic disease, disorder or condition, in particular, but not limited to, basal and squamous cell skin cancers, melanoma skin cancer, Merkel cell carcinoma, lymphoma of the skin and Kaposi sarcoma.

Suitable as amounts of pharmaceutical association for implementing embodiments of the invention in the dermatological field include the group consisting of between about 0.0001 m g/day to about 5000 mg/day, between about 0.0001 m g/day to about 1000 mg/day, between about 0.0001 m g/day to about 10 mg/day, between about 0.0001 m g/day to about 1 mg/day, or between about 0.0001 m g/day to about 100 m g/day, all being preferred for implementing embodiments of the invention.

Advantageously, the subject who has need thereof is a subject chosen from a population having an average age of more than 30 years old or who has had sunlight over-exposure, has a family history of skin cancer, has or has had certain other skin conditions or previous radiotherapy, has been exposed to certain chemicals and has a weakened immune system.

## IX. Ophthalmic applications

Preferable dosage forms for the pharmaceutical association, combination or composition as defined herein for treating ophthalmic neoplastic diseases, disorders or conditions include, for example, in certain embodiments, eye drops and eye ointments. These can be prepared using conventional techniques. For instance, eye drops may be prepared, using isotonic agents such as sodium chloride, buffers such as sodium phosphate, and preservatives such as benzalkonium chloride. A suitable pH is within an ophthalmologically acceptable range. Preferred pH is within pH 4 to 8.

Particularly preferred administration routes include vitreal, intraocular and intra-tumoral.

A suitable dose of pharmaceutical association, combination or composition for treating eye disorders is appropriately selected, depending on the symptoms, age of patients, dosage form and the like. For eye drops, suitable concentration may be 0.0001 to 10 w/v %, preferably 0.0001 to 0.01 w/v % for administration into eyes once or several times a day.

## X. Surgical treatments

In one aspect, the pharmaceutical association, combination or composition as defined herein may be used in a surgical method suitable for treating or preventing a neoplastic disease such as a tumour or a cancer.

In one aspect, the present disclosure provides a surgical method for surgical treatment of a neoplastic disease comprising the provision of a pharmaceutical association, combination or composition as defined herein, and the contacting or administration of said pharmaceutical association, combination or composition with a body part of a patient to be treated.

For example, in certain embodiments, the surgical treatment of the invention may include the provision of a placement, insertion or depositing device and the contacting of said pharmaceutical association, combination or composition with a body part of a patient using said placement, insertion or depositing device.

In one example, said placement, insertion or depositing device comprises an injection device such as a syringe, and comprises the positioning of said pharmaceutical association, combination or composition inside said injection device for injection into a subject/patient or into a body part of a subject/patient.

## XI. Pharmaceutical applications, uses and methods

The present invention provides for uses and methods of converting or inducing the conversion or the recoding of a neoplastic cell (e.g. a cancer cell) into a non-neoplastic cell (e.g. a non-cancerous cell) through extracellular, non-mutagenic, conversion or recoding of said neoplastic cell. In other words, the present disclosure provides methods for a neoplastic cell to perform self-healing or self-recovery into a more functional, healthy, non-neoplastic cell.

Conveniently, such a self-healing process is generally achieved within less than 7 days. In particular, such a self-healing process is generally achieved within less than 6 days. In particular, such a self-healing process is generally achieved within less than 5 days. In particular, such a self-healing process is generally achieved within less than 4 days. In particular, such a self-healing process is generally achieved within less than 3 days. In particular, such a self-healing process is generally achieved within less than 2 days. In particular, such a self-healing process is generally achieved within less than 24 hours. In particular, such a self-healing process is generally achieved within less than 18 hours.

Conveniently, such a self-healing process is achieved with a cell conversion yield greater than about 50%. In particular, such a self-healing process is achieved with a cell conversion yield greater than about 60%. In particular, such a self-healing process is achieved with a cell conversion yield greater than about 70%. In particular, such a self-healing process is achieved with a cell conversion yield greater than about 80%. In particular, such a self-healing process is achieved with a cell conversion yield greater than about 90%. In particular, such a self-healing process is achieved with a cell conversion yield greater than about 95%. In particular, such a self-healing process is achieved with a cell conversion yield is about 100%.

**Cell conversion yield:** As used herein, "cell conversion yield" means the percentage of neoplastic cells that are transformed into non-neoplastic cells. It is considered significant when it is higher than 10%. There are many ways to measure a cell conversion yield, but for the purpose of the present disclosure, and for the avoidance of any doubts, the cell conversion yield is herein measured by using a haemocytometer for precise cell counting using, for instance, a haemocytometer from Baxter Scientific and following the standard procedure below:

(a) Cleaning the chamber and cover slip with alcohol. Drying and fixing the coverslip in position.
(b) Harvesting the cells. Adding 10 μL of the cells to the haemocytometer.
(c) Placing the chamber in the inverted microscope under a 10X objective. Using phase contrast to distinguish the cells.
(d) Counting the cells in the large, central gridded square (1 $mm^2$). The gridded square is circled in the graphic below. Multiplying by $10^4$ to estimate the number of cells per mL.
(e) Preparing duplicate samples and averaging the count.

In certain embodiments, the present disclosure provides methods to convert a neoplastic cell into a non-neoplastic cell, wherein said obtained neoplastic cell is homogeneous and/or of substantially the same differentiation state. Conveniently, the converted non-neoplastic cells obtained using a method as defined herein, have a homogeneity of greater than 20%. In particular, the converted non-neoplastic cells obtained using a method as defined herein, have a homogeneity of greater than 50%. In particular, the converted non-neoplastic cells obtained using a method as defined

herein, have a homogeneity of greater than 70%. In particular, the converted non-neoplastic cells obtained using a method as defined herein, have a homogeneity of greater than 90%. In particular, the converted non-neoplastic cells obtained using a method as defined herein, have a homogeneity of greater than 99%.

**Homogeneous:** As used herein, "homogeneous", when used in relation to a non-neoplastic cell population obtained using methods as defined herein, means that substantially all of the obtained non-neoplastic cells within the population are in a G0 phase. There are many ways to test and measure the homogeneity of a cell population, but for the purpose of the present disclosure, and for the avoidance of any doubts, the cell homogeneity is herein measured using cell immunofluorescence staining by detecting phosphorylation of the Rb protein, an absence of phosphorylation meaning that substantially all cells are in a G0 phase.

Conveniently, the converted non-neoplastic cells obtained using a method as defined herein, are more than 20% identical. In particular, the converted non-neoplastic cells obtained using a method as defined herein, are more than 50% identical. In particular, the converted non-neoplastic cells obtained using a method as defined herein, are more than 70% identical. In particular, the converted non-neoplastic cells obtained using a method as defined herein, are more than 90% identical. In particular, the converted non-neoplastic cells obtained using a method as defined herein, are more than 99% identical.

As used herein, "substantially the same differentiation state" or "substantially identical differentiation state", when used in relation to non-neoplastic cells obtained using methods as defined herein, means exhibiting the same gene expression pattern. There are many ways to test and measure the differentiation state of a cell and group of cells, but for the purpose of the present disclosure, and for the avoidance of any doubts, the differentiation state of a cell or a group of cell is herein measured using RT-PCR for the quantification of the expression of well-defined marker genes for the particular differentiation state.

In certain aspects, a pharmaceutical association, combination or composition as defined herein is thus useful in the protection of a subject/patient from (e.g. in the treatment and/or prevention of) a neoplastic disease, condition, disorder or pathology and/or at least one symptom thereof such as tumors and cancers. In one aspect, the present disclosure provides a pharmaceutical association, combination or (therapeutic, dermatologic, ophthalmologic, diagnostic, etc.) composition defined herein for use in the treatment of a neoplastic disease, condition, disorder or pathology and/or at least one symptom thereof, said composition comprising a GFR-binding compound and an adhesion protein inhibitor, all as already defined herein. Also provided is a method of treating a neoplastic disease, comprising administering to a subject in need thereof an effective amount of a pharmaceutical association, combination or (therapeutic, dermatologic, ophthalmologic, diagnostic, etc.) composition as defined herein.

In one aspect, the present disclosure provides a pharmaceutical association, combination or (therapeutic, dermatologic, ophthalmologic, diagnostic, etc.) composition defined herein for use in a method of converting or recoding a neoplastic cell to induce and/or promote and/or improve self-healing and/or self-recovery thereof. Also provided is a method of converting or recoding a neoplastic cell to induce and/or promote and/or improve self-healing and/or self-recovery thereof, comprising administering to a subject in need thereof an effective amount of a pharmaceutical association, combination or (therapeutic, dermatologic, ophthalmologic, diagnostic, etc.) composition as defined herein.

In one example, the conversion or recoding of the neoplastic cell into a non-neoplastic cell is substantially permanent. As used herein, the term "permanent", when used in relation to the conversion or recoding of a neoplastic cell into a non-neoplastic cell, means physiologically permanent conversion as the neoplastic cell treated by the method of the invention then becomes a normal, functional, healthy cell just like any other non-neoplastic cell of the subject's body and is thus not prevented to develop a new neoplastic state or any other abnormal state in the future (like any normal cell could/would).

In one aspect, the present disclosure provides a pharmaceutical association, combination or (therapeutic, dermatologic, ophthalmologic, diagnostic, etc.) composition defined herein for use in a method of restoring the ability of a neoplastic cell to undergo differentiation. Also provided is a method of restoring the ability of a neoplastic cell to undergo differentiation, comprising administering to a subject in need thereof an effective amount of a pharmaceutical association, combination or (therapeutic, dermatologic, ophthalmologic, diagnostic, etc.) composition as defined herein.

In one aspect, the present disclosure provides a pharmaceutical association, combination or (therapeutic, dermatologic, ophthalmologic, diagnostic, etc.) composition defined herein for use in a method of converting and/or recoding a circulating or non-circulating neoplastic cell such as a metastatic or non-metastatic cancer cell, into a non-neoplastic cell. Also provided is a method of converting and/or recoding a circulating or non-circulating neoplastic cell such as a metastatic or non-metastatic cancer cell, into a non-neoplastic cell, comprising administering to a subject in need thereof an effective amount of a pharmaceutical association, combination or (therapeutic, dermatologic, ophthalmologic, diagnostic, etc.) composition as defined herein.

In one aspect, the present disclosure provides a pharmaceutical association, combination or (therapeutic, dermatologic, ophthalmologic, diagnostic, etc.) composition defined herein for use in a method of providing and/or producing and/or inducing the formation of a physiologically functional and/or healthy cell of the bone, cartilage, vascular, blood, fibroblast, muscle, neural, epithelial, renal, retinal cell lineage from a neoplastic cell. Also provided is a method

of providing and/or producing and/or inducing the formation of a physiologically functional and/or healthy cell of the bone, cartilage, vascular, blood, fibroblast, muscle, neural, epithelial, renal, retinal cell lineage from a neoplastic cell, comprising administering to a subject in need thereof an effective amount of a pharmaceutical association, combination or (therapeutic, dermatologic, ophthalmologic, diagnostic, etc.) composition as defined herein.

In one aspect, the present disclosure provides a pharmaceutical association, combination or (therapeutic, dermatologic, ophthalmologic, diagnostic, etc.) composition defined herein for use in a method of inducing and/or promoting and/or enhancing neoplastic cell differentiation. Also provided is a method of inducing and/or promoting and/or enhancing neoplastic cell differentiation, comprising administering to a subject in need thereof an effective amount of a pharmaceutical association, combination or (therapeutic, dermatologic, ophthalmologic, diagnostic, etc.) composition as defined herein.

In one aspect, the present disclosure provides a pharmaceutical association, combination or (therapeutic, dermatologic, ophthalmologic, diagnostic, etc.) composition defined herein for use in a method of non-mutagenically protecting a subject from a neoplastic disease i.e. without modifying or altering the genome of the treated neoplastic cells. Also provided is a method of non-mutagenically protecting a subject from a neoplastic disease i.e. without modifying or altering the genome of the treated neoplastic cells, comprising administering to a subject in need thereof an effective amount of a pharmaceutical association, combination or (therapeutic, dermatologic, ophthalmologic, diagnostic, etc.) composition as defined herein.

In one aspect, the present disclosure provides a pharmaceutical association, combination or (therapeutic, dermatologic, ophthalmologic, diagnostic, etc.) composition defined herein for use in a method of extracellular treatment of a neoplastic disease, disorder, condition, pathology, or any symptoms thereof. Also provided is a method of extracellular treatment of a neoplastic disease, disorder, condition, pathology, or any symptoms thereof, comprising administering to a subject in need thereof an effective amount of a pharmaceutical association, combination or (therapeutic, dermatologic, ophthalmologic, diagnostic, etc.) composition as defined herein.

An extracellular treatment as used herein implies at least one (in particular, all) biological action/effect to be provided or to occur outside the cell to be treated (i.e. a neoplastic cell). In other words, the biologically active agent (*e.g.* the pharmaceutical association, combination or composition as defined herein) delivers/provides its biological/pharmaceutical effect to the outside of the cell (e.g. on the cell's surface) without the need to penetrate through the cell membrane, inside the neoplastic cell to be treated. Once the extracellular action/effect has been administered/delivered to the neoplastic cell to be treated, said active agent may be, for instance, excreted from the host organism with or without being metabolised, and/or tagged to be destroyed through apoptotic routes, and/or internalised by nearby cells, etc...Without being bound to any specific theory, it is believed that once the extracellular biological action/effect/message/signal has been delivered by the pharmaceutical association or composition defined herein to a neoplastic cell to be treated, said cell then undergo self-healing into a functional, healthy, non-neoplastic cell.

Data have shown (FIGURE 6) that the administration or action of the pharmaceutical association or composition as defined herein on neoplastic cells to be treated, implicates and down-regulates (in particular, reduces, substantially reduces or suppress) the Ras/MAP kinase, FAK/Src kinase and/or PIP2 signalling pathways. Without wishing to be bound to any specific theory, it is conventionally known that these pathways control the expression of cyclins D, which in turn controls the activity of CDKs 4 and/or 6 so that down-regulating (in particular, reducing, significantly reducing or suppressing) the Ras/MAP kinase, FAK/Src kinase and/or PIP2 pathways would inhibit, reduce, damper or suppress the expression of cyclins D and/or CDK4 and CDK6 and/or the formation of the cyclins D-CDK4/6 complexes.

As already stated herein, cell cycle comprises four main phases: The S phase for DNA duplication, followed by a G2 phase for preparation of the entry into the M phase, the M or mitotic phase wherein the cell divides and finally the G1 phase for cell growth. During the G1 phase the cell makes decisions on whether to continue the cell cycle and undergo cell growth and further divide, or to exit the cycle in the G0 phase and remains quiescent, die or initiate differentiation. The progression through the cell cycle is governed by phosphorylation signals emitted by different bimolecular complexes composed of CDKs and cyclin proteins, both as defined herein.

The cyclin D-CDK4/6 complexes are capable of leading a cell through the restriction (R)-point which is generally known for controlling the passage of the cell from the G1 phase to the S phase of the cell cycle. This control gate is generally thought to be located at the end of the cell cycle's G1 phase, just before entry into S phase, and is involved in the decision of whether the cell should divide or proliferate, delay division, or enter the G0 resting state.

This control gate ensures that a series of surveillance or monitoring mechanisms are completed successfully before proceeding through to the next phase. These monitoring mechanisms are also commonly termed "checkpoints" or "checkpoint controls". If a checkpoint is not validated by the control gate, the cell should halt further advance through the cell cycle and enter the G0 phase. However, neoplastic cells, such as cancer cells, have developed mechanisms that somehow "disconnect" or "impair" one or more checkpoints from the control gate so that the cell is "tricked to "believe that all checkpoints are validated (or, in other words, "tricked to ignore" the non-validated status of relevant checkpoints) and thus never or rarely enter into the G0 phase.

Without wishing to be bound to any specific theory, it is thought that the pharmaceutical associations, combination or composition as defined herein can restore or re-establish (the integrity of) one or more impaired cell cycle checkpoints so as to restore the lost ability of a neoplastic cell to detect a malfunction or a defect in the cell cycle regulation, induce the cell cycle arrest and exit the cell cycle in G0.

One particular checkpoint that the present invention is able to restore is the cell adhesion checkpoint. In one aspect, the present disclosure provides a pharmaceutical association, combination or (therapeutic, dermatologic, ophthalmologic, diagnostic, etc.) composition defined herein for use in a method of treating a neoplastic cell or a neoplastic disease by restoring the cell adhesion checkpoint of said neoplastic cell.

The cell adhesion checkpoint is known to be in charge of monitoring the cells attachments to the extracellular matrix (ECM). When a cell does not detect a "correct" attachment it should halt further advance through the cell cycle and enter the Go phase. The ECM attachment is normally achieved via cell transmembrane proteins, such as the integrins, syndecans and different proteoglycans. The most important among these are the integrins, which assemble as alpha-beta heterodimers. In addition to physically linking the cell to the extracellular matrix, the binding of the extracellular integrin domains to specific components of the ECM activates the integrins and allows binding of different signalling molecules to its intracellular domain. This activates various signalling pathways that mediate signals to the adhesion checkpoint including the Ras/MAP kinase, FAK/Src kinase, and PIP2 pathways. These pathways are involved in the regulation of the expression of cyclins D, which in turn controls the activity of CDK4/6. The cyclins D-CDK4/6 complexes are capable of leading the cell through the R-point gate.

Data have also shown (FIGURES 6 and 7) that the administration or action of the pharmaceutical association or composition as defined herein on neoplastic cells to be treated down-regulated (in particular, substantially reduced) the expression of FAK genes and proteins and/or MAP kinase and/or reduced (in particular, substantially reduced) the expression or activity of GTPase Ras, Rho (Rho, Rac, Cdc42).

Focal adhesions are integrin-containing, multi-protein structures that can form mechanical links between the intracellular actin bundles and the extracellular substrate in many cell types. Focal adhesions are highly dynamic structures that grow or shrink due to the turnover of their component proteins in response to changing mechanical stresses (e.g. actomyosin-generated forces, external forces exerted by or through the surrounding matrix). Focal adhesions are consistently found at the end of stress fibers and are therefore highly integrated with the bulk of the cytoskeleton. Consequently, focal adhesions serve to transmit force, internally generated by the cytoskeletal network, to the ECM and vice versa vian adhesion receptors. Adhesion assembly and maturation are highly dependent on the presence of force, which is believed to instigate structural rearrangements that in turn foster the recruitment of additional proteins (growth) and induce signaling cascades leading to actin polymerization (strengthening). Focal adhesions serve as a biochemical signaling hub to concentrate and direct numerous signaling proteins as part of signal transduction cascades at sites of integrin binding and clustering.

Focal Adhesion Kinase (FAK) is a cytoplasmic tyrosine kinase that plays a role in integrin-mediated signal transductions and also participates in signalling via other cell surface receptors. FAK is typically located in structures known as focal adhesions. These are multi-protein structures that link the extracellular matrix (ECM) to the cytoplasmic cytoskeleton. FAK is known to be phosphorylated in response to integrin engagement, growth factor stimulation, and the action of mitogenic neuropeptides. This cytosolic kinase has been reported to participate to diverse cellular mechanisms including cell locomotion, mitogen response and cell survival. FAK has four defined regions, or tertiary structure domains. Two of these domains, the N-terminal FERM domain and the Kinase domain, form an auto-inhibitory interaction. This interaction is thought to be the result of hydrophobic interactions between the two domains and prevents the activation of the Kinase domain, thereby preventing the signalling function of FAK. Release of this auto-inhibitory interaction has been shown to occur within focal adhesions but not in the cytoplasm and therefore is thought to require interaction with focal adhesion proteins.

MAP kinase Mitogen-activated protein kinases or MAP kinases (or MAPK) include a large group of related serine/threonine eukaryotic protein kinases whose regulatory cell functions include proliferation, gene expression, differentiation, mitosis, cell survival, and apoptosis. All have been reported to become activated when they have been phosphorylated at a Tyrosine and a Threonine amino acid. MAP kinases are catalytically inactive in their basic form. In order to become active, (potentially multiple) phosphorylation events is required to occur in their activation loops. MAP kinases typically form multi-step pathways, receiving input signals at several levels above the actual MAP kinase. These pathways can effectively convey stimuli from the cell membrane to the nucleus or to many other subcellular targets.

GTPases (singular GTPase) are a large family of hydrolase enzymes that can bind and hydrolyze guanosine triphosphate (GTP). The GTP binding and hydrolysis takes place in the highly conserved G domain common to all GTPases. The hydrolysis of the $\gamma$ phosphate of GTP into guanosine diphosphate (GDP) and Pi, inorganic phosphate, occurs by the SN2 mechanism of nucleophilic substitution via a pentavalent intermediate state and is dependent on the magnesium ion $Mg^{2+}$. Regulatory GTPases, also called the GTPase superfamily, are GTPases used for regulation of other biochemical processes. Most prominent among the regulatory GTPases are the G proteins. Small GTPases have a molecular weight of about 21 kDa and generally serve as molecular switches for a variety of cellular signalling events.

According to their primary amino acid sequences and biochemical properties, the Ras superfamily is further

divided into five subfamilies: Ras, Rho, Rab, Arf and Ran. The Rho subfamily is further divided into RHOA, RAC1, and CDC42.

Ras/MAP kinase signalling pathway (also known as the Ras-Raf-MEK-ERK pathway) is a chain of proteins in the cell that communicates a signal from a receptor on the surface of the cell to the DNA in the nucleus of the cell. The signal starts when a signalling molecule binds to the receptor on the cell surface and ends when the DNA in the nucleus expresses a protein and produces some change in the cell, such as cell division. The pathway includes many proteins, including MAPK (mitogen-activated protein kinases, originally called ERK, extracellular signal-regulated kinases), which communicate by adding phosphate groups to a neighbouring protein.

The FAK/Src kinase signalling pathway involves focal adhesion kinase (FAK) and steroid receptor coactivator (Src) which are intracellular (non-receptor) tyrosine kinases that physically and functionally interact to promote a variety of cellular responses. The linked activities of these two kinases are a common intracellular point of convergence in the signalling initiated by the integrin-extracellular matrix (ECM) interaction. Integrins, a family of transmembrane receptors, interact with the ECM and the intracellular actin-cytoskeleton. The integrins cluster upon binding to the ECM to form focal adhesion (FA) contacts. In response to this clustering, FAK associates to the cytoplasmic tail of the integrins and undergoes phosphorylation at its tyrosine 397 residue (Y397). This phosphorylated tyrosine provides a docking site for Src which is then able to phosphorylate additional sites on FAK, leading to a further increase in FAK activity and allowing the recruitment of proteins that contain Src homology 2 (SH2) domains such as Grb2 and PI3K. The mutually activated FAK/Src complex then initiates a cascade of phosphorylation events of new protein-protein interactions to trigger several signalling pathways that eventually leads to different cellular responses.

Phosphatidylinositol 4,5-bisphosphate or PIP2 is a minor phospholipid component of cell membranes. PIP2 is enriched at the plasma membrane where it is a substrate for a number of important signalling proteins. PIP2 is phosphorylated by the Class I PI 3-kinases. Class I PI 3-kinases are a subgroup of the enzyme family, phosphoinositide 3-kinase that possess a common protein domain structure, substrate specificity, and method of activation. Class I PI 3-kinases are further divided into two subclasses, class IA PI 3-kinases and class IB PI 3-kinases. Class IA PI 3-kinases are activated by receptor tyrosine kinases (RTKs). Class IB PI3-kinases are activated by G-protein-coupled receptors (GPCRs). After their activation these kinases phosphorylate PIP2 to form phosphatidylinositol (3,4,5)-trisphosphate PIP3. Both PIP2 and PIP3 not only act as substrates for enzymes but also serve as docking phospholipids that bind specific domains that promote the recruitment of proteins to the plasma membrane and subsequent activation of signalling cascades. PIP3 functions to activate downstream signalling components, the most notable one being the protein kinase AKT, which activates downstream anabolic signalling pathways required for cell growth and survival.

Consequently, any associations, combinations or compositions comprising a GFR-binding compound and an adhesion protein inhibitor as defined herein, which, when used to convert a neoplastic cell into a non-neoplastic cell and treat/prevent/diagnose a neoplastic disease, down-regulate (in particular, substantially reduce) the expression of FAK genes and proteins and/or MAP kinase and/or reduce (in particular, substantially reduce) the expression or activity of GTPase Ras, Rho (Rho, Rac, Cdc42) in-vitro or in-vivo, shall be considered as being comprised within the scope of the present invention.

In one aspect, the present disclosure provides a pharmaceutical association, combination or (therapeutic, dermatologic, ophthalmologic, diagnostic, etc.) composition defined herein for use in a method of treating a neoplastic cell or a neoplastic disease by down-regulating (in particular, substantially reducing) the expression of FAK genes and proteins and/or MAP kinase and/or reducing (in particular, substantially reducing) the expression or activity of GTPase Ras, Rho (Rho, Rac, Cdc42) in-vitro or in-vivo. Also provided is a method of treating a neoplastic cell or a neoplastic disease by down-regulating (in particular, substantially reducing) the expression of FAK genes and proteins and/or MAP kinase and/or reducing (in particular, substantially reducing) the expression or activity of GTPase Ras, Rho (Rho, Rac, Cdc42) in-vitro or in-vivo, comprising administering to a subject in need thereof an effective amount of a pharmaceutical association, combination or (therapeutic, dermatologic, ophthalmologic, diagnostic, etc.) composition as defined herein.

Consequently, in certain embodiments, any associations, combinations or compositions comprising a GFR-binding compound and an adhesion protein inhibitor as defined herein, which, when used to convert a neoplastic cell into a non-neoplastic cell and treat/prevent a neoplastic disease, possesses at least one, preferably more than one, of the above defined biological and/or therapeutic parameters or effects (e.g. down-regulation of the Ras/MAP kinase, FAK/Src kinase and/or PIP2 signalling pathways and/or inhibition of gene expression or formation of cyclins D and/or down-regulation of the cyclin D/CDKs complexes), shall be considered as being comprised within the scope of the present invention.

In one aspect, the present disclosure provides a pharmaceutical association, combination or (therapeutic, dermatologic, ophthalmologic, diagnostic, etc.) composition defined herein for use in a method of converting a neoplastic cell into a non-neoplastic cell or treating/preventing a neoplastic disease by substantially inhibiting, down-regulating or dampening the gene expression or formation of at least one of, in particular a plurality of, the cyclin D proteins. Also provided is a method of converting a neoplastic cell into a non-neoplastic cell or treating/preventing a neoplastic disease by substantially inhibiting, down-regulating or dampening the gene expression or formation of at least one of, in particular

a plurality of, the cyclin D proteins, comprising administering to a subject in need thereof an effective amount of a pharmaceutical association, combination or (therapeutic, dermatologic, ophthalmologic, diagnostic, etc.) composition as defined herein.

In one aspect, the present disclosure provides a pharmaceutical association, combination or (therapeutic, dermatologic, ophthalmologic, diagnostic, etc.) composition defined herein for use in a method of converting a neoplastic cell into a non-neoplastic cell or treating/preventing a neoplastic disease by substantially inhibiting the formation of at least one of, in particular a plurality of, the protein complexes comprising at least one cyclin D and at least one CDK4 or CDK6. Also provided is a method of converting a neoplastic cell into a non-neoplastic cell or treating/preventing a neoplastic disease by substantially inhibiting the formation of at least one of, in particular a plurality of, the protein complexes comprising at least one cyclin D and at least one CDK4 or CDK6, comprising administering to a subject in need thereof an effective amount of a pharmaceutical association, combination or (therapeutic, dermatologic, ophthalmologic, diagnostic, etc.) composition as defined herein.

In one aspect, the present disclosure provides a pharmaceutical association, combination or (therapeutic, dermatologic, ophthalmologic, diagnostic, etc.) composition as defined herein for use in a method of converting a neoplastic cell into a non-neoplastic cell or treating/preventing a neoplastic disease without inducing the death of said neoplastic cell. Also provided is a method of converting a neoplastic cell into a non-neoplastic cell or treating/preventing a neoplastic disease without inducing the death of said neoplastic cell, comprising administering to a subject in need thereof an effective amount of a pharmaceutical association, combination or (therapeutic, dermatologic, ophthalmologic, diagnostic, etc.) composition as defined herein.

In one aspect, the present disclosure provides a pharmaceutical association, combination or (therapeutic, dermatologic, ophthalmologic, diagnostic, etc.) composition as defined herein for use as a G0 cell cycle phase inducer.

In one aspect, the present disclosure provides a pharmaceutical association, combination or (therapeutic, dermatologic, ophthalmologic, diagnostic, etc.) composition for use in a method of converting a neoplastic cell into a non-neoplastic cell or treating/preventing a neoplastic disease by dampening or arresting cell division and/or cell proliferation of said neoplastic cell. Also provided is a method of converting a neoplastic cell into a non-neoplastic cell or treating/preventing a neoplastic disease by dampening or arresting cell division and/or cell proliferation of said neoplastic cell, comprising administering to a subject in need thereof an effective amount of a pharmaceutical association, combination or (therapeutic, dermatologic, ophthalmologic, diagnostic, etc.) composition as defined herein.

In one aspect, the present disclosure provides a pharmaceutical association, combination or (therapeutic, dermatologic, ophthalmologic, diagnostic, etc.) composition for use in a method of converting a neoplastic cell into a non-neoplastic cell or treating/preventing a neoplastic disease by activating and/or promoting anti-mitogen activity and/or tumor suppressor pathways and/or anti-oncogenic activity in said neoplastic cell. Also provided is a method of converting a neoplastic cell into a non-neoplastic cell or treating/preventing a neoplastic disease by activating and/or promoting anti-mitogen activity and/or tumor suppressor pathways and/or anti-oncogenic activity in said neoplastic cell, comprising administering to a subject in need thereof an effective amount of a pharmaceutical association, combination or (therapeutic, dermatologic, ophthalmologic, diagnostic, etc.) composition as defined herein.

Data have also shown (FIGURE 7) that the administration or action of the pharmaceutical association, combination or composition as defined herein on neoplastic cells to be treated, down-regulated (in particular, substantially reduced) the expression of paxillin and/or up-regulated (in particular, substantially increased) the expression of vinculin.

Paxillin is a signal transduction adaptor protein. The C-terminal region of paxillin contains four LIM domains that target paxillin to focal adhesions through a direct association with the cytoplasmic tail of beta-integrin. The N-terminal region of paxillin is rich in protein-protein interaction sites. The proteins that bind to paxillin are diverse and include protein tyrosine kinases, such as Src and focal adhesion kinase (FAK), structural proteins, such as vinculin and actopaxin, and regulators of actin organization, such as COOL/PIX and PKL/GIT. Paxillin is tyrosine-phosphorylated by FAK and Src upon integrin engagement or growth factor stimulation, creating binding sites for the adapter protein Crk.

Vinculin is a focal adhesion protein that is involved in linkage of integrin adhesion molecules to the actin cytoskeleton. Vinculin is a cytoskeletal protein associated with cell-cell and cell-matrix junctions, where it is thought to function as one of several interacting proteins involved in anchoring F-actin to the membrane. Vinculin is a 117-kDa cytoskeletal protein of 1066 amino acids. The protein contains an acidic N-terminal domain and a basic C-terminal domain separated by a proline-rich middle segment. Vinculin consists of a globular head domain that contains binding sites for talin and $\alpha$-actinin as well as a tyrosine phosphorylation site, while the tail region contains binding sites for F-actin, paxillin, and lipids.

Consequently, any associations, combinations or compositions comprising a GFR-binding compound and an adhesion protein inhibitor as defined herein, which, when used to convert a neoplastic cell into a non-neoplastic cell and treat/prevent/diagnose a neoplastic disease, down-regulate the expression of paxillin and/or (preferably and) up-regulate the expression of vinculin in-vitro or in-vivo, shall be considered as being comprised within the scope of the present invention.

In one aspect, the present disclosure thus provides a pharmaceutical association, combination or (therapeutic,

dermatologic, ophthalmologic, diagnostic, etc.) composition defined herein for use in a method of treating a neoplastic cell or a neoplastic disease by down-regulating the expression of paxillin and/or (preferably and) up-regulating the expression of vinculin in-vitro or in-vivo. Also provided is a method of treating a neoplastic cell or a neoplastic disease by down-regulating the expression of paxillin and/or (preferably and) up-regulating the expression of vinculin in-vitro or in-vivo, comprising administering to a subject in need thereof an effective amount of a pharmaceutical association, combination or (therapeutic, dermatologic, ophthalmologic, diagnostic, etc.) composition as defined herein.

Data have also shown (FIGURES 1 to 4) that the administration or action of the pharmaceutical association, combination or composition as defined herein on neoplastic cells to be treated, up-regulated (in particular, substantially increased) the expression of at least one differentiation genes and proteins such as ADIPOQ (ACRP30), FABP4 and/or PPARG for adipocytes; ACAN (AGC1), COL10A1, COMP, Sox9, IBSP (Sialoprotein II) and/or COL4 for chondrocytes; CDH5, KDR (VEGFR3) and/or PECAM1 for the general endothelium; DLL4, EFNB2 and/or NRP1 for the arterial endothelium; LYVE1 and/or PROX1 for the lymphatic endothelium; NR2F2 and/or NRP2 for the venous endothelium; KRT1, KRT10 and/or KRT14 for the keratinocyte epithelium; PMEL (SILV), TYR and/or TYRP1 for the melanocyte epithelium; MMP-9, α-SMA and/or Vimentin for the fibrocyte; BGLAP, COL2A1, IBSP, RANK-L, OCN, DMP-1, Sclerostin and/or MEPE for Osteoblasts/Osteocytes; CALCR and/or CTSK for Osteoclasts; ITGB4 and/or KRT19 for cholangiocytes; ALB, G6PC and/or TAT for Hepatocytes; CD79A for early B-cell development; CD3E and/or PTCRA for early T-cell development; CCR5, CXCR4 and/or EMR1 for macrophages; ITGAM for the monocytes; GALC and/or GFAP for glial cells; MAP2, NEFH and/or TUBB3 for mature neurons; CHAT for cholinergic neurons; TH for dopaminergic neurons; GAD1 and/or SLC32A1 for GABA neurons; SLC17A6 and/or SLC17A7 for glutamatergic neurons; ISL1 for motor neurons; MBP for oligodendrocytes; POU4F2 for ganglion cells; RLBP1 for Muller cells; PDE6B and/or RCVRN for photoreceptor cells; NPHS2 for podocytes; AQP1, CYP27B1 and/or MIOX for proximal tubule cells; AQP2 for collecting duct cells; UMOD for distal tubule cells; SFTPB, SFTPC and/or SFTPD for lung cells; MYH6, MYH7 and/or NPPA for cardiomyocytes; CAV3, MYH1, MYOD1, GATA4 and/or MLC1 for skeletal muscle cells; MYH11, SMTN and/or TAGLN for smooth muscle cells; GCG, MAFB and/or POU3F4 for alpha cells; INS, MAFA and/or SLC2A2 for beta cells; SST for delta cells; GHRL (Ghrelin, Obestatin) for epsilon cells; PPY for pancreatic polypeptide producing (PP) cells; and/or CPA1 for exocrine cells.

Consequently, any associations, combinations or compositions comprising a GFR-binding compound and an adhesion protein inhibitor as defined herein, which, when used to convert a neoplastic cell into a non-neoplastic cell and treat/prevent/diagnose a neoplastic disease, up-regulate (in particular, substantially increase) the expression of at least one differentiation genes and proteins such as ADIPOQ (ACRP30), FABP4, PPARG, ACAN (AGC1), COL10A1, COMP, Sox9, IBSP (Sialoprotein II), COL4, CDH5, KDR (VEGFR3), PECAM1, DLL4, EFNB2, NRP1, LYVE1, PROX1, NR2F2, NRP2, KRT1, KRT10, KRT14, PMEL (SILV), TYR, TYRP1, MMP-9, α-SMA, Vimentin, BGLAP, COL2A1, IBSP, RANK-L, OCN, DMP-1, Sclerostin, MEPE, CALCR, CTSK, ITGB4, KRT19, ALB, G6PC, TAT, CD79A, CD3E, PTCRA, CCR5, CXCR4, EMR1, ITGAM, GALC, GFAP, MAP2, NEFH, TUBB3, CHAT, TH, GAD1, SLC32A1, SLC17A6, SLC17A7, ISL1, MBP, POU4F2, RLBP1, PDE6B, RCVRN, NPHS2, AQP1, CYP27B1, MIOX, AQP2, UMOD, SFTPB, SFTPC, SFTPD, MYH6, MYH7, NPPA, CAV3, MYH1, MYOD1, GATA4, MLC1, MYH11, SMTN, TAGLN, GCG, MAFB, POU3F4, INS, MAFA, SLC2A2, SST, GHRL (Ghrelin, Obestatin), PPY and/or CPA1, in-vitro or in-vivo, shall be considered as being comprised within the scope of the present invention.

In one aspect, the present disclosure thus provides a pharmaceutical association, combination or (therapeutic, dermatologic, ophthalmologic, diagnostic, etc.) composition defined herein for use in a method of treating a neoplastic cell or a neoplastic disease by up-regulating (in particular, substantially increasing) the expression of differentiation genes and proteins such as ADIPOQ (ACRP30), FABP4, PPARG, ACAN (AGC1), COL10A1, COMP, Sox9, IBSP (Sialoprotein II), COL4, CDH5, KDR (VEGFR3), PECAM1, DLL4, EFNB2, NRP1, LYVE1, PROX1, NR2F2, NRP2, KRT1, KRT10, KRT14, PMEL (SILV), TYR, TYRP1, MMP-9, α-SMA, Vimentin, BGLAP, COL2A1, IBSP, RANK-L, OCN, DMP-1, Sclerostin, MEPE, CALCR, CTSK, ITGB4, KRT19, ALB, G6PC, TAT, CD79A, CD3E, PTCRA, CCR5, CXCR4, EMR1, ITGAM, GALC, GFAP, MAP2, NEFH, TUBB3, CHAT, TH, GAD1, SLC32A1, SLC17A6, SLC17A7, ISL1, MBP, POU4F2, RLBP1, PDE6B, RCVRN, NPHS2, AQP1, CYP27B1, MIOX, AQP2, UMOD, SFTPB, SFTPC, SFTPD, MYH6, MYH7, NPPA, CAV3, MYH1, MYOD1, GATA4, MLC1, MYH11, SMTN, TAGLN, GCG, MAFB, POU3F4, INS, MAFA, SLC2A2, SST, GHRL (Ghrelin, Obestatin), PPY and/or CPA1, in-vitro or in-vivo. Also provided is a method of treating a neoplastic cell or a neoplastic disease by up-regulating (in particular, substantially increasing) the expression of differentiation genes and proteins such as ADIPOQ (ACRP30), FABP4, PPARG, ACAN (AGC1), COL10A1, COMP, Sox9, IBSP (Sialoprotein II), COL4, CDH5, KDR (VEGFR3), PECAM1, DLL4, EFNB2, NRP1, LYVE1, PROX1, NR2F2, NRP2, KRT1, KRT10, KRT14, PMEL (SILV), TYR, TYRP1, MMP-9, α-SMA, Vimentin, BGLAP, COL2A1, IBSP, RANK-L, OCN, DMP-1, Sclerostin, MEPE, CALCR, CTSK, ITGB4, KRT19, ALB, G6PC, TAT, CD79A, CD3E, PTCRA, CCR5, CXCR4, EMR1, ITGAM, GALC, GFAP, MAP2, NEFH, TUBB3, CHAT, TH, GAD1, SLC32A1, SLC17A6, SLC17A7, ISL1, MBP, POU4F2, RLBP1, PDE6B, RCVRN, NPHS2, AQP1, CYP27B1, MIOX, AQP2, UMOD, SFTPB, SFTPC, SFTPD, MYH6, MYH7, NPPA, CAV3, MYH1, MYOD1, GATA4, MLC1, MYH11, SMTN, TAGLN, GCG, MAFB, POU3F4, INS, MAFA, SLC2A2, SST, GHRL (Ghrelin, Obestatin), PPY and/or CPA1, in-vitro or in-vivo, comprising administering to a subject in need thereof an effective amount of a pharmaceutical association, combination or (therapeutic, dermatologic, ophthalmologic,

diagnostic, etc.) composition as defined herein.

Data have also shown (FIGURE 5) that the administration or action of the pharmaceutical association, combination or composition as defined herein on neoplastic cells to be treated increased the phosphorylation of the protein p53.

Data have also shown (FIGURE 5) that the administration or action of the pharmaceutical association, combination or composition as defined herein on neoplastic cells to be treated down-regulated (in particular, substantially decreased or suppressed) the phosphorylation of the protein pRb.

Data have also shown (FIGURE 6) that the administration or action of the pharmaceutical association, combination or composition as defined herein on neoplastic cells to be treated down-regulated the protein Src.

Tumor protein p53, also known as p53, cellular tumor antigen p53, phosphoprotein p53, or tumor suppressor p53, is a protein that in humans is encoded by the *TP53* gene. The p53 protein is involved in multicellular organisms, where it regulates the cell cycle and, thus, functions as a tumor suppressor, generally preventing neoplastic diseases such as cancers. As such, p53 has been described as "the guardian of the genome" because of its role in conserving stability by preventing genome mutation. *TP53* is thus said to be a tumor suppressor gene.

Src is a non-receptor protein tyrosine kinase that plays a multitude of roles in cell signalling. Src is involved in the control of many functions, including cell adhesion, growth, movement and differentiation. Src is widely expressed in many cell types, and can have different locations within a cell. It appears that the subcellular location of Src can affect its function. Src can associate with cellular membranes, such as the plasma membrane, the perinuclear membrane and the endosomal membrane. At the plasma membrane, Src can transduce signals from a variety of receptors to internal signalling pathways that convey these signals to the nucleus, cytoskeleton and other cellular components. For example, Src can act through growth factor receptors to affect cell growth and proliferation. Within the nucleus, Src is thought to help regulate the cell cycle and cell division by its interactions with other proteins. For example, Src can interact with Sam68 to help regulate gene expression. In bone osteoclasts, Src acts to regulate the respiratory enzyme cytochrome C oxidase (Cox), where Src-induced phosphorylation of Cox is required for maintaining high levels of ATP to meet the cells' high energy requirements. Src can also be found in the cytoplasm, and between cells at adherens junctions, where it takes on different roles.

pRb (Retinoblastoma protein) is a tumor suppressor protein that is dysfunctional in several major cancers. One function of pRb is to prevent excessive cell growth by inhibiting cell cycle progression until a cell is ready to divide. It is also a recruiter of several chromatin remodeling enzymes such as methylases and acetylases. Rb restricts the cell's ability to replicate DNA by preventing its progression from the G1 (first gap phase) to S (synthesis phase) phase of the cell cycle. Rb binds and inhibits transcription factors of the E2F family, which are composed of dimers of an E2F protein and a dimerization partner (DP) protein. When Rb is bound to E2F, the complex acts as a growth suppressor and prevents progression through the cell cycle. Rb is phosphorylated to pRb by certain Cyclin Dependent Kinases (CDKs). pRb is described as being hyperphosphorylated and when in this state, it is unable to complex E2F and therefore, unable to restrict progression from the G1 phase to the S phase of the cell cycle. During the M-to-G1 transition, pRb is progressively dephosphorylated by PP1, returning to its growth-suppressive hypophosphorylated state Rb.

Consequently, any associations, combinations or compositions comprising a GFR-binding compound and an adhesion protein inhibitor as defined herein, which, when used to convert a neoplastic cell into a non-neoplastic cell and treat/prevent/diagnose a neoplastic disease, down-regulate (in particular, substantially decrease or suppress) the expression of genes and proteins p53 and/or Src, in-vitro or in-vivo, shall be considered as being comprised within the scope of the present invention.

In one aspect, the present disclosure thus provides a pharmaceutical association, combination or (therapeutic, dermatologic, ophthalmologic, diagnostic, etc.) composition defined herein for use in a method of treating a neoplastic cell or a neoplastic disease by down-regulating (in particular, substantially decreasing or suppressing) the expression of genes and proteins p53 and/or Src. Also provided is a method of treating a neoplastic cell or a neoplastic disease by down-regulating (in particular, substantially decreasing or suppressing) the expression of genes and proteins p53 and/or Src, comprising administering to a subject in need thereof an effective amount of a pharmaceutical association, combination or (therapeutic, dermatologic, ophthalmologic, diagnostic, etc.) composition as defined herein.

In one aspect, the present disclosure provides a pharmaceutical association, combination or (therapeutic, dermatologic, ophthalmologic, diagnostic, etc.) composition defined herein for use in a method of treating a neoplastic cell or a neoplastic disease by:

- down-regulating (in particular, substantially reducing) the expression of FAK genes and proteins and/or MAP kinase; and/or
- reducing (in particular, substantially reducing) the expression or activity of GTPase Ras, Rho (Rho, Rac, Cdc42); and/or
- down-regulating (in particular, reducing, significantly reducing or suppressing) the Ras/MAP kinase, FAK/Src kinase and/or PIP2 signalling pathways; and/or
- substantially inhibiting the formation of at least one of, in particular a plurality of, the protein complexes comprising

at least one cyclin D and at least one CDK4 or CDK6; and/or

- substantially inhibiting, down-regulating or dampening the gene expression or formation of at least one of, in particular a plurality of, the cyclin D proteins; and/or
- down-regulating the expression of paxillin and/or (preferably and) up-regulating the expression of vinculin; and/or
- up-regulating (in particular, substantially increasing) the expression of at least one of differentiation genes and proteins such as ADIPOQ (ACRP30), FABP4, PPARG, ACAN (AGC1), COL10A1, COMP, Sox9, IBSP (Sialoprotein II), COL4, CDH5, KDR (VEGFR3), PECAM1, DLL4, EFNB2, NRP1, LYVE1, PROX1, NR2F2, NRP2, KRT1, KRT10, KRT14, PMEL (SILV), TYR, TYRP1, MMP-9, $\alpha$-SMA, Vimentin, BGLAP, COL2A1, IBSP, RANK-L, OCN, DMP-1, Sclerostin, MEPE, CALCR, CTSK, ITGB4, KRT19, ALB, G6PC, TAT, CD79A, CD3E, PTCRA, CCR5, CXCR4, EMR1, ITGAM, GALC, GFAP, MAP2, NEFH, TUBB3, CHAT, TH, GAD1, SLC32A1, SLC17A6, SLC17A7, ISL1, MBP, POU4F2, RLBP1, PDE6B, RCVRN, NPHS2, AQP1, CYP27B1, MIOX, AQP2, UMOD, SFTPB, SFTPC, SFTPD, MYH6, MYH7, NPPA, CAV3, MYH1, MYOD1, GATA4, MLC1, MYH11, SMTN, TAGLN, GCG, MAFB, POU3F4, INS, MAFA, SLC2A2, SST, GHRL (Ghrelin, Obestatin), PPY and/or CPA1; and/or
- increasing the phosphorylation of protein p53; and/or
- down-regulating (in particular, substantially decreasing or suppressing) the phosphorylation of the protein pRb; and/or
- down-regulating (in particular, substantially decreasing or suppressing) the gene and protein expression of Src; and/or
- not inducing the death of said neoplastic cell; and/or
- dampening or arresting cell division or cell proliferation of said neoplastic cell.

In one example, the pharmaceutical association as defined herein may thus be an animal or mammal (preferably a human) anti-neoplastic disease agent (e.g. anti-cancer agent) which has demonstrated the ability to convert a neoplastic cell into a non-neoplastic cell in vitro, ex-vivo and/or in vivo and/or to protect a subject from a neoplastic disease, disorder, condition, pathology or any symptoms thereof.

In one aspect, the present disclosure also provides methods of converting a neoplastic cell into a non-neoplastic cell, said method comprising the administration to a cell, in-vitro, ex-vivo or in-vivo, of an effective amount of a pharmaceutical association, combination or composition as defined herein. In one example, said methods induce the differentiation of said neoplastic cell. In one example, said conversion is effected in less than 7 days, in less than 6 days, 5 days, 4 days, 3 days, 2 days, 24 hours or 18 hours, in particular in less than 24 hours. In other examples:

- said neoplastic cell is a non-terminally differentiated cell and said methods comprise the differentiation of said non-terminally differentiated; and/or
- said methods comprise the conversion and/or recoding of a neoplastic cell to induce and/or promote and/or improve self-healing and/or self-recovery thereof; and/or
- said methods comprise restoring the ability of a neoplastic cell to undergo differentiation; and/or
- said methods comprise converting and/or recoding a circulating or non-circulating neoplastic cell such as a metastatic or non-metastatic cancer cell, into a non-neoplastic cell; and/or
- said methods comprise providing and/or producing and/or inducing the formation of a physiologically functional and/or healthy cell of the bone, chondrocyte, vascular, fibroblast, renal, retinal, neuronal, ligament, tendon, reproduction system, lung, blood, adipocyte cell lineage from a neoplastic cell; and/or
- said methods comprise inducing and/or promoting and/or enhancing neoplastic cell differentiation; and/or
- said methods comprise non-mutagenically protecting a subject from a neoplastic disease i.e. without modifying or altering the genome of the treated neoplastic cells; and/or
- said methods comprise the extracellular treatment of a neoplastic disease, disorder, condition, pathology, or any symptoms thereof; and/or
- said methods comprise down-regulating (in particular, substantially reducing) the expression of FAK genes and proteins and/or MAP kinase and/or reducing (in particular, substantially reducing) the expression or activity of GTPase Ras, Rho (Rho, Rac, Cdc42) in-vitro or in-vivo; and/or
- said methods comprise restoring the cell adhesion checkpoint of said neoplastic cell; and/or
- said methods comprise inhibiting the ECM binding of at least one integrin or one syndecan transmembrane proteins of said neoplastic cell; and/or
- said methods comprise substantially inhibiting, down-regulating or dampening the gene expression or formation of at least one of, in particular a plurality of, the cyclin D proteins; and/or
- said methods comprise substantially inhibiting the formation of at least one of, in particular a plurality of, the protein complexes comprising at least one cyclin D and at least one CDK4 or CDK6; and/or
- said methods comprise not inducing the death of said neoplastic cell; and/or
- said methods comprise dampening or arresting cell division and/or cell proliferation of said neoplastic cell; and/or
- said methods comprise activating and/or promoting anti-mitogen activity and/or tumor suppressor pathways and/or

anti-oncogenic activity in said neoplastic cell; and/or

- said methods comprise down-regulating the gene expression of paxillin and/or (preferably and) up-regulating the gene expression or increasing turn-over of vinculin in-vitro or in-vivo; and/or
- said methods comprise up-regulating (in particular, substantially increasing) the expression of differentiation genes and proteins such as ADIPOQ (ACRP30), FABP4, PPARG, ACAN (AGC1), COL10A1, COMP, Sox9, IBSP (Sialoprotein II), COL4, CDH5, KDR (VEGFR3), PECAM1, DLL4, EFNB2, NRP1, LYVE1, PROX1, NR2F2, NRP2, KRT1, KRT10, KRT14, PMEL (SILV), TYR, TYRP1, MMP-9, $\alpha$-SMA, Vimentin, BGLAP, COL2A1, IBSP, RANK-L, OCN, DMP-1, Sclerostin, MEPE, CALCR, CTSK, ITGB4, KRT19, ALB, G6PC, TAT, CD79A, CD3E, PTCRA, CCR5, CXCR4, EMR1, ITGAM, GALC, GFAP, MAP2, NEFH, TUBB3, CHAT, TH, GAD1, SLC32A1, SLC17A6, SLC17A7, ISL1, MBP, POU4F2, RLBP1, PDE6B, RCVRN, NPHS2, AQP1, CYP27B1, MIOX, AQP2, UMOD, SFTPB, SFTPC, SFTPD, MYH6, MYH7, NPPA, CAV3, MYH1, MYOD1, GATA4, MLC1, MYH11, SMTN, TAGLN, GCG, MAFB, POU3F4, INS, MAFA, SLC2A2, SST, GHRL (Ghrelin, Obestatin), PPY and/or CPA1, in-vitro or in-vivo; and/or
- said methods comprise down-regulating (in particular, substantially decreasing or suppressing) the gene expression of proteins p53, pRb and/or Src.

In one example, the pharmaceutical association as defined herein may thus be an animal or mammal (preferably a human) anti-neoplastic disease agent (e.g. anti-cancer agent) which has demonstrated the ability to convert a neoplastic cell into a non-neoplastic cell in vitro, ex-vivo and/or in vivo and/or to protect a subject from a neoplastic disease, disorder, condition, pathology or any symptoms thereof.

In one example, said administration induces the quiescence of the neoplastic cell. In one example, said administration prevents, reduces or suppresses the cell division and/or cell proliferation of said neoplastic cell. In one example, said administration regulates or promotes anti-mitogen activity and/or tumor suppressor activity and/or anti-oncogenic activity in said neoplastic cell. In one example, said method is cytostatic for the treated cell. In one example, said method is not cytotoxic for the treated cell. In one example, said method is non-mutagenic.

In one aspect, the present disclosure provides a method of producing a physiologically functional and healthy cell, comprising the administration in-vitro, ex-vivo or in-vivo to a neoplastic cell an effective amount of a pharmaceutical association or composition as defined herein and wherein said physiologically functional and healthy cell is selected from the group consisting of an osteoblast, osteocyte, chondroblast, chondrocyte, neuroblast, neurocyte, Sertoli cells, Leydig cell, Germ cell, Myoblast, Myocyte, keratinocyte, endothelial cells, angioblast, fibroblast, fibrocyte, podocyte.

In one aspect, the present disclosure provides a method of converting a cell "X" into a cell "Y", said method comprising the administration to a neoplastic cell of an effective amount of a pharmaceutical association, combination or composition as defined herein, wherein cell X is a neoplastic cell selected from the group consisting of:

a. a metastatic or non-metastatic neoplastic cell;
b. a cell comprising at least one alteration, mutation and/or deregulation of at least one tumour suppressor gene or of at least one of their gene-encoded product (such as for example the Rb and/or P53 genes or gene products);
c. a cell comprising at least one alteration, mutation and/or deregulation of at least one of proto-oncogene or of at least one of their gene-encoded product (such as for example Src -tyrosine kinase- and/or Ras -small GTPase);
d. a cell having an impaired DNA repair mechanism (such as for example BRCA1 and BRCA2);
e. a cell comprising at least one alteration, mutation and/or deregulation of at least one mitogen and/or anti-mitogen gene or at least one of their gene-encoded products (such as for example TGF-beta);
f. a cell having an defective, altered or impaired cell cycle or cell cycle control, wherein said defective cell cycle is due to either at least one alteration, mutation and/or deregulation of at least one cell cycle gene or gene-encoded product (for example Cyclin E1, Cyclin D1, CDK 4/6) at least one alteration, mutation and/or deregulation of at least one cell cycle checkpoint or diminution or reduction of a checkpoint sensibility and/or responsiveness;
g. a stem cell-like cell comprising a Stro-1 positive marker and having multi-potency;
h. a cell comprising at least one specific differentiation marker such as ADIPOQ (ACRP30), FABP4, PPARG, ACAN (AGC1), COL10A1, COMP, Sox9, IBSP (Sialoprotein II), COL4, CDH5, KDR (VEGFR3), PECAM1, DLL4, EFNB2, NRP1, LYVE1, PROX1, NR2F2, NRP2, KRT1, KRT10, KRT14, PMEL (SILV), TYR, TYRP1, MMP-9, $\alpha$-SMA, Vimentin, BGLAP, COL2A1, IBSP, RANK-L, OCN, DMP-1, Sclerostin, MEPE, CALCR, CTSK, ITGB4, KRT19, ALB, G6PC, TAT, CD79A, CD3E, PTCRA, CCR5, CXCR4, EMR1, ITGAM, GALC, GFAP, MAP2, NEFH, TUBB3, CHAT, TH, GAD1, SLC32A1, SLC17A6, SLC17A7, ISL1, MBP, POU4F2, RLBP1, PDE6B, RCVRN, NPHS2, AQP1, CYP27B1, MIOX, AQP2, UMOD, SFTPB, SFTPC, SFTPD, MYH6, MYH7, NPPA, CAV3, MYH1, MYOD1, GATA4, MLC1, MYH11, SMTN, TAGLN, GCG, MAFB, POU3F4, INS, MAFA, SLC2A2, SST, GHRL (Ghrelin, Obestatin), PPY and/or CPA1;
i. a cell having increased cytoplasmic and nuclear stiffness;
j. a cell having high cytoskeleton dynamics; and
k. any combination thereof;

and wherein cell Y is a non-neoplastic cell selected from the group consisting of:

a. a cell in a terminal differentiation state;
b. a cell in a non-terminal differentiation state and more differentiated than the X cell;
c. a cell having a physiologically normal proliferation;
d. a physiologically functional cell having healthy or normal genes expression and proteins activity.

In one aspect, the present disclosure provides methods to activate a growth factor receptor present on the surface of a neoplastic cell, said method comprising administering to said neoplastic cell an effective amount of a pharmaceutical association, combination or composition as defined herein, wherein administering said pharmaceutical association, combination or composition activates the growth factor receptor present on the surface of the neoplastic cell.

In one aspect, the present disclosure provides methods of delivering a pharmaceutical composition in-vitro, ex-vivo or in-vivo to a neoplastic cell, comprising the contacting of said neoplastic cell with said pharmaceutical composition, and wherein said pharmaceutical composition comprises a GFR-binding compound and an adhesion protein inhibitor as defined herein.

In one aspect, the present disclosure provides methods of administering a pharmaceutical association, combination or composition comprising a GFR-binding compound and an adhesion protein inhibitor to a subject comprising the contacting of at least one body part of said subject with said pharmaceutical association, combination or composition.

**Protected from a disease,** condition, disorder or pathology refers to the treatment of the underlying cause of the disease, condition, disorder or pathology as well as reducing the symptoms of the disease, condition, disorder or pathology; and/or reducing the occurrence of the disease, condition, disorder or pathology; and/or reducing the severity of the disease, condition, disorder or pathology. Protecting a patient can refer to the ability of a therapeutic composition of the present invention, when administered to a patient, to prevent a disease, condition, disorder or pathology from occurring and/or to cure or to alleviate disease, condition, disorder or pathology symptoms, signs or causes. As such, to protect a patient from a disease, condition, disorder or pathology includes both preventing disease, condition, disorder or pathology occurrence (prophylactic treatment) and treating a patient that has a disease, condition, disorder or pathology or that is experiencing initial symptoms or later stage symptoms of a disease, condition, disorder or pathology (therapeutic treatment).

**Treating:** As used herein, unless indicated otherwise or contradictory in context, the term "treating" or "treatment" refers to partially or completely alleviating, ameliorating, improving, relieving, delaying onset of, inhibiting progression of, reducing severity of, and/or reducing incidence of one or more symptoms or features of a particular disease, disorder, pathology and/or condition. For example, in certain embodiments, "treating" or "treatment of" cancer may refer to inhibiting survival, growth, and/or spread of a tumor. Treatment may be administered to a subject who does not exhibit signs of a disease, disorder, and/or condition and/or to a subject who exhibits only early signs of a disease, disorder, pathology and/or condition for the purpose of decreasing the risk of developing pathology associated with the disease, disorder, and/or condition.

As used herein, "treating a neoplastic cell" is used interchangeably with the expression "converting a neoplastic cell into a non-neoplastic cell".

**Preventing:** As used herein, unless indicated otherwise or contradictory in context, the term "preventing" refers to partially or completely delaying onset of an infection, disease, disorder and/or condition; partially or completely delaying onset of one or more symptoms, features, or clinical manifestations of a particular infection, disease, disorder, and/or condition; partially or completely delaying onset of one or more symptoms, features, or manifestations of a particular infection, disease, disorder, and/or condition; partially or completely delaying progression from an infection, a particular disease, disorder and/or condition; and/or decreasing the risk of developing pathology associated with the infection, the disease, disorder, and/or condition.

**Substantially diminished, reduced or inhibited:** As used herein, unless indicated otherwise or contradictory in context, the term "substantially diminished" or "substantially inhibited" as it relates to cellular activity and/or function, protein expression or complexes formation, refers to a diminution or reduction in activity or function, in comparison with the activity and/or function of a normal or healthy cell (e.g. a non-neoplastic cell), of 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99%, in particular of 50% to 99%, 55% to 99%, 60% to 99%, 65% to 99%, 70% to 99%, 75% to 99%, 80% to 99%, 85% to 99% or 90% to 99%. The same applies conversely to the term "substantially increased".

**Disease:** As used herein, unless indicated otherwise or contradictory in context, the term "disease" refers to any deviation from the normal health of a patient and includes a state when disease symptoms are present, as well as

conditions in which a deviation has occurred, but symptoms are not yet manifested. A defective, ill-functioning, malfunctioning, dysfunctioning, and/or deleterious cell, such as *e.g.* a neoplastic cell, may be a cause of a disease. The same applies to "condition", "disorder" and "pathology".

**Neoplasm:** As used herein, unless indicated otherwise or contradictory in context, the term "neoplasm" (sometimes also referred to as tumor or tumour) refers to an abnormal growth of tissue. This abnormal growth usually but not always forms a mass. The World Health Organization classifies neoplasms into four main groups: benign neoplasms, in situ neoplasms, malignant neoplasms, and neoplasms of uncertain or unknown behavior. A malignant neoplasm is a cancer.

**Neoplastic cell:** As used herein, unless indicated otherwise or contradictory in context, the term "neoplastic cell" refers to a cell with abnormal proliferation, generally due the presence of a defective cell cycle, as well as cells which may become neoplastic such as cancer stem cell for which a definition is given in "Refining the role for adult stem cells as cancer cells of origin", White AC, Lowry WE, Trends Cell Biol. 2014 Sep 18. pii: S0962-8924(14)00145-7, and "review article Stem cells, cancer, and cancer stem cells", Tannishtha Reya et al., Nature 414, 105-111 (1 November 2001), which are incorporated herein by reference in their entirety.

**Neoplastic disease:** As used herein, unless indicated otherwise or contradictory in context, the term "neoplastic disease", used interchangeably with "neoplastic conditions", "neoplastic disorder" and "neoplastic pathology", refers to a disease associated with an abnormal growth of tissue, generally due to the presence of a neoplastic cell.

**Circulating:** As used herein, unless indicated otherwise or contradictory in context, the term "circulating", when used in relation to a neoplastic cell, refers to a neoplastic cell that has detached itself from a primary tumor site and migrates to a different location in the body to become a metastatic tumor.

**Cancers:** As used herein, unless indicated otherwise or contradictory in context, the term "cancer" refers to a malign form of neoplasm which includes sarcomas, carcinomas, lymphomas, leukemias (or leukaemias), teratomas, mesotheliomas, myelomas and germinomas. Cancers include, but are not limited to, Acute lymphoblastic leukaemia (ALL), Acute myeloid leukaemia, Adrenocortical carcinoma, AIDS-related cancers, AIDS-related lymphoma, Anal cancer, Appendix cancer, Astrocytoma, childhood cerebellar or cerebral, Basal-cell carcinoma, Bile duct cancer, Bladder cancer, Bone tumor, osteosarcoma/malignant fibrous histiocytoma, Brainstem glioma, Brain cancer, Breast cancer, Bronchial adenomas/carcinoids, Burkitt's lymphoma, Carcinoid tumor, Central nervous system lymphoma, Cerebellar astrocytoma, Cerebral astrocytoma/malignant glioma, Cervical cancer, Childhood cancers, Chronic bronchitis, Chronic lymphocytic leukaemia, Chronic myelogenous leukaemia, Chronic myeloproliferative disorders, Chronic obstructive pulmonary disease (COPD), Colon cancer, Cutaneous T-cell lymphoma, Desmoplastic small round cell tumor, Emphysema, Endometrial cancer, Ependymoma, Esophageal cancer, Ewing's sarcoma in the Ewing family of tumors, Extracranial germ cell tumor, Extragonadal germ cell tumor, Extrahepatic bile duct cancer, Eye cancer, Gallbladder cancer, Gastric (stomach) cancer, Gastrointestinal carcinoid tumor, Gastrointestinal stromal tumor (GIST), Germ cell tumor: extracranial, extragonadal, or ovarian, Gestational trophoblastic tumor, Glioma of the brain stem, Glioma, Gastric carcinoid, Hairy cell leukemia, Head and neck cancer, Heart cancer, Hepatocellular (liver) cancer, Hodgkin lymphoma, Hypopharyngeal cancer, Hypothalamic and visual pathway glioma, childhood, Intraocular melanoma, Islet cell carcinoma (endocrine pancreas), Kaposi sarcoma, Kidney cancer (renal cell cancer), Laryngeal cancer, Leukemias, acute lymphoblastic leukemia, acute myeloid leukemia, chronic lymphocytic leukemia, chronic myelogenous leukemia, hairy cell Leukemia, Lip and oral cavity cancer, Liposarcoma, Liver cancer, Lung cancer, AIDS-related lymphoma, Burkitt lymphoma, cutaneous T-Cell lymphoma, Hodgkin lymphoma, Non-Hodgkin lymphoma, primary central nervous system lymphoma, Macroglobulinemia, Male breast cancer, Malignant fibrous histiocytoma of bone/osteosarcoma, Medulloblastoma, intraocular (eye) Melanoma, Merkel cell cancer, Mesothelioma, Metastatic squamous neck cancer with occult primary, Mouth cancer, Multiple endocrine neoplasia syndrome, Multiple myeloma/plasma cell neoplasm, Mycosis fungoides, Myelodysplastic syndromes, Myelodysplastic/myeloproliferative diseases, multiple Myeloma, Myeloproliferative disorders, Nasal cavity and paranasal sinus cancer, Nasopharyngeal carcinoma, Neuroblastoma, Oral cancer, Oropharyngeal cancer, Osteosarcoma/malignant fibrous histiocytoma of bone, Ovarian cancer, Ovarian epithelial cancer, Ovarian germ cell tumor, Ovarian low malignant potential tumor, Pancreatic cancer, Pancreatic cancer, islet cell Paranasal sinus and nasal cavity cancer, Parathyroid cancer, Penile cancer, Pharyngeal cancer, Pheochromocytoma, Pineal astrocytoma, Pineal germinoma, Pineoblastoma and supratentorial primitive neuroectodermal tumors, Pituitary adenoma, Plasma cell neoplasia/Multiple myeloma, Pleuropulmonary blastoma, Primary central nervous system lymphoma, Prostate cancer, Rectal cancer, Renal cell carcinoma (kidney cancer), Renal pelvis and ureter, transitional cell cancer, Retinoblastoma, Rhabdomyosarcoma, Salivary gland cancer, Ewing family of tumors Sarcoma, Kaposi Sarcoma, soft tissue Sarcoma, uterine Sarcoma, Sezary syndrome, Skin cancer (non-melanoma), Skin cancer (melanoma), Merkel cell Skin carcinoma, Small intestine cancer, Soft tissue sarcoma, metastatic squamous neck cancer with occult primary, Stomach cancer, Supratentorial primitive neuroectodermal tumor, cutaneous T-Cell lymphoma, Testicular cancer, Throat cancer, Thymoma and thymic carcinoma, Thyroid cancer, Transitional cell cancer of the renal pelvis and ureter, Trophoblastic tumor, transitional cell cancer, Urethral cancer, Uterine cancer, Uterine sarcoma, Vaginal cancer, Visual pathway and hypothalamic glioma, Vulvar cancer, Waldenström macroglobulinemia, and Wilms tumor (kidney cancer). In certain embodiments, cancers that may

be treated using the recoding therapy disclosed herein are selected from the group consisting of sarcomas such as Osteosarcoma, Osteoma, Chondrosarcoma, Chondroma, Leiomyosarcoma, Leiomyoma, Rhabdomyosarcoma, Rhabdomyoma, Mesothelioma, Fibrosarcoma, Fibroma, Malignant Histiocytoma, Hisitocytoma, Angiosarcoma, Hemangiosarcoma, Hemangioma, Hemangiopericytoma, Lymphangiosarcoma, Lymphangioma, Liposarcoma, Lipid Cell Tumor, Lipoma, Myxosarcoma, Chordoma, Cystosarcoma Phylloides, Fibroadenoma, Seminoma, Dysgerminoma, Sertoli-Leydig Cell Tumor, Arrhenoblastoma, Granulose-Theca Cell Tumor, Hilar Cell Tumor, Prostate Cancer, Prostatic Hypertrophy, Wilms Tumor, Melanoma, Nevus, Neurofibrosarcoma, Glioma, Anaplastic Glioma, Glioblastoma, Neuroblastoma, Medulloblastoma, Ganglioneuroma, Malignant meningioma, Meningioma, Malignant Schwannoma, Schwannoma, Neurilemmoma, Neurofibroma and Mesenchymous Tumor; Carcinomas such as Squamous Cell Carcinoma, Merkel Cell Neoplasm, Epidermoid Carcinoma, malignant Skin Adnexal Tumor, Adenocarcinoma, Hepatocellular Carcinoma, Renal Cell Carcinoma, Choriocarcinoma, Hypernephroma, Cholangiocarcinoma, Transitional Cell Carcinoma, Seminoma, Embryonal Cell Carcinoma, Papilloma, Seborrheic Keratosis, Skin Adnexal Tumors, Hepatoma, Adenoma, Hepatic Adenoma, Renal Tubular Adenoma, Bile Duct Adenoma, Transitional Cell Papilloma, Hydatidiform Mole, Parathyroid Carcinoma, Parathyroid Adenoma, Medullary Carcinoma of Thyroid, C Cell Hyperplasia, Malignant Pheochromocytoma, Pheochromocytoma, Islet Cell Carcinoma, Islet Cell Adenoma, Insulinoma, Gastrinoma, Malignant Carcinoid, Carcinoid, Malignant Paraganglioma, Chemodectoma and Paraganglioma; Myelomas; Leukemias such as Myelogenous Leukemia, Lymphatic Leukemia, Policythemia, Aleukemic Leukemia, Preleukemia and Myeloproliferative Disorders; Lymphoma such as Myeloma, Hodgkin Lymphoma, Non-Hodgkin Lymphoma, Plasmacytoma and Plasmacytosis; Adenosquamous Carcinoma, Mesodermal Tumor, Carcinosarcoma and Teratocarcinoma.

**Symptoms of neoplastic diseases:** In one example, the present invention treats or alleviates at least one symptom selected from Angina, Bradypnea, Breathing difficulties, Gradual vision change in both eyes, Gradual vision changes in one eye, Head symptoms, Heartburn, Muscle symptoms, Nerve symptoms, Perforated gastric ulcer, Perforated ulcer, Respirations, shallow, Sexual symptoms, Weight loss, Oesophagus symptoms, Weight symptoms, Anaemia, Bad breath, Breath odors, Burning feet, Burning Legs, Cleft lip with or without cleft palate in children, Communication symptoms, Diarrhea, Dyspepsia, Easily fatigued, Enlarged liver, Face symptoms, Foot pain, Gastritis, Gastrooesophageal reflux in pregnancy, Glossitis, Hairy Tongue, Hyperlipidaemia, Hypertriglyceridemia, Impotence, Lack of energy, Lag in breathing, Male infertility, Meniere's disease, Movement symptoms, Musculoskeletal symptoms, Night sweats in children, Non-respiratory causes of shallow respiration, Raised ambulatory 24hr pH monitoring, Rapid heartbeat, Respiratory lag, Sleep symptoms, Sleeplessness, Speech symptoms, Sweat symptoms, Trauma-related symptoms, Voice symptoms, Vomiting, White tongue, Abdominal symptoms, Abnormal blood test symptoms, Acute reflux-like regurgitation in pregnancy, Acute reflux-like symptoms in pregnancy, Acute reflux-like vomiting in pregnancy, Acute stomach ulcer-like symptoms in pregnancy, Birth symptoms, Blanching in the fingers, Bleeding symptoms, Blood symptoms, Blood vessel damage, Chronic pain in multiple muscles, Digestive symptoms, Elevated blood pressure in pregnancy, Episodic arterial vasospasm, Female infertility, Female reproductive symptoms, Fertility symptoms, Heart enlargement, Hiatus hernia, Hiatus hernia related to chronic digestive disorders, High blood pressure, Hypertension-like symptoms in pregnancy, Impaired myocardial contractility, Increased Systolic pressure, Infection, Infertility, Intermittent claudication, Intermittent claudication of both lower limbs, Intermittent claudication of one lower limb, Intermittent pain of the lower limb, Lower abdominal symptoms, Lower blood pressure in the legs than in the arms, Mouth symptoms, Mycocardial ischemia, Noncompliance, Occlusion of renal arteries, Pain, Peripheral arterial trauma, Poor appetite, Poor wound healing, Pregnancy symptoms, Pulsatile Abdominal swelling, Red blood cell symptoms, Reflux-like symptoms, Respiratory symptoms, Sensory symptoms, Severe chronic pain in multiple bones, Sore throat, Sweating, Throat symptoms, Urinary incontinence, Venous insufficiency, Women's health symptoms, Birth defects, Eating symptoms, 1 litre of sweat per hour, Aberrant behaviour in pregnancy, Abnormal eye movements, Abnormal Liver Function Tests in Pregnancy, Abnormal thinking in pregnancy, Absenteeism, Accentuated fall in systolic pressure, Acidosis, Action tremor, Acute epilepsy-like symptoms, Acute forgetfulness in pregnancy, Acute hemorrhagic pancreatitis, Acute intermittent forgetfulness in pregnancy, Acute onset of daytime sleepiness, Acute onset of daytime sleepiness in adults, Acute onset of daytime sleepiness in the elderly, Acute pancreatitis, Addiction symptoms, Aggression, Alcohol abuse, Alcohol breath odor, Alcohol use, Altered bladder habits in pregnancy, Altered mental state, Altered vital signs, Anxiety disorder in pregnancy, Anxiety in pregnancy, Apprehension in pregnancy, Arrhythmia, Arrhythmias, Atrial fibrillation, Balance symptoms, Behavior problems at school, Behavior problems at work, Behavior problems in adults, Behavior problems in children, Behavior problems in teens, Behavioral symptoms, Behaviour changes in pregnancy, Blackouts, Cerebellar ataxia, Chronic Diarrhea, Chronic orthostatic hypotension, Cleft palate in children, Clumsiness, CNS depression in children, Cognitive impairment, Coma, Confusion, Confusion in children, Coordination problems, De-personalization, Decreased level of consciousness, Deep stupor, Dehydration, Delayed ejaculation, Delirium, Delusions, Dementia, Depressed mental status, Depressive symptoms, Diabetic retinopathy, Diaphoresis of the feet, Diaphoresis of the forehead, Diaphoresis of the palms, Diaphoresis of the soles, Difficulty concentrating in adults, Disoriented state, Diuresis, Dramatic fall in blood pressure, Drowsiness, Drug abuse, Drug withdrawal causing new-onset seizures, Drugs, Drugs causing diarrhea, Drugs causing macrocytosis, Drugs causing persistent hypoglycemia, Drugs causing persistent hypoglycemia in adolescents, Drugs

causing persistent hypoglycemia in children, Drugs causing persistent hypoglycemia in infants, Dysphagia due to esophagitis, Emotional symptoms, Energy symptoms, Epilepsy-like symptoms, Episodic Diaphoresis, Episodic Diaphoresis as in case of diabetes mellitus, Euphoria, Excessive diaphoresis, Eye symptoms, Fainting, Fainting episodes similar as in pulmonary hypertension, Falls, Fatigue, Feeling the heartbeat, Flushing, Forgetfulness, Forgetfulness in pregnancy, Frequency of urination in pregnancy, Frequent urination, Frequent urination in pregnancy, Generalised diaphoresis, Generalised diaphoresis with heat loss via evaporation, Hallucinations, Headache, Heart arrhythmia, Heart arrhythmias, Heart flutters, High ALP, High ALT, High AST, Hyperosmolarity, Hypoperfusion state, Hypothermia, Hypothermic attack, Imbalance, Impaired circulation, Inadequate intravascular pressure, Inattention, Inattention symptoms, Increased bilirubin, Increased GGT, Increased prothrombin time, Increased sweating, Intercourse symptoms, Intermittent diaphoresis, Intermittent epilepsy-like symptoms, Intermittent seizures in early childhood, Involuntary tonic movements, Irregular heartbeat, Irritability, Jaundice in pregnancy, Level of consciousness symptoms, Liver tenderness in pregnancy, Low albumin, Low blood platelet level in pregnancy, Low blood pressure, Low blood sugar, Low temperature, Major depression in pregnancy, Male impotence, Memory disturbances, Memory Disturbances in pregnancy, Memory loss in pregnancy, Memory symptoms, Memory symptoms in pregnancy, Mental changes in pregnancy, Mental depression, Mental depression in pregnancy, Mental problems, Mental problems in pregnancy, Mild depression in pregnancy, Mild depression-like symptoms in pregnancy, Mild personality changes, Moderate depression in pregnancy, Mood swings, Muscle pain, Muscle weakness, Myoclonus, Nerve damage, Nervousness, Nervousness in pregnancy, Neuromuscular irritability, Neuromuscular irritability of lower limbs, Neuropathy, Night time urination in pregnancy, Night urination in pregnancy, Nocturia in pregnancy, Numbness in Both Feet, Numbness in one foot, Nystagmus, Ocular myokymia, Orthostatic hypotension, Ototoxic medications, Palpitations, Pancreatitis, Paresthesias, Penile Burning Sensation, Persistent painful erection, Personality change, Personality symptoms, Phobia, Progressive mental deterioration, Prolonged exposure to cold, Psychiatric symptom, Pulse irregularity in pregnancy, Red face, Reduced immune response, Reduced intravascular pressure, Reduced sperm count, Retinopathy, School problems, Seizures, Seizures in infants and early childhood, Self-esteem symptoms, Severe epilepsy-like symptoms, Sexual dysfunction in pregnancy, Short-term memory loss, Signs of circulatory collapse, Signs of circulatory collapse as seen in intestinal hemorrhage, Sleeping problems, Slurred speech, Social problems, Society problems, Sole numbness, Standing symptoms, Stupor, Subfertility, Substance abuse causing school underachievement and academic failure, Substances of abuse causing delirium, Sudden onset of confusion in children, Syncopal episode, Thigh Burning Sensation, Thrombocytopenia, Thrombocytopenia in pregnancy, Tingling in Both Feet, Tingling in both hands, Tingling in one foot, Tingling in one hand, Tonic-clonic seizure, Toxins causing vomiting, Tremor, Unusual body odor in children, Urinary burning, Urinary urgency, Urine retention, Vascular collapse, Vertigo, Violent behaviour, Walking symptoms, Weight loss in pregnancy, Abnormal uterine bleeding, Abortion, Abruptio Placentae in Pregnancy, Abscessed teeth, Acid Reflux in pregnancy, Acquired mental retardation, Acute acid reflux into mouth during pregnancy, Acute attack of asthma, Acute cerebral infarct, Acute seasonal asthma-like symptoms, Aphthous Ulcer, Arm burning sensation, Arm coldness, Arm infection, Arm inflammation, Arm numbness, Arm paresthesia, Arm redness, Arm sensitive, Arm spasm, Arm swelling, Atelectasis, Biceps burning sensation, Biceps cold, Biceps infection, Biceps inflammation, Biceps numb, Biceps redness, Biceps spasm, Biceps swelling, Biceps tingling, Bilateral cleft lip and palate, Bilateral complete cleft lip, Bilateral stroke, Black hairy tongue, Bladder symptoms, Blood clots, Blood lead concentration, Blood pressure symptoms, Blood vessel symptoms, Blue lips, Bone changes, Breath sound symptoms, Breath symptoms, Bronchial cancer, Bronchitis, Brown nails, Cancer-related symptoms, Cardiovascular symptoms, Cervix symptoms, Chronic bronchitis, Chronic cough, Chronic cough in adolescents, Circulation symptoms, Claudication, Cluster headache, Constant leg pain, Coracobrachialis infection, Coracobrachialis redness, Coracobrachialis swelling, Cough, Cramping leg pain, Cramping pain in both legs, Dark spots on teeth, Darkened tongue, Death, Death-related symptoms, Decreased salivary function, Digit burning sensation, Digit cold, Digit infection, Digit inflammation, Digit numb, Digit redness, Digit sensitive, Digit spasm, Digit swelling, Digit tingling, Disc herniation, Disc prolapse, Discolored teeth in children, Dry cough, Dry Gangrene, Dry mucous membrane as in case of Sjogren's syndrome, Dry skin, Electrocardiogram changes, Eructation, Exercise symptoms, Female genital symptoms, Female sexual symptoms, Femur burning sensation, Femur numb, Femur tingling, Fetal symptoms, Fibula burning sensation, Fibula numb, Fibula tingling, Finger burning sensation, Finger pulp burning sensation, Fingernail burning sensation, Fingers burning sensation, Fingers cold, Fingers infection, Fingers inflammation, Fingers numb, Fingers redness, Fingers sensitive, Fingers spasm, Fingers swelling, Fingers tingling, Forearm burning sensation, Forearm cold, Forearm infection, Forearm inflammation, Forearm numb, Forearm redness, Forearm sensitive, Forearm spasm, Forearm swelling, Forearm tingling, Foul mouth odour, Genital symptoms, Growth symptoms, Heart damage, Heart disease, Heart rhythm symptoms, Heart symptoms, Heartburn after eating in pregnancy, Heartburn after exercise in pregnancy, Heartburn in pregnancy, Heartburn pain resistant to treatment in pregnancy, Heartburn that worsens if lying down after eating in pregnancy, Heartburn unrelated to eating in pregnancy, Heartburn with acid reflux in pregnancy, Heartburn without reflux in pregnancy, Herniated disk, Hiccups, High lipoprotein level, Hoarse, Humerus burning sensation, Humerus numb, Humerus tingling, Hypoxia in pregnancy, Increased capillary refill time, Increased secretions in the lung, Indigestion in pregnancy, Infant symptoms, Inflammatory symptoms, Irregular rhythm, Kidney symptoms, Knee joint burning sensation, Knee joint cold, Knee joint numb, Knee

joint sensitive, Knee joint tingling, Kneecap burning sensation, Kneecap cold, and any combinations thereof.

In one aspect, the present disclosure provides methods to activate, promote, support, improve, or increase the activity of a growth factor receptor present in, on the surface of a neoplastic cell such that the neoplastic cell in converted into a non-neoplastic cell and/or a subject carrying said cell is protected from a neoplastic disease, said methods optionally comprising the administration of a pharmaceutical association as defined herein.

In one aspect, the present disclosure provides methods to activate, promote, support, improve, or increase the tumor suppressor activity of proteins p53 and/or pRb (retinoblastoma protein) in a neoplastic cell such that the neoplastic cell in converted into a non-neoplastic cell and/or a subject carrying said cell is protected from a neoplastic disease, said methods comprising the administration of a pharmaceutical association as defined herein.

In one aspect, the present disclosure provides methods to convert a neoplastic cell (e.g. a cancer cell) into a non-neoplastic cell (*e.g.* a non-cancerous cell) and/or to protect a subject carrying this cell from a neoplastic disease, disorder, condition, pathology or at least one symptom thereof, which involves dampening or inhibiting cell division and/or regulating, re-establishing or restoring a cell adhesion checkpoint and/or inducing differentiation of said neoplastic cell.

In one aspect, the present disclosure provides a GFR-binding compound as defined herein, for use in the prevention, diagnostic and/or treatment of a neoplastic disease in combination with at least one adhesion protein inhibitor as defined herein.

In one aspect, the present disclosure provides an adhesion protein inhibitor, for use in the prevention, diagnostic and/or treatment of a neoplastic disease in combination with at least one growth factor receptor-binding compound as defined herein.

## XII. Diagnostic methods

In one aspect, the present disclosure provides methods of identifying, diagnosing, and optionally classifying subjects on these bases, which may include clinical diagnosis, biomarker levels, and other methods known in the art.

In one aspect, the present disclosure provides a pharmaceutical association or composition as defined herein for use in a diagnostic method of a neoplastic disease (e.g. a cancer). In one example, such a diagnostic method comprises the detection of a cancer using a method described in US patent application No. 2002/0159986 A1, which is incorporated herein by reference in its entirety.

In one aspect, the present disclosure provides a diagnostic method for diagnosing of a neoplastic disease or condition comprising the provision of a pharmaceutical association or composition as defined herein, and the contacting or administration of said pharmaceutical association or composition with a body part of a subject to be diagnosed.

A method for the diagnosis of cancer in a patient, comprising obtaining a biological sample from a patient and apply fluorescent and/or radiolabeled GFR binding compounds, wherein high localisation of these compounds indicates cancer in the patient.

In one aspect, the present disclosure provides methods of determining the effectiveness of a pharmaceutical association, combination or composition as defined herein for converting a neoplastic cell into a non-neoplastic cell and/or for treating a neoplastic disease or at least one symptom thereof, comprising the administration of said pharmaceutical association to a cell; the measurement of the expression of specific differentiation and/or cancerous markers as defined herein in the cell; the comparison of the expression of said specific differentiation and/or cancerous markers in the cell to the expression of said specific differentiation and/or cancerous markers in a cell treated with a reference (or control) pharmaceutical association, compound or solvent; and determining the effectiveness of the pharmaceutical association or composition relative to the reference pharmaceutical association or compound.

## XIII. Screening methods

In one aspect, the present disclosure provides a screening method for selecting a GFR-binding compound having the ability, after being associated or combined with an adhesion protein inhibitor as defined herein (thus forming a pharmaceutical association or combination as defined herein), to convert or recode a neoplastic cell into a non-neoplastic cell.

In one aspect, the present disclosure provides a screening method for selecting a peptide or a peptidomimetic having the ability, after being associated or combined with an adhesion protein inhibitor as defined herein (thus forming a pharmaceutical association or combination as defined herein), to convert or recode a neoplastic cell into a non-neoplastic cell.

In one aspect, the present disclosure provides a screening method for selecting a peptide or peptidomimetic having the ability, after being associated or combined with an adhesion protein inhibitor as defined herein (thus forming a pharmaceutical association or combination as defined herein), to convert or recode a neoplastic cell into a non-neoplastic cell, the method comprising the steps of (a) providing a molecular model of the 3D structure coordinates of PEPREF as defined herein and (b) identifying a candidate analog having a RMSD value of 2.45Å or less, in particular

of 2 Å or less, and more particularly of 1 .79 Å or less. In one particular example, step (b) is performed using the method of RMSD calculation as already defined herein.

In certain embodiments, the method can be performed using a computer (i.e., in silico). In some embodiments, the method can include providing the three-dimensional models of a plurality of peptides or peptidomimetics (i.e., a library or database of peptides or peptidomimetics) and screening each compound individually. Thus, in one aspect, the method of screening peptides or peptidomimetics generally includes computationally evaluating the potential of a selected peptide(s) or peptidomimetic(s) to structurally match with the computational model of the three-dimensional structure of PEPREF. For example, this method can include the steps of (a) employing a computational approach to perform a fitting operation between the selected peptide(s) or peptidomimetic(s) and the three-dimensional structure of PEPREF; and (b) analysing the results of the fitting operation to quantify the three-dimensional structural similarities between the peptide(s) or peptidomimetic(s) and PEPREF using the RMSD procedure as defined herein.

In one aspect, the present disclosure provides a method of producing a peptide or peptidomimetic having the ability, after being associated or combined with an adhesion protein inhibitor as defined herein (thus forming a pharmaceutical association or combination as defined herein), to convert or recode a neoplastic cell into a non-neoplastic cell, the method comprising the steps of (a) providing a molecular model of the following 3D structure coordinates of PEPREF; b) identifying a candidate analog having a RMSD as defined herein of 2.45Å or less (in particular 2, more particularly 1.79); and (c) producing the candidate analog identified in step (b). In one particular example, said method further comprising the step of determining whether the compound produced in step (c) has a neoplastic cell recoding activity. In one particular example, steps (a) and (b) are performed by means of an electronic processor. In certain embodiments, step (a) comprises storing a representation of the atomic co-ordinates of PEPREF in a computer memory.

In one aspect, the present disclosure provides a method of producing a peptide or peptidomimetic having the ability, after being associated or combined with an adhesion protein inhibitor as defined herein (thus forming a pharmaceutical association or combination as defined herein), to convert or recode a neoplastic cell into a non-neoplastic cell, the method comprising the steps of: (a) providing in a computer memory atomic X-ray crystallographic co-ordinates of PEPREF; (b) generating with a processor a molecular model having a three-dimensional shape of PEPREF; (c) identifying a candidate analog having a RMSD of 2.45Å or less (in particular 2, more particularly 1.79); (d) producing the candidate analog identified in step (c); and (e) determining whether the candidate analog produced in step (d), once associated or combined with an adhesion protein inhibitor as defined herein (thus forming a pharmaceutical association or combination as defined herein), has the ability to convert or recode a neoplastic cell into a non-neoplastic cell. In one particular example, said method comprises the additional step of producing the peptide or peptidomimetic in a commercially useful quantity.

In one aspect, the present disclosure provides a computer system comprising: (a) a memory comprising atomic X-ray crystallographic coordinates of PEPREF; and (b) a processor in electrical communication with the memory; wherein the processor generates a molecular model having a three dimensional shape representative of PEPREF. In one particular example, said coordinates are stored on a computer readable diskette.

## XIV. Kits

The present disclosure provides a variety of kits for conveniently and/or effectively carrying out methods and uses of the present invention. Typically, kits will comprise sufficient amounts and/or numbers of components to allow a user to perform multiple treatments of a subject(s) and/or to perform multiple experiments.

In one aspect, the present disclosure provides kits for pharmaceutical association production, comprising at least one GFR-binding compound as defined herein, at least one adhesion protein inhibitor as defined herein, both provided in an amount effective to produce a pharmaceutical association to convert a neoplastic cell into a non-neoplastic cell and/or treat, prevent or diagnose a neoplastic disease when administered in-vitro, ex-vivo or in-vivo to a neoplastic cell or a subject carrying such a neoplastic cell, and packaging and instructions.

In one aspect, the present disclosure provides kits for pharmaceutical composition production, comprising a GFR-binding compound as defined herein, an adhesion protein inhibitor as defined herein, and a pharmaceutically acceptable excipient, carrier or vehicle, each of them provided in an amount effective to produce a pharmaceutical composition to convert a neoplastic cell into a non-neoplastic cell and/or treat or prevent a neoplastic disease when administered in-vitro, ex-vivo or in-vivo to a neoplastic cell or a subject carrying such a neoplastic cell, and packaging and instructions.

Suitable as solvents for use in kits of the invention include physiologically acceptable solvents, PBS, filtered and deionised water such as Milli-Q® water, alpha-MEM, DMEM and/or IMDM. All physiologically acceptable solvents suitable for implementing embodiments of the present invention are preferably deoxygenated before use.

In one example, said kit further comprises an administration device. In one example, said administration device is a dispensing device such as a syringe.

In one example, said kit comprises a first container containing a GFR-binding compound as defined herein and

a second container containing an adhesion protein inhibitor.

In one particular example, said kits of the invention may suitably be provided in the form of a sterile packaging.

For example, in certain embodiments, said kits of the invention comprises more than 2, between 2 and 25, between 2 and 15, or between 2 and 10 of GFR-binding compound as defined herein, and more than 2, between 2 and 25, between 2 and 15, or between 2 and 10 GFR-binding compound as defined herein.

In one example, each GFR-binding compound and each adhesion protein inhibitor is conditioned in distinct and separated compartments, in lyophilised form, in solution or in suspension in a pharmaceutically dermatologically, prophylactically, diagnostically, imaging or cosmetically acceptable excipient, carrier or vehicle.

In one aspect, the invention discloses kit-of-parts comprising an adhesion protein inhibitor and a GFR-binding compound both as defined herein for uses and methods as defined herein.

## XV. Sequence listing

Examples of (modified) non-cyclic GFR-binding compounds as defined herein are listed in the appended sequence listing which forms an integral part of the present application.

Examples of (modified) cyclic GFR-binding compounds as defined herein are listed in the appended sequence listing which forms an integral part of the present application. (Modified) cyclic GFR-binding compounds of the present disclosure may be represented in non-cyclic, linear, form solely for the purpose of ease of representation but nevertheless remains cyclic compounds wherein the first and the last functional group (e.g. an amino acid) of the linearly represented compound are covalently connected to each other thereby forming a cyclic structure.

Pharmaceutical associations, combinations and compositions as defined in the present disclosure, has been found to lead to multiple and distinct advantages in terms of neoplastic diseases treatment.

As supported by the examples of the present application, pharmaceutical associations, combinations and compositions as defined in the present disclosure provide for a new area of treatment of neoplastic diseases by extracellularly converting or recoding neoplastic cells into functional and healthy cells. The therapy of the invention containing pharmaceutical associations, combinations or compositions as defined herein display advantages over therapies that do not contain it, such as any one of, and preferably a plurality of:

- Enhanced and/or more practical and/or more efficient and/or more cost-effective and/or more adapted to the end-user needs;
- Converting or recoding a neoplastic cell to induce and/or promote and/or improve self-healing and/or self-recovery thereof, particularly in a shorter period of time;
- Converting a neoplastic cell into a non-neoplastic cell and/or protecting a subject from a neoplastic disease, disorder, condition, pathology, such as cancer, or symptoms thereof using non-mutagenic means;
- Converting a neoplastic cell into a non-neoplastic cell and/or protecting a subject from a neoplastic disease, disorder, condition, pathology, such as cancer, or symptoms thereof using extracellular means;
- Restoring a neoplastic cell ability to undergo differentiation, particularly in a shorter period of time;
- Converting and/or recoding a circulating or non-circulating neoplastic cell such as a metastatic or non-metastatic cancer cell, into a non-neoplastic cell, particularly in a shorter period of time;
- Protecting a subject from a neoplastic disease, disorder, condition or pathology such as cancer, and/or any symptoms thereof, particularly in a shorter period of time;
- Providing and/or producing a physiologically functional and/or healthy osteoblast, osteocyte, chondroblast, chondrocyte, neuroblast, neurocyte, Sertoli cells, Leydig cell, Germ cell, Myoblast, Myocyte, keratinocyte, endothelial cells, angioblast, fibroblast, fibrocyte or podocyte from a neoplastic cell, particularly within a shorter period of time;
- Re-establishing, reactivating or restoring the cell adhesion checkpoint of a neoplastic cell;
- Treating a neoplastic cell without substantially inducing cell death;
- Inducing quiescence of a neoplastic cell;
- Dampening, reducing or suppressing cell division/proliferation of a neoplastic cell;
- Activating and/or promoting and/or up-regulating anti-mitogen activity and/or tumor suppressor pathways and/or anti-oncogenic activity in a neoplastic cell;
- Adapted to the treatment of any neoplastic cell;
- Safer for the patient being treated as providing less or no harmful side-effects;
- Offers the possibility for local treatments of neoplastic diseases;
- Relies mainly or only on the activation of physiological cell mechanisms which increases the general safety of the herein defined therapy.

For example, in certain embodiments, pharmaceutical association or combinations, or compositions according to the present disclosure in which PEP1 is a peptide selected from the group consisting of SAIS, NAIS, SATS and SPIS,

in which PEP2 is a peptide selected from the group consisting of LKNYQ, LKKYR, LRKHR and LKYHY, have been found to lead to unexpectedly fast conversion of a cancerous cell into a healthy and functional cell of the bone lineage.

For example, in certain embodiments, pharmaceutical association or combinations, or compositions according to the present disclosure in which PEP1 is a peptide selected from the group consisting of SAIS, NAIS, SPIS, EPLP, and EPLT, in which PEP2 is a peptide selected from the group consisting of LKNYQ, LKKYR, YKQYE and EQLSN, have been found to lead to unexpectedly fast conversion of a cancerous cell into a healthy and functional cell of the chondrocytic cell lineage.

For example, in certain embodiments, pharmaceutical association or combinations, or compositions according to the present disclosure in which PEP1 is a peptide selected from the group consisting of SNIT, RPVQ and RSVK, in which PEP2 is a peptide selected from the group consisting of IGEMS, LGEMS, KEVQV and KKATV, have been found to lead to unexpectedly fast conversion of a cancerous cell into a healthy and functional cell of the vascular lineage.

For example, in certain embodiments, pharmaceutical association or combinations, or compositions according to the present disclosure in which PEP1 is selected from the group consisting of NAIS, SPIS and EPIS, wherein PEP2 is selected from the group consisting of LKKYR, LKYHY, YKQYE and KFKYE, have been found to lead to unexpectedly fast conversion of a cancerous cell into a healthy and functional cell of the neuron lineage.

For example, in certain embodiments, pharmaceutical association or combinations, or compositions according to the present disclosure in which PEP1 is SPIN, in which PEP2 is YGKIP, have been found to lead to unexpectedly fast conversion of a cancerous cell into a healthy and functional cell of the eye-retina lineage.

For example, in certain embodiments, pharmaceutical association or combinations, or compositions according to the present disclosure in which PEP1 is SPIN, and in which PEP2 is YGKIP, have been found to lead to unexpectedly fast conversion of a cancerous cell into a healthy and functional cell of the renal lineage.

For example, in certain embodiments, pharmaceutical association or combinations, or compositions according to the present disclosure in which PEP1 is a peptide selected from the group consisting of NAIS, SPIS, EPLP and EPLT, in which PEP2 is selected from the group consisting of LKKYR, YKQYE and EQLSN, have been found to lead to unexpectedly fast conversion of a cancerous cell into a healthy and functional cell of the ligament and tendon lineage.

For example, in certain embodiments, pharmaceutical association or combinations, or compositions according to the present disclosure in which PEP1 is a peptide selected from the group consisting of EPLP, EPLT, RSVK and RPVQ, in which PEP2 is a peptide selected from the group consisting of EQLSN, IGEMS, KEVQV and KKATV, have been found to lead to unexpectedly fast conversion of a cancerous cell into a healthy and functional cell of the fibroblast lineage.

For example, in certain embodiments, pharmaceutical association or combinations, or compositions according to the present disclosure in which PEP1 is NAIS, in which PEP2 is LKKYR, have been found to lead to unexpectedly fast conversion of a cancerous cell into a healthy and functional cell of the reproduction system lineage.

For example, in certain embodiments, pharmaceutical association or combinations, or compositions according to the present disclosure in which PEP1 is selected from the group consisting of NAIS, SATS, SPIS, EPIS and SPIN, in which PEP2 is selected from the group consisting of LKKYR, LRKHR, LKYHY, KFKYE and YGKIP, have been found to lead to unexpectedly fast conversion of a cancerous cell into a healthy and functional cell of the lung cell lineage.

For example, in certain embodiments, pharmaceutical association or combinations, or compositions according to the present disclosure in which PEP1 is selected from the group consisting of RSVK or RPVQ, in which PEP2 is selected from the group consisting of KEVQV or KKATV, have been found to lead to unexpectedly fast and qualitatively and quantitatively important muscle cells induction, producing highly functional differentiated cells.

For example, in certain embodiments, pharmaceutical association or combinations, or compositions according to the present disclosure in which PEP1 is SNIT, in which PEP2 is IGEMS or LGEMS, have been found to lead to unexpectedly fast conversion of a cancerous cell into a healthy and functional cell of the blood cell lineage.

For example, in certain embodiments, pharmaceutical association or combinations, or compositions according to the present disclosure in which PEP1 is SAIS or NAIS, in which PEP2 is LKNYQ or LKKYR, have been found to lead to unexpectedly fast conversion of a cancerous cell into a healthy and functional cell of the adipocyte lineage.

Accordingly, it is possible to achieve the conversion of a neoplastic cell (e.g. a cancer cell) into a non-neoplastic cell (*e.g.* a functional and/or healthy cell of the bone, chondrocytic, muscle, vascular, neuronal, retinal, renal, ligament, tendon, fibroblast, blood, lung, adipocyte, reproduction system cell lineages) using extracellular, non-mutagenic active principles and is thus useful in the treatment of neoplastic diseases such as cancers.

The present invention provides a recoding therapy for the treatment of neoplastic diseases, conditions or disorders such as cancer and aims at providing a suitable micro-environment to the neoplastic cell containing suitable biological signals so that the neoplastic cell is able to undergo self-healing and become a functional, healthy, non-neoplastic cell.

In addition, it has been found that using the recoding therapy of the invention instead of conventional therapies allows for improved treatment selectivity in such a way that reduced or substantially no adverse side-effect is observed. Without wishing to be bound to any specific theory, this may be explained by considering that the present therapy relies

on the activation of physiological cell mechanisms without imposing or forcing the treated cell to undergo e.g. apoptosis or cell cycle arrest (which would then most likely unselectively target any cells, healthy and neoplastic) but instead reestablishes the correct cellular microenvironment surrounding this neoplastic cell so that the cell is able to operate self-healing. As a result, a non-neoplastic cell *i.e.* a cell in which the cell cycle and/or cell adhesion checkpoint(s) need not be repaired, brought in contact with such a microenvironment would be less or not be substantially affected.

Remarkably, it has also been found that using the pharmaceutical associations or compositions as defined herein allows for the reduction (in most cases, important reduction) of the time of treatment of a neoplastic cell and by extension of a neoplastic disease in comparison with known technologies.

It is another aspect of the present invention to solve the technical problem of providing a therapy for treating neoplastic diseases being completely or at least partially devoid of one or more, preferably a plurality of the disadvantages of known treatments.

All combinations of any of the above-mentioned features described in all above part of the present description are specifically contemplated by the Applicant to be within the scope of the present invention unless contradictory in context. Examples of such combinations are detailed throughout the present description.

Further embodiments and advantages will become apparent to a skilled reader in light of the examples provided below.

## EXAMPLES

Disclosed and described, it is to be understood that this invention is not limited to the particular examples, process steps, and materials disclosed herein as such process steps and materials may vary somewhat. It is also to be understood that the terminology used herein is used for the purpose of describing particular embodiments only and not intended to be limiting since the scope of the present invention will be limited only by the appended claims and equivalents thereof.

The following Examples are representative of techniques employed by the inventor in carrying out aspects of the present invention. It should be appreciated that while these techniques are exemplary of preferred embodiments for the practice of the invention, those of skill in the art, in light of the present disclosure, will recognize that numerous modifications can be made without departing from the spirit and intended scope of the invention.

**The following starting materials and reagents were used:**

**[1235]** - Type-I collagen sponge was obtained from Sigma®.
- PBS 1X was obtained from Gibco®.
- MilliQ water: is water characterised in terms of resistivity (typically 18.2 $M\Omega \cdot cm$ at 25 °C).
- Low glucose Dulbecco's Modified Eagle Medium (DMEM) was obtained from Invitrogen®
- Minimum Essential Medium Eagle without ascorbic acid ($\alpha$MEM) was obtained from Invitrogen®.
- All of the cell culture experiments were carried out without any serum in the medium for the first 8 hours of culture.
- Osteosarcoma cells: MG63 cells were obtained from ATCC®. These cells were cultured in DMEM (Eagle's Minimum Essential Medium) medium supplemented with 10% fetal calf serum (FCS) and 1% penicillin/streptomycin. All cells were used at a low passage number (passage 10), were subconfluently cultured and were seeded at $10^4$cells/$cm^2$ for the purpose of the experiments.
- Chondrosarcoma cells Hs819.T cells were obtained from ATCC®. These cells were cultured in DMEM (Eagle's Minimum Essential Medium) medium supplemented with 10% fetal calf serum (FCS) and 1% penicillin/streptomycin. All cells were used at a low passage number (passage 10), were subconfluently cultured and were seeded at $10^4$cells/$cm^2$ for the purpose of the experiments.
- Rhabdomyosarcoma cells: A-673 cells were obtained from ATCC®. These cells were cultured in DMEM (Eagle's Minimum Essential Medium) medium supplemented with 10% fetal calf serum (FCS) and 1% penicillin/streptomycin. All cells were used at a low passage number (passage 10), were subconfluently cultured and were seeded at $10^4$cells/$cm^2$ for the purpose of the experiments.
- Adenocarcinoma cells: HCC4006 cells were obtained from ATCC®. These cells were cultured in RPMI-1640 medium (obtained from ATCC®) supplemented with 10% fetal calf serum (FCS) and 1% penicillin/streptomycin. All cells were used at a low passage number (passage 10), were subconfluently cultured and were seeded at $10^4$cells/$cm^2$ for the purpose of the experiments.
- CMFDA is a Cell Tracker Green obtained from Invitrogen®.
- DAPI was obtained from Sigma®.
- Fetal bovine serum (FBS) was obtained from Gibco®.
- Penicillin/streptomycin was obtained from Invitrogen®.
- The AlamarBlue® assay was obtained from Molecular Probes®.

- Anti phospho-p53 antibody was obtained from Santa Cruz Biotechnology®,
- Anti pRB antibody was obtained from Santa Cruz Biotechnology®,
- Monoclonal anti-integrin (extracellular domain) αv, α5, α3, α4, β1, β3, β5 and β9 antibodies were obtained from Santa Cruz Biotechnology®,
- Cell cytoskeletal filamentous actin (F-actin) was visualized by Alexa Fluor® 488 phalloidin Sigma® labeling.
- Monoclonal anti-vinculin antibody (clone hVIN-1, produced in mouse) was obtained from Sigma®.
- Primers for Cyclin D1: 5'-CCGTCCATGCGGAAGATC-3' (Forward) and 5'-GAAGACCTCCTCCTCGCACT-3' (Reverse) were obtained from Invitrogen®.
- Primers for Cyclin D2: 5'-CATCCAACCGTACATGCGCAG-3' (Forward) and 5'-CATGGCCAGAGGAAAGACCTC-3' (Reverse) were obtained from Invitrogen®.
- Primers for Cyclin D3: 5'-TGATTTCCTGGCCTTCATTC-3' (Forward) and 5'-CGGGTACATGGCAAAGGTAT-3' (Reverse) were obtained from Invitrogen®.
- Primers for Paxillin: 5'-AATTCCAGTGCCTCCAACAC-3' (Forward) and 5'-GAGCTCATGACGGTAGGTGA-3' (Reverse) were obtained from Invitrogen®.
- Primers for Vinculin: 5'-GCCAAGCAGTGCACAGATAA-3' (Forward) and 5'-TTCCTTTCTGGTGTGTGAAGC -3' (Reverse) were obtained from Invitrogen®.
- Primers for DMP-1: 5'-AGCATCCTGCTCATGTTCCTTT-3' (Forward) and 5'-GAGCCAAATGACCCTTCCATT-3' (Reverse) were obtained from Invitrogen®.
- Primers for Sclerostin: 5'-CTTAGTTTTCTCAGTCTGTGGTTGAAAT-3' (Forward) and 5'-AGAGTACCCCGAGCCTCC-3' (Reverse) were obtained from Invitrogen®.
- Primers for RANK-L: 5'-CTCAGCCTTTTGCTCATCTCACT-3' (Forward) and 5'-CCAAGAGGACAGACTCACTTTATGG-3' (Reverse) were obtained from Invitrogen®.
- Primers for MLC-1: 5'-AGAAGGGCTCCATGTCTGACA-3' (Forward) and 5'-AAGATTTCAGGACCCGAGCAG -3' (Reverse) were obtained from Invitrogen®.
- Primers for GATA-4: 5'-AGGCCTCTTGCAATGCGGA-3' (Forward) and 5'-CTGGTGGTGGCGTTGCTGG -3' (Reverse) were obtained from Invitrogen®.
- Primers for α-Sarcomeric actin: 5'-GACCACAGCTGAACGTGAGA-3' (Forward) and 5'-CATAGCACGATGGTCGATTG-3' (Reverse) were obtained from Invitrogen®.
- Primers for Sox9: 5'-GACTTCCGCGACGTGGAC-3' (Forward) and 5'-GTTGGGCGGCAGGTACTG-3' (Reverse) were obtained from Invitrogen®.
- Primers for IBSP: 5'-TGCCTTGAGCCTGCTTCC-3' (Forward) and 5'-GCAAAATTAAAGCAGTCTTCATTTTG-3' (Reverse) were obtained from Invitrogen®.
- Primers for Collagen IV: 5'-CCTGGTCTTGAAAGGTGATAAG-3' (Forward) and 5'-CCCGCTATCCCTTGATCTC -3' (Reverse) were obtained from Invitrogen®.
- Primers for Vimentin: 5'-TGTCCAAATCGATGTGGATGTTTC-3' (Forward) and 5'-TTGTACCATTCTTCTGCCTCCTG-3' (Reverse) were obtained from Invitrogen®.
- Primers for MMP-9: 5'- AATCTCTTCTAGAGACTGGGAAGG AG-3' (Forward) and 5'-AGCTGATTGACTAAAGTAGCTGGA-3' (Reverse) were obtained from Invitrogen®.
- Primers for α-SMA: 5'-CCGACCGAATGCAGAAGGA-3' (Forward) and 5'-ACAGAGTATTTGCGCTCCGAA -3' (Reverse) were obtained from Invitrogen®.
- Primers for GAPDH: 5'-GCAGTACAGCCCCAAAATGG-3' (Forward) and 5'-ACAAAGTCCGGCCTGTATCCAA-3' (Reverse) were obtained from Invitrogen®.
- All of the peptides were synthetized using conventional solution and/or solid phase peptide synthesis methods.
- All of the experiments were performed with a concentration of 400 ng/mL of GFR-binding compound(s) as defined herein.
- All cell culture experiments were performed with cultures on plastic coated with type-I collagen in order to mimic the physiological conditions of the human body.
- The compositions of pharmaceutical compositions used in the examples below are summarized in the following table:

| Compositions | Pharmaceutical associations according to certain embodiments of the present disclosure |
|---|---|
| Composition A | SEQ ID NO: 10 + RGD (peptide) |
| Composition B | SEQ ID NO: 10 + SiRNA Integrin alpha 3 + SiRNA Integrin beta 1 |
| Composition C | SEQ ID NO: 9 + integrin alpha 3 Antibody + integrin beta 1 Antibody |
| Composition D | SEQ ID NO: 3 + SiRNA Integrin alpha 3 + RGDSPC |
| Composition E | SEQ ID NO: 11 + RGD (peptide) |
| Composition F | SEQ ID NO: 14 + SiRNA Syndecan 1 + SiRNA Syndecan 4 + integrin alpha 3 antibody |
| Composition I | SEQ ID NO: 16 + E-Selectin antibody + L-Selectin antibody |

| | |
|---|---|
| Composition J | SEQ ID NO: 22 + RGD (peptide) |
| Composition K | SEQ ID NO: 24 + SiRNA Integrin alpha v + SiRNA Integrin beta 3 + SiRNA Integrin alpha 5 |
| Composition L | SEQ ID NO: 36 + RGDC (peptide) |
| Composition M | SEQ ID NO: 37 + RGDC + REDV (peptides) |

(continued)

| Compositions | Pharmaceutical associations according to certain embodiments of the present disclosure |
|---|---|
| Composition N | SEQ ID NO: 35 + integrin alpha 2 Antibody + integrin beta 1 Antibody |

- The two target sequences of human integrin α3 (Genbank accession number NM_002204) were designed to be unique relative to the sequences of other integrin members; catalogue number sc-35684 from Santa Cruz Biotechnology.
- The two target sequences of human integrin B1 (Genbank accession number NM_002211) were designed to be unique relative to the sequences of other integrin members; catalogue number sc-44310 from Santa Cruz Biotechnology.
- Integrin α3 Antibody is a goat polyclonal IgG, epitope mapping extracellular domain of Integrin α3 of human origin; catalogue number sc-28665 from Santa Cruz Biotechnology.
- Integrin β1 Antibody is a goat polyclonal IgG, epitope mapping extracellular domain of Integrin β1 of human origin; catalogue number sc-9936 from Santa Cruz Biotechnology.
- The two target sequences of human integrin a 3 (Genbank accession number NM_002204) were designed to be unique relative to the sequences of other integrin members; catalogue number sc-35684 from Santa Cruz Biotechnology.
- The two target sequences of human Syndecan 1 (Genbank accession number NM_002997) were designed to be unique relative to the sequences of other integrin members; catalogue number sc-36587 from Santa Cruz Biotechnology.
- The two target sequences of human Syndecan 4 (Genbank accession number NM_002999) were designed to be unique relative to the sequences of other integrin members; catalogue number sc-36588 from Santa Cruz Biotechnology.
- Integrin α3 Antibody is a goat polyclonal IgG, epitope mapping extracellular domain of Integrin α3 of human origin; catalogue number sc-28665 from Santa Cruz Biotechnology.
- E-Selectin Antibody is a goat polyclonal IgG, epitope mapping extracellular domain of E-Selectin of human origin; catalogue number sc-6937 from Santa Cruz Biotechnology.
- E-Selectin Antibody is a goat polyclonal IgG, epitope mapping extracellular domain of E-Selectin of human origin; catalogue number sc-6946 from Santa Cruz Biotechnology.
- The two target sequences of human integrin a v (Genbank accession number NM_002210) were designed to be unique relative to the sequences of other integrin members; catalogue number sc-29373 from Santa Cruz Biotechnology.
- The two target sequences of human integrin β3 (Genbank accession number NM_000212) were designed to be unique relative to the sequences of other integrin members; catalogue number sc-29375 from Santa Cruz Biotechnology.
- The two target sequences of human integrin α5 (Genbank accession number NM_002205) were designed to be unique relative to the sequences of other integrin members; catalogue number sc-29372 from Santa Cruz Biotechnology.
- Integrin α2 Antibody is a goat polyclonal IgG, epitope mapping extracellular domain of Integrin α2 of human origin; catalogue number sc-6586 from Santa Cruz Biotechnology.
- Integrin β3 Antibody is a goat polyclonal IgG, epitope mapping extracellular domain of Integrin β3 of human origin; catalogue number sc-9936 from Santa Cruz Biotechnology.

**The following general methods were used:**

- Immunostaining:

The cells were first fixed for 20 min with 4% paraformaldehyde/PBS at 4°C. After fixation, the cells were permeabilized in PBS containing 1% Triton X-100 for 15 min. Protein immunofluorescent visualization was performed by first treating the cells with 1% (v/v) specific monoclonal antibodies for 1 hour at 37 °C. Then the samples were incubated with Alexa fluor® 568 or 647 (F(ab')2 fragment of IgG(H + L)) during 30 min at room temperature. The cell nuclei were counterstained in 20 ng/mL DAPI for 10 min at room temperature.

- Quantification of positive contact numbers and areas:

For this type of quantification the freeware image analysis ImageJ® software was used. The raw image was first converted to an 8-bit file, and then the unsharp mask feature was used (settings 1:0.2) to remove the image background (rolling ball radius 10). After smoothing, the resulting image, which appears similar to the original photomicrograph but with minimal background, was then converted to a binary image by setting a threshold. Threshold values were determined empirically by selecting a setting, which gave the most accurate binary image for a subset of randomly selected photomicrographs. The cell area was determined by manual delineation on raw fluorescent images. Total contact area and mean contact area per cell were calculated by "analyse particules" in ImageJ®. A minimum of 50 cells per condition were analyzed.

- Quantitative Real Time Polymerase Chain Reaction (RT-PCR):

After 24 hours of culture, total RNA was extracted by using the RNeasy total RNA kit (Qiagen®) according to the manufacturer's instructions. Purified total RNA was used as a template in order to make cDNA by a reverse transcription reaction (Gibco Brl®) with random primers (Invitrogen®). The cDNA was then used as a template for a real-time PCR amplification in the presence of SYBR green reagents (BioRad®) by using a thermocycler (iCycler, Biorad®). Data were analyzed with the iCycler IQ™® software and compared by the ΔΔCt method. Briefly, the mean Ct value of the target gene was normalized to its averaged Ct values of the housekeeping gene (GAPDH) to give a ΔCt value, which was then normalized to a control sample to obtain a ΔΔCt value. The results were obtained from two series of experiments performed in triplicate.

- Western Blot:

After 24 hours of culture, cells were permeabilized (10% SDS, 25 mM NaCl, 10 nM pepstatin and 10 nM leupeptin in distilled water and loading buffer), boiled for 10 minutes and resolved by reducing PAGE (Invitrogen). Proteins were transferred onto nitrocellulose, blocked, and labeled with HRP-conjugated antibodies (Invitrogen). Integrin αv, α5, α3, α4, β1, β3, β5, β9, phosphorylated ERK, phosphorylated Src Kinase, PDK1 and phosphorylated pRB and p53 were blotted by treating the nitrocellulose with monoclonal anti-integrin (extracellular domain) αv, α5, α3, α4, β1, β3, β5 and β6 antibodies (Santa Cruz Biotechnology). Phospho-Smad1/5/8 was blotted by treating the nitrocellulose with monoclonal anti-p-Smad1/5/8 (Santa Cruz Biotechnology). For all of the experiments the western blot was performed in triplicate, along with an additional blot for actin and coomassie blue staining to ensure constant protein load among samples. An integrins α3, αv, α5 shRNAs, integrins β1, β3 shRNAs, Syndecans 1, 4 were obtained from Santa Cruz Biotechnology.

- Statistical Analysis:

In terms of real-time PCR assay, all of the data were expressed as mean ± standard error, and analyzed statistically by the paired Student's t test method.

- Covalent association with polymers (such as PET or PEEK)

The polymer used in this study is poly(ethylene terephthalate) (PET). The polymers were first treated in order to create -COOH (carboxyl) functions on the PET surfaces from -OH (hydroxyl) functions. Next, the PET-COOH samples were immersed in a solution of dimethylaminopropyl-3-ethylcarbodiimide hydrochloride (EDC, 0.2 M) + N-hydroxysuccinimide (NHS, 0.1 M) in 2-(N-morpholino)-ethanesulfonic acid (MES) buffer (0.1 M in MilliQ water) and the samples were rinsed in MilliQ water (50 mL for 30 min). Finally, the covalent immobilization of the compounds (e.g. peptides) was achieved by using a solution of compound/1X PBS (C = $10^{-3}$ M) incubated for 18 hours at room temperature. After grafting, the materials were rinsed in MilliQ water (100 ml) for 1 week.

**Example 1: Osteosarcoma recoding**

Pharmaceutical associations according to certain embodiments of the present disclosure were added to tumor cells, hereinafter osteosarcoma cells. Human osteosarcoma cells were obtained from ATCC®. Tumor cells were then cultured in Dulbecco's Modified Eagle Medium (DMEM, Invitrogen®) supplemented with 10% (v/v) fetal bovine serum (FBS), 1% penicillin/streptomycin and incubated in a humidified atmosphere containing 5% (v/v) $CO_2$ at 37°C. All of the cell culture experiments were performed without any serum in the medium for the first 8 hours of culture. All cells were used at a low passage number (≤ passage 10), were subconfluently cultured and were seeded at 10 000 cells/$cm^2$ for the purpose of the experiments.

The cell phenotype was analyzed after 24 hours of cell culture. The human sarcoma cells adhere, spread, and differentiate into osteocyte cells as confirmed by analysis of DMP-1 (Dentin matrix acidic phosphoprotein 1), Sclerostin and RANK-L (receptor activator of nuclear factor kappa B ligand) expression (FIG. 1). During cell recoding, osteosarcoma cells undergo important changes in their cell morphology such as cell shape modification, cell body size reduction and increase in cell processes in order to obtain the characteristic osteocyte morphology. These cells presented a characteristic dendritic-like morphology, a total reduction in cell body volume of around 50% and expressed a set of known osteocytic markers. These results demonstrate that pharmaceutical associations according to certain embodiments of the present disclosure induce the recoding of human osteosarcoma cells into osteocytes in a short period of time (24 hours).

**Example 2: Rhabdosarcoma recoding**

Pharmaceutical associations according to certain embodiments of the present disclosure were added to tumor cells, hereinafter rhabdomyosarcoma cells. Human rhabdosarcoma cells were obtained from ATCC®. Tumor cells were then cultured in Dulbecco's Modified Eagle Medium (DMEM, Invitrogen®) supplemented with 10% (v/v) fetal bovine serum (FBS), 1% penicillin/streptomycin and incubated in a humidified atmosphere containing 5% (v/v) $CO_2$ at 37°C. All cell culture experiments were performed without any serum in the medium for the first 8 hours of culture. All cells were used at a low passage number (≤ passage 10), were subconfluently cultured and were seeded at 10 000 cells/cm$^2$ for the purpose of the experiments.

It was observed that these tumor cells cultured in the presence of pharmaceutical associations according to certain embodiments of the present disclosure were converted into muscle cells (myocyte like-cells) by analyzing the expression of the myogenic biomarkers MLC-1 (myocyte light chain 1), GATA-4 and a-Sarcomeric actin. An increased expression of these genes was observed after 24 hours of culture (FIG. 2) as characterized using Quantitative Real Time PCR as described in the Methods section.

**Example 3: Mesenchymal chondrosarcoma recoding**

Pharmaceutical associations according to certain embodiments of the present disclosure were added to tumor cells, hereinafter chondrosarsoma cells. Human chondrosarcoma cells were obtained from ATCC®. Tumor cells were then cultured in Dulbecco's Modified Eagle Medium (DMEM, Invitrogen®) supplemented with 10% (v/v) fetal bovine serum (FBS), 1% penicillin/streptomycin and incubated in a humidified atmosphere containing 5% (v/v) $CO_2$ at 37°C. All cell culture experiments were performed without any serum in the medium for the first 8 hours of culture. All cells were used at a low passage number (≤ passage 10), were subconfluently cultured and were seeded at 10 000 cells/cm$^2$ for the purpose of the experiments.

It was observed that these tumor cells cultured in the presence of pharmaceutical associations according to certain embodiments of the present disclosure were converted into cartilage cells (chondrocyte like-cells) by analyzing the expression of the chondrocytic biomarkers Sox9, IBSP (Sialoprotein II) and collagen IV. An increased expression of these genes was observed after 24 hours of culture (FIG. 3) as characterized using Quantitative Real Time PCR as described in the Methods section.

**Example 4: adenocarcinoma recoding**

Pharmaceutical associations according to certain embodiments of the present disclosure were added to tumor cells, hereinafter adenocarcinoma cells. Human adenocarcinoma cells were obtained from ATCC®. Tumor cells were then cultured in RPMI-1640 medium supplemented with 10% (v/v) fetal bovine serum (FBS), 1% penicillin/streptomycin and incubated in a humidified atmosphere containing 5% (v/v) $CO_2$ at 37°C. All cell culture experiments were carried out without any serum in the medium for the first 8 hours of culture. All cells were used at a low passage number (≤ passage 10), were subconfluently cultured and were seeded at 10 000 cells/cm$^2$ for the purpose of the experiments.

It was observed that these tumor cells cultured in the presence of pharmaceutical associations according to certain embodiments of the present disclosure were converted into epithelial cells (fibrocyte like-cells) by analyzing the expression of the fibrocytic biomarkers MMP-9, Vimentin and $\alpha$-SMA. An increased expression of these genes was observed after 24 hours of culture (FIG. 4) as characterized using Quantitative Real Time PCR as described in the Methods section.

**Example 5: Recoding of neoplastic cells indicated by significantly reduced proliferation markers**

Neoplastic cells (in this example osteosarcoma cells) were cultured in the presence of pharmaceutical associations according to certain embodiments of the present disclosure. A rapid (24 hours) decrease of the tumor markers was observed (FIG. 5). The adhesion checkpoint then activates the tumor suppressor proteins p53 and pRB. It was observed that, in tumor cells cultured in the presence of pharmaceutical associations according to certain embodiments of the present disclosure, the expression of p53 decreases as demonstrated by quantification of protein expression level (immunofluorescence staining and quantification using ImageJ as described in the general methods section) (FIG. 5A) and the pRB is dephosphorylated as demonstrated by a quantification of protein phosphorylation level (immunofluorescence staining and subsequent quantification by using the ImageJ software as described in the general methods section) (FIG. 5B).

Cyclic GFR-binding peptides in association with SiRNA Integrin alpha 3 and SiRNA Integrin beta 1 were also tested against MG63 cells using the same procedure and displayed significant recoding activity as shown in Table 4 below:

**Table 4**

| SEQ ID N° | Sequences | P53 (%) | pRB (%) |
|---|---|---|---|
| SEQ ID NO: 38200 | VPTAASAISILYLDAANNVVLKNYQAAVVEDKGVVTY | 41 | 17 |
| SEQ ID NO: 38201 | VPEAASSLSMLFFEIVRKKPIFLKVYPAATVEDKGVVTY | 40 | 19 |
| SEQ ID NO: 38202 | VPTVVSAISTLYLRIKPHQGQHLKNYQVVVVENDKQQII | 42 | 9 |
| SEQ ID NO: 38203 | APTLLNAISMLYFNDKQQIILKKYRLLVVRNDKQQII | 44 | 12 |
| SEQ ID NO: 38204 | APTGGSATSMLYYDKGVVTYLRKHRGGVVKDKGVVTY | 37 | 11 |
| SEQ ID NO: 38205 | VPTSSSPISTLYIDKGVVTYLKYHYSSVAEDEYDKVV | 40 | 16 |
| SEQ ID NO: 38206 | VPTAAEPISMLYLDEYDKVVKFKYEAAAVSNDKQQII | 36 | 8 |
| SEQ ID NO: 38207 | TPTSSSPINTLYFDDMGVPTYGKIPSSVVDDEYDKVV | 41 | 13 |
| SEQ ID NO: 38208 | VPTVVSPISMLYIDDMGVPTYKQYEVVVVEDDMGVPT | 39 | 9 |
| SEQ ID NO: 38209 | APVAAKPLSMLYVDKGVVTYDHHKDAAVEEDEYDKVV | 38 | 11 |
| SEQ ID NO: 38210 | VPQAAEPLPMLYYDKGVVTYEQLSNAAVRSDKGVVTY | 40 | 12 |
| SEQ ID NO: 38211 | VPQQQEPLTTLYYDEYDKVVEQLSNQQVKSNDKQQII | 47 | 14 |
| SEQ ID NO: 38212 | VPTMMSNITTQIMRIKPHQGQHIGEMSMMQHNDEYDKVV | 51 | 14 |
| SEQ ID NO: 38213 | SRVAARSVKMAKVGGGGGGGKEVQVAAEEHGGGGGG | 49 | 21 |
| SEQ ID NO: 38214 | VPTDDSATSTLYYDKGVVTYLRKHRDDEHSDKGVVTY | 48 | 18 |
| SEQ ID NO: 38215 | VPTGGEPLTTLYYNDKQQIIEQLSNGGEHSDDMGVPT | 39 | 16 |
| SEQ ID NO: 38216 | SRVLLRPVQMRKIDKGVVTYKKATVLLEEHDKGVVTY | 41 | 11 |
| SEQ ID NO: 38217 | TQVAARPVQTRKIDDMGVPTKKATVAAEDHDKGVVTY | 42 | 9 |

Cyclic GFR-binding peptides in association with SiRNA Integrin alpha 3 + RGDSPC + SiRNA Integrin beta 1 + SiRNA Syndecan 1 were also tested against human rhabdosarcoma cells using the same procedure and displayed significant recoding activity as shown in Table 5 below:

**Table 5**

| SEQ ID N° | Sequences | P53 (%) | pRB (%) |
|---|---|---|---|
| SEQ ID NO: 38218 | IPKASSAPVGQRPVQMRKIDKGVVTYKKATVSSVEESGSLDKGVVTY | 36 | 12 |
| SEQ ID NO: 38219 | KASSVPQEERPVQMRKIGGGGGGGKKATVMIVRSSKSSDDMGVPT | 38 | 15 |
| SEQ ID NO: 38220 | VSQAARPVQTRKIDDMGVPTKKATVAAVKSDDMGVPT | 42 | 9 |
| SEQ ID NO: 38221 | PTSSAPTDLRSVKMAKVEIVRKKPIFKEVQVDMVVKASGDKGVVTY | 48 | 11 |
| SEQ ID NO: 38222 | KASSSRVKMSNITVQIMRIKPHQGQHIGEMSVVEEHASGSHDKGVVTY | 52 | 15 |
| SEQ ID NO: 38223 | VPTAAEPLTMLYYEIVRKKPIFEQLSNMVEHSDDMGVPT | 44 | 17 |
| SEQ ID NO: 38224 | EPSSSRVRLEPLTTLYYGGGGGGGEQLSNVVEEHSKDKGVVTY | 46 | 8 |
| SEQ ID NO: 38225 | APVKMEPLPTVYYDDMGVPTEQLSNDMVEESGSLDDMGVPT | 49 | 9 |

(continued)

| SEQ ID N° | Sequences | P53 (%) | pRB (%) |
|---|---|---|---|
| SEQ ID NO: 38226 | EPSSVSQAAEPLPIVYYRIKPHQGQHEQLSNMIVKSSKSSDDMGVPT | 29 | 12 |

**Example 6: Adhesion Checkpoint activation**

Neoplastic cells (in this example osteosarcoma cells) were cultured in the presence of pharmaceutical associations according to certain embodiments of the present disclosure. The main signaling pathways include: Ras/MAP kinase, FAK/Src kinase and PIP2 which are decreased after 24 hours as shown by western blot quantifications (FIG. 6). This demonstrated by the absence of phosphorylation at Thr202 and Tyr204 on ERK (FIG. 6A). Moreover, no phosphorylation at Thr416 but phosphorylation of Tyr527 was observed on Src kinase (FIG. 6B). Finally, a decrease of 3-phosphoinositide-dependent protein kinase 1 (PDK1) protein expression was also was observed (FIG. 6C).

**Example 7: Decrease of Paxillin and Vinculin gene expression**

Neoplastic cells (in this example osteosarcoma cells) were cultured in the presence of pharmaceutical associations according to certain embodiments of the present disclosure. After 24 hours, the Paxillin and Vinculin gene expression decreased as shown by RT-PCR (FIG. 7).

**Example 8: Cyclin D profile**

Neoplastic cells (in this example osteosarcoma cells) were cultured in the presence of pharmaceutical associations according to certain embodiments of the present disclosure. The Cyclin D (the arithmetic mean of Cyclin D1, Cyclin D2 and Cyclin D3) gene expression decreased after the first 30 min of cell culture as shown by RT-PCR (FIG. 8). These data indicate the entry of the cells into G0 quiescence phase.

**Example 9: Growth factor receptor activation**

The data summarized in Table 6 below demonstrate that the tumor cell recoding action ((in this example: human osteosarcoma cells, MG63, via quantification of pRB) of pharmaceutical associations according to certain embodiments of the present disclosure involves the activation of growth factor receptors as demonstrated by the activation of the Smad1/5/8 signaling pathway (via quantification of western blots of phospho-smad 1/5/8):

Table 6

| **Compositions** | **Pharmaceutical associations** | **p-Smad1/5/8 (%)** | **pRB** (%) |
|---|---|---|---|
| Composition R | SEQ ID NO: 54 + SiRNA Integrin alpha 3, v, 4 + SiRNA Integrin beta 1 | 41 | 12 |
| Composition V | SEQ ID NO: 55 + SiRNA Integrin alpha 3 + SiRNA Integrin beta 1 | 32 | 17 |
| Composition W | SEQ ID NO: 56 + integrin alpha 3 Antibody + integrin beta 1 Antibody + RGDSPC | 47 | 21 |
| Composition S | SEQ ID NO: 57 + SiRNA Integrin alpha 3 + RGDSPC + SiRNA Syndecan 1 | 38 | 9 |
| Composition Z | SEQ ID NO: 59 + E-Selectin antibody + L-Selectin antibody | 34 | 22 |
| Composition X | SEQ ID NO: 61 + SiRNA Syndecan 1 + SiRNA Syndecan 4 + integrin alpha 3 antibody | 44 | 18 |
| Composition Y | SEQ ID NO: 58 + RGD + SiRNA Integrin alpha 3, v, 4 + SiRNA Integrin beta 1 | 49 | 19 |

**Example 10: RMSD measurement and recoding activity**

The data summarized in Table 7 below demonstrate that pharmaceutical associations according to certain embodiments of the present disclosure containing GFR-binding compounds as disclosed herein having a RMSD value of 2.45Å or less, induce the particularly efficient recoding (as shown via quantification of pRB) of neoplastic cells (in this example: human osteosarcoma cells, MG63) into healthy cells:

Table 7

| SEQ ID N° | AA Sequences | RMSD (Å) | pRB (%) |
|---|---|---|---|
| SEQ ID NO: 54 | NDEGLECVPTEERPVQVRKIRIKPHQGQKKATVFLQHNK | 1,23 | 12 |
| SEQ ID NO: 55 | ASASPSSVPQDLEPLTILYYVGRTPKVEQLSNMV | 1,51 | 17 |
| SEQ ID NO: 56 | RNVQCRPTQLEPLPIVYYEIVRKKPEQLSNTLEDHLA | 1,7 | 21 |
| SEQ ID NO: 59 | NDEGLECVPTEERPVQVRKIRIKPHQGQKKATVFLQHNK | 1,1 | 22 |
| SEQ ID NO: 61 | RNVQCRPTQLEPLPIVYYEIVRKKPEQLSNTLEDHLA | 1,1 | 18 |
| SEQ ID NO: 58 | VSQDLSAISTLYLKIVRKEPLKNYQEMVEE | 0,79 | 19 |

Likewise, using the method detailed in the description, the data summarized in Table 8 below demonstrate that pharmaceutical associations according to certain embodiments of the present disclosure containing GFR-binding peptides as disclosed herein in association with SiRNA Integrin alpha 3, v, 4 and SiRNA Integrin beta 1, induce the particularly efficient recoding (as shown via quantification of pRB et P53) of neoplastic cells (in this example: human rhabdosarcoma cells) into healthy cells. It should also be noted that GFR-binding peptides having a RMSD value of 2.45Å or less, are particularly advantageous and efficient in recoding cells:

Table 8

| SEQ ID N° | Sequences | RMSD | P53 (%) | pRB (%) |
|---|---|---|---|---|
| SEQ ID NO: 123 | VPTAASAISILYLDAANNVVLKNYQAAVVE | 1,02 | 32 | 11 |
| SEQ ID NO: 124 | VPEAASSLSMLFFEIVRKKPIFLKVYPAATVE | 1,32 | 37 | 12 |
| SEQ ID NO: 125 | VPTVVSAISTLYLRIKPHQGQHLKNYQVVVVE | 1,56 | 42 | 9 |
| SEQ ID NO: 126 | APTLLNAISMLYFNDKQQIILKKYRLLVVR | 0,97 | 41 | 8 |
| SEQ ID NO: 127 | APTGGSATSMLYYDKGVVTYLRKHRGGVVK | 1,12 | 33 | 16 |
| SEQ ID NO: 128 | VPTSSSPISTLYIDKGVVTYLKYHYSSVAE | 1,52 | 36 | 21 |
| SEQ ID NO: 129 | VPTAAEPISMLYLDEYDKVVKFKYEAAAVS | 1,61 | 42 | 20 |
| SEQ ID NO: 130 | TPTSSSPINTLYFDDMGVPTYGKIPSSVVD | 1,31 | 48 | 15 |
| SEQ ID NO: 131 | VPTVVSPISMLYIDDMGVPTYKQYEVVVVE | 1,41 | 36 | 12 |
| SEQ ID NO: 132 | APVAAKPLSMLYVDKGVVTYDHHKDAAVEE | 0,98 | 38 | 9 |
| SEQ ID NO: 133 | VPQAAEPLPMLYYDKGVVTYEQLSNAAVRS | 0,95 | 42 | 7 |
| SEQ ID NO: 134 | VPQQQEPLTTLYYDEYDKVVEQLSNQQVKS | 1,16 | 50 | 8 |
| SEQ ID NO: 135 | VPTMMSNITTQIMRIKPHQGQHIGEMSMMQHN | 1,56 | 48 | 16 |
| SEQ ID NO: 136 | SRVAARSVKMAKVGGGGGGGKEVQVAAEEH | 2,08 | 38 | 10 |
| SEQ ID NO: 137 | VPTDDSATSTLYYDKGVVTYLRKHRDDEHS | 1,73 | 42 | 12 |
| SEQ ID NO: 138 | VPTGGEPLTTLYYNDKQQIIEQLSNGGEHS | 1,92 | 45 | 20 |
| SEQ ID NO: 139 | SRVLLRPVQMRKIDKGVVTYKKATVLLEEH | 1,85 | 49 | 18 |
| SEQ ID NO: 140 | TQVAARPVQTRKIDDMGVPTKKATVAAEDH | 1,93 | 35 | 17 |

Likewise, using the method detailed in the description, the data summarized in Table 9 below demonstrate that pharmaceutical associations according to certain embodiments of the present disclosure containing GFR-binding peptides as disclosed herein in association with:

- SiRNA Integrin alpha 3 + SiRNA Integrin beta 1, for SEQ ID NO: 141 to 163, on MG63 cells; and
- SiRNA Integrin alpha 3 + RGDSPC + SiRNA Integrin beta 1 + SiRNA Syndecan 1, for SEQ ID NO: 31, 32, and 164 to 187, on human rhabdosarcoma cells;

induce the particularly efficient recoding (as shown via quantification of pRB et P53) of neoplastic cells (in these examples: MG63 cells and human rhabdosarcoma cells as stated above) into healthy cells. It should also be noted that GFR-binding peptides having a RMSD value of 2.45Å or less, are particularly advantageous and efficient in recoding cells:

**Table 9**

| SEQ ID N° | Sequences | RMSD | P53 (%) | pRB (%) |
|---|---|---|---|---|
| SEQ ID NO: 141 | CKVPKPSSAPTQLSAISTLYLDEYDKVVLKNYQNMVVRA | 1,23 | 41 | 11 |
| SEQ ID NO: 142 | CKVGKASSVPTKLSAISTLYLDDMGVPTLKNYQNMVVRASGSH | 1,33 | 36 | 8 |
| SEQ ID NO: 143 | CASASPSSVSQDLSAISTLYLIGKTPKILKNYQMIVKSSKSS | 0,98 | 37 | 14 |
| SEQ ID NO: 144 | CKIPKASSVPTELNAISVLYFDENEKVILKKYRDMVVEGSG | 1,09 | 40 | 7 |
| SEQ ID NO: 145 | CASASPSSVSQDLNAISVLYFIGKTPKILKKYRMIVKSSKSS | 1,34 | 38 | 6 |
| SEQ ID NO: 146 | CAVPKASSAPTKLSATSVLYYDSSNNVVLRKHRNMVVK | 1,33 | 38 | 11 |
| SEQ ID NO: 147 | CTVPKPSSAPTQLSPISVLYKDDSSNVILKYHYNMVVRASGSH | 0,88 | 37 | 8 |
| SEQ ID NO: 148 | CAPTQLSPISVLYKDDSSNVILKYHYTLEDH | 1,93 | 42 | 9 |
| SEQ ID NO: 149 | CRNVQCRPTQVQLSAISTLYLEIVRKKPLKNYQTLEDH | 1,12 | 35 | 12 |
| SEQ ID NO: 150 | CPSSAPTQLSATSVLYYDDSSNVILRKHRQHNK | 1,33 | 41 | 11 |
| SEQ ID NO: 151 | CPKPSSAPTQLNAISMLYFGGGGGGGLKKYRNMVVRA | 2,16 | 39 | 9 |
| SEQ ID NO: 152 | CKVPKASSVPTELSAISTLYLAAAALKNYQEM | 2,21 | 37 | 8 |
| SEQ ID NO: 153 | CSIPKASSVPTELSATSVLYYDEYDEKVVLRKHEMVVEG | 1,43 | 39 | 10 |
| SEQ ID NO: 154 | CAPTKLSAISMLYLDSSNNVILKNYQ | 0,56 | 40 | 14 |
| SEQ ID NO: 155 | CASSAVSQELSAISMLYLGGGLKNYQDM | 2,01 | 47 | 6 |
| SEQ ID NO: 156 | CKASSVPTELSPISTLYKDENEKVVLKYHYDMVVEGSGSR | 1,03 | 45 | 8 |
| SEQ ID NO: 157 | CASAAPSSVPQKLNAISMLYFVGRKPKVLKKYRMIVRS | 1,04 | 34 | 11 |

(continued)

| SEQ ID N° | Sequences | RMSD | P53 (%) | pRB (%) |
|---|---|---|---|---|
| SEQ ID NO: 158 | CKVGKASSVPTKLNAISTLYLDENEKVVLKKYREGMS | 1,03 | 39 | 9 |
| SEQ ID NO: 159 | CPKPSSAPTQLSAISTLYLDDSSNVILKNYQMIVVRA | 1,41 | 44 | 9 |
| SEQ ID NO: 160 | CKVGKASSVPTKLNAISTLYLDDSSNVILKNYQ | 0,94 | 38 | 8 |
| SEQ ID NO: 161 | CGGGSSVPTELSAISTLYLDDYDKVILKKYRNM | 2,11 | 52 | 10 |
| SEQ ID NO: 162 | WWFWGKVPKPSSAPTQLSAISTLYLDEYDKVVLKNYQNMVVR | 0,87 | 39 | 8 |
| SEQ ID NO: 163 | GTPGPKVPKPSSAPTQLSAISTLYLDEYDKVVLKNYQNMAVR | 0,89 | 41 | 7 |
| SEQ ID NO: 164 | GTPGPPSSAPTQLSATSVLYYDDSSNVILRKHRMIVKS | 1,33 | 47 | 10 |
| SEQ ID NO: 31 | GTPGPAPTQLSPISVLYKDDSSNVILKYHY | 1,82 | 34 | 5 |
| SEQ ID NO: 32 | GTPGPASSAVSQELSAISMLYLGGGLKNYQDM | 2,3 | 46 | 7 |
| SEQ ID NO: 165 | GTPGPAPTKLSAISMLYLDSSNNVILRKHR | 1,44 | 42 | 9 |
| SEQ ID NO: 166 | GTPGPAPTKLSAISMLYLDSSNNVILKKYR | 1,67 | 37 | 10 |
| SEQ ID NO: 167 | GTPGPKASSVPTELSPISTLYKDENEKVVLKYHYDMVVEGSGSR | 1,17 | 41 | 11 |
| SEQ ID NO: 168 | GTPGPKASSVPTELSPISTLYKDENEKVVLKYHYDM | 1,65 | 51 | 13 |
| SEQ ID NO: 169 | GTPGPPKPSSAPTQLNAISMLYFGGGGGLKKYRDMQHN | 2,36 | 37 | 8 |
| SEQ ID NO: 170 | WWFWGKASSVPTELSPISTLYKDENEKVVLKYHYDMVVEGSGS R | 1,18 | 44 | 7 |
| SEQ ID NO: 171 | WWFWGASSAVSQELSAISMLYLGGGLKNYQDMQHN | 1,97 | 47 | 8 |
| SEQ ID NO: 172 | GTPGPRNVQCRPTQVQLSAISTLYLEIVRKKPLKNYQTLEDH | 1,27 | 33 | 12 |
| SEQ ID NO: 173 | GTPGPRNVQCRPTQVQLSAISTLYLAAALKNYQTLE | 2,06 | 37 | 15 |
| SEQ ID NO: 174 | CKIPKASSVPTELEPLPIVYYVGRKPKVEQLSNMIVRS | 1,84 | 40 | 6 |
| SEQ ID NO: 175 | CASAAPSSVPQALEPLPIVYYVGRTPKVEQLSNMIVRSSK | 1,54 | 45 | 7 |
| SEQ ID NO: 176 | CVPTEERSVKVAKVRIKPHQGQHKEVQV | 1,8 | 33 | 7 |
| SEQ ID NO: 177 | CVPTEESNITMQIMRIKPHQGQHIGEMSFL | 0,79 | 43 | 8 |

(continued)

| SEQ ID N° | Sequences | RMSD | P53 (%) | pRB (%) |
|---|---|---|---|---|
| SEQ ID NO: 178 | CRNVQCRPTQVQLRPVQVRKIEYVRKKPKLKKATVRLEDHLA | 0,88 | 42 | 6 |
| SEQ ID NO: 179 | CGIPEPSSVPEKMSSLSILFFDENKNVVLKVYPNM | 1,08 | 40 | 11 |
| SEQ ID NO: 180 | CGIPEPSSVPEKTSSLSLLFFDENKNVVLKVYPNM | 1,04 | 29 | 9 |
| SEQ ID NO: 181 | CAAPASSSVPARLSPISILYIDAANNVVYKQYEDMVVEASG | 1,11 | 45 | 9 |
| SEQ ID NO: 182 | CAAPASSSVPARLSPISILYIDAANNVVYKQYEDMVVE | 1,47 | 41 | 8 |
| SEQ ID NO: 183 | CAAPASSSVPARLSPISLLYIDAANNVVYKQYEDM | 1,45 | 44 | 13 |
| SEQ ID NO: 184 | CPARLSPISLLYIDAANNVVYKQYEDM | 1,82 | 39 | 11 |
| SEQ ID NO: 185 | CAAAPASSSVPARLSPISILYIAAAYKQYEDM | 1,77 | 47 | 8 |
| SEQ ID NO: 186 | CVPQRLSPISILYIDAANNVVYKQYE | 0,95 | 38 | 12 |
| SEQ ID NO: 187 | CTVPKPSSAPTQLSPISILYIDAANNVVYKQYEDMVVRA | 0,74 | 42 | 9 |

**Example 11: The presence of adhesion components in the extracellular microenvironment inhibits the cell conversion**

In order to demonstrate the importance of adhesion proteins inhibition in the recoding process of a neoplastic cell, a PET substrate was covalently functionalized with RGD peptides (the primary integrin recognition site present in many ECM proteins (e.g., fibronectin)) and an exemplary GFR-binding compound according to the present disclosure using methods already described herein.

This modified substrate was then cultured with human osteosarcoma cells from ATCC® in Dulbecco's Modified Eagle Medium (DMEM, Invitrogen®) supplemented with 10% (v/v) fetal bovine serum (FBS), 1% penicillin/streptomycin and incubated in a humidified atmosphere containing 5% (v/v) $CO_2$ at 37°C. All cell culture experiments were performed without any serum in the medium for the first 8 hours of culture. All cells were used at a low passage number (≤ passage 10), were subconfluently cultured and were seeded at 10 000 cells/$cm^2$ for the purpose of the experiments. As shown in FIG. 9A and 9B, the presence of covalently immobilized components favorising the adhesion inhibited the process of recoding of the osteosarcoma cells into osteocytes as characterized by RT-PCR and cell morphology observation.

It was also confirmed that the presence of RGD peptides did not modify the density of covently grafted GFR-binding compounds. Indeed, it was observed that the GFR-binding compound density was stable (around 1.36 pmol/$mm^2$) with or without RGD peptides (FIG. 9C).

It was also shown that the RGD peptides interacted specifically with integrin $\alpha3\beta1$. Indeed, inhibition of $\alpha3\beta1$, $\alpha5\beta3$ and $\alpha v\beta3$ integrins (i.e., the three predominant receptor dimers involved in binding ECM molecules containing RGD motifs for the osteosarcoma cells) by specific antibodies, indicated that cells predominantly engaged $\alpha3\beta1$ integrins to interact with the RGD-modified substrate on the basis of the observed decrease of osteosarcoma cells adhesion in response to anti- $\alpha3\beta1$ treatment (Fig. 9D). This was not observed when $\alpha v\beta3$ and $\alpha5\beta3$ were blocked (Fig. 9E). For these antibody inhibition studies, cells were preincubated with 5g/mL anti-$\alpha3\beta1$ (clone JBS5), anti-$\alpha v\beta1$ (clone LM609) and anti-$\alpha5\beta3$.

**Example 12: Integrin engagement inhibits cell conversion.**

Using the same setup as in Example 11 (tumor cell and PET functionalized with RGD peptides and an exemplary

GFR-binding compound), silencing of integrin a 3 and integrin $\beta$1 expression using shRNA was shown to reduce integrin $\alpha$3 and integrin $\beta$1 mRNA levels by 60-70% and 50-60% respectively and integrin $\alpha$3 and integrin $\beta$1 proteins levels by 60-70% after 24h. It was then shown that both integrins $\alpha$3 and $\beta$1 shRNAs modulated osteosarcoma adhesion and spreading on RGD-modified substrates (Fig. 10 A, B).

Without being bound to any specific theory, integrin $\alpha3\beta1$ thus appears to be an important factor in the conservation of osteosarcomas tumourous profile and appears to play an important role in the recoding of osteosarcomas into osteocytes. Herein, $\alpha3\beta1$ integrin engagement appears to be involved in the inhibition of osteocytic recoding but not in osteosarcoma induction. Indeed, it was observed, within the framework of this experiment, that spheroid-like structures of osteosarcomas became progressively predominant (Fig. 10C). These results seems to demonstrate that integrin engagement has the ability to influence the behavior of human osteosarcoma towards spheroid-like structures or towards osteocytes.

Items of the present disclosure

Item 1. A pharmaceutical association comprising at least one growth factor receptor-binding compound, which activates at least one growth factor receptor of a neoplastic cell, and at least one adhesion protein inhibitor which inhibits at least one transmembrane cell adhesion protein of said neoplastic cell.

Item 2. A pharmaceutical association according to Item 1, wherein said association reduces or suppresses, in the neoplastic cell, the gene expression of at least one cyclin D and/or reduces or suppresses the formation of at least one complex formed between said at least one cyclin D and at least one of cyclin dependent-kinase 4 or 6.

Item 3. A pharmaceutical association according to Item 1 or 2, wherein said growth factor receptor-binding compound is a non-cyclic growth factor receptor-binding compound and has a molecular weight of less than 8,000 Daltons.

Item 4. A pharmaceutical association according to Item 1 or 2, wherein said growth factor receptor-binding compound is a cyclic growth factor receptor-binding compound and has a molecular weight of less than 7,000 Daltons.

Item 5. A pharmaceutical association for a use according to any one of Items 1 to 4, wherein said growth factor receptor-binding compound comprises a peptide with four amino acids PEP1, and a peptide with five amino acids PEP2; wherein PEP1 is selected from the group consisting of SAIS, SSLS, NAIS, SATS, SPIS, EPIS, SPIN, KPLS, EPLP, EPLT, SNIT, RSVK and RPVQ ; and wherein PEP2 is selected from the group consisting of LKNYQ, LKVYP, LKKYR, LRKHR, LKYHY, KFKYE, YGKIP, YKQYE, DHHKD, EQLSN, IGEMS, LGEMS, KEVQV and KKATV.

Item 6. A pharmaceutical association according to any one of Items 1 to 5, wherein said growth factor receptor is selected from the group consisting of epidermal growth factor receptors, fibroblast growth factor receptors, vascular endothelial growth factor receptors, nerve growth factor receptors, insulin receptor family, Trk receptor family, Eph receptor family, AXL receptor family, LTK receptor family, TIE receptor family, ROR receptor family, DDR receptor family, RET receptor family, KLG receptor family, RYK receptor family, MuSK receptor family, hepatocyte growth factor receptors, somatomedin or insulin-like growth factor receptors, platelet-derived growth factor receptors, and transforming growth factor beta superfamily proteins.

Item 7. A pharmaceutical association according to any one of Items 1 to 6, wherein said adhesion inhibitor is selected from the group consisting of an integrin inhibitor which inhibits at least one integrin, a syndecan inhibitor which inhibits at least one syndecan, a selectin inhibitor which inhibits at least one selectin, a dystroglycan inhibitor which inhibits at least one dystroglycan, and any combinations thereof.

Item 8. A pharmaceutical association according to any one of Items 1 to 7, wherein the gene expression of cyclin D is reduced or suppressed during phase G1 of the cell cycle of the neoplastic cell.

Item 9. A pharmaceutical association according to Item 8, wherein the gene expression of cyclin D is reduced or suppressed during the entire duration of phase G1 of the cell cycle of the neoplastic cell.

Item 10. A pharmaceutical association according to any one of Items 1 to 9, wherein said treatment restores a function of at least one impaired cell cycle checkpoint.

Item 11. A pharmaceutical association according to Item 10, wherein said cell cycle checkpoint is a cell adhesion checkpoint.

Item 12. A pharmaceutical association according to any one of Items 1 to 11, wherein said treatment comprises down-regulating the expression or activity of a GTPase.

Item 13. A pharmaceutical association according to any one of Items 1 to 12, wherein said treatment is an extracellular treatment.

Item 14. A pharmaceutical association according to any one of Items 1 to 13, wherein said treatment is a non-mutagenic treatment.

Item 15. A pharmaceutical association according to any one of Items 1 to 14, wherein said treatment converts a neoplastic cell into a non-neoplastic cell.

Item 16. A pharmaceutical association according to any one of Items 1 to 15, wherein said treatment induces the differentiation of said neoplastic cell.

Item 17. A pharmaceutical association according to any one of Items 1 to 16, wherein said treatment induces the quiescence of said neoplastic cell.

Item 18. A pharmaceutical association according to any one of Items 1 to 17, wherein said treatment inhibits cell division and/or cell proliferation of said neoplastic cell.

Item 19. A pharmaceutical association according to any one of Items 1 to 18, wherein said treatment promotes anti-mitogen activity, tumour suppressor activity, anti-oncogenic activity or any combination thereof in said neoplastic cell.

Item 20. A pharmaceutical association according to any one of Items 1 to 19, wherein said growth factor receptor-binding compound comprises a peptide with eight amino acids PEP12, and a peptide with five amino acids PEP2 ; wherein PEP12 is a peptide of general formula PEP1-$AA^{17}$-PEP11; wherein $AA^{17}$ is selected from the group consisting of G, A, V, L, I, P, F, M, W, T and S ; wherein PEP11 is a peptide with 3 amino acids of general formula $AA^{18}$-$AA^{19}$-$AA^{20}$; wherein $AA^{18}$ is selected from the group consisting of L, V, Q, A and R; wherein $AA^{19}$ is selected from the group consisting of F, W, H, Y, I and K; wherein $AA^{20}$ is selected from the group consisting of L, F, Y, K, I, V and M.

Item 21. A pharmaceutical association according to Item 20, wherein PEP11 is selected from the group consisting of LYL, LFF, LYF, LYY, LYK, LYI, LFI, LYV, VYY, QIM, AKV and RKI.

Item 22. A pharmaceutical association according to any one of Items 5 to 21, wherein said growth factor receptor-binding compound comprises a peptide with three amino acids PEP3 and a peptide with three amino acids PEP4 ; wherein PEP3 is selected from the group consisting of VPT, VPE, APT, TPT, VPA, APV, VPQ, VSQ, SRV and TQV ; and wherein PEP4 is selected from the group consisting of VVE, TVE, VVR, VVK, VAE, AVS, VVD, VEE, VRS, VKS, QHN, EHS, EEH and EDH.

Item 23. A pharmaceutical association according to any one of Items 5 to 22, wherein said growth factor receptor-binding compound comprises a peptide with five amino acids PEP5 and a peptide with between five and seven amino acids PEP6; wherein PEP5 is a peptide of general formula PEP3-$AA^{11}$-$AA^{12}$; wherein PEP3 is selected from the group consisting of VPT, VPE, APT, TPT, VPA, APV, VPQ, VSQ, SRV and TQV; wherein $AA^{11}$ is selected from the group consisting of E, K, Q, R, A, D, G and H; and wherein $AA^{12}$ is selected from the group consisting of L, M, T, E, Q and H ; wherein PEP6 is a peptide of general formula $AA^{26}$-$AA^{27}$-$AA^{28}$-$AA^{29}$-PEP4; wherein PEP4 is selected from the group consisting of VVE, TVE, VVR, VVK, VAE, AVS, VVD, VEE, VRS, VKS, QHN, EHS, EEH and EDH; wherein $AA^{26}$ is absent or selected from the group consisting of $AA^{III}$ amino acids, preferably is absent or is E; wherein $AA^{27}$ and $AA^{28}$ are independently selected from the group consisting of $AA^{III}$ and $AA^{V}$ amino acids; and wherein $AA^{29}$ is absent or selected from the group consisting of $AA^{II}$ amino acids, preferably is absent or is S.

Item 24. A pharmaceutical association according to Item 23, wherein PEP5 is selected from the group consisting of VPTEL, VPEKM, APTKL, APTQL, VPTKL, TPTKM, VPARL, VPTRL, APVKT, VPQAL, VSQDL, VPQDL, VPTEE, VPTGQ, SRVHH and TQVQL.

Item 25. A pharmaceutical association according to Item 23 or 24, wherein PEP6 is selected from the group consisting of DMVVE, NMTVE, EMVVE, NMVVR, NMVVK, EGMSVAE, GMAVS, GMVVD, MIVEE, MIVRS, MIVKS, MWKS, FLQHN, LEEHS, RLEEH and TLEDH.

Item 26. A pharmaceutical association according to any one of Items 5 to 25, wherein said growth factor receptor-binding compound comprises a peptide with between six and twelve amino acids PEP9 and a peptide with between six and eleven amino acids PEP10 ; wherein PEP9 is a peptide of general formula PEP7-PEP5; wherein PEP5 is a peptide of formula PEP3-$AA^{11}$-$AA^{12}$; wherein PEP3 is selected from the group consisting of VPT, VPE, APT, TPT, VPA, APV, VPQ, VSQ, SRV and TQV; wherein $AA^{11}$ is selected from the group consisting of E, K, Q, R, A, D, G and H; and wherein $AA^{12}$ is selected from the group consisting of L, M, T, E, Q and H; wherein PEP7 is an amino acid or a peptide with between two and seven amino acids of general formula $AA^{1}$-$AA^{2}$-$AA^{3}$-$AA^{4}$-$AA^{5}$-$AA^{6}$-$AA^{7}$; wherein $AA^{1}$, $AA^{2}$, $AA^{3}$, $AA^{4}$, and $AA^{5}$ are independently absent or $AA^{I}$ as defined herein; wherein $AA^{6}$ is absent or selected from the group consisting of S, T, C, E, Q, P and R; wherein $AA^{7}$ is absent or is selected from the group consisting of S, T, C, E, Q, P and R ; wherein PEP10 is a peptide of general formula PEP6-PEP8; wherein PEP6 is a peptide of formula $AA^{26}$-$AA^{27}$-$AA^{28}$-$AA^{29}$-PEP4; wherein PEP4 is selected from the group consisting of WE, TVE, WR, VVK, VAE, AVS, VVD, VEE, VRS, VKS, QHN, EHS, EEH and EDH; wherein $AA^{26}$ is absent or selected from the group consisting of $AA^{III}$ amino acids, preferably is absent or is E; wherein $AA^{27}$ and $AA^{28}$ are independently selected from the group consisting of $AA^{III}$ and $AA^{V}$ amino acids; and wherein $AA^{29}$ is absent or selected from the group consisting of $AA^{II}$ amino acids, preferably is absent or is S; wherein PEP8 is an amino acid or a peptide with between two and six amino acids of general formula $AA^{33}$-$AA^{34}$-$AA^{35}$-$AA^{36}$-$AA^{37}$-$AA^{38}$; $AA^{33}$ is absent or is selected from the group consisting of $AA^{I}$ amino acids at the exception of $AA^{VI}$ amino acids; $AA^{34}$ is absent or is selected from the group consisting of $AA^{III}$ and $AA^{IV}$ amino acids; wherein $AA^{35}$ is absent or is selected from the group consisting of $AA^{II}$ amino acids, preferably is S or C; wherein $AA^{36}$ is absent or is selected from the group consisting of $AA^{III}$ and $AA^{IV}$ amino acids; wherein $AA^{37}$ is absent or is selected from the group consisting of $AA^{II}$ amino acids,

preferably is S or C; wherein $AA^{38}$ is absent or is selected from the group consisting of $AA^{I}$.

Item 27. A pharmaceutical association according to Item 26, wherein PEP9 is selected from the group consisting of KIPKAXXVPTEL, GIPEPXXVPEKM, SIPKAXXVPTEL, HVTKPTXAPTKL, YVPKPXXAPTKL, TVPKPXXAPTQL, AVPKAXXAPTKL, KVGKAXXVPTKL, KASKAXXVPTKL, GSAGPXXTPTKM, AAPASXXVPARL, STPPTXXVPTRL, HVPKPXXAPTKL, RVPSTXXAPVKT, ASAAPXXVPQAL, ASASPXXVSQDL, ASASPXXVPQDL, NDEGLEXVPTEE, NDEGLEXVPTGQ, SSVKXQPSRVHH and RNVQXRPTQVQL; and wherein X is C or S.

Item 28. A pharmaceutical association according to Item 26 or 27, wherein PEP10 is selected from the group consisting of DMVVEGXGXR, NMTVESXAXR, EMVVEGXGXR, NMWRSXGXH, NMVVRAXGXH, NMVVKAXGXH, EGMSVAEXGXR, GMAVSEXGXR, GMVVDRXGXS, DMVVEAXGXR, DMVVESXGXR, MIVEEXGXL, MIVRSXKXS, MIVKSXKXS, MVVKSXKXS, FLQHNKXEXR, LEEHSQXEXR, RLEEHLEXAXA and TLEDHLAXKXE; and wherein X is C or S.

Item 29. A pharmaceutical association according to any one of Items 5 to 28, wherein said growth factor receptor-binding compound comprises a peptide with three amino acids PEP3 and a peptide with between five and seven amino acids PEP6.

Item 30. A pharmaceutical association according to any one of Items 5 to 28, wherein said growth factor receptor-binding compound comprises a peptide with three amino acids PEP3 and a peptide with between six and eleven amino acids PEP10.

Item 31. A pharmaceutical association according to any one of Items 5 to 28, wherein said growth factor receptor-binding compound comprises a peptide with five amino acids PEP5 and a peptide with three amino acids PEP4.

Item 32. A pharmaceutical association according to any one of Items 5 to 28, wherein said growth factor receptor-binding compound comprises a peptide with between six and twelve amino acids PEP9 and a peptide with three amino acids PEP4.

Item 33. A pharmaceutical association according to any one of Items 5 to 28, wherein said growth factor receptor-binding compound comprises a peptide with five amino acids PEP5 and a peptide with between six and eleven amino acids PEP10.

Item 34. A pharmaceutical association according to any one of Items 5 to 28, wherein said growth factor receptor-binding compound comprises a peptide with between six and twelve amino acids PEP9 and a peptide with between five and seven amino acids PEP6.

Item 35. A pharmaceutical association according to any one of Items 5 to 28, wherein said growth factor receptor-binding compound comprises a peptide with three amino acids PEP3, an amino acid or a peptide with between two and seven amino acids PEP7, and a peptide with three amino acids PEP4; wherein PEP7 is an amino acid or a peptide with between two and seven amino acids of general formula $AA^{1}$-$AA^{2}$-$AA^{3}$-$AA^{4}$-$AA^{5}$-$AA^{6}$-$AA^{7}$; wherein $AA^{1}$, $AA^{2}$, $AA^{3}$, $AA^{4}$, and $AA^{5}$ are independently absent or $AA^{I}$; wherein $AA^{6}$ is absent or selected from the group consisting of S, T, C, E, Q, P and R; wherein $AA^{7}$ is absent or is selected from the group consisting of S, T, C, E, Q, P and R; wherein at least one of $AA^{1}$, $AA^{2}$, $AA^{3}$, $AA^{4}$, $AA^{5}$, $AA^{6}$ or $AA^{7}$ is not absent; wherein PEP8 is an amino acid or a peptide with between two and six amino acids of general formula $AA^{33}$-$AA^{34}$-$AA^{35}$-$AA^{36}$-$AA^{37}$-$AA^{38}$; wherein $AA^{33}$ is absent or is selected from the group consisting of $AA^{I}$ amino acids at the exception of $AA^{VI}$ amino acids; $AA^{34}$ is absent or is selected from the group consisting of $AA^{III}$ and $AA^{IV}$ amino acids; wherein $AA^{35}$ is absent or is selected from the group consisting of $AA^{II}$ amino acids, preferably is S or C; wherein $AA^{36}$ is absent or is selected from the group consisting of $AA^{III}$ and $AA^{IV}$ amino acids; wherein $AA^{37}$ is absent or is selected from the group consisting of $AA^{II}$ amino acids, preferably is S or C; wherein $AA^{38}$ is absent or is selected from the group consisting of $AA^{I}$; and wherein at least one of $AA^{33}$, $AA^{34}$, $AA^{35}$, $AA^{36}$, $AA^{37}$ or $AA^{38}$ is not absent; and wherein X is C or S.

Item 36. A pharmaceutical association according to Item 35, wherein PEP7 is selected from the group consisting of KIPKAXX, GIPEPXX, SIPKAXX, HVTKPTX, YVPKPXX, TVPKPXX, AVPKAXX, KVGKAXX, KASKAXX, GSAGPXX, AAPASXX, STPPTXX, HVPKPXX, RVPSTXX, ASAAPXX, ASASPXX, NDEGLEX, SSVKXQP and RNVQXRP; and wherein X is C or S.

Item 37. A pharmaceutical association according to Item 35 or 36, wherein PEP8 is selected from the group consisting of GXGXR, SXAXR, SXGXH, AXGXH, XGXR, EXGXR, RXGXS, AXGXR, SXGXR, XGXL, XKXS, KXEXR, QXEXR, LEXAXA and LAXKXE; and wherein X is C or S.

Item 38. A pharmaceutical association according to any one of Items 5 to 37, wherein said growth factor receptor-binding compound comprises a peptide with three amino acids PEP3, an amino acid or a peptide with between two and seven amino acids PEP7, and a peptide with between five and seven amino acids PEP6.

Item 39. A pharmaceutical association according to any one of Items 5 to 37, wherein said growth factor receptor-binding compound comprises a peptide with three amino acids PEP3, an amino acid or a peptide with between two and seven amino acids PEP7, and a peptide with between six and eleven amino acids PEP10.

Item 40. A pharmaceutical association according to any one of Items 5 to 37, wherein said growth factor receptor-binding compound comprises a peptide with three amino acids PEP3, an amino acid or a peptide with between two and seven amino acids PEP7, a peptide with three amino acids PEP4, and an amino acid or a peptide with between

two and six amino acids PEP8.

Item 41. A pharmaceutical association according to any one of Items 5 to 37, wherein said growth factor receptor-binding compound comprises a peptide with three amino acids PEP3, an amino acid or a peptide with between two and seven amino acids PEP7, a peptide with between five and seven amino acids PEP6, and an amino acid or a peptide with between two and six amino acids PEP8.

Item 42. A pharmaceutical association according to any one of Items 5 to 37, wherein said growth factor receptor-binding compound comprises a peptide with five amino acids PEP5, an amino acid or a peptide with between two and seven amino acids PEP7, and a peptide with three amino acids PEP4.

Item 43. A pharmaceutical association according to any one of Items 5 to 37, wherein said growth factor receptor-binding compound comprises a peptide with five amino acids PEP5, an amino acid or a peptide with between two and seven amino acids PEP7, and a peptide with between five and seven amino acids PEP6.

Item 44. A pharmaceutical association according to any one of Items 5 to 37, wherein said growth factor receptor-binding compound comprises a peptide with five amino acids PEP5, an amino acid or a peptide with between two and seven amino acids PEP7, and a peptide with between six and eleven amino acids PEP10.

Item 45. A pharmaceutical association according to any one of Items 5 to 37, wherein said growth factor receptor-binding compound comprises a peptide with five amino acids PEP5, an amino acid or a peptide with between two and seven amino acids PEP7, a peptide with three amino acids PEP4, and an amino acid or a peptide with between two and six amino acids PEP8.

Item 46. A pharmaceutical association according to any one of Items 5 to 37, wherein said growth factor receptor-binding compound comprises a peptide with five amino acids PEP5, an amino acid or a peptide with between two and seven amino acids PEP7, a peptide with between five and seven amino acids PEP6, and an amino acid or a peptide with between two and six amino acids PEP8.

Item 47. A pharmaceutical association according to any one of Items 5 to 37, wherein said growth factor receptor-binding compound comprises a peptide with between six and twelve amino acids PEP9, a peptide with three amino acids PEP4, and an amino acid or a peptide with between two and six amino acids PEP8.

Item 48. A pharmaceutical association according to any one of Items 5 to 37, wherein said growth factor receptor-binding compound comprises a peptide with between six and twelve amino acids PEP9, a peptide with between five and seven amino acids PEP6, and an amino acid or a peptide with between two and six amino acids PEP8.

Item 49. A pharmaceutical association according to any one of Items 1 to 48, wherein said growth factor receptor-binding compound comprises a peptide, a variant or analog thereof, or a peptidomimetic having the following general formula (Ia):

PEP(A)-LINKER-PEP(B) (Ia)

wherein one end of LINKER interacts covalently with one end of PEP(A); wherein another end of LINKER interacts covalently with one end of PEP(B); wherein PEP(A) comprises PEP1 or PEP12 and at least one amino acid or peptide selected from the group consisiting of PEP3, PEP5, PEP7 and PEP9; wherein PEP(B) comprises PEP2 and at least one amino acid or peptide selected from the group consisting of PEP4, PEP6, PEP8 and PEP10; wherein LINKER is a linear or branched organic divalent radical, moiety or compound having a molecular weight comprised between about 28 and about 2,000 Da.

Item 50. A pharmaceutical association according to any one of Items 1 to 3 and 5 to 48, wherein said growth factor receptor-binding compound has the following general formula (Ia):

PEP(A)-LINKER-PEP(B) (Ia)

wherein one end of LINKER interacts covalently with one end of PEP(A); wherein another end of LINKER interacts covalently with one end of PEP(B); wherein PEP(A) comprises at least one amino acid or peptide selected from the group consisiting of PEP1, PEP12, PEP3, PEP5, PEP7 and PEP9; wherein PEP(B) comprises at least one amino acid or peptide selected from the group consisting of PEP2, PEP4, PEP6, PEP8 and PEP10; wherein LINKER is a linear or branched organic divalent radical, moiety or compound having a molecular weight comprised between about 28 and about 2,000 Da.

Item 51. A pharmaceutical association according to any one of Items 1 to 48, wherein said growth factor receptor-binding compound comprises a peptide, a variant or analog thereof, or a peptidomimetic having the following general formula (IIa):

PEP(C)-PEP12-LINKER-PEP2-PEP(D) (IIa)

wherein LINKER is a linear or branched organic divalent radical, moiety or compound having a molecular weight

(Mw) comprised between about 28 and about 2,000 Da; wherein one end of PEP(C) interacts covalently with PEP12; wherein one end of PEP(D) interacts covalently with one end of PEP2; wherein one end of LINKER interacts covalently with one end of PEP12; wherein another end of LINKER interacts covalently with another end of PEP2; wherein PEP(C) is a peptide with at least 5 amino acids comprising at least one peptide selected from the group consisting of PEP3, PEP5, PEP7 and PEP9; and wherein PEP(D) is a peptide with at least 5 amino acids comprising at least one peptide selected from the group consisting of PEP4, PEP6, PEP8 and PEP10.

Item 52. A pharmaceutical association according to any one of Items 1 to 3 and 5 to 48, wherein said growth factor receptor-binding compound has the following general formula (IIa):

PEP(C)-PEP12-LINKER-PEP2-PEP(D) (IIa)

wherein LINKER is a linear or branched organic divalent radical, moiety or compound having a molecular weight (Mw) comprised between about 28 and about 2,000 Da; wherein one end of PEP(C) interacts covalently with PEP12; wherein one end of PEP(D) interacts covalently with one end of PEP2; wherein one end of LINKER interacts covalently with one end of PEP12; wherein another end of LINKER interacts covalently with another end of PEP2; wherein PEP(C) is a peptide with at least 5 amino acids comprising at least one amino acid or peptide selected from the group consisting of PEP3, PEP5, PEP7 and PEP9; and wherein PEP(D) is a peptide with at least 5 amino acids comprising at least one amino acid or peptide selected from the group consisting of PEP4, PEP6, PEP8 and PEP10.

Item 53. A pharmaceutical association according to Item 37, wherein PEP(C) is PEP5 and PEP(D) is PEP6.

Item 54. A pharmaceutical association according to Item 43, wherein PEP(C) is PEP9; and PEP(D) is PEP10.

Item 55. A pharmaceutical association according to Item 43, wherein PEP(C) comprises PEP3 and PEP(D) is PEP6.

Item 56. A pharmaceutical association according to Item 43, wherein PEP(C) comprises PEP3 and PEP(D) is PEP10.

Item 57. A pharmaceutical association according to Item 43, wherein PEP(C) is PEP5 and PEP(D) comprises PEP4.

Item 58. A pharmaceutical association according to Item 43, wherein PEP(C) is PEP9 and PEP(D) comprises PEP4.

Item 59. A pharmaceutical association according to Item 43, wherein PEP(C) is PEP5 and PEP(D) is PEP10.

Item 60. A pharmaceutical association according to Item 43, wherein PEP(C) is PEP9 and PEP(D) is PEP6.

Item 61. A pharmaceutical association according to Item 43, wherein PEP(C) comprises PEP3 and PEP7, and PEP(D) is PEP6.

Item 62. A pharmaceutical association according to Item 43, wherein PEP(C) comprises PEP3 and PEP7, and PEP(D) is PEP10.

Item 63. A pharmaceutical association according to any one of Items 1 to 48, wherein said growth factor receptor-binding compound comprises a peptide, a variant or analog thereof, or a peptidomimetic having the following general formula (IIIa):

PEP7-PEP5-PEP12-LINKER-PEP2-PEP6-PEP8 (IIIa)

wherein LINKER is a linear or branched organic divalent radical having a molecular weight comprised between about 28 and about 2,000 Da; wherein one end of LINKER interacts covalently with one end of PEP12 via $AA^{20}$; wherein another end of LINKER interacts covalently with one end of PEP2 via $AA^{21}$; wherein one end of PEP5 interacts covalently with another end of PEP12 via $AA^{12}$; wherein another end of PEP5 interacts covalently with one end of PEP7 via $AA^{8}$; wherein one end of PEP6 interacts covalently with another end of PEP2 via $AA^{26}$; wherein another end of PEP6 interacts covalently with one end of PEP8 via $AA^{32}$.

Item 64. A pharmaceutical association according to any one of Items 1 to 3 and 5 to 48, wherein said growth factor receptor-binding compound has the following general formula (IIIa):

PEP7-PEP5-PEP12-LINKER-PEP2-PEP6-PEP8 (IIIa)

wherein LINKER is a linear or branched organic divalent radical having a molecular weight comprised between about 28 and about 2,000 Da; wherein one end of LINKER interacts covalently with one end of PEP12 via $AA^{20}$; wherein another end of LINKER interacts covalently with one end of PEP2 via $AA^{21}$; wherein one end of PEP5 interacts covalently with another end of PEP12 via $AA^{12}$; wherein another end of PEP5 interacts covalently with one end of PEP7 via $AA^{8}$; wherein one end of PEP6 interacts covalently with another end of PEP2 via $AA^{26}$; wherein another end of PEP6 interacts covalently with one end of PEP8 via $AA^{32}$.

Item 65. A pharmaceutical association according to any one of Items 1 to 48, wherein said growth factor receptor-binding compound comprises a peptide, a variant or analog thereof, or a peptidomimetic having the following general formula (IVa):

$AA^1$-$AA^2$-$AA^3$-$AA^4$-$AA^5$-$AA^6$-$AA^7$-$AA^8$-$AA^9$-$AA^{10}$-$AA^{11}$-$AA^{12}$-$AA^{13}$-$AA^{14}$-$AA^{15}$-$AA^{16}$-$AA^{17}$-$AA^{18}$-$AA^{19}$-$AA^{20}$-LINKER-$AA^{21}$-$AA^{22}$-$AA^{23}$-$AA^{24}$-$AA^{25}$-$AA^{26}$-$AA^{27}$-$AA^{28}$-$AA^{29}$-$AA^{30}$-$AA^{31}$-$AA^{32}$-$AA^{33}$-$AA^{34}$-$AA^{35}$-$AA^{36}$-$AA^{37}$-$AA^{38}$ (IVa)

wherein LINKER is a linear or branched organic divalent radical having a molecular weight comprised between about 28 and about 2,000 Da; wherein $AA^1$-$AA^2$-$AA^3$-$AA^4$-$AA^5$-$AA^6$-$AA^7$ is PEP7 as defined herein; wherein $AA^{13}$-$AA^{14}$-$AA^{15}$-$AA^{16}$-$AA^{17}$-$AA^{18}$-$AA^{19}$-$AA^{20}$ is PEP12 as defined herein; wherein $AA^{21}$-$AA^{22}$-$AA^{23}$-$AA^{24}$-$AA^{25}$ is PEP2 as already defined herein; wherein $AA^8$-$AA^9$-$AA^{10}$ is PEP3 as defined herein; wherein $AA^{30}$-$AA^{31}$-$AA^{32}$ is PEP4 as defined herein; wherein $AA^{33}$-$AA^{34}$-$AA^{35}$-$AA^{36}$-$AA^{37}$-$AA^{38}$ is PEP8 as defined herein; wherein $AA^{11}$, $AA^{12}$, $AA^{26}$, $AA^{27}$, $AA^{28}$, and $AA^{29}$ are as defined herein; wherein one end of LINKER interacts covalently with $AA^{20}$; wherein another end of LINKER interacts covalently with $AA^{21}$; wherein $AA^1$ and $AA^{21}$ may be both N-terminal amino acids or both C-terminal amino acids; wherein $AA^{38}$ and $AA^{20}$ may be both N-terminal amino acids or both C-terminal amino acids.

Item 66. A pharmaceutical association according to any one of Items 1 to 3 and 5 to 48, comprising a peptide, a variant or analog thereof, or a peptidomimetic having the following general formula (IVa):

$AA^1$-$AA^2$-$AA^3$-$AA^4$-$AA^5$-$AA^6$-$AA^7$-$AA^8$-$AA^9$-$AA^{10}$-$AA^{11}$-$AA^{12}$-$AA^{13}$-$AA^{14}$-$AA^{15}$-$AA^{16}$-$AA^{17}$-$AA^{18}$-$AA^{19}$-$AA^{20}$-LINKER-$AA^{21}$-$AA^{22}$-$AA^{23}$-$AA^{24}$-$AA^{25}$-$AA^{26}$-$AA^{27}$-$AA^{28}$-$AA^{29}$-$AA^{30}$-$AA^{31}$-$AA^{32}$-$AA^{33}$-$AA^{34}$-$AA^{35}$-$AA^{36}$-$AA^{37}$-$AA^{38}$ (IVa)

wherein LINKER is a linear or branched organic divalent radical having a molecular weight (Mw) comprised between about 28 and about 2,000 Da; wherein $AA^1$-$AA^2$-$AA^3$-$AA^4$-$AA^5$-$AA^6$-$AA^7$ is PEP7 as defined herein; wherein $AA^{13}$-$AA^{14}$-$AA^{15}$-$AA^{16}$-$AA^{17}$-$AA^{18}$-$AA^{19}$-$AA^{20}$ is PEP12 as defined herein; wherein $AA^{21}$-$AA^{22}$-$AA^{23}$-$AA^{24}$-$AA^{25}$ is PEP2 as already defined herein; wherein $AA^8$-$AA^9$-$AA^{10}$ is PEP3 as defined herein; wherein $AA^{30}$-$AA^{31}$-$AA^{32}$ is PEP4 as defined herein; wherein $AA^{33}$-$AA^{34}$-$AA^{35}$-$AA^{36}$-$AA^{37}$-$AA^{38}$ is PEP8 as defined herein; wherein $AA^{11}$, $AA^{12}$, $AA^{26}$, $AA^{27}$, $AA^{28}$, and $AA^{29}$ are as defined herein; wherein one end of LINKER interacts covalently with $AA^{20}$; wherein another end of LINKER interacts covalently with $AA^{21}$; wherein $AA^1$ and $AA^{21}$ may be both N-terminal amino acids or both C-terminal amino acids; wherein $AA^{38}$ and $AA^{20}$ may be both N-terminal amino acids or both C-terminal amino acids.

Item 67. A pharmaceutical association according to any one of Items 1, 2 and 4 to 48, wherein said growth factor receptor-binding compound has any one of the schematic general formulae (V) to (XXVIII) as already disclosed herein; wherein LINKER is a linear or branched organic divalent radical having a molecular weight comprised between about 28 and about 2,000 Da; wherein PEP12 is a peptide with 8 amino acids of formula PEP1-$AA^{17}$-PEP11; wherein PEP2 is a peptide with five amino acids; wherein PEP5 is a peptide with five amino acids; wherein PEP6 is a peptide with between five and seven amino acids; wherein PEP7 an amino acid or a peptide with between two and seven amino acids; wherein PEP8 an amino acid or a peptide with between two and six amino acids; wherein PEP9 is a peptide with between six and twelve amino acids; wherein PEP10 is a peptide with between six and eleven amino acids; wherein curved lines represents covalent bonds between LINKERs and PEP1 to PEP12.

Item 68. A pharmaceutical association according to any one of Items 1, 2 and 4 to 48, wherein said growth factor receptor-binding compound has any one of the cyclic schematic general formulae (XXIX) to (XXXIV) as already disclosed herein.

wherein LINKER is a linear or branched organic divalent radical having a molecular weight (Mw) comprised between about 28 and about 2,000 Da; wherein $AA^{13}$-$AA^{14}$-$AA^{15}$-$AA^{16}$-$AA^{17}$-$AA^{18}$-$AA^{19}$-$AA^{20}$ is PEP12; wherein $AA^{21}$-$AA^{22}$-$AA^{23}$-$AA^{24}$-$AA^{25}$ is PEP2; wherein $AA^8$-$AA^9$-$AA^{10}$ is PEP3; wherein $AA^{30}$-$AA^{31}$-$AA^{32}$ is PEP4; wherein one end of LINKER interacts covalently with $AA^{16}$ or $AA^{20}$; and wherein another end of LINKER interacts covalently with $AA^{21}$.

Item 69. A pharmaceutical association according to any one of Items 49 to 68, wherein said LINKER is a peptide with 2 to 16 amino acids.

Item 70. A pharmaceutical association according to any one of Items 49 to 69, wherein said LINKER comprises a peptide of general formula (XXXV):

$$*AA^{201}\text{-}AA^{202}\text{-}AA^{203}\text{-}AA^{204}\text{-}AA^{205}\text{-}AA^{206}\text{-}AA^{207}\text{-}AA^{208}\text{-}AA^{209}** \qquad \text{(XXXV)}$$

wherein said peptide is selected from the group consisting of $*AA^{III}$-$AA^{I}$-$AA^{I}$-$AA^{II}$-$AA^{VII}$-$AA^{XII}$-$AA^{XII}$-$AA^{XIII}$-$AAX^{III}**$, $*AA^{III}$-$AA^{I}$-$AA^{VI}$-$AA^{II}$-$AA^{VII}$-$AA^{XII}$-$AA^{XII}$-$AA^{XIII}$-$AA^{XIII}**$, $*AA^{III}$-$AA^{I}$-$AA^{I}$-$AA^{I}$-$AA^{II}$-$AA^{XII}$-$AA^{XII}$-$AA^{XIII}$-$AA^{XIII}**$,

*$AA^{III}$-$AA^{I}$-$AA^{II}$-$AA^{II}$-$AA^{VII}$-$AA^{XII}$-$AA^{XII}$-$AA^{XIII}$-$AA^{XIII}$**, *$AA^{III}$-$AA^{I}$-$AA^{II}$-$AA^{IV}$-$AA^{IV}$-$AA^{XII}$-$AA^{XII}$-$AA^{XIII}$-$AA^{XIII}$**, *$AA^{II}$-$AA^{I}$-$AA^{II}$-$AA^{II}$-$AA^{II}$-$AA^{XII}$-$AA^{XII}$-$AA^{XIII}$-$AA^{XIII}$**, *$AA^{III}$-$AA^{III}$-$AA^{II}$-$AA^{VII}$-$AA^{II}$-$AA^{XII}$-$AA^{XII}$-$AA^{XIII}$-$AA^{XIII}$**, *$AA^{II}$-$AA^{I}$-$AA^{I}$-$AA^{I}$-$AA^{II}$-$AA^{XII}$-$AA^{XIII}$-$AA^{XIII}$-$AA^{XIII}$**, *$AA^{V}$-$AA^{V}$-$AA^{VII}$-$AA^{VII}$-$AA^{XII}$-$AA^{XII}$-$AA^{XIII}$-$AA^{XIII}$**, *$AA^{VIII}$-$AA^{V}$-$AA^{VII}$-$AA^{II}$-$AA^{XII}$-$AA^{XII}$-$AA^{XIII}$-$AA^{XIII}$** and *$AA^{V}$-$AA^{V}$-$AA^{V}$-$AA^{VII}$-$AA^{II}$-$AA^{XII}$-$AA^{XIII}$-$AA^{XIII}$**; wherein $AA^{I}$ is an amino acid; wherein $AA^{II}$ is a polar amino acid; wherein $AA^{III}$ is an acidic amino acid; wherein $AA^{IV}$ is a aliphatic amino acid; wherein $AA^{V}$ is a apolar amino acid; wherein $AA^{VI}$ is an aromatic amino acid; wherein $AA^{VII}$ is a basic amino acid; wherein $AA^{VIII}$ is L or I; wherein $AA^{XII}$ is an amino acid selected from the group consisting of G, A, V, L, I, P, M, K, R, H, Y and E; wherein $AA^{XIII}$ is absent, $AA^{II}$ or $AA^{VII}$; wherein any one of $AA^{201}$, $AA^{201}$-$AA^{202}$, $AA^{201}$-$AA^{202}$-$AA^{203}$, $AA^{201}$-$AA^{202}$-$AA^{203}$-$AA^{204}$, $AA^{201}$-$AA^{202}$-$AA^{203}$-$AA^{204}$ -$AA^{205}$, $AA^{201}$-$AA^{202}$-$AA^{203}$-$AA^{204}$-$AA^{205}$-$AA^{206}$, $AA^{201}$-$AA^{202}$-$AA^{203}$-$AA^{204}$-$AA^{205}$-$AA^{206}$-$AA^{207}$, $AA^{203}$-$AA^{204}$-$AA^{205}$-$AA^{206}$-$AA^{207}$-$AA^{208}$-$AA^{209}$, $AA^{204}$-$AA^{205}$-$AA^{206}$-$AA^{207}$-$AA^{208}$-$AA^{209}$, $AA^{205}$-$AA^{206}$-$AA^{207}$-$AA^{208}$-$AA^{209}$, $AA^{206}$-$AA^{207}$-$AA^{208}$-$AA^{209}$ $AA^{207}$-$AA^{208}$-$AA^{209}$, $AA^{208}$-$AA^{209}$ or $AA^{209}$, may be absent; and wherein the amino acids labelled "*" and "**" are either an N-terminal amino acid and a C-terminal amino acid.

Item 71. A pharmaceutical association according to Item 70, wherein said LINKER comprises a peptide selected from the group consisting of *$AA^{201}$-$AA^{202}$-$AA^{203}$-$AA^{204}$-$AA^{205}$-$AA^{206}$-$AA^{207}$-$AA^{208}$-$AA^{209}$** (XXXV), *$AA^{201}$-$AA^{202}$-$AA^{203}$-$AA^{204}$-$AA^{205}$-$AA^{206}$-$AA^{207}$** (XXXV-2) and *$AA^{201}$-$AA^{202}$-$AA^{203}$-$AA^{204}$-$AA^{205}$** (XXXV-4).

Item 72. A pharmaceutical association according to Item 69, wherein said LINKER comprises a peptide selected from the group consisting of DENEKVV, DENKNVV, DEYDKVV, DDSSNVI, DSSNNVI, DDMGVPT, DKGVVTY, NDKQQII, DAANNVV, DSANNVV, DDSSNVI, DNGRVLL, VGRKPKV, IGKTPKI, VGRTPKV, RIKPHQGQH, EYVRKKPKL, EIVRKKPIF, EYVRKKP, EIVRKKP, polyalanine ($A_{1-9}$) and polyglycine ($G_{1-9}$).

Item 73. A pharmaceutical association according to Item 69, wherein said LINKER is a peptide selected from the group consisting of DENEKVV, DENKNW, DEYDKW, DDSSNVI, DSSNNVI, DDMGVPT, DKGVVTY, NDKQQII, DAANNVV, DSANNVV, DDSSNVI, DNGRVLL, VGRKPKV, IGKTPKI, VGRTPKV, RIKPHQGQH, EYVRKKPKL, EIVRKKPIF, EYVRKKP, EIVRKKP, polyalanine ($A_{1-9}$) and polyglycine ($G_{1-9}$).

Item 74. A pharmaceutical association according to any one of Items 49 to 69, wherein said LINKER comprises a poly-alanine peptide $(A)_n$ and/or a poly-glycine $(G)_n$, n being an integer comprised between 2 and 16.

Item 75. A pharmaceutical association according to any one of Items 49 to 68, wherein said LINKER is a saturated or unsaturated hydrocarbon chain comprising between 1 and 22, wherein said hydrocarbon chain is optionally interrupted by one or more non-carbon atom, wherein said non-carbon atom is selected from the group consisting of -O-, -S-, -C(=O), $-SO_2-$, -N(Ri)(C=O)-, and -N(Ri)-, wherein Ri is selected from the group consisting of a hydrogen atom, a C1-C6 alkyl group and an aryl group, and wherein said hydrocarbon chain is non-substituted or substituted by at least one radical selected from the group consisting of a halogen, a monosaccharide, a poly(1-6)saccharide, a nucleotide, a poly(1-6)nucleotide, a C1-C10 alkyl group and an aryl group.

Item 76. A pharmaceutical association according to any one of Items 4 to 75, wherein the pair PEP1:PEP2 is selected from the group consisting of SAIS:LKNYQ, SSLS:LKVYP, NAIS:LKKYR, SATS:LRKHR, SPIS:LKYHY, EPIS:KFKYE, SPIN:YGKIP, SPIS:YKQYE, KPLS:DHHKD, EPLP:EQLSN, EPLT:EQLSN, SNIT:IGEMS, SNIT:LGEMS, RSVK:KEVQV and RPVQ:KKATV.

Item 77. A pharmaceutical association according to any one of Items 4 to 76, wherein the pair PEP12:PEP2 is selected from the group consisting of SAIS-$AA^{17}$-LYL:LKNYQ, SSLS-$AA^{17}$-LFF:LKVYP, NAIS-$AA^{17}$-LYF:LKKYR, SATS-$AA^{17}$-LYY:LRKHR, SPIS-$AA^{17}$-LYK:LKYHY, EPIS-$AA^{17}$-LYL:KFKYE, SPIN-$AA^{17}$-LYF:YGKIP, SPIS-$AA^{17}$-LYI:YKQYE, SPIS-$AA^{17}$-LFI:YKQYE, KPLS-$AA^{17}$-LYV:DHHKD, EPLP-$AA^{17}$-VYY:EQLSN, EPLT-$AA^{17}$-LYY:EQLSN, SNIT-$AA^{17}$-QIM:IGEMS, SNIT-$AA^{17}$-QIM:LGEMS, RSVK-$AA^{17}$-AKV:KEVQV and RPVQ-$AA^{17}$-RKI:KKATV; wherein $AA^{17}$ is selected from the group consisting of G, A, V, L, I, P, F, M, W, T and S.

Item 78. A pharmaceutical association according to any one of Items 4 to 77, wherein the pair PEP3:PEP4 is selected from the group consisting of VPT:VVE, VPE:TVE, APT:WR, APT:VVK, VPT:VAE, VPT:AVS, TPT:WD, VPA:VVE, APV:VEE, VPQ:VRS, VSQ:VKS, VPQ:VKS, VPT:QHN, VPT:EHS, SRV:EEH and TQV:EDH.

Item 79. A pharmaceutical association according to any one of Items 4 to 78, wherein the pair PEP5:PEP6 is selected from the group consisting of VPT-$AA^{11}$-$AA^{12}$:$AA^{26}$-$AA^{27}$-$AA^{28}$-$AA^{29}$-VVE, VPE-$AA^{11}$-$AA^{12}$:$AA^{26}$-$AA^{27}$-$AA^{28}$-$AA^{29}$-TVE, APT-$AA^{11}$-$AA^{12}$:$AA^{26}$-$AA^{27}$-$AA^{28}$-$AA^{29}$-VVR, APT-$AA^{11}$-$AA^{12}$:$AA^{26}$-$AA^{27}$-$AA^{28}$-$AA^{29}$-VVK, VPT-$AA^{11}$-$AA^{12}$:$AA^{26}$-$AA^{27}$-$AA^{28}$-$AA^{29}$-VAE, VPT-$AA^{11}$-$AA^{12}$:$AA^{26}$-$AA^{27}$-$AA^{28}$-$AA^{29}$-AVS, TPT-$AA^{11}$-$AA^{12}$:$AA^{26}$-$AA^{27}$-$AA^{28}$-$AA^{29}$-VVD, VPA-$AA^{11}$-$AA^{12}$:$AA^{26}$-$AA^{27}$-$AA^{28}$-$AA^{29}$-VVE, APV-$AA^{11}$-$AA^{12}$:$AA^{26}$-$AA^{27}$-$AA^{28}$-$AA^{29}$-VEE, VPQ-$AA^{11}$-$AA^{12}$:$AA^{26}$-$AA^{27}$-$AA^{28}$-$AA^{29}$-VRS, VSQ-$AA^{11}$-$AA^{12}$:$AA^{26}$-$AA^{27}$-$AA^{28}$-$AA^{29}$-VKS, VPQ-$AA^{11}$-$AA^{12}$:$AA^{26}$-$AA^{27}$-$AA^{28}$-$AA^{29}$-VKS, VPT-$AA^{11}$-$AA^{12}$:$AA^{26}$-$AA^{27}$-$AA^{28}$-$AA^{29}$-QHN, VPT-$AA^{11}$-$AA^{12}$:$AA^{26}$-$AA^{27}$-$AA^{28}$-$AA^{29}$-EHS, SRV-$AA^{11}$-$AA^{12}$:$AA^{26}$-$AA^{27}$-$AA^{28}$-$AA^{29}$-EEH and TQV-$AA^{11}$-$AA^{12}$ :$AA^{26}$-$AA^{27}$-$AA^{28}$-$AA^{29}$-EDH.

Item 80. A pharmaceutical association according to any one of Items 4 to 79, wherein the pair PEP5:PEP6 is selected from the group consisting of VPTEL:DMVVE, VPEKM:NMTVE, VPTEL:EMVVE, APTKL:NMVVR, APTQL:NMVVR, APTKL:NMVVK, VPTKL:EGMSVAE, VPTKL:GMAVS, TPTKM:GMVVD, VPARL:DMVVE, VPTRL:DMVVE, APVKT:MIVEE, VPQAL:MIVRS, VSQDL:MIVKS, VPQDL:MVVKS, VPTEE:FLQHN, VPTGQ:LEEHS, SRVHH:RLEEH and TQVQL:TLEDH.

Item 81. A pharmaceutical association according to any one of Items 4 to 80, wherein the pair PEP7:PEP8 is selected from the group consisting of KIPKAXX:GXGXR, GIPEPXX:SXAXR, SIPKAXX:GXGXR, HVTKPTX:SXGXH, YVPKPXX:SXGXH, TVPKPXX:AXGXH, AVPKAXX:AXGXH, KVGKAXX:XGXR, KASKAXX:EXGXR, GSAGPXX:RXGXS, AAPASXX:AXGXR, STPPTXX:SXGXR, HVPKPXX:SXGXH, RVPSTXX:XGXL, ASAAPXX:XKXS, ASASPXX:XKXS, NDEGLEX:KXEXR, NDEGLEX:QXEXR, SSVKXQP:LEXAXA and RNVQXRP:LAXKXE.

Item 82. A pharmaceutical association according to any one of Items 4 to 81, wherein the pair PEP9:PEP10 is selected from the group consisting of KIPKAXXVPT-AA$^{11}$-AA$^{12}$:AA$^{26}$-AA$^{27}$-AA$^{28}$-AA$^{29}$-WEGXGXR, GIPEPXXVPE-AA$^{11}$-AA$^{12}$:AA$^{26}$-AA$^{27}$-AA$^{28}$-AA$^{29}$-TVESXAXR, SIPKAXXVPT-AA$^{11}$-AA$^{12}$:AA$^{26}$-AA$^{27}$-AA$^{28}$-AA$^{29}$-VVEGXGXR, HVTKPTXAPT-AA$^{11}$-AA$^{12}$:AA$^{26}$-AA$^{27}$-AA$^{28}$-AA$^{29}$-VVRSXGXH, YVPKPXXAPT-AA$^{11}$-AA$^{12}$:AA$^{26}$-AA$^{27}$-AA$^{28}$-AA$^{29}$-VVRSXGXH, TVPKPXXAPT-AA$^{11}$-AA$^{12}$:AA$^{26}$-AA$^{27}$-AA$^{28}$-AA$^{29}$-VVRAXGXH, AVPKAXXAPT-AA$^{11}$-AA$^{12}$:AA$^{26}$-AA$^{27}$-AA$^{28}$-AA$^{29}$-WKAXGXH, KVGKAXXVPT-AA$^{11}$-AA$^{12}$:AA$^{26}$-AA$^{27}$-AA$^{28}$-AA$^{29}$-VAEXGXR, KASKAXXVPT-AA$^{11}$-AA$^{12}$-AA$^{11}$-AA$^{12}$:AA$^{26}$-AA$^{27}$-AA$^{28}$-AA$^{29}$-AVSEXGXR, GSAGPXXTPT-AA$^{11}$-AA$^{12}$:AA$^{26}$-AA$^{27}$-AA$^{28}$-AA$^{29}$-VVDRXGXS, AAPASXXVPA-AA$^{11}$-AA$^{12}$:AA$^{26}$-AA$^{27}$-AA$^{28}$-AA$^{29}$-VVEAXGXR, STPPTXXVPT-AA$^{11}$-AA$^{12}$-AA$^{11}$-AA$^{12}$:AA$^{26}$-AA$^{27}$-AA$^{28}$-AA$^{29}$-WESXGXR, HVPKPXXAPT-AA$^{11}$-AA$^{12}$:AA$^{26}$-AA$^{27}$-AA$^{28}$-AA$^{29}$-VVRSXGXH, RVPSTXXAPV-AA$^{11}$-AA$^{12}$:AA$^{26}$-AA$^{27}$-AA$^{28}$-AA$^{29}$-VEEXGXL, ASAAPXXVPQ-AA$^{11}$-AA$^{12}$:AA$^{26}$-AA$^{27}$-AA$^{28}$-AA$^{29}$-VRSXKXS, ASASPXXVSQ-AA$^{11}$-AA$^{12}$:AA$^{26}$-AA$^{27}$-AA$^{28}$-AA$^{29}$-VKSXKXS, ASASPXXVPQ-AA$^{11}$-AA$^{12}$:AA$^{26}$-AA$^{27}$-AA$^{28}$-AA$^{29}$-VKSXKXS, NDEGLEXVPT-AA$^{11}$-AA$^{12}$:AA$^{26}$-AA$^{27}$-AA$^{28}$-AA$^{29}$-QHNKXEXR, NDEGLEXVPT-AA$^{11}$-AA$^{12}$ :AA$^{26}$-AA$^{27}$-AA$^{28}$-AA$^{29}$-EHSQXEXR, SSVKXQPSRV-AA$^{11}$-AA$^{12}$:AA$^{26}$-AA$^{27}$-AA$^{28}$-AA$^{29}$-EEHLEXAXA and RNVQXRPTQV-AA$^{11}$-AA$^{12}$:AA$^{26}$-AA$^{27}$-AA$^{28}$-AA$^{29}$-EDHLAXKXE.

Item 83. A pharmaceutical association according to any one of Items 4 to 82, wherein the pair PEP9:PEP10 is selected from the group consisting of KIPKAXXVPTEL:DMVVEGXGXR, GIPEPXXVPEKM:NMTVESXAXR, SIPKAXXVPTEL:EMVVEGXGXR, HVTKPTXAPTKL:NMVVRSXGXH, YVPKPXXAPTKL:NMVVRSXGXH, TVPKPXXAPTQL:NMVVRAXGXH, AVPKAXXAPTKL:NMVVKAXGXH, KVGKAXXVPTKL:EGMSVAEXGXR, KASKAXXVPTKL:GMAVSEXGXR, GSAGPXXTPTKM:GMVVDRXGXS, AAPASXXVPARL:DMWEAXGXR, STPPTXXVPTRL:DMVVESXGXR, HVPKPXXAPTKL:NMVVRSXGXH, RVPSTXXAPVKT:MIVEEXGXL, ASAAPXXVPQAL:MIVRSXKXS, ASASPXXVSQDL:MIVKSXKXS, ASASPXXVPQDL:MVVKSXKXS, NDEGLEXVPTEE:FLQHNKXEXR, NDEGLEXVPTGQ:LEEHSQXEXR, NDEGLEXVPTEE:FLQHNKXEXR, NDEGLEXVPTEE:FLQHNKXEXR, SSVKXQPSRVHH:RLEEHLEXAXA and RNVQXRPTQVQL:TLEDH LAXKXE.

Item 84. A pharmaceutical association according to any one of Items 4 to 83, wherein the pair PEP5:PEP6 is not VPTEL:DMWE when PEP1 is SAIS and PEP2 is LKNYQ; wherein the pair PEP5:PEP6 is not VPEKM:NMTVE when PEP1 is SSLS and PEP2 is LKVYP; wherein the pair PEP5:PEP6 is not VPTEL:EMVVE when PEP1 is SAIS and PEP2 is LKNYQ; wherein the pair PEP5:PEP6 is not APTKL:NMWR when PEP1 is NAIS and PEP2 is LKKYR; wherein the pair PEP5:PEP6 is not APTQL:NMWR when PEP1 is NAIS and PEP2 is LKKYR; wherein the pair PEP5:PEP6 is not APTKL:NMWK when PEP1 is SATS and PEP2 is LRKHR; wherein the pair PEP5:PEP6 is not VPTKL:EGMSVAE when PEP1 is SPIS and PEP2 is LKYHY; wherein the pair PEP5:PEP6 is not VPTKL:GMAVS when PEP1 is EPIS and PEP2 is KFKYE; wherein the pair PEP5:PEP6 is not TPTKM:GMVVD when PEP1 is SPIN and PEP2 is YGKIP; wherein the pair PEP5:PEP6 is not VPARL:DMWE when PEP1 is SPIS and PEP2 is YKQYE; wherein the pair PEP5:PEP6 is not VPTRLDMWE when PEP1 is SPIS and PEP2 is YKQYE; wherein the pair PEP5:PEP6 is not APVKT:MIVEE when PEP1 is KPLS and PEP2 is DHHKD; wherein the pair PEP5:PEP6 is not VPQAL:MIVRS when PEP1 is EPLP and PEP2 is EQLSN; wherein the pair PEP5:PEP6 is not VSQDL:MIVKS when PEP1 is EPLT and PEP2 is EQLSN; wherein the pair PEP5:PEP6 is not VPQDL:MVVKS when PEP1 is EPLT and PEP2 is EQLSN; wherein the pair PEP5:PEP6 is not VPTEE:FLQHN when PEP1 is SNIT and PEP2 is IGEMS; wherein the pair PEP5:PEP6 is not VPTGQ:LEEHS when PEP1 is SNIT and PEP2 is LGEMS; wherein the pair PEP5:PEP6 is not SRVHH:RLEEH when PEP1 is RSVK and PEP2 is KEVQV; and wherein the pair PEP5:PEP6 is not TQVQL:TLEDH when PEP1 is RPVQ and PEP2 is KKATV.

Item 85. A pharmaceutical association according to any one of Items 4 to 84, wherein the pair PEP9:PEP10 is not KIPKAXXVPT-AA$^{11}$-AA$^{12}$:AA$^{26}$-AA$^{27}$-AA$^{28}$-AA$^{29}$-VVEGXGXR when PEP1 is SAIS and PEP2 is LKNYQ; wherein PEP9:PEP10 is not GIPEPXXVPE-AA$^{11}$-AA$^{12}$:AA$^{26}$-AA$^{27}$-AA$^{28}$-AA$^{29}$-TVESXAXR when PEP1 is SSLS and PEP2 is LKVYP; wherein PEP9:PEP10 is not SIPKAXXVPT-AA$^{11}$-AA$^{12}$:AA$^{26}$-AA$^{27}$-AA$^{28}$-AA$^{29}$-VVEGXGXR when PEP1 is SAIS and PEP2 is LKNYQ; wherein PEP9:PEP10 is not HVTKPTXAPT-AA$^{11}$-AA$^{12}$:AA$^{26}$-AA$^{27}$-AA$^{28}$-AA$^{29}$-WRSXGXH when PEP1 is NAIS and PEP2 is LKKYR; wherein PEP9:PEP10 is

not YVPKPXXAPT-$AA^{11}$-$AA^{12}$:$AA^{26}$-$AA^{27}$-$AA^{28}$-$AA^{29}$-VVRSXGXH when PEP1 is NAIS and PEP2 is LKKYR; wherein PEP9:PEP10 is not TVPKPXXAPT-$AA^{11}$-$AA^{12}$:$AA^{26}$-$AA^{27}$-$AA^{28}$-$AA^{29}$-VVRAXGXH when PEP1 is NAIS and PEP2 is LKKYR; wherein PEP9:PEP10 is not AVPKAXXAPT-$AA^{11}$-$AA^{12}$:$AA^{26}$-$AA^{27}$-$AA^{28}$-$AA^{29}$-VVKAXGXH when PEP1 is SATS and PEP2 is LRKHR; wherein PEP9:PEP10 is not KVGKAXXVPT-$AA^{11}$-$AA^{12}$:$AA^{26}$-$AA^{27}$-$AA^{28}$-$AA^{29}$-VAEXGXR when PEP1 is SPIS and PEP2 is LKYHY; wherein PEP9:PEP10 is not KASKAXXVPT-$AA^{11}$-$AA^{12}$-$AA^{11}$-$AA^{12}$:$AA^{26}$-$AA^{27}$-$AA^{28}$-$AA^{29}$-AVSEXGXR when PEP1 is EPIS and PEP2 is KFKYE; wherein PEP9:PEP10 is not GSAGPXXTPT-$AA^{11}$-$AA^{12}$:$AA^{26}$-$AA^{27}$-$AA^{28}$-$AA^{29}$-VVDRXGXS when PEP1 is SPIN and PEP2 is YGKIP; wherein PEP9:PEP10 is not AAPASXXVPA-$AA^{11}$-$AA^{12}$:$AA^{26}$-$AA^{27}$-$AA^{28}$-$AA^{29}$-VVEAXGXR when PEP1 is SPIS and PEP2 is YKQYE; wherein PEP9:PEP10 is not STPPTXXVPT-$AA^{11}$-$AA^{12}$-$AA^{11}$-$AA^{12}$:$AA^{26}$-$AA^{27}$-$AA^{28}$-$AA^{29}$-VVESXGXR when PEP1 is SPIS and PEP2 is YKQYE; wherein PEP9:PEP10 is not HVPKPXXAPT-$AA^{11}$-$AA^{12}$:$AA^{26}$-$AA^{27}$-$AA^{28}$-$AA^{29}$-VVRSXGXH when PEP1 is NAIS and PEP2 is LKKYR; wherein PEP9:PEP10 is not RVPSTXXAPV-$AA^{11}$-$AA^{12}$:$AA^{26}$-$AA^{27}$-$AA^{28}$-$AA^{29}$-VEEXGXL when PEP1 is KPLS and PEP2 is DHHKD; wherein PEP9:PEP10 is not ASAAPXXVPQ-$AA^{11}$-$AA^{12}$:$AA^{26}$-$AA^{27}$-$AA^{28}$-$AA^{29}$-VRSXKXS when PEP1 is EPLP and PEP2 is EQLSN; ASASPXXVSQ-$AA^{11}$-$AA^{12}$:$AA^{26}$-$AA^{27}$-$AA^{28}$-$AA^{29}$-VKSXKXS when PEP1 is EPLT and PEP2 is EQLSN; wherein PEP9:PEP10 is not ASASPXXVPQ-$AA^{11}$-$AA^{12}$:$AA^{26}$-$AA^{27}$-$AA^{28}$-$AA^{29}$-VKSXKXS when PEP1 is EPLT and PEP2 is EQLSN; wherein PEP9:PEP10 is not NDEGLEXVPT-$AA^{11}$-$AA^{12}$:$AA^{26}$-$AA^{27}$-$AA^{28}$-$AA^{29}$-QHNKXEXR when PEP1 is SNIT and PEP2 is IGEMS; wherein PEP9:PEP10 is not NDEGLEXVPT-$AA^{11}$-$AA^{12}$:$AA^{26}$-$AA^{27}$-$AA^{28}$-$AA^{29}$-EHSQXEXR when PEP1 is SNIT and PEP2 is LGEMS; wherein PEP9:PEP10 is not SSVKXQPSRV-$AA^{11}$-$AA^{12}$:$AA^{26}$-$AA^{27}$-$AA^{28}$-$AA^{29}$-EEHLEXAXA when PEP1 is RSVK and PEP2 is KEVQV; and wherein PEP9:PEP10 is not RNVQXRPTQV-$AA^{11}$-$AA^{12}$:$AA^{26}$-$AA^{27}$-$AA^{28}$-$AA^{29}$-EDHLAXKXE when PEP1 is RPVQ and PEP2 is KKATV.

Item 86. A pharmaceutical association according to any one of Items 4 to 85, wherein PEP9:PEP10 is not KIPKAXXVPTEL:DMWEGXGXR when PEP1 is SAIS and PEP2 is LKNYQ ; wherein PEP9:PEP10 is not GIPEPXXVPEKM:NMTVESXAXR when PEP1 is SSLS and PEP2 is LKVYP; wherein PEP9:PEP10 is not SIPKAXXVPTEL:EMVVEGXGXR when PEP1 is SAIS and PEP2 is LKNYQ; wherein PEP9:PEP10 is not HVTKPTXAPTKL:NMVVRSXGXH when PEP1 is NAIS and PEP2 is LKKYR; wherein PEP9:PEP10 is not YVPKPXXAPTKL:NMVVRSXGXH when PEP1 is NAIS and PEP2 is LKKYR; wherein PEP9:PEP10 is not TVPKPXXAPTQL:NMVVRAXGXH when PEP1 is NAIS and PEP2 is LKKYR; wherein PEP9:PEP10 is not AVPKAXXAPTKLNMWKAXGXH when PEP1 is SATS and PEP2 is LRKHR; wherein PEP9:PEP10 is not KVGKAXXVPTKL:EGMSVAEXGXR when PEP1 is SPIS and PEP2 is LKYHY; wherein PEP9:PEP10 is not KASKAXXVPTKL:GMAVSEXGXR when PEP1 is EPIS and PEP2 is KFKYE; wherein PEP9:PEP10 is not GSAGPXXTPTKM:GMVVDRXGXS when PEP1 is SPIN and PEP2 is YGKIP; wherein PEP9:PEP10 is not AAPASXXVPARL:DMVVEAXGXR when PEP1 is SPIS and PEP2 is YKQYE; wherein PEP9:PEP10 is not STPPTXXVPTRL:DMVVESXGXR when PEP1 is SPIS and PEP2 is YKQYE; wherein PEP9:PEP10 is not HVPKPXXAPTKLNMWRSXGXH when PEP1 is NAIS and PEP2 is LKKYR; wherein PEP9:PEP10 is not RVPSTXXAPVKT:MIVEEXGXL when PEP1 is KPLS and PEP2 is DHHKD; wherein PEP9:PEP10 is not ASAAPXXVPQAL:MIVRSXKXS when PEP1 is EPLP and PEP2 is EQLSN; ASASPXXVSQDL:MIVKSXKXS when PEP1 is EPLT and PEP2 is EQLSN; wherein PEP9:PEP10 is not ASASPXXVPQDL:MVVKSXKXS when PEP1 is EPLT and PEP2 is EQLSN; wherein PEP9:PEP10 is not NDEGLEXVPTEE:FLQHNKXEXR when PEP1 is SNIT and PEP2 is IGEMS; wherein PEP9:PEP10 is not NDEGLEXVPTGQ:LEEHSQXEXR when PEP1 is SNIT and PEP2 is LGEMS ; wherein PEP9:PEP10 is not NDEGLEXVPTEE:FLQHNKXEXR when PEP1 is SNIT and PEP2 is IGEMS; wherein PEP9:PEP10 is not NDEGLEXVPTEE:FLQHNKXEXR when PEP1 is SNIT and PEP2 is IGEMS; wherein PEP9:PEP10 is not SSVKXQPSRVHH:RLEEHLEXAXA when PEP1 is RSVK and PEP2 is KEVQV; and wherein PEP9:PEP10 is not RNVQXRPTQVQL:TLEDHLAXKXE when PEP1 is RPVQ and PEP2 is KKATV.

Item 87. A pharmaceutical association according to any one of Items 4 to 86, wherein the pair PEP1:PEP11 is selected from the group consisting of SAIS:LYL, SSLS:LFF, NAIS:LYF, SATS:LYY, SPIS:LYK, SPIS:LYI, SPIS:LFI, EPIS:LYL, SPIN:LYF, KPLS:LYV, EPLP:VYY, EPLT:LYY, SNIT:QIM, RSVK:AKV and RPVQ:RKI.

Item 88. A pharmaceutical association according to any one of Items 4 to 87, wherein the quadruplet PEP5:PEP1 :PEP2:PEP6 is as already disclosed herein from page 79 to page 86.

Item 89. A pharmaceutical association according to any one of Items 4 to 88, wherein the quadruplet PEP9:PEP1:PEP2:PEP10 is as already disclosed herein from page 97 to page 110.

Item 90. A pharmaceutical association according to any one of Items 4 to 89, wherein the hexaplet PEP7:PEP3:PEP1:PEP2:PEP4:PEP8 is as already disclosed herein from page 159 to page 167.

Item 91. A pharmaceutical association according to any one of Items 4 to 90, wherein the hexaplet PEP7:PEP5:PEP1:PEP2:PEP6:PEP8 is as already disclosed herein from page 167 to page 173.

Item 92. A pharmaceutical association according to any one of Items 1 to 3 and 5 to 91, wherein said growth factor receptor-binding compound is a peptide, or a variant or analog thereof, with between 25 and 60 amino acids having growth factor receptor-binding capability.

Item 93. A pharmaceutical association according to any one of Items 1 to 3 and 5 to 91, wherein said growth factor receptor-binding compound is a peptide, or a variant or analog thereof with between 25 and 50 amino acids.
Item 94. A pharmaceutical association according to any one of Items 1 to 3 and 5 to 91, wherein said growth factor receptor-binding compound is a peptide, or a variant or analog thereof with between 25 and 45 amino acids.
Item 95. A pharmaceutical association according to any one of Items 92 to 94, wherein the RMSD value of the structure coordinates of said peptide, variant or analog thereof with respect to PEPREF is 2.45Å (Angstroms) or less, and wherein PEPREF is as already disclosed herein.
Item 96. A pharmaceutical association according to any one of Items 1 to 3 and 5 to 91, wherein said growth factor receptor-binding compound is a peptidomimetic having growth factor receptor-binding capability, wherein said peptidomimetic has a molecular weight comprised between 2,000 and 8,000 Daltons.
Item 97. A pharmaceutical association according to any one of Items 1 to 3 and 5 to 91, wherein said growth factor receptor-binding compound is a peptidomimetic having growth factor receptor-binding capability, wherein said peptidomimetic has a molecular weight comprised between 2,000 and 6,000 Da.
Item 98. A pharmaceutical association according to Item 96 or 97, wherein the RMSD value of the atomic structure coordinates of said peptidomimetic with respect to PEPREF is 2.45Å (Angstroms) or less.
Item 99. A pharmaceutical association according to any one of Items 96 to 98, wherein said growth factor receptor-binding compound is a peptidomimetic having growth factor receptor-binding capability, comprising between 20 and 50 amino acids.
Item 100. A pharmaceutical association according to any one of Items 96 to 98, wherein said growth factor receptor-binding compound is a peptidomimetic having growth factor receptor-binding capability, comprising between 20 and 45 amino acids.
Item 101. A pharmaceutical association according to any one of Items 96 to 98, wherein said growth factor receptor-binding compound is a peptidomimetic having growth factor receptor-binding capability, comprising between 23 and 60 amino acids.
Item 102. A pharmaceutical association according to any one of Items 96 to 98, wherein said growth factor receptor-binding compound is a peptidomimetic having growth factor receptor-binding capability, comprising between 23 and 50 amino acids.
Item 103. A pharmaceutical association according to any one of Items 96 to 98, wherein said growth factor receptor-binding compound is a peptidomimetic having growth factor receptor-binding capability, comprising between 23 and 45 amino acids.
Item 104. A pharmaceutical association according to any one of Items 96 to 103, wherein said growth factor receptor-binding compound is a peptidomimetic comprising at least one peptide fragment with between 5-20 amino acids.
Item 105. A pharmaceutical association according to any one of Items 96 to 103, wherein said growth factor receptor-binding compound is a peptidomimetic comprising at least two peptide fragments, each of which with between 5-20 amino acids.
Item 106. A pharmaceutical association according to any one of Items 1, 2 and 4 to 91, wherein said growth factor receptor-binding compound is a cyclic peptide, or a variant or analog thereof, with between 15 and 60 amino acids, having a growth factor receptor-binding capability.
Item 107. A pharmaceutical association according to any one of Items 1, 2 and 4 to 91, wherein said growth factor receptor-binding compound is a cyclic peptide, or a variant or analog thereof, with between 15 and 55 amino acids.
Item 108. A pharmaceutical association according to any one of Items 1, 2 and 4 to 91, wherein said growth factor receptor-binding compound is a cyclic peptide, or a variant or analog thereof, with between 19 and 60 amino acids.
Item 109. A pharmaceutical association according to any one of Items 1, 2 and 4 to 91, wherein said growth factor receptor-binding compound is a cyclic peptide, or a variant or analog thereof, with between 19 and 55 amino acids.
Item 110. A pharmaceutical association according to any one of Items 1, 2 and 4 to 91, wherein said growth factor receptor-binding compound is a cyclic peptidomimetic comprising between 15 and 60 amino acids, having a growth factor receptor-binding capability.
Item 111. A pharmaceutical association according to any one of Items 1, 2 and 4 to 91, wherein said growth factor receptor-binding compound is a cyclic peptidomimetic comprising between 15 and 55 amino acids.
Item 112. A pharmaceutical association according to any one of Items 1, 2 and 4 to 91, wherein said growth factor receptor-binding compound is a cyclic peptidomimetic comprising between 19 and 60 amino acids.
Item 113. A pharmaceutical association according to any one of Items 1, 2 and 4 to 91, wherein said growth factor receptor-binding compound is a cyclic peptidomimetic comprising between 19 and 55 amino acids.
Item 114. A pharmaceutical association according to any one of Items 110 to 113, wherein said growth factor receptor-binding compound is a cyclic peptidomimetic comprising at least one peptide fragment with between 5-20 amino acids.
Item 115. A pharmaceutical association according to any one of Items 110 to 113, wherein said growth factor receptor-binding compound is a cyclic peptidomimetic comprising at least two peptide fragments, each of which

with between 5-20 amino acids.

Item 116. A pharmaceutical association according to any one of Items 1, 2, 4 to 91 and 106 to 115, wherein said growth factor receptor-binding compound is a cyclic peptide, or a variant or analog thereof, or a cyclic peptidomimetic, having a molecular weight of less than 6,000 Da.

Item 117. A pharmaceutical association according to any one of Items 1, 2, 4 to 91 and 106 to 115, wherein said growth factor receptor-binding compound is a cyclic peptide, or a variant or analog thereof, or a cyclic peptidomimetic, having a molecular weight comprised between 1,500 and 7,000 Da.

Item 118. A pharmaceutical association according to any one of Items 1, 2, 4 to 91 and 106 to 115, wherein said growth factor receptor-binding compound is a cyclic peptide, or a variant or analog thereof, or a cyclic peptidomimetic, having a molecular weight comprised between 1,500 and 6,000 Da.

Item 119. A pharmaceutical association according to any one of Items 1 to 118, wherein said growth factor receptor-binding compound is selected from the group consisting of any one of peptides of SEQ ID NO: 1 to 100371.

Item 120. A medical composition comprising at least one pharmaceutical association according to any one of Items 1 to 119 and at least one medically acceptable excipient, carrier or vehicle.

Item 121. A medical composition according to Item 120, further comprising at least one other anticancer agent.

Item 122. A pharmaceutical association according to any one of Items 1 to 119, or a medical composition according to Item 120 or 121, for use in the treatment, prevention and/or diagnostic of a neoplastic disease.

Item 123. A pharmaceutical association for a use according to Item 122, wherein said association is administered locally.

Item 124. A screening method for selecting a growth factor receptor-binding compound having the ability, after being associated with an adhesion protein inhibitor, to convert a neoplastic cell into a non-neoplastic cell.

Item 125. A screening method according to Item 124, wherein said method comprises providing a molecular model of the 3D structure coordinates of PEPREF and identifying a candidate analog having a RMSD value of 2.45Å or less.

Item 126. A method of producing a growth factor receptor-binding compound having the ability, after being associated with an adhesion protein inhibitor, to convert a neoplastic cell into a non-neoplastic cell, the method comprising (a) providing a molecular model of the 3D structure coordinates of PEPREF; (b) identifying a candidate analog having a RMSD of 2.45Å or less; and (c) producing said candidate analog identified in step (b).

Item 127. A method of producing a growth factor receptor-binding compound according to Item 126, wherein said method further comprising determining whether the compound produced in step (c) has a neoplastic cell recoding activity.

Item 128. A method of producing a growth factor receptor-binding compound according to Item 126 or 127, wherein steps (a) and (b) are performed by means of an electronic processor.

Item 129. A method of producing a growth factor receptor-binding compound according to any one of Items 126 to 128, wherein step (a) comprises storing a representation of the atomic coordinates of PEPREF in a computer memory.

Item 130. A process for manufacturing a neoplastic disease medicament, said process comprising providing at least one adhesion protein inhibitor and at least one growth factor receptor-binding compound both as defined in any one of Items 1 to 119, wherein providing said adhesion protein inhibitor and said growth factor receptor-binding compound manufactures said neoplastic disease medicament.

Item 131. A process for manufacturing a neoplastic disease medicament precursor, said process comprising providing at least one growth factor receptor-binding compound or at least one adhesion protein inhibitor both as defined in any one of Items 1 to 119, wherein providing said adhesion protein inhibitor or said growth factor receptor-binding compound manufactures said neoplastic disease medicament precursor.

Item 132. A method for converting a neoplastic cell into a non-neoplastic cell, in-vitro or ex-vivo, said method comprising administering to a neoplastic cell an effective amount of a pharmaceutical association or composition as defined in any one of Items 1 to 121.

Item 133. Kit-of-parts comprising at least one adhesion protein inhibitor and at least one growth factor receptor-binding compound both as defined in any one of Items 1 to 119 for use in the treatment, prevention and/or diagnostic of a neoplastic disease.

Item 134. A growth factor receptor-binding compound as defined in any one of Items 1 to 119, for use in the prevention, diagnostic and/or treatment of a neoplastic disease in combination with at least one adhesion protein inhibitor.

Item 135. An adhesion protein inhibitor, for use in the prevention, diagnostic and/or treatment of a neoplastic disease in combination with at least one growth factor receptor-binding compound as defined in any one of Items 1 to 119.

Item 136. A growth factor receptor-binding compound as defined in any one of Items 1 to 119, for use in the prevention, diagnostic and/or treatment of a neoplastic disease in combination with at least one adhesion protein inhibitor as defined in any one of Items 1 to 119.

Item 137. A process for manufacturing a neoplastic disease medicament, said process comprising providing at least one growth factor receptor-binding compound as defined in any one of Items 1 to 119 and at least one adhesion protein inhibitor, wherein providing said adhesion protein inhibitor and said growth factor receptor-binding compound

manufactures said neoplastic disease medicament.

Item 138. A process for manufacturing a neoplastic disease medicament precursor, said process comprising providing at least one growth factor receptor-binding compound as defined in any one of Items 1 to 119 or at least one adhesion protein inhibitor, wherein providing said adhesion protein inhibitor or said growth factor receptor-binding compound manufactures said neoplastic disease medicament precursor.

Item 139. Use of a pharmaceutical association as defined in any one of Items 1 to 123 for the preparation of a medical composition for the treatment or prevention of a neoplastic disease, in particular, cancer.

Item 140. A medical composition comprising at least one polynucleotide encoding at least one peptide as defined in any one of Items 1 to 119, and at least one polynucleotide encoding at least one adhesion protein inhibitor or antagonist as defined in Item 7.

Item 141. A medical composition comprising at least one polynucleotide encoding at least one peptide as defined in any one of Items 1 to 119, and at least one adhesion protein inhibitor or antagonist as defined in Item 7.

Item 142. A medical composition comprising at least one peptide as defined in any one of Items 1 to 119, and at least one polynucleotide encoding at least one adhesion protein inhibitor or antagonist as defined in Item 7.

Item 143. A medical composition according to any one of Items 140 to 142, wherein said polynucleotide is a messenger RNA or a primary construct thereof.

## Claims

**1.** A pharmaceutical association comprising at least one growth factor receptor-binding compound, which activates at least one growth factor receptor of a neoplastic cell, and at least one adhesion protein inhibitor which inhibits at least one transmembrane cell adhesion protein of said neoplastic cell,

wherein said growth factor receptor-binding compound comprises a peptide with four amino acids PEP1, and a peptide with five amino acids PEP2,
wherein PEP1 is NAIS, and
wherein PEP2 is LKKYR.

**2.** A pharmaceutical association according to claim 1, wherein said growth factor receptor-binding compound is a non-cyclic growth factor receptor-binding compound and has a molecular weight of less than 8,000 Daltons, or a cyclic growth factor receptor-binding compound and has a molecular weight of less than 7,000 Daltons.

**3.** A pharmaceutical association according to claims 1 or 2, wherein said growth factor receptor is selected from the group consisting of epidermal growth factor receptors, fibroblast growth factor receptors, vascular endothelial growth factor receptors, nerve growth factor receptors, insulin receptor family, Trk receptor family, Eph receptor family, AXL receptor family, LTK receptor family, TIE receptor family, ROR receptor family, DDR receptor family, RET receptor family, KLG receptor family, RYK receptor family, MuSK receptor family, hepatocyte growth factor receptors, somatomedin or insulin-like growth factor receptors, platelet-derived growth factor receptors, and transforming growth factor beta superfamily proteins.

**4.** A pharmaceutical association according to any one of claims 1 to 3, wherein said adhesion inhibitor is selected from the group consisting of an integrin inhibitor which inhibits at least one integrin, a syndecan inhibitor which inhibits at least one syndecan, a selectin inhibitor which inhibits at least one selectin, a dystroglycan inhibitor which inhibits at least one dystroglycan, and any combinations thereof.

**5.** A pharmaceutical association according to any one of claims 1 to 4, wherein said growth factor receptor-binding compound is a peptide or a peptidomimetic.

**6.** A pharmaceutical association according to any one of claims 1 to 5, wherein said growth factor receptor-binding compound is a non-cyclic peptide with between 20 and 60 amino acids having growth factor receptor-binding capability or is a non-cyclic peptidomimetic having growth factor receptor-binding capability, wherein said peptidomimetic has a molecular weight comprised between 2,000 and 8,000 Daltons.

**7.** A pharmaceutical association according to claim 6, wherein said peptidomimetic comprises between 20 and 60 amino acids.

**8.** A pharmaceutical association according to claim 6 or 7, wherein the RMSD value of the structure coordinates of said peptide or peptidomimetic with respect to PEPREF is 2.45Å (Angstroms) or less, and wherein PEPREF is

| | | | | | | | | |
|---|---|---|---|---|---|---|---|---|
| ATOM | 511 | N | LYS | A | 1 | -14.570 | 46.437 | 27.424 |
| ATOM | 512 | CA | LYS | A | 1 | -13.512 | 45.748 | 28.151 |
| ATOM | 513 | C | LYS | A | 1 | -13.655 | 44.259 | 27.884 |
| ATOM | 514 | O | LYS | A | 1 | -12.769 | 43.463 | 28.197 |
| ATOM | 515 | CB | LYS | A | 1 | -13.605 | 46.029 | 29.652 |
| ATOM | 516 | CG | LYS | A | 1 | -13.640 | 47.509 | 29.991 |
| ATOM | 517 | CD | LYS | A | 1 | -12.615 | 48.297 | 29.183 |
| ATOM | 518 | CE | LYS | A | 1 | -12.625 | 49.768 | 29.575 |
| ATOM | 519 | NZ | LYS | A | 1 | -13.994 | 50.369 | 29.497 |
| ATOM | 520 | N | ILE | A | 2 | -14.792 | 43.890 | 27.309 |
| ATOM | 521 | CA | ILE | A | 2 | -15.051 | 42.499 | 26.967 |
| ATOM | 522 | C | ILE | A | 2 | -14.911 | 42.370 | 25.444 |
| ATOM | 523 | O | ILE | A | 2 | -15.531 | 43.125 | 24.683 |
| ATOM | 524 | CB | ILE | A | 2 | -16.466 | 42.065 | 27.401 |
| ATOM | 525 | CG1 | ILE | A | 2 | -16.630 | 42.238 | 28.915 |
| ATOM | 526 | CG2 | ILE | A | 2 | -16.710 | 40.629 | 26.985 |
| ATOM | 527 | CD1 | ILE | A | 2 | -15.631 | 41.478 | 29.30 |
| ATOM | 528 | N | PRO | A | 3 | -14.085 | 41.411 | 24.989 |
| ATOM | 529 | CA | PRO | A | 3 | -13.789 | 41.109 | 23.588 |
| ATOM | 530 | C | PRO | A | 3 | -14.998 | 40.695 | 22.768 |
| ATOM | 531 | O | PRO | A | 3 | -15.969 | 40.164 | 23.305 |
| ATOM | 532 | CB | PRO | A | 3 | -12.785 | 39.968 | 23.688 |
| ATOM | 533 | CG | PRO | A | 3 | -12.156 | 40.166 | 25.007 |
| ATOM | 534 | CD | PRO | A | 3 | -13.330 | 40.506 | 25.867 |
| ATOM | 535 | N | LYS | A | 4 | -14.937 | 40.937 | 21.463 |
| ATOM | 536 | CA | LYS | A | 4 | -16.023 | 40.529 | 20.590 |
| ATOM | 537 | C | LYS | A | 4 | -15.886 | 39.015 | 20.391 |
| ATOM | 538 | O | LYS | A | 4 | -14.903 | 38.415 | 20.831 |
| ATOM | 539 | CB | LYS | A | 4 | -15.926 | 41.244 | 19.245 |
| ATOM | 540 | CG | LYS | A | 4 | -15.802 | 42.751 | 19.355 |
| ATOM | 541 | CD | LYS | A | 4 | -16.292 | 43.433 | 18.083 |
| ATOM | 542 | CE | LYS | A | 4 | -16.162 | 44.943 | 18.177 |
| ATOM | 543 | NZ | LYS | A | 4 | -16.825 | 45.628 | 17.019 |
| ATOM | 544 | N | ALA | A | 5 | -16.85 | 38.393 | 19.759 |
| ATOM | 545 | CA | ALA | A | 5 | -16.811 | 36.955 | 19.507 |
| ATOM | 546 | C | ALA | A | 5 | -15.772 | 36.771 | 18.416 |
| ATOM | 547 | O | ALA | A | 5 | -15.727 | 37.534 | 17.455 |
| ATOM | 548 | CB | ALA | A | 5 | -18.168 | 36.419 | 19.043 |

(continued)

| | | | | | | | | |
|---|---|---|---|---|---|---|---|---|
| ATOM | 549 | N | CYS | A | 6 | -14.935 | 35.756 | 18.562 |
| ATOM | 550 | CA | CYS | A | 6 | -13.887 | 35.518 | 17.584 |
| ATOM | 551 | C | CYS | A | 6 | -14.347 | 34.765 | 16.338 |
| ATOM | 552 | O | CYS | A | 6 | -15.327 | 34.018 | 16.368 |
| ATOM | 553 | CB | CYS | A | 6 | -12.743 | 34.768 | 18.241 |
| ATOM | 554 | SG | CYS | A | 6 | -11.198 | 34.959 | 17.353 |
| ATOM | 555 | N | CYS | A | 7 | -13.623 | 34.973 | 15.243 |
| ATOM | 556 | CA | CYS | A | 7 | -13.931 | 34.328 | 13.969 |
| ATOM | 557 | C | CYS | A | 7 | -13.091 | 33.071 | 13.798 |
| ATOM | 558 | O | CYS | A | 7 | -11.961 | 33.123 | 13.302 |
| ATOM | 559 | CB | CYS | A | 7 | -13.653 | 35.290 | 12.824 |
| ATOM | 560 | SG | CYS | A | 7 | -13.930 | 34.633 | 11.154 |
| ATOM | 561 | N | VAL | A | 8 | -13.654 | 31.941 | 14.209 |
| ATOM | 562 | CA | VAL | A | 8 | -12.949 | 30.684 | 14.110 |
| ATOM | 563 | C | VAL | A | 8 | -13.653 | 29.733 | 13.157 |
| ATOM | 564 | O | VAL | A | 8 | -14.759 | 30.016 | 12.687 |
| ATOM | 565 | CB | VAL | A | 8 | -12.814 | 30.038 | 15.492 |
| ATOM | 566 | CG1 | VAL | A | 8 | -11.807 | 30.825 | 16.337 |
| ATOM | 567 | CG2 | VAL | A | 8 | -14.161 | 30.006 | 16.170 |
| ATOM | 568 | N | PRO | A | 9 | -13.003 | 28.601 | 12.828 |
| ATOM | 569 | CA | PRO | A | 9 | -13.593 | 27.615 | 11.918 |
| ATOM | 570 | C | PRO | A | 9 | -14.726 | 26.886 | 12.631 |
| ATOM | 571 | O | PRO | A | 9 | -14.581 | 26.476 | 13.780 |
| ATOM | 572 | CB | PRO | A | 9 | -12.423 | 26.676 | 11.601 |
| ATOM | 573 | CG | PRO | A | 9 | -11.204 | 27.487 | 11.925 |
| ATOM | 574 | CD | PRO | A | 9 | -11.620 | 28.226 | 13.163 |
| ATOM | 575 | N | THR | A | 10 | -15.847 | 26.721 | 11.942 |
| ATOM | 576 | CA | THR | A | 10 | -16.999 | 26.060 | 12.527 |
| ATOM | 577 | C | THR | A | 10 | -17.334 | 24.767 | 11.804 |
| ATOM | 578 | O | THR | A | 10 | -18.097 | 23.943 | 12.303 |
| ATOM | 579 | CB | THR | A | 10 | -18.211 | 27.010 | 12.523 |
| ATOM | 580 | OG1 | THR | A | 10 | -18.491 | 27.445 | 11.185 |
| ATOM | 581 | CG2 | THR | A | 10 | -17.902 | 28.230 | 13.375 |
| ATOM | 582 | N | GLU | A | 11 | -16.750 | 24.586 | 10.627 |
| ATOM | 583 | CA | GLU | A | 11 | -16.980 | 23.377 | 9.848 |
| ATOM | 584 | C | GLU | A | 11 | -15.643 | 22.935 | 9.246 |
| ATOM | 585 | O | GLU | A | 11 | -15.029 | 23.666 | 8.464 |
| ATOM | 586 | CB | GLU | A | 11 | -17.981 | 23.624 | 8.715 |
| ATOM | 587 | CG | GLU | A | 11 | -19.421 | 23.807 | 9.163 |

(continued)

| | | | | | | | | |
|---|---|---|---|---|---|---|---|---|
| ATOM | 588 | CD | GLU | A | 11 | -19.686 | 25.166 | 9.770 |
| ATOM | 589 | OE1 | GLU | A | 11 | -19.478 | 26.175 | 9.073 |
| ATOM | 590 | OE2 | GLU | A | 11 | -20.111 | 25.227 | 10.939 |
| ATOM | 591 | N | LEU | A | 12 | -15.183 | 21.749 | 9.622 |
| ATOM | 592 | CA | LEU | A | 12 | -13.923 | 21.254 | 9.104 |
| ATOM | 593 | C | LEU | A | 12 | -14.062 | 19.912 | 8.386 |
| ATOM | 594 | O | LEU | A | 12 | -15.136 | 19.299 | 8.359 |
| ATOM | 595 | CB | LEU | A | 12 | -12.893 | 21.144 | 10.230 |
| ATOM | 596 | CG | LEU | A | 12 | -12.660 | 22.422 | 11.054 |
| ATOM | 597 | CD1 | LEU | A | 12 | -13.475 | 22.350 | 12.337 |
| ATOM | 598 | CD2 | LEU | A | 12 | -11.181 | 22.586 | 11.399 |
| ATOM | 599 | N | SER | A | 13 | -12.971 | 19.476 | 7.771 |
| ATOM | 600 | CA | SER | A | 13 | -12.964 | 18.218 | 7.046 |
| ATOM | 601 | C | SER | A | 13 | -11.568 | 17.628 | 7.164 |
| ATOM | 602 | O | SER | A | 13 | -10.613 | 18.320 | 7.550 |
| ATOM | 603 | CB | SER | A | 13 | -13.346 | 18.435 | 5.578 |
| ATOM | 604 | OG | SER | A | 13 | -12.404 | 19.261 | 4.923 |
| ATOM | 605 | N | ALA | A | 13 | -11.449 | 16.352 | 6.818 |
| ATOM | 606 | CA | ALA | A | 13 | -10.179 | 15.665 | 6.949 |
| ATOM | 607 | C | ALA | A | 13 | -9.421 | 15.471 | 5.652 |
| ATOM | 608 | O | ALA | A | 13 | -9.941 | 15.720 | 4.563 |
| ATOM | 609 | CB | ALA | A | 13 | -10.413 | 14.306 | 7.626 |
| ATOM | 610 | N | ILE | A | 14 | -8.171 | 15.046 | 5.783 |
| ATOM | 611 | CA | ILE | A | 14 | -7.343 | 14.746 | 4.623 |
| ATOM | 612 | C | ILE | A | 14 | -6.475 | 13.559 | 5.004 |
| ATOM | 613 | O | ILE | A | 14 | -6.212 | 13.316 | 6.183 |
| ATOM | 614 | CB | ILE | A | 14 | -6.401 | 15.916 | 4.183 |
| ATOM | 615 | CG1 | ILE | A | 14 | -5.284 | 16.106 | 5.200 |
| ATOM | 616 | CG2 | ILE | A | 14 | -7.188 | 17.211 | 3.982 |
| ATOM | 617 | CD1 | ILE | A | 14 | -4.173 | 16.973 | 4.696 |
| ATOM | 618 | N | SER | A | 15 | -6.045 | 12.806 | 3.999 |
| ATOM | 619 | CA | SER | A | 15 | -5.187 | 11.662 | 4.242 |
| ATOM | 620 | C | SER | A | 15 | -3.740 | 12.089 | 4.217 |
| ATOM | 621 | O | SER | A | 15 | -3.360 | 13.020 | 3.508 |
| ATOM | 622 | CB | SER | A | 15 | -5.416 | 10.584 | 3.185 |
| ATOM | 623 | OG | SER | A | 15 | -6.667 | 9.971 | 3.401 |
| ATOM | 624 | N | MET | A | 16 | -2.933 | 11.409 | 5.012 |
| ATOM | 625 | CA | MET | A | 16 | -1.518 | 11.700 | 5.047 |
| ATOM | 626 | C | MET | A | 16 | -0.778 | 10.414 | 5.244 |

(continued)

| | | | | | | | | |
|---|---|---|---|---|---|---|---|---|
| ATOM | 627 | O | MET | A | 16 | -1.137 | 9.594 | 6.078 |
| ATOM | 628 | CB | MET | A | 16 | -1.170 | 12.694 | 6.164 |
| ATOM | 629 | CG | MET | A | 16 | -1.848 | 14.042 | 5.974 |
| ATOM | 630 | SD | MET | A | 16 | -1.017 | 15.431 | 6.760 |
| ATOM | 631 | CE | MET | A | 16 | -0.799 | 14.823 | 8.475 |
| ATOM | 632 | N | LEU | A | 17 | 0.238 | 10.231 | 4.426 |
| ATOM | 633 | CA | LEU | A | 17 | 1.077 | 9.065 | 4.508 |
| ATOM | 634 | C | LEU | A | 17 | 2.289 | 9.610 | 5.264 |
| ATOM | 635 | O | LEU | A | 17 | 2.939 | 10.565 | 4.818 |
| ATOM | 636 | CB | LEU | A | 17 | 1.461 | 8.608 | 3.100 |
| ATOM | 637 | CG | LEU | A | 17 | 2.324 | 7.355 | 2.955 |
| ATOM | 638 | CD1 | LEU | A | 17 | 1.553 | 6.145 | 3.445 |
| ATOM | 639 | CD2 | LEU | A | 17 | 2.723 | 7.190 | 1.492 |
| ATOM | 640 | N | TYR | A | 18 | 2.581 | 9.029 | 6.418 |
| ATOM | 641 | CA | TYR | A | 18 | 3.706 | 9.501 | 7.196 |
| ATOM | 642 | C | TYR | A | 18 | 4.434 | 8.333 | 7.835 |
| ATOM | 643 | O | TYR | A | 18 | 4.081 | 7.186 | 7.603 |
| ATOM | 644 | CB | TYR | A | 18 | 3.222 | 10.458 | 8.281 |
| ATOM | 645 | CG | TYR | A | 18 | 2.386 | 9.782 | 9.346 |
| ATOM | 646 | CD1 | TYR | A | 18 | 1.029 | 9.527 | 9.147 |
| ATOM | 647 | CD2 | TYR | A | 18 | 2.961 | 9.379 | 10.550 |
| ATOM | 648 | CE1 | TYR | A | 18 | 0.273 | 8.894 | 10.128 |
| ATOM | 649 | CE2 | TYR | A | 18 | 2.218 | 8.745 | 11.526 |
| ATOM | 650 | CZ | TYR | A | 18 | 0.877 | 8.508 | 11.317 |
| ATOM | 651 | OH | TYR | A | 18 | 0.134 | 7.922 | 12.318 |
| ATOM | 652 | N | LEU | A | 19 | 5.439 | 8.651 | 8.650 |
| ATOM | 653 | CA | LEU | A | 19 | 6.255 | 7.661 | 9.347 |
| ATOM | 654 | C | LEU | A | 19 | 6.210 | 7.946 | 10.847 |
| ATOM | 655 | O | LEU | A | 19 | 6.685 | 8.992 | 11.288 |
| ATOM | 656 | CB | LEU | A | 19 | 7.701 | 7.763 | 8.871 |
| ATOM | 657 | CG | LEU | A | 19 | 7.901 | 7.850 | 7.359 |
| ATOM | 658 | CD1 | LEU | A | 19 | 9.300 | 8.379 | 7.039 |
| ATOM | 659 | CD2 | LEU | A | 19 | 7.669 | 6.482 | 6.748 |
| ATOM | 660 | N | ASP | A | 20 | 5.652 | 7.035 | 11.639 |
| ATOM | 661 | CA | ASP | A | 20 | 5.594 | 7.282 | 13.071 |
| ATOM | 662 | C | ASP | A | 20 | 7.001 | 7.289 | 13.662 |
| ATOM | 663 | O | ASP | A | 20 | 7.985 | 7.185 | 12.932 |
| ATOM | 664 | CB | ASP | A | 20 | 4.714 | 6.244 | 13.786 |
| ATOM | 665 | CG | ASP | A | 20 | 5.292 | 4.837 | 13.752 |

(continued)

| | | | | | | | | |
|---|---|---|---|---|---|---|---|---|
| ATOM | 666 | OD1 | ASP | A | 20 | 6.533 | 4.678 | 13.775 |
| ATOM | 667 | OD2 | ASP | A | 20 | 4.491 | 3.882 | 13.732 |
| ATOM | 668 | N | GLU | A | 21 | 7.089 | 7.413 | 14.981 |
| ATOM | 669 | CA | GLU | A | 21 | 8.375 | 7.451 | 15.669 |
| ATOM | 670 | C | GLU | A | 21 | 9.250 | 6.212 | 15.463 |
| ATOM | 671 | O | GLU | A | 21 | 10.459 | 6.246 | 15.738 |
| ATOM | 672 | CB | GLU | A | 21 | 8.164 | 7.699 | 17.171 |
| ATOM | 673 | CG | GLU | A | 21 | 7.220 | 6.731 | 17.868 |
| ATOM | 674 | CD | GLU | A | 21 | 5.828 | 6.726 | 17.257 |
| ATOM | 675 | OE1 | GLU | A | 21 | 5.347 | 7.810 | 16.856 |
| ATOM | 676 | OE2 | GLU | A | 21 | 5.210 | 5.640 | 17.185 |
| ATOM | 677 | N | ASN | A | 22 | 8.646 | 5.133 | 14.966 |
| ATOM | 678 | CA | ASN | A | 22 | 9.377 | 3.889 | 14.727 |
| ATOM | 679 | C | ASN | A | 22 | 9.682 | 3.647 | 13.249 |
| ATOM | 680 | O | ASN | A | 22 | 10.095 | 2.554 | 12.853 |
| ATOM | 681 | CB | ASN | A | 22 | 8.591 | 2.707 | 15.299 |
| ATOM | 682 | CG | ASN | A | 22 | 8.384 | 2.827 | 16.794 |
| ATOM | 683 | OD1 | ASN | A | 22 | 9.349 | 2.930 | 17.560 |
| ATOM | 684 | ND2 | ASN | A | 22 | 7.125 | 2.819 | 17.221 |
| ATOM | 685 | N | GLU | A | 23 | 9.485 | 4.672 | 12.435 |
| ATOM | 686 | CA | GLU | A | 23 | 9.749 | 4.558 | 11.015 |
| ATOM | 687 | C | GLU | A | 23 | 8.790 | 3.603 | 10.299 |
| ATOM | 688 | O | GLU | A | 23 | 9.145 | 2.996 | 9.292 |
| ATOM | 689 | CB | GLU | A | 23 | 11.203 | 4.129 | 10.784 |
| ATOM | 690 | CG | GLU | A | 23 | 12.232 | 5.210 | 11.082 |
| ATOM | 691 | CD | GLU | A | 23 | 12.072 | 6.410 | 10.166 |
| ATOM | 692 | OE1 | GLU | A | 23 | 12.108 | 6.218 | 8.928 |
| ATOM | 693 | OE2 | GLU | A | 23 | 11.907 | 7.541 | 10.677 |
| ATOM | 694 | N | LYS | A | 24 | 7.580 | 3.459 | 10.826 |
| ATOM | 695 | CA | LYS | A | 24 | 6.583 | 2.624 | 10.175 |
| ATOM | 696 | C | LYS | A | 24 | 5.815 | 3.540 | 9.228 |
| ATOM | 697 | O | LYS | A | 24 | 5.553 | 4.692 | 9.552 |
| ATOM | 698 | CB | LYS | A | 24 | 5.602 | 2.039 | 11.182 |
| ATOM | 699 | CG | LYS | A | 24 | 6.163 | 0.963 | 12.058 |
| ATOM | 700 | CD | LYS | A | 24 | 5.068 | 0.387 | 12.947 |
| ATOM | 701 | CE | LYS | A | 24 | 5.648 | -0.511 | 14.031 |
| ATOM | 702 | NZ | LYS | A | 24 | 4.577 | -1.091 | 14.886 |
| ATOM | 703 | N | VAL | A | 25 | 5.455 | 3.037 | 8.057 |
| ATOM | 704 | CA | VAL | A | 25 | 4.713 | 3.849 | 7.116 |

(continued)

| | | | | | | | | |
|---|---|---|---|---|---|---|---|---|
| ATOM | 705 | C | VAL | A | 25 | 3.237 | 3.737 | 7.444 |
| ATOM | 706 | O | VAL | A | 25 | 2.657 | 2.659 | 7.383 |
| ATOM | 707 | CB | VAL | A | 25 | 4.969 | 3.396 | 5.681 |
| ATOM | 708 | CG1 | VAL | A | 25 | 4.250 | 4.317 | 4.708 |
| ATOM | 709 | CG2 | VAL | A | 25 | 6.462 | 3.393 | 5.422 |
| ATOM | 710 | N | VAL | A | 25 | 2.637 | 4.864 | 7.800 |
| ATOM | 711 | CA | VAL | A | 25 | 1.231 | 4.916 | 8.170 |
| ATOM | 712 | C | VAL | A | 25 | 0.418 | 5.778 | 7.213 |
| ATOM | 713 | O | VAL | A | 25 | 0.871 | 6.841 | 6.774 |
| ATOM | 714 | CB | VAL | A | 25 | 1.062 | 5.531 | 9.577 |
| ATOM | 715 | CG1 | VAL | A | 25 | -0.341 | 5.253 | 10.115 |
| ATOM | 716 | CG2 | VAL | A | 25 | 2.128 | 5.006 | 10.506 |
| ATOM | 717 | N | LEU | A | 26 | -0.779 | 5.306 | 6.887 |
| ATOM | 718 | CA | LEU | A | 26 | -1.706 | 6.048 | 6.040 |
| ATOM | 719 | C | LEU | A | 26 | -2.862 | 6.358 | 6.992 |
| ATOM | 720 | O | LEU | A | 26 | -3.679 | 5.497 | 7.297 |
| ATOM | 721 | CB | LEU | A | 26 | -2.201 | 5.189 | 4.876 |
| ATOM | 722 | CG | LEU | A | 26 | -3.204 | 5.898 | 3.952 |
| ATOM | 723 | CD1 | LEU | A | 26 | -2.519 | 7.077 | 3.262 |
| ATOM | 724 | CD2 | LEU | A | 26 | -3.755 | 4.925 | 2.929 |
| ATOM | 725 | N | LYS | A | 27 | -2.910 | 7.584 | 7.489 |
| ATOM | 726 | CA | LYS | A | 27 | -3.952 | 7.965 | 8.430 |
| ATOM | 727 | C | LYS | A | 27 | -4.831 | 9.082 | 7.881 |
| ATOM | 728 | O | LYS | A | 27 | -4.374 | 9.924 | 7.103 |
| ATOM | 729 | CB | LYS | A | 27 | -3.305 | 8.409 | 9.738 |
| ATOM | 730 | CG | LYS | A | 27 | -4.265 | 8.720 | 10.859 |
| ATOM | 731 | CD | LYS | A | 27 | -3.509 | 9.382 | 11.994 |
| ATOM | 732 | CE | LYS | A | 27 | -4.397 | 9.654 | 13.186 |
| ATOM | 733 | NZ | LYS | A | 27 | -4.839 | 8.377 | 13.816 |
| ATOM | 734 | N | ASN | A | 28 | -6.098 | 9.078 | 8.279 |
| ATOM | 735 | CA | ASN | A | 28 | -7.045 | 10.095 | 7.843 |
| ATOM | 736 | C | ASN | A | 28 | -7.147 | 11.103 | 8.979 |
| ATOM | 737 | O | ASN | A | 28 | -7.815 | 10.842 | 9.969 |
| ATOM | 738 | CB | ASN | A | 28 | -8.408 | 9.455 | 7.560 |
| ATOM | 739 | CG | ASN | A | 28 | -9.518 | 10.485 | 7.364 |
| ATOM | 740 | OD1 | ASN | A | 28 | -9.360 | 11.444 | 6.597 |
| ATOM | 741 | ND2 | ASN | A | 28 | -10.653 | 10.288 | 8.050 |
| ATOM | 742 | N | TYR | A | 29 | -6.473 | 12.241 | 8.837 |
| ATOM | 743 | CA | TYR | A | 29 | -6.470 | 13.278 | 9.869 |

(continued)

| | | | | | | | | |
|---|---|---|---|---|---|---|---|---|
| ATOM | 744 | C | TYR | A | 29 | -7.680 | 14.204 | 9.836 |
| ATOM | 745 | O | TYR | A | 29 | -7.928 | 14.870 | 8.835 |
| ATOM | 746 | CB | TYR | A | 29 | -5.197 | 14.130 | 9.768 |
| ATOM | 747 | CG | TYR | A | 29 | -3.913 | 13.420 | 10.155 |
| ATOM | 748 | CD1 | TYR | A | 29 | -3.378 | 12.394 | 9.363 |
| ATOM | 749 | CD2 | TYR | A | 29 | -3.220 | 13.792 | 11.306 |
| ATOM | 750 | CE1 | TYR | A | 29 | -2.181 | 11.765 | 9.713 |
| ATOM | 751 | CE2 | TYR | A | 29 | -2.032 | 13.171 | 11.665 |
| ATOM | 752 | CZ | TYR | A | 29 | -1.516 | 12.167 | 10.869 |
| ATOM | 753 | OH | TYR | A | 29 | -0.319 | 11.603 | 11.231 |
| ATOM | 754 | N | GLN | A | 30 | -8.408 | 14.254 | 10.950 |
| ATOM | 755 | CA | GLN | A | 30 | -9.606 | 15.088 | 11.089 |
| ATOM | 756 | C | GLN | A | 30 | -9.287 | 16.541 | 11.431 |
| ATOM | 757 | O | GLN | A | 30 | -8.213 | 16.847 | 11.925 |
| ATOM | 758 | CB | GLN | A | 30 | -10.500 | 14.541 | 12.202 |
| ATOM | 759 | CG | GLN | A | 30 | -10.807 | 13.074 | 12.104 |
| ATOM | 760 | CD | GLN | A | 30 | -11.744 | 12.749 | 10.969 |
| ATOM | 761 | OE1 | GLN | A | 30 | -11.900 | 11.582 | 10.602 |
| ATOM | 762 | NE2 | GLN | A | 30 | -12.385 | 13.773 | 10.405 |
| ATOM | 763 | N | ASP | A | 31 | -10.238 | 17.430 | 11.173 |
| ATOM | 764 | CA | ASP | A | 31 | -10.085 | 18.851 | 11.495 |
| ATOM | 765 | C | ASP | A | 31 | -8.867 | 19.494 | 10.842 |
| ATOM | 766 | O | ASP | A | 31 | -8.195 | 20.335 | 11.442 |
| ATOM | 767 | CB | ASP | A | 31 | -10.000 | 19.025 | 13.019 |
| ATOM | 768 | CG | ASP | A | 31 | -11.247 | 18.520 | 13.742 |
| ATOM | 769 | OD1 | ASP | A | 31 | -12.349 | 18.587 | 13.155 |
| ATOM | 770 | OD2 | ASP | A | 31 | -11.130 | 18.069 | 14.908 |
| ATOM | 771 | N | MET | A | 32 | -8.582 | 19.113 | 9.606 |
| ATOM | 772 | CA | MET | A | 32 | -7.426 | 19.662 | 8.918 |
| ATOM | 773 | C | MET | A | 32 | -7.780 | 20.808 | 7.987 |
| ATOM | 774 | O | MET | A | 32 | -7.021 | 21.768 | 7.855 |
| ATOM | 775 | CB | MET | A | 32 | -6.741 | 18.559 | 8.125 |
| ATOM | 776 | CG | MET | A | 32 | -6.037 | 17.540 | 8.980 |
| ATOM | 777 | SD | MET | A | 32 | -4.559 | 18.231 | 9.714 |
| ATOM | 778 | CE | MET | A | 32 | -3.406 | 18.041 | 8.318 |
| ATOM | 779 | N | VAL | A | 33 | -8.942 | 20.707 | 7.351 |
| ATOM | 780 | CA | VAL | A | 33 | -9.381 | 21.719 | 6.401 |
| ATOM | 781 | C | VAL | A | 33 | -10.579 | 22.536 | 6.858 |
| ATOM | 782 | O | VAL | A | 33 | -11.586 | 21.988 | 7.292 |

(continued)

| | | | | | | | | |
|---|---|---|---|---|---|---|---|---|
| ATOM | 783 | CB | VAL | A | 33 | -9.701 | 21.056 | 5.027 |
| ATOM | 784 | CG1 | VAL | A | 33 | -10.388 | 22.040 | 4.087 |
| ATOM | 785 | CG2 | VAL | A | 33 | -8.423 | 20.561 | 4.412 |
| ATOM | 786 | N | VAL | A | 34 | -10.465 | 23.855 | 6.739 |
| ATOM | 787 | CA | VAL | A | 34 | -11.553 | 24.745 | 7.113 |
| ATOM | 788 | C | VAL | A | 34 | -12.610 | 24.835 | 6.016 |
| ATOM | 789 | O | VAL | A | 34 | -12.373 | 25.403 | 4.950 |
| ATOM | 790 | CB | VAL | A | 34 | -11.057 | 26.176 | 7.407 |
| ATOM | 791 | CG1 | VAL | A | 34 | -12.240 | 27.097 | 7.601 |
| ATOM | 792 | CG2 | VAL | A | 34 | -10.222 | 26.186 | 8.655 |
| ATOM | 793 | N | GLU | A | 35 | -13.779 | 24.266 | 6.282 |
| ATOM | 794 | CA | GLU | A | 35 | -14.870 | 24.311 | 5.321 |
| ATOM | 795 | C | GLU | A | 35 | -15.702 | 25.565 | 5.572 |
| ATOM | 796 | O | GLU | A | 35 | -16.011 | 26.303 | 4.645 |
| ATOM | 797 | CB | GLU | A | 35 | -15.733 | 23.054 | 5.447 |
| ATOM | 798 | CG | GLU | A | 35 | -14.969 | 21.782 | 5.128 |
| ATOM | 799 | CD | GLU | A | 35 | -14.515 | 21.732 | 3.676 |
| ATOM | 800 | OE1 | GLU | A | 35 | -15.036 | 22.519 | 2.858 |
| ATOM | 801 | OE2 | GLU | A | 35 | -13.643 | 20.902 | 3.344 |
| ATOM | 802 | N | GLY | A | 36 | -16.053 | 25.811 | 6.829 |
| ATOM | 803 | CA | GLY | A | 36 | -16.834 | 26.990 | 7.152 |
| ATOM | 804 | C | GLY | A | 36 | -16.322 | 27.785 | 8.341 |
| ATOM | 805 | O | GLY | A | 36 | -15.671 | 27.249 | 9.237 |
| ATOM | 806 | N | CYS | A | 37 | -16.616 | 29.077 | 8.360 |
| ATOM | 807 | CA | CYS | A | 37 | -16.177 | 29.917 | 9.466 |
| ATOM | 808 | C | CYS | A | 37 | -17.347 | 30.502 | 10.249 |
| ATOM | 809 | O | CYS | A | 37 | -18.467 | 30.628 | 9.741 |
| ATOM | 810 | CB | CYS | A | 37 | -15.301 | 31.058 | 8.967 |
| ATOM | 811 | SG | CYS | A | 37 | -13.785 | 30.558 | 8.098 |
| ATOM | 812 | N | GLY | A | 38 | -17.079 | 30.867 | 11.494 |
| ATOM | 813 | CA | GLY | A | 38 | -18.129 | 31.430 | 12.309 |
| ATOM | 814 | C | GLY | A | 38 | -17.622 | 32.176 | 13.518 |
| ATOM | 815 | O | GLY | A | 38 | -16.425 | 32.235 | 13.791 |
| ATOM | 816 | N | CYS | A | 39 | -18.564 | 32.753 | 14.245 |
| ATOM | 817 | CA | CYS | A | 39 | -18.253 | 33.505 | 15.439 |
| ATOM | 818 | C | CYS | A | 39 | -18.543 | 32.670 | 16.665 |
| ATOM | 819 | O | CYS | A | 39 | -19.620 | 32.084 | 16.794 |
| ATOM | 820 | CB | CYS | A | 39 | -19.068 | 34.779 | 15.442 |
| ATOM | 821 | SG | CYS | A | 39 | -18.539 | 35.799 | 14.053 |

9. A pharmaceutical association according to any one of claims 1 to 5, wherein said growth factor receptor-binding compound is a cyclic peptide with between 15 and 60 amino acids, having a growth factor receptor-binding capability, or a cyclic peptidomimetic comprising between 15 and 60 amino acids, having a growth factor receptor-binding capability.

10. A pharmaceutical association according to any one of claims 1 to 5 and 9, wherein said growth factor receptor-binding compound is a cyclic peptide or a cyclic peptidomimetic, having a molecular weight comprised between 1,500 and 7,000 Da.

11. A pharmaceutical association according to any one of claims 1 to 10, wherein said growth factor receptor-binding compound is selected from the group consisting of any one of peptides of SEQ ID NO: 14, SEQ ID NO: 41, SEQ ID NO: 49, SEQ ID NO: 53, SEQ ID NO: 66, SEQ ID NO: 67, SEQ ID NO: 126, SEQ ID NO: 144, SEQ ID NO: 145, SEQ ID NO: 151, SEQ ID NO: 157, SEQ ID NO: 158, SEQ ID NO: 169, SEQ ID NO: 257 to SEQ ID NO: 286, SEQ ID NO: 769 to SEQ ID NO: 798, SEQ ID NO: 2231, SEQ ID NO: 2232, SEQ ID NO: 2233, SEQ ID NO: 2240, SEQ ID NO: 2252, SEQ ID NO: 2253, SEQ ID NO: 5601, SEQ ID NO: 5603, SEQ ID NO: 5605, SEQ ID NO: 5608, SEQ ID NO: 5618, SEQ ID NO: 5625, SEQ ID NO: 5646, SEQ ID NO: 5647, SEQ ID NO: 5655, SEQ ID NO: 5673, SEQ ID NO: 5674, SEQ ID NO: 5675, SEQ ID NO: 5676, SEQ ID NO: 5678, SEQ ID NO: 5680, SEQ ID NO: 5687, SEQ ID NO: 5694, SEQ ID NO: 5699, SEQ ID NO: 5700, SEQ ID NO: 5703, SEQ ID NO: 5705, SEQ ID NO: 5707, SEQ ID NO: 5711, SEQ ID NO: 5715, SEQ ID NO: 5725, SEQ ID NO: 5729, SEQ ID NO: 5732, SEQ ID NO: 5744, SEQ ID NO: 5753, SEQ ID NO: 5757, SEQ ID NO: 5760, SEQ ID NO: 5774, SEQ ID NO: 5777, SEQ ID NO: 5781, SEQ ID NO: 5784, SEQ ID NO: 5785, SEQ ID NO: 5805, SEQ ID NO: 5810, SEQ ID NO: 5821, SEQ ID NO: 5824, SEQ ID NO: 5831, SEQ ID NO: 5832, SEQ ID NO: 5833, SEQ ID NO: 5835, SEQ ID NO: 5836, SEQ ID NO: 5842, SEQ ID NO: 5844, SEQ ID NO: 5848, SEQ ID NO: 5857, SEQ ID NO: 5865, SEQ ID NO: 5866, SEQ ID NO: 5868, SEQ ID NO: 5874, SEQ ID NO: 5880, SEQ ID NO: 5888, SEQ ID NO: 5890, SEQ ID NO: 5892, SEQ ID NO: 5893, SEQ ID NO: 5905, SEQ ID NO: 5912, SEQ ID NO: 5917, SEQ ID NO: 5931, SEQ ID NO: 5934, SEQ ID NO: 5935, SEQ ID NO: 5936, SEQ ID NO: 5937, SEQ ID NO: 5944, SEQ ID NO: 5959, SEQ ID NO: 5960, SEQ ID NO: 5966, SEQ ID NO: 5973, SEQ ID NO: 5982, SEQ ID NO: 5998, SEQ ID NO: 5999, SEQ ID NO: 6004, SEQ ID NO: 6008, SEQ ID NO: 6014, SEQ ID NO: 6017, SEQ ID NO: 6026, SEQ ID NO: 6032, SEQ ID NO: 6035, SEQ ID NO: 6036, SEQ ID NO: 6037, SEQ ID NO: 6043, SEQ ID NO: 6048, SEQ ID NO: 6049, SEQ ID NO: 6050, SEQ ID NO: 6051, SEQ ID NO: 6058, SEQ ID NO: 6086, SEQ ID NO: 6087, SEQ ID NO: 6088, SEQ ID NO: 6099, SEQ ID NO: 6100, SEQ ID NO: 6102, SEQ ID NO: 6105, SEQ ID NO: 6119, SEQ ID NO: 6120, SEQ ID NO: 6121, SEQ ID NO: 6133, SEQ ID NO: 6134, SEQ ID NO: 6136, SEQ ID NO: 6137, SEQ ID NO: 6139, SEQ ID NO: 6141, SEQ ID NO: 6149, SEQ ID NO: 6152, SEQ ID NO: 6154, SEQ ID NO: 6163, SEQ ID NO: 6183, SEQ ID NO: 6193, SEQ ID NO: 6199, SEQ ID NO: 6208, SEQ ID NO: 6215, SEQ ID NO: 6216, SEQ ID NO: 6226, SEQ ID NO: 6243, SEQ ID NO: 6253, SEQ ID NO: 6256, SEQ ID NO: 6259, SEQ ID NO: 6270, SEQ ID NO: 6271, SEQ ID NO: 6274, SEQ ID NO: 6276, SEQ ID NO: 6280, SEQ ID NO: 6283, SEQ ID NO: 6284, SEQ ID NO: 6290, SEQ ID NO: 6295, SEQ ID NO: 6297, SEQ ID NO: 6313, SEQ ID NO: 6325, SEQ ID NO: 6328, SEQ ID NO: 6330, SEQ ID NO: 6336, SEQ ID NO: 6355, SEQ ID NO: 6356, SEQ ID NO: 6357, SEQ ID NO: 6365, SEQ ID NO: 6366, SEQ ID NO: 6368, SEQ ID NO: 6372, SEQ ID NO: 6373, SEQ ID NO: 6377, SEQ ID NO: 6378, SEQ ID NO: 6384, SEQ ID NO: 6390, SEQ ID NO: 6394, SEQ ID NO: 6395, SEQ ID NO: 6404, SEQ ID NO: 6422, SEQ ID NO: 6428, SEQ ID NO: 6439, SEQ ID NO: 6443, SEQ ID NO: 6445, SEQ ID NO: 6454, SEQ ID NO: 6473, SEQ ID NO: 6476, SEQ ID NO: 6478, SEQ ID NO: 6481, SEQ ID NO: 6490, SEQ ID NO: 6500, SEQ ID NO: 6501, SEQ ID NO: 6514, SEQ ID NO: 6521, SEQ ID NO: 6523, SEQ ID NO: 6525, SEQ ID NO: 6537, SEQ ID NO: 6550, SEQ ID NO: 6557, SEQ ID NO: 6561, SEQ ID NO: 6568, SEQ ID NO: 6570, SEQ ID NO: 6576, SEQ ID NO: 6585, SEQ ID NO: 6586, SEQ ID NO: 6594, SEQ ID NO: 6596, SEQ ID NO: 6601, SEQ ID NO: 6602, SEQ ID NO: 6603, SEQ ID NO: 6607, SEQ ID NO: 6611, SEQ ID NO: 6616, SEQ ID NO: 6619, SEQ ID NO: 6633, SEQ ID NO: 6634, SEQ ID NO: 6637, SEQ ID NO: 6638, SEQ ID NO: 6639, SEQ ID NO: 6641, SEQ ID NO: 6643, SEQ ID NO: 6647, SEQ ID NO: 6648, SEQ ID NO: 6649, SEQ ID NO: 6657, SEQ ID NO: 6658, SEQ ID NO: 6662, SEQ ID NO: 6663, SEQ ID NO: 6665, SEQ ID NO: 6669, SEQ ID NO: 6674, SEQ ID NO: 6675, SEQ ID NO: 6681, SEQ ID NO: 6683, SEQ ID NO: 6686, SEQ ID NO: 6690, SEQ ID NO: 6697, SEQ ID NO: 6701, SEQ ID NO: 6702, SEQ ID NO: 6714, SEQ ID NO: 6715, SEQ ID NO: 6720, SEQ ID NO: 6726, SEQ ID NO: 6729, SEQ ID NO: 6732, SEQ ID NO: 6733, SEQ ID NO: 6734, SEQ ID NO: 6739, SEQ ID NO: 6741, SEQ ID NO: 6742, SEQ ID NO: 6745, SEQ ID NO: 6749, SEQ ID NO: 6758, SEQ ID NO: 6760, SEQ ID NO: 6764, SEQ ID NO: 6775, SEQ ID NO: 6779, SEQ ID NO: 6784, SEQ ID NO: 6788, SEQ ID NO: 6789, SEQ ID NO: 6790, SEQ ID NO: 6796, SEQ ID NO: 6799, SEQ ID NO: 6803, SEQ ID NO: 6816, SEQ ID NO: 6818, SEQ ID NO: 6820, SEQ ID NO: 6821, SEQ ID NO: 6829, SEQ ID NO: 6830, SEQ ID NO: 6831, SEQ ID NO: 6837, SEQ ID NO: 6838, SEQ ID NO: 6841, SEQ ID NO: 6842, SEQ ID NO: 6843, SEQ ID NO: 6846, SEQ ID NO: 6854, SEQ ID NO: 6857, SEQ ID NO: 6860, SEQ ID NO: 6864, SEQ ID NO: 6866, SEQ ID NO: 6877, SEQ ID NO: 6879, SEQ ID NO: 6888, SEQ ID NO: 6889, SEQ ID NO: 6892, SEQ ID NO: 6896, SEQ ID NO: 6901, SEQ ID NO: 6910, SEQ ID NO: 6913, SEQ

ID NO: 6918, SEQ ID NO: 6919, SEQ ID NO: 6922, SEQ ID NO: 6923, SEQ ID NO: 6924, SEQ ID NO: 6925, SEQ ID NO: 6927, SEQ ID NO: 6935, SEQ ID NO: 6939, SEQ ID NO: 6941, SEQ ID NO: 6942, SEQ ID NO: 6946, SEQ ID NO: 6947, SEQ ID NO: 6951, SEQ ID NO: 6956, SEQ ID NO: 6958, SEQ ID NO: 6965, SEQ ID NO: 6967, SEQ ID NO: 6968, SEQ ID NO: 6971, SEQ ID NO: 6972, SEQ ID NO: 6975, SEQ ID NO: 6982, SEQ ID NO: 6983, SEQ ID NO: 6985, SEQ ID NO: 6989, SEQ ID NO: 6991, SEQ ID NO: 6995, SEQ ID NO: 7000, SEQ ID NO: 7001, SEQ ID NO: 7005, SEQ ID NO: 7010, SEQ ID NO: 7022, SEQ ID NO: 7024, SEQ ID NO: 7027, SEQ ID NO: 7028, SEQ ID NO: 7031, SEQ ID NO: 7037, SEQ ID NO: 7048, SEQ ID NO: 7051, SEQ ID NO: 7052, SEQ ID NO: 7079, SEQ ID NO: 7080, SEQ ID NO: 7083, SEQ ID NO: 7084, SEQ ID NO: 7087, SEQ ID NO: 7093, SEQ ID NO: 7094, SEQ ID NO: 7101, SEQ ID NO: 7102, SEQ ID NO: 7108, SEQ ID NO: 7115, SEQ ID NO: 7122, SEQ ID NO: 7124, SEQ ID NO: 7125, SEQ ID NO: 7126, SEQ ID NO: 7128, SEQ ID NO: 7129, SEQ ID NO: 7133, SEQ ID NO: 7142, SEQ ID NO: 7149, SEQ ID NO: 7151, SEQ ID NO: 7152, SEQ ID NO: 7155, SEQ ID NO: 7157, SEQ ID NO: 7161, SEQ ID NO: 7170, SEQ ID NO: 7171, SEQ ID NO: 7178, SEQ ID NO: 7179, SEQ ID NO: 7184, SEQ ID NO: 7188, SEQ ID NO: 7189, SEQ ID NO: 7197, SEQ ID NO: 7199, SEQ ID NO: 7200, SEQ ID NO: 7210, SEQ ID NO: 7211, SEQ ID NO: 7214, SEQ ID NO: 7216, SEQ ID NO: 7220, SEQ ID NO: 7224, SEQ ID NO: 7227, SEQ ID NO: 7228, SEQ ID NO: 7229, SEQ ID NO: 7230, SEQ ID NO: 7231, SEQ ID NO: 7234, SEQ ID NO: 7235, SEQ ID NO: 7236, SEQ ID NO: 7237, SEQ ID NO: 7239, SEQ ID NO: 7241, SEQ ID NO: 7244, SEQ ID NO: 7245, SEQ ID NO: 7256, SEQ ID NO: 7261, SEQ ID NO: 7273, SEQ ID NO: 7274, SEQ ID NO: 7278, SEQ ID NO: 7281, SEQ ID NO: 7291, SEQ ID NO: 7297, SEQ ID NO: 7298, SEQ ID NO: 7299, SEQ ID NO: 7300, SEQ ID NO: 7301, SEQ ID NO: 7307, SEQ ID NO: 7309, SEQ ID NO: 7310, SEQ ID NO: 7314, SEQ ID NO: 7320, SEQ ID NO: 7324, SEQ ID NO: 7333, SEQ ID NO: 7335, SEQ ID NO: 7340, SEQ ID NO: 7345, SEQ ID NO: 7347, SEQ ID NO: 7352, SEQ ID NO: 7353, SEQ ID NO: 7354, SEQ ID NO: 7362, SEQ ID NO: 7372, SEQ ID NO: 7374, SEQ ID NO: 7378, SEQ ID NO: 7387, SEQ ID NO: 7389, SEQ ID NO: 7391, SEQ ID NO: 7397, SEQ ID NO: 7398, SEQ ID NO: 7401, SEQ ID NO: 7402, SEQ ID NO: 7409, SEQ ID NO: 7411, SEQ ID NO: 7412, SEQ ID NO: 7414, SEQ ID NO: 7417, SEQ ID NO: 7418, SEQ ID NO: 7419, SEQ ID NO: 7430, SEQ ID NO: 7432, SEQ ID NO: 7433, SEQ ID NO: 7438, SEQ ID NO: 7440, SEQ ID NO: 7446, SEQ ID NO: 7450, SEQ ID NO: 7458, SEQ ID NO: 7474, SEQ ID NO: 7476, SEQ ID NO: 7486, SEQ ID NO: 7487, SEQ ID NO: 7495, SEQ ID NO: 7498, SEQ ID NO: 7509, SEQ ID NO: 7511, SEQ ID NO: 7522, SEQ ID NO: 7533, SEQ ID NO: 7537, SEQ ID NO: 7541, SEQ ID NO: 7551, SEQ ID NO: 7553, SEQ ID NO: 7554, SEQ ID NO: 7562, SEQ ID NO: 7565, SEQ ID NO: 7574, SEQ ID NO: 7583, SEQ ID NO: 7588, SEQ ID NO: 7591, SEQ ID NO: 7593, SEQ ID NO: 7598, SEQ ID NO: 7600, SEQ ID NO: 7602, SEQ ID NO: 7608, SEQ ID NO: 7616, SEQ ID NO: 7617, SEQ ID NO: 7620, SEQ ID NO: 7624, SEQ ID NO: 7633, SEQ ID NO: 7635, SEQ ID NO: 7638, SEQ ID NO: 7639, SEQ ID NO: 7646, SEQ ID NO: 7647, SEQ ID NO: 7652, SEQ ID NO: 7655, SEQ ID NO: 7656, SEQ ID NO: 7657, SEQ ID NO: 7659, SEQ ID NO: 7662, SEQ ID NO: 7663, SEQ ID NO: 7665, SEQ ID NO: 7666, SEQ ID NO: 7675, SEQ ID NO: 7677, SEQ ID NO: 7681, SEQ ID NO: 7684, SEQ ID NO: 7689, SEQ ID NO: 7690, SEQ ID NO: 7692, SEQ ID NO: 7694, SEQ ID NO: 7695, SEQ ID NO: 7696, SEQ ID NO: 7712, SEQ ID NO: 7722, SEQ ID NO: 7741, SEQ ID NO: 7751, SEQ ID NO: 7752, SEQ ID NO: 7753, SEQ ID NO: 7754, SEQ ID NO: 7762, SEQ ID NO: 7768, SEQ ID NO: 7776, SEQ ID NO: 7777, SEQ ID NO: 7784, SEQ ID NO: 7785, SEQ ID NO: 7790, SEQ ID NO: 7792, SEQ ID NO: 7793, SEQ ID NO: 7798, SEQ ID NO: 7800, SEQ ID NO: 7807, SEQ ID NO: 7815, SEQ ID NO: 7817, SEQ ID NO: 7819, SEQ ID NO: 7821, SEQ ID NO: 7836, SEQ ID NO: 7838, SEQ ID NO: 7839, SEQ ID NO: 7841, SEQ ID NO: 7853, SEQ ID NO: 7854, SEQ ID NO: 7855, SEQ ID NO: 7857, SEQ ID NO: 7871, SEQ ID NO: 7890, SEQ ID NO: 7895, SEQ ID NO: 7898, SEQ ID NO: 7905, SEQ ID NO: 7908, SEQ ID NO: 7910, SEQ ID NO: 7919, SEQ ID NO: 7928, SEQ ID NO: 7939, SEQ ID NO: 7943, SEQ ID NO: 7957, SEQ ID NO: 7964, SEQ ID NO: 7974, SEQ ID NO: 7976, SEQ ID NO: 7990, SEQ ID NO: 7994, SEQ ID NO: 7995, SEQ ID NO: 7997, SEQ ID NO: 8003, SEQ ID NO: 8006, SEQ ID NO: 8011, SEQ ID NO: 8013, SEQ ID NO: 8019, SEQ ID NO: 8020, SEQ ID NO: 8024, SEQ ID NO: 8025, SEQ ID NO: 8029, SEQ ID NO: 8030, SEQ ID NO: 8036, SEQ ID NO: 8042, SEQ ID NO: 8046, SEQ ID NO: 8049, SEQ ID NO: 8055, SEQ ID NO: 8058, SEQ ID NO: 8059, SEQ ID NO: 8061, SEQ ID NO: 8070, SEQ ID NO: 8072, SEQ ID NO: 8076, SEQ ID NO: 8079, SEQ ID NO: 8091, SEQ ID NO: 8093, SEQ ID NO: 8097, SEQ ID NO: 8098, SEQ ID NO: 8113, SEQ ID NO: 8119, SEQ ID NO: 8120, SEQ ID NO: 8122, SEQ ID NO: 8123, SEQ ID NO: 8125, SEQ ID NO: 8126, SEQ ID NO: 8133, SEQ ID NO: 8136, SEQ ID NO: 8153, SEQ ID NO: 8155, SEQ ID NO: 8164, SEQ ID NO: 8167, SEQ ID NO: 8168, SEQ ID NO: 8172, SEQ ID NO: 8173, SEQ ID NO: 8176, SEQ ID NO: 8177, SEQ ID NO: 8185, SEQ ID NO: 8191, SEQ ID NO: 8196, SEQ ID NO: 8198, SEQ ID NO: 8203, SEQ ID NO: 8204, SEQ ID NO: 8211, SEQ ID NO: 8218, SEQ ID NO: 8222, SEQ ID NO: 8223, SEQ ID NO: 8224, SEQ ID NO: 8227, SEQ ID NO: 8228, SEQ ID NO: 8229, SEQ ID NO: 8230, SEQ ID NO: 8241, SEQ ID NO: 8244, SEQ ID NO: 8258, SEQ ID NO: 8259, SEQ ID NO: 8260, SEQ ID NO: 8268, SEQ ID NO: 8287, SEQ ID NO: 8290, SEQ ID NO: 8293, SEQ ID NO: 8306, SEQ ID NO: 8312, SEQ ID NO: 8313, SEQ ID NO: 8318, SEQ ID NO: 8334, SEQ ID NO: 8335, SEQ ID NO: 8339, SEQ ID NO: 8344, SEQ ID NO: 8349, SEQ ID NO: 8363, SEQ ID NO: 8367, SEQ ID NO: 8373, SEQ ID NO: 8374, SEQ ID NO: 8381, SEQ ID NO: 8389, SEQ ID NO: 8391, SEQ ID NO: 8392, SEQ ID NO: 8395, SEQ ID NO: 8402, SEQ ID NO: 8405, SEQ ID NO: 8410, SEQ ID NO: 8411, SEQ ID NO: 8419, SEQ ID NO: 8420, SEQ ID NO: 8421, SEQ ID NO: 8422, SEQ ID NO: 8425, SEQ ID NO: 8429, SEQ

ID NO: 8440, SEQ ID NO: 8443, SEQ ID NO: 8447, SEQ ID NO: 8449, SEQ ID NO: 8453, SEQ ID NO: 8455, SEQ ID NO: 8458, SEQ ID NO: 8461, SEQ ID NO: 8462, SEQ ID NO: 8463, SEQ ID NO: 8474, SEQ ID NO: 8475, SEQ ID NO: 8482, SEQ ID NO: 8483, SEQ ID NO: 8485, SEQ ID NO: 8486, SEQ ID NO: 8489, SEQ ID NO: 8492, SEQ ID NO: 8494, SEQ ID NO: 8498, SEQ ID NO: 8506, SEQ ID NO: 8511, SEQ ID NO: 8513, SEQ ID NO: 8517, SEQ ID NO: 8523, SEQ ID NO: 8525, SEQ ID NO: 8536, SEQ ID NO: 8542, SEQ ID NO: 8546, SEQ ID NO: 8547, SEQ ID NO: 8556, SEQ ID NO: 8558, SEQ ID NO: 8560, SEQ ID NO: 8567, SEQ ID NO: 8570, SEQ ID NO: 8594, SEQ ID NO: 8602, SEQ ID NO: 8608, SEQ ID NO: 8617, SEQ ID NO: 8621, SEQ ID NO: 8622, SEQ ID NO: 8623, SEQ ID NO: 8626, SEQ ID NO: 8632, SEQ ID NO: 8633, SEQ ID NO: 8639, SEQ ID NO: 8642, SEQ ID NO: 8644, SEQ ID NO: 8647, SEQ ID NO: 8648, SEQ ID NO: 8650, SEQ ID NO: 8651, SEQ ID NO: 8666, SEQ ID NO: 8670, SEQ ID NO: 8675, SEQ ID NO: 8676, SEQ ID NO: 8683, SEQ ID NO: 8685, SEQ ID NO: 8689, SEQ ID NO: 8690, SEQ ID NO: 8691, SEQ ID NO: 8692, SEQ ID NO: 8695, SEQ ID NO: 8696, SEQ ID NO: 8697, SEQ ID NO: 8707, SEQ ID NO: 8710, SEQ ID NO: 8712, SEQ ID NO: 8714, SEQ ID NO: 8715, SEQ ID NO: 8719, SEQ ID NO: 8726, SEQ ID NO: 8727, SEQ ID NO: 8730, SEQ ID NO: 8751, SEQ ID NO: 8752, SEQ ID NO: 8772, SEQ ID NO: 8774, SEQ ID NO: 8778, SEQ ID NO: 8780, SEQ ID NO: 8784, SEQ ID NO: 8788, SEQ ID NO: 8808, SEQ ID NO: 8814, SEQ ID NO: 8815, SEQ ID NO: 8817, SEQ ID NO: 8818, SEQ ID NO: 8820, SEQ ID NO: 8823, SEQ ID NO: 8826, SEQ ID NO: 8828, SEQ ID NO: 8830, SEQ ID NO: 8832, SEQ ID NO: 8833, SEQ ID NO: 8841, SEQ ID NO: 8844, SEQ ID NO: 8847, SEQ ID NO: 8854, SEQ ID NO: 8859, SEQ ID NO: 8863, SEQ ID NO: 8864, SEQ ID NO: 8876, SEQ ID NO: 8883, SEQ ID NO: 8887, SEQ ID NO: 8891, SEQ ID NO: 8892, SEQ ID NO: 11134, SEQ ID NO: 11196, SEQ ID NO: 11216, SEQ ID NO: 11226, SEQ ID NO: 11249, SEQ ID NO: 11264, SEQ ID NO: 11267, SEQ ID NO: 11277, SEQ ID NO: 11324, SEQ ID NO: 11331, SEQ ID NO: 11365, SEQ ID NO: 11376, SEQ ID NO: 11434, SEQ ID NO: 11456, SEQ ID NO: 11465, SEQ ID NO: 11470, SEQ ID NO: 11498, SEQ ID NO: 11507, SEQ ID NO: 11508, SEQ ID NO: 11554, SEQ ID NO: 11629, SEQ ID NO: 11718, SEQ ID NO: 11719, SEQ ID NO: 11743, SEQ ID NO: 11762, SEQ ID NO: 11767, SEQ ID NO: 11772, SEQ ID NO: 11781, SEQ ID NO: 11799, SEQ ID NO: 11823, SEQ ID NO: 11857, SEQ ID NO: 11910, SEQ ID NO: 11914, SEQ ID NO: 11961, SEQ ID NO: 11963, SEQ ID NO: 12002, SEQ ID NO: 12031, SEQ ID NO: 12041, SEQ ID NO: 12054, SEQ ID NO: 12060, SEQ ID NO: 12091, SEQ ID NO: 12103, SEQ ID NO: 12191, SEQ ID NO: 12194, SEQ ID NO: 12226, SEQ ID NO: 12233, SEQ ID NO: 12234, SEQ ID NO: 12240, SEQ ID NO: 12302, SEQ ID NO: 12304, SEQ ID NO: 12312, SEQ ID NO: 12365, SEQ ID NO: 12367, SEQ ID NO: 12386, SEQ ID NO: 12437, SEQ ID NO: 12486, SEQ ID NO: 12509, SEQ ID NO: 12538, SEQ ID NO: 12545, SEQ ID NO: 12644, SEQ ID NO: 12648, SEQ ID NO: 12671, SEQ ID NO: 12674, SEQ ID NO: 12710, SEQ ID NO: 12721, SEQ ID NO: 12750, SEQ ID NO: 12775, SEQ ID NO: 12778, SEQ ID NO: 12819, SEQ ID NO: 12822, SEQ ID NO: 12824, SEQ ID NO: 12855, SEQ ID NO: 12857, SEQ ID NO: 12869, SEQ ID NO: 12872, SEQ ID NO: 12890, SEQ ID NO: 12904, SEQ ID NO: 12927, SEQ ID NO: 12935, SEQ ID NO: 12975, SEQ ID NO: 13016, SEQ ID NO: 13054, SEQ ID NO: 13090, SEQ ID NO: 13156, SEQ ID NO: 13173, SEQ ID NO: 13175, SEQ ID NO: 13198, SEQ ID NO: 13218, SEQ ID NO: 13230, SEQ ID NO: 13237, SEQ ID NO: 13255, SEQ ID NO: 13261, SEQ ID NO: 13274, SEQ ID NO: 13299, SEQ ID NO: 13311, SEQ ID NO: 13339, SEQ ID NO: 18165, SEQ ID NO: 18169, SEQ ID NO: 18170, SEQ ID NO: 18184, SEQ ID NO: 18224, SEQ ID NO: 18226, SEQ ID NO: 18235, SEQ ID NO: 18250, SEQ ID NO: 18254, SEQ ID NO: 18256, SEQ ID NO: 18257, SEQ ID NO: 18274, SEQ ID NO: 18319, SEQ ID NO: 18326, SEQ ID NO: 18327, SEQ ID NO: 18328, SEQ ID NO: 18355, SEQ ID NO: 18363, SEQ ID NO: 18367, SEQ ID NO: 18373, SEQ ID NO: 18385, SEQ ID NO: 18399, SEQ ID NO: 18416, SEQ ID NO: 18445, SEQ ID NO: 18461, SEQ ID NO: 18493, SEQ ID NO: 18494, SEQ ID NO: 18531, SEQ ID NO: 18536, SEQ ID NO: 18559, SEQ ID NO: 18570, SEQ ID NO: 18588, SEQ ID NO: 18615, SEQ ID NO: 18659, SEQ ID NO: 18677, SEQ ID NO: 18692, SEQ ID NO: 18737, SEQ ID NO: 18746, SEQ ID NO: 18760, SEQ ID NO: 18763, SEQ ID NO: 18766, SEQ ID NO: 18770, SEQ ID NO: 18791, SEQ ID NO: 18819, SEQ ID NO: 18826, SEQ ID NO: 18827, SEQ ID NO: 18833, SEQ ID NO: 18836, SEQ ID NO: 18846, SEQ ID NO: 18853, SEQ ID NO: 18854, SEQ ID NO: 18860, SEQ ID NO: 18863, SEQ ID NO: 18865, SEQ ID NO: 18866, SEQ ID NO: 18871, SEQ ID NO: 18890, SEQ ID NO: 18898, SEQ ID NO: 18905, SEQ ID NO: 18920, SEQ ID NO: 18925, SEQ ID NO: 18926, SEQ ID NO: 18931, SEQ ID NO: 18934, SEQ ID NO: 18963, SEQ ID NO: 18971, SEQ ID NO: 18988, SEQ ID NO: 19008, SEQ ID NO: 19011, SEQ ID NO: 19015, SEQ ID NO: 19018, SEQ ID NO: 19082, SEQ ID NO: 19086, SEQ ID NO: 19137, SEQ ID NO: 19147, SEQ ID NO: 19155, SEQ ID NO: 19170, SEQ ID NO: 19187, SEQ ID NO: 19194, SEQ ID NO: 19196, SEQ ID NO: 19209, SEQ ID NO: 19210, SEQ ID NO: 19230, SEQ ID NO: 19233, SEQ ID NO: 19248, SEQ ID NO: 19292, SEQ ID NO: 19298, SEQ ID NO: 19299, SEQ ID NO: 19300, SEQ ID NO: 19305, SEQ ID NO: 19312, SEQ ID NO: 19315, SEQ ID NO: 19360, SEQ ID NO: 19364, SEQ ID NO: 19368, SEQ ID NO: 19379, SEQ ID NO: 19396, SEQ ID NO: 19419, SEQ ID NO: 19420, SEQ ID NO: 19421, SEQ ID NO: 19444, SEQ ID NO: 19454, SEQ ID NO: 19463, SEQ ID NO: 19466, SEQ ID NO: 19501, SEQ ID NO: 19507, SEQ ID NO: 19520, SEQ ID NO: 19522, SEQ ID NO: 19544, SEQ ID NO: 19551, SEQ ID NO: 19552, SEQ ID NO: 19558, SEQ ID NO: 19561, SEQ ID NO: 19567, SEQ ID NO: 19595, SEQ ID NO: 19596, SEQ ID NO: 19598, SEQ ID NO: 19600, SEQ ID NO: 19603, SEQ ID NO: 19605, SEQ ID NO: 19612, SEQ ID NO: 19620, SEQ ID NO: 19621, SEQ ID NO: 19647, SEQ ID NO: 19663, SEQ ID NO: 19665, SEQ ID NO: 19676, SEQ ID NO: 19702, SEQ ID NO: 19708, SEQ ID NO: 19713, SEQ ID NO: 19720, SEQ ID NO: 19725, SEQ ID NO:

19750, SEQ ID NO: 19756, SEQ ID NO: 19765, SEQ ID NO: 19768, SEQ ID NO: 19773, SEQ ID NO: 19775, SEQ ID NO: 19791, SEQ ID NO: 19819, SEQ ID NO: 19827, SEQ ID NO: 19837, SEQ ID NO: 19845, SEQ ID NO: 19852, SEQ ID NO: 19872, SEQ ID NO: 19893, SEQ ID NO: 19907, SEQ ID NO: 19911, SEQ ID NO: 19927, SEQ ID NO: 19931, SEQ ID NO: 19960, SEQ ID NO: 19973, SEQ ID NO: 20003, SEQ ID NO: 20009, SEQ ID NO: 20014, SEQ ID NO: 20025, SEQ ID NO: 20032, SEQ ID NO: 20051, SEQ ID NO: 20058, SEQ ID NO: 20060, SEQ ID NO: 20069, SEQ ID NO: 20089, SEQ ID NO: 20104, SEQ ID NO: 20105, SEQ ID NO: 20107, SEQ ID NO: 20128, SEQ ID NO: 20138, SEQ ID NO: 20145, SEQ ID NO: 20164, SEQ ID NO: 20165, SEQ ID NO: 20167, SEQ ID NO: 20170, SEQ ID NO: 20175, SEQ ID NO: 20188, SEQ ID NO: 20190, SEQ ID NO: 20202, SEQ ID NO: 20205, SEQ ID NO: 20231, SEQ ID NO: 20269, SEQ ID NO: 20279, SEQ ID NO: 20281, SEQ ID NO: 20296, SEQ ID NO: 20305, SEQ ID NO: 20324, SEQ ID NO: 20331, SEQ ID NO: 20334, SEQ ID NO: 20376, SEQ ID NO: 20404, SEQ ID NO: 20416, SEQ ID NO: 20439, SEQ ID NO: 20445, SEQ ID NO: 20446, SEQ ID NO: 31386 to SEQ ID NO: 31560, SEQ ID NO: 33328, SEQ ID NO: 33337, SEQ ID NO: 33350, SEQ ID NO: 33352, SEQ ID NO: 33367, SEQ ID NO: 33405, SEQ ID NO: 33441, SEQ ID NO: 33524, SEQ ID NO: 33557, SEQ ID NO: 33559, SEQ ID NO: 33636, SEQ ID NO: 33645, SEQ ID NO: 33665, SEQ ID NO: 33685, SEQ ID NO: 33706, SEQ ID NO: 33727, SEQ ID NO: 33732, SEQ ID NO: 33733, SEQ ID NO: 33744, SEQ ID NO: 33782, SEQ ID NO: 33799, SEQ ID NO: 33818, SEQ ID NO: 33836, SEQ ID NO: 33854, SEQ ID NO: 33858, SEQ ID NO: 33886, SEQ ID NO: 33901, SEQ ID NO: 33942, SEQ ID NO: 33959, SEQ ID NO: 33986, SEQ ID NO: 33996, SEQ ID NO: 34004, SEQ ID NO: 34042, SEQ ID NO: 34063, SEQ ID NO: 34089, SEQ ID NO: 34095, SEQ ID NO: 34100, SEQ ID NO: 34102, SEQ ID NO: 34142, SEQ ID NO: 34147, SEQ ID NO: 34178, SEQ ID NO: 34202, SEQ ID NO: 34209, SEQ ID NO: 34210, SEQ ID NO: 34217, SEQ ID NO: 34238, SEQ ID NO: 34244, SEQ ID NO: 34252, SEQ ID NO: 34276, SEQ ID NO: 34288, SEQ ID NO: 34342, SEQ ID NO: 34356, SEQ ID NO: 34367, SEQ ID NO: 34413, SEQ ID NO: 34430, SEQ ID NO: 34431, SEQ ID NO: 34453, SEQ ID NO: 34455, SEQ ID NO: 34458, SEQ ID NO: 34467, SEQ ID NO: 34533, SEQ ID NO: 34544, SEQ ID NO: 34558, SEQ ID NO: 34559, SEQ ID NO: 34565, SEQ ID NO: 34575, SEQ ID NO: 34577, SEQ ID NO: 34592, SEQ ID NO: 34604, SEQ ID NO: 34606, SEQ ID NO: 34608, SEQ ID NO: 34613, SEQ ID NO: 34642, SEQ ID NO: 34674, SEQ ID NO: 34685, SEQ ID NO: 34715, SEQ ID NO: 34717, SEQ ID NO: 34730, SEQ ID NO: 34756, SEQ ID NO: 34797, SEQ ID NO: 34805, SEQ ID NO: 34814, SEQ ID NO: 34826, SEQ ID NO: 34833, SEQ ID NO: 34873, SEQ ID NO: 34877, SEQ ID NO: 34925, SEQ ID NO: 34931, SEQ ID NO: 34981, SEQ ID NO: 34987, SEQ ID NO: 35009, SEQ ID NO: 35016, SEQ ID NO: 35037, SEQ ID NO: 35047, SEQ ID NO: 35058, SEQ ID NO: 38203, SEQ ID NO: 38240, SEQ ID NO: 38241, SEQ ID NO: 38264, SEQ ID NO: 38348, SEQ ID NO: 38349, SEQ ID NO: 38350, SEQ ID NO: 38351, SEQ ID NO: 38352, SEQ ID NO: 38353, SEQ ID NO: 38354, SEQ ID NO: 38355, SEQ ID NO: 38356, SEQ ID NO: 38357, SEQ ID NO: 38358, SEQ ID NO: 38359, SEQ ID NO: 38360, SEQ ID NO: 38361, SEQ ID NO: 38362, SEQ ID NO: 38363, SEQ ID NO: 38364, SEQ ID NO: 38365, SEQ ID NO: 38366, SEQ ID NO: 38367, SEQ ID NO: 38368, SEQ ID NO: 43939, SEQ ID NO: 43944, SEQ ID NO: 43945, SEQ ID NO: 43947, SEQ ID NO: 43950, SEQ ID NO: 43951, SEQ ID NO: 43953, SEQ ID NO: 43958, SEQ ID NO: 43963, SEQ ID NO: 43964, SEQ ID NO: 43965, SEQ ID NO: 43966, SEQ ID NO: 43969, SEQ ID NO: 43970, SEQ ID NO: 43983, SEQ ID NO: 43990, SEQ ID NO: 43991, SEQ ID NO: 43996, SEQ ID NO: 43998, SEQ ID NO: 44000, SEQ ID NO: 44002, SEQ ID NO: 44005, SEQ ID NO: 44008, SEQ ID NO: 44011, SEQ ID NO: 44019, SEQ ID NO: 44025, SEQ ID NO: 44029, SEQ ID NO: 44030, SEQ ID NO: 44031, SEQ ID NO: 44034, SEQ ID NO: 44036, SEQ ID NO: 44040, SEQ ID NO: 44043, SEQ ID NO: 44044, SEQ ID NO: 44050, SEQ ID NO: 44053, SEQ ID NO: 44060, SEQ ID NO: 44064, SEQ ID NO: 44065, SEQ ID NO: 44069, SEQ ID NO: 44075, SEQ ID NO: 44076, SEQ ID NO: 44082, SEQ ID NO: 44088, SEQ ID NO: 44092, SEQ ID NO: 44104, SEQ ID NO: 44107, SEQ ID NO: 44108, SEQ ID NO: 44112, SEQ ID NO: 44113, SEQ ID NO: 44125, SEQ ID NO: 44130, SEQ ID NO: 44134, SEQ ID NO: 44135, SEQ ID NO: 44148, SEQ ID NO: 44153, SEQ ID NO: 44155, SEQ ID NO: 44159, SEQ ID NO: 44162, SEQ ID NO: 44164, SEQ ID NO: 44167, SEQ ID NO: 44178, SEQ ID NO: 44189, SEQ ID NO: 44191, SEQ ID NO: 44198, SEQ ID NO: 44215, SEQ ID NO: 44217, SEQ ID NO: 44235, SEQ ID NO: 44240, SEQ ID NO: 44242, SEQ ID NO: 44244, SEQ ID NO: 44245, SEQ ID NO: 44248, SEQ ID NO: 44250, SEQ ID NO: 44251, SEQ ID NO: 44257, SEQ ID NO: 44259, SEQ ID NO: 44262, SEQ ID NO: 44264, SEQ ID NO: 44265, SEQ ID NO: 44269, SEQ ID NO: 44275, SEQ ID NO: 44280, SEQ ID NO: 44297, SEQ ID NO: 44298, SEQ ID NO: 44299, SEQ ID NO: 44303, SEQ ID NO: 44307, SEQ ID NO: 44311, SEQ ID NO: 44314, SEQ ID NO: 44319, SEQ ID NO: 44323, SEQ ID NO: 44326, SEQ ID NO: 44330, SEQ ID NO: 44333, SEQ ID NO: 44336, SEQ ID NO: 44340, SEQ ID NO: 44343, SEQ ID NO: 44345, SEQ ID NO: 44346, SEQ ID NO: 44347, SEQ ID NO: 44350, SEQ ID NO: 44353, SEQ ID NO: 44354, SEQ ID NO: 44359, SEQ ID NO: 44369, SEQ ID NO: 44374, SEQ ID NO: 44379, SEQ ID NO: 44390, SEQ ID NO: 44405, SEQ ID NO: 44407, SEQ ID NO: 44411, SEQ ID NO: 44413, SEQ ID NO: 44416, SEQ ID NO: 44426, SEQ ID NO: 44440, SEQ ID NO: 44442, SEQ ID NO: 44453, SEQ ID NO: 44458, SEQ ID NO: 44459, SEQ ID NO: 44465, SEQ ID NO: 44467, SEQ ID NO: 44471, SEQ ID NO: 44472, SEQ ID NO: 44480, SEQ ID NO: 44482, SEQ ID NO: 44485, SEQ ID NO: 44493, SEQ ID NO: 44496, SEQ ID NO: 44500, SEQ ID NO: 44511, SEQ ID NO: 44522, SEQ ID NO: 44523, SEQ ID NO: 44524, SEQ ID NO: 44536, SEQ ID NO: 44540, SEQ ID NO: 44542, SEQ ID NO: 44576, SEQ ID NO: 44586, SEQ ID NO: 44601, SEQ ID NO: 44613, SEQ ID NO: 44615, SEQ ID NO: 44619, SEQ ID NO: 44621, SEQ ID NO: 44624, SEQ ID NO: 44634, SEQ ID NO: 44635,

SEQ ID NO: 44637, SEQ ID NO: 44639, SEQ ID NO: 44645, SEQ ID NO: 44647, SEQ ID NO: 44655, SEQ ID NO: 44657, SEQ ID NO: 44659, SEQ ID NO: 44675, SEQ ID NO: 44678, SEQ ID NO: 44681, SEQ ID NO: 44686, SEQ ID NO: 44687, SEQ ID NO: 44701, SEQ ID NO: 44705, SEQ ID NO: 44707, SEQ ID NO: 44714, SEQ ID NO: 44720, SEQ ID NO: 44722, SEQ ID NO: 44728, SEQ ID NO: 44731, SEQ ID NO: 44732, SEQ ID NO: 44733, SEQ ID NO: 44739, SEQ ID NO: 44741, SEQ ID NO: 44749, SEQ ID NO: 44751, SEQ ID NO: 44755, SEQ ID NO: 44764, SEQ ID NO: 44777, SEQ ID NO: 44790, SEQ ID NO: 44797, SEQ ID NO: 44804, SEQ ID NO: 44806, SEQ ID NO: 44807, SEQ ID NO: 44811, SEQ ID NO: 44812, SEQ ID NO: 44813, SEQ ID NO: 44818, SEQ ID NO: 44822, SEQ ID NO: 44825, SEQ ID NO: 44828, SEQ ID NO: 44838, SEQ ID NO: 44844, SEQ ID NO: 44854, SEQ ID NO: 44856, SEQ ID NO: 44857, SEQ ID NO: 44858, SEQ ID NO: 44863, SEQ ID NO: 44866, SEQ ID NO: 44871, SEQ ID NO: 44874, SEQ ID NO: 44880, SEQ ID NO: 44883, SEQ ID NO: 44886, SEQ ID NO: 44892, SEQ ID NO: 44897, SEQ ID NO: 44905, SEQ ID NO: 44908, SEQ ID NO: 44914, SEQ ID NO: 44915, SEQ ID NO: 44931, SEQ ID NO: 44936, SEQ ID NO: 44941, SEQ ID NO: 44945, SEQ ID NO: 44949, SEQ ID NO: 44950, SEQ ID NO: 44961, SEQ ID NO: 44965, SEQ ID NO: 44971, SEQ ID NO: 44974, SEQ ID NO: 44976, SEQ ID NO: 44980, SEQ ID NO: 44983, SEQ ID NO: 44993, SEQ ID NO: 44996, SEQ ID NO: 45011, SEQ ID NO: 45013, SEQ ID NO: 45015, SEQ ID NO: 45026, SEQ ID NO: 45030, SEQ ID NO: 45031, SEQ ID NO: 45032, SEQ ID NO: 45034, SEQ ID NO: 45035, SEQ ID NO: 45046, SEQ ID NO: 45058, SEQ ID NO: 45061, SEQ ID NO: 45065, SEQ ID NO: 45066, SEQ ID NO: 45071, SEQ ID NO: 45077, SEQ ID NO: 45078, SEQ ID NO: 45080, SEQ ID NO: 45082, SEQ ID NO: 45085, SEQ ID NO: 45087, SEQ ID NO: 45095, SEQ ID NO: 45096, SEQ ID NO: 45102, SEQ ID NO: 45110, SEQ ID NO: 45112, SEQ ID NO: 45113, SEQ ID NO: 45116, SEQ ID NO: 45120, SEQ ID NO: 45123, SEQ ID NO: 45127, SEQ ID NO: 45129, SEQ ID NO: 45131, SEQ ID NO: 45144, SEQ ID NO: 45147, SEQ ID NO: 45148, SEQ ID NO: 45152, SEQ ID NO: 45157, SEQ ID NO: 45159, SEQ ID NO: 45162, SEQ ID NO: 45163, SEQ ID NO: 45169, SEQ ID NO: 45172, SEQ ID NO: 45177, SEQ ID NO: 45184, SEQ ID NO: 45188, SEQ ID NO: 45189, SEQ ID NO: 45202, SEQ ID NO: 45205, SEQ ID NO: 45212, SEQ ID NO: 45215, SEQ ID NO: 45219, SEQ ID NO: 45221, SEQ ID NO: 45222, SEQ ID NO: 45231, SEQ ID NO: 45241, SEQ ID NO: 45242, SEQ ID NO: 45248, SEQ ID NO: 45262, SEQ ID NO: 45264, SEQ ID NO: 45266, SEQ ID NO: 45271, SEQ ID NO: 45274, SEQ ID NO: 45275, SEQ ID NO: 45280, SEQ ID NO: 45290, SEQ ID NO: 45293, SEQ ID NO: 45294, SEQ ID NO: 45306, SEQ ID NO: 45308, SEQ ID NO: 45311, SEQ ID NO: 45312, SEQ ID NO: 45317, SEQ ID NO: 45320, SEQ ID NO: 45328, SEQ ID NO: 45329, SEQ ID NO: 45334, SEQ ID NO: 45335, SEQ ID NO: 45337, SEQ ID NO: 45339, SEQ ID NO: 45346, SEQ ID NO: 45352, SEQ ID NO: 45356, SEQ ID NO: 45357, SEQ ID NO: 45360, SEQ ID NO: 45362, SEQ ID NO: 45363, SEQ ID NO: 45375, SEQ ID NO: 45379, SEQ ID NO: 45389, SEQ ID NO: 45390, SEQ ID NO: 45401, SEQ ID NO: 45405, SEQ ID NO: 45406, SEQ ID NO: 45411, SEQ ID NO: 45421, SEQ ID NO: 45429, SEQ ID NO: 45431, SEQ ID NO: 45435, SEQ ID NO: 45437, SEQ ID NO: 45446, SEQ ID NO: 45449, SEQ ID NO: 45452, SEQ ID NO: 45453, SEQ ID NO: 45458, SEQ ID NO: 45461, SEQ ID NO: 45466, SEQ ID NO: 45467, SEQ ID NO: 45471, SEQ ID NO: 45473, SEQ ID NO: 45475, SEQ ID NO: 45478, SEQ ID NO: 45479, SEQ ID NO: 45483, SEQ ID NO: 45486, SEQ ID NO: 45487, SEQ ID NO: 45490, SEQ ID NO: 45492, SEQ ID NO: 45496, SEQ ID NO: 45498, SEQ ID NO: 45499, SEQ ID NO: 45505, SEQ ID NO: 45506, SEQ ID NO: 45507, SEQ ID NO: 45508, SEQ ID NO: 45512, SEQ ID NO: 45515, SEQ ID NO: 45517, SEQ ID NO: 45518, SEQ ID NO: 45521, SEQ ID NO: 45525, SEQ ID NO: 45526, SEQ ID NO: 45529, SEQ ID NO: 45537, SEQ ID NO: 45544, SEQ ID NO: 45552, SEQ ID NO: 45553, SEQ ID NO: 45554, SEQ ID NO: 45559, SEQ ID NO: 45560, SEQ ID NO: 45563, SEQ ID NO: 45567, SEQ ID NO: 45571, SEQ ID NO: 45576, SEQ ID NO: 45586, SEQ ID NO: 45589, SEQ ID NO: 45601, SEQ ID NO: 45605, SEQ ID NO: 45607, SEQ ID NO: 45613, SEQ ID NO: 45615, SEQ ID NO: 45616, SEQ ID NO: 45620, SEQ ID NO: 45624, SEQ ID NO: 45626, SEQ ID NO: 45627, SEQ ID NO: 45631, SEQ ID NO: 45633, SEQ ID NO: 45634, SEQ ID NO: 45636, SEQ ID NO: 45638, SEQ ID NO: 45640, SEQ ID NO: 45641, SEQ ID NO: 45643, SEQ ID NO: 45653, SEQ ID NO: 45655, SEQ ID NO: 45656, SEQ ID NO: 45660, SEQ ID NO: 45666, SEQ ID NO: 45671, SEQ ID NO: 45676, SEQ ID NO: 45685, SEQ ID NO: 45686, SEQ ID NO: 45690, SEQ ID NO: 45694, SEQ ID NO: 45700, SEQ ID NO: 45706, SEQ ID NO: 45707, SEQ ID NO: 45712, SEQ ID NO: 45716, SEQ ID NO: 45719, SEQ ID NO: 45721, SEQ ID NO: 45725, SEQ ID NO: 45726, SEQ ID NO: 45730, SEQ ID NO: 45732, SEQ ID NO: 45733, SEQ ID NO: 45738, SEQ ID NO: 45739, SEQ ID NO: 45740, SEQ ID NO: 45743, SEQ ID NO: 45748, SEQ ID NO: 45749, SEQ ID NO: 45750, SEQ ID NO: 45757, SEQ ID NO: 45764, SEQ ID NO: 45769, SEQ ID NO: 45774, SEQ ID NO: 45777, SEQ ID NO: 45778, SEQ ID NO: 45788, SEQ ID NO: 45790, SEQ ID NO: 45793, SEQ ID NO: 45798, SEQ ID NO: 45805, SEQ ID NO: 45806, SEQ ID NO: 45816, SEQ ID NO: 45818, SEQ ID NO: 45821, SEQ ID NO: 45825, SEQ ID NO: 45826, SEQ ID NO: 45838, SEQ ID NO: 45844, SEQ ID NO: 45848, SEQ ID NO: 45853, SEQ ID NO: 45855, SEQ ID NO: 45858, SEQ ID NO: 45864, SEQ ID NO: 45866, SEQ ID NO: 45874, SEQ ID NO: 45892, SEQ ID NO: 45895, SEQ ID NO: 45896, SEQ ID NO: 45900, SEQ ID NO: 45901, SEQ ID NO: 45904, SEQ ID NO: 45909, SEQ ID NO: 45913, SEQ ID NO: 45915, SEQ ID NO: 45916, SEQ ID NO: 45922, SEQ ID NO: 45923, SEQ ID NO: 45924, SEQ ID NO: 45925, SEQ ID NO: 45926, SEQ ID NO: 45928, SEQ ID NO: 45930, SEQ ID NO: 45931, SEQ ID NO: 45948, SEQ ID NO: 45953, SEQ ID NO: 45955, SEQ ID NO: 45963, SEQ ID NO: 45966, SEQ ID NO: 45969, SEQ ID NO: 45971, SEQ ID NO: 45974, SEQ ID NO: 45981, SEQ ID NO: 45982, SEQ ID NO: 45986, SEQ ID NO: 45989, SEQ ID NO: 45991, SEQ ID NO: 45995, SEQ ID NO: 45997, SEQ ID NO:

45998, SEQ ID NO: 46000, SEQ ID NO: 46007, SEQ ID NO: 46009, SEQ ID NO: 46011, SEQ ID NO: 46014, SEQ ID NO: 46016, SEQ ID NO: 46017, SEQ ID NO: 46018, SEQ ID NO: 46027, SEQ ID NO: 46030, SEQ ID NO: 46033, SEQ ID NO: 46034, SEQ ID NO: 46036, SEQ ID NO: 46042, SEQ ID NO: 46044, SEQ ID NO: 46048, SEQ ID NO: 46050, SEQ ID NO: 46065, SEQ ID NO: 46070, SEQ ID NO: 46071, SEQ ID NO: 46074, SEQ ID NO: 46076, SEQ ID NO: 46078, SEQ ID NO: 46080, SEQ ID NO: 46081, SEQ ID NO: 46084, SEQ ID NO: 46090, SEQ ID NO: 46096, SEQ ID NO: 46101, SEQ ID NO: 46103, SEQ ID NO: 46110, SEQ ID NO: 46115, SEQ ID NO: 46117, SEQ ID NO: 46122, SEQ ID NO: 46124, SEQ ID NO: 46127, SEQ ID NO: 46131, SEQ ID NO: 46133, SEQ ID NO: 46134, SEQ ID NO: 46141, SEQ ID NO: 46156, SEQ ID NO: 46160, SEQ ID NO: 46165, SEQ ID NO: 46169, SEQ ID NO: 46171, SEQ ID NO: 46176, SEQ ID NO: 46177, SEQ ID NO: 46179, SEQ ID NO: 46182, SEQ ID NO: 46186, SEQ ID NO: 46188, SEQ ID NO: 46197, SEQ ID NO: 46210, SEQ ID NO: 46214, SEQ ID NO: 46215, SEQ ID NO: 46220, SEQ ID NO: 46221, SEQ ID NO: 46229, SEQ ID NO: 46230, SEQ ID NO: 46235, SEQ ID NO: 46236, SEQ ID NO: 46238, SEQ ID NO: 46239, SEQ ID NO: 46243, SEQ ID NO: 46244, SEQ ID NO: 46254, SEQ ID NO: 46255, SEQ ID NO: 46256, SEQ ID NO: 46258, SEQ ID NO: 46261, SEQ ID NO: 46262, SEQ ID NO: 46263, SEQ ID NO: 46275, SEQ ID NO: 46280, SEQ ID NO: 46288, SEQ ID NO: 46289, SEQ ID NO: 46292, SEQ ID NO: 46303, SEQ ID NO: 46307, SEQ ID NO: 46308, SEQ ID NO: 46310, SEQ ID NO: 46322, SEQ ID NO: 46328, SEQ ID NO: 46332, SEQ ID NO: 46335, SEQ ID NO: 46339, SEQ ID NO: 46341, SEQ ID NO: 46342, SEQ ID NO: 46344, SEQ ID NO: 46353, SEQ ID NO: 46360, SEQ ID NO: 46362, SEQ ID NO: 46367, SEQ ID NO: 46369, SEQ ID NO: 46372, SEQ ID NO: 46378, SEQ ID NO: 46385, SEQ ID NO: 46386, SEQ ID NO: 46395, SEQ ID NO: 46396, SEQ ID NO: 46399, SEQ ID NO: 46400, SEQ ID NO: 46401, SEQ ID NO: 46402, SEQ ID NO: 46406, SEQ ID NO: 46409, SEQ ID NO: 46412, SEQ ID NO: 46416, SEQ ID NO: 46421, SEQ ID NO: 46422, SEQ ID NO: 46427, SEQ ID NO: 46428, SEQ ID NO: 46440, SEQ ID NO: 46442, SEQ ID NO: 46451, SEQ ID NO: 46455, SEQ ID NO: 46456, SEQ ID NO: 46461, SEQ ID NO: 46467, SEQ ID NO: 46471, SEQ ID NO: 46476, SEQ ID NO: 46478, SEQ ID NO: 46479, SEQ ID NO: 46480, SEQ ID NO: 46483, SEQ ID NO: 46486, SEQ ID NO: 46487, SEQ ID NO: 46492, SEQ ID NO: 46499, SEQ ID NO: 46506, SEQ ID NO: 46508, SEQ ID NO: 46512, SEQ ID NO: 46513, SEQ ID NO: 46519, SEQ ID NO: 46520, SEQ ID NO: 46521, SEQ ID NO: 46524, SEQ ID NO: 46532, SEQ ID NO: 46534, SEQ ID NO: 46536, SEQ ID NO: 46539, SEQ ID NO: 46542, SEQ ID NO: 46543, SEQ ID NO: 46552, SEQ ID NO: 46572, SEQ ID NO: 46590, SEQ ID NO: 46591, SEQ ID NO: 46603, SEQ ID NO: 46610, SEQ ID NO: 46621, SEQ ID NO: 46628, SEQ ID NO: 46635, SEQ ID NO: 46641, SEQ ID NO: 46642, SEQ ID NO: 46647, SEQ ID NO: 46650, SEQ ID NO: 46656, SEQ ID NO: 46662, SEQ ID NO: 46665, SEQ ID NO: 46671, SEQ ID NO: 46675, SEQ ID NO: 46676, SEQ ID NO: 46678, SEQ ID NO: 46683, SEQ ID NO: 46690, SEQ ID NO: 46691, SEQ ID NO: 46693, SEQ ID NO: 46695, SEQ ID NO: 46699, SEQ ID NO: 46700, SEQ ID NO: 46701, SEQ ID NO: 46705, SEQ ID NO: 46710, SEQ ID NO: 46712, SEQ ID NO: 46719, SEQ ID NO: 46727, SEQ ID NO: 46733, SEQ ID NO: 46735, SEQ ID NO: 46741, SEQ ID NO: 46744, SEQ ID NO: 46745, SEQ ID NO: 46753, SEQ ID NO: 46761, SEQ ID NO: 46768, SEQ ID NO: 46769, SEQ ID NO: 46770, SEQ ID NO: 46775, SEQ ID NO: 46779, SEQ ID NO: 46783, SEQ ID NO: 46785, SEQ ID NO: 46791, SEQ ID NO: 46796, SEQ ID NO: 46802, SEQ ID NO: 46816, SEQ ID NO: 46835, SEQ ID NO: 46841, SEQ ID NO: 46843, SEQ ID NO: 46844, SEQ ID NO: 46846, SEQ ID NO: 46851, SEQ ID NO: 46852, SEQ ID NO: 46861, SEQ ID NO: 46867, SEQ ID NO: 46871, SEQ ID NO: 46879, SEQ ID NO: 46891, SEQ ID NO: 46896, SEQ ID NO: 46911, SEQ ID NO: 46914, SEQ ID NO: 46918, SEQ ID NO: 46929, SEQ ID NO: 46933, SEQ ID NO: 46934, SEQ ID NO: 46939, SEQ ID NO: 46949, SEQ ID NO: 46950, SEQ ID NO: 46955, SEQ ID NO: 46963, SEQ ID NO: 46970, SEQ ID NO: 46974, SEQ ID NO: 46978, SEQ ID NO: 46985, SEQ ID NO: 46986, SEQ ID NO: 46987, SEQ ID NO: 46991, SEQ ID NO: 47000, SEQ ID NO: 47006, SEQ ID NO: 47009, SEQ ID NO: 47016, SEQ ID NO: 47023, SEQ ID NO: 47024, SEQ ID NO: 47030, SEQ ID NO: 47032, SEQ ID NO: 47035, SEQ ID NO: 47039, SEQ ID NO: 47055, SEQ ID NO: 47056, SEQ ID NO: 47065, SEQ ID NO: 47066, SEQ ID NO: 47067, SEQ ID NO: 47071, SEQ ID NO: 47074, SEQ ID NO: 47079, SEQ ID NO: 47086, SEQ ID NO: 47090, SEQ ID NO: 47093, SEQ ID NO: 47097, SEQ ID NO: 47098, SEQ ID NO: 47104, SEQ ID NO: 47106, SEQ ID NO: 47107, SEQ ID NO: 47109, SEQ ID NO: 47110, SEQ ID NO: 47113, SEQ ID NO: 47115, SEQ ID NO: 47117, SEQ ID NO: 47122, SEQ ID NO: 47123, SEQ ID NO: 47124, SEQ ID NO: 47126, SEQ ID NO: 47133, SEQ ID NO: 47136, SEQ ID NO: 47141, SEQ ID NO: 47143, SEQ ID NO: 47148, SEQ ID NO: 47151, SEQ ID NO: 47153, SEQ ID NO: 47155, SEQ ID NO: 47162, SEQ ID NO: 47172, SEQ ID NO: 47184, SEQ ID NO: 47186, SEQ ID NO: 47188, SEQ ID NO: 47192, SEQ ID NO: 47205, SEQ ID NO: 47210, SEQ ID NO: 47211, SEQ ID NO: 47212, SEQ ID NO: 47224, SEQ ID NO: 47228, SEQ ID NO: 47234, SEQ ID NO: 47236, SEQ ID NO: 47239, SEQ ID NO: 47240, SEQ ID NO: 47245, SEQ ID NO: 47246, SEQ ID NO: 47249, SEQ ID NO: 47251, SEQ ID NO: 47254, SEQ ID NO: 47255, SEQ ID NO: 47258, SEQ ID NO: 47265, SEQ ID NO: 47276, SEQ ID NO: 47280, SEQ ID NO: 47287, SEQ ID NO: 47293, SEQ ID NO: 47294, SEQ ID NO: 47296, SEQ ID NO: 47299, SEQ ID NO: 47300, SEQ ID NO: 47308, SEQ ID NO: 47316, SEQ ID NO: 47321, SEQ ID NO: 47325, SEQ ID NO: 47327, SEQ ID NO: 47336, SEQ ID NO: 47343, SEQ ID NO: 47345, SEQ ID NO: 47347, SEQ ID NO: 47353, SEQ ID NO: 47355, SEQ ID NO: 47363, SEQ ID NO: 47368, SEQ ID NO: 47369, SEQ ID NO: 47370, SEQ ID NO: 47381, SEQ ID NO: 47387, SEQ ID NO: 47392, SEQ ID NO: 47396, SEQ ID NO: 47397, SEQ ID NO: 47402, SEQ ID NO: 47408, SEQ ID NO: 47412, SEQ ID NO: 47416, SEQ ID NO: 47418, SEQ ID NO: 47421, SEQ ID NO: 47424, SEQ ID NO: 47430, SEQ

ID NO: 47439, SEQ ID NO: 47442, SEQ ID NO: 47445, SEQ ID NO: 47447, SEQ ID NO: 47469, SEQ ID NO: 47474, SEQ ID NO: 47479, SEQ ID NO: 47485, SEQ ID NO: 47488, SEQ ID NO: 47491, SEQ ID NO: 47497, SEQ ID NO: 47499, SEQ ID NO: 47507, SEQ ID NO: 47510, SEQ ID NO: 47512, SEQ ID NO: 47514, SEQ ID NO: 47517, SEQ ID NO: 47518, SEQ ID NO: 47530, SEQ ID NO: 47534, SEQ ID NO: 47539, SEQ ID NO: 47541, SEQ ID NO: 47547, SEQ ID NO: 47549, SEQ ID NO: 47554, SEQ ID NO: 47564, SEQ ID NO: 47566, SEQ ID NO: 47568, SEQ ID NO: 47577, SEQ ID NO: 47578, SEQ ID NO: 47579, SEQ ID NO: 47581, SEQ ID NO: 47588, SEQ ID NO: 47591, SEQ ID NO: 47593, SEQ ID NO: 47596, SEQ ID NO: 47613, SEQ ID NO: 47615, SEQ ID NO: 47621, SEQ ID NO: 47622, SEQ ID NO: 47624, SEQ ID NO: 47629, SEQ ID NO: 47630, SEQ ID NO: 47634, SEQ ID NO: 47635, SEQ ID NO: 47636, SEQ ID NO: 47639, SEQ ID NO: 47641, SEQ ID NO: 47646, SEQ ID NO: 47653, SEQ ID NO: 47656, SEQ ID NO: 47657, SEQ ID NO: 47660, SEQ ID NO: 47665, SEQ ID NO: 47669, SEQ ID NO: 47670, SEQ ID NO: 47671, SEQ ID NO: 47676, SEQ ID NO: 47679, SEQ ID NO: 47681, SEQ ID NO: 47683, SEQ ID NO: 47689, SEQ ID NO: 47694, SEQ ID NO: 47696, SEQ ID NO: 47706, SEQ ID NO: 47713, SEQ ID NO: 47716, SEQ ID NO: 47722, SEQ ID NO: 47724, SEQ ID NO: 47727, SEQ ID NO: 47737, SEQ ID NO: 47740, SEQ ID NO: 47741, SEQ ID NO: 47743, SEQ ID NO: 47749, SEQ ID NO: 47751, SEQ ID NO: 47754, SEQ ID NO: 47755, SEQ ID NO: 47759, SEQ ID NO: 47761, SEQ ID NO: 47773, SEQ ID NO: 47775, SEQ ID NO: 47777, SEQ ID NO: 47778, SEQ ID NO: 47780, SEQ ID NO: 47786, SEQ ID NO: 47809, SEQ ID NO: 47818, SEQ ID NO: 47820, SEQ ID NO: 47824, SEQ ID NO: 47827, SEQ ID NO: 47829, SEQ ID NO: 47836, SEQ ID NO: 47837, SEQ ID NO: 47838, SEQ ID NO: 47840, SEQ ID NO: 47852, SEQ ID NO: 47857, SEQ ID NO: 47861, SEQ ID NO: 47863, SEQ ID NO: 47874, SEQ ID NO: 47875, SEQ ID NO: 47877, SEQ ID NO: 47882, SEQ ID NO: 47888, SEQ ID NO: 47891, SEQ ID NO: 47892, SEQ ID NO: 47897, SEQ ID NO: 47898, SEQ ID NO: 47909, SEQ ID NO: 47916, SEQ ID NO: 47922, SEQ ID NO: 47924, SEQ ID NO: 47929, SEQ ID NO: 47931, SEQ ID NO: 47934, SEQ ID NO: 47936, SEQ ID NO: 47940, SEQ ID NO: 47959, SEQ ID NO: 47965, SEQ ID NO: 47969, SEQ ID NO: 47970, SEQ ID NO: 47972, SEQ ID NO: 47973, SEQ ID NO: 47980, SEQ ID NO: 47986, SEQ ID NO: 47988, SEQ ID NO: 47990, SEQ ID NO: 48008, SEQ ID NO: 48010, SEQ ID NO: 48014, SEQ ID NO: 48018, SEQ ID NO: 48019, SEQ ID NO: 48049, SEQ ID NO: 48051, SEQ ID NO: 48061, SEQ ID NO: 48062, SEQ ID NO: 48069, SEQ ID NO: 48091, SEQ ID NO: 48093, SEQ ID NO: 48098, SEQ ID NO: 48112, SEQ ID NO: 48119, SEQ ID NO: 48120, SEQ ID NO: 48121, SEQ ID NO: 48124, SEQ ID NO: 48132, SEQ ID NO: 48133, SEQ ID NO: 48140, SEQ ID NO: 48147, SEQ ID NO: 48151, SEQ ID NO: 48160, SEQ ID NO: 48167, SEQ ID NO: 48169, SEQ ID NO: 48197, SEQ ID NO: 48200, SEQ ID NO: 48203, SEQ ID NO: 48208, SEQ ID NO: 48209, SEQ ID NO: 48211, SEQ ID NO: 48213, SEQ ID NO: 48214, SEQ ID NO: 48222, SEQ ID NO: 48231, SEQ ID NO: 48233, SEQ ID NO: 48235, SEQ ID NO: 48236, SEQ ID NO: 48247, SEQ ID NO: 48258, SEQ ID NO: 48269, SEQ ID NO: 48270, SEQ ID NO: 48280, SEQ ID NO: 48295, SEQ ID NO: 48303, SEQ ID NO: 48304, SEQ ID NO: 48311, SEQ ID NO: 48312, SEQ ID NO: 48313, SEQ ID NO: 48316, SEQ ID NO: 48317, SEQ ID NO: 48318, SEQ ID NO: 48320, SEQ ID NO: 48330, SEQ ID NO: 48331, SEQ ID NO: 48332, SEQ ID NO: 48337, SEQ ID NO: 48338, SEQ ID NO: 48341, SEQ ID NO: 48343, SEQ ID NO: 48351, SEQ ID NO: 48353, SEQ ID NO: 48357, SEQ ID NO: 48361, SEQ ID NO: 48366, SEQ ID NO: 48368, SEQ ID NO: 48370, SEQ ID NO: 48372, SEQ ID NO: 48375, SEQ ID NO: 48376, SEQ ID NO: 48384, SEQ ID NO: 48388, SEQ ID NO: 48403, SEQ ID NO: 48404, SEQ ID NO: 48412, SEQ ID NO: 48414, SEQ ID NO: 48420, SEQ ID NO: 48421, SEQ ID NO: 48423, SEQ ID NO: 48425, SEQ ID NO: 48434, SEQ ID NO: 48435, SEQ ID NO: 48437, SEQ ID NO: 48439, SEQ ID NO: 48456, SEQ ID NO: 48460, SEQ ID NO: 48471, SEQ ID NO: 48480, SEQ ID NO: 48482, SEQ ID NO: 48485, SEQ ID NO: 48491, SEQ ID NO: 48507, SEQ ID NO: 48510, SEQ ID NO: 48514, SEQ ID NO: 48515, SEQ ID NO: 48518, SEQ ID NO: 48520, SEQ ID NO: 48524, SEQ ID NO: 48526, SEQ ID NO: 48528, SEQ ID NO: 48531, SEQ ID NO: 48541, SEQ ID NO: 48543, SEQ ID NO: 48549, SEQ ID NO: 48550, SEQ ID NO: 48552, SEQ ID NO: 48553, SEQ ID NO: 48554, SEQ ID NO: 48561, SEQ ID NO: 48562, SEQ ID NO: 48563, SEQ ID NO: 48564, SEQ ID NO: 48565, SEQ ID NO: 48576, SEQ ID NO: 48580, SEQ ID NO: 48586, SEQ ID NO: 48587, SEQ ID NO: 48589, SEQ ID NO: 48592, SEQ ID NO: 48598, SEQ ID NO: 48600, SEQ ID NO: 48604, SEQ ID NO: 48605, SEQ ID NO: 48606, SEQ ID NO: 48608, SEQ ID NO: 48610, SEQ ID NO: 48612, SEQ ID NO: 48614, SEQ ID NO: 48615, SEQ ID NO: 48621, SEQ ID NO: 48622, SEQ ID NO: 48631, SEQ ID NO: 48633, SEQ ID NO: 48636, SEQ ID NO: 48637, SEQ ID NO: 48638, SEQ ID NO: 48642, SEQ ID NO: 48644, SEQ ID NO: 48647, SEQ ID NO: 48648, SEQ ID NO: 48649, SEQ ID NO: 48650, SEQ ID NO: 48653, SEQ ID NO: 48654, SEQ ID NO: 48657, SEQ ID NO: 48667, SEQ ID NO: 48673, SEQ ID NO: 48678, SEQ ID NO: 48682, SEQ ID NO: 48688, SEQ ID NO: 48689, SEQ ID NO: 48690, SEQ ID NO: 48691, SEQ ID NO: 48702, SEQ ID NO: 48706, SEQ ID NO: 48711, SEQ ID NO: 48726, SEQ ID NO: 48730, SEQ ID NO: 48735, SEQ ID NO: 48741, SEQ ID NO: 48743, SEQ ID NO: 48747, SEQ ID NO: 48749, SEQ ID NO: 48754, SEQ ID NO: 48764, SEQ ID NO: 48767, SEQ ID NO: 48770, SEQ ID NO: 48771, SEQ ID NO: 48784, SEQ ID NO: 48793, SEQ ID NO: 48794, SEQ ID NO: 48797, SEQ ID NO: 48808, SEQ ID NO: 48812, SEQ ID NO: 48817, SEQ ID NO: 48822, SEQ ID NO: 48825, SEQ ID NO: 48827, SEQ ID NO: 48828, SEQ ID NO: 48831, SEQ ID NO: 48833, SEQ ID NO: 48835, SEQ ID NO: 48838, SEQ ID NO: 48840, SEQ ID NO: 48850, SEQ ID NO: 48855, SEQ ID NO: 48863, SEQ ID NO: 48865, SEQ ID NO: 48868, SEQ ID NO: 48873, SEQ ID NO: 48876, SEQ ID NO: 48880, SEQ ID NO: 48887, SEQ ID NO: 48889, SEQ ID NO: 48897, SEQ ID NO: 48901, SEQ ID NO: 48905, SEQ ID NO: 48906, SEQ ID NO: 48916, SEQ ID NO: 48921,

SEQ ID NO: 48924, SEQ ID NO: 48927, SEQ ID NO: 48931, SEQ ID NO: 48932, SEQ ID NO: 48934, SEQ ID NO: 48936, SEQ ID NO: 48941, SEQ ID NO: 52200, SEQ ID NO: 52216, SEQ ID NO: 52269, SEQ ID NO: 52297, SEQ ID NO: 52302, SEQ ID NO: 52317, SEQ ID NO: 52335, SEQ ID NO: 52340, SEQ ID NO: 52358, SEQ ID NO: 52374, SEQ ID NO: 52385, SEQ ID NO: 52395, SEQ ID NO: 52401, SEQ ID NO: 52411, SEQ ID NO: 52415, SEQ ID NO: 52465, SEQ ID NO: 52489, SEQ ID NO: 52498, SEQ ID NO: 52500, SEQ ID NO: 52537, SEQ ID NO: 52541, SEQ ID NO: 52550, SEQ ID NO: 52555, SEQ ID NO: 52609, SEQ ID NO: 52624, SEQ ID NO: 52635, SEQ ID NO: 52655, SEQ ID NO: 52665, SEQ ID NO: 52704, SEQ ID NO: 52717, SEQ ID NO: 52729, SEQ ID NO: 52741, SEQ ID NO: 52773, SEQ ID NO: 52786, SEQ ID NO: 52790, SEQ ID NO: 52856, SEQ ID NO: 52889, SEQ ID NO: 52913, SEQ ID NO: 52940, SEQ ID NO: 52956, SEQ ID NO: 53000, SEQ ID NO: 53024, SEQ ID NO: 53059, SEQ ID NO: 53061, SEQ ID NO: 53098, SEQ ID NO: 53106, SEQ ID NO: 53120, SEQ ID NO: 53128, SEQ ID NO: 53143, SEQ ID NO: 53188, SEQ ID NO: 53197, SEQ ID NO: 53201, SEQ ID NO: 53220, SEQ ID NO: 53276, SEQ ID NO: 53309, SEQ ID NO: 53453, SEQ ID NO: 53478, SEQ ID NO: 53535, SEQ ID NO: 53560, SEQ ID NO: 53605, SEQ ID NO: 53622, SEQ ID NO: 53705, SEQ ID NO: 53711, SEQ ID NO: 53717, SEQ ID NO: 53776, SEQ ID NO: 53787, SEQ ID NO: 53832, SEQ ID NO: 53834, SEQ ID NO: 53847, SEQ ID NO: 53875, SEQ ID NO: 53884, SEQ ID NO: 53887, SEQ ID NO: 53894, SEQ ID NO: 53897, SEQ ID NO: 53904, SEQ ID NO: 53907, SEQ ID NO: 53920, SEQ ID NO: 53925, SEQ ID NO: 53983, SEQ ID NO: 53993, SEQ ID NO: 54090, SEQ ID NO: 54092, SEQ ID NO: 54114, SEQ ID NO: 54115, SEQ ID NO: 54135, SEQ ID NO: 54182, SEQ ID NO: 54195, SEQ ID NO: 54197, SEQ ID NO: 54206, SEQ ID NO: 54207, SEQ ID NO: 54301, SEQ ID NO: 54334, SEQ ID NO: 54349, SEQ ID NO: 54360, SEQ ID NO: 54377, SEQ ID NO: 54465, SEQ ID NO: 54470, SEQ ID NO: 54473, SEQ ID NO: 54494, SEQ ID NO: 54497, SEQ ID NO: 54528, SEQ ID NO: 54537, SEQ ID NO: 54551, SEQ ID NO: 54563, SEQ ID NO: 54602, SEQ ID NO: 54644, SEQ ID NO: 54646, SEQ ID NO: 54702, SEQ ID NO: 54715, SEQ ID NO: 54739, SEQ ID NO: 54760, SEQ ID NO: 54766, SEQ ID NO: 54776, SEQ ID NO: 54777, SEQ ID NO: 54786, SEQ ID NO: 54810, SEQ ID NO: 54828, SEQ ID NO: 54931, SEQ ID NO: 54983, SEQ ID NO: 55026, SEQ ID NO: 55060, SEQ ID NO: 55067, SEQ ID NO: 55096, SEQ ID NO: 55102, SEQ ID NO: 55116, SEQ ID NO: 55162, SEQ ID NO: 55171, SEQ ID NO: 55192, SEQ ID NO: 55197, SEQ ID NO: 55198, SEQ ID NO: 55256, SEQ ID NO: 55265, SEQ ID NO: 55291, SEQ ID NO: 55294, SEQ ID NO: 55317, SEQ ID NO: 55319, SEQ ID NO: 55330, SEQ ID NO: 55343, SEQ ID NO: 55367, SEQ ID NO: 55382, SEQ ID NO: 55393, SEQ ID NO: 55394, SEQ ID NO: 55396, SEQ ID NO: 55414, SEQ ID NO: 62578, SEQ ID NO: 62587, SEQ ID NO: 62603, SEQ ID NO: 62614, SEQ ID NO: 62618, SEQ ID NO: 62620, SEQ ID NO: 62634, SEQ ID NO: 62670, SEQ ID NO: 62682, SEQ ID NO: 62705, SEQ ID NO: 62727, SEQ ID NO: 62729, SEQ ID NO: 62737, SEQ ID NO: 62760, SEQ ID NO: 62800, SEQ ID NO: 62801, SEQ ID NO: 62803, SEQ ID NO: 62848, SEQ ID NO: 62852, SEQ ID NO: 62861, SEQ ID NO: 62862, SEQ ID NO: 62869, SEQ ID NO: 62876, SEQ ID NO: 62878, SEQ ID NO: 62885, SEQ ID NO: 62905, SEQ ID NO: 62936, SEQ ID NO: 62961, SEQ ID NO: 62973, SEQ ID NO: 62992, SEQ ID NO: 63002, SEQ ID NO: 63008, SEQ ID NO: 63014, SEQ ID NO: 63021, SEQ ID NO: 63024, SEQ ID NO: 63036, SEQ ID NO: 63051, SEQ ID NO: 63057, SEQ ID NO: 63064, SEQ ID NO: 63081, SEQ ID NO: 63095, SEQ ID NO: 63096, SEQ ID NO: 63109, SEQ ID NO: 63114, SEQ ID NO: 63120, SEQ ID NO: 63127, SEQ ID NO: 63161, SEQ ID NO: 63187, SEQ ID NO: 63197, SEQ ID NO: 63198, SEQ ID NO: 63216, SEQ ID NO: 63217, SEQ ID NO: 63260, SEQ ID NO: 63264, SEQ ID NO: 63278, SEQ ID NO: 63299, SEQ ID NO: 63307, SEQ ID NO: 63313, SEQ ID NO: 63317, SEQ ID NO: 63322, SEQ ID NO: 63333, SEQ ID NO: 63336, SEQ ID NO: 63345, SEQ ID NO: 63358, SEQ ID NO: 63389, SEQ ID NO: 63400, SEQ ID NO: 63403, SEQ ID NO: 63404, SEQ ID NO: 63429, SEQ ID NO: 63434, SEQ ID NO: 63441, SEQ ID NO: 63461, SEQ ID NO: 63471, SEQ ID NO: 63472, SEQ ID NO: 63477, SEQ ID NO: 63478, SEQ ID NO: 63486, SEQ ID NO: 63513, SEQ ID NO: 63531, SEQ ID NO: 63557, SEQ ID NO: 63558, SEQ ID NO: 63581, SEQ ID NO: 63586, SEQ ID NO: 63591, SEQ ID NO: 63605, SEQ ID NO: 63608, SEQ ID NO: 63611, SEQ ID NO: 63636, SEQ ID NO: 63637, SEQ ID NO: 63640, SEQ ID NO: 63660, SEQ ID NO: 63681, SEQ ID NO: 63708, SEQ ID NO: 63724, SEQ ID NO: 63737, SEQ ID NO: 63739, SEQ ID NO: 63776, SEQ ID NO: 63785, SEQ ID NO: 63793, SEQ ID NO: 63796, SEQ ID NO: 63820, SEQ ID NO: 63822, SEQ ID NO: 63827, SEQ ID NO: 63832, SEQ ID NO: 63835, SEQ ID NO: 63871, SEQ ID NO: 63877, SEQ ID NO: 63892, SEQ ID NO: 63894, SEQ ID NO: 63901, SEQ ID NO: 63906, SEQ ID NO: 63915, SEQ ID NO: 63925, SEQ ID NO: 63933, SEQ ID NO: 63949, SEQ ID NO: 63959, SEQ ID NO: 63960, SEQ ID NO: 63965, SEQ ID NO: 63971, SEQ ID NO: 63984, SEQ ID NO: 64000, SEQ ID NO: 64004, SEQ ID NO: 64006, SEQ ID NO: 64008, SEQ ID NO: 64031, SEQ ID NO: 64034, SEQ ID NO: 64036, SEQ ID NO: 64043, SEQ ID NO: 64044, SEQ ID NO: 64055, SEQ ID NO: 64087, SEQ ID NO: 64092, SEQ ID NO: 64111, SEQ ID NO: 64115, SEQ ID NO: 64116, SEQ ID NO: 64143, SEQ ID NO: 64153, SEQ ID NO: 64158, SEQ ID NO: 64192, SEQ ID NO: 64198, SEQ ID NO: 64205, SEQ ID NO: 64221, SEQ ID NO: 64223, SEQ ID NO: 64230, SEQ ID NO: 64250, SEQ ID NO: 64274, SEQ ID NO: 64279, SEQ ID NO: 64283, SEQ ID NO: 64292, SEQ ID NO: 64295, SEQ ID NO: 64301, SEQ ID NO: 64310, SEQ ID NO: 64316, SEQ ID NO: 64323, SEQ ID NO: 64332, SEQ ID NO: 64367, SEQ ID NO: 64369, SEQ ID NO: 64371, SEQ ID NO: 64396, SEQ ID NO: 64429, SEQ ID NO: 64431, SEQ ID NO: 64439, SEQ ID NO: 64460, SEQ ID NO: 64468, SEQ ID NO: 64484, SEQ ID NO: 64492, SEQ ID NO: 64522, SEQ ID NO: 64566, SEQ ID NO: 64578, SEQ ID NO: 64585, SEQ ID NO: 64600, SEQ ID NO: 64634, SEQ ID NO: 64644, SEQ ID NO: 64650, SEQ ID NO: 64659, SEQ ID NO: 64663, SEQ ID NO: 64664, SEQ ID NO:

64676, SEQ ID NO: 64686, SEQ ID NO: 64693, SEQ ID NO: 64698, SEQ ID NO: 64768, SEQ ID NO: 64772, SEQ ID NO: 64776, SEQ ID NO: 64812, SEQ ID NO: 64823, SEQ ID NO: 64827, SEQ ID NO: 64852, SEQ ID NO: 64854, SEQ ID NO: 64859, SEQ ID NO: 64860, SEQ ID NO: 64871, SEQ ID NO: 64881, SEQ ID NO: 64885, SEQ ID NO: 64893, SEQ ID NO: 64905, SEQ ID NO: 64918, SEQ ID NO: 64962, SEQ ID NO: 64966, SEQ ID NO: 64967, SEQ ID NO: 64973, SEQ ID NO: 64984, SEQ ID NO: 64989, SEQ ID NO: 64990, SEQ ID NO: 65023, SEQ ID NO: 65033, SEQ ID NO: 65034, SEQ ID NO: 65063, SEQ ID NO: 65074, SEQ ID NO: 65077, SEQ ID NO: 65100, SEQ ID NO: 65102, SEQ ID NO: 65107, SEQ ID NO: 65145, SEQ ID NO: 65177, SEQ ID NO: 65190, SEQ ID NO: 65216, SEQ ID NO: 65219, SEQ ID NO: 65221, SEQ ID NO: 65247, SEQ ID NO: 65271, SEQ ID NO: 65283, SEQ ID NO: 65298, SEQ ID NO: 65321, SEQ ID NO: 65331, SEQ ID NO: 65333, SEQ ID NO: 65335, SEQ ID NO: 65343, SEQ ID NO: 65348, SEQ ID NO: 65356, SEQ ID NO: 65370, SEQ ID NO: 65373, SEQ ID NO: 65380, SEQ ID NO: 65382, SEQ ID NO: 65383, SEQ ID NO: 65397, SEQ ID NO: 65408, SEQ ID NO: 65420, SEQ ID NO: 65423, SEQ ID NO: 65441, SEQ ID NO: 65444, SEQ ID NO: 65445, SEQ ID NO: 65447, SEQ ID NO: 65448, SEQ ID NO: 65471, SEQ ID NO: 65493, SEQ ID NO: 65505, SEQ ID NO: 65547, SEQ ID NO: 65566, SEQ ID NO: 65587, SEQ ID NO: 65609, SEQ ID NO: 65612, SEQ ID NO: 65616, SEQ ID NO: 65619, SEQ ID NO: 65633, SEQ ID NO: 65640, SEQ ID NO: 65647, SEQ ID NO: 65665, SEQ ID NO: 65686, SEQ ID NO: 65695, SEQ ID NO: 65716, SEQ ID NO: 65722, SEQ ID NO: 65726, SEQ ID NO: 65740, SEQ ID NO: 65754, SEQ ID NO: 65773, SEQ ID NO: 65774, SEQ ID NO: 65800, SEQ ID NO: 65808, SEQ ID NO: 65826, SEQ ID NO: 65832, SEQ ID NO: 65833, SEQ ID NO: 65836, SEQ ID NO: 65842, SEQ ID NO: 65845, SEQ ID NO: 65854, SEQ ID NO: 65856, SEQ ID NO: 65890, SEQ ID NO: 65907, SEQ ID NO: 65921, SEQ ID NO: 65933, SEQ ID NO: 65948, SEQ ID NO: 65953, SEQ ID NO: 65962, SEQ ID NO: 66046, SEQ ID NO: 66056, SEQ ID NO: 66061, SEQ ID NO: 66068, SEQ ID NO: 82852 to SEQ ID NO: 83126, SEQ ID NO: 85833, SEQ ID NO: 85844, SEQ ID NO: 85849, SEQ ID NO: 85883, SEQ ID NO: 85901, SEQ ID NO: 85910, SEQ ID NO: 85911, SEQ ID NO: 85956, SEQ ID NO: 86010, SEQ ID NO: 86041, SEQ ID NO: 86086, SEQ ID NO: 86099, SEQ ID NO: 86285, SEQ ID NO: 86287, SEQ ID NO: 86292, SEQ ID NO: 86339, SEQ ID NO: 86351, SEQ ID NO: 86353, SEQ ID NO: 86364, SEQ ID NO: 86383, SEQ ID NO: 86418, SEQ ID NO: 86441, SEQ ID NO: 86442, SEQ ID NO: 86447, SEQ ID NO: 86448, SEQ ID NO: 86462, SEQ ID NO: 86470, SEQ ID NO: 86497, SEQ ID NO: 86518, SEQ ID NO: 86525, SEQ ID NO: 86529, SEQ ID NO: 86530, SEQ ID NO: 86531, SEQ ID NO: 86533, SEQ ID NO: 86552, SEQ ID NO: 86577, SEQ ID NO: 86580, SEQ ID NO: 86587, SEQ ID NO: 86606, SEQ ID NO: 86660, SEQ ID NO: 86695, SEQ ID NO: 86725, SEQ ID NO: 86753, SEQ ID NO: 86755, SEQ ID NO: 86756, SEQ ID NO: 86760, SEQ ID NO: 86797, SEQ ID NO: 86799, SEQ ID NO: 86806, SEQ ID NO: 86868, SEQ ID NO: 86898, SEQ ID NO: 86914, SEQ ID NO: 86935, SEQ ID NO: 86936, SEQ ID NO: 86954, SEQ ID NO: 86958, SEQ ID NO: 86992, SEQ ID NO: 87014, SEQ ID NO: 87055, SEQ ID NO: 87068, SEQ ID NO: 87077, SEQ ID NO: 87099, SEQ ID NO: 87104, SEQ ID NO: 87121, SEQ ID NO: 87122, SEQ ID NO: 87129, SEQ ID NO: 87135, SEQ ID NO: 87143, SEQ ID NO: 87148, SEQ ID NO: 87183, SEQ ID NO: 87196, SEQ ID NO: 87202, SEQ ID NO: 87208, SEQ ID NO: 87234, SEQ ID NO: 87252, SEQ ID NO: 87292, SEQ ID NO: 87301, SEQ ID NO: 87325, SEQ ID NO: 87344, SEQ ID NO: 87367, SEQ ID NO: 87373, SEQ ID NO: 87389, SEQ ID NO: 87390, SEQ ID NO: 87409, SEQ ID NO: 87430, SEQ ID NO: 87460, SEQ ID NO: 87483, SEQ ID NO: 87485, SEQ ID NO: 87488, SEQ ID NO: 87519, SEQ ID NO: 87535, SEQ ID NO: 87585, SEQ ID NO: 87591, SEQ ID NO: 87592, SEQ ID NO: 87653, SEQ ID NO: 87662, SEQ ID NO: 87672, SEQ ID NO: 87690, SEQ ID NO: 87692, SEQ ID NO: 87710, SEQ ID NO: 87719, SEQ ID NO: 87721, SEQ ID NO: 87726, SEQ ID NO: 87748, SEQ ID NO: 87749, SEQ ID NO: 87757, SEQ ID NO: 87779, SEQ ID NO: 87781, SEQ ID NO: 87782, SEQ ID NO: 87784, SEQ ID NO: 87791, SEQ ID NO: 87800, SEQ ID NO: 87815, SEQ ID NO: 87848, SEQ ID NO: 87869, SEQ ID NO: 87883, SEQ ID NO: 87892, SEQ ID NO: 87916, SEQ ID NO: 87954, SEQ ID NO: 87969, SEQ ID NO: 87970, SEQ ID NO: 87984, SEQ ID NO: 87998, SEQ ID NO: 88014, SEQ ID NO: 88069, SEQ ID NO: 88076, SEQ ID NO: 88089, SEQ ID NO: 88110, SEQ ID NO: 88175, SEQ ID NO: 88205, SEQ ID NO: 88211, SEQ ID NO: 88229, SEQ ID NO: 88251, SEQ ID NO: 88255, SEQ ID NO: 88286, SEQ ID NO: 88365, SEQ ID NO: 88370, SEQ ID NO: 88378, SEQ ID NO: 88385, SEQ ID NO: 88399, SEQ ID NO: 88412, SEQ ID NO: 88422, SEQ ID NO: 88433, SEQ ID NO: 88516, SEQ ID NO: 93347, SEQ ID NO: 93351, SEQ ID NO: 93352, SEQ ID NO: 93355, SEQ ID NO: 93361, SEQ ID NO: 93363, SEQ ID NO: 93364, SEQ ID NO: 93368, SEQ ID NO: 93369, SEQ ID NO: 93427, SEQ ID NO: 93483, SEQ ID NO: 93491, SEQ ID NO: 93609, SEQ ID NO: 93610, SEQ ID NO: 93637, SEQ ID NO: 93719, SEQ ID NO: 93723, SEQ ID NO: 93724, SEQ ID NO: 93727, SEQ ID NO: 93733, SEQ ID NO: 93735, SEQ ID NO: 93736, SEQ ID NO: 93740, SEQ ID NO: 93741, SEQ ID NO: 93799, SEQ ID NO: 93855, SEQ ID NO: 93863, SEQ ID NO: 93981, SEQ ID NO: 93982, SEQ ID NO: 94009, SEQ ID NO: 94091, SEQ ID NO: 94095, SEQ ID NO: 94096, SEQ ID NO: 94099, SEQ ID NO: 94105, SEQ ID NO: 94107, SEQ ID NO: 94108, SEQ ID NO: 94111, SEQ ID NO: 94112, SEQ ID NO: 94170, SEQ ID NO: 94226, SEQ ID NO: 94234, SEQ ID NO: 94352, SEQ ID NO: 94353, SEQ ID NO: 94380, SEQ ID NO: 94462, SEQ ID NO: 94466, SEQ ID NO: 94467, SEQ ID NO: 94470, SEQ ID NO: 94476, SEQ ID NO: 94478, SEQ ID NO: 94479, SEQ ID NO: 94483, SEQ ID NO: 94484, SEQ ID NO: 94542, SEQ ID NO: 94598, SEQ ID NO: 94606, SEQ ID NO: 94724, SEQ ID NO: 94725, SEQ ID NO: 94752, SEQ ID NO: 94834, SEQ ID NO: 94838, SEQ ID NO: 94839, SEQ ID NO: 94842, SEQ ID NO: 94848, SEQ ID NO: 94850, SEQ ID NO: 94851, SEQ ID NO: 94855, SEQ ID NO: 94856, SEQ ID NO: 94913, SEQ ID NO: 94969, SEQ ID NO: 94977,

SEQ ID NO: 95095, SEQ ID NO: 95096, SEQ ID NO: 95123, SEQ ID NO: 95205, SEQ ID NO: 95209, SEQ ID NO: 95210, SEQ ID NO: 95213, SEQ ID NO: 95219, SEQ ID NO: 95221, SEQ ID NO: 95222, SEQ ID NO: 95226, SEQ ID NO: 95227, SEQ ID NO: 95284, SEQ ID NO: 95340, SEQ ID NO: 95348, SEQ ID NO: 95466, SEQ ID NO: 95467, SEQ ID NO: 95494, SEQ ID NO: 95576, SEQ ID NO: 95579, SEQ ID NO: 95580, SEQ ID NO: 95583, SEQ ID NO: 95589, SEQ ID NO: 95591, SEQ ID NO: 95592, SEQ ID NO: 95596, SEQ ID NO: 95597, SEQ ID NO: 95655, SEQ ID NO: 95711, SEQ ID NO: 95719, SEQ ID NO: 95837, SEQ ID NO: 95838, SEQ ID NO: 95865, SEQ ID NO: 95947, SEQ ID NO: 95951, SEQ ID NO: 95952, SEQ ID NO: 95955, SEQ ID NO: 95961, SEQ ID NO: 95962, SEQ ID NO: 95963, SEQ ID NO: 95967, SEQ ID NO: 95968, SEQ ID NO: 96026, SEQ ID NO: 96082, SEQ ID NO: 96090, SEQ ID NO: 96208, SEQ ID NO: 96209, SEQ ID NO: 96236, SEQ ID NO: 96318, SEQ ID NO: 96322, SEQ ID NO: 96323, SEQ ID NO: 96326, SEQ ID NO: 96332, SEQ ID NO: 96334, SEQ ID NO: 96335, SEQ ID NO: 96339, SEQ ID NO: 96340, SEQ ID NO: 96398, SEQ ID NO: 96454, SEQ ID NO: 96462, SEQ ID NO: 96580, SEQ ID NO: 96581, SEQ ID NO: 96608, SEQ ID NO: 96690, SEQ ID NO: 96694, SEQ ID NO: 96695, SEQ ID NO: 96698, SEQ ID NO: 96704, SEQ ID NO: 96706, SEQ ID NO: 96707, SEQ ID NO: 96711, SEQ ID NO: 96712, SEQ ID NO: 96770, SEQ ID NO: 96826, SEQ ID NO: 96834, SEQ ID NO: 96952, SEQ ID NO: 96953, SEQ ID NO: 96980, SEQ ID NO: 97062, SEQ ID NO: 97066, SEQ ID NO: 97067, SEQ ID NO: 97070, SEQ ID NO: 97076, SEQ ID NO: 97078, SEQ ID NO: 97079, SEQ ID NO: 97083, SEQ ID NO: 97084, SEQ ID NO: 97142, SEQ ID NO: 97198, SEQ ID NO: 97206, SEQ ID NO: 97324, SEQ ID NO: 97325, SEQ ID NO: 97352, SEQ ID NO: 97434, SEQ ID NO: 97438, SEQ ID NO: 97439, SEQ ID NO: 97442, SEQ ID NO: 97448, SEQ ID NO: 97450, SEQ ID NO: 97451, SEQ ID NO: 97455, SEQ ID NO: 97456, SEQ ID NO: 97514, SEQ ID NO: 97570, SEQ ID NO: 97578, SEQ ID NO: 97696, SEQ ID NO: 97697, SEQ ID NO: 97724, SEQ ID NO: 97806, SEQ ID NO: 97810, SEQ ID NO: 97811, SEQ ID NO: 97814, SEQ ID NO: 97820, SEQ ID NO: 97822, SEQ ID NO: 97823, SEQ ID NO: 97827, SEQ ID NO: 97828, SEQ ID NO: 97886, SEQ ID NO: 97942, SEQ ID NO: 97950, SEQ ID NO: 98068, SEQ ID NO: 98069, SEQ ID NO: 98096, SEQ ID NO: 98178, SEQ ID NO: 98182, SEQ ID NO: 98183, SEQ ID NO: 98186, SEQ ID NO: 98192, SEQ ID NO: 98194, SEQ ID NO: 98195, SEQ ID NO: 98198, SEQ ID NO: 98199, SEQ ID NO: 98257, SEQ ID NO: 98313, SEQ ID NO: 98321, SEQ ID NO: 98439, SEQ ID NO: 98440, SEQ ID NO: 98467, SEQ ID NO: 98549, SEQ ID NO: 98553, SEQ ID NO: 98554, SEQ ID NO: 98557, SEQ ID NO: 98563, SEQ ID NO: 98565, SEQ ID NO: 98566, SEQ ID NO: 98570, SEQ ID NO: 98571, SEQ ID NO: 98628, SEQ ID NO: 98684, SEQ ID NO: 98692, SEQ ID NO: 98810, SEQ ID NO: 98811, SEQ ID NO: 98838, SEQ ID NO: 98920, SEQ ID NO: 98924, SEQ ID NO: 98925, SEQ ID NO: 98928, SEQ ID NO: 98933, SEQ ID NO: 98935, SEQ ID NO: 98936, SEQ ID NO: 98940, SEQ ID NO: 98941, SEQ ID NO: 98999, SEQ ID NO: 99055, SEQ ID NO: 99063, SEQ ID NO: 99181, SEQ ID NO: 99182, SEQ ID NO: 99209, SEQ ID NO: 99291, SEQ ID NO: 99295, SEQ ID NO: 99296, SEQ ID NO: 99299, SEQ ID NO: 99305, SEQ ID NO: 99307, SEQ ID NO: 99308, SEQ ID NO: 99312, SEQ ID NO: 99313, SEQ ID NO: 99371, SEQ ID NO: 99427, SEQ ID NO: 99435, SEQ ID NO: 99553, SEQ ID NO: 99554, SEQ ID NO: 99581, SEQ ID NO: 99663, SEQ ID NO: 99667, SEQ ID NO: 99668, SEQ ID NO: 99671, SEQ ID NO: 99677, SEQ ID NO: 99679, SEQ ID NO: 99680, SEQ ID NO: 99684, SEQ ID NO: 99685, SEQ ID NO: 99743, SEQ ID NO: 99799, SEQ ID NO: 99807, SEQ ID NO: 99925, SEQ ID NO: 99926, SEQ ID NO: 99953, SEQ ID NO: 100035, SEQ ID NO: 100039, SEQ ID NO: 100040, SEQ ID NO: 100043, SEQ ID NO: 100048, SEQ ID NO: 100050, SEQ ID NO: 100051, SEQ ID NO: 100055, SEQ ID NO: 100056, SEQ ID NO: 100114, SEQ ID NO: 100170, SEQ ID NO: 100178, SEQ ID NO: 100296, SEQ ID NO: 100297 and SEQ ID NO: 100324.

**12.** A medical composition comprising at least one pharmaceutical association according to any one of claims 1 to 11 and at least one medically acceptable excipient, carrier or vehicle.

**13.** A pharmaceutical association according to any one of claims 1 to 11, or a medical composition according to claim 12, for use in the treatment, prevention and/or diagnostic of a neoplastic disease.

**14.** A method for converting a neoplastic cell into a non-neoplastic cell, in-vitro or ex-vivo, said method comprising administering to a neoplastic cell an effective amount of a pharmaceutical association or composition as defined in any one of claims 1 to 12.

**15.** A pharmaceutical association according to any one of claims 1 to 10, wherein said growth factor receptor-binding compound comprises a peptide selected from the group consisting of peptides of SEQ ID NOs as defined in claim 11.

FIGURE 1

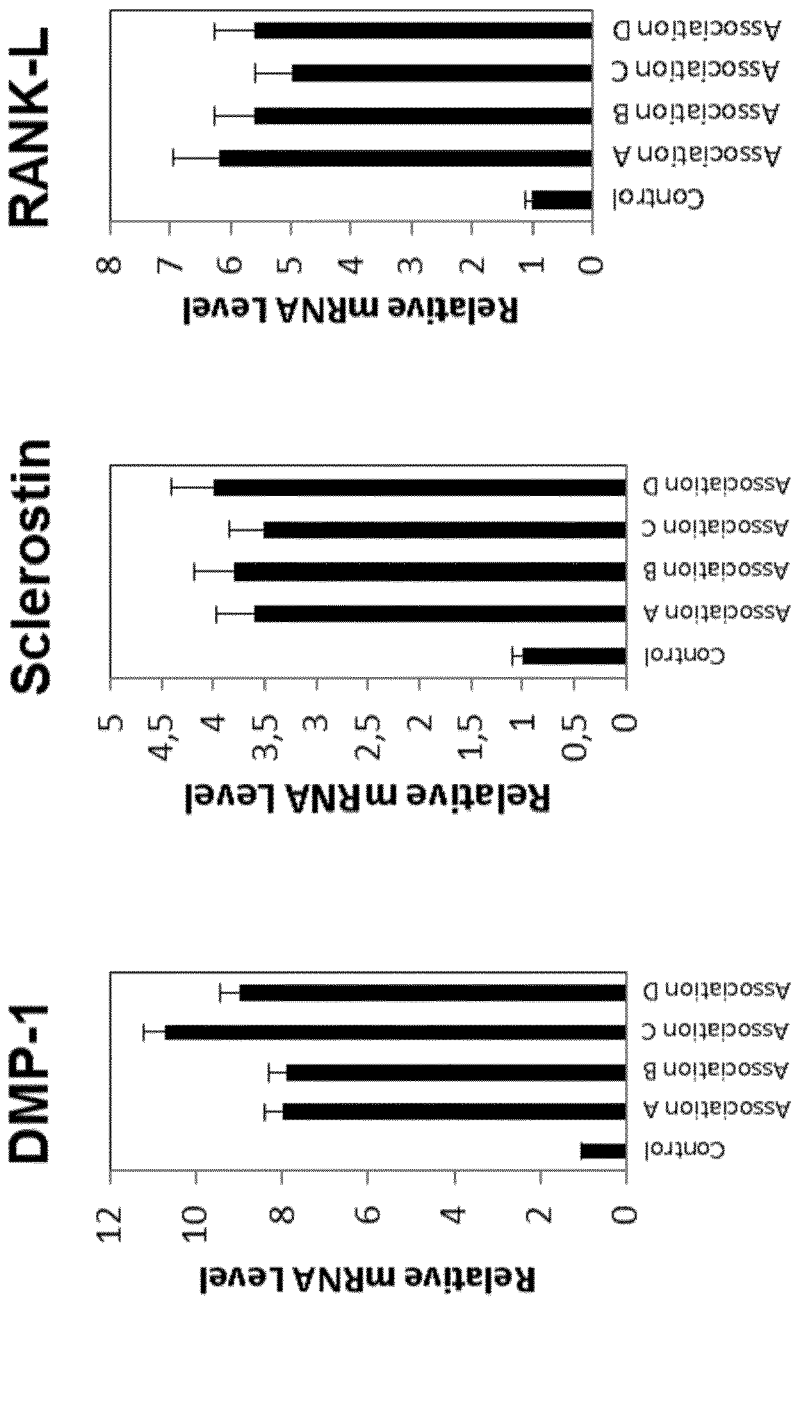
RANK-L
Sclerostin
DMP-1
Relative mRNA Level
Control
Association A
Association B
Association C
Association D
0
1
2
3
4
5
6
7
8
0,5
1,5
2,5
3,5
4,5
10
12

FIGURE 2

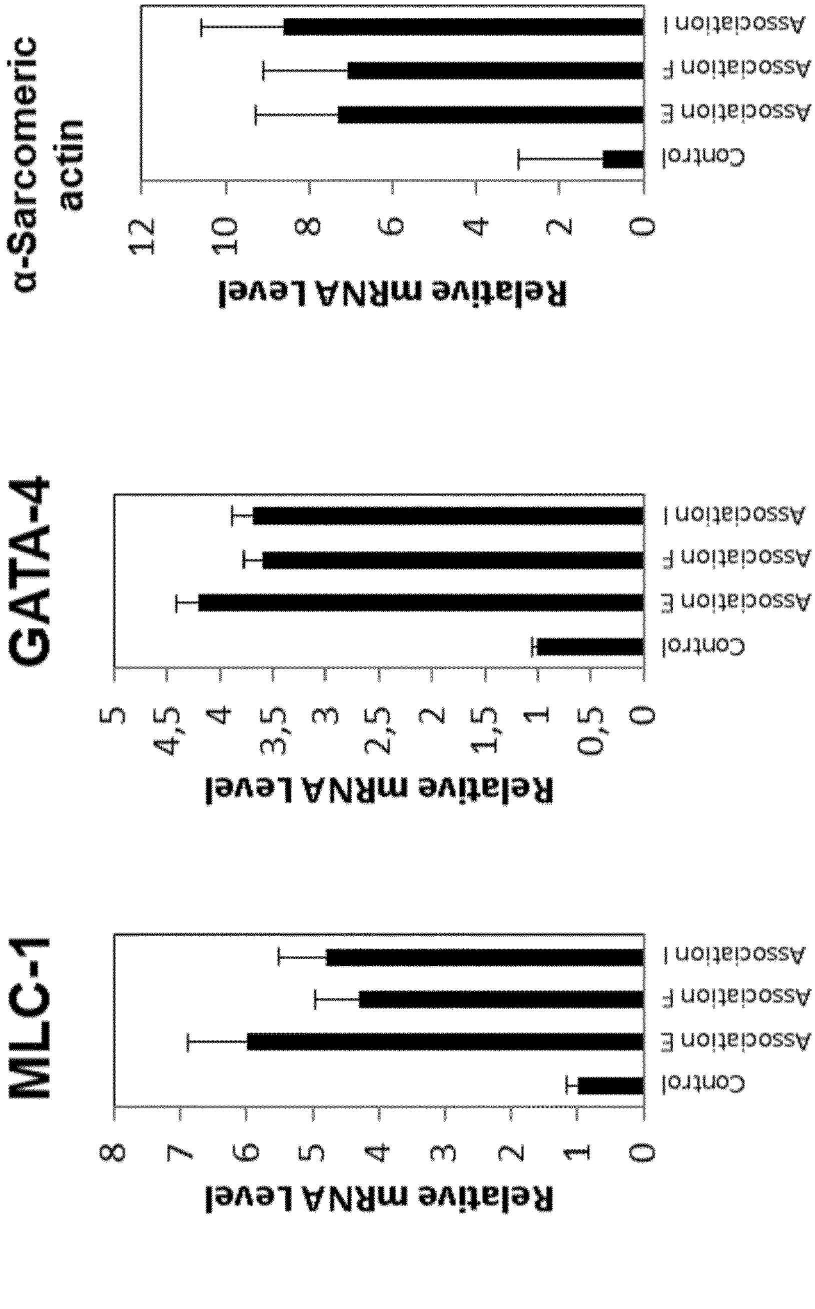
α-Sarcomeric actin
GATA-4
MLC-1
Relative mRNA Level
Control
Association E
Association F
Association I
0
2
4
6
8
10
12
0,5
1
1,5
2
2,5
3
3,5
4
4,5
5
7

FIGURE 3

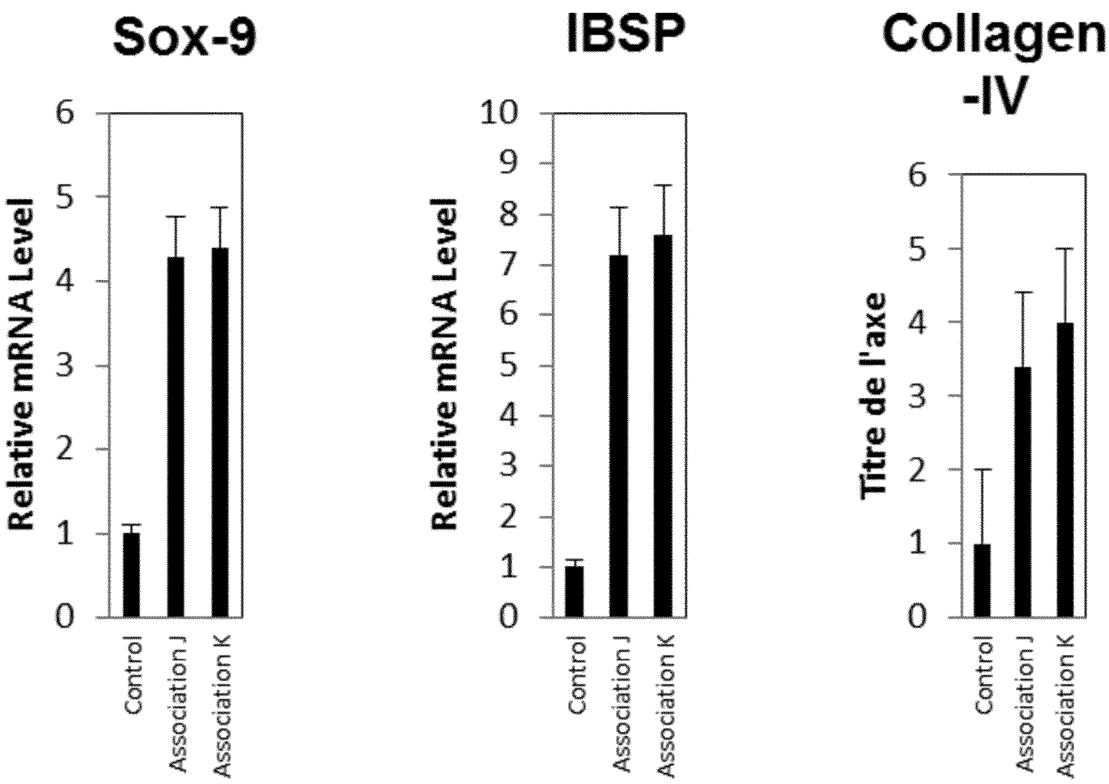
Sox-9
Relative mRNA Level
0
1
2
3
4
5
6
Control
Association J
Association K
IBSP
Relative mRNA Level
0
1
2
3
4
5
6
7
8
9
10
Control
Association J
Association K
Collagen -IV
Titre de l'axe
0
1
2
3
4
5
6
Control
Association J
Association K

FIGURE 4

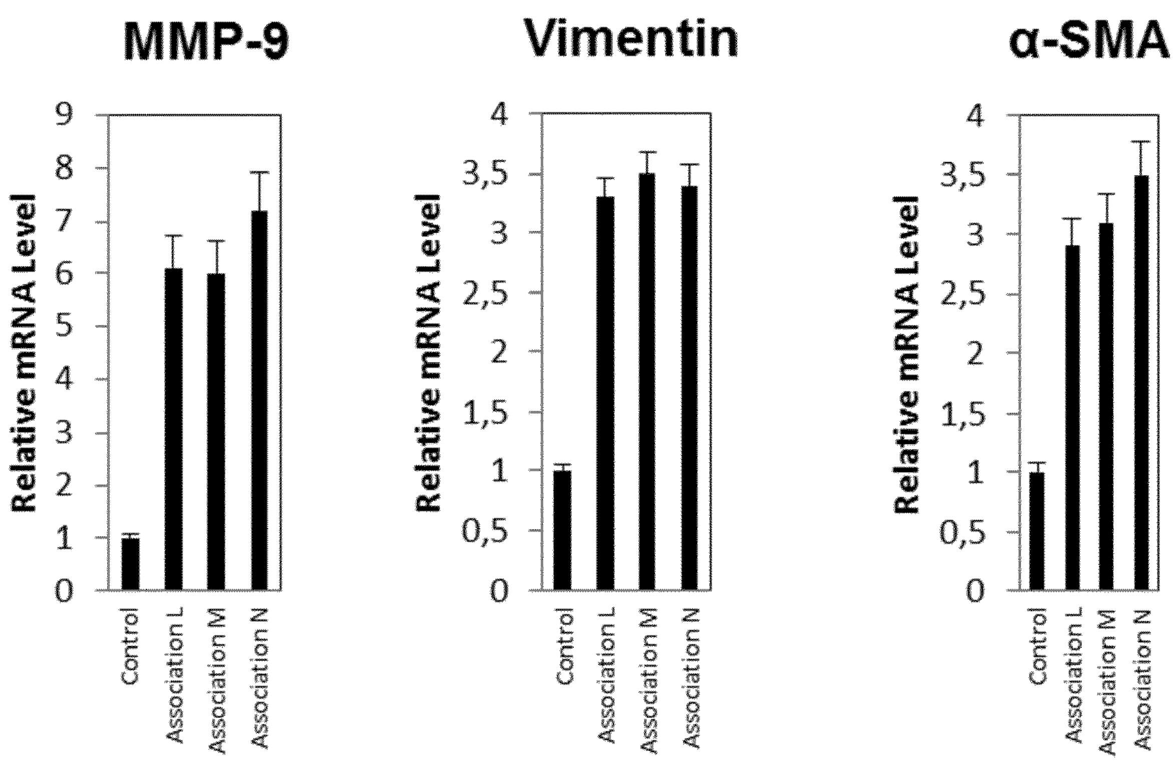
MMP-9
Vimentin
α-SMA
Relative mRNA Level
9
8
7
6
5
4
3
2
1
0
4
3,5
3
2,5
2
1,5
1
0,5
Control
Association L
Association M
Association N

FIGURE 5

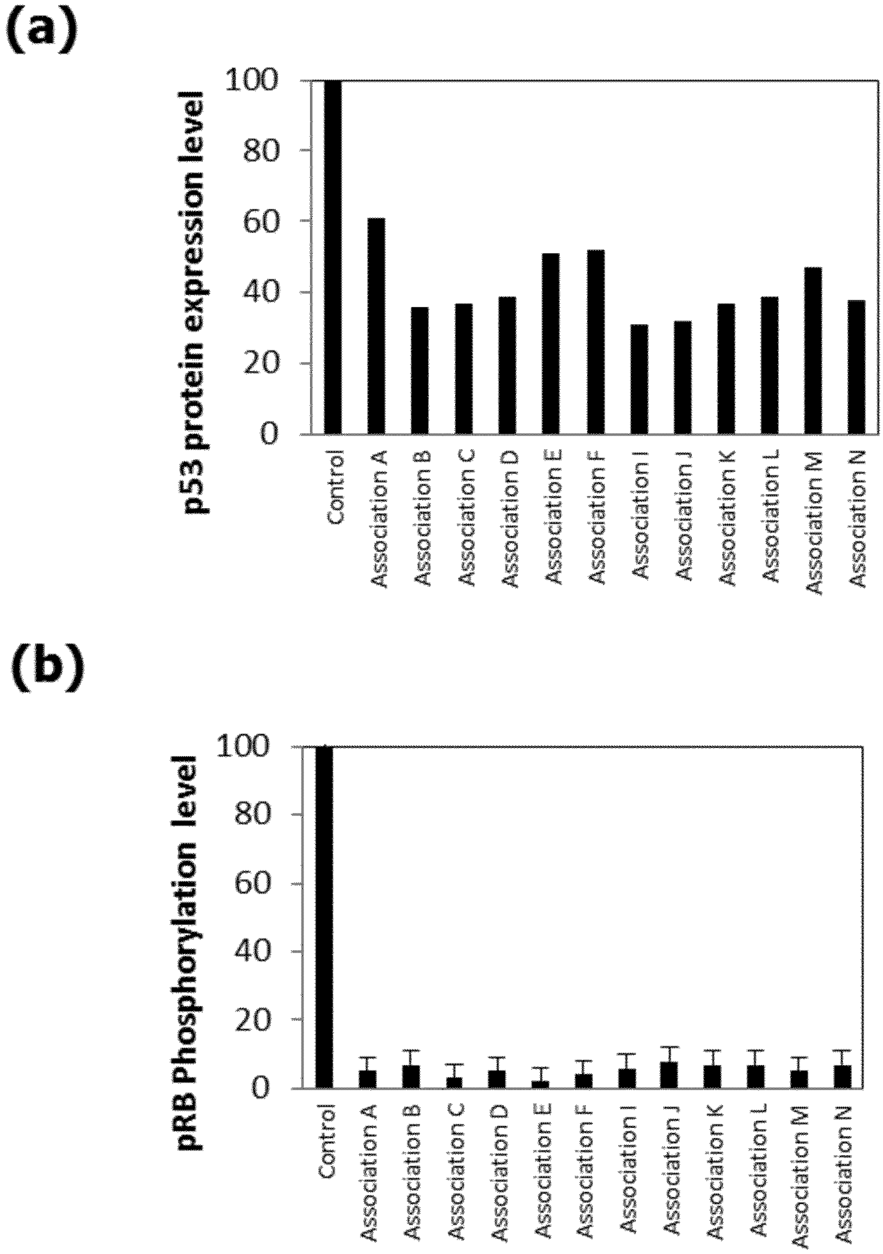
(a)
p53 protein expression level
100
80
60
40
20
0
Control
Association A
Association B
Association C
Association D
Association E
Association F
Association I
Association J
Association K
Association L
Association M
Association N
(b)
pRB Phosphorylation level
100
80
60
40
20
0
Control
Association A
Association B
Association C
Association D
Association E
Association F
Association I
Association J
Association K
Association L
Association M
Association N

FIGURE 6

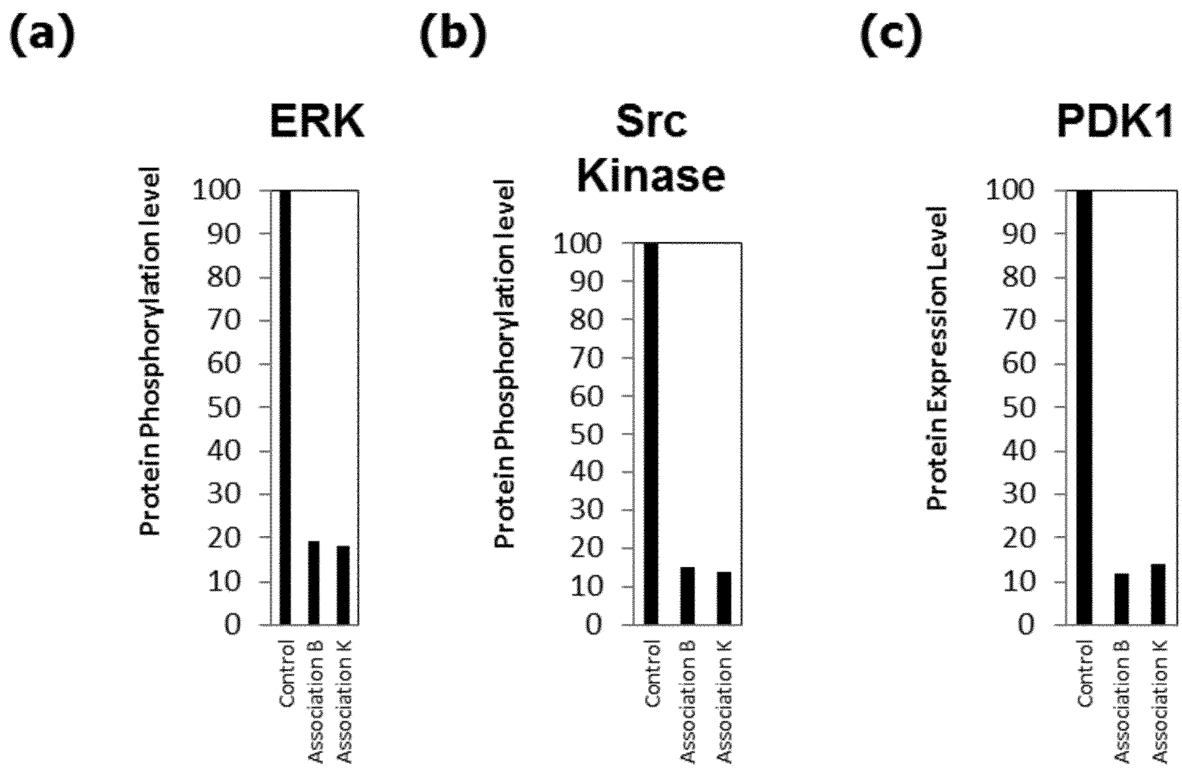
(a)
(b)
(c)
ERK
Src Kinase
PDK1
Protein Phosphorylation level
Protein Phosphorylation level
Protein Expression Level
0
10
20
30
40
50
60
70
80
90
100
Control
Association B
Association K

FIGURE 7

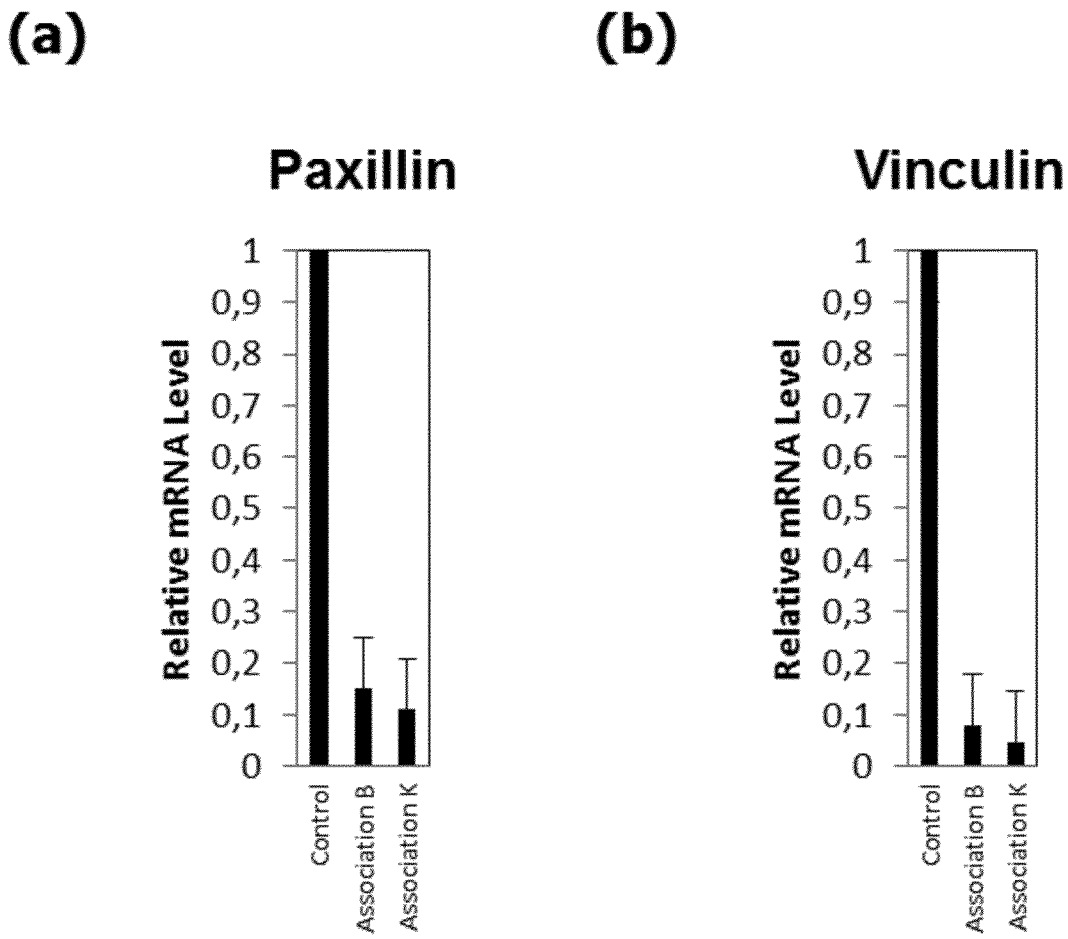
(a)
(b)
Paxillin
Vinculin
Relative mRNA Level
1
0,9
0,8
0,7
0,6
0,5
0,4
0,3
0,2
0,1
0
Control
Association B
Association K

FIGURE 8

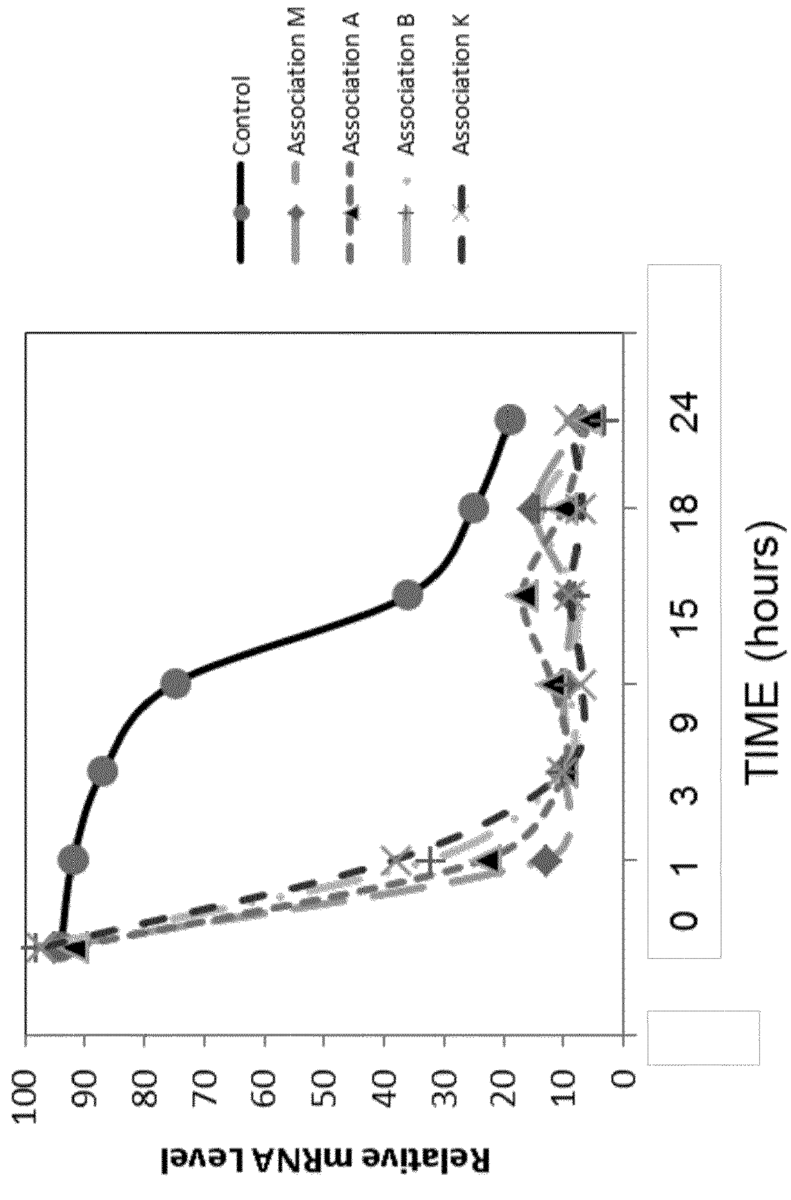
Relative mRNA Level
100
90
80
70
60
50
40
30
20
10
0
TIME (hours)
0
1
3
9
15
18
24
Control
Association M
Association A
Association B
Association K

FIGURE 9

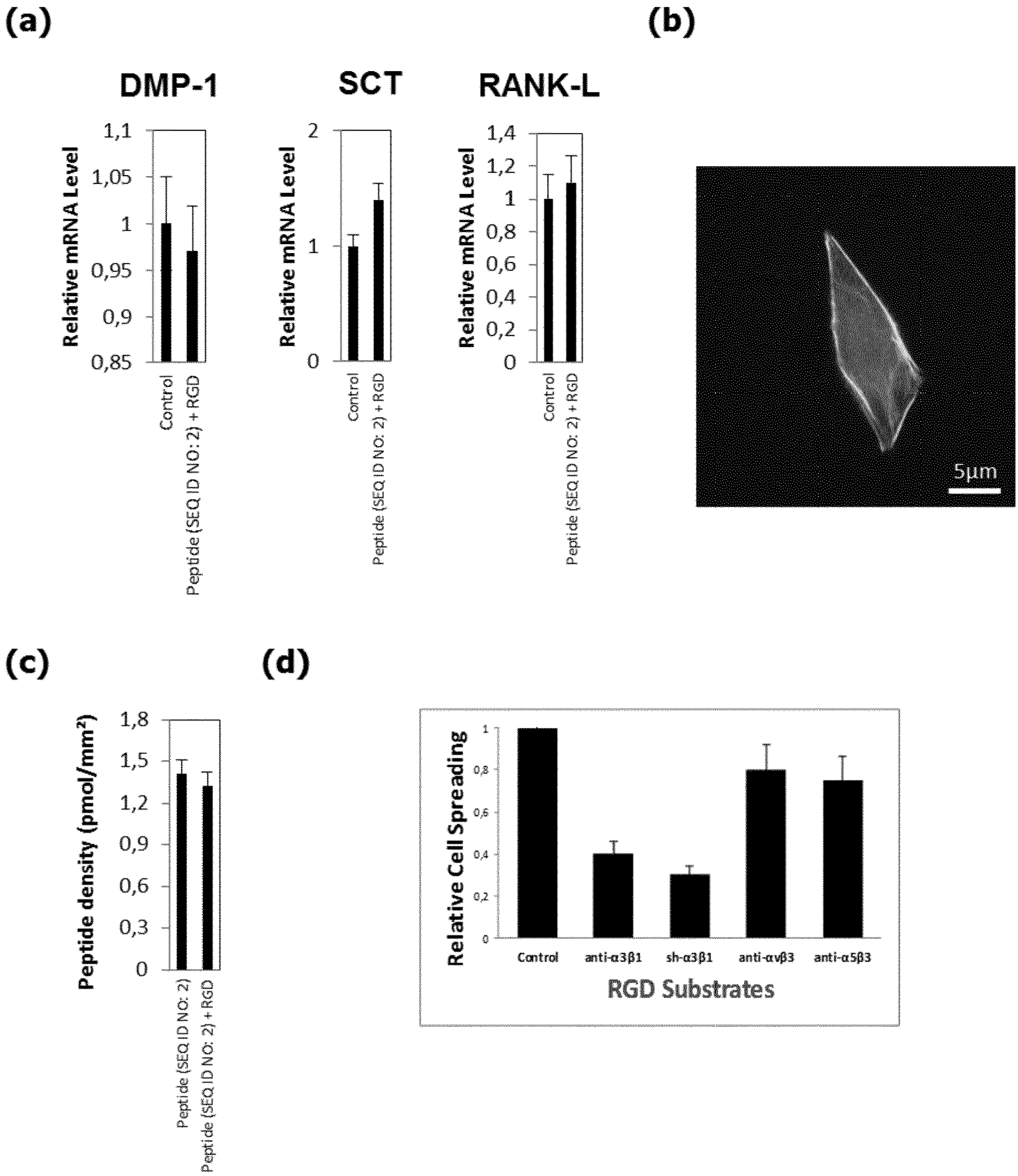
(a)
(b)
DMP-1
SCT
RANK-L
Relative mRNA Level
1,1
1,05
1
0,95
0,9
0,85
2
1
0
1,4
1,2
1
0,8
0,6
0,4
0,2
0
Control
Peptide (SEQ ID NO: 2) + RGD
5µm
(c)
(d)
Peptide density (pmol/mm²)
1,8
1,5
1,2
0,9
0,6
0,3
0
Peptide (SEQ ID NO: 2)
Peptide (SEQ ID NO: 2) + RGD
Relative Cell Spreading
1
0,8
0,6
0,4
0,2
0
Control
anti-α3β1
sh-α3β1
anti-αvβ3
anti-α5β3
RGD Substrates

FIGURE 10

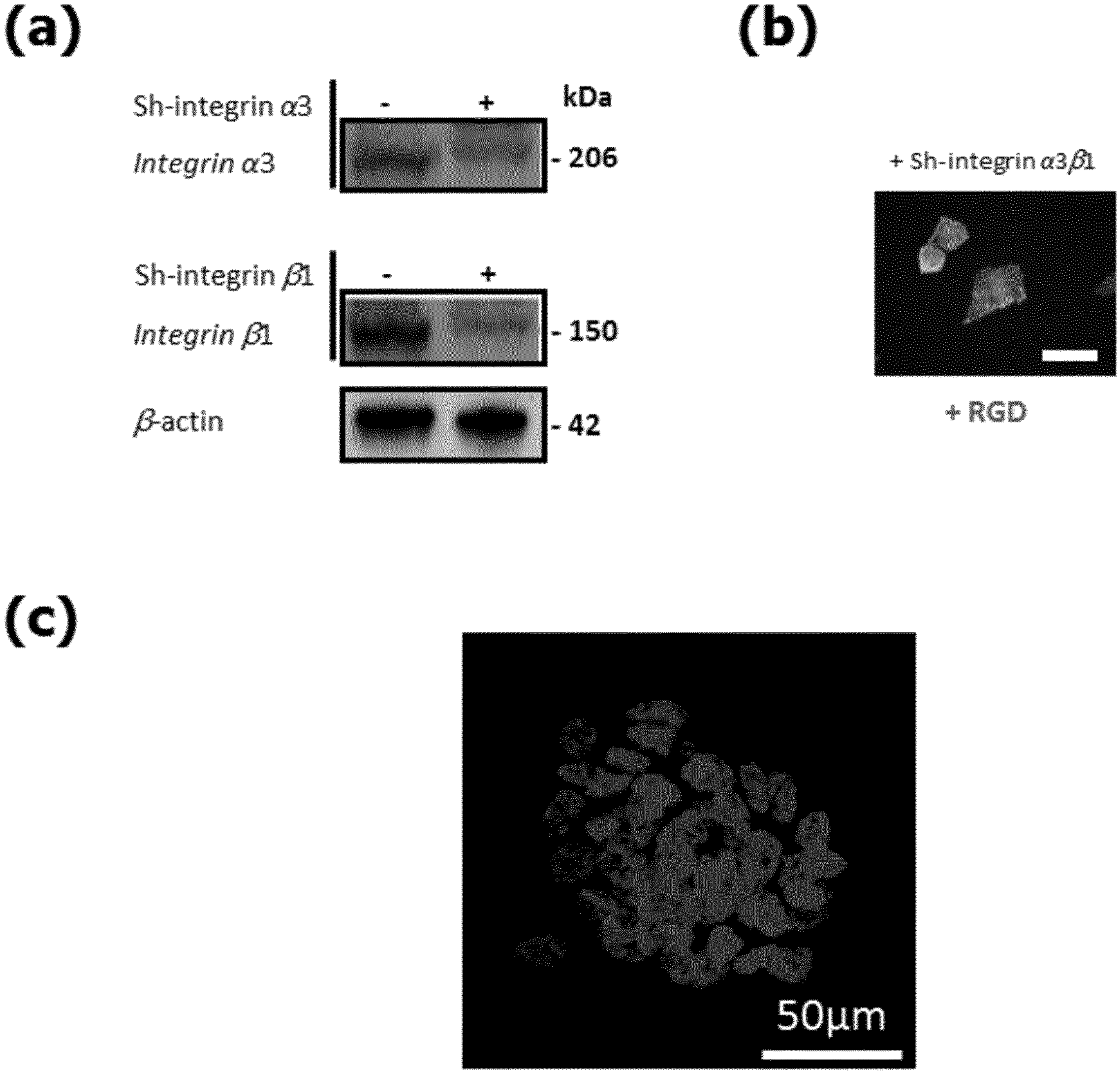
(a)
Sh-integrin α3
-
+
kDa
Integrin α3
- 206
Sh-integrin β1
-
+
Integrin β1
- 150
β-actin
- 42
(b)
+ Sh-integrin α3β1
+ RGD
(c)
50µm

## FIGURE 11

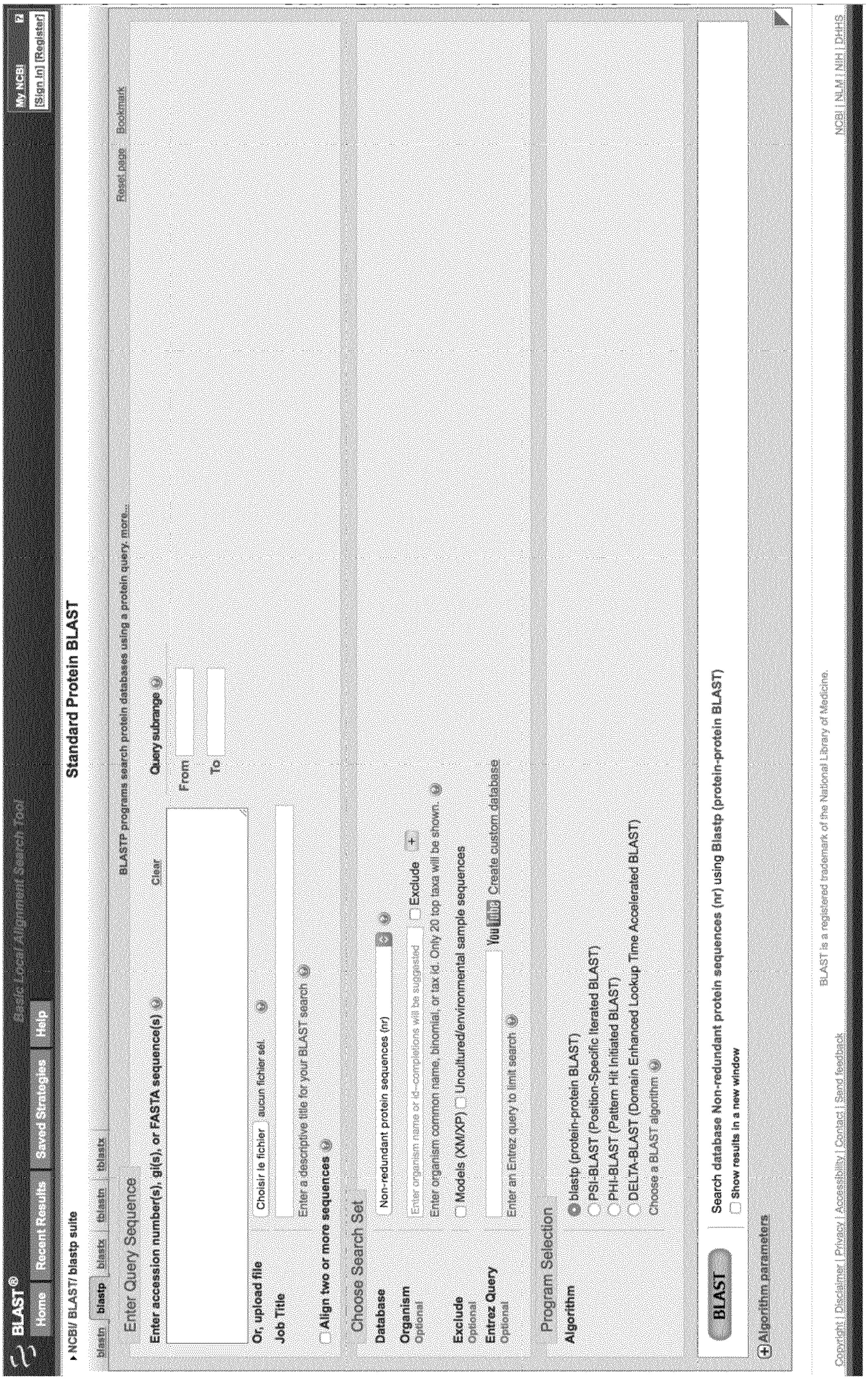
BLAST®
Basic Local Alignment Search Tool
Home
Recent Results
Saved Strategies
Help
My NCBI
[Sign In] [Register]
NCBI/ BLAST/ blastp suite
Standard Protein BLAST
blastn
blastp
blastx
tblastn
tblastx
Reset page
Bookmark
BLASTP programs search protein databases using a protein query. more...
Enter Query Sequence
Enter accession number(s), gi(s), or FASTA sequence(s)
Clear
Query subrange
From
To
Or, upload file
Choisir le fichier
aucun fichier sél.
Job Title
Enter a descriptive title for your BLAST search
Align two or more sequences
Choose Search Set
Database
Non-redundant protein sequences (nr)
Organism
Optional
Enter organism name or id--completions will be suggested
Exclude
Enter organism common name, binomial, or tax id. Only 20 top taxa will be shown.
Exclude
Optional
Models (XM/XP)
Uncultured/environmental sample sequences
Entrez Query
Optional
Create custom database
Enter an Entrez query to limit search
Program Selection
Algorithm
blastp (protein-protein BLAST)
PSI-BLAST (Position-Specific Iterated BLAST)
PHI-BLAST (Pattern Hit Initiated BLAST)
DELTA-BLAST (Domain Enhanced Lookup Time Accelerated BLAST)
Choose a BLAST algorithm
BLAST
Search database Non-redundant protein sequences (nr) using Blastp (protein-protein BLAST)
Show results in a new window
Algorithm parameters
BLAST is a registered trademark of the National Library of Medicine.
Copyright | Disclaimer | Privacy | Accessibility | Contact | Send feedback
NCBI | NLM | NIH | DHHS

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 4816567 A
- US 5204244 A
- US 20020159986 A1


### Non-patent literature cited in the description


- **GREENE.** Protective Groups in Organic Synthesis. Wiley, 2007
- **HOJO H.** Recent progress in the chemical synthesis of proteins. *Curr Opin Struct Biol.,* 2014, vol. 26C, 16-23
- **SARANYA CHANDRUDU et al.** Chemical Methods for Peptide and Protein Production. *Molecules,* 2013, vol. 18, 4373-4388
- *Scientific World Journal,* 2013, vol. 2013, 732340
- *Curr Opin Chem Biol.,* 1998, vol. 2 (6), 733-42
- *J Pept Sci.,* 1999, vol. 5 (5), 203-20
- *J Pept Sci.,* 2008, vol. 14 (1), 2-43
- **KOHLER et al.** *Nature,* 1975, vol. 256, 495
- **CLACKSON et al.** *Nature,* 1991, vol. 352, 624-628
- **MARKS et al.** *J. Mol. Biol.,* 1991, vol. 222, 581-597
- **MORRISON, S. L. et al.** *Proc. Natl. Acad. Sci. USA,* 1984, vol. 81, 6851-6855
- **KELLERMANN, S. A. et al.** *Curr Opin Biotechnol.,* 2002, vol. 13, 593-597
- **KABAT et al.** Sequences of Proteins of Immunological Interest. Public Health Service, National Institutes of Health, 1991
- *Protein Sci.,* 2003, vol. 12 (9), 2001-14
- *J. Computat. Chem.,* 2005, vol. 26, 1668-1688
- **QIANG WANG.** SCWRL and MolIDE: computer programs for side-chain conformation prediction and homology modeling. *Nature Protocols,* 2008, vol. 3 (12
- **HESS B ; KUTZNER C ; VAN DER SPOEL D ; LINDAHL E.** GROMACS 4: Algorithms for Highly Efficient, Load-Balanced, and Scalable Molecular Simulation. *J Chem Theory Comput,* 2008, vol. 4 (2), 435
- **MARK A. LEMMON ; JOSEPH SCHLESSINGER.** Cell Signaling by Receptor Tyrosine Kinases. *Cell,* 2010, vol. 141 (7), 1117-1134
- **LIVAK, K. J. ; T. D. SCHMITTGEN.** Analysis of relative gene expression data using real-time quantitative PCR and the 2(-Delta Delta C(T)) Method. *Methods,* 2001, vol. 25 (4), 402-408
- **CHIGAEV, A. ; BURANDA, T. ; DWYER, D. C. ; PROSSNITZ, E. R. ; SKLAR, L. A.** FRET detection of cellular $\alpha$4-integrin conformational activation. *Biophys., J.,* 2003, vol. 85, 3951-3962
- L-Asparaginase: A Promising Enzyme for Treatment of Acute Lymphoblastic Leukiemia. *People's Journal of Scientific Research,* January 2012, vol. 5 (1
- Remington's Pharmaceutical Sciences. Mack Publishing Company, 1985, 1418
- Pharmaceutical Salts: Properties, Selection, and Use. Wiley-VCH, 2008
- Prodrugs as Novel Delivery Systems. **T. HIGUCHI ; W. STELLA.** ACS Symposium Series, and Bioreversible Carriers in Drug Design. Pergamon Press, 1987, vol. 14
- Remington's Pharmaceutical Sciences. Mack Publishing Company, 1995
- The CTFA Cosmetic Ingredient Handbook. 1992
- **WHITE AC ; LOWRY WE.** Refining the role for adult stem cells as cancer cells of origin. *Trends Cell Biol.,* 18 September 2014
- **TANNISHTHA REYA et al.** review article Stem cells, cancer, and cancer stem cells. *Nature,* 01 November 2001, vol. 414, 105-111